# EUROPEAN PATENT SPECIFICATION

(11) **EP 4 013 385 B1**
(45) Date of publication and mention of the grant of the patent: **03.07.2024**
(21) Application number: 20765121.7
(22) Date of filing: 14.08.2020
(51) Int. Cl.: A61K 9/00, A61K 9/127

(54) **IMPROVED LIPID NANOPARTICLES FOR DELIVERY OF NUCLEIC ACIDS**
VERBESSERTE LIPIDNANOPARTIKEL ZUR VERABREICHUNG VON NUKLEINSÄUREN
NANOPARTICULES LIPIDIQUES AMÉLIORÉES POUR L'ADMINISTRATION D'ACIDES NUCLÉIQUES

(30) Priority: 14.08.2019 US 201962886894 P
(43) Date of publication of application: 22.06.2022
(73) Proprietor: Acuitas Therapeutics, Inc., Vancouver, British Columbia V6T 1W5 (CA)
(72) Inventor: TAM, Ying, K., Vancouver, British Columbia V6S 1C3 (CA); LIN, Paulo, Jia, Ching, Vancouver, British Columbia V5S 1Y4 (CA); SEMPLE, Sean, Vancouver, British Columbia V6T 1W5 (CA); BARBOSA, Christopher, J., Coquitlam, British Columbia V3J 4Z8 (CA)
(74) Representative: Grünecker Patent- und Rechtsanwälte PartG mbB
(86) International application number: PCT/US2020/046407
(87) International publication number: WO 2021/030701

(56) References cited:
- WO-A1-2018/081480
- WO-A1-2018/107026
- WO-A1-2019/089828
- US-A1- 2015 203 446

## Description

### Technical Field

Embodiments of the present invention generally relate to lipid nanoparticles (LNPs) having improved properties. The LNPs are useful for facilitating the intracellular delivery of therapeutic agents, such as nucleic acids (e.g., oligonucleotides, messenger RNA), to primates, including humans.

### Description of the Related Art

There are many challenges associated with the delivery of nucleic acids to affect a desired response in a biological system. Nucleic acid based therapeutics have enormous potential but there remains a need for more effective delivery of nucleic acids to appropriate sites within a cell or organism in order to realize this potential. Therapeutic nucleic acids include, e.g., messenger RNA (mRNA), antisense oligonucleotides, ribozymes, DNAzymes, plasmids, immune stimulating nucleic acids, antagomir, antimir, mimic, supermir, and aptamers. Some nucleic acids, such as mRNA or plasmids, can be used to effect expression of specific cellular products as would be useful in the treatment of, for example, diseases related to a deficiency of a protein or enzyme. The therapeutic applications of translatable nucleotide delivery are extremely broad as constructs can be synthesized to produce any chosen protein sequence, whether or not indigenous to the system. The expression products of the nucleic acid can augment existing levels of protein, replace missing or non-functional versions of a protein, or introduce new protein and associated functionality in a cell or organism.

However, problems currently face the use of oligonucleotides in therapeutic contexts. First, free RNAs are susceptible to nuclease digestion in plasma. Second, free RNAs have limited ability to gain access to the intracellular compartment where the relevant translation machinery resides. Lipid nanoparticles formed from cationic lipids with other lipid components, such as neutral lipids, cholesterol, PEG, PEGylated lipids, and oligonucleotides have been used to protect the RNAs in plasma and facilitate the cellular uptake of the oligonucleotides.

WO2018081480A1 discloses lipid nanoparticle comprising 40-50 mol.% cationic lipid, neutral lipid, steroid, polymer conjugated lipid, and atherapeutic agent or its salt encapsulated within or associated with lipid nanoparticle.

Additionally, while lipid nanoparticle formulations have shown tremendous promise for enhancing nucleic acid therapies in both *in vitro* and *in vivo* animal models, the performance in rodent models vastly exceeds that observed in non-human primate models in nearly every measure, including toxicity and tolerability, pharmacokinetics, tissue targeting and efficacy. Notably, achieving therapeutically relevant outcomes at tolerable dose levels in primate models remains a significant challenge. Thus, there remains a need for improved lipid nanoparticles for the delivery of oligonucleotides in primates such that an efficacious and reproducible therapeutic result can be realized. Embodiments of the present disclosure provide these and related advantages.

### BRIEF SUMMARY

The subject matter of the invention is as set out in the appended claims.

One aspect of the invention relates to lipid nanoparticles (LNPs) for use in a method of treating or preventing a disease in a primate in need thereof, wherein the method comprises administering the LNPs to the primate, each of the LNPs comprising:
i) a nucleic acid, or a pharmaceutically acceptable salt thereof, encapsulated within the LNP;
ii) a cationic lipid;
iii) a neutral lipid;
iv) a steroid; and
v) a polymer-conjugated lipid,
wherein a mean particle diameter of the LNPs ranges from 40 nm to 68 nm as determined by quasi-elastic light scattering.

Preferred embodiments are set forth in the dependent claims.

Any references in the description to methods of treatment refer to the compounds, pharmaceutical compositions and medicaments of the present invention for use in a method for treatment of the human (or animal) body by therapy.

The following embodiments are not according to the invention unless they are embraced by the claims.

Embodiments of the present disclosure provide improved lipid nanoparticles (LNPs) and methods of use of the same, for example, for delivery of nucleic acid therapeutic agents to human and/or non-human primates. In an exemplary embodiment, a method for delivering a nucleic acid to a primate in need thereof is disclosed, the method comprising administering a lipid nanoparticle (LNP) to the primate, the LNP comprising:
i) a nucleic acid, or a pharmaceutically acceptable salt thereof, encapsulated within the LNP;
ii) a cationic lipid;
iii) a neutral lipid;
iv) a steroid; and
v) from 2.0 to 3.5 mol percent of a polymer-conjugated lipid based on total mol of lipids in the LNP.

In other embodiments, the present disclosure is directed to a method for delivering a nucleic acid to a primate in need thereof, comprising administering a lipid nanoparticle (LNP) to the primate, the LNP comprising:
i) a nucleic acid, or a pharmaceutically acceptable salt thereof, encapsulated within the LNP;
ii) a cationic lipid;
iii) a neutral lipid;
iv) a steroid; and
v) a polymer-conjugated lipid,
wherein a plurality of the LNPs has a mean particle diameter ranging from 40 nm to 70 nm.

In still more exemplary embodiments, the present disclosure provides a method for delivering a nucleic acid to a primate in need thereof, comprising administering a lipid nanoparticle (LNP) to the primate, the LNP comprising:
i) a nucleic acid, or a pharmaceutically acceptable salt thereof, encapsulated within the LNP;
ii) a cationic lipid;
iii) a neutral lipid;
iv) a steroid; and
v) a polymer-conjugated lipid having the following structure: 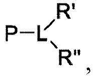
wherein:
P is a polymer;
L is a trivalent linker of 1 to 15 atoms in length; and
R' and R" are each independently a saturated alkyl having from 8 to 14 carbon atoms, provided that the total number of carbon atoms collectively in both of R' and R" is no more than 27.

Further embodiments are directed to improved components for lipid nanoparticles, as well as lipid nanoparticles comprising the same and use of the same. For example, one embodiment is directed to a compound having the following structure: 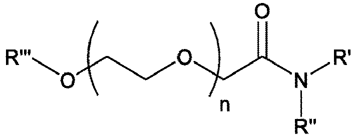 or a salt thereof, wherein R', R", R‴ and n are as defined herein. LNPs comprising the above compound, and methods of using the same in various methods, including administering a therapeutic nucleic acid to a primate, are also disclosed.

These and other aspects of various embodiments will be apparent upon reference to the following detailed description.

### BRIEF DESCRIPTION OF THE SEVERAL VIEWS OF THE DRAWINGS

In the figures, identical reference numbers identify similar elements. The sizes and relative positions of elements in the figures are not necessarily drawn to scale and some of these elements are arbitrarily enlarged and positioned to improve figure legibility. Further, the particular shapes of the elements as drawn are not intended to convey any information regarding the actual shape of the particular elements, and have been solely selected for ease of recognition in the figures.
Figures 1 and 2 show relative concentrations of expressed luciferase in mouse liver for different embodiments of lipid nanoparticles..
Figure 3 and 4 show relative concentrations of expressed luciferase in mouse liver for different embodiments of lipid nanoparticles as a function of the quantity of PEG lipid in the LNP.
Figure 5 shows levels of IgG1 present in non-human primate blood plasma for different embodiments of lipid nanoparticles.
Figure 6 plots the concentration of amino lipid in non-human primate blood plasma for different embodiments of lipid nanoparticles.
Figure 7 plots the concentration of amino lipids in non-human primate liver for different embodiments of lipid nanoparticles as a function of time.
Figures 8 - 11 show *in situ* hybridization images demonstrating the distribution of LNPs in certain liver tissue regions for different embodiments of the LNP.
Figure 12 shows cytokine data for monkeys treated with the LNPs of example 4.
Figure 13 compares plasma IgG1 levels for two diferent sizes of LNPs.
Figure 14 presents igG expression in mice for two different sizes of LNPs.
Figure 15 is cytokine data for two different LNP sizes.
Figure 16 shows *in situ* hybridization images demonstrating the distribution of LNPs in certain liver tissue regions for different sizes of LNPs.
Figure 17 is igG expression in NHPs for two different LNPs.
Figure 18 is igG expression in mice for two different LNPs.
Figure 19 presents igG expression data for LNPs 10-1 and 10-2.

### DETAILED DESCRIPTION

In the following description, certain specific details are set forth in order to provide a thorough understanding of various embodiments of the invention. However, one skilled in the art will understand that the invention may be practiced without these details.

The invention is defined by the claims. Any subject-matter falling outside the scope of the claims is provided for information purposes only.

In particular embodiments, the present invention provides lipid nanoparticles and methods for the *in vitro* and *in vivo* delivery of mRNA and/or other oligonucleotides. In some embodiments, these improved lipid nanoparticle compositions are useful for expression of protein encoded by mRNA. In other embodiments, these improved lipid nanoparticles are useful for upregulation of endogenous protein expression by delivering miRNA inhibitors targeting one specific miRNA or a group of miRNA regulating one target mRNA or several mRNA. In other embodiments, these improved lipid nanoparticles are useful for upregulation of endogenous protein expression by delivering smaRNA targeting a gene promotor or group of gene promotors. In other embodiments, these improved lipid nanoparticles are useful for down-regulating (e.g., silencing) the protein levels and/or mRNA levels of target genes. In some other embodiments, the lipid nanoparticles are also useful for delivery of mRNA, self amplifying RNA (saRNA) and plasmids for expression of transgenes. In yet other embodiments, the lipid nanoparticles are useful for inducing a pharmacological effect resulting from expression of a protein, e.g., increased production of red blood cells through the delivery of a suitable erythropoietin mRNA, or protection against infection through delivery of mRNA encoding for a suitable antigen or antibody. In yet other embodiments, the lipid nanoparticles can be employed in gene editing applications, for example those based on Clustered Regularly Interspaced Short Palindrome Repeats (CRISPR) methods, through the delivery of mRNA capable of expressing Cas9 in combination with an appropriate single guide RNA (sgRNA). Gene editing approaches can be used to treat, for example, hypercholesterolemia by targeting an appropriate gene target, e.g., PCSK9 in a murine model for the disease. The lipid nanoparticles of embodiments of the present invention may be used for a variety of purposes, including the delivery of encapsulated or associated (e.g., complexed) therapeutic agents such as nucleic acids to cells, both *in vitro* and *in vivo.* Accordingly, embodiments of the present invention provide a method for administering a therapeutic agent to a patient, for example a primate, in need thereof, the method comprising administering a lipid nanoparticle as described herein to the patient.

As described herein, embodiments of the lipid nanoparticles of the present invention are particularly useful for the delivery of nucleic acids, including, e.g., mRNA, guide RNA, circular RNA, antisense oligonucleotide, plasmid DNA, closed ended DNA (ceDNA), circular DNA, microRNA (miRNA), miRNA inhibitors (antagomirs/antimirs), messenger-RNA-interfering complementary RNA (micRNA), self amplifying RNA (saRNA), small activating RNA (smaRNA), DNA, multivalent RNA, dicer substrate RNA, complementary DNA (cDNA), peptide nucleic acid (PNA) etc. Therefore, the lipid nanoparticles of embodiments of the present invention may be used to induce expression of a desired protein both *in vitro* and *in vivo* by contacting cells with a lipid nanoparticle. The expressed protein may have a biological effect, such as inducing an immune response. Alternatively, the lipid nanoparticles and compositions of embodiments of the present invention may be used to decrease the expression of target genes and proteins both *in vitro* and *in vivo* by contacting cells with a lipid nanoparticle. The lipid nanoparticles and compositions of embodiments of the present invention may also be used for co-delivery of different nucleic acids (e.g., mRNA and plasmid DNA) separately or in combination, such as may be useful to provide an effect requiring colocalization of different nucleic acids (e.g. mRNA encoding for a suitable gene modifying enzyme with an associated guide RNA sequence if applicable, and optionally, DNA segment(s) for incorporation into the host genome).

Nucleic acids for use with embodiments of this invention may be prepared according to the techniques described herein. For mRNA, the primary methodology of preparation is, but not limited to, enzymatic synthesis (also termed *in vitro* transcription) which currently represents the most efficient method to produce long sequence-specific mRNA. *In vitro* transcription describes a process of template-directed synthesis of RNA molecules from an engineered DNA template comprised of an upstream bacteriophage promoter sequence (e.g. including but not limited to that from the T7, T3 and SP6 coliphage) linked to a downstream sequence encoding the gene of interest. Template DNA can be prepared for *in vitro* transcription from a number of sources with appropriate techniques which are well known in the art including, but not limited to, plasmid DNA and polymerase chain reaction amplification (see Linpinsel, J.L and Conn, G.L., General protocols for preparation of plasmid DNA template and Bowman, J.C., Azizi, B., Lenz, T.K., Ray, P., and Williams, L.D. in RNA in vitro transcription and RNA purification by denaturing PAGE in Recombinant and in vitro RNA syntheses Methods v. 941 Conn G.L. (ed), New York, N.Y. Humana Press, 2012).

Transcription of the RNA occurs *in vitro* using the linearized DNA template in the presence of the corresponding RNA polymerase and adenosine, guanosine, uridine and cytidine ribonucleoside triphosphates (rNTPs) under conditions that support polymerase activity while minimizing potential degradation of the resultant mRNA transcripts. *In vitro* transcription can be performed using a variety of commercially available kits including, but not limited to RiboMax Large Scale RNA Production System (Promega), MegaScript Transcription kits (Life Technologies) as well as with commercially available reagents including RNA polymerases and rNTPs. The methodology for *in vitro* transcription of mRNA is well known in the art. (see, *e.g.* Losick, R., 1972, In vitro transcription, Ann Rev Biochem v.41 409-46; Kamakaka, R. T. and Kraus, W. L. 2001. In Vitro Transcription. Current Protocols in Cell Biology. 2:11.6:11.6.1-11.6.17; Beckert, B. And Masquida, B.,(2010) Synthesis of RNA by In Vitro Transcription in RNA in Methods in Molecular Biology v. 703 (Neilson, H. Ed), New York, N.Y. Humana Press, 2010; Brunelle, J.L. and Green, R., 2013, Chapter Five - In vitro transcription from plasmid or PCR-amplified DNA, Methods in Enzymology v. 530, 101-114.

The desired *in vitro* transcribed mRNA is then purified from the undesired components of the transcription or associated reactions (including unincorporated rNTPs, protein enzyme, salts, short RNA oligos, etc.). Techniques for the isolation of the mRNA transcripts are well known in the art. Well known procedures include phenol/chloroform extraction or precipitation with either alcohol (e.g., ethanol, isopropanol) in the presence of monovalent cations or lithium chloride. Additional, non-limiting examples of purification procedures which can be used include size exclusion chromatography (Lukavsky, P.J. and Puglisi, J.D., 2004, Large-scale preparation and purification of polyacrylamide-free RNA oligonucleotides, RNA v.10, 889-893), silica-based affinity chromatography and polyacrylamide gel electrophoresis (Bowman, J.C., Azizi, B., Lenz, T.K., Ray, P., and Williams, L.D. in RNA in vitro transcription and RNA purification by denaturing PAGE in Recombinant and in vitro RNA syntheses Methods v. 941 Conn G.L. (ed), New York, N.Y. Humana Press, 2012 ). Purification can be performed using a variety of commercially available kits including, but not limited to SV Total Isolation System (Promega) and In Vitro Transcription Cleanup and Concentration Kit (Norgen Biotek).

Furthermore, while reverse transcription can yield large quantities of mRNA, the products can contain a number of aberrant RNA impurities associated with undesired polymerase activity which may need to be removed from the full-length mRNA preparation. These include short RNAs that result from abortive transcription initiation as well as double-stranded RNA (dsRNA) generated by RNA-dependent RNA polymerase activity, RNA-primed transcription from RNA templates and self-complementary 3' extension. It has been demonstrated that these contaminants with dsRNA structures can lead to undesired immunostimulatory activity through interaction with various innate immune sensors in eukaryotic cells that function to recognize specific nucleic acid structures and induce potent immune responses. This in turn, can dramatically reduce mRNA translation when protein synthesis is reduced during the innate cellular immune response. Therefore, additional techniques to remove these dsRNA contaminants have been developed and are known in the art including but not limited to scaleable HPLC purification (*see*, e.g., Kariko, K., Muramatsu, H., Ludwig, J. and Weissman, D., 2011, Generating the optimal mRNA for therapy: HPLC purification eliminates immune activation and improves translation of nucleoside-modified, protein-encoding mRNA, Nucl Acid Res, v. 39 e142; Weissman, D., Pardi, N., Muramatsu, H., and Kariko, K., HPLC Purification of in vitro transcribed long RNA in Synthetic Messenger RNA and Cell Metabolism Modulation in Methods in Molecular Biology v.969 (Rabinovich, P.H. Ed), 2013). Purified mRNA has been reported to be translated at much greater levels, particularly in primary cells and *in vivo.*

A significant variety of modifications have been described in the art which are used to alter specific properties of *in vitro* transcribed mRNA, and improve its utility. These include, but are not limited to modifications to the 5' and 3' termini of the mRNA. Endogenous eukaryotic mRNA typically contain a cap structure on the 5'-end of a mature molecule which plays an important role in mediating binding of the mRNA Cap Binding Protein (CBP), which is in turn responsible for enhancing mRNA stability in the cell and efficiency of mRNA translation. Therefore, highest levels of protein expression are achieved with capped mRNA transcripts. The 5'-cap contains a 5'-5'-triphosphate linkage between the 5'-most nucleotide and guanine nucleotide. The conjugated guanine nucleotide is methylated at the N7 position. Additional modifications include methylation of the ultimate and penultimate most 5'-nucleotides on the 2'-hydroxyl group.

Multiple distinct cap structures can be used to generate the 5'-cap of *in vitro* transcribed synthetic mRNA. 5'-capping of synthetic mRNA can be performed co-transcriptionally with chemical cap analogs (i.e., capping during *in vitro* transcription). For example, CleanCap^{®} technology provides high efficiency capping (90%+) in a co-transcriptional reaction using commercially available reagents with an AG initiator to provide a natural Cap 1 structure with a 2'-O-methyl group and N7 methyl on separate guanine components. As another example, the Anti-Reverse Cap Analog (ARCA) cap contains a 5'-5'-triphosphate guanine-guanine linkage where one guanine contains an N7 methyl group as well as a 3'-O-methyl group. However, up to 20% of transcripts remain uncapped during this co-transcriptional process and the synthetic cap analog is not identical to the 5'-cap structure of an authentic cellular mRNA, potentially reducing translatability and cellular stability. Alternatively, synthetic mRNA molecules may also be enzymatically capped post-transcriptionally. These may generate a more authentic 5'-cap structure that more closely mimics, either structurally or functionally, the endogenous 5'-cap which have enhanced binding of cap binding proteins, increased half-life, reduced susceptibility to 5' endonucleases and/or reduced 5' decapping. Numerous synthetic 5'-cap analogs have been developed and are known in the art to enhance mRNA stability and translatability *(see,* e.g., Grudzien-Nogalska, E., Kowalska, J., Su, W., Kuhn, A.N., Slepenkov, S.V., Darynkiewicz, E., Sahin, U., Jemielity, J., and Rhoads, R.E., Synthetic mRNAs with superior translation and stability properties in Synthetic Messenger RNA and Cell Metabolism Modulation in Methods in Molecular Biology v.969 (Rabinovich, P.H. Ed), 2013).

On the 3'-terminus, a long chain of adenine nucleotides (poly-A tail) is normally added to mRNA molecules during RNA processing. Immediately after transcription, the 3' end of the transcript is cleaved to free a 3' hydroxyl to which poly-A polymerase adds a chain of adenine nucleotides to the RNA in a process called polyadenylation. The poly-A tail has been extensively shown to enhance both translational efficiency and stability of mRNA (see Bernstein, P. and Ross, J., 1989, Poly (A), poly (A) binding protein and the regulation of mRNA stability, Trends Bio Sci v. 14 373-377; Guhaniyogi, J. And Brewer, G., 2001, Regulation of mRNA stability in mammalian cells, Gene, v. 265, 11-23; Dreyfus, M. And Regnier, P., 2002, The poly (A) tail of mRNAs: Bodyguard in eukaryotes, scavenger in bacteria, Cell, v.111, 611-613).

Poly (A) tailing of *in vitro* transcribed mRNA can be achieved using various approaches including, but not limited to, cloning of a poly (T) tract into the DNA template or by post-transcriptional addition using Poly (A) polymerase. The first case allows *in vitro* transcription of mRNA with poly (A) tails of defined length, depending on the size of the poly (T) tract, but requires additional manipulation of the template. The latter case involves the enzymatic addition of a poly (A) tail to *in vitro* transcribed mRNA using poly (A) polymerase which catalyzes the incorporation of adenine residues onto the 3'termini of RNA, requiring no additional manipulation of the DNA template, but results in mRNA with poly(A) tails of heterogeneous length. 5'-capping and 3'-poly (A) tailing can be performed using a variety of commercially available kits including, but not limited to Poly (A) Polymerase Tailing kit (EpiCenter), mMESSAGE mMACHINE T7 Ultra kit and Poly (A) Tailing kit (Life Technologies) as well as with commercially available reagents, various ARCA caps, Poly (A) polymerase, etc.

In addition to 5' cap and 3' poly adenylation, other modifications of the *in* vitro transcripts have been reported to provide benefits as related to efficiency of translation and stability. It is well known in the art that pathogenic DNA and RNA can be recognized by a variety of sensors within eukaryotes and trigger potent innate immune responses. The ability to discriminate between pathogenic and self DNA and RNA has been shown to be based, at least in part, on structure and nucleoside modifications since most nucleic acids from natural sources contain modified nucleosides In contrast, *in vitro* synthesized RNA lacks these modifications, thus rendering it immunostimulatory which in turn can inhibit effective mRNA translation as outlined above. The introduction of modified nucleosides into *in vitro* transcribed mRNA can be used to prevent recognition and activation of RNA sensors, thus mitigating this undesired immunostimulatory activity and enhancing translation capacity (see *e.g*. Kariko, K. And Weissman, D. 2007, Naturally occurring nucleoside modifications suppress the immunostimulatory activity of RNA: implication for therapeutic RNA development, Curr Opin Drug Discov Devel, v.10 523-532; Pardi, N., Muramatsu, H., Weissman, D., Kariko, K., In vitro transcription of long RNA containing modified nucleosides in Synthetic Messenger RNA and Cell Metabolism Modulation in Methods in Molecular Biology v.969 (Rabinovich, P.H. Ed), 2013); Kariko, K., Muramatsu, H., Welsh, F.A., Ludwig, J., Kato, H., Akira, S., Weissman, D., 2008, Incorporation of Pseudouridine Into mRNA Yields Superior Nonimmunogenic Vector With Increased Translational Capacity and Biological Stability, Mol Ther v.16, 1833-1840. The modified nucleosides and nucleotides used in the synthesis of modified RNAs can be prepared monitored and utilized using general methods and procedures known in the art. A large variety of nucleoside modifications are available that may be incorporated alone or in combination with other modified nucleosides to some extent into the *in vitro* transcribed mRNA *(see,* e.g., U.S. Pub. No. 2012/0251618). *In vitro* synthesis of nucleoside-modified mRNA have been reported to have reduced ability to activate immune sensors with a concomitant enhanced translational capacity.

Other components of mRNA which can be modified to provide benefit in terms of translatability and stability include the 5' and 3' untranslated regions (UTR). Optimization of the UTRs (favorable 5' and 3' UTRs can be obtained from cellular or viral RNAs), either both or independently, have been shown to increase mRNA stability and translational efficiency of *in vitro* transcribed mRNA *(see,* e.g., Pardi, N., Muramatsu, H., Weissman, D., Kariko, K., In vitro transcription of long RNA containing modified nucleosides in Synthetic Messenger RNA and Cell Metabolism Modulation in Methods in Molecular Biology v.969 (Rabinovich, P.H. Ed), 2013).

In addition to mRNA, other nucleic acid payloads may be used for this invention. For oligonucleotides, methods of preparation include but are not limited to chemical synthesis and enzymatic, chemical cleavage of a longer precursor, *in vitro* transcription as described above, etc. Methods of synthesizing DNA and RNA nucleotides are widely used and well known in the art (*see,* e.g., Gait, M. J. (ed.) Oligonucleotide synthesis: a practical approach, Oxford [Oxfordshire], Washington, D.C.: IRL Press, 1984; and Herdewijn, P. (ed.) Oligonucleotide synthesis: methods and applications, Methods in Molecular Biology, v. 288 (Clifton, N.J.) Totowa, N.J.: Humana Press, 2005.

For plasmid DNA, preparation for use with embodiments of this invention commonly utilizes but is not limited to expansion and isolation of the plasmid DNA *in vitro* in a liquid culture of bacteria containing the plasmid of interest. The presence of a gene in the plasmid of interest that encodes resistance to a particular antibiotic (penicillin, kanamycin, etc.) allows those bacteria containing the plasmid of interest to selectively grow in antibiotic-containing cultures. Methods of isolating plasmid DNA are widely used and well known in the art (*see,* e.g., Heilig, J., Elbing, K. L. and Brent, R (2001) Large-Scale Preparation of Plasmid DNA. Current Protocols in Molecular Biology. 41:II:1.7:1.7.1-1.7.16; Rozkov, A., Larsson, B., Gillström, S., Björnestedt, R. and Schmidt, S. R. (2008), Large-scale production of endotoxin-free plasmids for transient expression in mammalian cell culture. Biotechnol. Bioeng., 99: 557-566; and U.S. Pat. No. 6,197,553 B1). Plasmid isolation can be performed using a variety of commercially available kits including, but not limited to Plasmid Plus (Qiagen), GenJET plasmid MaxiPrep (Thermo) and PureYield MaxiPrep (Promega) kits as well as with commercially available reagents.

As used herein, the following terms have the meanings ascribed to them unless specified otherwise.

Unless the context requires otherwise, throughout the present specification and claims, the word "comprise" and variations thereof, such as, "comprises" and "comprising" are to be construed in an open and inclusive sense, that is, as "including, but not limited to".

Reference throughout this specification to "one embodiment" or "an embodiment" means that a particular feature, structure or characteristic described in connection with the embodiment is included in at least one embodiment of the present invention. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

Unless defined otherwise, all technical and scientific terms used herein have the same meaning as is commonly understood by one of skill in the art to which this invention belongs. As used in the specification and claims, the singular form "a", "an" and "the" include plural references unless the context clearly dictates otherwise.

The phrase "induce expression of a desired protein" refers to the ability of a nucleic acid to increase expression of the desired protein. To examine the extent of protein expression, a test sample (e.g., a sample of cells in culture expressing the desired protein) or a test mammal (e.g., a mammal such as a human or an animal model such as a rodent (e.g. mouse) or a non-human primate (e.g., monkey) model) is contacted with a nucleic acid (e.g., nucleic acid in combination with a lipid of the present invention). Expression of the desired protein in the test sample or test animal is compared to expression of the desired protein in a control sample (e.g. a sample of cells in culture expressing the desired protein) or a control mammal (e.g., a mammal such as a human or an animal model such as a rodent (e.g., mouse) or non-human primate (e.g., monkey) model) that is not contacted with or administered the nucleic acid. When the desired protein is present in a control sample or a control mammal, the expression of a desired protein in a control sample or a control mammal may be assigned a value of 1.0. In particular embodiments, inducing expression of a desired protein is achieved when the ratio of desired protein expression in the test sample or the test mammal to the level of desired protein expression in the control sample or the control mammal is greater than 1, for example, about 1.1, 1.5, 2.0. 5.0 or 10.0. When a desired protein is not present in a control sample or a control mammal, inducing expression of a desired protein is achieved when any measurable level of the desired protein in the test sample or the test mammal is detected. One of ordinary skill in the art will understand appropriate assays to determine the level of protein expression in a sample, for example dot blots, northern blots, *in situ* hybridization, ELISA, immunoprecipitation, enzyme function, and phenotypic assays, or assays based on reporter proteins that can produce fluorescence or luminescence under appropriate conditions.

The phrase "inhibiting expression of a target gene" refers to the ability of a nucleic acid to silence, reduce, or inhibit the expression of a target gene. To examine the extent of gene silencing, a test sample (e.g., a sample of cells in culture expressing the target gene) or a test mammal (e.g., a mammal such as a human or an animal model such as a rodent (e.g., mouse) or a non-human primate (e.g., monkey) model) is contacted with a nucleic acid that silences, reduces, or inhibits expression of the target gene. Expression of the target gene in the test sample or test animal is compared to expression of the target gene in a control sample (e.g., a sample of cells in culture expressing the target gene) or a control mammal (e.g., a mammal such as a human or an animal model such as a rodent (e.g., mouse) or non-human primate (e.g., monkey) model) that is not contacted with or administered the nucleic acid. The expression of the target gene in a control sample or a control mammal may be assigned a value of 100%. In particular embodiments, silencing, inhibition, or reduction of expression of a target gene is achieved when the level of target gene expression in the test sample or the test mammal relative to the level of target gene expression in the control sample or the control mammal is about 95%, 90%, 85%, 80%, 75%, 70%, 65%, 60%, 55%, 50%, 45%, 40%, 35%, 30%, 25%, 20%, 15%, 10%, 5%, or 0%. In other words, the nucleic acids are capable of silencing, reducing, or inhibiting the expression of a target gene by at least about 5%, 10%, 15%, 20%, 25%, 30%, 35%, 40%, 45%, 50%, 55%, 60%, 65%, 70%, 75%, 80%, 85%, 90%, 95%, or 100% in a test sample or a test mammal relative to the level of target gene expression in a control sample or a control mammal not contacted with or administered the nucleic acid. Suitable assays for determining the level of target gene expression include, without limitation, examination of protein or mRNA levels using techniques known to those of skill in the art, such as, e.g., dot blots, northern blots, *in situ* hybridization, ELISA, immunoprecipitation, enzyme function, as well as phenotypic assays known to those of skill in the art.

An "effective amount" or "therapeutically effective amount" of an active agent or therapeutic agent such as a therapeutic nucleic acid is an amount sufficient to produce the desired effect, e.g. an increase or inhibition of expression of a target sequence in comparison to the normal expression level detected in the absence of the nucleic acid. An increase in expression of a target sequence is achieved when any measurable level is detected in the case of an expression product that is not present in the absence of the nucleic acid. In the case where the expression product is present at some level prior to contact with the nucleic acid, an in increase in expression is achieved when the fold increase in value obtained with a nucleic acid such as mRNA relative to control is about 1.05, 1.1, 1.2, 1.3, 1.4, 1.5, 1.75, 2, 2.5, 3, 4, 5, 6, 7, 8, 9, 10, 15, 20, 25, 30, 40, 50, 75, 100, 250, 500, 750, 1000, 5000, 10000 or greater. Inhibition of expression of a target gene or target sequence is achieved when the value obtained with a nucleic acid such as antisense oligonucleotide relative to the control is about 95%, 90%, 85%, 80%, 75%, 70%, 65%, 60%, 55%, 50%, 45%, 40%, 35%, 30%, 25%, 20%, 15%, 10%, 5%, or 0%. Suitable assays for measuring expression of a target gene or target sequence include, e.g., examination of protein or RNA levels using techniques known to those of skill in the art such as dot blots, northern blots, *in situ* hybridization, ELISA, immunoprecipitation, enzyme function, fluorescence or luminescence of suitable reporter proteins, as well as phenotypic assays known to those of skill in the art.

The term "nucleic acid" as used herein refers to a polymer containing at least two deoxyribonucleotides or ribonucleotides in either single- or double-stranded form and includes DNA, RNA, and hybrids thereof. DNA may be in the form of antisense molecules, plasmid DNA, cDNA, PCR products, or vectors. RNA may be in the form of small hairpin RNA (shRNA), messenger RNA (mRNA), self amplifying RNA (saRNA), small activating RNA, antisense RNA, miRNA, micRNA, multivalent RNA, dicer substrate RNA or viral RNA (vRNA), and combinations thereof. Nucleic acids include nucleic acids containing known nucleotide analogs or modified backbone residues or linkages, which are synthetic, naturally occurring, and non-naturally occurring, and which have similar binding properties as the reference nucleic acid. Examples of such analogs include, without limitation, phosphorothioates, phosphoramidates, methyl phosphonates, chiral-methyl phosphonates, 2'-O-methyl ribonucleotides, and peptide-nucleic acids (PNAs). Unless specifically limited, the term encompasses nucleic acids containing known analogues of natural nucleotides that have similar binding properties as the reference nucleic acid. Unless otherwise indicated, a particular nucleic acid sequence also implicitly encompasses conservatively modified variants thereof (e.g., degenerate codon substitutions), alleles, orthologs, single nucleotide polymorphisms, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res., 19:5081 (1991); Ohtsuka et al., J. Biol. Chem., 260:2605-2608 (1985); Rossolini et al., Mol. Cell. Probes, 8:91-98 (1994)). "Nucleotides" contain a sugar deoxyribose (DNA) or ribose (RNA), a base, and a phosphate group. Nucleotides are linked together through the phosphate groups. "Bases" include purines and pyrimidines, which further include natural compounds adenine, thymine, guanine, cytosine, uracil, inosine, and natural analogs, and synthetic derivatives of purines and pyrimidines, which include, but are not limited to, modifications which place new reactive groups such as, but not limited to, amines, alcohols, thiols, carboxylates, and alkylhalides.

The term "gene" refers to a nucleic acid (e.g., DNA or RNA) sequence that comprises partial length or entire length coding sequences necessary for the production of a polypeptide or precursor polypeptide, or provides regulation of gene expression. "Gene" can refer to both coding and non-coding (does not encode a protein sequence) sequences of nucleic acids. For example, a non-coding "gene" may be transcribed into functional RNA products, including regulatory RNA, transfer RNA (tRNA), microRNA (miRNA), and ribosomal RNA (rRNA).

"Gene product," as used herein, refers to a product of a gene such as an RNA transcript, including coding and non-coding variants, or a polypeptide.

The term "lipid" refers to a group of organic compounds that include, but are not limited to, esters of fatty acids and are generally characterized by being poorly soluble in water, but soluble in many organic solvents. They are usually divided into at least three classes: (1) "simple lipids," which include fats and oils as well as waxes; (2) "compound lipids," which include phospholipids and glycolipids; and (3) "derived lipids" such as steroids.

A "steroid" is a compound comprising the following carbon skeleton: 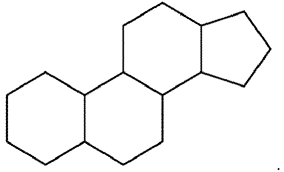 Non-limiting examples of steroids include cholesterol, and the like.

A "cationic lipid" refers to a lipid capable of being positively charged. Exemplary cationic lipids include one or more amine group(s) which bear the positive charge. Preferred cationic lipids are ionizable such that they can exist in a positively charged or neutral form depending on pH. The ionization of the cationic lipid affects the surface charge of the lipid nanoparticle under different pH conditions. This charge state can influence plasma protein absorption, blood clearance and tissue distribution (Semple, S.C., et al., Adv. Drug Deliv Rev 32:3-17 (1998)) as well as the ability to form nonbilayer structures (Hafez, I.M., et al., Gene Ther 8:1188-1196 (2001)) critical to the intracellular delivery of nucleic acids.

An "anionic lipid" refers to a lipid capable of being negatively charged. Exemplary anionic lipids include one or more phosphate group(s) which bear a negative charge, for example at physiological pHs. In some embodiments, the anionic lipid does not include a serine moiety, including phosphatidylserine lipids.

"Phosphatidylglycerol lipid" refers to a lipid with a structure that generally comprises a glycerol 3-phosphate backbone which is attached to saturated or unsaturated fatty acids via and ester linkage. Exemplary phosphatidylglycerol lipids have the following structure: 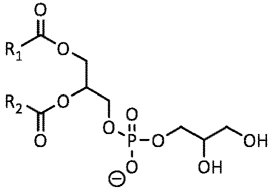 wherein R₁ and R₂ are each independently a branched or straight, saturated or unsaturated carbon chain (e.g., alkyl, alkenyl, alkynyl).

The term "polymer conjugated lipid" refers to a molecule comprising both a lipid portion and a polymer portion. An example of a polymer conjugated lipid is a pegylated lipid. The term "pegylated lipid" refers to a molecule comprising both a lipid portion and a polyethylene glycol portion. Pegylated lipids are known in the art and include 1-(monomethoxy-polyethyleneglycol)-2,3-dimyristoylglycerol (PEG-DMG) and the like. The term "pegylated lipid" is used interchangeably with "PEGylated lipid."

The term "neutral lipid" refers to any of a number of lipid species that exist either in an uncharged or neutral zwitterionic form at a selected pH. At physiological pH, such lipids include, but are not limited to, phosphotidylcholines such as 1,2-Distearoyl-*sn*-glycero-3-phosphocholine (DSPC), 1,2-Dipalmitoyl-*sn*-glycero-3-phosphocholine (DPPC), 1,2-Dimyristoyl-*sn*-glycero-3-phosphocholine (DMPC), 1-Palmitoyl-2-oleoyl-*sn*-glycero-3-phosphocholine (POPC), 1,2-dioleoyl-*sn*-glycero-3-phosphocholine (DOPC), phophatidylethanolamines such as 1,2-Dioleoyl-*sn*-glycero-3-phosphoethanolamine (DOPE), sphingomyelins (SM), ceramides, steroids such as sterols and their derivatives. Neutral lipids may be synthetic or naturally derived. Neutral lipids include those lipids sometimes referred to as 'non-cationic' lipids.

The term "charged lipid" refers to any of a number of lipid species that exist in either a positively charged or negatively charged form independent of the pH within a useful physiological range, e.g., pH ~3 to pH ~9. Charged lipids may be synthetic or naturally derived. Examples of charged lipids include phosphatidylserines, phosphatidic acids, phosphatidylglycerols, phosphatidylinositols, sterol hemisuccinates, dialkyl trimethylammonium-propanes, (e.g., DOTAP, DOTMA), dialkyl dimethylaminopropanes, ethyl phosphocholines, dimethylaminoethane carbamoyl sterols (e.g., DC-Chol).

The term "lipid nanoparticle" refers to particles having at least one dimension on the order of nanometers (e.g., 1-1,000 nm) which include one or more specified lipids. In some embodiments, lipid nanoparticles are included in a formulation that can be used to deliver an active agent or therapeutic agent, such as a nucleic acid (e.g., mRNA) to a target site of interest (e.g., cell, tissue, organ, tumor, and the like). In some embodiments, the lipid nanoparticles of the invention comprise a nucleic acid. Such lipid nanoparticles typically comprise a cationic lipid and one or more excipient selected from neutral lipids, charged lipids, steroids and polymer conjugated lipids. In some embodiments, the active agent or therapeutic agent, such as a nucleic acid, may be encapsulated in the lipid portion of the lipid nanoparticle or an aqueous space enveloped by some or all of the lipid portion of the lipid nanoparticle, thereby protecting it from enzymatic degradation or other undesirable effects induced by the mechanisms of the host organism or cells, e.g., an adverse immune response.

In various embodiments, the lipid nanoparticles have a mean diameter of from about 30 nm to about 150 nm, from about 40 nm to about 150 nm, from about 50 nm to about 150 nm, from about 60 nm to about 130 nm, from about 70 nm to about 110 nm, from about 70 nm to about 100 nm, from about 80 nm to about 100 nm, from about 90 nm to about 100 nm, from about 70 to about 90 nm, from about 80 nm to about 90 nm, from about 70 nm to about 80 nm, from about 40 nm to about 50 nm, from about 40 nm to about 60 nm, from about 40 nm to about 70 nm, from about 40 nm to about 80 nm, from about 45 nm to about 50 nm, from about 45 nm to about 55 nm, from about 45 nm to about 60 nm, from about 45 nm to about 65 nm, from about 45 nm to about 70 nm, from about 50 nm to about 70 nm, from about 50 nm to about 60 nm, from about 60 nm to about 70 nm, from about 55 nm to about 65 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm and are substantially non-toxic (not according to the invention unless embraced by the claims). In certain embodiments, nucleic acids, when present in the lipid nanoparticles, are resistant in aqueous solution to degradation with a nuclease. Lipids and their method of preparation are disclosed in, e.g., U.S. Patent Nos. 8,569,256, 5,965,542 and U.S. Patent Publication Nos. 2016/0199485, 2016/0009637, 2015/0273068, 2015/0265708, 2015/0203446, 2015/0005363, 2014/0308304, 2014/0200257, 2013/086373, 2013/0338210, 2013/0323269, 2013/0245107, 2013/0195920, 2013/0123338, 2013/0022649, 2013/0017223, 2012/0295832, 2012/0183581, 2012/0172411, 2012/0027803, 2012/0058188, 2011/0311583, 2011/0311582, 2011/0262527, 2011/0216622, 2011/0117125, 2011/0091525, 2011/0076335, 2011/0060032, 2010/0130588, 2007/0042031, 2006/0240093, 2006/0083780, 2006/0008910, 2005/0175682, 2005/017054, 2005/0118253, 2005/0064595, 2004/0142025, 2007/0042031, 1999/009076 and PCT Pub. Nos. WO 99/39741, WO 2017/117528, WO 2017/004143, WO 2017/075531, WO 2015/199952, WO 2014/008334, WO 2013/086373, WO 2013/086322, WO 2013/016058, WO 2013/086373, WO2011/141705, and WO 2001/07548. LNPs are prepared according to the methods disclosed herein.

Other exemplary lipids and their manufacture are described in the art, for example in U.S. Patent Application Publication No. U.S. 2012/0276209, Semple et al., 2010, Nat Biotechnol., 28(2):172-176; Akinc et al., 2010, Mol Ther., 18(7): 1357-1364; Basha et al., 2011, Mol Ther, 19(12): 2186-2200; Leung et al., 2012, J Phys Chem C Nanomater Interfaces, 116(34): 18440-18450; Lee et al., 2012, Int J Cancer., 131(5): E781-90; Belliveau et al., 2012, Mol Ther nucleic Acids, 1: e37; Jayaraman et al., 2012, Angew Chem Int Ed Engl., 51(34): 8529-8533; Mui et al., 2013, Mol Ther Nucleic Acids. 2, e139; Maier et al., 2013, Mol Ther., 21(8): 1570-1578; and Tam et al., 2013, Nanomedicine, 9(5): 665-74. Lipids and their manufacture can be found, for example, in U.S. Pub. No. 2015/0376115 and 2016/0376224.

As used herein, "lipid encapsulated" refers to a lipid nanoparticle that provides an active agent or therapeutic agent, such as a nucleic acid (e.g., mRNA), with full encapsulation, partial encapsulation, or both. In an embodiment, the nucleic acid (e.g., mRNA) is fully encapsulated in the lipid nanoparticle.

As used herein, the term "aqueous solution" refers to a composition comprising water.

"Serum-stable" in relation to nucleic acid-lipid nanoparticles means that the nucleotide is not significantly degraded after exposure to a serum or nuclease assay that would significantly degrade free DNA or RNA. Suitable assays include, for example, a standard serum assay, a DNAse assay, or an RNAse assay.

"Systemic delivery," as used herein, refers to delivery of a therapeutic product that can result in a broad exposure of an active agent within an organism. Some techniques of administration can lead to the systemic delivery of certain agents, but not others. Systemic delivery means that a useful, preferably therapeutic, amount of an agent is exposed to most parts of the body. Systemic delivery of lipid nanoparticles can be by any means known in the art including, for example, intravenous, intraarterial, subcutaneous, and intraperitoneal delivery. In some embodiments, systemic delivery of lipid nanoparticles is by intravenous delivery.

"Local delivery," as used herein, refers to delivery of an active agent directly to a target site within an organism. For example, an agent can be locally delivered by direct injection into a disease site such as a tumor, other target site such as a site of inflammation, or a target organ such as the liver, heart, pancreas, kidney, and the like. Local delivery can also include topical applications or localized injection techniques such as intramuscular, subcutaneous or intradermal injection. Local delivery does not preclude a systemic pharmacological effect.

"Amino acid" refers to naturally-occurring and non-naturally occurring amino acids. An amino acid lipid can be made from a genetically encoded amino acid, a naturally occurring non-genetically encoded amino acid, or a synthetic amino acid. Examples of amino acids include Ala, Arg, Asn, Asp, Cys, Gln, Glu, Gly, His, Ile, Leu, Lys, Met, Phe, Pro, Ser, Thr, Trp, Tyr, and Val. Examples of amino acids also include azetidine, 2-aminooctadecanoic acid, 2-aminoadipic acid, 3-aminoadipic acid, 2,3-diaminopropionic acid, 2-aminobutyric acid, 4-aminobutyric acid, 2,3-diaminobutyric acid, 2,4-diaminobutyric acid, 2-aminoisobutyric acid, 4-aminoisobutyric acid, 2-aminopimelic acid, 2,2'-diaminopimelic acid, 6-aminohexanoic acid, 6-aminocaproic acid, 2-aminoheptanoic acid, desmosine, omithine, citrulline, N-methylisoleucine, norleucine, tert-leucine, phenylglycine, t-butylglycine, N-methylglycine, sacrosine, N-ethylglycine, cyclohexylglycine, 4-oxo-cyclohexylglycine, N-ethylasparagine, cyclohexylalanine, t-butylalanine, naphthylalanine, pyridylalanine, 3-chloroalanine, 3-benzothienylalanine, 4-halophenylalanine, 4-chlorophenylalanine, 2-fluorophenylalanine, 3-fluorophenylalanine, 4-fluorophenylalanine, penicillamine, 2-thienylalanine, methionine, methionine sulfoxide, homoarginine, norarginine, nor-norarginine, N-acetyllysine, 4-aminophenylalanine, N-methylvaline, homocysteine, homoserine, hydroxylysine, allo-hydroxylysine, 3-hydroxyproline, 4-hydroxyproline, isodesmosine, allo-isoleucine, 6-N-methyllysine, norvaline, 0-allyl-serine, 0-allyl-threonine, alpha-aminohexanoic acid, alpha-aminovaleric acid, pyroglutamic acid, and derivatives thereof. "Amino acid" includes alpha- and beta- amino acids. Examples of amino acid residues can be found in Fasman, CRC Practical Handbook of Biochemistry and Molecular Biology, CRC Press, Inc. (1989).

"Alkyl" refers to a straight or branched hydrocarbon chain radical consisting solely of carbon and hydrogen atoms, which is saturated or unsaturated (i.e., contains one or more double (alkenyl) and/or triple bonds (alkynyl)), having, for example, from one to twenty-four carbon atoms (C₁-C₂₄ alkyl), four to twenty carbon atoms (C₄-C₂₀ alkyl), six to sixteen carbon atoms (C₆-C₁₆ alkyl), six to nine carbon atoms (C₆-C₉ alkyl), one to fifteen carbon atoms (C₁-C₁₅ alkyl),one to twelve carbon atoms (Ci-C₁₂ alkyl), one to eight carbon atoms (C₁-C₈ alkyl) or one to six carbon atoms (C₁-C₆ alkyl) and which is attached to the rest of the molecule by a single bond, e.g., methyl, ethyl, n propyl, 1 methylethyl (iso propyl), n butyl, n pentyl, 1,1-dimethylethyl (t butyl), 3-methylhexyl, 2-methylhexyl, ethenyl, prop-1-enyl, but-1-enyl, pent-1-enyl, penta-1,4-dienyl, ethynyl, propynyl, butynyl, pentynyl, hexynyl, and the like. Unless stated otherwise specifically in the specification, an alkyl group is optionally substituted.

"Alkylene" or "alkylene chain" refers to a straight or branched divalent hydrocarbon chain linking the rest of the molecule to a radical group, consisting solely of carbon and hydrogen, which is saturated or unsaturated (i.e., contains one or more double (alkenylene) and/or triple bonds (alkynylene)), and having, for example, from one to twenty-four carbon atoms (C₁-C₂₄ alkylene), one to fifteen carbon atoms (C₁-C₁₅ alkylene),one to twelve carbon atoms (C₁-C₁₂ alkylene), one to eight carbon atoms (Ci-C₈ alkylene), one to six carbon atoms (C₁-C₆ alkylene), two to four carbon atoms (C₂-C₄ alkylene), one to two carbon atoms (C₁-C₂ alkylene), e.g., methylene, ethylene, propylene, n-butylene, ethenylene, propenylene, n-butenylene, propynylene, *n*-butynylene, and the like. The alkylene chain is attached to the rest of the molecule through a single or double bond and to the radical group through a single or double bond. The points of attachment of the alkylene chain to the rest of the molecule and to the radical group can be through one carbon or any two carbons within the chain. Unless stated otherwise specifically in the specification, an alkylene chain may be optionally substituted.

The term "alkenyl" refers to an alkyl, as defined above, containing at least one double bond between adjacent carbon atoms. Alkenyls include both *cis* and *trans* isomers. Representative straight chain and branched alkenyls include, but are not limited to, ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like.

"Alkoxy" refers to an alkyl, cycloalkyl, alkenyl, or alkynyl group covalently bonded to an oxygen atom.

"Alkanoyloxy" refers to -O-C(=O)-alkyl groups.

"Alkylamino" refers to the group -NRR', where R and R' are each either hydrogen or alkyl, and at least one of R and R' is alkyl. Alkylamino includes groups such as piperidino wherein R and R' form a ring. The term "alkylaminoalkyl" refers to -alkyl-NRR'.

The term "alkynyl" includes any alkyl or alkenyl, as defined above, which additionally contains at least one triple bond between adjacent carbons. Representative straight chain and branched alkynyls include, without limitation, acetylenyl, propynyl, 1-butynyl, 2-butynyl, 1-pentynyl, 2-pentynyl, 3-methyl-1 butynyl, and the like.

The terms "acyl," "carbonyl," and "alkanoyl" refer to any alkyl, alkenyl, or alkynyl wherein the carbon at the point of attachment is substituted with an oxo group, as defined below. The following are non-limiting examples of acyl, carbonyl or alkanoyl groups: -C(=O)alkyl, -C(=O)alkenyl, and -C(=O)alkynyl.

"Aryl" refers to any stable monocyclic, bicyclic, or polycyclic carbon ring system of from 4 to 12 atoms in each ring, wherein at least one ring is aromatic. Some examples of an aryl include phenyl, naphthyl, tetrahydro-naphthyl, indanyl, and biphenyl. Where an aryl substituent is bicyclic and one ring is non-aromatic, it is understood that attachment is to the aromatic ring. An aryl may be substituted or unsubstituted.

"Carboxyl" refers to a functional group of the formula -C(=O)OH.

"Cyano" refers to a functional group of the formula -CN.

"Cycloalkyl" or "carbocyclic ring" refers to a stable non-aromatic monocyclic or polycyclic hydrocarbon radical consisting solely of carbon and hydrogen atoms, which may include fused or bridged ring systems, having from three to fifteen carbon atoms, preferably having from three to ten carbon atoms, and which is saturated or unsaturated and attached to the rest of the molecule by a single bond. Monocyclic radicals include, for example, cyclopropyl, cyclobutyl, cyclopentyl, cyclohexyl, cycloheptyl, and cyclooctyl. Polycyclic radicals include, for example, adamantyl, norbornyl, decalinyl, 7,7-dimethyl-bicyclo[2.2.1]heptanyl, and the like. Unless otherwise stated specifically in the specification, a cycloalkyl group may be optionally substituted.

"Cycloalkylene" is a divalent cycloalkyl group. Unless otherwise stated specifically in the specification, a cycloalkylene group may be optionally substituted.

The term "diacylglycerol" or "DAG" includes a compound having 2 fatty acyl chains, both of which have independently between 2 and 30 carbons bonded to the 1- and 2-position of glycerol by ester linkages. The acyl groups can be saturated or have varying degrees of unsaturation. Suitable acyl groups include, but are not limited to, lauroyl (C12), myristoyl (C14), palmitoyl (C16), stearoyl (C18), and icosoyl (C20). In preferred embodiments, the fatty acid acyl chains of one compound are the same, i.e., both myristoyl (i.e., dimyristoyl), both stearoyl (i.e., distearoyl), etc.

The term "heterocycle" or "heterocyclyl" refers to an aromatic or nonaromatic ring system of from five to twenty-two atoms, wherein from 1 to 4 of the ring atoms are heteroatoms selected from oxygen, nitrogen, and sulfur. Thus, a heterocycle may be a heteroaryl or a dihydro or tetrathydro version thereof. Heterocycles include, but are not limited to, pyrrolidine, tetryhydrofuran, thiolane, azetidine, oxetane, thietane, diazetidine, dioxetane, dithietane, piperidine, tetrahydrofuran, pyran, tetrahydropyran, thiacyclohexane, tetrahydrothiophene, pyridine, pyrimidine and the like.

"Heteroaryl" refers to any stable monocyclic, bicyclic, or polycyclic carbon ring system of from 4 to 12 atoms in each ring, wherein at least one ring is aromatic and contains from 1 to 4 heteroatoms selected from oxygen, nitrogen and sulfur. Some examples of a heteroaryl include acridinyl, quinoxalinyl, pyrazolyl, indolyl, benzotriazolyl, furanyl, thienyl, benzothienyl, benzofuranyl, quinolinyl, isoquinolinyl, oxazolyl, isoxazolyl, pyrazinyl, pyridazinyl, pyridinyl, pyrimidinyl, pyrrolyl, and tetrahydroquinolinyl. A heteroaryl includes the N-oxide derivative of a nitrogen-containing heteroaryl.

The terms "alkylamine" and "dialkylamine" refer to ---NH(alkyl) and ---N(alkyl)₂ radicals respectively.

The term "alkylphosphate" refers to ---O---P(Q')(Q")-O---R, wherein Q' and Q" are each independently O, S, N(R)₂, optionally substituted alkyl or alkoxy; and R is optionally substituted alkyl, ω-aminoalkyl or ω-(substituted)aminoalkyl.

The term "alkylphosphorothioate" refers to an alkylphosphate wherein at least one of Q' or Q" is S.

The term "alkylphosphonate" refers to an alkylphosphate wherein at least one of Q' or Q" is alkyl.

"Hydroxyalkyl" refers to an ---O-alkyl radical.

The term "alkylheterocycle" refers to an alkyl where at least one methylene has been replaced by a heterocycle.

The term "ω-aminoalkyl" refers to -alkyl-NH₂ radical. And the term "ω-(substituted)aminoalkyl refers to an ω-aminoalkyl wherein at least one of the H on N has been replaced with alkyl.

The term "ω-phosphoalkyl" refers to -alkyl-O---P(Q')(Q")-O---R, wherein Q' and Q" are each independently O or S and R optionally substituted alkyl.

The term "ω-thiophosphoalkyl" refers to ω-phosphoalkyl wherein at least one of Q' or Q" is S.

The term "substituted" used herein means any of the above groups (e.g., alkyl, alkylene, cycloalkyl or cycloalkylene) wherein at least one hydrogen atom is replaced by a bond to a non-hydrogen atom such as, but not limited to: a halogen atom such as F, Cl, Br, or I; oxo groups (=O); hydroxyl groups (-OH); C₁-C₁₂ alkyl groups; cycloalkyl groups; -(C=O)OR'; -O(C=O)R'; -C(=O)R^{'}; -OR^{'}; -S(O)ₓR^{'}; -S-SR^{'}; -C(=O)SR^{'}; -SC(=O)R^{'}; -NR^{'}R^{'}; -NR^{'}C(=O)R^{'}; -C(=O)NR^{'}R^{'}; -NR^{'}C(=O)NR^{'}R^{'}; -OC(=O)NR^{'}R^{'}; -NR^{'}C(=O)OR^{'}; -NR^{'}S(O)ₓNR^{'}R^{'}; -NR^{'}S(O)ₓR^{'}; and -S(O)ₓNR^{'}R^{'}, wherein: R^{'} is, at each occurrence, independently H, C₁-C₁₅ alkyl or cycloalkyl, and x is 0, 1 or 2. In some embodiments the substituent is a C₁-C₁₂ alkyl group. In other embodiments, the substituent is a cycloalkyl group. In other embodiments, the substituent is a halo group, such as fluoro. In other embodiments, the substituent is an oxo group. In other embodiments, the substituent is a hydroxyl group. In other embodiments, the substituent is an alkoxy group (-OR). In other embodiments, the substituent is a carboxyl group. In other embodiments, the substituent is an amine group(-NR^{'}R^{'}).

"Optional" or "optionally" (e.g., optionally substituted) means that the subsequently described event of circumstances may or may not occur, and that the description includes instances where said event or circumstance occurs and instances in which it does not. For example, "optionally substituted alkyl" means that the alkyl radical may or may not be substituted and that the description includes both substituted alkyl radicals and alkyl radicals having no substitution.

"Prodrug" is meant to indicate a compound, such as a therapeutic agent, that may be converted under physiological conditions or by solvolysis to a biologically active compound of the invention. Thus, the term "prodrug" refers to a metabolic precursor of a compound of the invention that is pharmaceutically acceptable. A prodrug may be inactive when administered to a subject in need thereof, but is converted *in vivo* to an active compound of the invention. Prodrugs are typically rapidly transformed *in vivo* to yield the parent compound of the invention, for example, by hydrolysis in blood. The prodrug compound often offers advantages of solubility, tissue compatibility or delayed release in a mammalian organism (see, Bundgard, H., Design of Prodrugs (1985), pp. 7-9, 21-24 (Elsevier, Amsterdam)). A discussion of prodrugs is provided in Higuchi, T., et al., A.C.S. Symposium Series, Vol. 14, and in Bioreversible Carriers in Drug Design, Ed. Edward B. Roche, American Pharmaceutical Association and Pergamon Press, 1987.

The term "prodrug" is also meant to include any covalently bonded carriers, which release the active compound of the invention *in vivo* when such prodrug is administered to a mammalian subject. Prodrugs (e.g., a prodrug of a therapeutic agent) may be prepared by modifying functional groups present in the compound of the invention in such a way that the modifications are cleaved, either in routine manipulation or *in vivo,* to the parent compound of the invention. Prodrugs include compounds wherein a hydroxy, amino or mercapto group is bonded to any group such that, when the prodrug is administered to a mammalian subject, cleaves to form a free hydroxy, free amino or free mercapto group, respectively. Examples of prodrugs include, but are not limited to, acetate, formate and benzoate derivatives of alcohol or amide derivatives of amine functional groups in the therapeutic agents of the invention and the like.

Embodiments of the invention disclosed herein are also meant to encompass all pharmaceutically acceptable lipid nanoparticles and components thereof (e.g., cationic lipid, therapeutic agent, etc.) being isotopically-labelled by having one or more atoms replaced by an atom having a different atomic mass or mass number. Examples of isotopes that can be incorporated into the disclosed compounds include isotopes of hydrogen, carbon, nitrogen, oxygen, phosphorous, fluorine, chlorine, and iodine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. These radiolabeled LNPs could be useful to help determine or measure the effectiveness of the compounds, by characterizing, for example, the site or mode of action, or binding affinity to pharmacologically important site of action. Certain isotopically-labelled LNPs, for example, those incorporating a radioactive isotope, are useful in drug and/or substrate tissue distribution studies. The radioactive isotopes tritium, i.e., ³H, and carbon-14, that is, ¹⁴C, are particularly useful for this purpose in view of their ease of incorporation and ready means of detection.

Substitution with heavier isotopes such as deuterium, that is, ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of used in the present disclosure can generally be prepared by conventional techniques known to those skilled in the art or by processes analogous to those described in the Examples as set out below using an appropriate isotopically-labeled reagent in place of the non-labeled reagent previously employed.

"Stable compound" and "stable structure" are meant to indicate a compound that is sufficiently robust to survive isolation to a useful degree of purity from a reaction mixture, and formulation into an efficacious therapeutic agent.

"Mammal" includes humans and both domestic animals such as laboratory animals and household pets (e.g., cats, dogs, swine, cattle, sheep, goats, horses, rabbits), and non-domestic animals such as wildlife and the like. "Primate" includes both human and non-human primates.

"Pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

"Pharmaceutically acceptable salt" includes both acid and base addition salts.

"Pharmaceutically acceptable acid addition salt" refers to those salts which retain the biological effectiveness and properties of the free bases, which are not biologically or otherwise undesirable, and which are formed with inorganic acids such as, but are not limited to, hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, *p*-toluenesulfonic acid, trifluoroacetic acid, undecylenic acid, and the like.

"Pharmaceutically acceptable base addition salt" refers to those salts which retain the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Preferred inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, *N*-ethylpiperidine, polyamine resins and the like. Particularly preferred organic bases are isopropylamine, diethylamine, ethanolamine, trimethylamine, dicyclohexylamine, choline and caffeine.

A "pharmaceutical composition" refers to a formulation of an LNP of the invention and a medium generally accepted in the art for the delivery of the biologically active compound to mammals, e.g., humans. Such a medium includes all pharmaceutically acceptable carriers, diluents or excipients therefor.

"Effective amount" or "therapeutically effective amount" refers to that amount of a compound of the invention which, when administered to a mammal, preferably a human, is sufficient to effect treatment in the mammal, preferably a human. The amount of a lipid nanoparticle of the invention which constitutes a "therapeutically effective amount" will vary depending on the compound, the condition and its severity, the manner of administration, and the age of the mammal to be treated, but can be determined routinely by one of ordinary skill in the art having regard to his own knowledge and to this disclosure.

"Treating" or "treatment" as used herein covers the treatment of the disease or condition of interest in a mammal, preferably a human, having the disease or condition of interest, and includes:
(i) preventing the disease or condition from occurring in a mammal, in particular, when such mammal is predisposed to the condition but has not yet been diagnosed as having it;
(ii) inhibiting the disease or condition, i.e., arresting its development;
(iii) relieving the disease or condition, i.e., causing regression of the disease or condition; or
(iv) relieving the symptoms resulting from the disease or condition, i.e., relieving pain without addressing the underlying disease or condition. As used herein, the terms "disease" and "condition" may be used interchangeably or may be different in that the particular malady or condition may not have a known causative agent (so that etiology has not yet been worked out) and it is therefore not yet recognized as a disease but only as an undesirable condition or syndrome, wherein a more or less specific set of symptoms have been identified by clinicians.

### Lipid Nanoparticles and Methods of Use Thereof

Embodiments disclosed herein are directed to methods of using LNPs for delivery of a therapeutic agent, such as a nucleic acid, to a primate, such as a human, for treatment of various diseases treatable with the nucleic acid. The present Applicant has discovered that the disclosed methods are surprisingly more effective for delivery of therapeutic agents to primates, compared with delivery of the same therapeutic agent to a non-primate, such as a mouse. For example, some methods include use of LNPs having a diameter smaller than typical LNPs, for example a mean particle diameter ranging from about 40-70 nm, or for instance, a mean particle diameter ranging from about 50-70 nm, and such LNPs have unexpectedly improved delivery in primates relative to rodent. Other methods comprise use of LNPs with higher concentrations of PEGylated lipid (e.g., from about 2.0 to 3.5%). Othere exemplary methods comprise delivering LNPs to primates, wherein the LNPs include a PEGylated lipid having two acyl chains independently comprising from 8 to 14 carbon atoms, with the sum of the carbon atoms in the acyl chains not exceeding 27. The LNPs can be delivered intraveneously or via other administration routes known in the art. Further details of these exemplary embodiments, and others, will be apparent in view of the details described herein.

Accordingly, in one embodiment is provided a method for delivering a nucleic acid to a primate in need thereof, comprising administering a lipid nanoparticle (LNP) to the primate, the LNP comprising:
i) a nucleic acid, or a pharmaceutically acceptable salt thereof, encapsulated within the LNP;
ii) a cationic lipid;
iii) a neutral lipid;
iv) a steroid; and
v) from 2.0 to 3.5 mol percent of a polymer-conjugated lipid based on total mol of lipids in the LNP.

The mol percent of polymer-conjugated lipid is determined based on the total mol percent of lipid present in the LNP. For this calculation, all lipid components, including for example, cationic lipid, neutral lipid, steroid and any other lipids, such as anionic or other lipids, are included in the calculation.

In certain embodiments, the LNP comprises from 2.0 to 3.4 mol of the polymer conjugated lipid. In other embodiments, the LNP comprises from 2.1 to 3.5 mol of the polymer conjugated lipid. In more embodiments, the LNP comprises from 2.2 to 3.3 mol percent of the polymer-conjugated lipid, for example 2.3 to 2.8 mol percent of the polymer-conjugated lipid. In other embodiments, the LNP comprises from 2.1 to 2.5 mol percent of the polymer-conjugated lipid. In other different embodiments, the LNP comprises from 2.5 to 2.9 mol percent of the polymer-conjugated lipid. In other embodiments, the LNP comprises from 2.4 to 2.6 mol percent of the polymer conjugated lipid, from 2.6 to 2.8 mol percent of the polymer conjugated lipid, from 2.4 to 2.5 mol percent of the polymer conjugated lipid or from 2.5 to 2.7 mol percent of the polymer conjugated lipid. In still different embodiments, the LNP comprises about 2.3, about 2.35, about 2.4, about 2.45, about 2.5, about 2.55, about 2.6, about 2.65 about 2.7, about 2.75 or about 2.8 mol percent of the polymer-conjugated lipid.

Another embodiment is directed to a method for delivering a nucleic acid to a primate in need thereof, comprising administering a lipid nanoparticle (LNP) to the primate, the LNP comprising:
i) a nucleic acid, or a pharmaceutically acceptable salt thereof, encapsulated within the LNP;
ii) a cationic lipid;
iii) a neutral lipid;
iv) a steroid; and
v) a polymer-conjugated lipid,
wherein a plurality of the LNPs has a mean particle diameter ranging from 40 nm to 70 nm.

In certain embodiments, the mean particle diameter ranges from 45 nm to 70 nm, 50 nm to 70 nm, 55 nm to 65 nm, from 50 nm to 60 nm or from 60 nm to 70 nm. In different embodiments, the mean particle diameter ranges from 45 nm to 50 nm, 50 nm to 55 nm, from 55 nm to 60 nm, from 60 nm to 65 nm or from 65 nm to 70 nm. In still more embodiments, the mean particle diameter is about 45 nm, 46 nm, 47 nm, 48 nm, 49 nm, 50 nm, about 51 nm, about 52 nm, about 53 nm, about 54 nm, about 55 nm, about 56 nm, about 57 nm, about 58 nm, about 59 nm, about 60 nm, about 61 nm, about 62 nm, about 63 nm, about 64 nm or about 65 nm, about 66 nm, about 67 nm, about 68 nm, about 69 nm or about 70 nm (not according to the invention unless embraced by the claims).

In any of the foregoing embodiments, the polymer-conjugated lipid has the following structure: 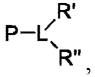 wherein:
P is a polymer;
L is a trivalent linker of 1 to 15 atoms in length; and
R' and R" are each independently a saturated alkyl having from 8 to 14 carbon atoms.

In some embodiments, P comprises a polyethylene glycol polymer, for example a hydroxyl or alkoxyl-terminating (PEG-OR) polyethylene glycol polymer. A hydroxyl-terminating polyethylene glycol polymer (PEG-OH) is a polyethylene glycol polymer which terminates with a hydroxyl group, while an alkoxyl-terminating polyethylene glycol polymer (PEG-OR) is a polyethylene glycol polymer which terminates with an alkoxyl group, such as methoxy.

Any suitable linker can be used for L. In some exemplary embodiments, L comprises amide, ester and/or carbamate functional groups. For example, in some embodiments the polymer conjugated lipid has one of the following structures: 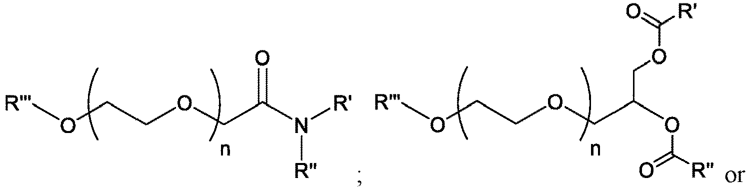 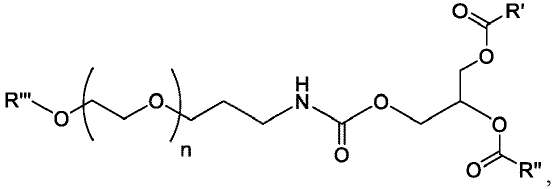 wherein n is an integer ranging from 30 to 60, R' and R" are each independently a saturated alkyl having from 8 to 14 carbon atoms and R‴ is H or C₁-C₆ alkyl.

In other more specific embodiments, the polymer conjugated lipid has the following structure: 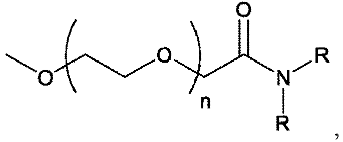 wherein n is an integer ranging from 40 to 50, and each R is a saturated alkyl having from 8 to 14 carbon atoms, or 8 to 12 carbon atoms, or 8 carbon atoms, or 10 carbon atoms, or 12 carbon atoms. In some embodiments, each R is 8, each R is 9, each R is 10, each R is 11, each R is 12, each R is 13 or each R is 14. Embodiments wherein each R is not the same are also envisioned, such as embodiments wherein one R is 12 and one R is 13, or one R is 13 and one R is 14, or one R is 11 and one R is 12, or one R is 10 and one R is 11 and the like.

In other different embodiments, the polymer-conjugated lipid has the following structure: 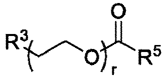 wherein:
R³ is -OR^{O};
R^{O} is hydrogen or alkyl;
r is an integer from 30 to 60, inclusive;
R⁵ is C₁₀₋₂₀ alkyl.

For example, in certain embodiments:
R³ is OH or OCH₃;
R⁵ is C₁₈, C₁₉ or C₂₀; and
r is selected such that 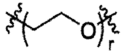 has an average molecular weight ranging from 1,800 Da to 2,200 Da.

In yet other embodiments is provided a method for delivering a nucleic acid to a primate in need thereof, comprising administering a lipid nanoparticle (LNP) to the primate, the LNP comprising:
i) a nucleic acid, or a pharmaceutically acceptable salt thereof, encapsulated within the LNP;
ii) a cationic lipid;
iii) a neutral lipid;
iv) a steroid; and
v) a polymer-conjugated lipid having the following structure: 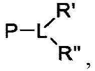 wherein:
   P is a polymer;
   L is a trivalent linker of 1 to 15 atoms in length; and
   R' and R" are each independently a saturated alkyl having from 8 to 14 carbon atoms, provided that the total number of carbon atoms collectively in both of R' and R" is no more than 27.

In certain embodiments of the foregoing, P comprises a polyethylene glycol polymer, such as a hydroxyl or alkoxyl-terminating polyethylene glycol polymer.

In other embodiments, L comprises amide, ester and/or carbamate functional groups, for example in some embodiments the polymer conjugated lipid has one of the following structures: 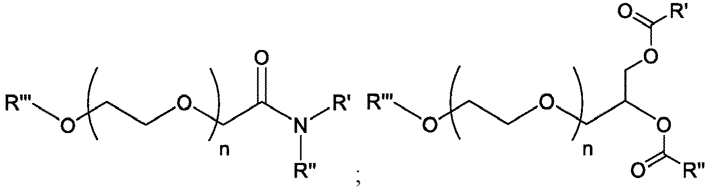 or 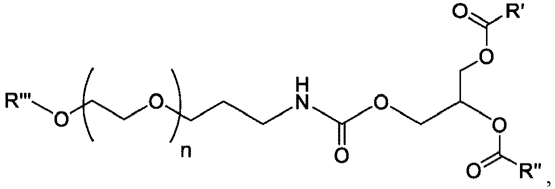 wherein R‴ is H or C₁-C₆ alkyl, and n is an integer ranging from 30 to 60.

In more specific embodiments, the polymer conjugated lipid has the following structure: 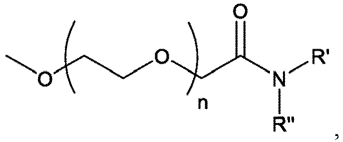 wherein n is an integer ranging from 40 to 50.

In certain of the foregoing embodiments, the total number of carbon atoms in R' and R" ranges from 16 to 25, 16 to 24, 17 to 24 or 18 to 24. For example, in some embodiments:
a) R' and R" are each a saturated alkyl having 8 carbon atoms;
b) R' and R" are each a saturated alkyl having 9 carbon atoms;
c) R' and R" are each a saturated alkyl having 10 carbon atoms;
d) R' and R" are each a saturated alkyl having 11 carbon atoms;
e) R' and R" are each a saturated alkyl having 12 carbon atoms; or
f) R' and R" are each a saturated alkyl having 13 carbon atoms.

Asymmetric polymer conjugated lipids, wherein R' and R" are different are also included in various embodiments, such as wherein R' is 12 and R" is 13, or R' is 13 and R" is 14, or R' is 11 and R" is 12, or R' is 10 and R" is 11 and the like

In some embodiments, the lipid nanoparticle comprises a cationic lipid, a PEGylated lipid, a sterol and a neutral lipid. In some embodiments, the lipid nanoparticle comprises a molar ratio of about 20-60% cationic lipid: 5-25% neutral lipid: 25-55% sterol; and 0.1-15% PEGylated lipid. In some embodiments, the cationic lipid is an ionizable cationic lipid. In some embodiments, the neutral lipid is a phospholipid. In some embodiments, the sterol is a cholesterol. In some embodiments, the cationic lipid is selected from 2,2-dilinoleyl-4-dimethylaminoethyl-[1,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319). In some embodiments, the lipid nanoparticle has a polydispersity value of less than 0.4. In some embodiments, the lipid nanoparticle has a net neutral charge at a neutral pH. In some embodiments, the lipid nanoparticle has a mean diameter of 40-200 nm..

Lipid nanoparticles may comprise one or more lipid species, including, but not limited to, cationic/ionizable lipids, neutral lipids, structural lipids, phospholipids, and helper lipids. Any of these lipids may be conjugated to polyethylene glycol (PEG) and thus may be referred to as PEGylated lipids or PEG-modified lipids.

The formation of the lipid nanoparticle (LNP) may be accomplished by methods known in the art and/or as described in U.S. Pub. No. 2012/0178702.

A lipid nanoparticle formulation may be influenced by, but not limited to, the selection of the cationic lipid component, the degree of cationic lipid saturation, the selection of the neutral lipid component, the degree of neutral lipid saturation, the selection of the structural lipid component, the nature of the PEGylation, ratio of all components and biophysical parameters such as size. In certain non-limiting examples, a LNP comprises four basic components: (1) a cationic lipid; (2) a neutral lipid (e.g., a phospholipid such as DSPC); (3) a structural lipid (e.g., a sterol such as cholesterol); and (4) a PEGylated lipid. In one example by Semple et al. (Nature Biotech. 2010 28:172-176), the lipid nanoparticle formulation is composed of molar ratios as follows: 57.1% cationic lipid, 7.1% dipalmitoylphosphatidylcholine, 34.3% cholesterol, and 1.4% PEG-c-DMA. As another example, changing the composition of the cationic lipid can more effectively deliver siRNA to various antigen presenting cells (Basha et al., Mol Ther. 2011 19:2186-2200).

In certain embodiments, the lipid nanoparticle comprises a cationic lipid and a neutral lipid. In certain embodiments, the LNP comprises a cationic lipid and a DSPC substitute. In certain embodiments, the LNP comprises a cationic lipid and a fatty acid. In certain embodiments, the LNP a cationic lipid and oleic acid. In certain embodiments, the LNP comprises a cationic lipid and an analog of oleic acid.

In certain embodiments, the lipid nanoparticle formulation comprises a cationic lipid, a neutral lipid, and a structural lipid. In certain embodiments, the LNP comprises a cationic lipid, a fatty acid, and a structural lipid. In certain embodiments, the LNP comprises a cationic lipid, oleic acid, and a structural lipid. In certain embodiments, the LNP comprises a cationic lipid, an analog of oleic acid, and a structural lipid. In certain embodiments, the LNP comprises a cationic lipid, a fatty acid, and a sterol. In certain embodiments, the LNP comprises a cationic lipid, oleic acid, and a sterol. In certain embodiments, the LNP comprises a cationic lipid, oleic acid, and cholesterol.

In certain embodiments, the lipid nanoparticle comprises a cationic lipid, a neutral lipid, and a PEGylated lipid. In certain embodiments, the LNP formulation comprises a cationic lipid, a neutral lipid, and a PEG-OH lipid. In certain embodiments, the lipid nanoparticle comprises a cationic lipid, a fatty acid, and a PEG-OH lipid. In certain embodiments, the lipid nanoparticle comprises a cationic lipid, oleic acid, and a PEG-OH lipid. In certain embodiments, the lipid nanoparticle comprises a cationic lipid, an analog of oleic acid, and a PEG-OH lipid.

In certain embodiments, the lipid nanoparticle comprises a cationic lipid, a neutral lipid (e.g., a phospholipid or fatty acid), a structural lipid, and a PEG lipid. In certain embodiments, the lipid nanoparticle formulation comprises a cationic lipid, a neutral lipid (e.g., phospholipid or fatty acid), a structural lipid, and a PEG-OH lipid. In certain embodiments, the LNP comprises a cationic lipid, a neutral lipid (e.g., phospholipid or fatty acid), and a structural lipid. In certain embodiments, the LNP comprises a cationic lipid, a fatty acid (e.g., oleic acid or an analog thereof), a structural lipid, and a PEG lipid. In certain embodiments, the LNP comprises a cationic lipid, a fatty acid (e.g., oleic acid or an analog thereof), a structural lipid, and a PEG-OH lipid. In certain embodiments, the LNP comprises a cationic lipid, oleic acid, a structural lipid (e.g., a sterol), and a PEG-OH lipid. In certain embodiments, the LNP comprises a cationic lipid, oleic acid, and a structural lipid (e.g., cholesterol). In certain embodiments, the LNP comprises one or more cationic or neutral lipids, a fatty acid (e.g., oleic acid), and a PEG lipid. In certain embodiments, the LNP comprises one or more cationic or neutral lipids, a fatty acid (e.g., oleic acid), and a PEG-OH lipid.

In some embodiments, the LNP comprises a fatty acid. In certain embodiments, the fatty acid is a monounsaturated fatty acid. In certain embodiments, the fatty acid is a polyunsaturated fatty acid. In some embodiments, the LNP comprises oleic acid. In certain embodiments, the LNP comprises one or more cationic or neutral lipids, and a fatty acid (e.g., oleic acid). In certain embodiments, the LNP comprises one or more cationic or neutral lipids, and oleic acid. In certain embodiments, when the LNP includes oleic acid, the LNP does not include a phospholipid. In certain embodiments, when the LNP includes oleic acid, the LNP does not include DSPC. In certain embodiments, when the LNP includes a fatty acid, the LNP does not include a phospholipid. In certain embodiments, when the LNP includes a fatty acid, the LNP does not include DSPC.

In some embodiments, LNPs may comprise, in molar percentages, 35 to 45% cationic lipid, 40% to 50% cationic lipid, 45% to 55% cationic lipid, 50% to 60% cationic lipid and/or 55% to 65% cationic lipid. In some embodiments, the ratio of lipid to nucleic acid (e.g., mRNA) in lipid nanoparticles may be 5:1 to 20:1, 10:1 to 25:1, 15:1 to 40:1, 20:1 to 30:1, 25:1 to 50:1, 30:1 to 60:1 and/or at least 40:1.

In some embodiments, the ratio of PEG in the LNPs may be increased or decreased and/or the carbon chain length of the alkyl portion of the PEG lipid may be varied from C8 to C18 (eight to eighteen carbons) to alter the pharmacokinetics and/or biodistribution of the LNPs. In certain embodiments, LNPs may contain 0.1% to 3.0%, 1.0% to 3.5%, 1.5% to 4.0%, 2.0% to 4.5%, 2.0% to 3.0%, 2.5% to 5.0%, and/or 3.0% to 6.0% of PEGylated lipid relative to the other components. As a non-limiting example, LNPs may contain 0.5% to 3.0%, 1.0% to 3.5%, 1.5% to 4.0%, 2.0% to 4.5%, 2.0% to 3.0%, 2.5% to 5.0%, and/or 3.0% to 6.0% of PEG-c-DOMG (R-3-[(ω-methoxy-poly(ethyleneglycol)2000)carbamoyl)]-1,2-dimyristyloxypropyl-3-amine) (also referred to herein as PEG-DOMG) as compared to the cationic lipid, DSPC, and cholesterol. In some embodiments, the PEG-c-DOMG may be replaced with a PEG lipid such as, but not limited to, PEG-DSG (1,2-distearoyl-sn-glycerol, methoxypolyethylene glycol), DMG-PEG (1,2-dimyristoyl-sn-glycerol) and/or PEG-DPG (1,2-dipalmitoyl-sn-glycerol, methoxypolyethylene glycol). The cationic lipid may be selected from any lipid known in the art such as, but not limited to, DLin-MC3-DMA, DLin-DMA, C12-200, and DLin-KC2-DMA. In certain embodiments, the lipid nanoparticle does not contain a PEG lipid. In certain embodiments, the lipid nanoparticle contains a PEG lipid such as a PEG-OH lipid. Incorporation of PEG-OH lipids in the nanoparticle formulation can improve the pharmacokinetics and/or biodistribution of the LNPs. For example, incorporation of PEG-OH lipids in the nanoparticle formulation can reduce the ABC effect. In certain embodiments, LNPs may contain 0.5% to 3.0%, 1.0% to 3.5%, 1.5% to 4.0%, 2.0% to 4.5%, 2.0% to 5.0%, 2.5% to 5.0%, and/or 3.0% to 6.0% of the lipid molar ratio of PEG-OH lipid to the other components (e.g., the cationic, neutral, and structural lipids). Each possibility represents a separate embodiment of the present invention.

In some embodiments, a LNP comprises at least one lipid. In certain embodiments, the lipids is selected from cationic/ionizable lipids, neutral lipids (e.g., fatty acids and phospholipids), PEG lipids (e.g., PEG-OH lipids, methyl PEG (mPEG) lipids, ethyl PEG lipids, and other derivatized PEG lipid conjugates), and structural lipids (e.g., sterols). The lipid may be selected from, but is not limited to, DLin-DMA, DLin-K-DMA, 98N12-5, C12-200, DLin-MC3-DMA, DLin-KC2-DMA, DODMA, PLGA, PEG, PEG-DMG, PEGylated lipids, and amino alcohol lipids. In some embodiments, the lipid may be a cationic lipid, such as, but not limited to, DLin-DMA, DLin-D-DMA, DLin-MC3-DMA, DLin-KC2-DMA, DODMA, and amino alcohol lipids. The amino alcohol cationic lipid may be the lipids described in and/or made by the methods described in US Patent Publication No. US2013/0150625. As a non-limiting example, the cationic lipid may be 2-amino-3-[(9Z,12Z)-octadeca-9,12-dien-1-yloxy]-2-{[(9Z,2Z)-octadeca-9,12-dien-1-yloxy]methyl}propan-1-ol (Compound 1 in US2013/0150625); 2-amino-3-[(9Z)-octadec-9-en-1-yloxy]-2-{[(9Z)-octadec-9-en-1-yloxy]methyl}propan-1-ol (Compound 2 in US20130150625); 2-amino-3-[(9Z,12Z)-octadeca-9,12-dien-1-yloxy]-2-[(octyloxy)methyl]propan-1-ol (Compound 3 in US2013/0150625); and 2-(dimethylamino)-3 -[(9Z,12Z)-octadeca-9,12-dien-1-yloxy]-2-{[(9Z,12Z)-octadeca-9,12-dien-1-yloxy]methyl}propan-1-ol (Compound 4 in US2013/0150625); or any pharmaceutically acceptable salt or stereoisomer thereof. Each possibility represents a separate embodiment of the present invention.

Lipid nanoparticle formulations can comprise a lipid, in particular, an ionizable cationic lipid, for example, 2,2-dilinoleyl-4-dimethylaminoethyl-[1,3]-dioxolane (DLin-KC2-DMA), dilinoleyl-methyl-4-dimethylaminobutyrate (DLin-MC3-DMA), or di((Z)-non-2-en-1-yl) 9-((4-(dimethylamino)butanoyl)oxy)heptadecanedioate (L319), and further comprise a neutral lipid (e.g., phospholipid or fatty acid), a structural lipid (e.g., a sterol such as cholesterol), and a molecule capable of reducing particle aggregation, for example, a PEG or PEGylated lipid (e.g., mPEG lipid or PEG-OH lipid). In certain embodiments, the formulation does not contain the PEG lipid.

In some embodiments, the LNP formulation consists essentially of a molar ratio of 20-60% cationic lipid; 5-25% neutral lipid; 25-55% sterol; 0.1-15% PEG lipid. In some embodiments, the LNP formulation consists essentially of a molar ratio of 20-60% cationic lipid; 5-25% neutral lipid; 25-55% sterol; 0.1-15% mPEG lipid. In some embodiments, the LNP formulation consists essentially of in a molar ratio of 20-60% cationic lipid; 5-25% neutral lipid; and 25-55% sterol. In certain embodiments, the neutral lipid is a fatty acid. In certain embodiments, the neutral lipid is oleic acid or an analog thereof. In certain embodiments, the PEG lipid is a mPEG lipid or a PEG-OH lipid.

In some embodiments, a LNP consists essentially of (i) at least one lipid selected from the group consisting of 2,2-dilinoleyl-4-dimethylaminoethyl-[1,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319); (ii) a neutral lipid selected from DSPC, DPPC, POPC, DOPE, and SM; (iii) a sterol, e.g., cholesterol; and (iv) a PEG-lipid, e.g., PEG-DMG or PEG-cDMA, in a molar ratio of 20-60% cationic lipid; 5-25% neutral lipid; 25-55% sterol; 0.1-15% PEG-lipid. Each possibility represents a separate embodiment of the present invention.

In some embodiments, a LNP consists essentially of (i) at least one lipid selected from the group consisting of 2,2-dilinoleyl-4-dimethylaminoethyl-[1,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319); (ii) a neutral lipid as a DSPC substitute (e.g., a different phospholipid, or a fatty acid); (iii) a structural lipid (e.g., a sterol such as cholesterol); and (iv) a PEG-lipid or a PEG-OH lipid (e.g., PEG-DMG or PEG-cDMA), in a molar ratio of 20-60% cationic lipid; 5-25% DSPC substitute; 25-55% structural lipid; 0.1-15% PEG-lipid. Each possibility represents a separate embodiment of the present invention.

In some embodiments, a LNP includes 25% to 75% on a molar basis of a cationic lipid. The cationic lipid may be selected from 2,2-dilinoleyl-4-dimethylaminoethyl-[1,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319), e.g., 35 to 65%, 45 to 65%, 60%, 57.5%, 50% or 40% on a molar basis. Each possibility represents a separate embodiment of the present invention.

In some embodiments, a LNP includes 0.5% to 15% on a molar basis of the neutral lipid, e.g., 3 to 12%, 5 to 10% or 15%, 10%, or 7.5% on a molar basis. In certain embodiments, the neutral lipid is a phospholipid. In certain embodiments, the neutral lipid is a DSPC substitute (e.g., a phospholipid other than DSPC, %or a fatty acid). In certain embodiments, the neutral lipid is a fatty acid (e.g., oleic acid or an analog thereof). Other examples of neutral lipids include, without limitation, POPC, DPPC, DOPE and SM. In some embodiments, a LNP includes 0.5% to 15% on a molar basis of a fatty acid, e.g., 3 to 12%, 5 to 10% or 15%, 10%, or 7.5% on a molar basis. In some embodiments, a LNP includes 0.5% to 15% on a molar basis of oleic acid, e.g., 3 to 12%, 5 to 10% or 15%, 10%, or 7.5% on a molar basis. In some embodiments, a LNP includes 0.5% to 15% on a molar basis of an analog of oleic acid, e.g., 3 to 12%, 5 to 10% or 15%, 10%, or 7.5% on a molar basis.

In some embodiments, the formulation includes 5% to 50% on a molar basis of the structural lipid, e.g., 15 to 45%, 20 to 40%, 41%, 38.5%, 35%, or 31% on a molar basis. In some embodiments, the formulation includes 5% to 50% on a molar basis of a sterol, e.g., 15 to 45%, 20 to 40%, 41%, 38.5%, 35%, or 31% on a molar basis. In some other embodiments, the formulation includes about 35%, about 36%, about 37%, about 38%, about 39%, about 40%, about 41%, about 42%, about 43%, about 44% or about 45% on a molar basis. A non-limiting example of a sterol is cholesterol.

In some embodiments, a LNP includes 0.5% to 20% on a molar basis of the PEG or PEGylated lipid, e.g., 0.5 to 10%, 0.5 to 5%, 1.5%, 0.5%, 1.5%, 2.0%, 2.5%, 3.0%3.5%, or 5% on a molar basis. In some embodiments, a PEG or PEGylated lipid comprises a PEG molecule of an average molecular weight of 2,000 Da. In some embodiments, a PEG or PEGylated lipid comprises a PEG molecule of an average molecular weight of less than 2,000, for example, around 1,500 Da, around 1,000 Da, or around 500 Da. Non-limiting examples of PEGylated lipids include PEG-distearoyl glycerol (PEG-DMG) (also referred herein as Cmpd422), PEG-cDMA (further discussed in Reyes et al. J. Controlled Release, 107, 276-287 (2005). As described herein, any PEG lipids or PEGylated lipids may be PEG-OH lipids. In some embodiments, a LNP includes 0.5% to 20% on a molar basis of a PEG-OH lipid, e.g., 0.5 to 10%, 0.5 to 5%, 1.5%, 0.5%, 1.5%, 3.5%, or 5% on a molar basis.

In some embodiments, LNPs include 25-75% of a cationic lipid, 0.5-15% of the neutral lipid; 5-50% of the structural lipid, and 0.5-20% of the PEG or PEGylated lipid on a molar basis. In some embodiments, LNPs include 25-75% of a cationic lipid, 0.5-15% of the neutral lipid; 5-50% of the structural lipid, and 0.5-20% of a PEG-OH lipid on a molar basis. In some embodiments, LNPs include 25-75% of a cationic lipid, 0.5-15% of the neutral lipid, and 5-50% of the structural lipid on a molar basis. In some embodiments, LNPs include 25-75% of a cationic lipid selected from 2,2-dilinoleyl-4-dimethylaminoethyl-[1,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319).

In some embodiments, LNPs include 35-65% of a cationic lipid, 3-12% of the neutral lipid, 15-45% of the structural lipid, and 0.5-10% of the PEG or PEGylated lipid on a molar basis. In some embodiments, LNPs include 35-65% of a cationic lipid, 3-12% of the neutral lipid, 15-45% of the structural lipid, and 0.5-10% of the PEG-OH lipid on a molar basis. In some embodiments, LNPs include 35-65% of a cationic lipid, 3-12% of the neutral lipid, and 15-45% of the structural lipid on a molar basis. In some embodiments, LNPs include 35-65% of a cationic lipid selected from 2,2-dilinoleyl-4-dimethylaminoethyl-[1,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319). Each possibility represents a separate embodiment of the present invention.

In some embodiments, LNPs include 45-65% of a cationic lipid, 5-10% of the neutral lipid, 25-40% of the structural lipid, and 0.5-10% of the PEG or PEGylated lipid on a molar basis. In some embodiments, LNPs include 45-65% of a cationic lipid, 5-10% of the neutral lipid, 25-40% of the structural lipid, and 0.5-10% of a PEG-OH lipid on a molar basis. In some embodiments, LNPs include 45-65% of a cationic lipid, 5-10% of the neutral lipid, and 25-40% of the structural lipid on a molar basis. In some embodiments, LNPs include 45-65% of a cationic lipid selected from 2,2-dilinoleyl-4-dimethylaminoethyl-[1,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319). Each possibility represents a separate embodiment of the present invention.

In some embodiments, LNPs include 60% of a cationic lipid, 7.5% of the neutral lipid, 31% of a structural lipid, and 1.5% of the PEG or PEGylated lipid on a molar basis. In some embodiments, LNPs include 60% of a cationic lipid, 7.5% of the neutral lipid, 31% of a structural lipid, and 1.5% of a PEG-OH lipid on a molar basis. In some embodiments, LNPs include 60% of a cationic lipid, 9% of the neutral lipid, and 31% of a structural lipid on a molar basis. In some embodiments, LNPs include 60% of a cationic lipid selected from 2,2-dilinoleyl-4-dimethylaminoethyl-[1,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319). Each possibility represents a separate embodiment of the present invention.

In some embodiments, LNPs include 50% of a cationic lipid, 10% of the neutral lipid, 38.5% of the structural lipid, and 1.5% of the PEG or PEGylated lipid on a molar basis. In some embodiments, LNPs include 50% of a cationic lipid, 10% of the neutral lipid, 38.5% of a structural lipid, and 1.5% of a PEG-OH lipid on a molar basis. In some embodiments, LNPs include 50% of a cationic lipid, 10% of the neutral lipid, and 40% of a structural lipid on a molar basis. In some embodiments, LNPs include 50% of a cationic lipid selected from 2,2-dilinoleyl-4-dimethylaminoethyl-[1,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319). Each possibility represents a separate embodiment of the present invention.

In some embodiments, LNPs include 40% of a cationic lipid, 15% of the neutral lipid, 40% of the structural lipid, and 5% of the PEG or PEGylated lipid on a molar basis. In some embodiments, LNPs include 40% of a cationic lipid, 15% of the neutral lipid, 40% of the structural lipid, and 5% of a PEG-OH lipid on a molar basis. In some embodiments, LNPs include 40% of a cationic lipid, 20% of the neutral lipid, 40% of the structural lipid on a molar basis. In some embodiments, LNPs include 40% of a cationic lipid selected from 2,2-dilinoleyl-4-dimethylaminoethyl-[1,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319). Each possibility represents a separate embodiment of the present invention.

In some embodiments, LNPs include 57.2% of a cationic lipid, 7.1% of the neutral lipid 34.3% of the sterol, and 1.4% of the PEG or PEGylated lipid on a molar basis. In some embodiments, LNPs include 57.2% of a cationic lipid, 7.1% of the neutral lipid, 34.3% of the structural lipid, and 1.4% of the PEG-OH lipid on a molar basis. In some embodiments, LNPs include 57.2% of a cationic lipid, 8.5% of the neutral lipid, and 34.3% of the structural lipid on a molar basis. In some embodiments, LNPs include 57.2% of a cationic lipid selected from 2,2-dilinoleyl-4-dimethylaminoethyl-[1,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319). Each possibility represents a separate embodiment of the present invention.

In some embodiments, LNPs consists essentially of a lipid mixture in molar ratios of 20-70% cationic lipid; 5-45% neutral lipid; 20-55% structural lipid; 0.1-15% PEGylated lipid. In some embodiments, LNPs consists essentially of a lipid mixture in molar ratios of 20-70% cationic lipid; 5-45% neutral lipid (e.g., phospholipid or fatty acid); 20-55% structural lipid; and 0.1-15% PEG-OH lipid. In some embodiments, LNPs consists essentially of a lipid mixture in molar ratios of 20-70% cationic lipid; 5-45% neutral lipid (e.g., phospholipid or fatty acid); 20-55% structural lipid (e.g., sterols); and 0.1-15% PEG-OH lipid. In some embodiments, LNPs consists essentially of a lipid mixture in molar ratios of 20-70% cationic lipid; 5-45% neutral lipid (e.g., phospholipid or fatty acid); and 20-55% structural lipid (e.g., sterols). In some embodiments, LNPs consists essentially of a lipid mixture in molar ratios of 20-70% cationic lipid; 5-45% fatty acid (e.g., oleic acid or analog thereof); 20-55% structural lipid (e.g., sterols); and 0.1-15% PEG-OH lipid. In some embodiments, LNPs consists essentially of a lipid mixture in molar ratios of 20-70% cationic lipid; 5-45% fatty acid (e.g., oleic acid or analog thereof); and 20-55% structural lipid (e.g., sterols). In some embodiments, LNPs consists essentially of a lipid mixture in molar ratios of 20-70% cationic lipid; 5-45% oleic acid; 20-55% structural lipid (e.g., sterols); and 0.1-15% PEG-OH lipid. In some embodiments, LNPs consists essentially of a lipid mixture in molar ratios of 20-70% cationic lipid; 5-45% oleic acid; and 20-55% structural lipid (e.g., sterols).

Non-limiting examples of lipid nanoparticle compositions and methods of making them are described, for example, in Semple et al. (2010) Nat. Biotechnol. 28:172-176; Jayarama et al. (2012), Angew. Chem. Int. Ed., 51: 8529-8533; and Maier et al. (2013) Molecular Therapy 21, 1570-1578.

In some embodiments, LNPs may comprise a cationic lipid, a PEG lipid (e.g., PEG-OH lipid) and optionally comprise a neutral lipid (e.g., phospholipid or fatty acid). In some embodiments, LNPs may comprise a cationic lipid, a PEG lipid (e.g., PEG-OH lipid) and a structural lipid (e.g., a sterol) and optionally comprise a neutral lipid (e.g., phospholipid or fatty acid).

Lipid nanoparticles described herein may comprise 2 or more components (e.g., lipids), not including the payload. In certain embodiments, the LNP comprises two components (e.g., lipids), not including the payload. In certain embodiments, the lipid nanoparticle comprises 5 components (e.g., lipids), not including the payload. In certain embodiments, the LNP comprises 6 components (e.g., lipids), not including the payload.

In some embodiments, the LNPs described herein may be four component lipid nanoparticles. A 4 component LNP may comprise four different lipids selected from any described herein. The four components do not include the payload. The lipid nanoparticle may comprise a cationic lipid, a neutral lipid, a PEG lipid, and a structural lipid. In certain embodiments, the lipid nanoparticle comprises a cationic lipid, a fatty acid, a PEG lipid, and a structural lipid. In certain embodiments, the lipid nanoparticle comprises a cationic lipid, a fatty acid, a PEG-OH lipid, and a structural lipid. Each possibility represents a separate embodiment of the present invention.

In some embodiments, the LNPs described herein may be three component lipid nanoparticles. A three component LNP may comprise three different lipids described herein. The lipid nanoparticle may comprise a cationic lipid, a neutral lipid (e.g., phospholipid or fatty acid), and a structural lipid. In certain embodiments, the lipid nanoparticle comprises a cationic lipid, a fatty acid, and a structural lipid. In certain embodiments, the lipid nanoparticle comprises a cationic lipid, a phospholipid, and a structural lipid.

In one embodiment, the LNP formulation may be formulated by the methods described in International Publication Nos. WO2011127255 or WO2008103276. As a non-limiting example, LNP formulations as described in WO2011127255 and/or WO2008103276.

In one embodiment, the lipid nanoparticle may be formulated by the methods described in US Patent Publication No US2013/0156845 or International Publication No WO2013/093648 or WO2012024526.

The lipid nanoparticles described herein may be made in a sterile environment by the system and/or methods described in US Patent Publication No. US20130164400.

In one embodiment, the LNP formulation may be formulated in a nanoparticle such as a nucleic acid-lipid nanoparticle described in U.S. Pat. No. 8,492,359.

As a non-limiting example, the lipid nanoparticle may comprise one or more active agents or therapeutic agents (e.g., RNA); one or more cationic lipids comprising from about 50 mol % to about 85 mol % of the total lipid present in the particle; one or more neutral lipid lipids comprising from about 13 mol % to about 49.5 mol % of the total lipid present in the particle; and one or more structural lipids that inhibit aggregation of particles comprising from about 0.5 mol % to about 2 mol % of the total lipid present in the particle.

In one embodiment, the LNP formulation may be formulated by the methods described in International Publication Nos. WO2011127255 or WO2008103276.

As a non-limiting example, LNP formulations as described in WO2011 127255 and/or WO2008103276. In one embodiment, LNP formulations described herein may comprise a polycationic composition. As a non-limiting example, the polycationic composition may be selected from formula 1-60 of US Patent Publication No. US20050222064.

In some embodiments, LNPs comprise the lipid KL52 (an amino-lipid disclosed in U.S. Application Publication No. 2012/0295832). Activity and/or safety (as measured by examining one or more of ALT/AST, white blood cell count and cytokine induction) of LNP administration may be improved by incorporation of such lipids. LNPs comprising KL52 may be administered intravenously and/or in one or more doses. In some embodiments, administration of LNPs comprising KL52 results in equal or improved mRNA and/or protein expression as compared to LNPs comprising MC3.

As a non-limiting example, the LNP may include a cationic peptide or a polypeptide such as, but not limited to, polylysine, polyornithine and/or polyarginine and the cationic peptides described in International Pub. No. WO2012013326 or US Patent Pub. No. US20130142818. In some embodiments, the lipid nanoparticle includes a neutral lipid such as, but not limited to, cholesterol or dioleoyl phosphatidylethanolamine (DOPE).

A nanoparticle composition may be relatively homogenous. A polydispersity index may be used to indicate the homogeneity of a nanoparticle composition, e.g., the particle size distribution of the nanoparticle compositions. A small (e.g., less than 0.3) polydispersity index generally indicates a narrow particle size distribution. A nanoparticle composition may have a polydispersity index from about 0 to about 0.25, such as 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, or 0.25. In some embodiments, the polydispersity index of a nanoparticle composition may be from about 0.10 to about 0.20, or about 0.05 to about 0.15, or less than about 0.1, or less than about 0.15. Each possibility represents a separate embodiment of the present invention.

The zeta potential of a nanoparticle composition may be used to indicate the electrokinetic potential of the composition. For example, the zeta potential may describe the surface charge of a nanoparticle composition. Nanoparticle compositions with relatively low charges at physiological pH, positive or negative, are generally desirable, as more highly charged species may interact undesirably with cells, tissues, and other elements in the body. In some embodiments, the zeta potential of a nanoparticle composition may be from about -10 mV to about +20 mV, from about -10 mV to about +15 mV, from about -10 mV to about +10 mV, from about -10 mV to about +5 mV, from about -10 mV to about 0 mV, from about -10 mV to about -5 mV, from about -5 mV to about +20 mV, from about -5 mV to about +15 mV, from about -5 mV to about +10 mV, from about -5 mV to about +5 mV, from about -5 mV to about 0 mV, from about 0 mV to about +20 mV, from about 0 mV to about +15 mV, from about 0 mV to about +10 mV, from about 0 mV to about +5 mV, from about +5 mV to about +20 mV, from about +5 mV to about +15 mV, or from about +5 mV to about +10 mV. Each possibility represents a separate embodiment of the present invention.

The efficiency of encapsulation of a therapeutic agent describes the amount of therapeutic agent that is encapsulated or otherwise associated with a nanoparticle composition after preparation, relative to the initial amount provided. The encapsulation efficiency is desirably high (e.g., close to 100%). The encapsulation efficiency may be measured, for example, by comparing the amount of therapeutic agent in a solution containing the nanoparticle composition before and after breaking up the nanoparticle composition with one or more organic solvents or detergents. Fluorescence may be used to measure the amount of free therapeutic agent (e.g., nucleic acids) in a solution. For the nanoparticle compositions described herein, the encapsulation efficiency of a therapeutic agent may be at least 50%, for example 50%, 55%, 60%, 65%, 70%, 75%, 80%, 85%, 90%, 91%, 92%, 93%, 94%, 95%, 96%, 97%, 98%, 99%, or 100%. In some embodiments, the encapsulation efficiency may be at least 80%. In certain embodiments, the encapsulation efficiency may be at least 90%. In certain embodiments, the encapsulation efficiency may be at least 95%. Each possibility represents a separate embodiment of the present invention.

A nanoparticle composition may optionally comprise one or more coatings. For example, a nanoparticle composition may be formulated in a capsule, film, or tablet having a coating. A capsule, film, or tablet including a composition described herein may have any useful size, tensile strength, hardness, or density.

In some embodiments, such LNPs are synthesized using methods comprising microfluidic mixers. Exemplary microfluidic mixers may include, but are not limited to a slit interdigitial micromixer including, but not limited to those manufactured by Microinnova (Allerheiligen bei Wildon, Austria) and/or a staggered herringbone micromixer (SHM) (Zhigaltsev, I.V. et al., Bottom-up design and synthesis of limit size lipid nanoparticle systems with aqueous and triglyceride cores using millisecond microfluidic mixing have been published (Langmuir. 2012. 28:3633-40; Belliveau, N. M. et al., Microfluidic synthesis of highly potent limit-size lipid nanoparticles for in vivo delivery of siRNA. Molecular Therapy-Nucleic Acids. 2012. 1:e37; Chen, D. et al., Rapid discovery of potent siRNA-containing lipid nanoparticles enabled by controlled microfluidic formulation. J Am Chem Soc. 2012. 134(16):6948-51).

In some embodiments, methods of LNP generation comprising SHM, further comprise the mixing of at least two input streams wherein mixing occurs by microstructure-induced chaotic advection (MICA). According to this method, fluid streams flow through channels present in a herringbone pattern causing rotational flow and folding the fluids around each other. This method may also comprise a surface for fluid mixing wherein the surface changes orientations during fluid cycling. Methods of generating LNPs using SHM include those disclosed in U.S. Application Publication Nos. 2004/0262223 and 2012/0276209.

In one embodiment, the lipid nanoparticles may be formulated using a micromixer such as, but not limited to, a Slit Interdigital Microstructured Mixer (SIMM-V2) or a Standard Slit Interdigital Micro Mixer (SSIMM) or Caterpillar (CPMM) or Impinging jet (UMM) from the Institut für Mikrotechnik Mainz GmbH, Mainz Germany).

In one embodiment, the lipid nanoparticles are created using microfluidic technology (see Whitesides, George M. The Origins and the Future of Microfluidics. Nature, 2006 442: 368-373; and Abraham et al. Chaotic Mixer for Microchannels. Science, 2002 295: 647-651). As a non-limiting example, controlled microfluidic formulation includes a passive method for mixing streams of steady pressure-driven flows in micro channels at a low Reynolds number (See e.g., Abraham et al. Chaotic Mixer for Microchannels. Science, 2002 295: 647651).

In one embodiment, a therapeutic nucleic acid (e.g., mRNA) may be formulated in lipid nanoparticles created using a micromixer chip such as, but not limited to, those from Harvard Apparatus (Holliston, Mass.) or Dolomite Microfluidics (Royston, UK). A micromixer chip can be used for rapid mixing of two or more fluid streams with a split and recombine mechanism.

### Cationic Lipids

Cationic lipids useful in embodiments of the present invention are neutral while in circulation but become positively charged upon acidification of the endosome. A positive charge on the LNP may promote association with the negatively charged cell membrane to enhance cellular uptake. Cationic lipids may also combine with negatively charged lipids to induce nonbilayer structures that facilitate intracellular delivery. Suitable cationic lipids for use in making the LNPs disclosed herein can be ionizable cationic lipids, as disclosed herein. The cationic lipids may be prepared according to the procedures set forth in the Examples or according to methods known or derivable by one of ordinary skill in the art.

In some embodiments, LNPs may comprise, in molar percentages, 35 to 45% cationic lipid, 40% to 50% cationic lipid, 45% to 55% cationic lipid, 50% to 60% cationic lipid and/or 55% to 65% cationic lipid. In some embodiments, the ratio of lipid to nucleic acid (e.g., mRNA) in lipid nanoparticles may be 5:1 to 20:1, 10:1 to 25:1, 15:1 to 40:1, 20:1 to 30:1, 25:1 to 50:1, 30:1 to 60:1 and/or at least 40:1.

Such lipids include, but are not limited to, N,N-dioleyl-N,N-dimethylammonium chloride (DODAC); N-(2,3-dioleyloxy)propyl)-N,N,N-trimethylammonium chloride (DOTMA); N,N-distearyl-N,N-dimethylammonium bromide (DDAB); N-(2,3dioleoyloxy)propyl)-N,N,N-trimethylammonium chloride (DOTAP); 3-(N---(N',N'dimethylaminoethane)-carbamoyl)cholesterol (DC-Chol), N-(1-(2,3-dioleoyloxy)propyl)N-2-(sperminecarboxamido)ethyl)-N,N-dimethylammonium trifluoracetate (DOSPA), dioctadecylamidoglycyl carboxyspermine (DOGS), 1,2-dioleoyl-3-dimethylammonium propane (DODAP), N,N-dimethyl-2,3-dioleoyloxy)propylamine (DODMA), and N-(1,2-dimyristyloxyprop-3-yl)-N,N-dimethyl-N-hydroxyethyl ammonium bromide (DMRIE).

Additionally, a number of commercial preparations of cationic lipids are available which can be used in any of the described embodiments. These include, for example, LIPOFECTIN^{®} (commercially available cationic liposomes comprising DOTMA and 1,2-dioleoyl-sn-3phosphoethanolamine (DOPE), from GIBCO/BRL, Grand Island, N.Y.); LIPOFECTAMINE^{®} (commercially available cationic liposomes comprising N-(1-(2,3dioleyloxy)propyl)-N-(2-(sperminecarboxamido)ethyl)-N,N-dimethylammonium trifluoroacetate (DOSPA) and (DOPE), from GIBCOBRL); and TRANSFECTAM^{®} (commercially available cationic lipids comprising dioctadecylamidoglycyl carboxyspermine (DOGS) in ethanol from Promega Corp., Madison, Wis.). The following lipids are cationic and have a positive charge at below physiological pH: DODAP, DODMA, DMDMA, 1,2-dilinoleyloxy-N,N-dimethylaminopropane (DLinDMA), 1,2-dilinolenyloxy-N,N-dimethylaminopropane (DLenDMA).

In one specific embodiment, the cationic lipid for use in any of the described embodiments is independently an amino lipid. Suitable amino lipids include those described in WO 2010/054401 and WO 2012/016184. Representative amino lipids include, but are not limited to, 1,2-dilinoleyoxy-3-(dimethylamino)acetoxypropane (DLin-DAC), 1,2-dilinoleyoxy-3morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazino)propane (DLin-MPZ), 3-(N,Ndilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), and 2,2-dilinoleyl-4-dimethylaminomethyl-[1,3]-dioxolane (DLin-K-DMA).In some of the described embodiments, the cationic lipid has the following formula: 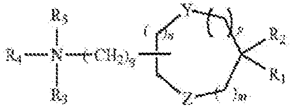 wherein R₁ and R₂ are either the same or different and independently optionally substituted C₁₀-C₂₄ alkyl, optionally substituted C₁₀-C₂₄ alkenyl, optionally substituted C₁₀-C₂₄ alkynyl, or optionally substituted C₁₀-C₂₄ acyl;
R₃ and R₄ are either the same or different and independently optionally substituted C₁-C₆ alkyl, optionally substituted C₂-C₆ alkenyl, or optionally substituted C₂-C₆ alkynyl or R₃ and R₄ may join to form an optionally substituted heterocyclic ring of 4 to 6 carbon atoms and 1 or 2 heteroatoms chosen from nitrogen and oxygen;
R₅ is either absent or present and when present is hydrogen or C₁-C₆ alkyl; m, n, and p are either the same or different and independently either 0 or 1 with the proviso that m, n, and p are not simultaneously 0; q is 0, 1, 2, 3, or 4; and
Y and Z are either the same or different and independently O, S, or NH. In one embodiment, R₁ and R₂ are each linoleyl, and the amino lipid is a dilinoleyl amino lipid. In one embodiment, the amino lipid is a dilinoleyl amino lipid. In various other embodiments, the cationic lipid has the following structure: 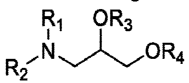 or a pharmaceutically acceptable salt, tautomer, prodrug or stereoisomer thereof,
   wherein:
   R₁ and R₂ are independently selected from the group consisting of H, and C₁-C₃ alkyls;
   R₃ and R₄ are independently selected from the group consisting of alkyl groups having from about 10 to about 20 carbon atoms, wherein at least one of R₃ and R₄ comprises at least two sites of unsaturation. (e.g., R₃ and R₄ may be, for example, dodecadienyl, tetradecadienyl, hexadecadienyl, linoleyl, and icosadienyl. In a preferred embodiment, R₃ and R₄ are both linoleyl. R₃ and R₄ may comprise at least three sites of unsaturation (e.g., R₃ and R₄ may be, for example, dodecatrienyl, tetradectrienyl, hexadecatrienyl, linolenyl, and icosatrienyl).

In some embodiments, the cationic lipid has the following structure: 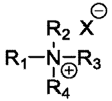 or a pharmaceutically acceptable salt, tautomer, prodrug or stereoisomer thereof,
wherein:
R₁ and R₂ are independently selected and are H or C₁-C₃ alkyls. R₃ and R₄ are independently selected and are alkyl groups having from about 10 to about 20 carbon atoms, wherein at least one of R₄ and R₄ comprises at least two sites of unsaturation. In one embodiment, R₃ and R₄ are both the same, for example, in some embodiments R₃ and R₄ are both linoleyl (i.e., C18), etc. In another embodiment, R₃ and R₄ are different, for example, in some embodiments R₃ is tetradectrienyl (C14) and R₄ is linoleyl (C18). In a preferred embodiment, the cationic lipid(s) of the present invention are symmetrical, i.e., R₃ and R₄ are the same. In another preferred embodiment, both R₃ and R₄ comprise at least two sites of unsaturation. In some embodiments, R₃ and R₄ are independently selected from dodecadienyl, tetradecadienyl, hexadecadienyl, linoleyl, and icosadienyl. In a preferred embodiment, R₃ and R₄ are both linoleyl. In some embodiments, R₄ and R₄ comprise at least three sites of unsaturation and are independently selected from, e.g., dodecatrienyl, tetradectrienyl, hexadecatrienyl, linolenyl, and icosatrienyl.

In various embodiments, the cationic lipid has the formula: 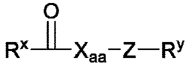 or a pharmaceutically acceptable salt, tautomer, prodrug or stereoisomer thereof, wherein:
Xₐₐ is a D- or L-amino acid residue having the formula -NR^{N}-CR¹R²-C(C=O)-, or a peptide or a peptide of amino acid residues having the formula ---(NR^{N}-CR¹R²---(C=O)}ₙ---, wherein n is 2 to 20;
R¹ is independently, for each occurrence, a non-hydrogen, substituted or unsubstituted side chain of an amino acid;
R² and R^{N} are independently, for each occurrence, hydrogen, an organic group consisting of carbon, oxygen, nitrogen, sulfur, and hydrogen atoms, or any combination of the foregoing, and having from 1 to 20 carbon atoms, C₍₁₋₅₎alkyl, cycloalkyl, cycloalkylalkyl, C₍₃₋₅₎alkenyl, C₍₃₋₅₎alkynyl, C₍₁₋₅₎alkanoyl, C₍₁₋₅₎alkanoyloxy, C₍₁₋₅₎alkoxy, C₍₁₋₅₎alkoxy-C₍₁₋₅₎alkyl, C₍₁₋₅₎alkoxy-C₍₁₋₅₎alkoxy, C₍₁₋₅₎alkyl-amino-C₍₁₋₅₎alkyl-, C₍₁₋₅₎dialkyl-amino-C₍₁₋₅₎alkyl-, nitro-C₍₁₋₅₎alkyl, cyano-C₍₁₋₅₎alkyl, aryl-C₍₁₋₅₎alkyl, 4-biphenyl-C₍₁₋₅₎alkyl, carboxyl, or hydroxyl;
Z is NH, O, S, -CH₂S-, -CH₂S(O)-, or an organic linker consisting of 1-40 atoms selected from hydrogen, carbon, oxygen, nitrogen, and sulfur atoms (preferably, 2 is NH or O);
R^{x} and R^{y} are, independently, (i) a lipophilic tail derived from a lipid (which can be naturally-occurring or synthetic), phospholipid, glycolipid, triacylglycerol, glycerophospholipid, sphingolipid, ceramide, sphingomyelin, cerebroside, or ganglioside, wherein the tail optionally includes a steroid; (ii) an amino acid terminal group selected from hydrogen, hydroxyl, amino, and an organic protecting group; or (iii) a substituted or unsubstituted C₍₃₋₂₂₎alkyl, C₍₆₋₁₂₎cycloalkyl, C₍₆₋₁₂₎cycloalkyl-C₍₃₋₂₂₎alkyl, C₍₃₋₂₂₎alkenyl, C₍₃₋₂₂₎alkynyl, C₍₃₋₂₂₎alkoxy, or C₍₆₋₁₂₎-alkoxy-C₍₃₋₂₂₎alkyl;
one of R^{x} and R^{y} is a lipophilic tail as defined above and the other is an amino acid terminal group, or both R^{x} and R^{y} are lipophilic tails;
at least one of R^{x} and R^{y} is interrupted by one or more biodegradable groups (e.g., -OC(O)-, -C(O)O-, -SC(O)-, -C(O)S-, -OC(S)-, -C(S)O-, -S-S-, -C(R⁵)=N-, -N=C(R⁵)--, -C(R³)=N-O-, -O-N=C(R⁵)-, -C(O)(NR⁵)-, -N(R⁵)C(O)-, -C(S)(NR⁵)-, - N(R⁵)C(O)-, -N(R⁵)C(O)N(R⁵)-, -OC(O)O-, -OSi(R⁵)₂O-, -C(O)(CR³R⁴)C(O)O-, - OC(O)(CR³R⁴)C(O)- or 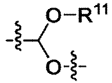
wherein R¹¹ is a C₂-C₈ alkyl or alkenyl and each occurrence of R⁵ is, independently, H or alkyl; and each occurrence of R³ and R⁴ are, independently H, halogen, OH, alkyl, alkoxy, --NH₂, alkylamino, or dialkylamino; or R³ and R⁴, together with the carbon atom to which they are directly attached, form a cycloalkyl group (in one preferred embodiment, each occurrence of R³ and R⁴ are, independently H or C₁-C₄ alkyl)); and R^{x} and R^{y} each, independently, optionally have one or more carbon-carbon double bonds.

In some embodiments, the cationic lipid is one of the following: 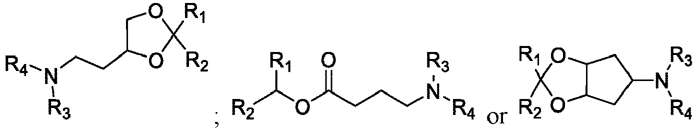 or a pharmaceutically acceptable salt, tautomer, prodrug or stereoisomer thereof,
wherein:
R₁ and R₂ are independently alkyl, alkenyl or alkynyl, and each can be optionally substituted;
R₃ and R₄ are independently a C₁-C₆ alkyl, or R₃ and R₄ can be taken together to form an optionally substituted heterocyclic ring.

A representative useful dilinoleyl amino lipid has the formula: 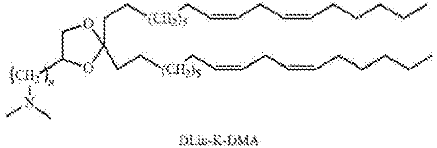 wherein n is 0, 1, 2, 3, or 4.

In one embodiment, the cationic lipid is DLin-K-DMA. In one embodiment, a cationic lipid is DLin-KC2-DMA (DLin-K-DMA above, wherein n is 2).

In one embodiment, the cationic lipid has the following structure: 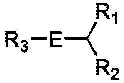 or a pharmaceutically acceptable salt, tautomer, prodrug or stereoisomer thereof, wherein:
R₁ and R₂ are each independently for each occurrence optionally substituted C₁₀-C₃₀ alkyl, optionally substituted C₁₀-C₃₀ alkenyl, optionally substituted C₁₀-C₃₀ alkynyl or optionally substituted C₁₀-C₃₀ acyl, or linker-ligand;
R₃ is H, optionally substituted C₁-C₁₀ alkyl, optionally substituted C₂-C₁₀ alkenyl, optionally substituted C₂-C₁₀ alkynyl, alkylhetrocycle, alkylphosphate, alkylphosphorothioate, alkylphosphorodithioate, alkylphosphonate, alkylamine, hydroxyalkyl, ω-aminoalkyl, ω-(substituted)aminoalkyl, ω-phosphoalkyl, ω-thiophosphoalkyl, optionally substituted polyethylene glycol (PEG, mw 100-40K), optionally substituted mPEG (mw 120-40K), heteroaryl, or heterocycle, or linker-ligand, for example in some embodiments R₃ is (CH₃)₂N(CH₂)ₙ-, wherein n is 1, 2, 3 or 4;
E is O, S, N(Q), C(O), OC(O), C(O)O, N(Q)C(O), C(O)N(Q), (Q)N(CO)O, O(CO)N(Q), S(O), NS(O)₂N(Q), S(O)₂, N(Q)S(O)₂, SS, O=N, aryl, heteroaryl, cyclic or heterocycle, for example -C(O)O, wherein - is a point of connection to R₃; and
Q is H, alkyl, ω-aminoalkyl, ω-(substituted)aminoalkyl, ω-phosphoalkyl or ω-thiophosphoalkyl.

In one specific embodiment, the cationic has the following structure: 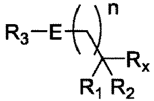 or a pharmaceutically acceptable salt, tautomer, prodrug or stereoisomer thereof,
wherein:
E is O, S, N(Q), C(O), N(Q)C(O), C(O)N(Q), (Q)N(CO)O, O(CO)N(Q), S(O), NS(O)₂N(Q), S(O)₂, N(Q)S(O)₂, SS, O=N, aryl, heteroaryl, cyclic or heterocycle;
Q is H, alkyl, ω-amninoalkyl, ω-(substituted)amninoalky, ω-phosphoalkyl or ω-miophosphoalkyl;
R₁ and R₂ and Rₓ are each independently for each occurrence H, optionally substituted C₁-C₁₀ alkyl, optionally substituted C₁₀-C₃₀ alkyl, optionally substituted C₁₀-C₃₀ alkenyl, optionally substituted C₁₀-C₃₀ alkynyl, optionally substituted C₁₀-C₃₀ acyl, or linker-ligand, provided that at least one of R₁, R₂ and Rₓ is not H;
R₃ is H, optionally substituted C₁-C₁₀ alkyl, optionally substituted C₂-C₁₀ alkenyl, optionally substituted C₂-C₁₀ alkynyl, alkylhetrocycle, alkylphosphate, alkylphosphorothioate, alkylphosphorodithioate, alkylphosphonate, alkylamine, hydroxyalkyl, ω-aminoalkyl, ω-(substituted)aminoalkyl, ω-phosphoalkyl, ω-thiophosphoalkyl, optionally substituted polyethylene glycol (PEG, mw 100-40K), optionally substituted mPEG (mw 120-40K), heteroaryl, or heterocycle, or linker-ligand; and
n is 0, 1, 2, or 3.

In another embodiment, the cationic lipid has one of the following structures:

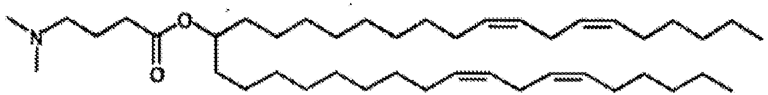

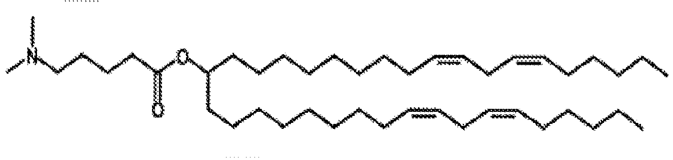

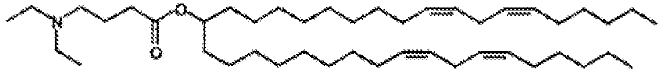

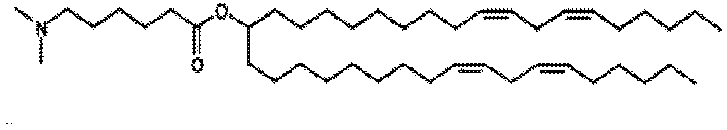

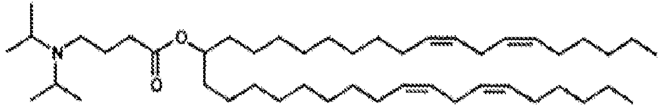

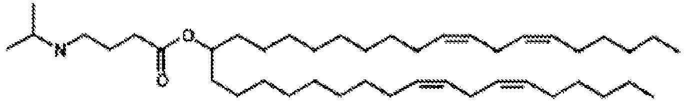

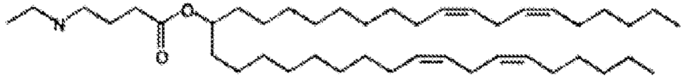

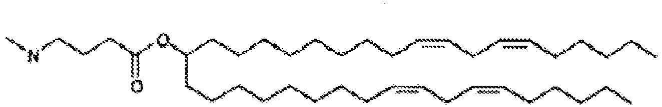

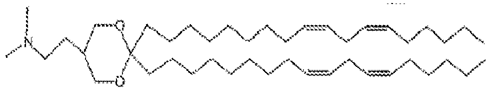

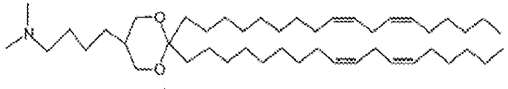

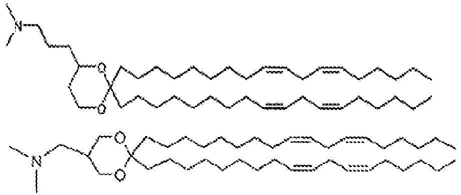

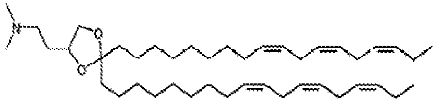

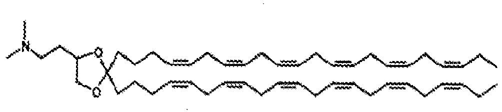

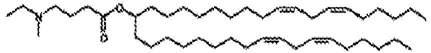

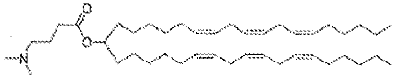

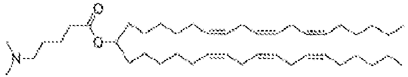

In some embodiments, the cationic lipid is DLin-M-C3-DMA, MC3 or M-C3 and has been described in WO 2010/054401, and WO 2010/144740 A1.

In different embodiments, the cationic lipid has one of the following structures:

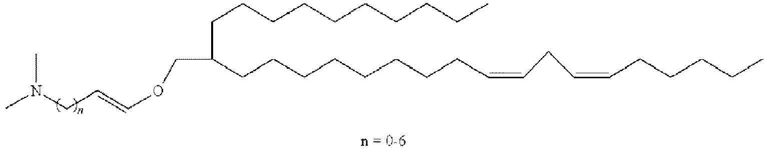

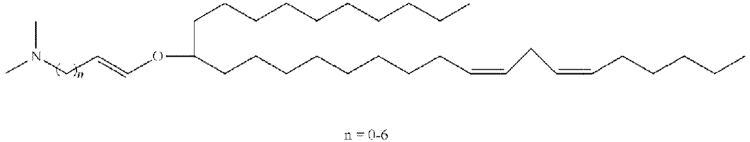

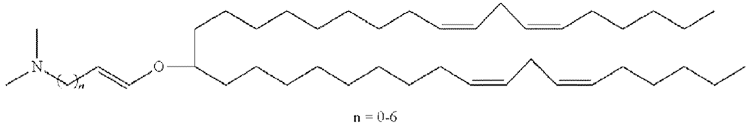

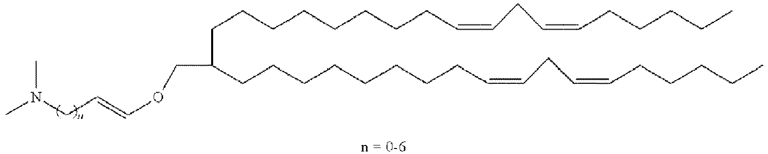

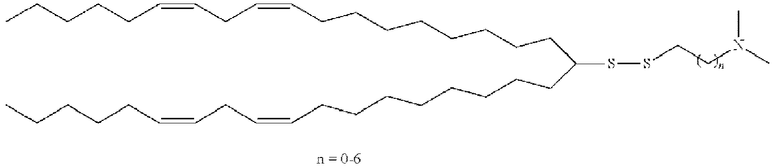

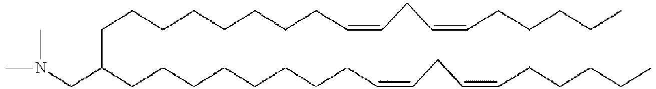

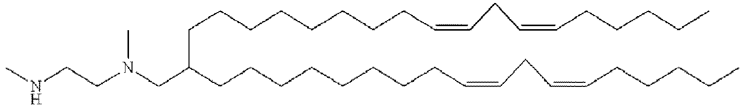

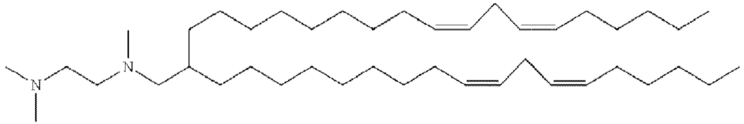

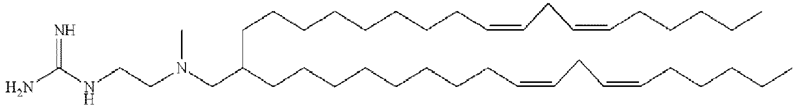

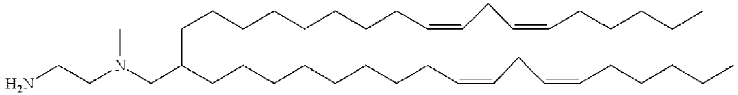

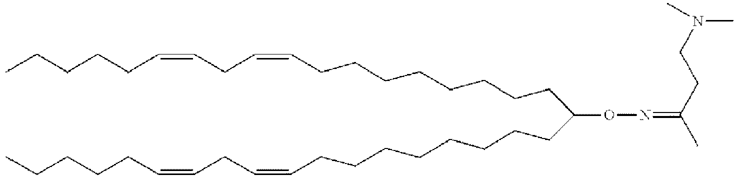

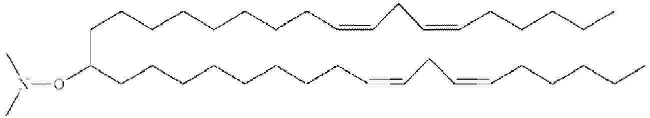

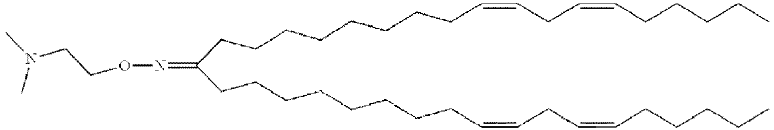

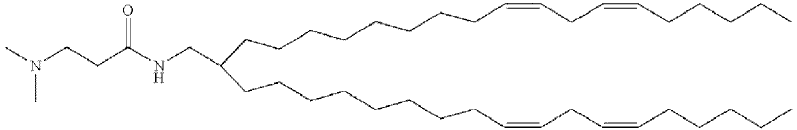

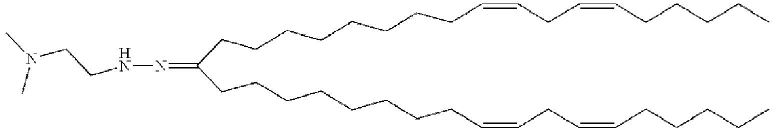

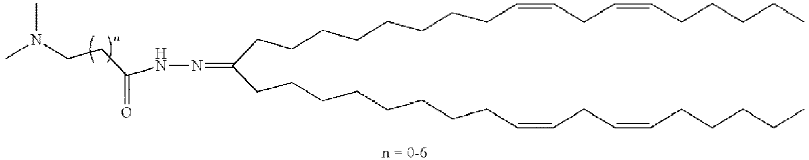

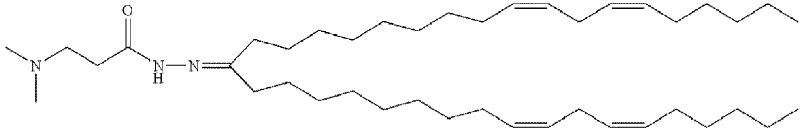

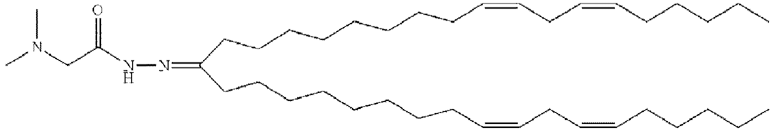

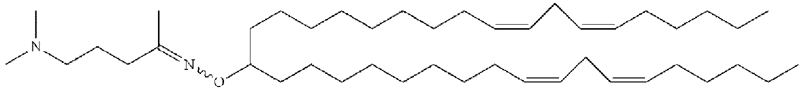

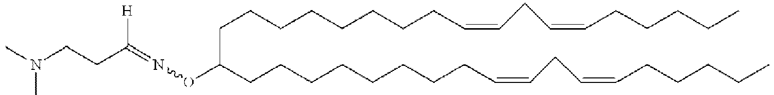

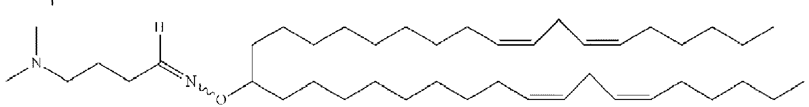

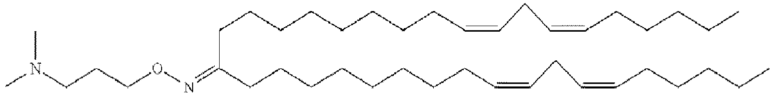

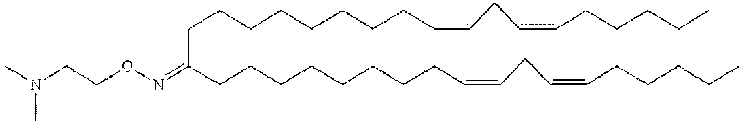

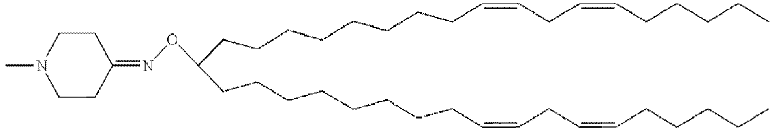

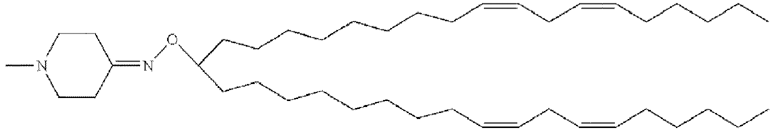

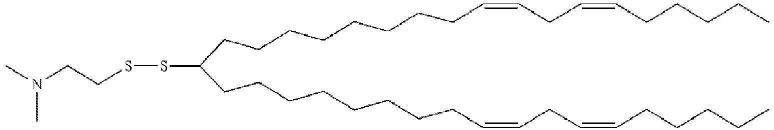

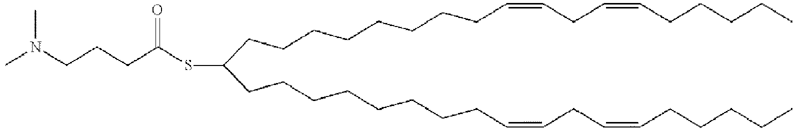

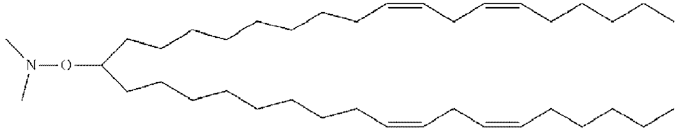

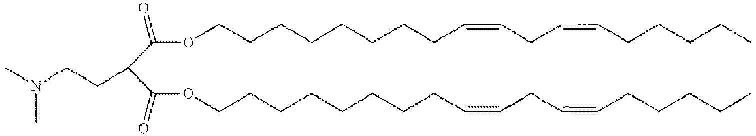

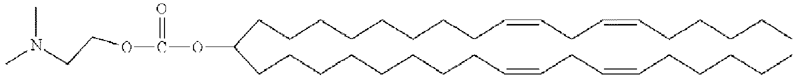

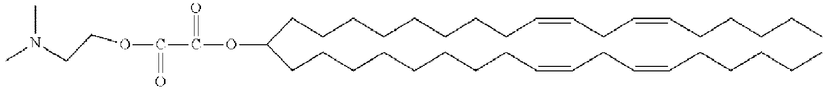

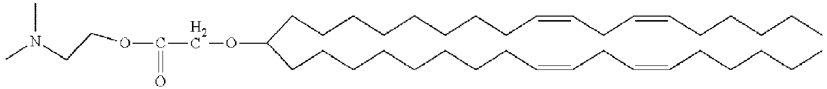

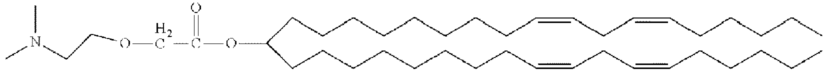

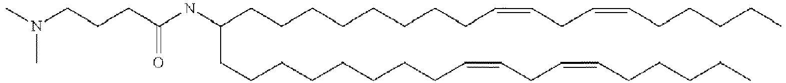

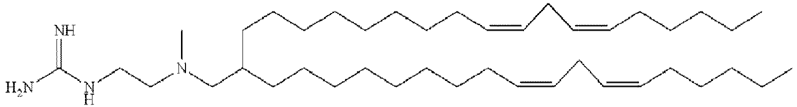

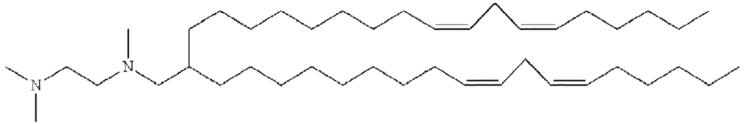

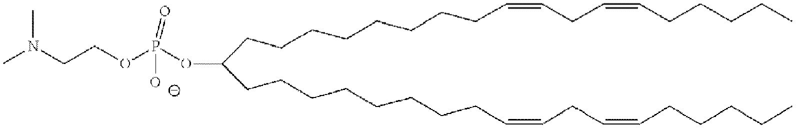

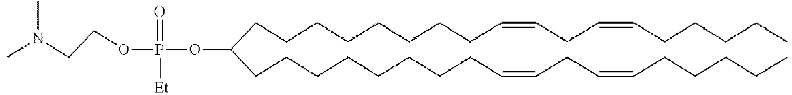

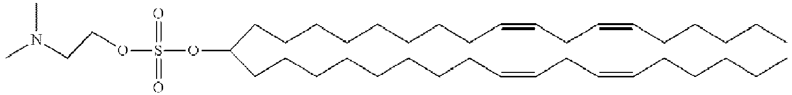

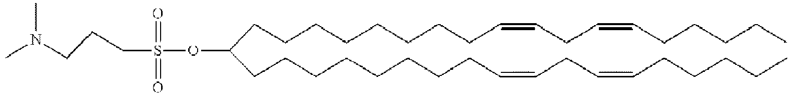

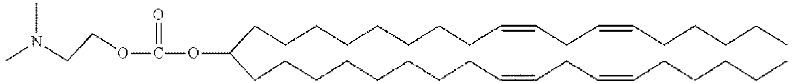

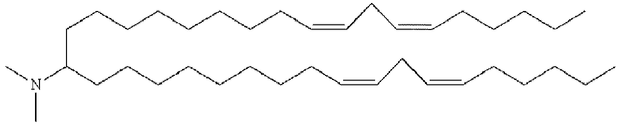

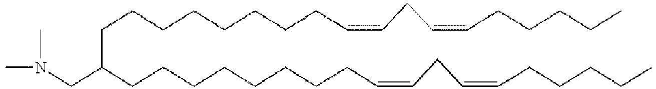

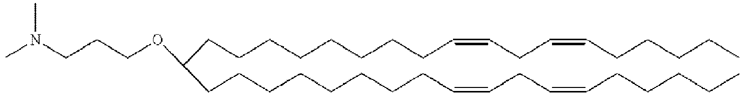

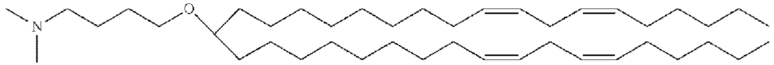

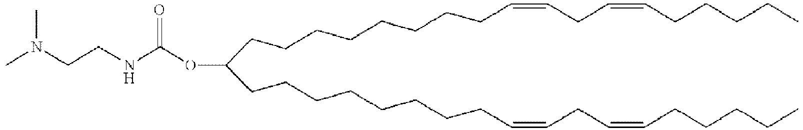

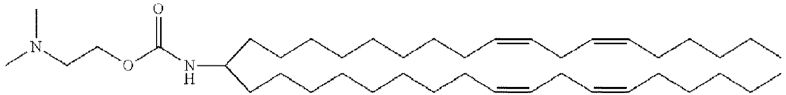

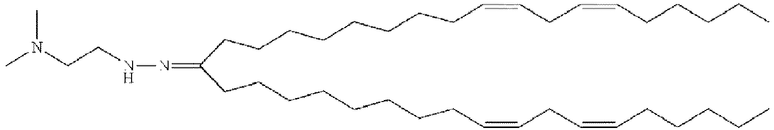

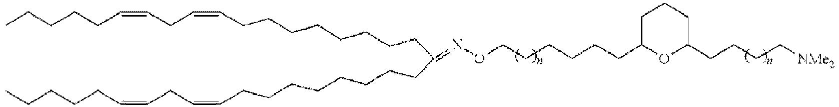

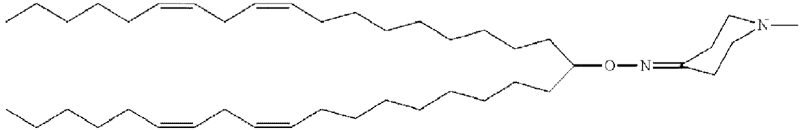

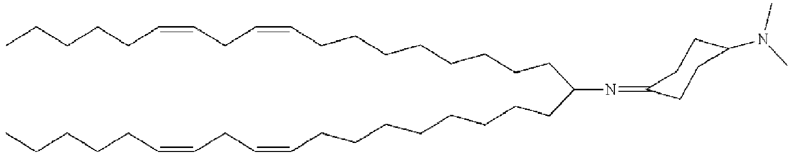

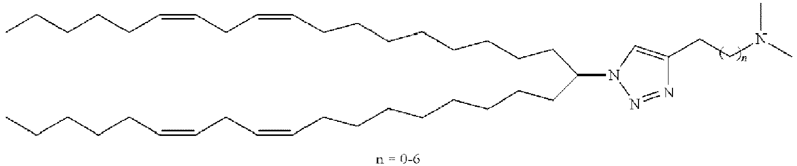

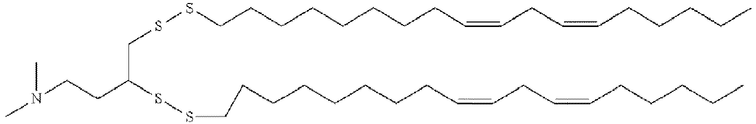

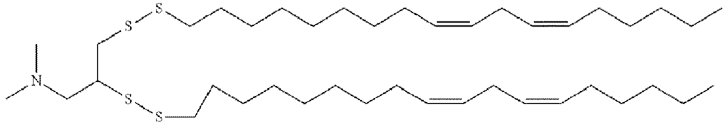

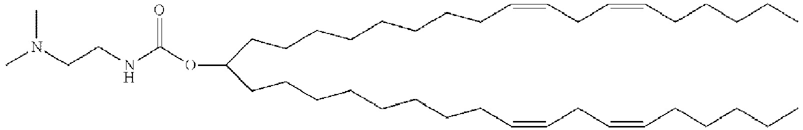

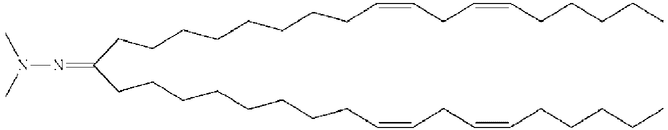

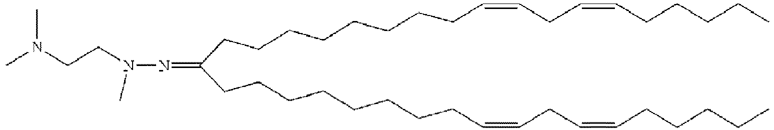

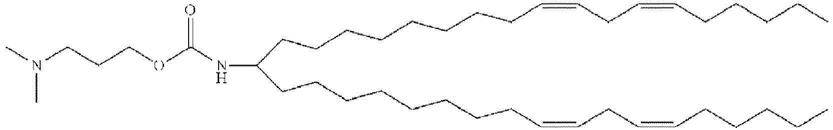

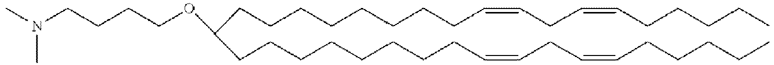

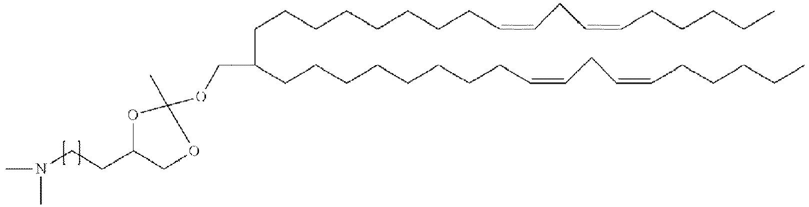

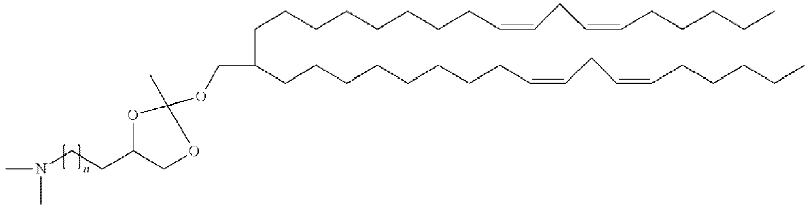

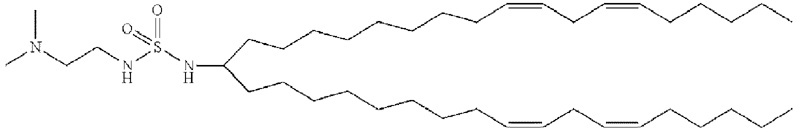

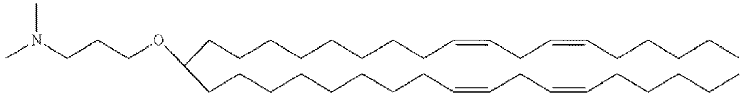

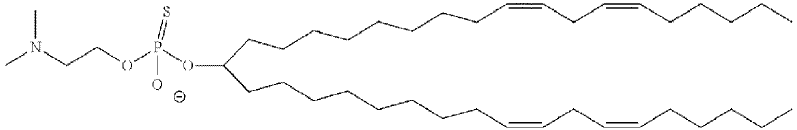

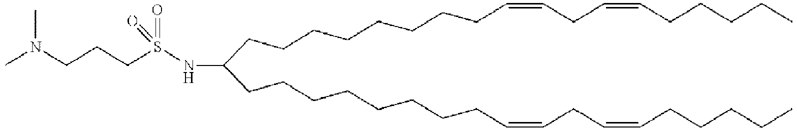

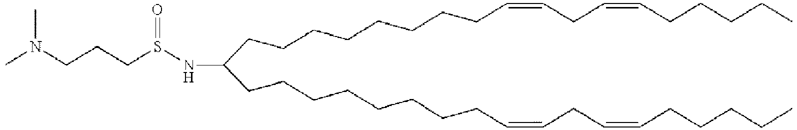

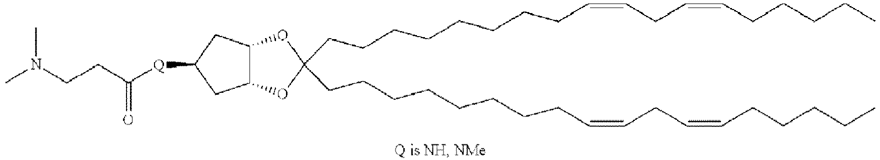

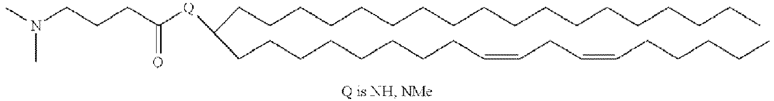

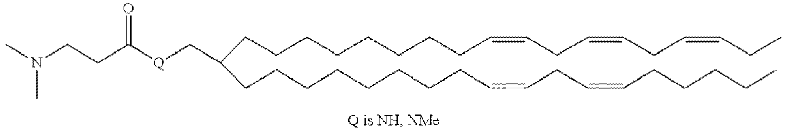

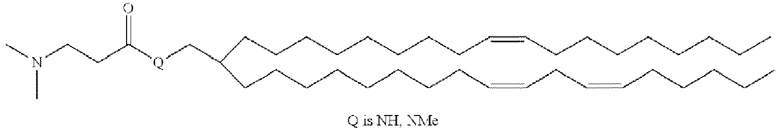

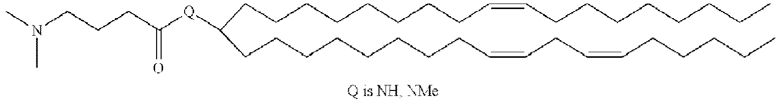

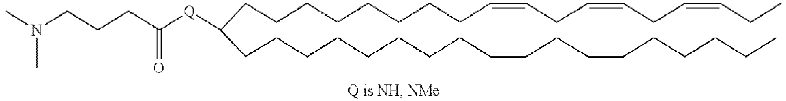

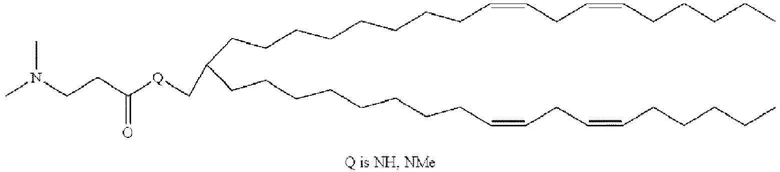

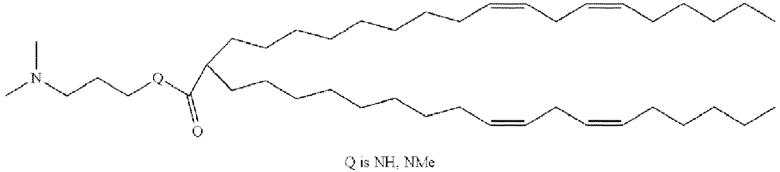

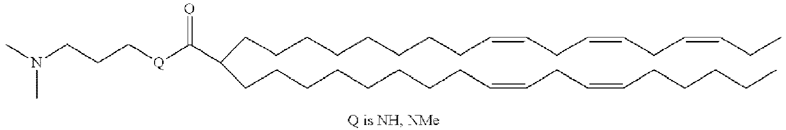

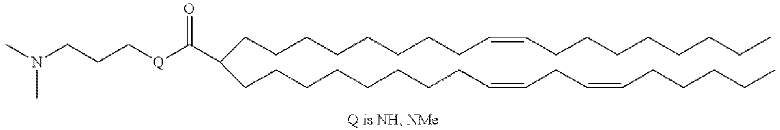

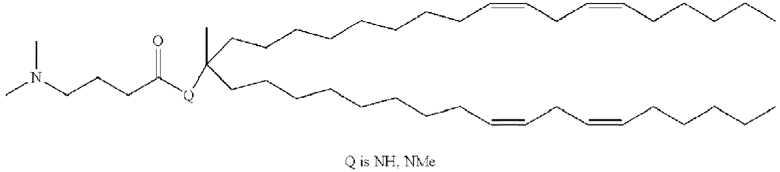

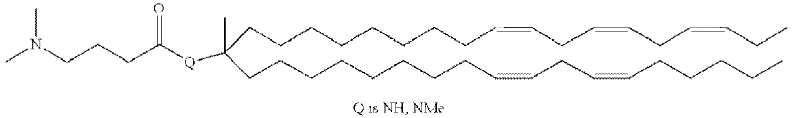

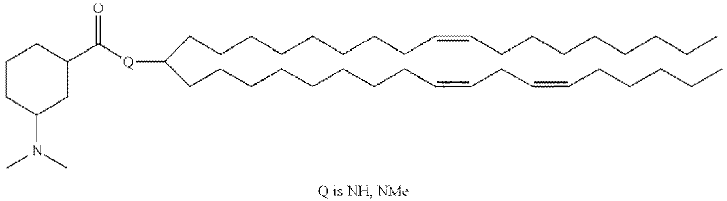

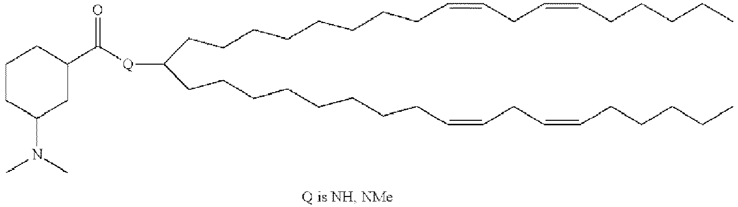

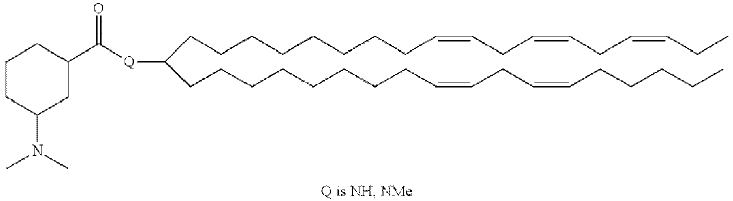

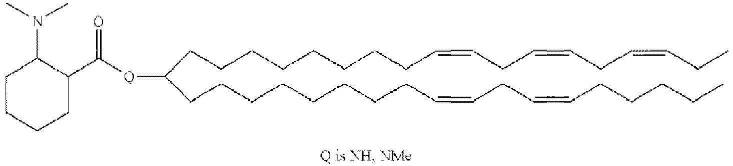

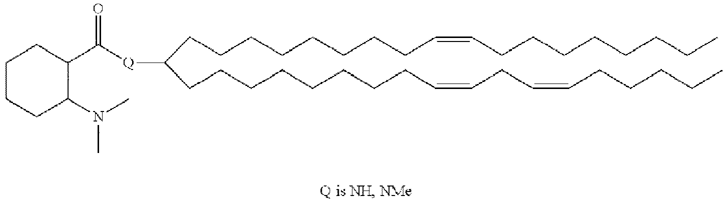

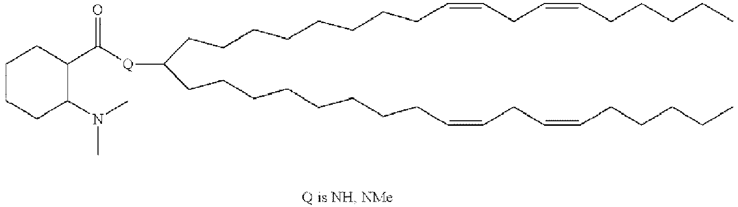

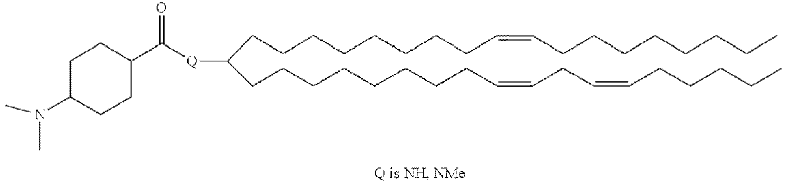

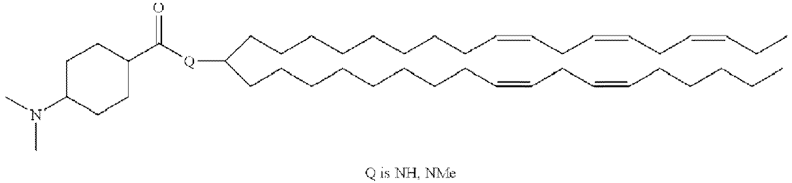

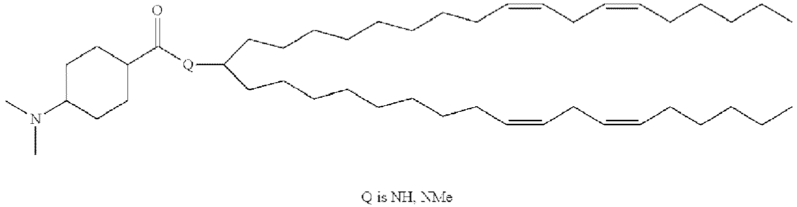

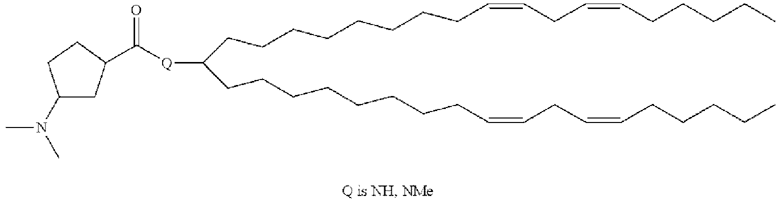

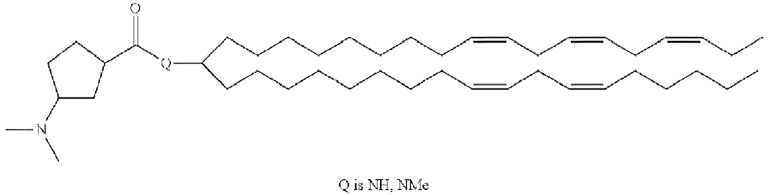

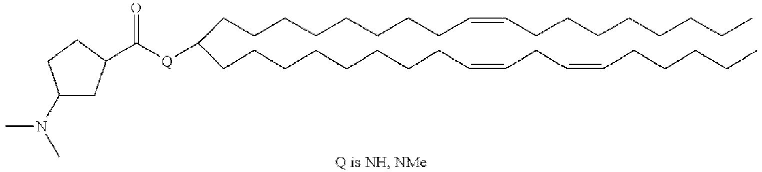

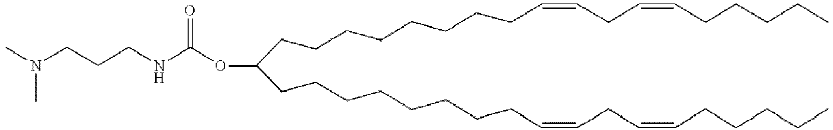

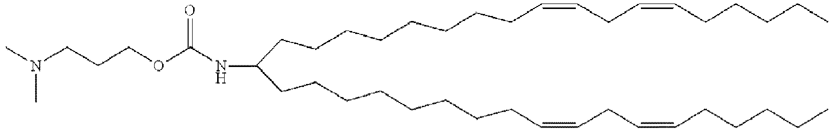

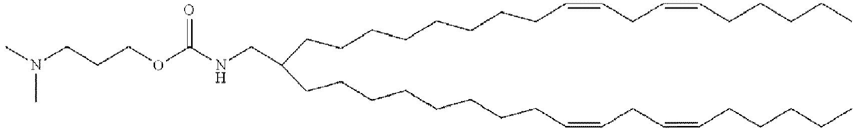

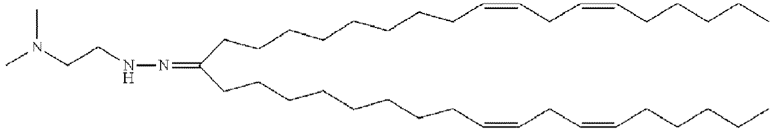

In another embodiment, the cationic lipid has the following structure: 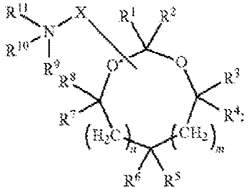 or a pharmaceutically acceptable salt, tautomer, prodrug or stereoisomer thereof, wherein:
R¹, R², R³, R⁴, R⁵, R⁶, R⁷ and R⁸ are independently selected from the group consisting of hydrogen, optionally substituted C₇-C₃₀ alkyl, optionally substituted C₇-C₃₀ alkenyl and optionally substituted C₇-C₃₀ alkynyl:
provided that (a) at least two of R¹, R², R³, R⁴, R⁵, R⁶, R⁷ and R⁸ are not hydrogen, and (b) two of the at least two of R¹, R², R³, R⁴, R⁵, R⁶, R⁷ and R⁸ that are not hydrogen are present in a 1,3 arrangement, a 1,4 arrangement or a 1,5 arrangement with respect to each other;
X is selected from the group consisting of Ci-Cs alkyl, C₂-C₆ alkenyl and C₂-C₆ alkynyl;
R⁹, R¹⁰, and R¹¹ are independently selected from the group consisting of hydrogen, optionally substituted C₁-C₇, alkyl, optionally substituted C₂-C₇, alkenyl and option ally substituted C₂-C₇, alkynyl, provided that one of R⁹, R¹⁰, and R¹¹ may be absent; and n and m are each independently 0 or 1.

In a specific embodiment, the cationic lipid has the structure: 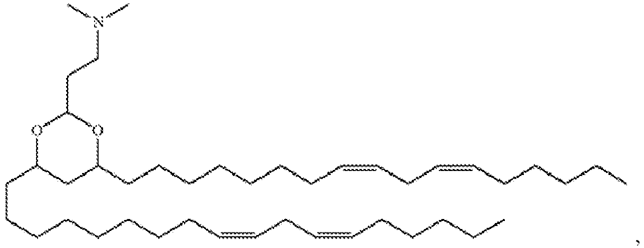 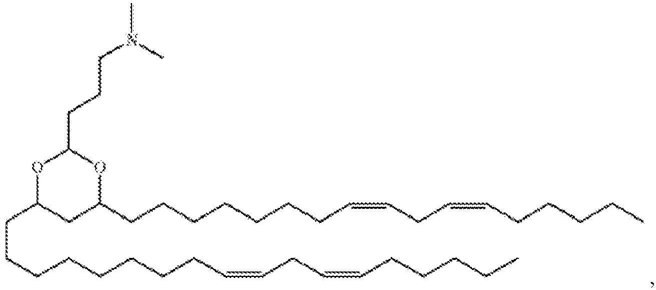 or

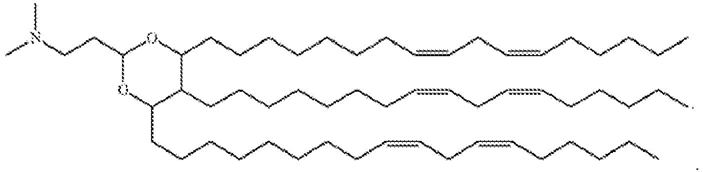

In one embodiment, the cationic lipid is a cyclic lipid having the following structure: 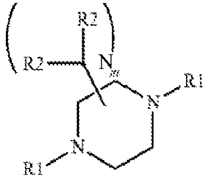 or a pharmaceutically acceptable salt, tautomer, prodrug or stereoisomer thereof,
wherein:
R1 is independently selected from -(CH₂)₂-N(R)₂, -(CH₂)₂-N(R)-(CH₂)₂-N(R)₂, wherein
R is independently selected from -H, C₆₋₄₀ alkyl, C₆₋₄₀ alkenyl and C₆₋₄₀ alkynyl, provided that -N(R)₂ is not NH₂;
R2 is C₆₋₄₀ alkyl, C₆₋₄₀ alkenyl or C₆₋₄₀ alkynyl; and
m is 0 or 1.

In a more specific embodiment, the cationic lipid has a structure selected from:

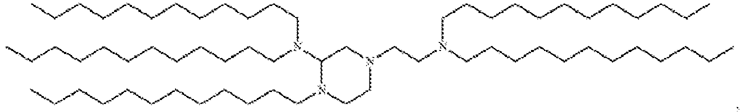

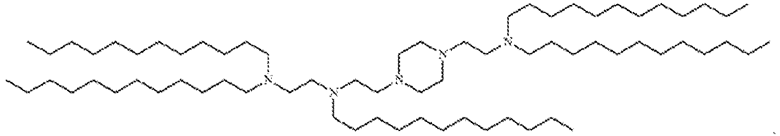

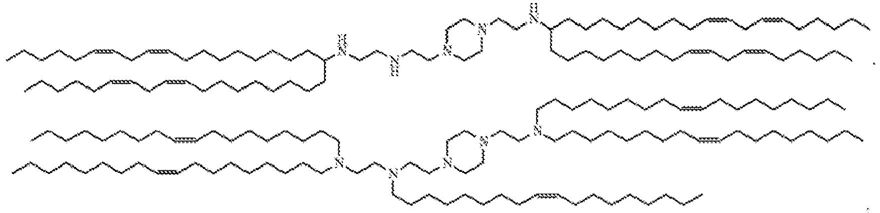

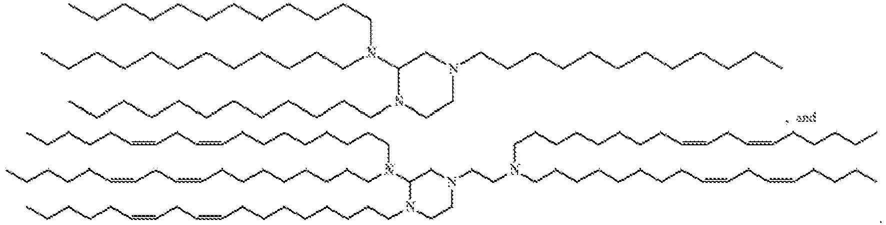

In another embodiment, the cationic lipid has the structure: 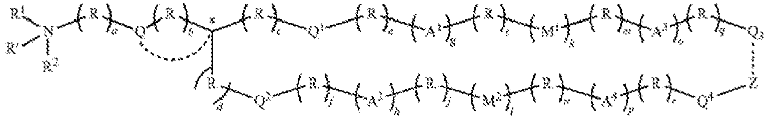 or a salt thereof; wherein
R' is absent, hydrogen, or alkyl;
with respect to R¹ and R²,
   (i) R¹ and R² are each, independently, optionally substituted alkyl, alkenyl, alkynyl, cycloalkyl, cycloalkylalkyl, or heterocycle;
   (ii) R¹ and R², together with the nitrogen atom to which they are attached, form an optionally substituted heterocyclic ring; or
   (iii) one of R¹ and R² is optionally substituted alkyl, alkenyl, alkynyl, cycloalkyl, cycloalkylalkyl, or heterocycle, and the other forms a 4-10 member heterocyclic ring or heteroaryl with (a) the adjacent nitrogen atom and (b) the (R)ₐ group adjacent to the nitrogen atom;
each occurrence of Ris, independently, -(CR³R⁴)-;
each occurrence of R³ and R⁴ are, independently H, OH, alkyl, alkoxy, - NH₂, alkylamino, or dialkylamino;
or R³ and R⁴, together with the carbon atom to which they are directly attached, form a cycloalkyl group, wherein no more than three R groups in each chain attached to the carbon C* are cycloalkyl;
the dashed line to Q is absent or a bond;
when the dashed line to Q is absent then Q is absent or is -O-, -NH-, -S-, - C(O)O-, -OC(O)-, -C(O)N(R⁴)-, -N(R⁵)C(O)-, -S-S-, -OC(O)O-, -O-N=C(R⁵)-, - C(R⁵)=N-O-, -OC(O)N(R⁵)-, -N(R⁵)C(O)N(R⁵)-, -N(R⁵)C(O)O-, -C(O)S-, -C(S)O- or - C(R⁵)=N-O-C(O)-; or
when the dashed line to Q is a bond then (i) b is 0 and (ii) Q and the tertiary carbon adjacent to it (C*) form a substituted or unsubstituted, mono- or bi-cyclic heterocyclic group having from 5 to 10 ring atoms;
Q¹ and Q² are each, independently, absent, -O-, -S-, -OC(O)-, -C(O)O-, - SC(O)-, -C(O)S-, -OC(S)-, -C(S)O-, -S-S-, -C(O)(NR⁵)-, -N(R⁵)C(O)-, -C(S)(NR⁵)-, - N(R⁵)C(O)-, -N(R⁵)C(O)N(R⁵)-, or -OC(O)O-;
Q³ and Q⁴ are each, independently, H, -(CR³R⁴)-, aryl, or a cholesterol moiety;
each occurrence of A¹, A², A³ and A⁴ is, independently, -(CR⁵R⁵-CR⁵=CR⁵)-;
each occurrence of R⁵ is, independently, H or alkyl;
M¹ and M² are each, independently, a biodegradable group; wherein
the biodegradable group is selected from -OC(O)-, -C(O)O-, -SC(O)-, - C(O)S-, -OC(S)-, -C(S)O-, -S-S-, -C(R⁵)=N-, -N=C(R⁵)-, -C(R⁵)=N-O-, -O-N=C(R⁵)-, - C(O)(NR⁵)-, -N(R⁵)C(O)-, -C(S)(NR⁵)-, -N(R⁵)C(O)-, -N(R⁵)C(O)N(R⁵)-, -OC(O)O-, - OSi(R⁵)₂O-, -C(O)(CR³R⁴)C(O)O-, and -OC(O)(CR³R⁴)C(O)-;
Z is absent, alkylene or -O-P(O)(OH)-O-;
each ------ attached to Z is an optional bond, such that when Z is absent, Q³ and Q⁴ are not directly covalently bound together;
a is 1, 2, 3, 4, 5 or 6;
b is 0, 1, 2, or 3;
c, d, e, f, i, j, m, n, q and r are each, independently, 0, 1, 2, 3, 4, 5, 6, 7, 8, 9, or 10;
g and h are each, independently, 0, 1 or 2;
k and 1 are each, independently, 0 or 1, where at least one of k and 1 is 1; and
o and p are each, independently, 0, 1 or 2,
wherein
   (i) the compound does not contain the following moiety: 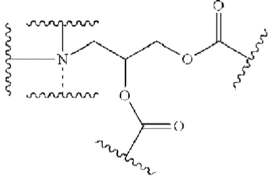
wherein ---- is an optional bond; and
Q³ and Q⁴ are each, independently, separated from the tertiary carbon atom marked with an asterisk (*) by a chain of 8 or more atoms.

In a more specific embodiment, the cationic lipid is selected from the following compounds: 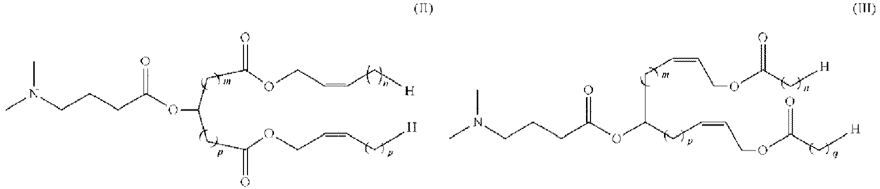 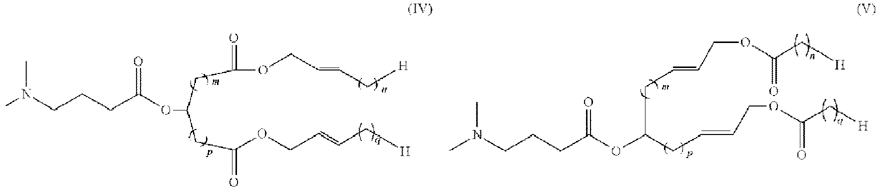 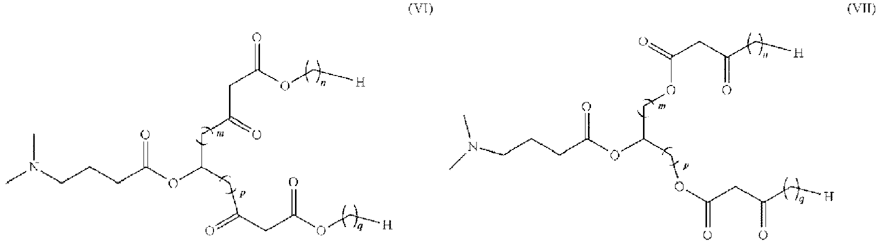 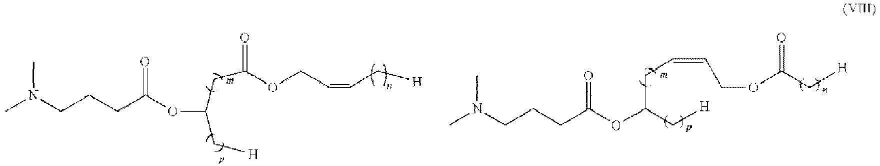 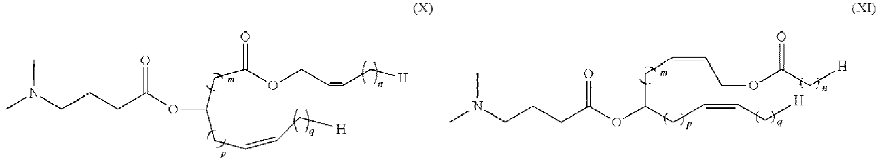 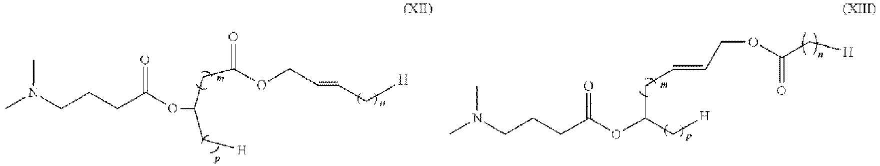 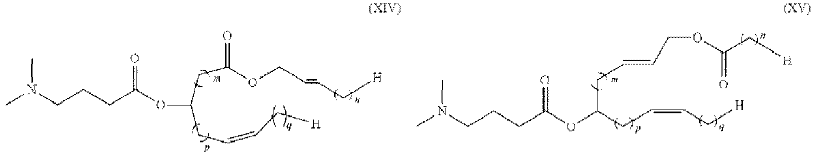 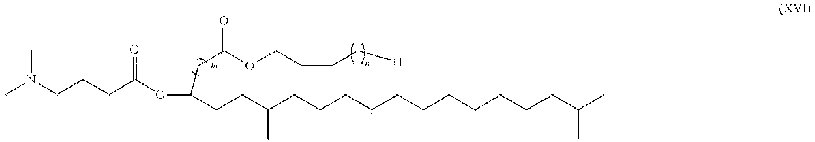 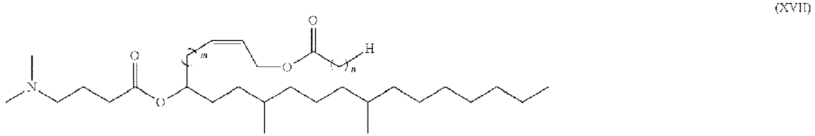 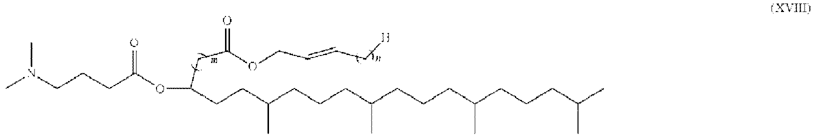 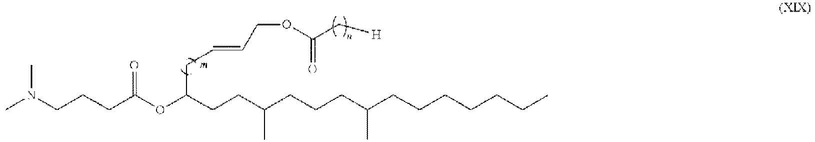 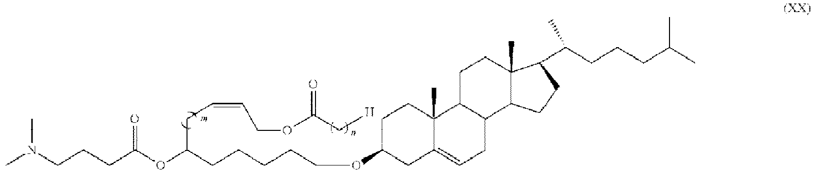 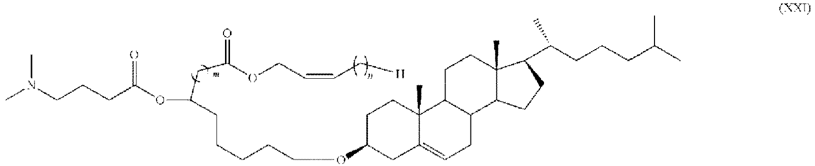 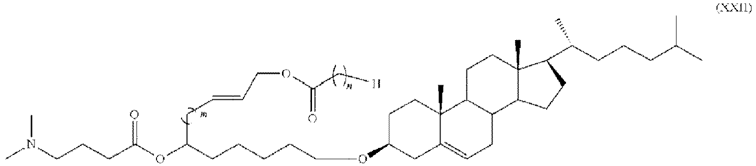 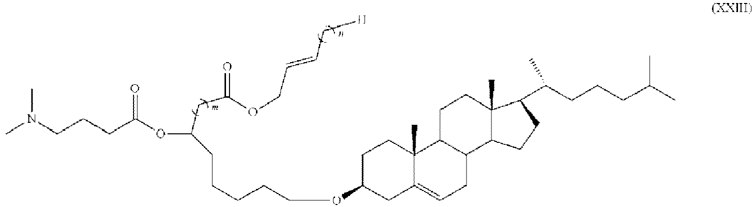 and salts thereof (e.g., pharmaceutically acceptable salts thereof),
wherein
m, n, o and p are each, individually, 1-25, with the proviso that:
(i) in structure (II), (IV), (VI) and (VII), m and p are both 4 or greater;
(ii) in structure (VIII), (X), (XII), (XIV), (XVI), (XVIII), (XXI) and (XXIII), m is 4 or greater; and
(iii) in structure (VIII), (IX), (XII) and (XIII), p is 8 or greater (e.g., 12 or 14 or greater).

In yet another more specific embodiment, the cationic lipid has the structure of:

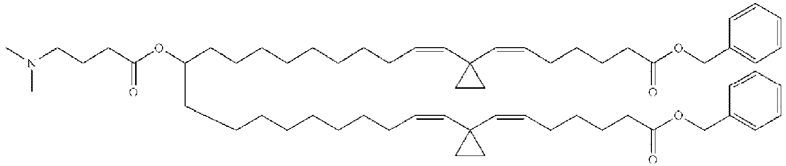

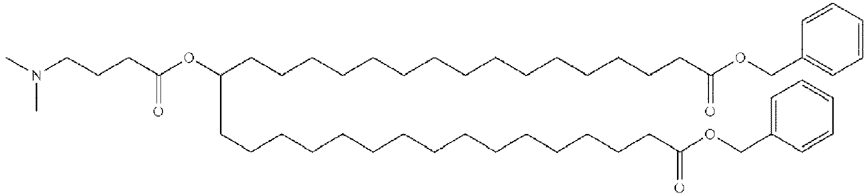

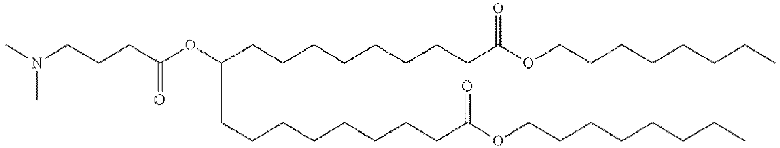

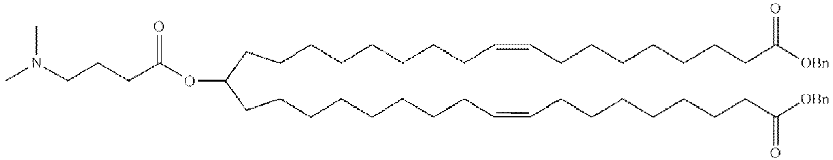

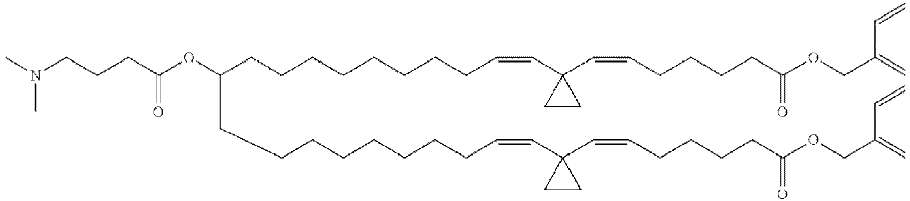

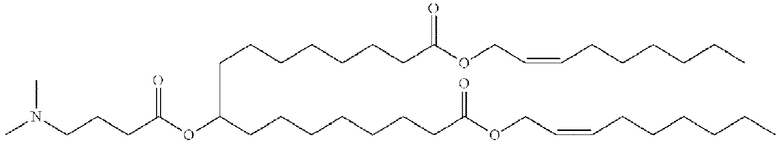

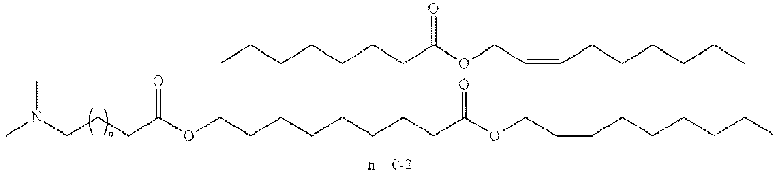

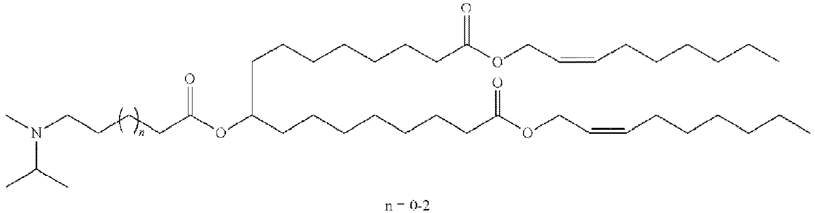

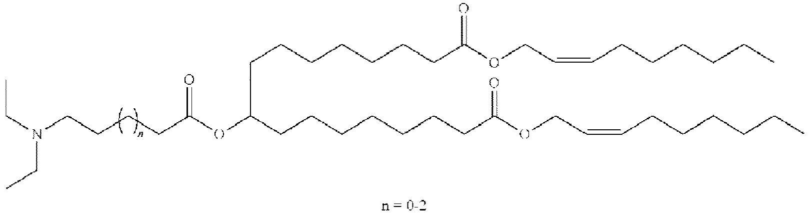

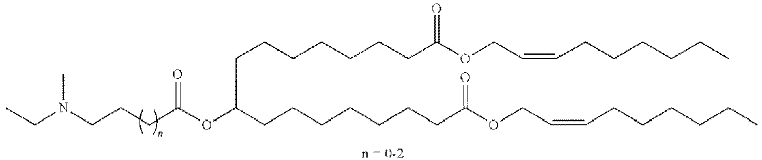

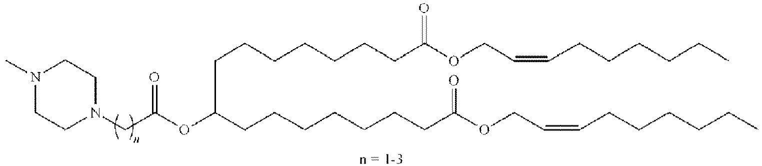

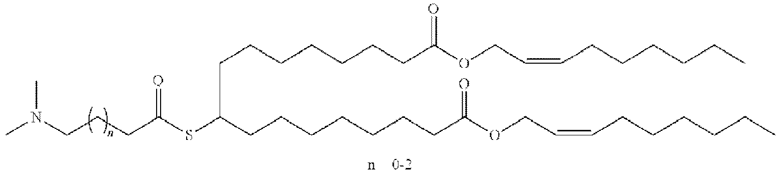

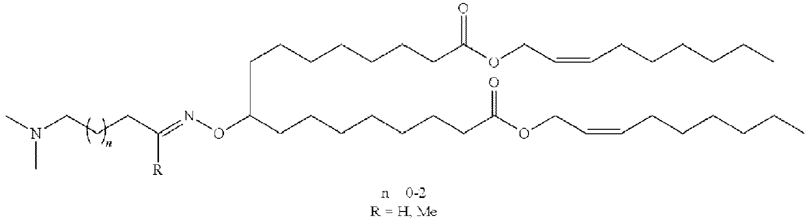

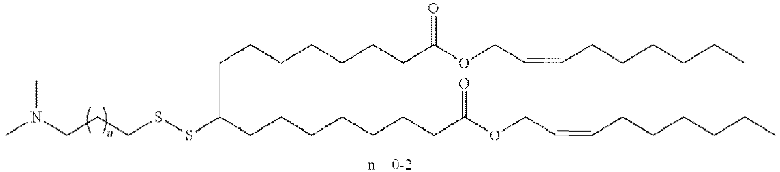

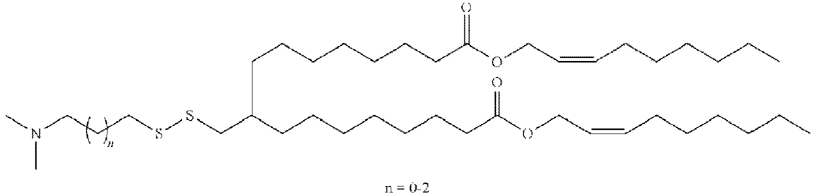

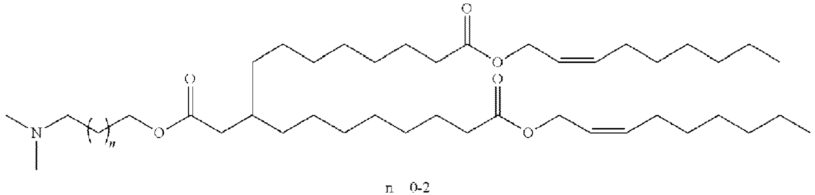

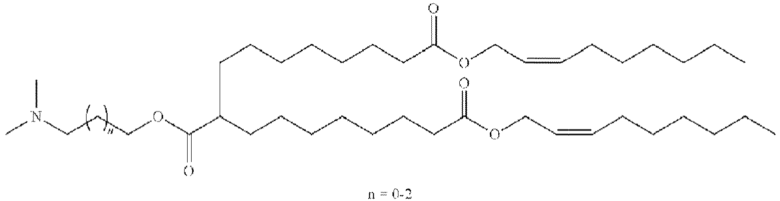

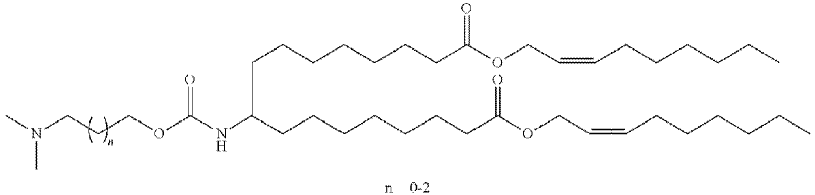

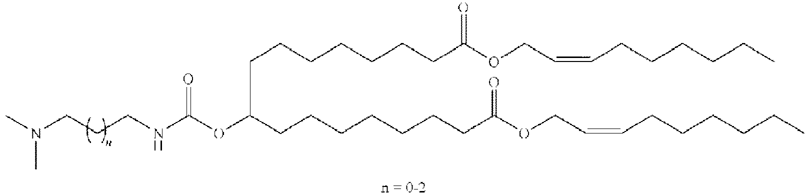

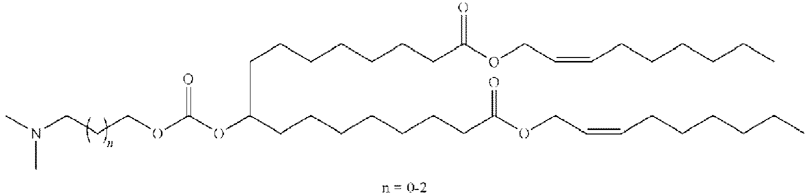

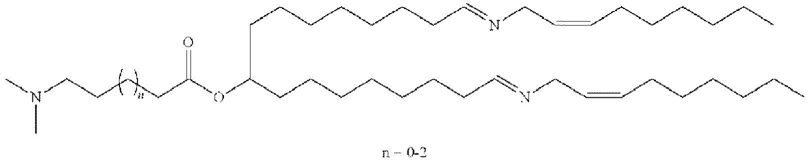

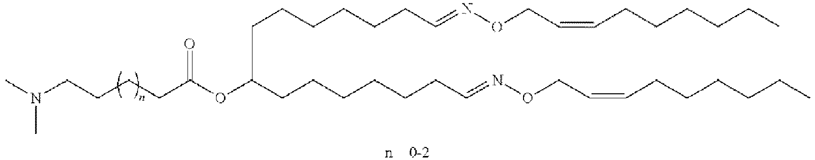

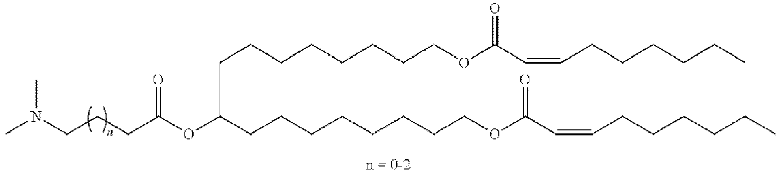

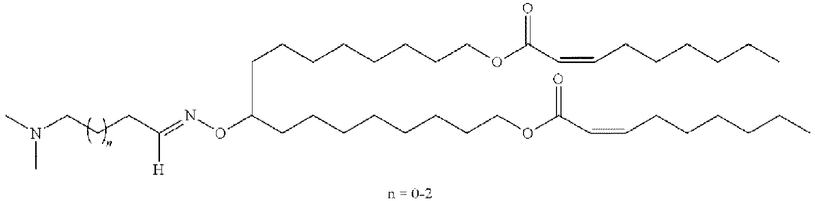

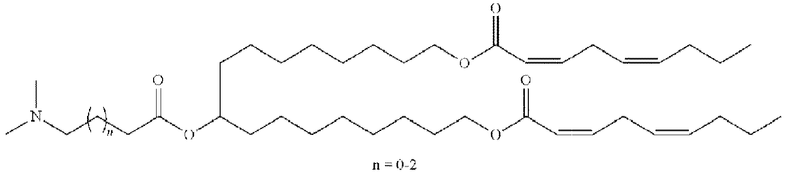

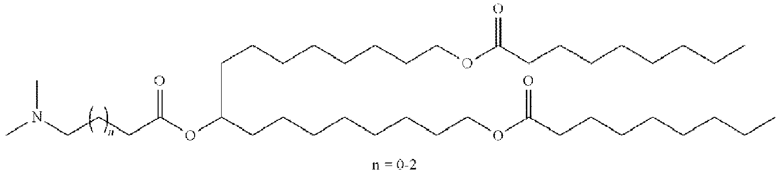

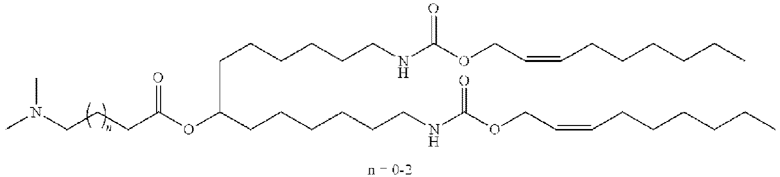

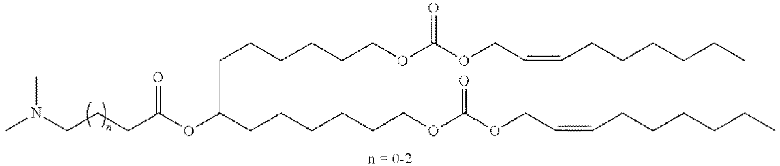

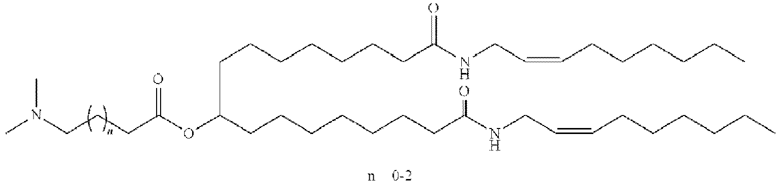

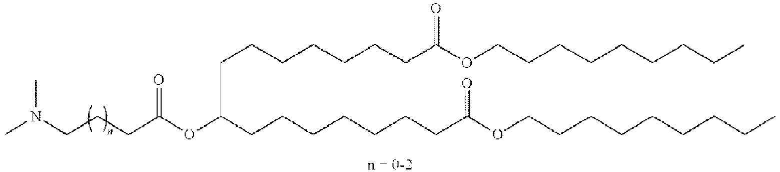

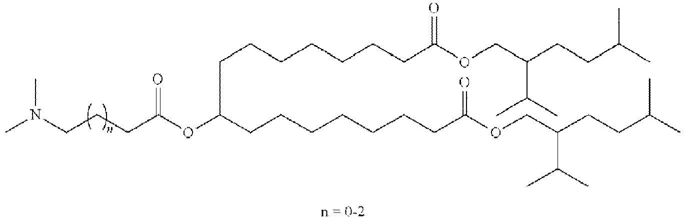

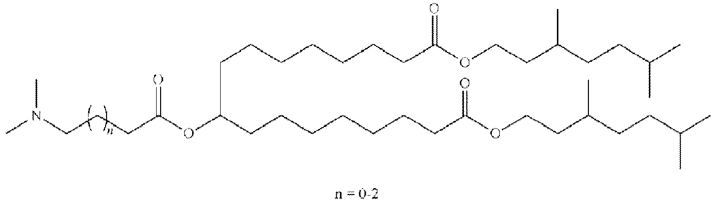

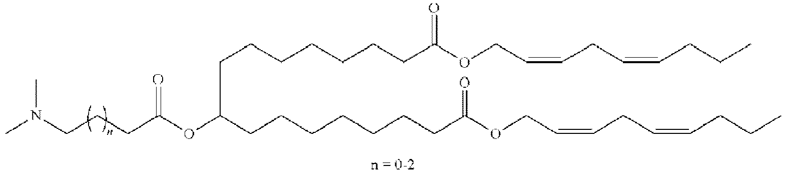

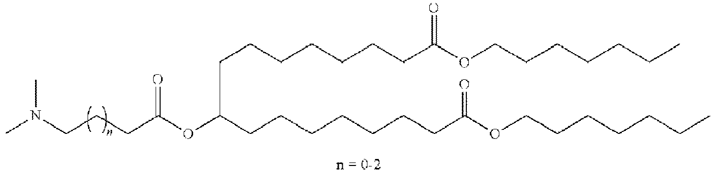

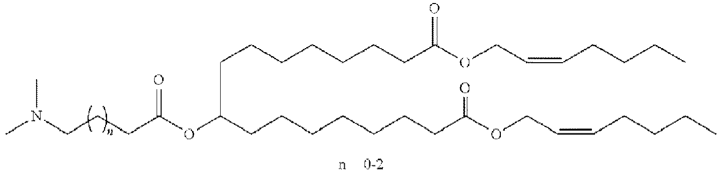

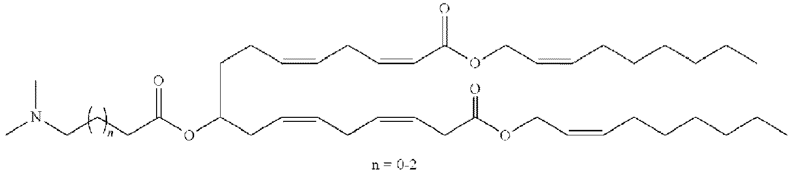

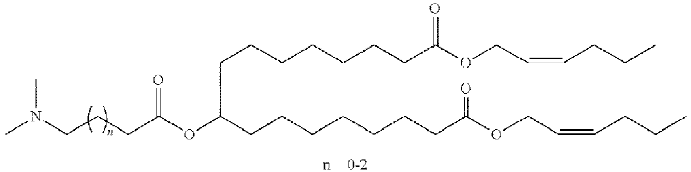

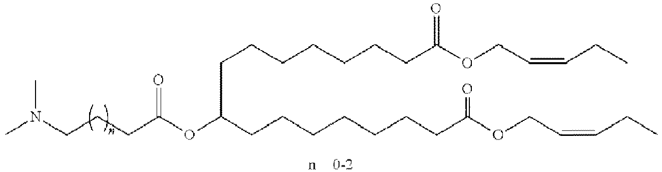

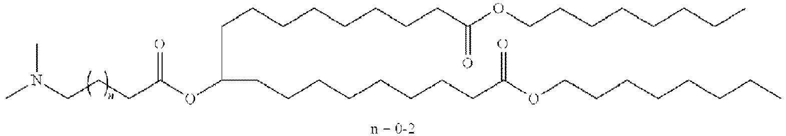

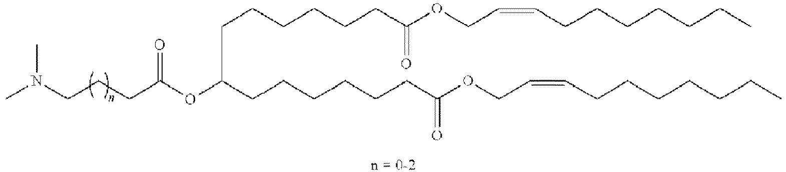

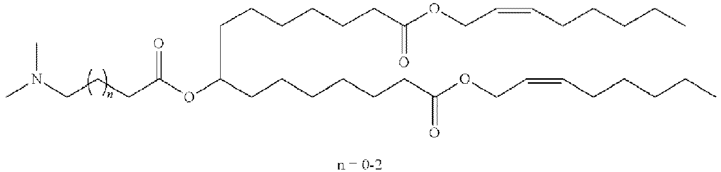

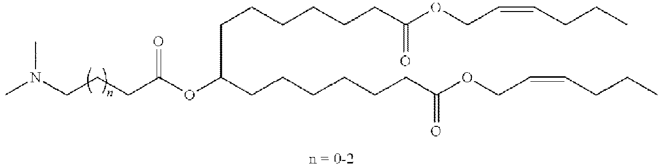

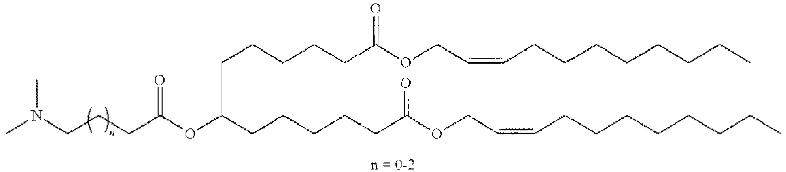

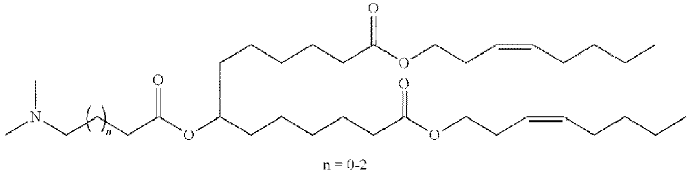

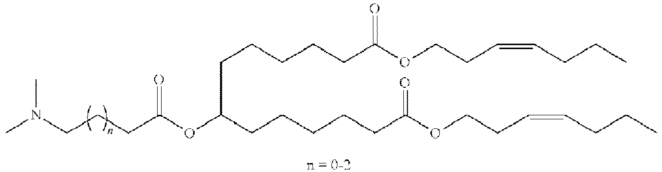

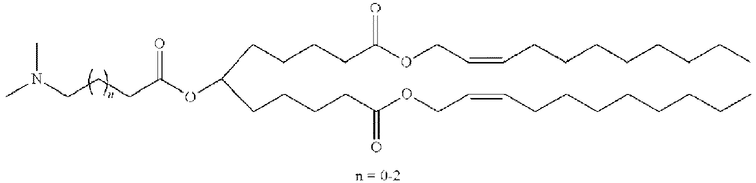

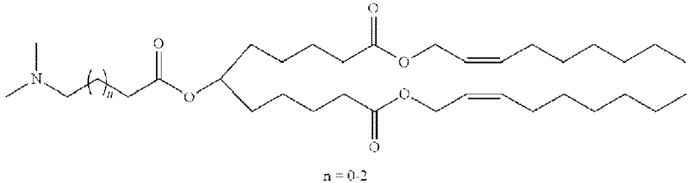

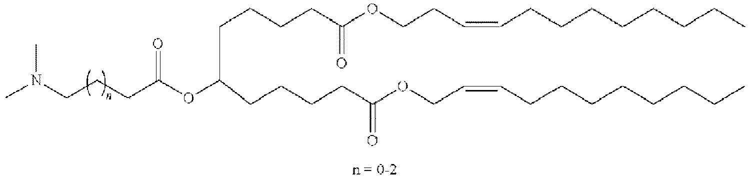

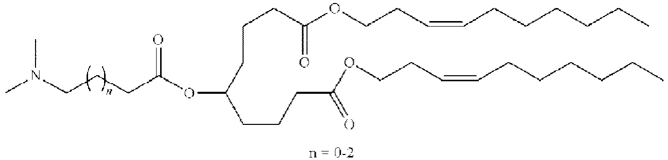

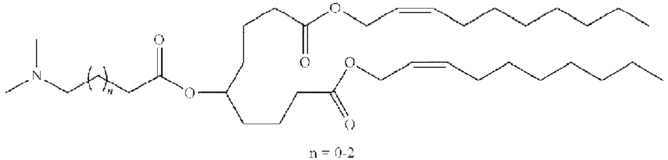

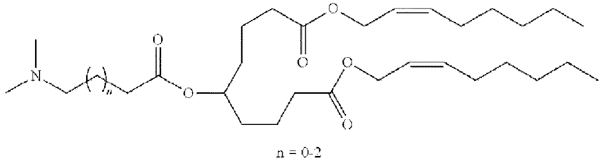

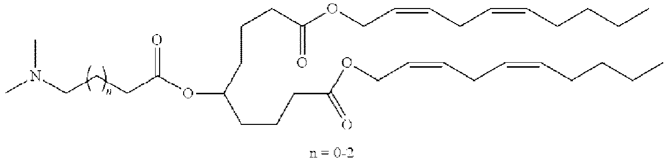

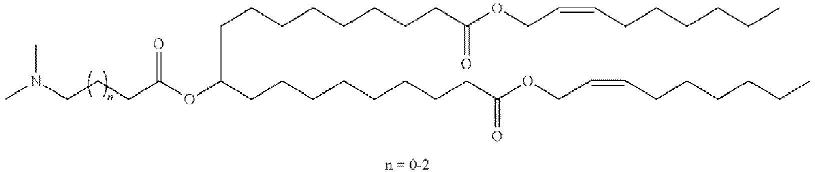

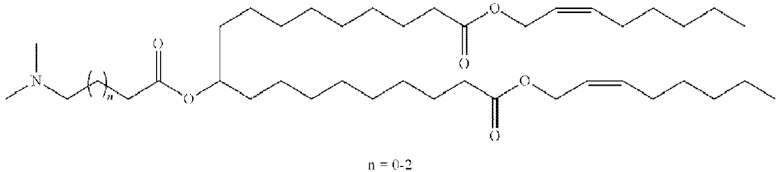

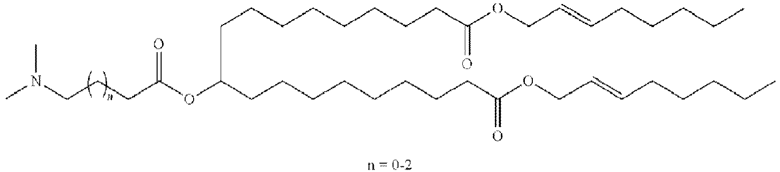

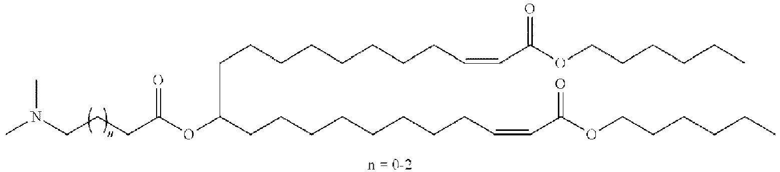

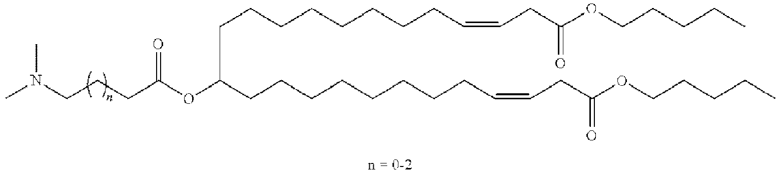

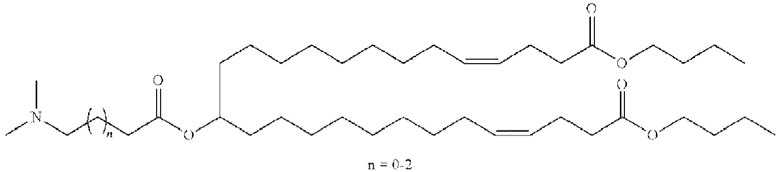

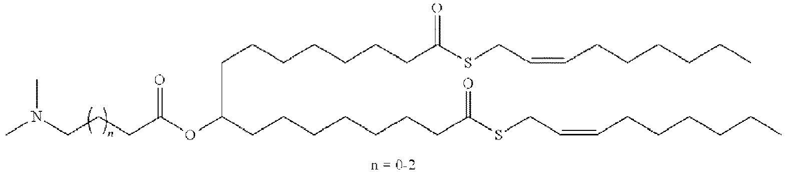

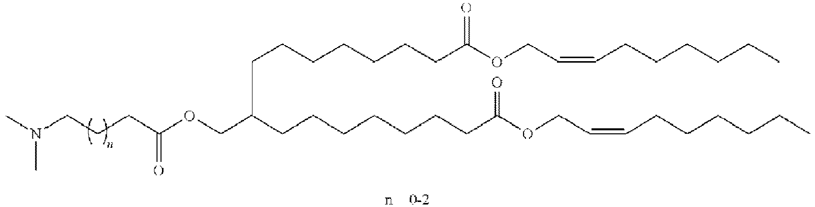

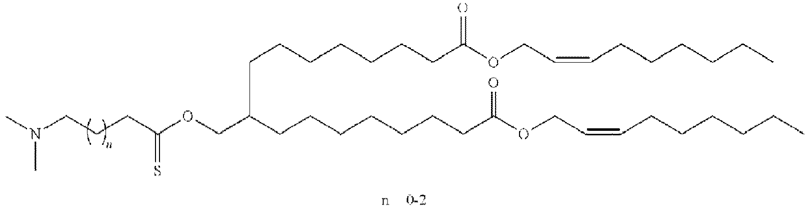

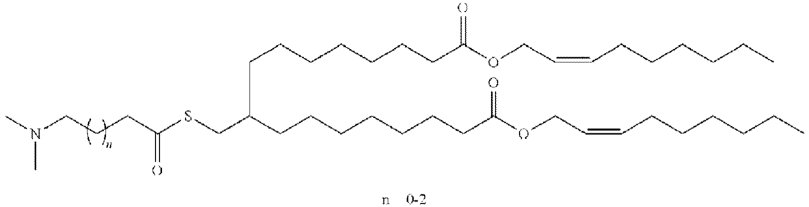

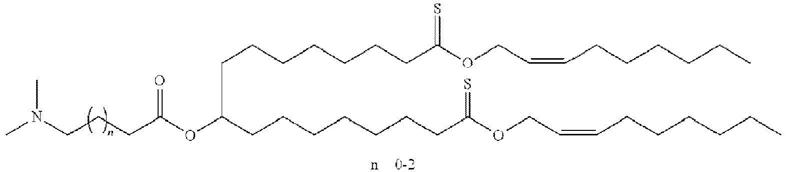

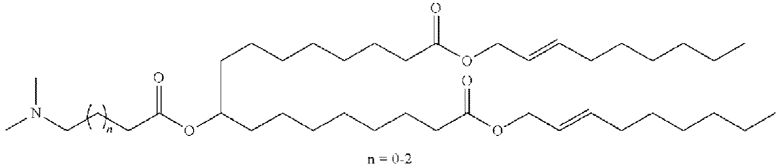

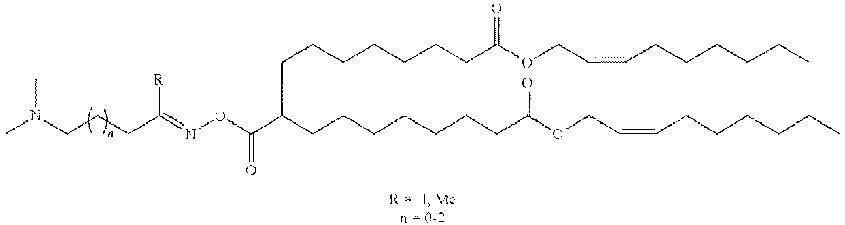

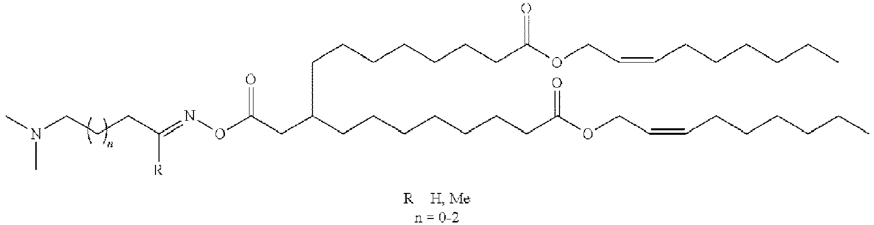

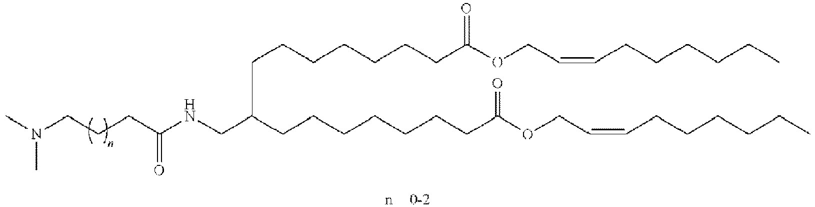

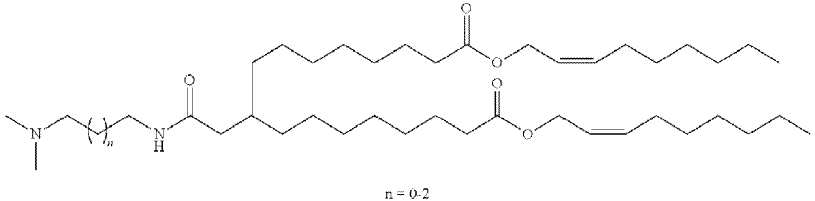

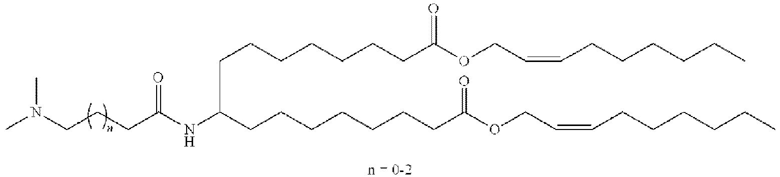

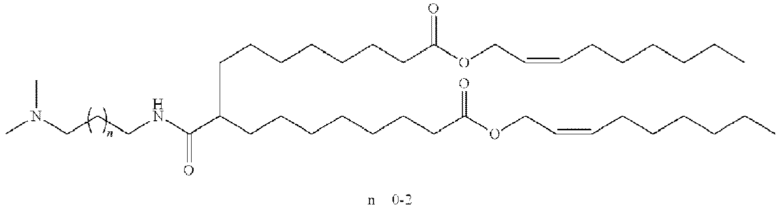

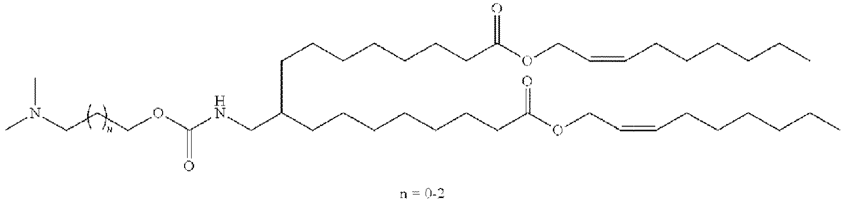

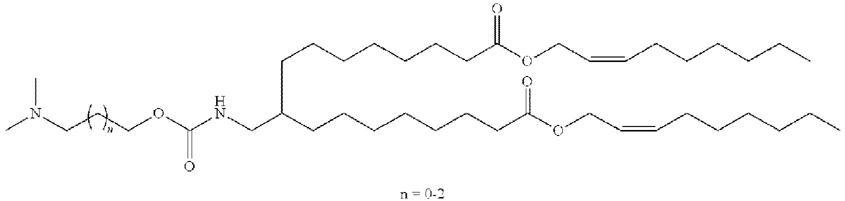

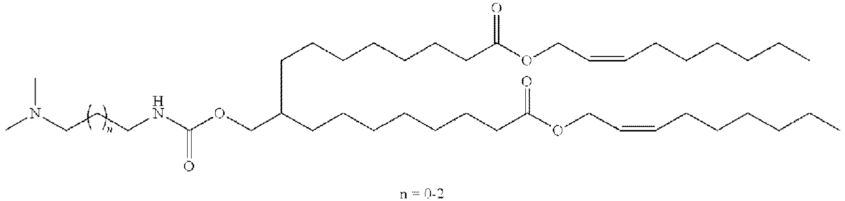

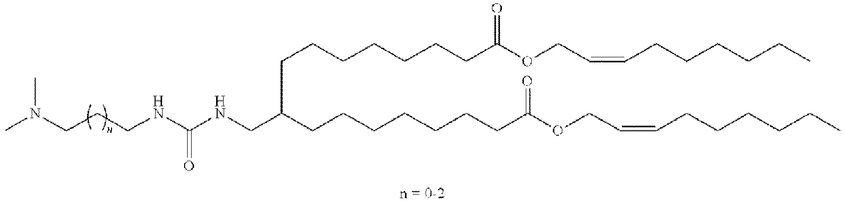

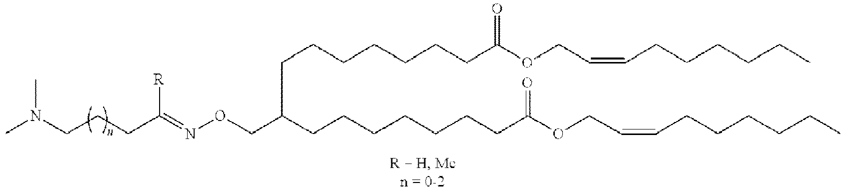

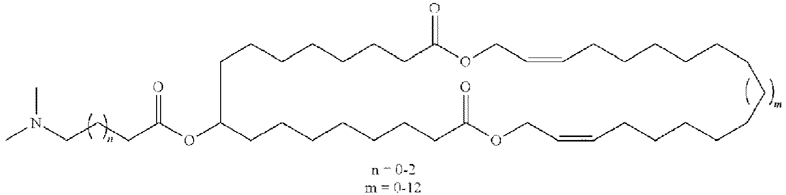

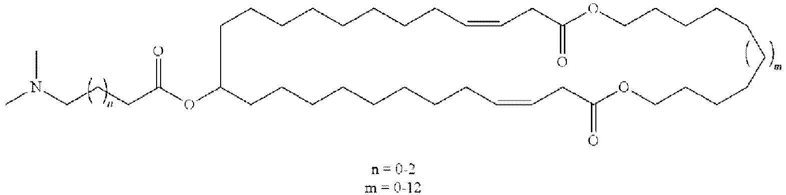

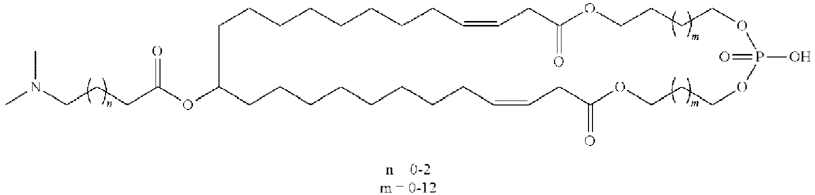

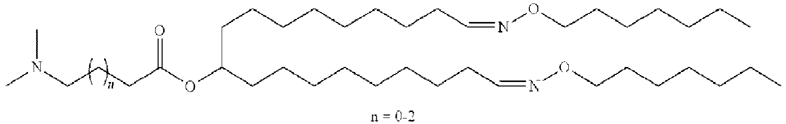

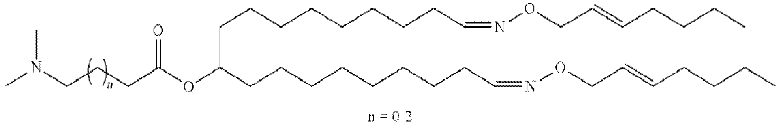

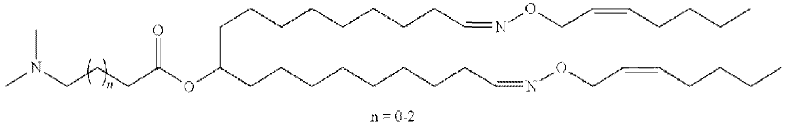

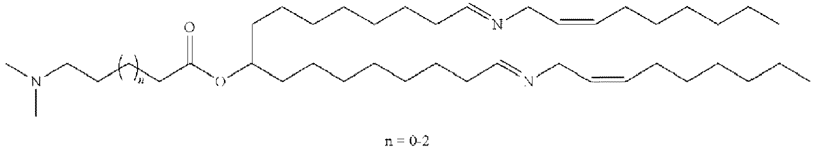

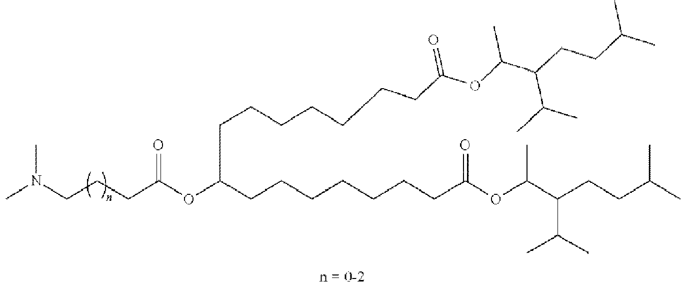

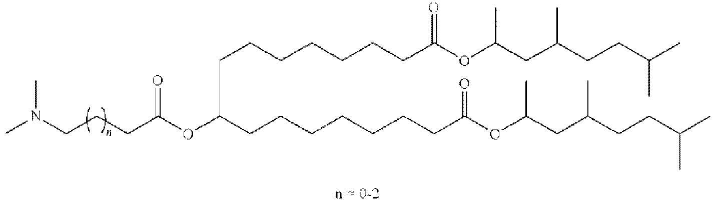

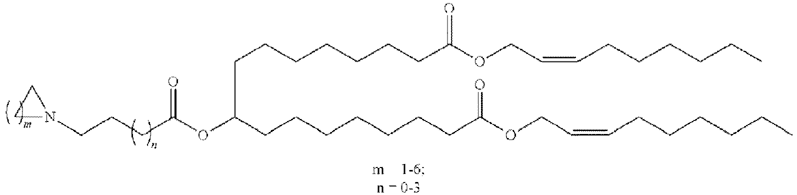

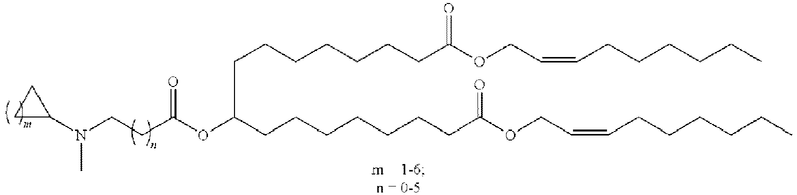

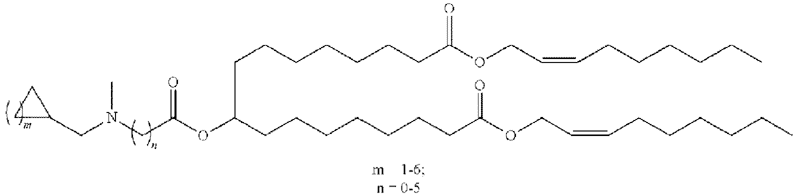

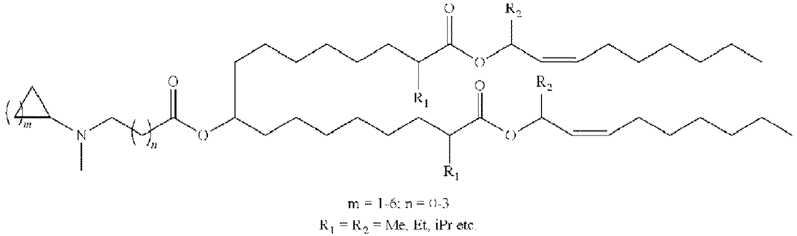

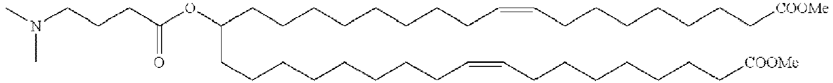

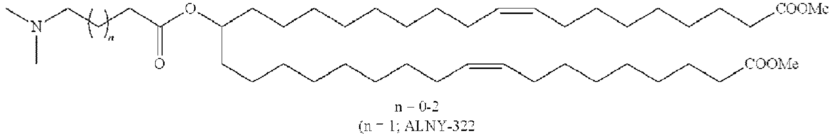

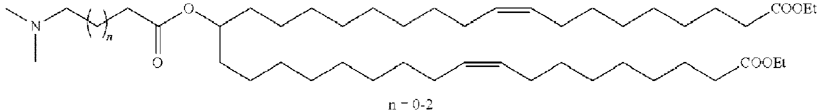

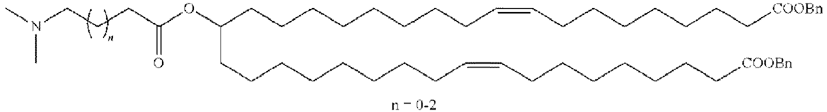

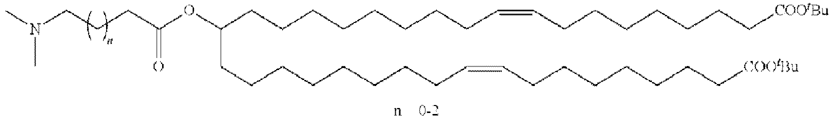

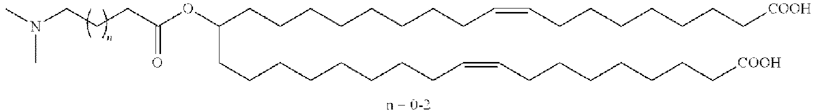

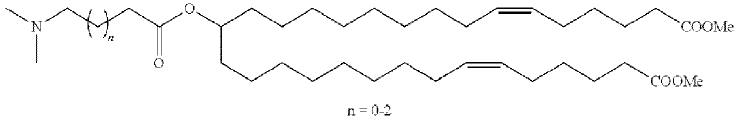

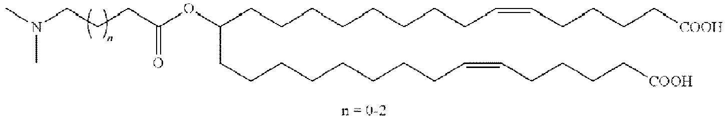

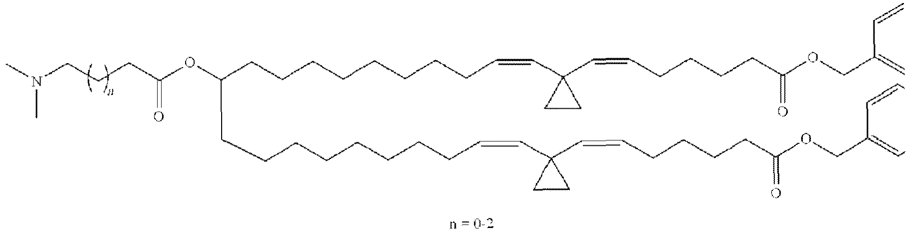

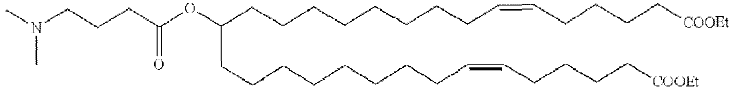

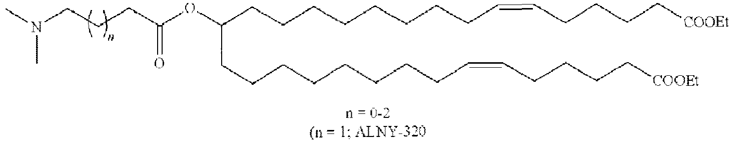

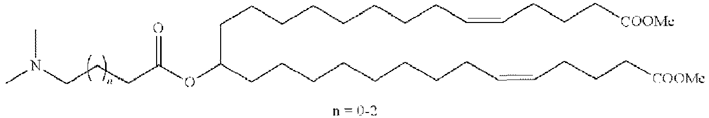

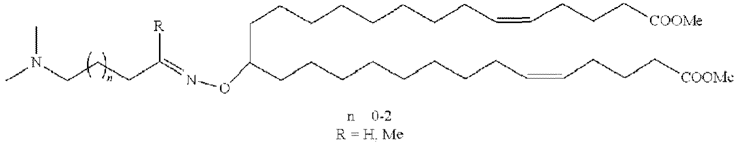

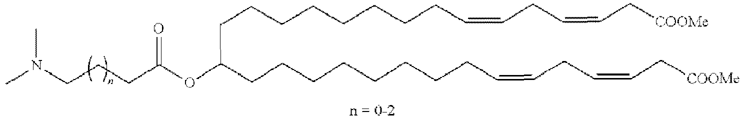

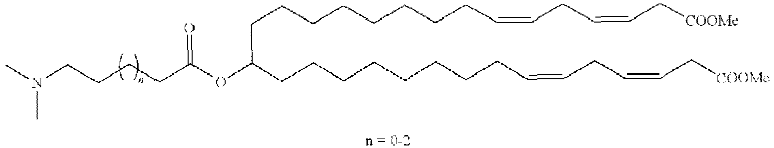

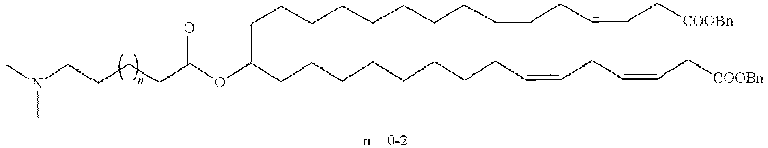

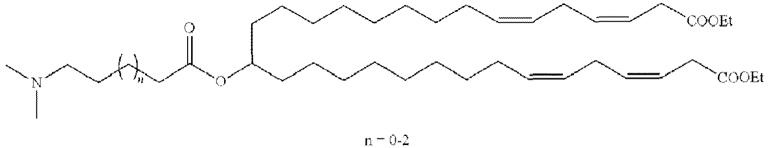

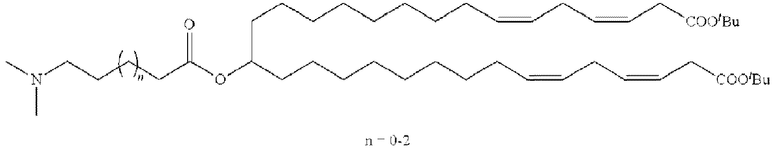

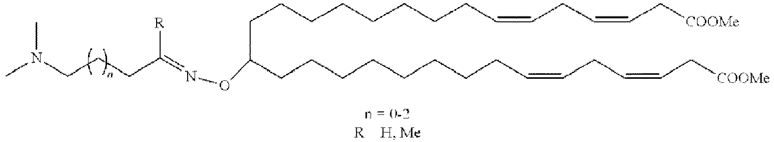

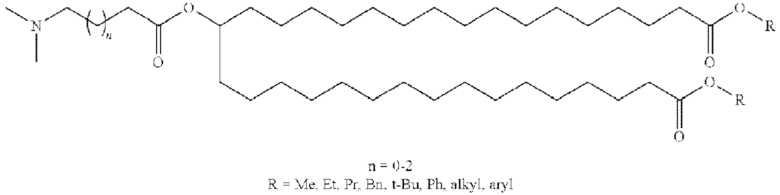

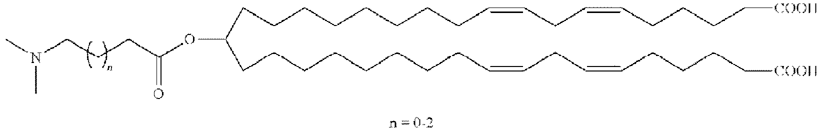

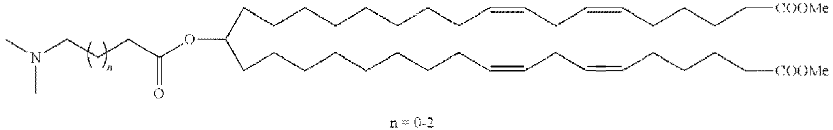

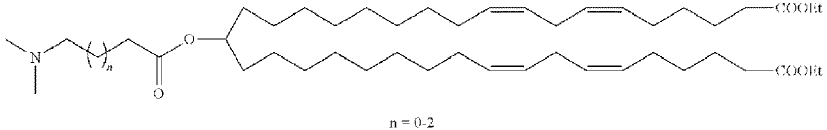

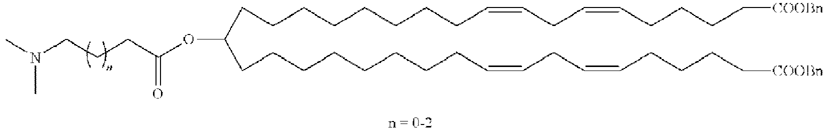

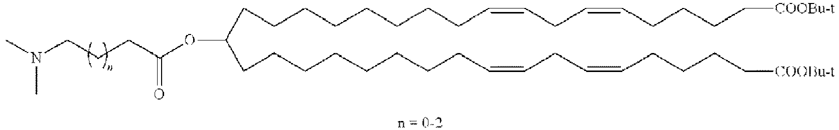

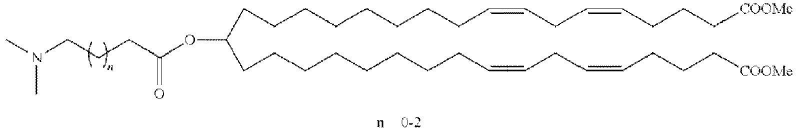

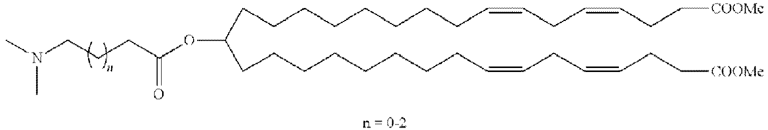

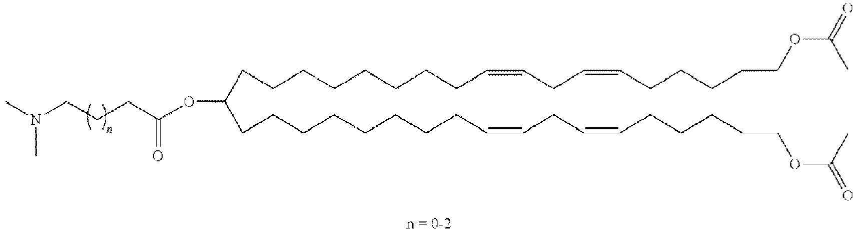

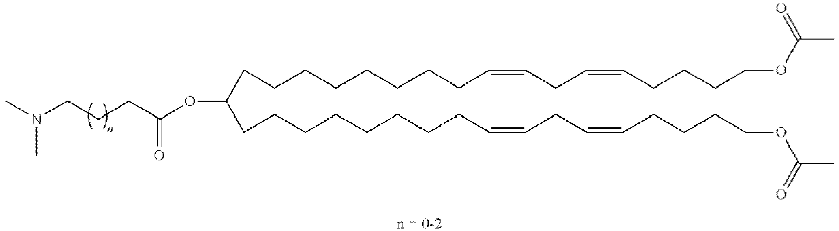

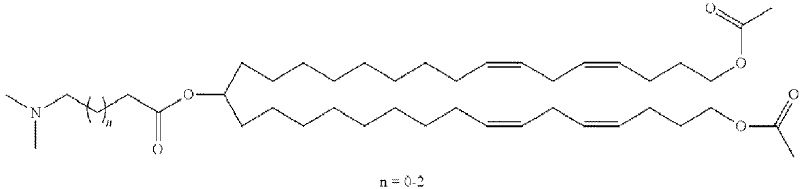

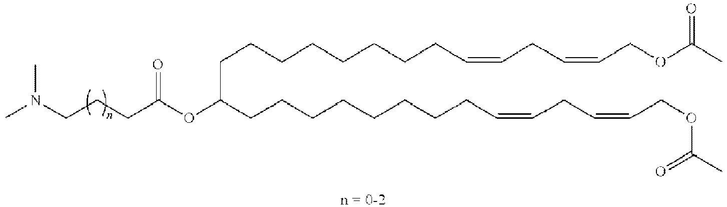

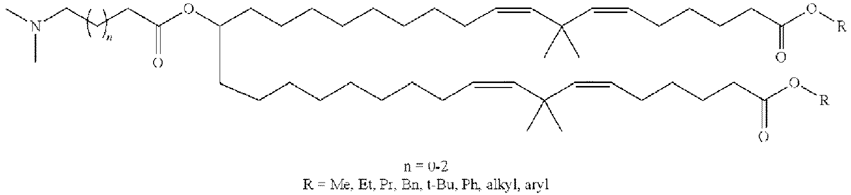

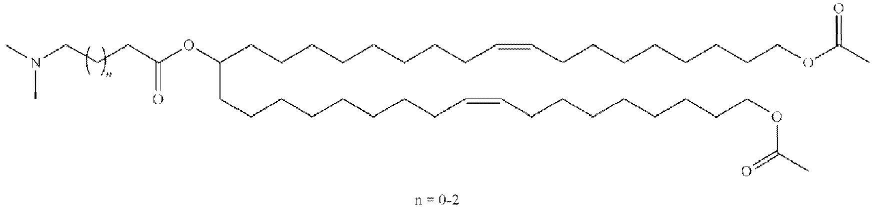

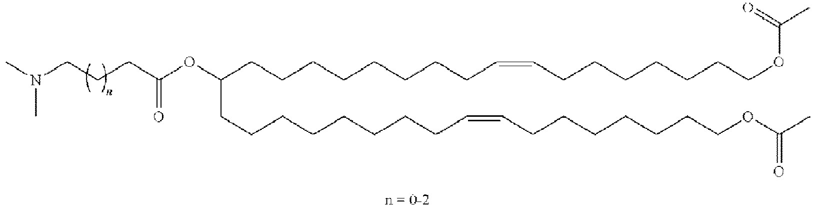

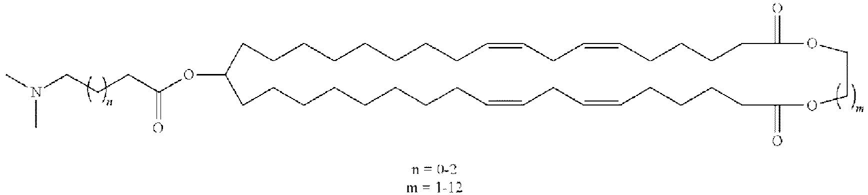

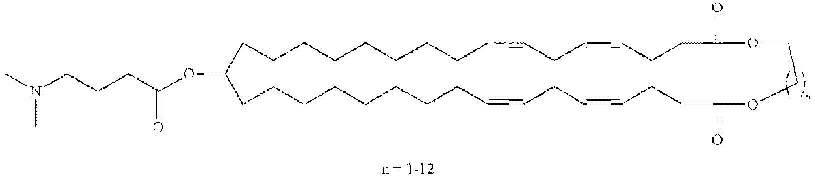

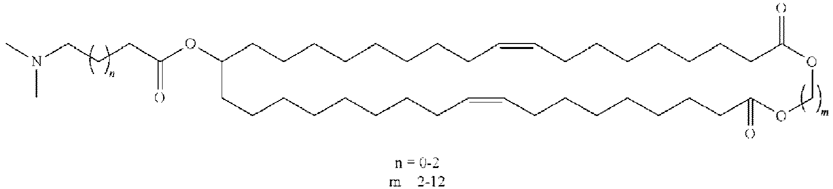

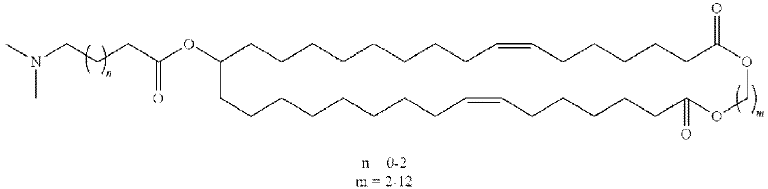

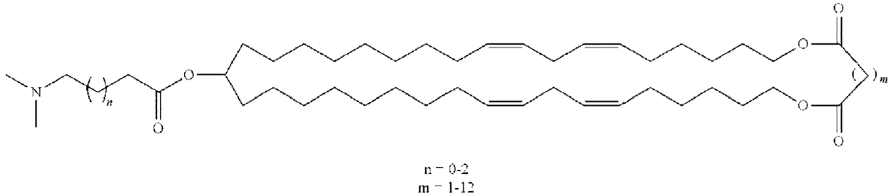

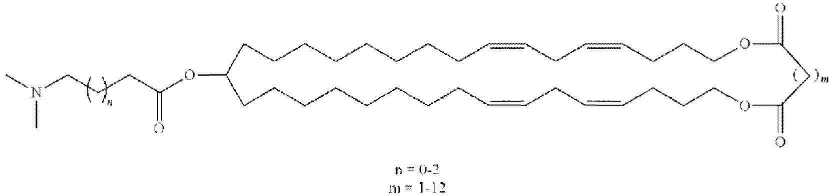

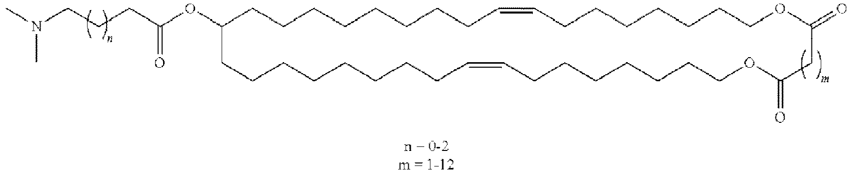

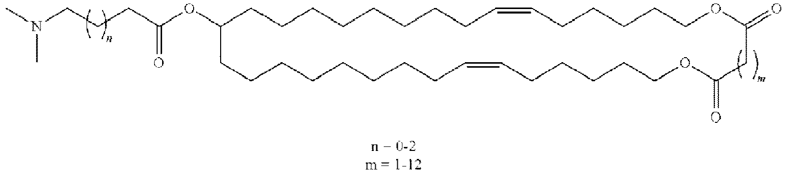

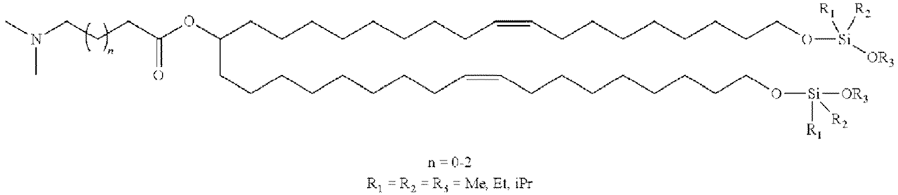

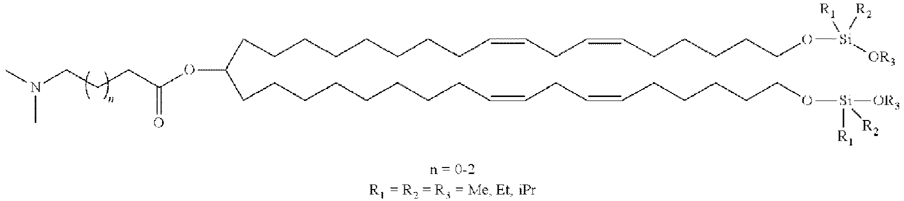

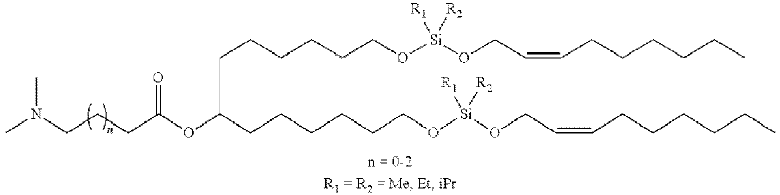

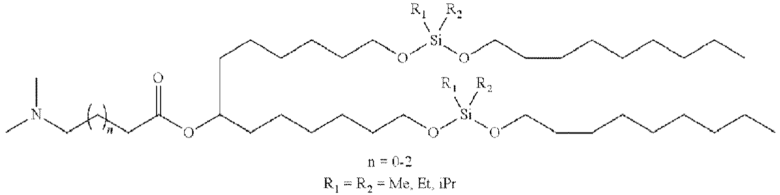

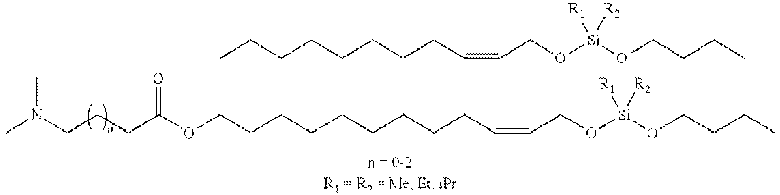

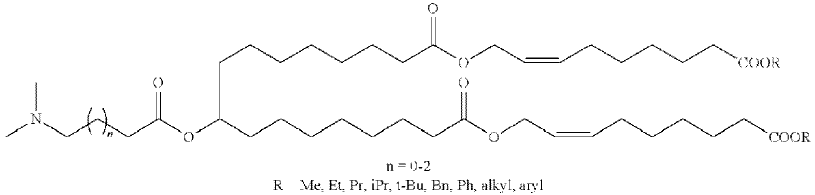

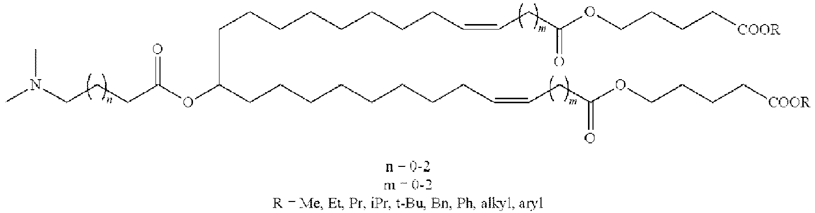

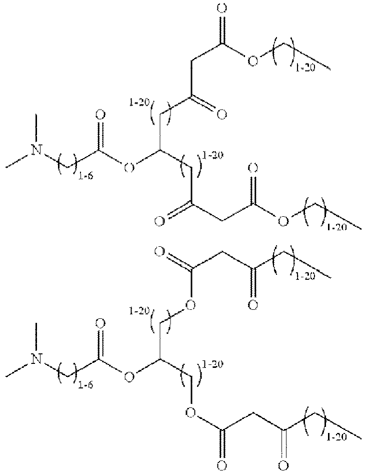

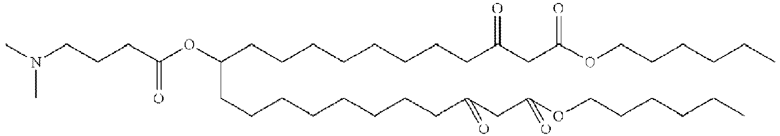

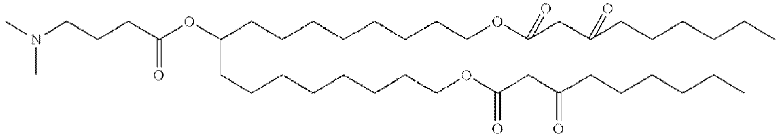

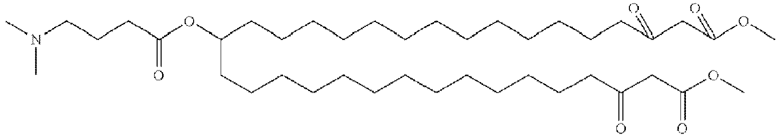

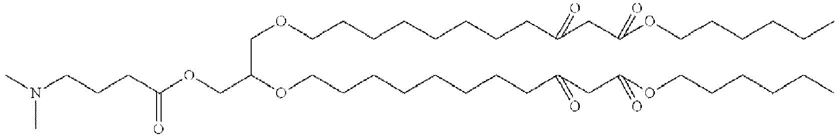

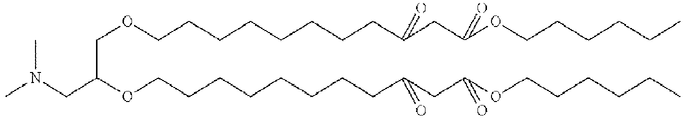

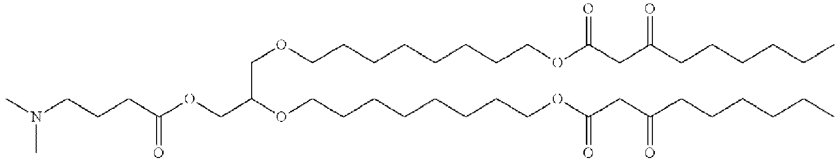

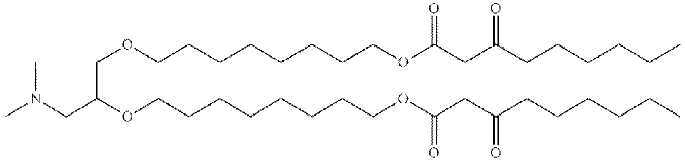

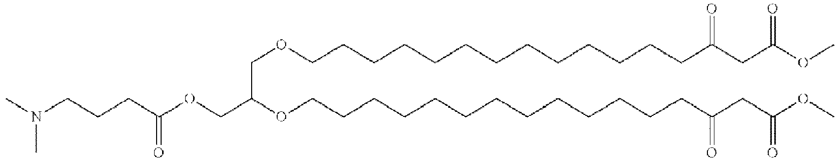

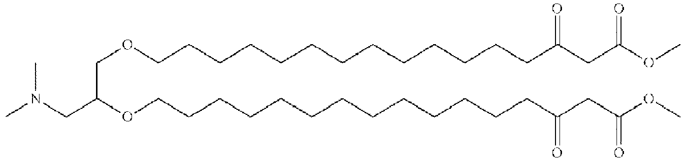

In an embodiment, the cationic lipid has the structure: 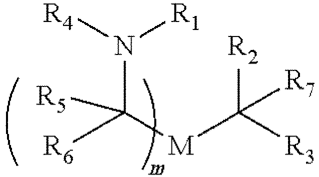 or a salt or isomer thereof, wherein:
R¹ is selected from the group consisting of C₅₋₃₀ alkyl, C₅₋₂₀alkenyl, - R*YR", -YR", and -R"M'R';
R₂ and R₃ are independently selected from the group consisting of H, C₁₋₁₄ alkyl, C₂₋₁₄ alkenyl, -R*YR", -YR", and -R*OR", or R₂ and R₃, together with the atom to which they are attached, form a heterocycle or carbocycle;
R⁴ is selected from the group consisting of a C₃₋₆ carbocycle, -(CH₂)nQ, - (CH₂)nCHQR, -CHQR, -CQ(R)₂, and unsubstituted C₁₋₆ alkyl, where Q is selected from a carbocycle, heterocycle, -OR, -O(CH₂)nN(R)₂, -C(O)OR, -OC(O)R, -CX₃, -CX₂H, - CXH₂, -CN, -N(R)₂, -C(O)N(R)₂, -N(R)C(O)R, -N(R)S(O)₂R, -N(R)C(O)N(R)₂, - N(R)C(S)N(R)₂, -N(R)R₈, O(CH₂)nOR, -N(R)C(=NR₉)N(R)₂, -N(R)C(=CHR₉)N(R)₂, - OC(O)N(R)₂, -N(R)C(O)OR, -N(OR)C(O)R, -N(OR)S(O)₂R, -N(OR)C(O)OR, - N(OR)C(O)N(R)₂, -N(OR)C(S)N(R)₂, -N(OR)C(=NR₉)N(R)₂, -N(OR)C(=CHR₉)N(R)₂, -C(=NR₉)N(R)₂, -C(=NR₉)R, -C(O)N(R)OR, and -C(R)N(R)₂C(O)OR, and each n is independently selected from 1, 2, 3, 4, and 5;
each R₅ is independently selected from the group consisting of C₁₋₃ alkyl, C₂₋₃ alkenyl, and H;
each R₆ is independently selected from the group consisting of C₁₋₃ alkyl, C₂₋₃ alkenyl, and H;
M and M' are independently selected from -C(O)O-, -OC(O)-, - C(O)N(R')-, -N(R')C(O)-, -C(O)-, -C(S)-, -C(S)S-, -SC(S)-, -CH(OH)-, -P(O)(OR')O-, - S(O)₂-, -S-S-, an aryl group, and a heteroaryl group;
R₇ is selected from the group consisting of C₁₋₃ alkyl, C₂₋₃ alkenyl, and H;
R₈ is selected from the group consisting of C₃₋₆ carbocycle and heterocycle;
R₉ is selected from the group consisting of H, CN, NO₂, C₁₋₆ alkyl, -OR, - S(O)₂R, -S(O)₂N(R)₂, C₂₋₆ alkenyl, C₃₋₆ carbocycle and heterocycle;
each R is independently selected from the group consisting of C₁₋₃alkyl, C₂₋₃ alkenyl, and H;
each R' is independently selected from the group consisting of C₁₋₁₈ alkyl, C₂₋₁₈ alkenyl, -R*YR", -YR", and H;
each R" is independently selected from the group consisting of C₃₋₁₄ alkyl and C₃₋₁₄ alkenyl;
each R* is independently selected from the group consisting of C₁₋₁₂ alkyl and C₂₋₁₂ alkenyl;
each Y is independently a C₃₋₆ carbocycle;
each X is independently selected from the group consisting of F, Cl, Br, and I; and
m is selected from 5, 6, 7, 8, 9, 10, 11, 12, and 13.

In yet another embodiment, the cationic lipid is selected from the compounds:

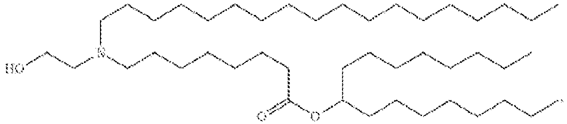

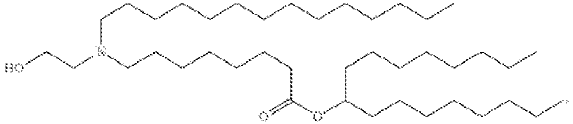

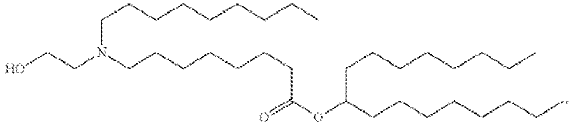

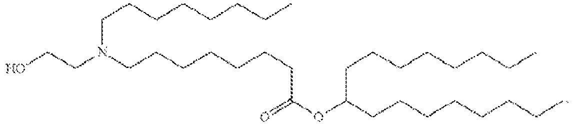

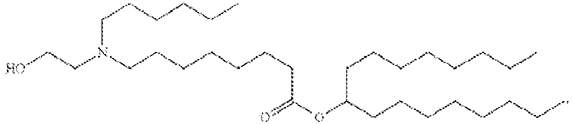

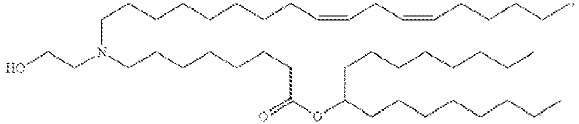

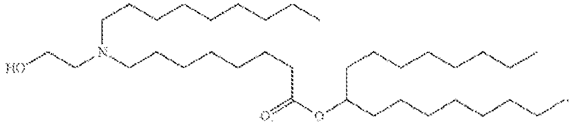

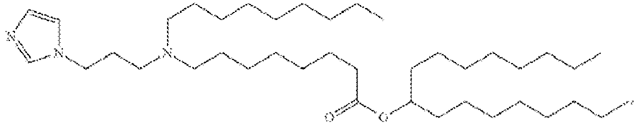

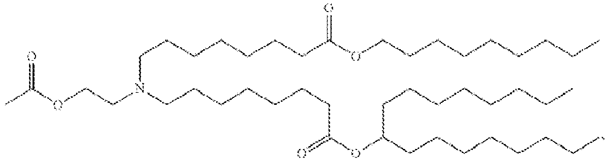

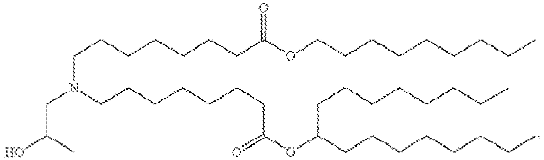

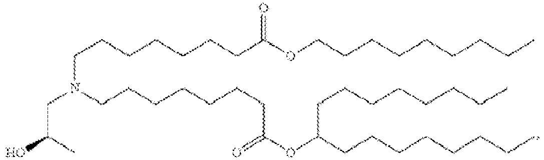

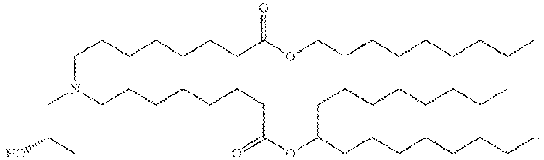

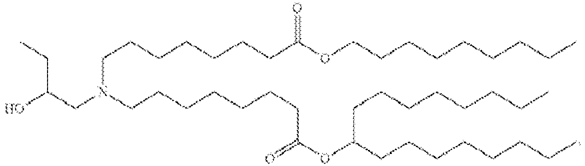

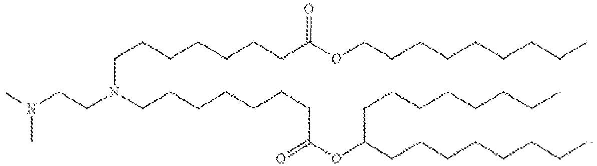

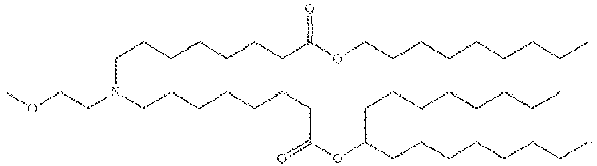

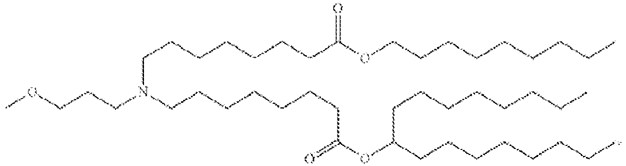

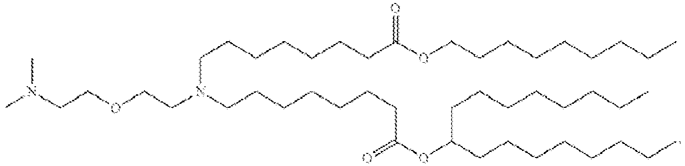

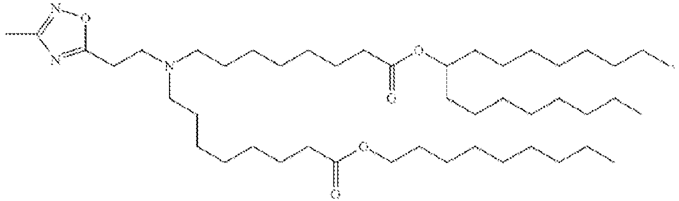

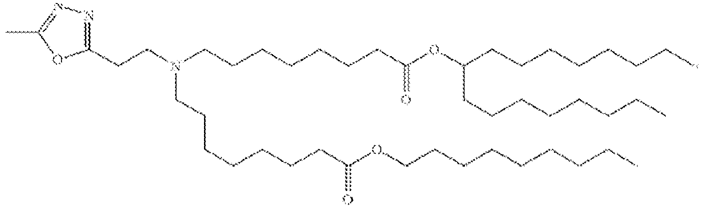

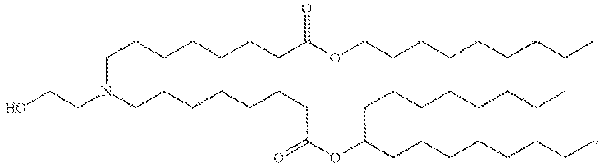

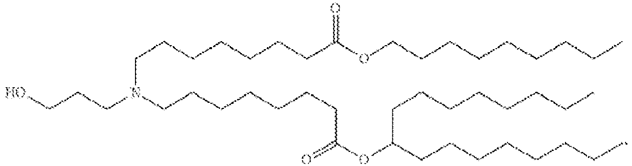

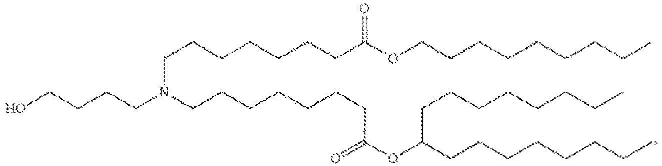

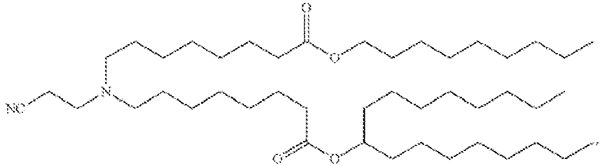

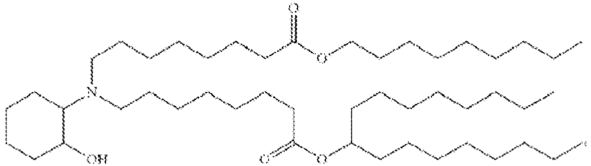

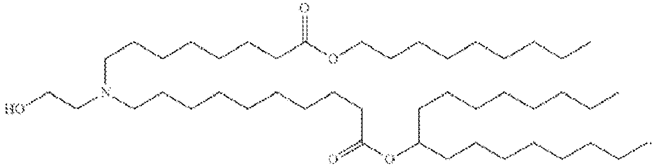

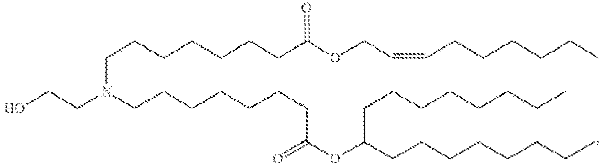

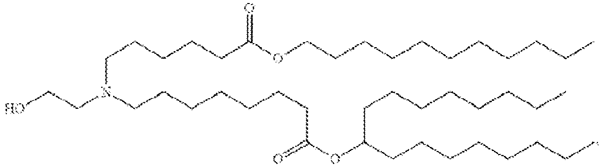

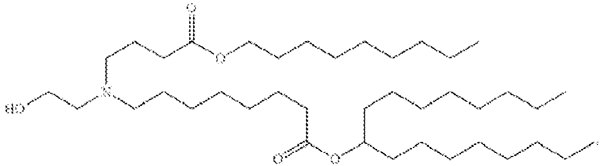

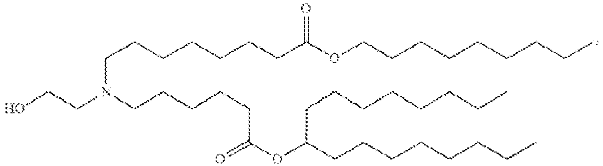

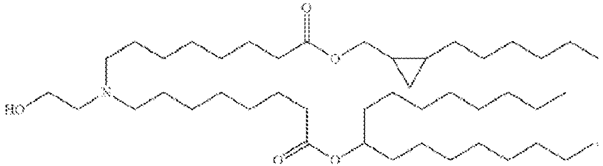

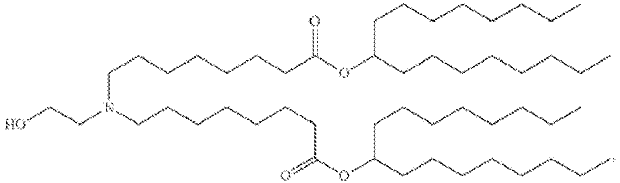

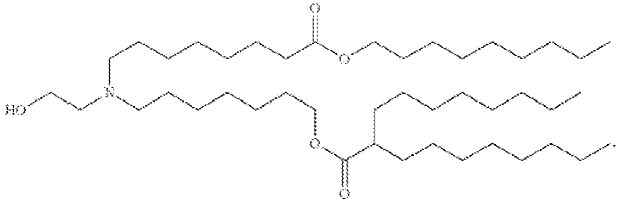

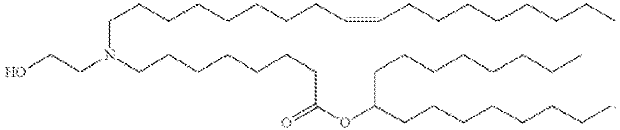

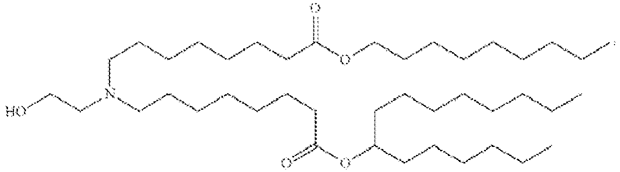

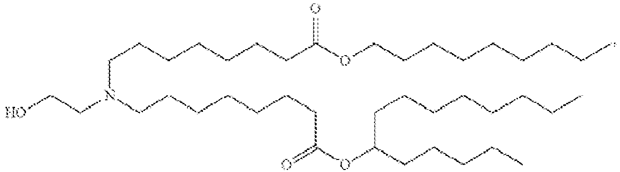

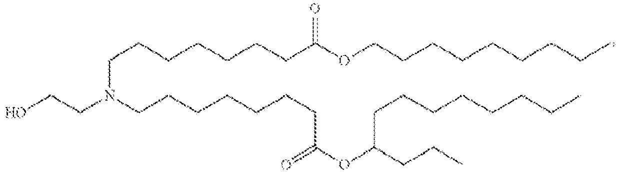

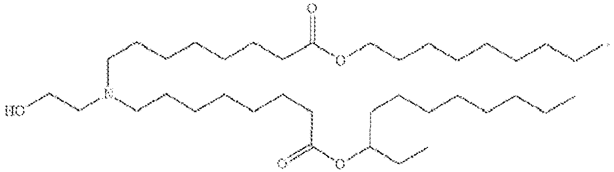

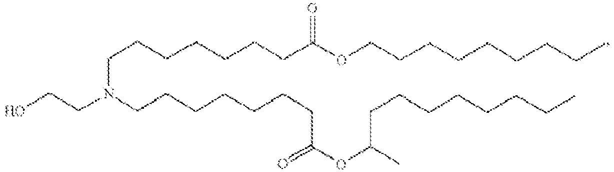

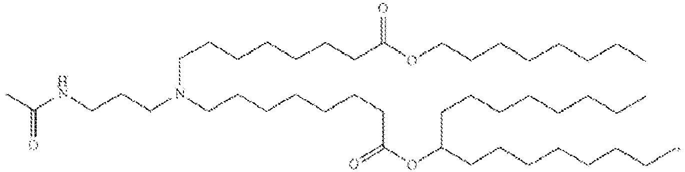

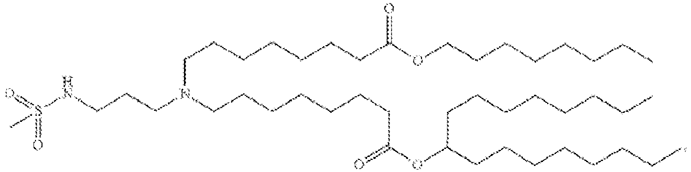

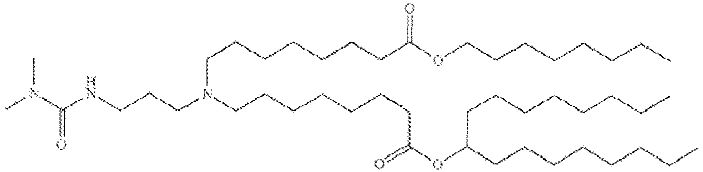

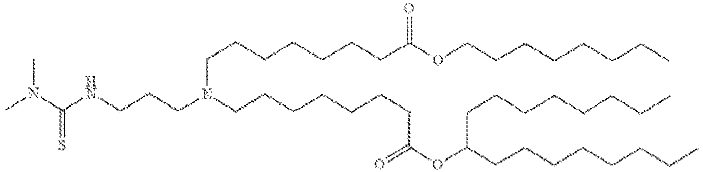

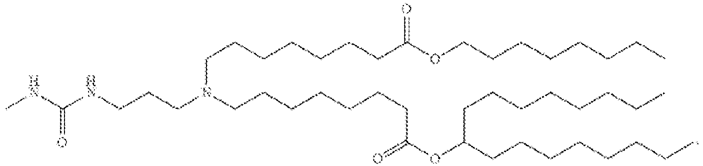

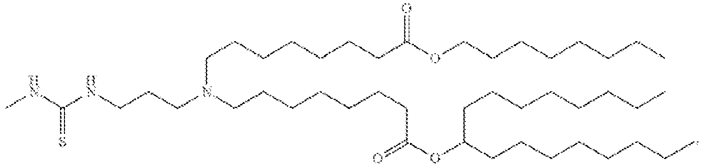

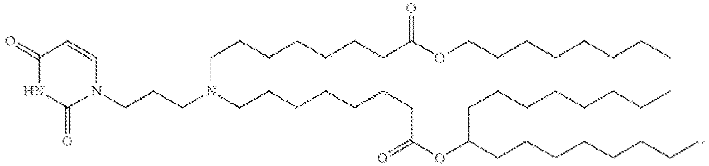

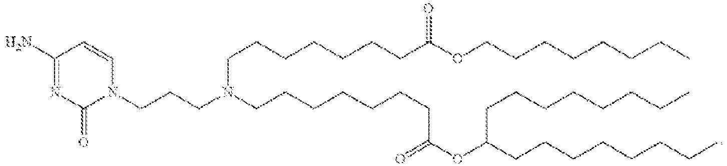

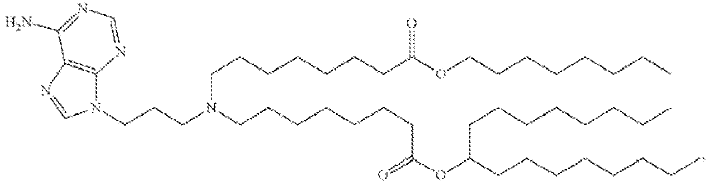

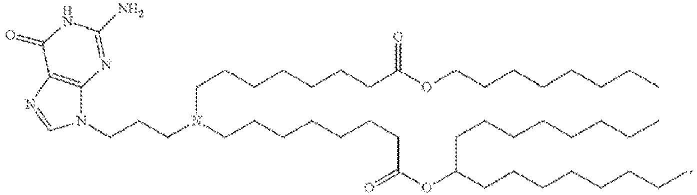

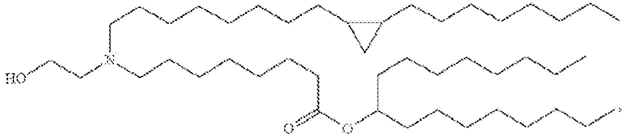

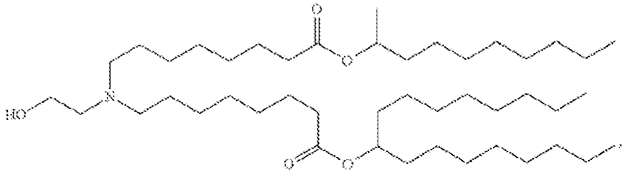

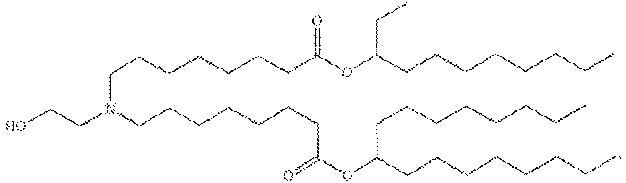

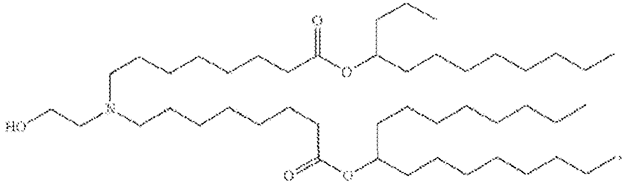

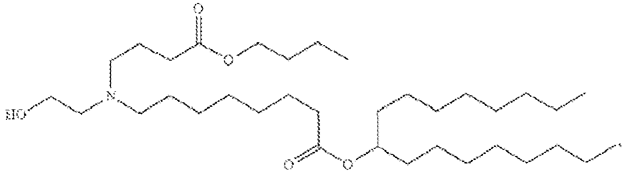

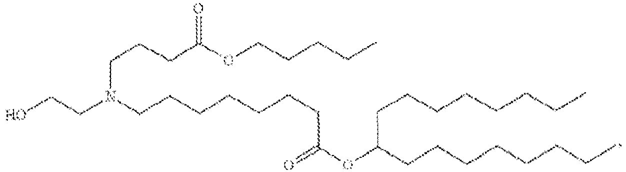

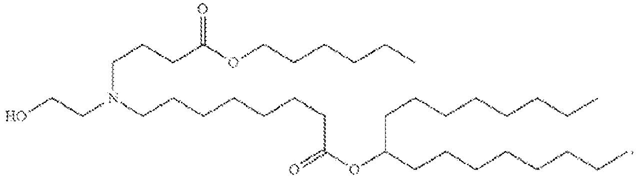

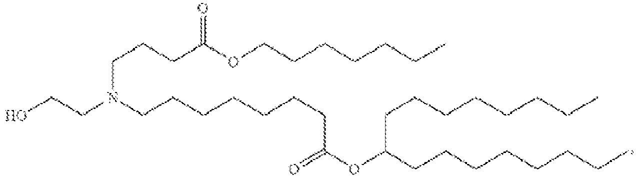

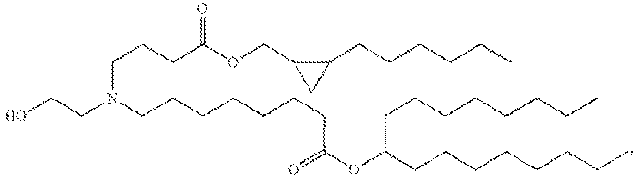

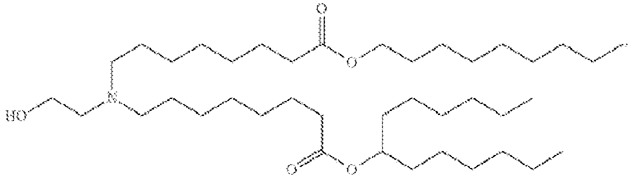

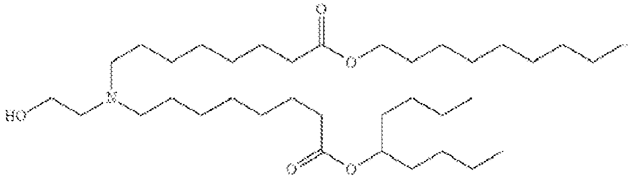

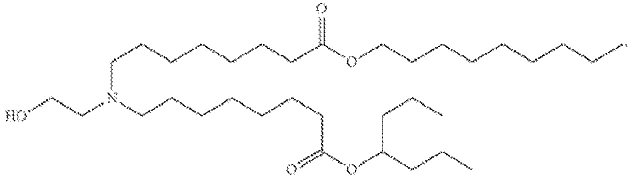

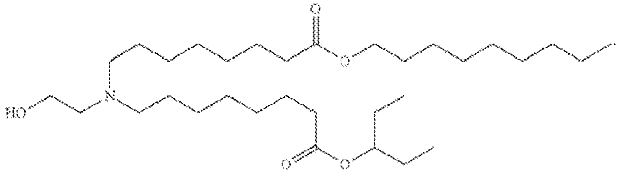

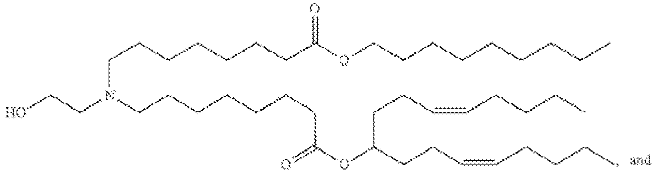

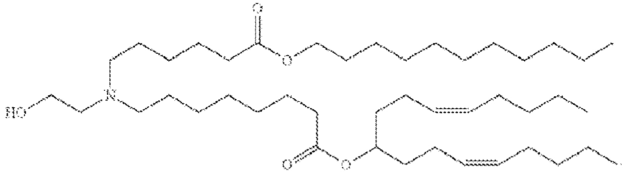

In an embodiment, the cationic lipid has the following structure: 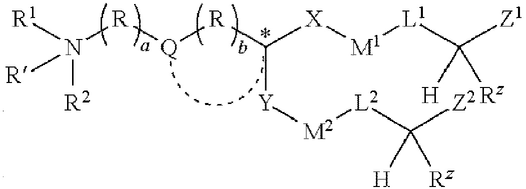 or a salt thereof, wherein
R' is absent, hydrogen, or C₁-C₄ alkyl;
with respect to R¹ and R²,
   (i) R¹ and R² are each, independently, optionally substituted alkyl, alkenyl, alkynyl, cycloalkylalkyl, heterocycle, or R¹⁰;
   (ii) R¹ and R², together with the nitrogen atom to which they are attached, form an optionally substituted heterocylic ring; or
   (iii) one of R¹ and R² is optionally substituted alkyl, alkenyl, alkynyl, cycloalkyl, cycloalkylalkyl, or heterocycle, and the other forms a 4-10 member heterocyclic ring or heteroaryl with (a) the adjacent nitrogen atom and (b) the (R)ₐ group adjacent to the nitrogen atom;
each occurrence of R is, independently, -(CR³R⁴)-;
each occurrence of R³ and R⁴ are, independently H, halogen, OH, alkyl, alkoxy, -NH₂, R¹⁰, alkylamino, or dialkylamino;
each occurrence of R¹⁰ is independently selected from PEG and polymers based on poly(oxazoline), poly(ethylene oxide), poly(vinyl alcohol), poly(glycerol), poly(N-vinylpyrrolidone), poly[N-(2-hydroxypropyl)methacrylamide] and poly(amino acid)s, wherein (i) the PEG or polymer is linear or branched, (ii) the PEG or polymer is polymerized by n subunits, (iii) n is a number-averaged degree of polymerization between 10 and 200 units, and (iv) the compound of said formula has at most two R¹⁰ groups;
the dashed line to Q is absent or a bond;
when the dashed line to Q is absent then Q is absent or is -O-, -NH-, -S-, - C(O)-, -C(O)O-, -OC(O)-, -C(O)N(R⁴)-, -N(R⁵)C(O)-, -S-S-, -OC(O)O-, -O-N=C(R⁵)-, - C(R⁵)=N-O-, -OC(O)N(R⁵)-, -N(R⁵)C(O)N(R⁵)-, -N(R⁵)C(O)O-, -C(O)S-, -C(S)O- or - C(R⁵)=N-O-C(O)-; or
when the dashed line to Q is a bond then (i) b is 0 and (ii) Q and the tertiary carbon adjacent to it (C*) form a substituted or unsubstituted, mono- or bi-cyclic heterocyclic group having from 5 to 10 ring atoms;
each occurrence of R⁵ is, independently, H or C₁-C₄ alkyl;
M¹ and M² are each, independently, a biodegradable group selected from - OC(O)-, -C(O)O-, -SC(O)-, -C(O)S-, -OC(S)-, -C(S)O-, -S-S-, -C(R⁵)=N-, -N=C(R⁵)-, - C(R⁵)=N-O-, -O-N=C(R⁵)-, -C(O)(NR⁵)-, -N(R⁵)C(O)-, -C(S)(NR⁵)-, -N(R⁵)C(O)-, - N(R⁵)C(O)N(R⁵)-, -OC(O)O-, -OSi(R⁵)₂O-, -C(O)(CR³R⁴)C(O)O-, and - OC(O)(CR³R⁴)C(O)-, or 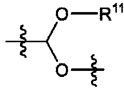 wherein R¹¹ is a C₂-C₈ alkyl or alkenyl;
each occurrence of R^{z} is, independently, C₁-C₈ alkyl;
   a is 1, 2, 3, 4, 5 or 6;
   b is 0, 1, 2, or 3;
   L¹ and L² are each, independently, C₁-C₅ alkylene or C₂-C₅ alkenylene;
   X and Y are each, independently, alkylene or alkenylene; and
   Z¹ and Z² are each, independently, C₈-C₁₄ alkyl or C₈-C₁₄ alkenyl, wherein the alkenyl group may optionally be substituted with one or two fluorine atoms at the alpha position to a double bond which is between the double bond and the terminus of Z¹ or Z², and with the proviso that the terminus of at least one of Z¹ and Z² is separated from the group M¹ or M² by at least 8 carbon atoms.

In yet another embodiment, the cationic lipid selected from the compounds:

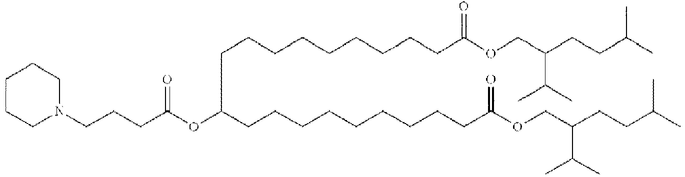

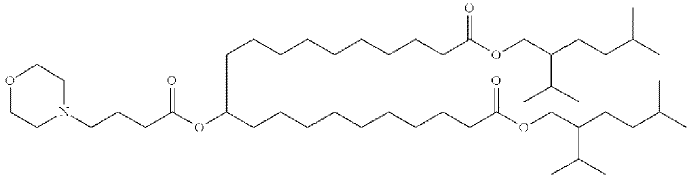

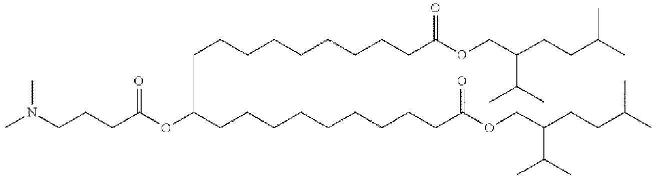

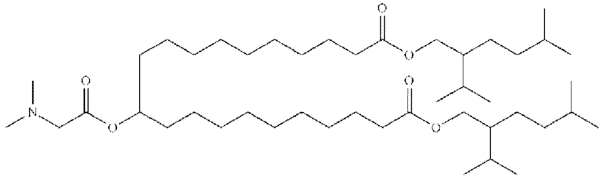

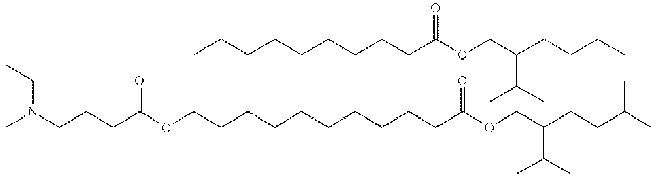

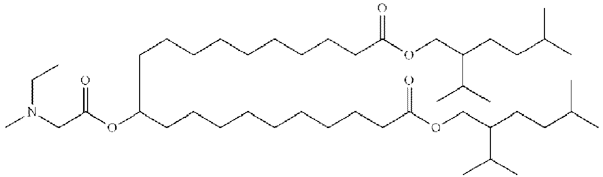

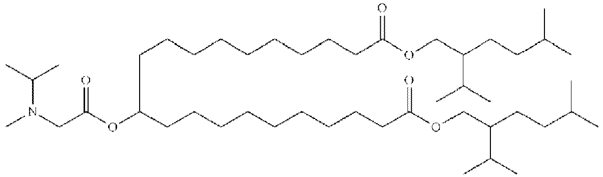

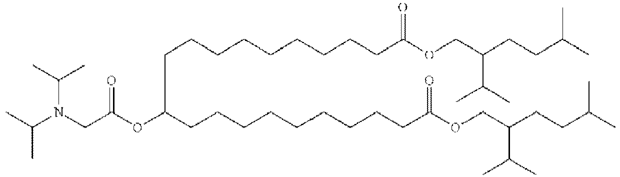

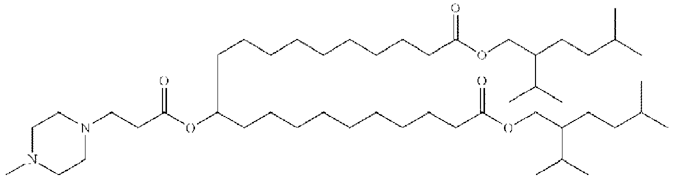

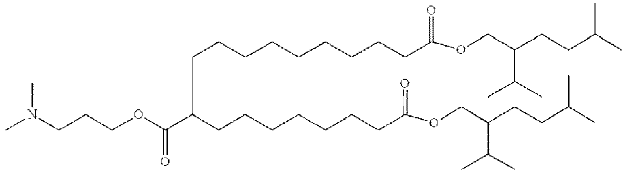

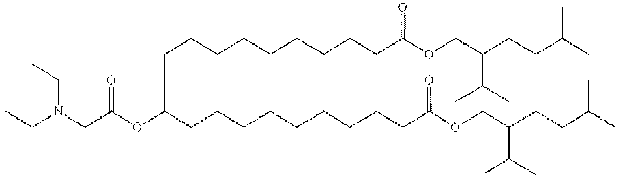

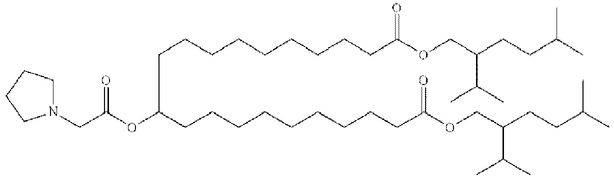

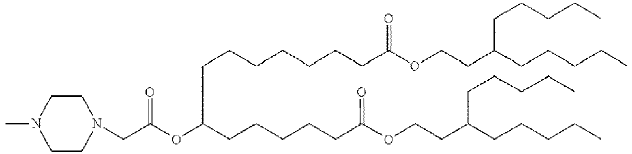

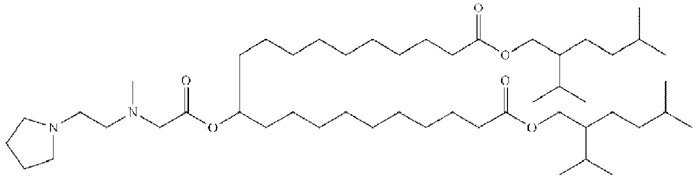

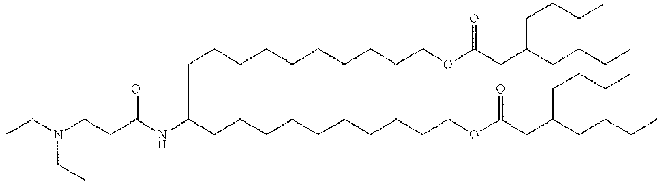

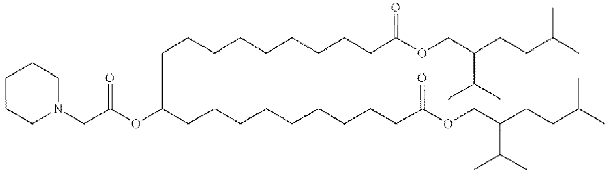

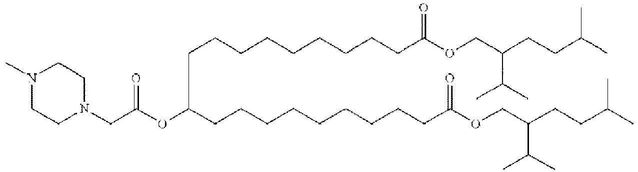

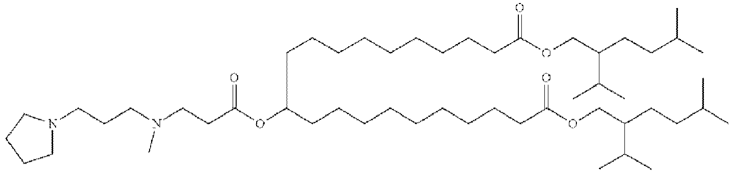

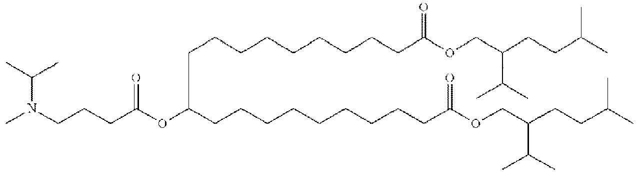

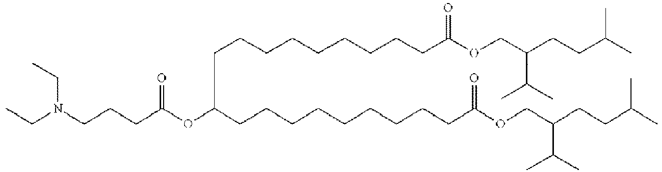

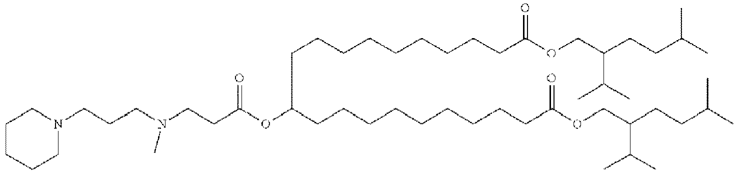

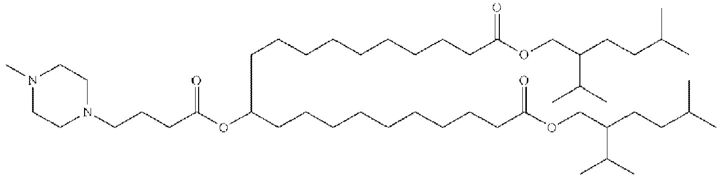

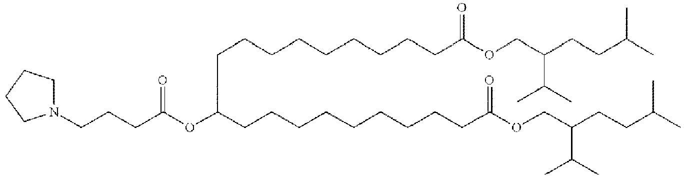

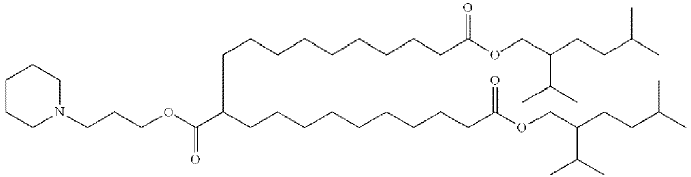

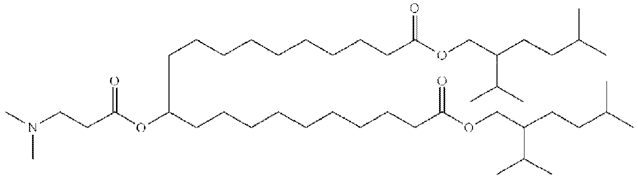

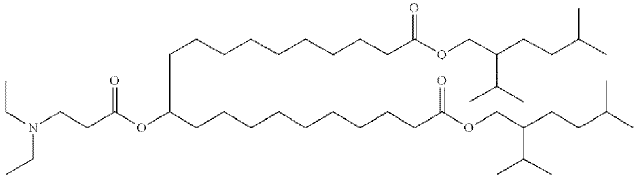

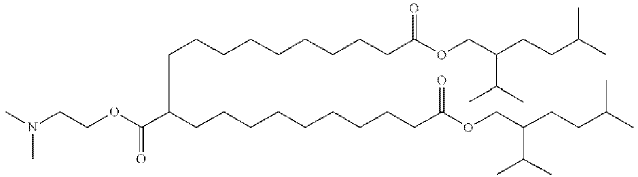

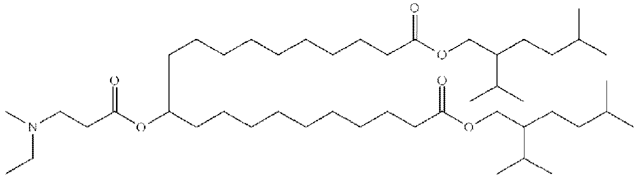

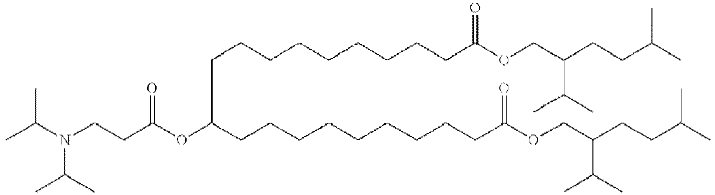

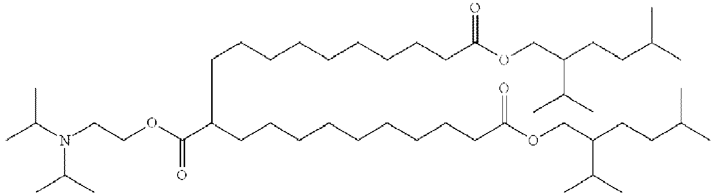

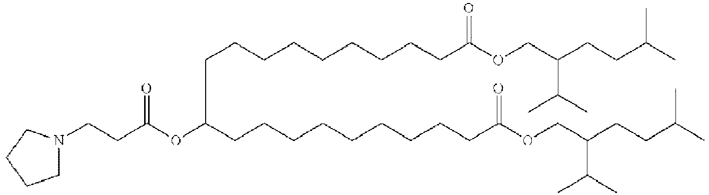

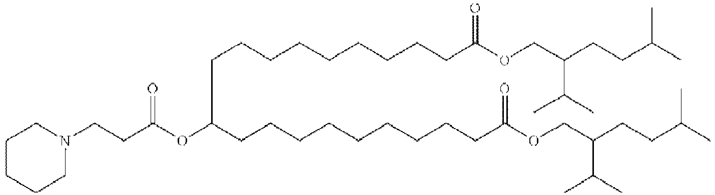

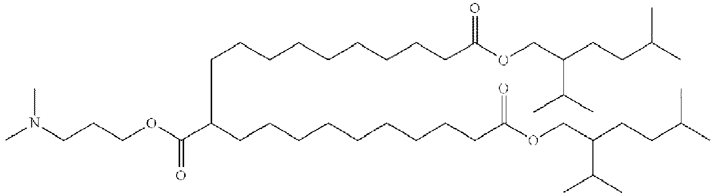

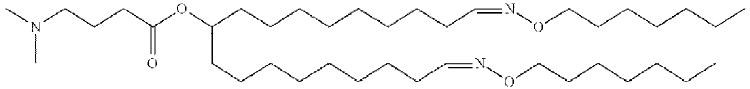

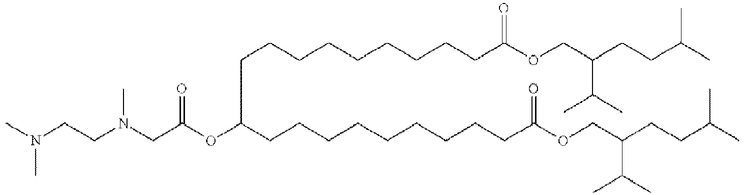

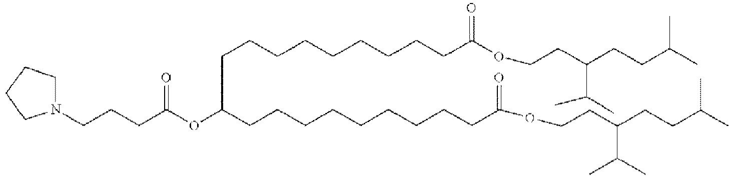

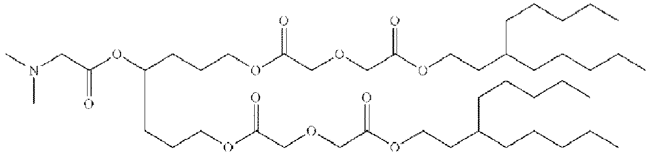

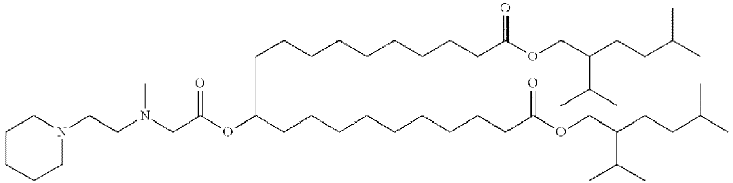

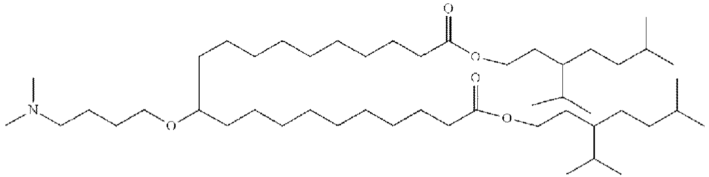

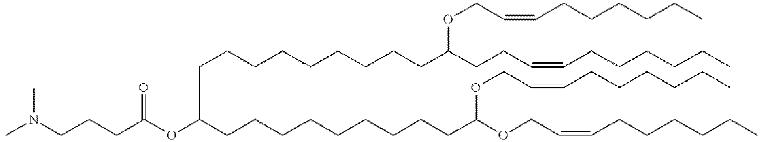

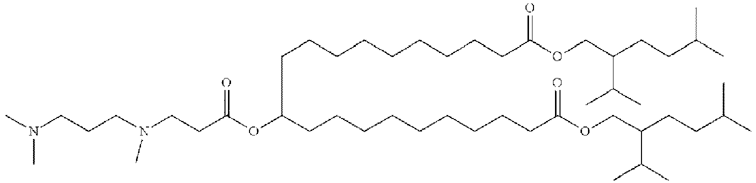

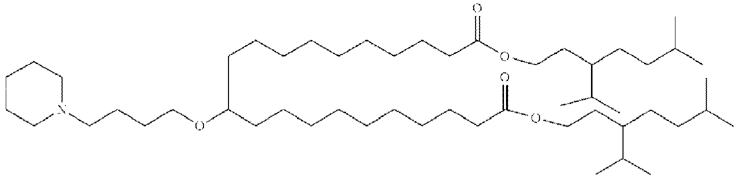

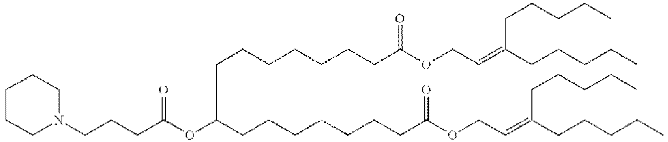

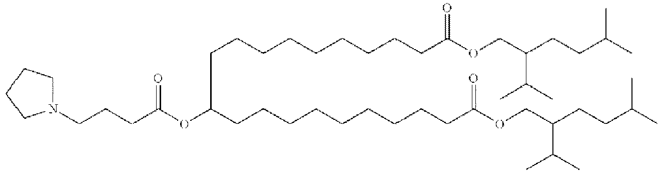

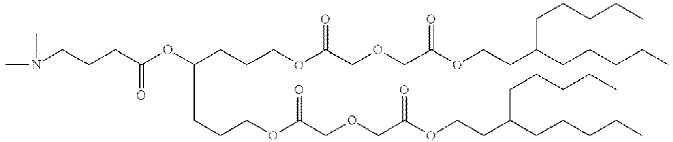

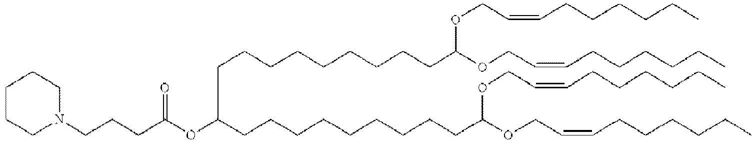

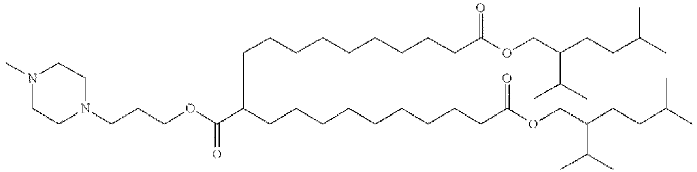

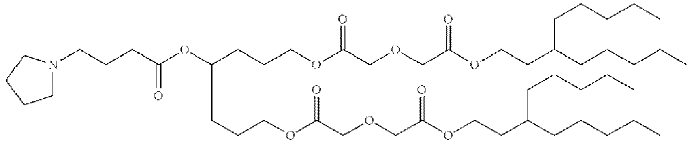

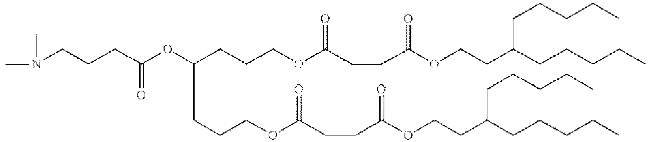

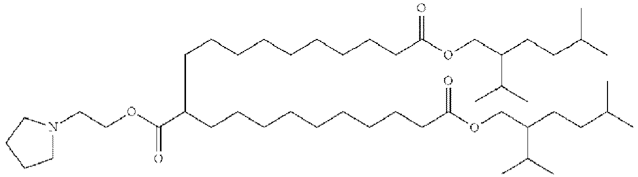

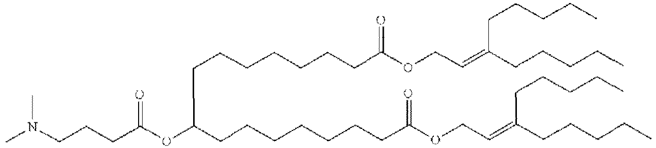

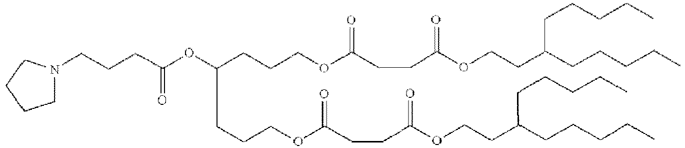

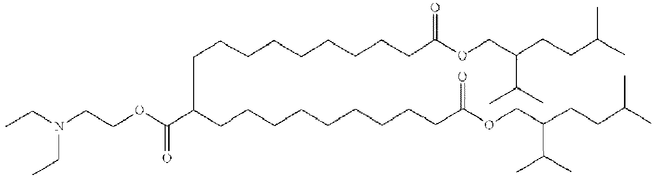

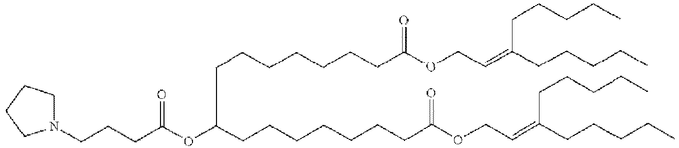

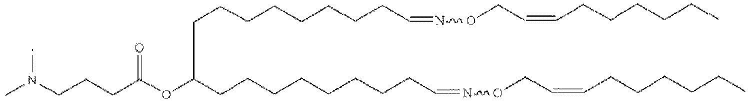

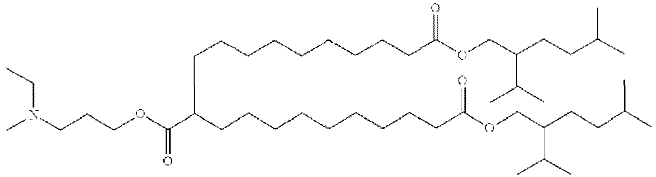

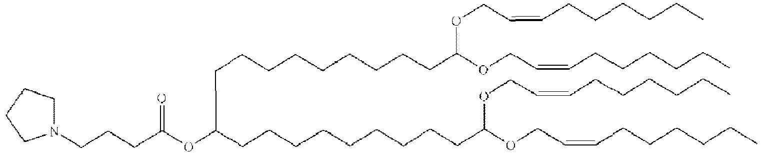

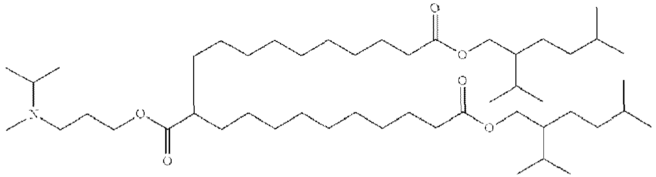

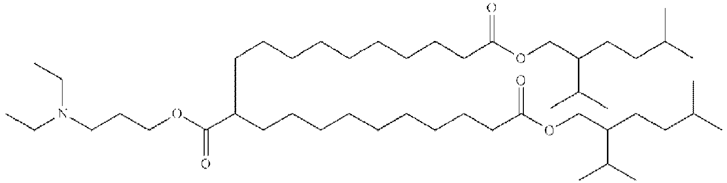

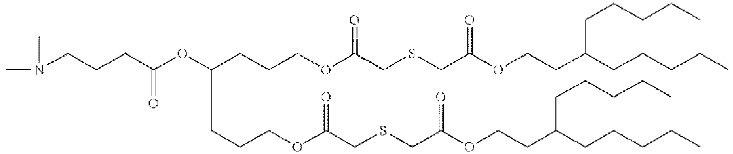

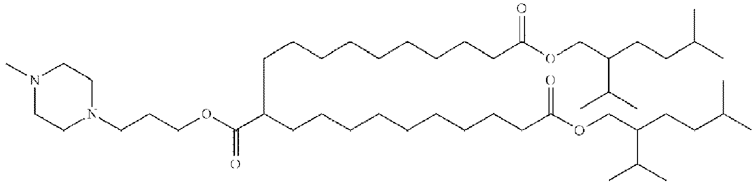

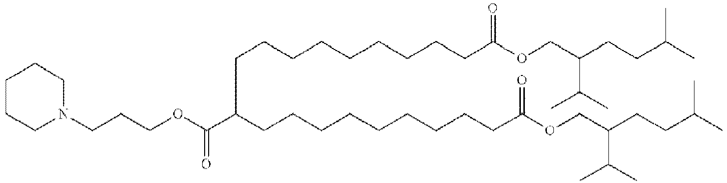

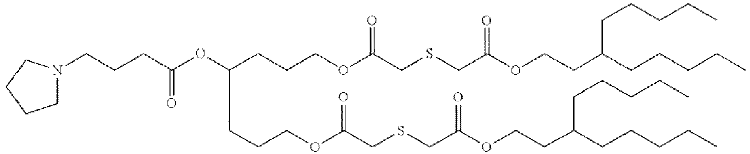

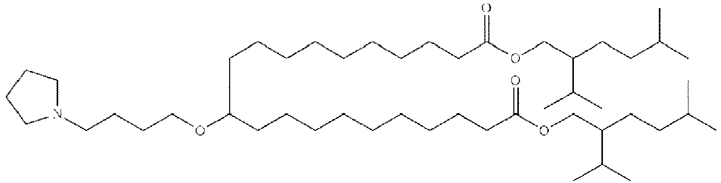

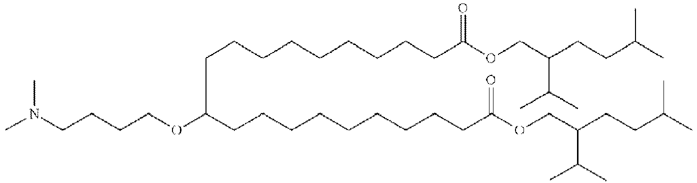

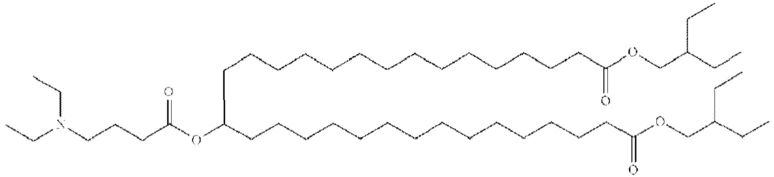

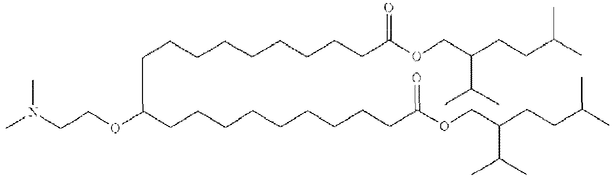

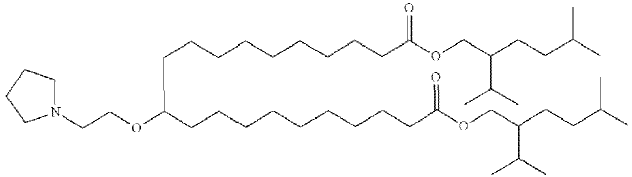

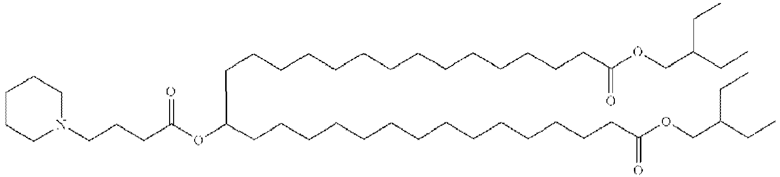

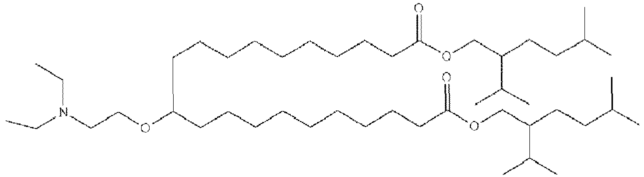

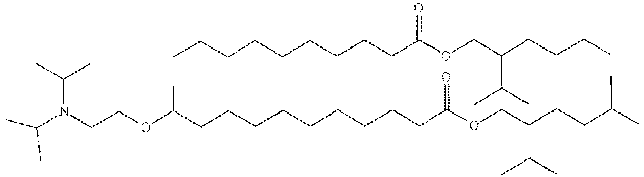

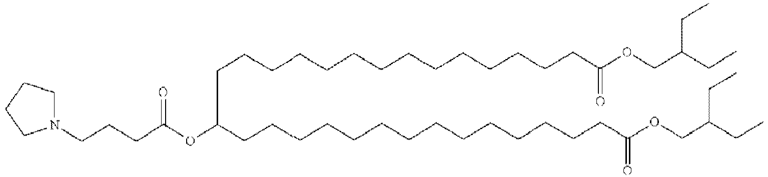

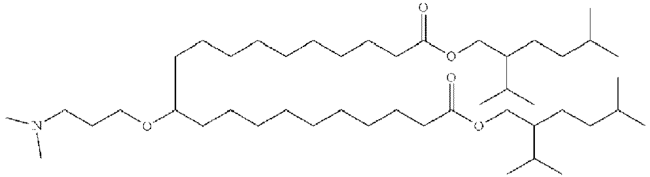

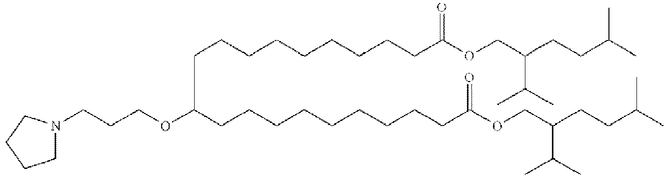

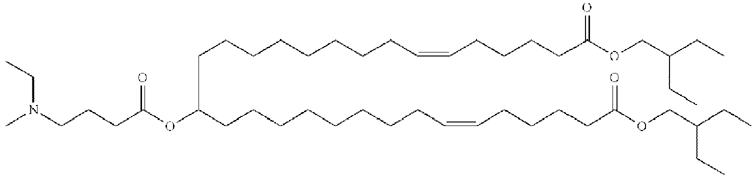

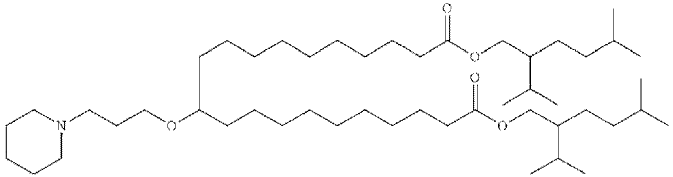

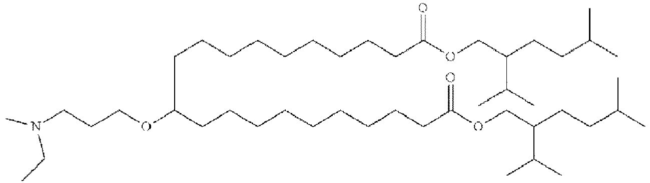

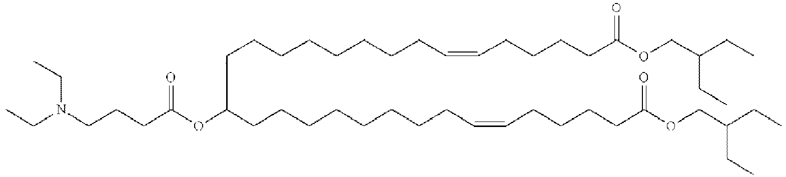

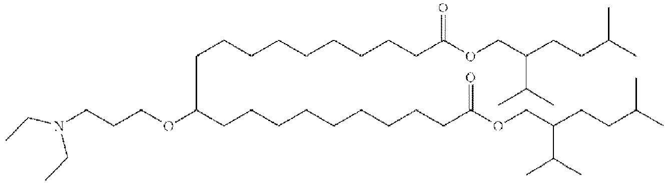

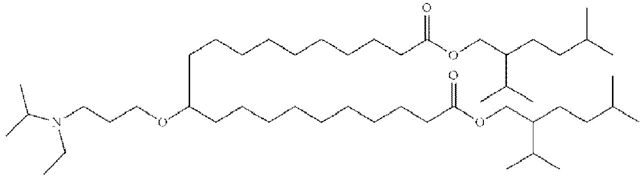

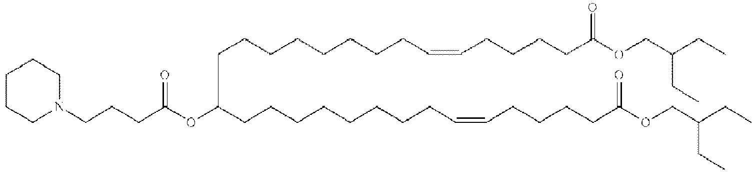

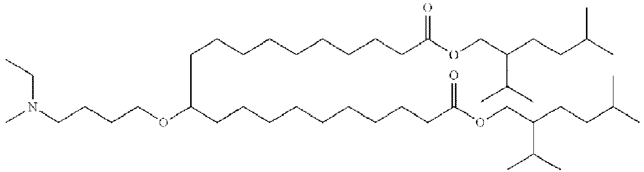

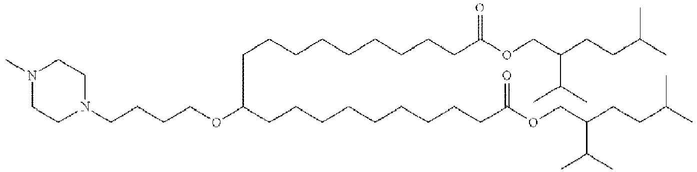

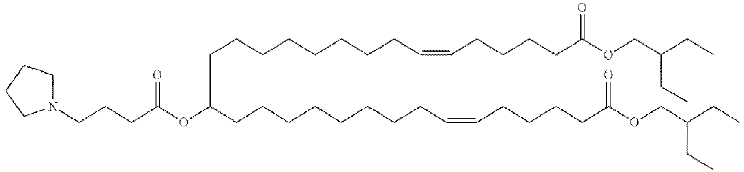

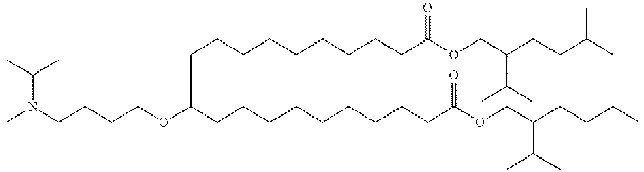

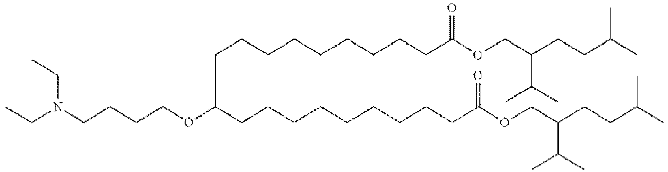

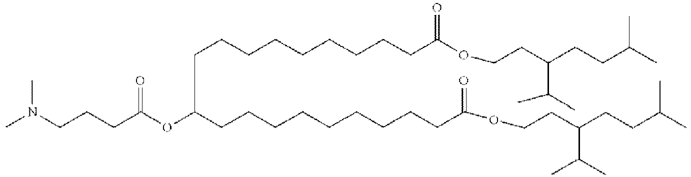

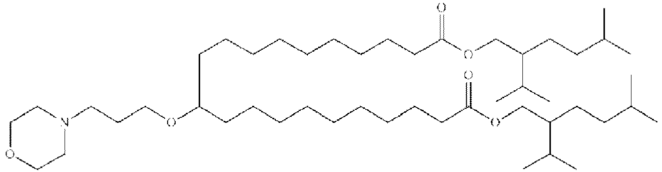

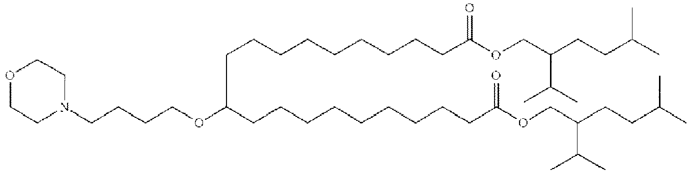

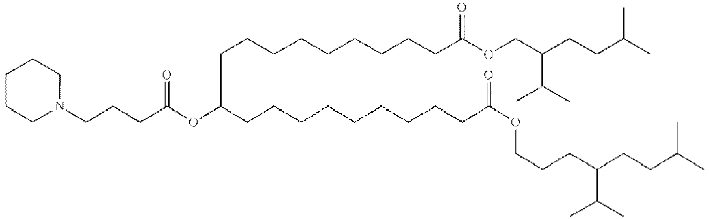

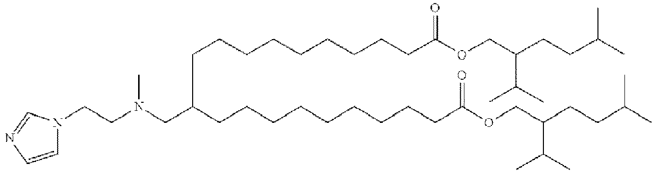

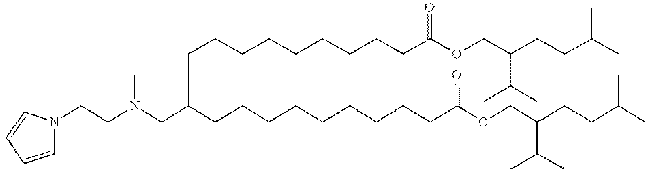

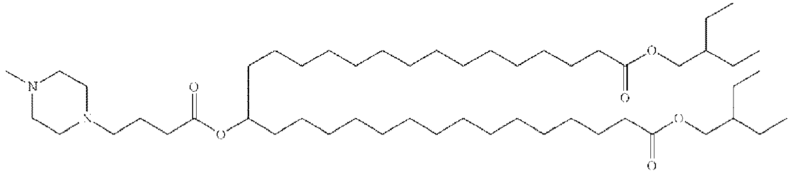

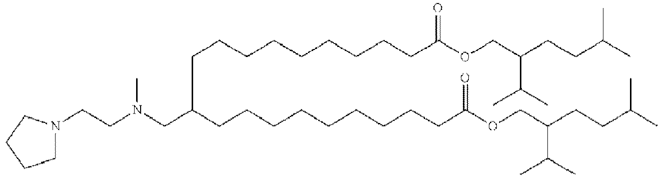

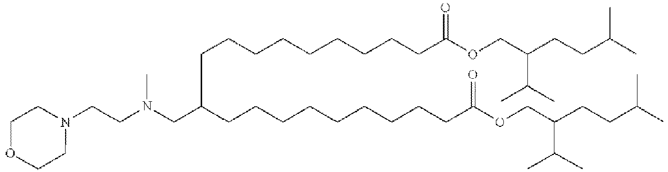

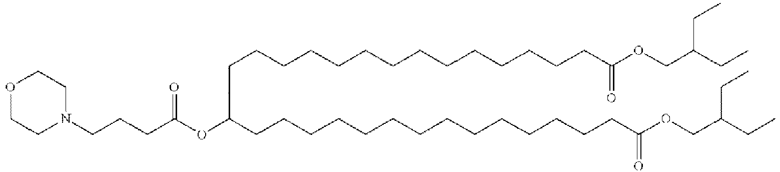

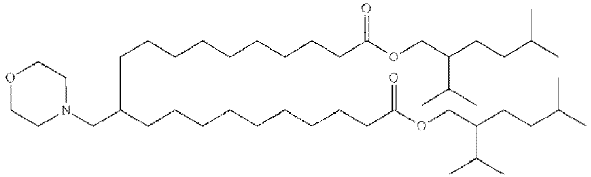

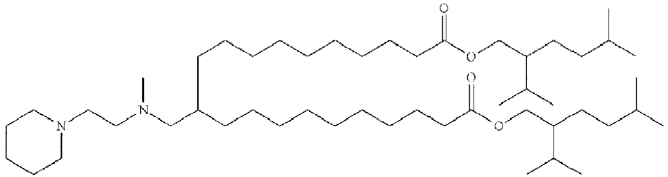

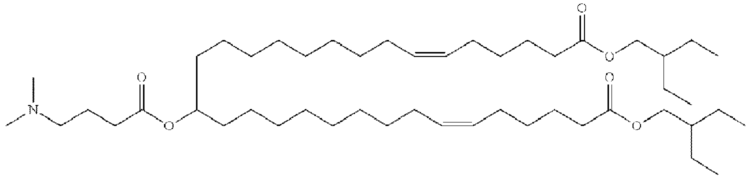

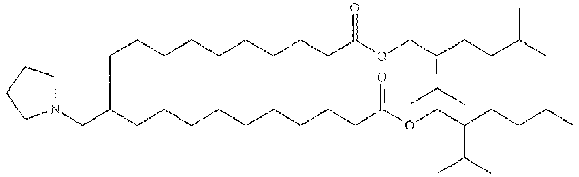

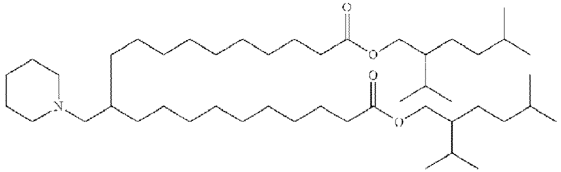

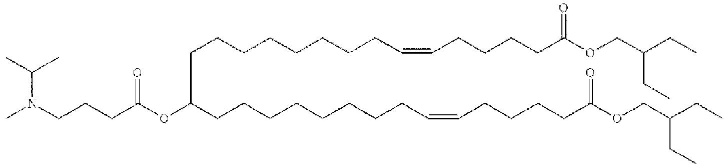

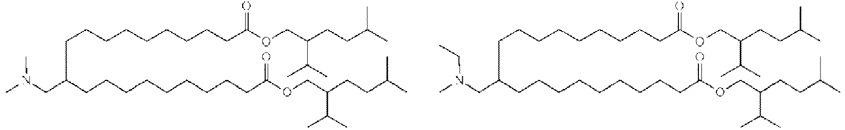

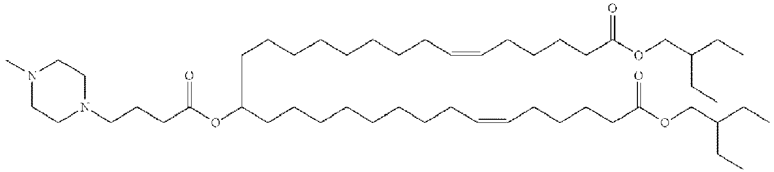

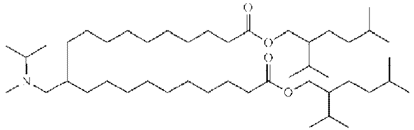

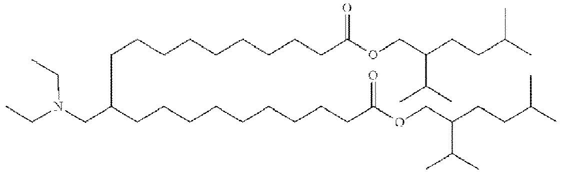

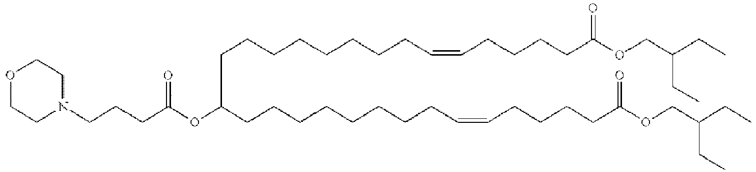

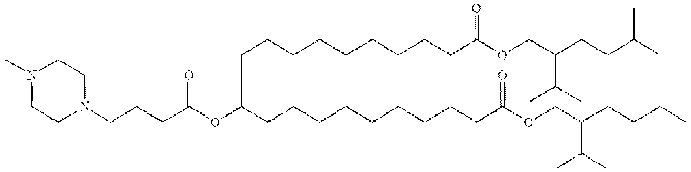

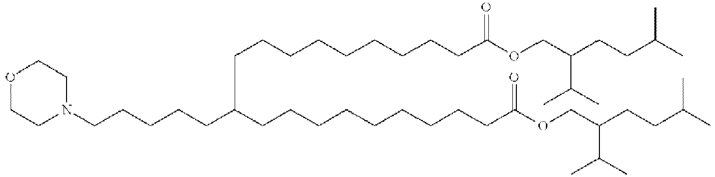

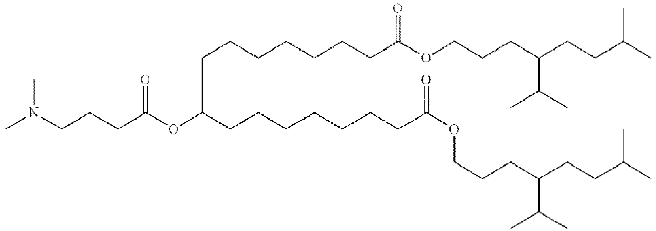

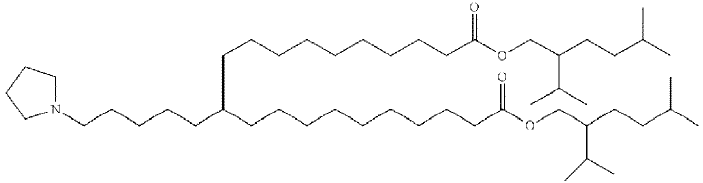

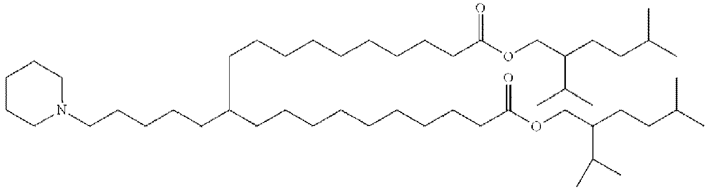

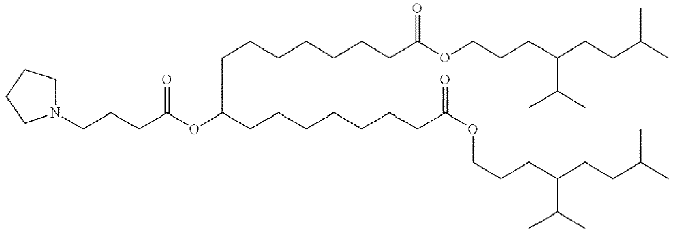

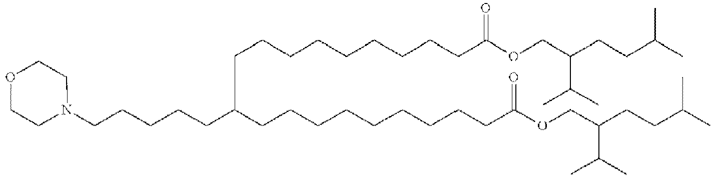

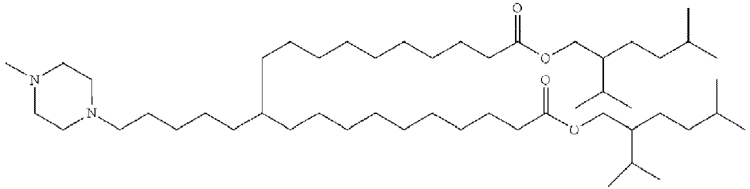

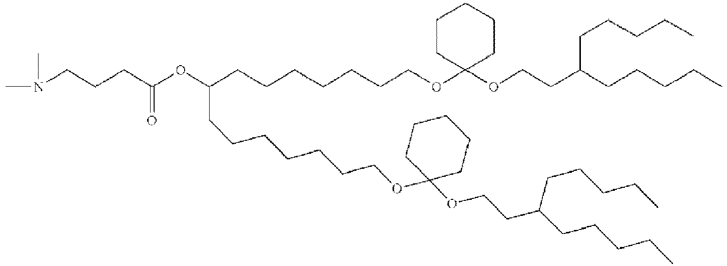

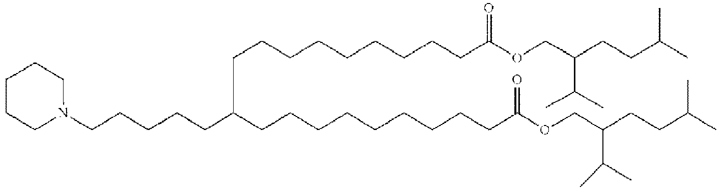

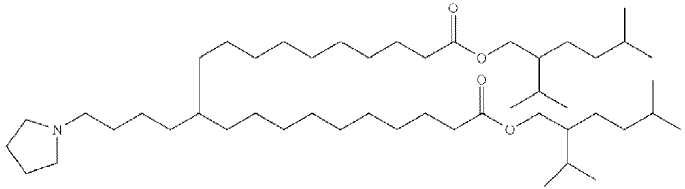

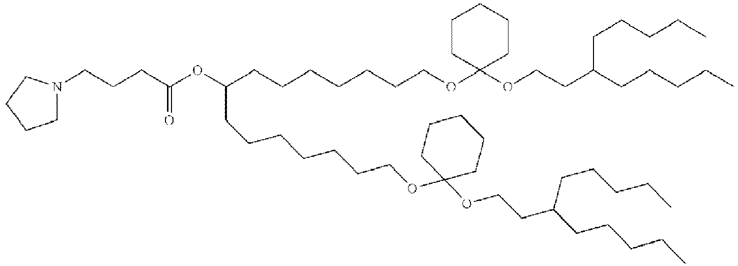

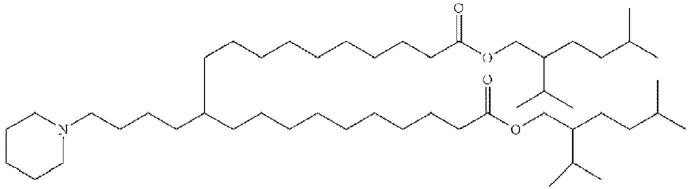

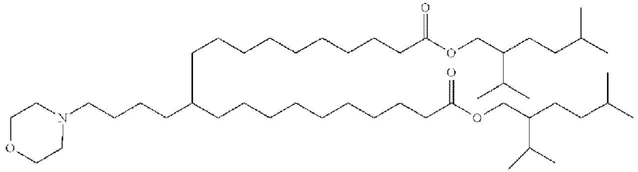

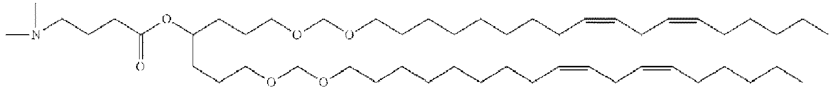

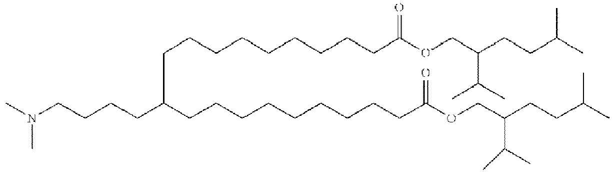

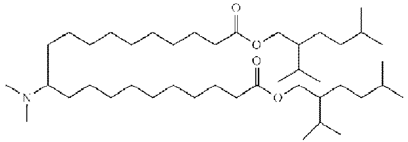

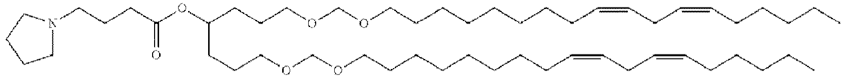

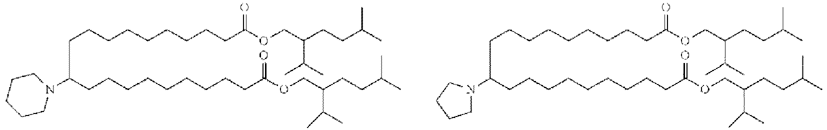

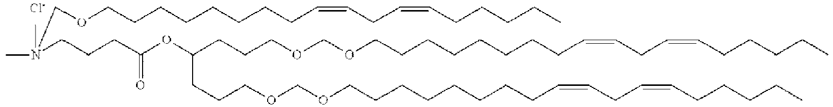

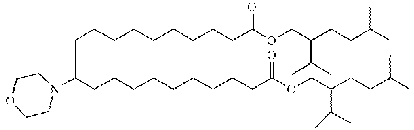

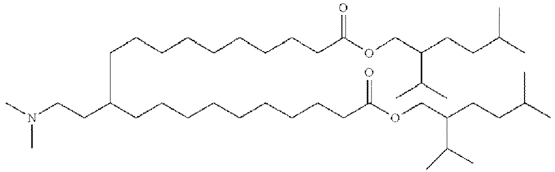

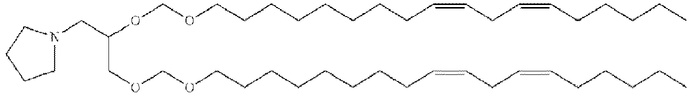

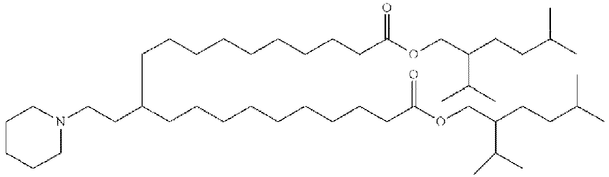

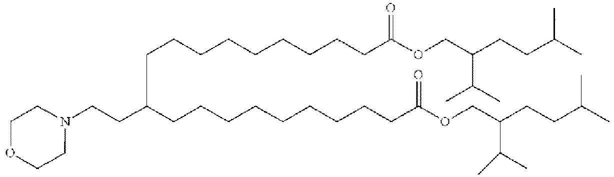

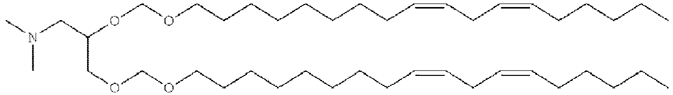

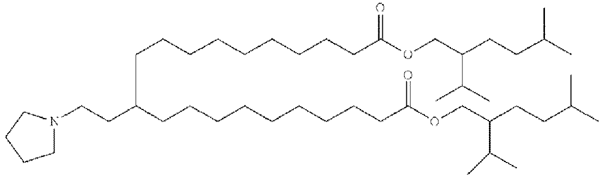

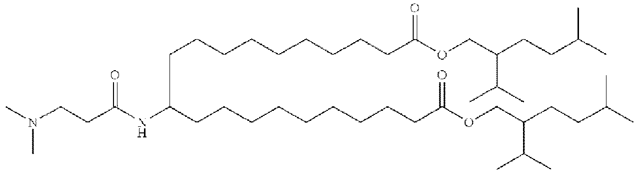

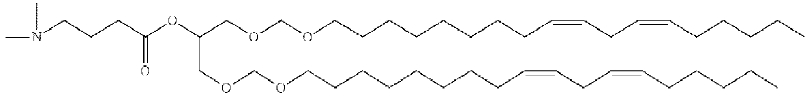

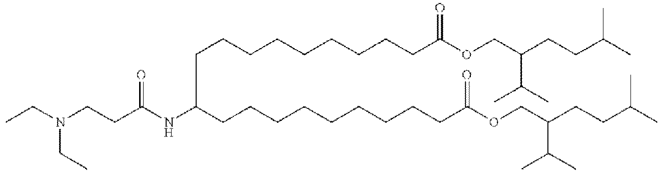

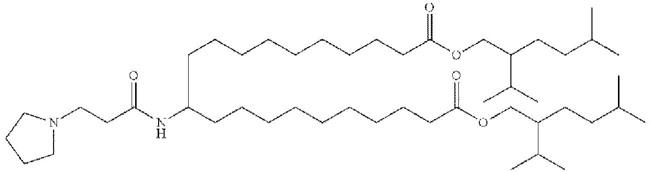

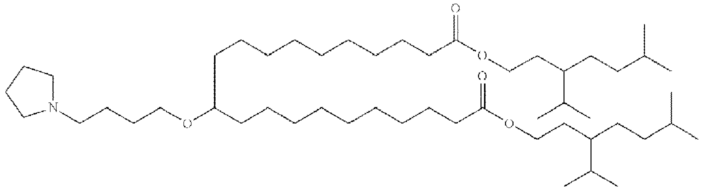

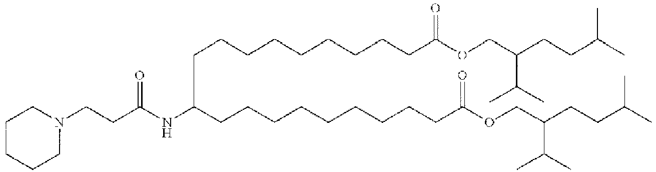

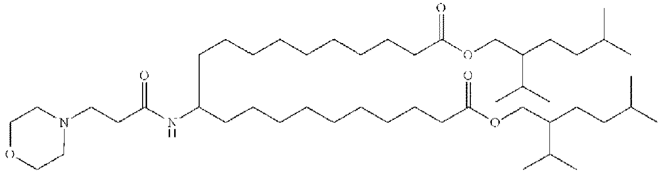

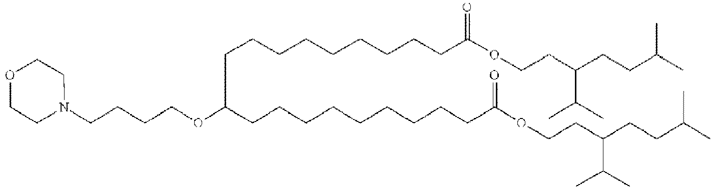

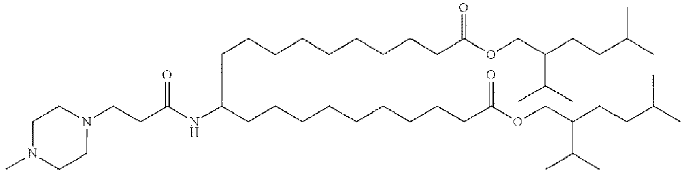

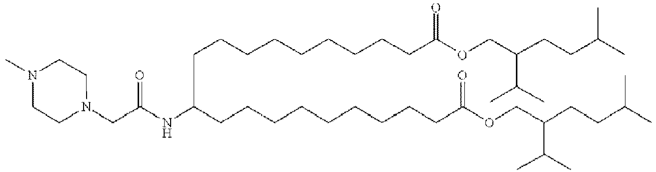

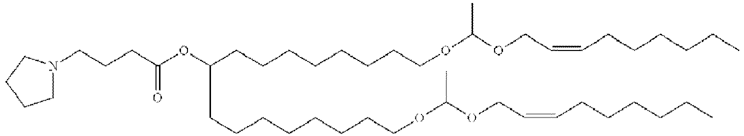

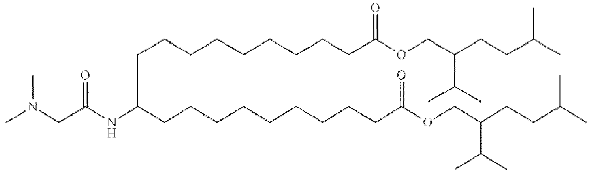

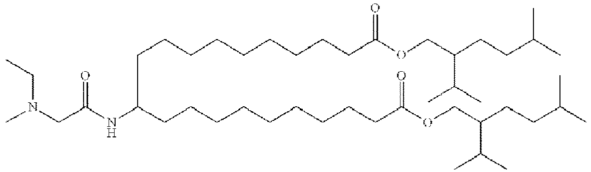

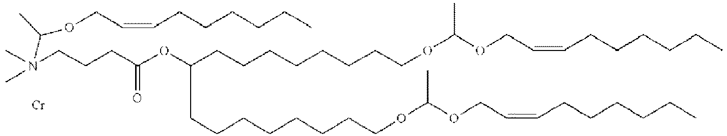

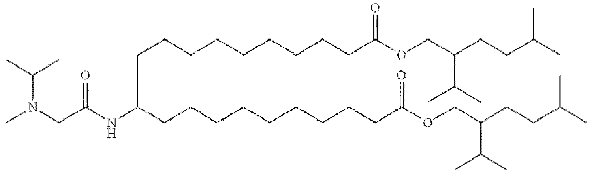

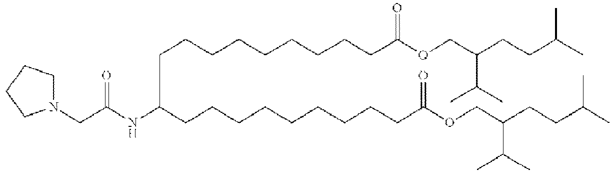

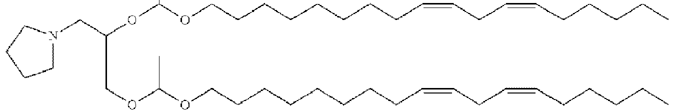

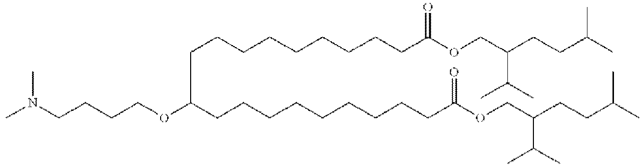

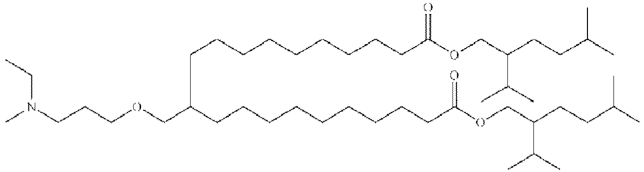

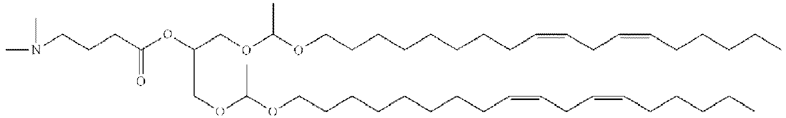

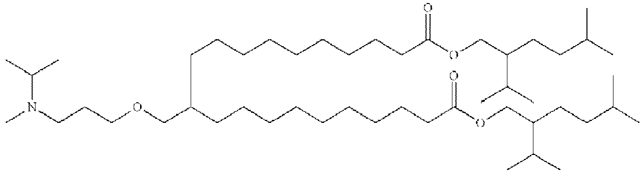

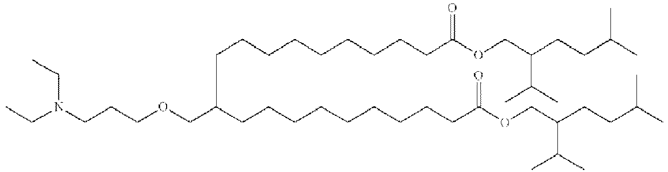

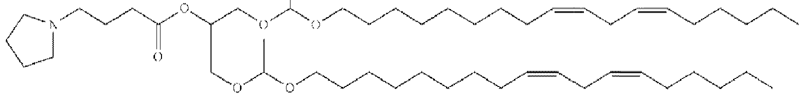

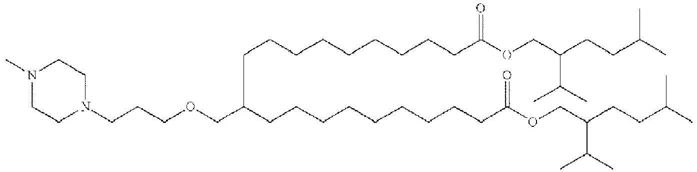

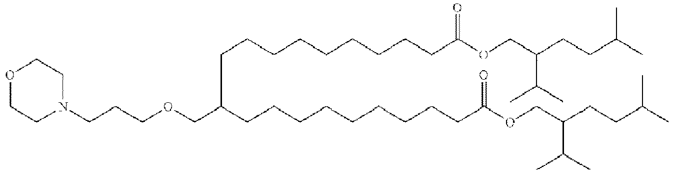

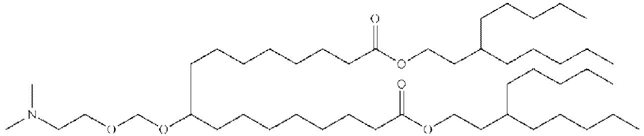

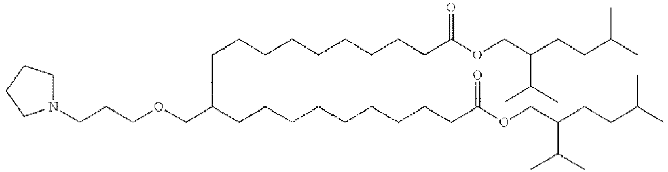

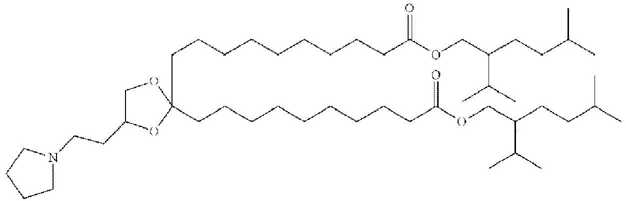

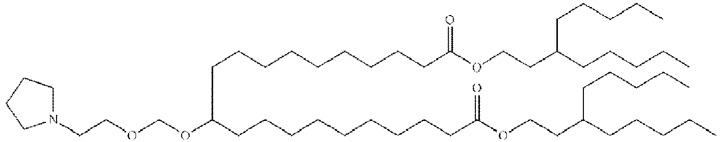

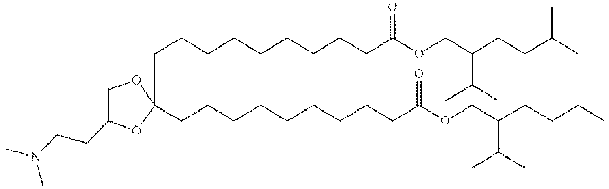

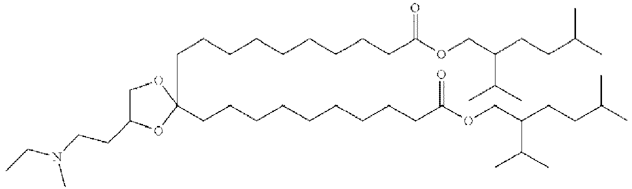

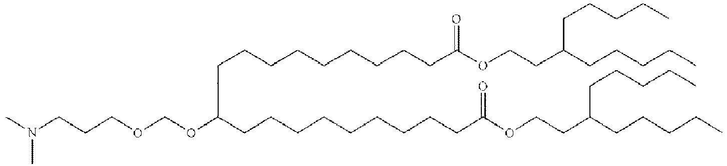

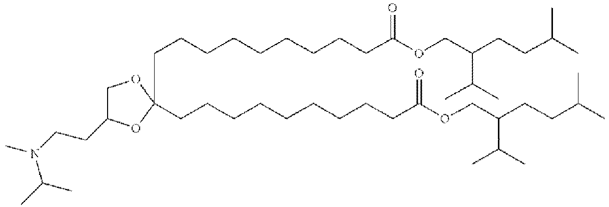

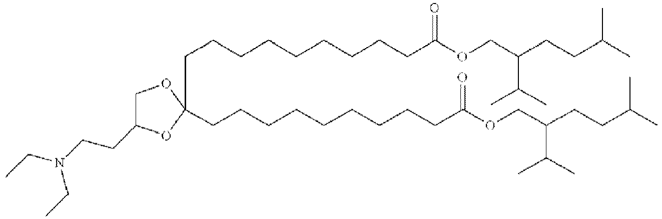

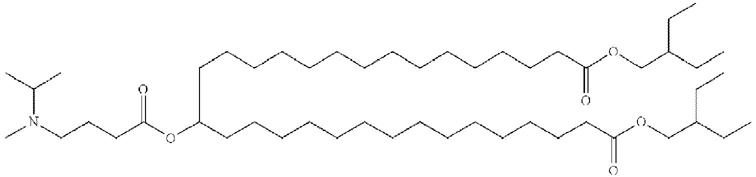

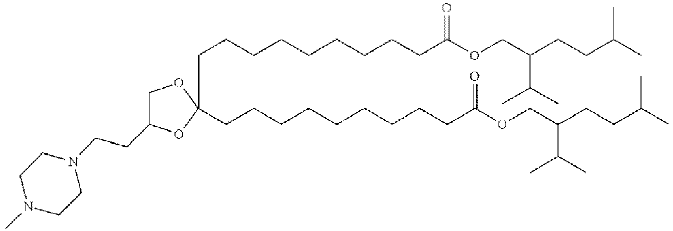

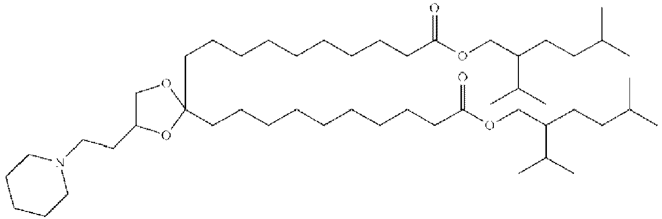

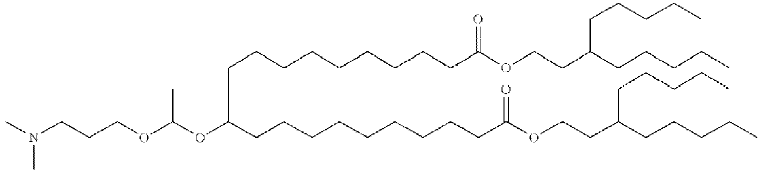

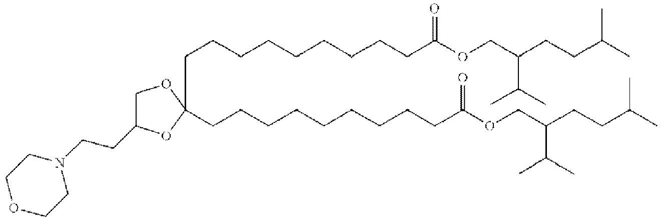

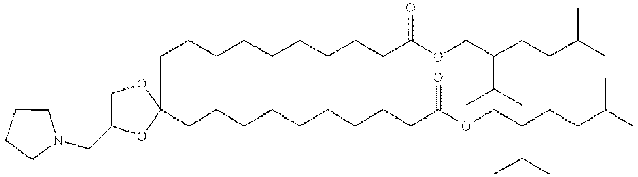

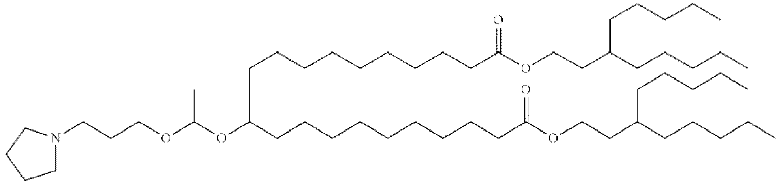

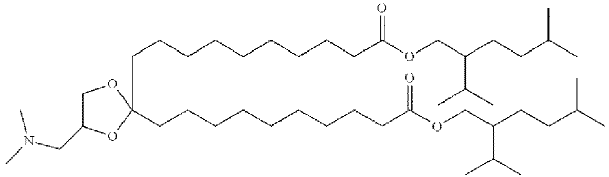

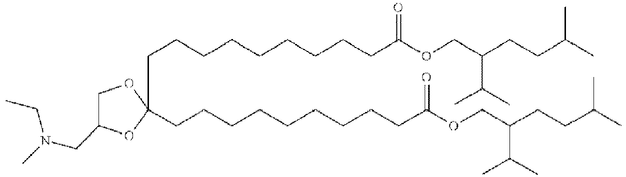

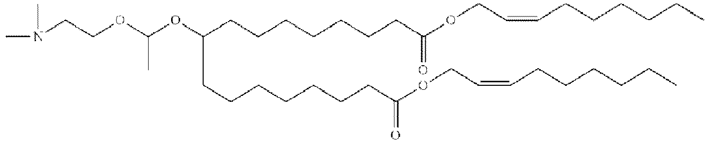

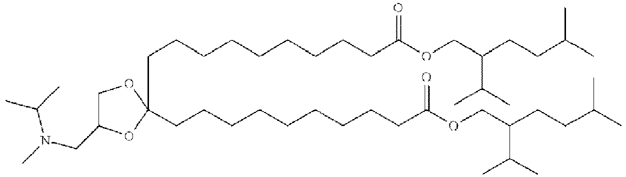

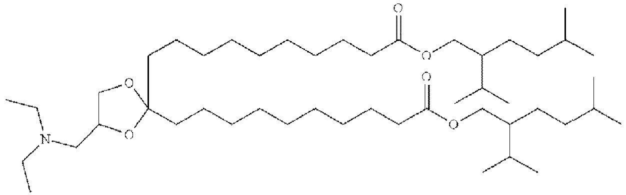

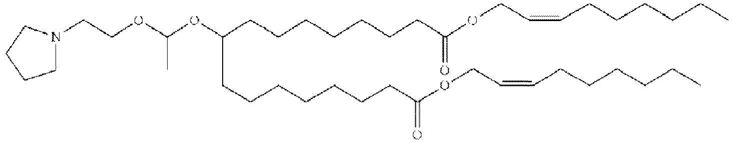

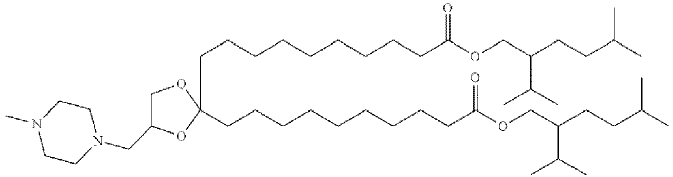

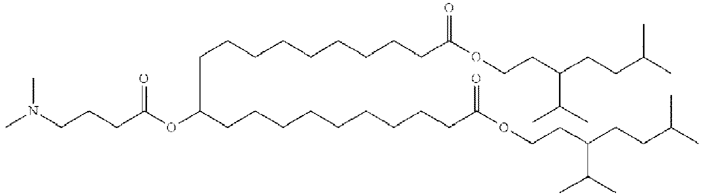

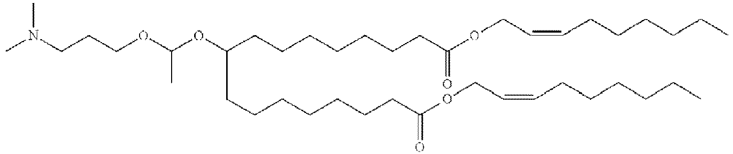

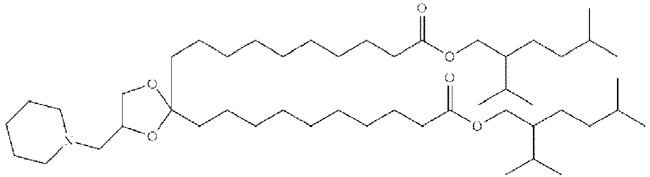

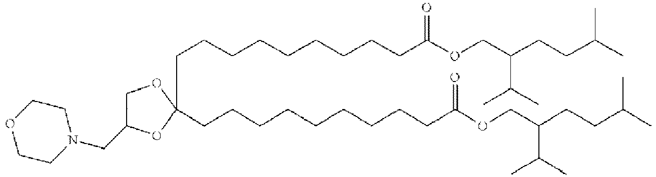

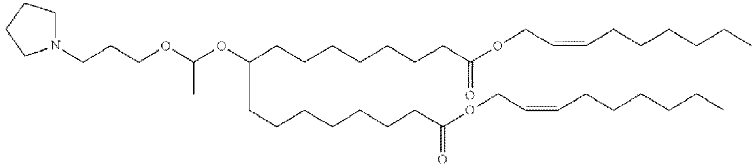

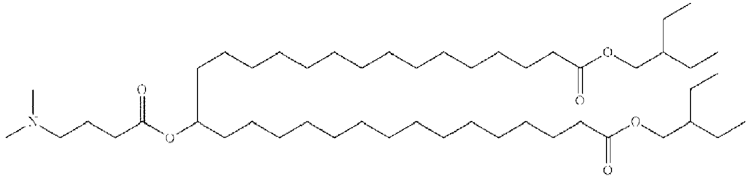

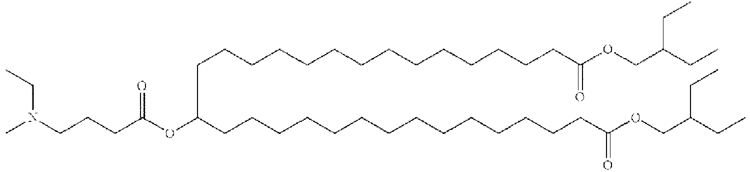

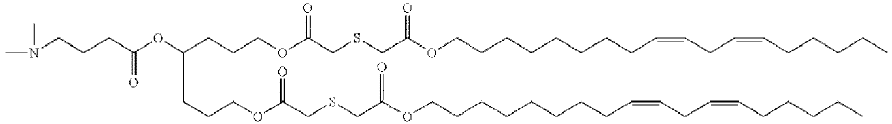

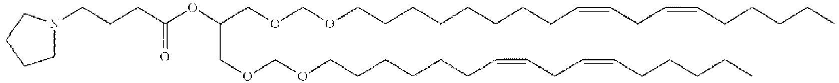

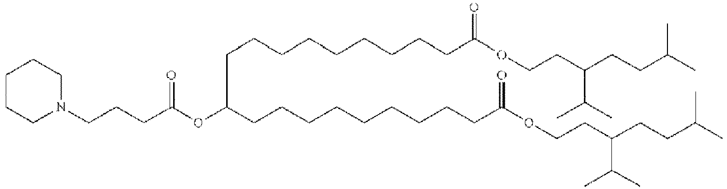

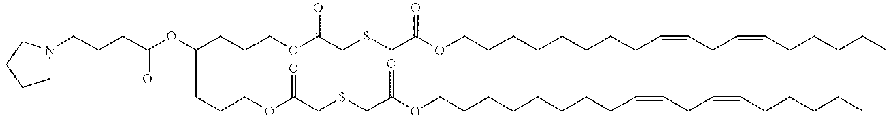

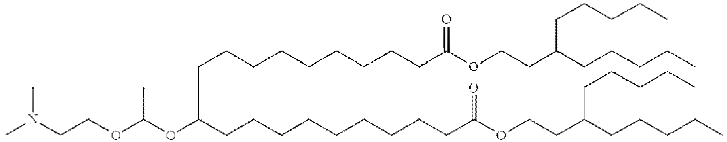

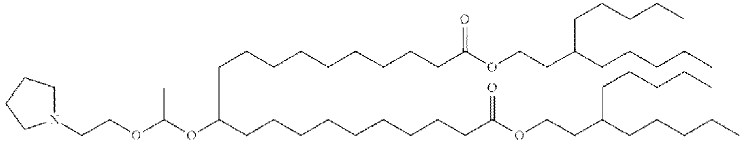

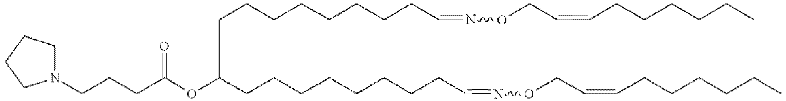

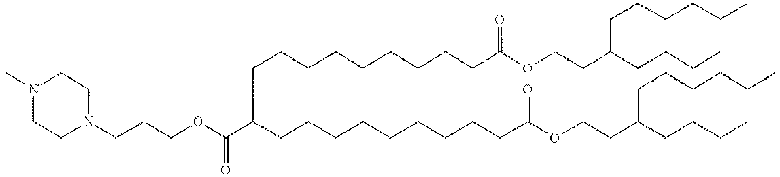

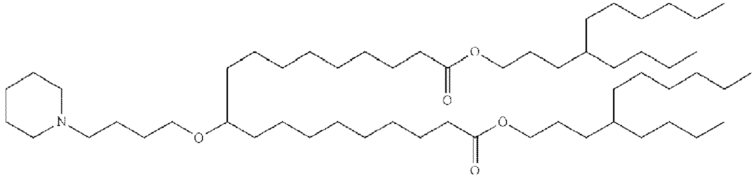

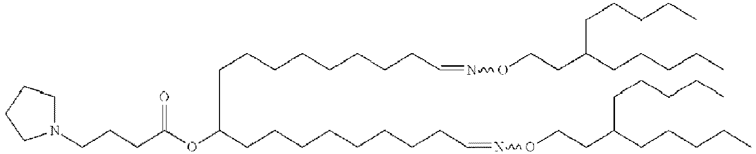

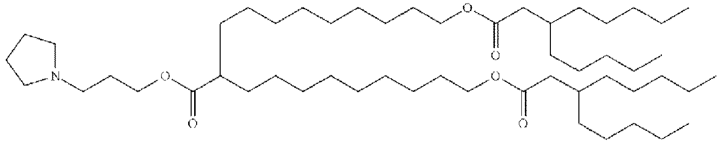

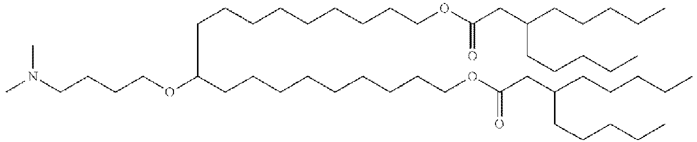

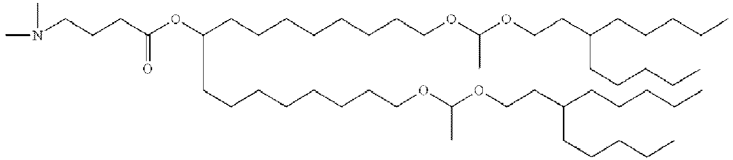

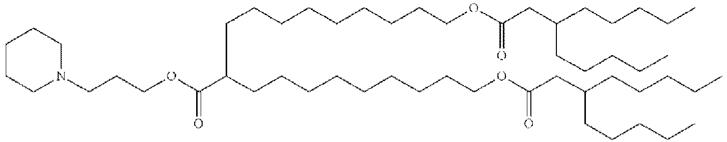

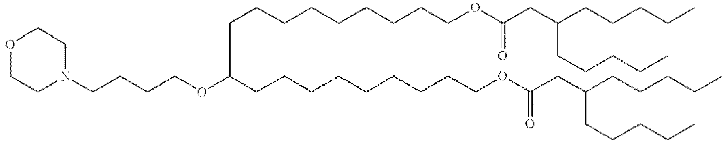

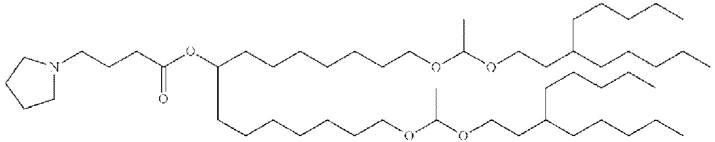

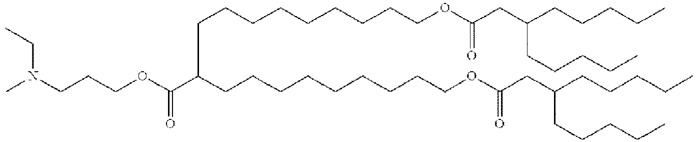

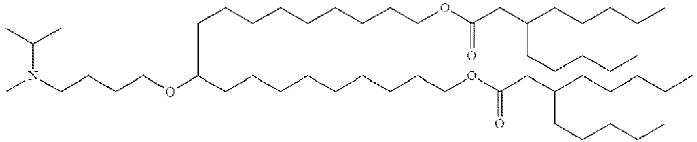

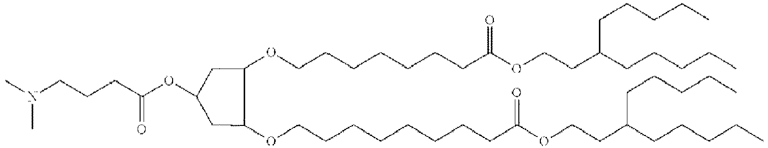

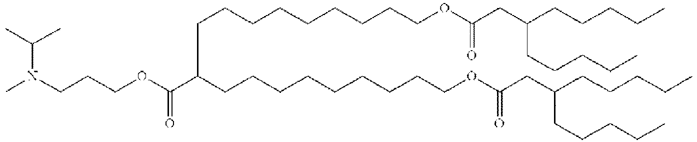

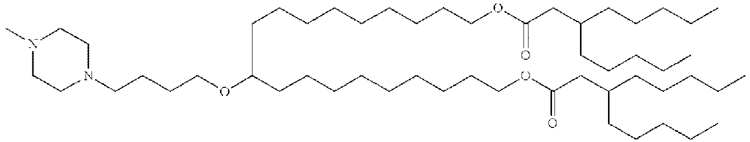

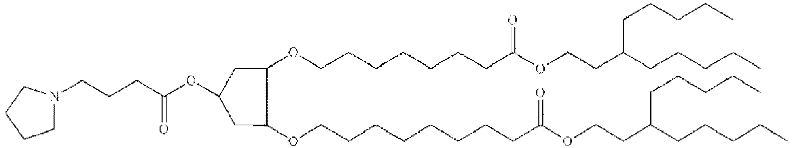

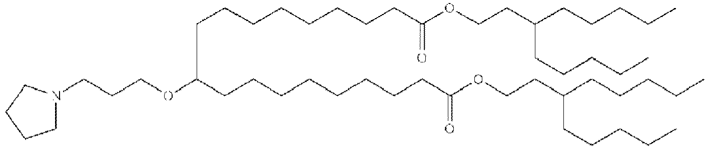

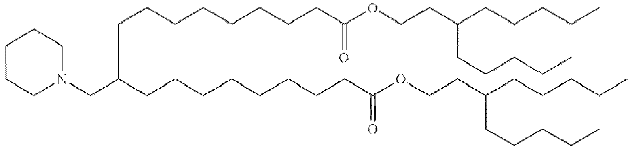

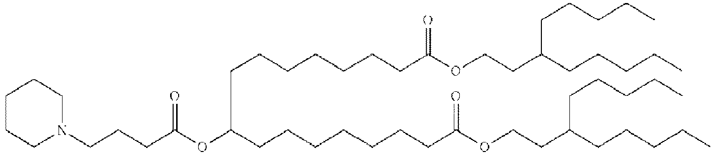

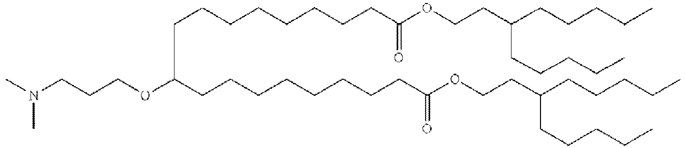

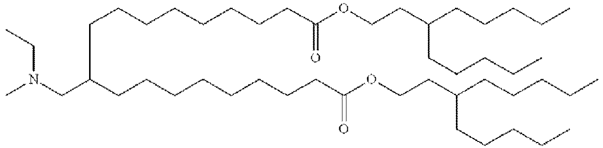

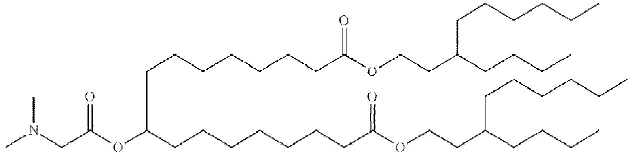

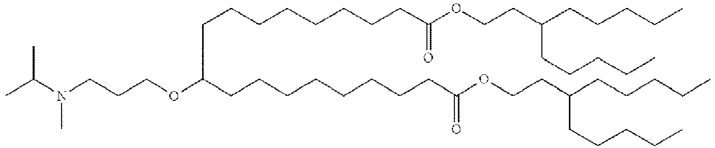

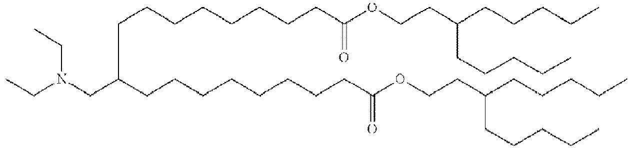

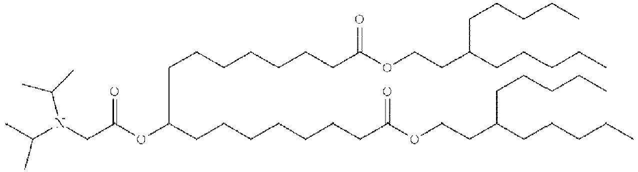

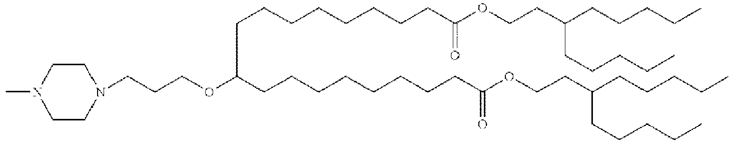

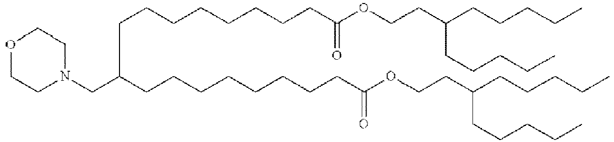

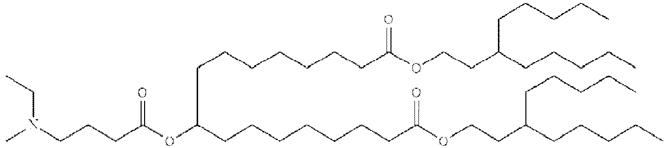

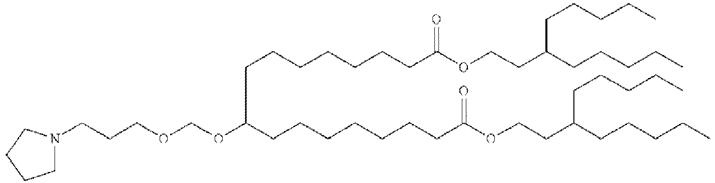

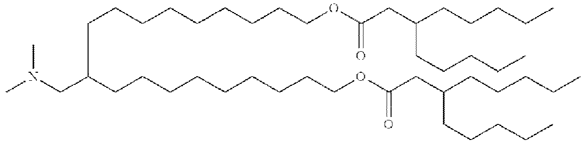

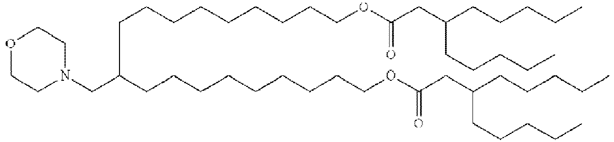

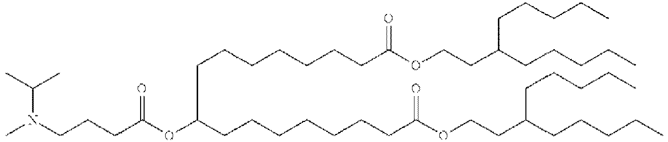

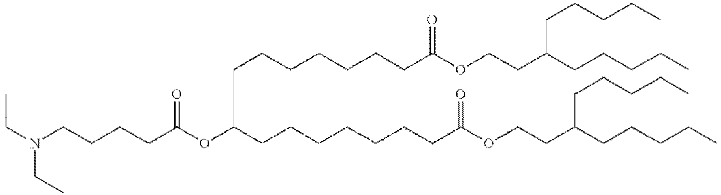

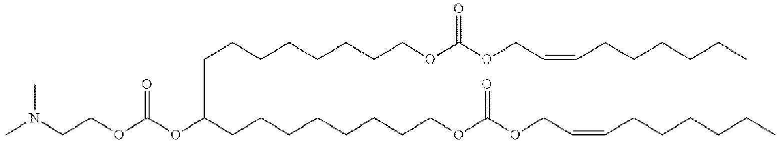

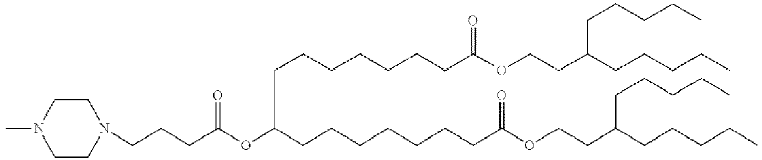

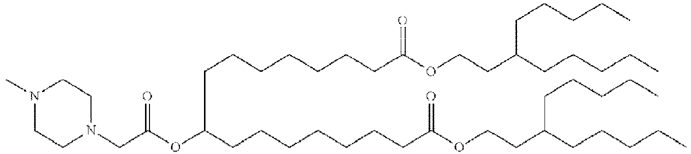

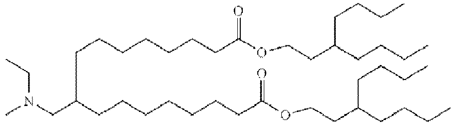

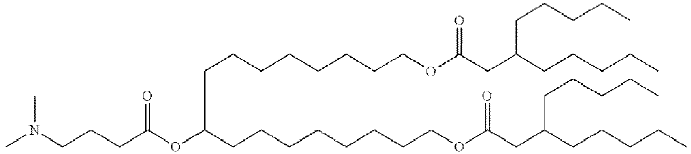

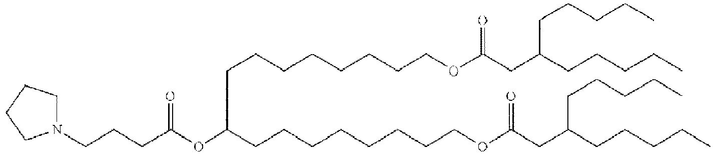

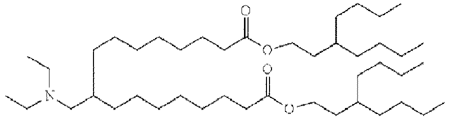

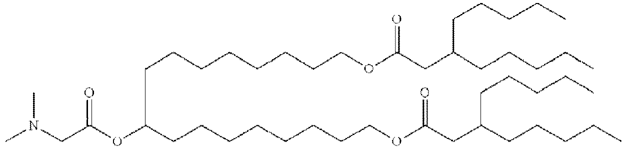

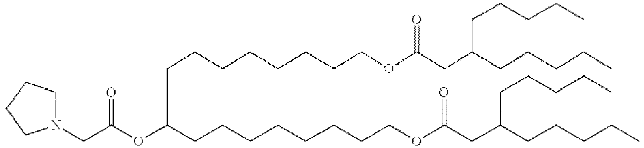

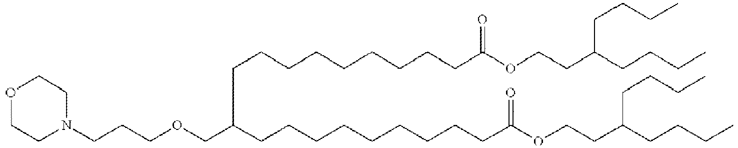

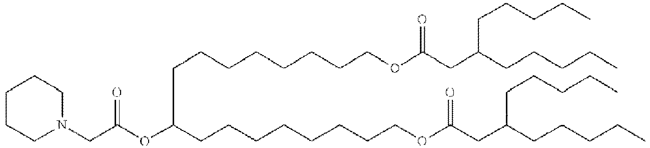

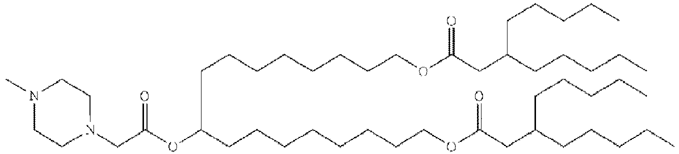

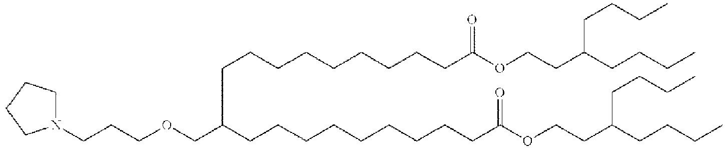

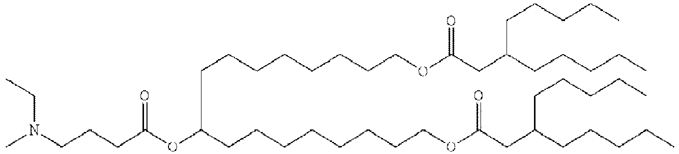

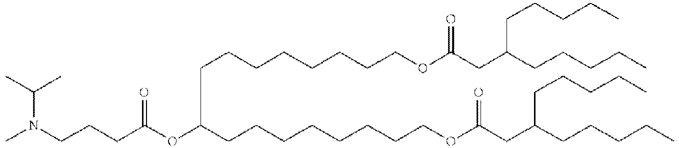

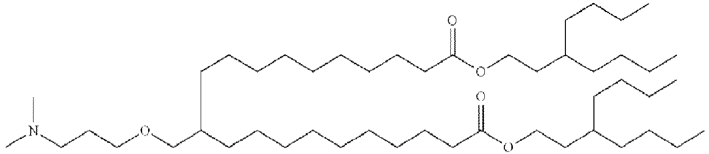

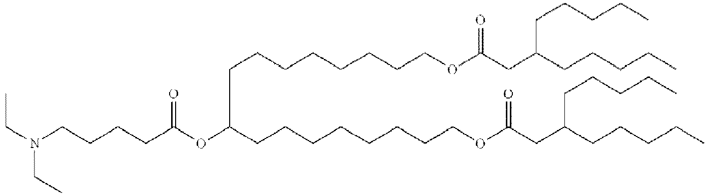

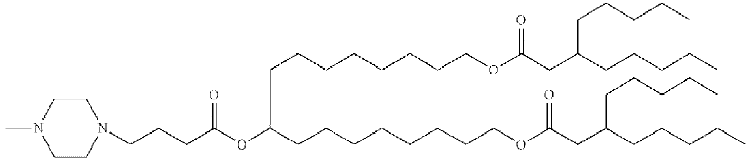

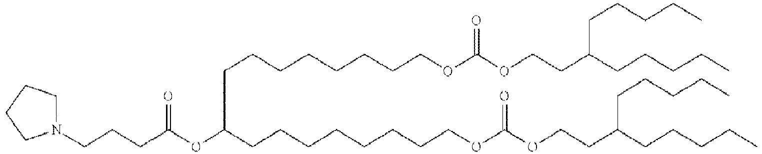

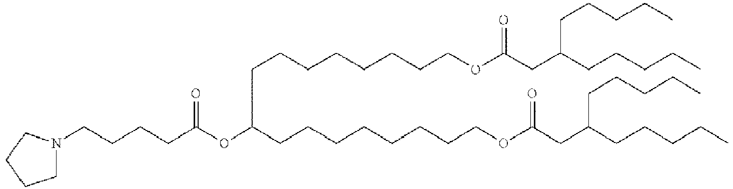

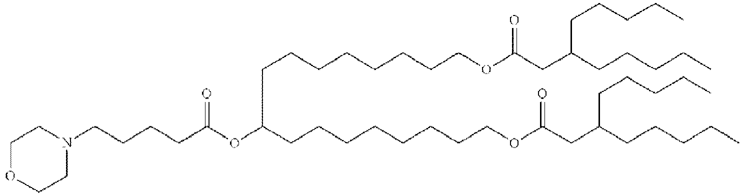

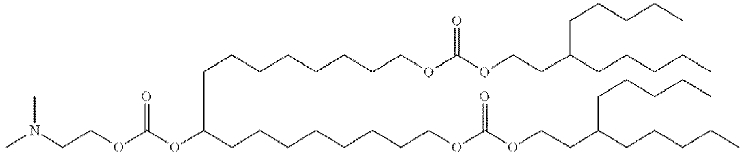

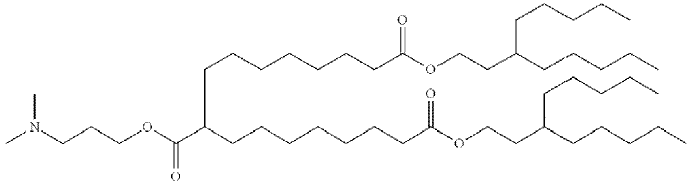

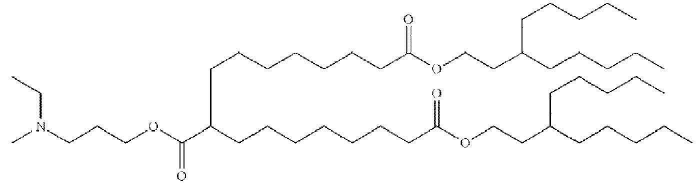

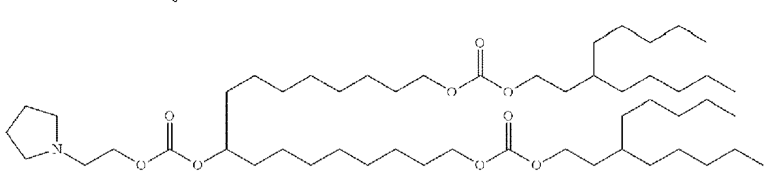

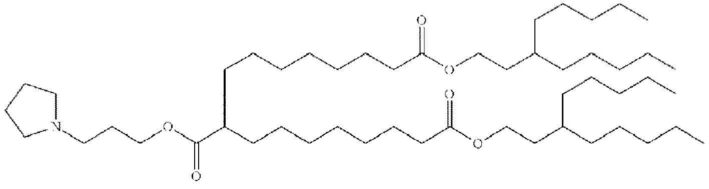

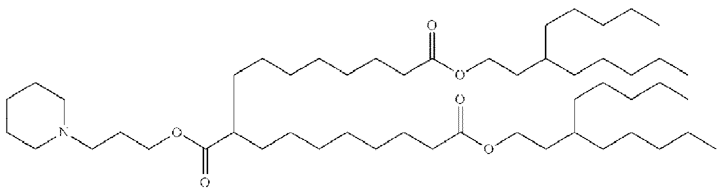

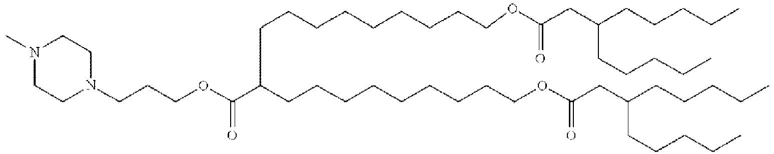

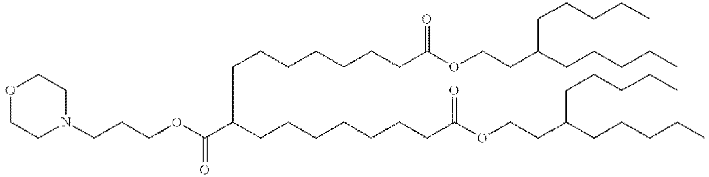

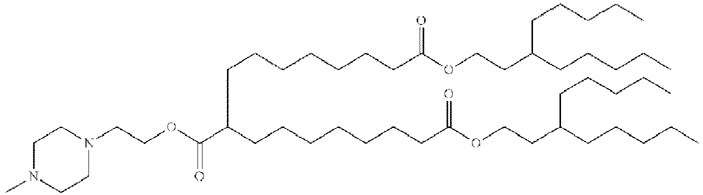

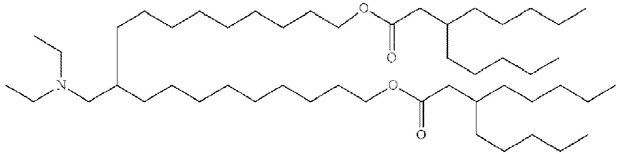

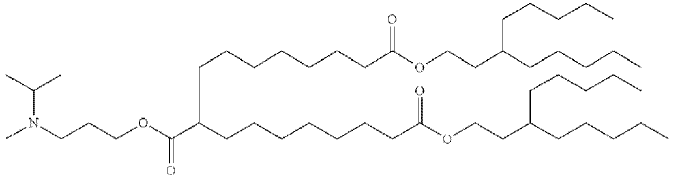

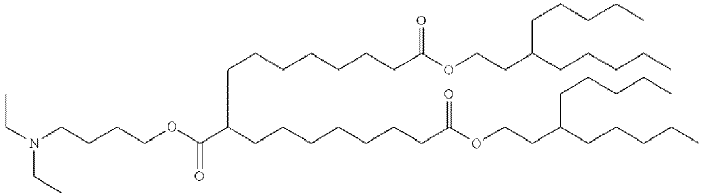

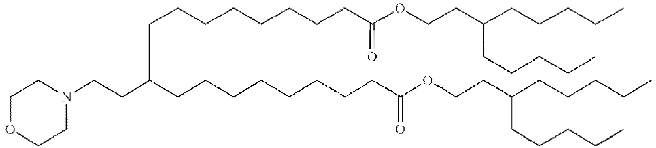

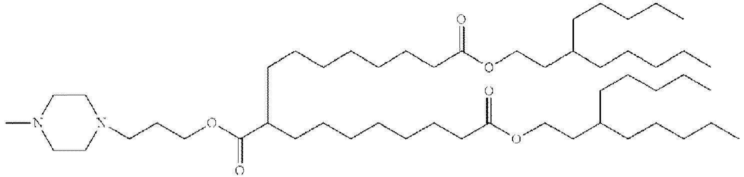

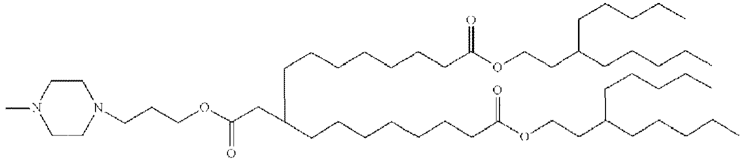

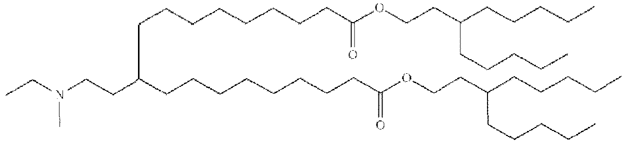

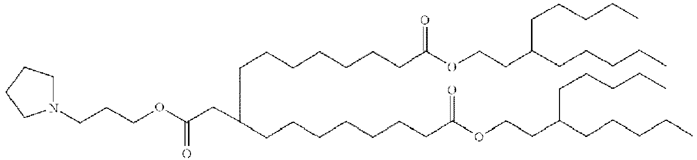

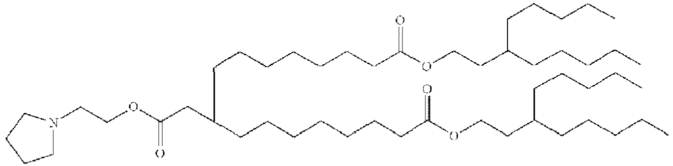

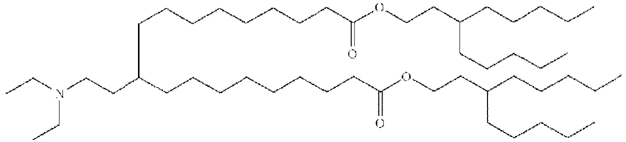

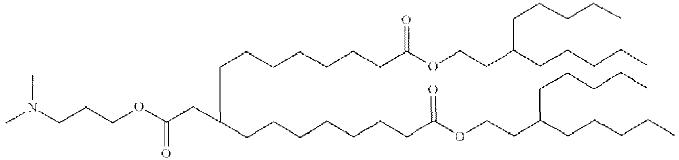

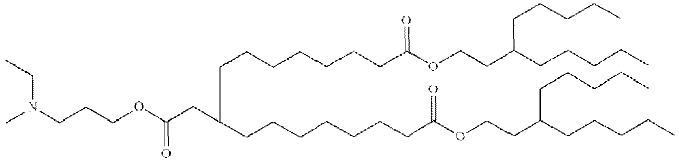

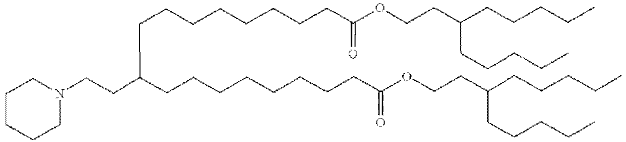

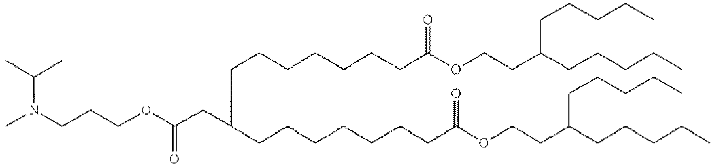

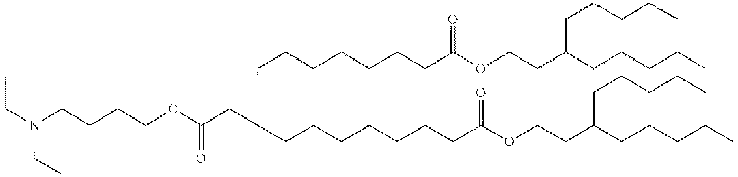

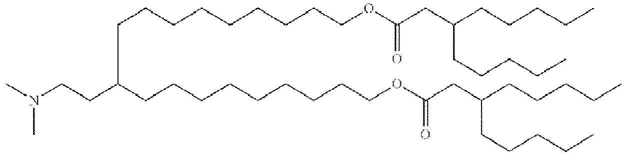

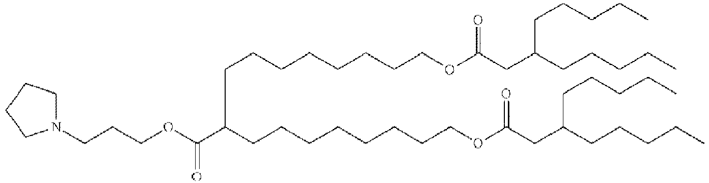

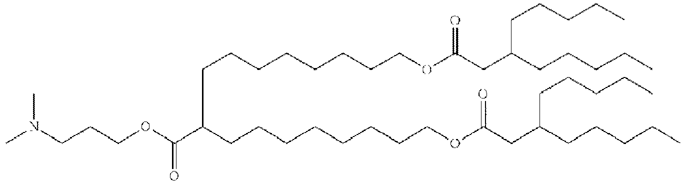

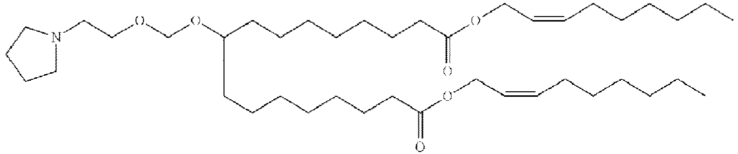

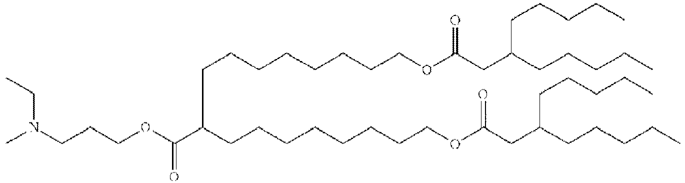

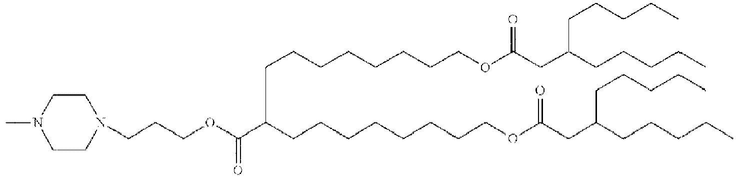

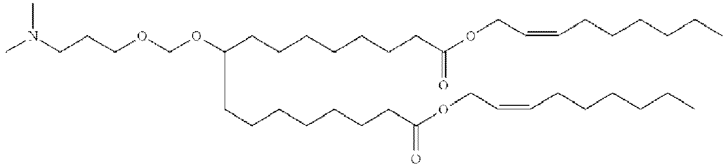

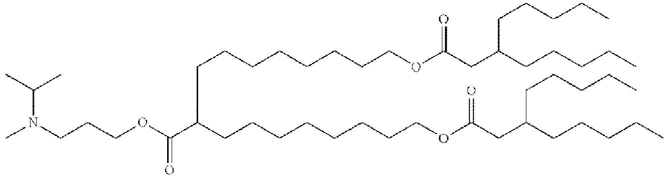

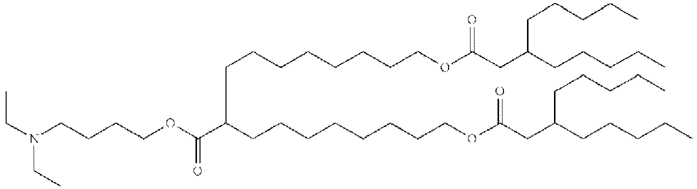

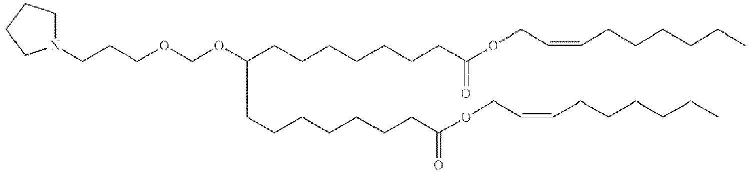

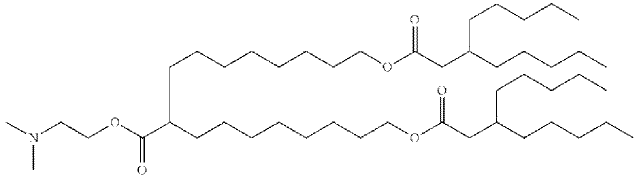

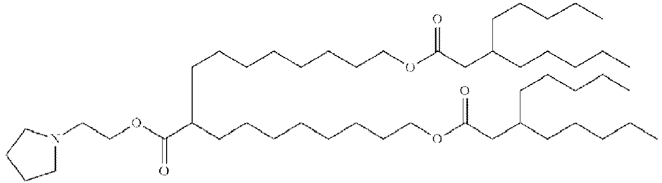

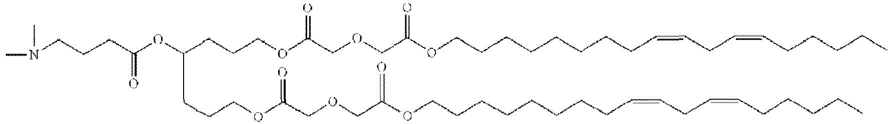

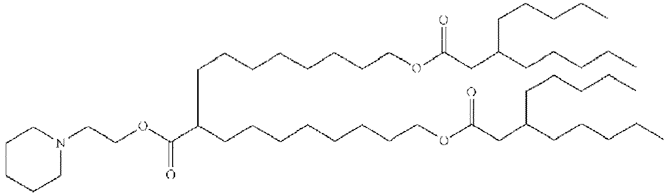

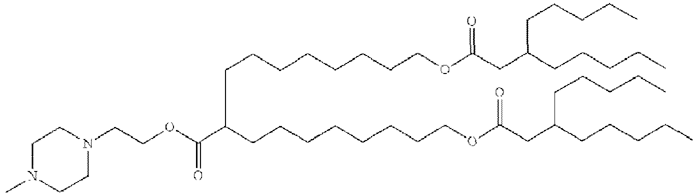

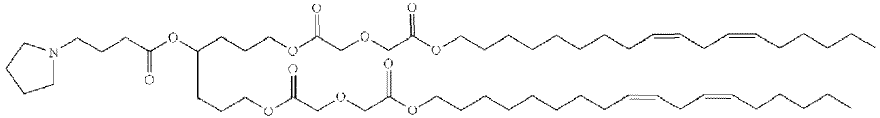

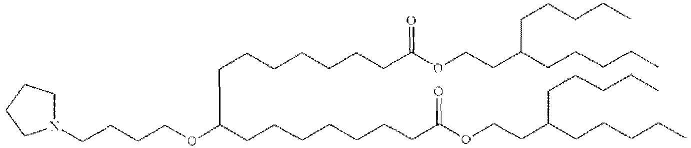

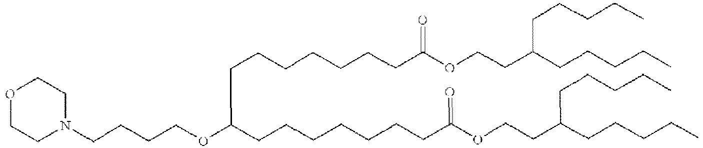

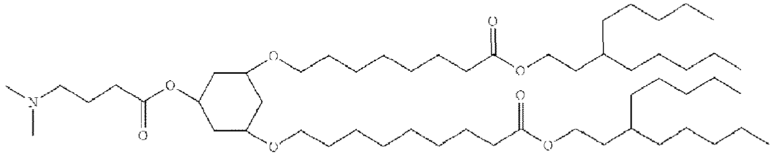

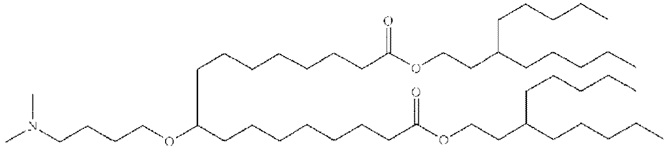

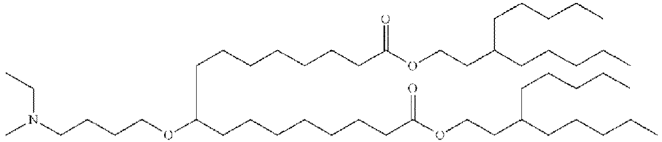

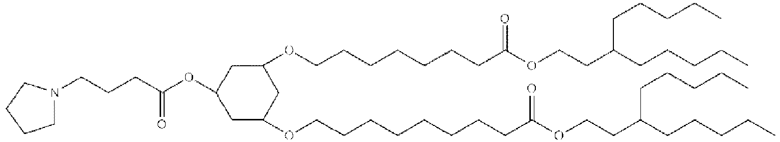

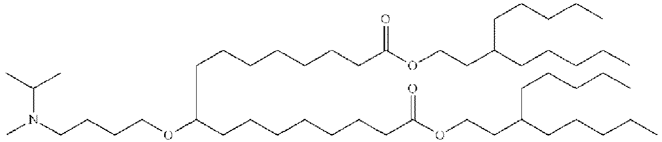

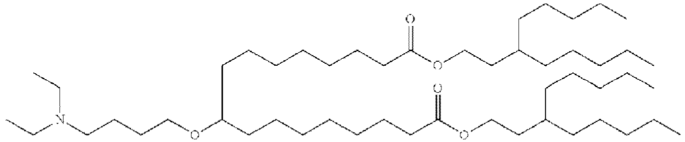

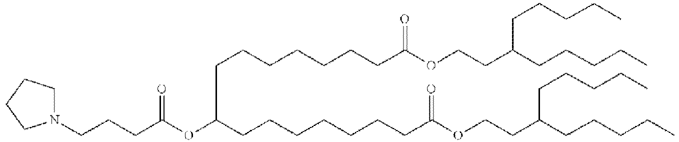

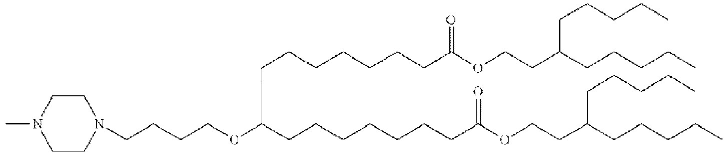

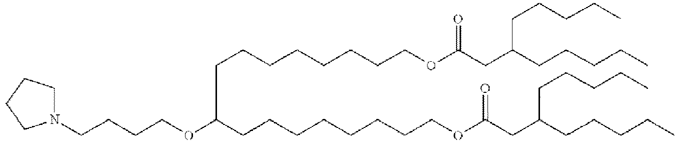

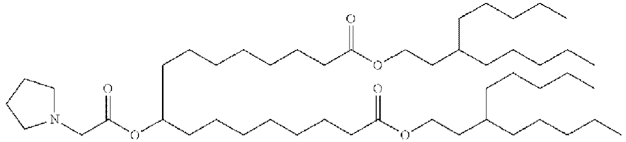

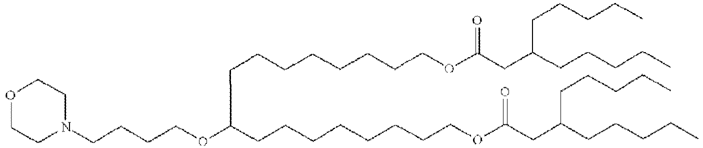

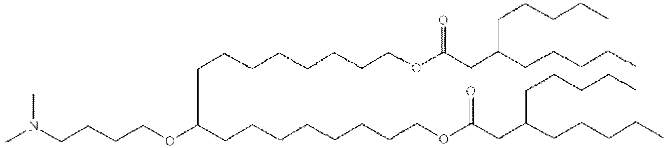

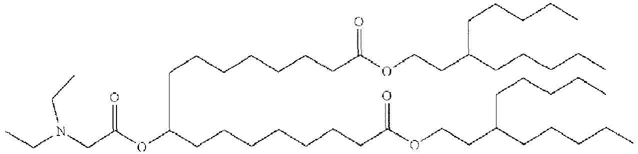

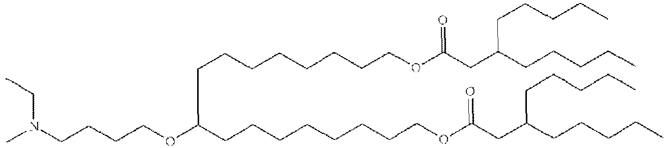

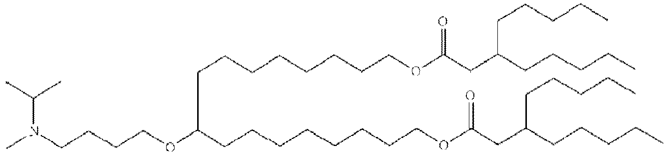

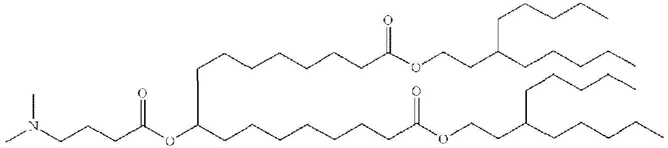

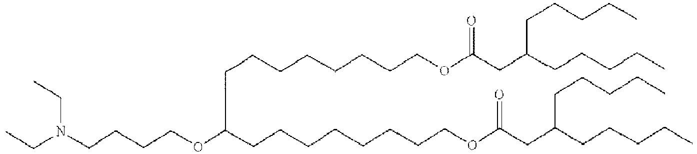

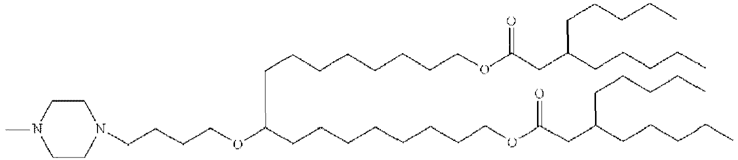

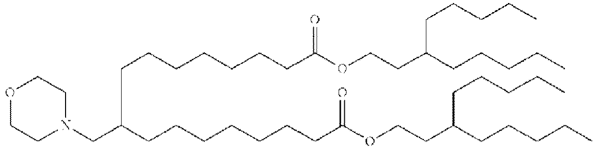

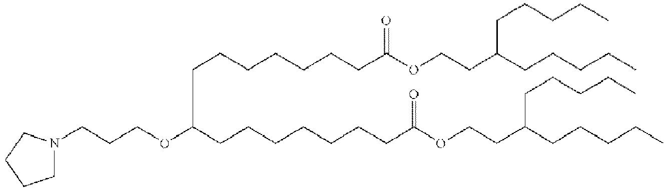

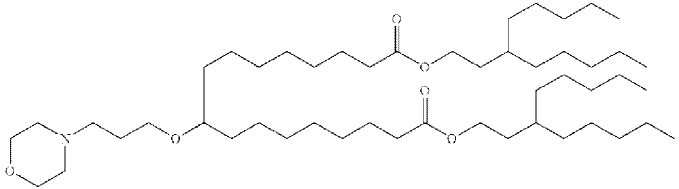

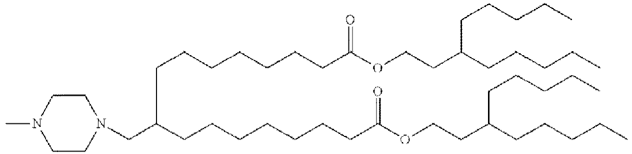

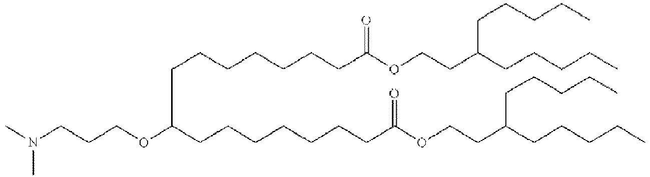

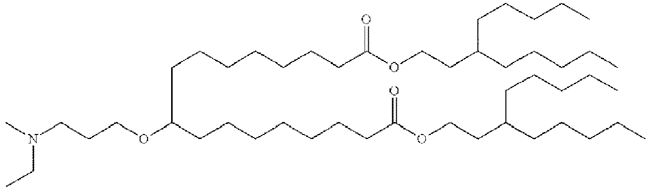

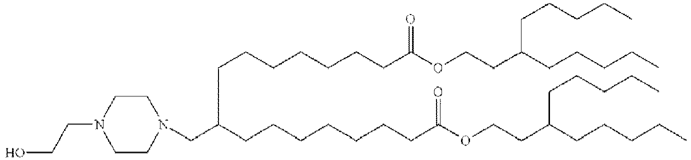

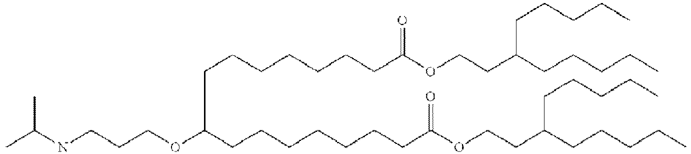

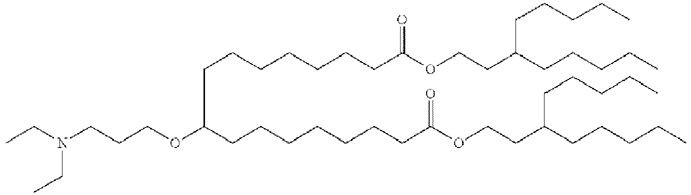

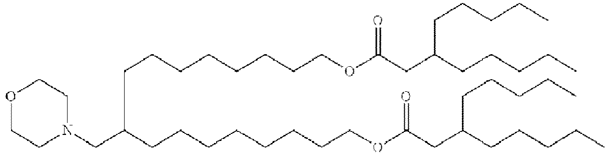

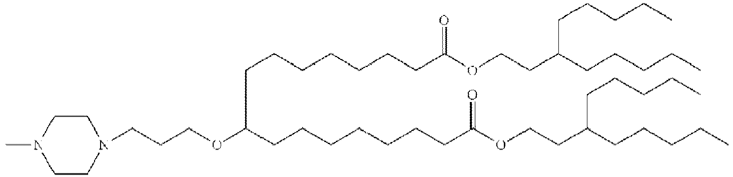

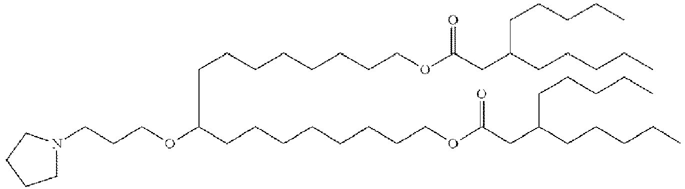

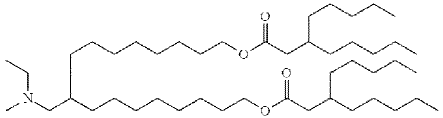

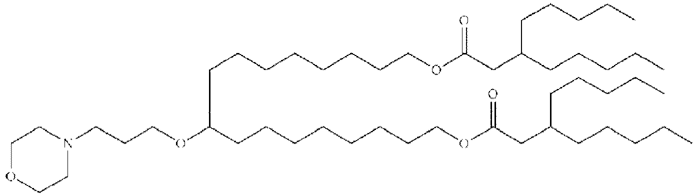

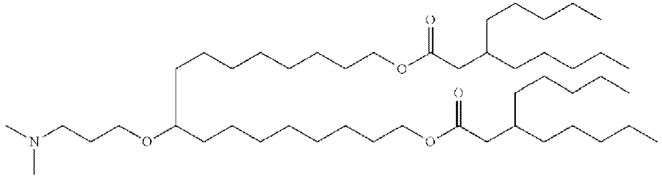

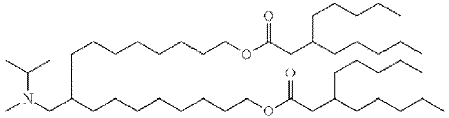

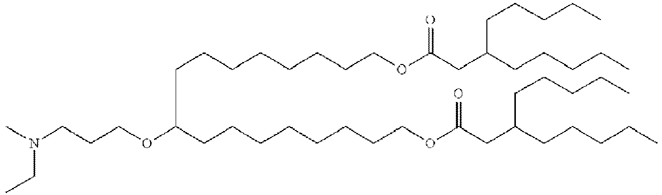

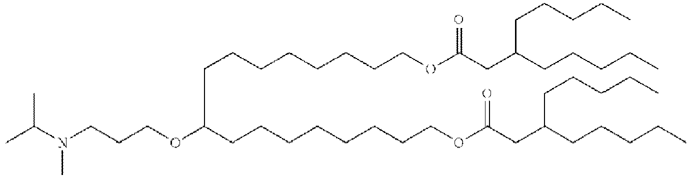

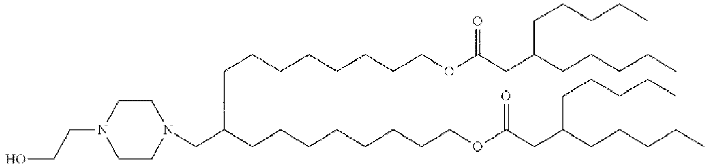

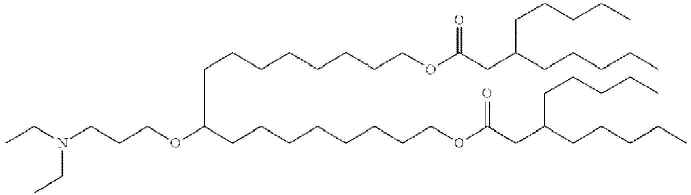

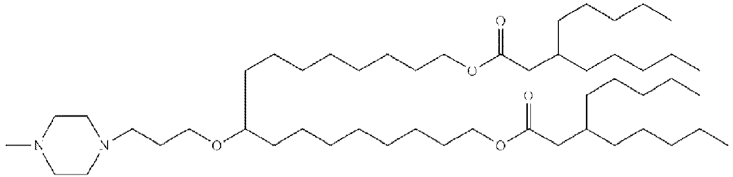

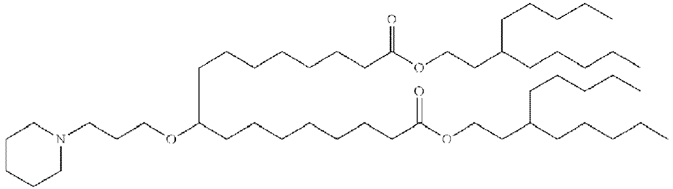

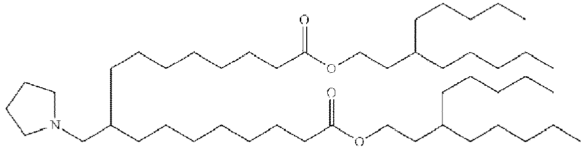

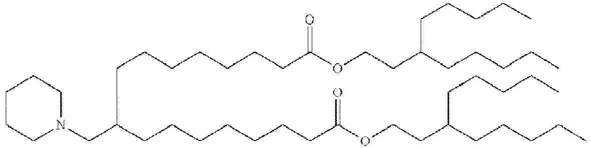

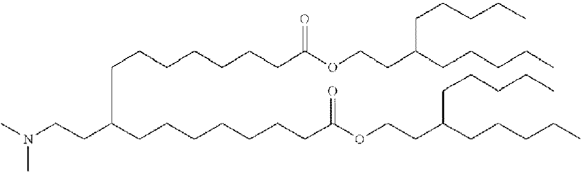

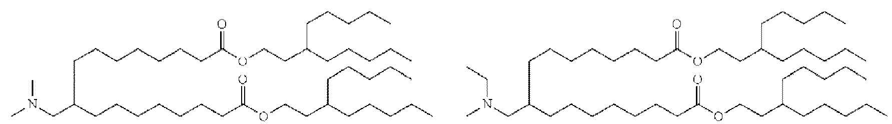

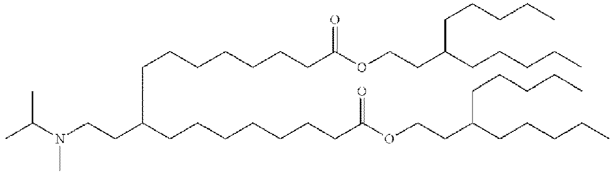

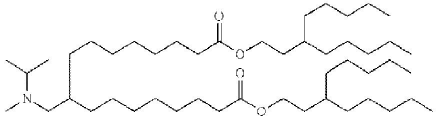

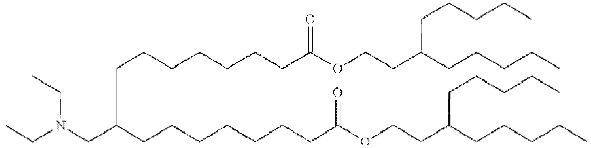

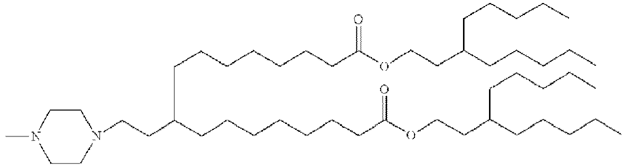

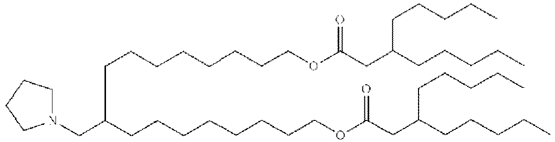

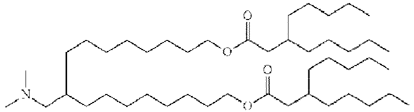

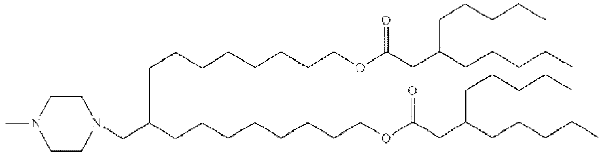

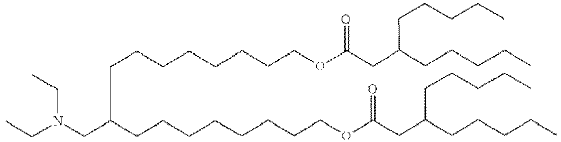

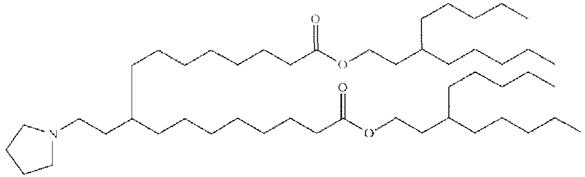

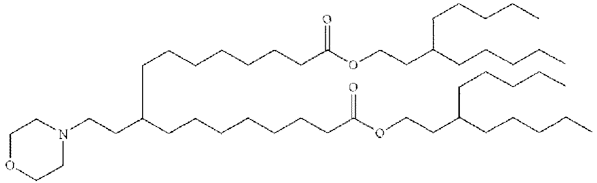

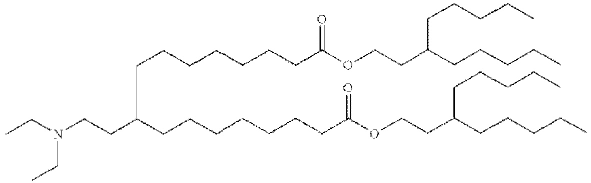

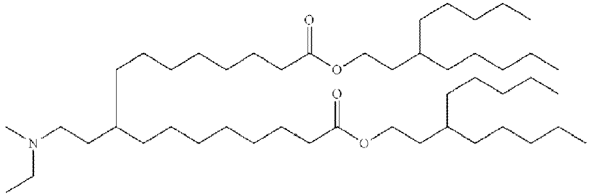

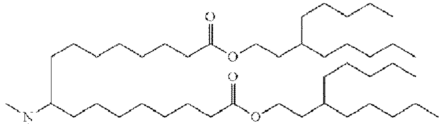

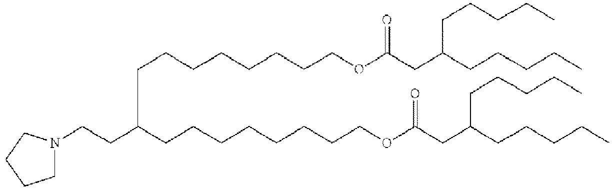

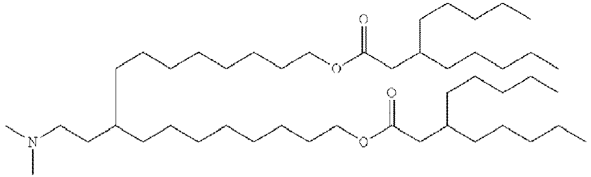

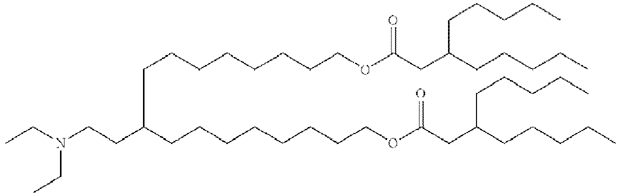

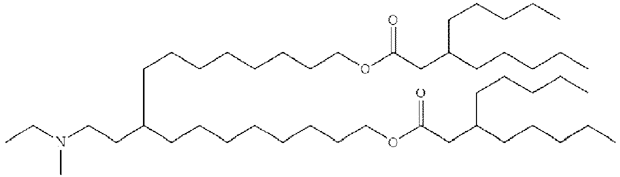

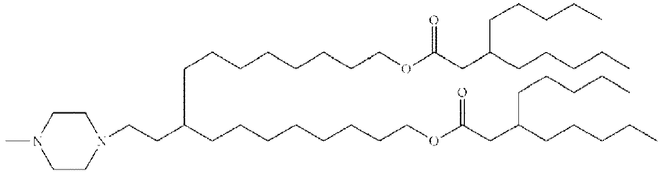

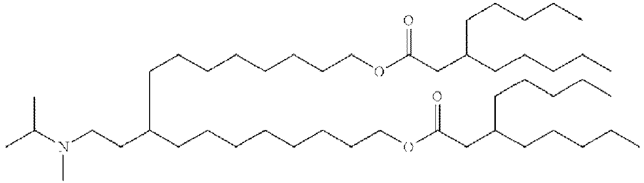

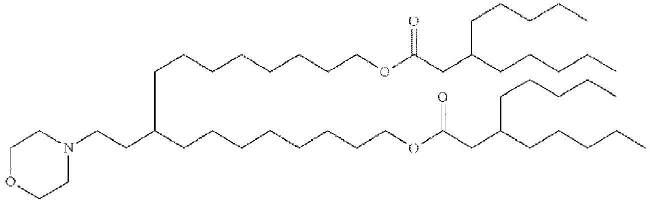

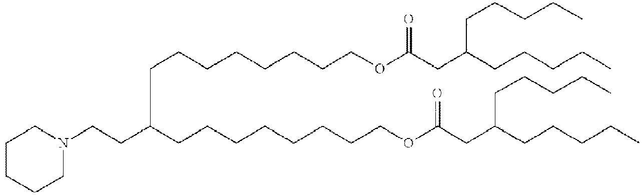

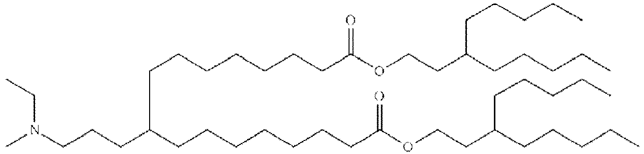

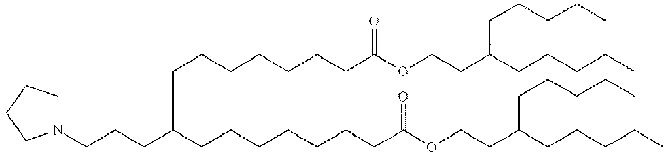

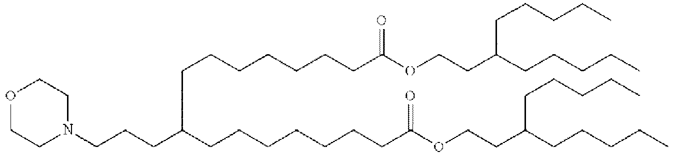

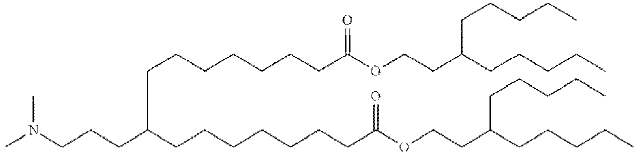

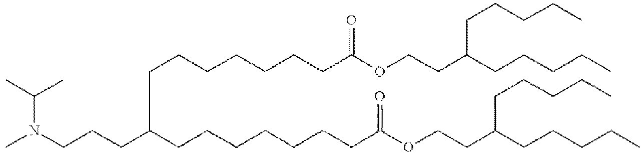

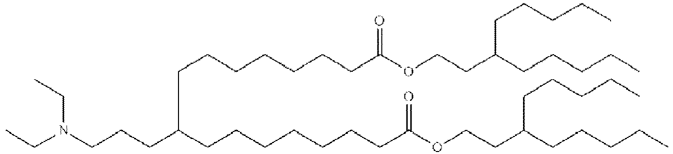

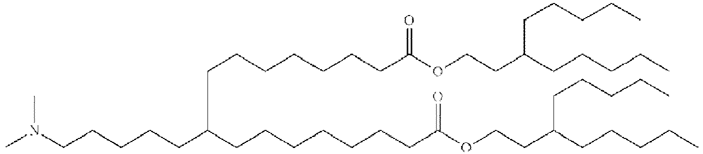

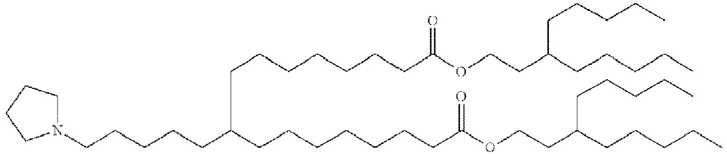

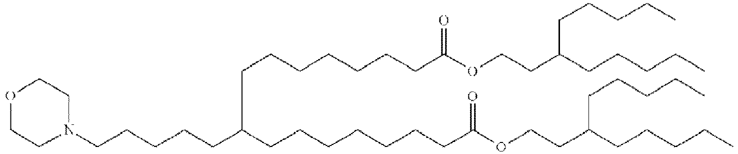

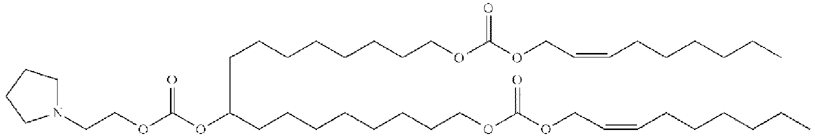

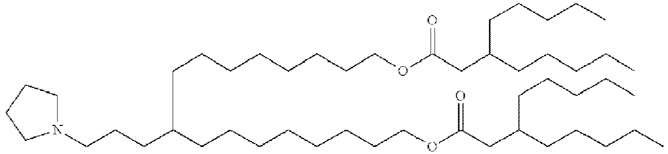

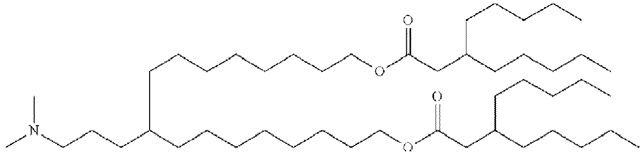

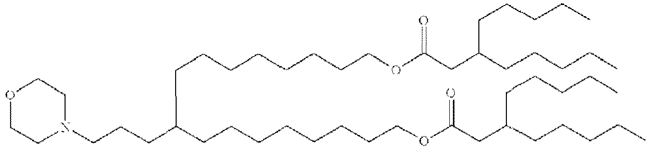

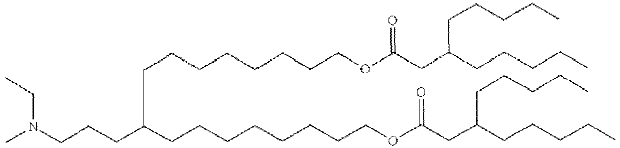

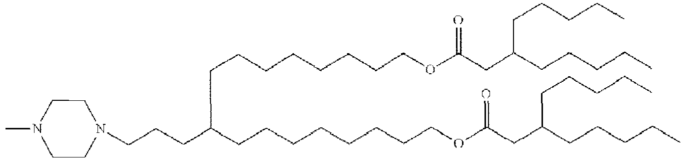

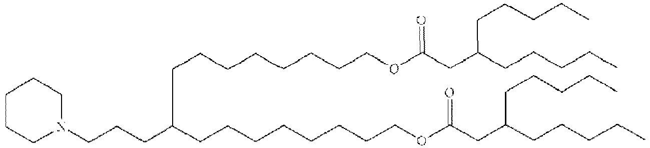

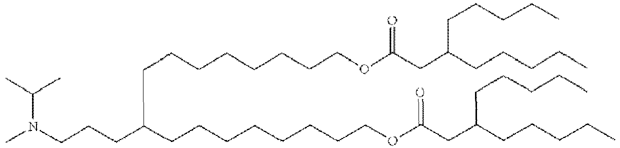

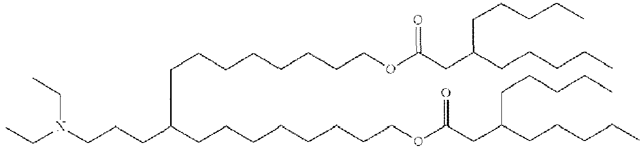

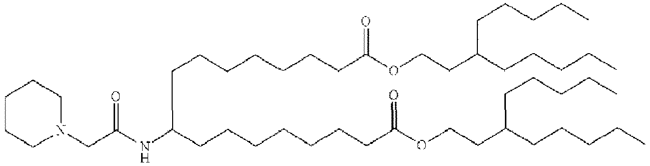

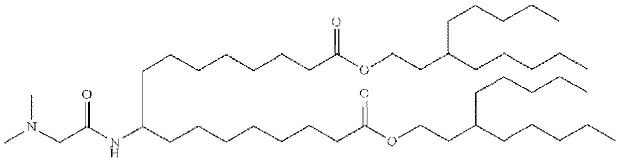

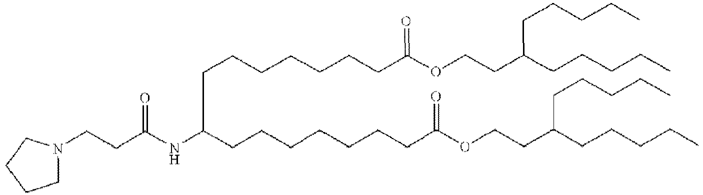

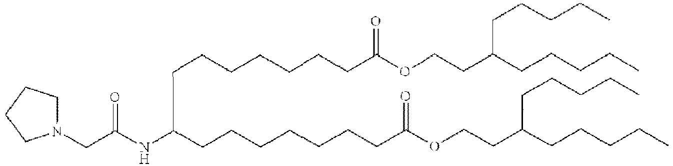

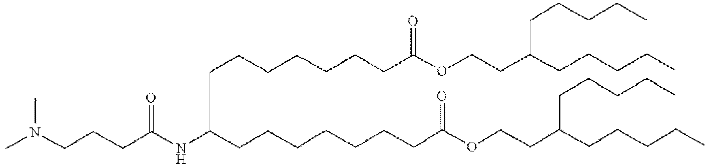

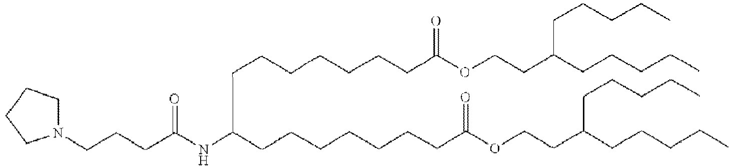

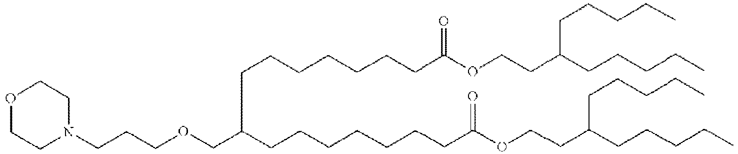

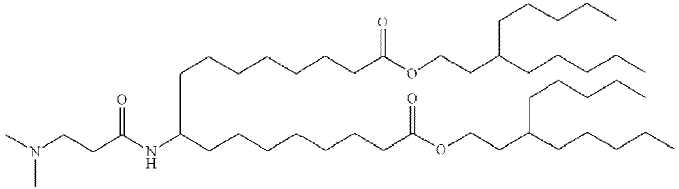

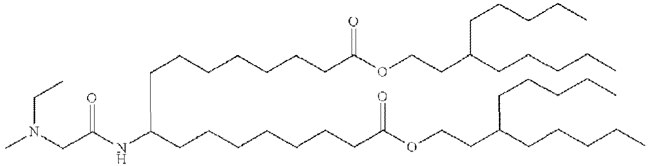

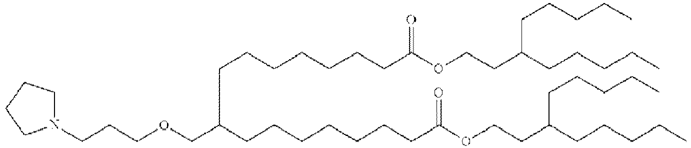

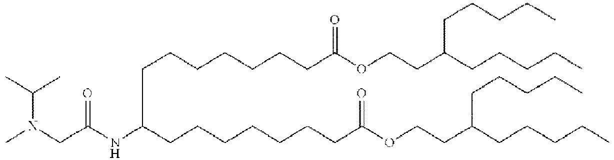

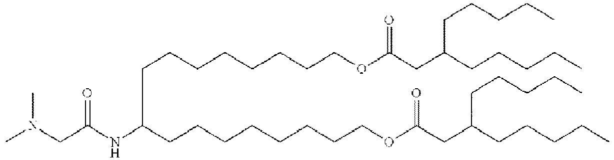

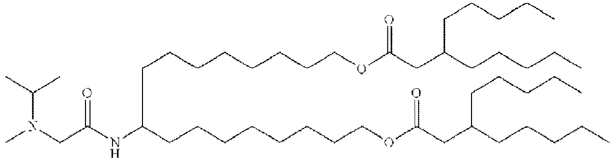

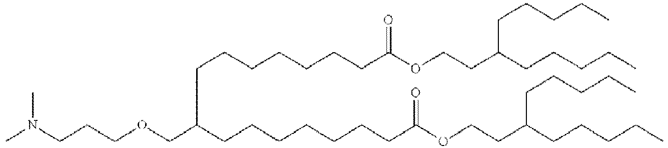

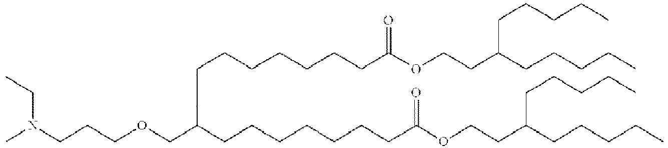

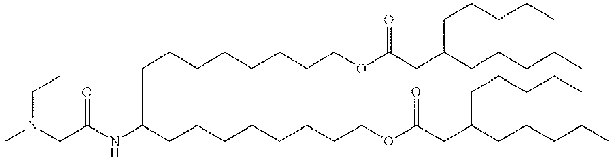

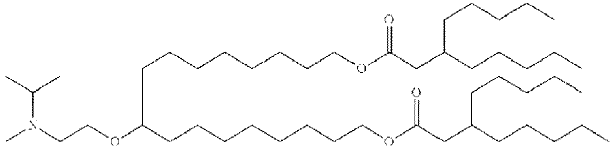

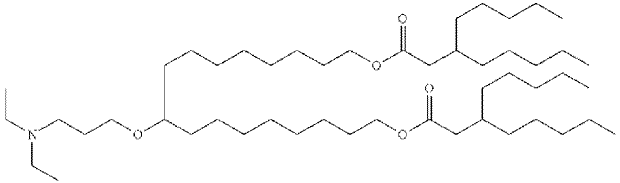

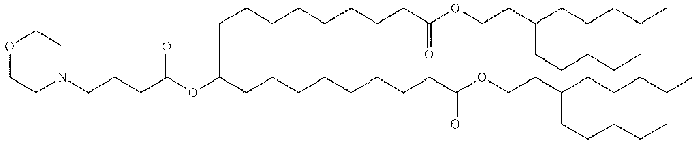

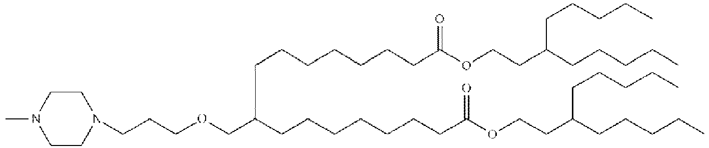

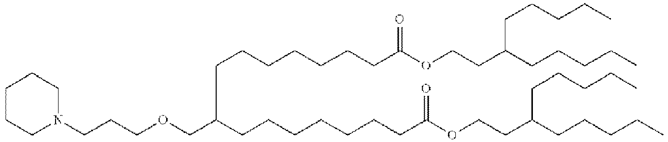

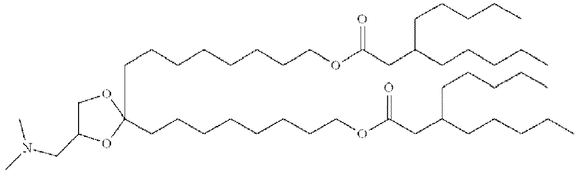

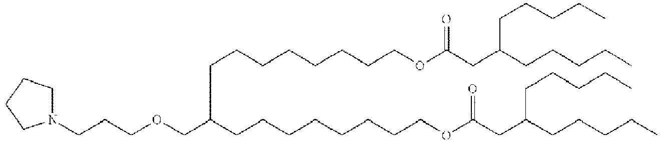

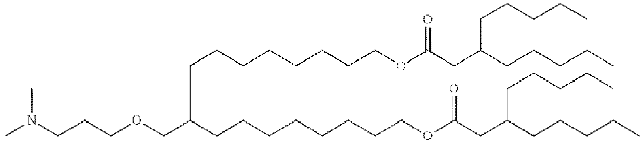

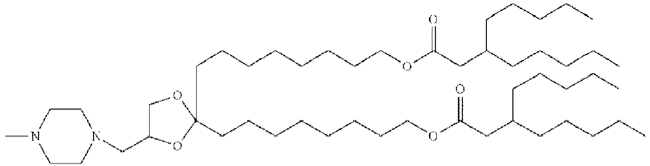

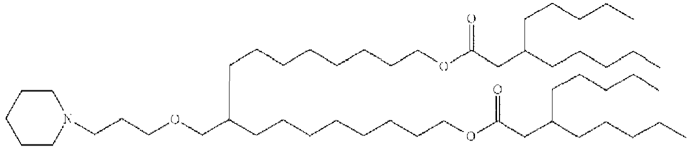

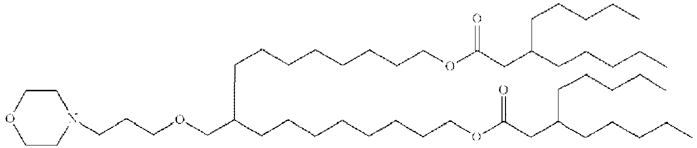

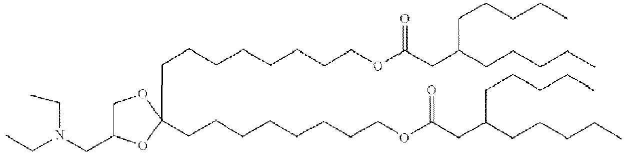

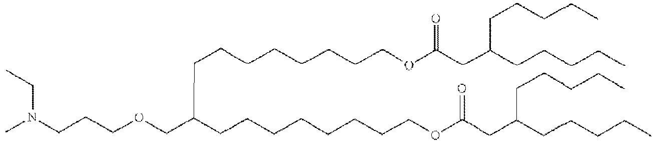

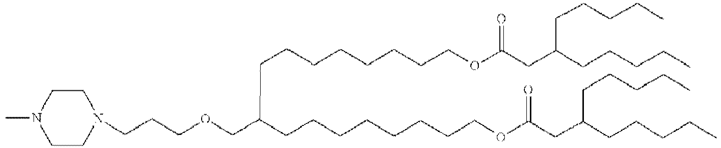

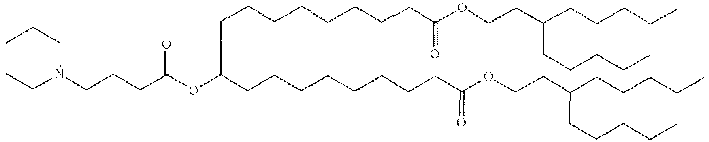

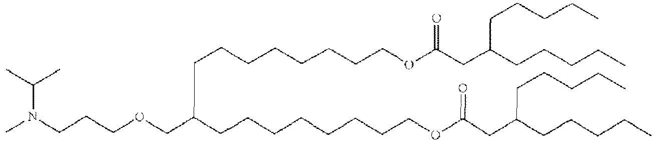

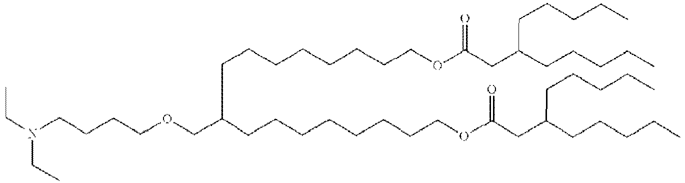

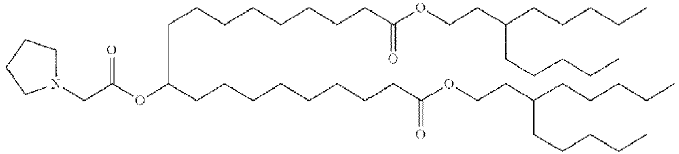

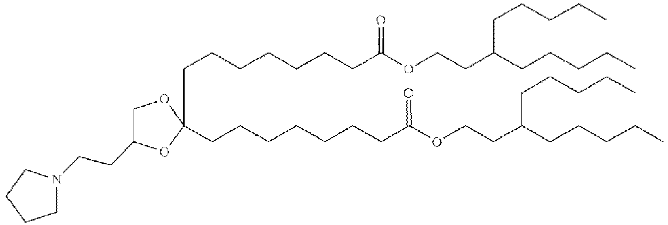

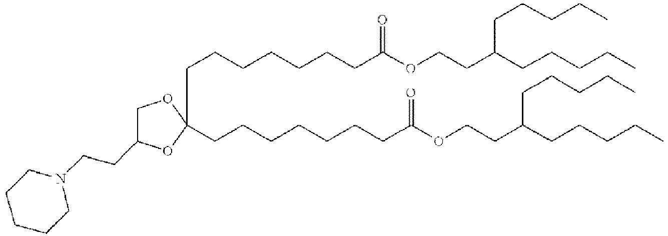

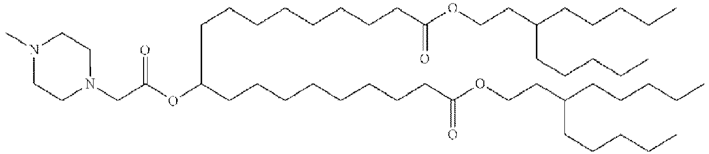

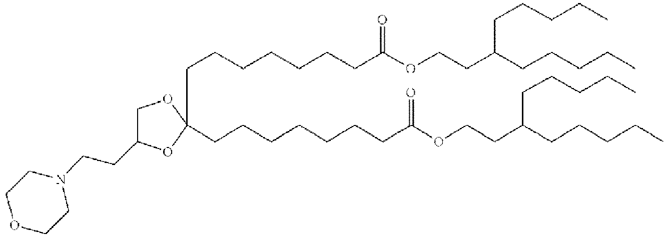

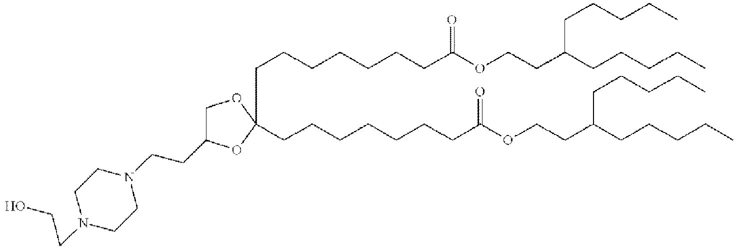

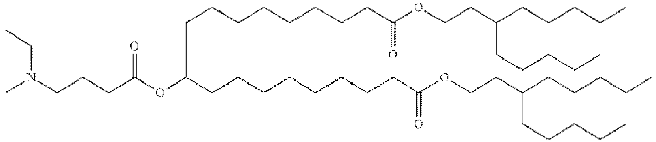

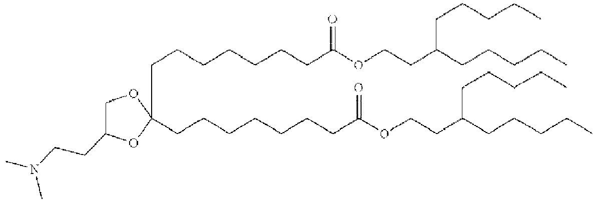

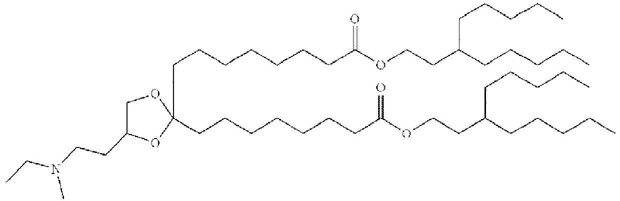

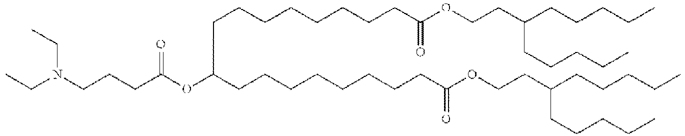

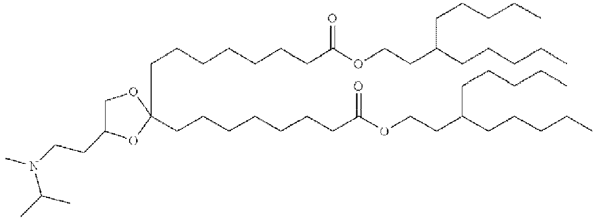

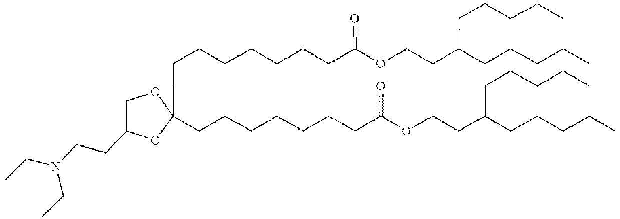

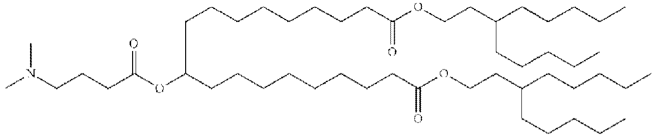

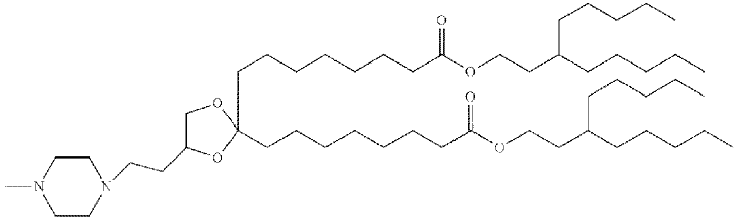

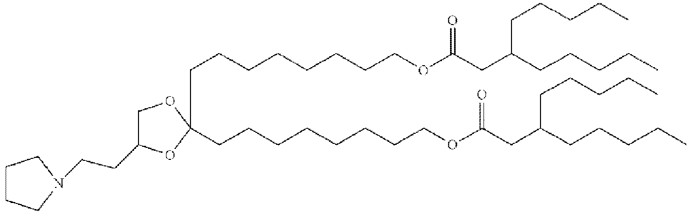

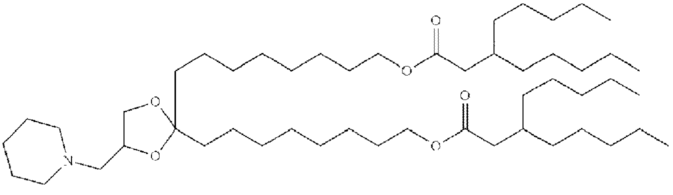

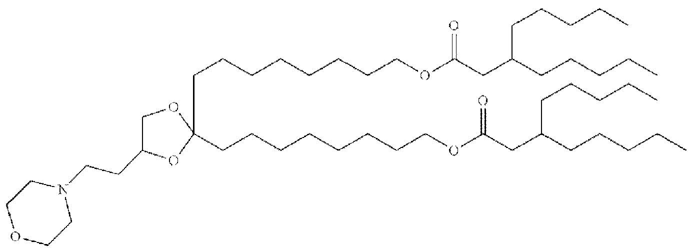

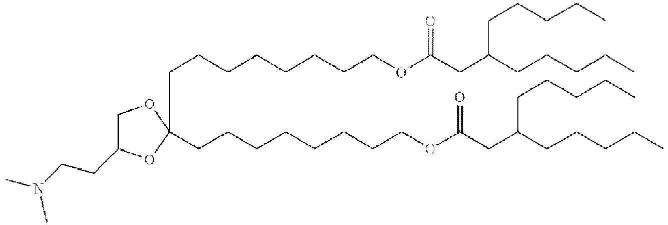

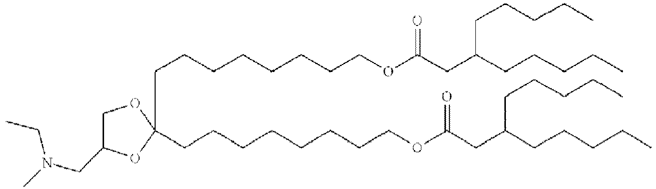

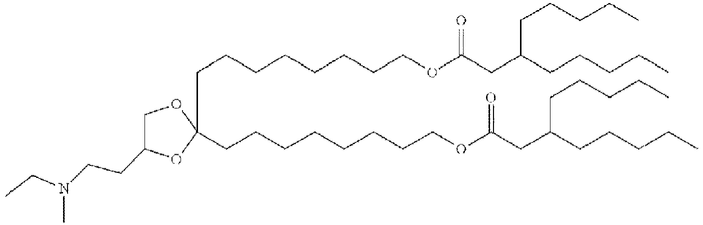

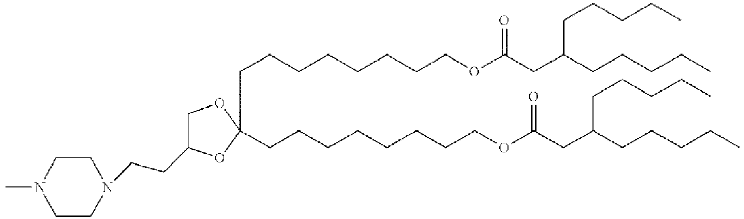

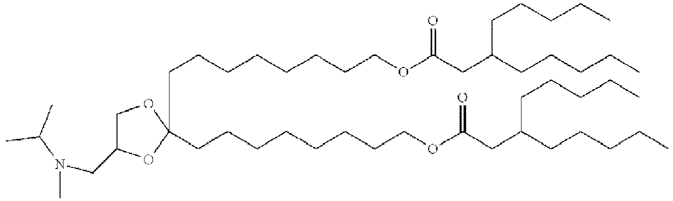

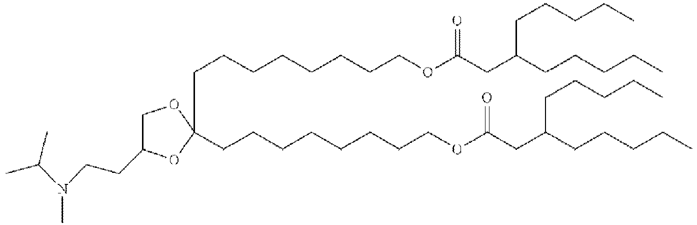

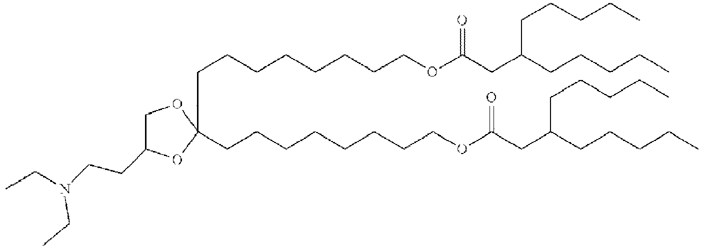

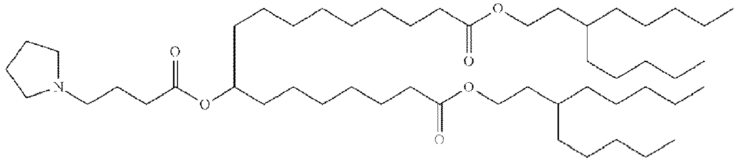

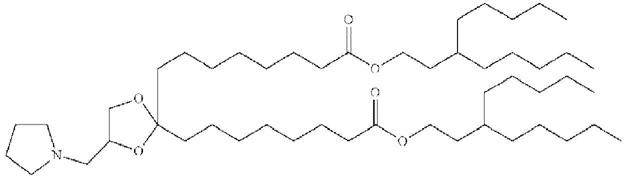

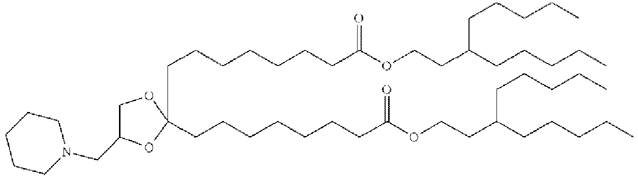

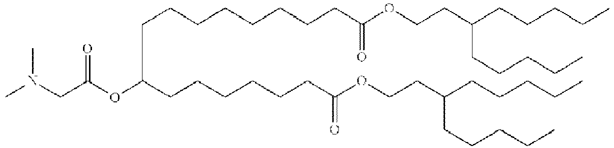

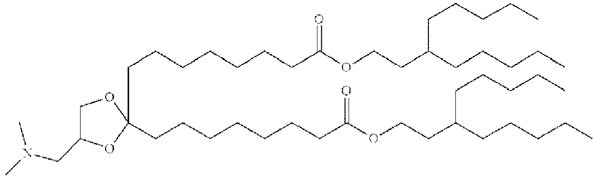

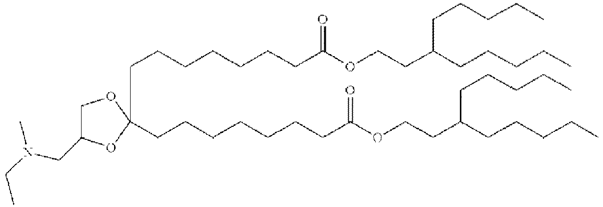

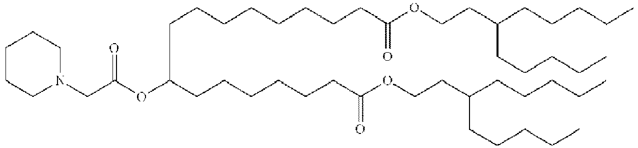

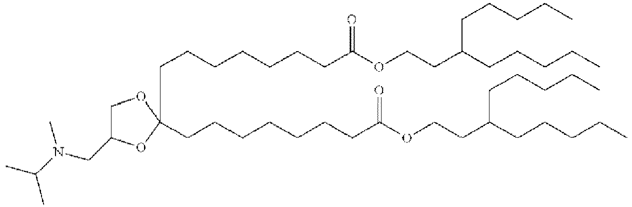

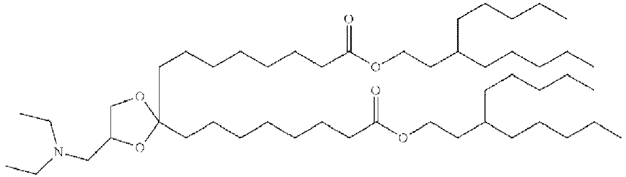

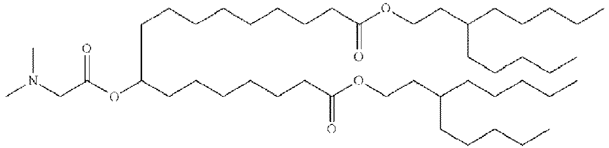

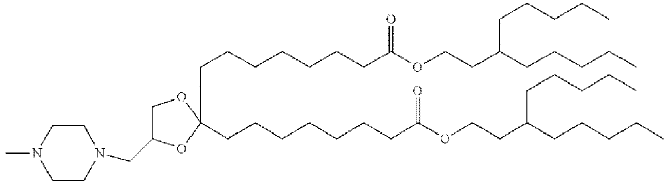

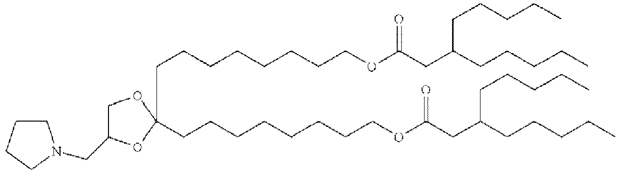

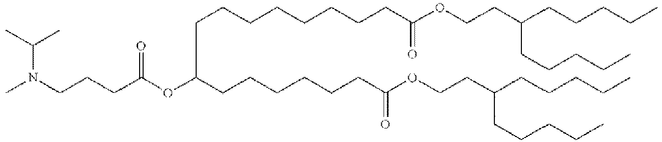

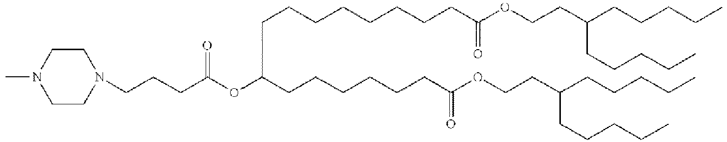

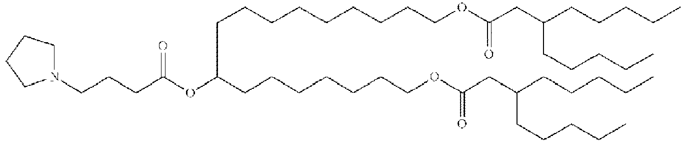

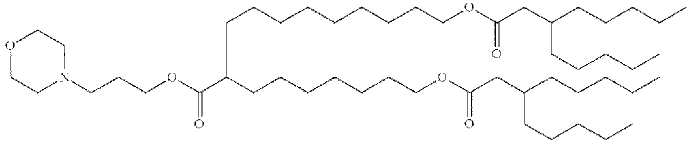

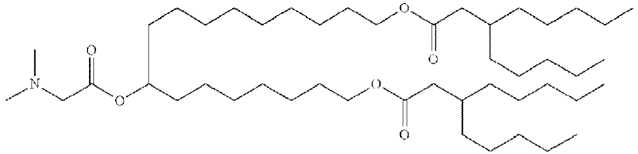

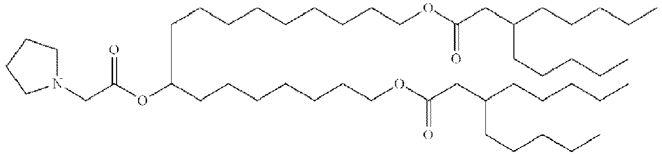

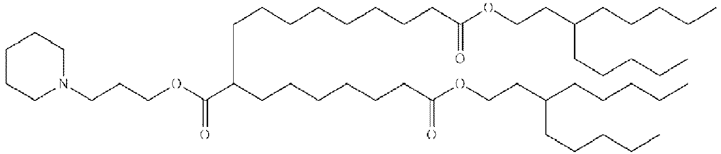

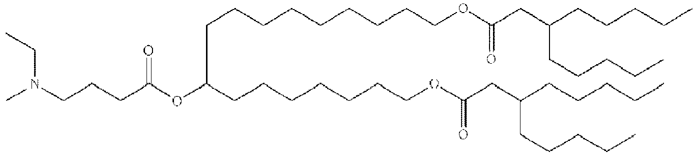

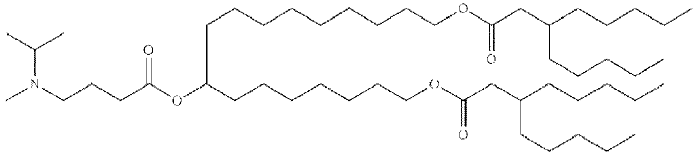

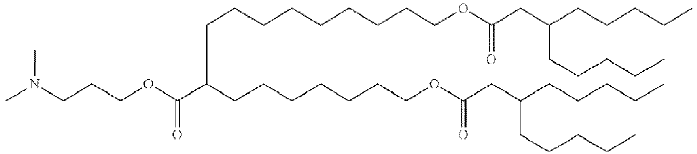

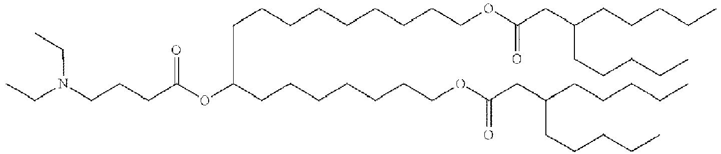

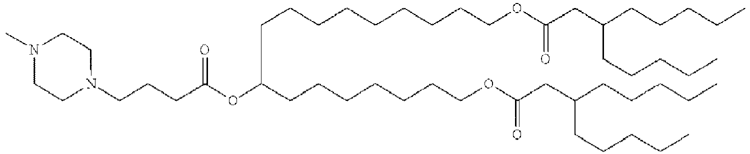

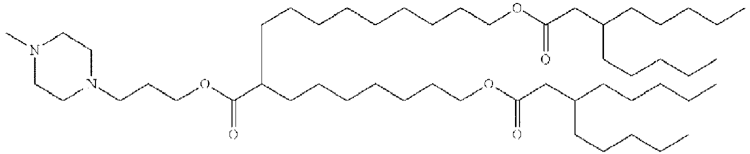

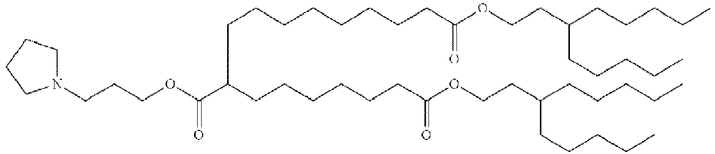

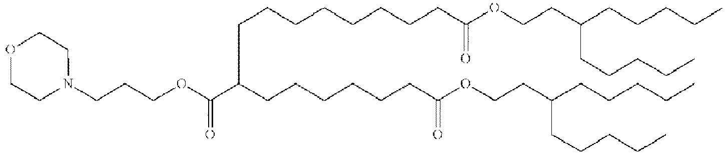

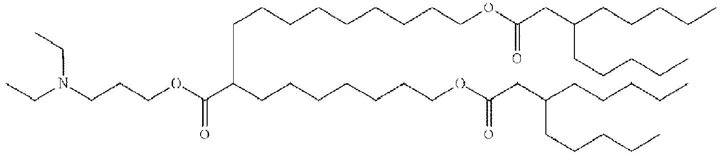

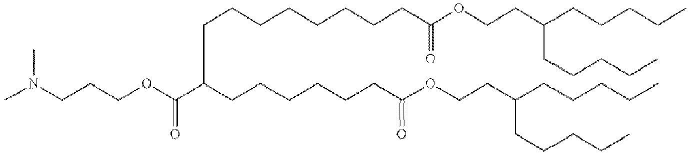

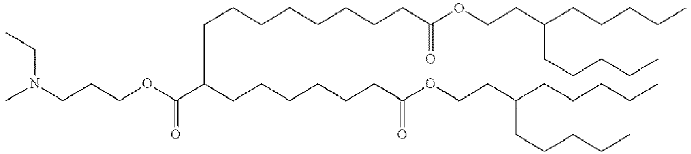

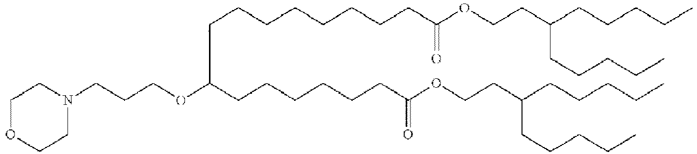

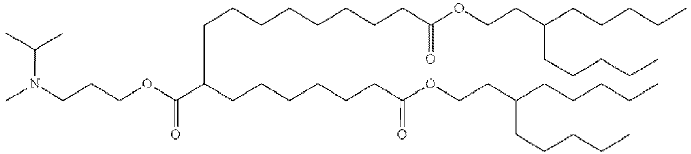

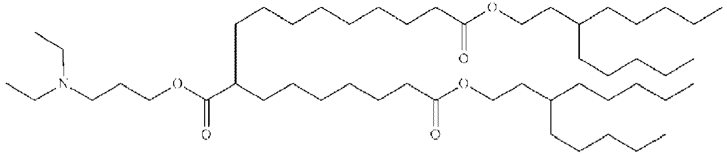

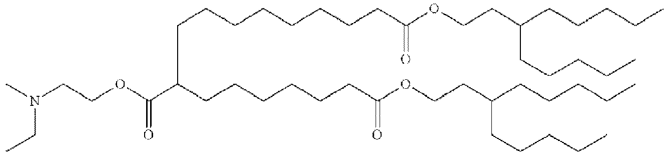

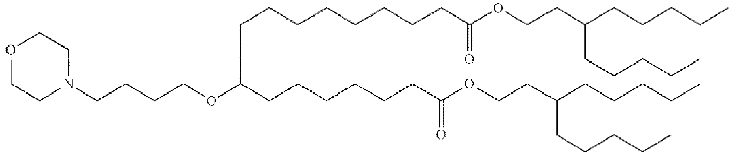

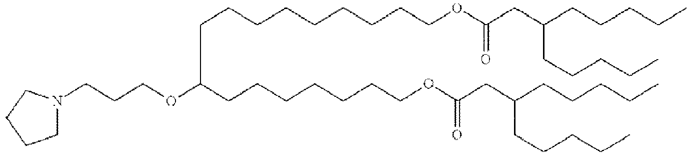

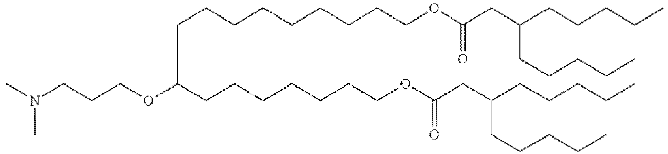

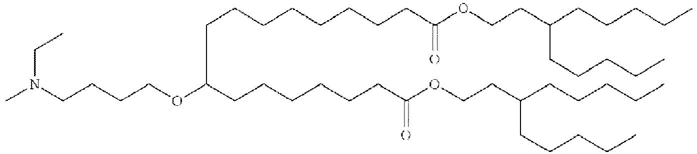

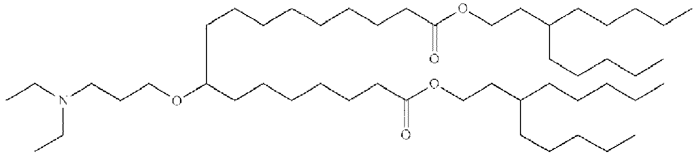

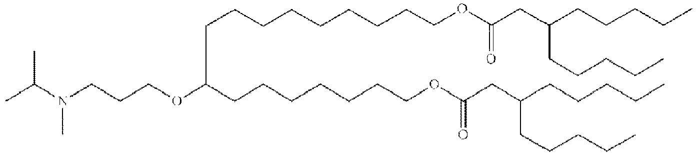

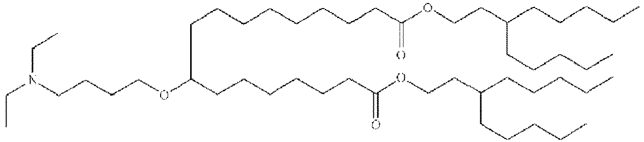

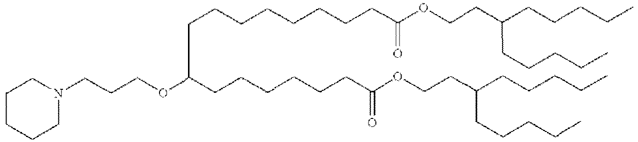

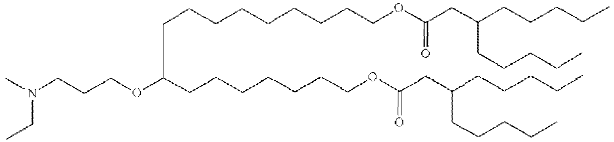

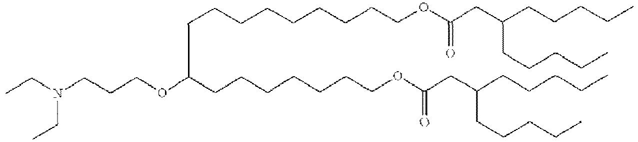

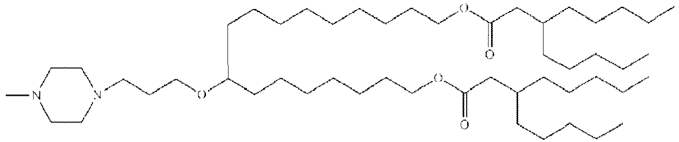

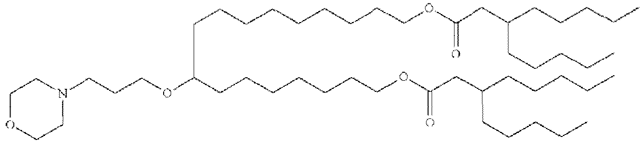

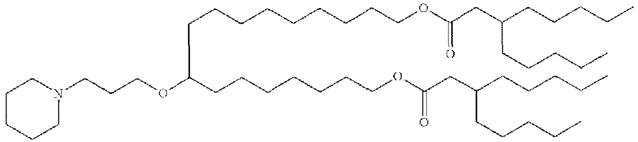

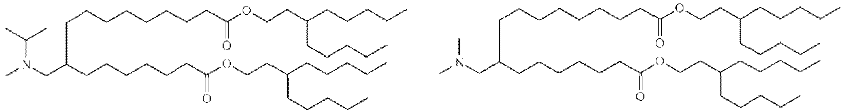

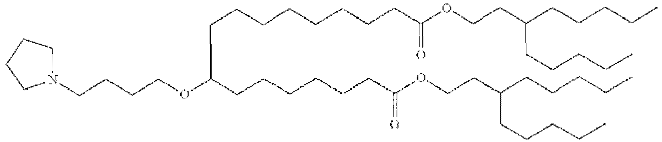

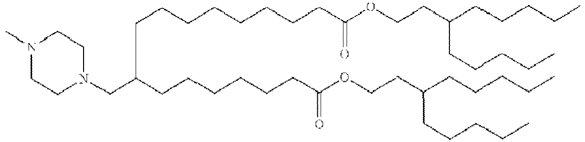

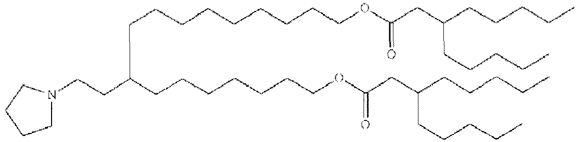

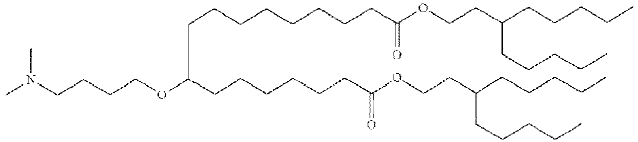

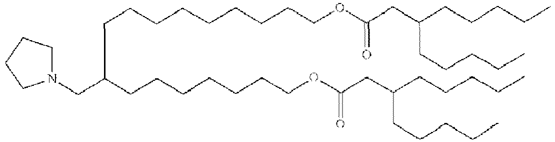

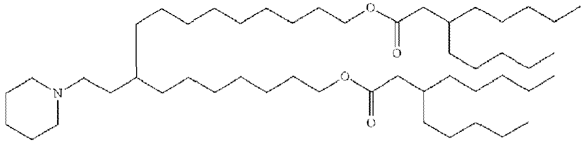

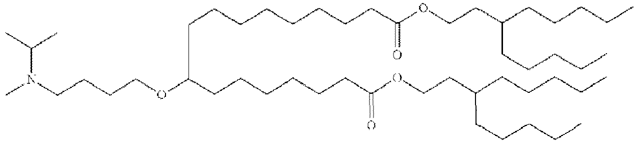

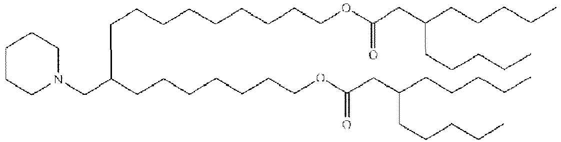

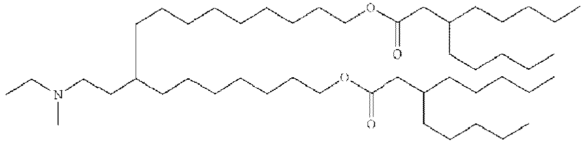

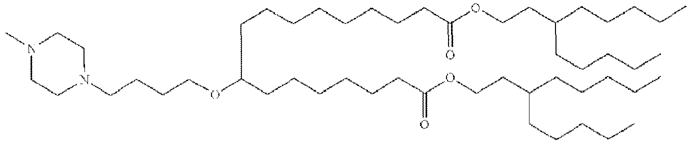

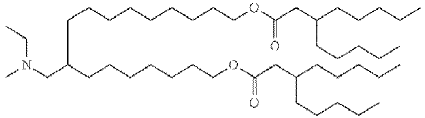

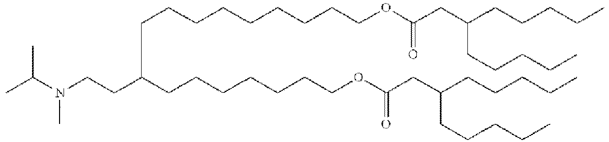

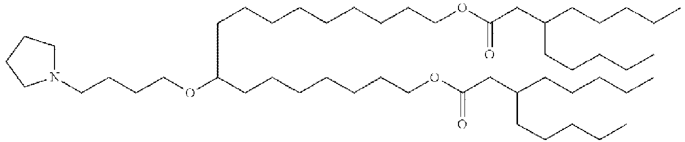

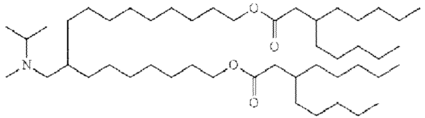

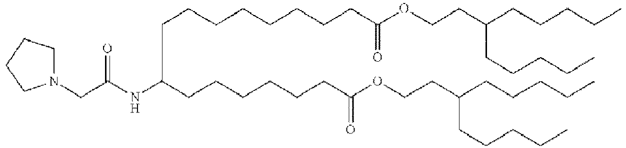

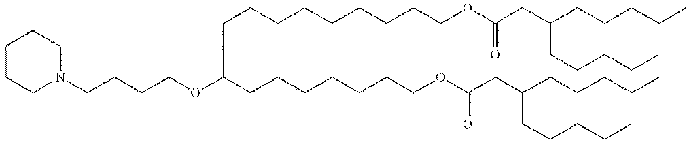

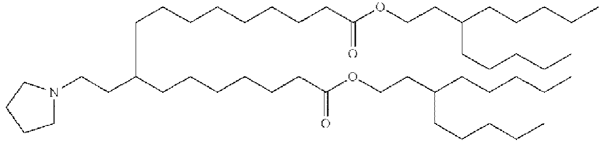

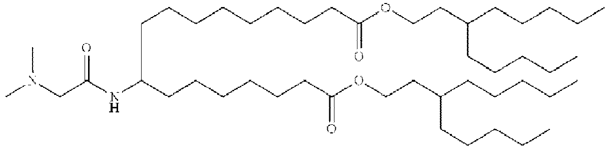

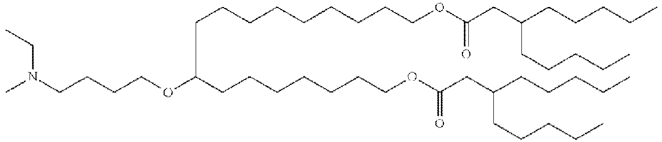

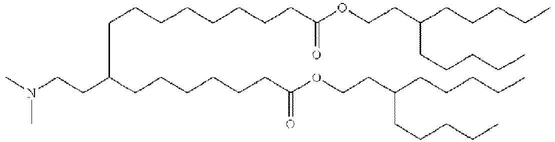

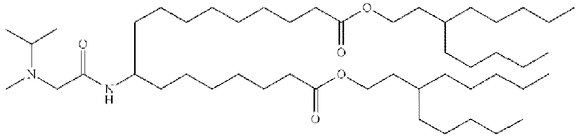

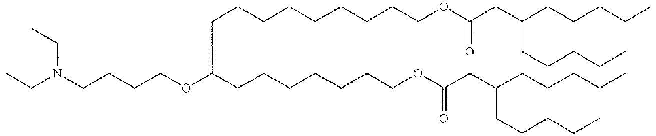

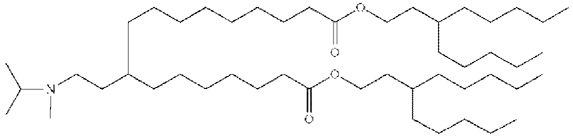

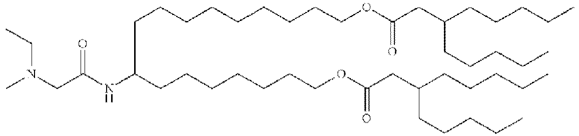

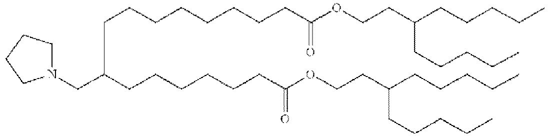

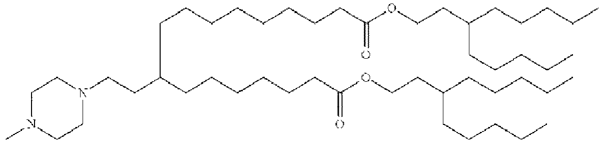

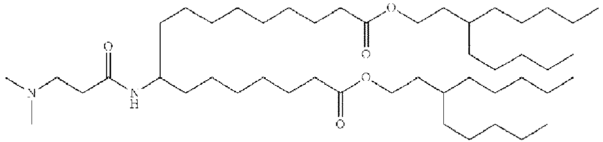

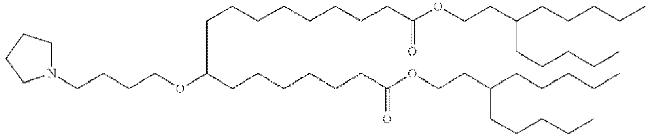

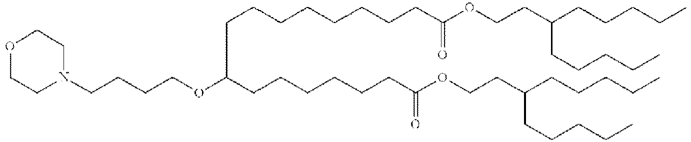

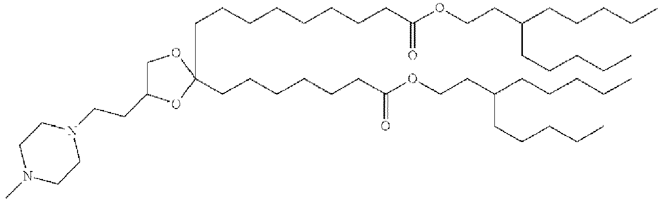

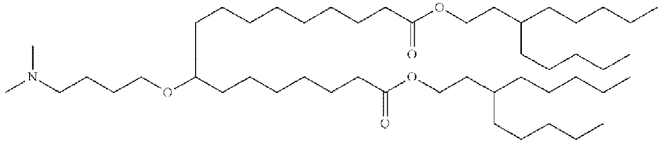

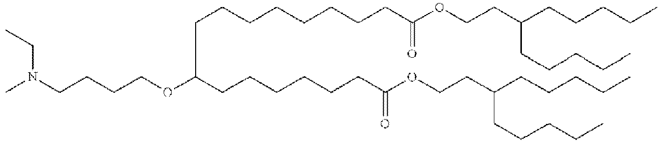

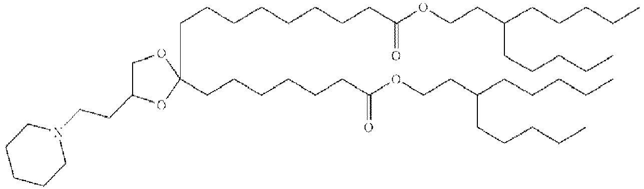

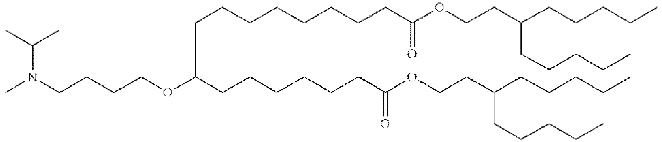

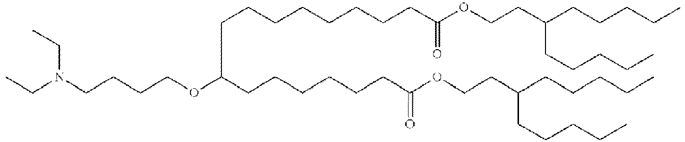

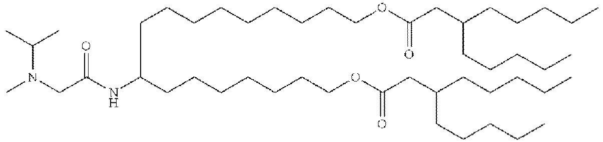

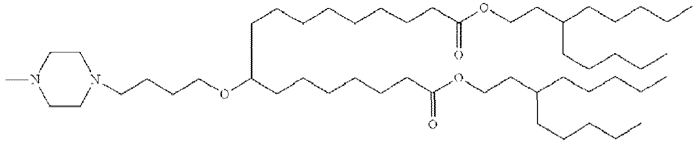

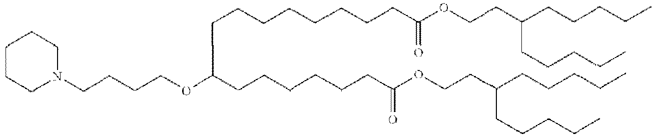

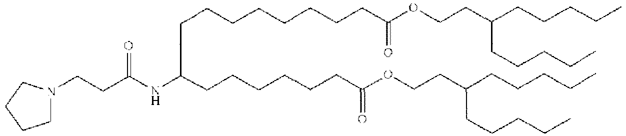

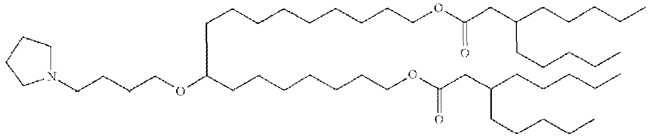

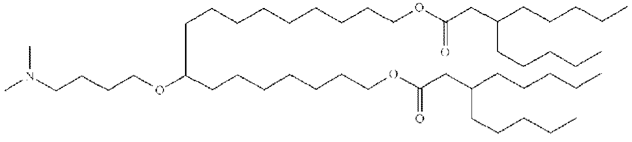

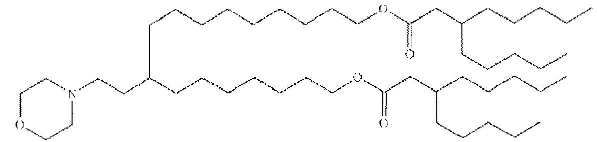

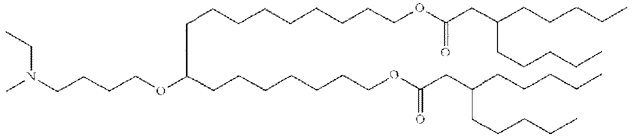

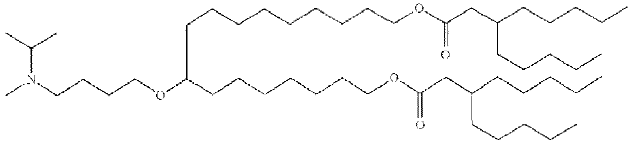

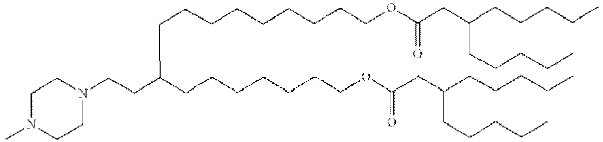

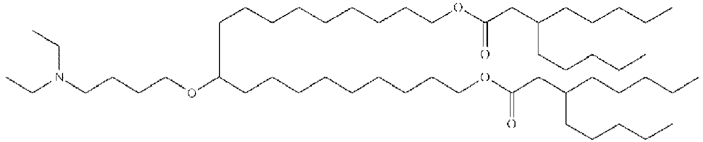

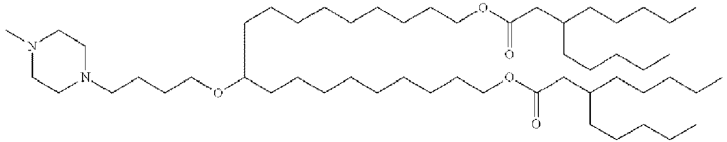

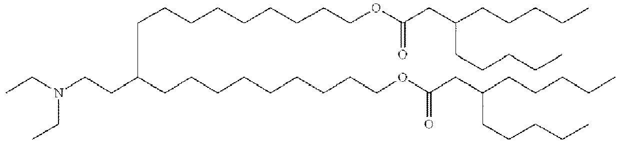

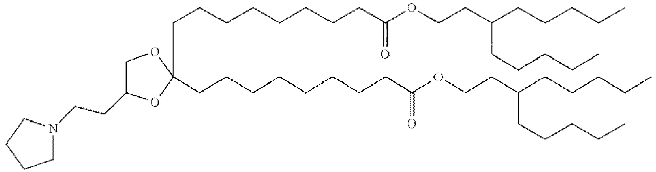

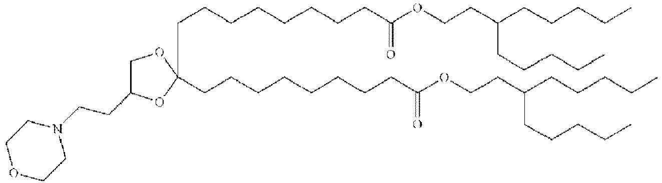

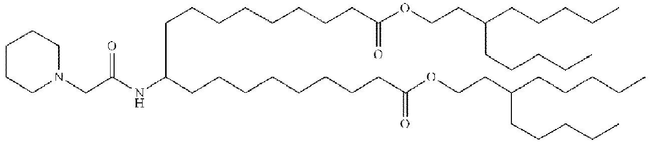

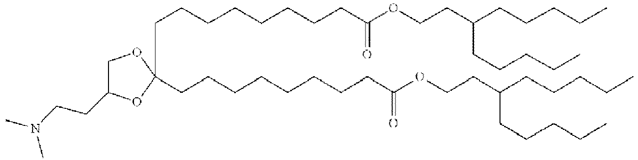

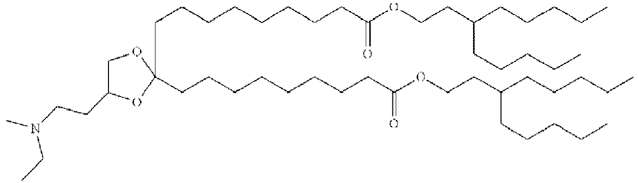

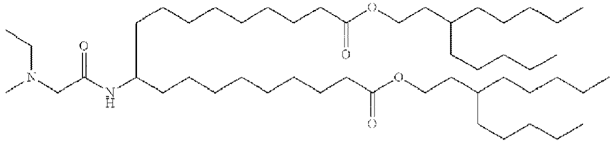

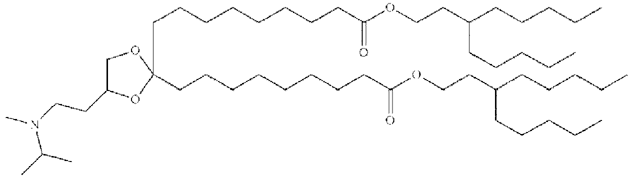

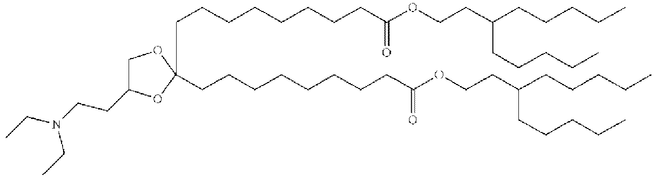

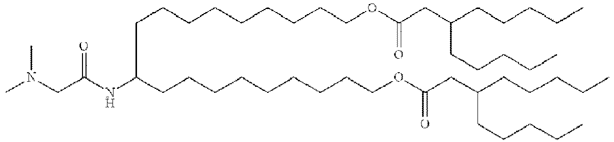

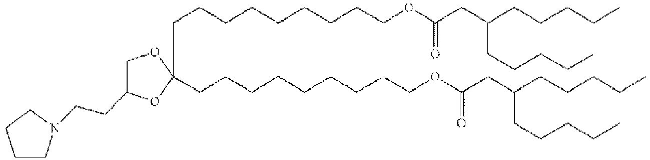

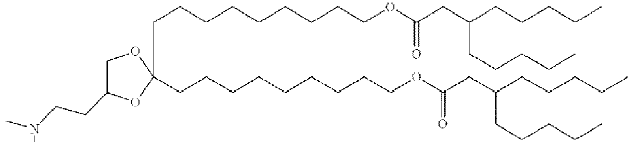

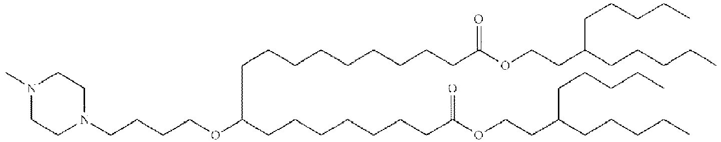

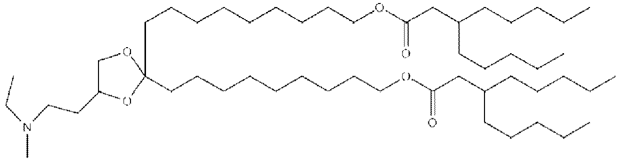

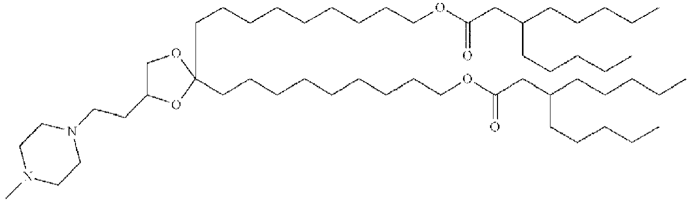

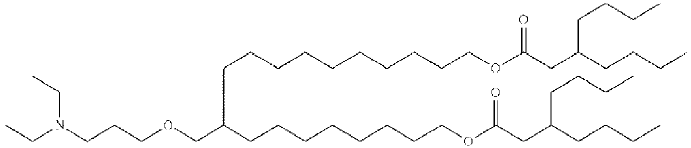

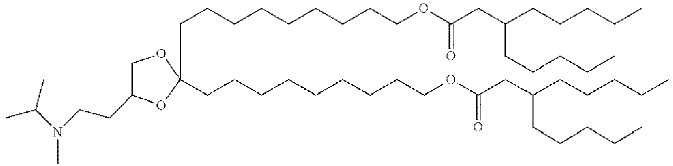

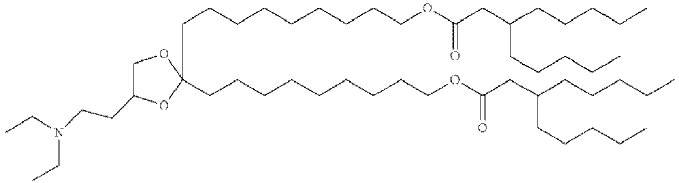

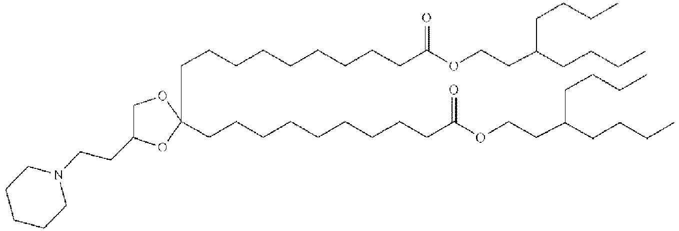

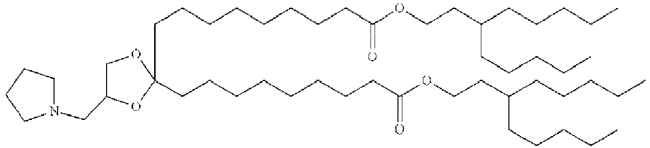

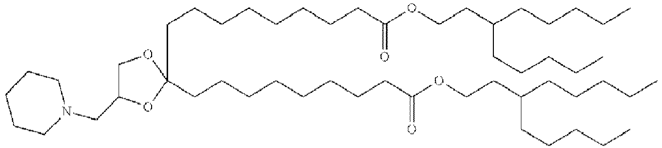

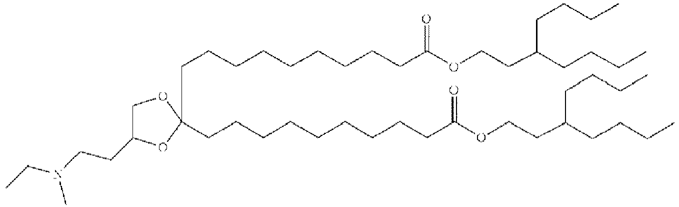

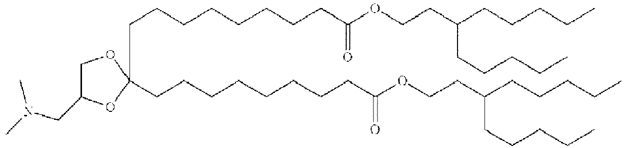

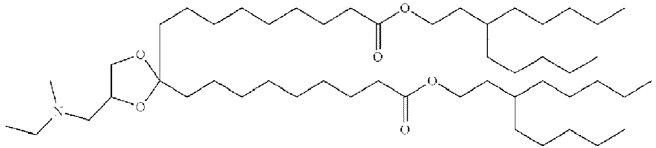

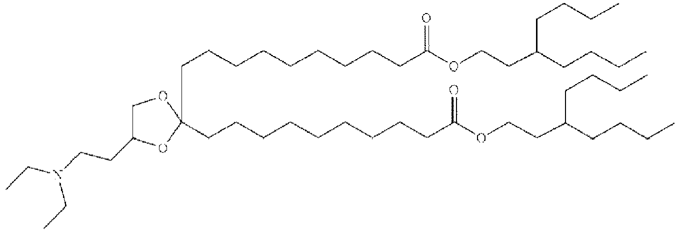

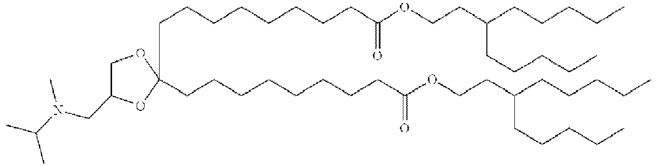

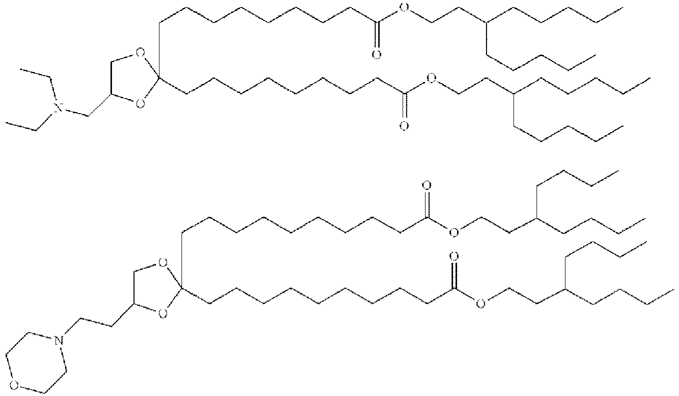

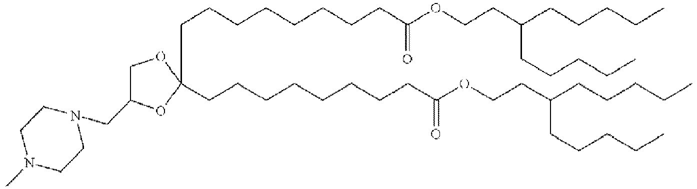

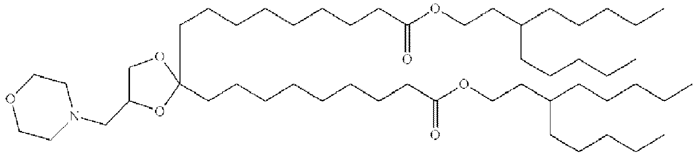

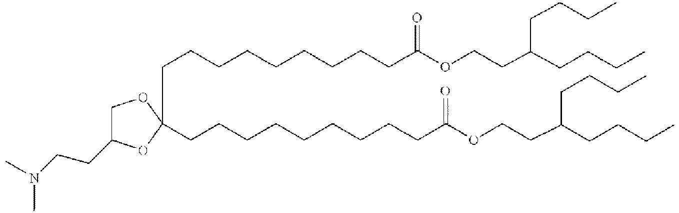

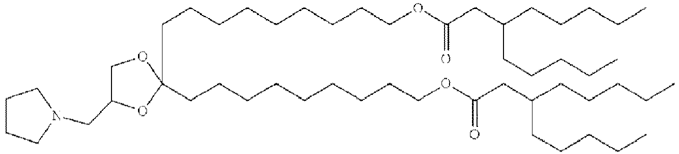

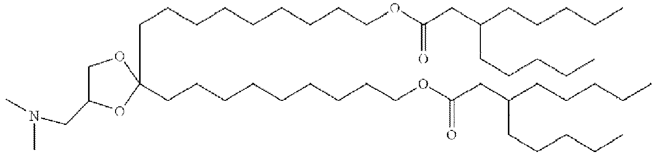

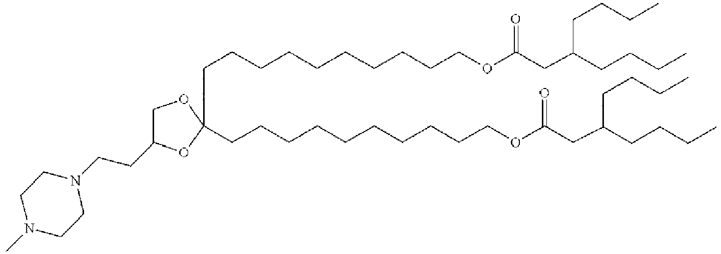

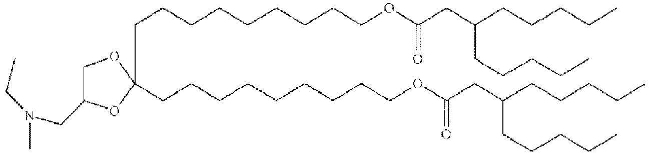

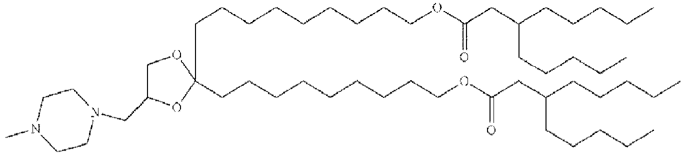

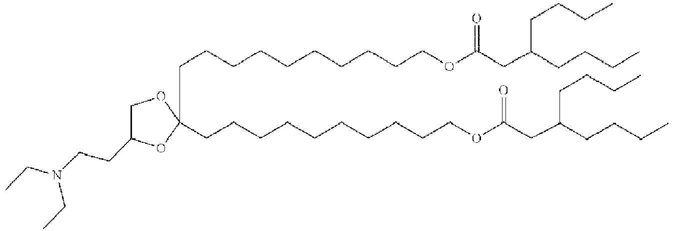

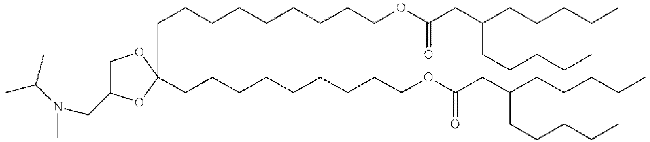

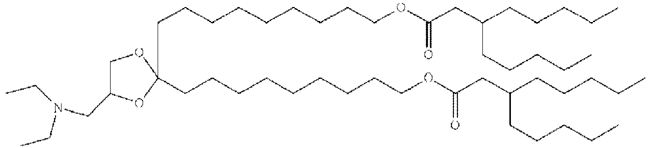

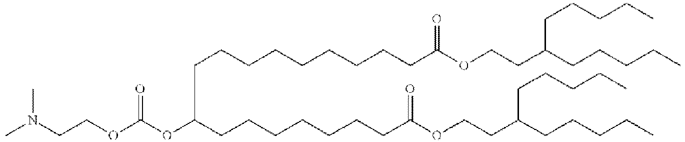

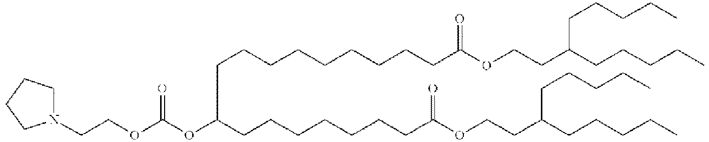

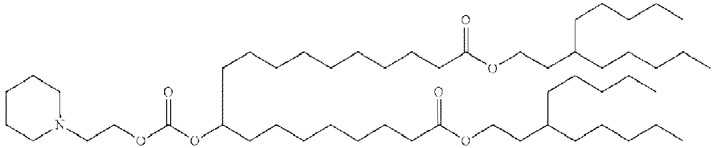

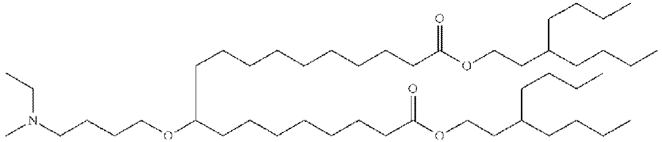

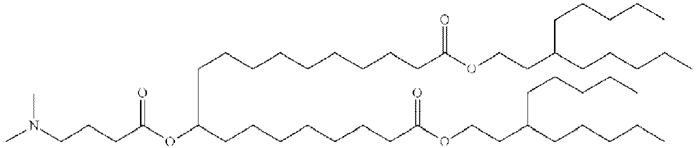

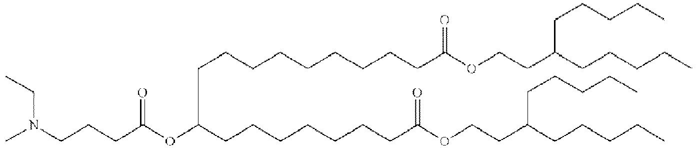

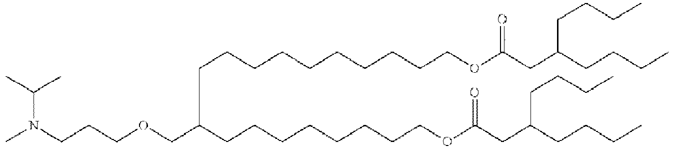

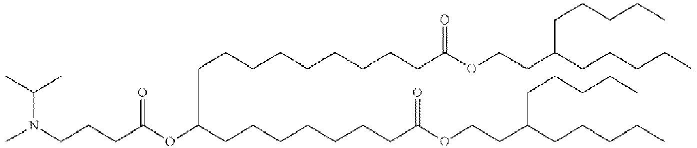

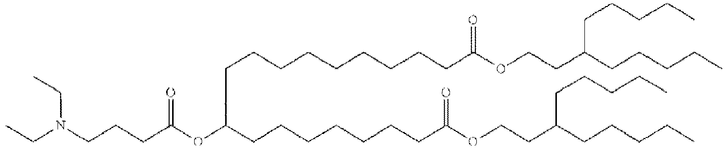

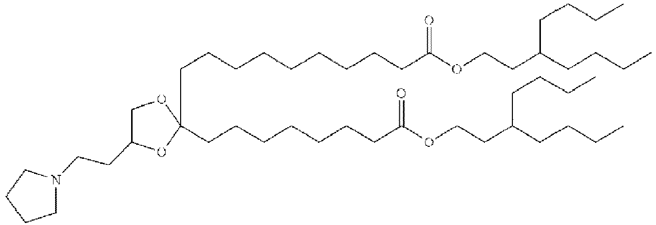

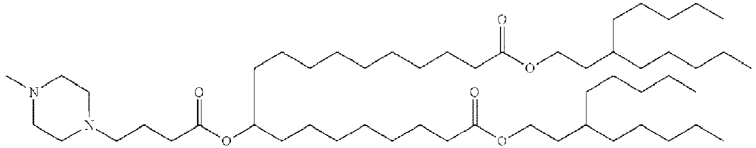

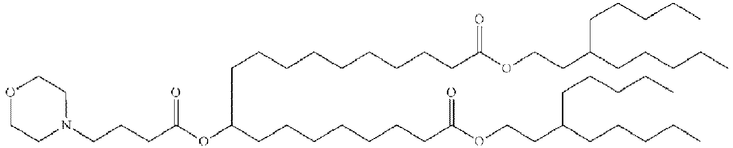

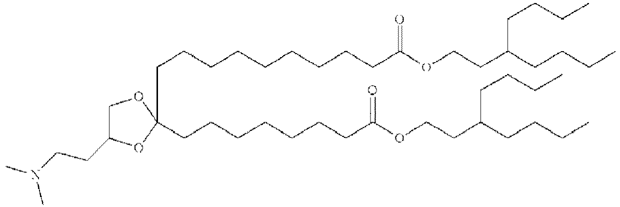

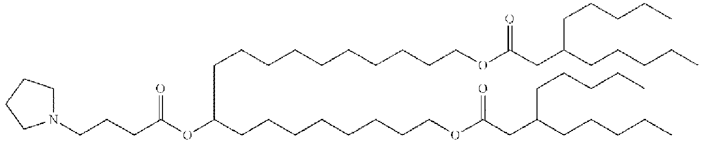

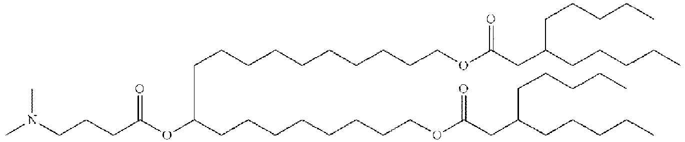

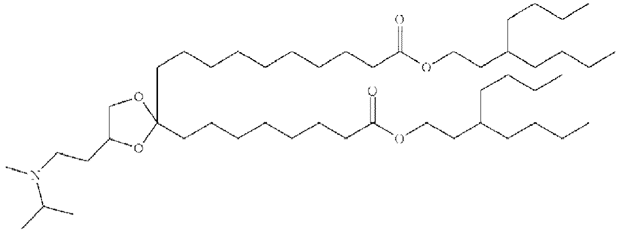

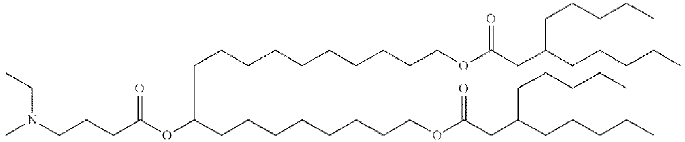

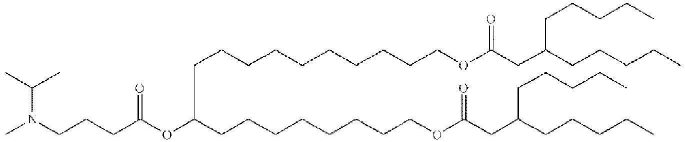

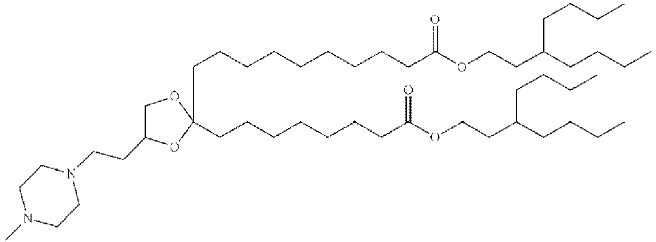

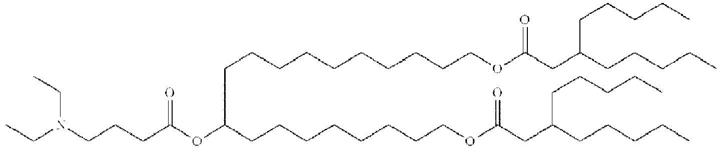

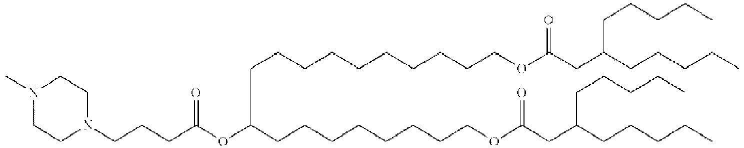

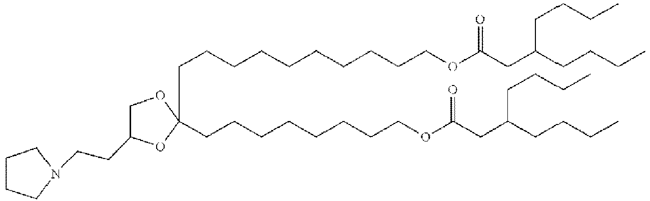

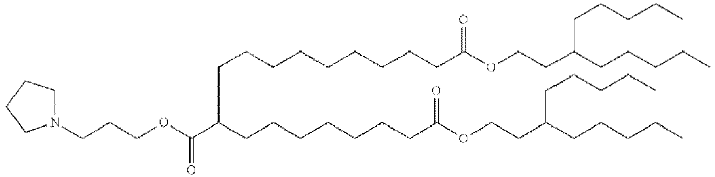

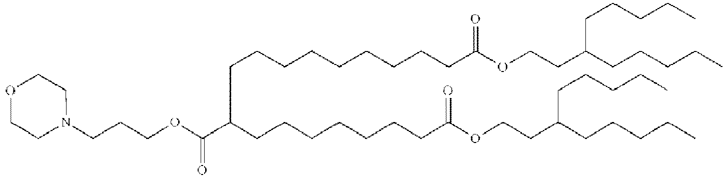

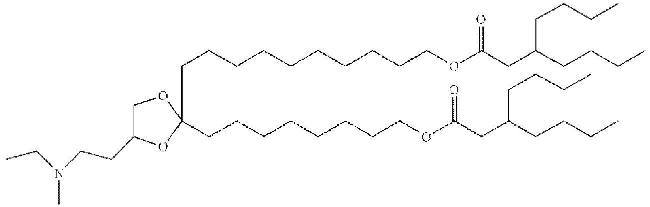

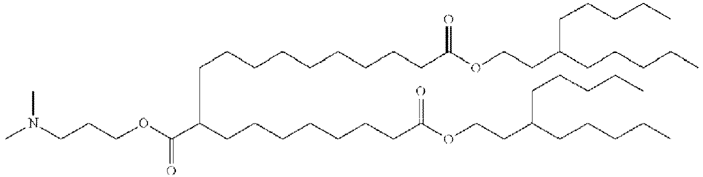

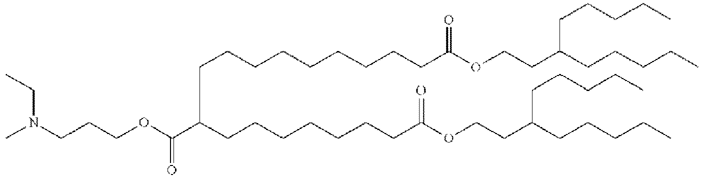

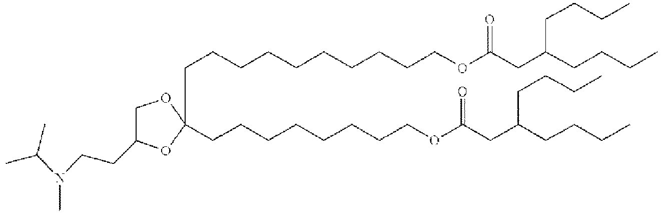

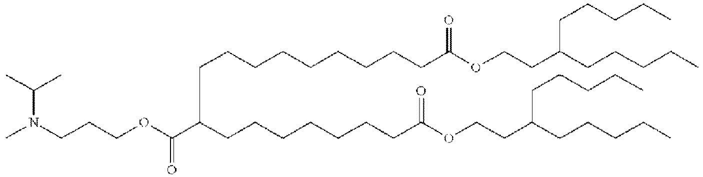

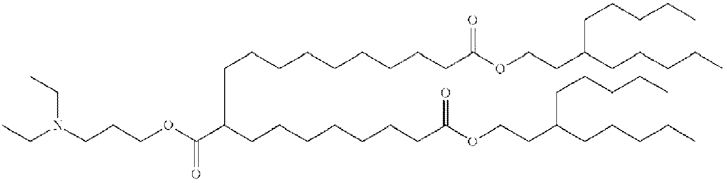

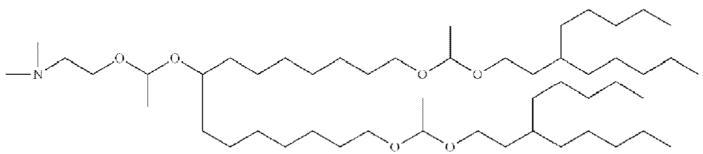

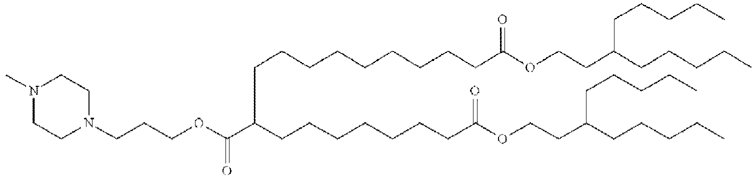

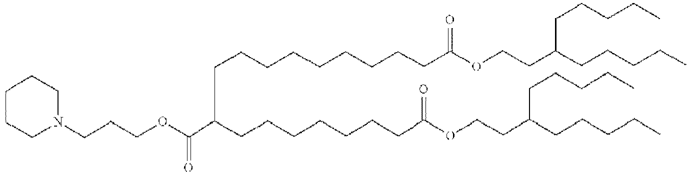

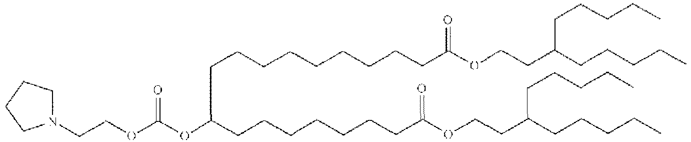

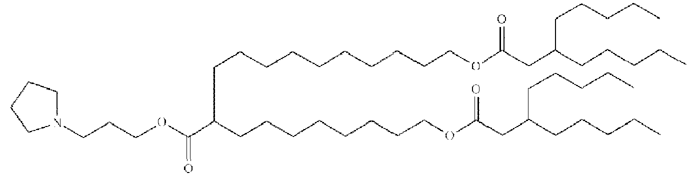

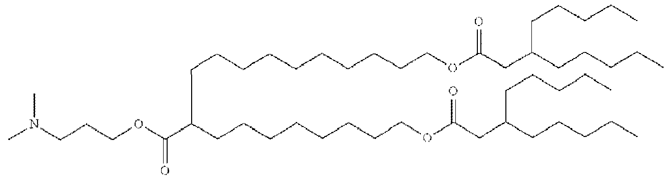

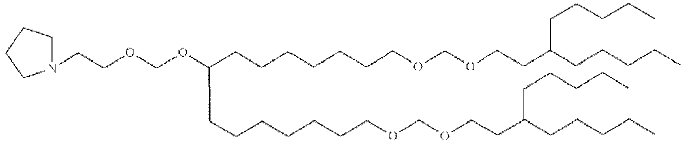

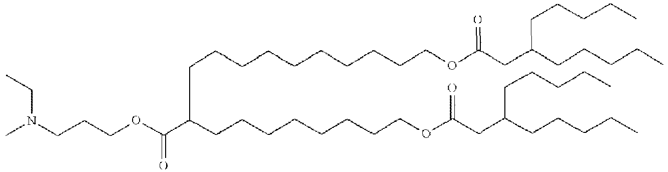

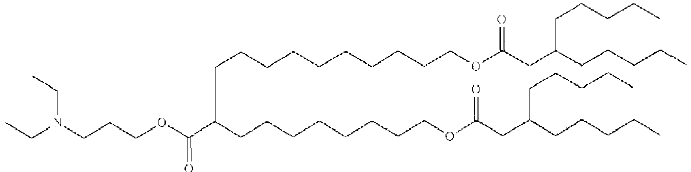

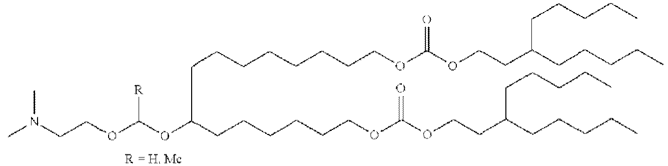

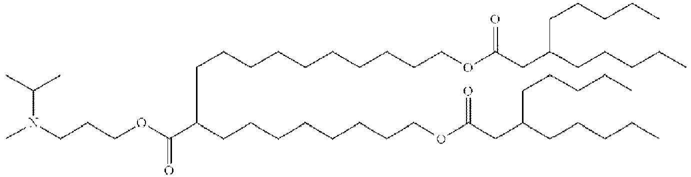

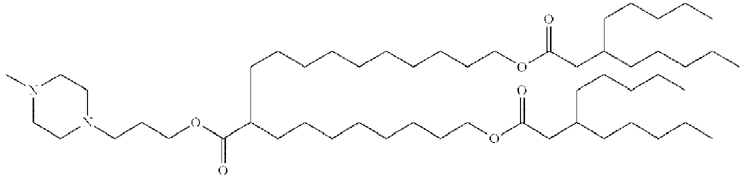

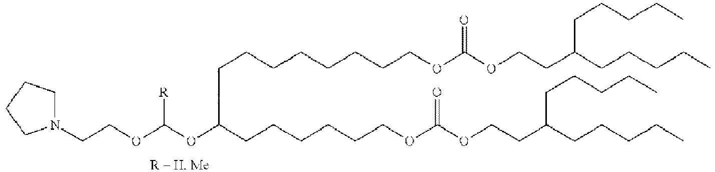

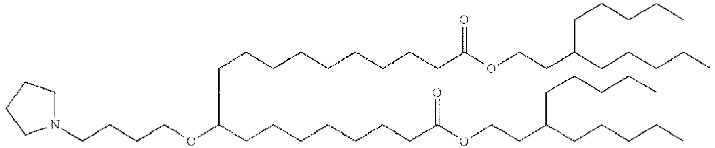

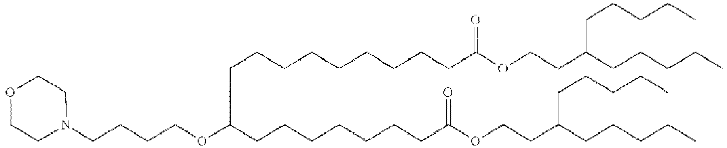

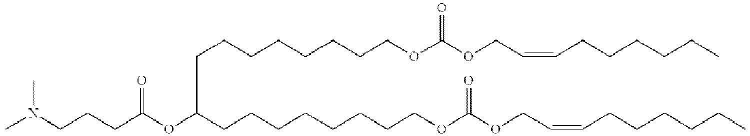

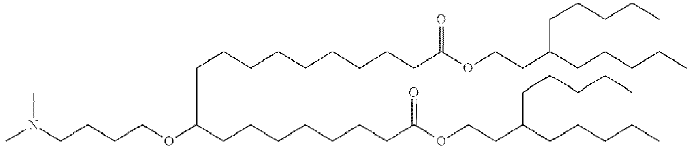

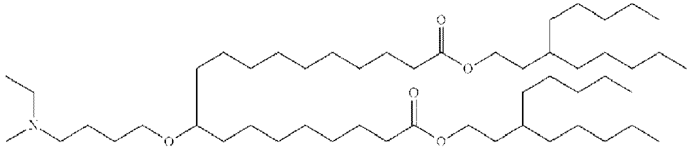

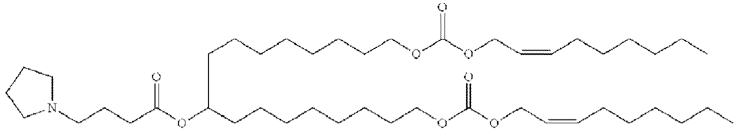

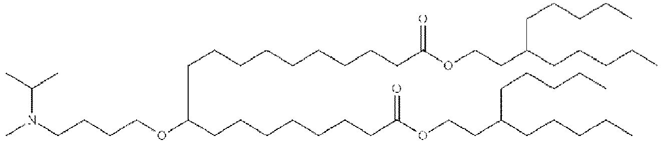

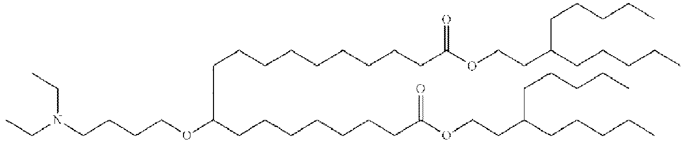

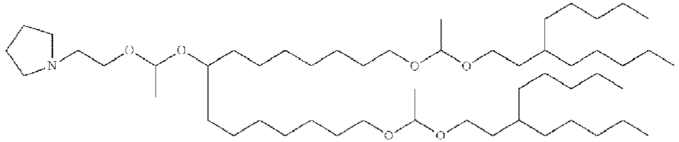

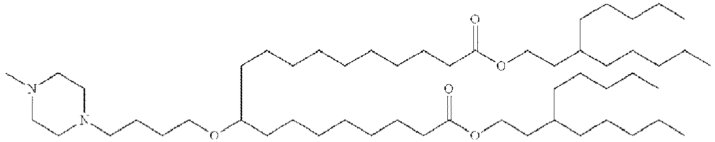

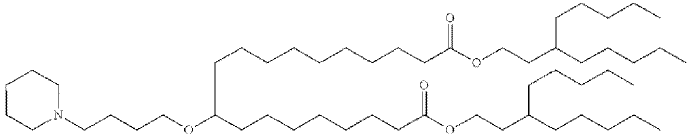

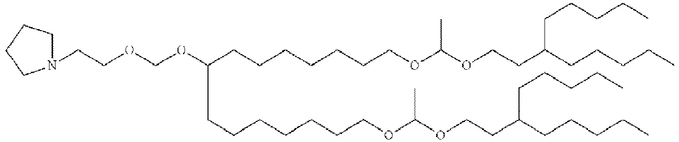

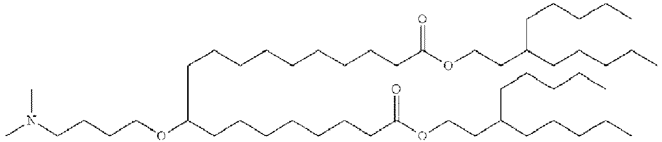

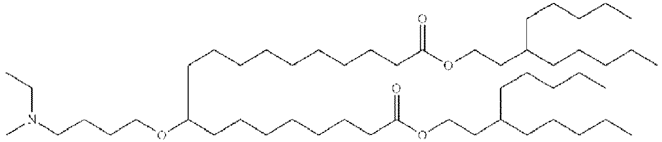

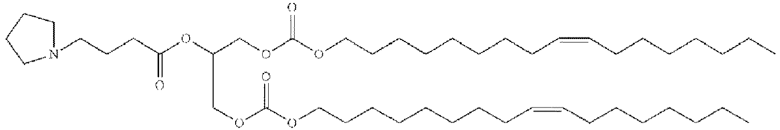

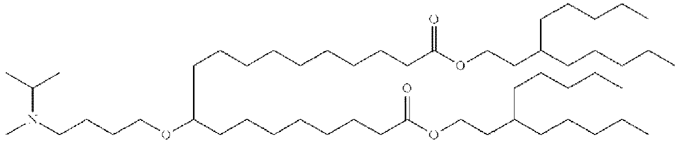

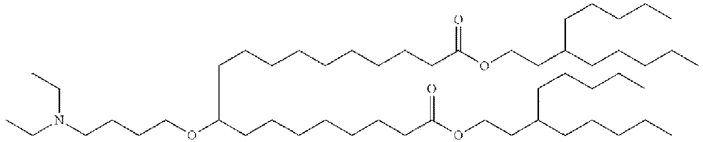

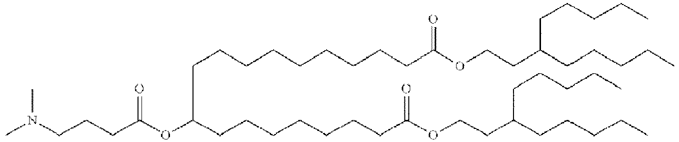

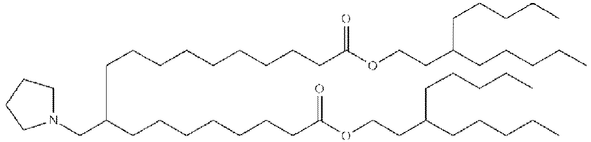

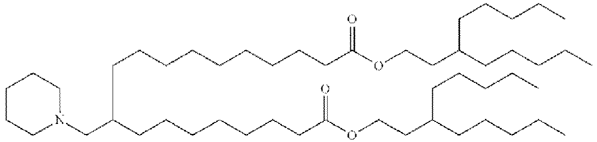

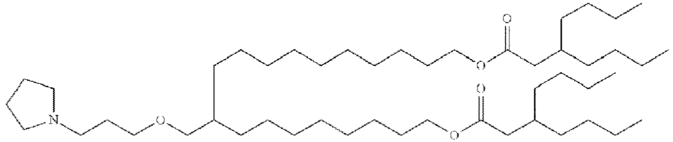

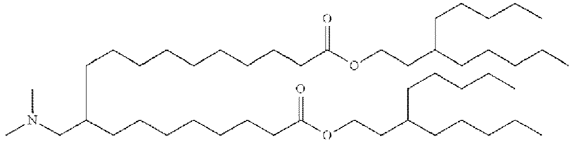

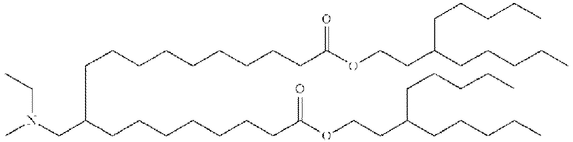

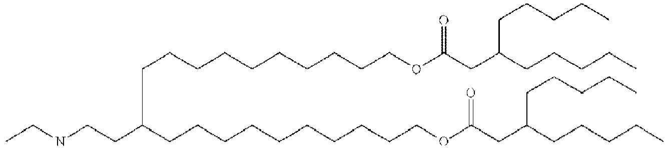

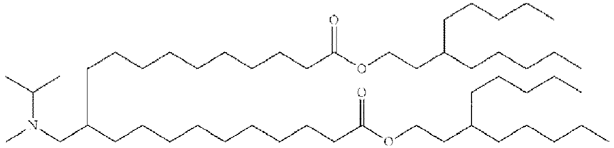

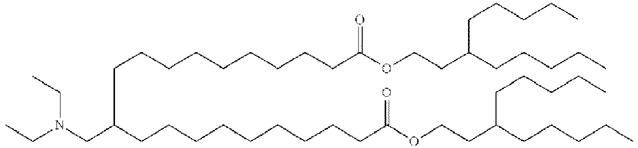

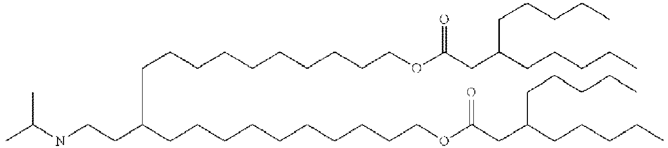

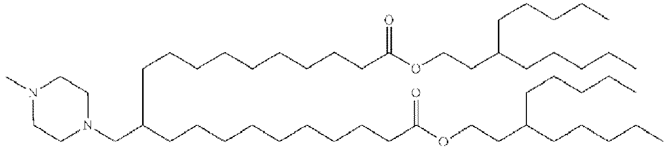

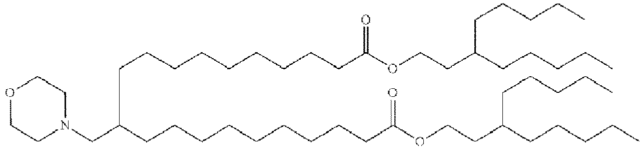

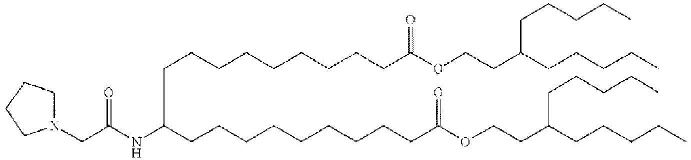

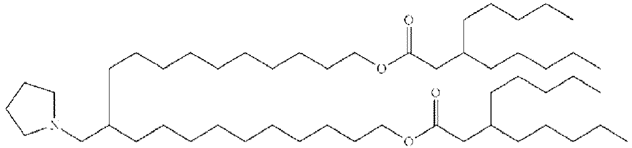

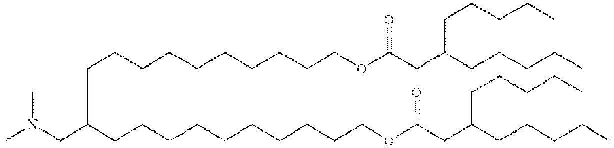

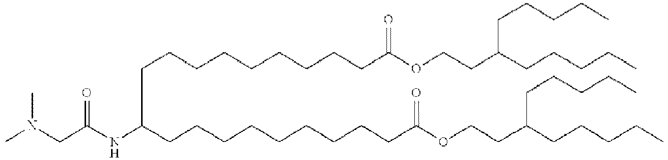

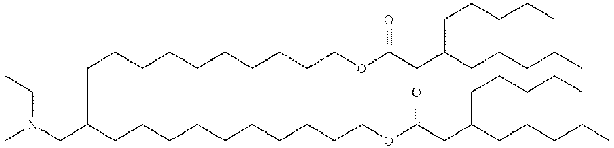

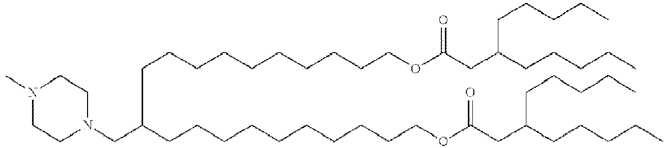

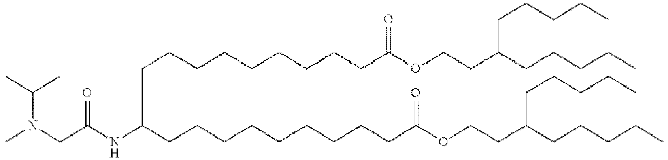

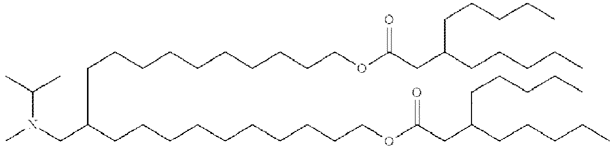

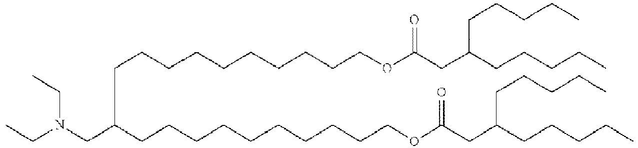

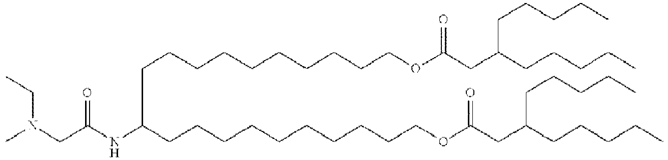

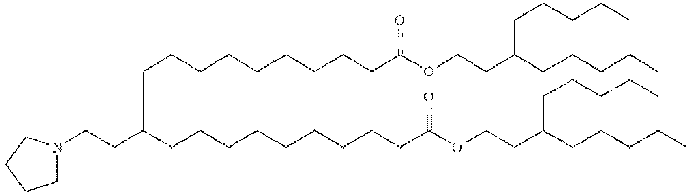

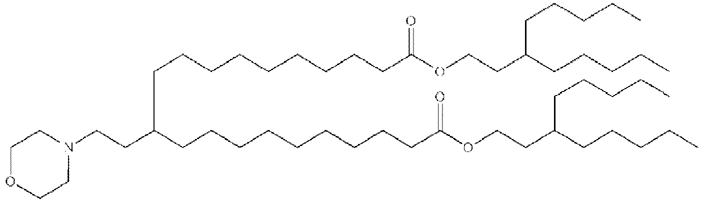

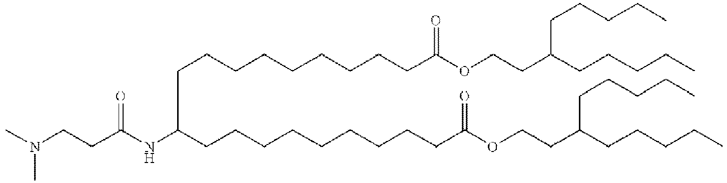

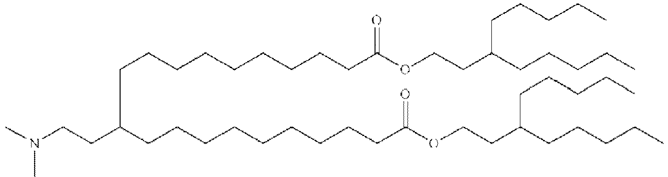

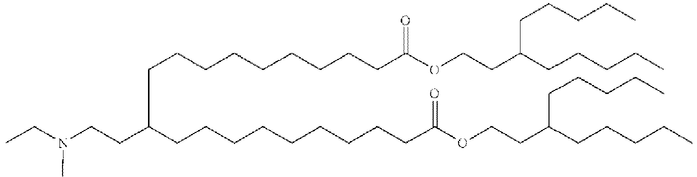

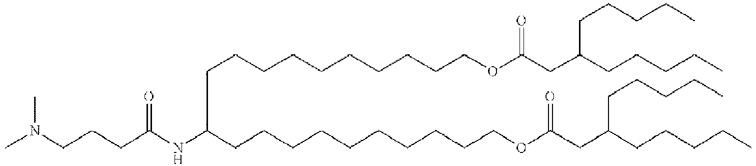

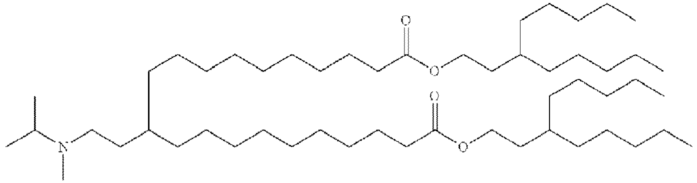

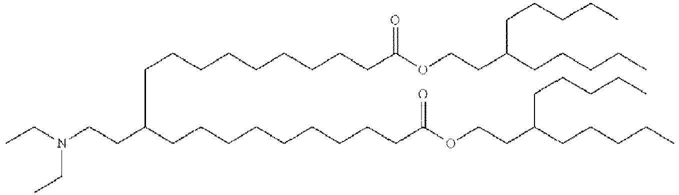

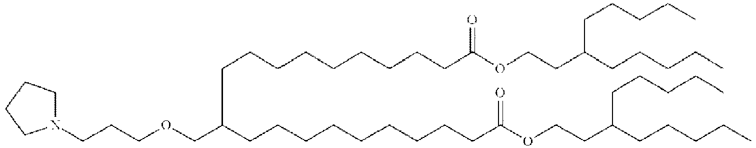

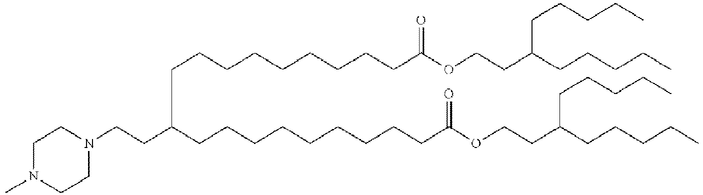

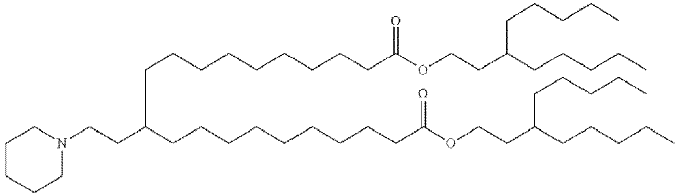

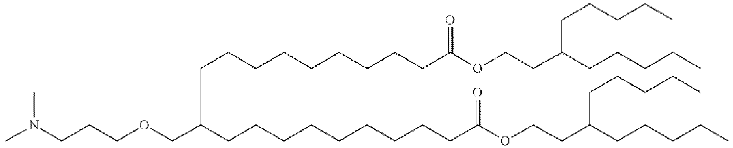

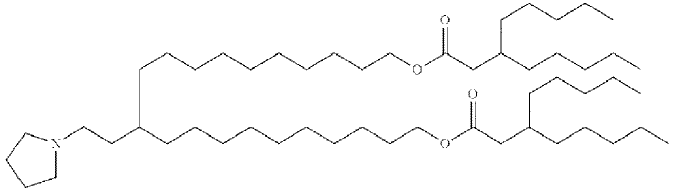

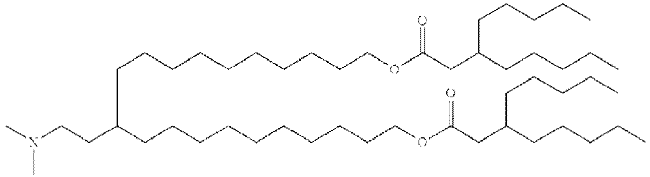

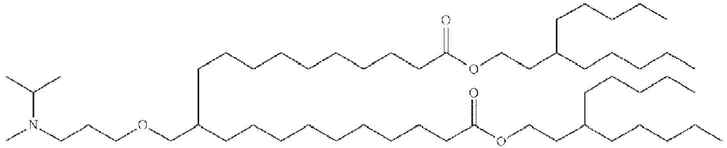

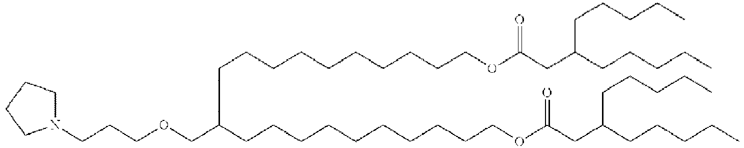

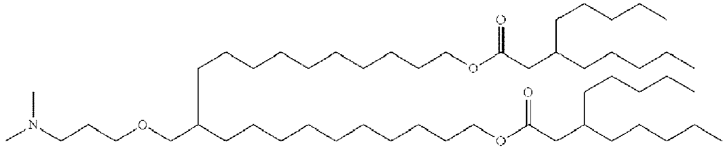

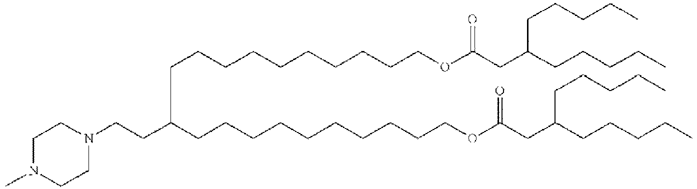

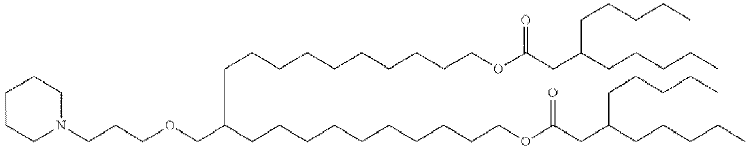

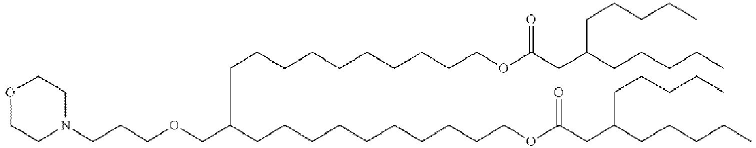

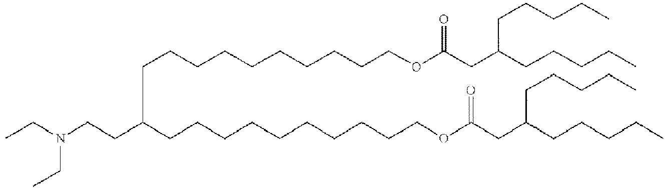

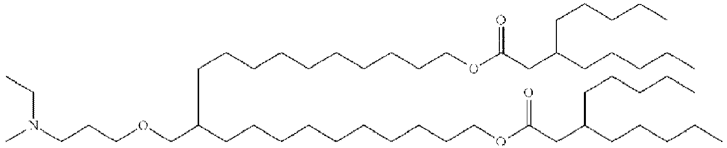

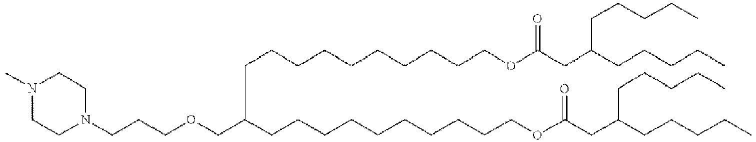

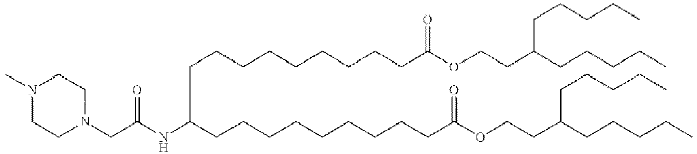

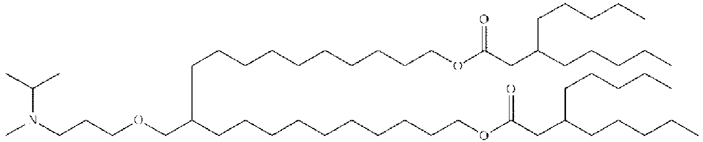

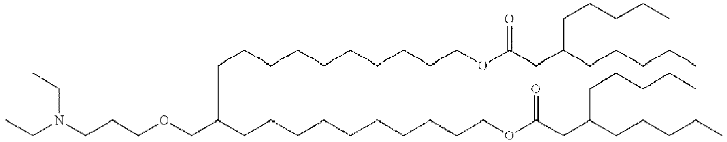

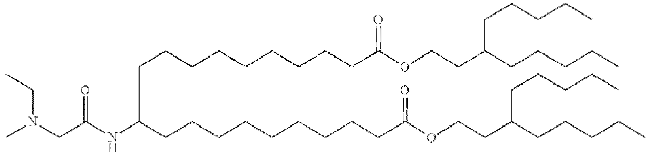

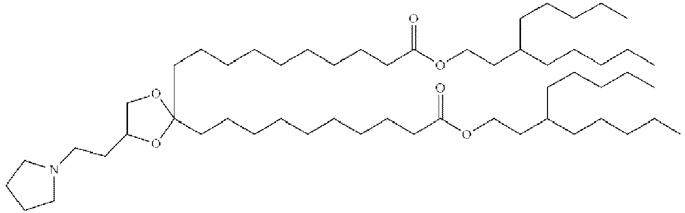

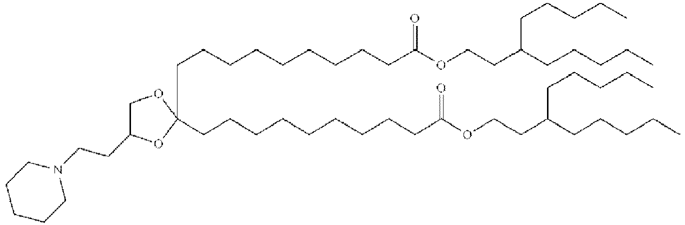

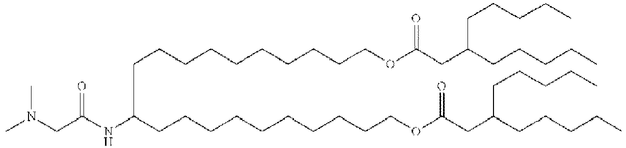

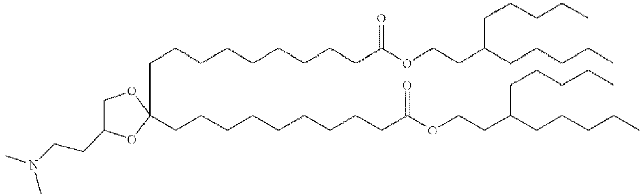

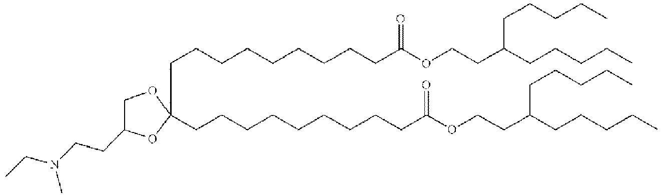

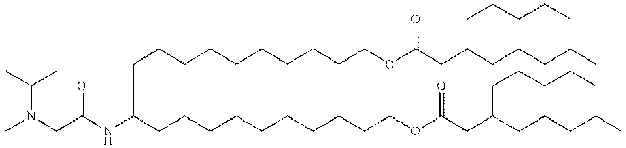

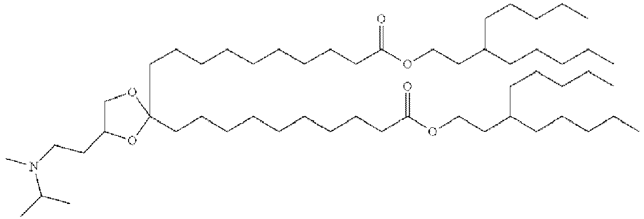

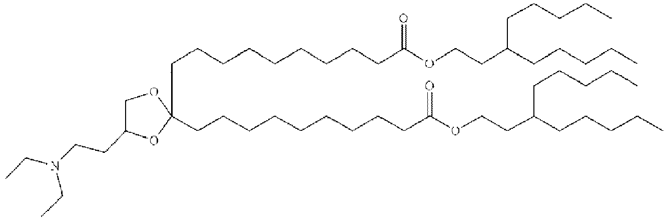

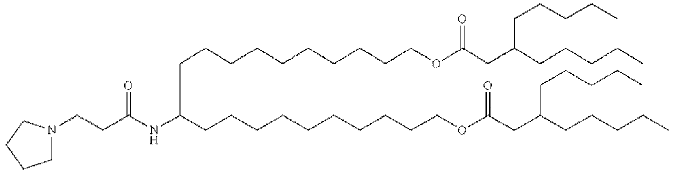

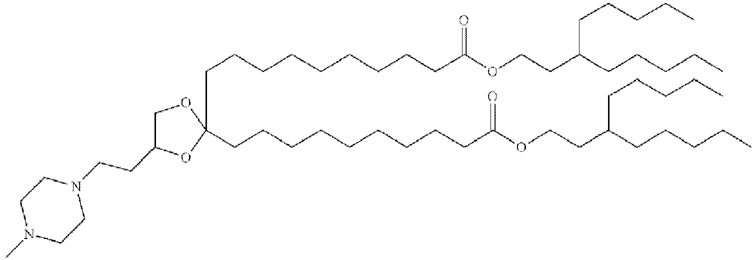

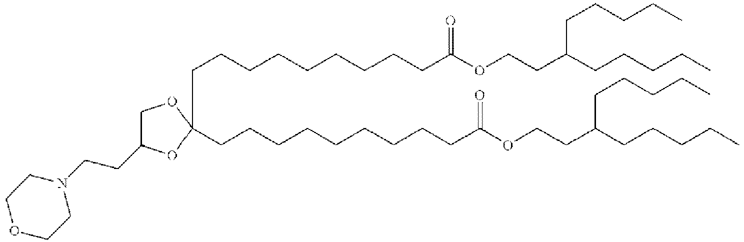

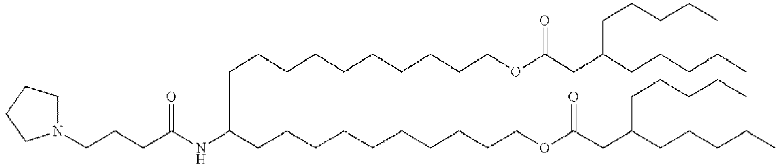

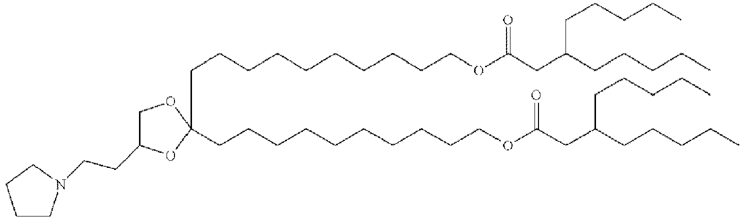

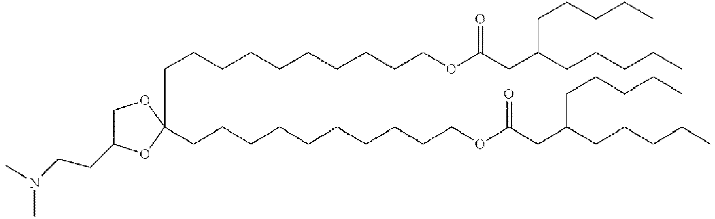

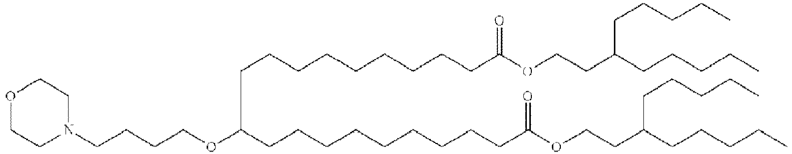

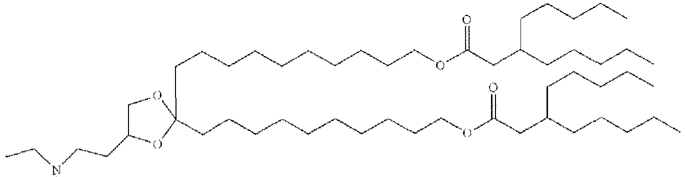

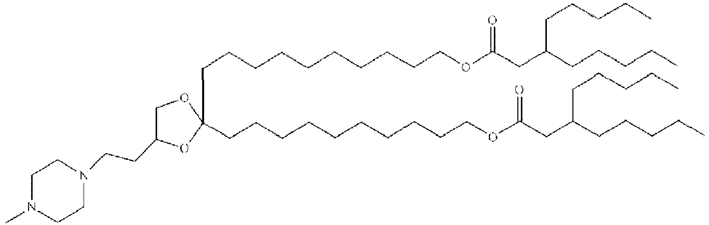

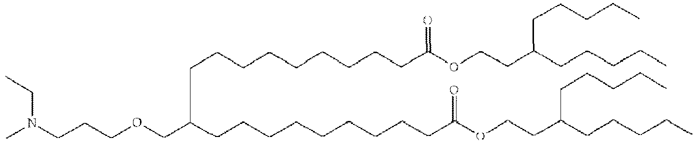

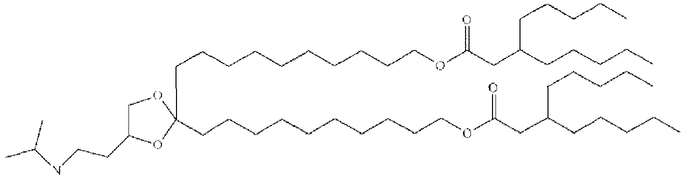

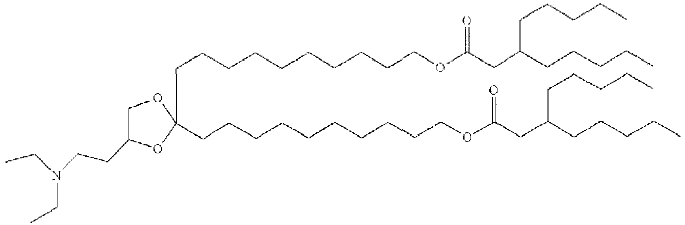

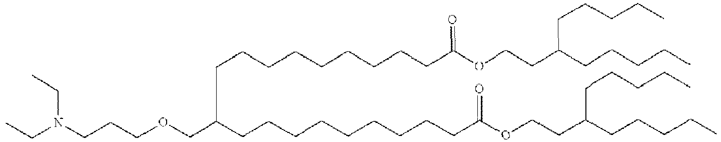

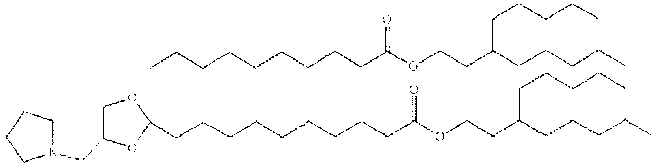

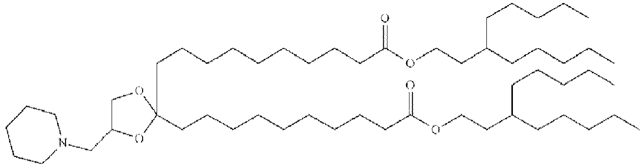

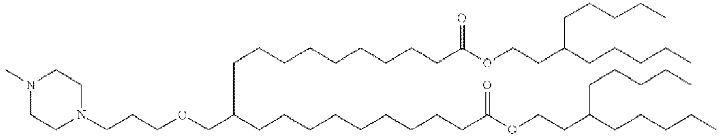

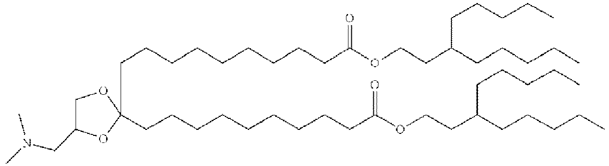

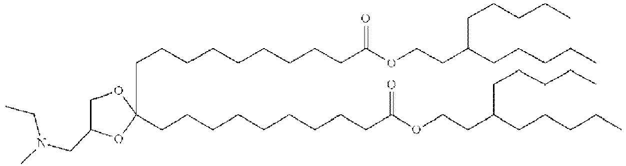

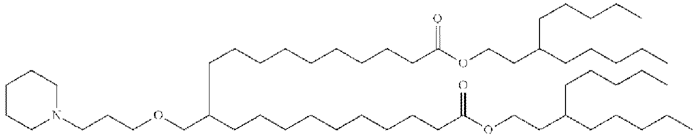

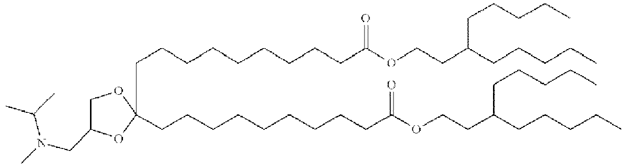

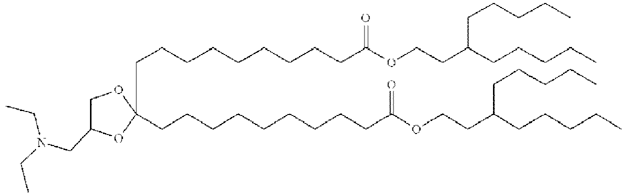

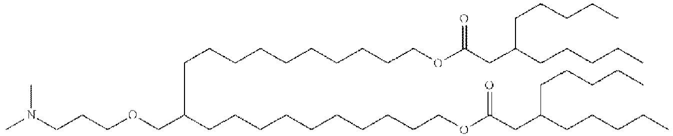

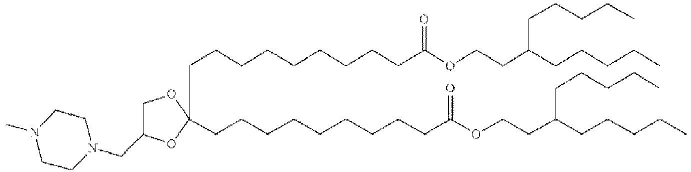

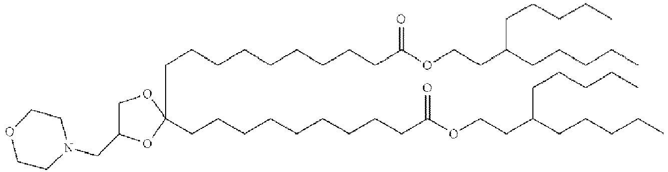

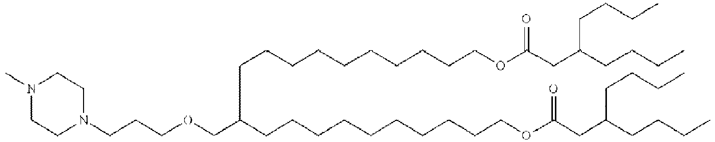

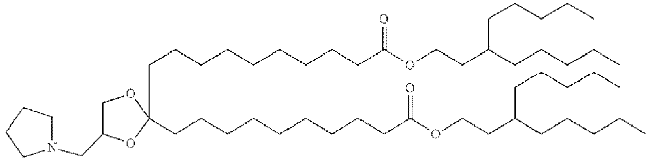

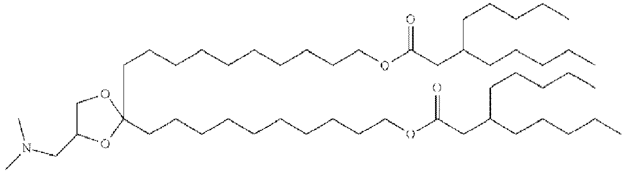

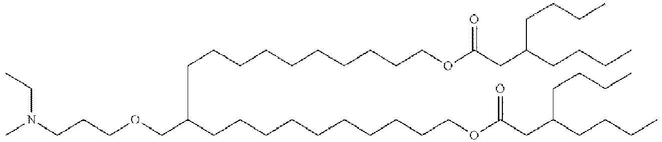

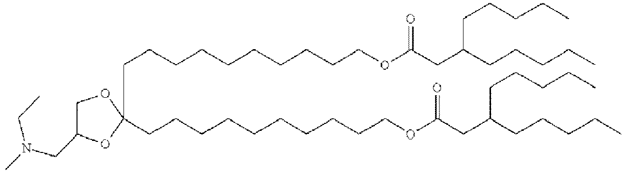

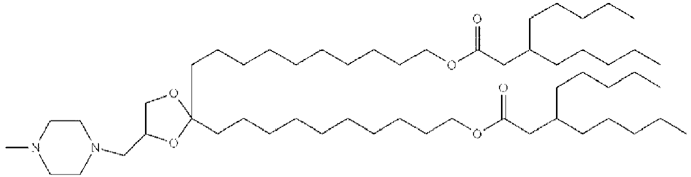

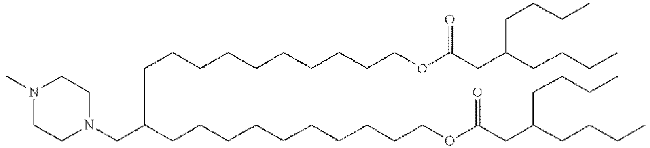

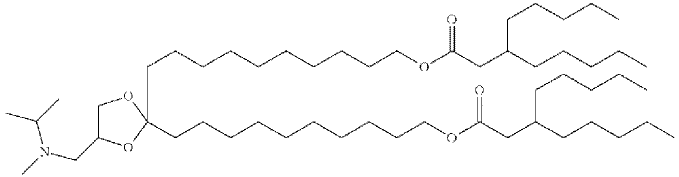

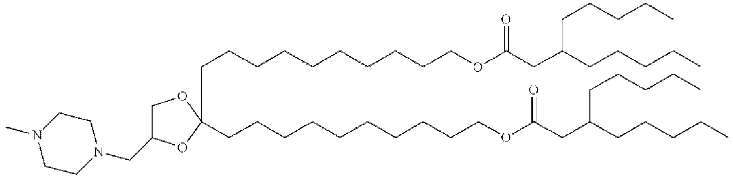

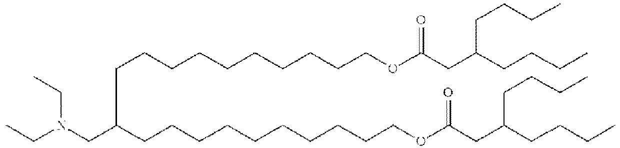

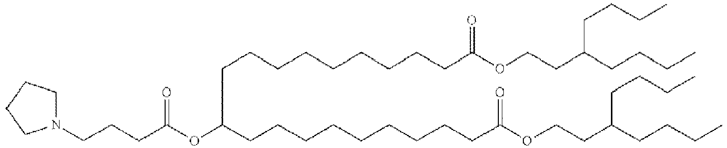

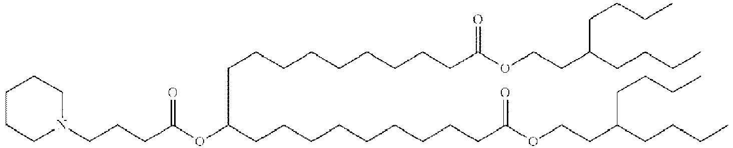

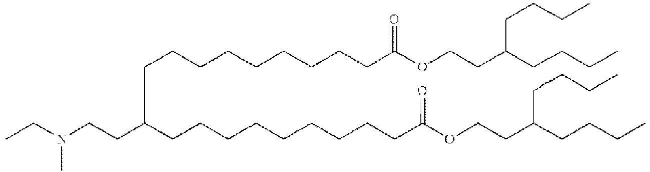

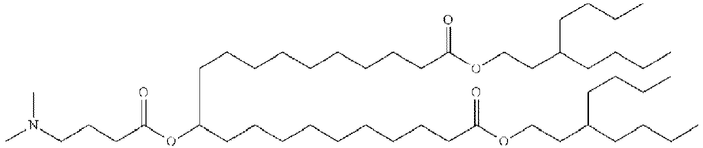

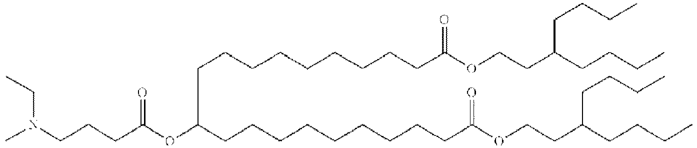

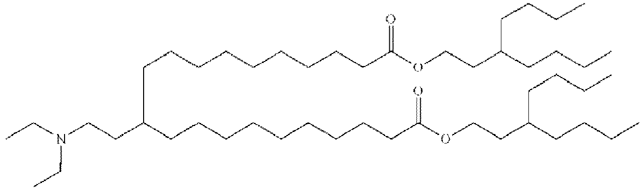

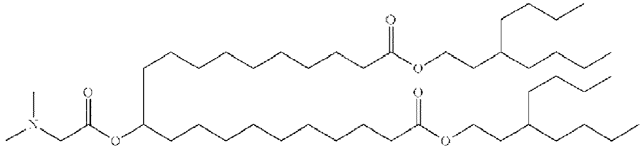

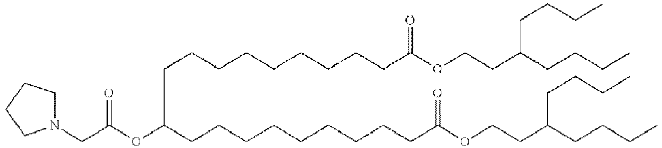

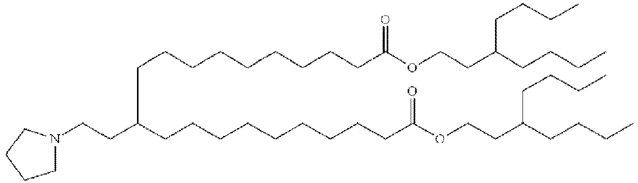

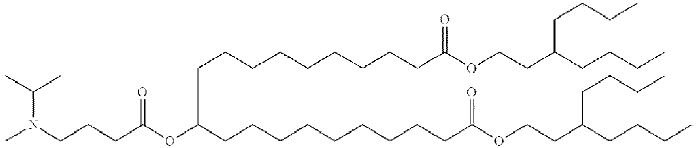

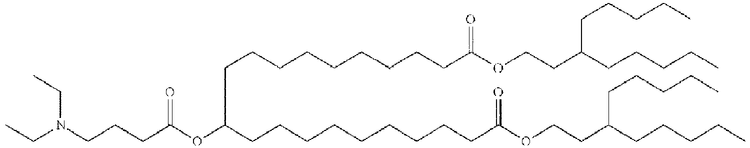

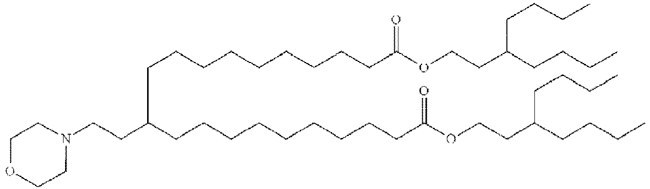

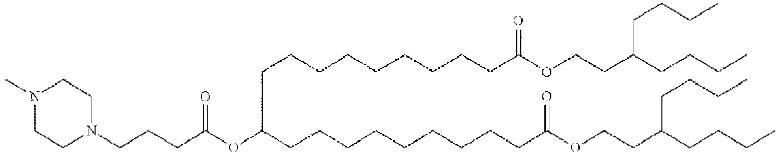

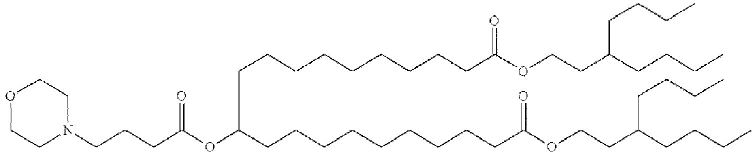

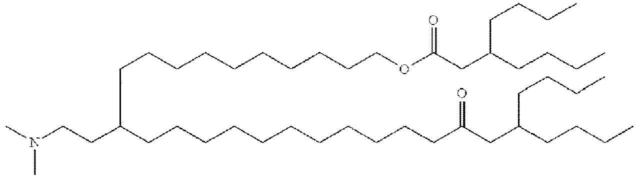

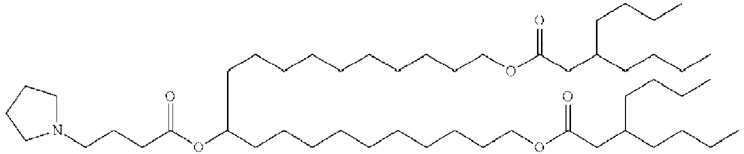

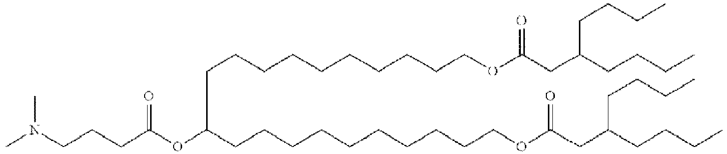

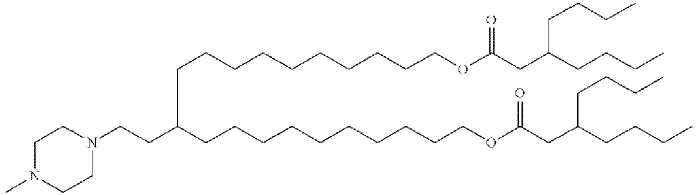

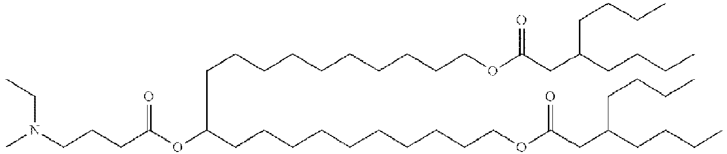

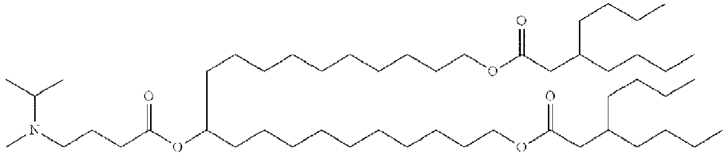

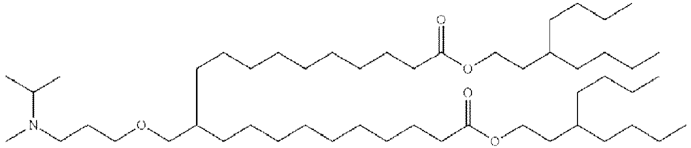

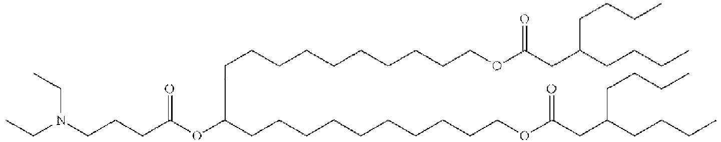

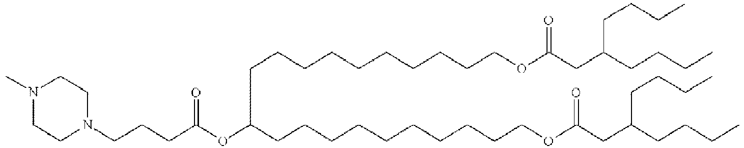

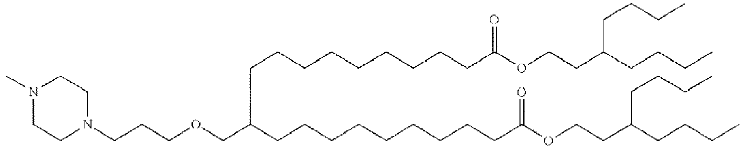

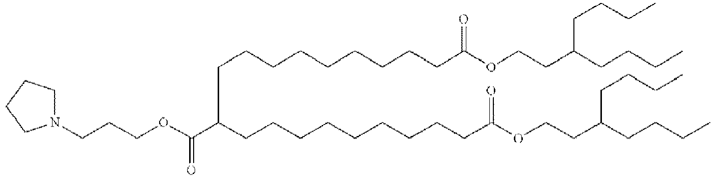

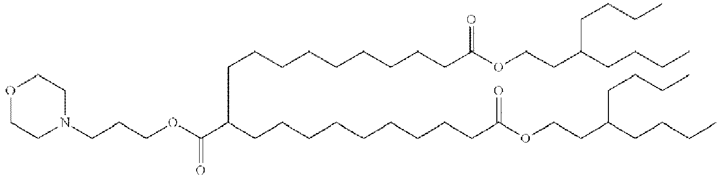

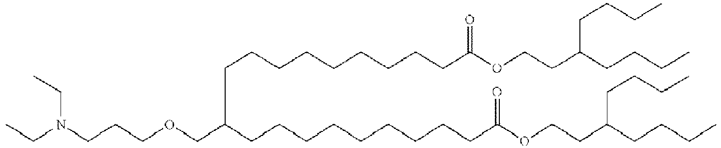

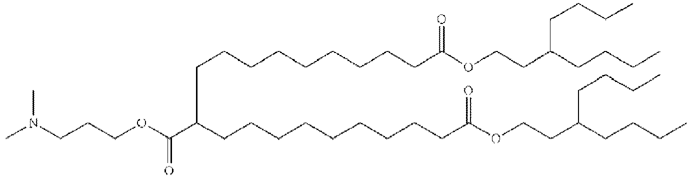

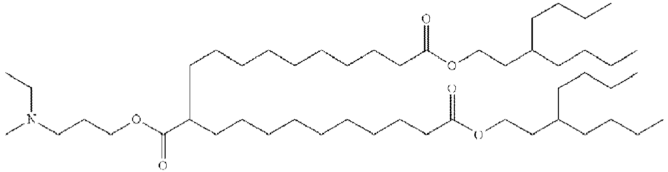

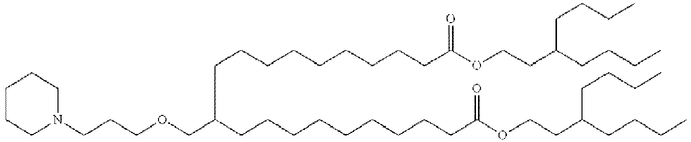

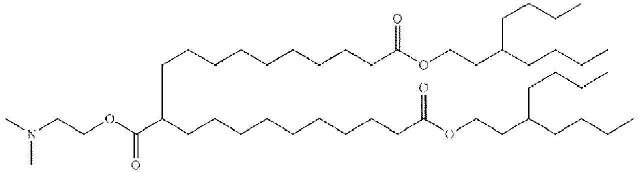

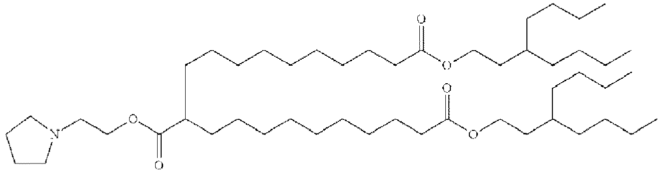

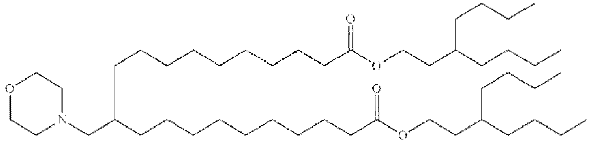

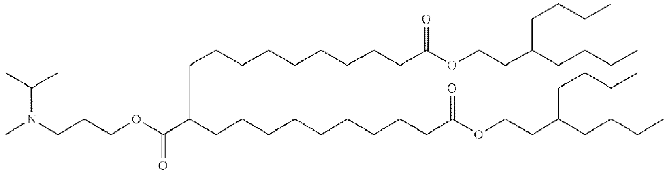

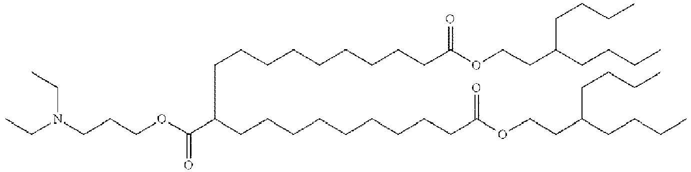

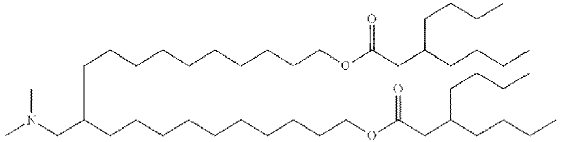

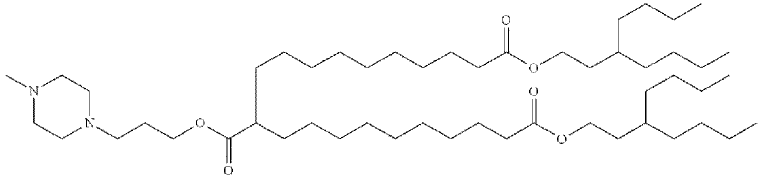

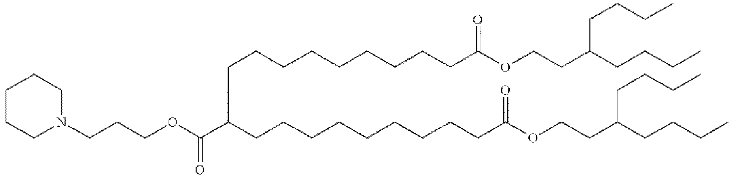

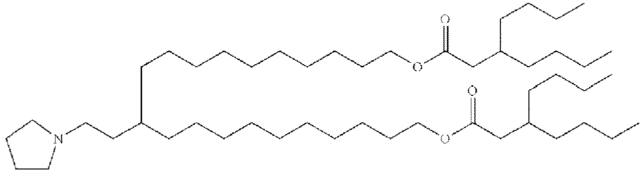

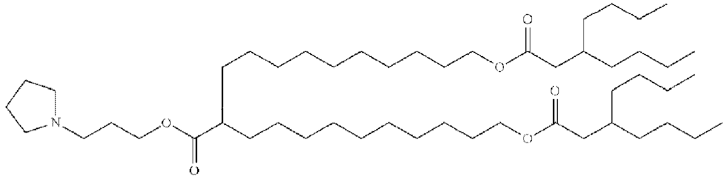

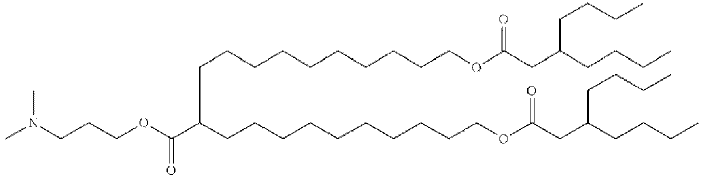

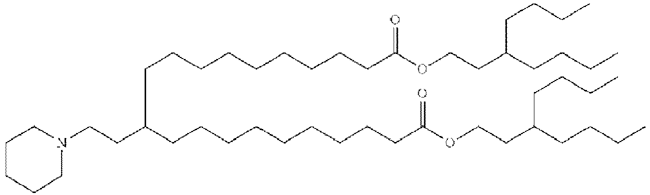

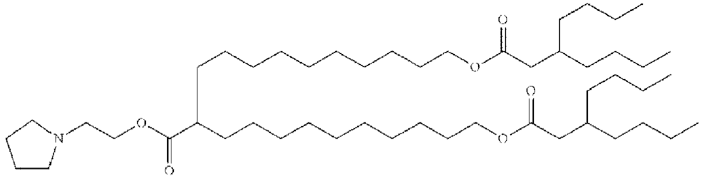

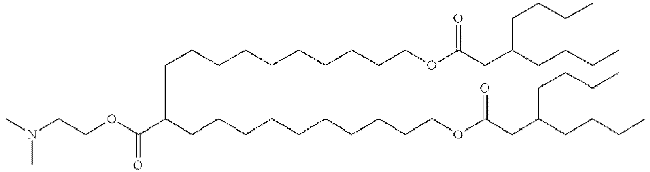

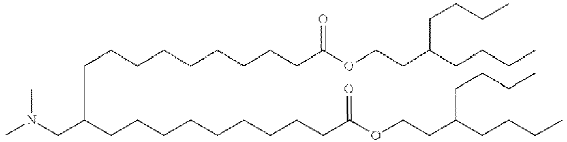

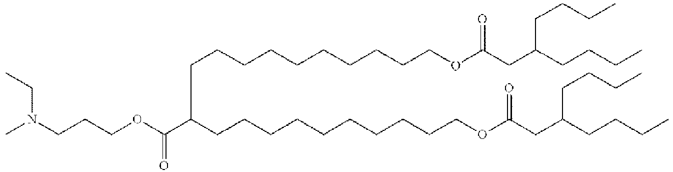

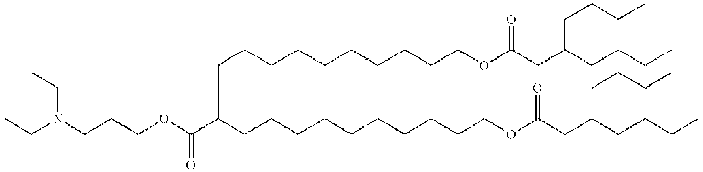

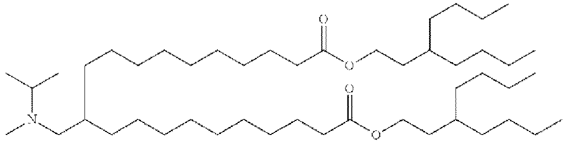

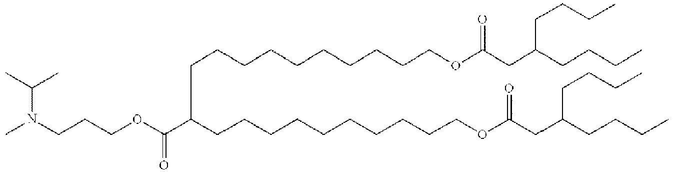

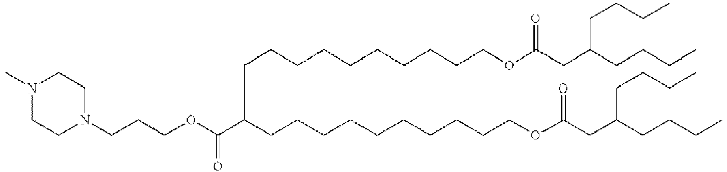

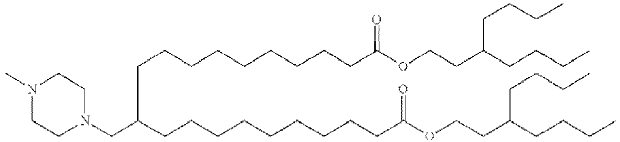

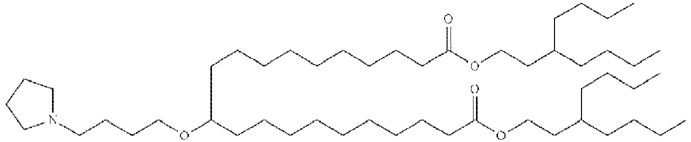

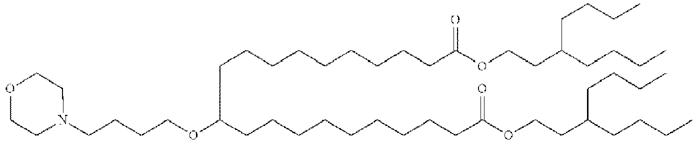

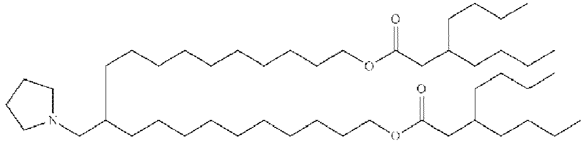

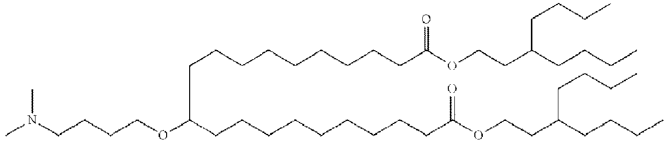

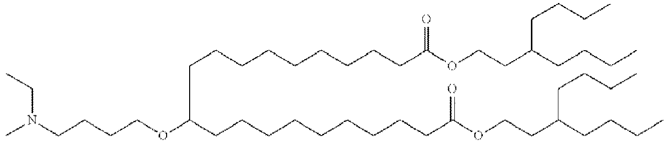

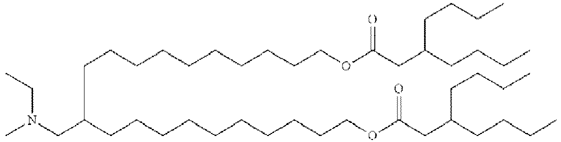

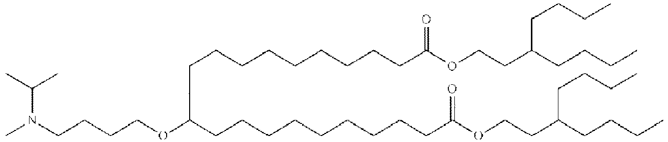

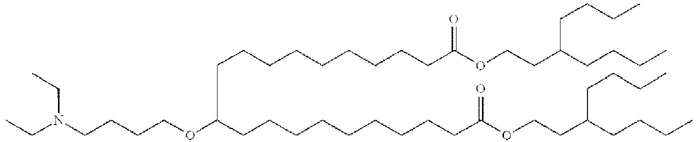

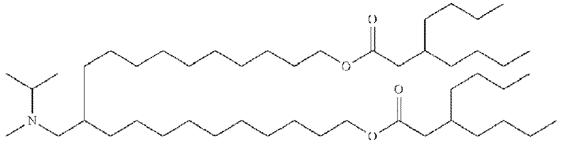

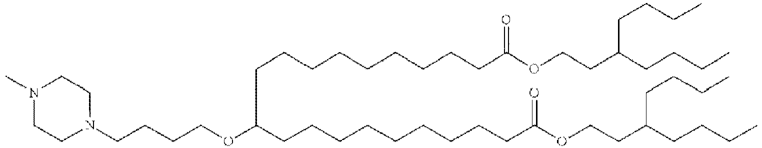

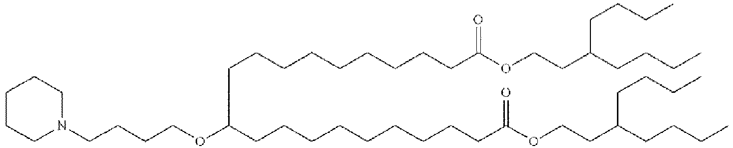

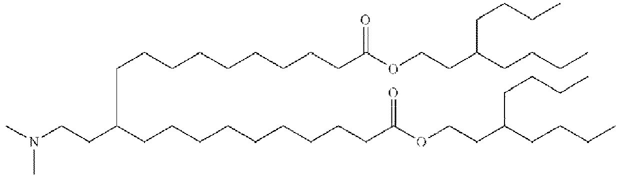

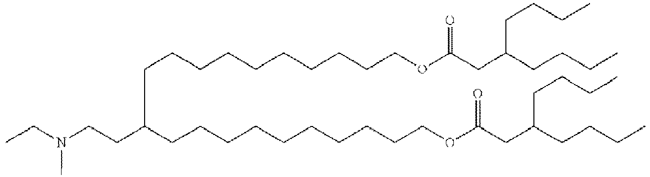

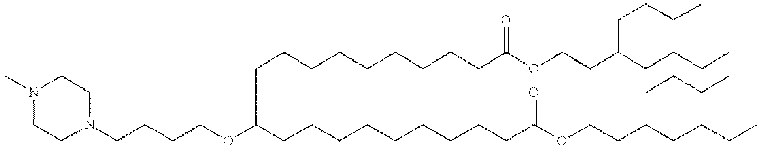

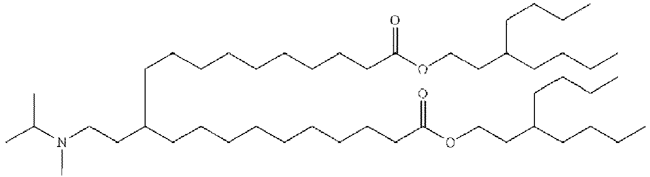

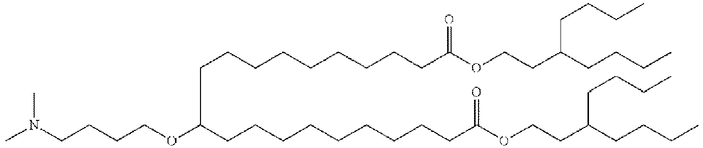

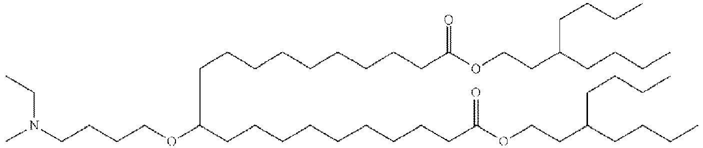

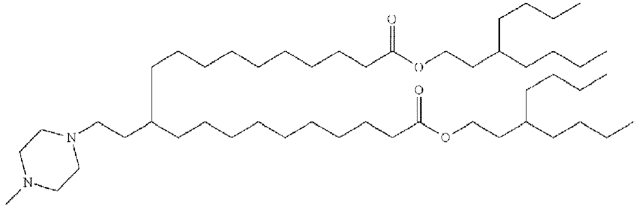

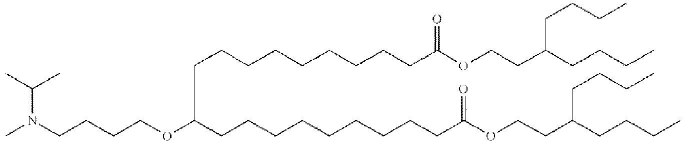

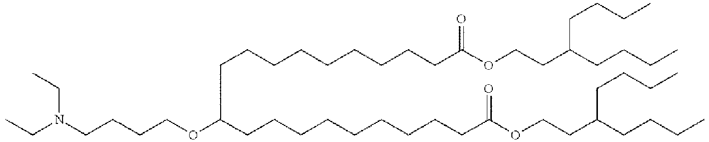

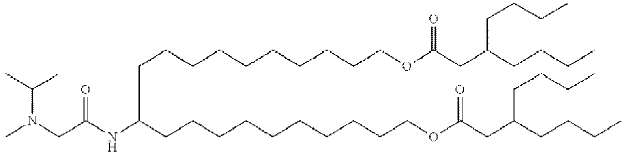

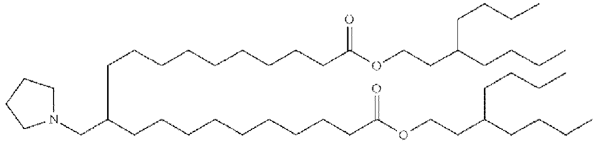

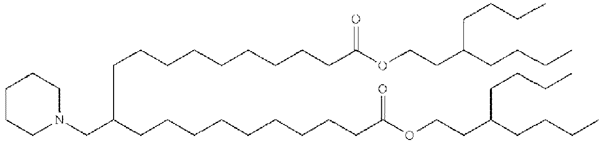

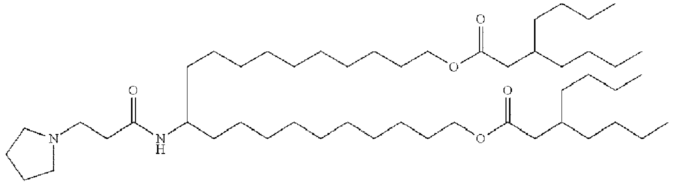

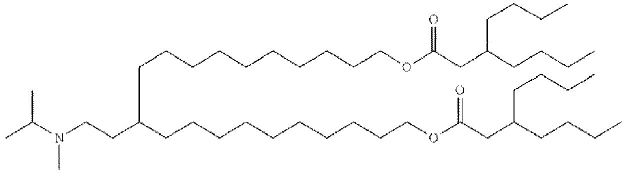

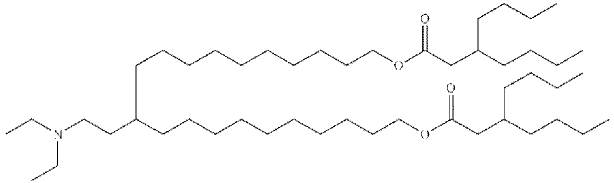

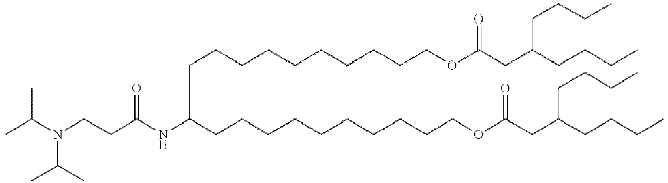

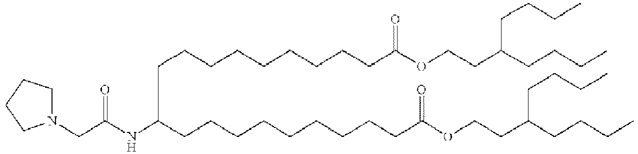

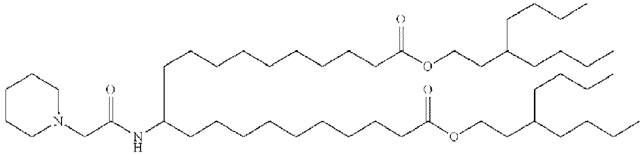

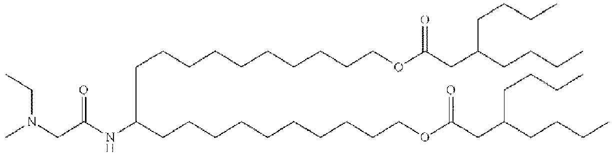

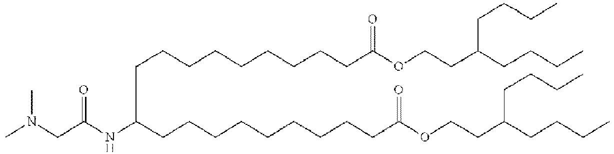

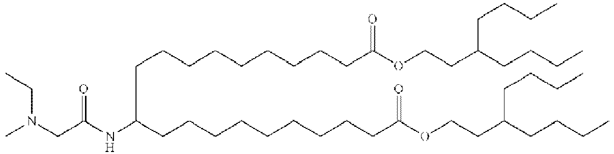

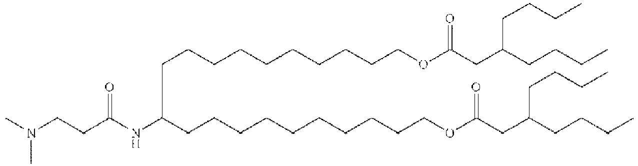

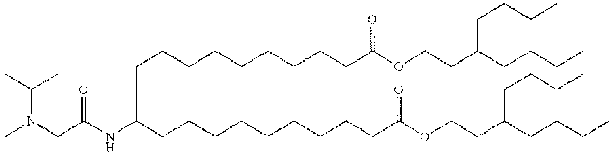

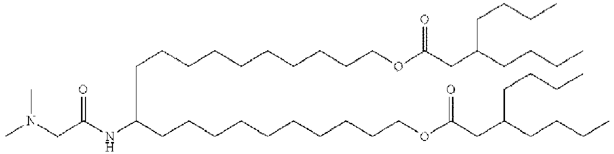

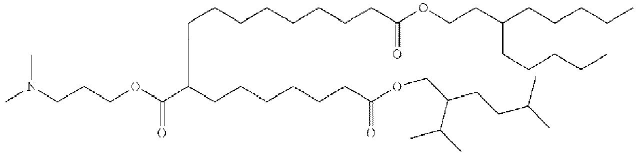

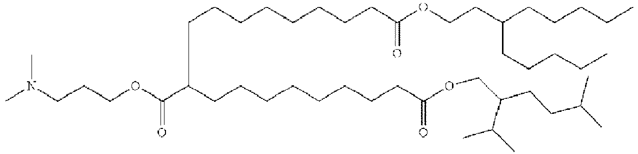

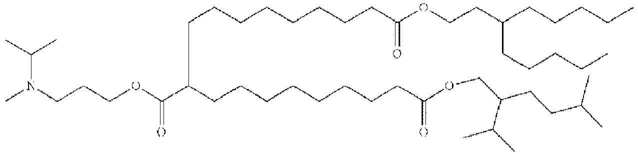

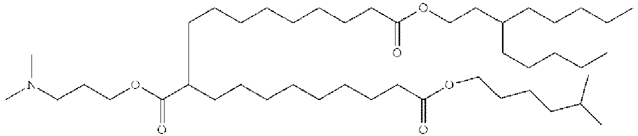

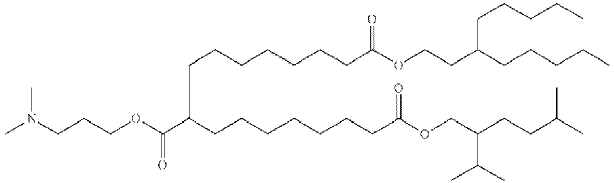

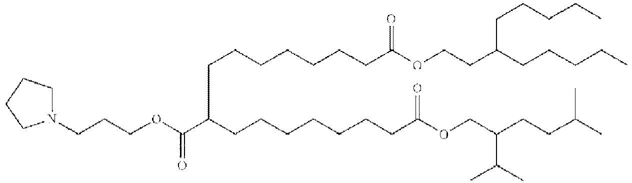

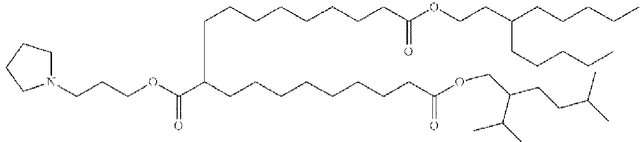

In one embodiment, the cationic lipid has a structure of one of the following compounds, and salts thereof:

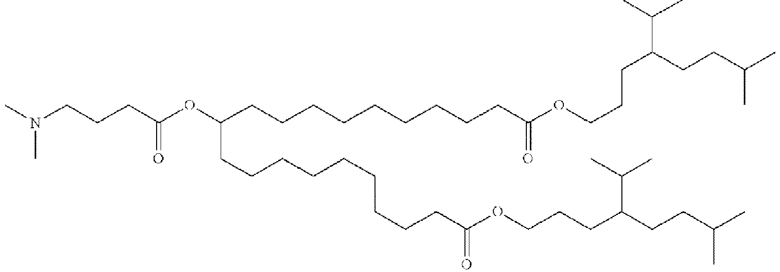

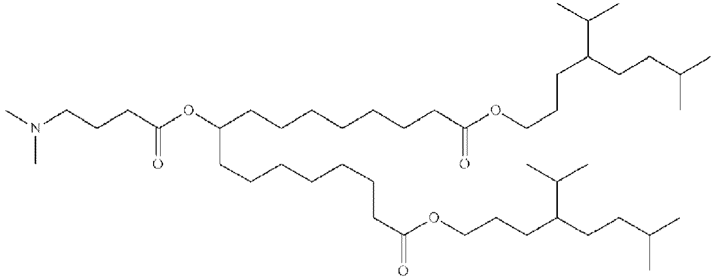

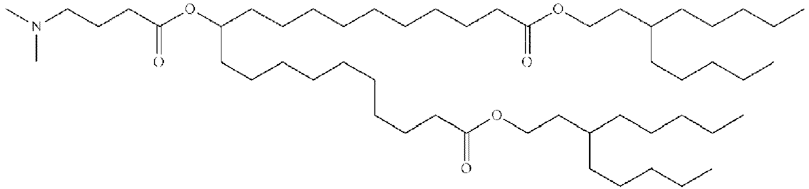

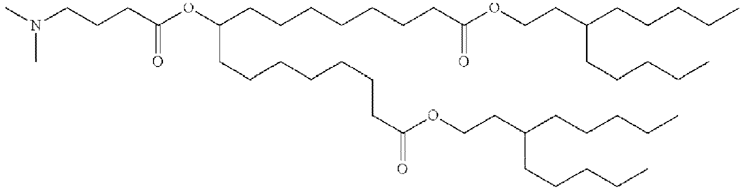

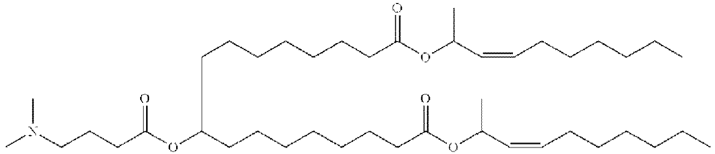

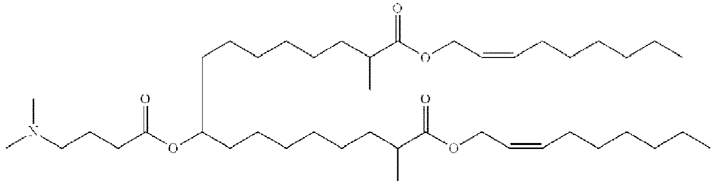

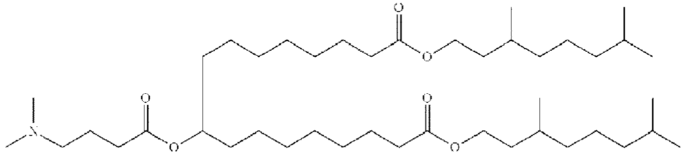

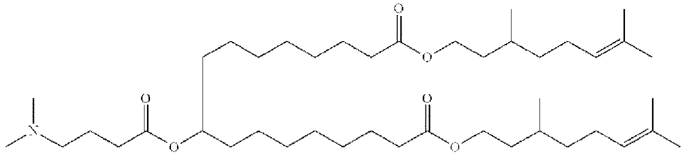

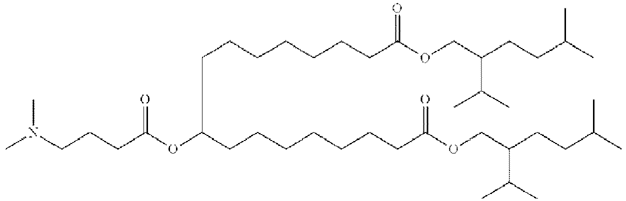

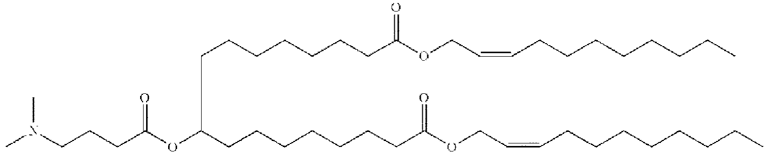

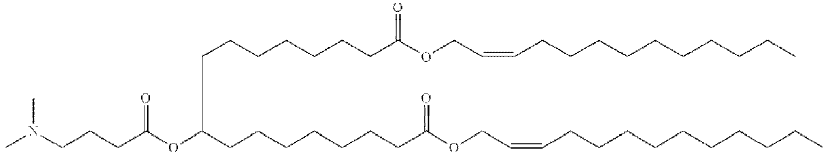

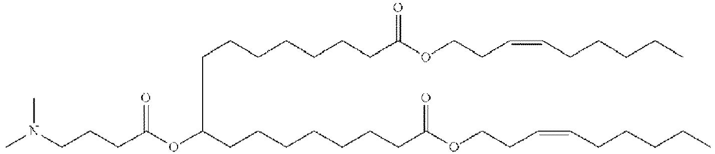

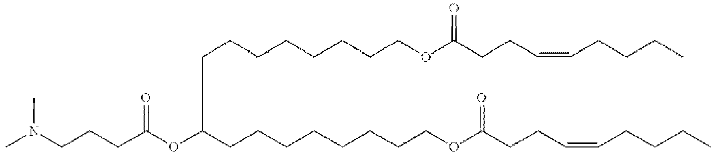

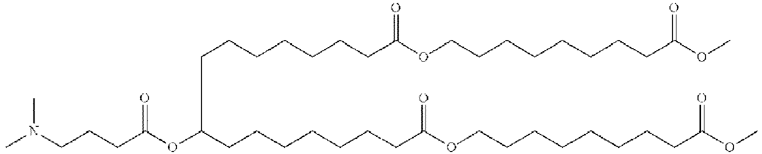

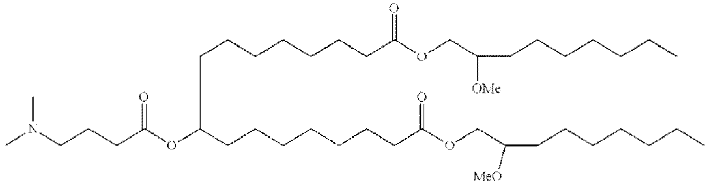

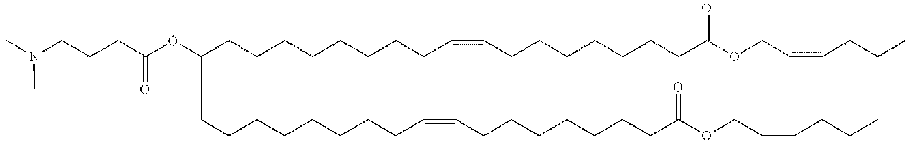

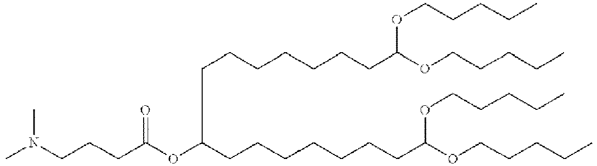

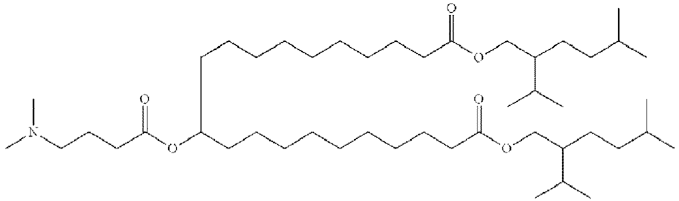

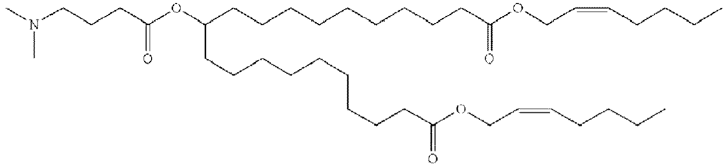

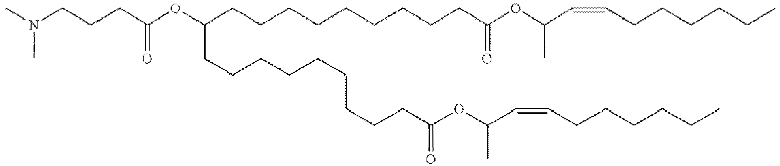

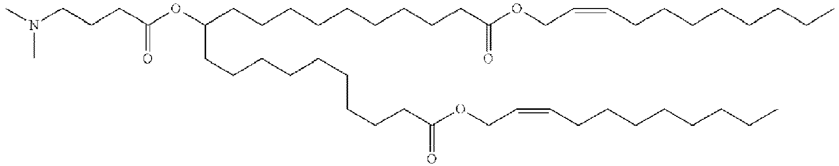

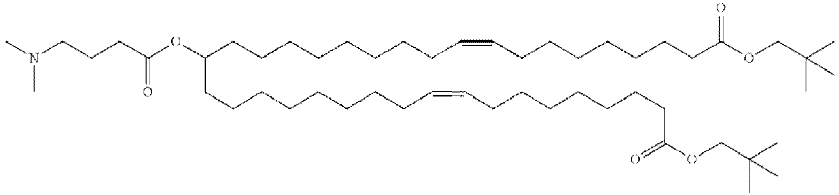

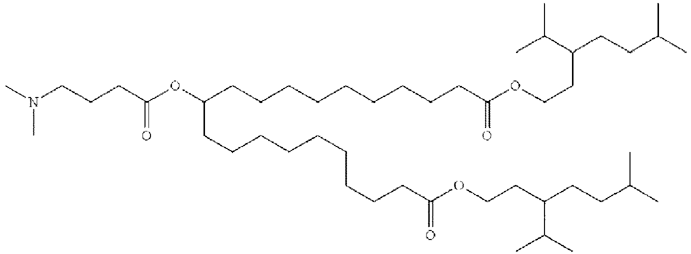

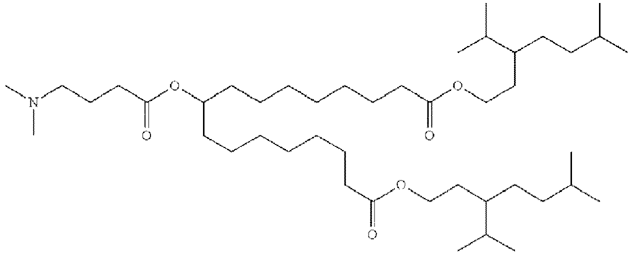

In yet one more embodiment, the cationic lipid has a structure of one of the following compounds, and salts thereof:

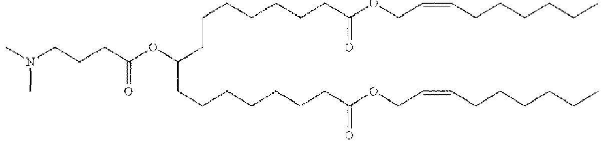

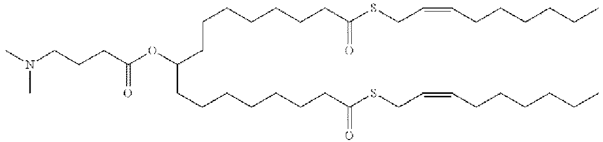

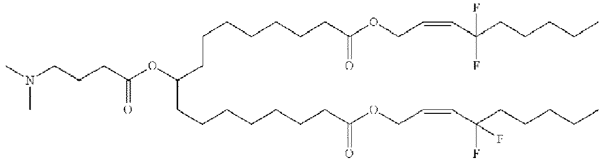

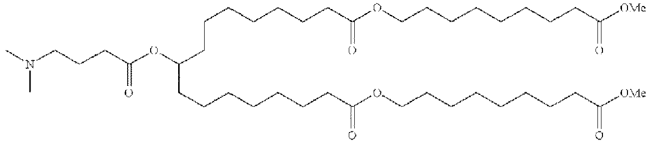

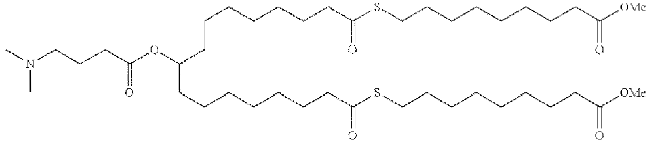

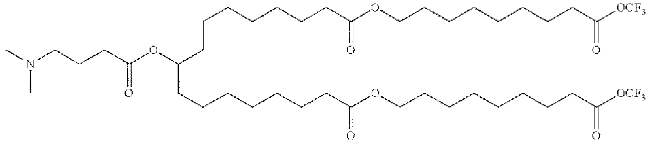

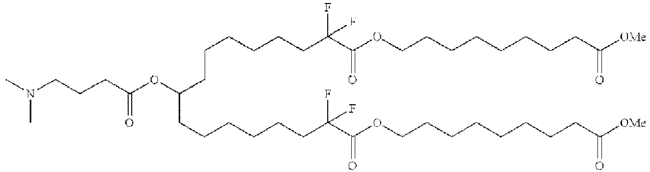

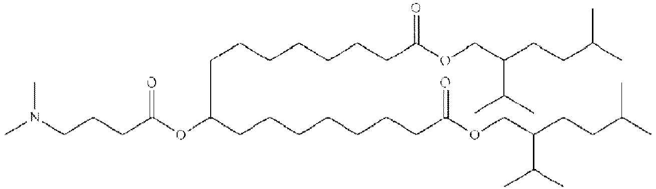

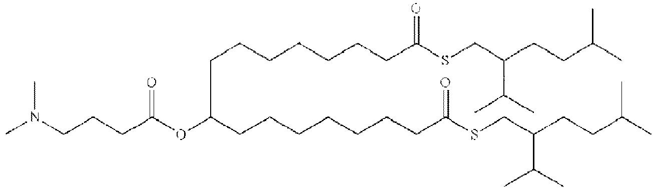

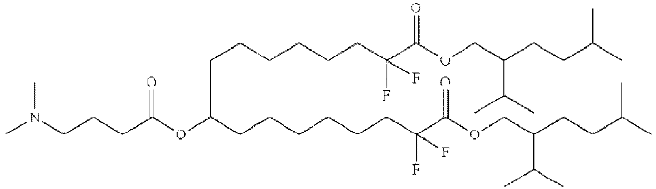

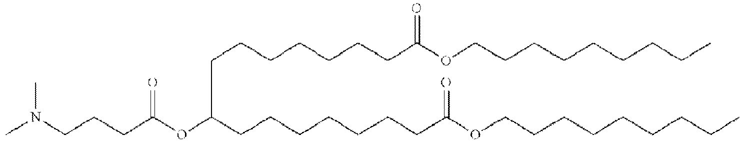

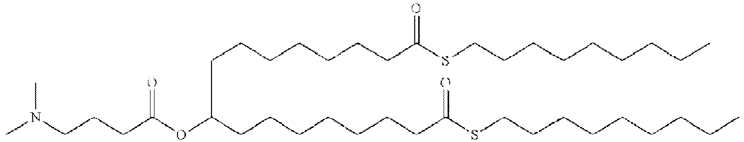

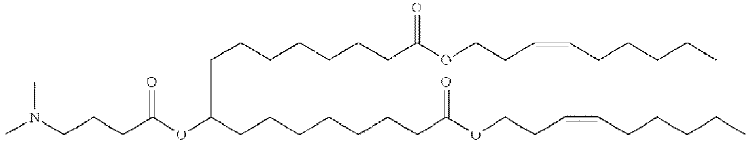

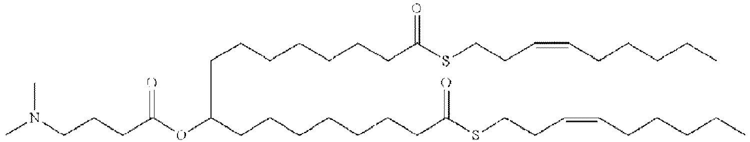

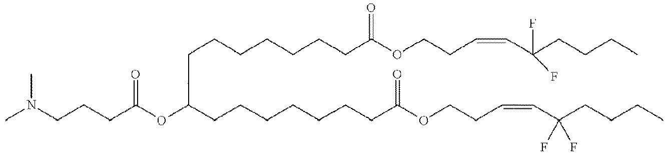

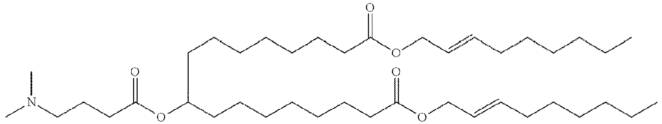

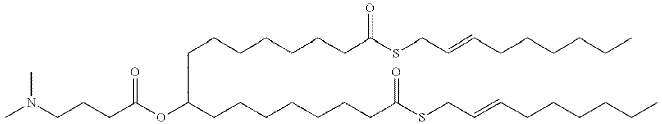

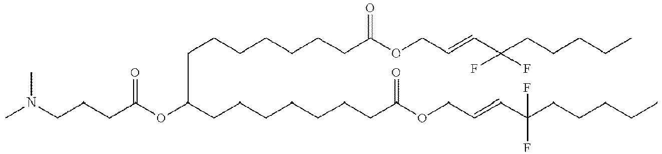

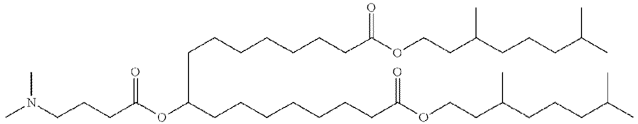

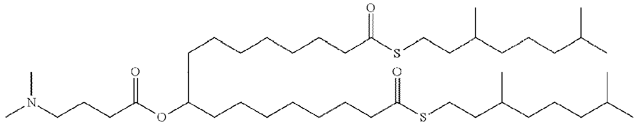

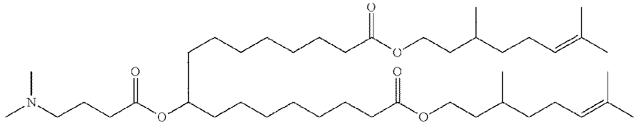

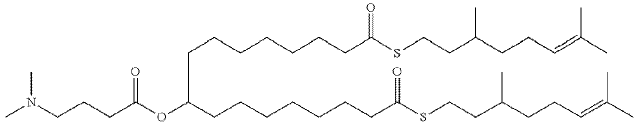

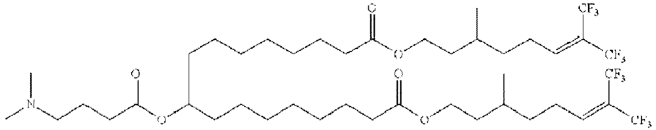

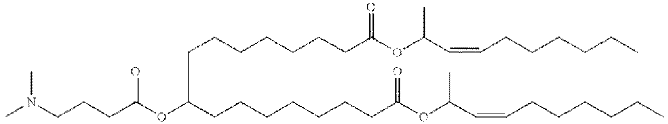

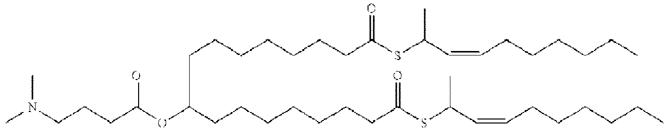

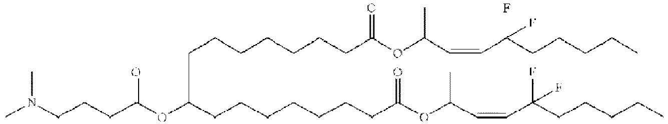

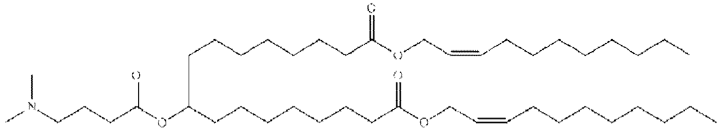

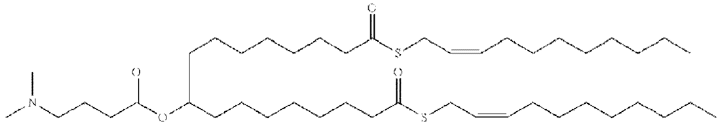

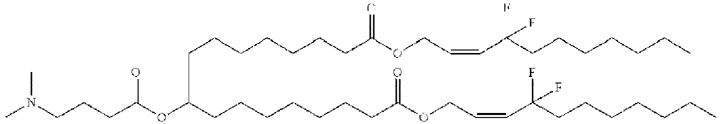

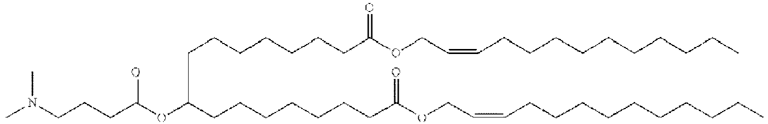

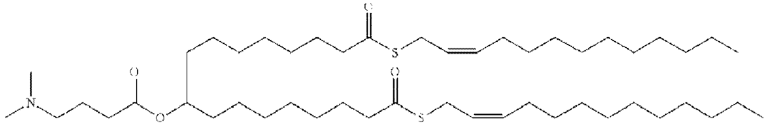

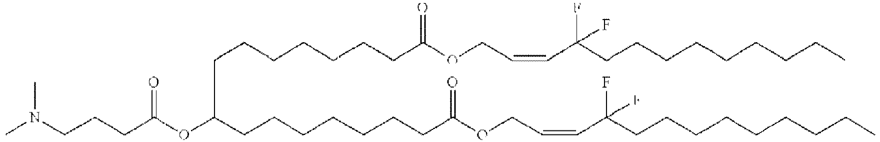

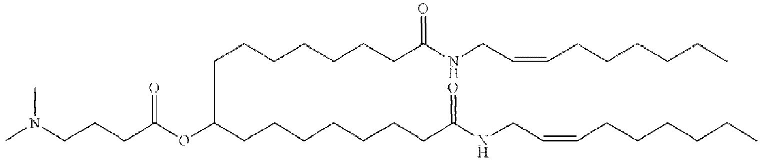

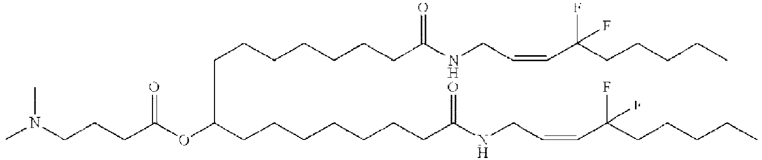

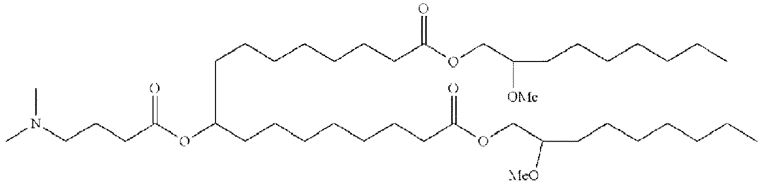

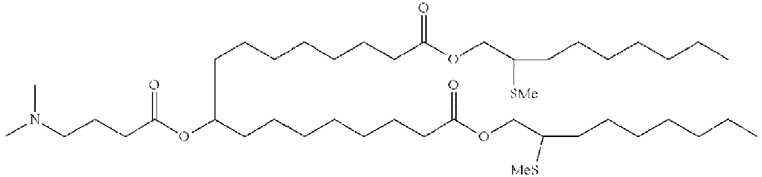

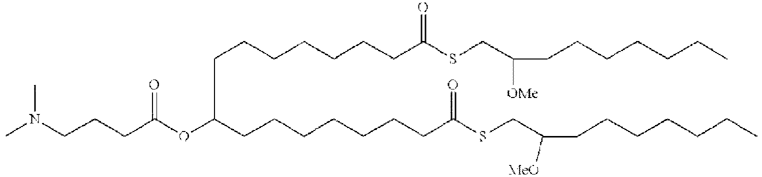

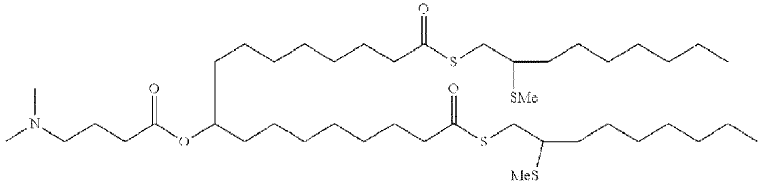

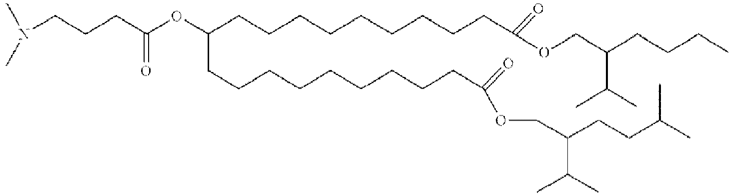

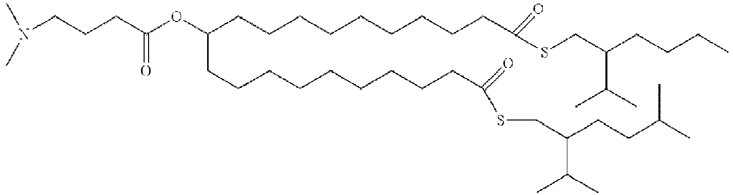

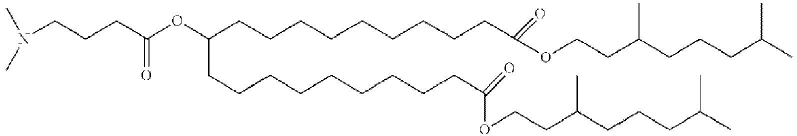

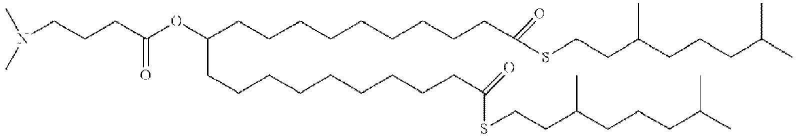

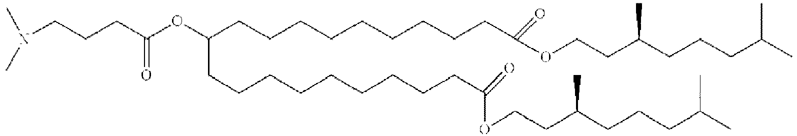

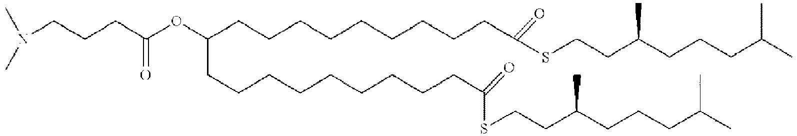

In an embodiment, the cationic lipid has a structure of one of the following compounds, and salts thereof:

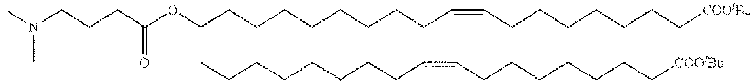

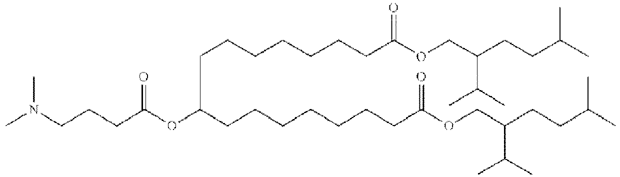

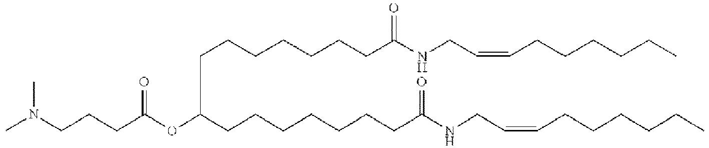

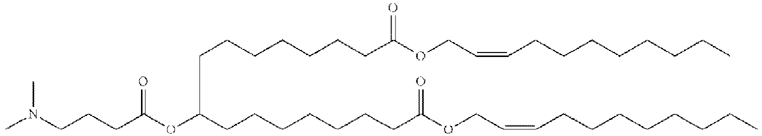

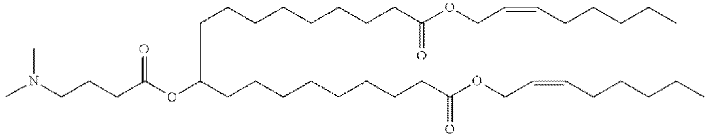

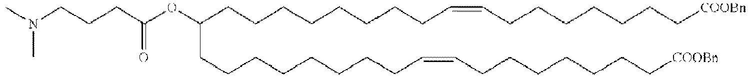

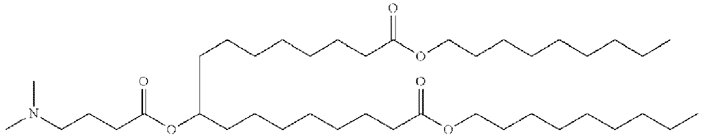

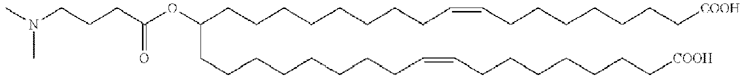

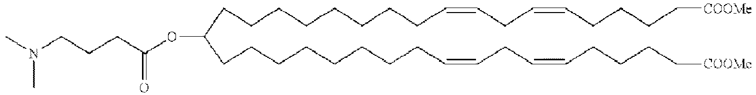

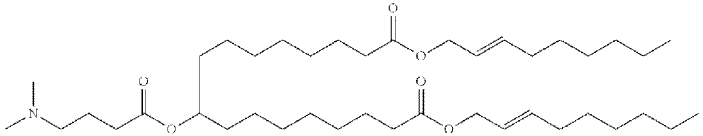

Additional representative cationic lipids include, but are not limited to:

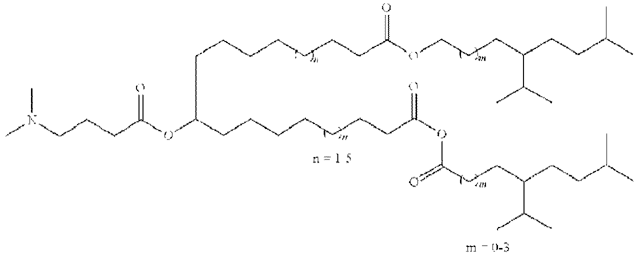

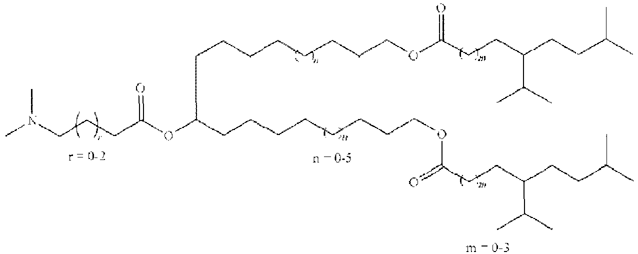

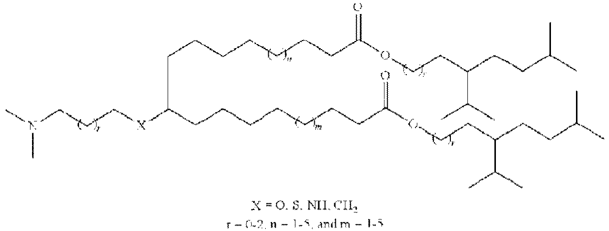

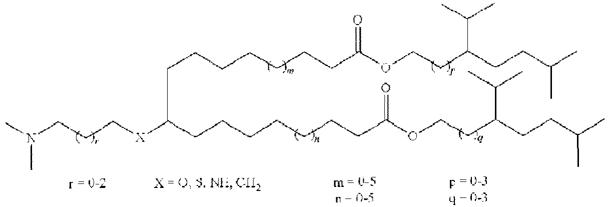

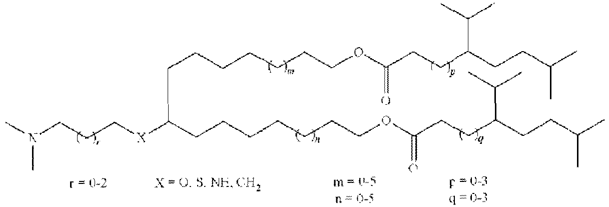

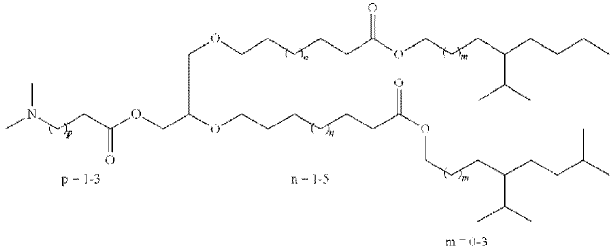

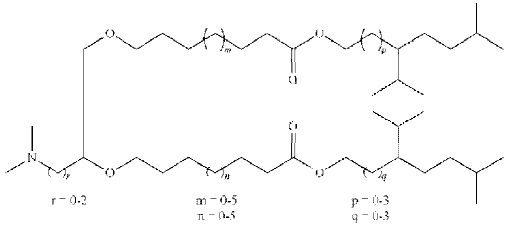

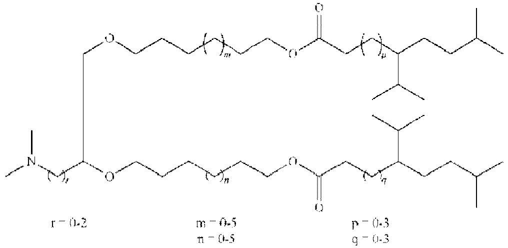

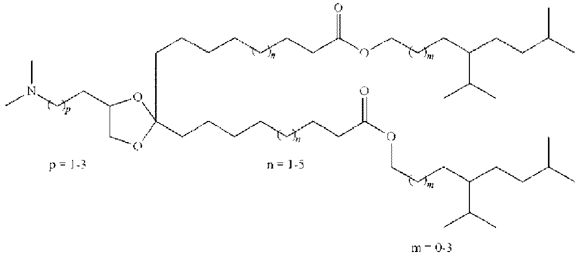

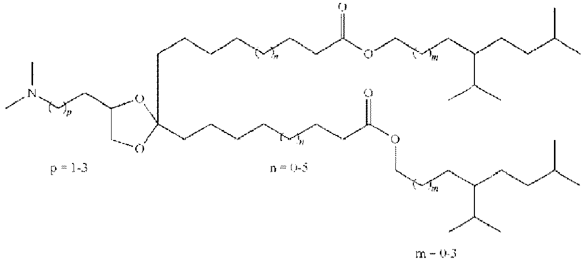

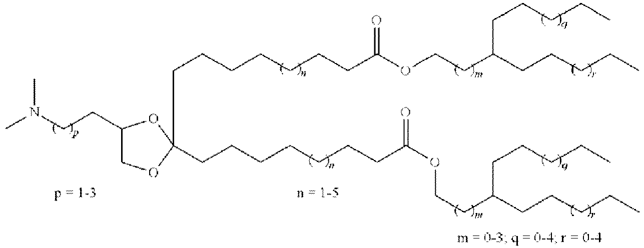

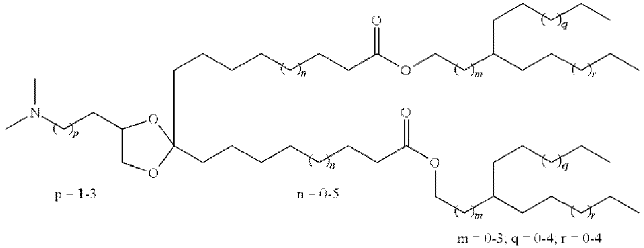

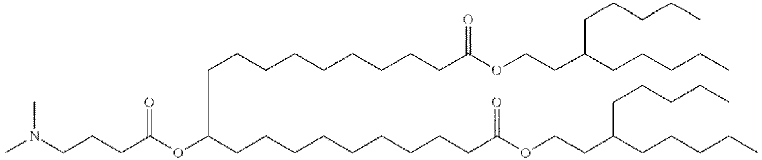

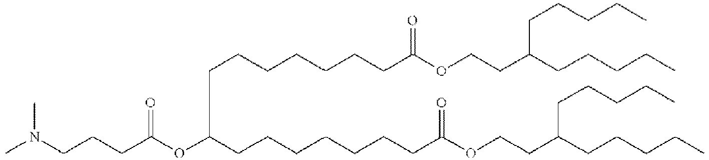

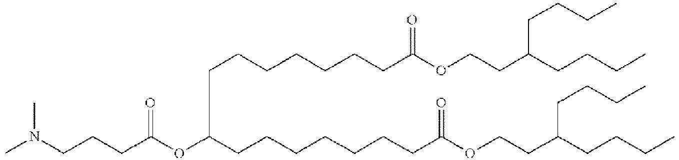

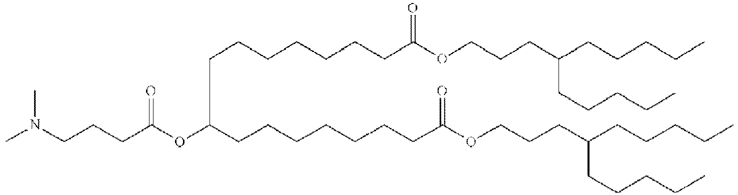

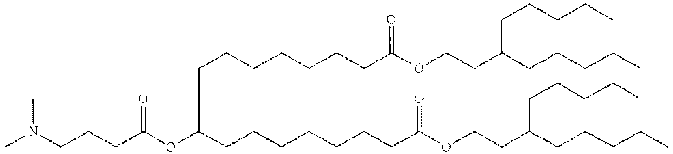

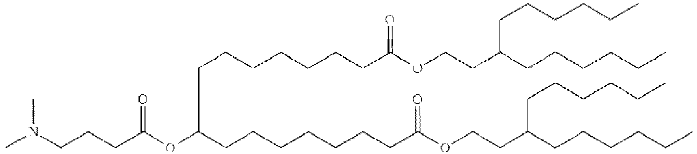

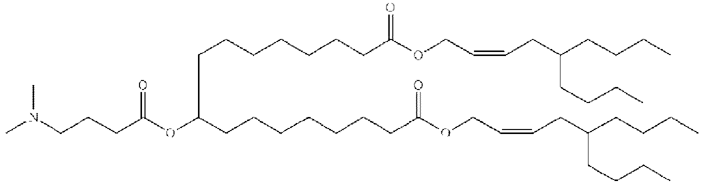

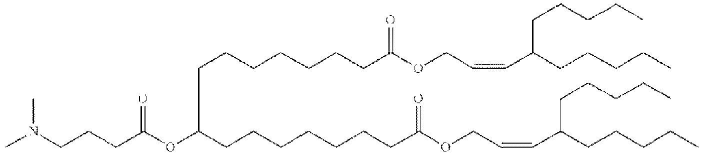

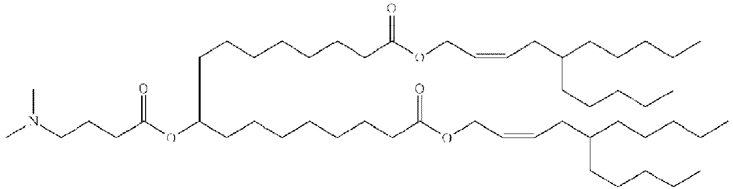

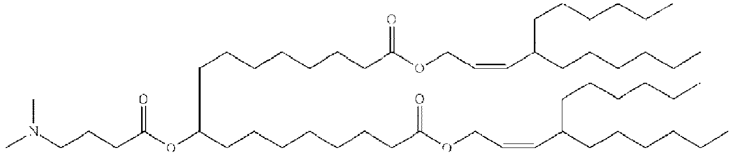

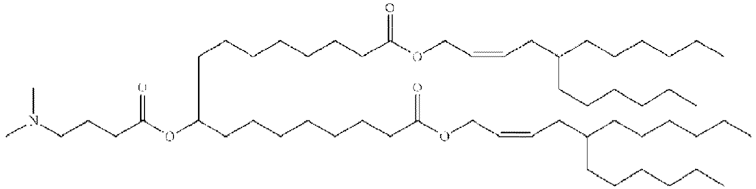

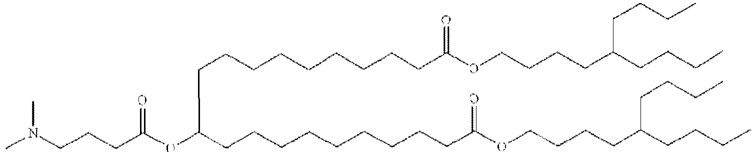

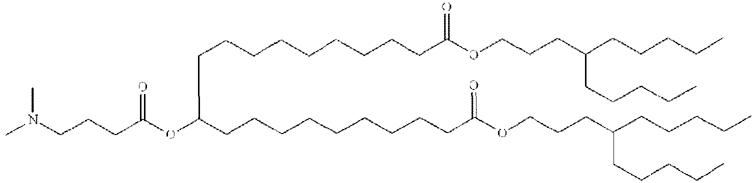

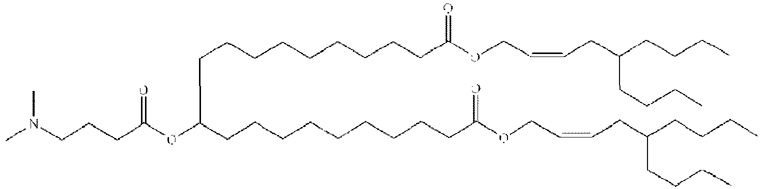

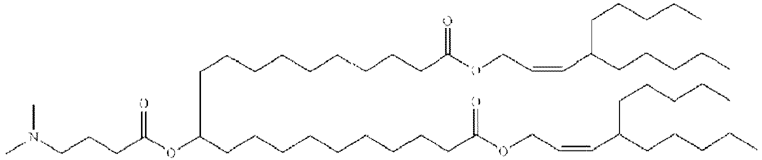

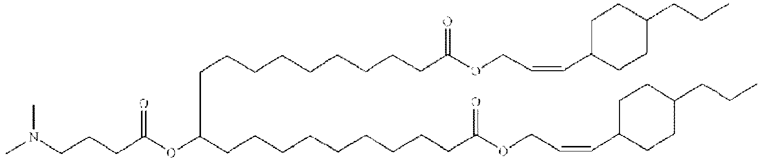

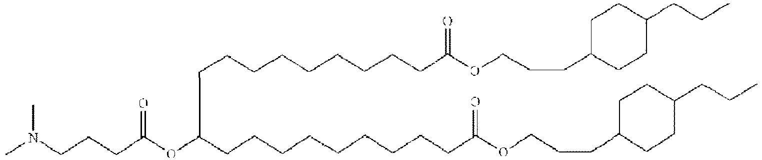

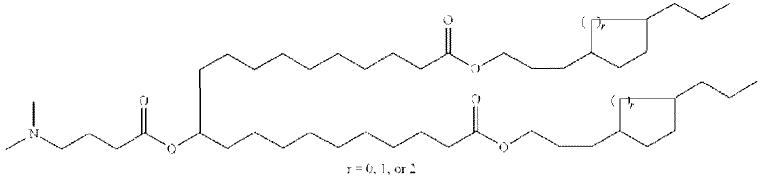

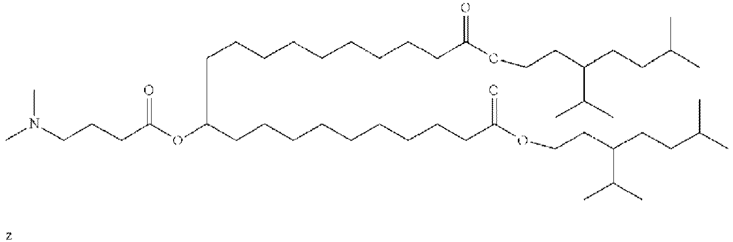

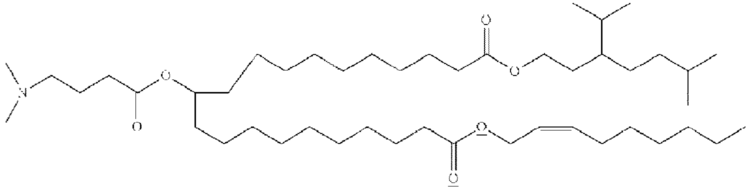

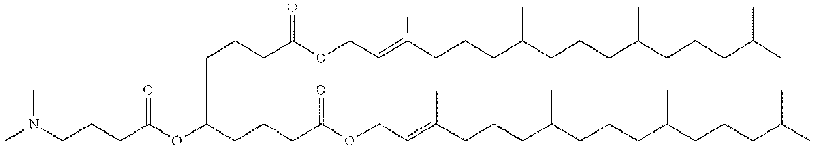

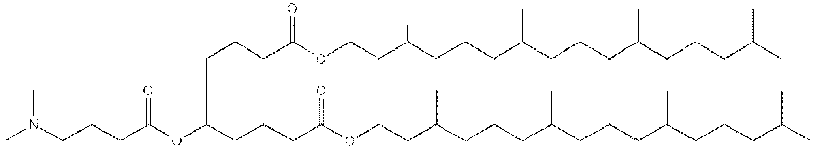

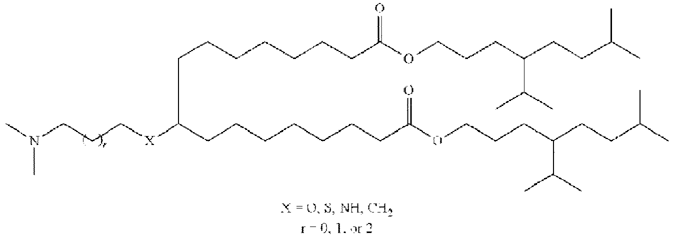

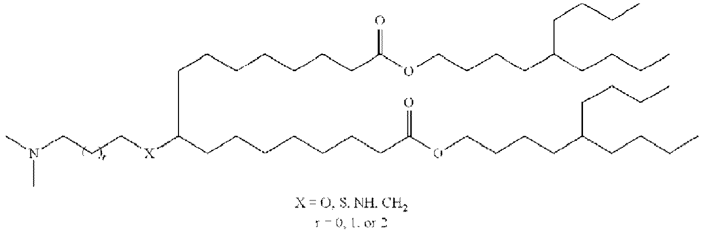

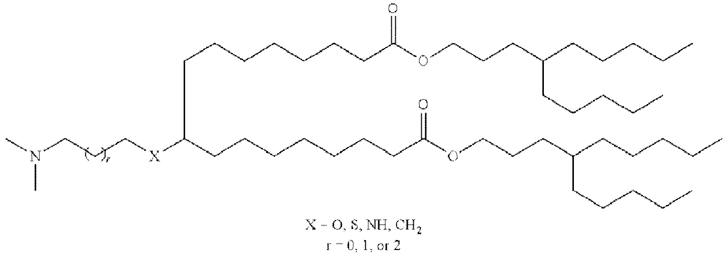

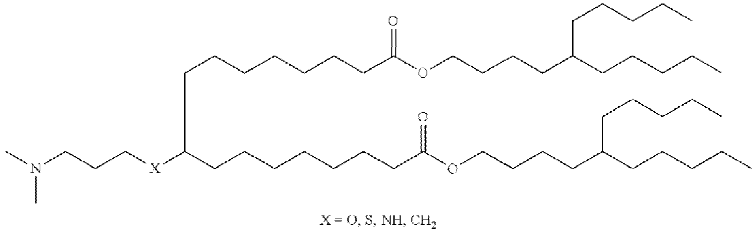

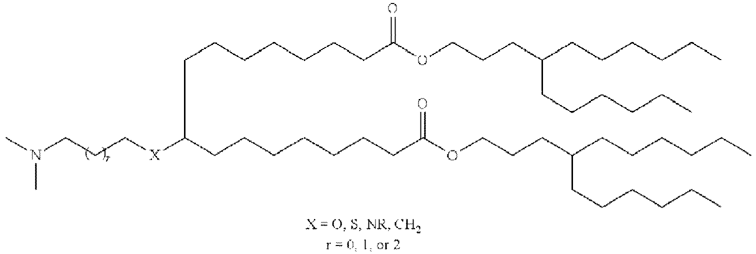

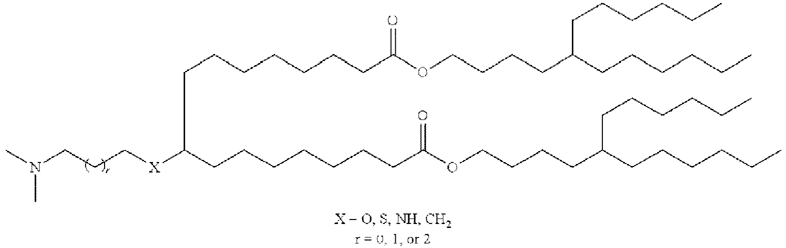

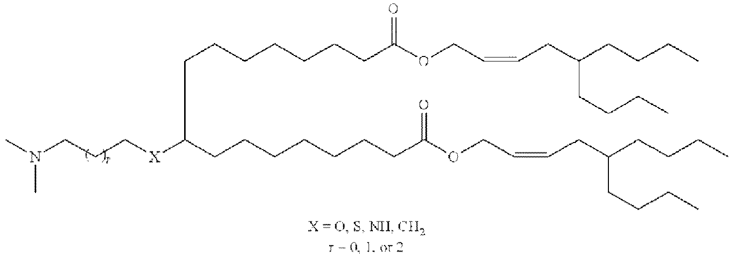

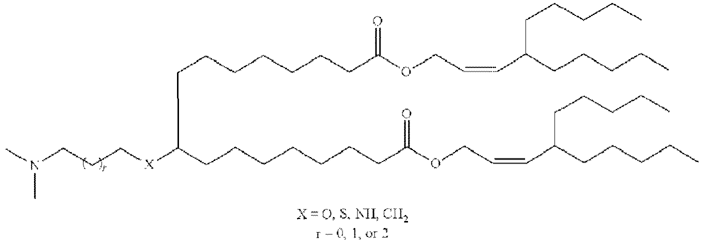

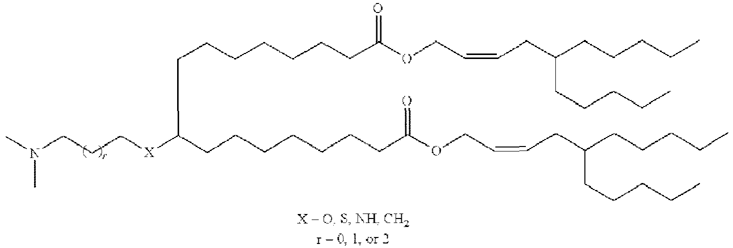

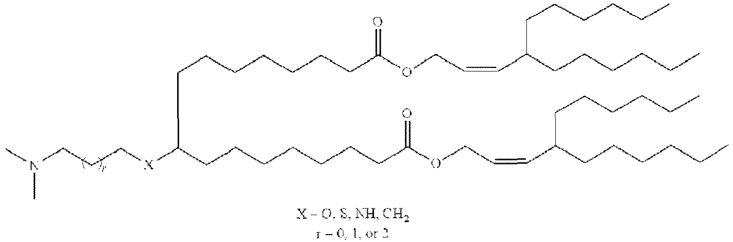

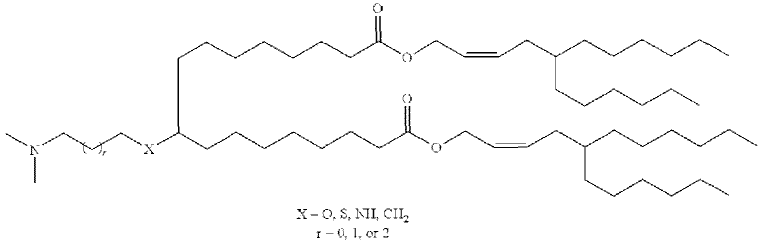

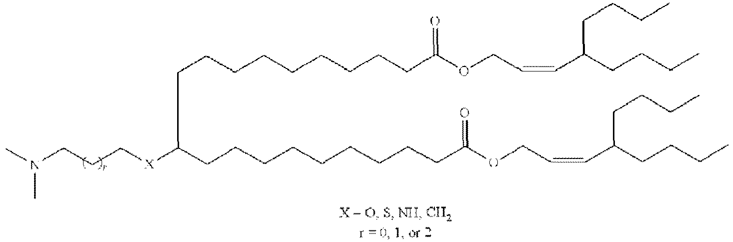

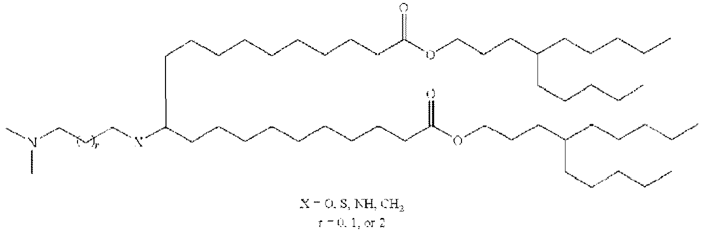

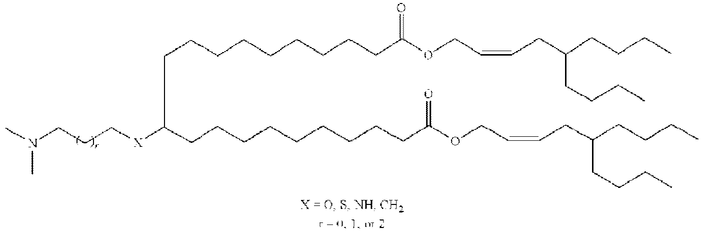

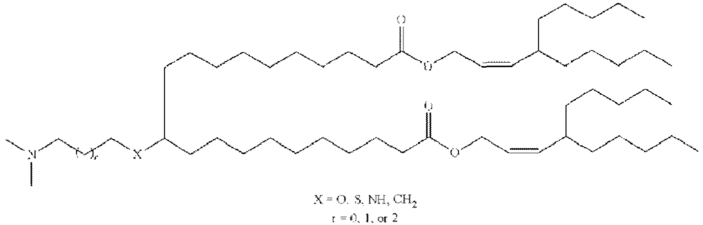

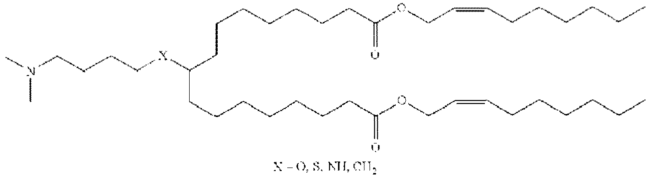

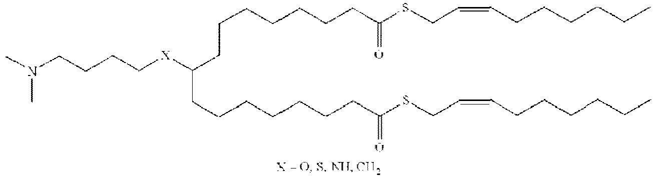

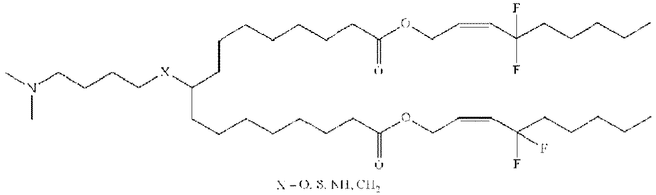

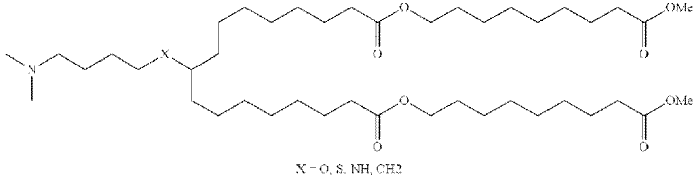

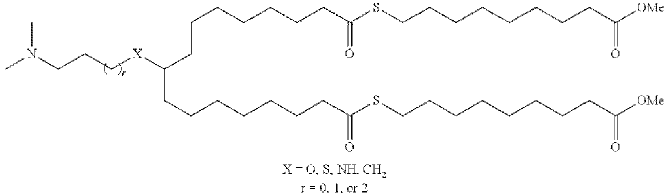

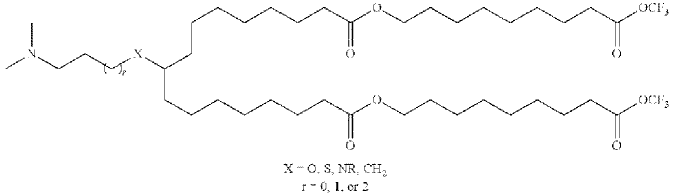

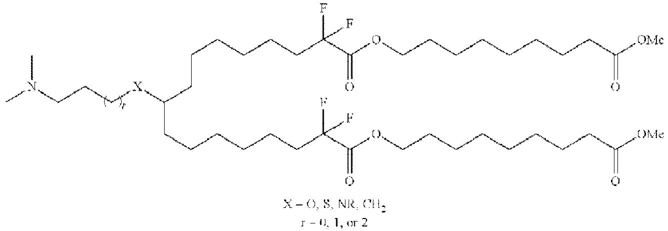

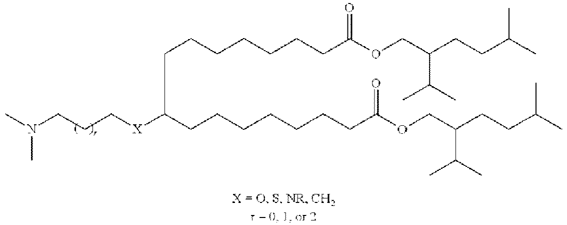

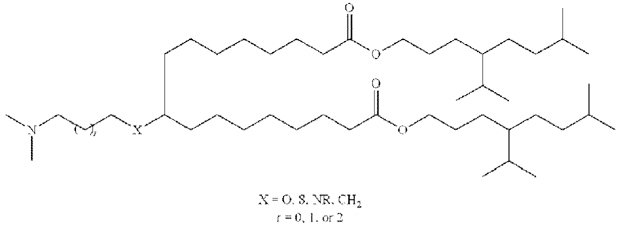

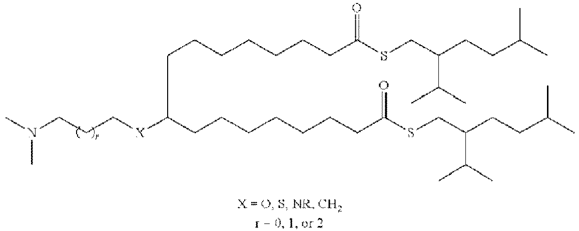

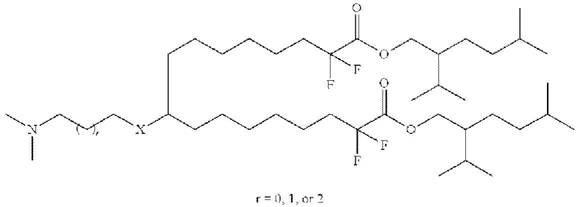

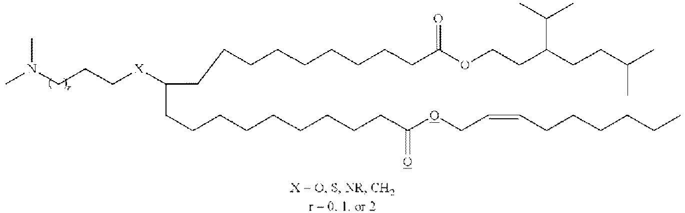

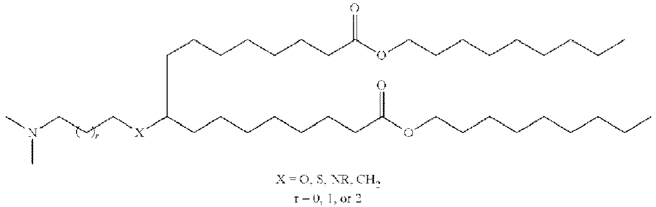

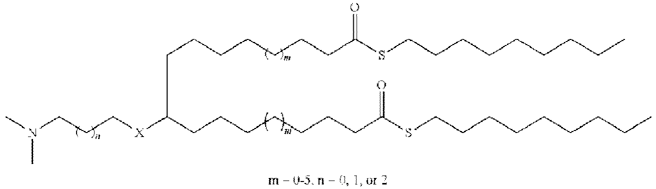

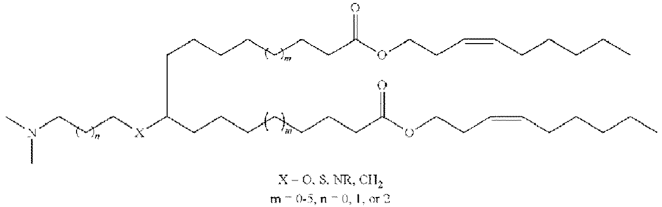

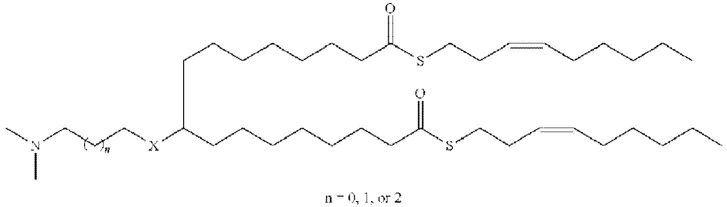

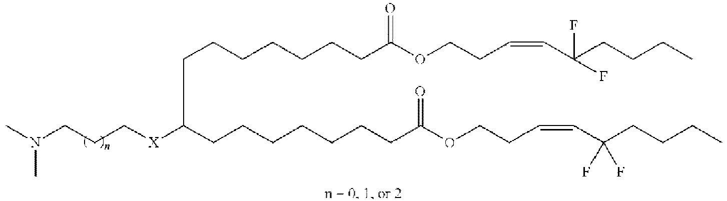

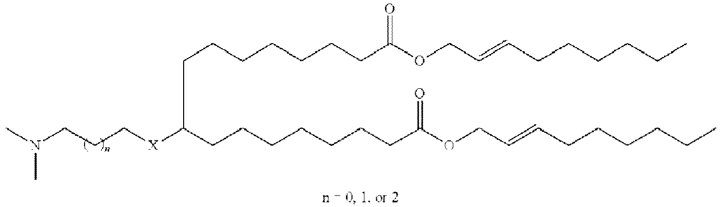

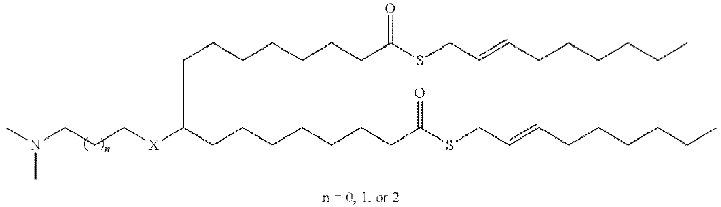

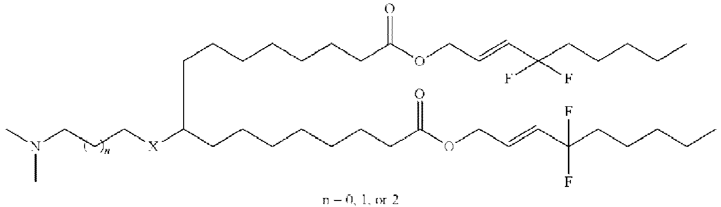

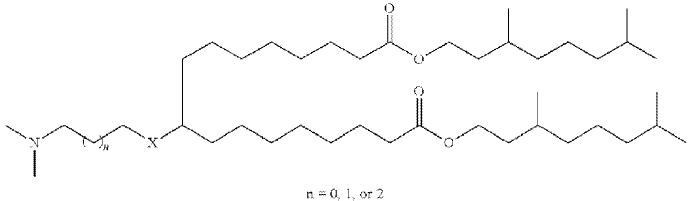

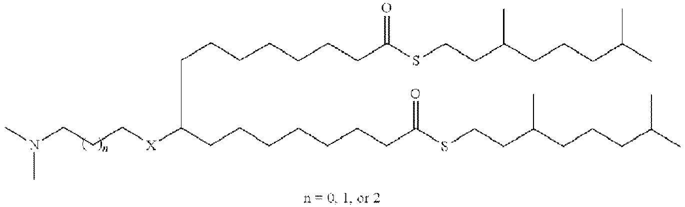

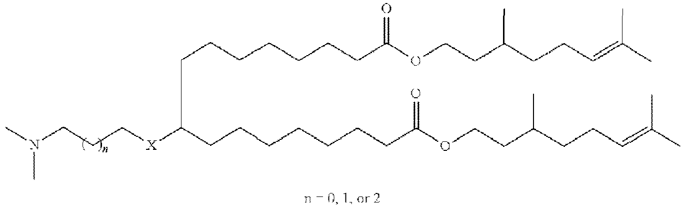

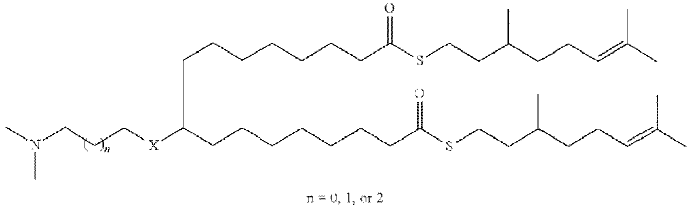

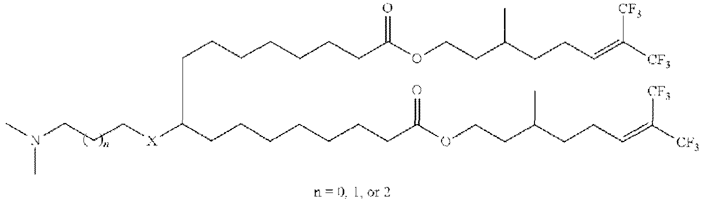

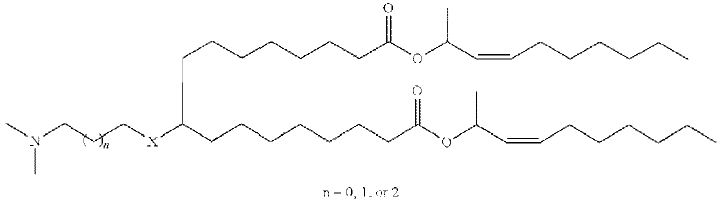

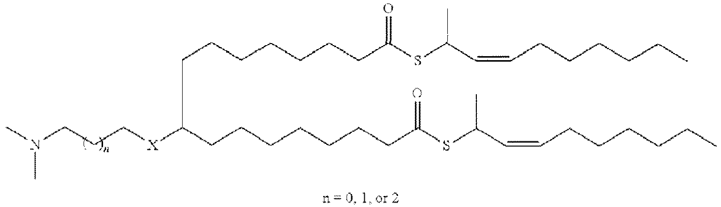

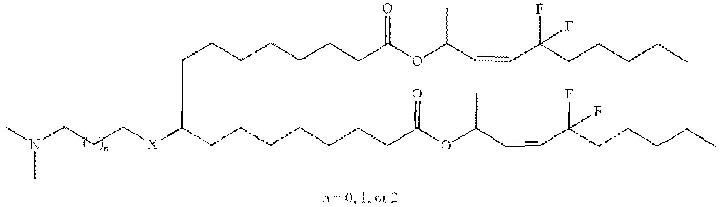

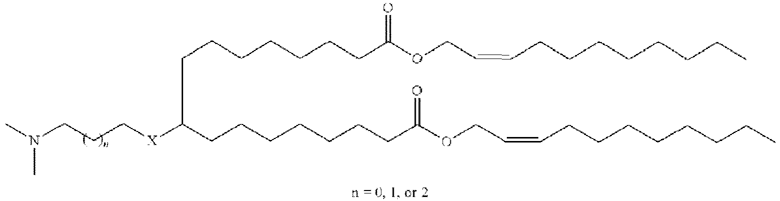

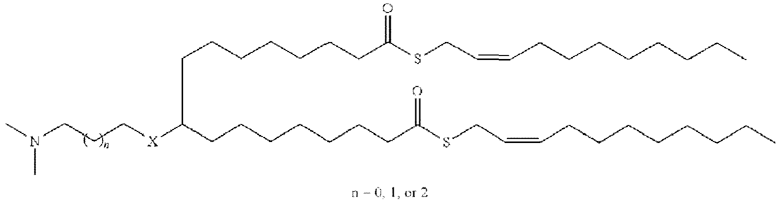

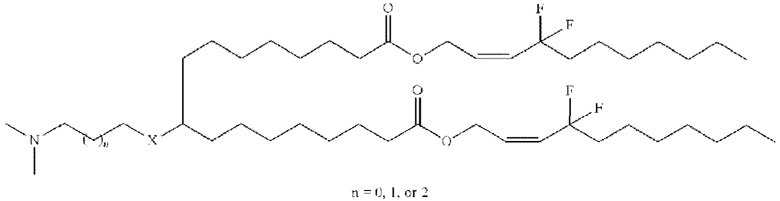

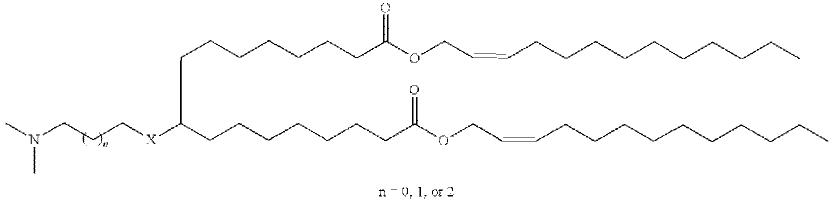

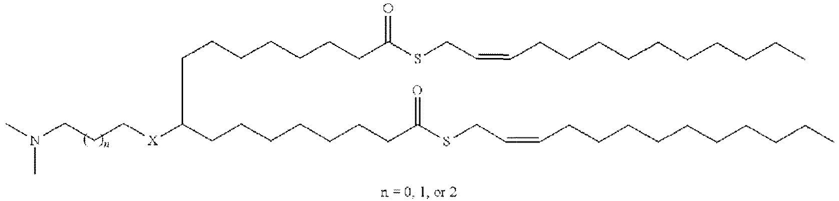

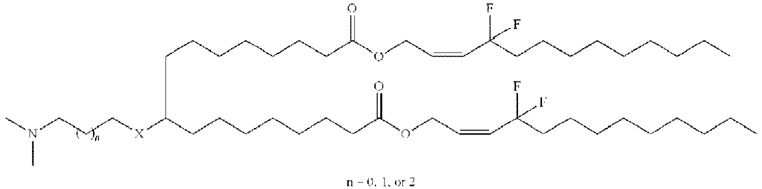

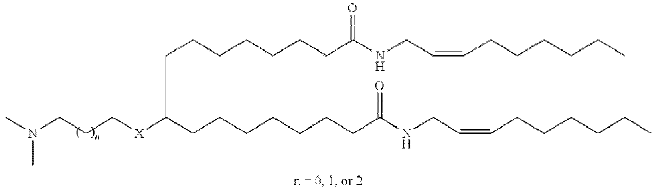

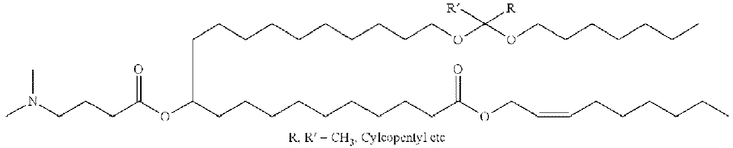

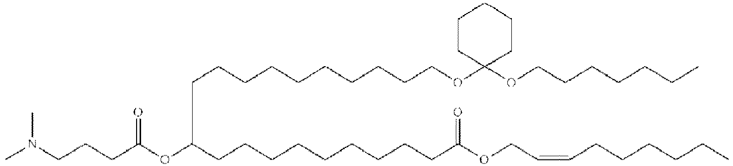

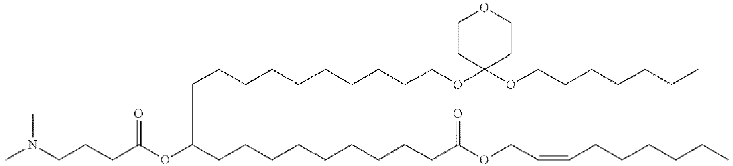

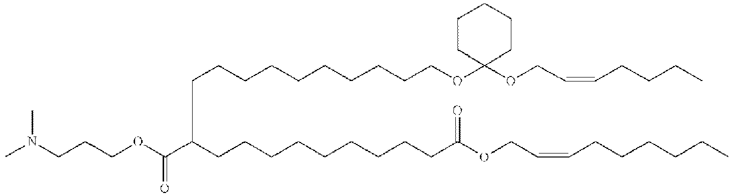

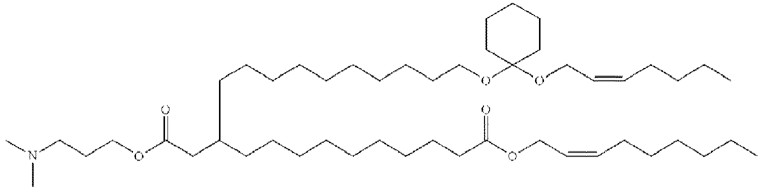

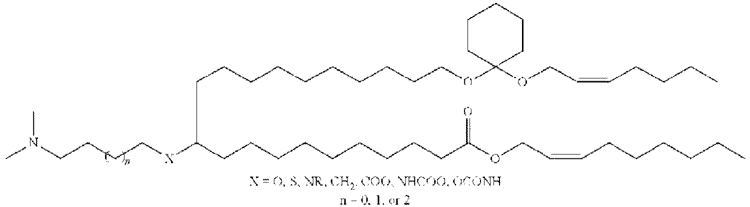

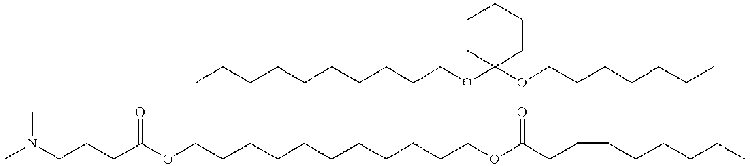

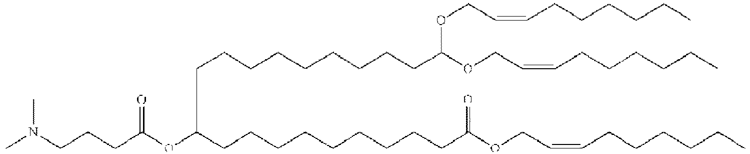

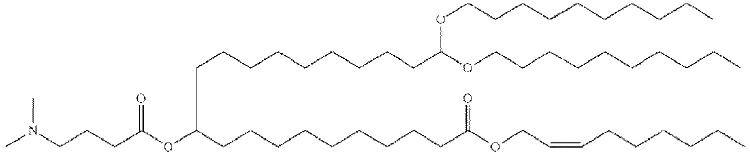

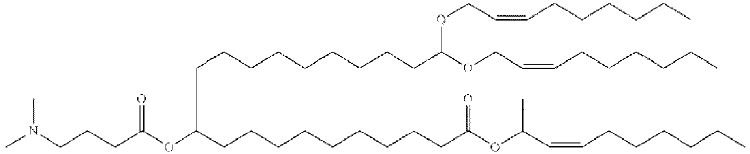

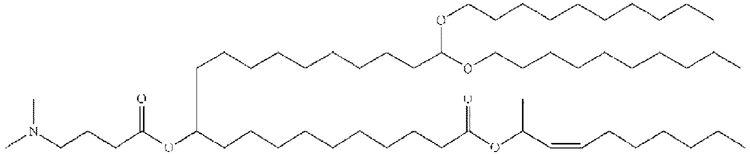

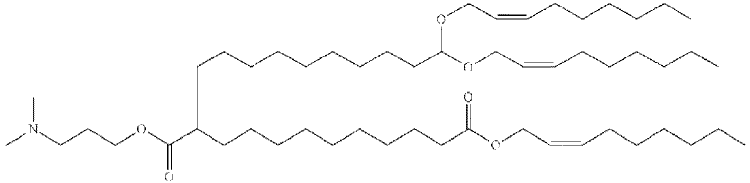

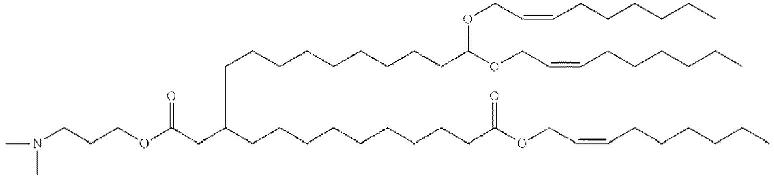

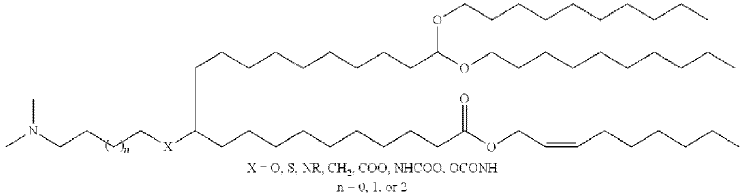

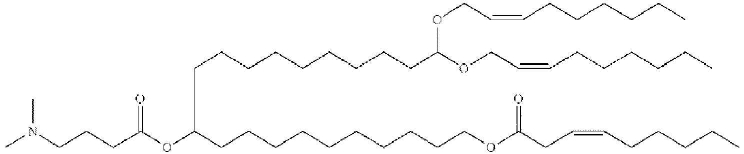

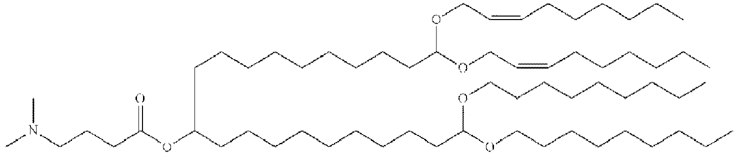

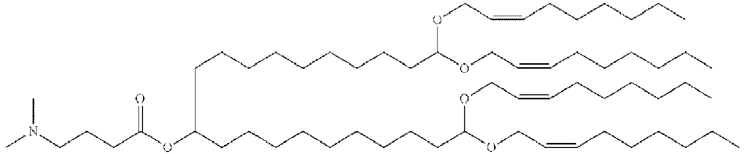

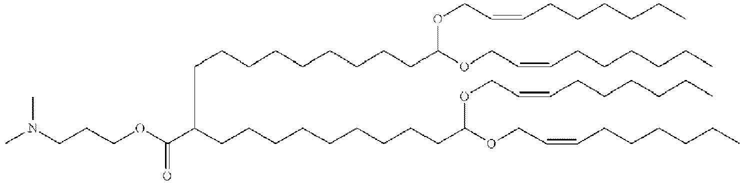

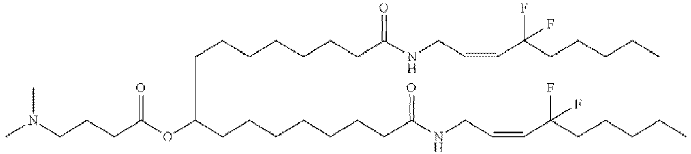

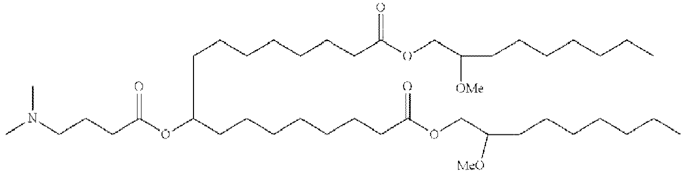

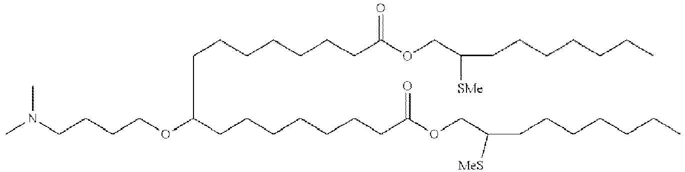

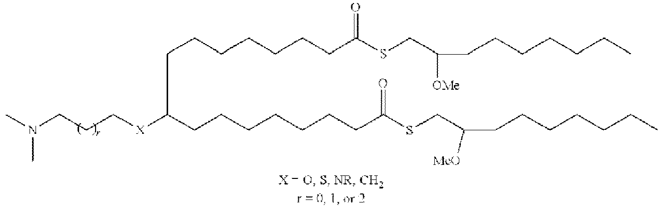

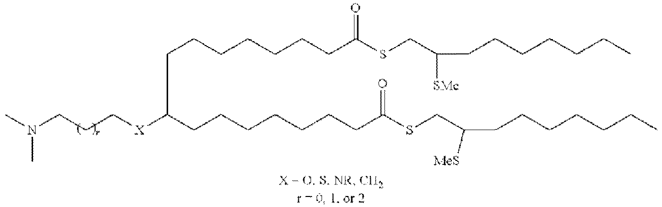

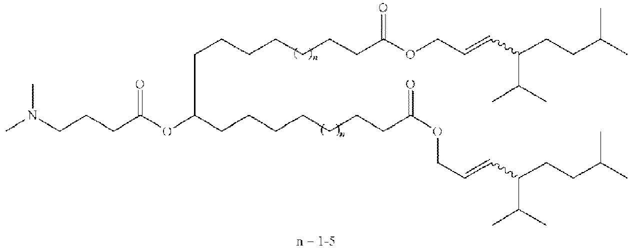

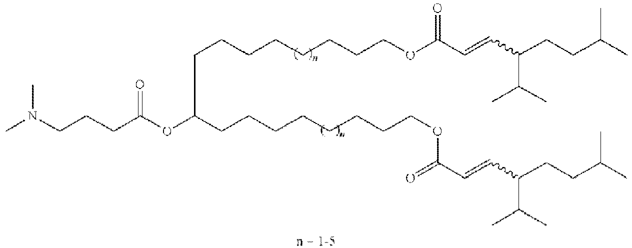

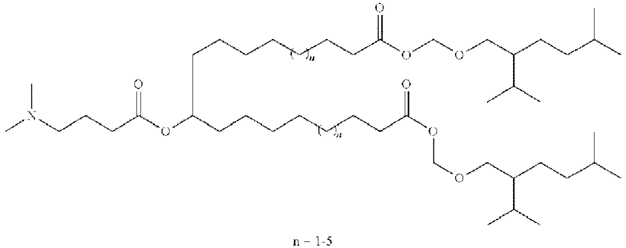

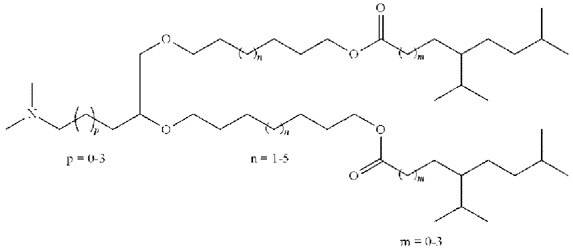

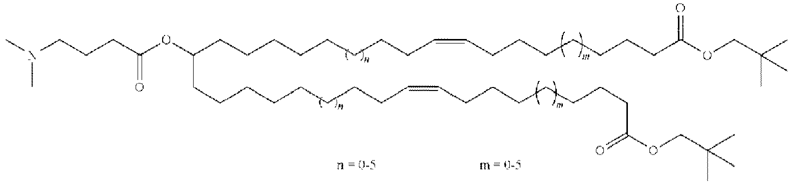

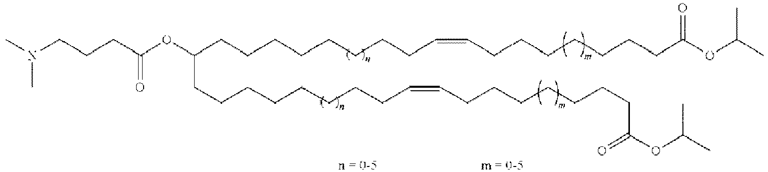

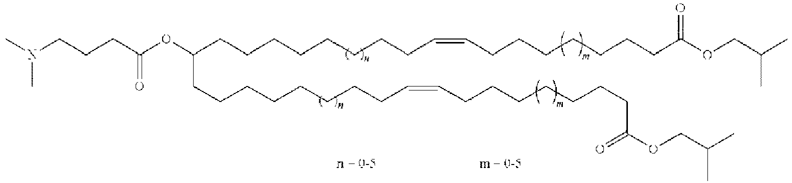

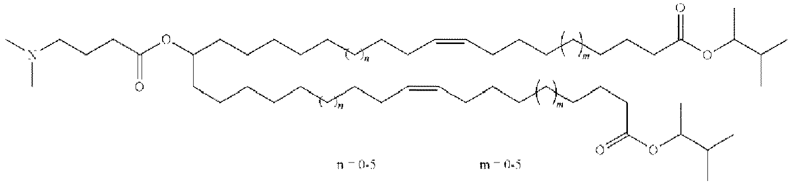

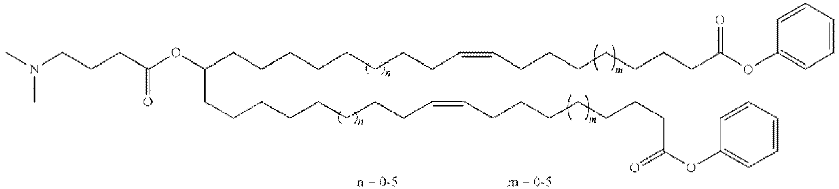

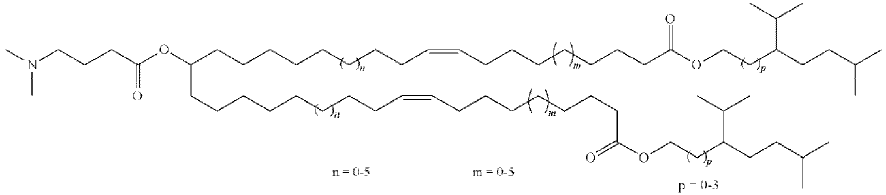

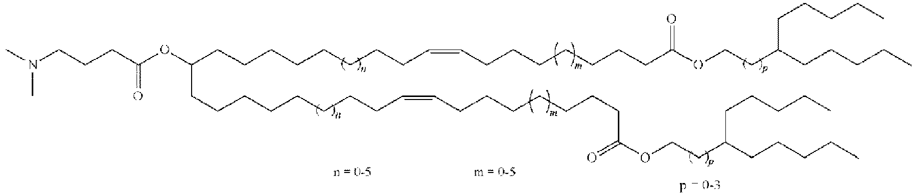

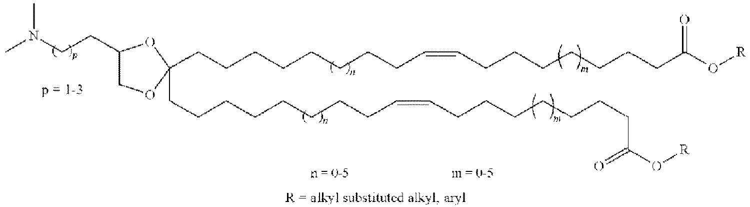

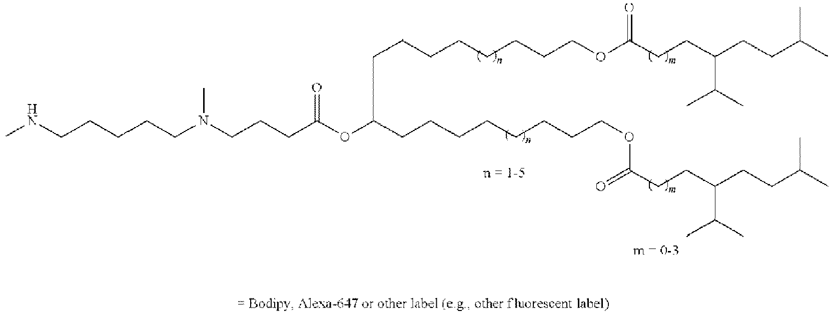

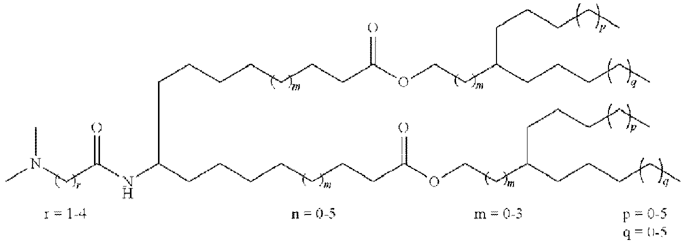

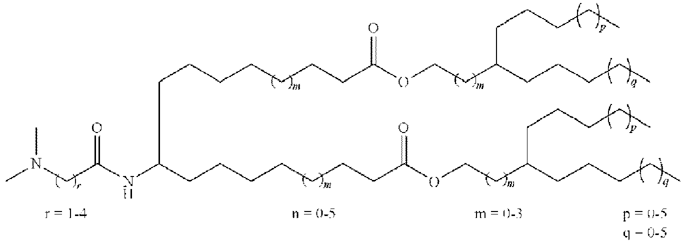

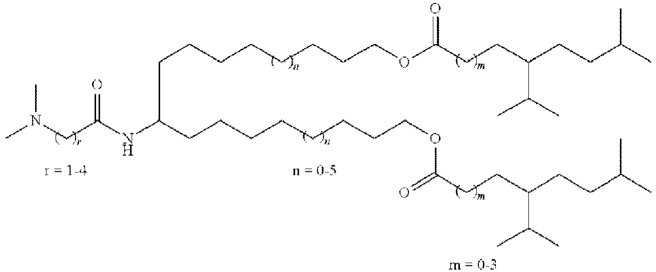

In another embodiment, the cationic lipid has the following structure: 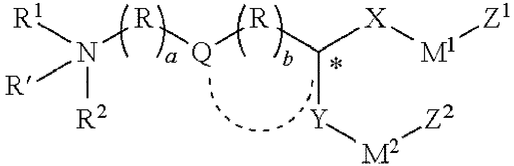 or a salt thereof, wherein
R' is absent, hydrogen, or C₁-C₄ alkyl;
with respect to R¹ and R²,
R' is absent, hydrogen, or alkyl;
with respect to R¹ and R²,
   (i) R¹ and R² are each, independently, optionally substituted alkyl, alkenyl, alkynyl, cycloalkyl, cycloalkylalkyl, heterocycle, or R¹⁰;
   (ii) R¹ and R², together with the nitrogen atom to which they are attached, form an optionally substituted heterocylic ring; or
   (iii) one of R¹ and R² is optionally substituted alkyl, alkenyl, alkynyl, cycloalkyl, cycloalkylalkyl, or heterocycle, and the other forms a 4-10 member heterocyclic ring or heteroaryl with (a) the adjacent nitrogen atom and (b) the (R)ₐ group adjacent to the nitrogen atom;
each occurrence of R is, independently, -(CR³R⁴)-;
each occurrence of R³ and R⁴ are, independently hydrogen, OH, alkyl, alkoxy, -NH₂, R¹⁰, alkylamino, or dialkylamino;
each occurrence of R¹⁰ is independently selected from PEG and polymers based on poly(oxazoline), poly(ethylene oxide), poly(vinyl alcohol), poly(glycerol), poly(N-vinylpyrrolidone), poly[N-(2-hydroxypropyl)methacrylamide] and poly(amino acid)s, wherein (i) the PEG or polymer is linear or branched, (ii) the PEG or polymer is polymerized by n subunits, (iii) n is a number-averaged degree of polymerization between 10 and 200 units, and (iv) wherein the compound of said formula has at most two R¹⁰ groups;
the dashed line to Q is absent or a bond;
when the dashed line to Q is absent then Q is absent or is -O-, -NH-, -S-, - C(O)-, -C(O)O, -OC(O)-, -C(O)N(R⁴)-, -N(R⁵)C(O)-, -S-S-, -OC(O)O-, -O-N=C(R⁵)-, - C(R⁵)=N-O-, -OC(O)N(R⁵)-, -N(R⁵)C(O)N(R⁵)-, -N(R⁵)C(O)O-, -C(O)S-, -C(S)O- or - C(R⁵)=N-O-C(O)-; or
when the dashed line to Q is a bond then (i) b is 0 and (ii) Q and the tertiary carbon adjacent to it (C*) form a substituted or unsubstituted, mono- or bi-cyclic heterocyclic group having from 5 to 10 ring atoms;
each occurrence of R⁵ is, independently, hydrogen or alkyl;
X and Y are each, independently, -(CR⁶R⁷)_{c}-;
each occurrence of R⁶ and R⁷ are, independently hydrogen, OH, alkyl, alkoxy, -NH₂, alkylamino, or dialkylamino;
M¹ and M² are each, independently, a biodegradable group;
a is 1, 2, 3, 4, 5 or 6;
b is 0, 1, 2, or 3;
each occurrence of c is, independently, 2-10; and
Z¹ and Z² are each, independently (i) C₃-C₁₀ cycloalkyl, (ii) C₃-C₁₀cycloalkyl(C₁-C₆ alkyl), or (iii) 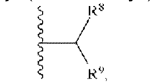
wherein each of R⁸ and R⁹ is a C₂-C₈ alkyl.

In yet another embodiment, the cationic lipid is selected from the compounds:

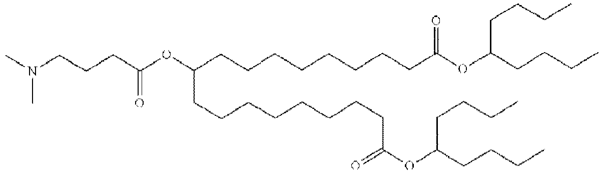

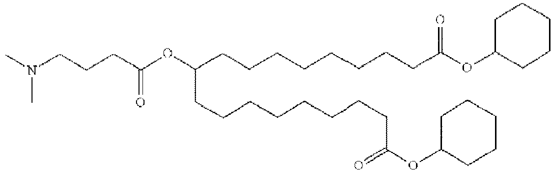

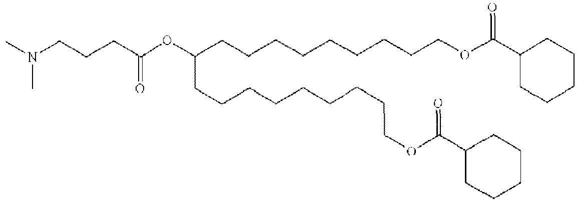

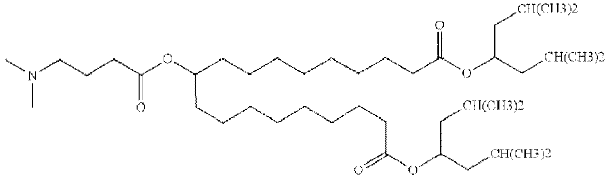

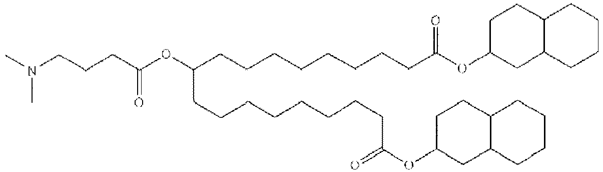

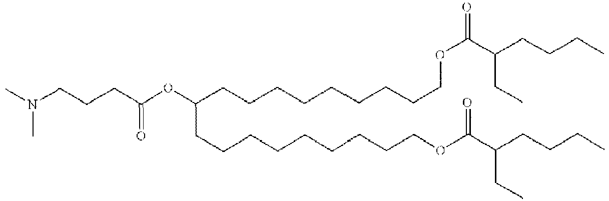

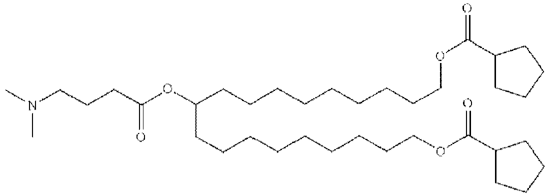

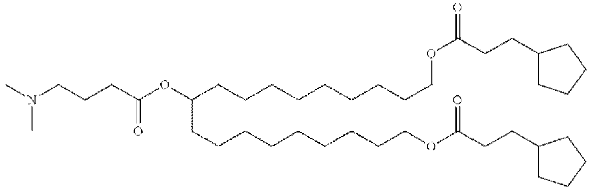

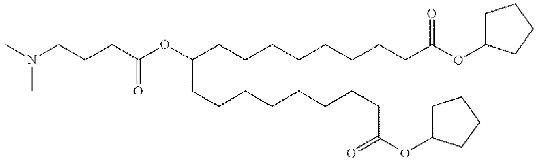

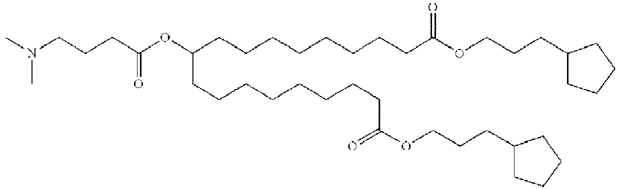

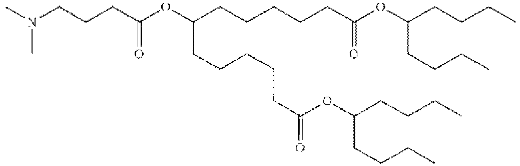

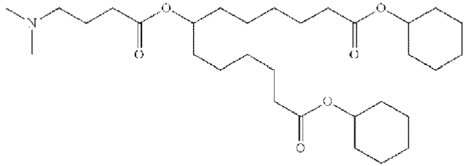

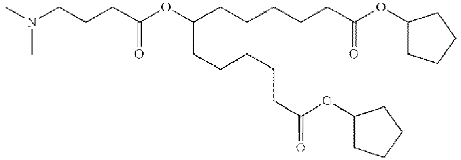

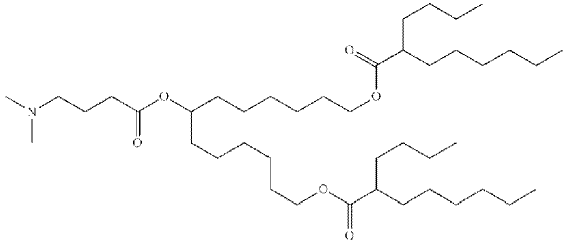

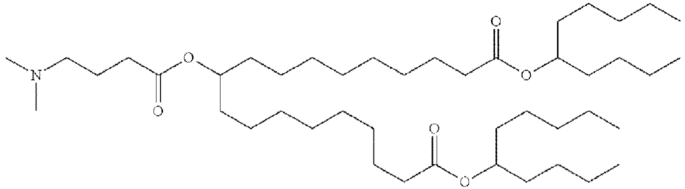

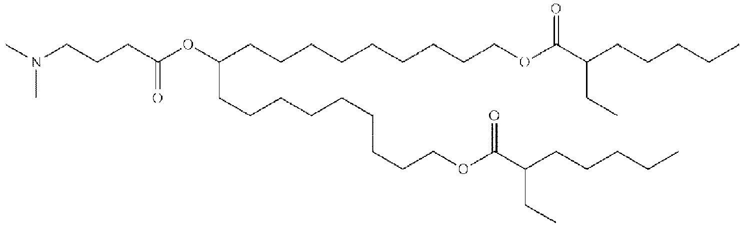

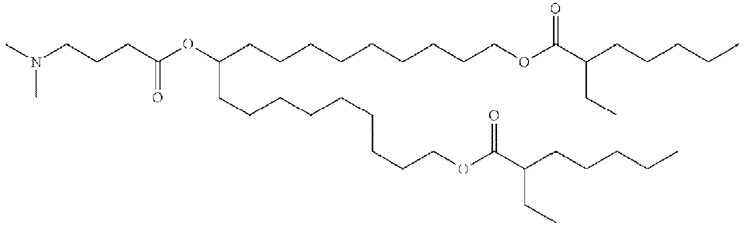

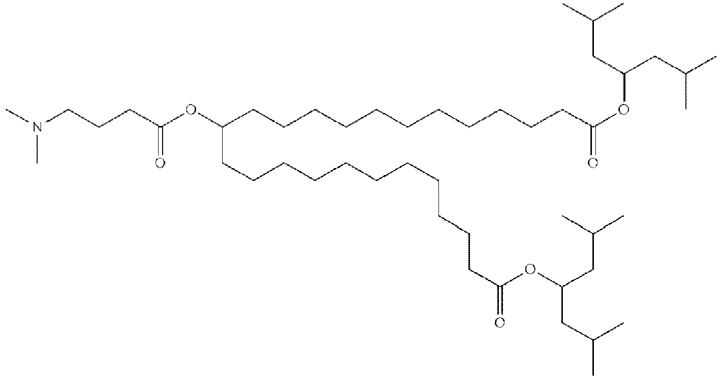

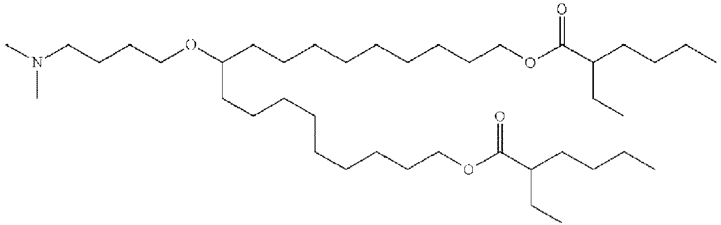

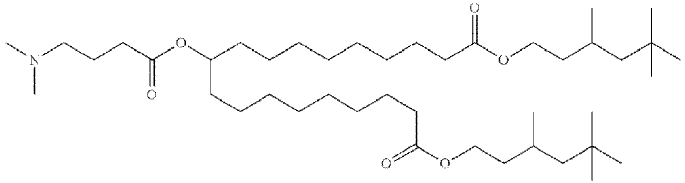

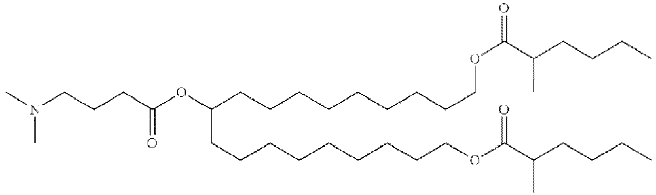

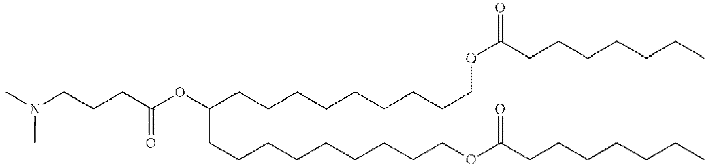

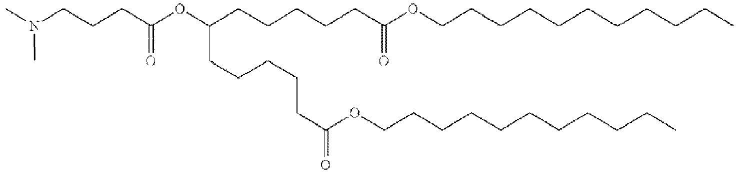

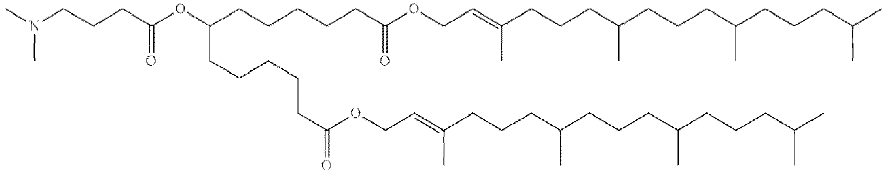

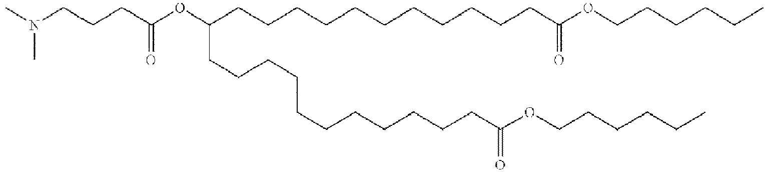

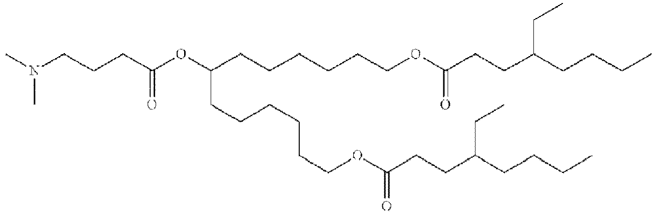

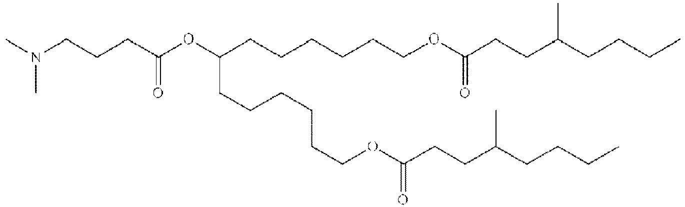

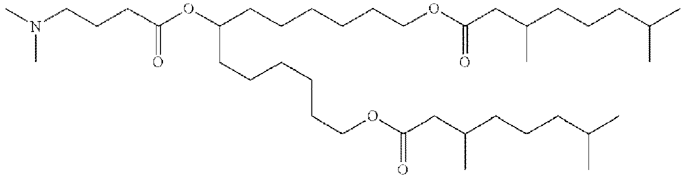

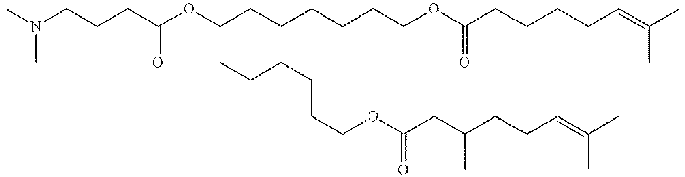

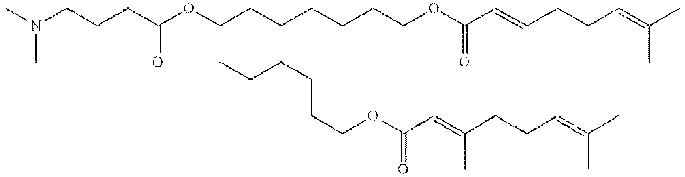

In one embodiment, the cationic lipid has the structure of Formula I: 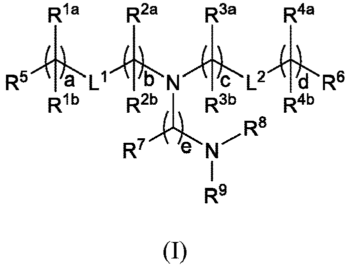 or a pharmaceutically acceptable salt, tautomer or stereoisomer thereof, wherein:
one of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- or -NR^{a}C(=O)O-, and the other of L¹ or L² is -O(C=O)-, -(C=O)O-, -C(=O)-, -0-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}-or
   -NR^{a}C(=O)O- or a direct bond;
R^{a} is H or C₁-C₁₂ alkyl;
R^{1a} and R^{1b} are, at each occurrence, independently either (a) H or C₁-C₁₂ alkyl, or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
R^{2a} and R^{2b} are, at each occurrence, independently either (a) H or C₁-C₁₂ alkyl, or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
R^{3a} and R^{3b} are, at each occurrence, independently either (a) H or C₁-C₁₂ alkyl, or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
R^{4a} and R^{4b} are, at each occurrence, independently either (a) H or C₁-C₁₂ alkyl, or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
R₅ and R₆ are each independently methyl or cycloalkyl;
R⁷ is, at each occurrence, independently H or C₁-C₁₂ alkyl;
R⁸ and R⁹ are each independently unsubstituted C₁-C₁₂ alkyl; or R⁸ and R⁹, together with the nitrogen atom to which they are attached, form a 5, 6 or 7-membered heterocyclic ring comprising one nitrogen atom;
a and d are each independently an integer from 0 to 24;
b and c are each independently an integer from 1 to 24;
e is 1 or 2; and
x is 0, 1 or 2.

In some embodiments of Formula (I), L¹ and L² are independently - O(C=O)- or -(C=O)O-.

In certain embodiments of Formula (I), at least one of R^{1a}, R^{2a}, R^{3a} or R^{4a} is C₁-C₁₂ alkyl, or at least one of L¹ or L² is -O(C=O)- or -(C=O)O-. In other embodiments, R^{1a} and R^{1b} are not isopropyl when a is 6 or n-butyl when a is 8.

In still further embodiments of Formula (I), at least one of R^{1a}, R^{2a}, R^{3a} or R^{4a} is C₁-C₁₂ alkyl, or at least one of L¹ or L² is -O(C=O)- or -(C=O)O-; and
R^{1a} and R^{1b} are not isopropyl when a is 6 or n-butyl when a is 8.

In other embodiments of Formula (1), R₈ and R₉ are each independently unsubstituted C₁-C₁₂ alkyl; or R⁸ and R⁹, together with the nitrogen atom to which they are attached, form a 5, 6 or 7-membered heterocyclic ring comprising one nitrogen atom;
In certain embodiments of Formula (I), any one of L¹ or L² may be -O(C=O)- or a carbon-carbon double bond. L¹ and L² may each be -O(C=O)- or may each be a carbon-carbon double bond.

In some embodiments of Formula (I), one of L¹ or L² is -O(C=O)-. In other embodiments, both L¹ and L² are -O(C=O)-.

In some embodiments of Formula (I), one of L¹ or L² is -(C=O)O-. In other embodiments, both L¹ and L² are -(C=O)O-.

In some other embodiments of Formula (I), one of L¹ or L² is a carbon-carbon double bond. In other embodiments, both L¹ and L² are a carbon-carbon double bond.

In still other embodiments of Formula (I), one of L¹ or L² is -O(C=O)- and the other of L¹ or L² is -(C=O)O-. In more embodiments, one of L¹ or L² is -O(C=O)- and the other of L¹ or L² is a carbon-carbon double bond. In yet more embodiments, one of L¹ or L² is -(C=O)O- and the other of L¹ or L² is a carbon-carbon double bond.

It is understood that "carbon-carbon" double bond, as used throughout the specification, refers to one of the following structures: 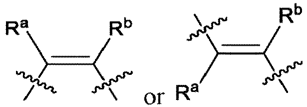 wherein R^{a} and R^{b} are, at each occurrence, independently H or a substituent. For example, in some embodiments R^{a} and R^{b} are, at each occurrence, independently H, Ci-C₁₂ alkyl or cycloalkyl, for example H or C₁-C₁₂ alkyl.

In other embodiments, the lipid compounds of Formula (I) have the following Formula (Ia):

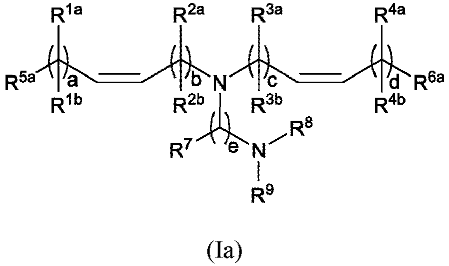

In other embodiments, the lipid compounds of Formula (I) have the following Formula (Ib):

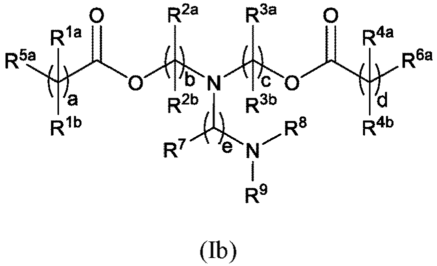

In yet other embodiments, the lipid compounds of Formula (I) have the following Formula (Ic):

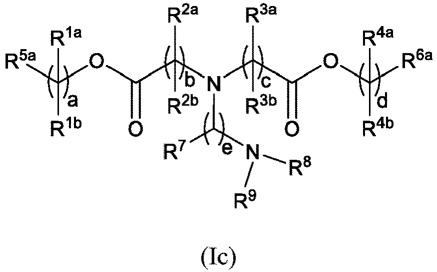

In certain embodiments of the lipid compound of Formula (I), a, b, c and d are each independently an integer from 2 to 12 or an integer from 4 to 12. In other embodiments, a, b, c and d are each independently an integer from 8 to 12 or 5 to 9. In some certain embodiments, a is 0. In some embodiments, a is 1. In other embodiments, a is 2. In more embodiments, a is 3. In yet other embodiments, a is 4. In some embodiments, a is 5. In other embodiments, a is 6. In more embodiments, a is 7. In yet other embodiments, a is 8. In some embodiments, a is 9. In other embodiments, a is 10. In more embodiments, a is 11. In yet other embodiments, a is 12. In some embodiments, a is 13. In other embodiments, a is 14. In more embodiments, a is 15. In yet other embodiments, a is 16.

In some other embodiments of Formula (I), b is 1. In other embodiments, b is 2. In more embodiments, b is 3. In yet other embodiments, b is 4. In some embodiments, b is 5. In other embodiments, b is 6. In more embodiments, b is 7. In yet other embodiments, b is 8. In some embodiments, b is 9. In other embodiments, b is 10. In more embodiments, b is 11. In yet other embodiments, b is 12. In some embodiments, b is 13. In other embodiments, b is 14. In more embodiments, b is 15. In yet other embodiments, b is 16.

In some more embodiments of Formula (I), c is 1. In other embodiments, c is 2. In more embodiments, c is 3. In yet other embodiments, c is 4. In some embodiments, c is 5. In other embodiments, c is 6. In more embodiments, c is 7. In yet other embodiments, c is 8. In some embodiments, c is 9. In other embodiments, c is 10. In more embodiments, c is 11. In yet other embodiments, c is 12. In some embodiments, c is 13. In other embodiments, c is 14. In more embodiments, c is 15. In yet other embodiments, c is 16.

In some certain other embodiments of Formula (I), d is 0. In some embodiments, d is 1. In other embodiments, d is 2. In more embodiments, d is 3. In yet other embodiments, d is 4. In some embodiments, d is 5. In other embodiments, d is 6. In more embodiments, d is 7. In yet other embodiments, d is 8. In some embodiments, d is 9. In other embodiments, d is 10. In more embodiments, d is 11. In yet other embodiments, d is 12. In some embodiments, d is 13. In other embodiments, d is 14. In more embodiments, d is 15. In yet other embodiments, d is 16.

In some other various embodiments of Formula (I), a and d are the same. In some other embodiments, b and c are the same. In some other specific embodiments, a and d are the same and b and c are the same.

The sum of a and b and the sum of c and d in Formula (I) are factors which may be varied to obtain a lipid of Formula (I) having the desired properties. In one embodiment, a and b are chosen such that their sum is an integer ranging from 14 to 24. In other embodiments, c and d are chosen such that their sum is an integer ranging from 14 to 24. In further embodiment, the sum of a and b and the sum of c and d are the same. For example, in some embodiments the sum of a and b and the sum of c and d are both the same integer which may range from 14 to 24. In still more embodiments, a. b, c and d are selected such the sum of a and b and the sum of c and d is 12 or greater.

In some embodiments of Formula (I), e is 1. In other embodiments, e is 2.

The substituents at R^{1a}, R^{2a}, R^{3a} and R^{4a} of Formula (I) are not particularly limited. In certain embodiments R^{1a}, R^{2a}, R^{3a} and R^{4a} are H at each occurrence. In certain other embodiments at least one of R^{1a}, R^{2a}, R^{3a} and R^{4a} is C₁-C₁₂ alkyl. In certain other embodiments at least one of R^{1a}, R^{2a}, R^{3a} and R^{4a} is C₁-C₈ alkyl. In certain other embodiments at least one of R^{1a}, R^{2a}, R^{3a} and R^{4a} is C₁-C₆ alkyl. In some of the foregoing embodiments, the C₁-C₈ alkyl is methyl, ethyl, n-propyl, iso-propyl, n-butyl, iso-butyl, tert-butyl, n-hexyl or n-octyl.

In certain embodiments of Formula (I), R^{1a}, R^{1b}, R^{4a} and R^{4b} are C₁-C₁₂ alkyl at each occurrence.

In further embodiments of Formula (I), at least one of R^{1b}, R^{2b}, R^{3b} and R^{4b} is H or R^{1b}, R^{2b}, R^{3b} and R^{4b} are H at each occurrence.

In certain embodiments of Formula (I), R^{1b} together with the carbon atom to which it is bound is taken together with an adjacent R^{1b} and the carbon atom to which it is bound to form a carbon-carbon double bond. In other embodiments of the foregoing R^{4b} together with the carbon atom to which it is bound is taken together with an adjacent R^{4b} and the carbon atom to which it is bound to form a carbon-carbon double bond.

The substituents at R⁵ and R⁶ of Formula (I) are not particularly limited in the foregoing embodiments. In certain embodiments one or both of R⁵ or R⁶ is methyl. In certain other embodiments one or both of R⁵ or R⁶ is cycloalkyl for example cyclohexyl. In these embodiments the cycloalkyl may be substituted or not substituted. In certain other embodiments the cycloalkyl is substituted with C₁-C₁₂ alkyl, for example tert-butyl.

The substituents at R⁷ are not particularly limited in the foregoing embodiments of Formula I. In certain embodiments at least one R⁷ is H. In some other embodiments, R⁷ is H at each occurrence. In certain other embodiments R⁷ is C₁-C₁₂ alkyl.

In certain other of the foregoing embodiments of Formula (I), one of R⁸ or R⁹ is methyl. In other embodiments, both R⁸ and R⁹ are methyl.

In some different embodiments of Formula (I), R⁸ and R⁹, together with the nitrogen atom to which they are attached, form a 5, 6 or 7-membered heterocyclic ring. In some embodiments of the foregoing, R⁸ and R⁹, together with the nitrogen atom to which they are attached, form a 5-membered heterocyclic ring, for example a pyrrolidinyl ring.

In various different embodiments, the lipid of Formula (I) has one of the structures set forth in Table 1 below.

**Table 1: Representative Lipids of Formula (I)**

| **No.** | **Structure** | **pKa** |
|---|---|---|
| I-1 | 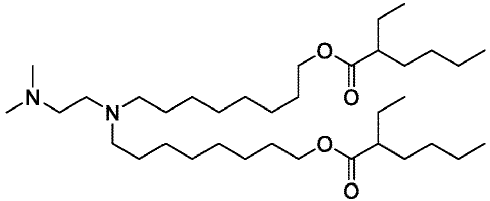 | - |
| I-2 | 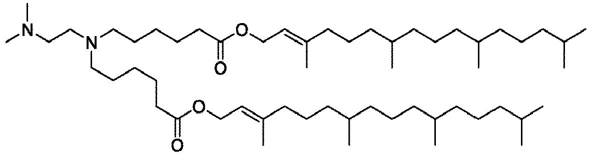 | 5.64 |
| I-3 | 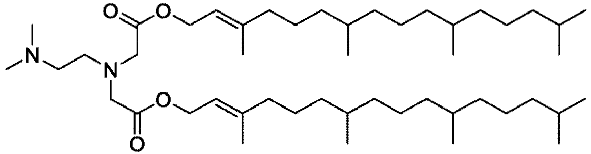 | 7.15 |
| I-4 | 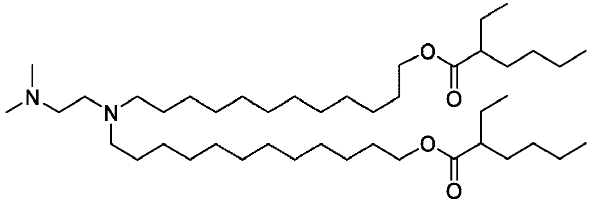 | 6.43 |
| I-5 | 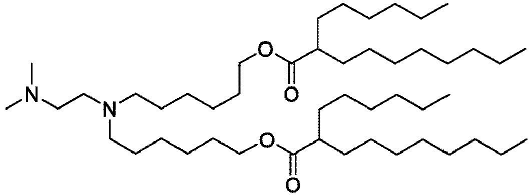 | 6.28 |
| I-6 | 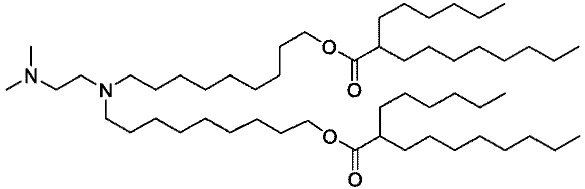 | 6.12 |
| I-7 | 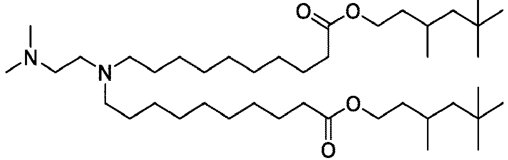 | - |
| I-8 | 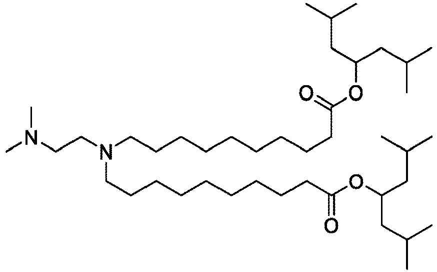 | - |
| I-9 | 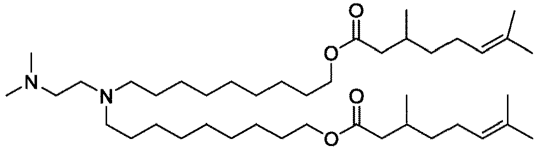 | - |
| I-10 | 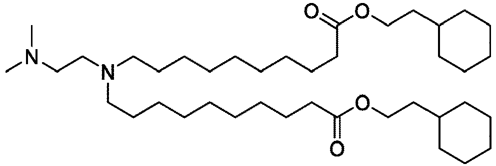 | - |
| I-11 | 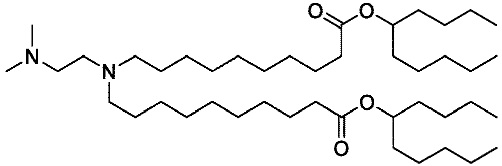 | 6.36 |
| I-12 | 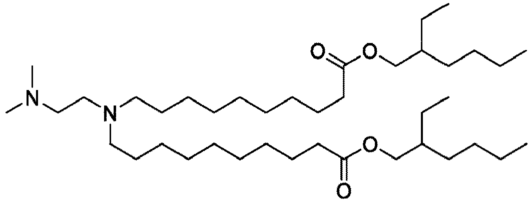 | - |
| I-13 | 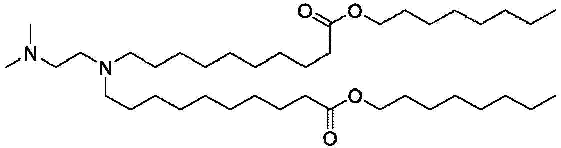 | 6.51 |
| I-14 | 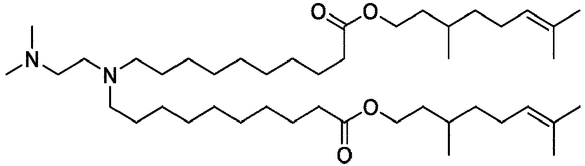 | - |
| I-15 | 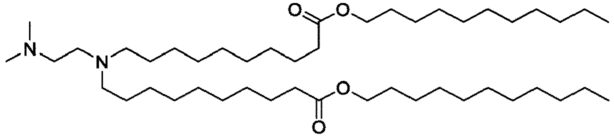 | 6.30 |
| I-16 | 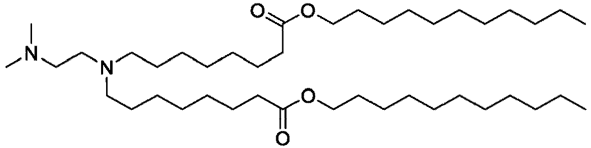 | 6.63 |
| I-17 | 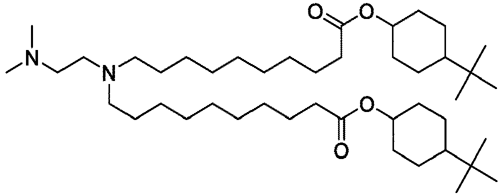 | - |
| I-18 | 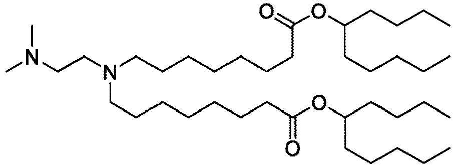 | - |
| I-13 | 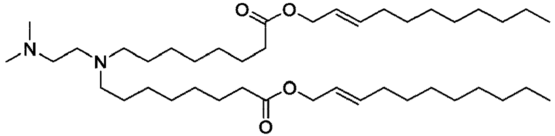 | 6.72 |
| I-20 | 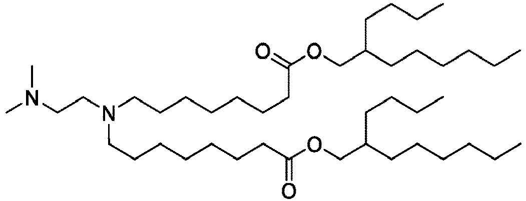 | 6.44 |
| I-21 | 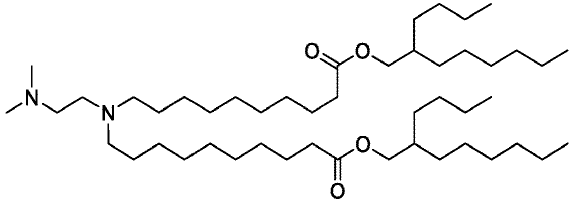 | 6.28 |
| I-22 | 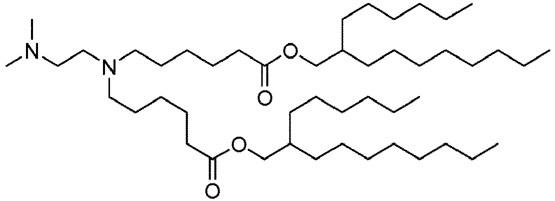 | 6.53 |
| I-23 | 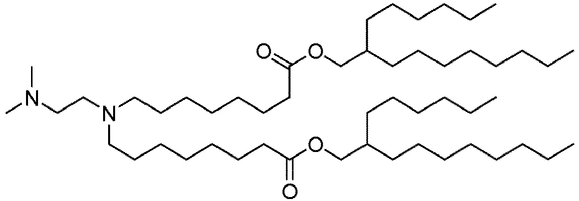 | 6.24 |
| I-24 | 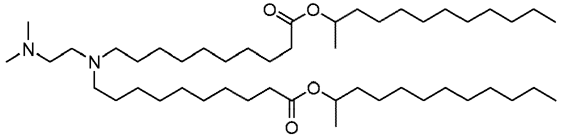 | 6.28 |
| I-25 | 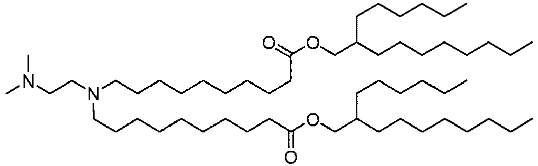 | 6.20 |
| I-26 | 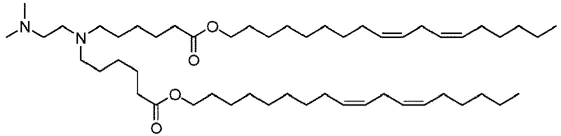 | 6.89 |
| I-27 | 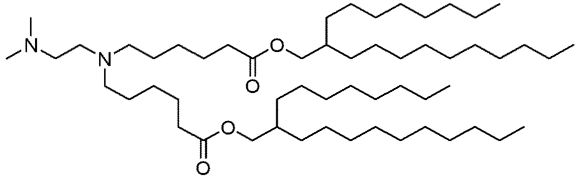 | 6.30 |
| I-28 | 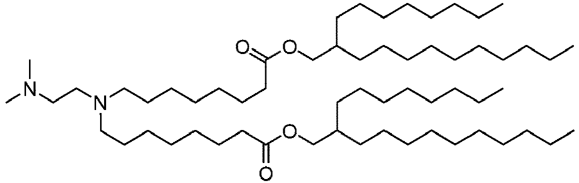 | 6.20 |
| I-29 | 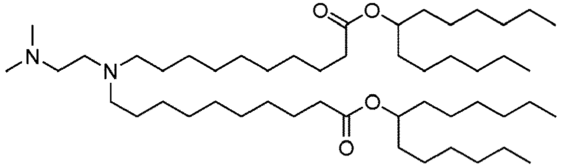 | 6.22 |
| I-30 | 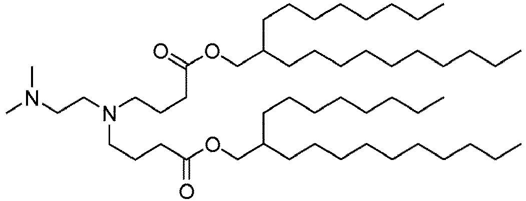 | - |
| I-31 | 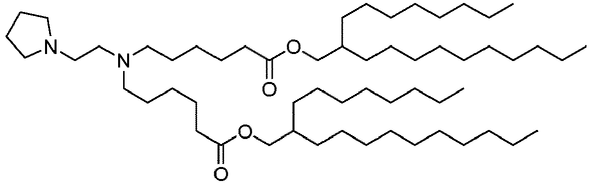 | 6.33 |
| I-32 | 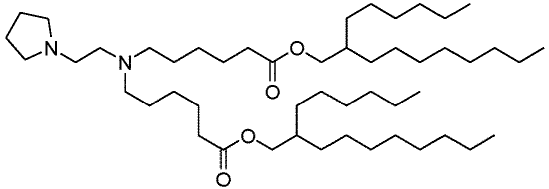 | 6.47 |
| I-33 | 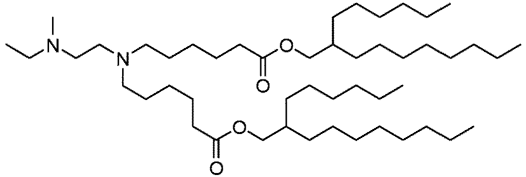 | 6.27 |
| I-34 | 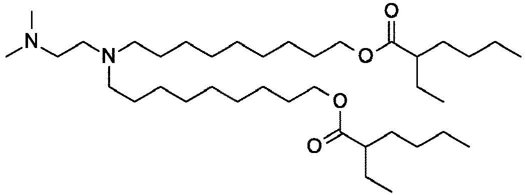 | - |
| I-35 | 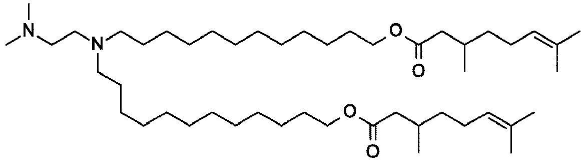 | 6.21 |
| I-36 | 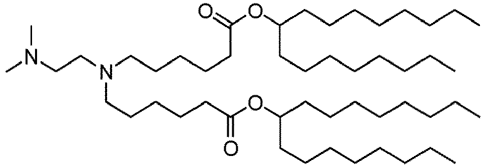 | - |
| I-37 | 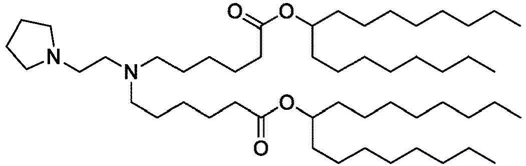 | - |
| I-38 | 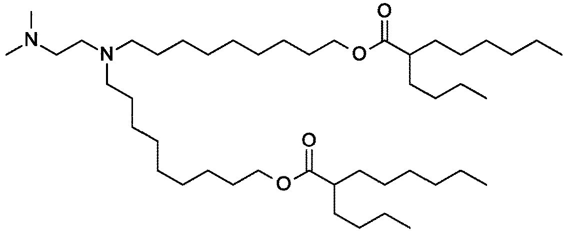 | 6.24 |
| I-39 | 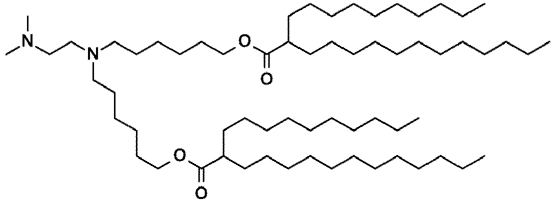 | 5.82 |
| I-40 | 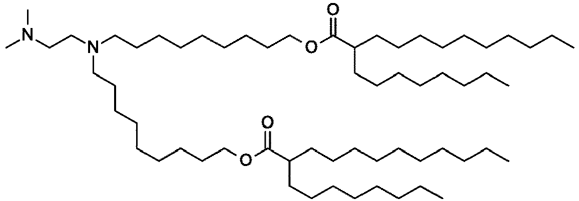 | 6.38 |
| I-41 | 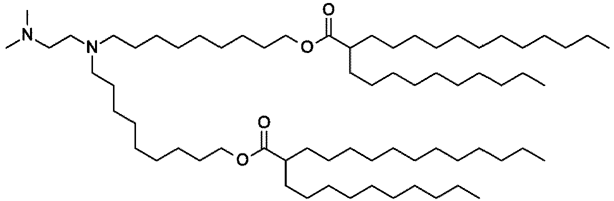 | 5.91 |

In some embodiments, the cationic lipid has a structure of Formula II: 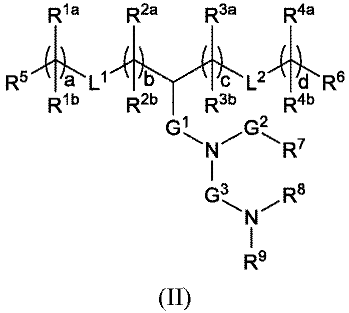 or a pharmaceutically acceptable salt, tautomer or stereoisomer thereof, wherein:
one of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- or -NR^{a}C(=O)O-, and the other of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, ,NR^{a}C(=O)NR^{a}-, -OC(=O)N^{Ra}- or
   -NR^{a}C(=O)O- or a direct bond;
G¹ is C₁-C₂ alkylene, -(C=O)-, -O(C=O)-, -SC(=O)-, -NR^{a}C(=O)- or a direct bond;
G² is -C(=O)-, -(C=O)O-, -C(=O)S-, -C(=O)NR^{a}- or a direct bond;
G³ is C₁-C₆ alkylene;
R^{a} is H or C₁-C₁₂ alkyl;
R^{1a} and R^{1b} are, at each occurrence, independently either: (a) H or C₁-C₁₂ alkyl; or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
R^{2a} and R^{2b} are, at each occurrence, independently either: (a) H or C₁-C₁₂ alkyl; or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
R^{3a} and R^{3b} are, at each occurrence, independently either (a): H or C₁-C₁₂ alkyl; or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
R^{4a} and R^{4b} are, at each occurrence, independently either: (a) H or C₁-C₁₂ alkyl; or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
R⁵ and R⁶ are each independently H or methyl;
R⁷ is C₄-C₂₀ alkyl;
R⁸ and R⁹ are each independently C₁-C₁₂ alkyl; or R⁸ and R⁹, together with the nitrogen atom to which they are attached, form a 5, 6 or 7-membered heterocyclic ring;
a, b, c and d are each independently an integer from 1 to 24; and
x is 0, 1 or 2.

In some embodiments of Formula (II), L¹ and L² are each independently -O(C=O)-, -(C=O)O- or a direct bond. In other embodiments, G¹ and G² are each independently -(C=O)- or a direct bond. In some different embodiments, L¹ and L² are each independently -O(C=O)-, -(C=O)O- or a direct bond; and G¹ and G² are each independently -(C=O)- or a direct bond.

In some different embodiments of Formula (II), L¹ and L² are each independently -C(=O)-, -O-, -S(O)ₓ-, -S-S-, -C(=O)S-, -SC(=O)-, -NR^{a}-, -NR^{a}C(=O)-, -C(=O)NR^{a}-, -NR^{a}C(=O)NR^{a}, -OC(=O)NR^{a}-, -NR^{a}C(=O)O-, -NR^{a}S(O)ₓNR^{a}-, -NR^{a}S( O)ₓ- or -S(O)ₓNR^{a}-.

In other of the foregoing embodiments of Formula (II), the lipid compound has one of the following Formulae (IIA) or (IIB):

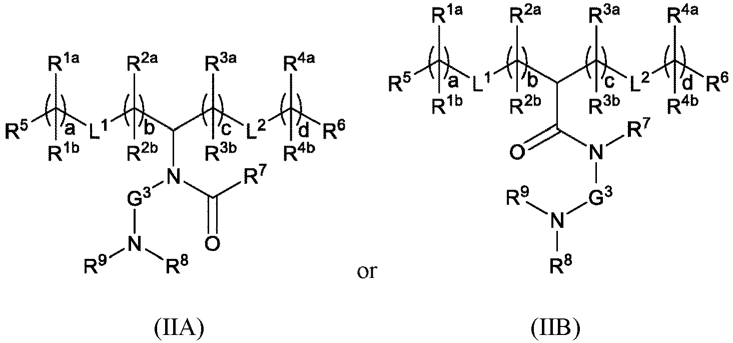

In some embodiments of Formula (II), the lipid compound has Formula (IIA). In other embodiments, the lipid compound has Formula (IIB).

In any of the foregoing embodiments of Formula (II), one of L¹ or L² is -O(C=O)-. For example, in some embodiments each of L¹ and L² are -O(C=O)-.

In some different embodiments of Formula (II), one of L¹ or L² is -(C=O)O-. For example, in some embodiments each of L¹ and L² is -(C=O)O-.

In different embodiments of Formula (II), one of L¹ or L² is a direct bond. As used herein, a "direct bond" means the group (e.g., L¹ or L²) is absent. For example, in some embodiments each of L¹ and L² is a direct bond.

In other different embodiments of Formula (II), for at least one occurrence of R^{1a} and R^{1b}, R^{1a} is H or C₁-C₁₂ alkyl, and R^{1b} together with the carbon atom to which it is bound is taken together with an adjacent R^{1b} and the carbon atom to which it is bound to form a carbon-carbon double bond.

In still other different embodiments of Formula (II), for at least one occurrence of R^{4a} and R^{4b}, R^{4a} is H or C₁-C₁₂ alkyl, and R^{4b} together with the carbon atom to which it is bound is taken together with an adjacent R^{4b} and the carbon atom to which it is bound to form a carbon-carbon double bond.

In more embodiments of Formula (II), for at least one occurrence of R^{2a} and R^{2b}, R^{2a} is H or C₁-C₁₂ alkyl, and R^{2b} together with the carbon atom to which it is bound is taken together with an adjacent R^{2b} and the carbon atom to which it is bound to form a carbon-carbon double bond.

In other different embodiments of Formula (II), for at least one occurrence of R^{3a} and R^{3b}, R^{3a} is H or C₁-C₁₂ alkyl, and R^{3b} together with the carbon atom to which it is bound is taken together with an adjacent R^{3b} and the carbon atom to which it is bound to form a carbon-carbon double bond.

In various other embodiments of Formula (II), the lipid compound has one of the following Formulae (IIC) or (IID): 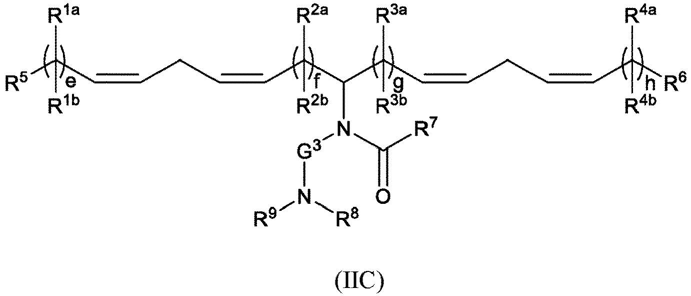 or 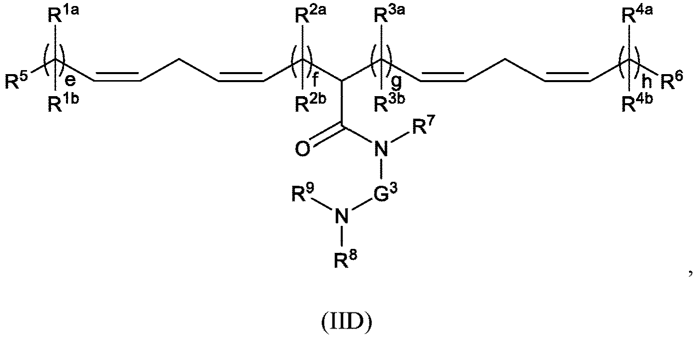 wherein e, f, g and h are each independently an integer from 1 to 12.

In some embodiments of Formula (II), the lipid compound has Formula (IIC). In other embodiments, the lipid compound has Formula (IID).

In various embodiments of Formulae (IIC) or (IID), e, f, g and h are each independently an integer from 4 to 10.

In certain embodiments of Formula (II), a, b, c and d are each independently an integer from 2 to 12 or an integer from 4 to 12. In other embodiments, a, b, c and d are each independently an integer from 8 to 12 or 5 to 9. In some certain embodiments, a is 0. In some embodiments, a is 1. In other embodiments, a is 2. In more embodiments, a is 3. In yet other embodiments, a is 4. In some embodiments, a is 5. In other embodiments, a is 6. In more embodiments, a is 7. In yet other embodiments, a is 8. In some embodiments, a is 9. In other embodiments, a is 10. In more embodiments, a is 11. In yet other embodiments, a is 12. In some embodiments, a is 13. In other embodiments, a is 14. In more embodiments, a is 15. In yet other embodiments, a is 16.

In some embodiments of Formula (II), b is 1. In other embodiments, b is 2. In more embodiments, b is 3. In yet other embodiments, b is 4. In some embodiments, b is 5. In other embodiments, b is 6. In more embodiments, b is 7. In yet other embodiments, b is 8. In some embodiments, b is 9. In other embodiments, b is 10. In more embodiments, b is 11. In yet other embodiments, b is 12. In some embodiments, b is 13. In other embodiments, b is 14. In more embodiments, b is 15. In yet other embodiments, b is 16.

In some embodiments of Formula (II), c is 1. In other embodiments, c is 2. In more embodiments, c is 3. In yet other embodiments, c is 4. In some embodiments, c is 5. In other embodiments, c is 6. In more embodiments, c is 7. In yet other embodiments, c is 8. In some embodiments, c is 9. In other embodiments, c is 10. In more embodiments, c is 11. In yet other embodiments, c is 12. In some embodiments, c is 13. In other embodiments, c is 14. In more embodiments, c is 15. In yet other embodiments, c is 16.

In some certain embodiments of Formula (II), d is 0. In some embodiments, d is 1. In other embodiments, d is 2. In more embodiments, d is 3. In yet other embodiments, d is 4. In some embodiments, d is 5. In other embodiments, d is 6. In more embodiments, d is 7. In yet other embodiments, d is 8. In some embodiments, d is 9. In other embodiments, d is 10. In more embodiments, d is 11. In yet other embodiments, d is 12. In some embodiments, d is 13. In other embodiments, d is 14. In more embodiments, d is 15. In yet other embodiments, d is 16.

In some embodiments of Formula (II), e is 1. In other embodiments, e is 2. In more embodiments, e is 3. In yet other embodiments, e is 4. In some embodiments, e is 5. In other embodiments, e is 6. In more embodiments, e is 7. In yet other embodiments, e is 8. In some embodiments, e is 9. In other embodiments, e is 10. In more embodiments, e is 11. In yet other embodiments, e is 12.

In some embodiments of Formula (II), f is 1. In other embodiments, f is 2. In more embodiments, f is 3. In yet other embodiments, f is 4. In some embodiments, f is 5. In other embodiments, f is 6. In more embodiments, f is 7. In yet other embodiments, f is 8. In some embodiments, f is 9. In other embodiments, f is 10. In more embodiments, f is 11. In yet other embodiments, f is 12.

In some embodiments of Formula (II), g is 1. In other embodiments, g is 2. In more embodiments, g is 3. In yet other embodiments, g is 4. In some embodiments, g is 5. In other embodiments, g is 6. In more embodiments, g is 7. In yet other embodiments, g is 8. In some embodiments, g is 9. In other embodiments, g is 10. In more embodiments, g is 11. In yet other embodiments, g is 12.

In some embodiments of Formula (II), h is 1. In other embodiments, e is 2. In more embodiments, h is 3. In yet other embodiments, h is 4. In some embodiments, e is 5. In other embodiments, h is 6. In more embodiments, h is 7. In yet other embodiments, h is 8. In some embodiments, h is 9. In other embodiments, h is 10. In more embodiments, h is 11. In yet other embodiments, h is 12.

In some other various embodiments of Formula (II), a and d are the same. In some other embodiments, b and c are the same. In some other specific embodiments and a and d are the same and b and c are the same.

The sum of a and b and the sum of c and d of Formula (II) are factors which may be varied to obtain a lipid having the desired properties. In one embodiment, a and b are chosen such that their sum is an integer ranging from 14 to 24. In other embodiments, c and d are chosen such that their sum is an integer ranging from 14 to 24. In further embodiment, the sum of a and b and the sum of c and d are the same. For example, in some embodiments the sum of a and b and the sum of c and d are both the same integer which may range from 14 to 24. In still more embodiments, a. b, c and d are selected such that the sum of a and b and the sum of c and d is 12 or greater.

The substituents at R^{1a}, R^{2a}, R^{3a} and R^{4a} of Formula (II) are not particularly limited. In some embodiments, at least one of R^{1a}, R^{2a}, R^{3a} and R^{4a} is H. In certain embodiments R^{1a}, R^{2a}, R^{3a} and R^{4a} are H at each occurrence. In certain other embodiments at least one of R^{1a}, R^{2a}, R^{3a} and R^{4a} is C₁-C₁₂ alkyl. In certain other embodiments at least one of R^{1a}, R^{2a}, R^{3a} and R^{4a} is Ci-Cs alkyl. In certain other embodiments at least one of R^{1a}, R^{2a}, R^{3a} and R^{4a} is C₁-C₆ alkyl. In some of the foregoing embodiments, the C₁-C₈ alkyl is methyl, ethyl, n-propyl, iso-propyl, n-butyl, iso-butyl, tert-butyl, n-hexyl or n-octyl.

In certain embodiments of Formula (II), R^{1a}, R^{1b}, R^{4a} and R^{4b} are C₁-C₁₂ alkyl at each occurrence.

In further embodiments of Formula (II), at least one of R^{1b}, R^{2b}, R^{3b} and R^{4b} is H or R^{1b}, R^{2b}, R^{3b} and R^{4b} are H at each occurrence.

In certain embodiments of Formula (II), R^{1b} together with the carbon atom to which it is bound is taken together with an adjacent R^{1b} and the carbon atom to which it is bound to form a carbon-carbon double bond. In other embodiments of the foregoing R^{4b} together with the carbon atom to which it is bound is taken together with an adjacent R^{4b} and the carbon atom to which it is bound to form a carbon-carbon double bond.

The substituents at R⁵ and R⁶ of Formula (II) are not particularly limited in the foregoing embodiments. In certain embodiments one of R⁵ or R⁶ is methyl. In other embodiments each of R⁵ or R⁶ is methyl.

The substituents at R⁷ of Formula (II) are not particularly limited in the foregoing embodiments. In certain embodiments R⁷ is C₆-C₁₆ alkyl. In some other embodiments, R⁷ is C₆-C₉ alkyl. In some of these embodiments, R⁷ is substituted with -(C=O)OR^{b}, -O(C=O)R^{b}, -C(=O)R^{b}, -OR^{b}, -S(O)ₓR^{b}, -S-SR^{b}, -C(=O)SR^{b}, -SC(=O)R^{b}, -NR^{a}R^{b}, -NR^{a}C(=O)R^{b}, -C(=O)NR^{a}R^{b}, -NR^{a}C(=O)NR^{a}R^{b}, -OC(=O)NR^{a}R^{b}, -NR^{a}C(=O)OR^{b}, -NR^{a}S(O)ₓNR^{a}R^{b}, =NR^{a}S(O)ₓR^{b} or -S(O)ₓNR^{a}R^{b}, wherein: R^{a} is H or C₁-C₁₂ alkyl; R^{b} is C₁-C₁₅ alkyl; and x is 0, 1 or 2. For example, in some embodiments R⁷ is substituted with -(C=O)OR^{b} or -O(C=O)R^{b}.

In some of the foregoing embodiments of Formula (II), R^{b} is branched Ci-C₁₆ alkyl. For example, in some embodiments R^{b} has one of the following structures: 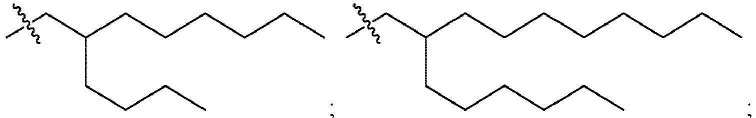 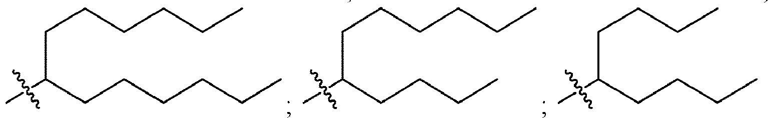 or

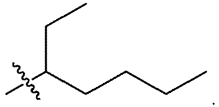

In certain other of the foregoing embodiments of Formula (II), one of R⁸ or R⁹ is methyl. In other embodiments, both R⁸ and R⁹ are methyl.

In some different embodiments of Formula (II), R⁸ and R⁹, together with the nitrogen atom to which they are attached, form a 5, 6 or 7-membered heterocyclic ring. In some embodiments of the foregoing, R⁸ and R⁹, together with the nitrogen atom to which they are attached, form a 5-membered heterocyclic ring, for example a pyrrolidinyl ring. In some different embodiments of the foregoing, R⁸ and R⁹, together with the nitrogen atom to which they are attached, form a 6-membered heterocyclic ring, for example a piperazinyl ring.

In still other embodiments of the foregoing lipids of Formula (II), G³ is C₂-C₄ alkylene, for example C₃ alkylene. In various different embodiments, the lipid compound has one of the structures set forth in Table 2 below

**Table 2: Representative Lipids of Formula (II)**

| **No.** | **Structure** | **pKa** |
|---|---|---|
| II-1 | 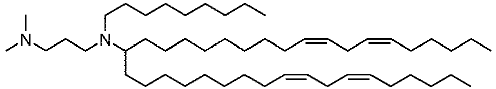 | 5.64 |
| II-2 | 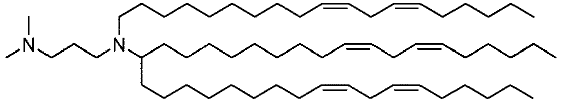 | - |
| II-3 | 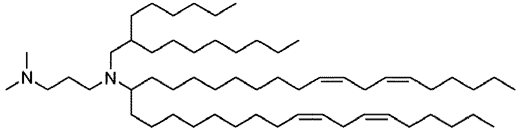 | - |
| II-4 | 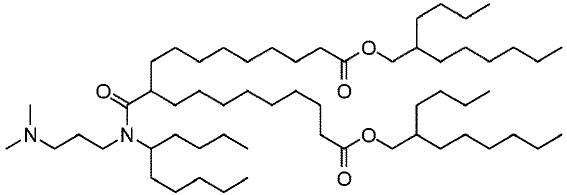 | - |
| II-5 | 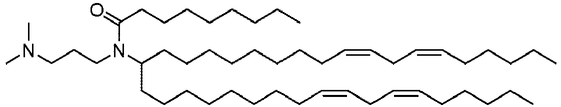 | 6.27 |
| II-6 | 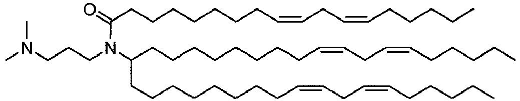 | 6.14 |
| II-7 | 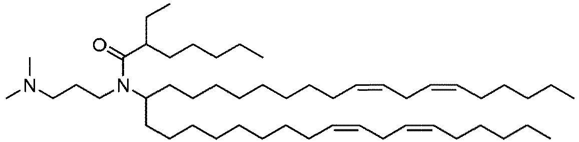 | 5.93 |
| II-8 | 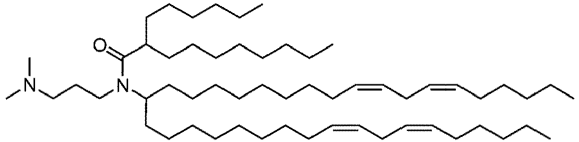 | 5.35 |
| II-9 | 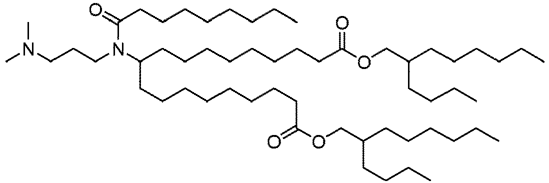 | 6.27 |
| II-10 | 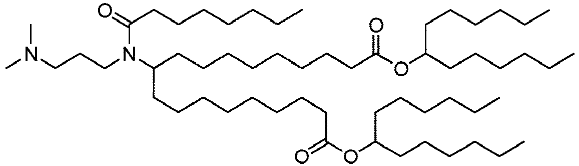 | 6.16 |
| II-11 | 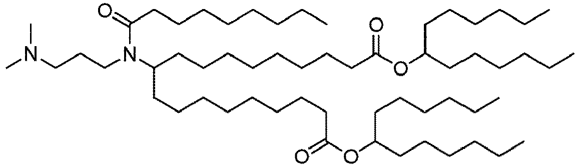 | 6.13 |
| II-12 | 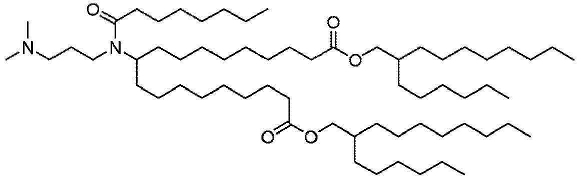 | 6.21 |
| II-13 | 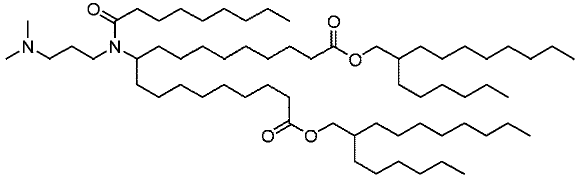 | 6.22 |
| II-14 | 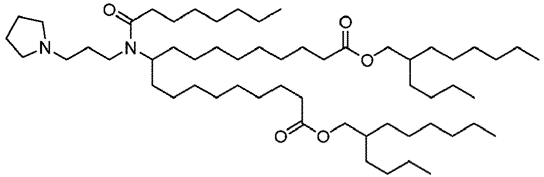 | 6.33 |
| II-15 | 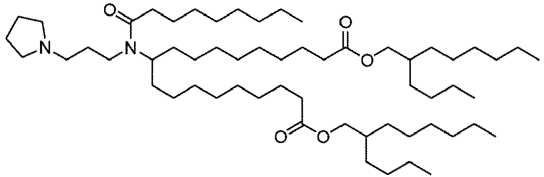 | 6.32 |
| II-16 | 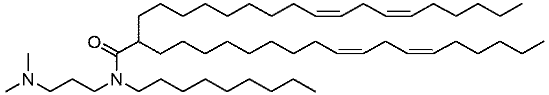 | 6.37 |
| II-17 | 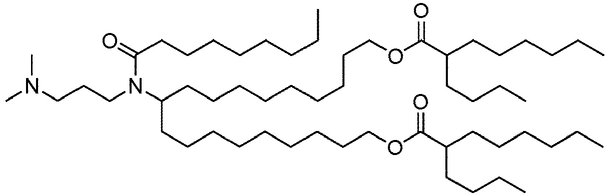 | 6.27 |
| II-18 | 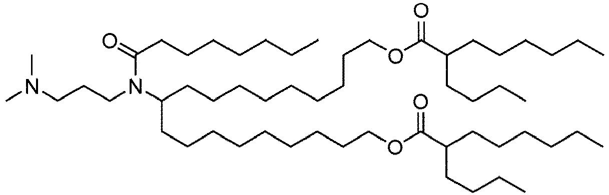 | - |
| II-19 | 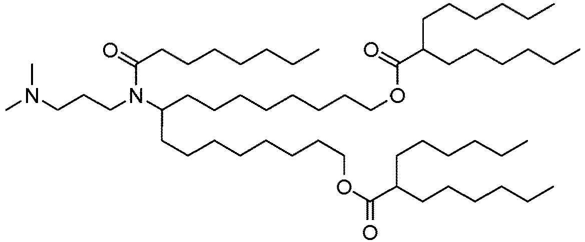 | - |
| II-20 | 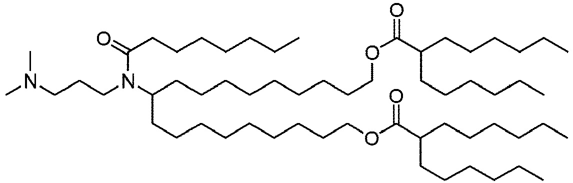 | - |
| II-21 | 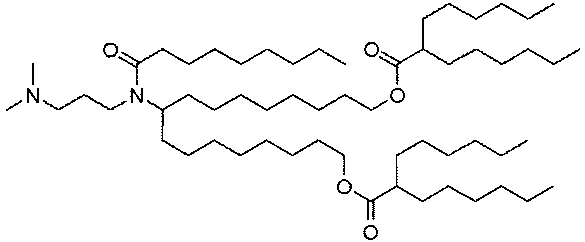 | - |
| II-22 | 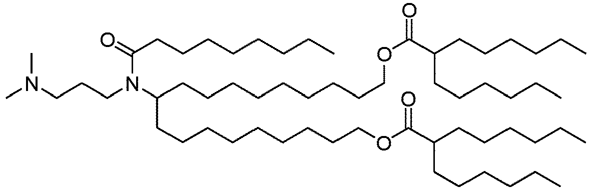 | - |
| II-23 | 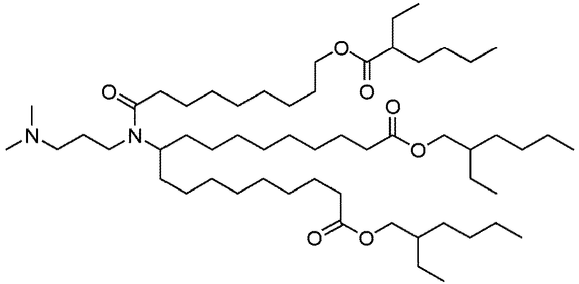 | - |
| II-24 | 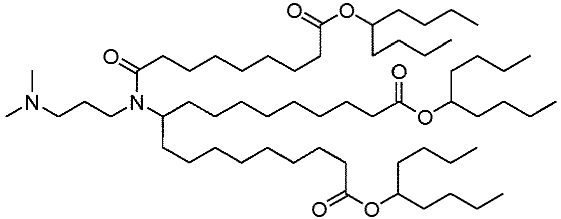 | 6.14 |
| II-25 | 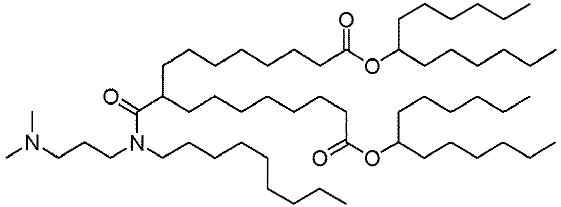 | - |
| II-26 | 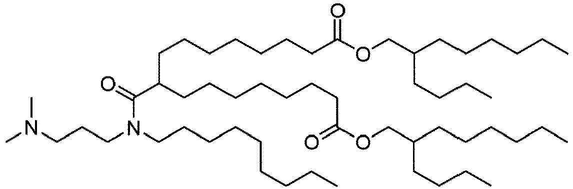 | - |
| II-27 | 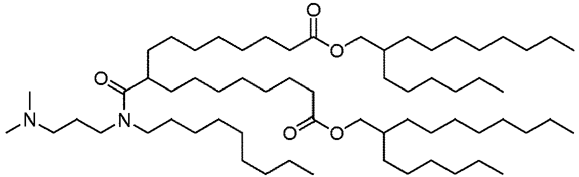 | - |
| II-28 | 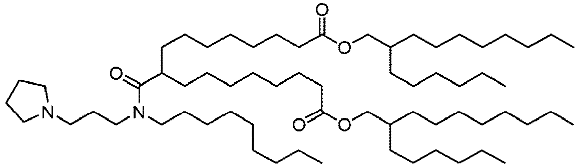 | - |
| II-29 | 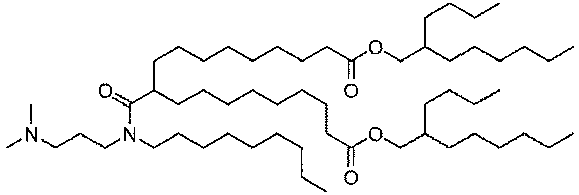 | - |
| II-30 | 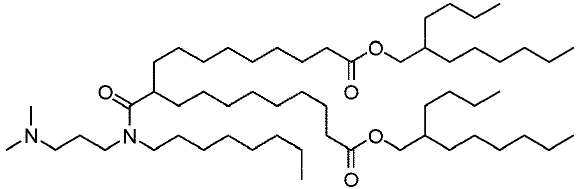 | - |
| II-31 | 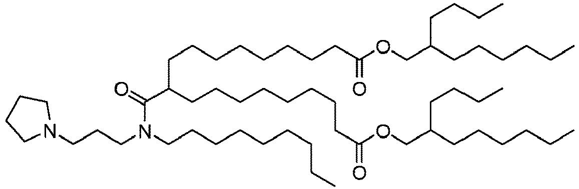 | - |
| II-32 | 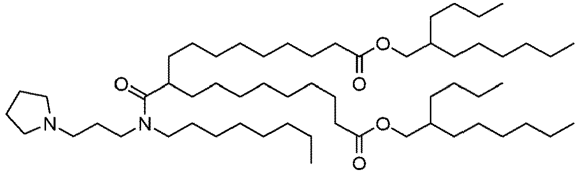 | - |
| II-33 | 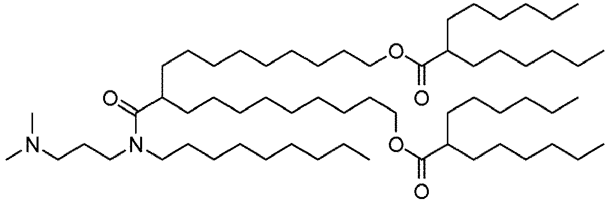 | - |
| II-34 | 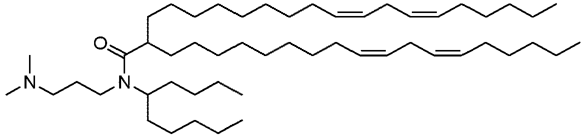 | - |
| II-35 | 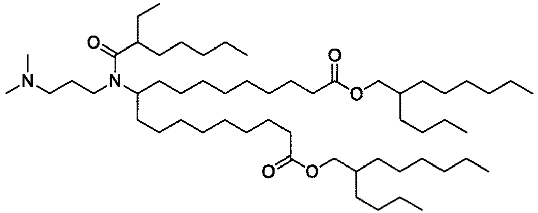 | 5.97 |
| II-36 | 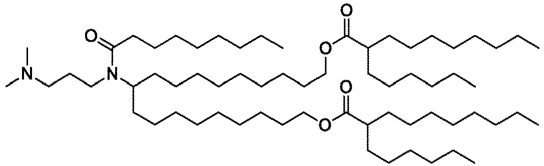 | 6.13 |
| II-37 | 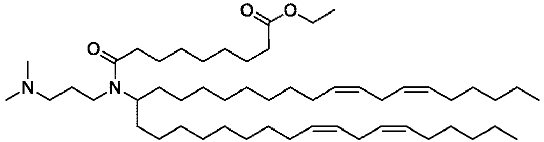 | 5.61 |
| II-38 | 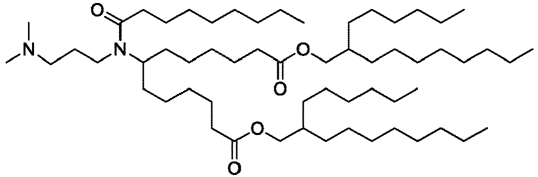 | 6.45 |
| II-39 | 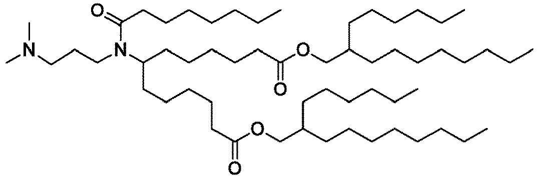 | 6.45 |
| II-40 | 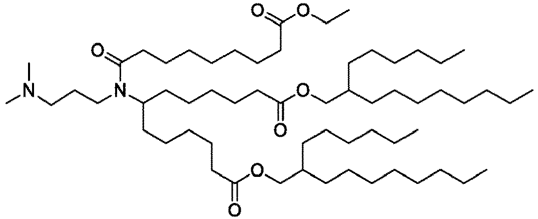 | 6.57 |
| II-41 | 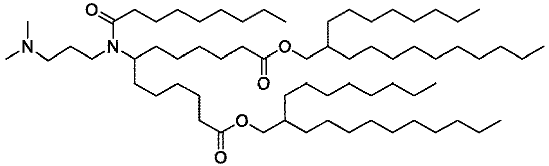 | - |
| II-42 | 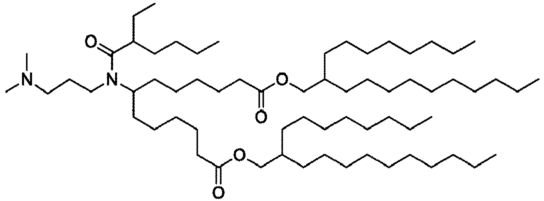 | - |
| II-43 | 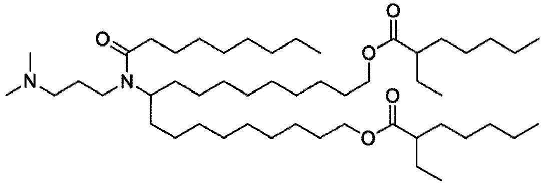 | - |
| II-44 | 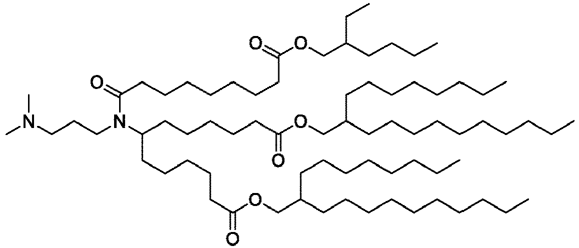 | - |
| II-45 | 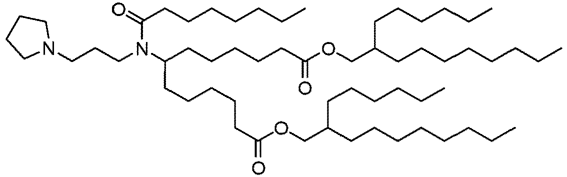 | - |
| II-46 | 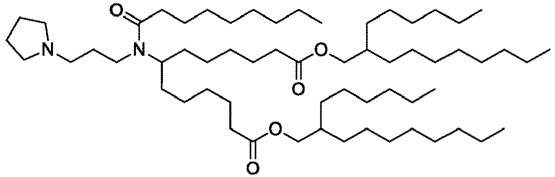 | - |

In some other embodiments, the cationic lipid has a structure of Formula (III): 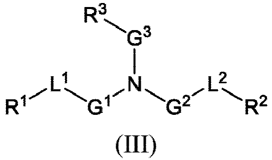 or a pharmaceutically acceptable salt or stereoisomer thereof, wherein:
one of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- or -NR^{a}C(=O)O-, and the other of L¹ or L² is -O(C=O)-, -(C=O)O-, -C(=O)-, -O-, -S(O)ₓ-, -S-S-, -C(=O)S-, SC(=0)-, -NR^{a}C(=O)-, -C(=O)NR^{a}-, ,NR^{a}C(=O)NR^{a}-, -OC(=O)NR^{a}- or
   -NR^{a}C(=O)O- or a direct bond;
G¹ and G² are each independently unsubstituted C₁-C₁₂ alkylene or C₁-C₁₂ alkenylene;
G³ is C₁-C₂₄ alkylene, C₁-C₂₄ alkenylene, C₃-C₈ cycloalkylene, C₃-C₈ cycloalkenylene;
R^{a} is H or C₁-C₁₂ alkyl;
R¹ and R² are each independently C₆-C₂₄ alkyl or C₆-C₂₄ alkenyl;
R³ is H, OR⁵, CN, -C(=O)OR⁴, -OC(=O)R⁴ or -NR⁵C(=O)R⁴;
R⁴ is C₁-C₁₂ alkyl;
R⁵ is H or C₁-C₆ alkyl; and
x is 0, 1 or 2.

In some of the foregoing embodiments of Formula (III), the lipid has one of the following Formulae (IIIA) or (IIIB): 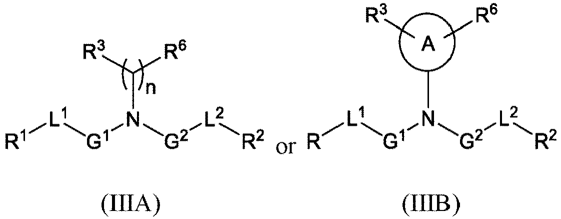 wherein:
A is a 3 to 8-membered cycloalkyl or cycloalkylene ring;
R⁶ is, at each occurrence, independently H, OH or C₁-C₂₄ alkyl;
n is an integer ranging from 1 to 15.

In some of the foregoing embodiments of Formula (III), the lipid has Formula (IIIA), and in other embodiments, the lipid has Formula (IIIB).

In other embodiments of Formula (III), the lipid has one of the following Formulae (IIIC) or (IIID): 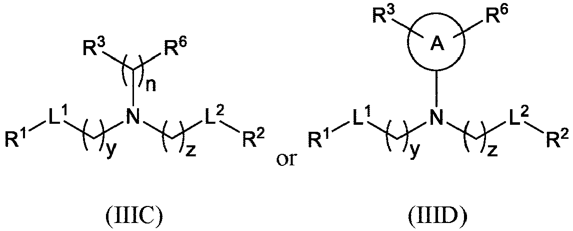 wherein y and z are each independently integers ranging from 1 to 12.

In any of the foregoing embodiments of Formula (III), one of L¹ or L² is -O(C=O)-. For example, in some embodiments each of L¹ and L² are -O(C=O)-. In some different embodiments of any of the foregoing, L¹ and L² are each independently -(C=O)O- or -O(C=O)-. For example, in some embodiments each of L¹ and L² is -(C=O)O-.

In some different embodiments of Formula (III), the lipid has one of the following Formulae (IIIE) or (IIIF):

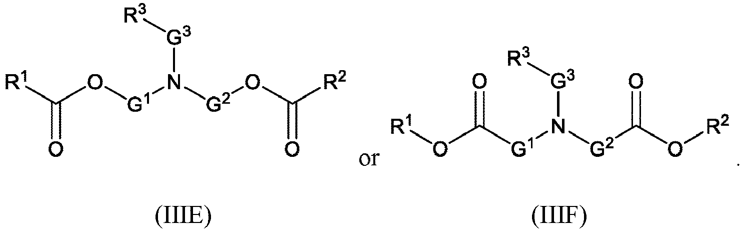

In some of the foregoing embodiments of Formula (III), the lipid has one of the following Formulae (IIIG), (IIIH), (IIII), or (IIIJ):

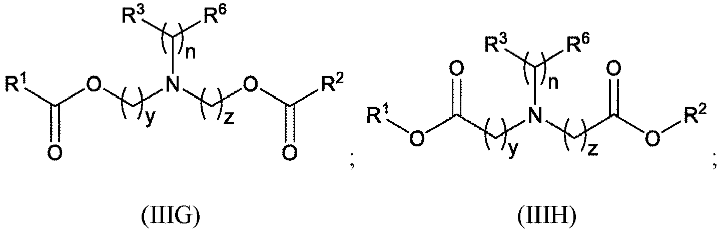

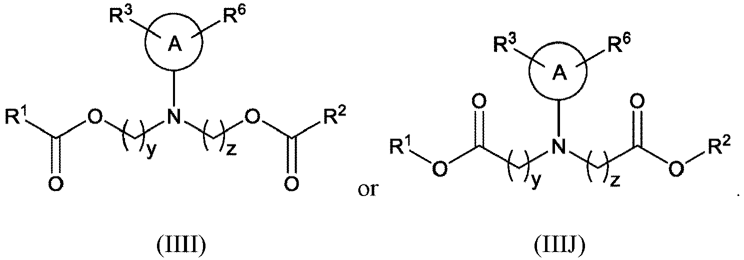

In some of the foregoing embodiments of Formula (III), n is an integer ranging from 2 to 12, for example from 2 to 8 or from 2 to 4. For example, in some embodiments, n is 3, 4, 5 or 6. In some embodiments, n is 3. In some embodiments, n is 4. In some embodiments, n is 5. In some embodiments, n is 6.

In some other of the foregoing embodiments of Formula (III), y and z are each independently an integer ranging from 2 to 10. For example, in some embodiments, y and z are each independently an integer ranging from 4 to 9 or from 4 to 6.

In some of the foregoing embodiments of Formula (III), R⁶ is H. In other of the foregoing embodiments, R⁶ is C₁-C₂₄ alkyl. In other embodiments, R⁶ is OH.

In some embodiments of Formula (III), G³ is unsubstituted. In other embodiments, G3 is substituted. In various different embodiments, G³ is linear C₁-C₂₄ alkylene or linear C₁-C₂₄ alkenylene.

In some other foregoing embodiments of Formula (III), R¹ or R², or both, is C₆-C₂₄ alkenyl. For example, in some embodiments, R¹ and R² each, independently have the following structure: 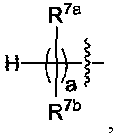 wherein:
R^{7a} and R^{7b} are, at each occurrence, independently H or C₁-C₁₂ alkyl; and
a is an integer from 2 to 12,
wherein R^{7a}, R^{7b} and a are each selected such that R¹ and R² each independently comprise from 6 to 20 carbon atoms. For example, in some embodiments a is an integer ranging from 5 to 9 or from 8 to 12.

In some of the foregoing embodiments of Formula (III), at least one occurrence of R^{7a} is H. For example, in some embodiments, R^{7a} is H at each occurrence. In other different embodiments of the foregoing, at least one occurrence of R^{7b} is C₁-C₈ alkyl. For example, in some embodiments, C₁-C₈ alkyl is methyl, ethyl, n-propyl, isopropyl, n-butyl, iso-butyl, tert-butyl, n-hexyl or n-octyl.

In different embodiments of Formula (III), R¹ or R², or both, has one of the following structures:

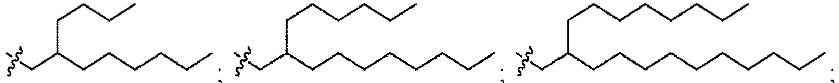

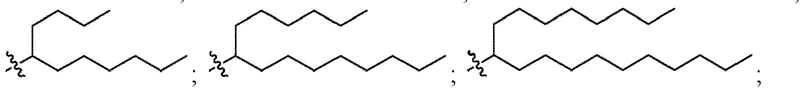

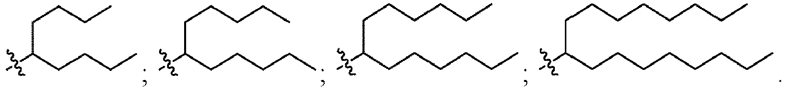

In some of the foregoing embodiments of Formula (III), R³ is OH, CN, -C(=O)OR⁴, -OC(=O)R⁴ or -NHC(=O)R⁴. In some embodiments, R⁴ is methyl or ethyl.

In various different embodiments, a cationic lipid has one of the structures set forth in Table 3 below.

**Table 3: Representative Compounds of Formula (III)**

| **No.** | **Structure** | **pKa** |
|---|---|---|
| III-1 | 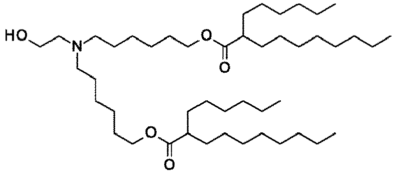 | 5.89 |
| III-2 | 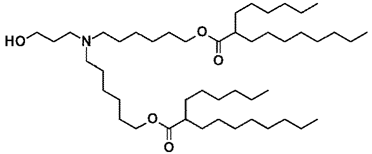 | 6.05 |
| III-3 | 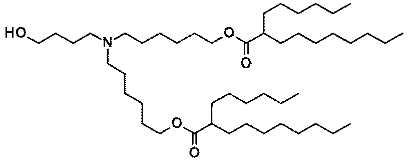 | 6.09 |
| III-4 | 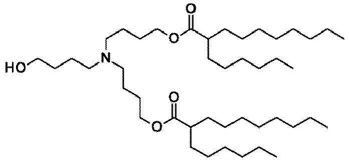 | 5.60 |
| III-5 | 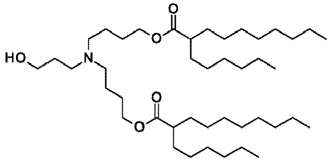 | 5.59 |
| III-6 | 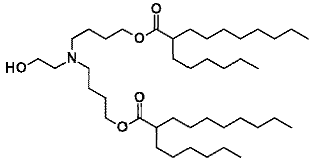 | 5.42 |
| III-7 | 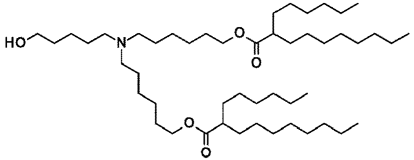 | 6.11 |
| III-8 | 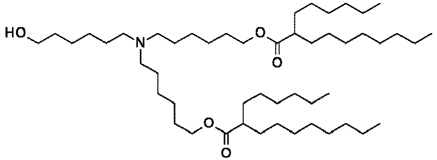 | 5.84 |
| III-9 | 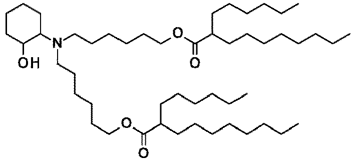 | - |
| III-10 | 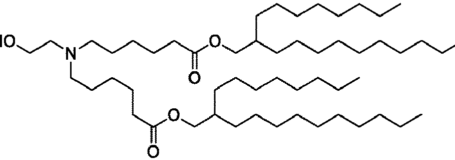 | - |
| III-11 | 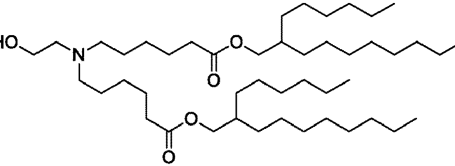 | - |
| III-12 | 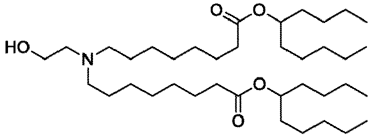 | - |
| III-13 | 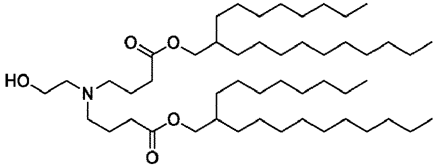 | - |
| III-14 | 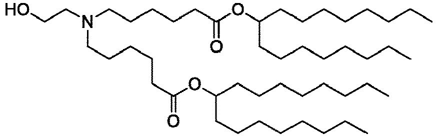 | - |
| III-15 | 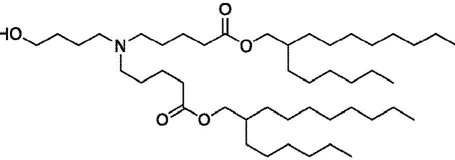 I | 6.14 |
| III-16 | 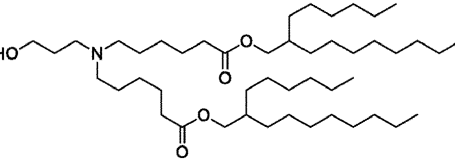 | 6.31 |
| III-17 | 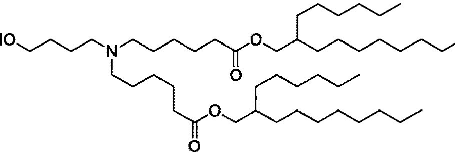 | 6.28 |
| III-18 | 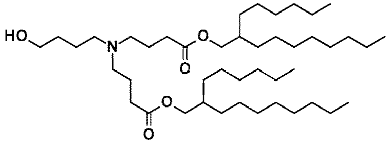 | - |
| III-19 | 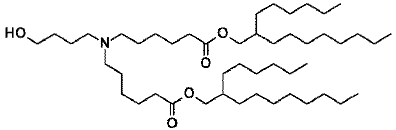 | - |
| III-20 | 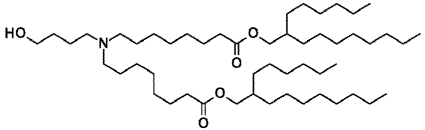 | 6.36 |
| III-21 | 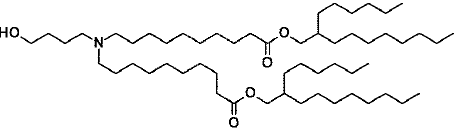 | - |
| III-22 | 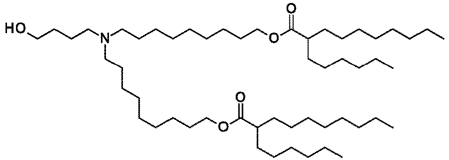 | 6.10 |
| III-23 | 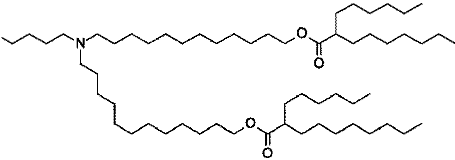 | 5.98 |
| III-24 | 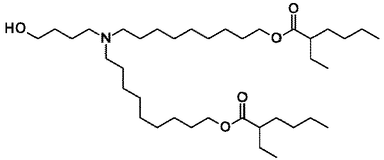 | - |
| III-25 | 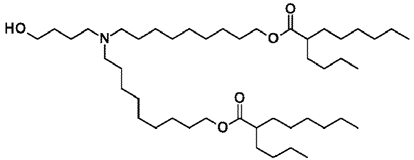 | 6.22 |
| III-26 | 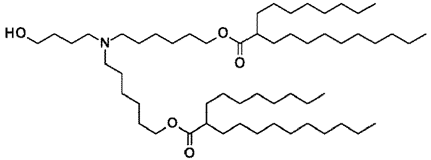 | 5.84 |
| III-27 | 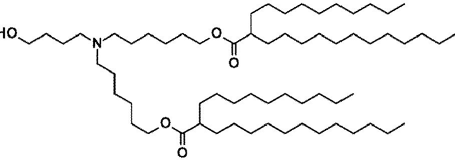 | 5.77 |
| III-28 | 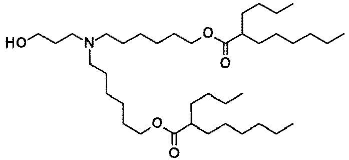 | - |
| III-29 | 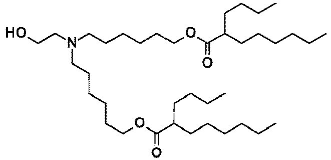 | - |
| III-30 | 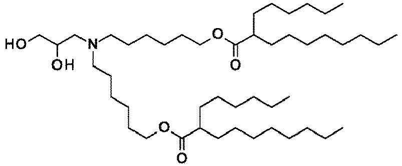 | 6.09 |
| III-31 | 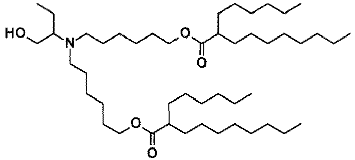 | - |
| III-32 | 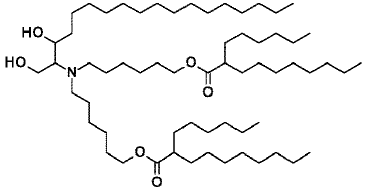 | - |
| III-33 | 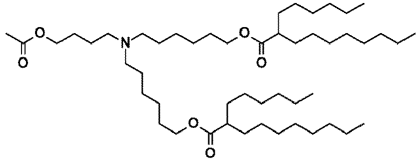 | - |
| III-34 | 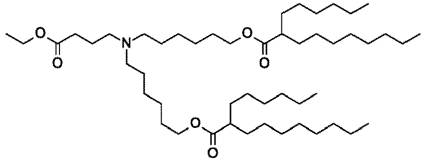 | - |
| III-35 | 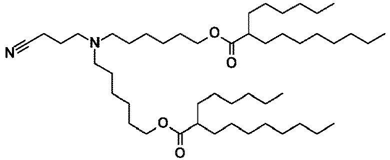 | - |
| III-36 | 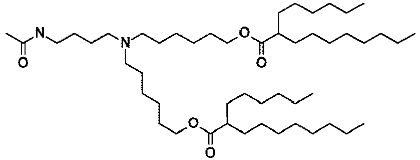 | - |
| III-37 | 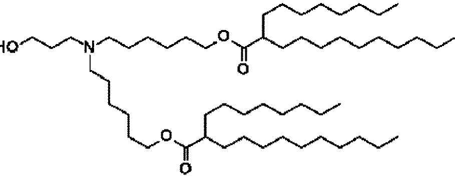 | - |
| III-38 | 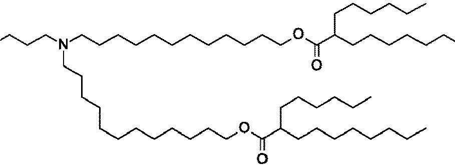 | - |
| III-39 | 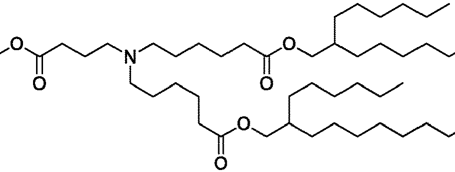 | - |
| III-40 | 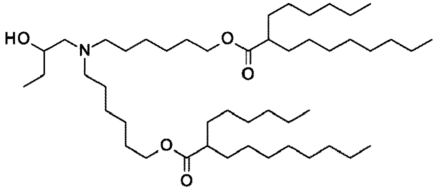 | - |
| III-41 | 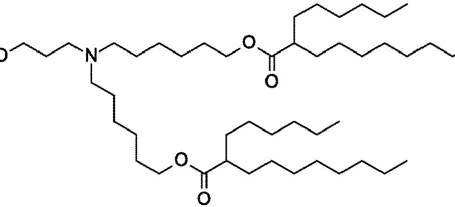 | - |
| III-42 | 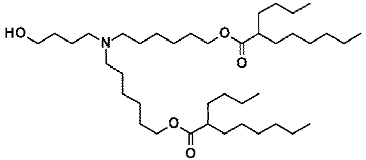 | - |
| III-43 | 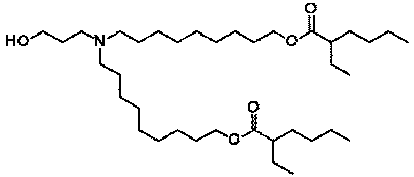 | - |
| III-44 | 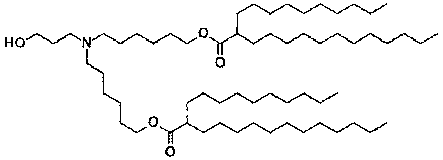 | - |
| III-45 | 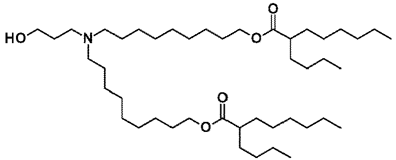 | - |
| III-46 | 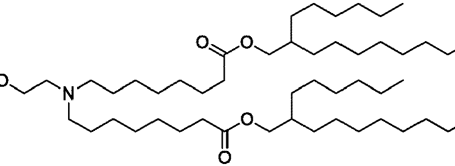 | - |
| III-47 | 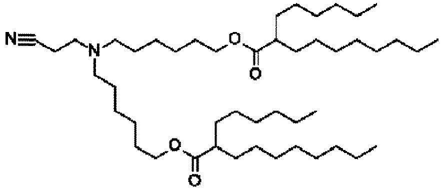 | - |
| III-48 | 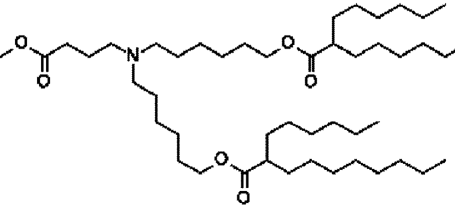 | - |
| III-49 | 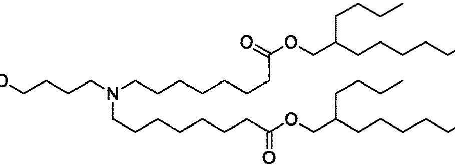 | - |

In one embodiment, the cationic lipid has a structure of Formula (IV): 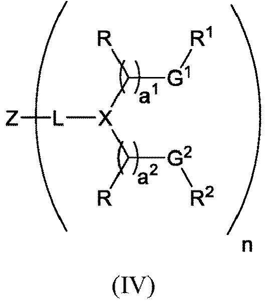 or a pharmaceutically acceptable salt or stereoisomer thereof, wherein:
one of G¹ or G² is, at each occurrence, -O(C=O)-, -(C=O)O-, -C(=O)-, -O-, -S(O)_{y}-, -S-S-, -C(=O)S-, SC(=O)-, -N(R^{a})C(=O)-, -C(=O)N(R^{a})-, -N(R^{a})C(=O)N(R^{a})-, -OC(=O)N(R^{a})- or -N(R^{a})C(=O)O-, and the other of G¹ or G² is, at each occurrence, -O(C=O)-, -(C=O)O-, -C(=O)-, -O-, -S(O)_{y}-, -S-S-, -C(=O)S-, -SC(=O)-, -N(R^{a})C(=O)-, -C(=O)N(R^{a})-, -N(R^{a})C(=O)N(R^{a})-, -OC(=O)N(R^{a})- or -N(R^{a})C(=O)O- or a direct bond;
L is, at each occurrence, ~O(C=O)-, wherein ~ represents a covalent bond to X;
X is CR^{a};
Z is alkyl, cycloalkyl or a monovalent moiety comprising at least one polar functional group when n is 1; or Z is alkylene, cycloalkylene or a polyvalent moiety comprising at least one polar functional group when n is greater than 1;
R^{a} is, at each occurrence, independently H, C₁-C₁₂ alkyl, C₁-C₁₂ hydroxylalkyl, C₁-C₁₂ aminoalkyl, C₁-C₁₂ alkylaminylalkyl, C₁-C₁₂ alkoxyalkyl, C₁-C₁₂ alkoxycarbonyl, C₁-C₁₂ alkylcarbonyloxy, C₁-C₁₂ alkylcarbonyloxyalkyl or C₁-C₁₂ alkylcarbonyl;
R is, at each occurrence, independently either: (a) H or C₁-C₁₂ alkyl; or (b) R together with the carbon atom to which it is bound is taken together with an adjacent R and the carbon atom to which it is bound to form a carbon-carbon double bond;
R¹ and R² have, at each occurrence, the following structure, respectively: 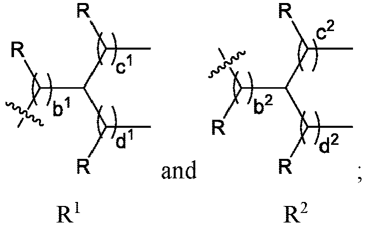
a¹ and a² are, at each occurrence, independently an integer from 3 to 12;
b¹ and b² are, at each occurrence, independently 0 or 1;
c¹ and c² are, at each occurrence, independently an integer from 5 to 10;
d¹ and d² are, at each occurrence, independently an integer from 5 to 10;
y is, at each occurrence, independently an integer from 0 to 2; and
n is an integer from 1 to 6,
wherein each alkyl, alkylene, hydroxylalkyl, aminoalkyl, alkylaminylalkyl, alkoxyalkyl, alkoxycarbonyl, alkylcarbonyloxy, alkylcarbonyloxyalkyl and alkylcarbonyl is optionally substituted with one or more substituent.

In some embodiments of Formula (IV), G¹ and G² are each independently -O(C=O)- or -(C=O)O-.

In other embodiments of Formula (IV), X is CH.

In different embodiments of Formula (IV), the sum of a¹ + b¹ + c¹ or the sum of a² + b² + c² is an integer from 12 to 26.

In still other embodiments of Formula (IV), a¹ and a² are independently an integer from 3 to 10. For example, in some embodiments a¹ and a² are independently an integer from 4 to 9.

In various embodiments of Formula (IV), b¹ and b² are 0. In different embodiments, b¹ and b² are 1.

In more embodiments of Formula (IV), c¹, c², d¹ and d² are independently an integer from 6 to 8.

In other embodiments of Formula (IV), c¹ and c² are, at each occurrence, independently an integer from 6 to 10, and d¹ and d² are, at each occurrence, independently an integer from 6 to 10.

In other embodiments of Formula (IV), c¹ and c² are, at each occurrence, independently an integer from 5 to 9, and d¹ and d² are, at each occurrence, independently an integer from 5 to 9.

In more embodiments of Formula (IV), Z is alkyl, cycloalkyl or a monovalent moiety comprising at least one polar functional group when n is 1. In other embodiments, Z is alkyl.

In various embodiments of the foregoing Formula (IV), R is, at each occurrence, independently either: (a) H or methyl; or (b) R together with the carbon atom to which it is bound is taken together with an adjacent R and the carbon atom to which it is bound to form a carbon-carbon double bond. In certain embodiments, each R is H. In other embodiments at least one R together with the carbon atom to which it is bound is taken together with an adjacent R and the carbon atom to which it is bound to form a carbon-carbon double bond.

In other embodiments of the compound of Formula (IV), R¹ and R² independently have one of the following structures:

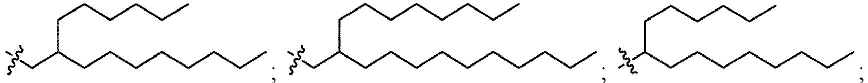

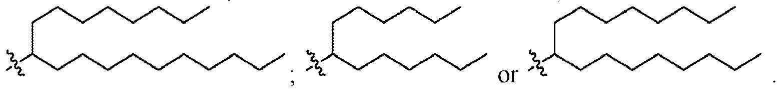

In certain embodiments of Formula (IV), the compound has one of the following structures: 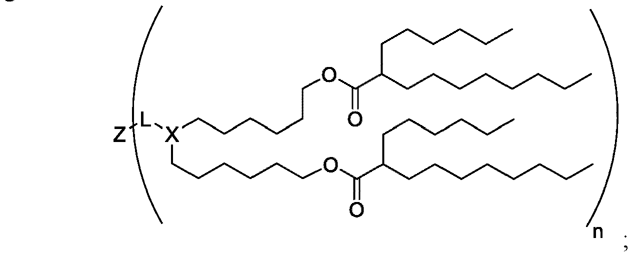 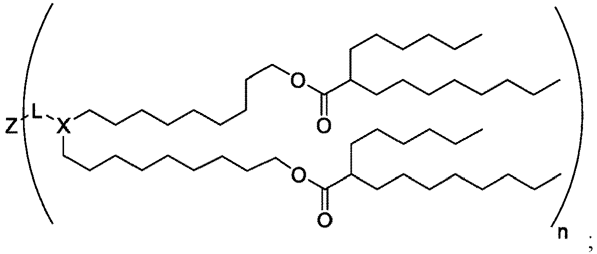 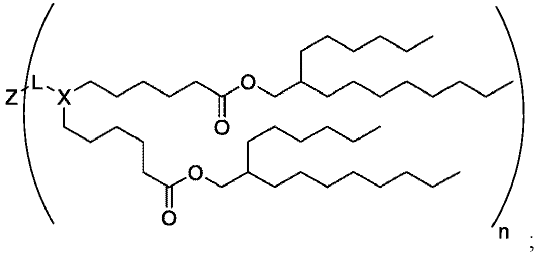 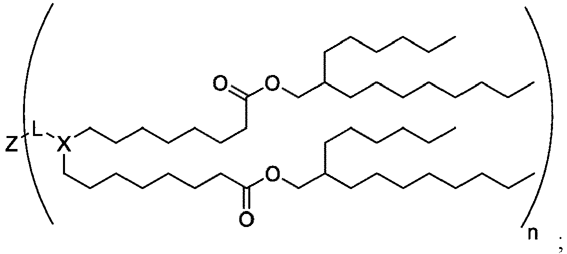 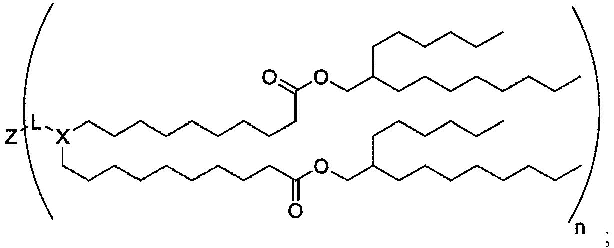 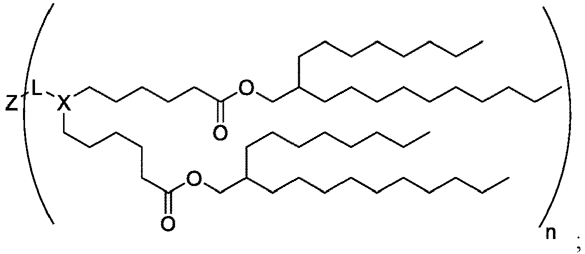 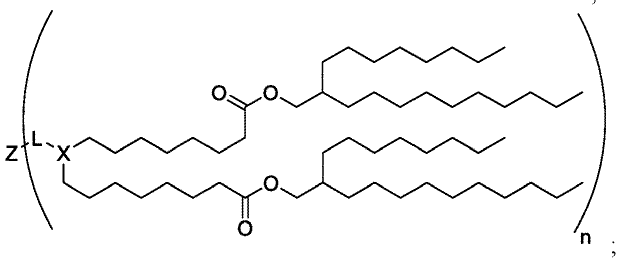 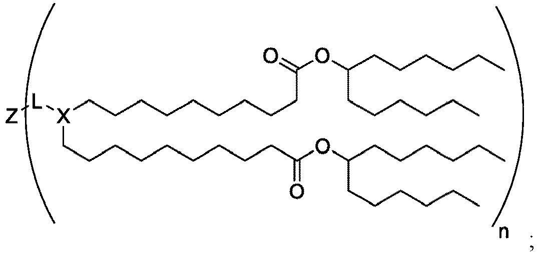 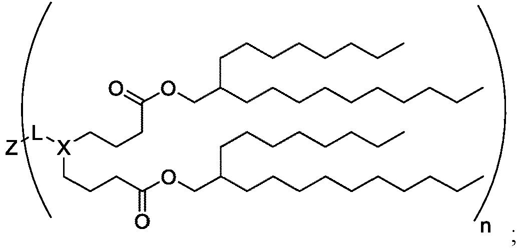 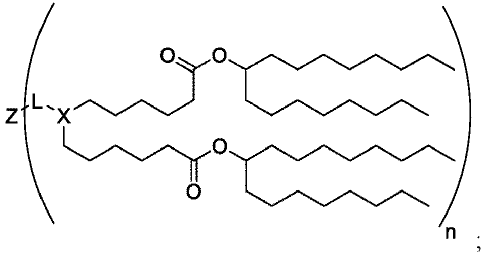 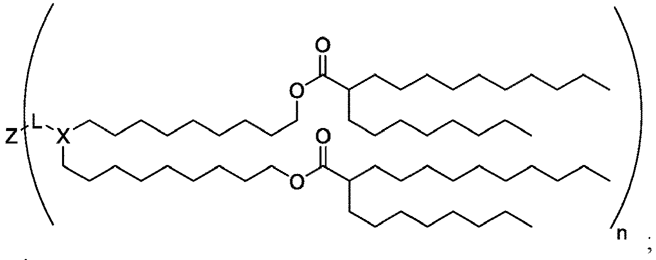 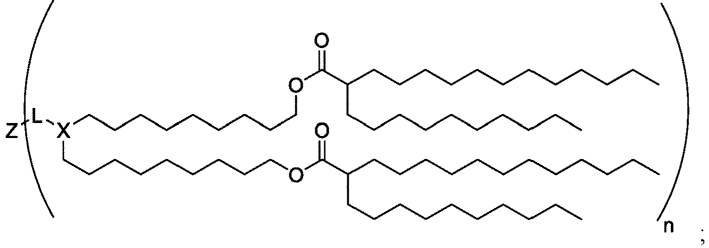 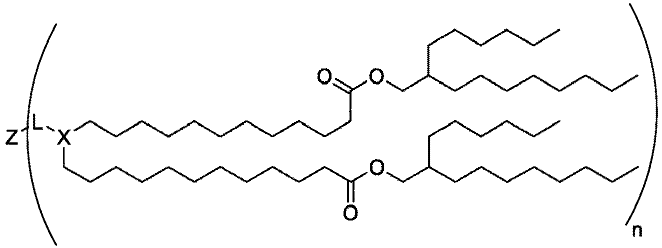 or

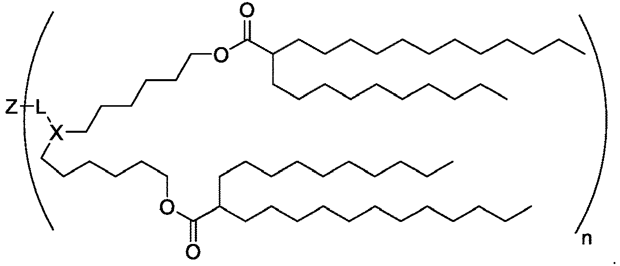

In still different embodiments the cationic lipid has the structure of Formula (V): 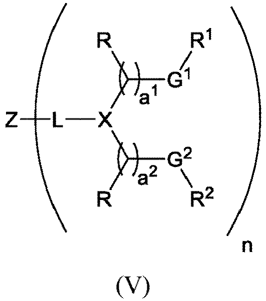 or a pharmaceutically acceptable salt or stereoisomer thereof, wherein:
one of G¹ or G² is, at each occurrence, -O(C=O)-, -(C=O)O-, -C(=O)-, -O-, -S(O)_{y}-, -S-S-, -C(=O)S-, SC(=O)-, -N(R^{a})C(=O)-, -C(=O)N(R^{a})-, -N(R^{a})C(=O)N(R^{a})-, -OC(=O)N(R^{a})- or -N(R^{a})C(=O)O-, and the other of G¹ or G² is, at each occurrence, -O(C=O)-, -(C=O)O-, -C(=O)-, -O-, -S(O)_{y}-, -S-S-, -C(=O)S-, -SC(=O)-, -N(R^{a})C(=O)-, -C(=O)N(R^{a})-, -N(R^{a})C(=O)N(R^{a})-, -OC(=O)N(R^{a})- or -N(R^{a})C(=O)O- or a direct bond;
L is, at each occurrence, ~O(C=O)-, wherein ~ represents a covalent bond to X;
X is CR^{a};
Z is alkyl, cycloalkyl or a monovalent moiety comprising at least one polar functional group when n is 1; or Z is alkylene, cycloalkylene or a polyvalent moiety comprising at least one polar functional group when n is greater than 1;
R^{a} is, at each occurrence, independently H, C₁-C₁₂ alkyl, C₁-C₁₂ hydroxylalkyl, C₁-C₁₂ aminoalkyl, C₁-C₁₂ alkylaminylalkyl, C₁-C₁₂ alkoxyalkyl, C₁-C₁₂ alkoxycarbonyl, C₁-C₁₂ alkylcarbonyloxy, C₁-C₁₂ alkylcarbonyloxyalkyl or C₁-C₁₂ alkylcarbonyl;
R is, at each occurrence, independently either: (a) H or C₁-C₁₂ alkyl; or (b) R together with the carbon atom to which it is bound is taken together with an adjacent R and the carbon atom to which it is bound to form a carbon-carbon double bond;
R¹ and R² have, at each occurrence, the following structure, respectively: 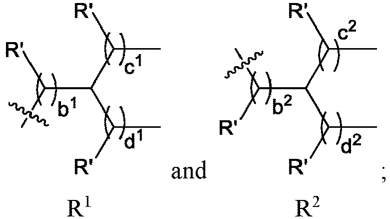
R' is, at each occurrence, independently H or C₁-C₁₂ alkyl;
a¹ and a² are, at each occurrence, independently an integer from 3 to 12;
b¹ and b² are, at each occurrence, independently 0 or 1;
c¹ and c² are, at each occurrence, independently an integer from 2 to 12;
d¹ and d² are, at each occurrence, independently an integer from 2 to 12;
y is, at each occurrence, independently an integer from 0 to 2; and
n is an integer from 1 to 6,
wherein a¹, a², c¹, c², d¹ and d² are selected such that the sum of a¹+c¹+d¹ is an integer from 18 to 30, and the sum of a²+c²+d² is an integer from 18 to 30, and wherein each alkyl, alkylene, hydroxylalkyl, aminoalkyl, alkylaminylalkyl, alkoxyalkyl, alkoxycarbonyl, alkylcarbonyloxy, alkylcarbonyloxyalkyl and alkylcarbonyl is optionally substituted with one or more substituent.

In certain embodiments of Formula (V), G¹ and G² are each independently -O(C=O)- or -(C=O)O-.

In other embodiments of Formula (V), X is CH.

In some embodiments of Formula (V), the sum of a¹+c¹+d¹ is an integer from 20 to 30, and the sum of a²+c²+d² is an integer from 18 to 30. In other embodiments, the sum of a¹+c¹+d¹ is an integer from 20 to 30, and the sum of a²+c²+d² is an integer from 20 to 30. In more embodiments of Formula (V), the sum of a¹ + b¹ + c¹ or the sum of a² + b² + c² is an integer from 12 to 26. In other embodiments, a¹, a², c¹, c², d¹ and d² are selected such that the sum of a¹+c¹+d¹ is an integer from 18 to 28, and the sum of a²+c²+d² is an integer from 18 to 28,
In still other embodiments of Formula (V), a¹ and a² are independently an integer from 3 to 10, for example an integer from 4 to 9.

In yet other embodiments of Formula (V), b¹ and b² are 0. In different embodiments b¹ and b² are 1.

In certain other embodiments of Formula (V), c¹, c², d¹ and d² are independently an integer from 6 to 8.

In different other embodiments of Formula (V), Z is alkyl or a monovalent moiety comprising at least one polar functional group when n is 1; or Z is alkylene or a polyvalent moiety comprising at least one polar functional group when n is greater than 1.

In more embodiments of Formula (V), Z is alkyl, cycloalkyl or a monovalent moiety comprising at least one polar functional group when n is 1. In other embodiments, Z is alkyl.

In other different embodiments of Formula (V), R is, at each occurrence, independently either: (a) H or methyl; or (b) R together with the carbon atom to which it is bound is taken together with an adjacent R and the carbon atom to which it is bound to form a carbon-carbon double bond. For example in some embodiments each R is H. In other embodiments at least one R together with the carbon atom to which it is bound is taken together with an adjacent R and the carbon atom to which it is bound to form a carbon-carbon double bond.

In more embodiments, each R' is H.

In certain embodiments of Formula (V), the sum of a¹+c¹+d¹ is an integer from 20 to 25, and the sum of a²+c²+d² is an integer from 20 to 25.

In other embodiments of Formula (V), R¹ and R² independently have one of the following structures: 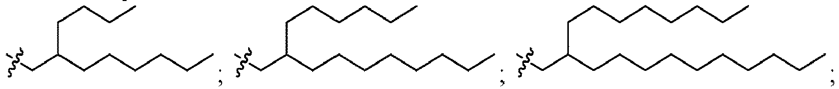 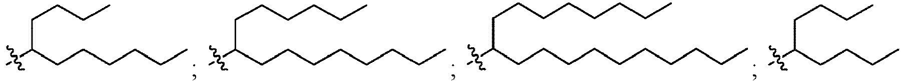 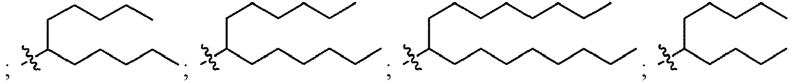 or

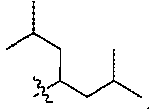

In more embodiments of Formula (V), the compound has one of the following structures: 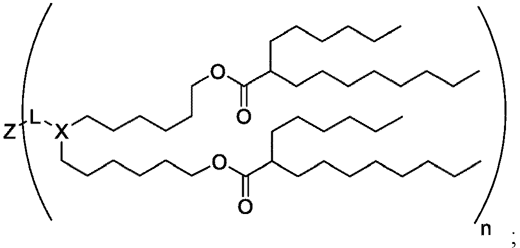 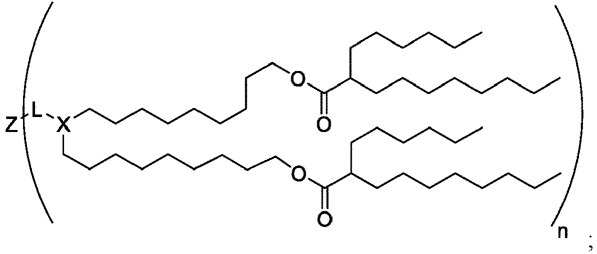 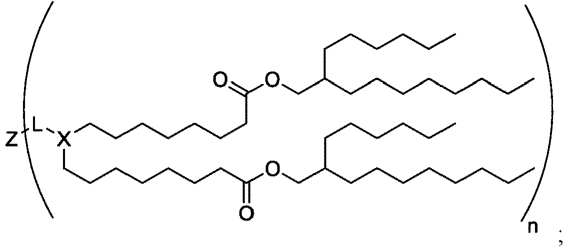 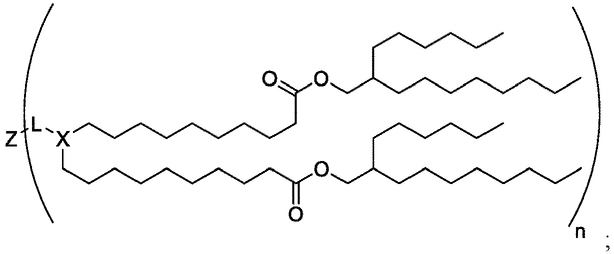 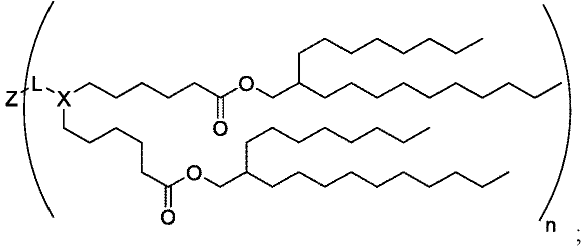 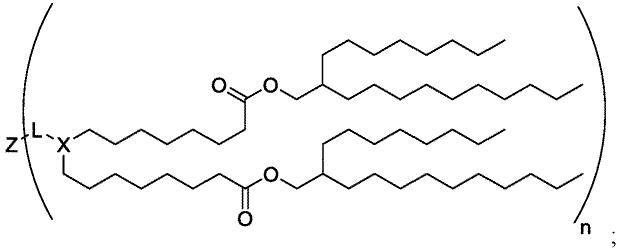 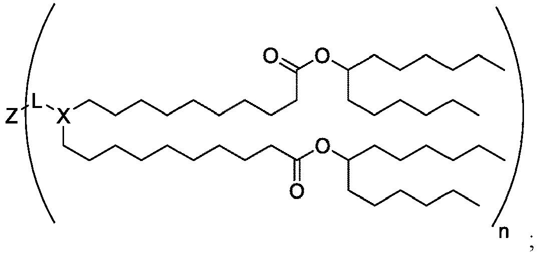 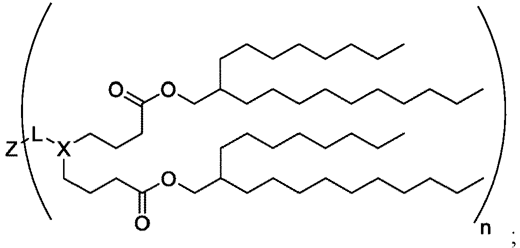 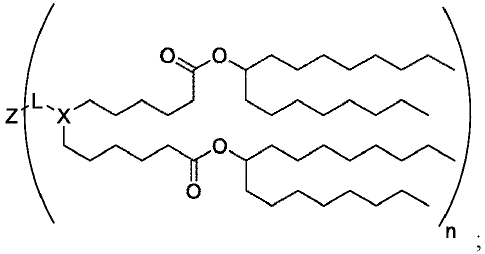 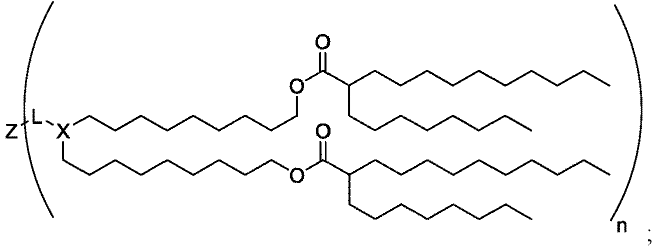 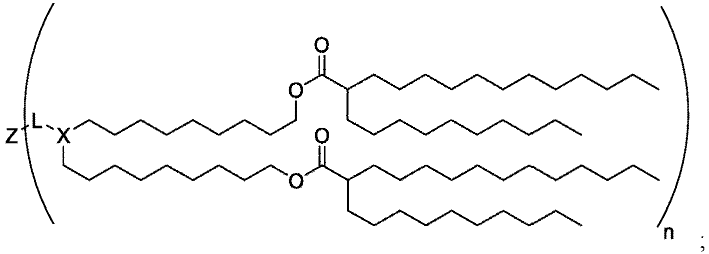 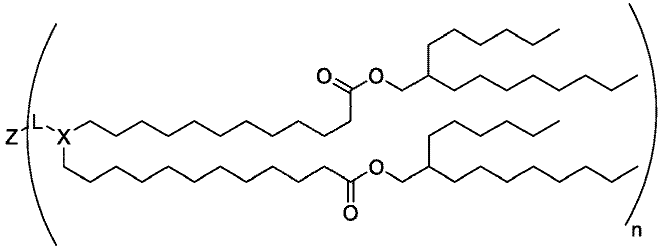 or

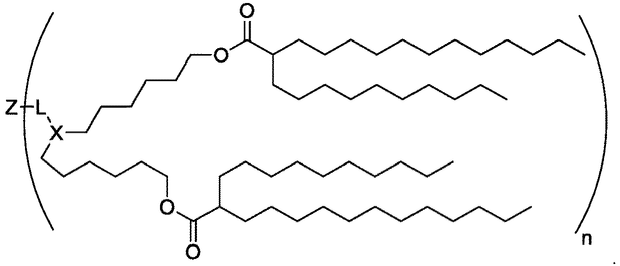

In any of the foregoing embodiments of Formula (IV) or (V), n is 1. In other of the foregoing embodiments of Formula (IV) or (V), n is greater than 1.

In more of any of the foregoing embodiments of Formula (IV) or (V), Z is a mono- or polyvalent moiety comprising at least one polar functional group. In some embodiments, Z is a monovalent moiety comprising at least one polar functional group. In other embodiments, Z is a polyvalent moiety comprising at least one polar functional group.

In more of any of the foregoing embodiments of Formula (IV) or (V), the polar functional group is a hydroxyl, alkoxy, ester, cyano, amide, amino, alkylaminyl, heterocyclyl or heteroaryl functional group.

In any of the foregoing embodiments of Formula (IV) or (V), Z is hydroxyl, hydroxylalkyl, alkoxyalkyl, amino, aminoalkyl, alkylaminyl, alkylaminylalkyl, heterocyclyl or heterocyclylalkyl.

In some other embodiments of Formula (IV) or (V), Z has the following structure: 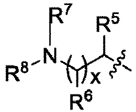 wherein:
R⁵ and R⁶ are independently H or C₁-C₆ alkyl;
R⁷ and R⁸ are independently H or C₁-C₆ alkyl or R⁷ and R⁸, together with the nitrogen atom to which they are attached, join to form a 3-7 membered heterocyclic ring; and
x is an integer from 0 to 6.

In still different embodiments of Formula (IV) or (V), Z has the following structure: 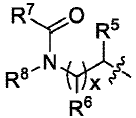 wherein:
R⁵ and R⁶ are independently H or C₁-C₆ alkyl;
R⁷ and R⁸ are independently H or C₁-C₆ alkyl or R⁷ and R⁸, together with the nitrogen atom to which they are attached, join to form a 3-7 membered heterocyclic ring; and
x is an integer from 0 to 6.

In still different embodiments of formula (IV) or (V), Z has the following structure: 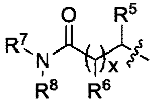 wherein:
R⁵ and R⁶ are independently H or C₁-C₆ alkyl;
R⁷ and R⁸ are independently H or C₁-C₆ alkyl or R⁷ and R⁸, together with the nitrogen atom to which they are attached, join to form a 3-7 membered heterocyclic ring; and
x is an integer from 0 to 6.

In some other embodiments of Formula (IV) or (V), Z is hydroxylalkyl, cyanoalkyl or an alkyl substituted with one or more ester or amide groups.

For example, in any of the foregoing embodiments of Formula (IV) or (V), Z has one of the following structures:

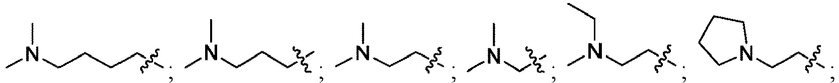

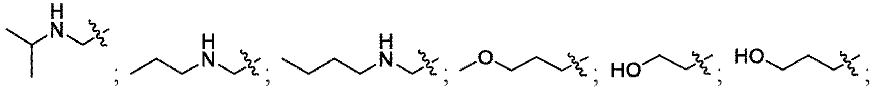

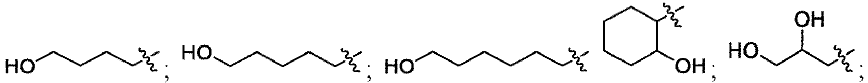

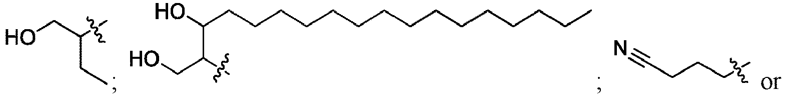

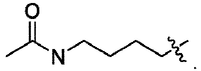

In other embodiments of Formula (IV) or (V), Z-L has one of the following structures:

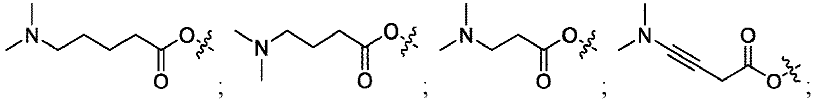

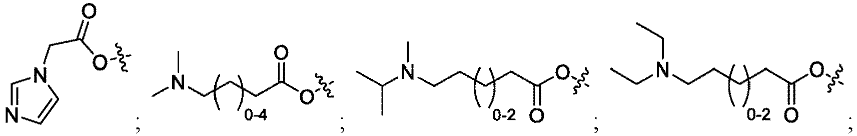

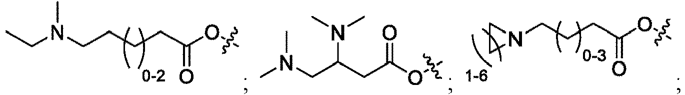

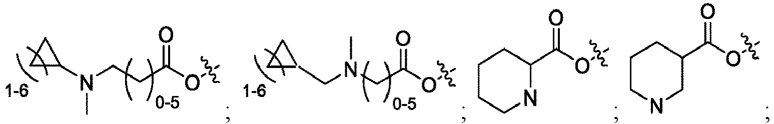

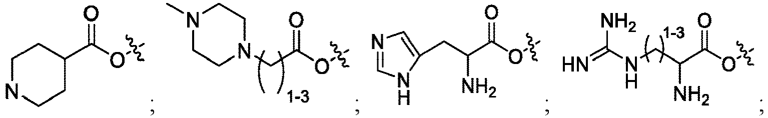

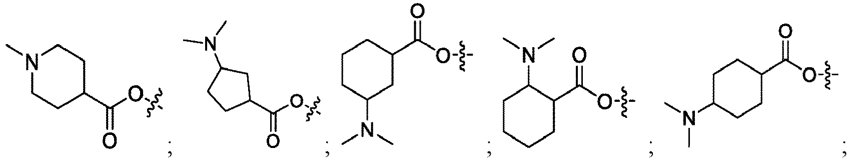

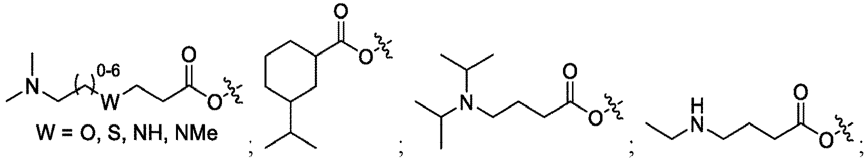

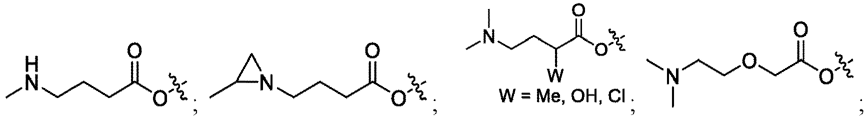

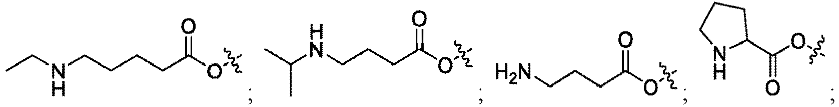

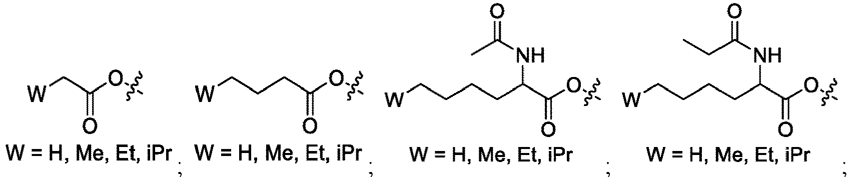

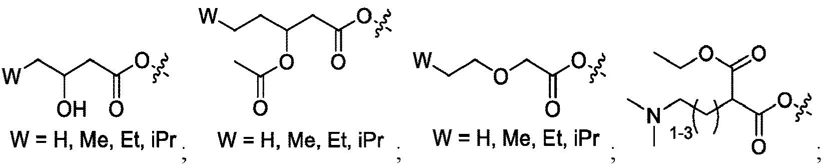

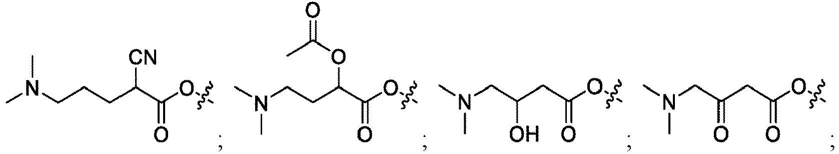

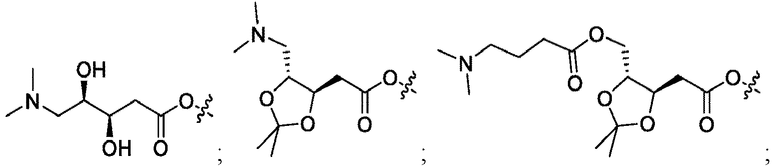

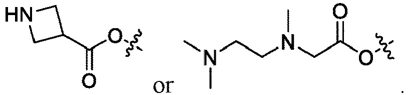

In other embodiments, Z-L has one of the following structures:

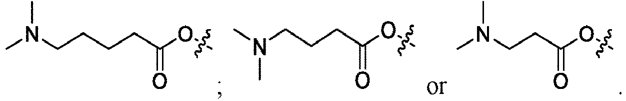

In still other embodiments, X is CH and Z-L has one of the following structures:

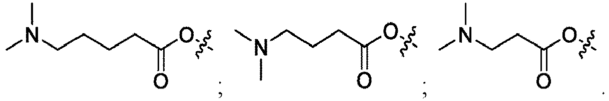

In various different embodiments, a cationic lipid has one of the structures set forth in Table 4 below.

**Table 4: Representative Compounds of Formula (IV) or (V)**

| **No.** | **Structure** |
|---|---|
| IV-1 | 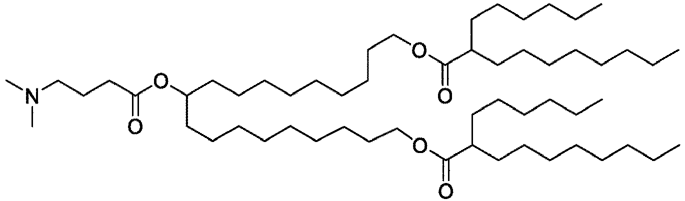 |
| IV-2 | 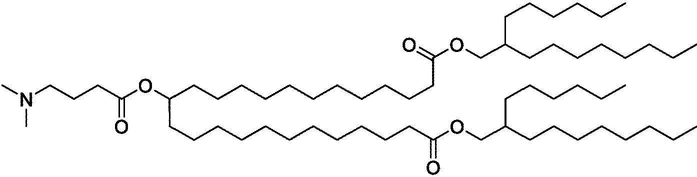 |
| IV-3 | 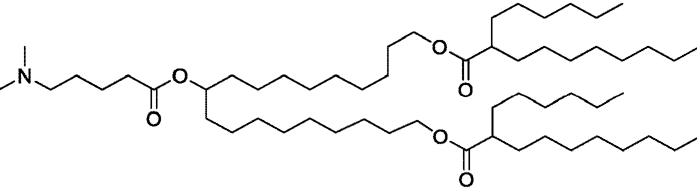 |

In one embodiment, the cationic lipid has the following Formula (VI): 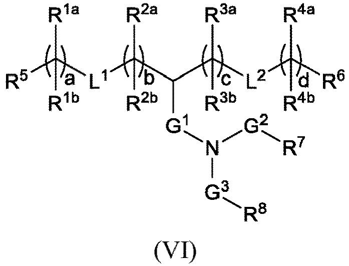 or a pharmaceutically acceptable salt, tautomer or stereoisomer thereof, wherein:
L¹ and L² are each independently -O(C=O)-, -(C=O)O-, -C(=O)-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, -NR^{a}C(=O)O- or a direct bond;
G¹ is C₁-C₂ alkylene, -(C=O)-, -O(C=O)-, -SC(=O)-, -NR^{a}C(=O)- or a direct bond;
G² is -C(=O)-, -(C=O)O-, -C(=O)S-, -C(=O)NR^{a}- or a direct bond;
G³ is C₁-C₆ alkylene;
R^{a} is H or C₁-C₁₂ alkyl;
R^{1a} and R^{1b} are, at each occurrence, independently either: (a) H or C₁-C₁₂ alkyl; or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
R^{2a} and R^{2b} are, at each occurrence, independently either: (a) H or C₁-C₁₂ alkyl; or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
R^{3a} and R^{3b} are, at each occurrence, independently either (a): H or C₁-C₁₂ alkyl; or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
R^{4a} and R^{4b} are, at each occurrence, independently either: (a) H or C₁-C₁₂ alkyl; or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
R⁵ and R⁶ are each independently H or methyl;
R⁷ is H or C₁-C₂₀ alkyl;
R⁸ is OH, -N(R⁹)(C=O)R¹⁰, -(C=O)NR⁹R¹⁰, -NR⁹R¹⁰, -(C=O)OR¹¹ or -O(C=O)R¹¹, provided that G³ is C₄-C₆ alkylene when R⁸ is -NR⁹R¹⁰,
R⁹ and R¹⁰ are each independently H or C₁-C₁₂ alkyl;
R¹¹ is aralkyl;
a, b, c and d are each independently an integer from 1 to 24; and
x is 0, 1 or 2,
wherein each alkyl, alkylene and aralkyl is optionally substituted.

In some embodiments, L¹ and L² are each independently -O(C=O)-, -(C=O)O- or a direct bond. In other embodiments, G¹ and G² are each independently -(C=O)- or a direct bond. In some different embodiments, L¹ and L² are each independently -O(C=O)-, -(C=O)O- or a direct bond; and G¹ and G² are each independently - (C=O)- or a direct bond.

In some different embodiments, L¹ and L² are each independently -C(=O)-, -O-, -S(O)ₓ-, -S-S-, -C(=O)S-, -SC(=O)-, -NR^{a}-, -NR^{a}C(=O)-, -C(=O)NR^{a}-, -NR^{a}C(=O)NR^{a}, -OC(=O)NR^{a}-, -NR^{a}C(=O)O-, -NR^{a}S(O)ₓNR^{a}-, -NR^{a}S(O)ₓ- or -S(O)ₓNR^{a}-.

In other of the foregoing embodiments, the compound has one of the following Formulas (VIA) or (VIB):

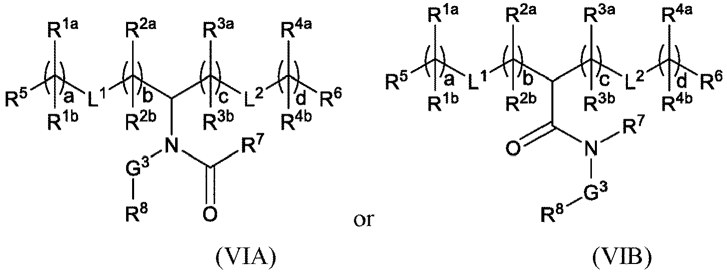

In some embodiments, the compound has Formula (VIA). In other embodiments, the compound has Formula (VIB).

In any of the foregoing embodiments, one of L¹ or L² is -O(C=O)-. For example, in some embodiments each of L¹ and L² are -O(C=O)-.

In some different embodiments of any of the foregoing, one of L¹ or L² is -(C=O)O-. For example, in some embodiments each of L¹ and L² is -(C=O)O-.

In different embodiments, one of L¹ or L² is a direct bond. As used herein, a "direct bond" means the group (e.g., L¹ or L²) is absent. For example, in some embodiments each of L¹ and L² is a direct bond.

In other different embodiments of the foregoing, for at least one occurrence of R^{1a} and R^{1b}, R^{1a} is H or C₁-C₁₂ alkyl, and R^{1b} together with the carbon atom to which it is bound is taken together with an adjacent R^{1b} and the carbon atom to which it is bound to form a carbon-carbon double bond.

In still other different embodiments, for at least one occurrence of R^{4a} and R^{4b}, R^{4a} is H or C₁-C₁₂ alkyl, and R^{4b} together with the carbon atom to which it is bound is taken together with an adjacent R^{4b} and the carbon atom to which it is bound to form a carbon-carbon double bond.

In more embodiments, for at least one occurrence of R^{2a} and R^{2b}, R^{2a} is H or C₁-C₁₂ alkyl, and R^{2b} together with the carbon atom to which it is bound is taken together with an adjacent R^{2b} and the carbon atom to which it is bound to form a carbon-carbon double bond.

In other different embodiments of any of the foregoing, for at least one occurrence of R^{3a} and R^{3b}, R^{3a} is H or C₁-C₁₂ alkyl, and R^{3b} together with the carbon atom to which it is bound is taken together with an adjacent R^{3b} and the carbon atom to which it is bound to form a carbon-carbon double bond.

It is understood that "carbon-carbon" double bond refers to one of the following structures: 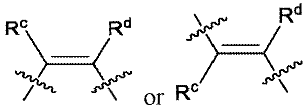 wherein R^{c} and R^{d} are, at each occurrence, independently H or a substituent. For example, in some embodiments R^{c} and R^{d} are, at each occurrence, independently H, C₁-C₁₂ alkyl or cycloalkyl, for example H or C₁-C₁₂ alkyl.

In various other embodiments, the compound has one of the following Formulas (VIC) or (VID): 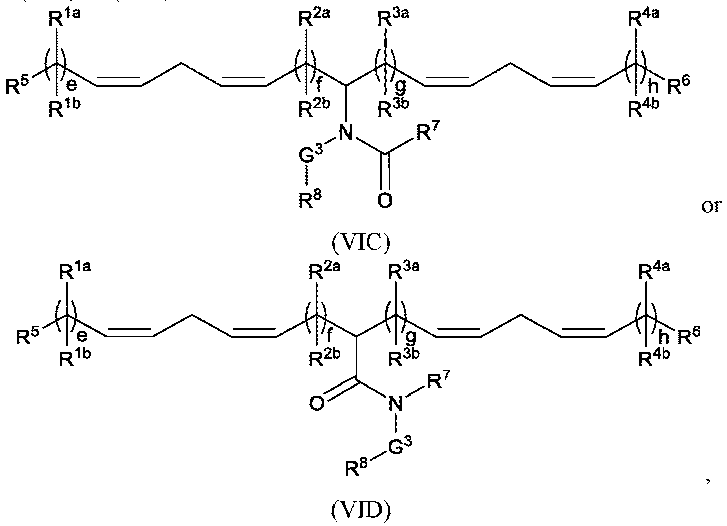 wherein e, f, g and h are each independently an integer from 1 to 12.

In some embodiments, the compound has Formula (VIC). In other embodiments, the compound has Formula (VID).

In various embodiments of the compounds of Formulas (VIC) or (VID), e, f, g and h are each independently an integer from 4 to 10.

In other different embodiments, 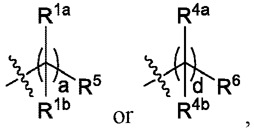 or both, independently has one of the following structures: 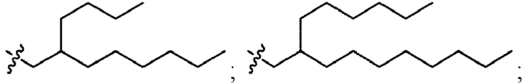 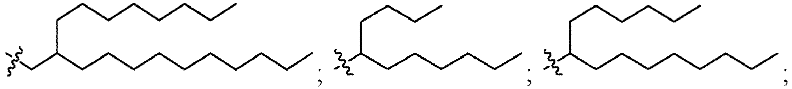 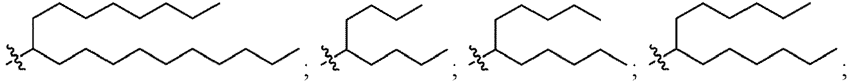 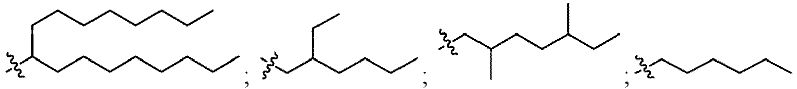 or

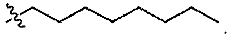

In certain embodiments of the foregoing, a, b, c and d are each independently an integer from 2 to 12 or an integer from 4 to 12. In other embodiments, a, b, c and d are each independently an integer from 8 to 12 or 5 to 9. In some certain embodiments, a is 0. In some embodiments, a is 1. In other embodiments, a is 2. In more embodiments, a is 3. In yet other embodiments, a is 4. In some embodiments, a is 5. In other embodiments, a is 6. In more embodiments, a is 7. In yet other embodiments, a is 8. In some embodiments, a is 9. In other embodiments, a is 10. In more embodiments, a is 11. In yet other embodiments, a is 12. In some embodiments, a is 13. In other embodiments, a is 14. In more embodiments, a is 15. In yet other embodiments, a is 16.

In some embodiments, b is 1. In other embodiments, b is 2. In more embodiments, b is 3. In yet other embodiments, b is 4. In some embodiments, b is 5. In other embodiments, b is 6. In more embodiments, b is 7. In yet other embodiments, b is 8. In some embodiments, b is 9. In other embodiments, b is 10. In more embodiments, b is 11. In yet other embodiments, b is 12. In some embodiments, b is 13. In other embodiments, b is 14. In more embodiments, b is 15. In yet other embodiments, b is 16.

In some embodiments, c is 1. In other embodiments, c is 2. In more embodiments, c is 3. In yet other embodiments, c is 4. In some embodiments, c is 5. In other embodiments, c is 6. In more embodiments, c is 7. In yet other embodiments, c is 8. In some embodiments, c is 9. In other embodiments, c is 10. In more embodiments, c is 11. In yet other embodiments, c is 12. In some embodiments, c is 13. In other embodiments, c is 14. In more embodiments, c is 15. In yet other embodiments, c is 16.

In some certain embodiments, d is 0. In some embodiments, d is 1. In other embodiments, d is 2. In more embodiments, d is 3. In yet other embodiments, d is 4. In some embodiments, d is 5. In other embodiments, d is 6. In more embodiments, d is 7. In yet other embodiments, d is 8. In some embodiments, d is 9. In other embodiments, d is 10. In more embodiments, d is 11. In yet other embodiments, d is 12. In some embodiments, d is 13. In other embodiments, d is 14. In more embodiments, d is 15. In yet other embodiments, d is 16.

In some embodiments, e is 1. In other embodiments, e is 2. In more embodiments, e is 3. In yet other embodiments, e is 4. In some embodiments, e is 5. In other embodiments, e is 6. In more embodiments, e is 7. In yet other embodiments, e is 8. In some embodiments, e is 9. In other embodiments, e is 10. In more embodiments, e is 11. In yet other embodiments, e is 12.

In some embodiments, f is 1. In other embodiments, f is 2. In more embodiments, f is 3. In yet other embodiments, f is 4. In some embodiments, f is 5. In other embodiments, f is 6. In more embodiments, f is 7. In yet other embodiments, f is 8. In some embodiments, f is 9. In other embodiments, f is 10. In more embodiments, f is 11. In yet other embodiments, f is 12.

In some embodiments, g is 1. In other embodiments, g is 2. In more embodiments, g is 3. In yet other embodiments, g is 4. In some embodiments, g is 5. In other embodiments, g is 6. In more embodiments, g is 7. In yet other embodiments, g is 8. In some embodiments, g is 9. In other embodiments, g is 10. In more embodiments, g is 11. In yet other embodiments, g is 12.

In some embodiments, h is 1. In other embodiments, e is 2. In more embodiments, h is 3. In yet other embodiments, h is 4. In some embodiments, e is 5. In other embodiments, h is 6. In more embodiments, h is 7. In yet other embodiments, h is 8. In some embodiments, h is 9. In other embodiments, h is 10. In more embodiments, h is 11. In yet other embodiments, h is 12.

In some other various embodiments, a and d are the same. In some other embodiments, b and c are the same. In some other specific embodiments a and d are the same and b and c are the same.

The sum of a and b and the sum of c and d are factors which may be varied to obtain a lipid having the desired properties. In one embodiment, a and b are chosen such that their sum is an integer ranging from 14 to 24. In other embodiments, c and d are chosen such that their sum is an integer ranging from 14 to 24. In further embodiment, the sum of a and b and the sum of c and d are the same. For example, in some embodiments the sum of a and b and the sum of c and d are both the same integer which may range from 14 to 24. In still more embodiments, a. b, c and d are selected such that the sum of a and b and the sum of c and d is 12 or greater.

The substituents at R^{1a}, R^{2a}, R^{3a} and R^{4a} are not particularly limited. In some embodiments, at least one of R^{1a}, R^{2a}, R^{3a} and R^{4a} is H. In certain embodiments R^{1a}, R^{2a}, R^{3a} and R^{4a} are H at each occurrence In certain other embodiments at least one of R^{1a}, R^{2a}, R^{3a} and R^{4a} is C₁-C₁₂ alkyl. In certain other embodiments at least one of R^{1a}, R^{2a}, R^{3a} and R^{4a} is Ci-Cs alkyl. In certain other embodiments at least one of R^{1a}, R^{2a}, R^{3a} and R^{4a} is C₁-C₆ alkyl. In some of the foregoing embodiments, the C₁-C₈ alkyl is methyl, ethyl, n-propyl, iso-propyl, n-butyl, iso-butyl, tert-butyl, n-hexyl or n-octyl.

In certain embodiments of the foregoing, R^{1a}, R^{1b}, R^{4a} and R^{4b} are C₁-C₁₂ alkyl at each occurrence.

In further embodiments of the foregoing, at least one of R^{1b}, R^{2b}, R^{3b} and R^{4b} is H or R^{1b}, R^{2b}, R^{3b} and R^{4b} are H at each occurrence.

In certain embodiments of the foregoing, R^{1b} together with the carbon atom to which it is bound is taken together with an adjacent R^{1b} and the carbon atom to which it is bound to form a carbon-carbon double bond. In other embodiments of the foregoing R^{4b} together with the carbon atom to which it is bound is taken together with an adjacent R^{4b} and the carbon atom to which it is bound to form a carbon-carbon double bond.

The substituents at R⁵ and R⁶ are not particularly limited in the foregoing embodiments. In certain embodiments one of R⁵ or R⁶ is methyl. In other embodiments each of R⁵ or R⁶ is methyl.

The substituents at R⁷ are not particularly limited in the foregoing embodiments. In certain embodiments R⁷ is C₆-C₁₆ alkyl. In some other embodiments, R⁷ is C₆-C₉ alkyl. In some of these embodiments, R⁷ is substituted with -(C=O)OR^{b}, -O(C=O)R^{b}, -C(=O)R^{b}, -OR^{b}, -S(O)ₓR^{b}, -S-SR^{b}, -C(=O)SR^{b}, -SC(=O)R^{b}, -NR^{a}R^{b}, -NR^{a}C(=O)R^{b}, -C(=O)NR^{a}R^{b}, -NR^{a}C(=O)NR^{a}R^{b}, -OC(=O)NR^{a}R^{b}, -NR^{a}C(=O)OR^{b}, -NR^{a}S(O)ₓNR^{a}R^{b}, -NR^{a}S(O)ₓR^{b} or -S(O)ₓNR^{a}R^{b}, wherein: R^{a} is H or C₁-C₁₂ alkyl; R^{b} is C₁-C₁₅ alkyl; and x is 0, 1 or 2. For example, in some embodiments R⁷ is substituted with -(C=O)OR^{b} or -O(C=O)R^{b}.

In various of the foregoing embodiments, R^{b} is branched C₃-C₁₅ alkyl. For example, in some embodiments R^{b} has one of the following structures: 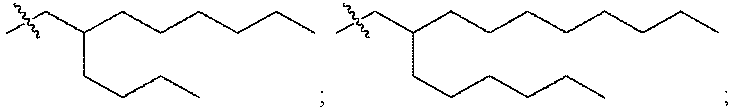 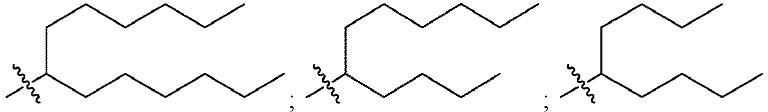 or

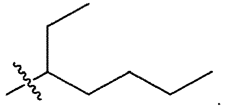

In certain embodiments, R⁸ is OH.

In other embodiments, R⁸ is -N(R⁹)(C=O)R¹⁰. In some other embodiments, R⁸ is -(C=O)NR⁹R¹⁰. In still more embodiments, R⁸ is -NR⁹R¹⁰. In some of the foregoing embodiments, R⁹ and R¹⁰ are each independently H or C₁-C₈ alkyl, for example H or C₁-C₃ alkyl. In more specific of these embodiments, the C₁-C₈ alkyl or C₁-C₃ alkyl is unsubstituted or substituted with hydroxyl. In other of these embodiments, R⁹ and R¹⁰ are each methyl.

In yet more embodiments, R⁸ is -(C=O)OR¹¹. In some of these embodiments R¹¹ is benzyl.

In yet more specific embodiments, R⁸ has one of the following structures: 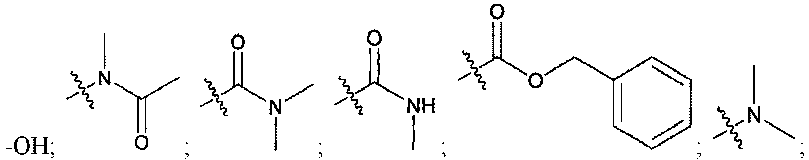 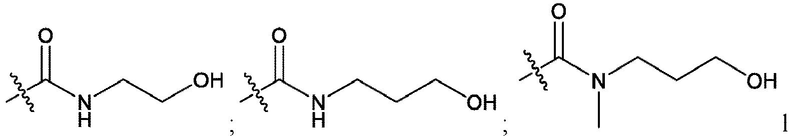 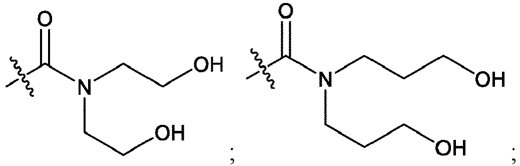 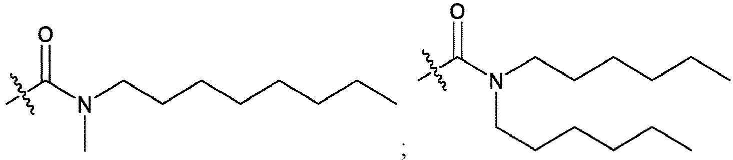 or

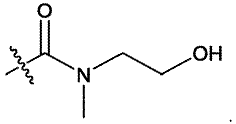

In still other embodiments of the foregoing compounds, G³ is C₂-C₅ alkylene, for example C₂-C₄ alkylene, C₃ alkylene or C₄ alkylene. In some of these embodiments, R⁸ is OH. In other embodiments, G² is absent and R⁷ is C₁-C₂ alkylene, such as methyl.

In various different embodiments, the compound has one of the structures set forth in Table 5 below.

**Table 5: Representative Compounds of Formula (VI)**

| **No.** | **Structure** |
|---|---|
| VI-1 | 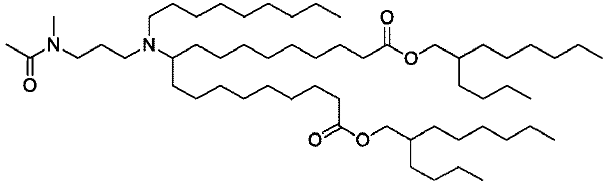 |
| VI-2 | 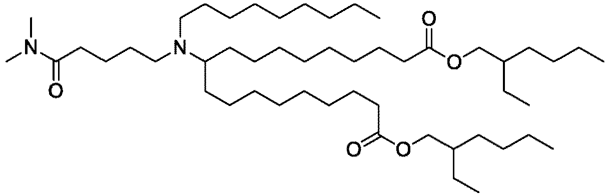 |
| VI-3 | 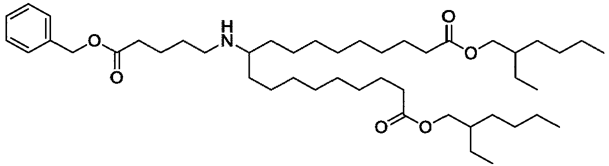 |
| VI-4 | 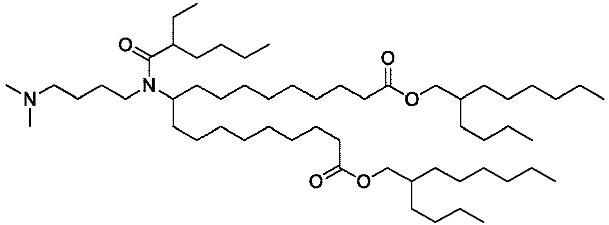 |
| VI-5 | 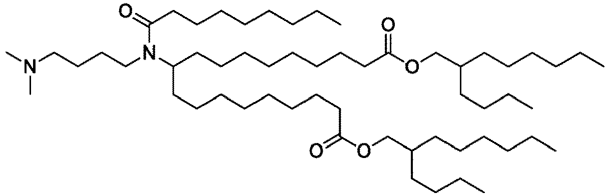 |
| VI-6 | 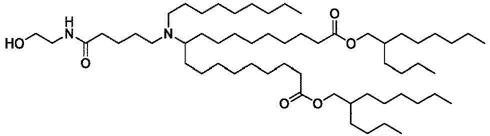 |
| VI-7 | 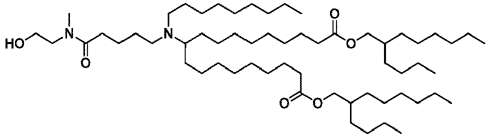 |
| VI-8 | 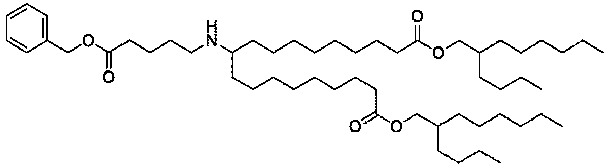 |
| VI-9 | 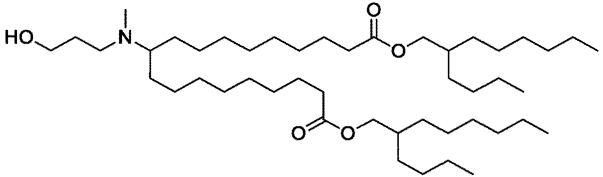 |
| VI-10 | 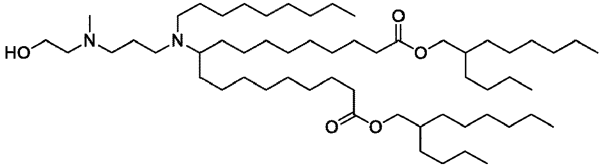 |
| VI-11 | 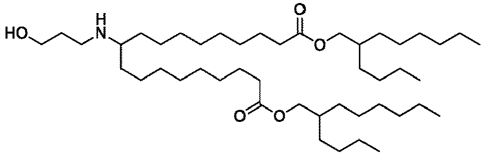 |
| VI-12 | 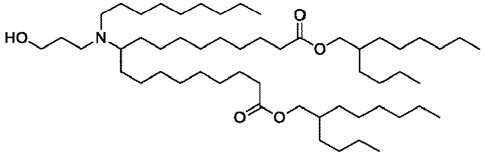 |
| VI-13 | 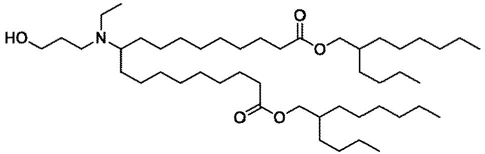 |
| VI-14 | 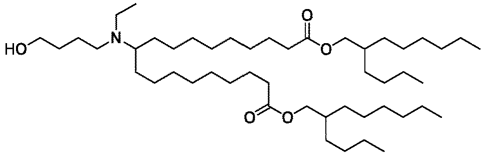 |
| VI-15 | 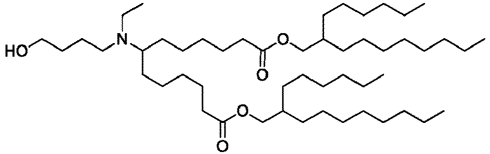 |
| VI-16 | 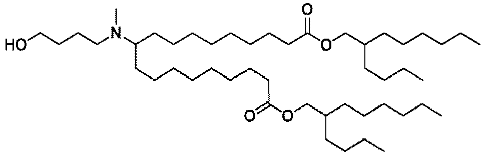 |
| VI-17 | 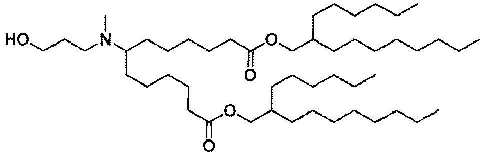 |
| VI-18 | 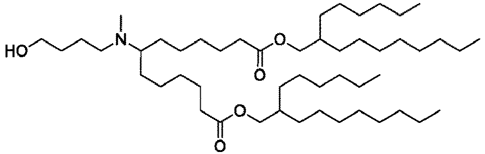 |
| VI-19 | 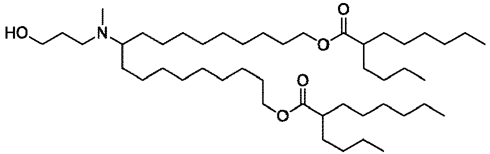 |
| VI-20 | 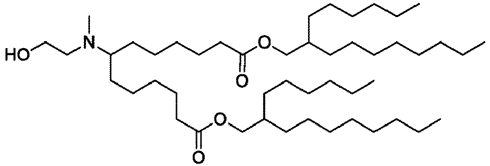 |
| VI-21 | 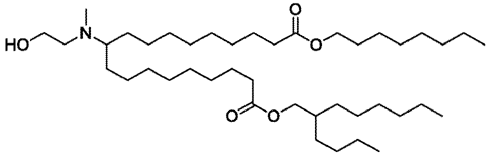 |
| VI-22 | 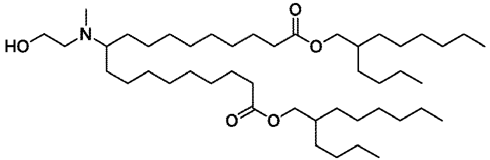 |
| VI-23 | 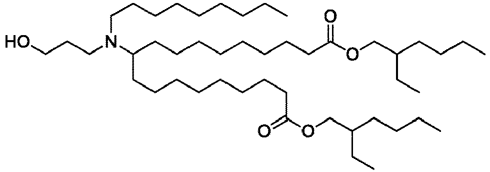 |
| VI-24 | 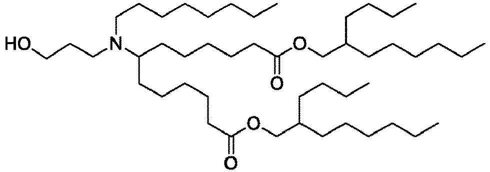 |
| VI-25 | 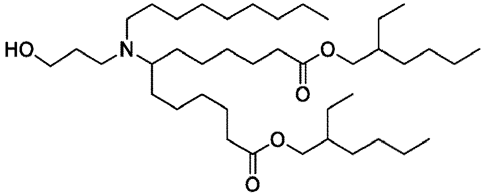 |
| VI-26 | 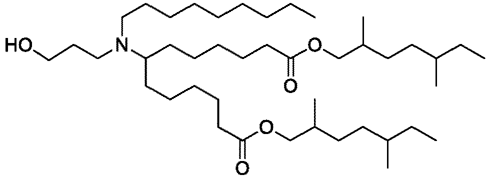 |
| VI-27 | 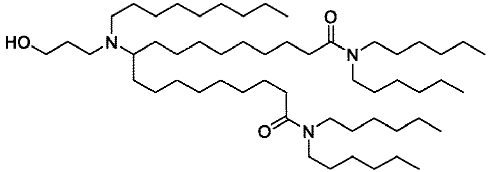 |
| VI-28 | 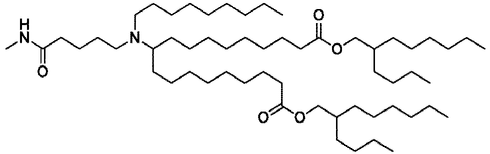 |
| VI-29 | 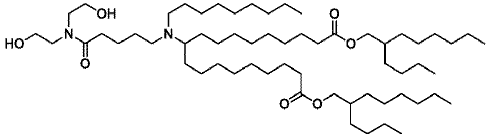 |
| VI-30 | 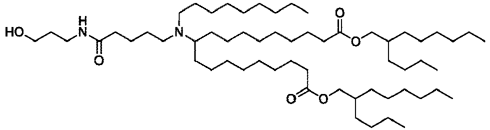 |
| VI-31 | 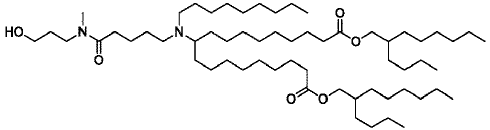 |
| VI-32 | 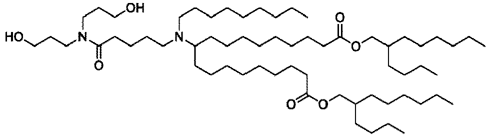 |
| VI-33 | 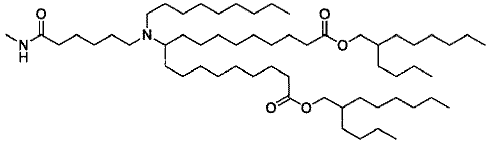 |
| VI-34 | 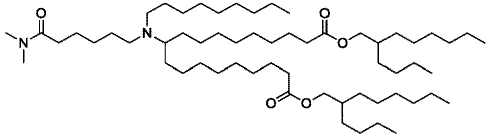 |
| VI-35 | 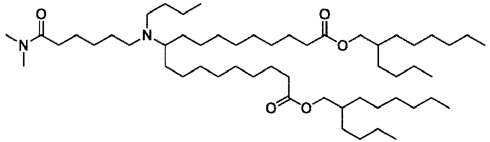 |
| VI-36 | 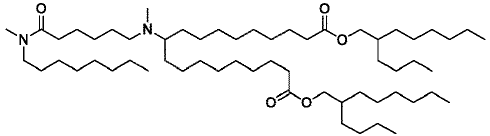 |
| VI-37 | 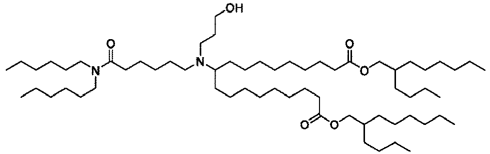 |

In one embodiment, the cationic lipid has the following Formula (VII): 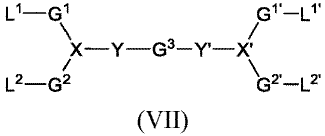 or a pharmaceutically acceptable salt or stereoisomer thereof, wherein:
X and X' are each independently N or CR;
Y and Y' are each independently absent, -O(C=O)-, -(C=O)O- or NR, provided that:
   a)Y is absent when X is N;
   b) Y' is absent when X' is N;
   c) Y is -O(C=O)-, -(C=O)O- or NR when X is CR; and
   d) Y' is -O(C=O)-, -(C=O)O- or NR when X' is CR,
L¹ and L^{1'} are each independently -O(C=O)R¹, -(C=O)OR¹, -C(=O)R¹, -OR¹, -S(O)_{z}R¹, -S-SR¹, -C(=O)SR¹, -SC(=O)R¹, -NR^{a}C(=O)R¹, -C(=O)NR^{b}R^{c}, -NR^{a}C(=O)NR^{b}R^{c}, -OC(=O)NR^{b}R^{c} or -NR^{a}C(=O)OR¹;
L² and L^{2'} are each independently -O(C=O)R², -(C=O)OR², -C(=O)R², -OR², -S(O)_{z}R², -S-SR², -C(=O)SR², -SC(=O)R², -NR^{d}C(=O)R², -C(=O)NR^{e}R^{f}, -NR^{d}C(=O)NR^{e}R^{f}, -OC(=O)NR^{e}R^{f};-NR^{d}C(=O)OR² or a direct bond to R²;
G¹, G^{1'}, G² and G^{2'} are each independently C₂-C₁₂ alkylene or C₂-C₁₂ alkenylene;
G³ is C₂-C₂₄ heteroalkylene or C₂-C₂₄ heteroalkenylene;
R^{a}, R^{b}, R^{d} and R^{e} are, at each occurrence, independently H, C₁-C₁₂ alkyl or C₂-C₁₂ alkenyl;
R^{c} and R^{f} are, at each occurrence, independently C₁-C₁₂ alkyl or C₂-C₁₂ alkenyl;
R is, at each occurrence, independently H or C₁-C₁₂ alkyl;
R¹ and R² are, at each occurrence, independently branched C₆-C₂₄ alkyl or branched C₆-C₂₄ alkenyl;
z is 0, 1 or 2, and
wherein each alkyl, alkenyl, alkylene, alkenylene, heteroalkylene and heteroalkenylene is independently substituted or unsubstituted unless otherwise specified.

In other different embodiments of Formula (VII):
X and X' are each independently N or CR;
Y and Y' are each independently absent or NR, provided that:
   a)Y is absent when X is N;
   b) Y' is absent when X' is N,
   c) Y is NR when X is CR; and
   d) Y' is NR when X' is CR,
L¹ and L^{1'} are each independently -O(C=O)R¹, -(C=O)OR¹, -C(=O)R¹, -OR¹, -S(O)_{z}R¹, -S-SR¹, -C(=O)SR¹, -SC(=O)R¹, -NR^{a}C(=O)R¹, -C(=O)NR^{b}R^{c}, -NR^{a}C(=O)NR^{b}R^{c}, -OC(=O)NR^{b}R^{c} or -NR^{a}C(=O)OR¹;
L² and L^{2'} are each independently -O(C=O)R², -(C=O)OR², -C(=O)R², -OR², -S(O)_{z}R², -S-SR², -C(=O)SR², -SC(=O)R², -NR^{d}C(=O)R², -C(=O)NR^{e}R^{f}, -NR^{d}C(=O)NR^{e}R^{f}, -OC(=O)NR^{e}R^{f};-NR^{d}C(=O)OR² or a direct bond to R²;
G¹, G^{1'}, G² and G^{2'} are each independently C₂-C₁₂ alkylene or C₂-C₁₂ alkenylene;
G³ is C₂-C₂₄ alkyleneoxide or C₂-C₂₄ alkenyleneoxide;
R^{a}, R^{b}, R^{d} and R^{e} are, at each occurrence, independently H, C₁-C₁₂ alkyl or C₂-C₁₂ alkenyl;
R^{c} and R^{f} are, at each occurrence, independently C₁-C₁₂ alkyl or C₂-C₁₂ alkenyl;
R is, at each occurrence, independently H or C₁-C₁₂ alkyl;
R¹ and R² are, at each occurrence, independently branched C₆-C₂₄ alkyl or branched C₆-C₂₄ alkenyl;
z is 0, 1 or 2, and
wherein each alkyl, alkenyl, alkylene, alkenylene, alkyleneoxide and alkenyleneoxide is independently substituted or unsubstituted unless otherwise specified.

In some embodiments, G³ is C₂-C₂₄ alkyleneoxide or C₂-C₂₄ alkenyleneoxide. In certain embodiments, G³ is unsubstituted. In other embodiments, G³ is substituted, for example substituted with hydroxyl. In more specific embodiments G³ is C₂-C₁₂ alkyleneoxide, for example, in some embodiments G³ is C₃-C₇ alkyleneoxide or in other embodiments G³ is C₃-C₁₂ alkyleneoxide.

In other embodiments, G³ is C₂-C₂₄ alkyleneaminyl or C₂-C₂₄ alkenyleneaminyl, for example C₆-C₁₂ alkyleneaminyl. In some of these embodiments, G³ is unsubstituted. In other of these embodiments, G³ is substituted with C₁-C₆ alkyl.

In some embodiments, X and X' are each N, and Y and Y' are each absent. In other embodiments, X and X' are each CR, and Y and Y' are each NR. In some of these embodiments, R is H.

In certain embodiments, X and X' are each CR, and Y and Y' are each independently -O(C=O)- or -(C=O)O-.

In some of the foregoing embodiments, the compound has one of the following Formulas (VIIA), (VIIB), (VIIC), (VIID), (VIIE), (VIIF), (VIIG) or (VIIH): 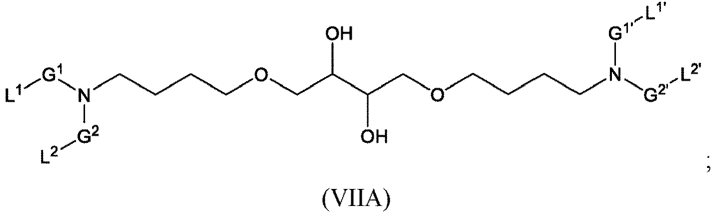 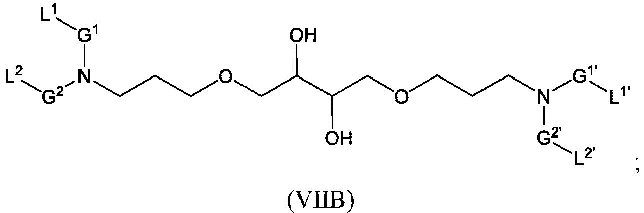 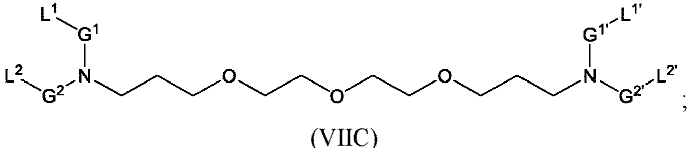 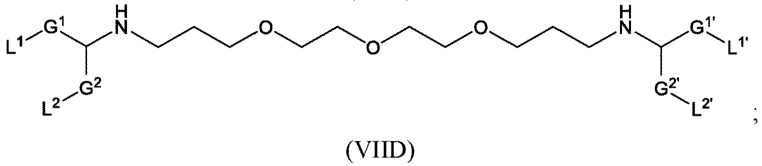 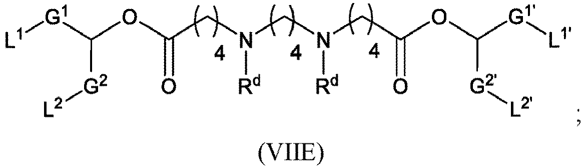 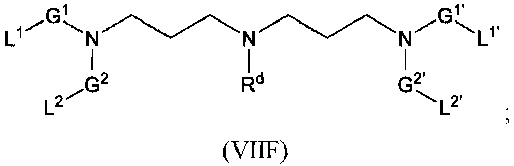 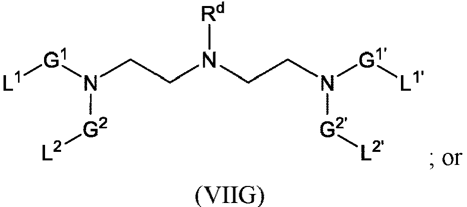 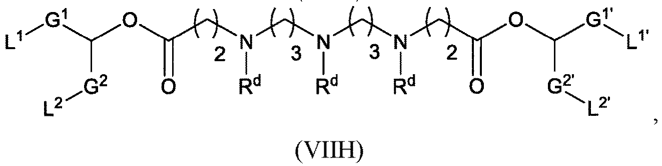 wherein R^{d} is, at each occurrence, independently H or optionally substituted C₁-C₆ alkyl. For example, in some embodiments R^{d} is H. In other embodiments, R^{d} is C₁-C₆ alkyl, such as methyl. In other embodiments, R^{d} is substituted C₁-C₆ alkyl, such as C₁-C₆ alkyl substituted with -O(C=O)R, -(C=O)OR, -NRC(=O)R or -C(=O)N(R)₂, wherein R is, at each occurrence, independently H or C₁-C₁₂ alkyl.

In some of the foregoing embodiments, L¹ and L^{1'} are each independently -O(C=O)R¹, -(C=O)OR¹ or -C(=O)NR^{b}R^{c}, and L² and L^{2'} are each independently - O(C=O)R², -(C=O)OR² or -C(=O)NR^{e}R^{f} For example, in some embodiments L¹ and L^{1'} are each -(C=O)OR¹, and L² and L^{2'} are each -(C=O)OR².. In other embodiments L¹ and L^{1'} are each -(C=O)OR¹, and L² and L^{2'} are each -C(=O)NR^{e}R^{f}. In other embodiments L¹ and L^{1'} are each -C(=O)NR^{b}R^{c}, and L² and L^{2'} are each -C(=O)NR^{e}R^{f}.

In some embodiments of the foregoing, G¹, G^{1'}, G² and G^{2'} are each independently C₂-C₈ alkylene, for example C₄-C₈ alkylene.

In some of the foregoing embodiments, R¹ or R², are each, at each occurrence, independently branched C₆-C₂₄ alkyl. For example, in some embodiments, R¹ and R² at each occurrence, independently have the following structure: 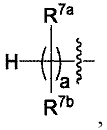 wherein:
R^{7a} and R^{7b} are, at each occurrence, independently H or C₁-C₁₂ alkyl; and
a is an integer from 2 to 12,
wherein R^{7a}, R^{7b} and a are each selected such that R¹ and R² each independently comprise from 6 to 20 carbon atoms. For example, in some embodiments a is an integer ranging from 5 to 9 or from 8 to 12.

In some of the foregoing embodiments, at least one occurrence of R^{7a} is H. For example, in some embodiments, R^{7a} is H at each occurrence. In other different embodiments of the foregoing, at least one occurrence of R^{7b} is C₁-C₈ alkyl. For example, in some embodiments, C₁-C₈ alkyl is methyl, ethyl, n-propyl, iso-propyl, n-butyl, iso-butyl, tert-butyl, n-hexyl or n-octyl.

In different embodiments, R¹ or R², or both, at each occurrence independently has one of the following structures: 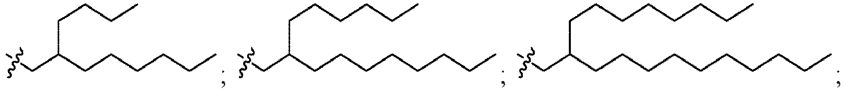 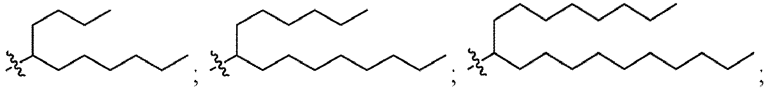 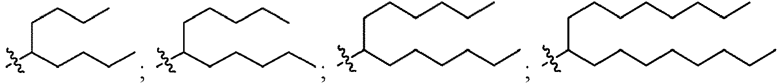 or

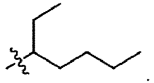

In some of the foregoing embodiments, R^{b}, R^{c}, R^{e} and R^{f}, when present, are each independently C₃-C₁₂ alkyl. For example, in some embodiments R^{b}, R^{c}, R^{e} and R^{f}, when present, are n-hexyl and in other embodiments R^{b}, R^{c}, R^{e} and R^{f}, when present, are n-octyl.

In various different embodiments, the compound has one of the structures set forth in Table 6 below.

**Table 6: Representative Compounds of Formula (VII)**

| **No.** | **Structure** |
|---|---|
| VII-1 | 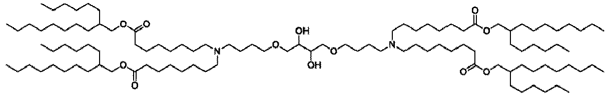 |
| VII-2 | 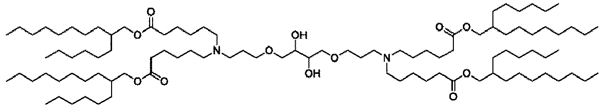 |
| VII-3 | 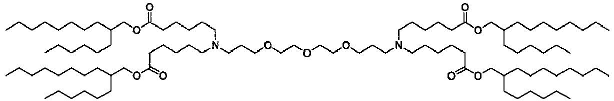 |
| VII-4 | 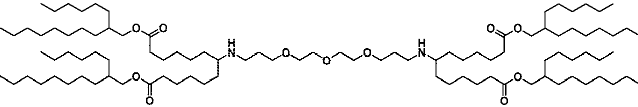 |
| VII-5 | 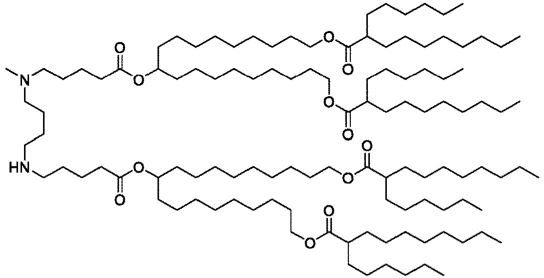 |
| VII-6 | 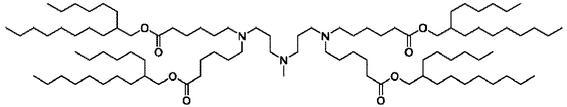 |
| VII-7 | 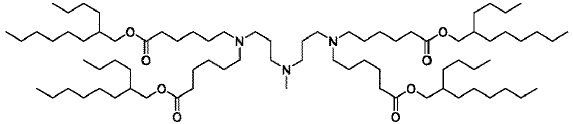 |
| VII-8 | 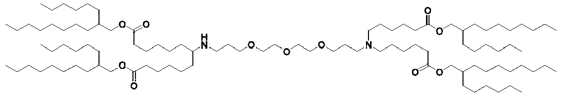 |
| VII-9 | 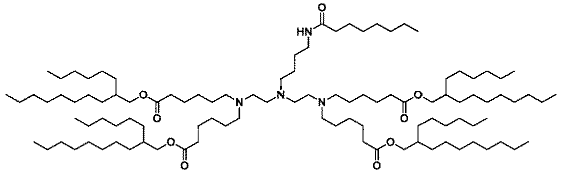 |
| VII-10 | 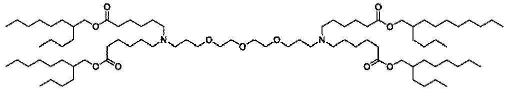 |
| VII-11 | 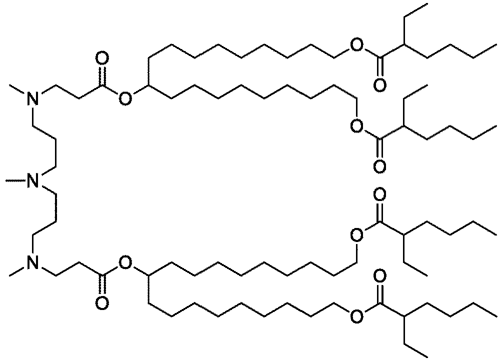 |

In one embodiment, the cationic lipid has the following Formula (VIII): 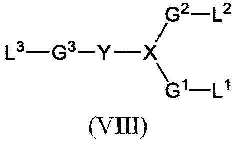 or a pharmaceutically acceptable salt or stereoisomer thereof, wherein:
X is N, and Y is absent; or X is CR, and Y is NR;
L¹ is -O(C=O)R¹, -(C=O) OR¹, -C(=O)R¹, -OR¹, -S(O)xR¹, -S-SR¹, -C(=O)SR¹, -SC(=O)R¹, -NR^{a}C(=O)R¹, -C(=O)NR^{b}R^{c}, -NR^{a}C(=O)NR^{b}R^{c}, -OC(=O)NR^{b}R^{c} or -NR^{a}C(=O)OR¹;
L² is -O(C=O)R², -(C=O)OR², -C(=O)R², -OR², -S(O)ₓR², -S-SR², -C(=O)SR², -SC(=O)R², -NR^{d}C(=O)R², -C(=O)NR^{e}R^{f}, -NR^{d}C(=O)NR^{e}R^{f}, -OC(=O)NR^{e}R^{f}; -NR^{d}C(=O)OR² or a direct bond to R²;
L³ is -O(C=O)R³ or -(C=O)OR³;
G¹ and G² are each independently C₂-C₁₂ alkylene or C₂-C₁₂ alkenylene;
G³ is C₁-C₂₄ alkylene, C₂-C₂₄ alkenylene, C₁-C₂₄ heteroalkylene or C₂-C₂₄ heteroalkenylene;
R^{a}, R^{b}, R^{d} and R^{e} are each independently H or C₁-C₁₂ alkyl or C₁-C₁₂ alkenyl;
R^{c} and R^{f} are each independently C₁-C₁₂ alkyl or C₂-C₁₂ alkenyl;
each R is independently H or C₁-C₁₂ alkyl;
R¹, R² and R³ are each independently C₁-C₂₄ alkyl or C₂-C₂₄ alkenyl; and
x is 0, 1 or 2, and
wherein each alkyl, alkenyl, alkylene, alkenylene, heteroalkylene and heteroalkenylene is independently substituted or unsubstituted unless otherwise specified.

In more embodiments of Formula (VIII):
X is N, and Y is absent; or X is CR, and Y is NR;
L¹ is -O(C=O)R¹, -(C=O)OR¹, -C(=O)R¹, -OR¹, -S(O)ₓR¹, -S-SR¹, -C(=O)SR¹, -SC(=O)R¹, -NR^{a}C(=O)R¹, -C(=O)NR^{b}R^{c}, -NR^{a}C(=O)NR^{b}R^{c}, -OC(=O)NR^{b}R^{c} or -NR^{a}C(=O)OR¹;
L² is -O(C=O)R², -(C=O)OR², -C(=O)R², -OR², -S(O)ₓR², -S-SR², -C(=O)SR², -SC(=O)R², -NR^{d}C(=O)R², -C(=O)NR^{e}R^{f}, -NR^{d}C(=O)NR^{e}R^{f}, -OC(=O)NR^{e}R^{f}; -NR^{d}C(=O)OR² or a direct bond to R²;
L³ is -O(C=O)R³ or -(C=O)OR³;
G¹ and G² are each independently C₂-C₁₂ alkylene or C₂-C₁₂ alkenylene;
G³ is C₁-C₂₄ alkylene, C₂-C₂₄ alkenylene, C₁-C₂₄ heteroalkylene or C₂-C₂₄ heteroalkenylene when X is CR, and Y is NR; and G³ is C₁-C₂₄ heteroalkylene or C₂-C₂₄ heteroalkenylene when X is N, and Y is absent;
R^{a}, R^{b}, R^{d} and R^{e} are each independently H or C₁-C₁₂ alkyl or C₁-C₁₂ alkenyl;
R^{c} and R^{f} are each independently C₁-C₁₂ alkyl or C₂-C₁₂ alkenyl;
each R is independently H or C₁-C₁₂ alkyl;
R¹, R² and R³ are each independently C₁-C₂₄ alkyl or C₂-C₂₄ alkenyl; and
x is 0, 1 or 2, and
wherein each alkyl, alkenyl, alkylene, alkenylene, heteroalkylene and heteroalkenylene is independently substituted or unsubstituted unless otherwise specified.

In other embodiments of Formula (VIII):
X is N and Y is absent, or X is CR and Y is NR;
L¹ is -O(C=O)R¹, -(C=O)OR¹, -C(=O)R¹, -OR¹, -S(O)ₓR¹, -S-SR¹, -C(=O)SR¹, -SC(=O)R¹, -NR^{a}C(=O)R¹, -C(=O)NR^{b}R^{c}, -NR^{a}C(=O)NR^{b}R^{c}, -OC(=O)NR^{b}R^{c} or -NR^{a}C(=O)OR¹;
L² is -O(C=O)R², -(C=O)OR², -C(=O)R², -OR², -S(O)ₓR², -S-SR², -C(=O)SR², -SC(=O)R², -NR^{d}C(=O)R², -C(=O)NR^{e}R^{f}, -NR^{d}C(=O)NR^{e}R^{f}, -OC(=O)NR^{e}R^{f}; -NR^{d}C(=O)OR² or a direct bond to R²;
L³ is -O(C=O)R³ or -(C=O)OR³;
G¹ and G² are each independently C₂-C₁₂ alkylene or C₂-C₁₂ alkenylene;
G³ is C₁-C₂₄ alkylene, C₂-C₂₄ alkenylene, C₁-C₂₄ heteroalkylene or C₂-C₂₄ heteroalkenylene;
R^{a}, R^{b}, R^{d} and R^{e} are each independently H or C₁-C₁₂ alkyl or C₁-C₁₂ alkenyl;
R^{c} and R^{f} are each independently C₁-C₁₂ alkyl or C₂-C₁₂ alkenyl;
each R is independently H or C₁-C₁₂ alkyl;
R¹, R² and R³ are each independently branched C₆-C₂₄ alkyl or branched C₆-C₂₄ alkenyl; and
x is 0, 1 or 2, and
wherein each alkyl, alkenyl, alkylene, alkenylene, heteroalkylene and heteroalkenylene is independently substituted or unsubstituted unless otherwise specified.

In certain embodiments, G³ is unsubstituted. In more specific embodiments G³ is C₂-C₁₂ alkylene, for example, in some embodiments G³ is C₃-C₇ alkylene or in other embodiments G³ is C₃-C₁₂ alkylene. In some embodiments, G³ is C₂ or C₃ alkylene.

In other embodiments, G³ is C₁-C₁₂ heteroalkylene, for example C₁-C₁₂ aminylalkylene.

In certain embodiments, X is N and Y is absent. In other embodiments, X is CR and Y is NR, for example in some of these embodiments R is H.

In some of the foregoing embodiments, the compound has one of the following Formulas (VIIIA), (VIIIB), (VIIIC) or (VIIID):

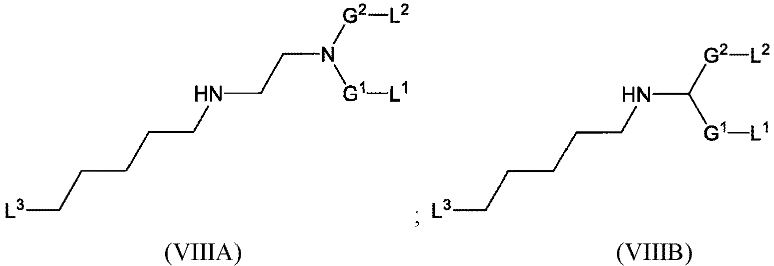

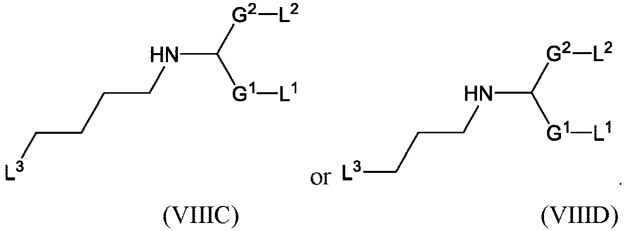

In some of the foregoing embodiments, L¹ is -O(C=O)R¹, -(C=O)OR¹ or -C(=O)NR^{b}R^{c}, and L² is -O(C=O)R², -(C=O)OR² or -C(=O)NR^{e}R^{f}. In other specific embodiments, L¹ is -(C=O)OR¹ and L² is -(C=O)OR². In any of the foregoing embodiments, L³ is -(C=O)OR³.

In some of the foregoing embodiments, G¹ and G² are each independently C₂-C₁₂ alkylene, for example C₄-C₁₀ alkylene.

In some of the foregoing embodiments, R¹, R² and R³ are each, independently branched C₆-C₂₄ alkyl. For example, in some embodiments, R¹, R² and R³ each, independently have the following structure: 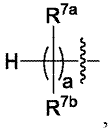 wherein:
R^{7a} and R^{7b} are, at each occurrence, independently H or C₁-C₁₂ alkyl; and
a is an integer from 2 to 12,
wherein R^{7a}, R^{7b} and a are each selected such that R¹ and R² each independently comprise from 6 to 20 carbon atoms. For example, in some embodiments a is an integer ranging from 5 to 9 or from 8 to 12.

In some of the foregoing embodiments, at least one occurrence of R^{7a} is H. For example, in some embodiments, R^{7a} is H at each occurrence. In other different embodiments of the foregoing, at least one occurrence of R^{7b} is C₁-C₈ alkyl. For example, in some embodiments, C₁-C₈ alkyl is methyl, ethyl, n-propyl, iso-propyl, n-butyl, iso-butyl, tert-butyl, n-hexyl or n-octyl.

In some of the foregoing embodiments, X is CR, Y is NR and R³ is C₁-C₁₂ alkyl, such as ethyl, propyl or butyl. In some of these embodiments, R¹ and R² are each independently branched C₆-C₂₄ alkyl.

In different embodiments, R¹, R² and R³ each, independently have one of the following structures: 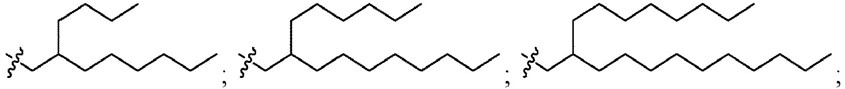 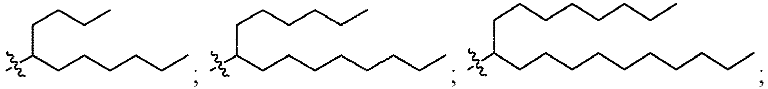 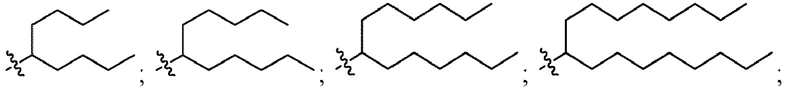 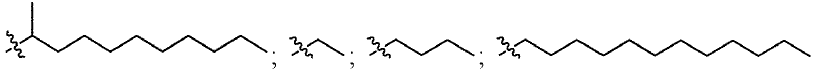 or

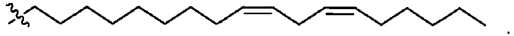

In certain embodiments, R¹ and R² and R³ are each, independently, branched C₆-C₂₄ alkyl and R³ is C₁-C₂₄ alkyl or C₂-C₂₄ alkenyl.

In some of the foregoing embodiments, R^{b}, R^{c}, R^{e} and R^{f} are each independently C₃-C₁₂ alkyl. For example, in some embodiments R^{b}, R^{c}, R^{e} and R^{f} are n-hexyl and in other embodiments R^{b}, R^{c}, R^{e} and R^{f} are n-octyl.

In various different embodiments, the compound has one of the structures set forth in Table 7 below.

**Table 7: Representative Compounds of Formula (VIII)**

| **No.** | **Structure** |
|---|---|
| VIII -1 | 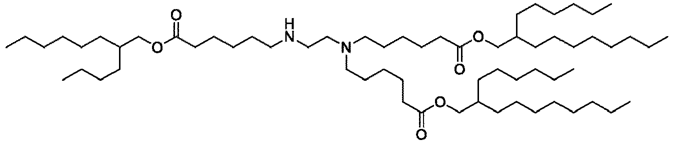 |
| VIII -2 | 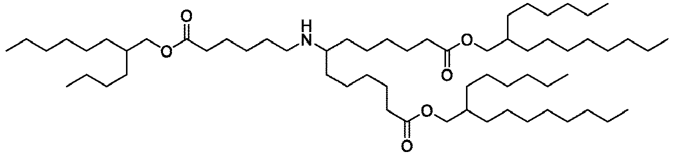 |
| VIII -3 | 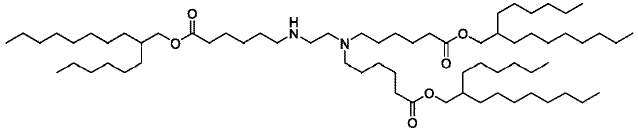 |
| VIII -4 | 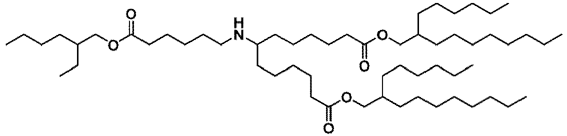 |
| VIII -5 | 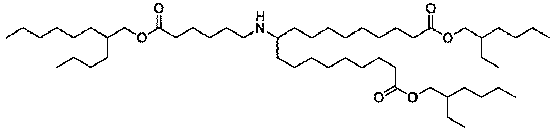 |
| VIII -6 | 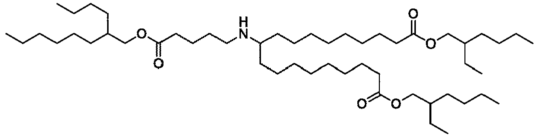 |
| VIII -7 | 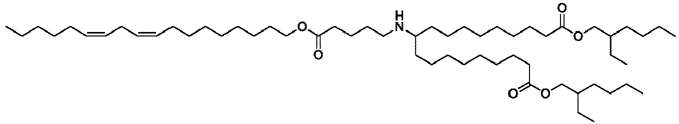 |
| VIII -8 | 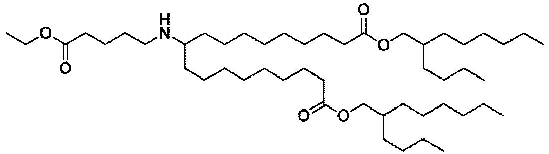 |
| VIII -9 | 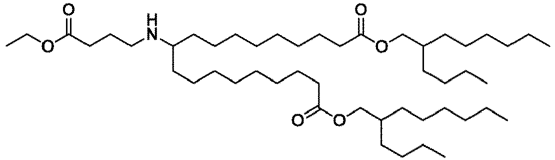 |
| VIII -10 | 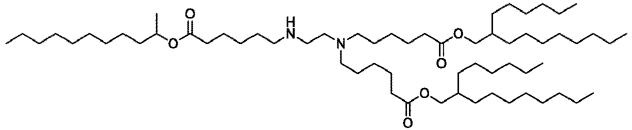 |
| VIII -11 | 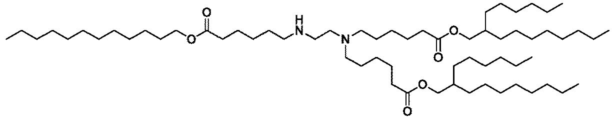 |
| VIII -12 | 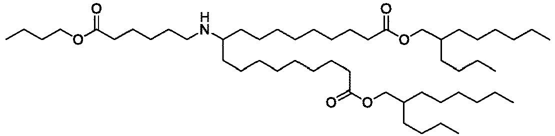 |

In one embodiment, the cationic lipid has the following Formula (IX): 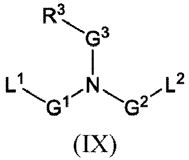 or a pharmaceutically acceptable salt or stereoisomer thereof, wherein:
L¹ is -O(C=O)R¹, -(C=O)OR¹, -C(=O)R¹, -OR¹, -S(O)ₓR¹, -S-SR¹, -C(=O)SR¹, -SC(=O)R¹, -NR^{a}C(=O)R¹, -C(=O)NR^{b}R^{c}, -NR^{a}C(=O)NR^{b}R^{c}, - OC(=O)NR^{b}R^{c} or -NR^{a}C(=O)OR¹;
L² is -O(C=O)R², -(C=O)OR², -C(=O)R², -OR², -S(O)ₓR², -S-SR², -C(=O)SR², -SC(=O)R², -NR^{d}C(=O)R², -C(=O)NR^{e}R^{f}, -NR^{d}C(=O)NR^{e}R^{f}, - OC(=O)NR^{e}R^{f};
   -NR^{d}C(=O)OR² or a direct bond to R²;
G¹ and G² are each independently C₂-C₁₂ alkylene or C₂-C₁₂ alkenylene;
G³ is C₁-C₂₄ alkylene, C₂-C₂₄ alkenylene, C₃-C₈ cycloalkylene or C₃-C₈ cycloalkenylene;
R^{a}, R^{b}, R^{d} and R^{e} are each independently H or C₁-C₁₂ alkyl or C₁-C₁₂ alkenyl;
R^{c} and R^{f} are each independently C₁-C₁₂ alkyl or C₂-C₁₂ alkenyl;
R¹ and R² are each independently branched C₆-C₂₄ alkyl or branched C₆-C₂₄ alkenyl;
R³ is -N(R⁴)R⁵;
R⁴ is C₁-C₁₂ alkyl;
R⁵ is substituted C₁-C₁₂ alkyl; and
x is 0, 1 or 2, and
wherein each alkyl, alkenyl, alkylene, alkenylene, cycloalkylene, cycloalkenylene, aryl and aralkyl is independently substituted or unsubstituted unless otherwise specified.

In certain embodiments, G³ is unsubstituted. In more specific embodiments G³ is C₂-C₁₂ alkylene, for example, in some embodiments G³ is C₃-C₇ alkylene or in other embodiments G³ is C₃-C₁₂ alkylene. In some embodiments, G³ is C₂ or C₃ alkylene.

In some of the foregoing embodiments, the compound has the following Formula (IXA): 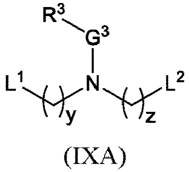 wherein y and z are each independently integers ranging from 2 to 12, for example an integer from 2 to 6, from 4 to 10, or for example 4 or 5. In certain embodiments, y and z are each the same and selected from 4, 5, 6, 7, 8 and 9.

In some of the foregoing embodiments, L¹ is -O(C=O)R¹, -(C=O)OR¹ or -C(=O)NR^{b}R^{c}, and L² is -O(C=O)R², -(C=O)OR² or -C(=O)NR^{e}R^{f}. For example, in some embodiments L¹ and L² are -(C=O)OR¹ and -(C=O)OR², respectively. In other embodiments L¹ is -(C=O)OR¹ and L² is -C(=O)NR^{e}R^{f}. In other embodiments L¹ is -C(=O)NR^{b}R^{c} and L² is -C(=O)NR^{e}R^{f}.

In other embodiments of the foregoing, the compound has one of the following Formulas (IXB), (IXC), (IXD) or (IXE):

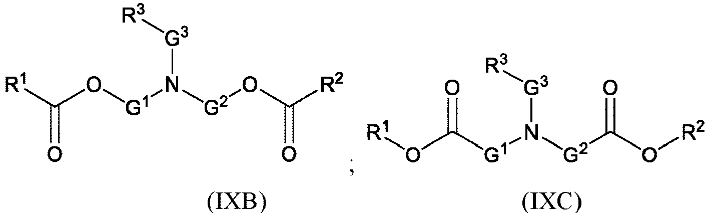

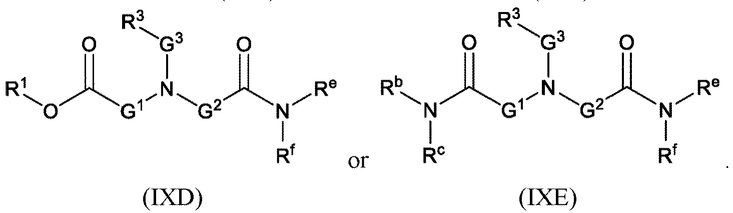

In some of the foregoing embodiments, the compound has Formula (IXB), in other embodiments, the compound has Formula (IXC) and in still other embodiments the compound has the Formula (IXD). In other embodiments, the compound has Formula (IXE).

In some different embodiments of the foregoing, the compound has one of the following Formula (IXF), (IXG), (IXH) or (IXJ): 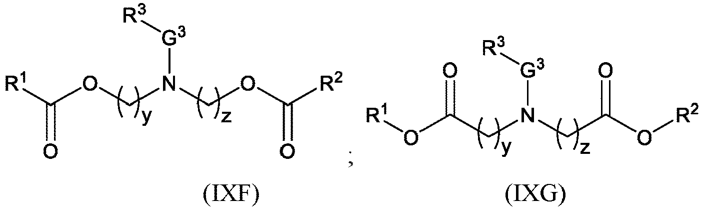 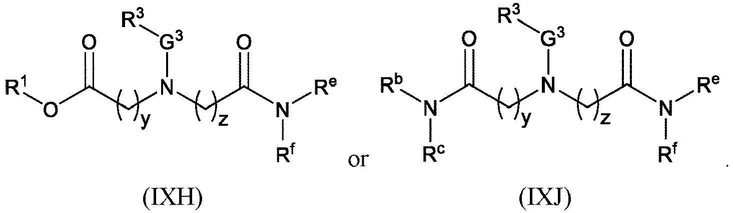 wherein y and z are each independently integers ranging from 2 to 12, for example an integer from 2 to 6, for example 4.

In some of the foregoing embodiments, y and z are each independently an integer ranging from 2 to 10, 2 to 8, from 4 to 10 or from 4 to 7. For example, in some embodiments, y is 4, 5, 6, 7, 8, 9, 10, 11 or 12. In some embodiments, a is 4, 5, 6, 7, 8, 9, 10, 11 or 12. In some embodiments, y and z are the same, while in other embodiments y and z are different.

In some of the foregoing embodiments, R¹ or R², or both is branched C₆-C₂₄ alkyl. For example, in some embodiments, R¹ and R² each, independently have the following structure: 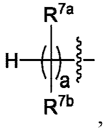 wherein:
R^{7a} and R^{7b} are, at each occurrence, independently H or C₁-C₁₂ alkyl; and
a is an integer from 2 to 12,
wherein R^{7a}, R^{7b} and a are each selected such that R¹ and R² each independently comprise from 6 to 20 carbon atoms. For example, in some embodiments a is an integer ranging from 5 to 9 or from 8 to 12.

In some of the foregoing embodiments, at least one occurrence of R^{7a} is H. For example, in some embodiments, R^{7a} is H at each occurrence. In other different embodiments of the foregoing, at least one occurrence of R^{7b} is C₁-C₈ alkyl. For example, in some embodiments, C₁-C₈ alkyl is methyl, ethyl, n-propyl, iso-propyl, n-butyl, iso-butyl, tert-butyl, n-hexyl or n-octyl.

In different embodiments, R¹ or R², or both, has one of the following structures:

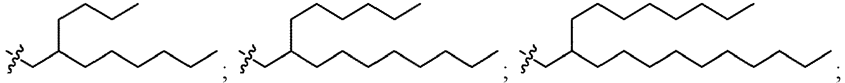

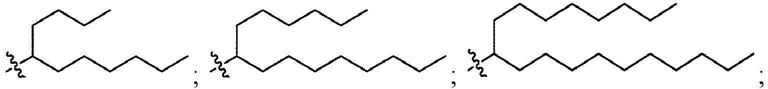

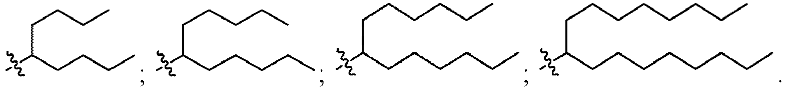

In some of the foregoing embodiments, R^{b}, R^{c}, R^{e} and R^{f} are each independently C₃-C₁₂ alkyl. For example, in some embodiments R^{b}, R^{c}, R^{e} and R^{f} are n-hexyl and in other embodiments R^{b}, R^{c}, R^{e} and R^{f} are n-octyl.

In any of the foregoing embodiments, R⁴ is substituted or unsubstituted: methyl, ethyl, propyl, n-butyl, n-hexyl, n-octyl or n-nonyl. For example, in some embodiments R⁴ is unsubstituted. In other R⁴ is substituted with one or more substituents selected from the group consisting of -OR^{g}, -NR^{g}C(=O)R^{h}, -C(=O)NR^{g}R^{h}, - C(=O)R^{h}, -OC(=O)R^{h}, -C(=O)OR^{h} and -ORⁱOH, wherein:
R^{g} is, at each occurrence independently H or C₁-C₆ alkyl;
R^{h} is at each occurrence independently C₁-C₆ alkyl; and
Rⁱ is, at each occurrence independently C₁-C₆ alkylene.

In other of the foregoing embodiments, R⁵ is substituted: methyl, ethyl, propyl, n-butyl, n-hexyl, n-octyl or n-nonyl. In some embodiments, R⁵ is substituted ethyl or substituted propyl. In other different embodiments, R⁵ is substituted with hydroxyl. In still more embodiments, R⁵ is substituted with one or more substituents selected from the group consisting of -OR^{g}, -NR^{g}C(=O)R^{h}, -C(=O)NR^{g}R^{h}, -C(=O)R^{h}, - OC(=O)R^{h}, -C(=O)OR^{h} and -ORⁱOH, wherein:
R^{g} is, at each occurrence independently H or C₁-C₆ alkyl;
R^{h} is at each occurrence independently C₁-C₆ alkyl; and
Rⁱ is, at each occurrence independently C₁-C₆ alkylene.

In other embodiments, R⁴ is unsubstituted methyl, and R⁵ is substituted: methyl, ethyl, propyl, n-butyl, n-hexyl, n-octyl or n-nonyl. In some of these embodiments, R⁵ is substituted with hydroxyl.

In some other specific embodiments, R³ has one of the following structures:

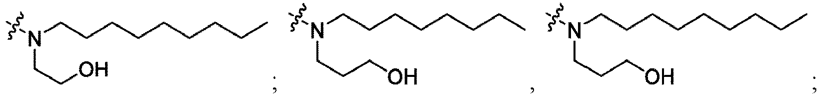

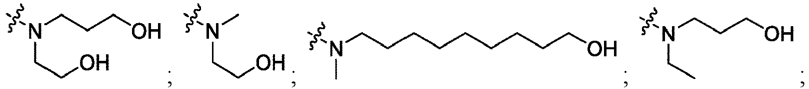

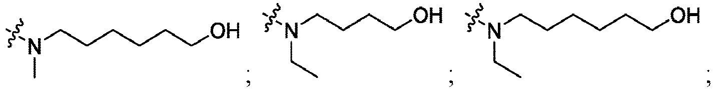

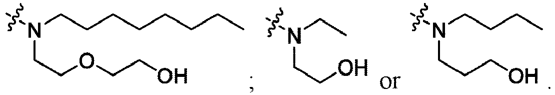

In various different embodiments, the compound has one of the structures set forth in Table 8 below.

**Table 8: Representative Compounds of Formula (IX)**

| **No.** | **Structure** |
|---|---|
| IX-1 | 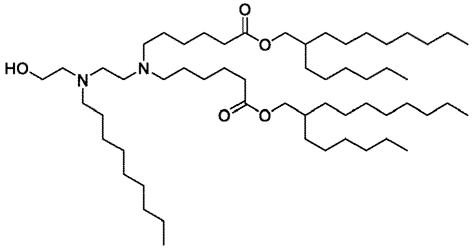 |
| IX-2 | 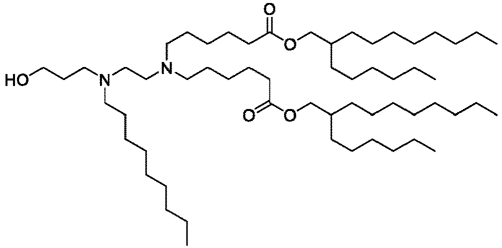 |
| IX-3 | 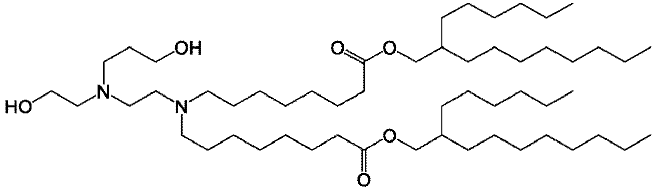 |
| IX-4 | 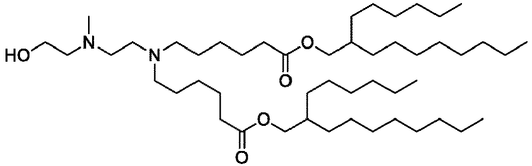 |
| IX-5 | 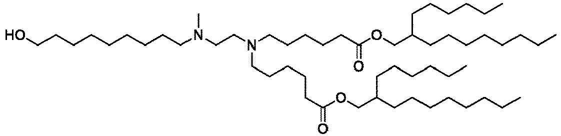 |
| IX-6 | 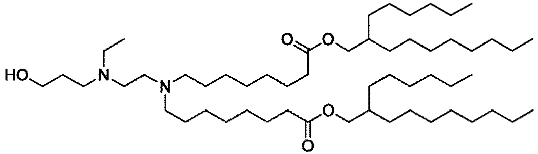 |
| IX-7 | 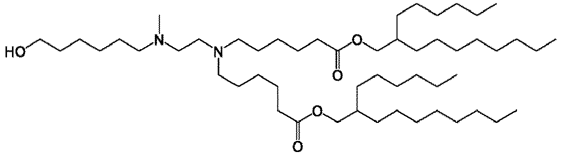 |
| IX-8 | 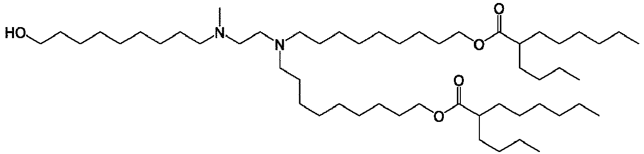 |
| IX-9 | 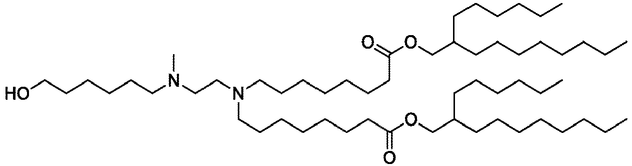 |
| IX-10 | 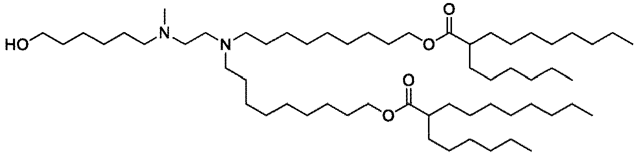 |
| IX-11 | 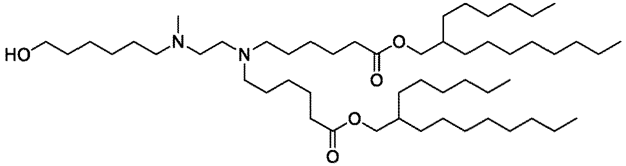 |
| IX-12 | 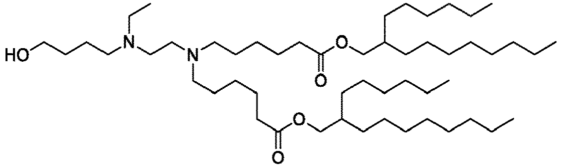 |
| IX-13 | 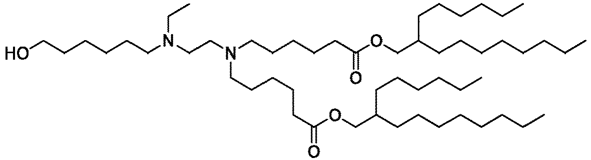 |
| IX-14 | 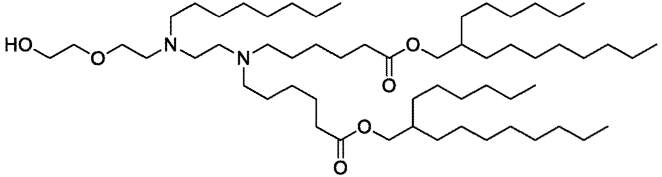 |
| IX-15 | 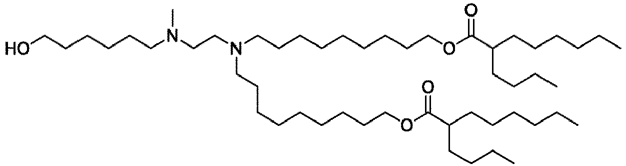 |
| IX-16 | 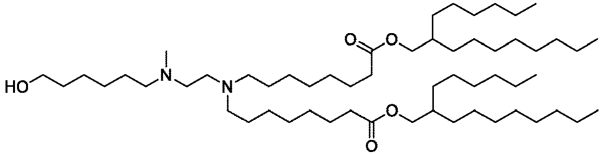 |
| IX-17 | 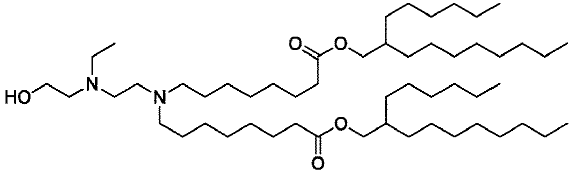 |
| IX-18 | 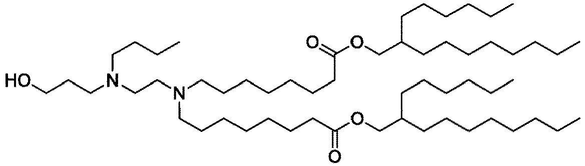 |

In one embodiment, the cationic lipid has the following Formula (X): 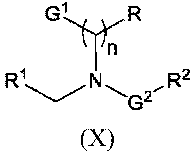 or a pharmaceutically acceptable salt, tautomer or stereoisomer thereof, wherein:
G¹ is -OH, -NR³R⁴, -(C=O)NR⁵ or -NR³(C=O)R⁵;
G² is -CH₂- or -(C=O)-;
R is, at each occurrence, independently H or OH;
R¹ and R² are each independently branched, saturated or unsaturated C₁₂-C₃₆ alkyl;
R³ and R⁴ are each independently H or straight or branched, saturated or unsaturated C₁-C₆ alkyl;
R⁵ is straight or branched, saturated or unsaturated C₁-C₆ alkyl; and
n is an integer from 2 to 6.

In some embodiments, R¹ and R² are each independently branched, saturated or unsaturated C₁₂-C₃₀ alkyl, C₁₂-C₂₀ alkyl, or C₁₅-C₂₀ alkyl. In some specific embodiments, R¹ and R² are each saturated. In certain embodiments, at least one of R¹ and R² is unsaturated.

In some of the foregoing embodiments, R¹ and R² have the following structure:

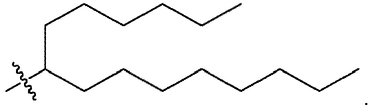

In some of the foregoing embodiments, the compound has the following Formula (XA): 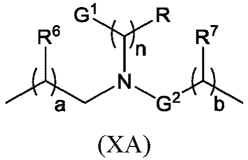 wherein:
R⁶ and R⁷ are, at each occurrence, independently H or straight or branched, saturated or unsaturated C₁-C₁₄ alkyl;
a and b are each independently an integer ranging from 1 to 15,
provided that R⁶ and a, and R⁷ and b, are each independently selected such that R¹ and R², respectively, are each independently branched, saturated or unsaturated C₁₂-C₃₆ alkyl.

In some of the foregoing embodiments, the compound has the following Formula (XB): 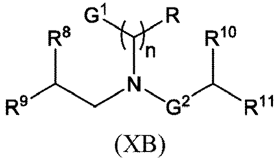 wherein:
R⁸, R⁹, R¹⁰ and R¹¹ are each independently straight or branched, saturated or unsaturated C₄-C₁₂ alkyl, provided that R⁸ and R⁹, and R¹⁰ and R¹¹, are each independently selected such that R¹ and R², respectively, are each independently branched, saturated or unsaturated C₁₂-C₃₆ alkyl. In some embodiments of (XB), R⁸, R⁹, R¹⁰ and R¹¹ are each independently straight or branched, saturated or unsaturated C₆-C₁₀ alkyl. In certain embodiments of (XB), at least one of R⁸, R⁹, R¹⁰ and R¹¹ is unsaturated. In other certain specific embodiments of (XB), each of R⁸, R⁹, R¹⁰ and R¹¹ is saturated.

In some of the foregoing embodiments, the compound has Formula (XA), and in other embodiments, the compound has Formula (XB).

In some of the foregoing embodiments, G¹ is -OH, and in some embodiments G¹ is -NR³R⁴. For example, in some embodiments, G¹ is -NH₂, -NHCH₃ or -N(CH₃)₂. In certain embodiments, G¹ is -(C=O)NR⁵. In certain other embodiments, G¹ is -NR³(C=O)R⁵. For example, in some embodiments G¹ is -NH(C=O)CH₃ or -NH(C=O)CH₂CH₂CH₃.

In some of the foregoing embodiments, G² is -CH₂-. In some different embodiments, G² is -(C=O)-.

In some of the foregoing embodiments, n is an integer ranging from 2 to 6, for example, in some embodiments n is 2, 3, 4, 5 or 6. In some embodiments, n is 2. In some embodiments, n is 3. In some embodiments, n is 4.

In certain of the foregoing embodiments, at least one of R¹, R², R³, R⁴ and R⁵ is unsubstituted. For example, in some embodiments, R¹, R², R³, R⁴ and R⁵ are each unsubstituted. In some embodiments, R³ is substituted. In other embodiments R⁴ is substituted. In still more embodiments, R5 is substituted. In certain specific embodiments, each of R³ and R⁴ are substituted. In some embodiments, a substituent on R³, R⁴ or R⁵ is hydroxyl. In certain embodiments, R³ and R⁴ are each substituted with hydroxyl.

In some of the foregoing embodiments, at least one R is OH. In other embodiments, each R is H.

In various different embodiments, the compound has one of the structures set forth in Table 9 below.

**Table 9: Representative Compounds of Formula (X)**

| **No.** | **Structure** |
|---|---|
| X-1 | 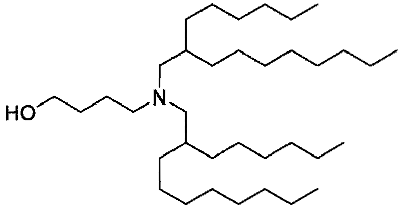 |
| X-2 | 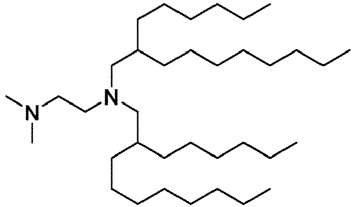 |
| X-3 | 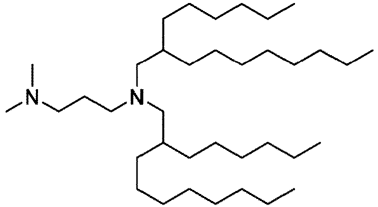 |
| X-4 | 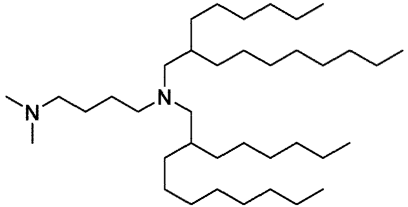 |
| X-5 | 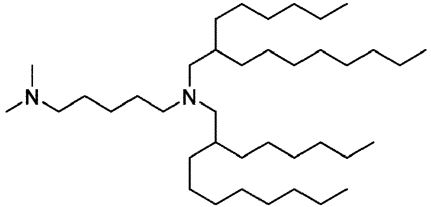 |
| X-6 | 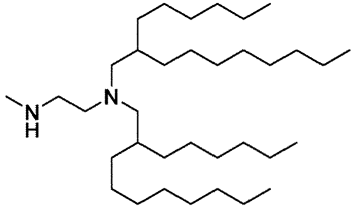 |
| X-7 | 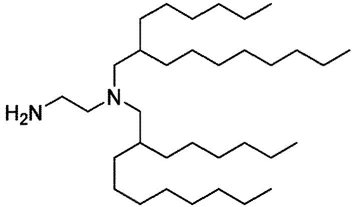 |
| X-8 | 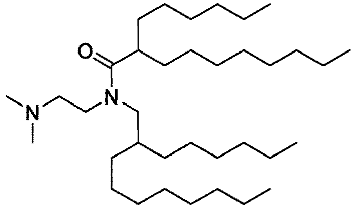 |
| X-9 | 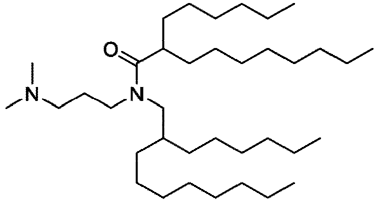 |
| X-10 | 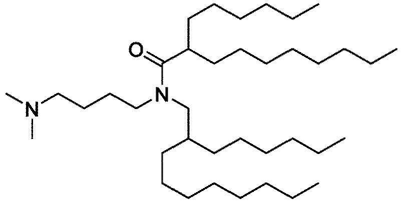 |
| X-11 | 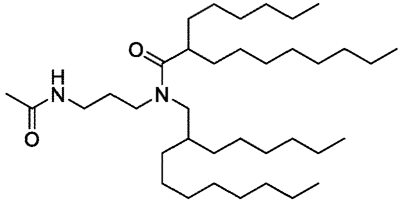 |
| X-12 | 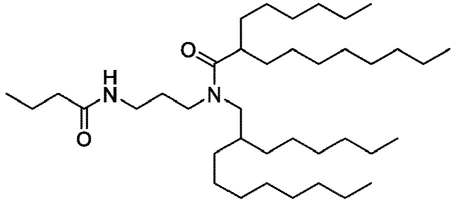 |
| X-13 | 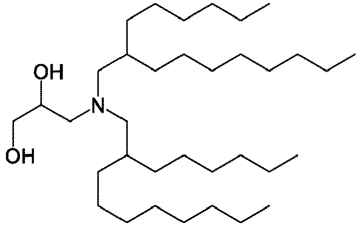 |
| X-14 | 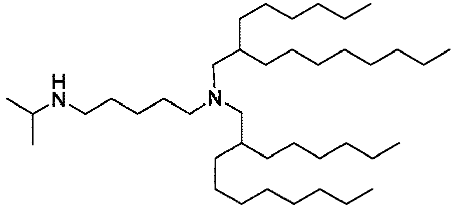 |
| X-15 | 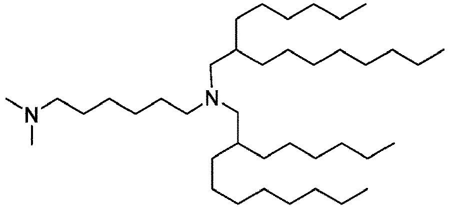 |
| X-16 | 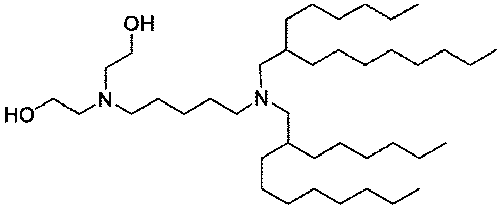 |
| X-17 | 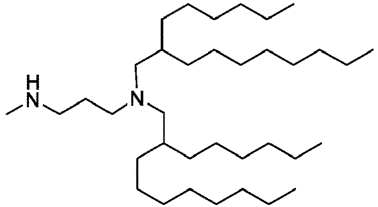 |

In one embodiment, the cationic lipid has the following Formula (XI): 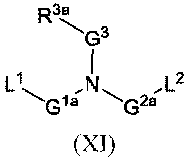 or a pharmaceutically acceptable salt, tautomer or stereoisomer thereof, wherein:
L¹ is -O(C=O)R¹, -(C=O)OR¹, -C(=O)R¹, -OR¹, -S(O)ₓR¹, -S-SR¹, -C(=O)SR¹, -SC(=O)R¹, -NR^{a}C(=O)R¹, -C(=O)NR^{b}R^{c}, -NR^{a}C(=O)NR^{b}R^{c}, -OC(=O)NR^{b}R^{c} or -NR^{a}C(=O)OR¹;
L² is -O(C=O)R², -(C=O)OR², -C(=O)R², -OR², -S(O)ₓR², -S-SR², -C(=O)SR², -SC(=O)R², -NR^{d}C(=O)R², -C(=O)NR^{e}R^{f}, -NR^{c}C(=O)NR^{e}R^{f}, -OC(=O)NR^{e}R^{f}; -NR^{d}C(=O)OR² or a direct bond to R²;
G^{1a} and G^{2a} are each independently C₂-C₁₂ alkylene or C₂-C₁₂ alkenylene;
G³ is C₁-C₂₄ alkylene, C₂-C₂₄ alkenylene, C₃-C₈ cycloalkylene or C₃-C₈ cycloalkenylene;
R^{a}, R^{b}, R^{d} and R^{e} are each independently H or C₁-C₁₂ alkyl or C₂-C₁₂ alkenyl;
R^{c} and R^{f} are each independently C₁-C₁₂ alkyl or C₂-C₁₂ alkenyl;
R¹ and R² are each independently branched C₆-C₂₄ alkyl or branched C₆-C₂₄ alkenyl;
R^{3a} is -C(=O)N(R^{4a})R^{5a} or -C(=O)OR⁶;
R^{4a} is C₁-C₁₂ alkyl;
R^{5a} is H or Ci-Cs alkyl or C₂-C₈ alkenyl;
R⁶ is H, aryl or aralkyl; and
x is 0, 1 or 2, and
wherein each alkyl, alkenyl, alkylene, alkenylene, cycloalkylene, cycloalkenylene, aryl and aralkyl is independently substituted or unsubstituted.

In certain embodiments of Formula (XI), G³ is unsubstituted. In more specific embodiments of Formula (XI), G³ is C₃-C₁₂ alkylene. In some embodiments of Formula (XI), G³ is C₂ or C₃ alkylene.

In some of the foregoing embodiments of Formula (XI), the compound has the following structure (IA): 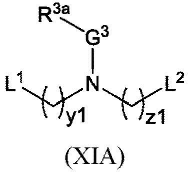 wherein y1 and z1 are each independently integers ranging from 2 to 12, for example an integer from 2 to 6, for example 4.

In some of the foregoing embodiments of Formula (XI), L¹ is -O(C=O)R¹, -(C=O)OR¹ or -C(=O)NR^{b}R^{c}, and L² is -O(C=O)R², -(C=O)OR² or -C(=O)NR^{e}R^{f}. For example, in some embodiments of Formula (XI) L¹ and L² are -(C=O)OR¹ and - (C=O)OR², respectively. In other embodiments of Formula (XI) L¹ is -(C=O)OR¹ and L² is -C(=O)NR^{e}R^{f}. In other embodiments of Formula (XI) L¹ is -C(=O)NRbR^{c} and L² is -C(=O)NR^{e}R^{f}.

In other embodiments of the foregoing, the compound has one of the following Formulas (IB), (IC), (ID) or (IE):

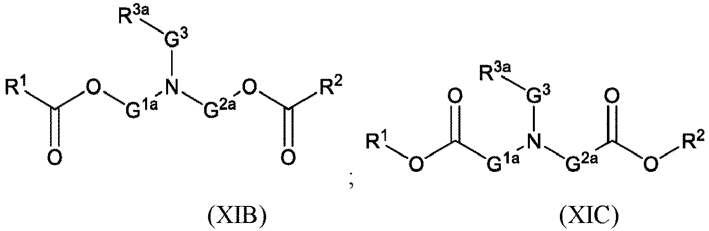

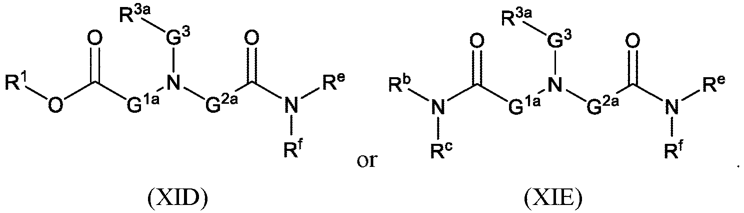

In some of the foregoing embodiments, the compound has Formula (XIB), in other embodiments, the compound has Formula (XIC) and in still other embodiments the compound has the Formula (XID). In other embodiments, the compound has Formula (XIE).

In some different embodiments of the foregoing, the compound has one of the following Formulas (XIF), (XIG), (XIH) or (XU): 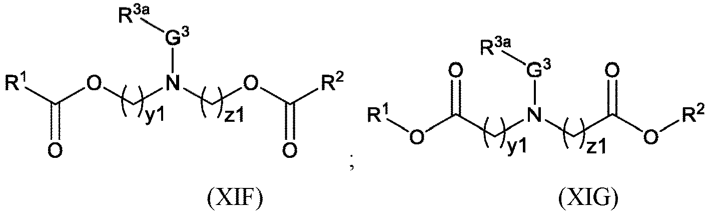 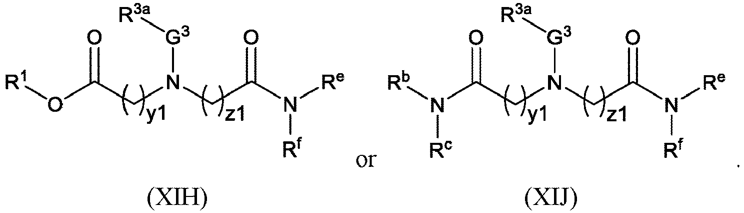 wherein y1 and z1 are each independently integers ranging from 2 to 12, for example an integer from 2 to 6, for example 4.

In some of the foregoing embodiments of Formula (XI), y1 and z1 are each independently an integer ranging from 2 to 10, 2 to 8, from 4 to 10 or from 4 to 7. For example, in some embodiments of Formula (XI), y1 is 4, 5, 6, 7, 8, 9, 10, 11 or 12. In some embodiments of Formula (XI), z1 is 4, 5, 6, 7, 8, 9, 10, 11 or 12. In some embodiments of Formula (XI), y1 and z1 are the same, while in other embodiments of Formula (XI) y1 and z1 are different.

In some of the foregoing embodiments of Formula (XI), R¹ or R², or both is branched C₆-C₂₄ alkyl. For example, in some embodiments of Formula (XI), R¹ and R² each, independently have the following structure: 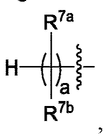 wherein:
R^{7a} and R^{7b} are, at each occurrence, independently H or C₁-C₁₂ alkyl; and
a is an integer from 2 to 12,
wherein R^{7a}, R^{7b} and a are each selected such that R¹ and R² each independently comprise from 6 to 20 carbon atoms. For example, in some embodiments a is an integer ranging from 5 to 9 or from 8 to 12.

In some of the foregoing embodiments of Formula (XI), at least one occurrence of R^{7a} is H. For example, in some embodiments of Formula (XI), R^{7a} is H at each occurrence. In other different embodiments of the foregoing, at least one occurrence of R^{7b} is C₁-C₈ alkyl. For example, in some embodiments, C₁-C₈ alkyl is methyl, ethyl, n-propyl, iso-propyl, n-butyl, iso-butyl, tert-butyl, n-hexyl or n-octyl.

In different embodiments of Formula (XI), R¹ or R², or both, has one of the following structures:

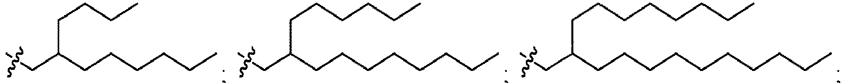

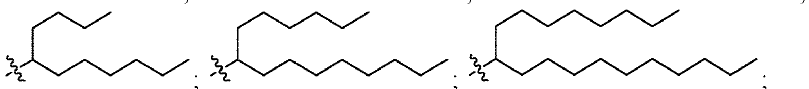

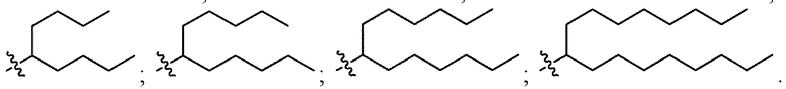

In some of the foregoing embodiments of Formula (XI), R^{b}, R^{c}, R^{e} and R^{f} are each independently C₃-C₁₂ alkyl. For example, in some embodiments of Formula (XI) R^{b}, R^{c}, R^{e} and R^{f} are n-hexyl and in other embodiments of Formula (XI) R^{b}, R^{c}, R^{e} and R^{f} are n-octyl.

In some of the foregoing embodiments of Formula (XI), R^{3a} is -C(=O)N(R^{4a})R^{5a}. In more specific embodiments of Formula (XI), R^{4a} is ethyl, propyl, n-butyl, n-hexyl, n-octyl or n-nonyl. In certain embodiments of Formula (XI), R^{5a} is H, methyl, ethyl, propyl, n-butyl, n-hexyl or n-octyl. In some of these embodiments of Formula (XI), R^{4a} and/or R^{5a} is optionally substituted with a substituent, for example hydroxyl.

In some embodiments of Formula (XI), R^{3a} is -C(=O)OR⁶. In certain embodiments of Formula (XI), R⁶ is benzyl and in other embodiments R⁶ is H.

In some of the foregoing embodiments of Formula (XI), R^{4a}, R^{5a} and R⁶ are independently optionally substituted with one or more substituents selected from the group consisting of -OR^{g}, -NR^{g}C(=O)R^{h}, -C(=O)NR^{g}R^{h}, -C(=O)R^{h}, -OC(=O)R^{h}, - C(=O)OR^{h} and -ORⁱOH, wherein:
R^{g} is, at each occurrence independently H or C₁-C₆ alkyl;
R^{h} is at each occurrence independently C₁-C₆ alkyl; and
Rⁱ is, at each occurrence independently C₁-C₆ alkylene.

In certain specific embodiments of Formula (XI), R^{3a} has one of the following structures:

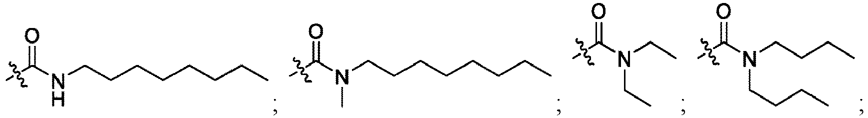

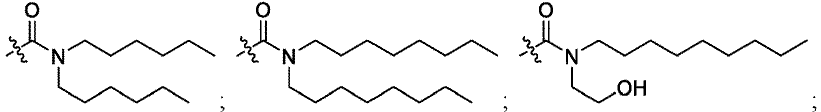

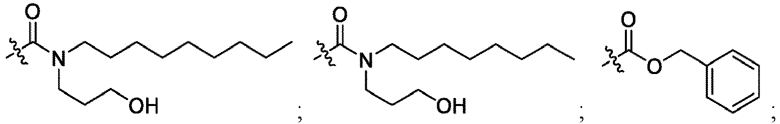

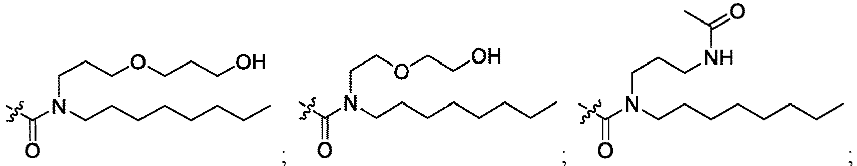

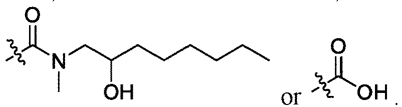

In various different embodiments, the compound has one of the structures set forth in Table 10 below.

**Table 10: Representative Compounds of Formula (XI)**

| **No.** | **Structure** |
|---|---|
| XI-1 | 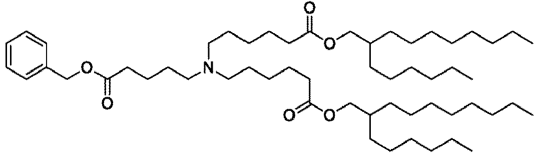 |
| XI-2 | 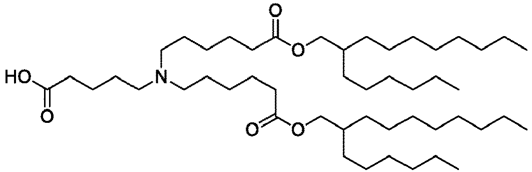 |
| XI-3 | 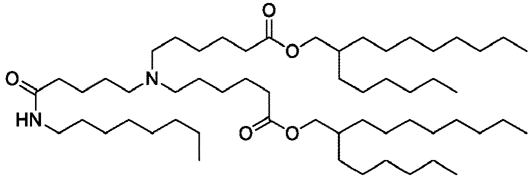 |
| XI-4 | 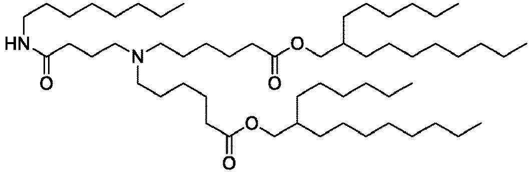 |
| XI-5 | 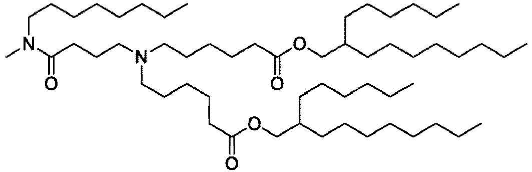 |
| XI-6 | 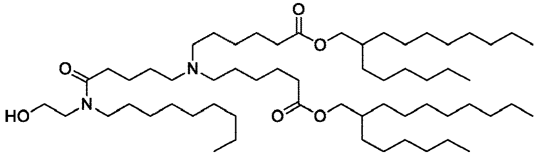 |
| XI-7 | 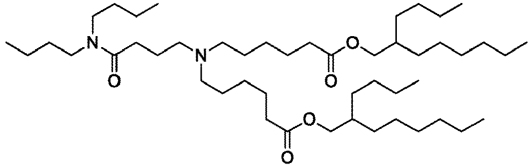 |
| XI-8 | 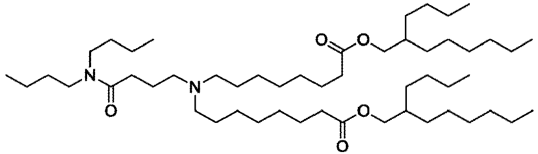 |
| XI-9 | 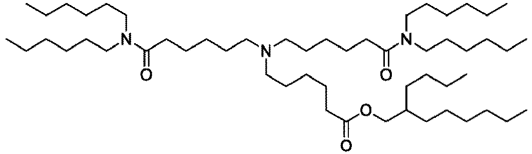 |
| XI-10 | 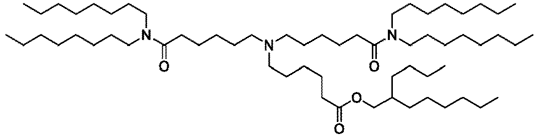 |
| XI-11 | 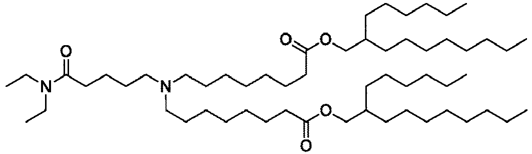 |
| XI-12 | 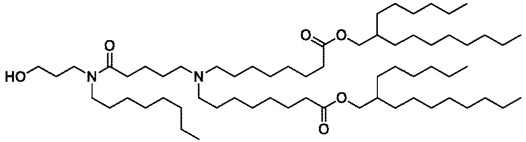 |
| XI-13 | 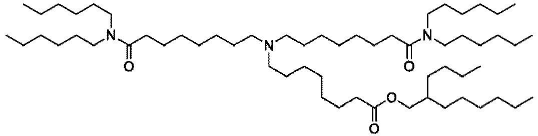 |
| XI-14 | 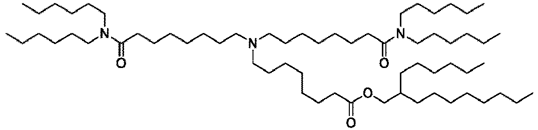 |
| XI-15 | 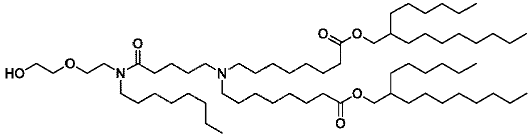 |
| XI-16 | 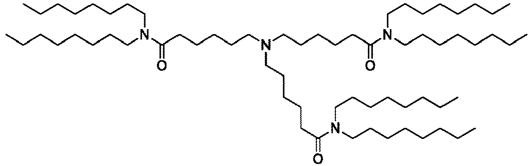 |
| XI-17 | 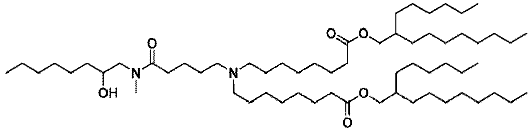 |
| XI-18 | 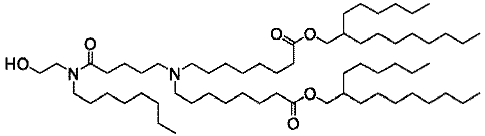 |
| XI-19 | 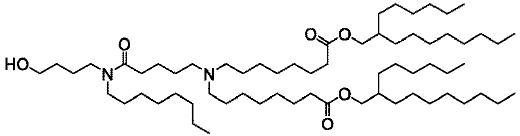 |

In another embodiment, the cationic lipid has the following Formula (XII): 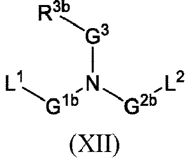 or a pharmaceutically acceptable salt, tautomer or stereoisomer thereof, wherein:
L¹ is -O(C=O)R¹, -(C=O)OR¹, -C(=O)R¹, -OR¹, -S(O)ₓR¹, -S-SR¹, -C(=O)SR¹, -SC(=O)R¹, -NR^{a}C(=O)R¹, -C(=O)NR^{b}R^{c}, -NR^{a}C(=O)NR^{b}R^{c}, -OC(=O)NR^{b}R^{c} or -NR^{a}C(=O)OR¹;
L² is -O(C=O)R², -(C=O)OR², -C(=O)R², -OR², -S(O)ₓR², -S-SR², -C(=O)SR², -SC(=O)R², -NR^{d}C(=O)R², -C(=O)NR^{e}R^{f}, -NR^{c}C(=O)NR^{e}R^{f}, -OC(=O)NR^{e}R^{f}; -NR^{d}C(=O)OR² or a direct bond;
G^{1b} and G^{2b} are each independently C₁-C₁₂ alkylene or C₂-C₁₂ alkenylene;
G³ is C₁-C₂₄ alkylene, C₂-C₂₄ alkenylene, C₃-C₈ cycloalkylene, C₃-C₈ cycloalkenylene;
R^{a}, R^{b}, R^{d} and R^{e} are each independently H or C₁-C₁₂ alkyl or C₂-C₁₂ alkenyl;
R^{c} and R^{f} are each independently C₁-C₁₂ alkyl or C₂-C₁₂ alkenyl;
R¹ and R² are each independently branched C₆-C₂₄ alkyl or branched C₆-C₂₄ alkenyl;
R^{3b} is -NR^{4b}C(=O)R^{5b};
R^{4b} is H, C₁-C₁₂ alkyl or C₂-C₁₂ alkenyl;
R^{5b} is C₂-C₁₂ alkyl or C₂-C₁₂ alkenyl when R^{4b} is H; or R⁵ is C₁-C₁₂ alkyl or C₂-C₁₂ alkenyl when R^{4b} is C₁-C₁₂ alkyl or C₂-C₁₂ alkenyl; and
x is 0, 1 or 2, and
wherein each alkyl, alkenyl, alkylene, alkenylene, cycloalkylene and cycloalkenylene is independently substituted or unsubstituted.

In certain embodiments of Formula (XII), G³ is unsubstituted. In more specific embodiments of Formula (XII) G³ is C₁-C₁₂ alkylene, for example, G³ is C₃-C₅ alkylene or G³ is C₃-C₁₂ alkylene.

In some of the foregoing embodiments, the cationic lipid has the following Formula (XIIA): 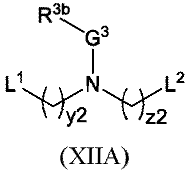 or a pharmaceutically acceptable salt, tautomer or stereoisomer thereof, wherein y2 and z2 are each independently integers ranging from 1 to 12.

In some of the foregoing embodiments of Formula (XIIA), L¹ and L² are each independently -O(C=O)R¹ or -(C=O)OR¹.

In other embodiments of the foregoing, the compound has one of the following Formulas (XIIB) or (XIIC):

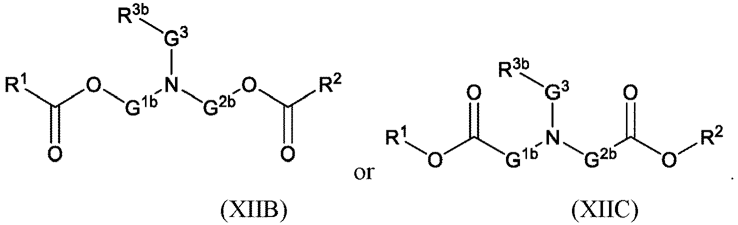

In some of the foregoing embodiments, the compound has Formula (XIIB), in other embodiments, the compound has Formula (XIIC).

In some embodiments, the compound has one of the following Formulas (XIID) or (XIIE): 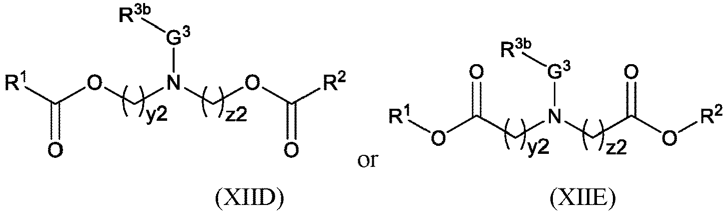 wherein y2 and z2 are each independently integers ranging from 1 to 12.

In some of the foregoing embodiments of Formula (XII), y2 and z2 are each independently an integer ranging from 2 to 12, for example from 2 to 10, from 2 to 8, from 4 to 7 or from 4 to 10. For example, in some embodiments of structure (II), y2 is 4, 5, 6, 7, 8, 9, 10, 11 or 12. In some embodiments of Formula (XII), z2 is 4, 5, 6, 7, 8, 9, 10, 11 or 12. In some embodiments of Formula (XII), y2 and z2 are the same, while in other embodiments of Formula (XII), y2 and z2 are different.

In some of the foregoing embodiments of Formula (XII), R¹ or R², or both is branched C₆-C₂₄ alkyl. For example, in some embodiments of Formula (XII), R¹ and R² each, independently have the following structure: 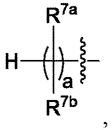 wherein:
R^{7a} and R^{7b} are, at each occurrence, independently H or C₁-C₁₂ alkyl; and
a is an integer from 2 to 12,
wherein R^{7a}, R^{7b} and a are each selected such that R¹ and R² each independently comprise from 6 to 20 carbon atoms. For example, in some embodiments a is an integer ranging from 5 to 9 or from 8 to 12.

In some of the foregoing embodiments of Formula (XII), at least one occurrence of R^{7a} is H. For example, in some embodiments of Formula (XII), R^{7a} is H at each occurrence. In other different embodiments of the foregoing, at least one occurrence of R^{7b} is C₁-C₈ alkyl. For example, in some embodiments of Formula (XII), C₁-C₈ alkyl is methyl, ethyl, n-propyl, iso-propyl, n-butyl, iso-butyl, *tert*-butyl, n-hexyl or n-octyl.

In different embodiments of Formula (XII), R¹ or R², or both, has one of the following structures:

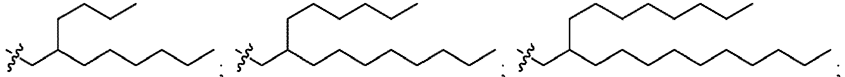

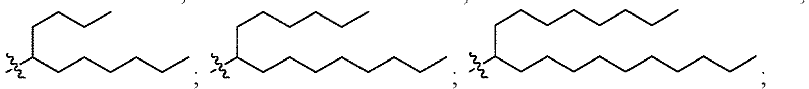

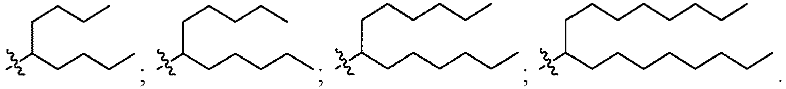

In some of the foregoing embodiments of Formula (XII), R^{4b} is H, methyl, ethyl, propyl or octyl. In some embodiments of Formula (XII), R^{5b} is methyl, ethyl, propyl, heptyl or octyl, for example n-heptyl or n-octyl.

In certain related embodiments of Formula (XII), R^{4b} and R^{5b} are independently optionally substituted with one or more substituents selected from the group consisting of -OR^{g}, -NR^{g}C(=O)R^{h}, -C(=O)NR^{g}R^{h}, -C(=O)R^{h}, -OC(=O)R^{h}, -C(=O)OR ^{h} and -OR^{h}OH, wherein:
R^{g} is, at each occurrence independently H or C₁-C₆ alkyl;
R^{h} is at each occurrence independently C₁-C₆ alkyl; and
R¹ is, at each occurrence independently C₁-C₆ alkylene.

In certain specific embodiments of Formula (XII), R^{3b} has one of the following structures:

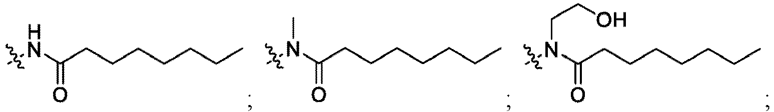

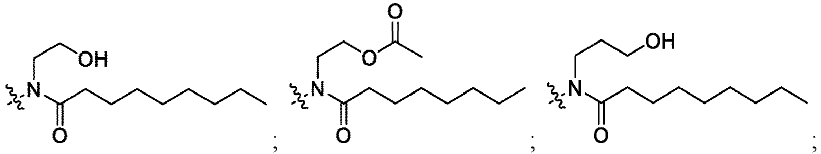

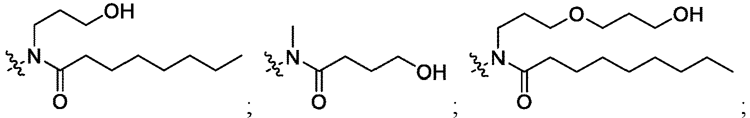

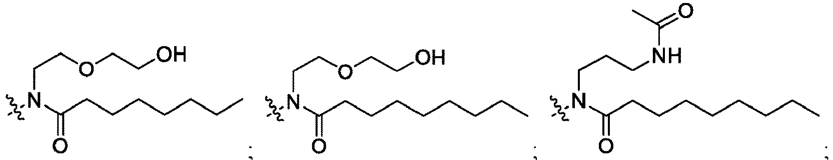

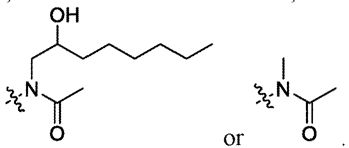

In various different embodiments, the compound of Formula (XII) has one of the structures set forth in Table 11 below.

**Table 11: Representative Compounds of Formula (XII)**

| **No.** | **Structure** |
|---|---|
| XII-1 | 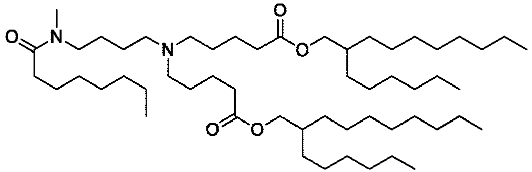 |
| XII-2 | 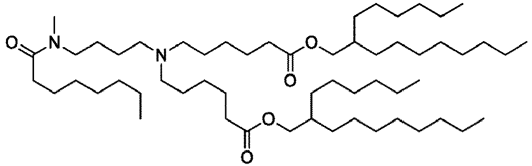 |
| XII-3 | 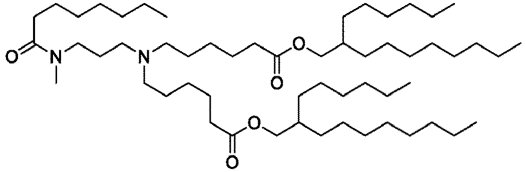 |
| XII-4 | 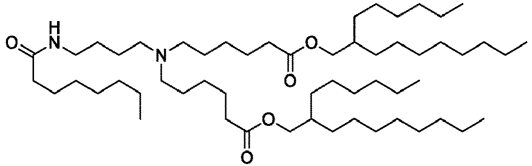 |
| XII-5 | 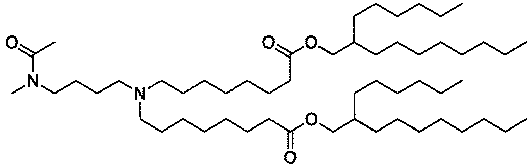 |
| XII-6 | 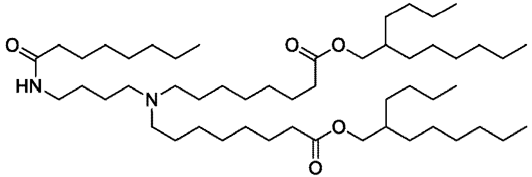 |
| XII-7 | 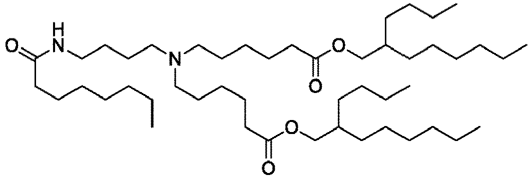 |
| XII-8 | 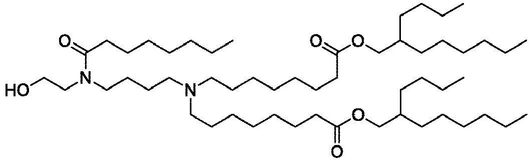 |
| XII-9 | 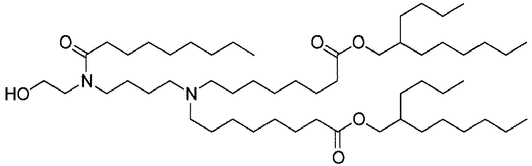 |
| XII-10 | 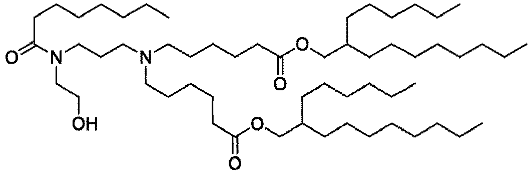 |
| XII-11 | 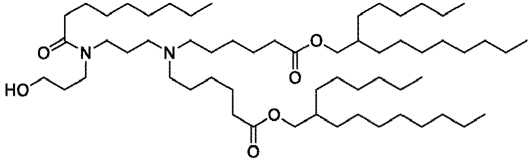 |
| XII-12 | 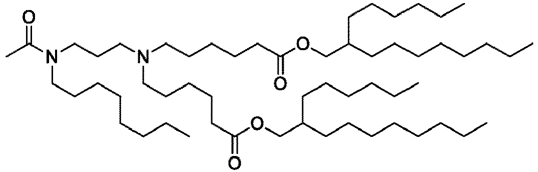 |
| XII-13 | 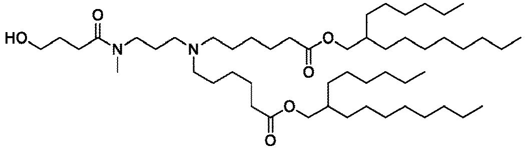 |
| XII-14 | 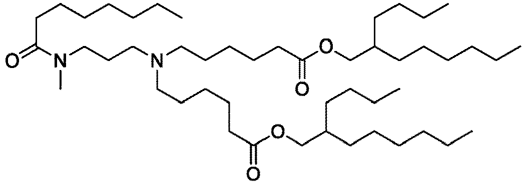 |
| XII-15 | 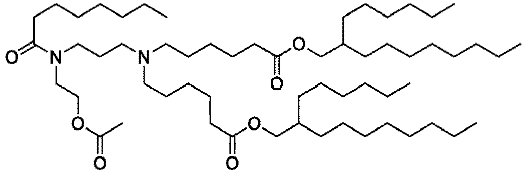 |
| XII-16 | 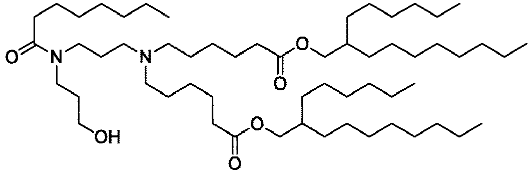 |
| XII-17 | 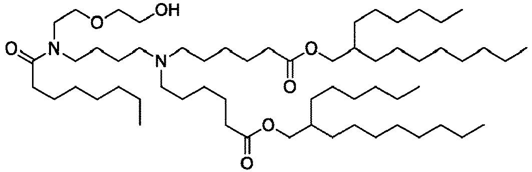 |
| XII-18 | 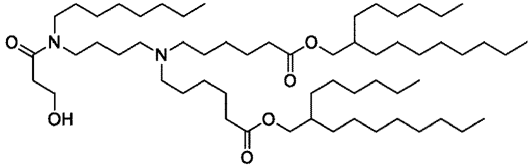 |
| XII-19 | 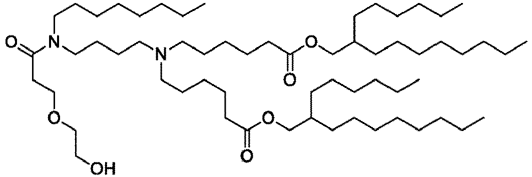 |
| XII-20 | 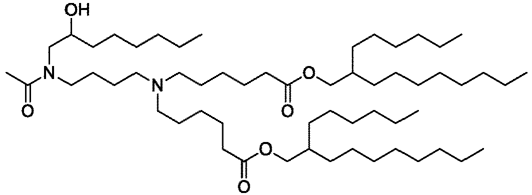 |

In one embodiment, the cationic lipids have the following structure: 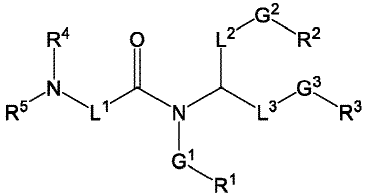 or a pharmaceutically acceptable salt, tautomer, or stereoisomer thereof, wherein:
R¹ is optionally substituted C₁-C₂₄ alkyl or optionally substituted C₂-C₂₄ alkenyl;
R² and R³ are each independently optionally substituted C₁-C₃₆ alkyl;
R⁴ and R⁵ are each independently optionally substituted C₁-C₆ alkyl, or R⁴ and R³ join, along with the N to which they are attached, to form a heterocyclyl or heteroaryl;
L¹, L², and L³ are each independently optionally substituted C₁-C₁₈ alkylene;
G¹ is a direct bond, -(CH₂)ₙO(C=O)-, -(CH₂)ₙ(C=O)O-, or -(C=O)-;
G² and G³ are each independently -(C=O)O- or -O(C=O)-; and
n is an integer greater than 0.

In some embodiments, the compound has the following structure:

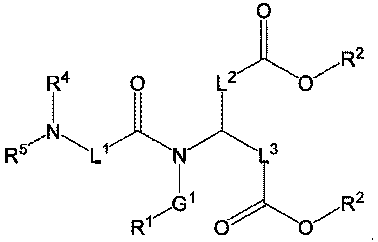

In some embodiments, the compound has the following structure:

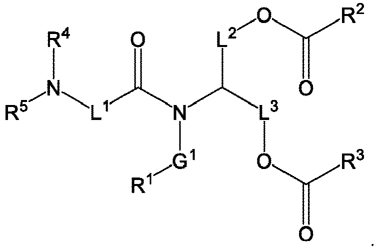

In some embodiments, R¹ is optionally substituted C₆-C₁₈ alkyl or C₁₄-C₁₈ alkenyl. In certain embodiments, R¹ is C₈ alkyl, C₉ alkyl, C₁₀ alkyl, C₁₂ alkyl, C₁₄ alkyl, or C₁₆ alkyl. In some more specific embodiments, R¹ is C₁₆ alkenyl. In certain more specific embodiments, R¹ is unbranched. In some embodiments, R¹ is branched. In certain embodiments, R¹ is unsubstituted.

In some embodiments, G¹ is a direct bond, -(CH₂)ₙO(C=O)-, or - (CH₂)ₙ(C=O)O-. In certain embodiments, G¹ is a direct bond. In some more specific embodiments, G¹ is -(CH₂)ₙ(C=O)O- and n is greater than 1. In some embodiments, n is 1-20. In some embodiments n is 1-10. In some embodiments n is 5-11. In some embodiments, n is 6-10. In certain more specific embodiments, n is 5, 6, 7, 8, 9, or 10. In some embodiments, n is 5. In some embodiments, n is 6. In some embodiments, n is 7. In certain embodiments, n is 8. In some embodiments, n is 9. In some embodiments, n is 10.

In some embodiments, L¹ is C₁-C₆ alkylene. In certain embodiments, L¹ is C₂ alkylene, C₃ alkylene, or C₄ alkylene. In some more specific embodiments, L¹ is unbranched. In certain more specific embodiments, L¹ is unsubstituted.

In some embodiments, R² is C₈-C₂₄ alkyl. In some embodiments, R³ is C₈-C₂₄ alkyl. In some more specific embodiments, R² and R³ are both C₈-C₂₄ alkyl. In some embodiments, R² and R³ are each independently C₁₁ alkyl, C₁₂ alkyl, C₁₃ alkyl, C₁₄ alkyl, C₁₅ alkyl, C₁₆ alkyl, C₁₈ alkyl, or C₂₀ alkyl. In certain embodiments, R² is branched. In more specific embodiments, R³ is branched. In some more specific embodiments, R² and R³ each independently have one of the following structures: 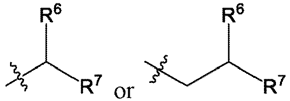 wherein:
R⁶ and R⁷ are each independently C₂-C₁₂ alkyl.

In some embodiments, R² and R³ each independently have one of the following structures: 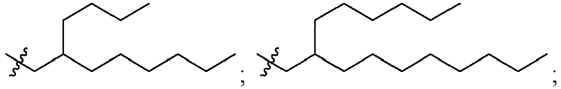 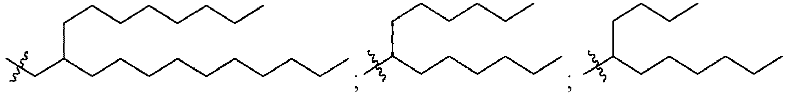 or

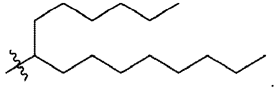

In some embodiments, L² and L³ are each independently C₄-C₁₀ alkylene. In certain embodiments, L² and L³ are both C₅ alkylene. In some more specific embodiments, L² and L³ are both C₆ alkylene. In certain embodiments, L² and L³ are both C₈ alkylene. In some more specific embodiments, L² and L³ are both C₉ alkylene. In some embodiments, L² is unbranched. In some embodiments, L³ is unbranched. In more specific embodiments, L² is unsubstituted. In some embodiments, L² is unsubstituted.

In some embodiments, R⁴ and R⁵ are each independently C₁-C₆ alkyl. In more specific embodiments, R⁴ and R⁵ are both methyl. In certain embodiments, R⁴ and R⁵ are both ethyl. In certain embodiments, R⁴ is methyl and R⁵ is n-butyl. In some embodiments, R⁴ and R⁵ are both n-butyl. In different embodiments, R⁴ is methyl and R⁵ is n-hexyl.

In some embodiments, R⁴ and R⁵ join, along with the N to which they are attached, to form a heterocyclyl. In certain embodiments, the heterocyclyl is a 5-membered heterocyclyl. In some embodiments, the heterocyclyl has the following structure:

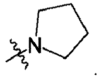

In various different embodiments, the compound has one of the structures set forth in Table 12 below.

**Table 12. Representative Lipid Compounds**

| **No.** | **Structure** | **pKa** |
|---|---|---|
| XIII-1 | 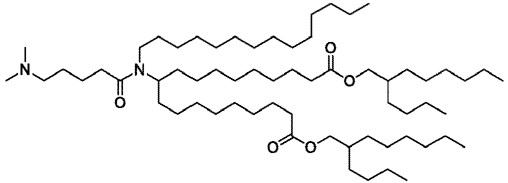 | - |
| XIII-2 | 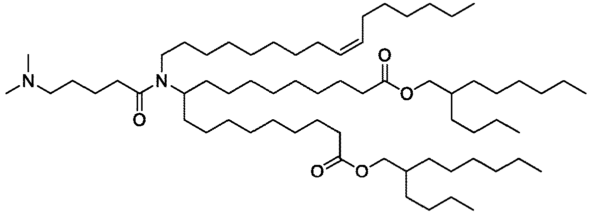 | - |
| XIII-3 | 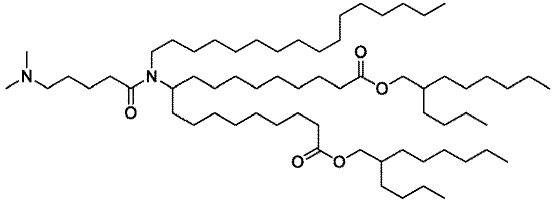 | - |
| XIII-4 | 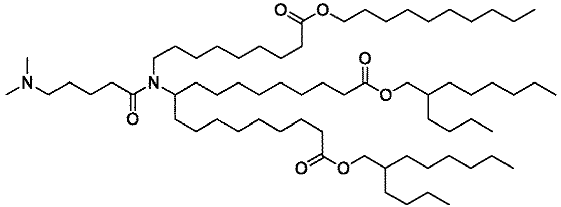 | - |
| XIII-5 | 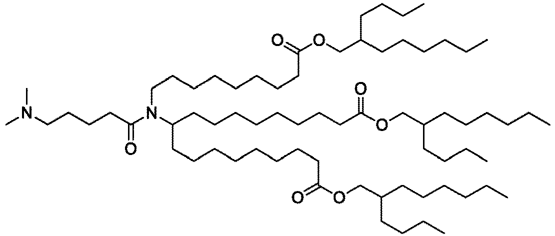 | - |
| XIII-6 | 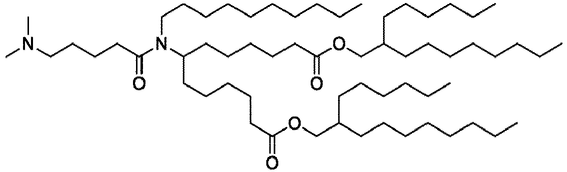 | - |
| XIII-7 | 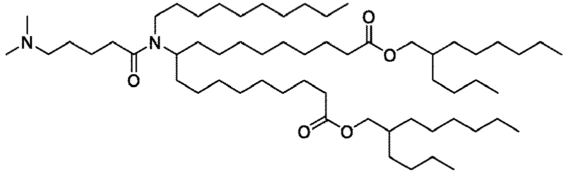 | 6.74 |
| XIII-8 | 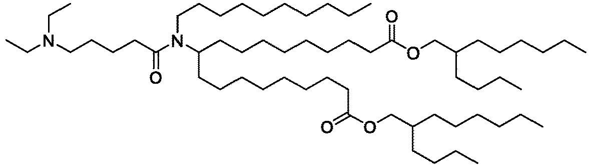 | 6.68 |
| XIII-9 | 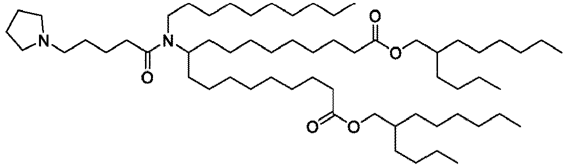 | 6.83 |
| XIII-10 | 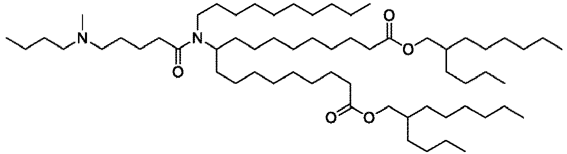 | - |
| XIII-11 | 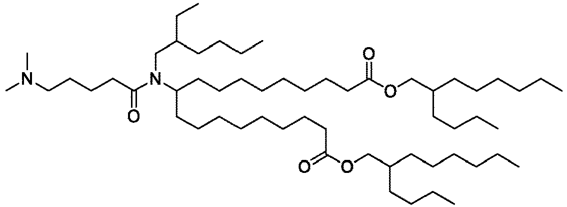 | - |
| XIII-12 | 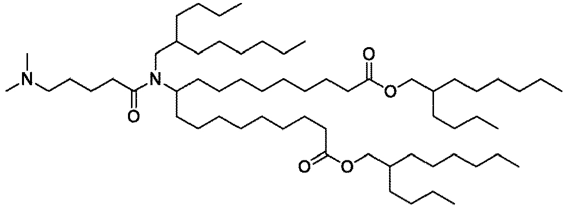 | - |
| XIII-13 | 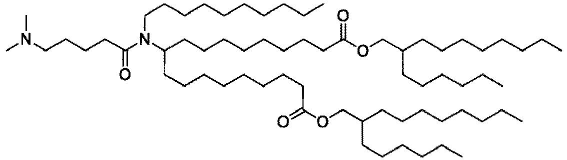 | - |
| XIII-14 | 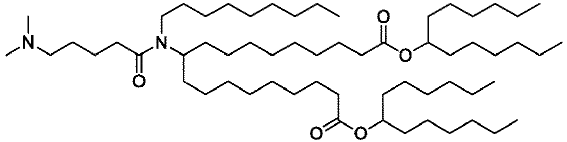 | - |
| XIII-15 | 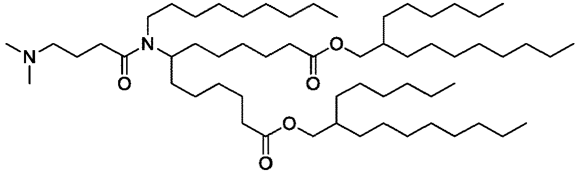 | - |
| XIII-16 | 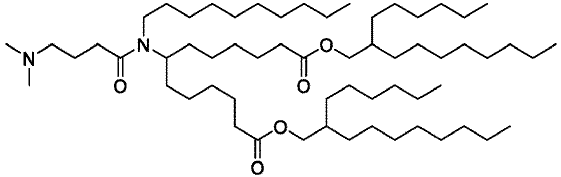 | 6.77 |
| XIII-17 | 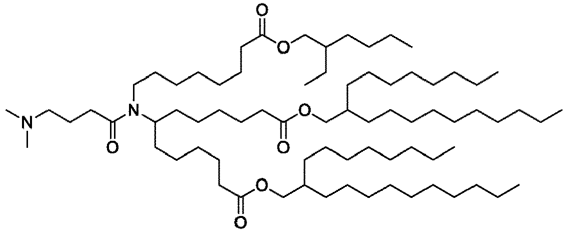 | - |
| XIII-18 | 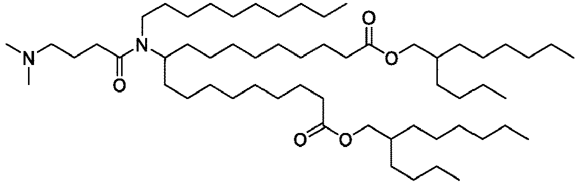 | 6.47 |
| XIII-19 | 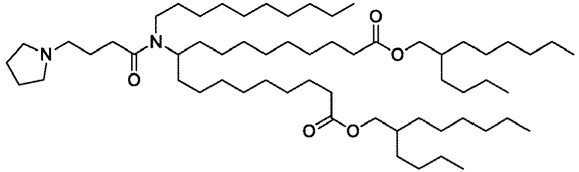 | - |
| XIII-20 | 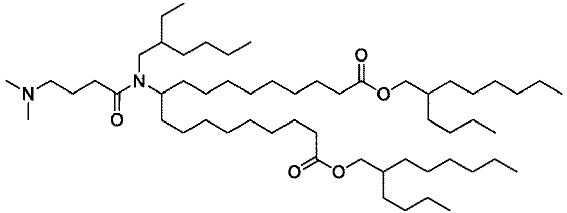 | 6.84 |
| XIII-21 | 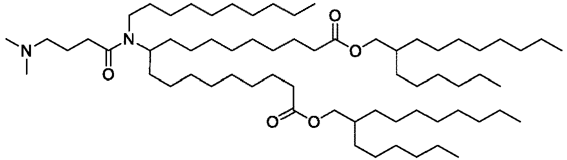 | - |
| XIII-22 | 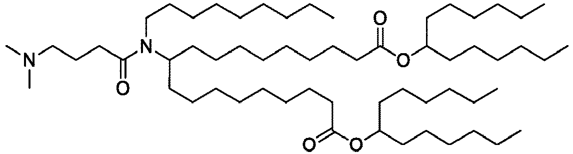 | - |
| XIII-23 | 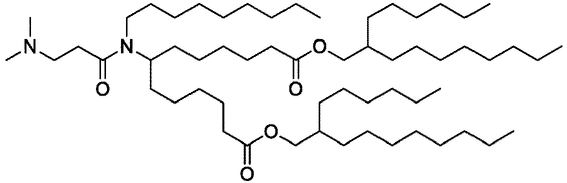 | - |
| XIII-24 | 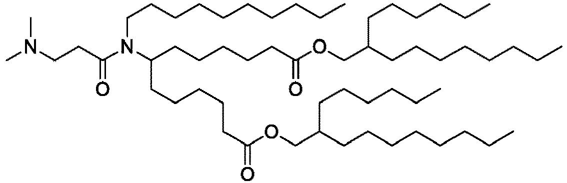 | - |
| XIII-25 | 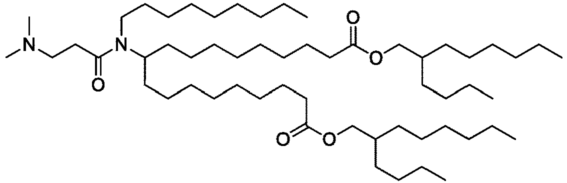 | 6.20 |
| XIII-26 | 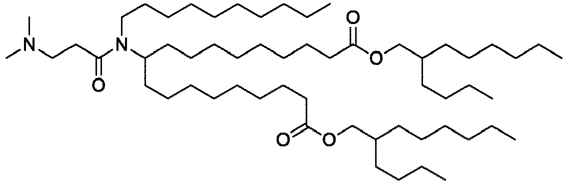 | - |
| XIII-27 | 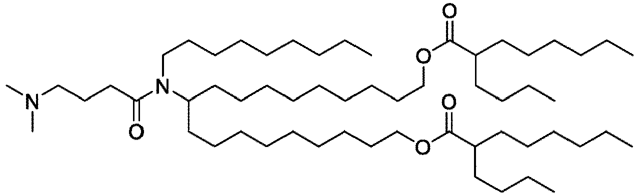 | - |
| XIII-28 | 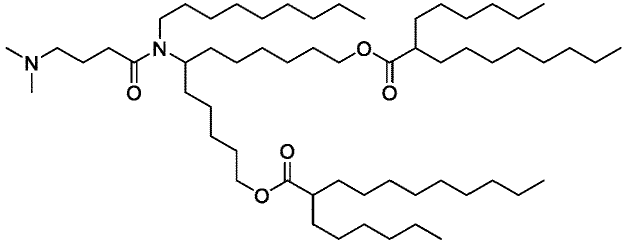 | - |
| XIII-29 | 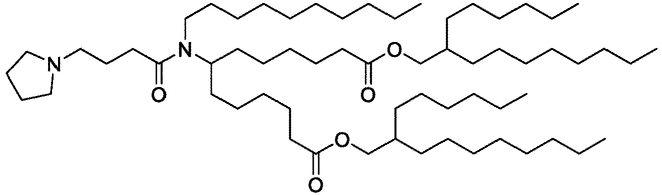 | 6.81 |
| XIII-30 | 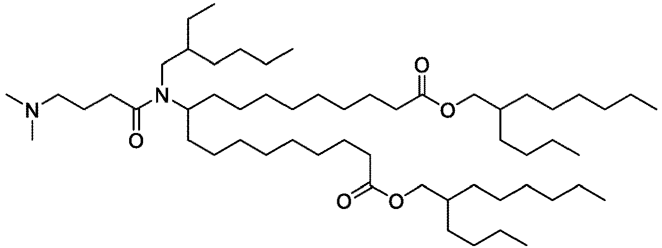 | 6.47 |
| XIII-31 | 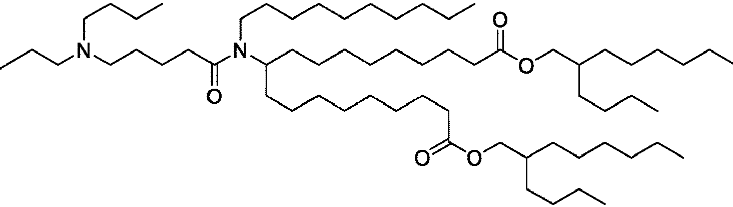 | 5.05 |
| XIII-32 | 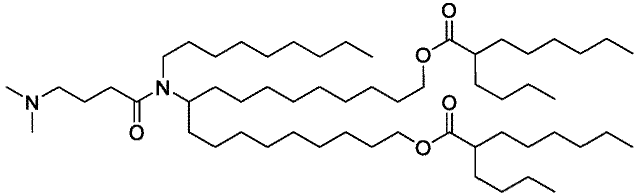 | 6.41 |
| XIII-33 | 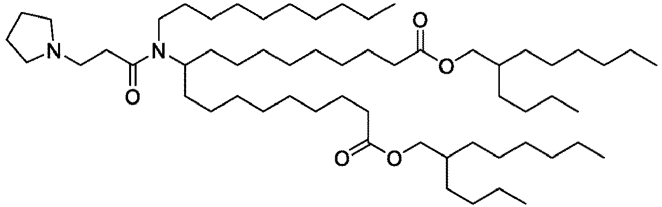 | 6.19 |
| XIII-34 | 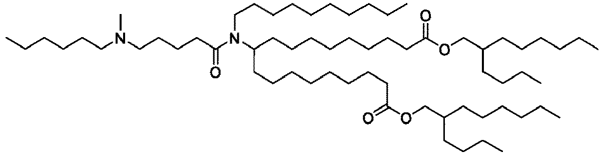 | - |
| XIII-35 | 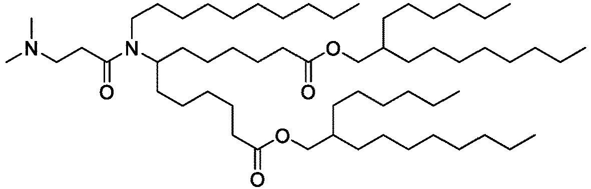 | - |
| XIII-36 | 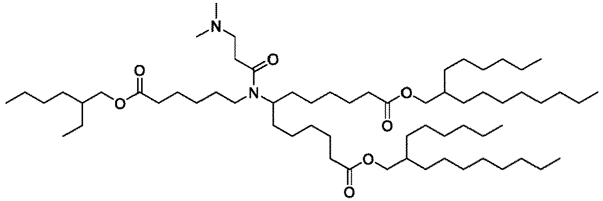 | - |
| XIII-37 | 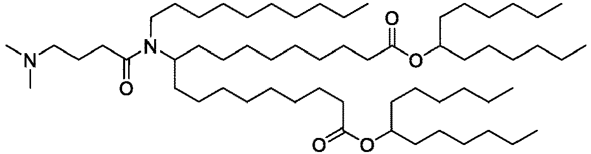 | - |
| XIII-38 | 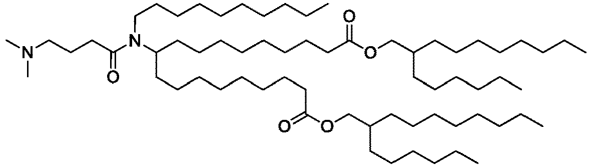 | - |
| XIII-39 | 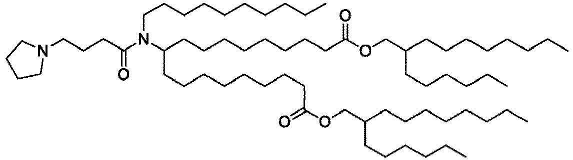 | - |
| **XIII-**40 | 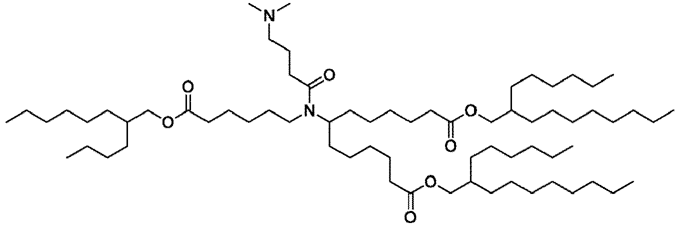 | - |

In one embodiment, the lipid compound has the following structure: 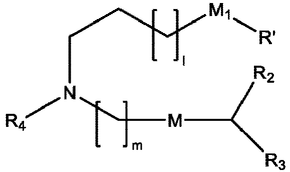 or salts or isomers thereof, wherein:
R₂ and R₃ are independently selected from the group consisting of H, C₁-₁₄ alkyl, C₂-₁₄ alkenyl, -R*YR" and YR";
R₄ is selected from the group consisting of C₃-₆ carbocycle, -(CH₂)ₙQ, - (CH₂)ₙCHQR, -CHQR, -CQR₂, and unsubstituted C₁-₆ alkyl, where Q is selected from a carbocycle, heterocycle, -OR, -N(R)₂, -C(O)NR₂, -N(R)C(O)R, -N(R)S(O)₂R, - N(R)C(O)N(R)₂, -N(R)C(S)N(R)₂, -and N(R)R₈, and each n is independently selected from 1, 2, 3, 4, and 5;
R₈ is selected from the group consisting of C3-6 carbocycle and heterocycle;
Each R is independently selected from the group consisting of C₁-₃ alkyl, C₂-₃ alkenyl, and H;
Each R' is independently selected from the group consisting of C₁-₁₈ alkyl, C₂-₁₈ alkenyl, -R*YR", -YR", and H;
Each R" is independently selected from the group consisting of C₃-₁₄ alkyl and C₃-₁₄ alkenyl;
Each R* is independently selected from the group consisting of C₁-₁₂ alkyl and C₂-₁₂ alkenyl;
Each Y is independently C₃-₆ carbocycle;
1 is selected from 1, 2, 3, 4, and 5
m is selected from 5, 6, 7. 8, and 9;
M₁ is a bond of M'; and
M and M' are independently selected from -C(O)O-, -OC(O)-, - C(O)N(R')-, -P(O)(OR')O-, -S-S-, an aryl group, and a heteroaryl group.

In a specific embodiment, the lipid compound has the following structure:

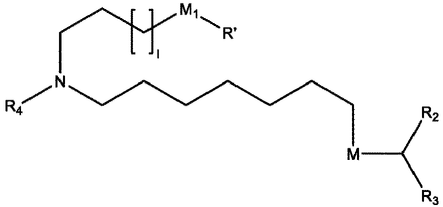

In a specific embodiment, the lipid compound has formula:

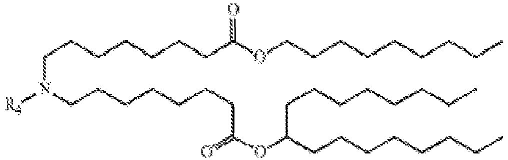

In one embodiment, the lipid compound has formula:

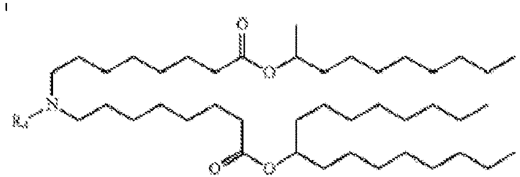

In another embodiment, the lipid compound has formula:

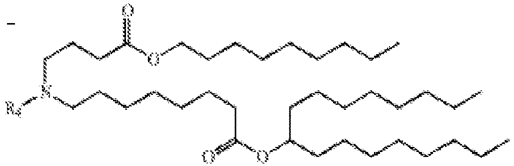

In a specific embodiment, the lipid compound has formula:

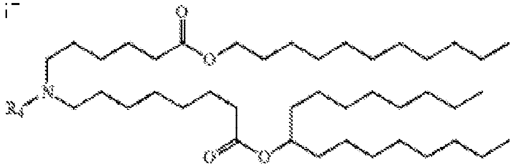

In a certain embodiment, the lipid compound has formula, wherein R₄ is selected from the group consisting of -(CH₂)ₙQ, -(CH₂)ₙCHQR, -CHQR, and -CQ(R)₂, where Q is -N(R)R₈.

In some embodiments, M and M' are independently -C(O)O- or -OC(O)-

In other embodiments, R₄ is selected from any of the following groups:

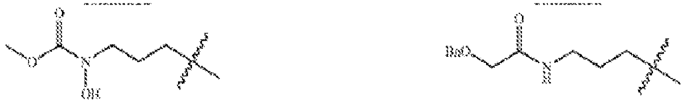

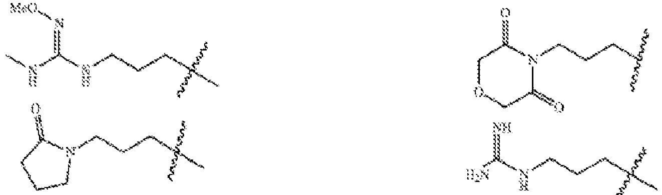

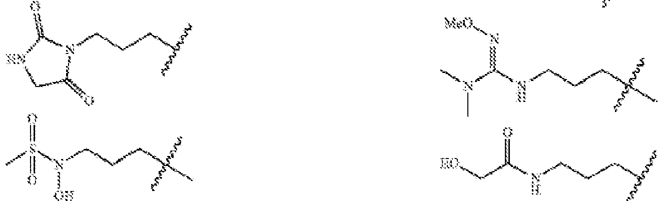

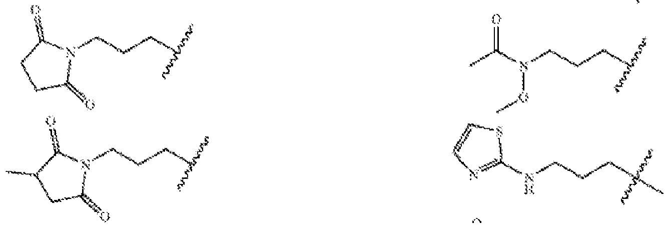

In other embodiments, R₄ is selected from any of the following groups:

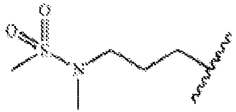

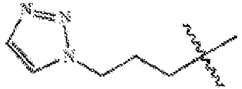

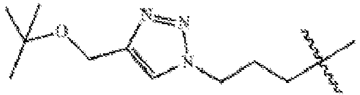

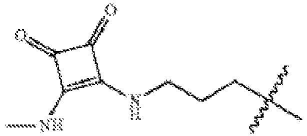

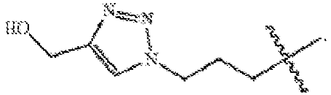

In other embodiments, the cationic lipid is a lipid as disclosed in WO 2020/0061367. For example, in some aspects of the disclosure, the cationic lipids described herein are of Formula (I): 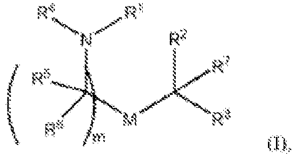 or their N-oxides, or salts or isomers thereof, wherein:
R1 is selected from the group consisting of C5-30 alkyl, C5-20 alkenyl, - R*YR", -YR", and -R'M'R';
R2 and R3 are independently selected from the group consisting of H, C1-14 alkyl, C2-14 alkenyl, -R*YR", -YR", and -R*OR", or R2 and R3, together with the atom to which they are attached, form a heterocycle or carbocycle;
R4 is selected from the group consisting of hydrogen, a C3-6 carbocycle, -(CH2)nQ, -(CH2)nCHQR, -(CH2)oC(R10)2(CH2)n-oQ, -CHQR, -CQ(R)2, -C(0)NQR and unsubstituted Ci-e alkyl, where Q is selected from a carbocycle, heterocycle, -OR, - 0(CH2)nN(R)2, -C(0)OR, -OC(0)R, -CX3, -CX2H, -CXH2, -CN, -N(R)2, -C(0)N(R)2, - N(R)C(0)R, -N(R)S(0)2R, -N(R)C(0)N(R)2, -N(R)C(S)N(R)2, -N(R)R8, -N(R)S(0)2R8, -0(CH2)nOR, -N(R)C(=NR9)N(R)2, -N(R)C(=CHR9)N(R)2, -OC(0)N(R)2, - N(R)C(0)OR, -N(OR)C(0)R, -N(OR)S(0)2R, -N(OR)C(0)OR, -N(OR)C(0)N(R)2, - N(OR)C(S)N(R)2, -N(OR)C(=NR9)N(R)2, -N(OR)C(=CHR9)N(R)2, -C(=NR9)N(R)2, - C(=NR9)R, -C(0)N(R)OR, -(CH2)nN(R)2 and -C(R)N(R)2C(0)OR, each 0 is independently selected from 1, 2, 3, and 4, and each n is independently selected from 1, 2, 3, 4, and 5;
each R5 is independently selected from the group consisting of OH, C1-3 alkyl, C2-3 alkenyl, and H;
each R6 is independently selected from the group consisting of OH, C1-3 alkyl, C2-3 alkenyl, and H;
M and M' are independently selected from -C(0)0-, -OC(O)-, -0C(0)-M"-C(0)0-, -C(0)N(R ,
-N(R')C(0)-, -C(O)-, -C(S)-, -C(S)S-, -SC(S)-, -CH(OH)-, -P(0)(0R')0-, - S(0)2-, -S-S-, an aryl group, and a heteroaryl group, in which M" is a bond, C1-13 alkyl or C2-13 alkenyl;
R7 is selected from the group consisting of C1-3 alkyl, C2-3 alkenyl, and H;
R8 is selected from the group consisting of C3-6 carbocycle and heterocycle;
R9 is selected from the group consisting of H, CN, NO2, C1-6 alkyl, -OR, -S(0)2R, -S(0)2N(R)2, C2-6 alkenyl, C3-6 carbocycle and heterocycle;
R10 is selected from the group consisting of H, OH, C1-3 alkyl, and C2-3 alkenyl;
each R is independently selected from the group consisting of C1-6 alkyl, C1-3 alkyl-aryl, C2-3 alkenyl, (CH2)qOR*, and H,
and each q is independently selected from 1, 2, and 3;
each R' is independently selected from the group consisting of C1-18 alkyl, C2-18 alkenyl, -R*YR", -YR", and H;
each R" is independently selected from the group consisting of C3-15 alkyl and
C3-15 alkenyl;
each R* is independently selected from the group consisting of C₁-₁₂ alkyl and
C2-12 alkenyl;
each Y is independently a C3-6 carbocycle;
each X is independently selected from the group consisting of F, Cl, Br, and I; and m is selected from 5, 6, 7, 8, 9, 10, 11, 12, and 13; and wherein when R4 is - (CH2)nQ, -(CH2)nCHQR, -CHQR, or -CQ(R)2, then (i) Q is not -N(R)2 when n is 1, 2, 3, 4 or 5, or (ii) Q is not 5, 6, or 7-membered heterocycloalkyl when n is 1 or 2.

Other cationic lipids relate to a compound of Formula (III): 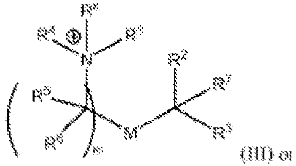 r its N-oxide, or a salt or isomer thereof, wherein
or a salt or isomer thereof, wherein
R1 is selected from the group consisting of C5-30 alkyl, C5-20 alkenyl, - R*YR", -YR", and -R'M'R';
R2 and R3 are independently selected from the group consisting of H, Ci-i4 alkyl, C2-14 alkenyl, -R*YR", -YR", and -R*OR", or R2 and R3, together with the atom to which they are attached, form a heterocycle or carbocycle;
R4 is selected from the group consisting of hydrogen, a C3-6 carbocycle, -(CH2)nQ, -(CH2)nCHQR, -(CH2)oC(R10)2(CH2)n-oQ, -CHQR, -CQ(R)2, -C(0)NQR and unsubstituted Ci-e alkyl, where Q is selected from a carbocycle, heterocycle, -OR, - 0(CH2)nN(R)2, -C(0)0R, -0C(0)R, -CX3, -CX2H, -CXH2, -CN, -N(R)2, -C(0)N(R)2, - N(R)C(0)R, -N(R)S(0)2R, -N(R)C(0)N(R)2, -N(R)C(S)N(R)2, -N(R)R8, -N(R)S(0)2R8, -0(CH2)n0R, -N(R)C(=NR9)N(R)2, -N(R)C(=CHR9)N(R)2, -0C(0)N(R)2, - N(R)C(0)0R, -N(OR)C(0)R, -N(0R)S(0)2R, -N(0R)C(0)0R, -N(0R)C(0)N(R)2, - N(OR)C(S)N(R)2, -N(OR)C(=NR9)N(R)2, -N(OR)C(=CHR9)N(R)2, -C(=NR9)N(R)2, - C(=NR9)R, -C(O)N(R)OR, -(CH2)nN(R)2 and -C(R)N(R)2C(0)0R, each 0 is independently selected from 1, 2, 3, and 4, and each n is independently selected from 1, 2, 3, 4, and 5;
Rx is selected from the group consisting of C1-6 alkyl, C2-6 alkenyl, - (CH2)vOH, and -(CH2)VN(R)2,
wherein v is selected from 1, 2, 3, 4, 5, and 6;
each R5 is independently selected from the group consisting of OH, C1-3 alkyl, C2-3 alkenyl, and H;
each R6 is independently selected from the group consisting of OH, C1-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C 1-13 alkyl or C2-i3 alkenyl;
R7 is selected from the group consisting of C1-3 alkyl, C2-3 alkenyl, and H;
R8 is selected from the group consisting of C3-6 carbocycle and heterocycle;
R9 is selected from the group consisting of H, CN, N02, C1-6 alkyl, -OR, -S(0)2R, -S(0)2N(R)2, C2-6 alkenyl, C3-6 carbocycle and heterocycle;
R10 is selected from the group consisting of H, OH, C1-3 alkyl, and C2-3 alkenyl;
each R is independently selected from the group consisting of C1-6 alkyl, C1-3 alkyl-aryl, C2-3 alkenyl, (CH2)qOR*. and H,
and each q is independently selected from 1, 2, and 3;
each R' is independently selected from the group consisting of C1-18 alkyl, C2-i8 alkenyl, -R*YR", -YR", and H;
each R" is independently selected from the group consisting of C3-15 alkyl and
C3-15 alkenyl;
each R* is independently selected from the group consisting of C₁-₁₂ alkyl and
C2-12 alkenyl;
each Y is independently a C3-6 carbocycle;
each X is independently selected from the group consisting of F, Cl, Br, and I; and m is selected from 5, 6, 7, 8, 9, 10, 11, 12, and 13.

Other aspects the disclosure relate to a compound of Formula (I), wherein R4 is selected from the group consisting -(CH2)nQ, -(CH2)nCHQR, - (CH2)oC(R12)2(CH2)n-oQ, -CHQR, -CQ(R)2, and -C(0)NQR, where Q is - (CH2)nN(R)2.

Other aspects the disclosure relate to a compound of Formula (III), wherein R4 is selected from the group consisting -(CH2)nQ, -(CH2)nCHQR, - (CH2)oC(R12)2(CH2)n-oQ, -CHQR, -CQ(R)2, and -C(0)NQR, where Q is - (CH2)nN(R)2.

In some embodiments, a subset of compounds of Formula (I) includes those in which when R4 is -(CH2)nQ, -(CH2)nCHQR, -CHQR, or -CQ(R)2, then (i) Q is not -N(R)2 when n is 1, 2, 3, 4 or 5, or (ii) Q is not 5, 6, or 7-membered heterocycloalkyl when n is 1 or 2.

For example, when R4 is -(CH2)nQ, -(CH2)nCHQR, - (CH2)oC(R10)2(CH2)n-oQ, -CHQR, or -CQ(R)2, then (i) Q is not -N(R)2 when n is 1, 2, 3, 4 or 5, or (ii) Q is not 5, 6, or 7-membered heterocycloalkyl when n is 1 or 2.

In another embodiments, another subset of compounds of Formula (I) includes those in which
R1 is selected from the group consisting of C5-30 alkyl, C5-20 alkenyl, - R*YR", -YR", and -R'M'R';
R2 and R3 are independently selected from the group consisting of H, C1-14 alkyl, C2-14 alkenyl, -R*YR", -YR", and -R*OR", or R2 and R3, together with the atom to which they are attached, form a heterocycle or carbocycle;
R4 is selected from the group consisting of hydrogen, a C3-6 carbocycle, -(CH2)nQ, -(CH2)nCHQR, -(CH2)oC(R10)2(CH2)n-oQ,-CHQR, -CQ(R)2, -C(0)NQR and unsubstituted Ci-e alkyl, where Q is selected from a C3-6 carbocycle, a 5- to 14-membered heteroaryl having one or more heteroatoms selected from N, O, and S, -OR, - 0(CH2)nN(R)2, -C(0)OR, -OC(0)R, -CX3, -CX2H, -CXH2, -CN, -C(0)N(R)2, - N(R)C(0)R, -N(R)S(0)2R, -N(R)C(0)N(R)2, -N(R)C(S)N(R)2, -CRN(R)2C(0)OR, - N(R)R8, -N(R)S(0)2R8, -0(CH2)nOR, -N(R)C(=NR9)N(R)2, -N(R)C(=CHR9)N(R)2, - OC(0)N(R)2, -N(R)C(0)OR, -N(OR)C(0)R, -N(OR)S(0)2R, -N(OR)C(0)OR, - N(OR)C(0)N(R)2, -N(OR)C(S)N(R)2, -N(OR)C(=NR9)N(R)2, -N(0R)C(=CHR9)N(R)2, -C(=NR9)N(R)2, -C(=NR9)R, -C(0)N(R)OR, -(CH2)nN(R)2, and a 5- to
14-membered heterocycloalkyl having one or more heteroatoms selected fromN, O, and S which is substituted with one or more substituents selected from oxo (=0), OH, amino, mono- or di-alkylamino, and Ci-3 alkyl, each o is independently selected from 1, 2, 3, and 4, and each n is independently selected from 1, 2, 3, 4, and 5;
each R5 is independently selected from the group consisting of OH, Ci-3 alkyl, C2-3 alkenyl, and H;
each R6 is independently selected from the group consisting of OH, C1-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C1-13 alkyl or C2-13 alkenyl;
R7 is selected from the group consisting of C1-3 alkyl, C2-3 alkenyl, and H;
R8 is selected from the group consisting of C3-6 carbocycle and heterocycle;
R9 is selected from the group consisting of H, CN, NO2, C1-6 alkyl, -OR, -S(0)2R, -S(0)2N(R)2, C2-6 alkenyl, C3-6 carbocycle and heterocycle;
R10 is selected from the group consisting of H, OH, C 1-3 alkyl, and C2-3 alkenyl;
each R is independently selected from the group consisting of C1-6 alkyl, C 1-3 alkyl-aryl, C2-3 alkenyl, (CH2)qOR*, and H;
each R' is independently selected from the group consisting of Ci-ib alkyl, C2-18 alkenyl, -R*YR", -YR", and H,
and each q is independently selected from 1, 2, and 3;
each R" is independently selected from the group consisting of C3-15 alkyl and C3-15 alkenyl;
each R* is independently selected from the group consisting of C1-12 alkyl and C2-12 alkenyl;
each Y is independently a C3-6 carbocycle;
each X is independently selected from the group consisting of F, Cl, Br, and I; and m is selected from 5, 6, 7, 8, 9, 10, 11, 12, and 13,
or their N-oxides, or salts or isomers thereof.

In yet another embodiments, another subset of compounds of Formula (I) includes those in which
R1 is selected from the group consisting of C5-30 alkyl, C5-20 alkenyl, - R*YR", -YR", and -R'M'R';
R2 and R3 are independently selected from the group consisting of H, Ci-i4 alkyl, C2-14 alkenyl, -R*YR", -YR", and -R*OR", or R2 and R3, together with the atom to which they are attached, form a heterocycle or carbocycle;
R4 is selected from the group consisting of hydrogen, a C3-6 carbocycle, -(CH2)nQ, -(CH2)nCHQR, -(CH2)oC(R10)2(CH2)n-oQ,-CHQR, -CQ(R)2, -C(0)NQR and unsubstituted Ci-e alkyl, where Q is selected from a C3-6 carbocycle, a 5- to 14-membered heterocycle having one or more heteroatoms selected from N, O, and S, -OR, -0(CH2)nN(R)2, -C(0)0R, -0C(0)R, -CX3, -CX2H, -CXH2, -CN, -C(0)N(R)2, - N(R)C(0)R, -N(R)S(0)2R, -N(R)C(0)N(R)2, -N(R)C(S)N(R)2, -CRN(R)2C(0)0R, - N(R)R8, -N(R)S(0)2R8, -0(CH2)n0R, -N(R)C(=NR9)N(R)2, -N(R)C(=CHR9)N(R)2, - 0C(0)N(R)2, -N(R)C(0)0R, -N(0R)C(0)R, -N(OR)S(0)2R, -N(0R)C(0)0R, - N(0R)C(0)N(R)2, -N(OR)C(S)N(R)2, -N(OR)C(=NR9)N(R)2, -N(OR)C(=CHR9)N(R)2, -C(=NR9)R, -C(0)N(R)0R, -(CH2)nN(R)2 and -C(=NR9)N(R)2, each 0 is independently selected from 1, 2, 3, and 4, and each n is independently selected from 1, 2, 3, 4, and 5; and when Q is a 5- to 14-membered heterocycle and (i) R4 is -(CH2)nQ in which n is 1 or 2, or (ii) R4 is -(CH2)nCHQR in which n is 1, or (iii) R4 is -CHQR, and -CQ(R)2, then Q is either a 5- to 14-membered heteroaryl or 8- to 14-membered heterocycloalkyl;
each R5 is independently selected from the group consisting of OH, C1-3 alkyl, C2-3 alkenyl, and H;
each R6 is independently selected from the group consisting of OH, C1-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C1-13 alkyl or C2-i3 alkenyl;
R7 is selected from the group consisting of C1-3 alkyl, C2-3 alkenyl, and H;
R8 is selected from the group consisting of C3-6 carbocycle and heterocycle;
R9 is selected from the group consisting of H, CN, N02, C1-6 alkyl, -OR, -S(0)2R, -S(0)2N(R)2, C2-6 alkenyl, C3-6 carbocycle and heterocycle;
R10 is selected from the group consisting of H, OH, C1-3 alkyl, and C2-3 alkenyl;
each R is independently selected from the group consisting of C1-6 alkyl, C1-3 alkyl-aryl, C2-3 alkenyl, (CH2)qOR*. and H,
and each q is independently selected from 1, 2, and 3;
each R' is independently selected from the group consisting of C1-18 alkyl, C2-i8 alkenyl, -R*YR", -YR", and H;
each R" is independently selected from the group consisting of C3-15 alkyl and C3-15 alkenyl;
each R* is independently selected from the group consisting of C1-12 alkyl and C2-12 alkenyl;
each Y is independently a C3-6 carbocycle;
each X is independently selected from the group consisting of F, Cl, Br, and I; and m is selected from 5, 6, 7, 8, 9, 10, 11, 12, and 13,
or their N-oxides, or salts or isomers thereof.

In still another embodiments, another subset of compounds of Formula (I) includes those in which
R1 is selected from the group consisting of C5-30 alkyl, C5-20 alkenyl, - R*YR", -YR", and -R'M'R';
R2 and R3 are independently selected from the group consisting of H, C1-14 alkyl, C2-14 alkenyl, -R*YR", -YR", and -R*OR", or R2 and R3, together with the atom to which they are attached, form a heterocycle or carbocycle;
R4 is selected from the group consisting of hydrogen, a C3-6 carbocycle, -(CH2)nQ, -(CH2)nCHQR, -(CH2)oC(R10)2(CH2)n-oQ,-CHQR, -CQ(R)2, -C(0)NQR and unsubstituted Ci-e alkyl, where Q is selected from a C3-6 carbocycle, a 5- to 14-membered heteroaryl having one or more heteroatoms selected from N, O, and S, -OR, - 0(CH2)nN(R)2, -C(0)OR, -OC(0)R, -CX3, -CX2H, -CXH2, -CN, -C(0)N(R)2, - N(R)C(0)R, -N(R)S(0)2R, -N(R)C(0)N(R)2, -N(R)C(S)N(R)2, -CRN(R)2C(0)OR, - N(R)R8, -N(R)S(0)2R8, -0(CH2)nOR, -N(R)C(=NR9)N(R)2, -N(R)C(=CHR9)N(R)2, - OC(0)N(R)2, -N(R)C(0)OR, -N(OR)C(0)R, -N(OR)S(0)2R, -N(OR)C(0)OR, - N(OR)C(0)N(R)2, -N(OR)C(S)N(R)2, -N(OR)C(=NR9)N(R)2, -N(0R)C(=CHR9)N(R)2, -C(=NR9)R, -C(0)N(R)OR, -(CH2)nN(R)2, each 0 is independently selected from 1, 2, 3, and 4, and -C(=NR9)N(R)2, and each n is independently selected from 1, 2, 3, 4, and 5;
each R5 is independently selected from the group consisting of OH, C1-3 alkyl, C2-3 alkenyl, and H;
each R6 is independently selected from the group consisting of OH, C1-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C1-13 alkyl or C2-13 alkenyl;
R7 is selected from the group consisting of C1-3 alkyl, C2-3 alkenyl, and H;
R8 is selected from the group consisting of C3-6 carbocycle and heterocycle;
R9 is selected from the group consisting of H, CN, NO2, C1-6 alkyl, -OR, -S(0)2R, -S(0)2N(R)2, C2-6 alkenyl, C3-6 carbocycle and heterocycle;
R10 is selected from the group consisting of H, OH, C1-3 alkyl, and C2-3 alkenyl, each R is independently selected from the group consisting of C 1 -6 alkyl, C1-3 alkyl-aryl, C2-3 alkenyl, (CH2)qOR*, and H,
and each q is independently selected from 1, 2, and 3;
each R' is independently selected from the group consisting of C1-18 alkyl, C2-18 alkenyl, -R*YR", -YR", and H;
each R" is independently selected from the group consisting of C3-15 alkyl and C3-15 alkenyl;
each R* is independently selected from the group consisting of C1-12 alkyl and C2-12 alkenyl;
each Y is independently a C3-6 carbocycle;
each X is independently selected from the group consisting of F, Cl, Br, and I; and m is selected from 5, 6, 7, 8, 9, 10, 11, 12, and 13,
or their N-oxides, or salts or isomers thereof.

In still another embodiments, another subset of compounds of Formula (I) includes those in which
R1 is selected from the group consisting of C5-30 alkyl, C5-20 alkenyl, - R*YR", -YR", and -R"M'R';
R2 and R3 are independently selected from the group consisting of H, C1-14 alkyl, C2-14 alkenyl, -R*YR", -YR", and -R*OR", or R2 and R3, together with the atom to which they are attached, form a heterocycle or carbocycle;
R4 is selected from the group consisting of hydrogen, a C3-6 carbocycle, -(CH2)nQ, -(CH2)nCHQR, -(CH2)oC(R10)2(CH2)n-oQ, -CHQR, -CQ(R)2, -C(0)NQR and unsubstituted Ci-e alkyl, where Q is selected from a carbocycle, -OR, - 0(CH2)nN(R)2, -C(0)OR, -OC(0)R, -CX3, -CX2H, -CXH2, -CN, -C(0)N(R)2, - N(R)C(0)R, -N(R)S(0)2R, -N(R)C(0)N(R)2, -N(R)C(S)N(R)2, -N(R)R8, -N(R)S(0)2R8, -0(CH2)nOR, -N(R)C(=NR9)N(R)2, -N(R)C(=CHR9)N(R)2, -OC(0)N(R)2, - N(R)C(0)OR, -N(OR)C(0)R, -N(OR)S(0)2R, -N(OR)C(0)OR, -N(OR)C(0)N(R)2, - N(OR)C(S)N(R)2, -N(OR)C(=NR9)N(R)2, -N(OR)C(=CHR9)N(R)2, -C(=NR9)N(R)2, - C(=NR9)R, -C(0)N(R)OR, -(CH2)nN(R)2 and -C(R)N(R)2C(0)OR, each 0 is independently selected from 1, 2, 3, and 4, and each n is independently selected from 1, 2, 3, 4, and 5;
each R5 is independently selected from the group consisting of OH,CI-3 alkyl, C2-3 alkenyl, and H;
each R6 is independently selected from the group consisting of OH,CI-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C1-13 alkyl or C2-13 alkenyl;
R7 is selected from the group consisting of C1-3 alkyl, C2-3 alkenyl, and H;
R8 is selected from the group consisting of C3-6 carbocycle and heterocycle;
R9 is selected from the group consisting of H, CN, NO2, C1-6 alkyl, -OR, -S(0)2R, -S(0)2N(R)2, C2-6 alkenyl, C3-6 carbocycle and heterocycle;
R10 is selected from the group consisting of H, OH, C1-3 alkyl, and C2-3 alkenyl;
each R is independently selected from the group consisting of C1-6 alkyl, C1-3 alkyl-aryl, C2-3 alkenyl, and H;
each R' is independently selected from the group consisting of C1-18 alkyl, C2-18 alkenyl, -R*YR", -YR", (CH2)qOR*. and H,
and each q is independently selected from 1, 2, and 3;
each R" is independently selected from the group consisting of C3-15 alkyl and
C3-15 alkenyl;
each R* is independently selected from the group consisting of C1-12 alkyl and
C2-12 alkenyl;
each Y is independently a C3-6 carbocycle;
each X is independently selected from the group consisting of F, Cl, Br, and I; and m is selected from 5, 6, 7, 8, 9, 10, 11, 12, and 13.
In yet another embodiments, another subset of compounds of Formula (I) includes those in which
R1 is selected from the group consisting of C5-30 alkyl, C5-20 alkenyl, - R*YR", -YR", and -R"M'R';
R2 and R3 are independently selected from the group consisting of H, C2-14 alkyl, C2-14 alkenyl, -R*YR", -YR", and -R*OR", or R2 and R3, together with the atom to which they are attached, form a heterocycle or carbocycle;
R4 is -(CH2)nQ or -(CH2)nCHQR, where Q is -N(R)2, and n is selected from 3, 4, and 5; each R5 is independently selected from the group consisting of C1-3 alkyl, C2-3 alkenyl, and H;
each R6 is independently selected from the group consisting of C1-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C1-13 alkyl or C2-13 alkenyl;
R7 is selected from the group consisting of C1-3 alkyl, C2-3 alkenyl, and H;
each R is independently selected from the group consisting of C1-6 alkyl, C1-3 alkyl-aryl, C2-3 alkenyl, and H;
each R' is independently selected from the group consisting of C1-18 alkyl, C2-18 alkenyl, -R*YR", -YR", and H;
each R" is independently selected from the group consisting of C3-15 alkyl and C3-15 alkenyl;
each R* is independently selected from the group consisting of C1-12 alkyl and C1-12 alkenyl;
each Y is independently a C3-6 carbocycle;
each X is independently selected from the group consisting of F, Cl, Br, and I; and m is selected from 5, 6, 7, 8, 9, 10, 11, 12, and 13,
or their N-oxides, or salts or isomers thereof.

In still another embodiment, another subset of compounds of Formula (I) includes those in which
R1 is selected from the group consisting of C5-30 alkyl, C5-20 alkenyl, - R*YR", -YR", and -R"M'R';
R2 and R3 are independently selected from the group consisting of C1-14 alkyl, C2-14 alkenyl, -R*YR", -YR", and -R*OR", or R2 and R3, together with the atom to which they are attached, form a heterocycle or carbocycle;
R4 is selected from the group consisting of -(CH2)nQ, -(CH2)nCHQR, - CHQR, and -CQ(R)2, where Q is -N(R)2, and n is selected from 1, 2, 3, 4, and 5;
each R5 is independently selected from the group consisting of C1-3 alkyl, C2-3 alkenyl, and H;
each R6 is independently selected from the group consisting of C1-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C1-13 alkyl or C2-13 alkenyl;
R7 is selected from the group consisting of C1-3 alkyl, C2-3 alkenyl, and H;
each R is independently selected from the group consisting of C1-6 alkyl, C 1-3 alkyl-aryl, C2-3 alkenyl, and H;
each R' is independently selected from the group consisting of C1-18 alkyl, C2-18 alkenyl, -R*YR", -YR", and H;
each R" is independently selected from the group consisting of C3-15 alkyl and C3-15 alkenyl;
each R* is independently selected from the group consisting of C1-12 alkyl and C1-12 alkenyl;
each Y is independently a C3-6 carbocycle;
each X is independently selected from the group consisting of F, Cl, Br, and I; and m is selected from 5, 6, 7, 8, 9, 10, 11, 12, and 13,
or their N-oxides, or salts or isomers thereof.

In still another embodiment, another subset of compounds of Formula (I) includes those in which
R1 is selected from the group consisting of C5-30 alkyl, C5-20 alkenyl, - R*YR", -YR", and -R"M'R';
R2 and R3 are independently selected from the group consisting of H, C1-14 alkyl, C2-14 alkenyl, -R*YR", -YR", and -R*OR", or R2 and R3, together with the atom to which they are attached, form a heterocycle or carbocycle;
R4 is -C(0)NQR, where Q is selected from a carbocycle, heterocycle, - C(0)OR, -OC(0)R, -CX3, -CX2H, -CXH2, -CN, -C(0)N(R)2, -(CH2)nN(R)2, - C(=NR9)N(R)2, -C(=NR9)R, -C(0)N(R)OR, and -C(R)N(R)2C(0)OR, and each n is independently selected from 1, 2, 3, 4, and 5;
each R5 is independently selected from the group consisting of OH, C1-3 alkyl, C2-3 alkenyl, and H;
each R6 is independently selected from the group consisting of OH, C1-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C1-13 alkyl or C2-13 alkenyl;
R7 is selected from the group consisting of C1-3 alkyl, C2-3 alkenyl, and H;
R9 is selected from the group consisting of H, CN, NO2, C1-6 alkyl, -OR, -S(0)2R, -S(0)2N(R)2, C2-6 alkenyl, C3-6 carbocycle and heterocycle;
each R is independently selected from the group consisting of Ci-6 alkyl, C1-3 alkyl-aryl, C2-3 alkenyl, and H;
each R' is independently selected from the group consisting of C1-18 alkyl, C2-18 alkenyl, -R*YR", -YR", (CH2)qOR*, and H, and each q is independently selected from 1, 2, and 3;
each R" is independently selected from the group consisting of C3-15 alkyl and
C3-15 alkenyl;
each R* is independently selected from the group consisting of C1-12 alkyl and
C2-12 alkenyl;
each Y is independently a C3-6 carbocycle;
each X is independently selected from the group consisting of F, Cl, Br, and I; and m is selected from 5, 6, 7, 8, 9, 10, 11, 12, and 13.

In some embodiments, a subset of compounds of Formula (III) includes those in which, when R4 is -(CH2)nQ, -(CH2)nCHQR, -CHQR, or -CQ(R)2, then (i) Q is not -N(R)2 when n is 1, 2, 3, 4 or 5, or (ii) Q is not 5, 6, or 7-membered heterocycloalkyl when n is 1 or 2.

In another embodiments, another subset of compounds of Formula (III) includes those in which
R1 is selected from the group consisting of C5-30 alkyl, C5-20 alkenyl, - R*YR", -YR", and -R"M'R';
R2 and R3 are independently selected from the group consisting of H, C1-14 alkyl, C2-14 alkenyl, -R*YR", -YR", and -R*OR", or R2 and R3, together with the atom to which they are attached, form a heterocycle or carbocycle;
R4 is selected from the group consisting of hydrogen, a C3-6 carbocycle, -(CH2)nQ, -(CH2)nCHQR, -(CH2)oC(R10)2(CH2)n-oQ,-CHQR, -CQ(R)2, -C(0)NQR and unsubstituted Ci-e alkyl, where Q is selected from a C3-6 carbocycle, a 5- to 14-membered heteroaryl having one or more heteroatoms selected from N, O, and S, -OR, - 0(CH2)nN(R)2, -C(0)OR, -OC(0)R, -CX3, -CX2H, -CXH2, -CN, -C(0)N(R)2, - N(R)C(0)R, -N(R)S(0)2R, -N(R)C(0)N(R)2, -N(R)C(S)N(R)2, -CRN(R)2C(0)OR, - N(R)R8, -N(R)S(0)2R8, -0(CH2)nOR, -N(R)C(=NR9)N(R)2, -N(R)C(=CHR9)N(R)2, - OC(0)N(R)2, -N(R)C(0)OR, -N(OR)C(0)R, -N(OR)S(0)2R, -N(OR)C(0)OR, - N(OR)C(0)N(R)2, -N(OR)C(S)N(R)2, -N(OR)C(=NR9)N(R)2, -N(0R)C(=CHR9)N(R)2, -C(=NR9)N(R)2, -C(=NR9)R, -C(0)N(R)OR, -(CH2)nN(R)2 and a 5- to 14-membered heterocycloalkyl having one or more heteroatoms selected fromN, O, and S which is substituted with one or more substituents selected from oxo (=0), OH, amino, mono- or di-alkylamino, and C1-3 alkyl, each 0 is independently selected from 1, 2, 3, and 4, and each n is independently selected from 1, 2, 3, 4, and 5;
Rx is selected from the group consisting of Ci-6 alkyl, C2-6 alkenyl, - (CfkXOH, and -(CH2)VN(R)2,
wherein v is selected from 1, 2, 3, 4, 5, and 6;
each R5 is independently selected from the group consisting of OH, C1-3 alkyl, C2-3 alkenyl, and H;
each R6 is independently selected from the group consisting of OH, C1-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C1-13 alkyl or C2-13 alkenyl;
R7 is selected from the group consisting of C1-3 alkyl, C2-3 alkenyl, and H;
R8 is selected from the group consisting of C3-6 carbocycle and heterocycle;
R9 is selected from the group consisting of H, CN, NO2, C1-6 alkyl, -OR, -S(0)2R, -S(0)2N(R)2, C2-6 alkenyl, C3-6 carbocycle and heterocycle;
R10 is selected from the group consisting of H, OH, C1-3 alkyl, and C2-3 alkenyl;
each R is independently selected from the group consisting of C1-6 alkyl, C1-3 alkyl-aryl, C2-3 alkenyl, (CH2)qOR*, and H;
each R' is independently selected from the group consisting of C1-18 alkyl, C2-18 alkenyl, -R*YR", -YR", and H,
and each q is independently selected from 1, 2, and 3;
each R" is independently selected from the group consisting of C3-15 alkyl and C3-15 alkenyl;
each R* is independently selected from the group consisting of C1-12 alkyl and C2-12 alkenyl;
each Y is independently a C3-6 carbocycle;
each X is independently selected from the group consisting of F, Cl, Br, and I; and m is selected from 5, 6, 7, 8, 9, 10, 11, 12, and 13,
or their N-oxides, or salts or isomers thereof.

In yet another embodiments, another subset of compounds of Formula (III) includes those in which
R1 is selected from the group consisting of C5-30 alkyl, C5-20 alkenyl, - R*YR", -YR", and -R'M'R';
R2 and R3 are independently selected from the group consisting of H, Ci-i4 alkyl, C2-14 alkenyl, -R*YR", -YR", and -R*OR", or R2 and R3, together with the atom to which they are attached, form a heterocycle or carbocycle;
R4 is selected from the group consisting of hydrogen, a C3-6 carbocycle, -(CH2)nQ, -(CH2)nCHQR, -(CH2)oC(R12)2(CH2)n-oQ,-CHQR, -CQ(R)2, -C(0)NQR and unsubstituted Ci-e alkyl, where Q is selected from a C3-6 carbocycle, a 5- to 14-membered heterocycle having one or more heteroatoms selected from N, O, and S, -OR, -0(CH2)nN(R)2, -C(0)0R, -0C(0)R, -CX3, -CX2H, -CXH2, -CN, -C(0)N(R)2, - N(R)C(0)R, -N(R)S(0)2R, -N(R)C(0)N(R)2, -N(R)C(S)N(R)2, -CRN(R)2C(0)0R, - N(R)R8, -N(R)S(0)2R8, -0(CH2)n0R, -N(R)C(=NR9)N(R)2, -N(R)C(=CHR9)N(R)2, - 0C(0)N(R)2, -N(R)C(0)0R, -N(OR)C(0)R, -N(OR)S(0)2R, -N(0R)C(0)0R, - N(0R)C(0)N(R)2, -N(OR)C(S)N(R)2, -N(OR)C(=NR9)N(R)2, -N(OR)C(=CHR9)N(R)2, -C(=NR9)R, -C(0)N(R)OR, -(CH2)nN(R)2 and -C(=NR9)N(R)2, each 0 is independently selected from 1, 2, 3, and 4, and each n is independently selected from 1, 2, 3,
4, and 5; and when Q is a 5- to 14-membered heterocycle and (i) R4 is - (CH2)nQ in which n is 1 or 2, or (ii) R4 is -(CH2)nCHQR in which n is 1, or (iii) R4 is - CHQR, and -CQ(R)2, then Q is either a 5- to 14-membered heteroaryl or 8- to 14-membered heterocycloalkyl;
Rx is selected from the group consisting of Ci-6 alkyl, C2-6 alkenyl, - (CH2)vOH, and -(CH2)VN(R)2,
wherein v is selected from 1, 2, 3, 4, 5, and 6;
each R5 is independently selected from the group consisting of OH, C1-3 alkyl, C2-3 alkenyl, and H;
each R6 is independently selected from the group consisting of OH, C1-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C1-13 alkyl or C2-i3 alkenyl;
R7 is selected from the group consisting of C1-3 alkyl, C2-3 alkenyl, and H;
R8 is selected from the group consisting of C3-6 carbocycle and heterocycle;
R9 is selected from the group consisting of H, CN, N02, C1-6 alkyl, -OR, -S(0)2R, -S(0)2N(R)2, C2-6 alkenyl, C3-6 carbocycle and heterocycle;
R12 is selected from the group consisting of H, OH, C1-3 alkyl, and C2-3 alkenyl;
each R is independently selected from the group consisting of C1-6 alkyl, C1-3 alkyl-aryl, C2-3 alkenyl, (CH2)qOR*. and H,
and each q is independently selected from 1, 2, and 3;
each R' is independently selected from the group consisting of Ci-ib alkyl, C2-18 alkenyl, -R*YR", -YR", and H;
each R" is independently selected from the group consisting of C3-15 alkyl and C3-15 alkenyl;
each R* is independently selected from the group consisting of C1-12 alkyl and C2-12 alkenyl;
each Y is independently a C3-6 carbocycle;
each X is independently selected from the group consisting of F, Cl, Br, and I; and m is selected from 5, 6, 7, 8, 9, 10, 11, 12, and 13,
or their N-oxides, or salts or isomers thereof.

In still another embodiments, another subset of compounds of Formula (III) includes those in which
R1 is selected from the group consisting of C5-30 alkyl, C5-20 alkenyl, - R*YR", -YR", and -R"M'R';
R2 and R3 are independently selected from the group consisting of H, C1-14 alkyl, C2-14 alkenyl, -R*YR", -YR", and -R*OR", or R2 and R3, together with the atom to which they are attached, form a heterocycle or carbocycle;
R4 is selected from the group consisting of hydrogen, a C3-6 carbocycle, -(CH2)nQ, -(CH2)nCHQR, -(CH2)oC(R12)2(CH2)n-oQ,-CHQR, -CQ(R)2, -C(0)NQR and unsubstituted Ci-e alkyl, where Q is selected from a C3-6 carbocycle, a 5- to 14-membered heteroaryl having one or more heteroatoms selected from N, O, and S, -OR, - 0(CH2)nN(R)2, -C(0)OR, -OC(0)R, -CX3, -CX2H, -CXH2, -CN, -C(0)N(R)2, - N(R)C(0)R, -N(R)S(0)2R, -N(R)C(0)N(R)2, -N(R)C(S)N(R)2, -CRN(R)2C(0)OR, - N(R)R8, -N(R)S(0)2R8, -0(CH2)nOR, -N(R)C(=NR9)N(R)2, -N(R)C(=CHR9)N(R)2, - OC(0)N(R)2, -N(R)C(0)OR, -N(OR)C(0)R, -N(OR)S(0)2R, -N(OR)C(0)OR, - N(OR)C(0)N(R)2, -N(OR)C(S)N(R)2, -N(OR)C(=NR9)N(R)2, -N(0R)C(=CHR9)N(R)2, -C(=NR9)R, -C(0)N(R)OR, -(CH2)nN(R)2, each 0 is independently selected from 1, 2, 3, and 4, and -C(=NR9)N(R)2, each 0 is independently selected from 1, 2, 3, and 4, and each n is independently selected from 1, 2, 3, 4, and 5;
Rx is selected from the group consisting of C1-6 alkyl, C2-6 alkenyl, - (CH2)vOH, and -(CH2)VN(R)2,
wherein v is selected from 1, 2, 3, 4, 5, and 6;
each R5 is independently selected from the group consisting of OH, C 1-3 alkyl, C2-3 alkenyl, and H;
each R6 is independently selected from the group consisting of OH, C1-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C1-13 alkyl or C2-13 alkenyl;
R7 is selected from the group consisting of C1-3 alkyl, C2-3 alkenyl, and H;
R8 is selected from the group consisting of C3-6 carbocycle and heterocycle;
R9 is selected from the group consisting of H, CN, NO2, C1-6 alkyl, -OR, -S(0)2R, -S(0)2N(R)2, C2-6 alkenyl, C3-6 carbocycle and heterocycle;
R12 is selected from the group consisting of H, OH, C1-3 alkyl, and C2-3 alkenyl;
each R is independently selected from the group consisting of C1-6 alkyl, C1-3 alkyl-aryl, C2-3 alkenyl, (CH2)qOR*, and H,
and each q is independently selected from 1, 2, and 3;
each R' is independently selected from the group consisting of Ci-is alkyl, C2-18 alkenyl, -R*YR", -YR", and H;
each R" is independently selected from the group consisting of C3-15 alkyl and C3-15 alkenyl;
each R* is independently selected from the group consisting of C1-12 alkyl and C2-12 alkenyl;
each Y is independently a C3-6 carbocycle;
each X is independently selected from the group consisting of F, Cl, Br, and I; and m is selected from 5, 6, 7, 8, 9, 10, 11, 12, and 13,
or their N-oxides, or salts or isomers thereof.
In still another embodiments, another subset of compounds of Formula (III) includes those in which
R1 is selected from the group consisting of C5-30 alkyl, C5-20 alkenyl, - R*YR", -YR", and -R"M'R';
R2 and R3 are independently selected from the group consisting of H, C1-14 alkyl, C2-14 alkenyl, -R*YR", -YR", and -R*OR", or R2 and R3, together with the atom to which they are attached, form a heterocycle or carbocycle;
R4 is selected from the group consisting of hydrogen, a C3-6 carbocycle, -(CH2)nQ, -(CH2)nCHQR, -(CH2)oC(R12)2(CH2)n-oQ, -CHQR, -CQ(R)2, -C(0)NQR and unsubstituted Ci-e alkyl, where Q is selected from a carbocycle, -OR, - 0(CH2)nN(R)2, -C(0)OR, -OC(0)R, -CX3, -CX2H, -CXH2, -CN, -C(0)N(R)2, - N(R)C(0)R, -N(R)S(0)2R, -N(R)C(0)N(R)2, -
N(R)C(S)N(R)2, -N(R)R8, -N(R)S(0)2R8, -0(CH2)n0R, - N(R)C(=NR9)N(R)2, -N(R)C(=CHR9)N(R)2, -0C(0)N(R)2, -N(R)C(0)0R, - N(0R)C(0)R, -N(0R)S(0)2R, -N(0R)C(0)0R, -N(0R)C(0)N(R)2, -N(OR)C(S)N(R)2, - N(OR)C(=NR9)N(R)2, -N(OR)C(=CHR9)N(R)2, -C(=NR9)N(R)2, -C(=NR9)R, - C(0)N(R)0R, -(CH2)nN(R)2 and -C(R)N(R)2C(0)0R, each o is independently selected from 1, 2, 3, and 4, and each n is independently selected from 1, 2, 3, 4, and 5;
Rx is selected from the group consisting of Ci-6 alkyl, C2-6 alkenyl, - (CH2)vOH, and -(CH2)VN(R)2,
wherein v is selected from 1, 2, 3, 4, 5, and 6;
each R5 is independently selected from the group consisting of OH, Ci-3 alkyl, C2-3 alkenyl, and H;
each R6 is independently selected from the group consisting of OH, Ci-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C1-13 alkyl or C2-i3 alkenyl;
R7 is selected from the group consisting of C1-3 alkyl, C2-3 alkenyl, and H;
R8 is selected from the group consisting of C3-6 carbocycle and heterocycle;
R9 is selected from the group consisting of H, CN, N02, C1-6 alkyl, -OR, -S(0)2R, -S(0)2N(R)2, C2-6 alkenyl, C3-6 carbocycle and heterocycle;
R12 is selected from the group consisting of H, OH, C 1-3 alkyl, and C2-3 alkenyl;
each R is independently selected from the group consisting of C1-6 alkyl, C 1-3 alkyl-aryl, C2-3 alkenyl, and H;
each R' is independently selected from the group consisting of Ci-ib alkyl, C2-ie alkenyl, -R*YR", -YR", (CH2)qOR*, and H,
and each q is independently selected from 1, 2, and 3;
each R" is independently selected from the group consisting of C3-15 alkyl and
C3-15 alkenyl;
each R* is independently selected from the group consisting of Ci-i2 alkyl and
C2-i2 alkenyl;
each Y is independently a C3-6 carbocycle;
each X is independently selected from the group consisting of F, Cl, Br, and I; and m is selected from 5, 6, 7, 8, 9, 10, 11, 12, and 13.

In yet another embodiments, another subset of compounds of Formula (III) includes those in which
R1 is selected from the group consisting of C5-30 alkyl, C5-20 alkenyl, - R*YR", -YR", and -R"M'R';
R2 and R3 are independently selected from the group consisting of H, C2-14 alkyl, C2-14 alkenyl, -R*YR", -YR", and -R*OR", or R2 and R3, together with the atom to which they are attached, form a heterocycle or carbocycle;
R4 is -(CH2)nQ or -(CH2)nCHQR, where Q is -N(R)2, and n is selected from 3, 4, and 5; Rx is selected from the group consisting of Ci-6 alkyl, C2-6 alkenyl, - (CH2)vOH, and -(CH2)VN(R)2,
wherein v is selected from 1, 2, 3, 4, 5, and 6;
each R5 is independently selected from the group consisting of C1-3 alkyl, C2-3 alkenyl, and H;
each R6 is independently selected from the group consisting of C1-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C1-13 alkyl or C2-i3 alkenyl;
R7 is selected from the group consisting of C1-3 alkyl, C2-3 alkenyl, and H;
each R is independently selected from the group consisting of C1-6 alkyl, C1-3 alkyl-aryl, C2-3 alkenyl, and H;
each R' is independently selected from the group consisting of Ci-is alkyl, C2-i8 alkenyl, -R*YR", -YR", and H;
each R" is independently selected from the group consisting of C3-15 alkyl and C3-lsalkenyl;
each R* is independently selected from the group consisting of Ci-i2 alkyl and Ci-i2 alkenyl;
each Y is independently a C3-6 carbocycle;
each X is independently selected from the group consisting of F, Cl, Br, and I; and m is selected from 5, 6, 7, 8, 9, 10, 11, 12, and 13,
or their N-oxides, or salts or isomers thereof.
In still another embodiments, another subset of compounds of Formula (III) includes those in which
R1 is selected from the group consisting of C5-30 alkyl, C5-20 alkenyl, - R*YR", -YR", and -R"M'R';
R2 and R3 are independently selected from the group consisting of C1-14 alkyl, C2-14 alkenyl, -R*YR", -YR", and -R*OR", or R2 and R3, together with the atom to which they are attached, form a heterocycle or carbocycle;
R4 is selected from the group consisting of -(CH2)nQ, -(CH2)nCHQR, - CHQR, and -CQ(R)2, where Q is -N(R)2, and n is selected from 1, 2, 3, 4, and 5;
Rx is selected from the group consisting of Ci-6 alkyl, C2-6 alkenyl, - (CH2)vOH, and -(CH2)VN(R)2,
wherein v is selected from 1, 2, 3, 4, 5, and 6;
each R5 is independently selected from the group consisting of C1-3 alkyl, C2-3 alkenyl, and H;
each R6 is independently selected from the group consisting of C1-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C1-13 alkyl or C2-13 alkenyl;
R7 is selected from the group consisting of C1-3 alkyl, C2-3 alkenyl, and H;
each R is independently selected from the group consisting of C1-6 alkyl, C1-3 alkyl-aryl, C2-3 alkenyl, and H;
each R' is independently selected from the group consisting of C1-18 alkyl, C2-18 alkenyl, -R*YR", -YR", and H;
each R" is independently selected from the group consisting of C3-15 alkyl and C3-15 alkenyl;
each R* is independently selected from the group consisting of C 1-12 alkyl and C1-12 alkenyl;
each Y is independently a C3-6 carbocycle;
each X is independently selected from the group consisting of F, Cl, Br, and I; and m is selected from 5, 6, 7, 8, 9, 10, 11, 12, and 13,
or their N-oxides, or salts or isomers thereof.In certain embodiments, a subset of compounds of Formula (I) includes those of Formula (IA): 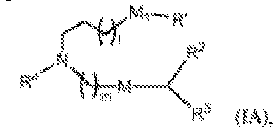
or its N-oxide, or a salt or isomer thereof, wherein 1 is selected from 1, 2, 3, 4, and 5; m is selected from 5, 6, 7, 8, and 9; Mi is a bond or M'; R4 is hydrogen, unsubstituted C1-3 alkyl, -(CH2)oC(R12)2(CH2)n-oQ, -C(0)NQR or -(CH2)nQ, in which Q is OH, -NHC(S)N(R)2, -NHC(0)N(R)2, -N(R)C(0)R, -N(R)S(0)2R, -N(R)R8, - NHC(=NR9)N(R)2, -NHC(=CHR9)N(R)2, -0C(0)N(R)2, -N(R)C(0)0R, -(CH2)nN(R)2, heteroaryl or heterocycloalkyl; M and M' are independently selected from -C(0)0-, - OC(O)-, -0C(0)-M"-C(0)0-, -C(0)N(R , -P(0)(0R')0-, -S-S-, an aryl group, and a heteroaryl group,; and R2 and R3 are independently selected from the group consisting of H, C1-14 alkyl, and C2-i4 alkenyl. For example, m is 5, 7, or 9. For example, Q is OH, -NHC(S)N(R)2, or -NHC(0)N(R)2. For example, Q is -N(R)C(0)R, or - N(R)S(0)2R.

In certain embodiments, a subset of compounds of Formula (I) includes those of Formula (IB): 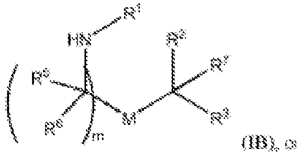 r its N-oxide, or a salt or isomer thereof in which all variables are as defined herein. For example, m is selected from 5, 6, 7, 8, and 9; M and M' are independently selected from -C(0)0-, -OC(O)-, -0C(0)-M"-C(0)0-, -C(0)N(R ,
-P(0)(0R')0-, -S-S-, an aryl group, and a heteroaryl group; and R2 and R3 are independently selected from the group consisting of H, Ci-i4 alkyl, and C2-14 alkenyl. For example, m is 5, 7, or 9. In certain embodiments, a subset of compounds of Formula (I) includes those of Formula 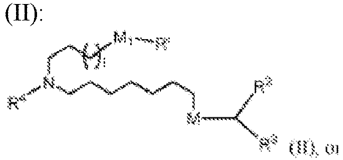 r its N-oxide, or a salt or isomer thereof, wherein 1 is selected from 1, 2, 3, 4, and 5; Mi is a bond or M'; R4 is hydrogen, unsubstituted C 1-3 alkyl, - (CH2)0C(R12)2(CH2)n-oQ, -C(0)NQR or -(CH2)nQ, in which n is 2, 3, or 4, and Q is OH, - NHC(S)N(R)2, - NHC(0)N(R)2, -N(R)C(0)R, -N(R)S(0)2R, -N(R)R8, -NHC(=NR9)N(R)2, -
NHC(=CHR9)N(R)2, -0C(0)N(R)2, -N(R)C(0)0R, -(CH2)nN(R)2, heteroaryl or
heterocycloalkyl; M and M' are independently selected from -C(0)0-, - OC(O)-, -0C(0)-M"-C(0)0-, -C(0)N(R')-, -P(0)(0R')0-, -S-S-, an aryl group, and a heteroaryl group; and R2 and R3 are independently selected from the group consisting of H, CI-M alkyl, and C2-M alkenyl.

In certain embodiments, a subset of compounds of Formula (I) includes those of Formula (Ila), (lib), (lie), or (He): 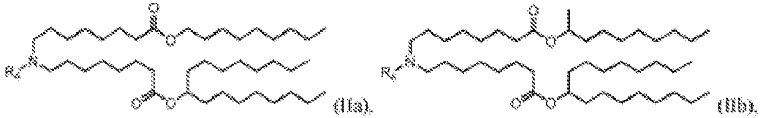 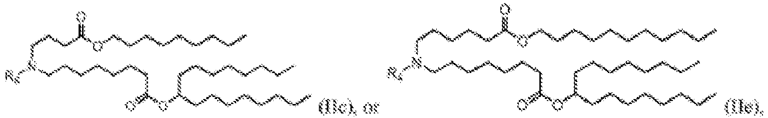 or its N-oxide, or a salt or isomer thereof, wherein R4 is as described herein.

In certain embodiments, a subset of compounds of Formula (I) includes those of Formula (lid): 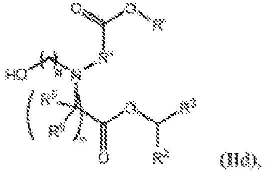 or its N-oxide, or a salt or isomer thereof, wherein n is 2, 3, or 4; and m, R', R", and R2 through R6 are as described herein. For example, each of R2 and R3 may be independently selected from the group consisting of C5-14 alkyl and C5-14 alkenyl.

In another embodiment, a subset of compounds of Formula (I) includes those of Formula (Ilf): 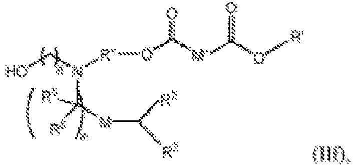
or its N-oxide, or a salt or isomer thereof, wherein n is 2, 3, or 4; and m, M, M", R', R", and R2 through R6 are as described herein. For example, each of R2 and R3 may be independently
selected from the group consisting of C5-14 alkyl and CS-14 alkenyl, and n is selected from 2, 3, and 4.

In another embodiment, a subset of compounds of Formula (I) includes those of Formula (Ilg): 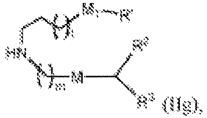 or its N-oxide, or a salt or isomer thereof, wherein 1, m, M, Mi, R', R2 and R3 are as described herein. For example, each of R2 and R3 may be independently selected from the group consisting of C5-14 alkyl and C5-14 alkenyl, 1 is selected from 1, 2, 3, 4, and 5, and m is selected from 5, 6, 7, 8, and 9.

Other aspects of the disclosure relate to compounds of Formula (VI): 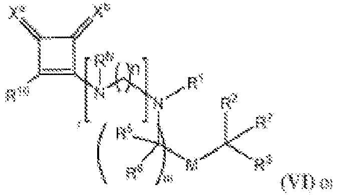 r its N-oxide, or a salt or isomer thereof, wherein
R1 is selected from the group consisting of C5-30 alkyl, C5-20 alkenyl, - R*YR", -YR", and -R'M'R';
R2 and R3 are independently selected from the group consisting of H, C1-14 alkyl, C2-14 alkenyl, -R*YR", -YR", and -R*OR", or R2 and R3, together with the atom to which they are attached, form a heterocycle or carbocycle;
each R5 is independently selected from the group consisting of OH, C 1-3 alkyl, C2-3 alkenyl, and H;
each R6 is independently selected from the group consisting of OH, C1-3 alkyl, C2-3 alkenyl, and H;
M and M' are independently selected from -C(0)0-, -OC(O)-, -0C(0)-M"-C(0)0-, -C(0)N(R')-, -N(R')C(0)-, -C(O)-, -C(S)-, -C(S)S-, -SC(S)-, -CH(OH)-, - P(0)(0R')0-, -S(0)2-, -S-S-, an aryl group, and a heteroaryl group, in which M" is a bond, C1-13 alkyl or C2-13 alkenyl;
R7 is selected from the group consisting of C1-3 alkyl, C2-3 alkenyl, and H;
each R is independently selected from the group consisting of H, C 1-3 alkyl, and C2-3 alkenyl;
RN is H, or Ci-3 alkyl;
each R' is independently selected from the group consisting of C1-18 alkyl, C2-18 alkenyl, -R*YR", -YR", and H;
each R" is independently selected from the group consisting of C3-15 alkyl and
C3-15 alkenyl;
each R* is independently selected from the group consisting of C1-12 alkyl and
C2-12 alkenyl;
each Y is independently a C3-6 carbocycle;
each X is independently selected from the group consisting of F, Cl, Br, and I;
Xa and Xb are each independently O or S;
R10 is selected from the group consisting of H, halo, -OH, R, -N(R)2, - CN, -N3, -C(0)0H, -C(0)0R, -0C(0)R, -OR, -SR, -S(0)R, -S(0)0R, -S(0)20R, -N02, - S(0)2N(R)2, -N(R)S(0)2R, -NH(CH2)tiN(R)2, -NH(CH2)PiO(CH2)qiN(R)2, - NH(CH2)SIOR, -N((CH2)SIOR)2, -N(R)-carbocycle, -N(R)-heterocycle, -N(R)-aryl, - N(R)-heteroaryl, -N(R)(CH2)ti-carbocycle, -N(R)(CH2)ti-heterocycle, -N(R)(CH2)ti-aryl, -N(R)(CH2)u-heteroaryl, a carbocycle, a heterocycle, aryl and heteroaryl;
m is selected from 5, 6, 7, 8, 9, 10, 11, 12, and 13;
n is selected from 1, 2, 3, 4, 5, 6, 7, 8, 9, and 10;
r is 0 or 1;
t1 is selected from 1, 2, 3, 4, and 5;
p1 is selected from 1, 2, 3, 4, and 5;
q1 is selected from 1, 2, 3, 4, and 5; and
s1 is selected from 1, 2, 3, 4, and 5.

In some embodiments, a subset of compounds of Formula (VI) includes those of Formula (VI-a): 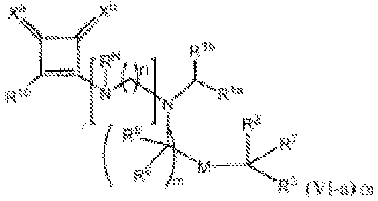 r its N-oxide, or a salt or isomer thereof, wherein
Rla and Rib are independently selected from the group consisting of C 1-14 alkyl and C2-14 alkenyl; and
R2 and R3 are independently selected from the group consisting of C 1-14 alkyl, C2-14 alkenyl, -R*YR", -YR", and -R*OR", or R2 and R3, together with the atom to which they are attached, form a heterocycle or carbocycle.

In other embodiments, a subset of compounds of Formula (VI) includes those of Formula (VII): 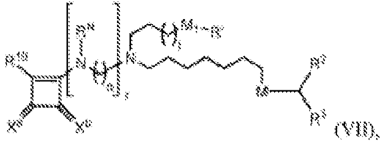
or its N-oxide, or a salt or isomer thereof, wherein
1 is selected from 1, 2, 3, 4, and 5;
Mi is a bond or M'; and
R2 and R3 are independently selected from the group consisting of H, Ci- i4 alkyl, and C2-14 alkenyl.

In other embodiments, a subset of compounds of Formula (VI) includes those of Formula (VIII): 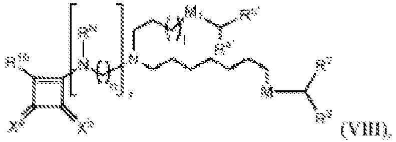
or its N-oxide, or a salt or isomer thereof, wherein
1 is selected from 1, 2, 3, 4, and 5;
Mi is a bond or M'; and
Ra and Rb are independently selected from the group consisting of C1-14 alkyl and C2-14 alkenyl; and
R2 and R3 are independently selected from the group consisting of C1-14 alkyl, and C2-14 alkenyl.

The compounds of any one of formula (I), (IA), (VI), (Vl-a), (VII) or (VIII) include one or more of the following features when applicable.

In some embodiments, Mi is M'.

In some embodiments, M and M' are independently -C(0)0- or -OC(O)-.

In some embodiments, at least one of M and M' is -C(0)0- or -OC(O)-.

In certain embodiments, at least one of M and M' is -OC(O)-.

In certain embodiments, M is -OC(O)- and M' is -C(0)0-. In some embodiments, M is -C(0)0- and M' is -OC(O)-. In certain embodiments, M and M' are each -OC(O)-. In some embodiments, M and M' are each -C(0)0-.

In certain embodiments, at least one of M and M' is -0C(0)-M"-C(0)0-.

In some embodiments, M and M' are independently -S-S-.

In some embodiments, at least one of M and M' is -S-S.

In some embodiments, one of M and M' is -C(0)0- or -OC(O)- and the other is -S-S-. For example, M is -C(0)0- or -OC(O)- and M' is -S-S- or M' is -C(0)0-, or -OC(O)- and M is -S-S-.

In some embodiments, one of M and M' is -0C(0)-M"-C(0)0-, in which M" is a bond, Ci-i3 alkyl or C2-13 alkenyl. In other embodiments, M" is C1-6 alkyl or C2-6 alkenyl. In certain embodiments, M" is C1-4 alkyl or C2-4 alkenyl. For example, in some embodiments, M" is Ci alkyl. For example, in some embodiments, M" is C2 alkyl. For example, in some embodiments, M" is C3 alkyl. For example, in some embodiments, M" is C4 alkyl. For example, in some embodiments, M" is C2 alkenyl. For example, in some embodiments, M" is C3 alkenyl. For example, in some embodiments, M" is C4 alkenyl.

In some embodiments, 1 is 1, 3, or 5.

In some embodiments, R4 is hydrogen.

In some embodiments, R4 is not hydrogen.

In some embodiments, R4 is unsubstituted methyl or -(CH2)nQ, in which Q is OH, -NHC(S)N(R)2, -NHC(0)N(R)2, -N(R)C(0)R, or -N(R)S(0)2R.

In some embodiments, Q is OH.

In some embodiments, Q is -NHC(S)N(R)2.

In some embodiments, Q is -NHC(0)N(R)2.

In some embodiments, Q is -N(R)C(0)R.

In some embodiments, Q is -N(R)S(0)2R.

In some embodiments, Q is -0(CH2)nN(R)2.

In some embodiments, Q is -0(CH2)nOR.

In some embodiments, Q is -N(R)R8.

In some embodiments, Q is -NHC(=NR9)N(R)2.

In some embodiments, Q is -NHC(=CHR9)N(R)2.

In some embodiments, Q is -OC(0)N(R)2.

In some embodiments, Q is -N(R)C(0)OR.

In some embodiments, n is 2.

In some embodiments, n is 3.

In some embodiments, n is 4.

In some embodiments, Mi is absent.

In some embodiments, at least one R5 is hydroxyl. For example, one R5 is hydroxyl.

In some embodiments, at least one R6 is hydroxyl. For example, one R6 is hydroxyl.

In some embodiments one of R5 and R6 is hydroxyl. For example, one R5 is hydroxyl and each R6 is hydrogen. For example, one R6 is hydroxyl and each R5 is hydrogen.

In some embodiments, Rx is Ci-6 alkyl. In some embodiments, Rx is Ci-3 alkyl. For example, Rx is methyl. For example, Rx is ethyl. For example, Rx is propyl.

In some embodiments, Rx is -(CFkXOFl and, v is 1, 2 or 3. For example, Rx is methanoyl. For example, Rx is ethanoyl. For example, Rx is propanoyl.

In some embodiments, Rx is -(CH2)vN(R)2, v is 1, 2 or 3 and each R is H or methyl. For example, Rx is methanamino, methylmethanamino, or dimethylmethanamino. For example, Rx is aminomethanyl, methylaminomethanyl, or dimethylaminomethanyl. For example, Rx is aminoethanyl, methylaminoethanyl, or dimethylaminoethanyl. For example, Rx is
aminopropanyl, methylaminopropanyl, or dimethylaminopropanyl.

In some embodiments, R' is Ci-ib alkyl, C2-18 alkenyl, -R*YR", or - YR".

In some embodiments, R2 and R3 are independently C3-14 alkyl or C3-14 alkenyl.

In some embodiments, Rib is Ci-14 alkyl. In some embodiments, Rlb is C2-14 alkyl. In some embodiments, Rib is C3-14 alkyl. In some embodiments, Rlb is Ci-8 alkyl. In some embodiments, Rib is C1-5 alkyl. In some embodiments, Rlb is C1-3 alkyl. In some embodiments, Rlb is selected from Ci alkyl, C2 alkyl, C3 alkyl, C4 alkyl, and C5 alkyl. For example, in some embodiments, Rlb is Ci alkyl. For example, in some embodiments, Rlb is C2 alkyl. For example, in some embodiments, Rib is C3 alkyl. For example, in some embodiments, Rlb is C4 alkyl. For example, in some embodiments, Rlb is C5 alkyl.

In some embodiments, R1 is different from -(CHR5R6)m-M-CR2R3R7.

In some embodiments, -CHRlaRIb- is different from -(CHR5R6)m-M-CR2R3R7.

In some embodiments, R7 is H. In some embodiments, R7 is selected from C1-3 alkyl. For example, in some embodiments, R7 is Ci alkyl. For example, in some embodiments, R7 is C2 alkyl. For example, in some embodiments, R7 is C3 alkyl. In some embodiments, R7 is selected from C4 alkyl, C4 alkenyl, C5 alkyl, C5 alkenyl, Ce alkyl, Ce alkenyl, C7 alkyl, C7 alkenyl, C9 alkyl, C9 alkenyl, C11 alkyl, C11 alkenyl, C17 alkyl, C17 alkenyl, Cie alkyl, and Cie alkenyl.

In some embodiments, Rb is Ci-i4 alkyl. In some embodiments, Rb is C2-14 alkyl. In some embodiments, Rb is C3-14 alkyl. In some embodiments, Rb is Ci-8 alkyl. In some embodiments, Rb is C1-5 alkyl. In some embodiments, Rb is C1-3 alkyl. In some embodiments, Rb is selected from Ci alkyl, C2 alkyl, C3 alkyl, C4 alkyl and C5 alkyl. For example, in some embodiments, Rb is Ci alkyl. For example, in some embodiments, Rb is C2 alkyl. For example, some embodiments, Rb is C3 alkyl. For example, some embodiments, Rb is C4 alkyl.

In some embodiments, the compounds of Formula (I) are of Formula (Ila): 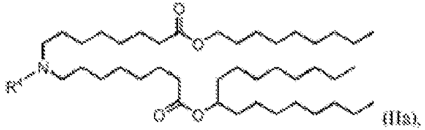 or their N-oxides, or salts or isomers thereof, wherein R4 is as described herein.

In other embodiments, the compounds of Formula (I) are of Formula (lib): 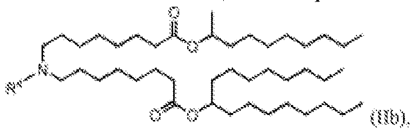 or their N-oxides, or salts or isomers thereof, wherein R4 is as described herein.

In other embodiments, the compounds of Formula (I) are of Formula (lie) or (He): 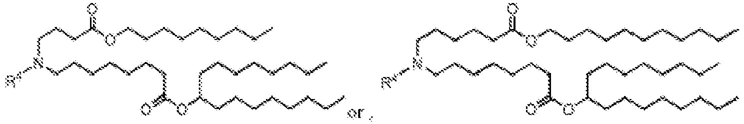 (lie) (He) or their N-oxides, or salts or isomers thereof, wherein R4 is as described herein.

In other embodiments, the compounds of Formula (I) are of Formula (Ilf): 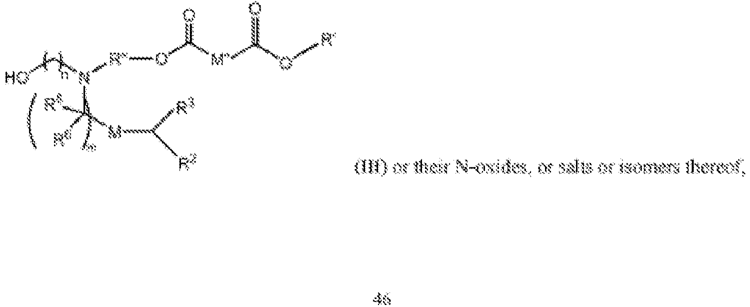 wherein M is -C(0)0- or -OC(O)-, M" is C1-6 alkyl or C2-6 alkenyl, R2 and R3 are independently selected from the group consisting of C5-14 alkyl and C5-14 alkenyl, and n is selected from 2, 3, and 4.

In a further embodiment, the compounds of Formula (I) are of Formula (lid): 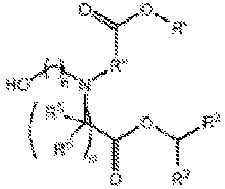 (lid),
or their N-oxides, or salts or isomers thereof, wherein n is 2, 3, or 4; and m, R', R", and R2 through R6 are as described herein. For example, each of R2 and R3 may be independently selected from the group consisting of C5-14 alkyl and C5-14 alkenyl.

In a further embodiment, the compounds of Formula (I) are of Formula (Ilg): 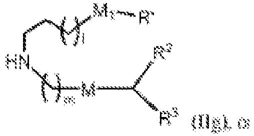 r their N-oxides, or salts or isomers thereof, wherein 1 is selected from 1, 2, 3, 4, and 5; m is selected from 5, 6, 7, 8, and 9; Mi is a bond or M'; M and M' are independently selected from -C(0)0-, -OC(O)-, -0C(0)-M"-C(0)0-, -C(0)N(R')-, -P(0)(0R')0-, -S-S-, an aryl group, and a heteroaryl group; and R2 and R3 are independently selected from the group consisting of H, C1-14 alkyl, and C2-14 alkenyl. For example, M" is Ci-6 alkyl (e.g., C 1-4 alkyl) or C2-6 alkenyl (e.g. C2-4 alkenyl). For example, R2 and R3 are independently selected from the group consisting of C5-14 alkyl and C5-14 alkenyl.

In other embodiments, a subset of compounds of Formula (VI) includes those of Formula (Vila): 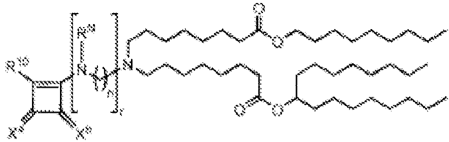 (Vila), or its N-oxide, or a salt or isomer thereof.

In other embodiments, a subset of compounds of Formula (VI) includes those of Formula (Villa): 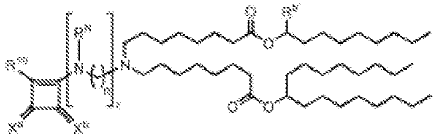 (Villa), or its N-oxide, or a salt or isomer thereof.

In other embodiments, a subset of compounds of Formula (VI) includes those of Formula (Vlllb): 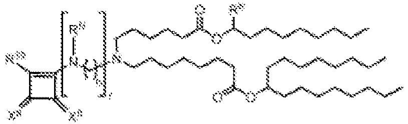 (Vlllb), or its N-oxide, or a salt or isomer thereof.

In other embodiments, a subset of compounds of Formula (VI) includes those of Formula (Vllb-l): 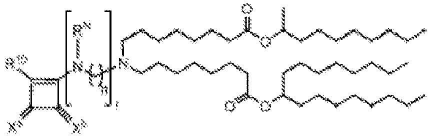 (Vllb-l), or its N-oxide, or a salt or isomer thereof.

In other embodiments, a subset of compounds of Formula (VI) includes those of Formula (VIIb-2): 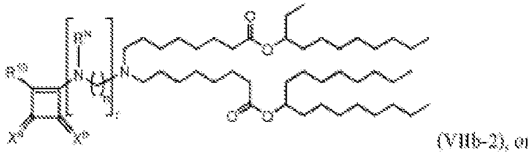 r its N-oxide, or a salt or isomer thereof.

In other embodiments, a subset of compounds of Formula (VI) includes those of Formula (VIIb-3): 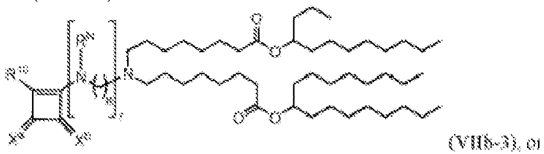 r its N-oxide, or a salt or isomer thereof.

In other embodiments, a subset of compounds of Formula (VI) includes those of Formula (VIIb-4): 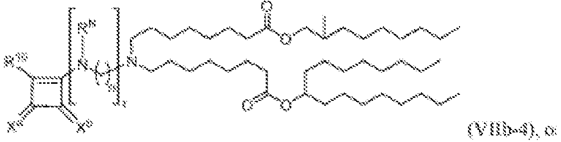 r its N-oxide, or a salt or isomer thereof.

In other embodiments, a subset of compounds of Formula (VI) includes those of Formula (Vile):

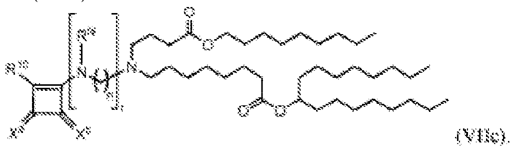

In other embodiments, a subset of compounds of Formula (VI) includes those of Formula (Vlld): 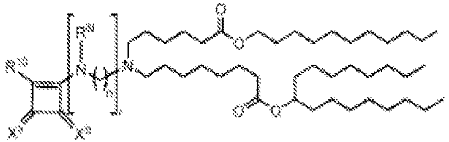 (Vlld), or its N-oxide, or a salt or isomer thereof.

In other embodiments, a subset of compounds of Formula (VI) includes those of Formula (VIIIc):

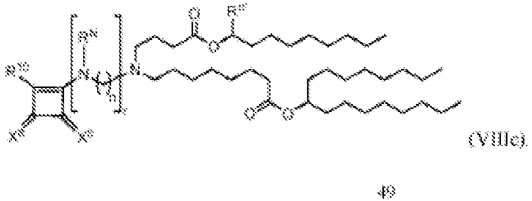

In other embodiments, a subset of compounds of Formula (VI) includes those of Formula (VUId): 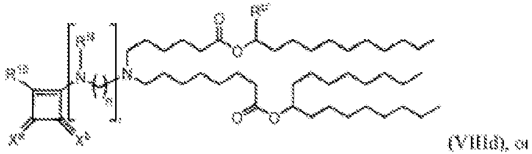 r its N-oxide, or a salt or isomer thereof.

The compounds of any one of formulae (I), (IA), (IB), (II), (Ila), (lib), (lie), (lid), (He), (Ilf), (Ilg), (III), (VI), (Vl-a), (VII), (VIII), (Vila), (Villa), (VUIb), (Vllb-l), (VIIb-2), (VIIb-3), (Vile), (Vlld), (VIIIc), or (VUId) include one or more of the following features when applicable.

In some embodiments, R4 is selected from the group consisting of a C3-6 carbocycle, -(CH2)nQ, -(CH2)nCHQR, -(CH2)0C(R12)2(CH2)n-oQ, -CHQR, and - CQ(R)2, where Q is selected from a C3-6 carbocycle, 5- to 14- membered aromatic or non-aromatic heterocycle having one or more heteroatoms selected from N, O, S, and P, -OR, -0(CH2)nN(R)2, -C(0)OR, -OC(0)R, -CX3, -CX2H, -CXH2, -CN, -N(R)2, - N(R)S(0)2R8, -C(0)N(R)2, -N(R)C(0)R, -N(R)S(0)2R, - N(R)C(0)N(R)2, - N(R)C(S)N(R)2, and -C(R)N(R)2C(0)OR, each 0 is independently selected from 1, 2, 3, and 4, and each n is independently selected from 1, 2, 3, 4, and 5.

In some embodiments, R4 is selected from the group consisting of a C3-6 carbocycle, - (CH2)nQ, -(CHQnCHQR, -(CH2)0C(R12)2(CH2)n-oQ, -CHQR, and - CQ(R)2, where Q is selected from a C3-6 carbocycle, a 5- to 14-membered heteroaryl having one or more heteroatoms selected from N, O, and S, -OR, -0(CH2)nN(R)2, - C(0)OR, -OC(0)R, -CX3, -CX2H, -CXH2, -CN, -C(0)N(R)2, -N(R)S(0)2R8, - N(R)C(0)R, -N(R)S(0)2R, -N(R)C(0)N(R)2, - N(R)C(S)N(R)2, -C(R)N(R)2C(0)OR, and a 5- to l4-membered heterocycloalkyl having one or more heteroatoms selected from N, O, and S which is substituted with one or more substituents selected from oxo (=0), OH, amino, and C1-3 alkyl, each 0 is independently selected from 1, 2,

3, and 4, and each n is independently selected from 1, 2, 3, 4, and 5.

In some embodiments, R4 is selected from the group consisting of a C3-6 carbocycle, -(CH2)nQ, -(CH2)nCHQR, -(CH2)0C(R12)2(CH2)n-oQ, -CHQR, and - CQ(R)2, where Q is selected from a C3-6 carbocycle, a 5- to 14-membered heterocycle having one or more heteroatoms selected from N, O, and S, -OR, -0(CH2)nN(R)2, - C(0)OR, -OC(0)R, -CX3, -CX2H, -CXH2, -CN, -C(0)N(R)2, -N(R)S(0)2R8, - N(R)C(0)R, -N(R)S(0)2R, -N(R)C(0)N(R)2, -N(R)C(S)N(R)2, -C(R)N(R)2C(0)OR, each 0 is independently selected from 1, 2, 3, and 4, and
each n is independently selected from 1, 2, 3, 4, and 5; and when Q is a 5-to 14-membered heterocycle and (i) R4 is -(CH2)nQ in which n is 1 or 2, or (ii) R4 is - (CH2)nCHQR in which n is 1, or (iii) R4 is -CHQR, and -CQ(R)2, then Q is either a 5-to 14-membered heteroaryl or 8- to 14-membered heterocycloalkyl.

In some embodiments, R4 is selected from the group consisting of a C3-6 carbocycle, -(CH2)nQ, -(CH2)nCHQR, -(CH2)oC(R12)2(CH2)n-oQ, -CHQR, and - CQ(R)2, where Q is selected from a C3-6 carbocycle, a 5- to 14-membered heteroaryl having one or more heteroatoms selected from N, O, and S, -OR, -0(CH2)nN(R)2, - C(0)OR, -OC(0)R, -CX3, -CX2H, -CXH2, -CN, -C(0)N(R)2, -N(R)S(0)2R8, - N(R)C(0)R, -N(R)S(0)2R, -N(R)C(0)N(R)2, -N(R)C(S)N(R)2, -C(R)N(R)2C(0)OR, each 0 is independently selected from 1, 2, 3, and 4, and each n is independently selected from 1, 2, 3, 4, and 5.

In some embodiments, R4 is -(CH2)nQ, where Q is -N(R)S(0)2R8 and n is selected from 1, 2, 3, 4, and 5. In a further embodiment, R4 is -(CH2)nQ, where Q is - N(R)S(0)2R8, in whichR8 is a C3-6 carbocycle such as C3-6 cycloalkyl, and n is selected from 1, 2, 3, 4, and 5.

For example, R4 is -(CH2)3NHS(0)2R8 and R8 is cyclopropyl.

In some embodiments, R4 is -(CH2)oC(R12)2(CH2)n-oQ, where Q is - N(R)C(0)R, n is selected from 1, 2, 3, 4, and 5, and 0 is selected from 1, 2, 3, and 4. In a further embodiment, R4 is -(CH2)oC(R12)2(CH2)n-oQ, where Q is -N(R)C(0)R, wherein R is C1-C3 alkyl and n is selected from 1, 2, 3, 4, and 5, and 0 is selected from 1, 2, 3, and 4. In a another embodiment, R4 is is -(CH2)oC(R12)2(CH2)n-oQ, where Q is -N(R)C(0)R, wherein R is C1-C3 alkyl, n is 3, and 0 is 1.

In some embodiments, R12 is H, OH, C1-3 alkyl, or C2-3 alkenyl. For example, R4 is 3-acetamido-2,2-dimethylpropyl.

In some embodiments, R4 is -C(0)NQR, where Q is -(CH2)nN(R)2. In a further embodiments, R4 is -C(0)NH(CH2)3N(CH3)2, -C(0)NH(CH2)4N(CH3)2, or - C(0)NH(CH₂)2N(CH3)2.

In some embodiments, one R12 is H and one R12 is C1-3 alkyl or C2-3 alkenyl. In some embodiments, each R12 is is C1-3 alkyl or C2-3 alkenyl. In some embodiments, each R12 is is C1-3 alkyl (e.g. methyl, ethyl or propyl). For example, one R12 is methyl and one R12 is ethyl or propyl. For example, one R12 is ethyl and one R12 is methyl or propyl. For example, one R12 is propyl and one R12 is methyl or ethyl. For example, each R12 is methyl. For example, each R12 is ethyl. For example, each R12 is propyl.

In some embodiments, one R12 is H and one R12 is OH. In some embodiments, each R12 is is OH.

In some embodiments, R4 is unsubstituted C1-4 alkyl, e.g., unsubstituted methyl.

In some embodiments, R4 is hydrogen.

In certain embodiments, the disclosure provides a compound having the Formula (I), wherein R4 is -(CF JnQ or -(CH2)nCHQR, where Q is -N(R)2, and n is selected from 3, 4, and 5.

In certain embodiments, the disclosure provides a compound having the Formula (I), wherein R4 is selected from the group consisting of -(CH2)nQ, - (CH2)nCHQR, -CHQR, and -CQ(R)2, where Q is -N(R)2, and n is selected from 1, 2, 3, 4, and 5.

In certain embodiments, the disclosure provides a compound having the Formula (I), wherein R2 and R3 are independently selected from the group consisting of C2-14 alkyl, C2-14 alkenyl, -R*YR", -YR", and -R*OR", or R2 and R3, together with the atom to which they are attached, form a heterocycle or carbocycle, and R4 is - (CH2)nQ or -(CH2)nCHQR, where Q is -N(R)2, and n is selected from 3, 4, and 5.

In certain embodiments, R2 and R3 are independently selected from the group consisting of C2-14 alkyl, C2-14 alkenyl, -R*YR", -YR", and -R*OR", or R2 and R3, together with the atom to which they are attached, form a heterocycle or carbocycle. In some embodiments,

R2 and R3 are independently selected from the group consisting of C2-14 alkyl, and C2-14 alkenyl. In some embodiments, R2 and R3 are independently selected from the group consisting of -R*YR", -YR", and -R * OR" . In some embodiments, R2 and R3 together with the atom to which they are attached, form a heterocycle or carbocycle.

In some embodiments, R1 is selected from the group consisting of C5-20 alkyl and C5-20 alkenyl. In some embodiments, R1 is C5-20 alkyl substituted with hydroxyl.

In other embodiments, R1 is selected from the group consisting of -R*YR", -YR", and -R"M'R\

In certain embodiments, R1 is selected from -R*YR" and -YR". In some
embodiments, Y is a cyclopropyl group. In some embodiments, R* is Cx alkyl or Cx alkenyl. In certain embodiments, R" is C3-12 alkyl. For example, in some embodiments, R" is C3 alkyl. For example, in some embodiments, R" is C4-8 alkyl (e.g., C4, C5, Ce, C7, or Cs alkyl).

In some embodiments, R is (CH2)qOR*, q is selected from 1, 2, and 3, and R* is C1-12 alkyl substituted with one or more substituents selected from the group consisting of amino, Ci-Ce alkylamino, and C1-C6 dialkylamino. For example, R is (CFh)qOR*, q is selected from 1, 2, and 3 and R* is C1-12 alkyl substituted with C1-C6 dialkylamino. For example, R is (CH2)qOR*, q is selected from 1, 2, and 3 and R* is C1-3 alkyl substituted with C1-C6 dialkylamino. For example, R is (CH2)qOR*, q is selected from 1, 2, and 3 and R* is C1-3 alkyl substituted with dimethylamino (e.g., dimethylaminoethanyl).

In some embodiments, R1 is C5-20 alkyl. In some embodiments, R1 is G, alkyl. In some embodiments, R1 is Cs alkyl. In other embodiments, R1 is C9 alkyl. In certain
embodiments, R1 is C 14 alkyl. In other embodiments, R1 is Cie alkyl.

In some embodiments, R1 is C21-30 alkyl. In some embodiments, R1 is C26 alkyl. In some embodiments, R1 is C28 alkyl. In certain embodiments, R1 is

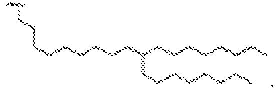

In some embodiments, R1 is C5-20 alkenyl. In certain embodiments, R1 is Cie alkenyl. In some embodiments, R1 is linoleyl.

In certain embodiments, R1 is branched (e.g., decan-2 -yl, undecan-3-yl, dodecan-4-yl, tridecan-5-yl, tetradecan-6-yl, 2-methylundecan-3-yl, 2-methyldecan-2-yl, 3-methylundecan-3-yl, 4-methyldodecan-4-yl, or heptadeca-9-yl). In certain embodiments, R1 is

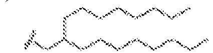

In certain embodiments, R1 is unsubstituted C5-20 alkyl or C5-20 alkenyl. In certain embodiments, R' is substituted C5-20 alkyl or C5-20 alkenyl (e.g., substituted with a C3-6 carbocycle such as l-cyclopropylnonyl or substituted with OH or alkoxy). For example, R1 is

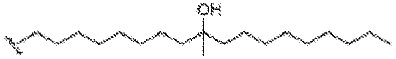

In other embodiments, R1 is -R"M'R\ In certain embodiments, M' is - OC(0)-M"-C(0)0-. For example, R¹ is 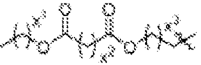 , wherein x1 is an integer between 1 and
13 (e.g., selected from 3, 4, 5, and 6), x2 is an integer between 1 and 13 (e.g., selected from 1, 2, and 3), and x3 is an integer between 2 and 14 (e.g., selected from 4, 5, and 6). For example, x1 is selected from 3, 4, 5, and 6, x2 is selected from 1, 2, and 3, and x3 is selected from 4, 5, and 6.

In other embodiments, R1 is different from -(CHR5R6)m-M-CR2R3R7.

In some embodiments, R' is selected from -R*YR" and -YR". In some
embodiments, Y is C3-8 cycloalkyl. In some embodiments, Y is Ce-io aryl. In some
embodiments, Y is a cyclopropyl group. In some embodiments, Y is a cyclohexyl group. In certain embodiments, R* is Ci alkyl.

In some embodiments, R" is selected from the group consisting of C3-12 alkyl and C3- 12 alkenyl. In some embodiments, R" is Cs alkyl. In some embodiments, R" adjacent to Y is Ci
alkyl. In some embodiments, R" adjacent to Y is C4-9 alkyl (e.g., C4, C5, Ce, Ci or Cs or C9 alkyl).

In some embodiments, R" is substituted C3-12 alkyl (e.g., C3-12 alkyl substituted with, e.g., an hydroxyl). For example, R" is

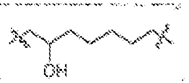

In some embodiments, R' is selected from C4 alkyl and C4 alkenyl. In certain embodiments, R' is selected from C5 alkyl and C5 alkenyl. In some embodiments, R' is selected from C6 alkyl and Ce alkenyl. In some embodiments, R' is selected from C7 alkyl and C7 alkenyl. In some embodiments, R' is selected from C9 alkyl and C9 alkenyl.

In some embodiments, R' is selected from C4 alkyl, C4 alkenyl, C5 alkyl, C5 alkenyl, C6 alkyl, Ce alkenyl, C7 alkyl, C7 alkenyl, C9 alkyl, C9 alkenyl, C 11 alkyl, C 11 alkenyl, C 17 alkyl, C17 alkenyl, Cie alkyl, and Cie alkenyl, each of which is either linear or branched.

In some embodiments, R' is C4 alkyl or C4 alkenyl. In some embodiments, R' is C5 alkyl or C5 alkenyl. In some embodiments, R' is G, alkyl or G, alkenyl. In some embodiments, R' is C7 alkyl or C7 alkenyl. In some embodiments, R' is Cs alkyl or Cs alkenyl. In some embodiments, R' is C9 alkylor C9 alkenyl. In some embodiments, R' is C10 alkyl or C 10 alkenyl. In some embodiments, R' is C 11 alkyl or C11 alkenyl.

In some embodiments, R' is linear. In some embodiments, R' is branched.

In some embodiments, R' is

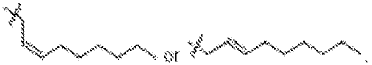

In some embodiments, R' is 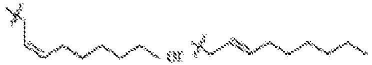 and M' is -OC(O-), In other embodiments, R' is 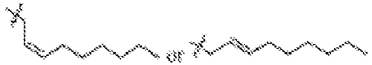 and M' is -C(O)O-.

In other embodiments, R' is selected from C11 alkyl and C 11 alkenyl. In other embodiments, R' is selected from C12 alkyl, C12 alkenyl, C13 alkyl, C13 alkenyl, C14 alkyl, C14 alkenyl, C15 alkyl, C15 alkenyl, Ci6 alkyl, Ci6 alkenyl, C17 alkyl, C 17 alkenyl, Cie alkyl, and Cie alkenyl. In certain embodiments, R' is linear C4-18 alkyl or C4-18 alkenyl. In certain
embodiments, R' is branched (e.g., decan-2-yl, undecan-3-yl, dodecan-4-yl, tridecan-5-yl, tetradecan-6-yl, 2-methylundecan-3-yl, 2-methyldecan-2-yl, 3-methylundecan-3-yl, 4-
methyldodecan-4-yl or heptadeca-9-yl). In certain embodiments, R' is

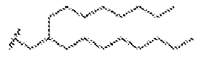

In certain embodiments, R' is unsubstituted Ci-ie alkyl. In certain embodiments, R' is substituted Ci-ie alkyl (e.g., C1-15 alkyl substituted with, e.g., an alkoxy such as methoxy, or a C3-6 carbocycle such as l-cyclopropylnonyl, or C(0)0-alkyl or 0C(0)-alkyl such as C(0)0CH3
or OC(O)CH₃). For example, R' is 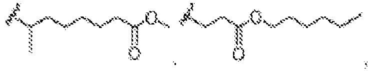 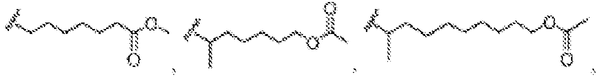 or

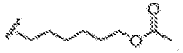

In certain embodiments, R' is branched Ci-ib alkyl. For example, R' is

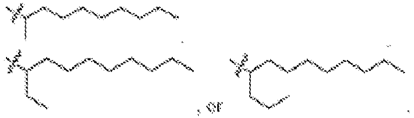

In some embodiments, R" is selected from the group consisting of C3-15 alkyl and C3-15 alkenyl. In some embodiments, R" is C3 alkyl, C4 alkyl, C5 alkyl, Ce alkyl, C7 alkyl, or Cs alkyl. In some embodiments, R" is C9 alkyl, C10 alkyl, C11 alkyl, C12 alkyl, C13 alkyl, C14 alkyl, or C15 alkyl.

In some embodiments, M' is -C(0)0-. In some embodiments, M' is - OC(O)-. In some embodiments, M' is -0C(0)-M"-C(0)0-. In some embodiments, M' is - S-S-.

In some embodiments, M' is -C(0)0-, -OC(O)-, or -0C(0)-M"-C(0)0-. In some embodiments wherein M' is -0C(0)-M"-C(0)0-, M" is Ci-4 alkyl or C2-4 alkenyl.

In other embodiments, M' is an aryl group or heteroaryl group. For example, in some embodiments, M' is selected from the group consisting of phenyl, oxazole, and thiazole.

In some embodiments, M is -C(0)0-. In some embodiments, M is -OC(O)-. In some embodiments, M is -C(0)N(R')-. In some embodiments, M is -P(0)(0R')0-. In some embodiments, M is -0C(0)-M"-C(0)0-. In some embodiments, M is -S-S-.

In some embodiments, M is -C(O). In some embodiments, M is -OC(O)- and M' is -C(0)0-. In some embodiments, M is -C(0)0- and M' is -OC(O)-. In some embodiments, M and M' are each -OC(O)-. In some embodiments, M and M' are each - C(0)0-.

In other embodiments, M is an aryl group or heteroaryl group. For example, in some embodiments, M is selected from the group consisting of phenyl, oxazole, and thiazole.

In some embodiments, M is the same as M'. In other embodiments, M is different from M'.

In some embodiments, M" is a bond. In some embodiments, M" is C1-13 alkyl or C2-13 alkenyl. In some embodiments, M" is C1-6 alkyl or C2-6 alkenyl. In certain embodiments, M" is linear alkyl or alkenyl. In certain embodiments, M" is branched, e.g., -CH(CH3)CH2-.

In some embodiments, each R5 is H. In some embodiments, each R6 is H. In certain such embodiments, each R5 and each R6 is H.

In some embodiments, R7 is H. In other embodiments, R7 is Ci-3 alkyl (e.g., methyl, ethyl, propyl, or i-propyl).

In some embodiments, R2 and R3 are independently C5-14 alkyl or C5-14 alkenyl.

In some embodiments, R2 and R3 are the same. In some embodiments, R2 and R3 are C8 alkyl. In certain embodiments, R2 and R3 are C2 alkyl. In other embodiments, R2 and R3 are C3 alkyl. In some embodiments, R2 and R3 are C4 alkyl. In certain embodiments, R2 and R3 are C5 alkyl. In other embodiments, R2 and R3 are Ce alkyl. In some embodiments, R2 and R3 are C7 alkyl.

In other embodiments, R2 and R3 are different. In certain embodiments, R2 is G alkyl. In some embodiments, R3 is C1-7 (e.g., Ci, C2, C3, C4, C5, Ce, or C7 alkyl) or C9 alkyl.

In some embodiments, R3 is Ci alkyl. In some embodiments, R3 is C2 alkyl. In some embodiments, R3 is C3 alkyl. In some embodiments, R3 is C4 alkyl. In some embodiments, R3 is C5 alkyl. In some embodiments, R3 is G, alkyl. In some embodiments, R3 is C7 alkyl. In some embodiments, R3 is C9 alkyl.

In some embodiments, R7 and R3 are H.

In certain embodiments, R2 is H.

In some embodiments, m is 5, 6, 7, 8, or 9. In some embodiments, m is 5, 7, or 9.

For example, in some embodiments, m is 5. For example, in some embodiments, m is 7. For example, in some embodiments, m is 9.

In some embodiments, R4 is selected from -(CH2)nQ and -(CH2)nCHQR.

In some embodiments, Q is selected from the group consisting of -OR, - OH, -0(CH2)nN(R)2, -0C(0)R, -CX3, -CN, -N(R)C(0)R, -N(H)C(0)R, -N(R)S(0)2R, -N(H)S(0)2R, -N(R)C(0)N(R)2, -N(H)C(0)N(R)2, -N(H)C(0)N(H)(R), - N(R)C(S)N(R)2, -N(H)C(S)N(R)2, -N(H)C(S)N(H)(R), -C(R)N(R)2C(0)0R, - N(R)S(0)2R8, a carbocycle, and a heterocycle.

In certain embodiments, Q is -N(R)R8, -N(R)S(0)2R8, -0(CH2)n0R, - N(R)C(=NR9)N(R)2, -N(R)C(=CHR9)N(R)2, -0C(0)N(R)2, or -N(R)C(0)0R.

In certain embodiments, Q is -N(0R)C(0)R, -N(0R)S(0)2R, - N(0R)C(0)0R, -N(0R)C(0)N(R)2, -N(OR)C(S)N(R)2, -N(OR)C(=NR9)N(R)2, or - N(0R)C(=CHR9)N(R)2.

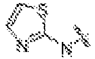

In certain embodiments, Q is thiourea or an isostere thereof, e.g., H or - NHC(=NR9)N(R)2.

In certain embodiments, Q is -C(=NR9)N(R)2. For example, when Q is - C(=NR9)N(R)2, n is 4 or 5. For example, R9 is -S(0)2N(R)2.

In certain embodiments, Q is -C(=NR9)R or -C(0)N(R)OR, e g., -CH(=N-OCH3), -C(0)NH-OH, -C(0)NH-OCH3, -C(0)N(CH3)-OH, or -C(0)N(CH3)-0CH3.

In certain embodiments, Q is -OH.

In certain embodiments, Q is a substituted or unsubstituted 5- to 10-membered heteroaryl, e.g., Q is a triazole, an imidazole, a pyrimidine, a purine, 2-amino-1 9-dihydro-6//-purin-6-one-9-yl (or guanin-9-yl), adenin-9-yl, cytosin-l-yl, or uracil-1-yl, each of which is optionally substituted with one or more substituents selected from alkyl, OH, alkoxy, -alkyl-OH, -alkyl-O-alkyl, and the substituent can be further substituted. In certain embodiments, Q is a substituted 5- to 14-membered heterocycloalkyl, e.g., substituted with one or more substituents selected from oxo (=0), OH, amino, mono- or di-alkylamino, and Ci-3 alkyl. For example, Q is 4-methylpiperazinyl, 4-(4-methoxybenzyl)piperazinyl, isoindolin-2-yl-l,3-dione, pyrrolidin-l-yl-2,5-dione, or imidazolidin-3-yl-2,4-dione.

In certain embodiments, Q is -NHR8, in which R8 is a C3-6 cycloalkyl optionally substituted with one or more substituents selected from oxo (=0), amino (NH2), mono- or di-alkylamino, Ci-3 alkyl and halo. For example, R8 is cyclobutenyl, e.g., 3-(dimethylamino)-cyclobut-3-ene-4-yl-1,2-dione. In further embodiments, R8 is a C3-6 cycloalkyl optionally substituted with one or more substituents selected from oxo (=0), thio (=S), amino (NH2), mono- or di-alkylamino, Ci-3 alkyl, heterocycloalkyl, and halo, wherein the mono- or di-alkylamino, Ci-3 alkyl, and heterocycloalkyl are further substituted. For example R8 is cyclobutenyl substituted with one or more of oxo, amino, and alkylamino, wherein the alkylamino is further substituted, e.g., with one or more of Ci-3 alkoxy, amino, mono- or di-alkylamino, and halo. For example, R8 is 3-(((dimethylamino)ethyl)amino)cyclobut-3-enyl-l,2-dione. For example R8 is cyclobutenyl substituted with one or more of oxo, and alkylamino.

For example, R8 is 3-(ethylamino)cyclobut-3-ene-1,2-dione. For example R8 is cyclobutenyl substituted with one or more of oxo, thio, and alkylamino. For example R8 is 3-(ethylamino)-4-thioxocyclobut-2-en-1-one or 2-(ethylamino)-4-thioxocyclobut-2-en-1-one. For example R8 is cyclobutenyl substituted with one or more of thio, and alkylamino. For example R8 is 3-(ethylamino)cyclobut-3-ene-1,2-dithione. For example R8 is cyclobutenyl substituted with one or more of oxo and dialkylamino. For example R8 is 3-(diethylamino)cyclobut-3-ene-1,2-dione. For example, R8 is cyclobutenyl substituted with one or more of oxo, thio, and dialkylamino.

For example, R8 is 2-(diethylamino)-4-thioxocyclobut-2-en-l-one or 3-(diethylamino)-4-thioxocyclobut-2-en-l-one. For example, R8 is cyclobutenyl substituted with one or more of thio, and dialkylamino. For example, R8 is 3-(diethylamino)cyclobut-3-ene-1,2-dithione. For example, R8 is cyclobutenyl substituted with one or more of oxo and alkylamino or dialkylamino, wherein alkylamino or dialkylamino is further substituted, e.g. with one or more alkoxy. For example, R8 is 3-(bis(2-methoxyethyl)amino)cyclobut-3-ene-1,2-dione. For example, R8 is cyclobutenyl substituted with one or more of oxo, and heterocycloalkyl. For example, R8 is cyclobutenyl substituted with one or more of oxo, and piperidinyl, piperazinyl, or morpholinyl. For example, R8 is cyclobutenyl substituted with one or more of oxo, and heterocycloalkyl, wherein heterocycloalkyl is further substituted, e.g., with one or more C1-3 alkyl. For example, R8 is cyclobutenyl substituted with one or more of oxo, and

heterocycloalkyl, wherein heterocycloalkyl (e.g., piperidinyl, piperazinyl, or morpholinyl) is further substituted with methyl.

In certain embodiments, Q is -NHR8, in which R8 is a heteroaryl optionally substituted with one or more substituents selected from amino (NH2), mono- or di-alkylamino, C1-3 alkyl and halo. For example, R8 is thiazole or imidazole.

In certain embodiments, Q is -NHR8 and R8 is purine.

In certain embodiments, Q is -NHC(=NR9)N(R)2 in which R9 is CN, Ci-6 alkyl, NO2, -S(0)2N(R)2, -OR, -S(0)2R, or H. For example, Q is - NHC(=NR9)N(CH3)2, -NHC(=NR9)NHCH3, -NHC(=NR9)NH2. In some embodiments, Q is -NHC(=NR9)N(R)2 in which R9 is CN and R is Ci-3 alkyl substituted with mono- or di-alkylamino, e.g., R is
((dimethylamino)ethyl)amino. In some embodiments, Q is - NHC(=NR9)N(R)2 in which R9 is Ci-6 alkyl, NO2, -S(0)2N(R)2, -OR, -S(0)2R, or H and R is Ci-3 alkyl substituted with mono- or di-alkylamino, e.g., R is ((dimethylamino)ethyl)amino.

In certain embodiments, Q is -NHC(=CHR9)N(R)2, in which R9 is NO2, CN, Ci-6 alkyl, -S(0)2N(R)2, -OR, -S(0)2R, or H. For example, Q is - NHC(=CHR9)N(CH3)2, -NHC(=CHR9)NHCH3, or -NHC(=CHR9)NH2.

In certain embodiments, Q is -OC(0)N(R)2, -N(R)C(0)OR, - N(OR)C(0)OR, such as -OC(0)NHCH3, -N(OH)C(0)OCH3, -N(OH)C(0)CH3, - N(0CH3)C(0)0CH3, -N(0CH3)C(0)CH3, -N(0H)S(0)2CH3, or -NHC(0)OCH3.

In certain embodiments, Q is -N(R)C(0)R, in which R is alkyl optionally substituted with Ci-3 alkoxyl or S(0)zCi-3 alkyl, in which z is 0, 1, or 2.

In certain embodiments, Q is an unsubstituted or substituted C6-10 aryl (such as phenyl) or C3-6 cycloalkyl.

In some embodiments, n is 1. In other embodiments, n is 2. In further embodiments, n is 3. In certain other embodiments, n is 4. In some embodiments, n is 5. For example, in
some embodiments, R4 is -(Cth^OH. For example, in some embodiments, R4 is -(CFh^OFl.

For example, in some embodiments, R4 is -(CFh^OFl. For example, in some embodiments, R4 is -(CH2)5OH. For example, in some embodiments, R4 is benzyl. For example, in some embodiments, R4 may be 4-methoxybenzyl.

In some embodiments, R4 is a C3-6 carbocycle. In some embodiments, R4 is a C3-6 cycloalkyl. For example, in some embodiments, R4 is cyclohexyl optionally substituted with e.g., OH, halo, C1-6 alkyl, etc. For example, in some embodiments, R4 is 2-hydroxy cyclohexyl.

In some embodiments, R is H.

In some embodiments, R is C1-3 alkyl substituted with mono- or dialkylamino, e.g.,

R is ((dimethylamino)ethyl)amino.

In some embodiments, R is C 1-6 alkyl substituted with one or more substituents selected from the group consisting of C 1-3 alkoxyl, amino, and C1-C3 dialkylamino.

In some embodiments, R is unsubstituted C1-3 alkyl or unsubstituted C2-3 alkenyl.

For example, in some embodiments R4 is -CH2CH(OH)CH3, - CH(CH3)CH20H, or -CH2CH(OH)CH2CH3.

In some embodiments, R is substituted C1-3 alkyl, e.g., CH2OH. For example, in some embodiments, R4 is -CH2CH(OH)CH2OH, -(CH2)3NHC(0)CH20H, - (CH2)3NHC(0)CH20Bn, -(CH2)20(CH2)20H, -(CTH^NHCTBOCTB, - (Ca^NHCTBOCTBCTB, CH2SCH3, CH2S(0)CH3, CH2S(0)2CH3, or -CH(CH2OH)2.

In some embodiments, R4 is selected from any of the following groups:

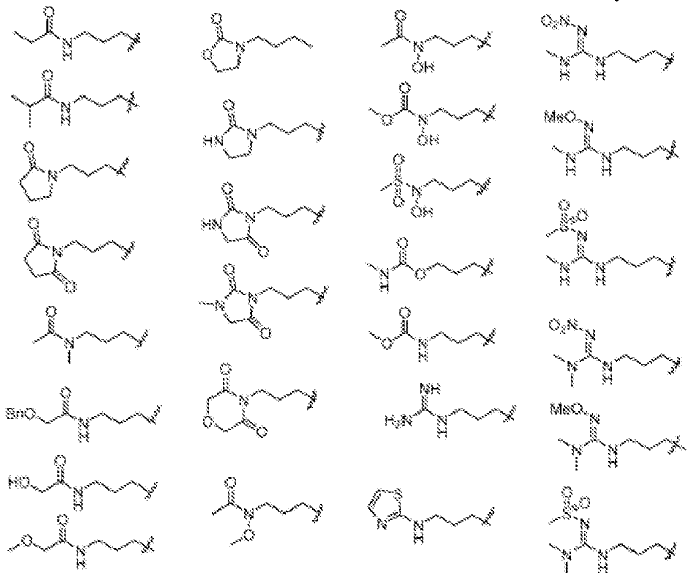

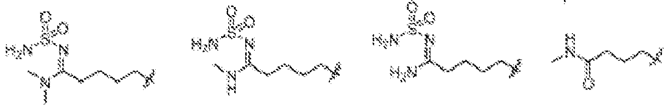

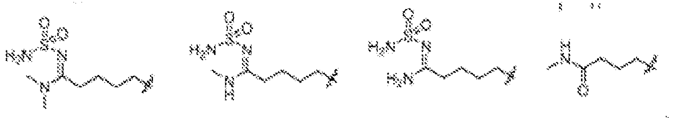

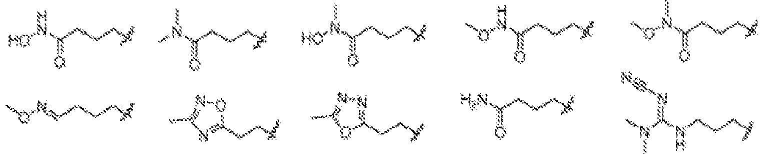

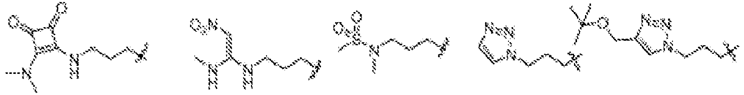

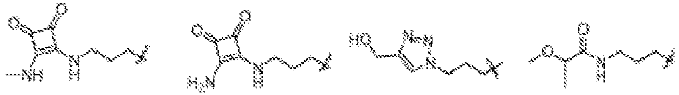

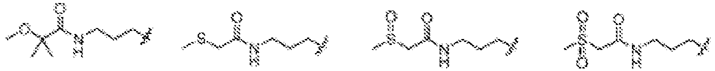

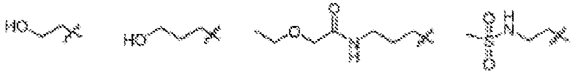

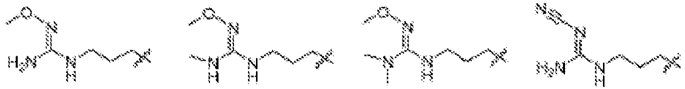

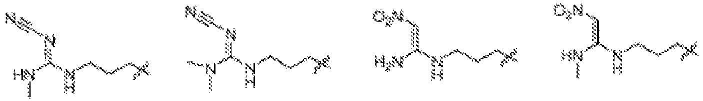

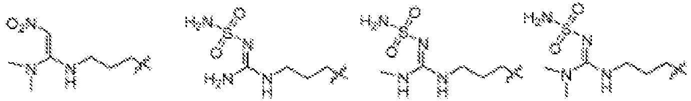

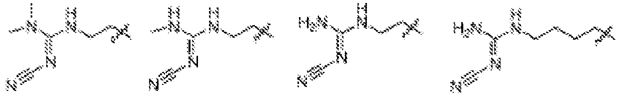

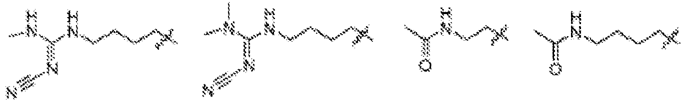

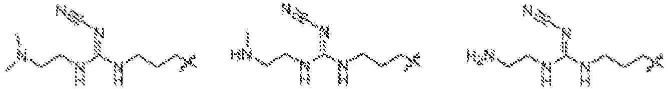

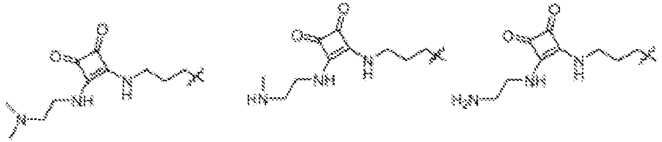

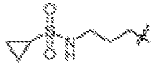

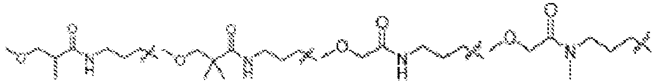

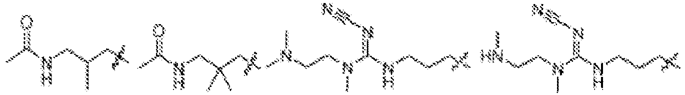

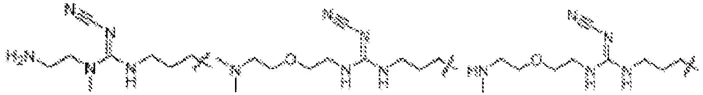

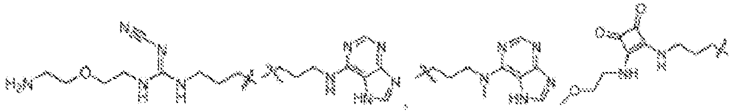

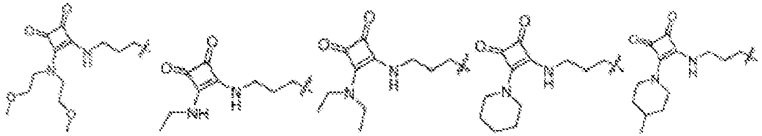

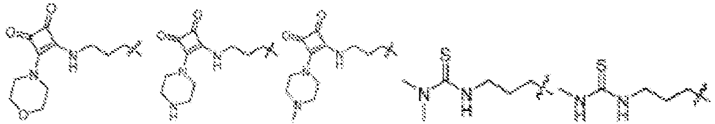

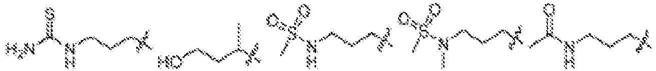

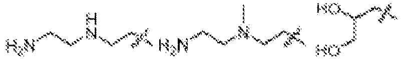

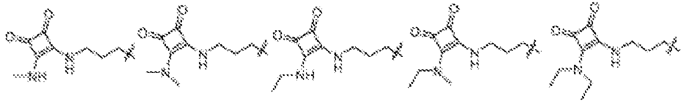

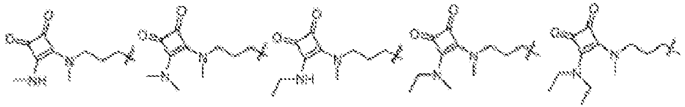

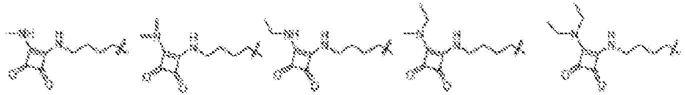

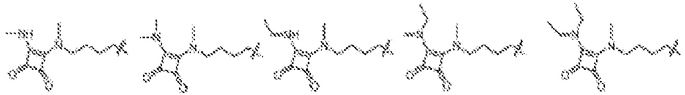

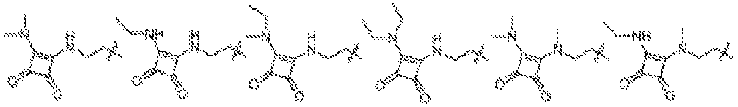

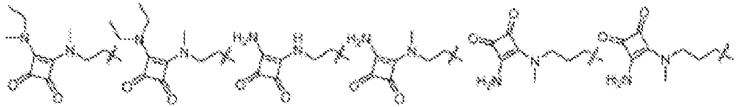

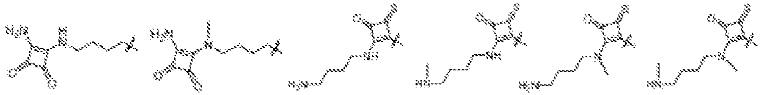

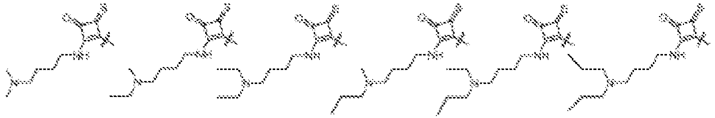

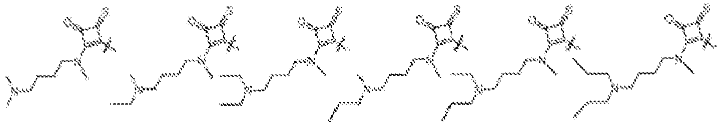

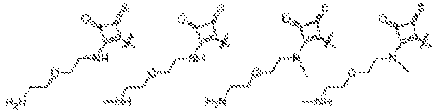

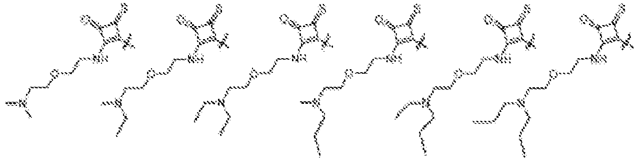

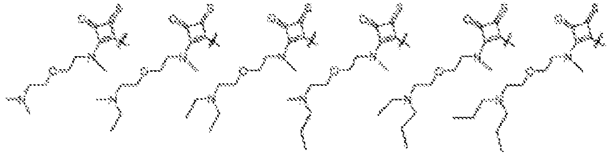

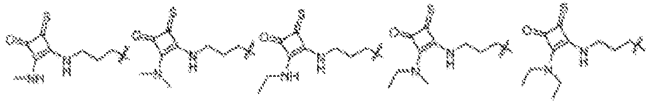

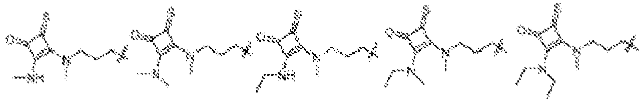

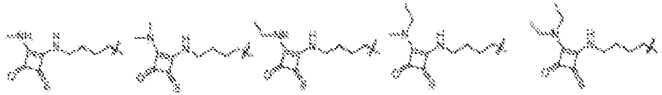

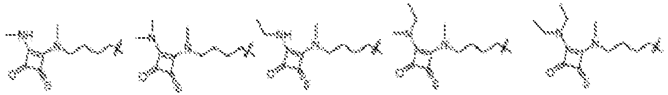

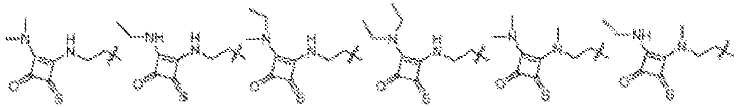

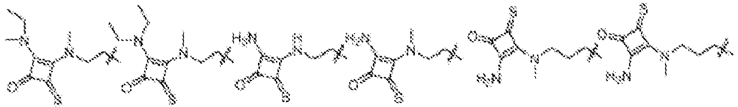

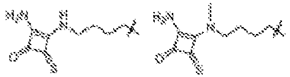

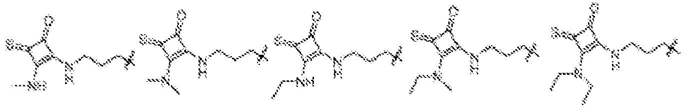

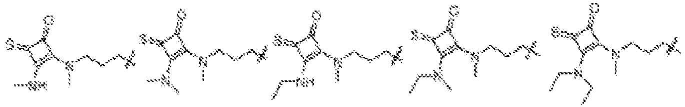

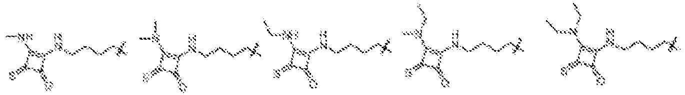

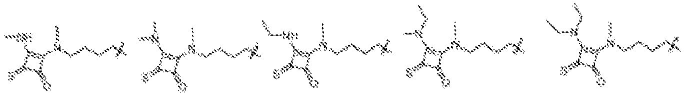

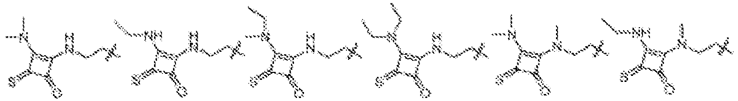

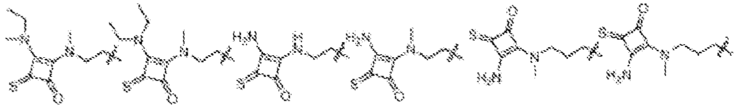

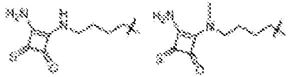

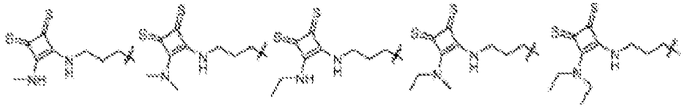

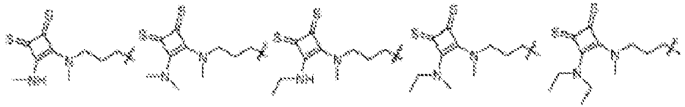

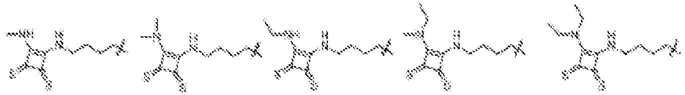

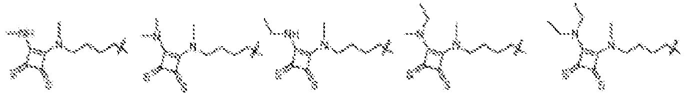

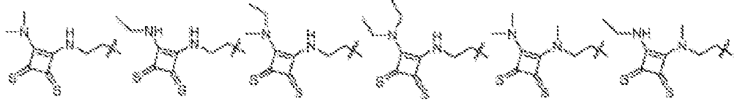

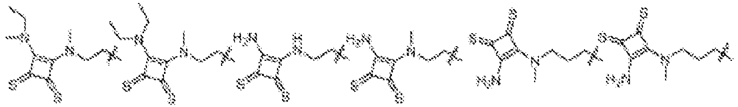

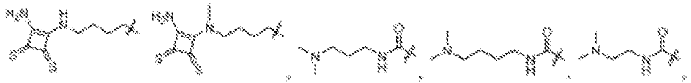

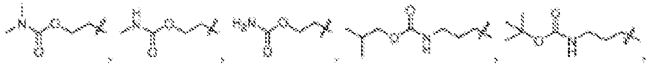

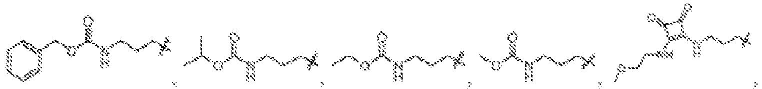

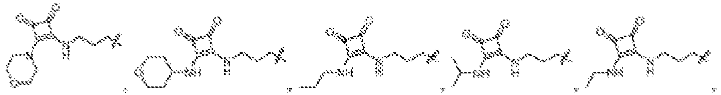

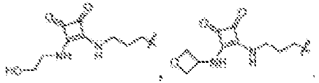

In some embodiments, 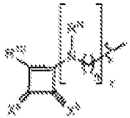 is selected from any of the following groups

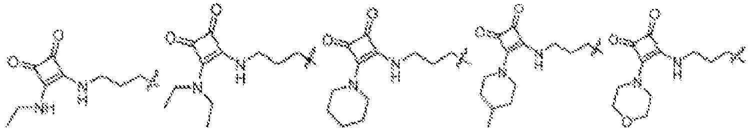

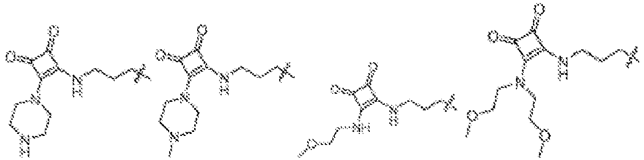

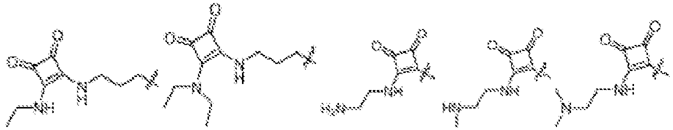

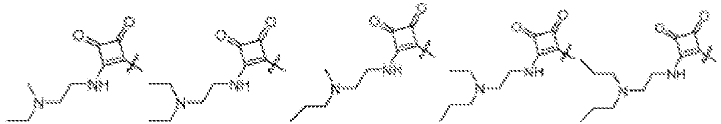

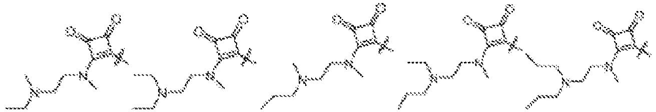

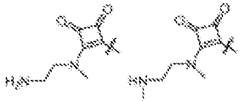

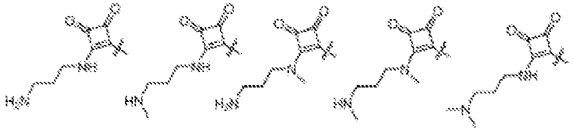

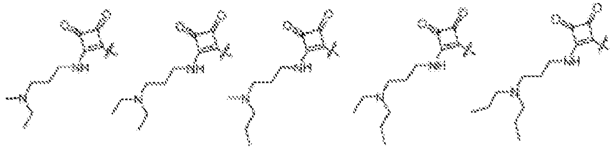

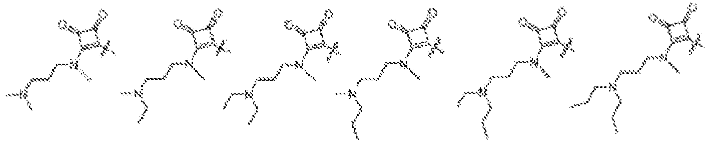

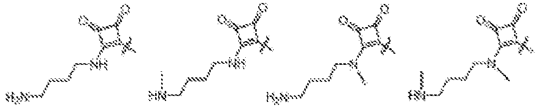

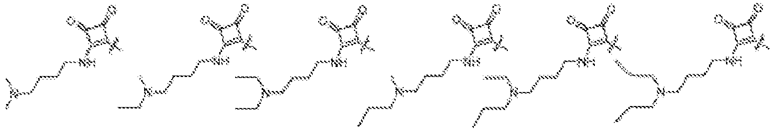

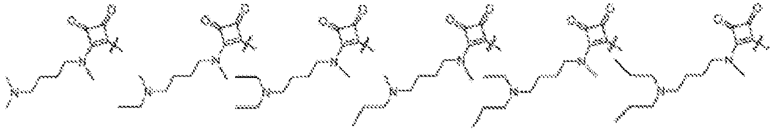

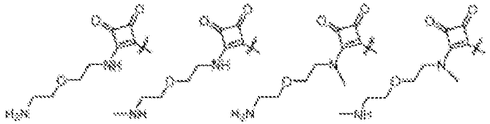

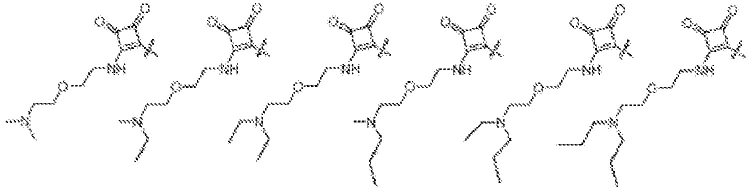

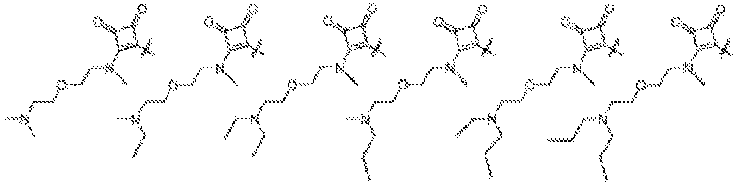

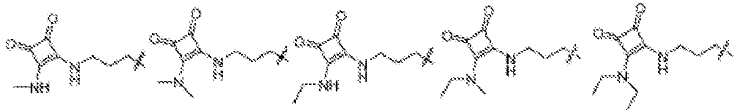

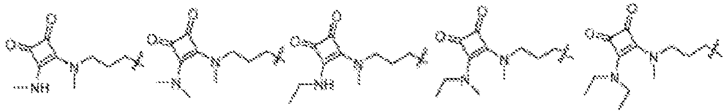

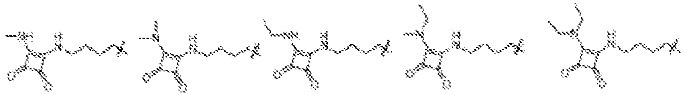

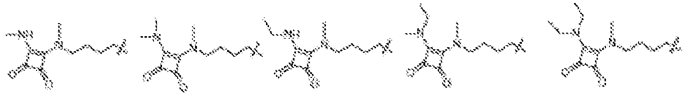

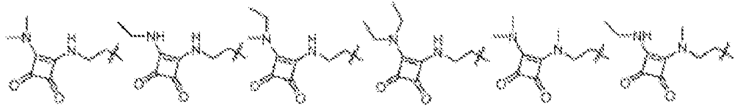

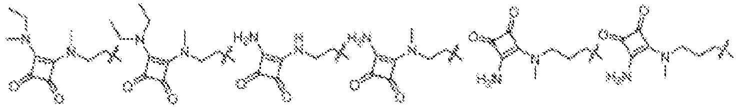

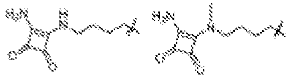

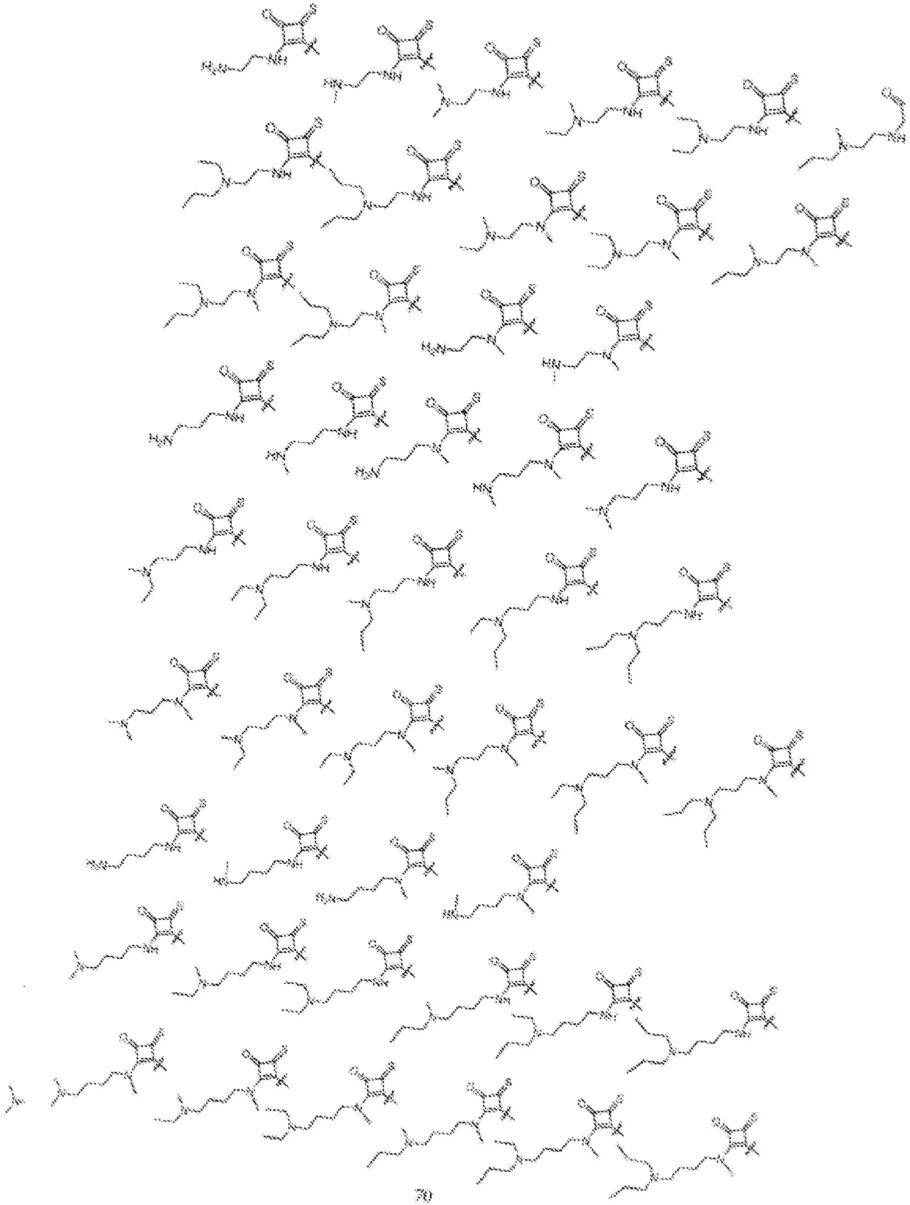

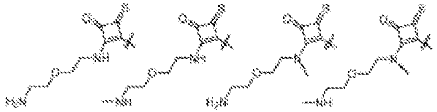

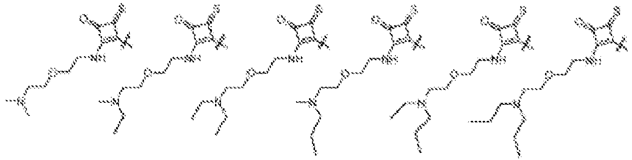

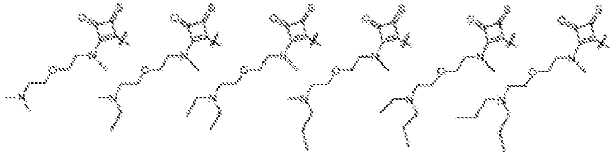

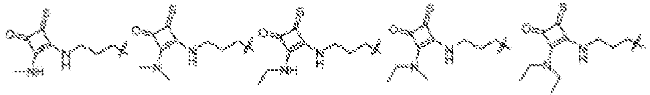

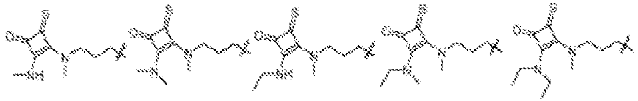

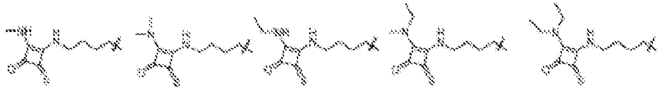

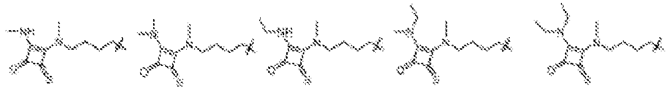

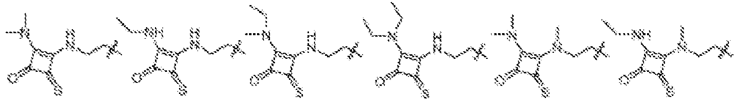

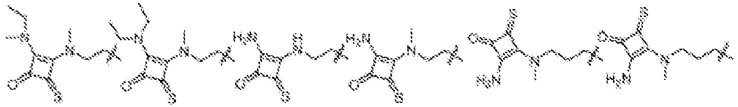

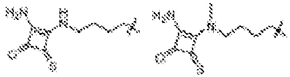

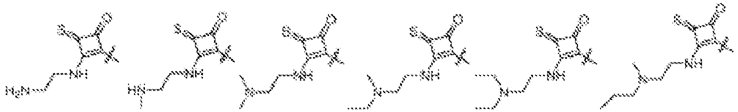

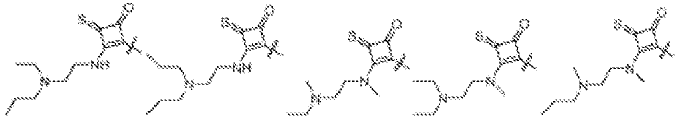

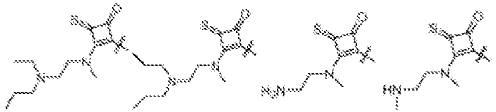

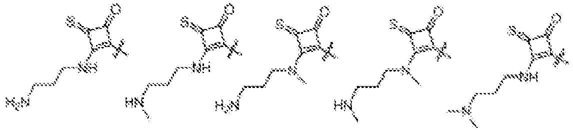

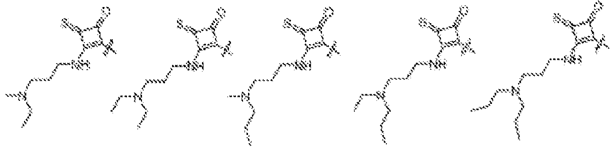

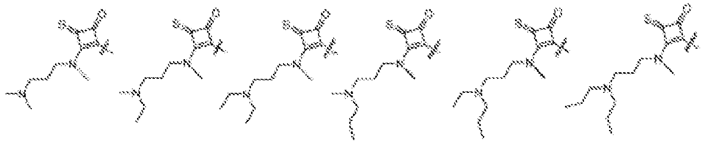

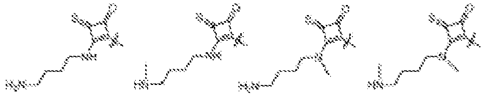

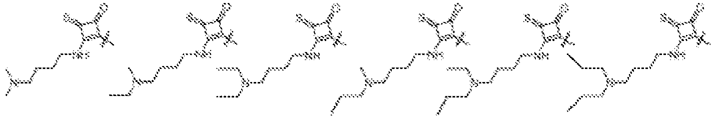

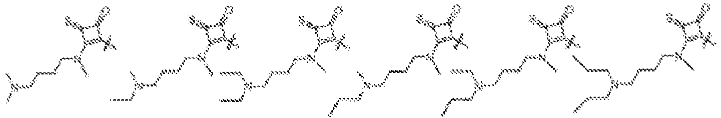

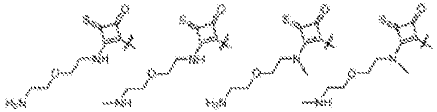

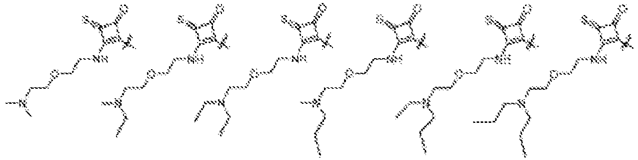

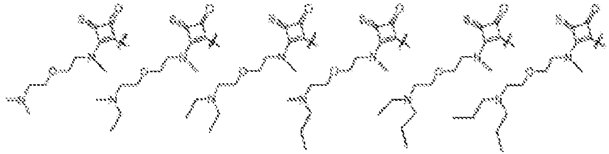

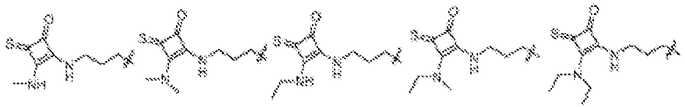

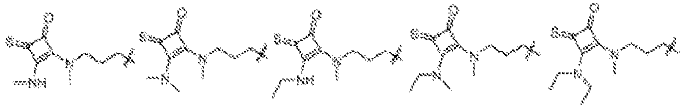

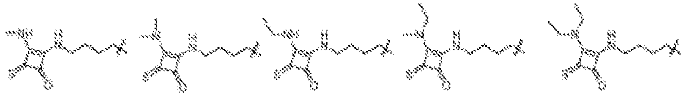

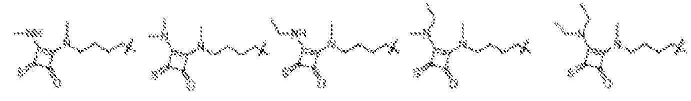

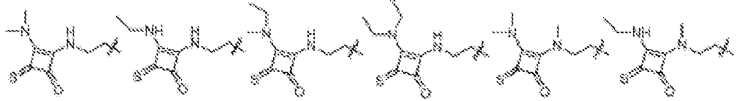

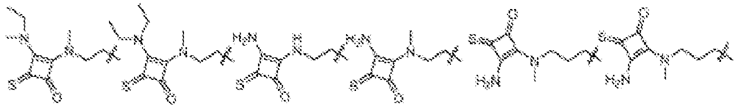

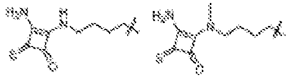

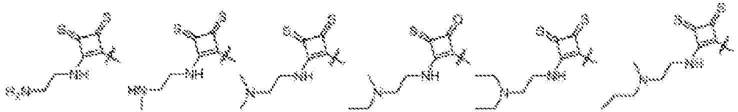

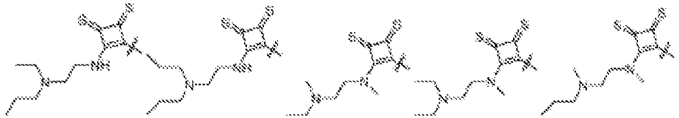

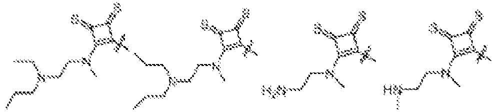

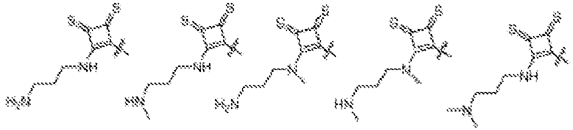

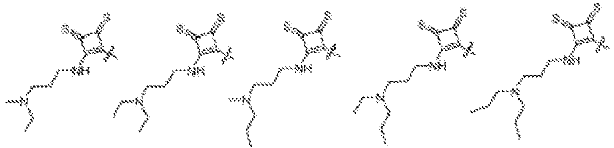

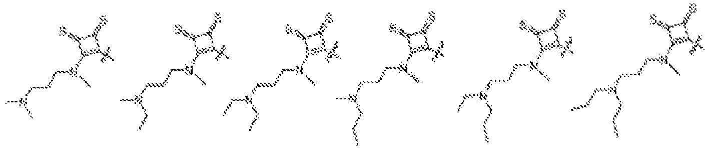

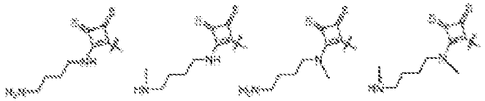

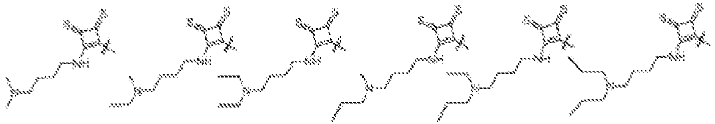

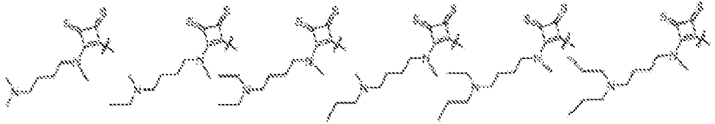

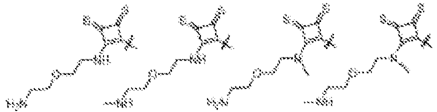

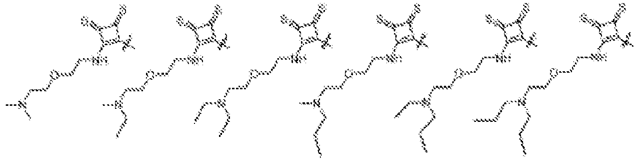

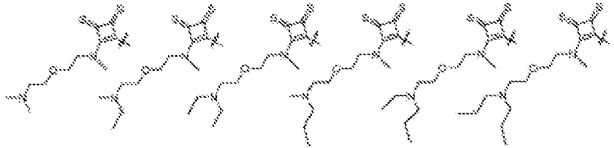

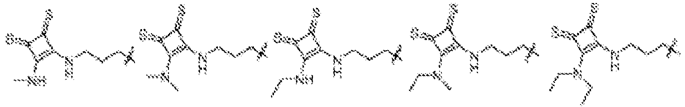

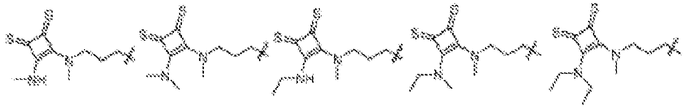

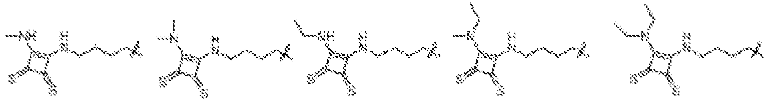

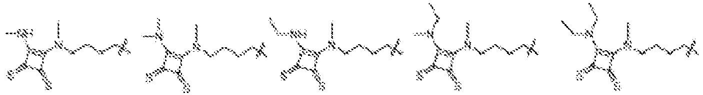

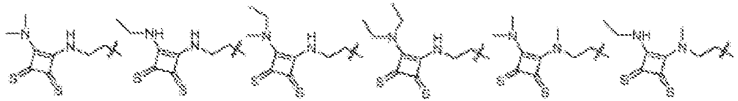

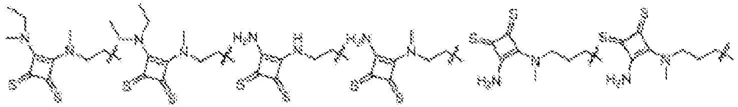

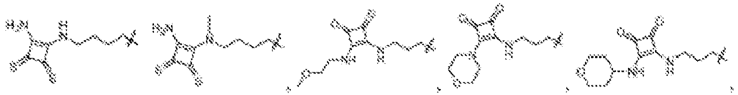

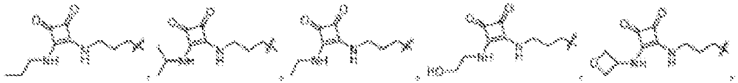

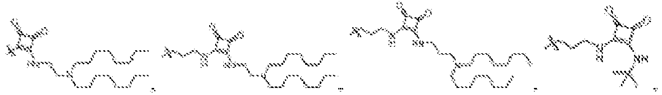

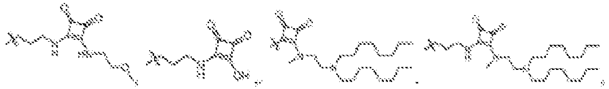

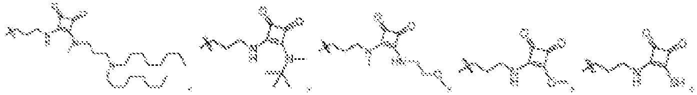

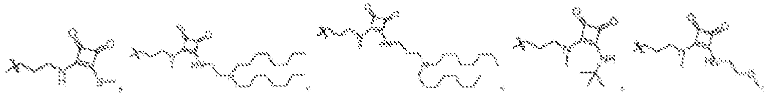

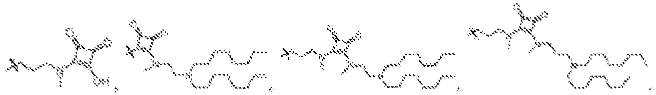

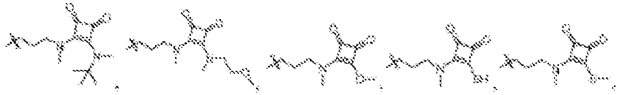

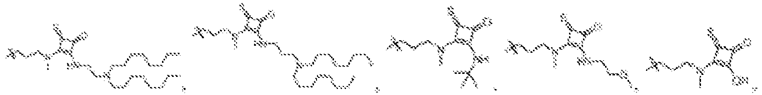

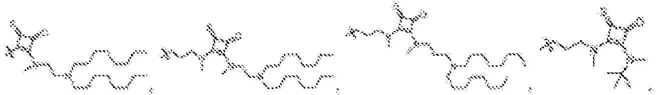

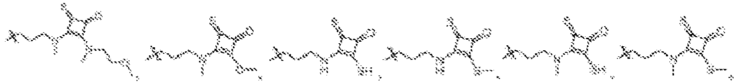

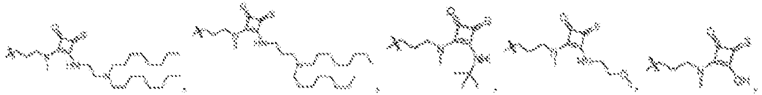

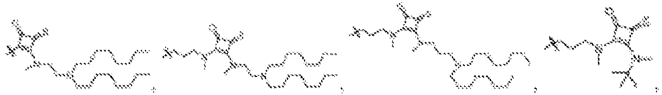

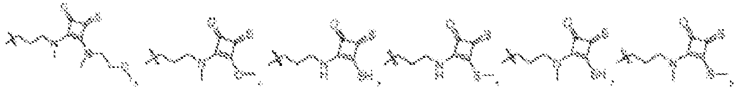

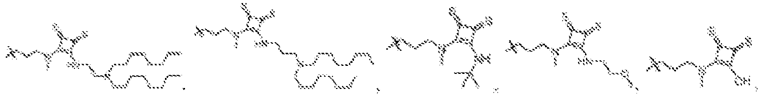

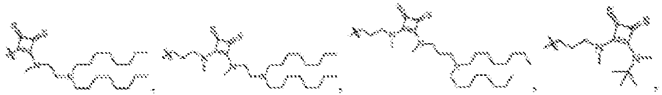

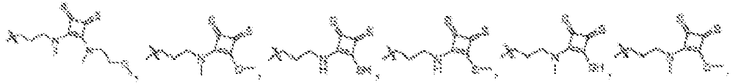

In some embodiments, R4 is selected from any of the following groups:

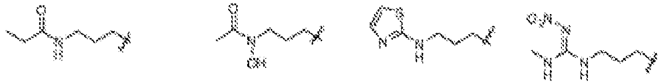

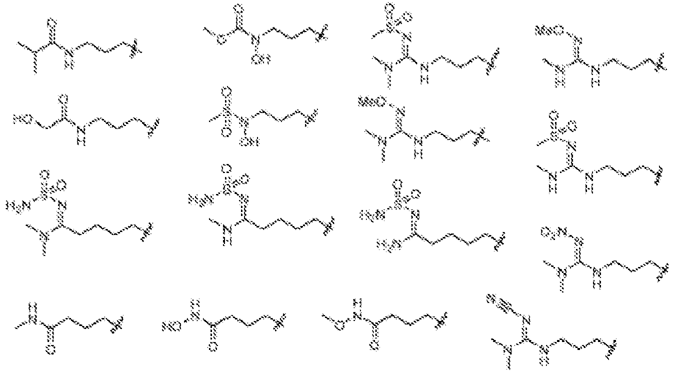

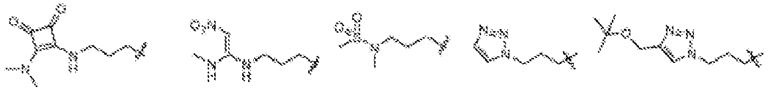

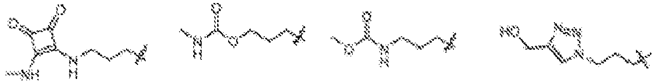

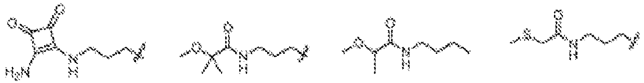

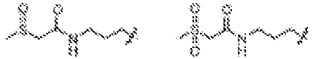

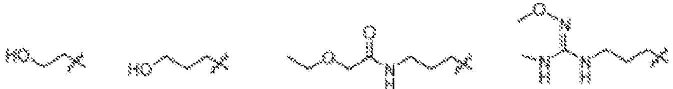

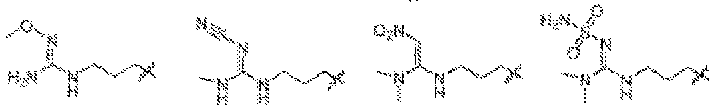

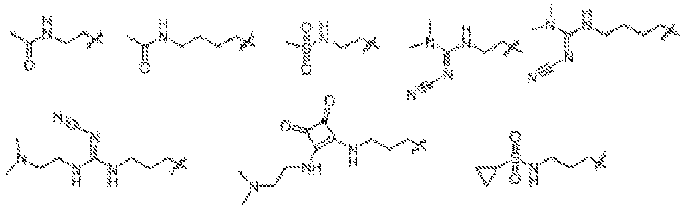

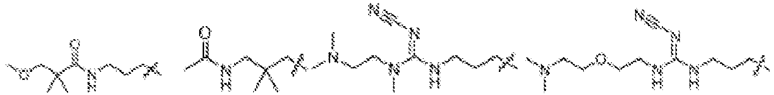

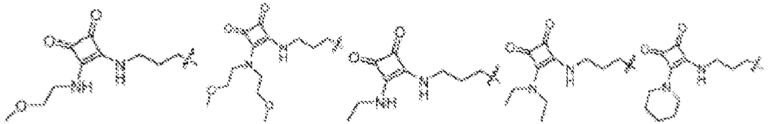

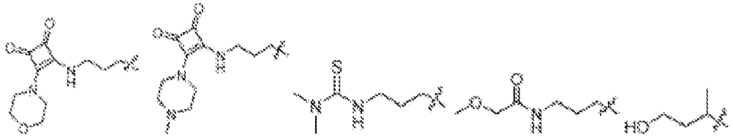

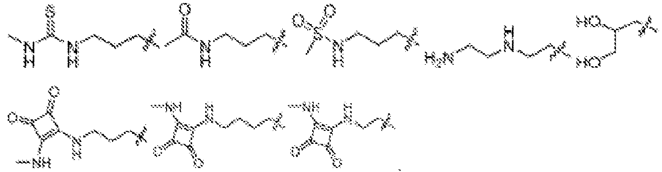

In some embodiments, 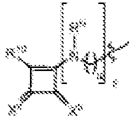 is selected from any of the following groups:

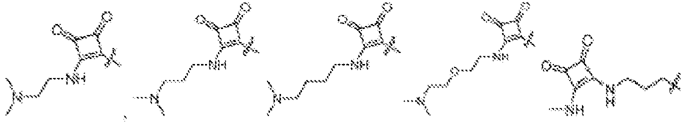

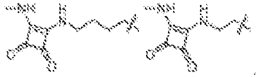

In some embodiments, R4 is selected from any of the following groups: 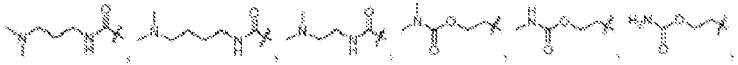 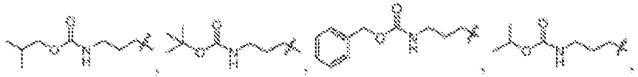 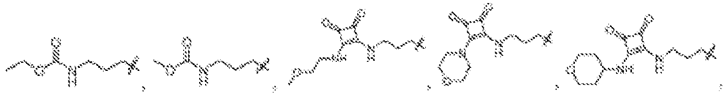 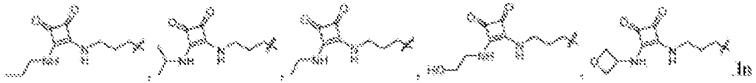 some embodiments, 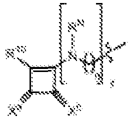 is selected from any of the following groups

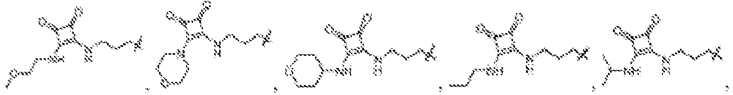

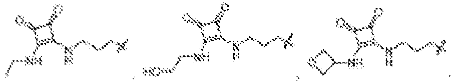

In some embodiments, 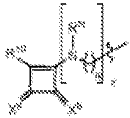 is selected from any of the following groups:

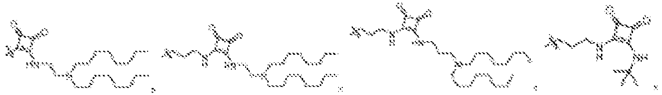

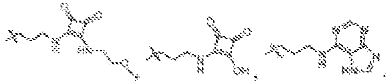

In a specific embodiment, the lipid compound has the following structure:

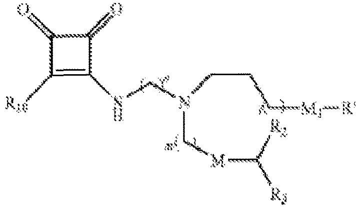

In one embodiment, R₁₀ is selected from the group consisting of hydroxyl, amino, alkylamino, dialkylamino, NH-heterocyclyl and heterocyclyl, wherein the alkyl portion of the alkylamino and dialkylamino are optionally substituted with hydroxyl, alkoxy, amino, alkylamino and/or dialkylamino.In one embodiment, the cationic lipid compound has the following structure:

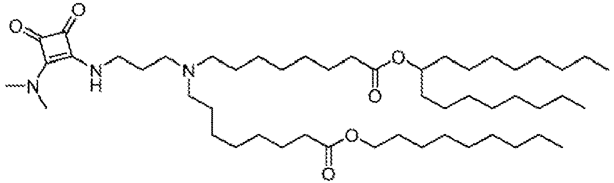

In another embodiment, the cationic lipid compound has the following structure:

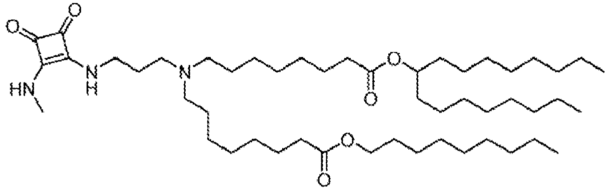

In yet another embodiment, the cationic lipid compound has the following structure:

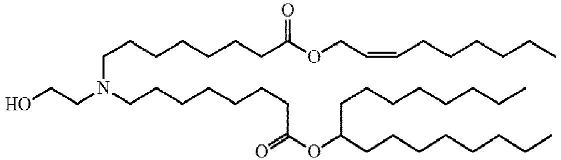

In one embodiment, the cationic lipid compound has the following structure:

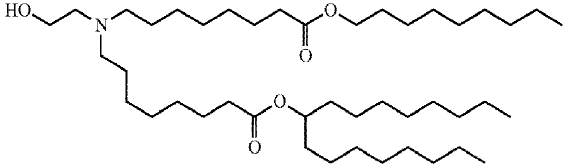

In another embodiment, the cationic lipid compound has the following structure:

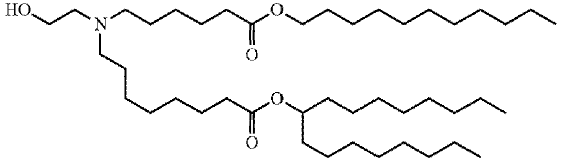

In yet another embodiment, the cationic lipid compound has the following structure:

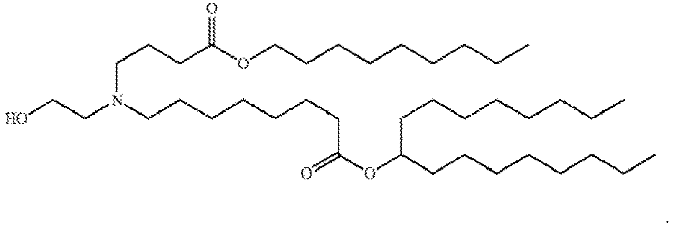

In some embodiments, the cationic lipid has one of the following structures:

| **Cpd** | **Structure** | **Cpd** | **Structure** |
|---|---|---|---|
| 1 | 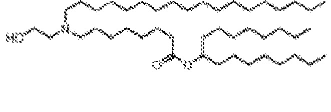 | 32 | 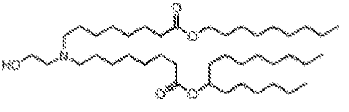 |
| 2 | 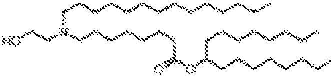 | 33 | 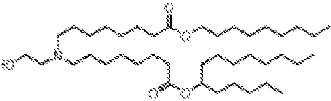 |
| 3 | 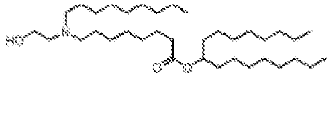 | 34 | 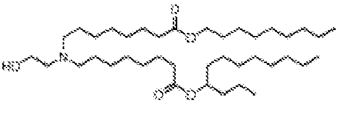 |
| 4 | 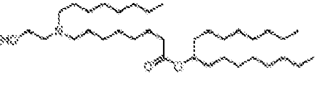 | 35 | 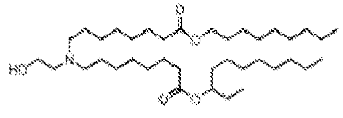 |
| 5 | 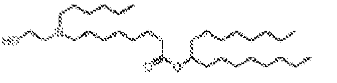 | 36 | 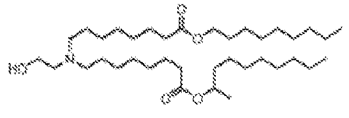 |
| 6 | 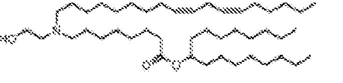 | 37 | 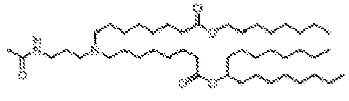 |
| 7 | 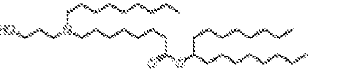 | 38 | 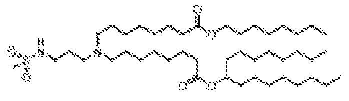 |
| 8 | 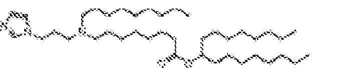 | 39 | 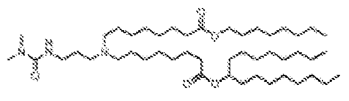 |
| 9 | 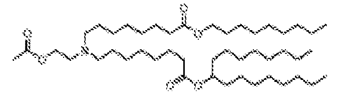 | 40 | 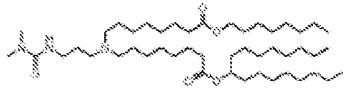 |
| 10 | 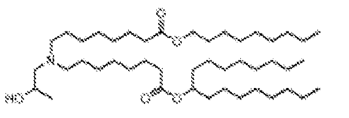 | 41 | 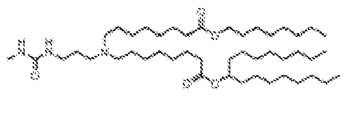 |
| 11 | 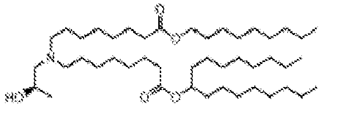 | 42 | 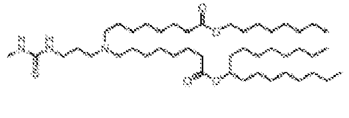 |
| 12 | 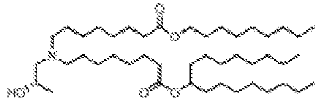 | 43 | 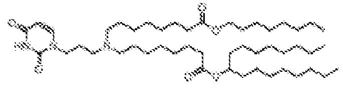 |
| 13 | 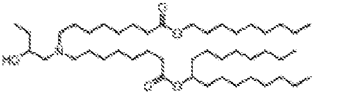 | 44 | 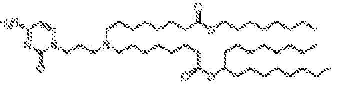 |
| 14 | 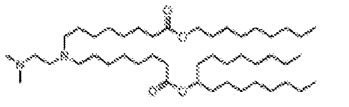 | 45 | 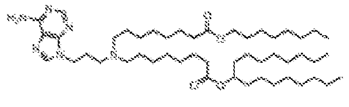 |
| 15 | 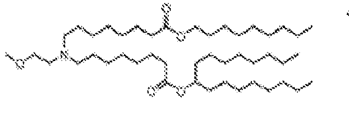 | 46 | 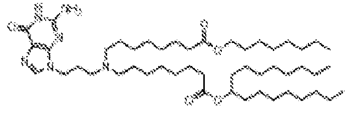 |
| 16 | 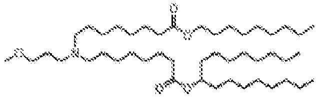 | 47 | 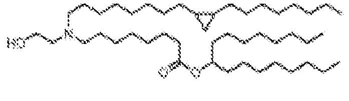 |
| 17 | 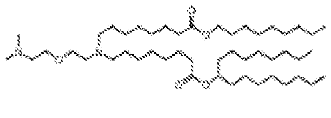 | 48 | 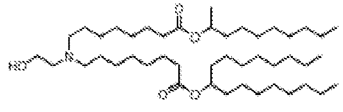 |
| 18 | 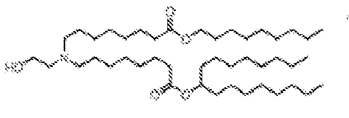 | 49 | 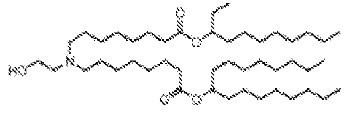 |
| 19 | 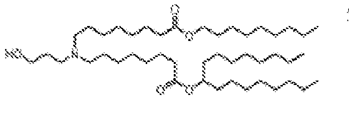 | 50 | 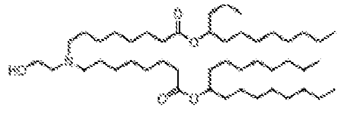 |
| 20 | 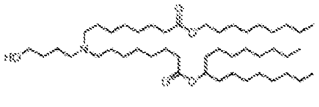 | 51 | 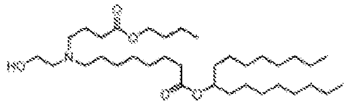 |
| 21 | 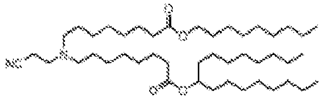 | 52 | 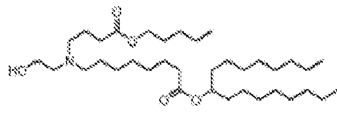 |
| 22 | 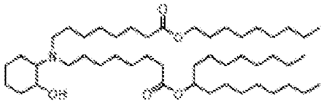 | 53 | 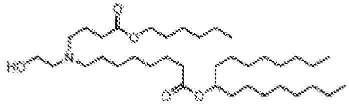 |
| 23 | 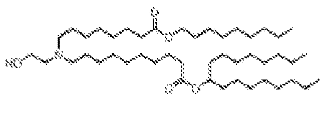 | 54 | 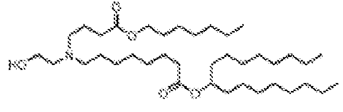 |
| 24 | 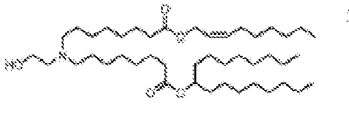 | 55 | 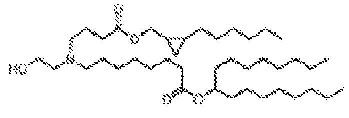 |
| 25 | 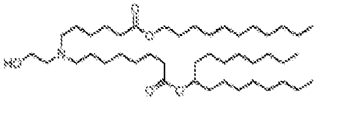 | 56 | 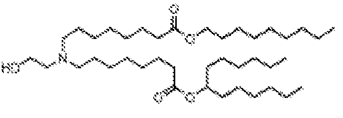 |
| 26 | 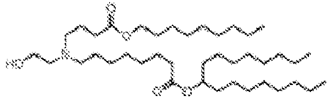 | 57 | 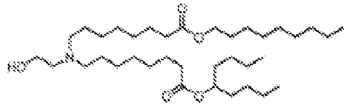 |
| 27 | 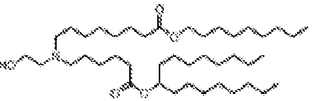 | 58 | 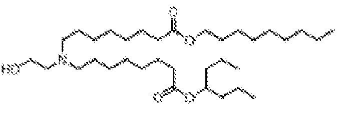 |
| 28 | 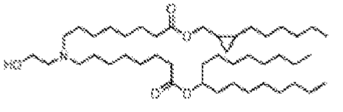 | 59 | 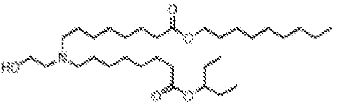 |
| 29 | 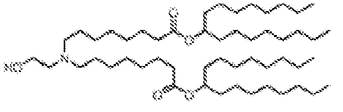 | 60 | 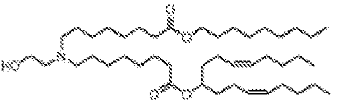 |
| 30 | 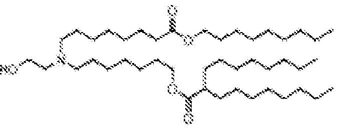 | 61 | 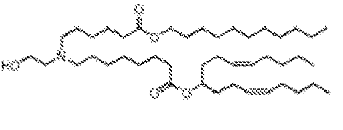 |
| 31 | 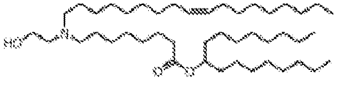 | | |

In further embodiments, the cationic lipid has one of the following structures:

| **Cpd** | **Structure** | **Cpd** | **Structure** |
|---|---|---|---|
| 62 | 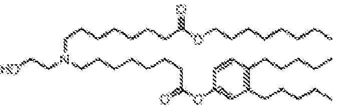 | 64 | 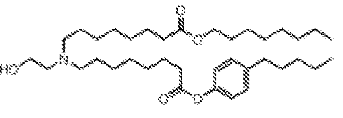 |

| | | | |
|---|---|---|---|
| 63 | 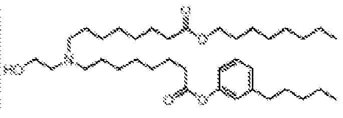 | | |

In some embodiments, the cationic lipid has one of the following structures:

| **Cpd** | **Structure** | **Cpd** | **Structure** |
|---|---|---|---|
| 65 | 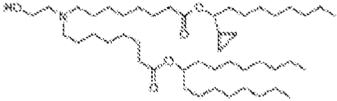 | M2 | 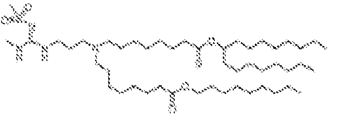 |
| 66 | 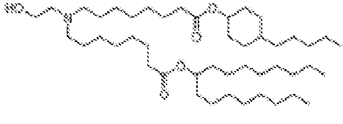 | 213 | 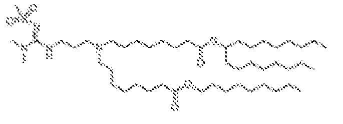 |
| 67 | 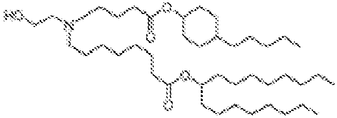 | 214 | 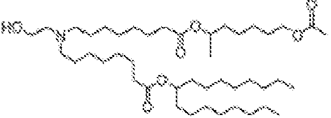 |
| 68 | 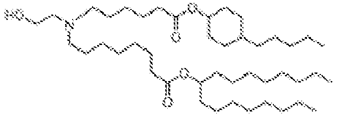 | 215 | 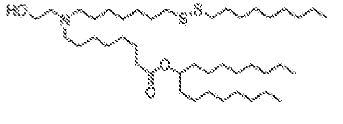 |
| 69 | 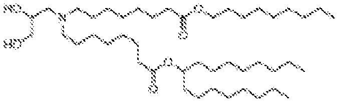 | 216 | 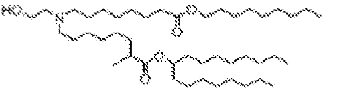 |
| 70 | 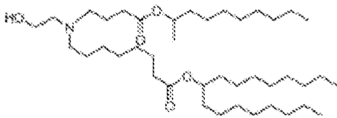 | 217 | 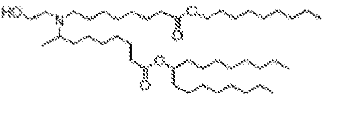 |
| 71 | 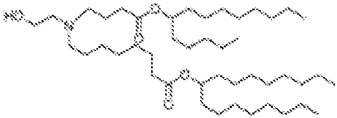 | 218 | 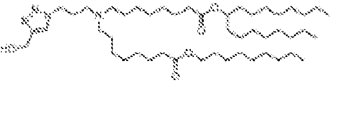 |
| 72 | 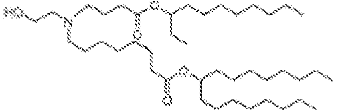 | 219 | 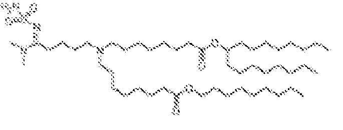 |
| 73 | 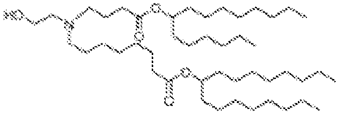 | 220 | 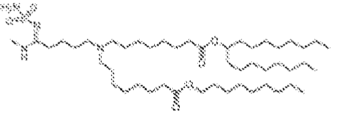 |
| 74 | 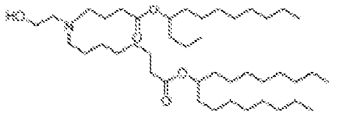 | 221 | 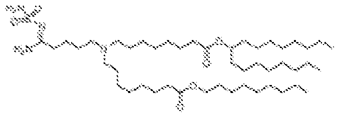 |
| 75 | 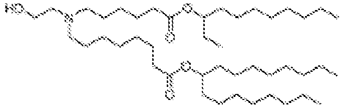 | 212 | 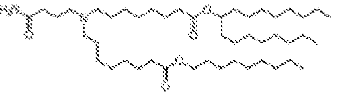 |
| 76 | 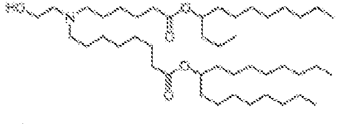 | 223 | 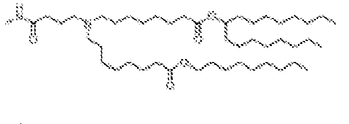 |
| 77 | 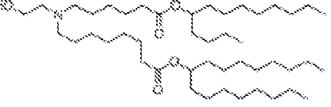 | 224 | 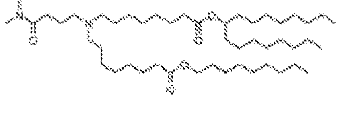 |
| 78 | 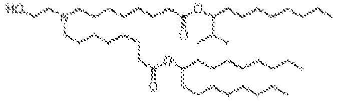 | 225 | 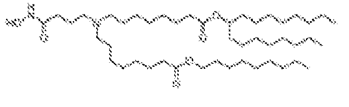 |
| 79 | 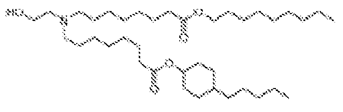 | 226 | 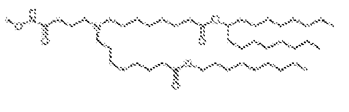 |
| 80 | 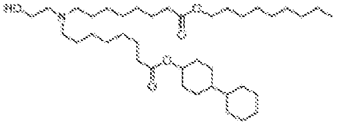 | 227 | 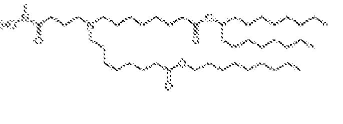 |
| 81 | 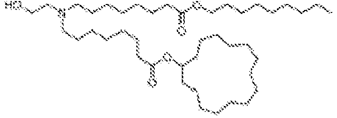 | 228 | 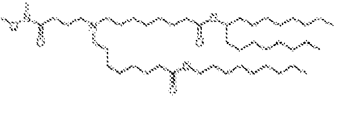 |
| 82 | 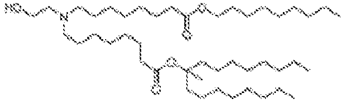 | 229 | 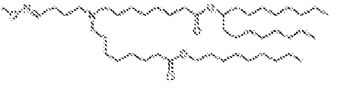 |
| 83 | 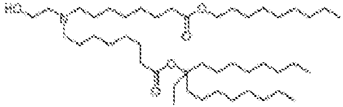 | 230 | 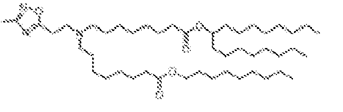 |
| 84 | 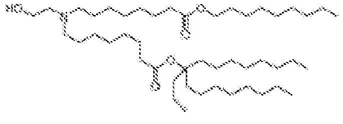 | 231 | 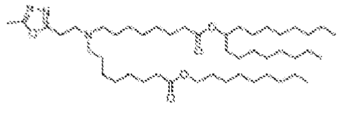 |
| 85 | 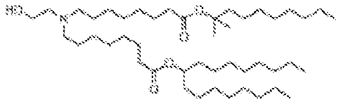 | 232 | 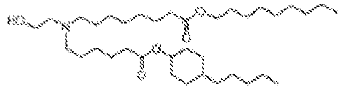 |
| 86 | 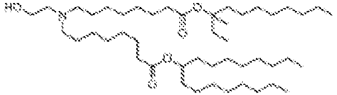 | 233 | 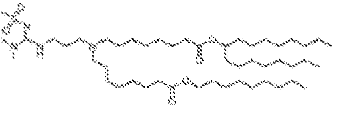 |
| 87 | 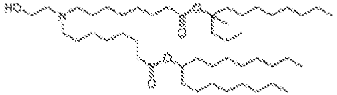 | 234 | 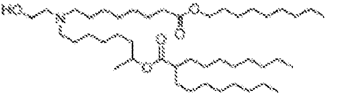 |
| 88 | 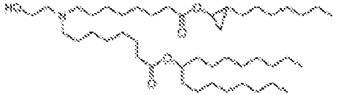 | 235 | 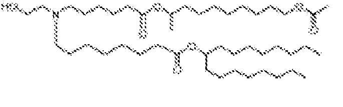 |
| 89 | 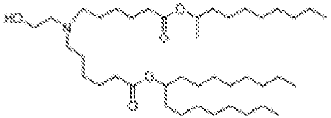 | 236 | 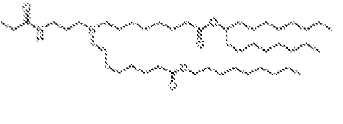 |
| 90 | 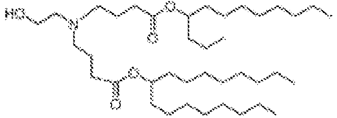 | 237 | 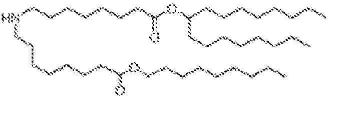 |
| 91 | 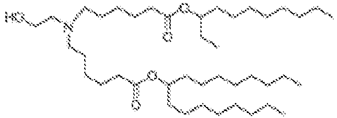 | 238 | 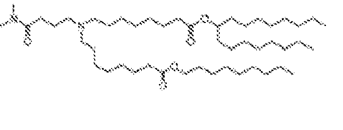 |
| 92 | 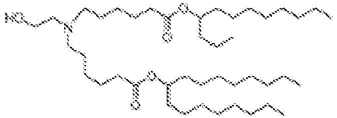 | 239 | 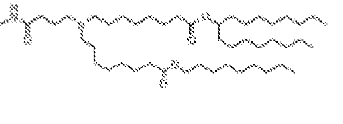 |
| 93 | 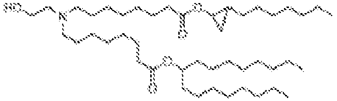 | 240 | 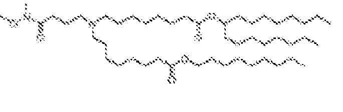 |
| 94 | 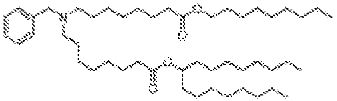 | 241 | 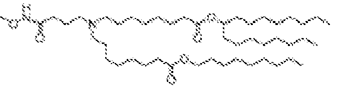 |
| 95 | 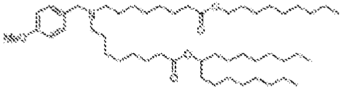 | 242 | 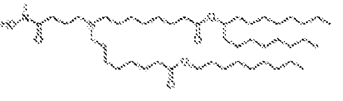 |
| 96 | 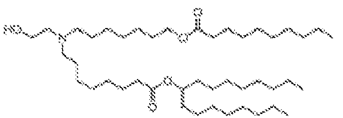 | 243 | 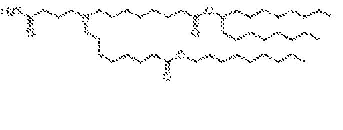 |
| 97 | 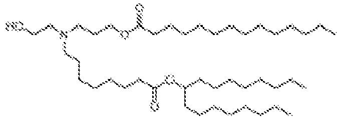 | 244 | 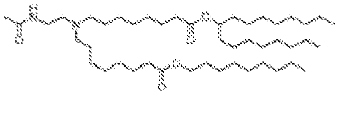 |
| 98 | 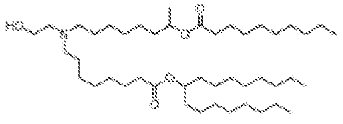 | 245 | 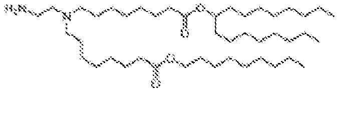 |
| 99 | 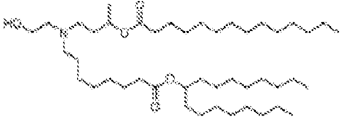 | 246 | 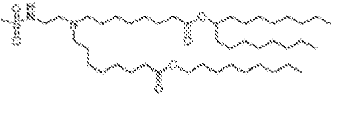 |
| 100 | 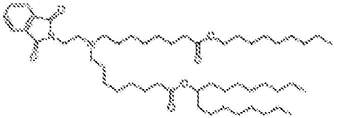 | 247 | 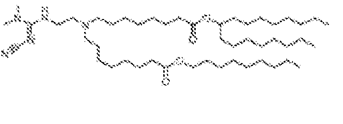 |
| 101 | 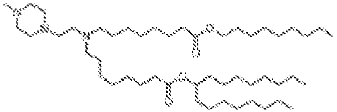 | 248 | 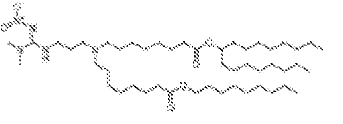 |
| 102 | 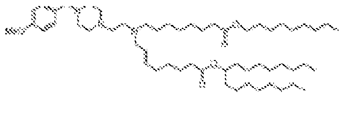 | 249 | 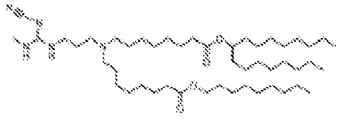 |
| 103 | 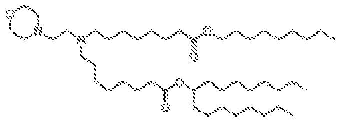 | 250 | 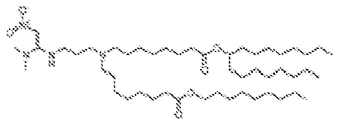 |
| 104 | 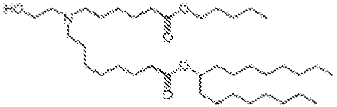 | 251 | 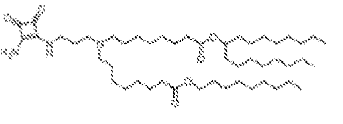 |
| 105 | 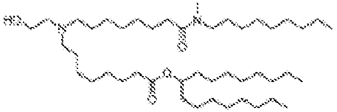 | 252 | 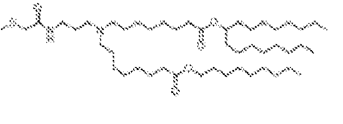 |
| 106 | 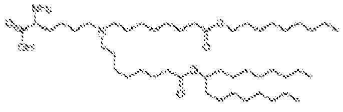 | 253 | 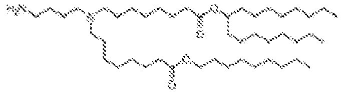 |
| 107 | 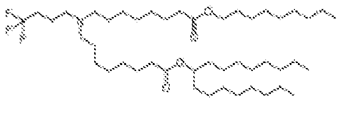 | 254 | 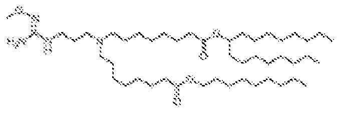 |
| 108 | 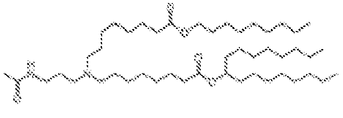 | 255 | 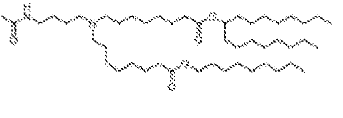 |
| 109 | 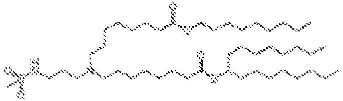 | 256 | 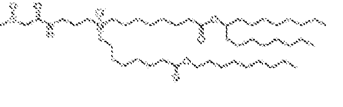 |
| 110 | 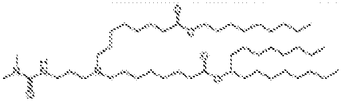 | 257 | 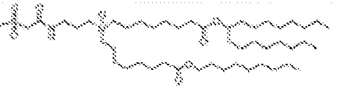 |
| 111 | 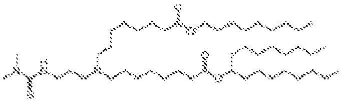 | 258 | 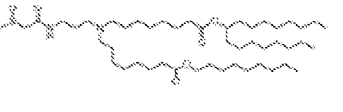 |
| 112 | 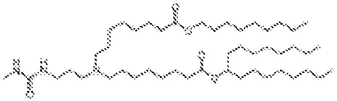 | 259 | 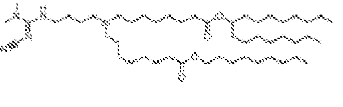 |
| 113 | 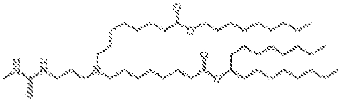 | 260 | 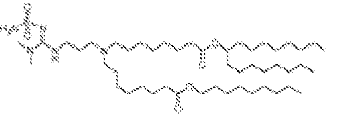 |
| 114 | 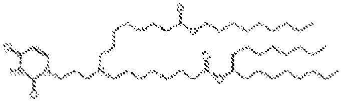 | 261 | 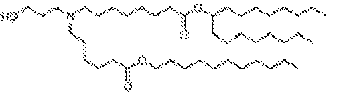 |
| 115 | 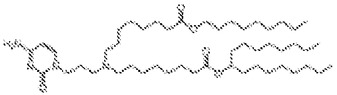 | 262 | 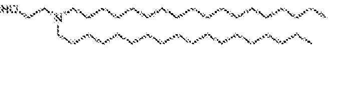 |
| 116 | 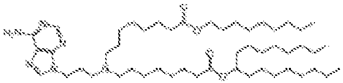 | 263 | 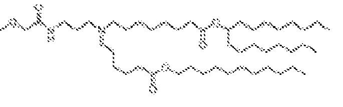 |
| 117 | 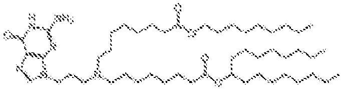 | 264 | 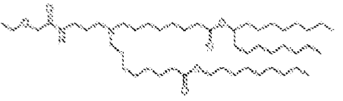 |
| 118 | 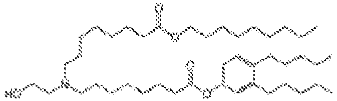 | 265 | 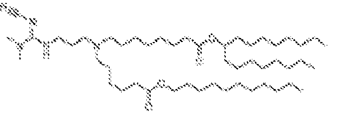 |
| 119 | 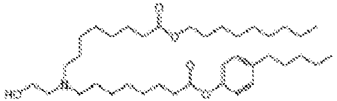 | 266 | 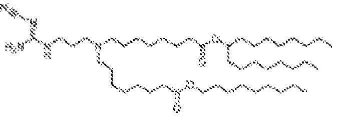 |
| 120 | 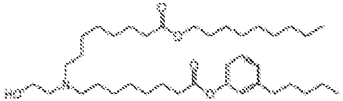 | 267 | 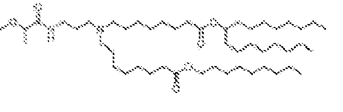 |
| 121 | 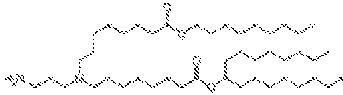 | 26S | 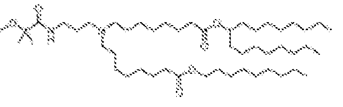 |
| 122 | 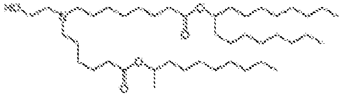 | 269 | 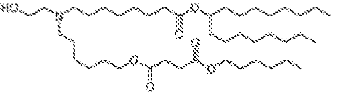 |
| 123 | 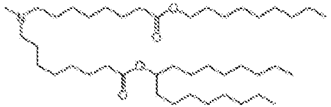 | 270 | 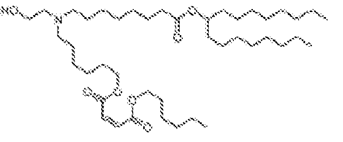 |
| 124 | 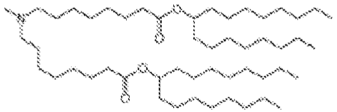 | 271 | 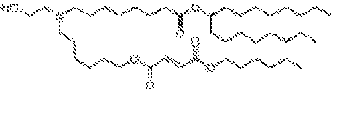 |
| 125 | 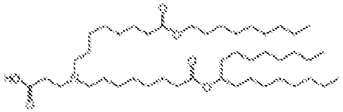 | 272 | 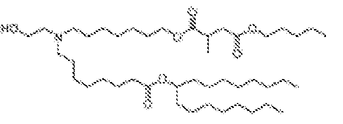 |
| 126 | 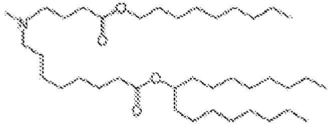 | 273 | 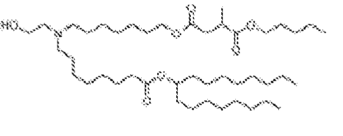 |
| 127 | 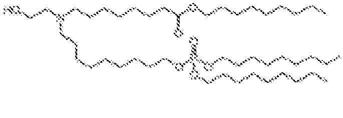 | 274 | 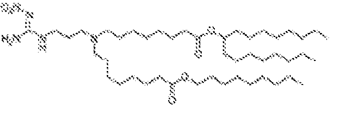 |
| 128 | 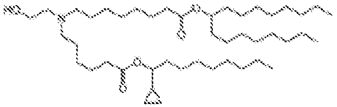 | 275 | 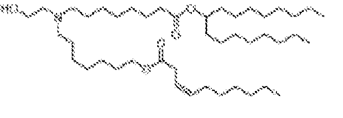 |
| 129 | 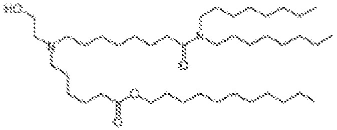 | 276 | 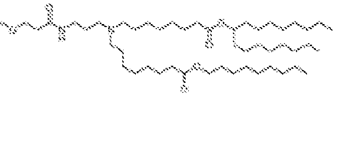 |
| 130 | 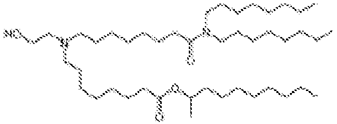 | 277 | 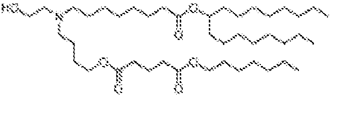 |
| 131 | 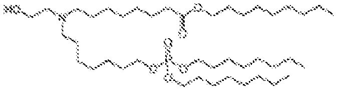 | 278 | 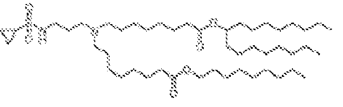 |
| 132 | 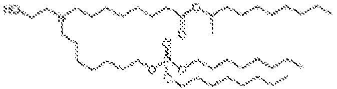 | 279 | 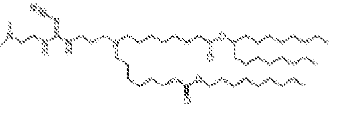 |
| 133 | 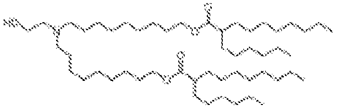 | 280 | 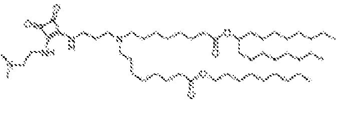 |
| 134 | 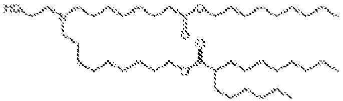 | 281 | 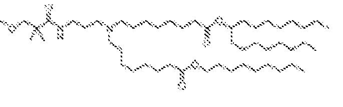 |
| 135 | 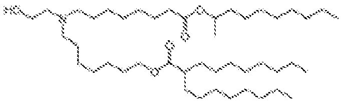 | 282 | 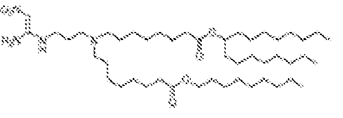 |
| 136 | 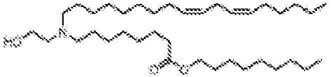 | 283 | 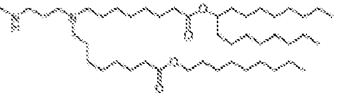 |
| 137 | 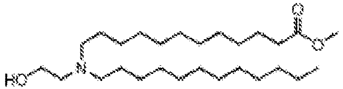 | 284 | 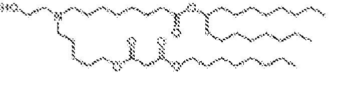 |
| 138 | 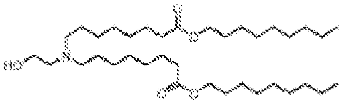 | 285 | 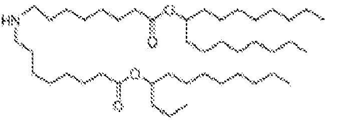 |
| 139 | 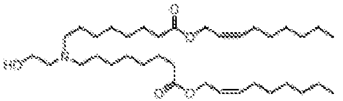 | 286 | 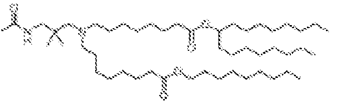 |
| 140 | 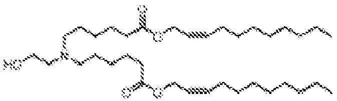 | 287 | 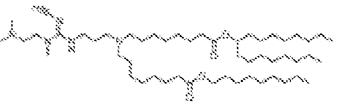 |
| 141 | 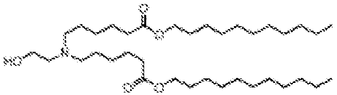 | 288 | 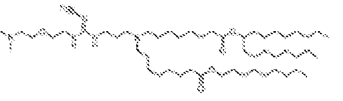 |
| 142 | 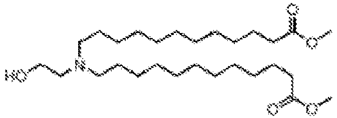 | 289 | 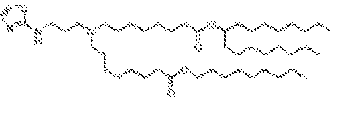 |
| 143 | 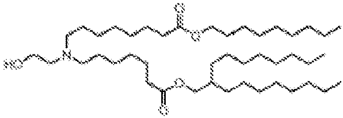 | 290 | 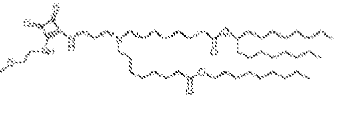 |
| 144 | 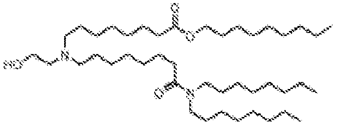 | 291 | 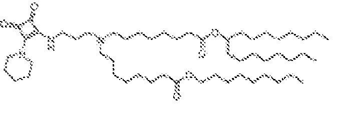 |
| 145 | 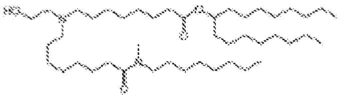 | 292 | 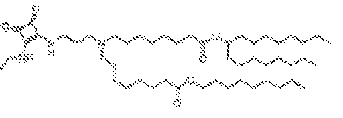 |
| 146 | 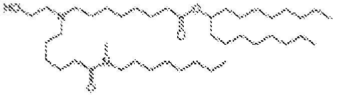 | 293 | 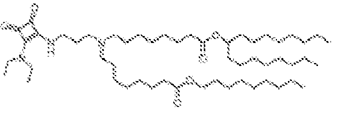 |
| 147 | 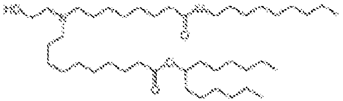 | 294 | 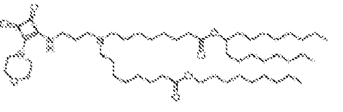 |
| 148 | 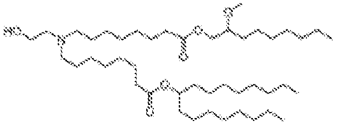 | 295 | 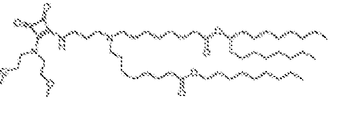 |
| i49 | 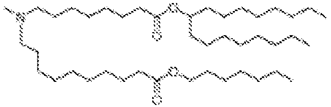 | 296 | 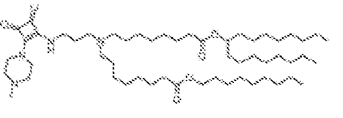 |
| 150 | 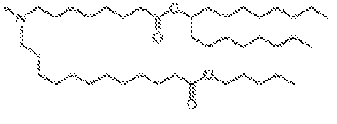 | 297 | 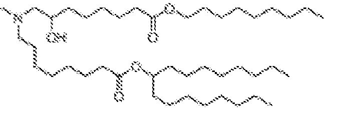 |
| 151 | 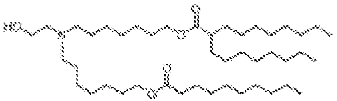 | 298 | 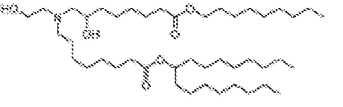 |
| 152 | 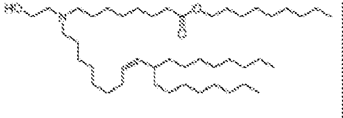 | 299 | 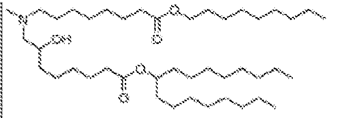 |
| 153 | 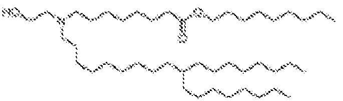 | 300 | 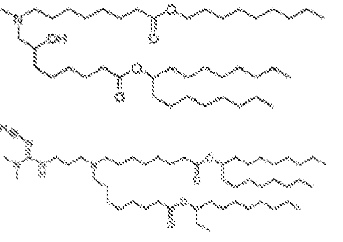 |
| 154 | 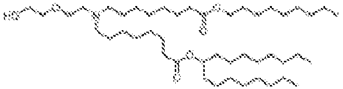 | 301 | 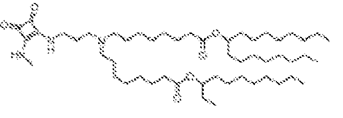 |
| 155 | 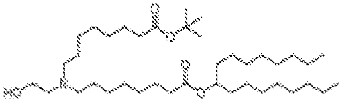 | 302 | 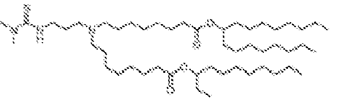 |
| 156 | 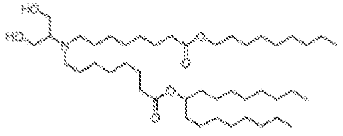 | 303 | 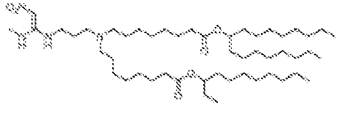 |
| 157 | 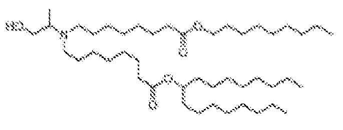 | 304 | 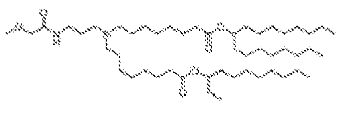 |
| 158 | 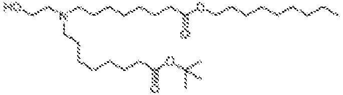 | 305 | 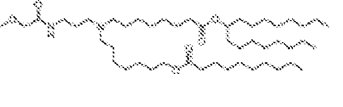 |
| 159 | 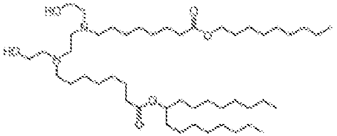 | 306 | 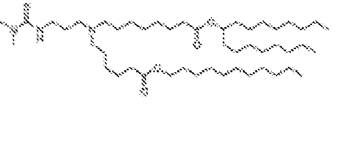 |
| 160 | 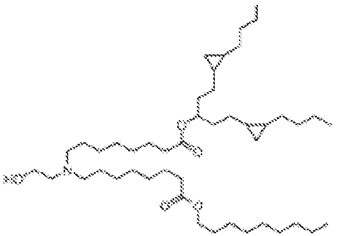 | 307 | 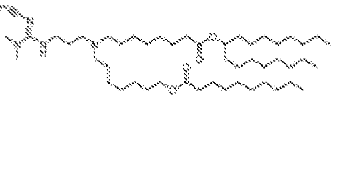 |
| 161 | 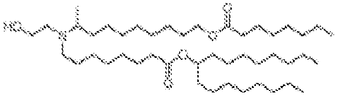 | 308 | 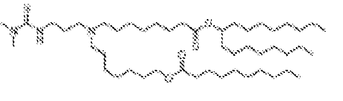 |
| 162 | 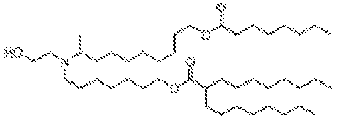 | 309 | 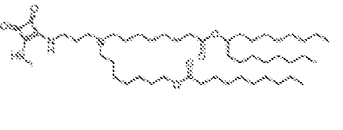 |
| 163 | 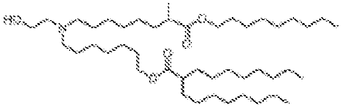 | 310 | 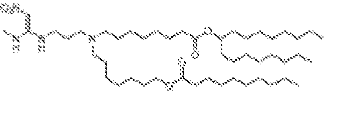 |
| 164 | 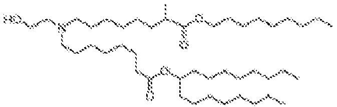 | 311 | 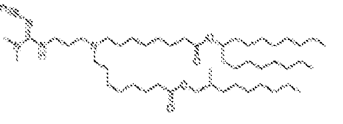 |
| 165 | 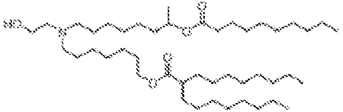 | 312 | 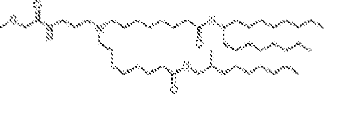 |
| 166 | 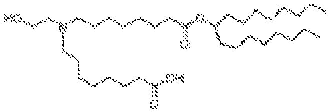 | 313 | 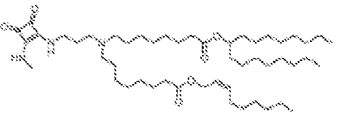 |
| 167 | 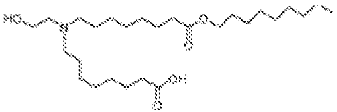 | 314 | 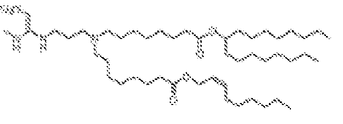 |
| 168 | 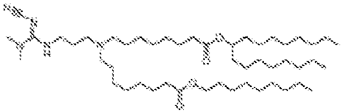 | 315 | 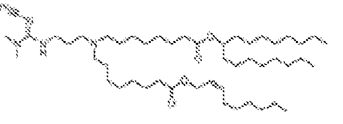 |
| 168 | 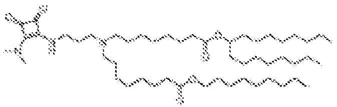 | 316 | 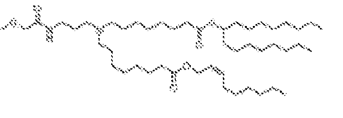 |
| 170 | 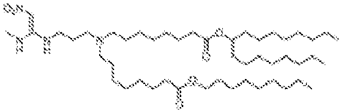 | 317 | 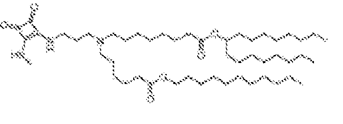 |
| 171 | 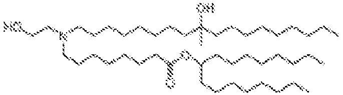 | 318 | 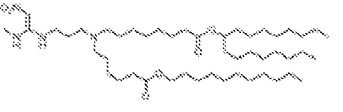 |
| 172 | 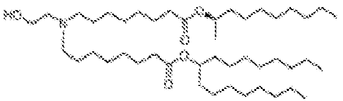 | 319 | 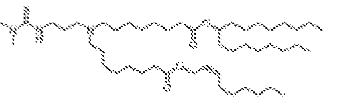 |
| 173 | 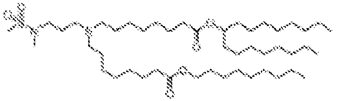 | 320 | 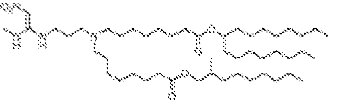 |
| 174 | 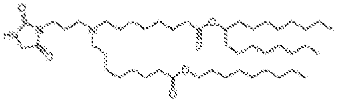 | 321 | 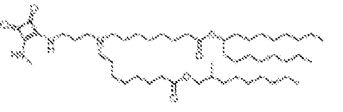 |
| 175 | 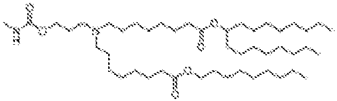 | 322 | 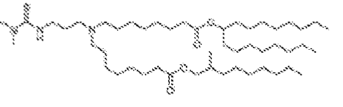 |
| 176 | 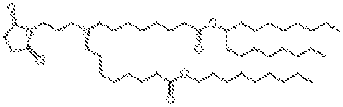 | 323 | 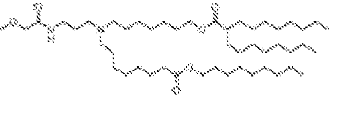 |
| 177 | 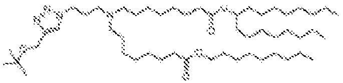 | 324 | 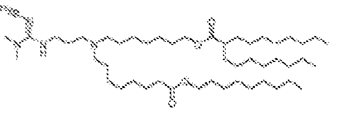 |
| 178 | 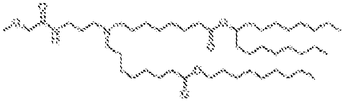 | 325 | 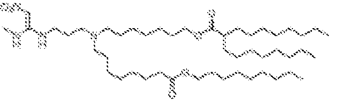 |
| 179 | 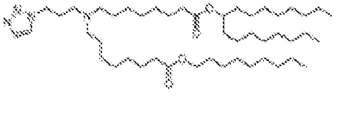 | 326 | 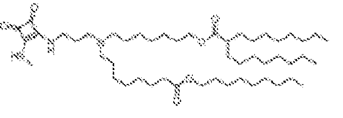 |
| 180 | 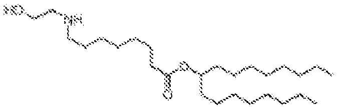 | 327 | 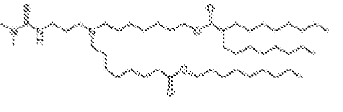 |
| 181 | 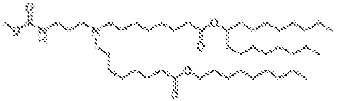 | 328 | 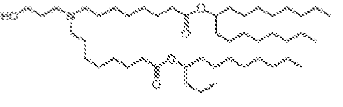 |
| 182 | 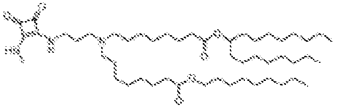 | 329 | 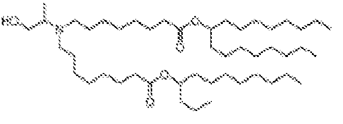 |
| 183 | 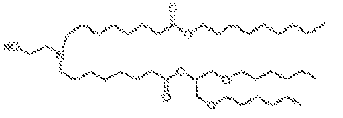 | 330 | 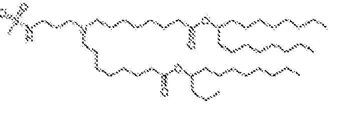 |
| 184 | 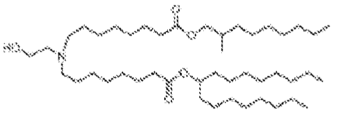 | 331 | 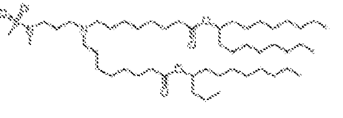 |
| 185 | 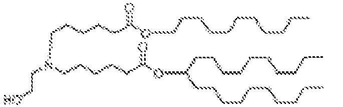 | 332 | 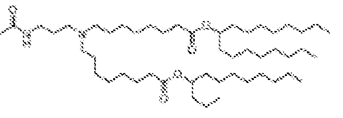 |
| 186 | 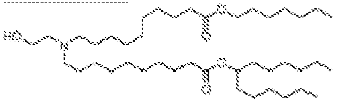 | 333 | 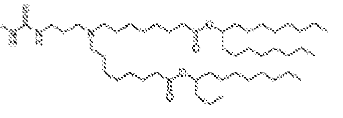 |
| 187 | 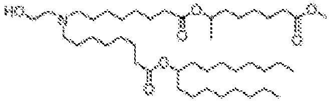 | 334 | 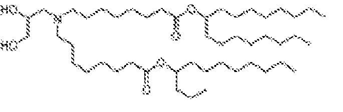 |
| 189 | 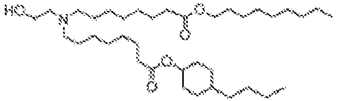 | 335 | 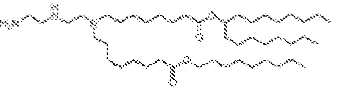 |
| 189 | 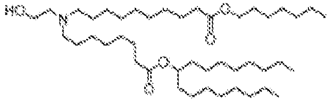 | 336 | 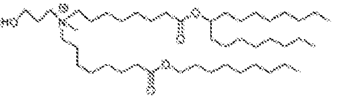 |
| 190 | 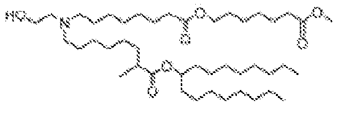 | 337 | 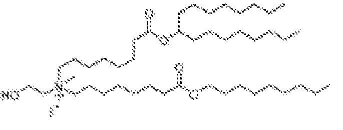 |
| 191 | 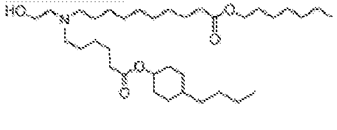 | 338 | 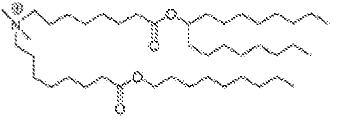 |
| 192 | 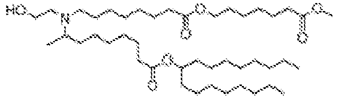 | 339 | 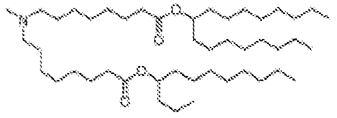 |
| 193 | 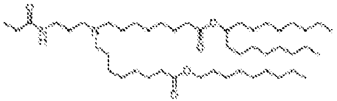 | 340 | 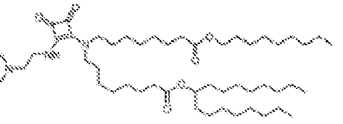 |
| 194 | 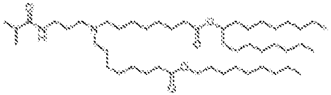 | 341 | 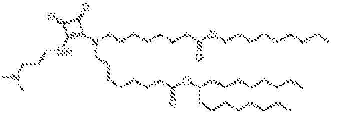 |
| 195 | 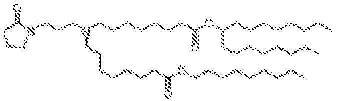 | 342 | 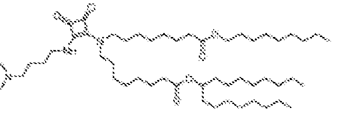 |
| 196 | 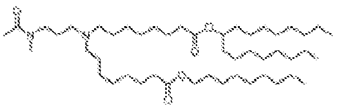 | 343 | 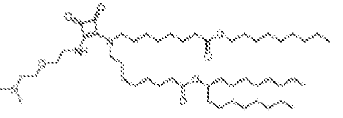 |
| 197 | 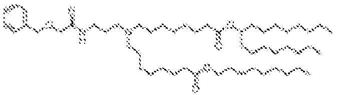 | 344 | 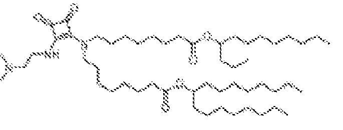 |
| 198 | 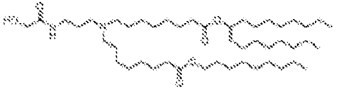 | 345 | 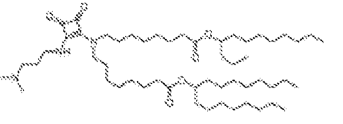 |
| 199 | 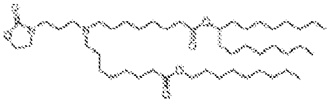 | 344 | 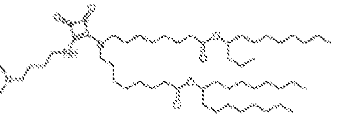 |
| 200 | 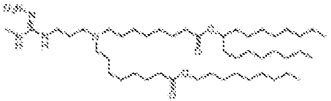 | 347 | 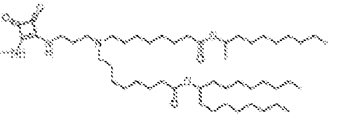 |
| 201 | 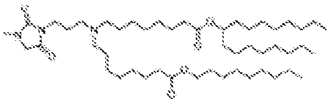 | 348 | 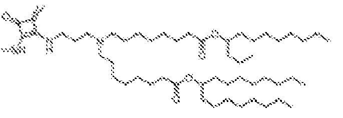 |
| 202 | 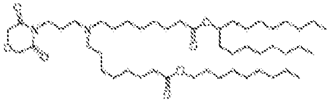 | 349 | 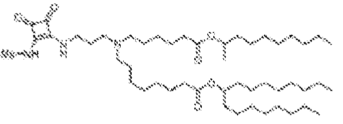 |
| 203 | 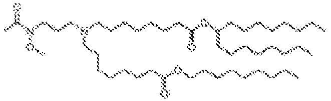 | 350 | 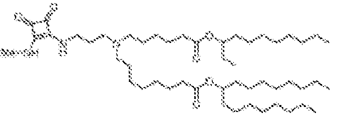 |
| 204 | 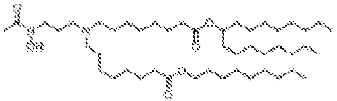 | 351 | 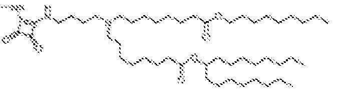 |
| 205 | 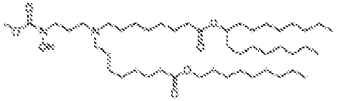 | 352 | 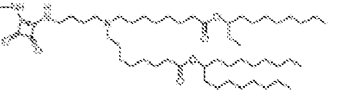 |
| 206 | 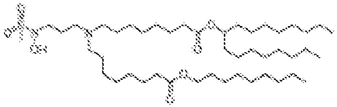 | 353 | 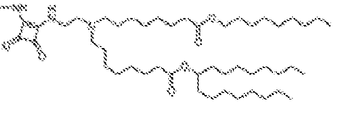 |
| 207 | 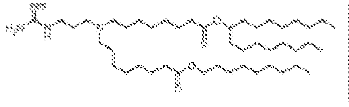 | 354 | 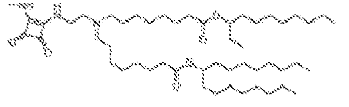 |
| 208 | 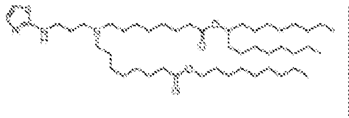 | 355 | 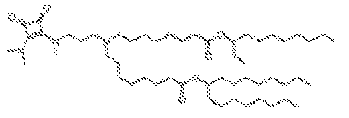 |
| 209 | 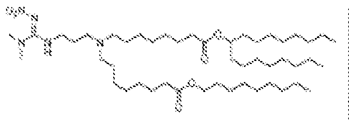 | 356 | 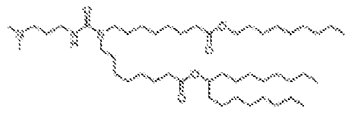 |
| 210 | 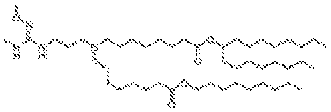 | 357 | 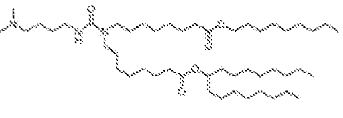 |
| 211 | 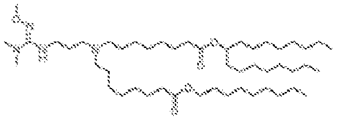 | 358 | 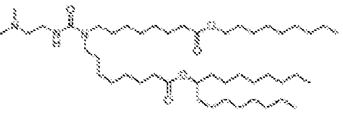 |
| 359 | 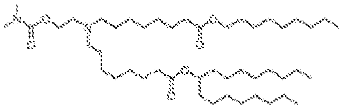 | 360 | 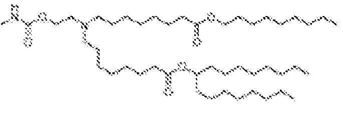 |
| 361 | 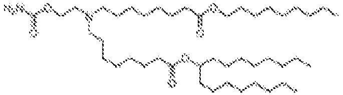 | 362 | 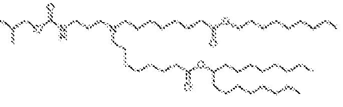 |
| 363 | 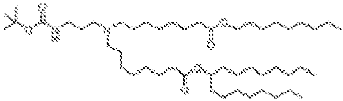 | 364 | 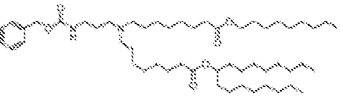 |
| 365 | 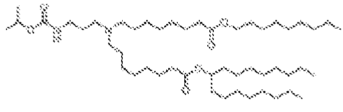 | 366 | 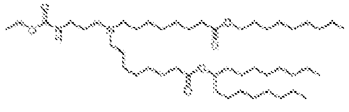 |
| 367 | 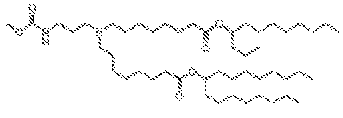 | 368 | 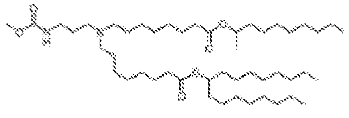 |
| 369 | 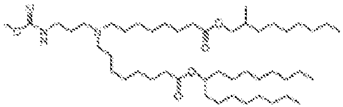 | 370 | 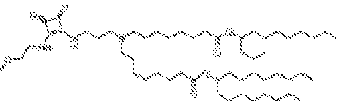 |
| 371 | 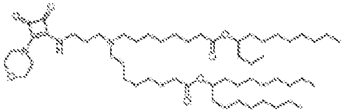 | 372 | 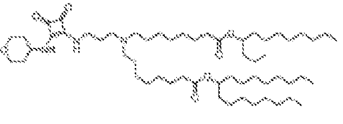 |
| 373 | 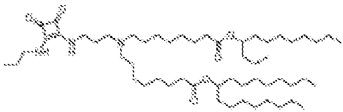 | 374 | 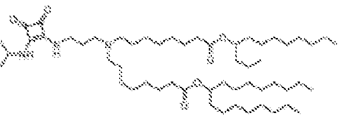 |
| 375 | 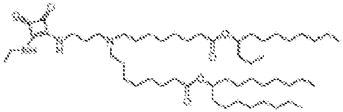 | 376 | 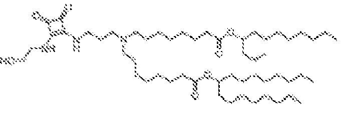 |
| 377 | 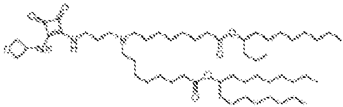 | 378 | 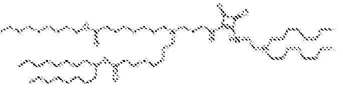 |
| 379 | 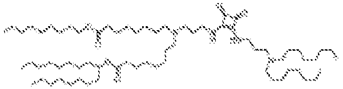 | 380 | 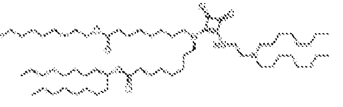 |
| 381 | 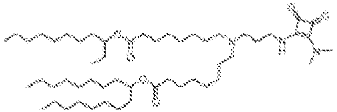 | 382 | 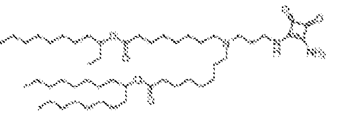 |
| 383 | 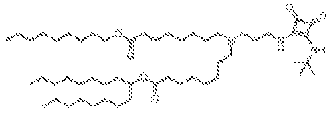 | 384 | 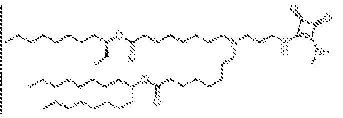 |
| 385 | 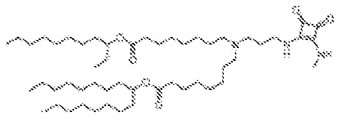 | 386 | 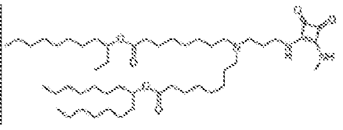 |
| 387 | 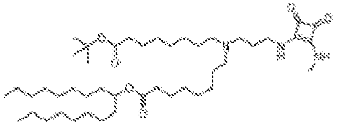 | 388 | 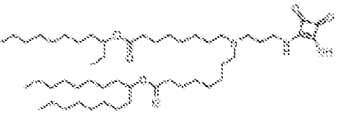 |
| 389 | 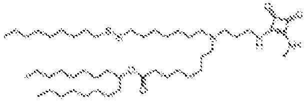 | 390 | 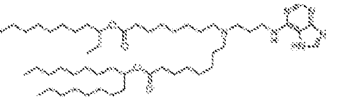 |
| 391 | 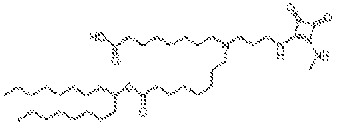 | 392 | 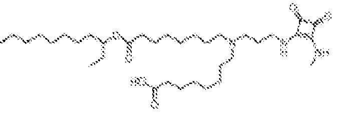 |

In some embodiments, the cationic lipid has the following structure:

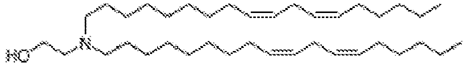

### Neutral/Non-cationic Lipids

In various embodiments, the LNPs comprise a neutral lipid. In various embodiments, the molar ratio of the cationic lipid to the neutral lipid ranges from about 2:1 to about 8:1. In certain embodiments, the neutral lipid is present in any of the foregoing LNPs in a concentration ranging from 5 to 10 mol percent, from 5 to 15 mol percent, 7 to 13 mol percent, or 9 to 11 mol percent. In certain specific embodiments, the neutral lipid is present in a concentration of about 9.5, 10 or 10.5 mol percent. In some embodiments, the molar ratio of cationic lipid to the neutral lipid ranges from about 4.1:1.0 to about 4.9:1.0, from about 4.5:1.0 to about 4.8:1.0, or from about 4.7:1.0 to 4.8:1.0. In some embodiments, the molar ratio of total cationic lipid to the neutral lipid ranges from about 4.1:1.0 to about 4.9: 1.0, from about 4.5:1.0 to about 4.8:1.0, or from about 4.7:1.0 to 4.8:1.0.

Exemplary neutral lipids include, for example, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoyl-phosphatidylethanolamine (POPE) and dioleoyl-phosphatidylethanolamine 4-(N-maleimidomethyl)-cyclohexane-1carboxylate (DOPE-mal), dipalmitoyl phosphatidyl ethanolamine (DPPE), dimyristoylphosphoethanolamine (DMPE), distearoyl-phosphatidylethanolamine (DSPE), 16-O-monomethyl PE, 16-O-dimethyl PE, 18-1-trans PE, 1-stearioyl-2-oleoylphosphatidyethanol amine (SOPE), and 1,2-dielaidoyl-sn-glycero-3-phophoethanolamine (transDOPE). In one embodiment, the neutral lipid is 1,2-distearoyl-sn-glycero-3phosphocholine (DSPC). In some embodiments, the neutral lipid is selected from DSPC, DPPC, DMPC, DOPC, POPC, DOPE and SM. In some embodiments, the neutral lipid is DSPC.

In certain embodiments, neutral lipids useful in the present invention are DSPC analogs wherein the phosphocholine moiety is replaced by a different zwitterionic group. In certain embodiments, the different zwitterionic group is not a phosphocholine group. In certain embodiments, a neutral lipid useful in the present invention is a compound of formula: 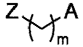 or a salts thereof, wherein:
Z is a zwitterionic moiety,
m is 0, 1, 2, 3, 4, 5, 6, 7, 8, 9, or 10;
A is of the formula: 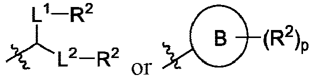
each instance of L² is independently a bond or optionally substituted C₁₋₆ alkylene, wherein one methylene unit of the optionally substituted C₁₋₆ alkylene is optionally replaced with -O-, -N(R^{N} )-, -S-, -C(O)-, -C(O)N(R^{N})-, -NR^{N}C(O)-, -C(O)O-, -OC(O)-, -OC(O)O-, -OC(O)N(R^{N})-, -NRC(O)O-, or -NR^{N}C(O)N(R ^{N})-;
each instance of R² is independently optionally substituted C₁₋₃₀ alkyl, optionally substituted C₁₋₃₀ alkenyl, or optionally substituted C₁₋₃₀ alkynyl; optionally wherein one or more methylene units of R² are independently replaced with optionally substituted carbocyclylene, optionally substituted heterocyclylene, optionally substituted arylene, optionally substituted heteroarylene, -N(R^{N})-, -O-, -S-, -C(O)-, -C(O)N(R^{N})-, - NR^{N}C(O)-, -NR^{N}C(O)N(R^{N})-, -C(O)O-, -OC(O)-, -OC(O)O-, -OC(O)N(R^{N})-, - NR^{N}C(O)O-, -C(O)S- -SC(O)-, -C(=NR^{N})-, -C(=NR^{N})N(R^{N})-, -NR^{N}C(=N R^{N})-, - NR^{N}C(=NR^{N})N(R^{N})-, - C(S)-, C(S)N(R^{N})-, -NR^{N}C(S)-, -NR^{N}C(S)N(R^{N})-, -S(O)-, - OS(O)-, -S(O)O-, -OS(O)O-, -OS(O)₂-, -S(O)₂O-, -OS(O)₂O -, -N(R^{N})S(O)-, - S(O)N(R^{N})-, -N(R^{N})S(O)N(R^{N})-, -OS(O)N(R^{N})-, -N(R^{N})S(O)O-, -S(O)₂-, -N(R^{N})S(O)₂-,-S(O)₂N(R^{N})-, -N(R^{N})S(O)₂N(R^{N})-, -OS(O)₂N(R^{N})-, or -N(R^{N})S(O)₂O-;
each instance of R^{N} is independently hydrogen, optionally substituted alkyl, or a nitrogen protecting group;
Ring B is optionally substituted carbocyclyl, optionally substituted heterocyclyl, optionally substituted aryl, or optionally substituted hctcroaryl; and
p is 1 or 2.

In certain embodiments, Z is an amino acid or a derivative thereof. In certain embodiments, Z is of one of the following formulas: 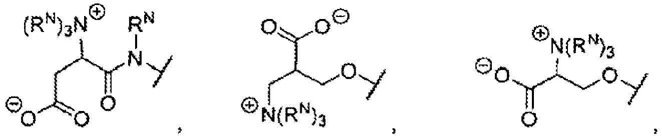 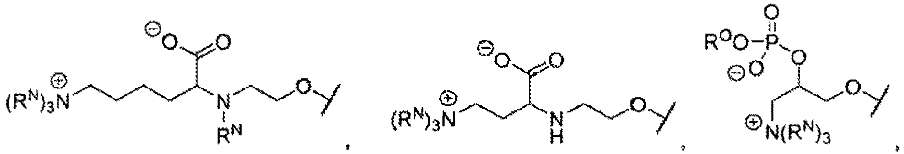 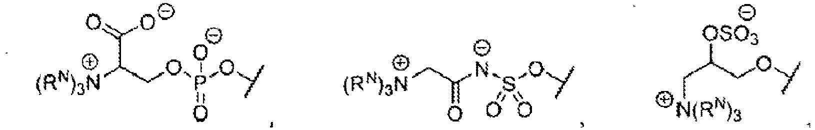 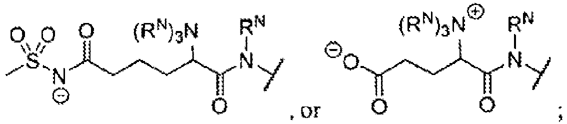 wherein R^{O} is hydrogen, optionally substituted alkyl or an oxygen protecting group. In certain embodiments, a compound of said formula is of one of the following: 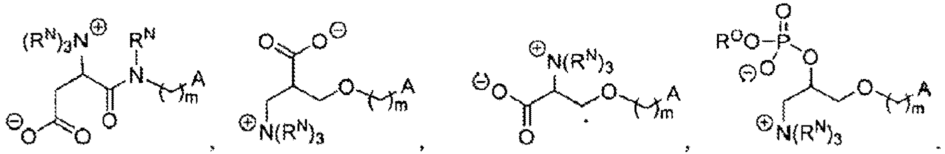 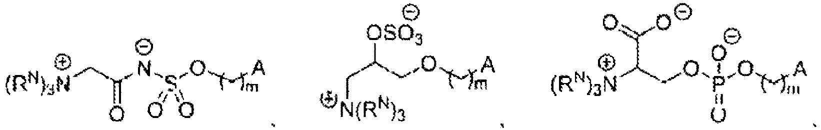 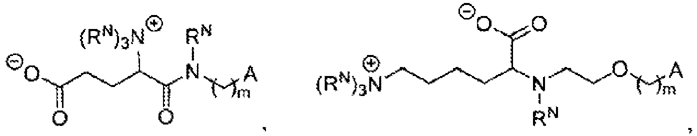 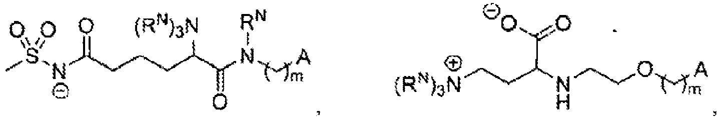 or a salt thereof.

In certain embodiments, a compound of formula 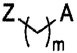 is of one of the following formulas: 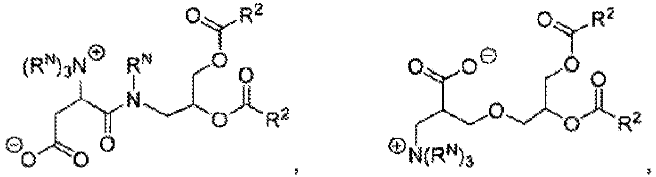 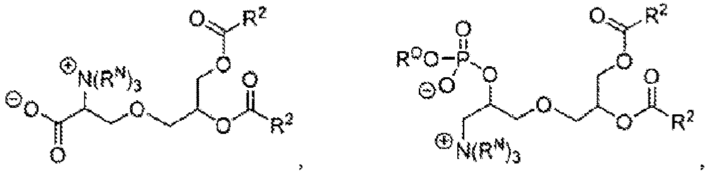 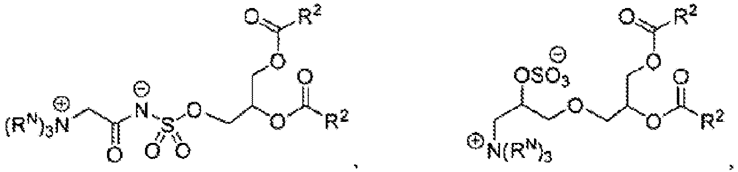 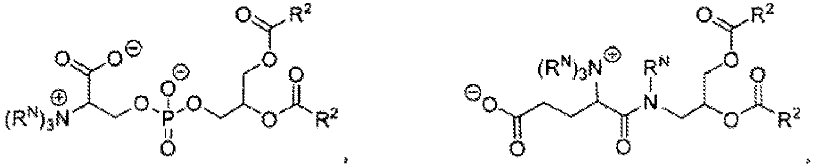 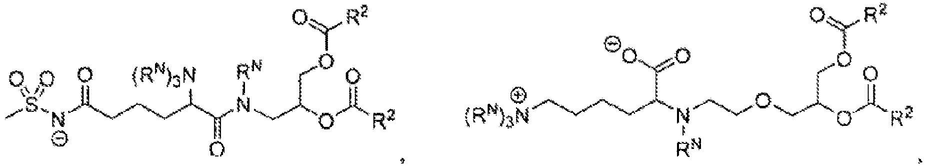 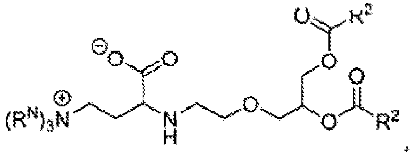 or a salt thereof.

For example, in certain embodiments, a compound of formula 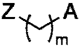 is one of the following: 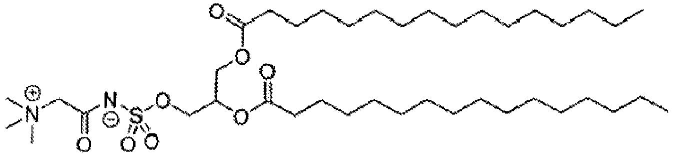 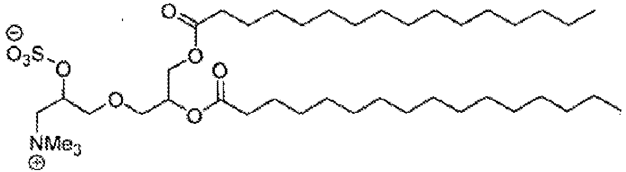 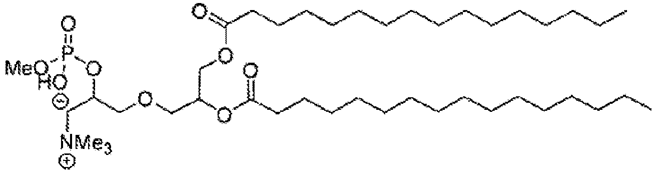 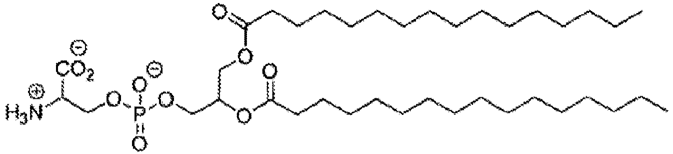 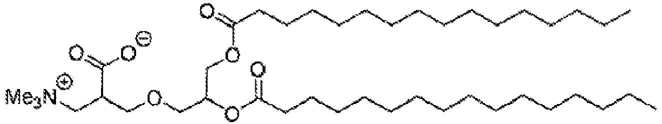 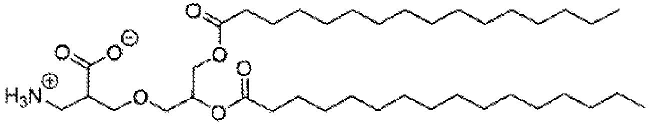 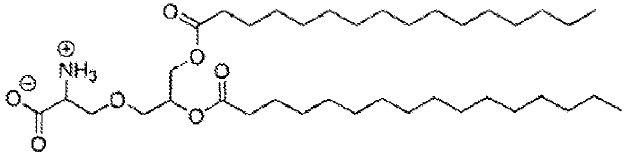 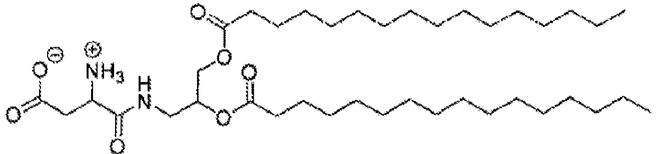 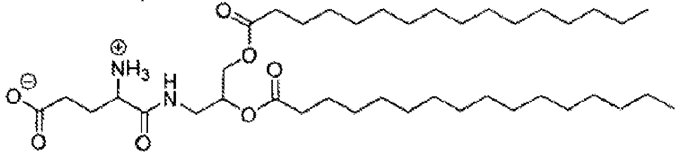 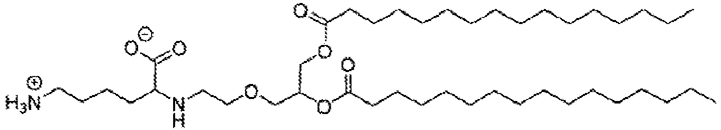 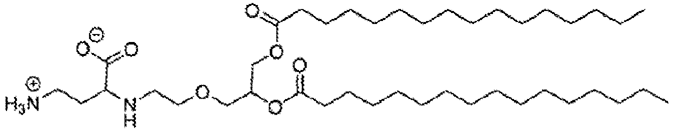 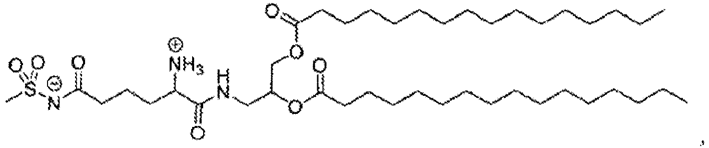 or salts thereof.

Other neutral lipids useful in the present invention include analogs of oleic acid. As described herein, an oleic acid analog can comprise a modified oleic acid tail, a modified carboxylic acid moiety, or both. In certain embodiments, an analog of oleic acid is a compound of formula: 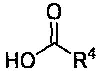 or a salt thereof, wherein:
R⁴ is optionally substituted, C1-40 alkyl; optionally substituted, C2-20 alkenyl;
optionally substituted, C2-40 alkynyl; wherein at least one methylene group of R⁴ is independently replaced with optionally substituted carbocyclylene, optionally substituted heterocyclylene, optionally substituted arylene, optionally substituted heteroarylene, - N(R^{N})-, -O-, -S-, -C(O)-, -C(O)N(R^{N})-, -NR^{N}C(O)-, -NR^{N}C(O)N(R^{N})-, -C(O)O-, - OC(O)-, -OC(O)O-, -OC(O)N(R^{N})-, -NR^{N}C(O)O-, -C(O)S-, -SC(O)-, -C(=NR^{N})-, - C(=NR^{N})N(R^{N})-, -NR^{N}C(=N R^{N})-, -NR^{N}C(=NR^{N})N(R^{N})-, - C(S)-, C(S)N(R^{N})-, - NR^{N}C(S)-, -NR^{N}C(S)N(R^{N})-, -S(O)-, OS(O)-, -S(O)O-, -OS(O)O-, -OS(O)₂-, -S(O)₂O-, - OS(O)₂O -, -N(R^{N})S(O)-, -S(O)N(R^{N})-, -N(R^{N})S(O)N(R^{N})-, -OS(O)N(R^{N})-, - N(R^{N})S(O)O-, -S(O)₂-, -N(R^{N})S(O)₂-,-S(O) 2N(R^{N})-, -N(R^{N})S(O)₂N(R^{N})-, - OS(O)₂N(R^{N})-, or -N(R^{N})S(O)₂O-; and
   each instance of R is independently hydrogen, optionally substituted alkyl, or a nitrogen protecting group.

In certain embodiments, the compound of said formula is one of the following: 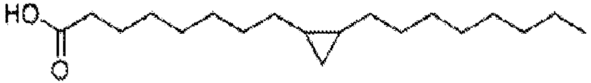 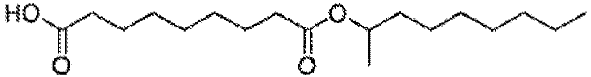 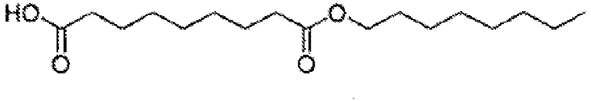 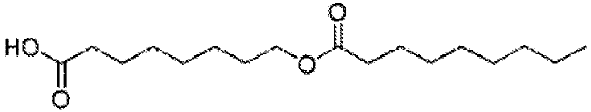 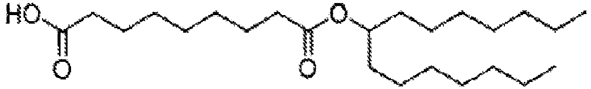 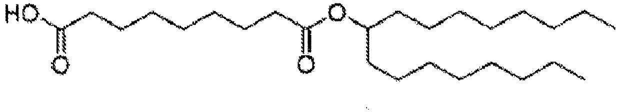 or salts thereof.

In certain embodiments, an oleic acid analog is a compound wherein the carboxylic acid moiety of oleic acid replaced by a different group. In certain embodiments, an oleic acid analog useful in the present invention is one of the following: 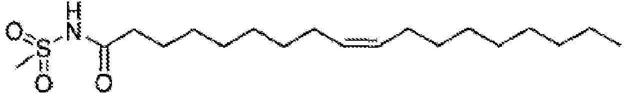 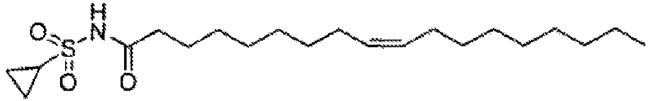 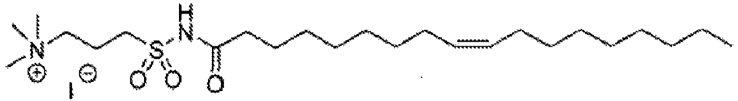 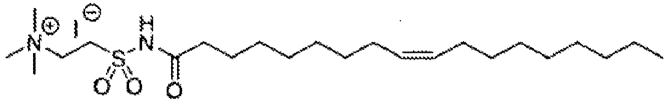 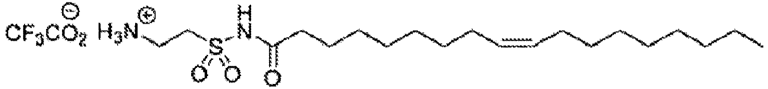 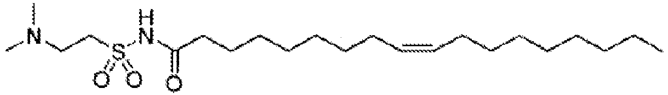 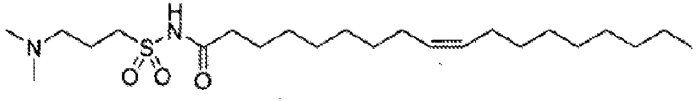 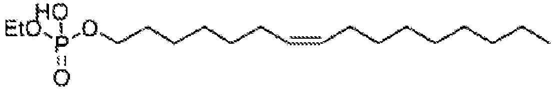 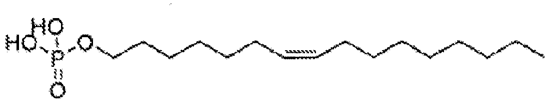 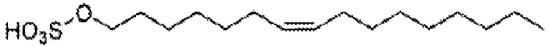 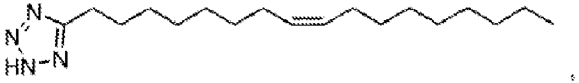 or salts thereof.

Phospholipids, as defined herein, are any lipids that comprise a phosphate group. Phospholipids are a subset of neutral lipids. The lipid component of a nanoparticle composition may include one or more phospholipids, such as one or more (poly)unsaturated lipids. Phospholipids may assemble into one or more lipid bilayers. In general, phospholipids may include a phospholipid moiety and one or more fatty acid moieties. A phospholipid moiety may be selected from the non-limiting group consisting of phosphatidyl choline, phosphatidyl ethanolamine, phosphatidyl glycerol, phosphatidyl serine, phosphatidic acid, 2-lysophosphatidyl choline, and a sphingomyelin. A fatty acid moiety may be selected from the non-limiting group consisting of lauric acid, myristic acid, myristoleic acid, palmitic acid, palmitoleic acid, stearic acid, oleic acid, linoleic acid, alpha-linolenic acid, erucic acid, phytanoic acid, arachidic acid, arachidonic acid, eicosapentaenoic acid, behenic acid, docosapentaenoic acid, and docosahexaenoic acid. Non-natural species including natural species with modifications and substitutions including branching, oxidation, cyclization, and alkynes are also contemplated. For example, a phospholipid may be functionalized with or cross-linked to one or more alkynes (e.g., an alkenyl group in which one or more double bonds is replaced with a triple bond). Under appropriate reaction conditions, an alkyne group may undergo a copper-catalyzed cycloaddition upon exposure to an azide. Such reactions may be useful in functionalizing a lipid bilayer of a nanoparticle composition to facilitate membrane permeation or cellular recognition or in conjugating a nanoparticle composition to a useful component such as a targeting or imaging moiety (e.g., a dye). Each possibility represents a separate embodiment of the present invention.

Phospholipids useful in the compositions and methods may be selected from the nonlimiting group consisting of 1,2-distearoyl-sn-glycero-3-phosphocholine (DSPC),

1,2-dioleoyl-sn-glycero-3-phosphoethanolamine (DOPE);1,2-dilinoleoyl-sn-glycero-3-phosphocholine (DLPC); 1,2-dimyristoyl-sn-glycero-phosphocholine (DMPC); 1,2 dioleoyl-sn-glycero-3-phosphocholine (DOPC); 1,2-dipalmitoyl-sn-glycero-3-phosphocholine (DPPC); 1,2-diundecanoyl-sn-glycero-phosphocholine (DUPC); 1-palmitoyl-2-oleoyl-sn-glycero-3-phosphocholine (POPC); 1,2-di-O-octadecenyl-sn-glycero-3-phosphocholine (18:0 Diether PC); l-oleoyl-2-cholesterylhemisuccinoyl-sn-glycero-3-phosphocholine (OChemsPC); 1-hexadecyl-sn-glycero-3-phosphocholine (CI 6 Lyso PC); 1,2-dilinolenoyl-sn-glycero-3-phosphocholine; 1,2-diarachidonoyl-sn-glycero-3-phosphocholine; 1,2-didocosahexaenoyl-sn-glycero-3-phosphocholine; 1,2-diphytanoyl-sn-glycero-3-phosphoethanolamine (ME 16.0 PE); 1,2-distearoyl-sn-glycero-3-phosphoethanolamine; 1,2-dilinoleoyl-sn-glycero-3-phosphoethanolamine; 1,2-dilinolenoyl-sn-glycero-3-phosphoethanolamine; 1,2-diarachidonoyl-sn-glycero-3-phosphoethanolamine; 1,2-didocosahexaenoyl-sn-glycero-3-phosphoethanolamine; or 1,2-dioleoyl-sn-glycero-3-phospho-rac-(1 -glycerol) sodium salt (DOPG), and sphingomyelin.

In some embodiments, a nanoparticle composition includes DSPC. In certain embodiments, a nanoparticle composition includes DOPE. In some embodiments, a nanoparticle composition includes both DSPC and DOPE.

Examples of phospholipids include, but are not limited to, the following: 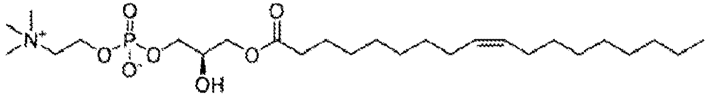 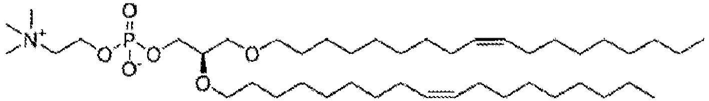 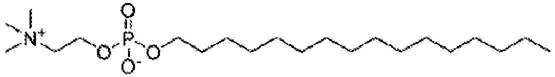 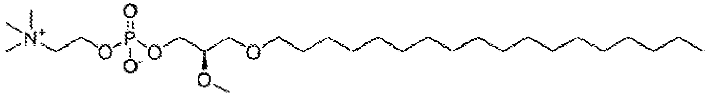 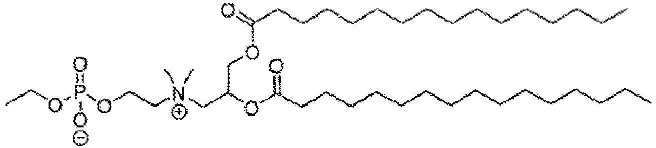 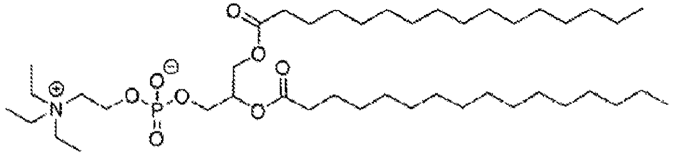 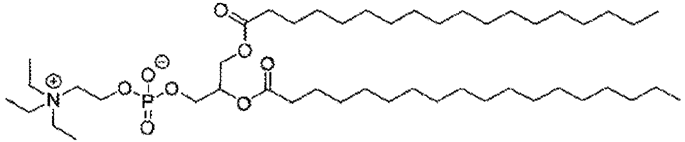 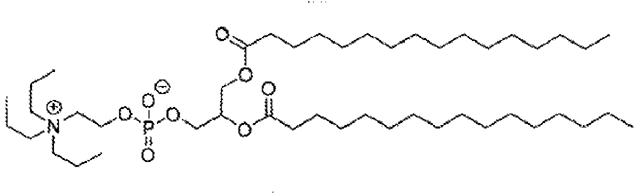 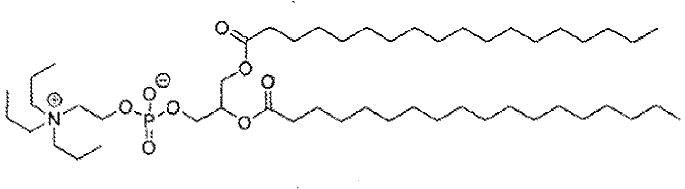 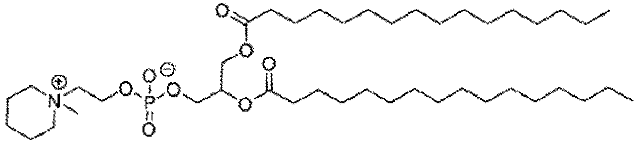 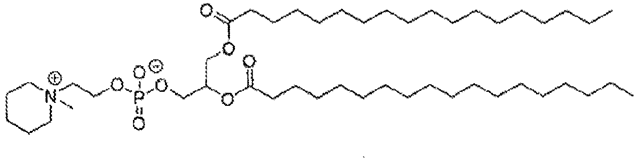 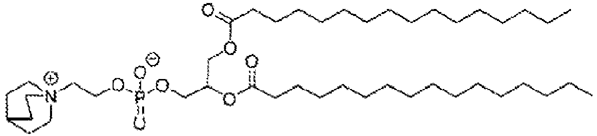 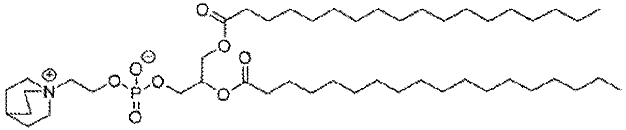 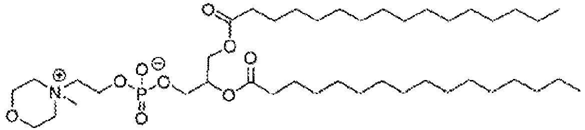 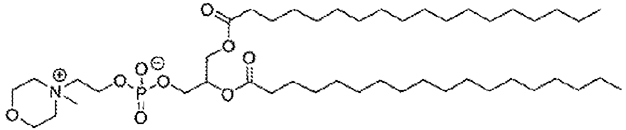 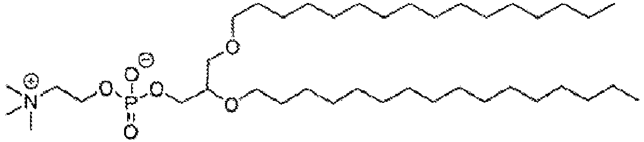 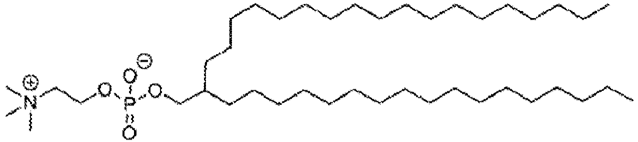 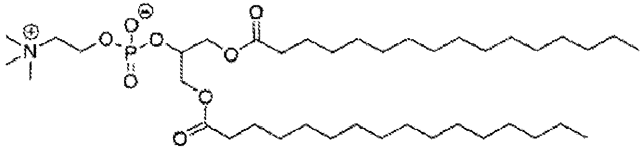 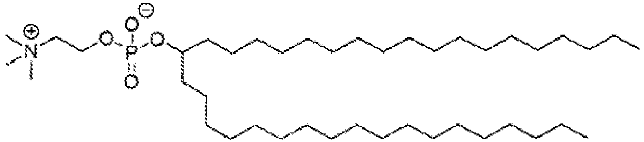 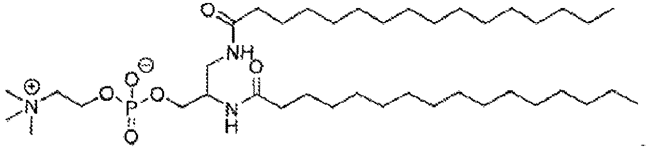 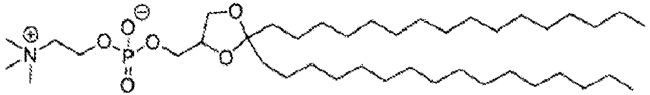 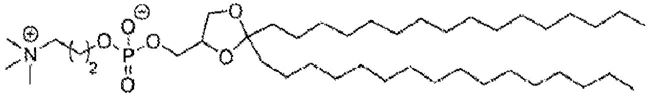 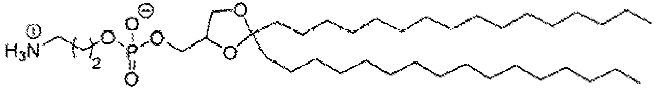 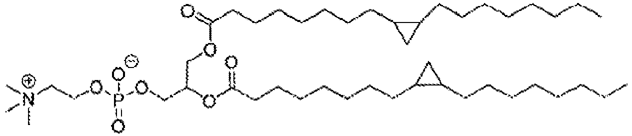 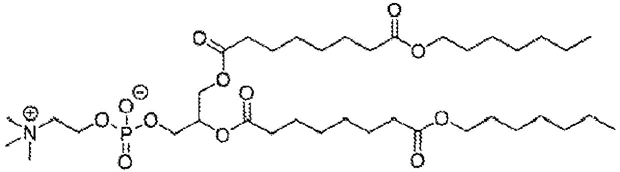 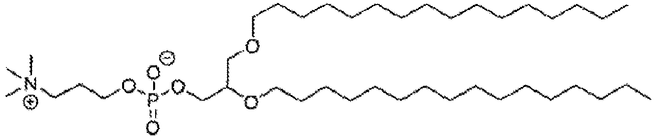 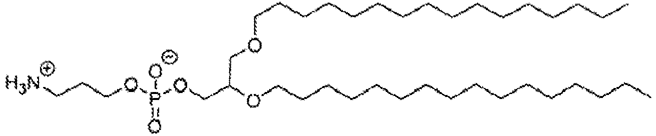 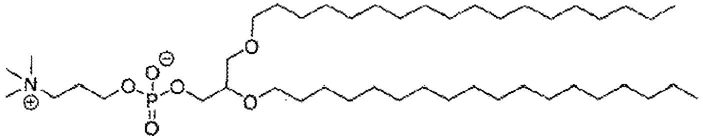 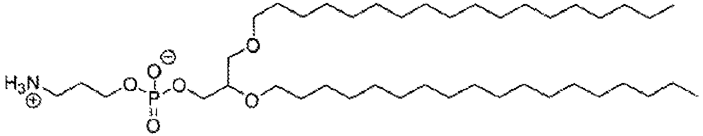 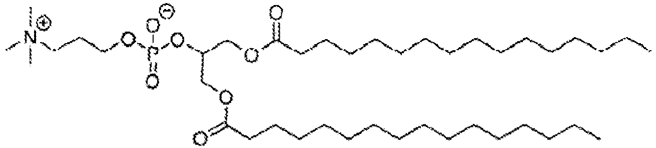 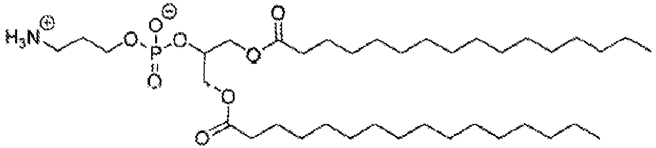 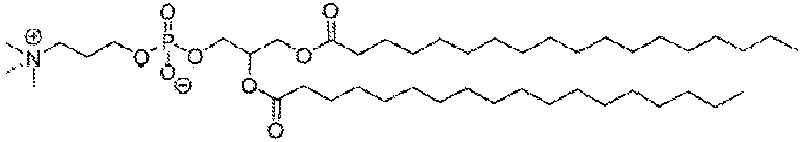 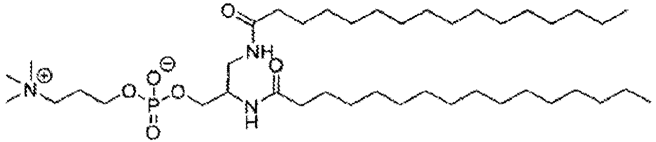 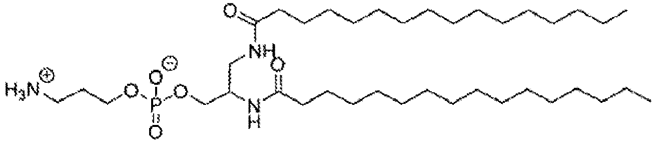 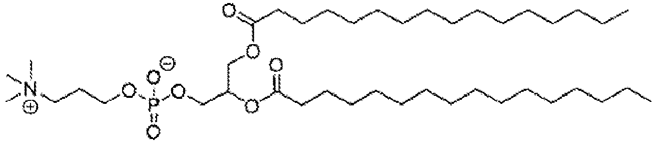 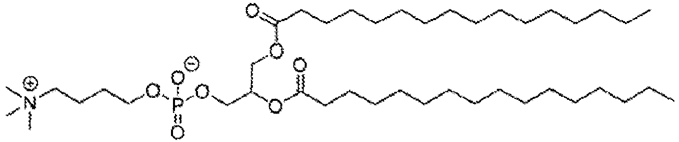 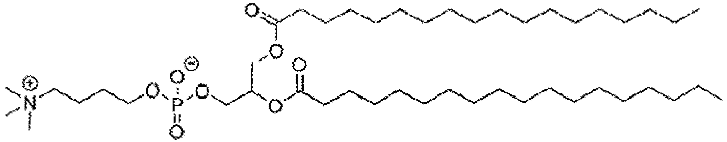 or salts thereof.

Examples of neutral/non-cationic lipids include, but are not limited to, the following: 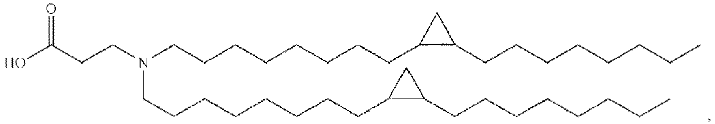 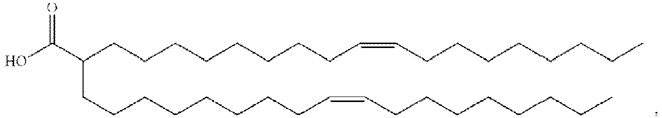 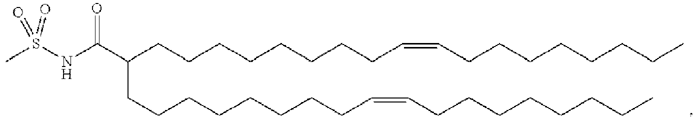 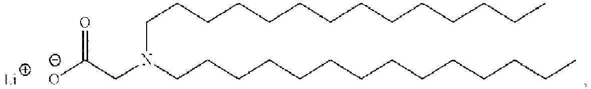 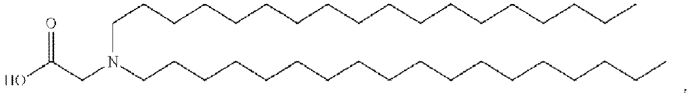 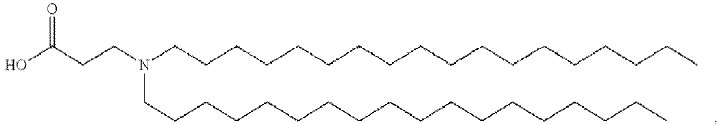 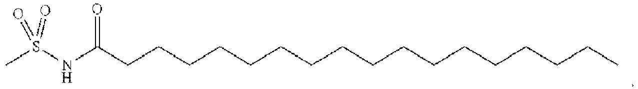 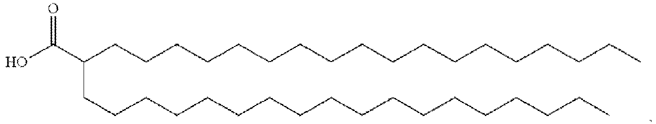 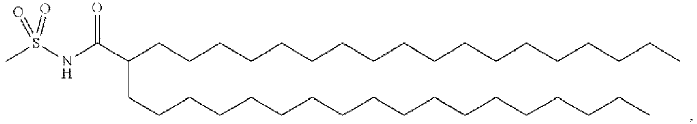 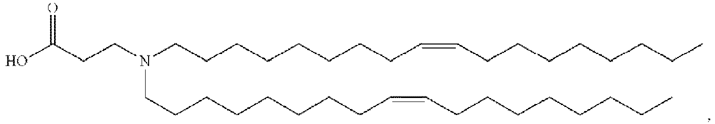 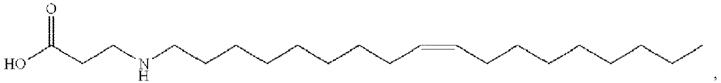 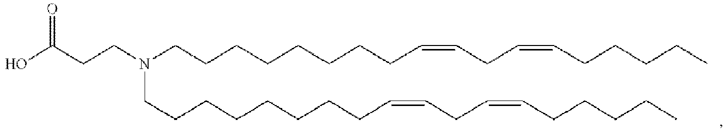 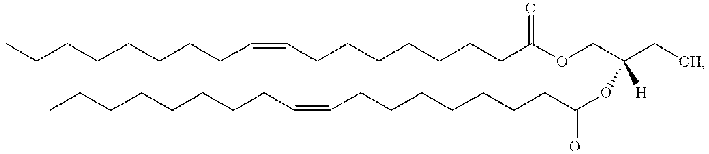 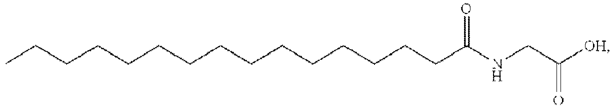 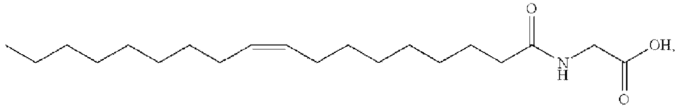 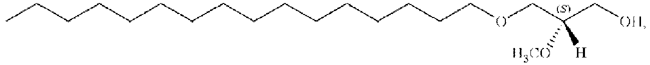 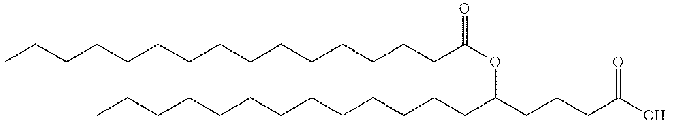 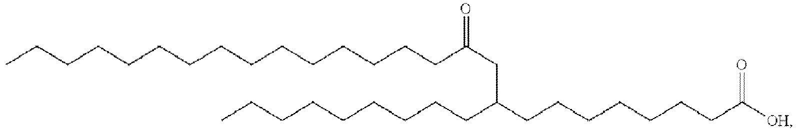 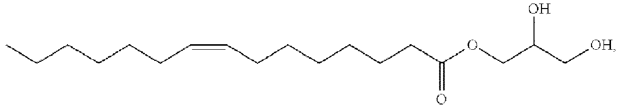 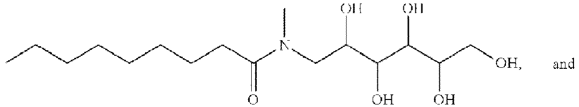 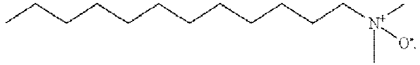 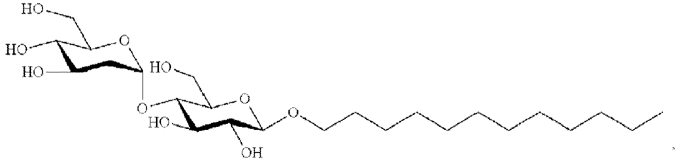 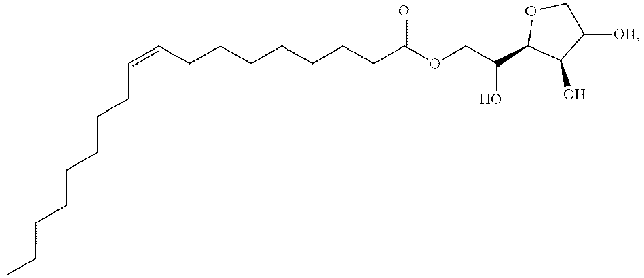 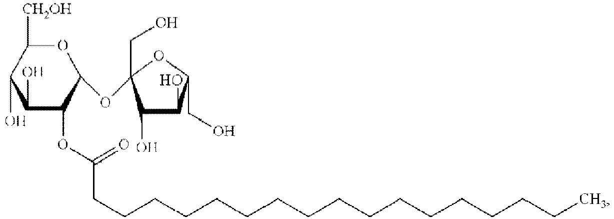 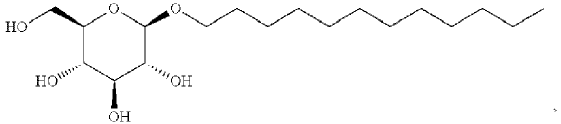 and

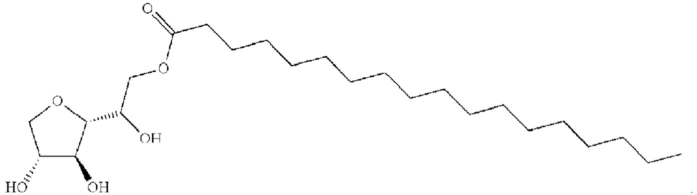

### Steroids

In various embodiments any of the disclosed lipid nanoparticles comprise a steroid or steroid analogue. In certain embodiments, the steroid or steroid analogue is cholesterol. In some embodiments, the steroid is present in a concentration ranging from 35 to 49 molar percent, 37 to 46 molar percent, from 38 to 44 molar percent, from 38 to 40 molar percent, from 40 to 42 molar percent, from 42 to 44 molar percent, or from 44 to 46 molar percent. In certain specific embodiments, the steroid is present in a concentration of 37, 38, 39, 40, 41, 42, 43, 44, 45, or 46 molar percent.

In certain embodiments, the molar ratio of cationic lipid to the steroid ranges from 1.0:0.9 to 1.0:1.2, or from 1.0:1.0 to 1.0:1.2. In some of these embodiments, the molar ratio of cationic lipid to cholesterol ranges from about 5:1 to 1:1. In certain embodiments, the steroid is present in a concentration ranging from 35 to 45 mol percent of the steroid.

In certain embodiments, the molar ratio of total cationic to the steroid ranges from 1.0:0.9 to 1.0:1.2, or from 1.0:1.0 to 1.0:1.2. In some of these embodiments, the molar ratio of total cationic lipid to cholesterol ranges from about 5:1 to 1:1. In certain embodiments, the steroid is present in a concentration ranging from 35 to 45 mol percent of the steroid.

### Polymer Conjugated Lipids

In certain embodiments are provided polymer-conjugated lipids useful in various methods, such as delivery of a therapeutic nucleic acid to a primate. One such polymer-conjugated lipid is a compound having the following structure: 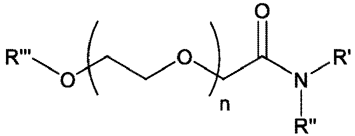 or a salt thereof, wherein:
R' and R" are each independently a saturated alkyl having from 8 to 12 carbon atoms, provided that the total number of carbon atoms collectively in both of R' and R" is no more than 23;
R‴ is H or C₁-C₆ alkyl; and
n is an integer ranging from 30 to 60.

As used herein, the R' and R" moieties are collectively referred to as the di-acyl chains of a polymer conjugated lipid. For example, a C12 di-acyl chain polymer conjugated lipid refers to a polymer-conjugated lipid, such as the above structure, having two C12 acyl chains (e.g., the R' and R" moieties). Similarly, a C12/14 di-acyl chain polymer-conjugated lipid refers to a polymer-conjugated lipid, such as the above structure, having one C12 acyl chain and one C 14 acyl chain (e.g., the R' and R" moieties). Other polymer-conjugated lipids are identified similarly.

In some embodiments, n is an integer from 40 to 50.

In other embodiments, R‴ is H or CH₃.

In various different embodiments, the total number of carbon atoms collectively in both of R' and R" ranges from 16 to 22, 16 to 21, 16 to 20, 18 to 23, 18 to 22, 18 to 21, 19 to 23, 19 to 22, 19 to 21, 20 to 23, or 20 to 22.

In still more embodiments:
a) R' and R" are each a saturated alkyl having 8 carbon atoms;
b) R' and R" are each a saturated alkyl having 9 carbon atoms;
c) R' and R" are each a saturated alkyl having 10 carbon atoms; or
d) R' and R" are each a saturated alkyl having 11 carbon atoms.

LNPs comprising the foregoing polymer-conjugated lipid are also provided.

In some embodiments, the LNPs comprise a polymer conjugated lipid. In various other embodiments the polymer conjugated lipid is a pegylated lipid. For example, some embodiments include a pegylated diacylglycerol (PEG-DAG) such as 1-(monomethoxy-polyethyleneglycol)-2,3-dimyristoylglycerol (PEG-DMG), a pegylated phosphatidylethanoloamine (PEG-PE), a PEG succinate diacylglycerol (PEG-S-DAG) such as 4-O-(2',3'-di(tetradecanoyloxy)propyl-]-O-(ω-methoxy(polyethoxy)ethyl)butanedioate (PEG-S-DMG), a pegylated ceramide (PEG-cer), or a PEG dialkoxypropylcarbamate such as ω-methoxy(polyethoxy)ethyl-N-(2,3-di(tetradecanoxy)propyl)carbamate or 2,3-di(tetradecanoxy)propyl-N-(ω-methoxy(polyethoxy)ethyl)carbamate.

In yet more embodiments, a polymer conjugated lipid may be selected from the non-limiting group consisting of PEGylated phosphatidylethanolamines, PEGmodified phosphatidic acids, PEGylated ceramides, PEGylated dialkylamines, PEGylated diacylglycerols, PEGylated dialkylglycerols, and mixtures thereof. For example, a PEG lipid may be PEG-c-DOMG, PEG-DLPE, PEG-DMPE, PEG-DPPC, or a PEG-DSPE lipid.

In some embodiments the PEGylated lipids are a modified form of PEG DMG. PEG-DMG has the following structure:

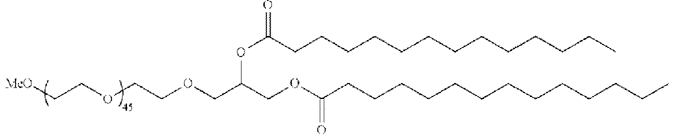

In one embodiment, PEG lipids useful in the present invention can be PEGylated lipids described in International Publication No. WO2012/099755. Any of these exemplary PEG lipids described herein may be modified to comprise a hydroxyl group on the PEG chain. In certain embodiments, the PEG lipid is a PEG-OH lipid. As generally defined herein, a "PEG-OH lipid" (also referred to herein as "hydroxy-PEGylated lipid") is a PEGylated lipid having one or more hydroxyl (-OH) groups on the lipid. In certain embodiments, the PEG-OH lipid includes one or more hydroxyl groups on the PEG chain. In certain embodiments, a PEG-OH or hydroxy-PEGylated lipid comprises an -OH group at the terminus of the PEG chain. Each possibility represents a separate embodiment of the present invention.

In certain embodiments, a PEG lipid useful in the present invention is a compound of formula: 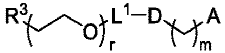 or salts thereof, wherein:
R³ is -OR^{O};
R^{O} is hydrogen, optionally substituted alkyl, or an oxygen protecting group;
r is an integer between 1 and 150, inclusive;
L¹ is optionally substituted C1-10alkylene, wherein at least one methylene of the optionally substituted C1-10alkylene is independently replaced with optionally substituted carbocyclyclene, optionally substituted heterocyclylene, optionally substituted arylene, optionally substituted heteroarylene, -O-, -N(R^{N})-, -S-, -C(O)-, - C(O)N(R^{N})-, -NR^{N}C(O )-, - C(O)O-, -OC(O)-, -OC (O)O-, -OC(O)N(R^{N}) -, -NR^{N}C(O)O -, or -NR^{N}C(O)N(R^{N} )-;
D is a moiety obtained by click chemistry or a moiety cleavable under physiological conditions;
m is 0, 1, 2, 3, 4, 5, 6, 7, 8, 9, or 10;
A is of the formula: 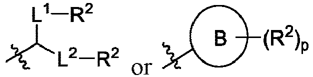
each instance of L₂ is independently a bond or optionally substituted C 1-6 alkylene, wherein one methylene unit of the optionally substituted C1-6 alkylene is optionally replaced with -O-, -N(R^{N} )-, -S-, -C(O)-, -C(O)N(R^{N})-, -NR^{N}C(O)-, -C(O)O-, -OC(O)-, -OC(O)O-, -OC(O)N(R^{N})-, -NRC(O)O-, or -NR^{N}C(O)N(R ^{N})-;
each instance of R2 is independently optionally substituted C 1-30 alkyl, optionally substituted C1-30 alkenyl, or optionally substituted C1-30 alkynyl; optionally wherein one or more methylene units of R2 are independently replaced with optionally substituted carbocyclylene, optionally substituted heterocyclylene, optionally substituted arylene, optionally substituted heteroarylene, -N(R^{N})-, -O-, -S-, -C(O)-, -C(O)N(R^{N})-, - NR^{N}C(O)-, -NR^{N}C(O)N(R^{N})-, -C(O)O-, -OC(O)-, -OC(O)O-, -OC(O)N(R^{N})-, - NR^{N}C(O)O-, -C(O)S- -SC(O)-, -C(=NR^{N})-, -C(=NR^{N})N(R^{N})-, -NR^{N}C(=N R^{N})-, - NR^{N}C(=NR^{N})N(R^{N})-, - C(S)-, C(S)N(R^{N})-, -NR^{N}C(S)-, -NR^{N}C(S)N(R^{N})-, -S(O)-, - OS(O)-, -S(O)O-, -OS(O)O-, -OS(O)₂-, -S(O)₂O-, -OS(O)₂O -, -N(R^{N})S(O)-, - S(O)N(R^{N})-, -N(R^{N})S(O)N(R^{N})-, -OS(O)N(R^{N})-, -N(R^{N})S(O)O-, -S(O)₂-, -N(R^{N})S(O)₂-,-S(O)₂N(R^{N})-, -N(R^{N})S(O) ₂N(R^{N})-, -OS(O)₂N(R^{N})-, or -N(R^{N})S(O)₂O-;
each instance of R^{N} is independently hydrogen, optionally substituted alkyl, or a nitrogen protecting group;
Ring B is optionally substituted carbocyclyl, optionally substituted heterocyclyl, optionally substituted aryl, or optionally substituted hctcroaryl; and
p is 1or 2.

In certain embodiments, the PEGylated lipid is of one of the following formulas:

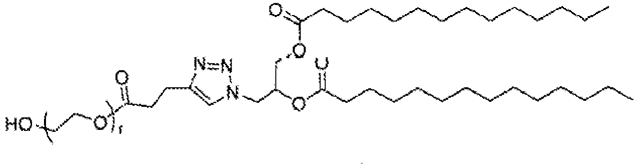

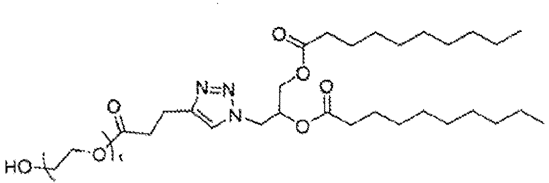

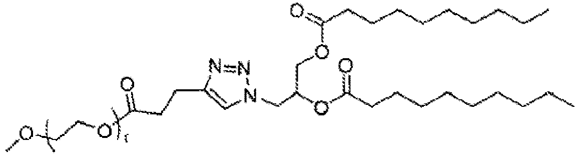

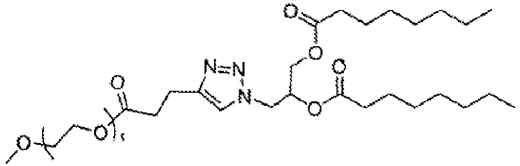

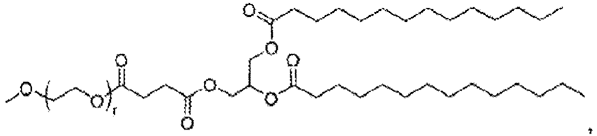

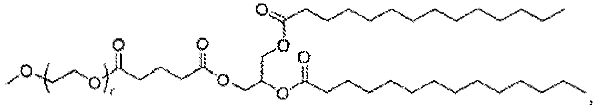

In certain embodiments, a PEG lipid useful in embodiments of the present invention is a PEGylated fatty acid. In certain embodiments, a PEG lipid useful in embodiments of the present invention is a compound of formula: 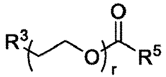 or salts thereof, wherein:
R³ is -OR^{O};
R^{O} is hydrogen, optionally substituted alkyl or an oxygen protecting group;
r is an integer between 1 and 100, inclusive;
R⁵ is optionally substituted C₁₀₋₄₀ alkyl, optionally substituted C₁₀₋₄₀ alkenyl, or optionally substituted C₁₀₋₄₀ alkynyl; and optionally one or more methylene groups of R⁵ are replaced with optionally substituted carbocyclylene, optionally substituted heterocyclylene, optionally substituted arylene, optionally substituted heteroarylene, -N(R^{N})-, -O-, -S-, -C(O)-, -C(O)N(R^{N})-, -NR^{N}C(O)-, -NR^{N}C(O)N(R^{N})-, - C(O)O-, -OC(O)-, -OC(O)O-, -OC(O)N(R^{N})-, -NR^{N}C(O)O-, -C(O)S- -SC(O)-, - C(=NR^{N})-, -C(=NR^{N})N(R^{N})-, -NR^{N}C(=N R^{N})-, -NR^{N}C(=NR^{N})N(R^{N})-, - C(S)-, C(S)N(R^{N})-, -NR^{N}C(S)-, -NR^{N}C(S)N(R^{N})-, -S(O)-, -OS(O)-, -S(O)O-, -OS(O)O-, - OS(O)₂-, -S(O)₂O-, -OS(O)₂O -, -N(R^{N})S(O)-, -S(O)N(R^{N})-, -N(R^{N})S(O)N(R^{N})-, - OS(O)N(R^{N})-, -N(R^{N})S(O)O-, -S(O)₂-, -N(R^{N})S(O)₂-,-S(O) ₂N(R^{N})-, -N(R^{N})S(O) ₂N(R^{N})-, -OS(O)₂N(R^{N})-, or -N(R^{N})S(O)₂O-; and
each instance of R^{N} is independently hydrogen, optionally substituted alkyl, or a nitrogen protecting group.

In certain embodiments, a compound of said formula is of one of the following compounds: 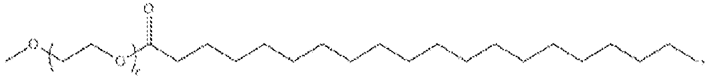 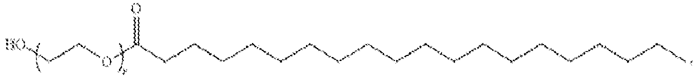 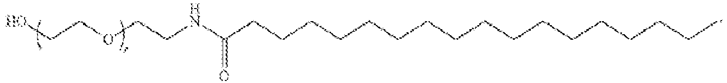 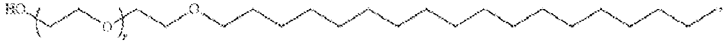 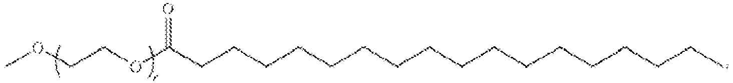 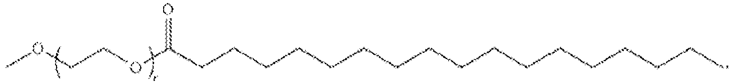 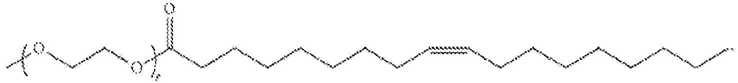 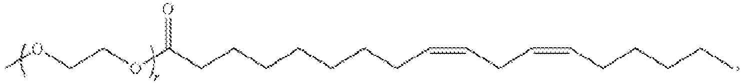 or a salt thereof,

Wherein r is an integer between 1 and 100.

In yet other embodiments the present invention relates to a compound of formula: 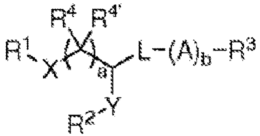 or a pharmaceutically acceptable salt thereof,
wherein:
each of R¹ and R², independently, is a C₁₀ to C₃₀ aliphatic group, where the aliphatic group is optionally substituted by one or more groups each independently selected from R^{a}; and where the aliphatic group is optionally interrupted by cycloalkylene, -O-, -S-, -C(O)-, -OC(O)-,-C(O)O-, -N(R^{c})-, -C(O)N(R^{c})-, or -N(R^{c})C(O)-
X is -(CR^{a}R^{b} )i-, -O-, -S-, -C(O)-, -N(R^{c} )-, -OC(O)-, -C(O)O-, -OC(O)O-, -C(O)N(R^{c})-,-N(R^{c})C(O)-, -OC(O)N(R^{c})-, -N(R^{c})C(O)O-, -N(R^{c})C(O)N(R^{c})-, - SC(O)N(R^{c})-, or -N(R^{c})C(O)S-;
Y is -(CR^{a}R^{b} )i-, -O-, -S-, -C(O)-, -N(R^{c} )-, -OC(O)-, -C(O)O-, -OC(O)O-, -C(O)N(R^{c})-,-N(R^{c})C(O)-, -OC(O)N(R^{c})-, -N(R^{c})C(O)O-, -N(R^{c})C(O)N(R^{c})-, - SC(O)N(R^{c})-, or -N(R^{c})C(O)S-;
L is -L'-Z'-(L²-Z²) -L³- ;
L¹ is a bond, -(CR'R^{5'})i-, or -(CR⁵R^{5'})i-(C(R^{a})=C(R^{b}))ₖ-(C≡C)ₖ, -(CR^{a}R^{b} )j -;
Z¹ is -O-, -S-, -N(R^{c})-, -OC(O)-, -C(O)O-, -OC(O)O-, -OC(O)N(R^{c})-, - N(R^{c})C(O)O-, -N(R^{c})C(O)-, -C(O)N(R^{c})-, -N=C(R^{a})-, -C(R^{a})=N-, -O-N=C(R^{a})-, or -ON(R^{c})-;
L² is -(CR^{a}R^{b})ₚ- or -(CR^{a}R^{b})ⱼ-(C(Ra)=C(R^{b}))ₖ-(C≡C)ₖ-(CR^{a}R )ⱼ;
Z² is -O-, -S-, -N(R^{c})-, -OC(O)-, -C(O)O-, -OC(O)O-, -OC(O)N(R^{c})-, - N(R^{c})C(O)O-, -N(R^{c})C(O)-, -C(O)N(R^{c})-, -N=C(R^{a})-, -C(R^{a})=N-, -O-N=C(R^{a})-, or -ON(R^{c})-;
L³ is -(CR^{a}R^{b})ᵢ-;
each A, independently, is -L⁴-, -NH-(L⁴)_{q} -(CR^{a}R^{b} )ᵣ-C(O)- or -C(O)-(CR^{a}R^{b} )ᵣ -(L⁴)_{q} -NH-; where each q, independently, is 0, 1, 2, 3, or 4; and each r, independently, is 0, 1, 2, 3, or 4;
each L⁴, independently, is -(CR^{a}R^{b})ₛO- or -O(CR^{a}R^{b})ₛ-; where each s, independently, is 0, 1, 2, 3, or 4;
R³ is -H, -R , or -OR ;
each of R⁴ and R⁴' , independently, is -H, halo, cyano, hydroxy, nitro, alkyl, alkenyl, alkynyl, cycloalkyl, alkoxy, or cycloalkoxy;
each R⁵ and each R⁵ , independently, is -H, halo, cyano, hydroxy, nitro, alkyl, alkenyl, alkynyl, or cycloalkyl;
or R⁴ and one R⁵, taken together, can form a 5- to 8-membered cycloalkyl or heterocyclic ring;
each R^{a} , independently, is -H, halo, cyano, hydroxy, nitro, amino, alkylamino, dialkylamino, alkyl, alkenyl, alkynyl, cycloalkyl, alkoxy, cycloalkoxy, aryl, heteroaryl, or heterocyclyl;
each R^{b} , independently, is -H, halo, cyano, hydroxy, nitro, amino, alkylamino, dialkylamino, alkyl, alkenyl, alkynyl, cycloalkyl, alkoxy, cycloalkoxy, aryl, heteroaryl, or heterocyclyl;
each R^{c} is -H, alkyl, acyl, cycloalkyl, alkenyl, alkynyl, aryl, heteroaryl, or heterocyclyl;
a is 0 or 1;
b is an integer from 1 to 1,000;
c is 0 or 1;
each occurrence of i, independently, is 1, 2, 3, 4, 5, or 6;
each occurrence of j , independently, is 0, 1, 2, or 3;
each occurrence of k, independently, is 0, 1, 2, or 3; and
p is 1 to 10; with the proviso that
   (i) X and Y are not simultaneously -CH₂-; and
   (ii) when a is 1 and L¹ is -CH₂-, then
      (a) X and Y are not simultaneously -O-; and
      (b) X and Y are not simultaneously -C(O)O-.

In an embodiment, the polymer conjugated lipid is selected from: 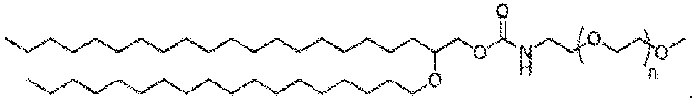 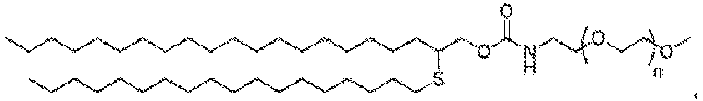 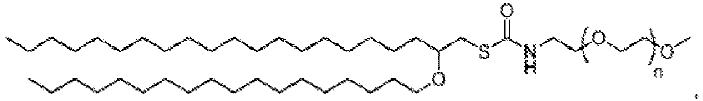 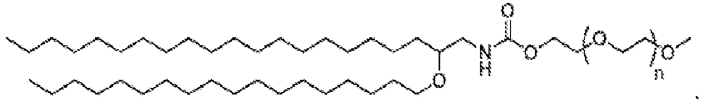 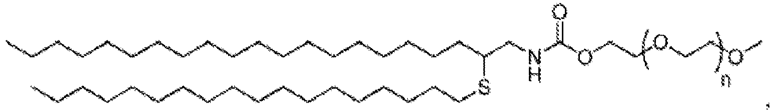 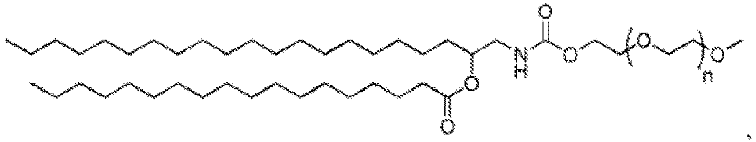 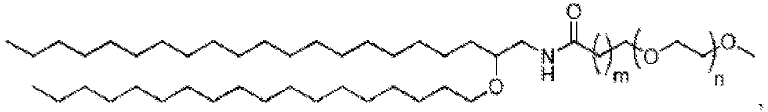 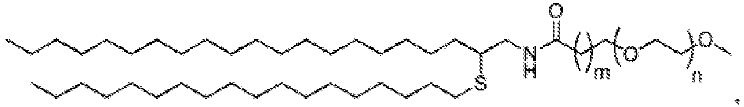 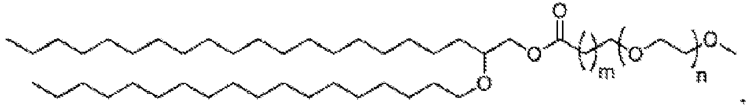 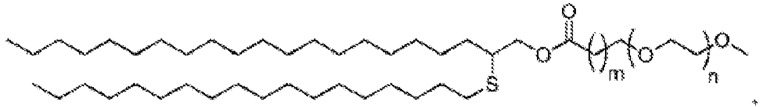 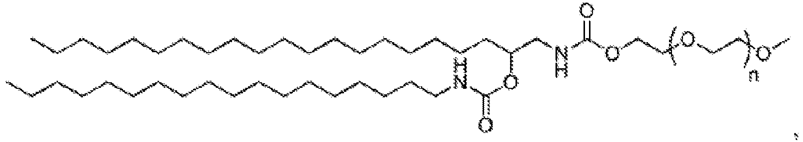 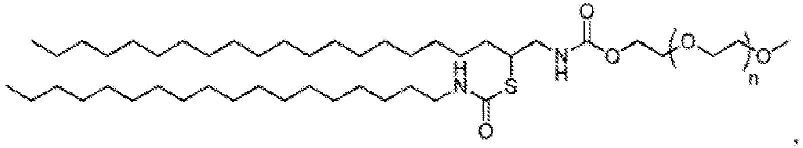 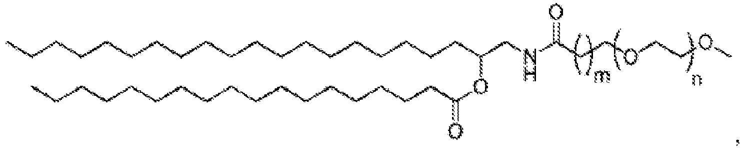 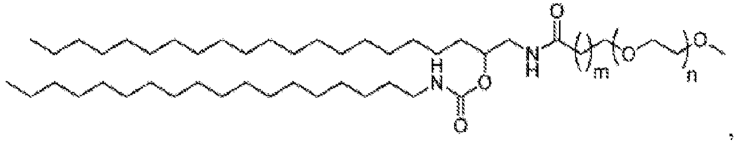 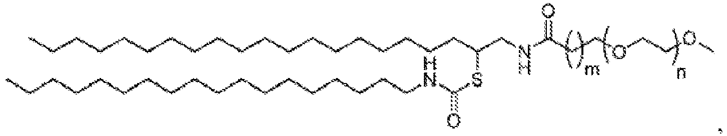 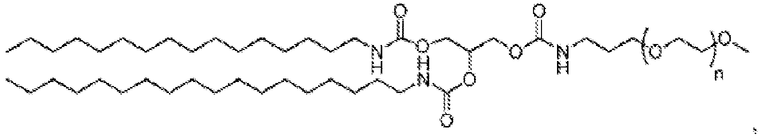 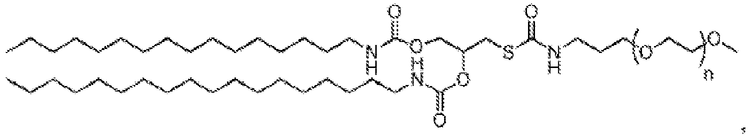 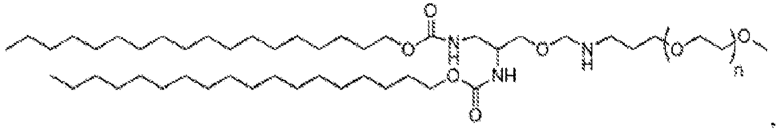 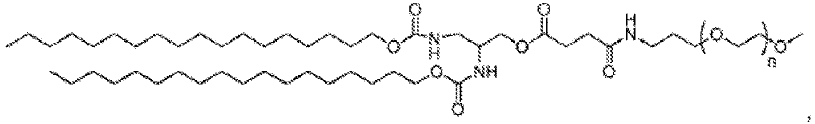 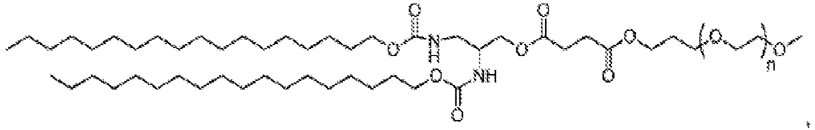 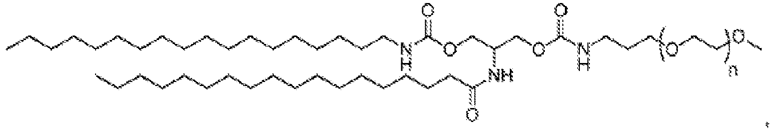 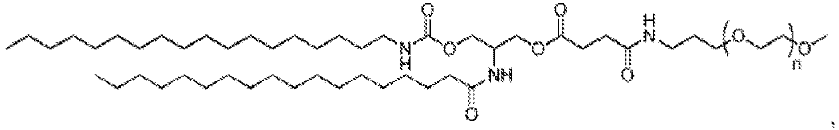 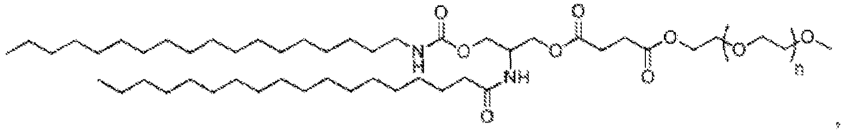 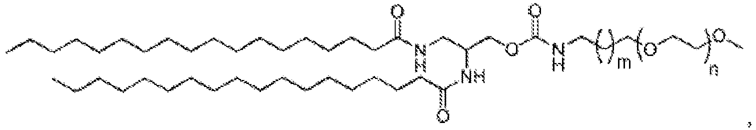 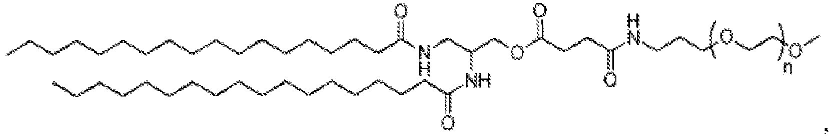 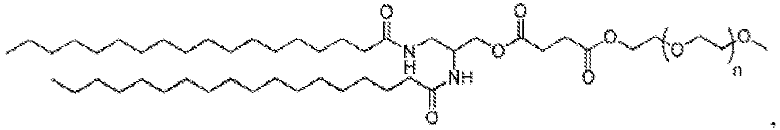 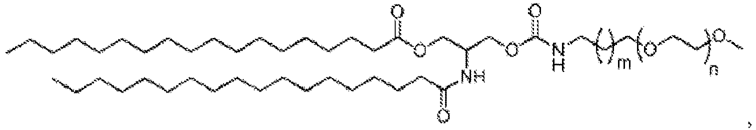 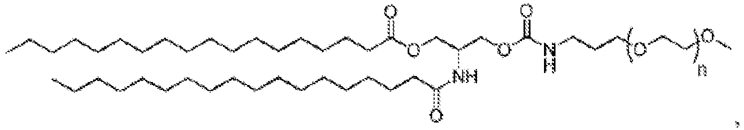 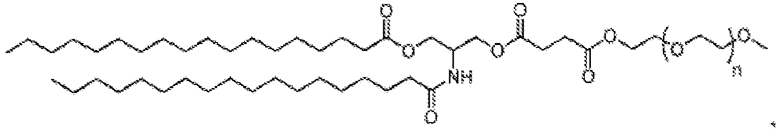 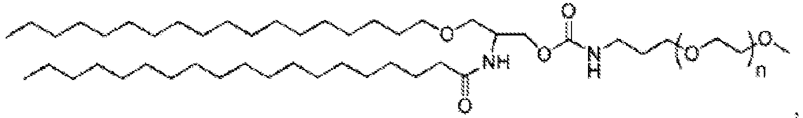 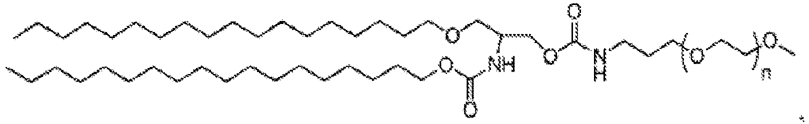 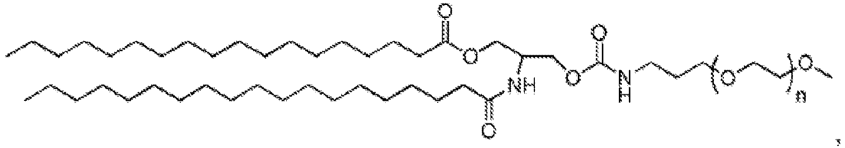 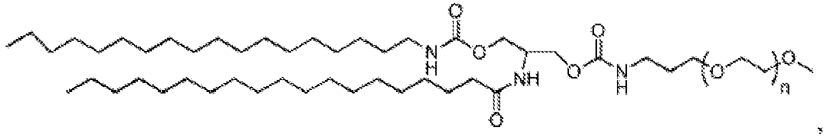 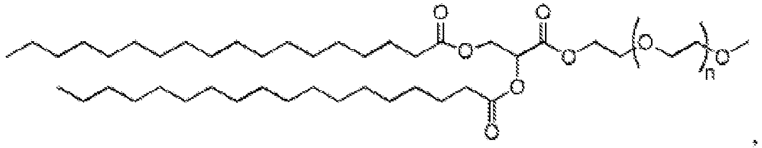 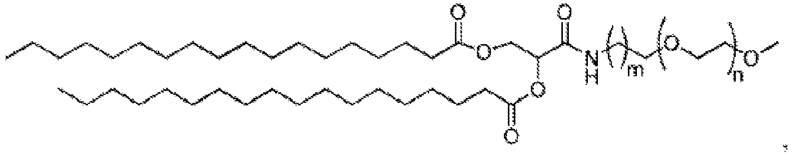 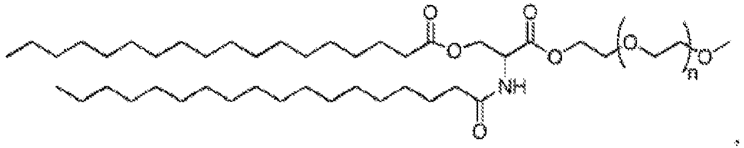 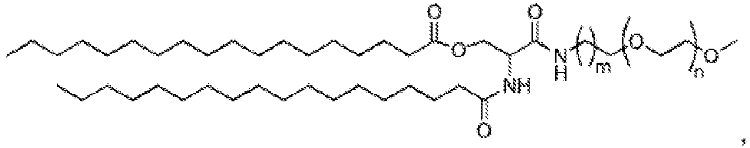 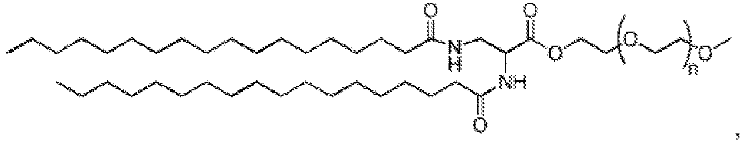 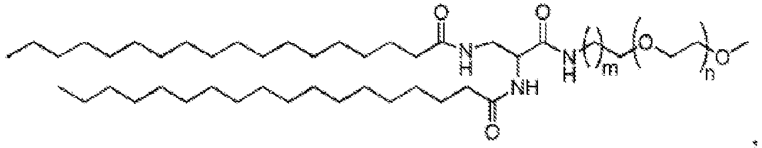 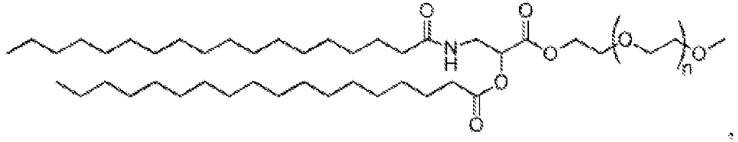 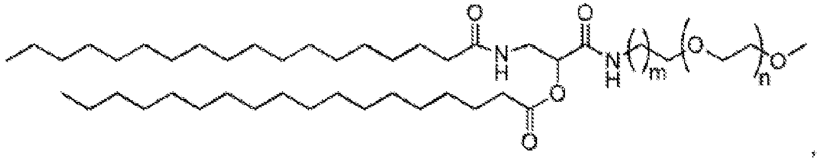 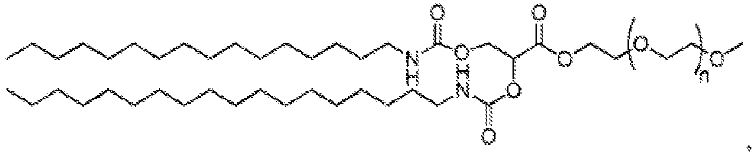 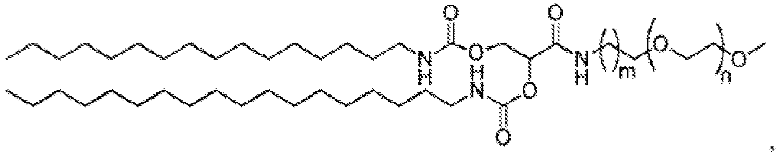 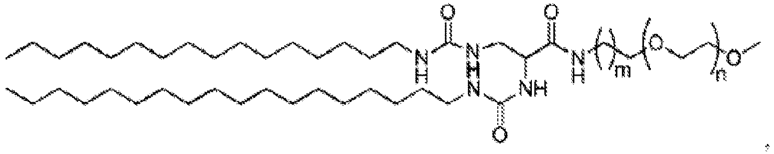 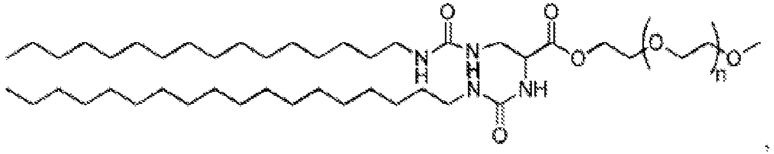 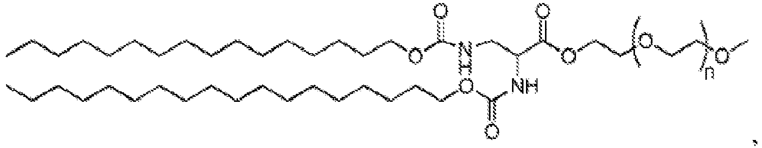 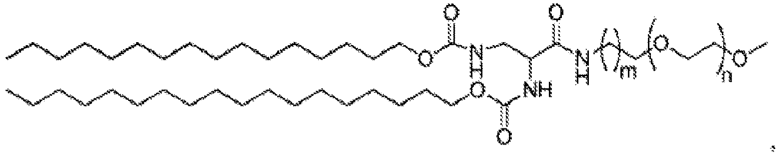 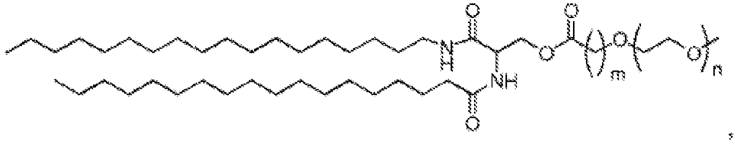 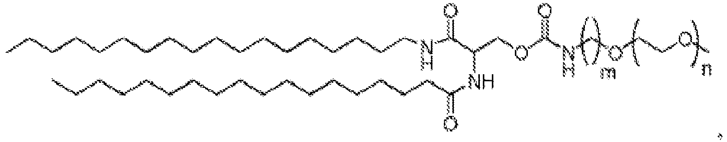 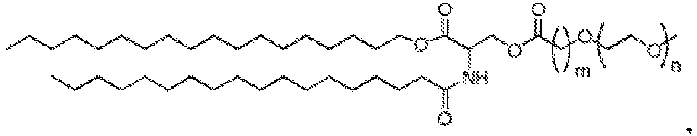 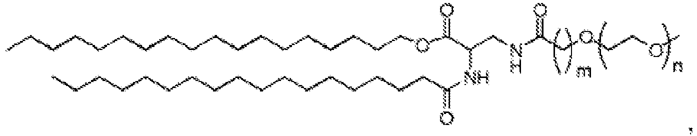 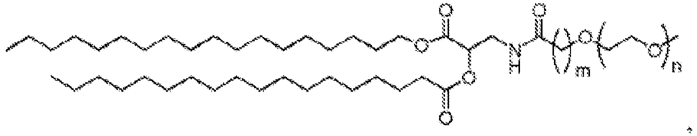 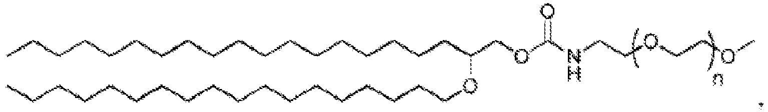 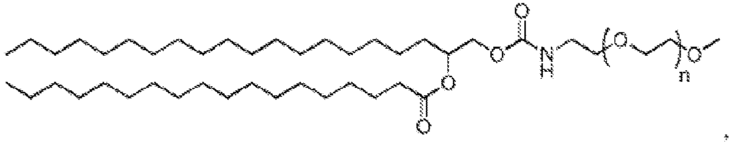 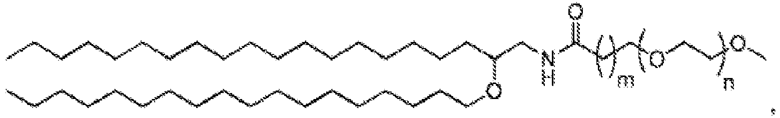 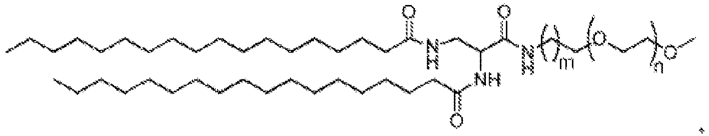 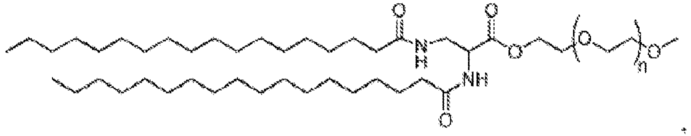 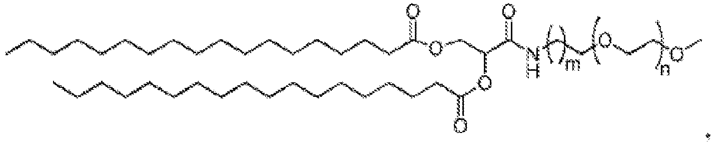 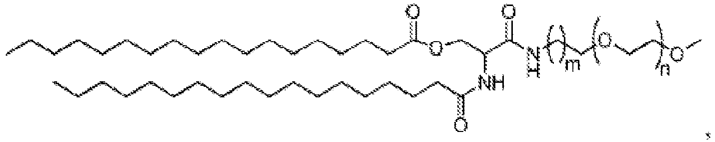 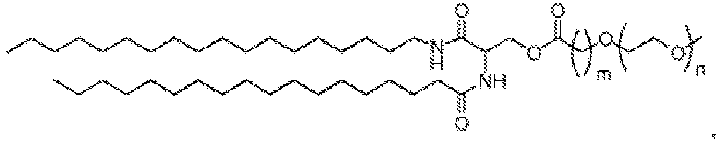 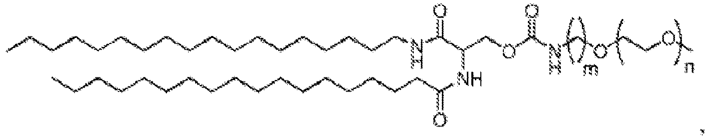 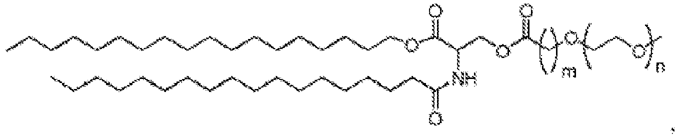 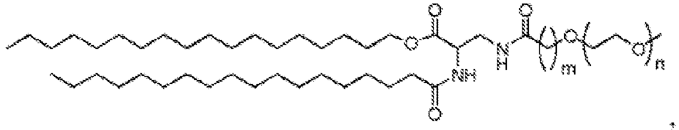 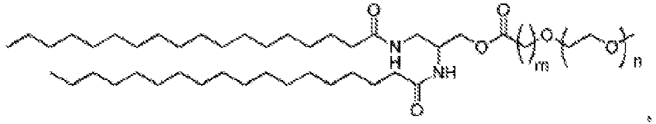 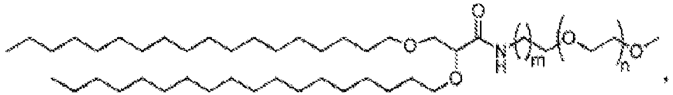 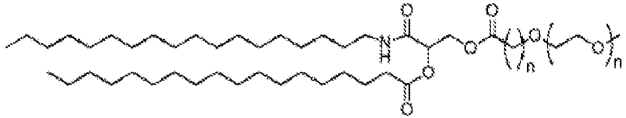 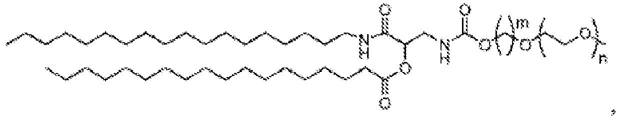 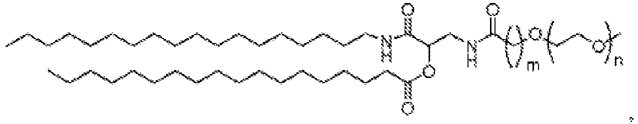 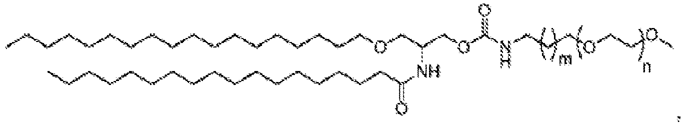 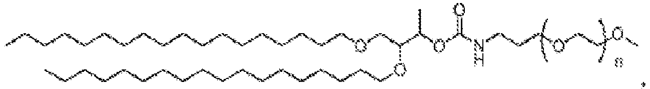 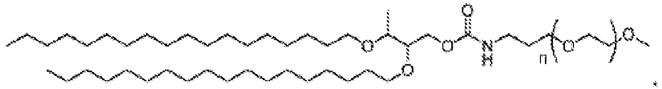 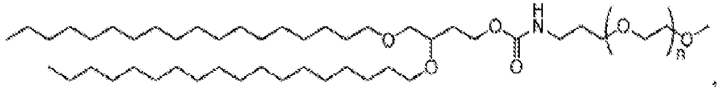 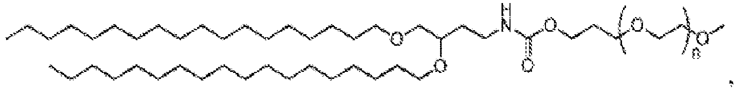 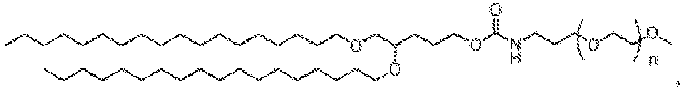 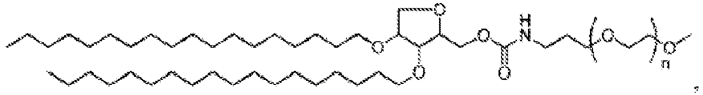 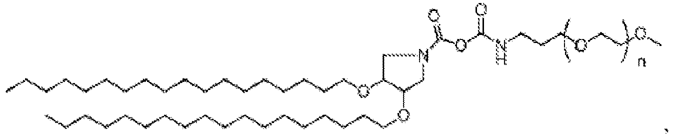 and a pharmaceutically acceptable salts thereof;
wherein
n is an integer from 1 to 1,000; and
m is 1, 2, 3, 4, 5, or 6.

In some other embodiments the LNPs further comprise a polymer conjugated lipid compound of formula: 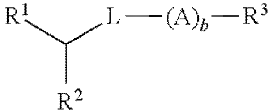 or a pharmaceutically acceptable salt thereof, wherein
R¹ and R² are each, independently, a C₁₀ to C₃₀ aliphatic group, wherein each aliphatic group is optionally substituted by one or more groups each independently selected from R^{a};
L is -L¹-Z¹-(L₂-Z²)_{c}-L³-;
L¹ is a bond, -(CR⁵R^{5'})ᵢ-, or -(CR⁵R^{5'})ᵢ-(C(R^{a})=C(R^{b}))ₖ-(C≡C)ₖ-(CR^{a}R^{b})ⱼ-;
Z¹ is -O-, -S-, -N(R^{c})-, -OC(O)-, -C(O)O-, -OC(O)O-, -N(R^{c})C(O)O-, - N(R^{c})C(O)N(R^{c})-, -N(R^{c})C(O)-, -C(O)N(R^{c})-, -N=C(R^{a})-, -C(R^{a})=N-, -O-N=C(R^{a})-, - O-N(R^{c})-; heteroaryl, or heterocyclyl;
L² is -(CR^{a}R^{b})ₚ- or -(CR^{a}R^{b})ⱼ-(C(R^{a})=C(R^{b}))ₖ-(C≡C)ₖ-(CR^{a}R^{b})ⱼ-;
Z² is -O-, -S-, -N(R^{c})-, -OC(O)-, -C(O)O-, -OC(O)O-, -OC(O)N(R^{c})-, - N(R^{c})C(O)O-, -N(R^{c})C(O)-, -C(O)N(R^{c})-, -N=C(R^{a})-, -C(R^{a})=N-, -O-N=C(R^{a})-, -ON(R^{c})-, heteroaryl, or heterocyclyl;
L³ is -(CR^{a}R^{b})ᵢ-;
each A, independently, is -L⁴-, -NH-(L⁴)_{q}-(CR^{a}R^{b})ᵣ-C(O)-, or -C(O)-(CR^{a}R^{b})ᵣ-(L⁴)_{q}-NH-; wherein each q, independently, is 0, 1, 2, 3, or 4; and each r, independently, is 0, 1, 2, 3, or 4;
each L⁴, independently, is -(CR^{a}R^{b})ₛO- or -O(CR^{a}R^{b})ₛ-, wherein each s, independently, is 0, 1, 2, 3, or 4;
R³ is H, -R^{c}, or -OR^{c};
each occurrence of R⁵ and R^{5'} is, independently, H, halo, cyano, hydroxy, nitro, alkyl, alkenyl, alkynyl, or cycloalkyl;
each occurrence of R^{a} and R^{b} is, independently, H, halo, cyano, hydroxy, nitro, amino, alkylamino, dialkylamino, alkyl, alkenyl, alkynyl, cycloalkyl, alkoxy, aryl, heteroaryl, or heterocyclyl;
each R^{c} is, independently, H, alkyl, acyl, cycloalkyl, alkenyl, alkynyl, aryl, heteroaryl, or heterocyclyl;
b ranges from 5 to about 500;
c is 0 or 1;
each i is, independently, 1, 2, 3, 4, 5, 6, 7, 8, or 9;
each occurrence of j and k, independently, is 0, 1, 2, or 3; and
p is an integer from 1 to 10;
   or
R¹ and R² are each, independently C₁₀ to C₃₀ aliphatic group;
L is -L¹-Z¹-L³-;
L¹ is a bond or -(CR⁵R^{5'})ᵢ-;
Z¹ is -N(R^{c})-, -N(R^{c})C(O)O-, -N(R^{c})C(O)N(R^{c})-, -N(R^{c})C(O)-, or - N=C(R^{a})-, wherein the leftmost nitrogen atom in Z¹ is bound to L¹ or if L¹ is a bond, then to the central tertiary carbon atom of formula (II)), or
Z¹ is a nitrogen-containing heteroaryl or heterocyclyl, wherein the nitrogen atom of the heteroaryl or heterocyclyl is bound to L¹ or if L¹ is a bond, then to the central tertiary carbon atom of formula (II));
L³ is -(CR^{a}R^{b})ᵢ-;
each A is, independently, -L⁴-;
b ranges from about 5 to about 500;
each L⁴, independently, is -OCH₂CH₂-, -CH₂CH₂O-, -OCH(CH₃)CH₂- or -OCH₂CH(CH₃)-;
R³ is -OR^{c};
each occurrence of R^{a}, R^{c}, R⁵ and R^{5'} is, independently, H or alkyl; and i is 2, 3, 4 or 5;
   or
R¹ and R² are each, independently C₁₂ to C₂₀ alkyl or C₁₂ to C₂₀alkenyl;
L is -L¹-Z¹-L²-Z²-L³-;
L¹ is a bond or -(CR⁵R^{5'})ᵢ-;
Z¹ is -N(R^{c})-, -N(R^{c})C(O)O-, -N(R^{c})C(O)N(R^{c})-, -N(R^{c})C(O)-, or - N=C(R^{a})-, wherein the leftmost nitrogen atom in Z¹ is bound to L¹ or if L¹ is a bond, then to the central tertiary carbon atom of formula (II)), or
Z¹ is a nitrogen-containing heteroaryl or heterocyclyl, wherein the nitrogen atom of the heteroaryl or heterocyclyl is bound to L¹ or if L¹ is a bond, then to the central tertiary carbon atom of formula (II);
L² is -(CR^{a}R^{b})ₚ;
Z² is -O-, -C(O)O-, -C(O)N(R^{c})-, or heteroaryl;
L³ is -(CR^{a}R^{b})ᵢ-;
each A is, independently, -L⁴-;
b ranges from about 5 to about 500;
each L⁴, independently, is -OCH₂CH₂-, -CH₂CH₂O-, -OCH(CH₃)CH₂- or -OCH₂CH(CH₃)-;
R³ is -OR^{c};
each occurrence of R^{a}, R^{b}, R^{c}, R⁵ and R^{5'} is, independently, H or alkyl;
i is 2, 3, 4 or 5; and
p is 1 to 10.

In other embodiments, the LNPs comprise a polymer conjugated lipid compound selected from: 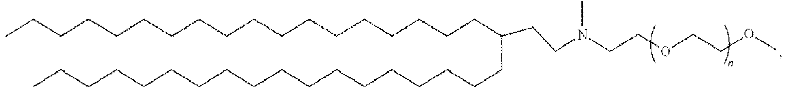 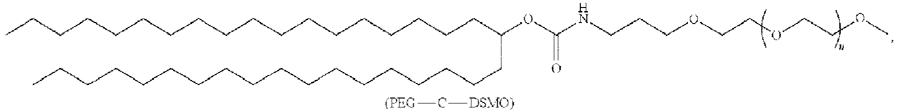 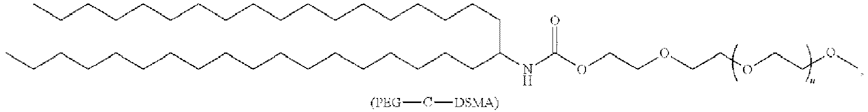 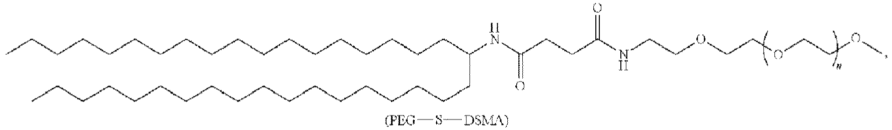 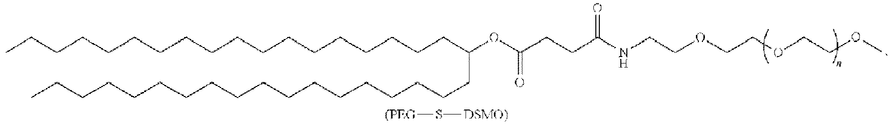 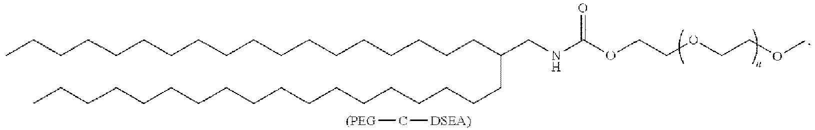 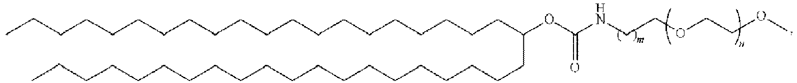 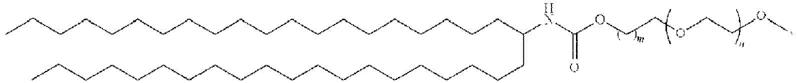 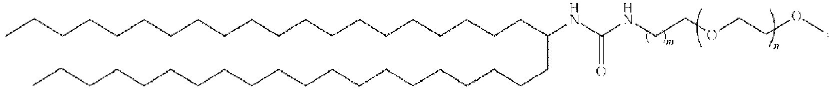 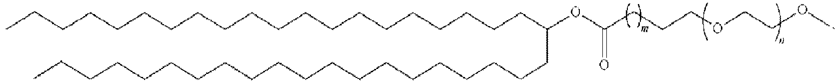 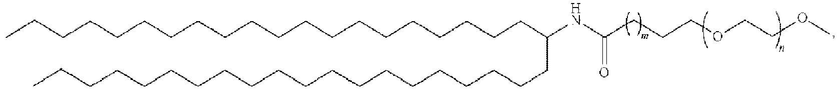 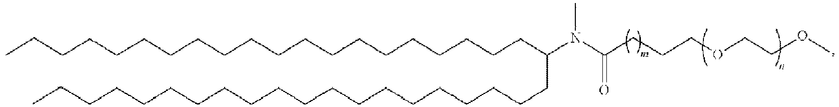 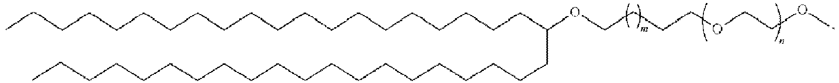 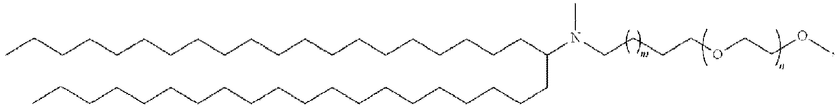 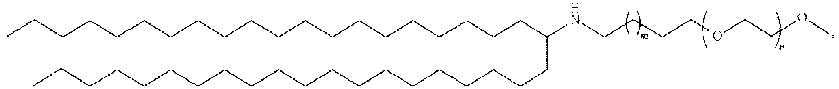 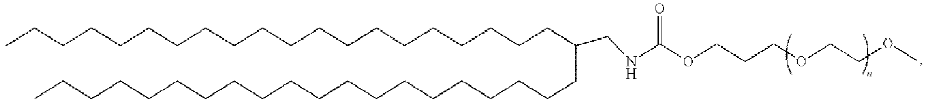 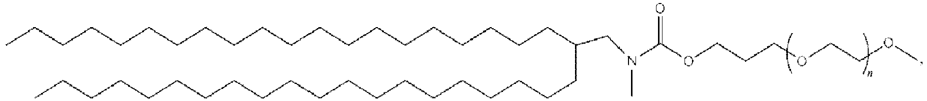 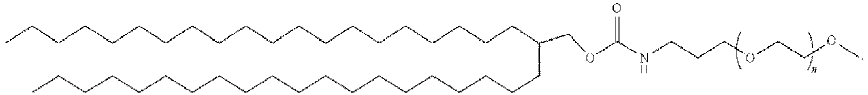 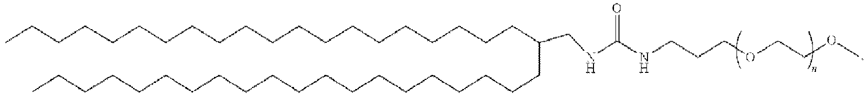 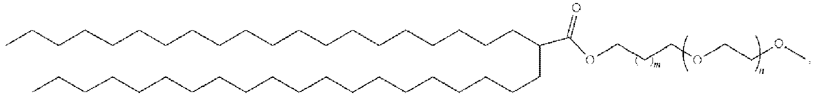 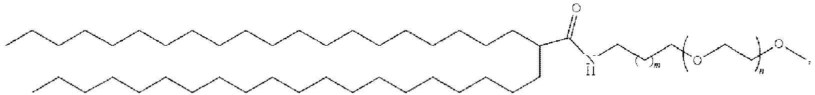 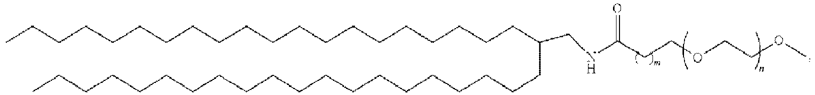 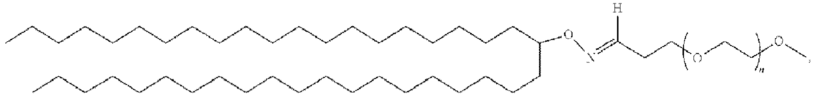 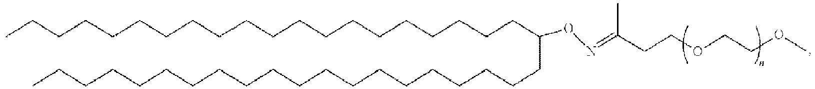 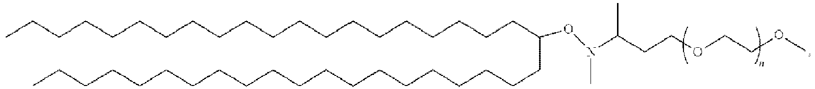 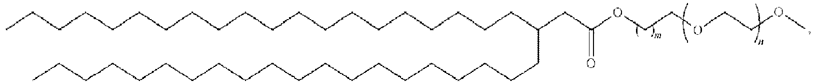 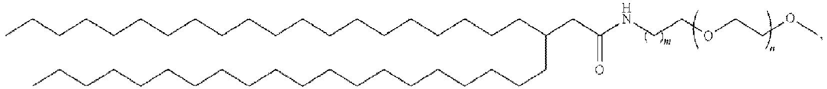 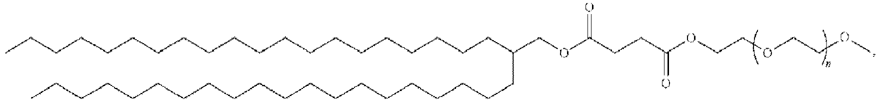 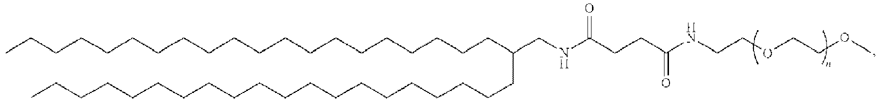 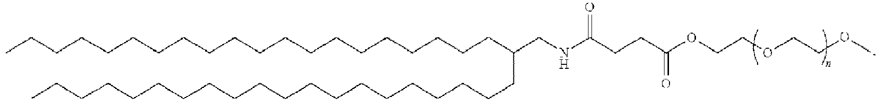 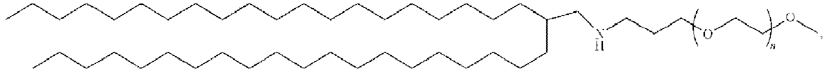 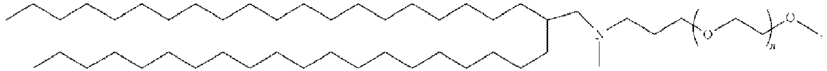 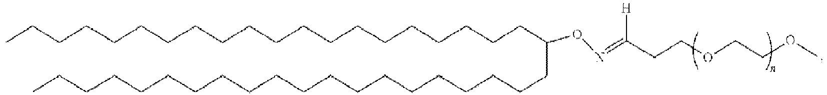 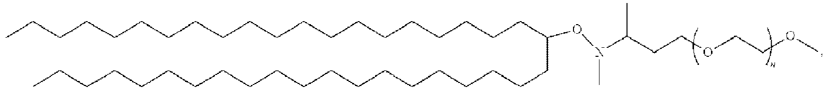 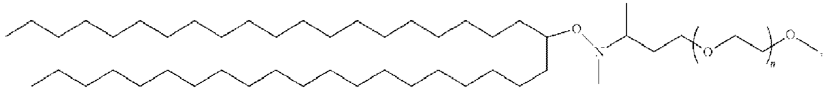 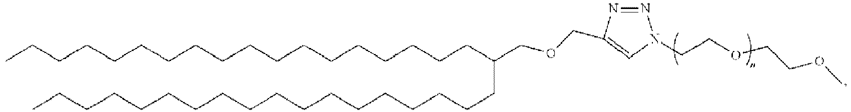 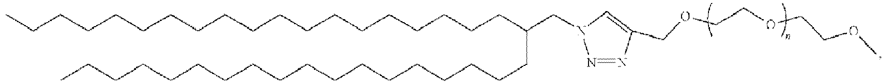 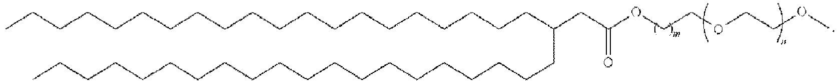 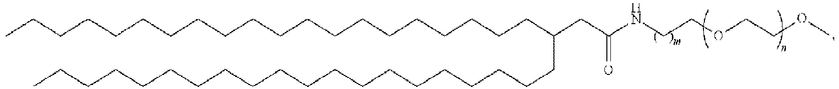 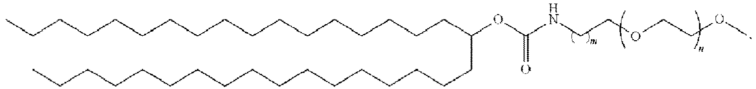 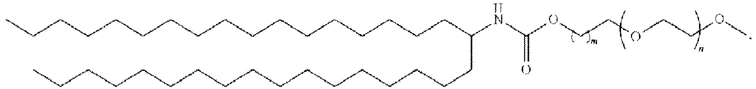 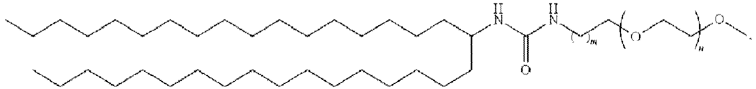 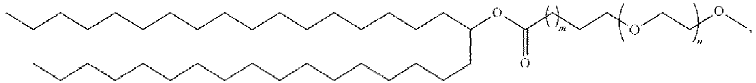 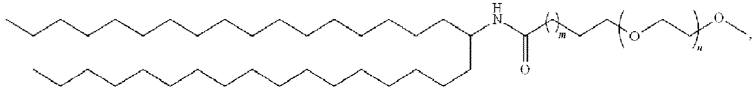 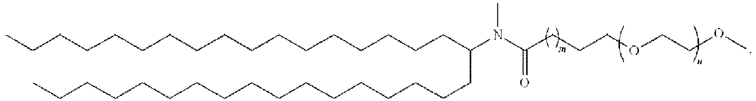 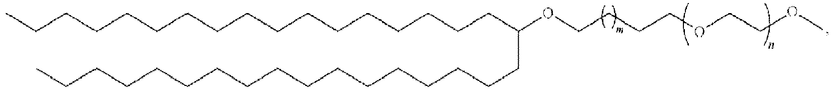 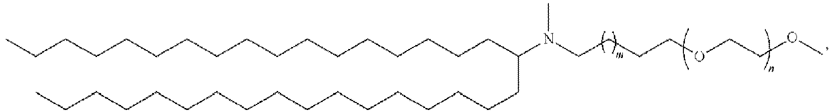 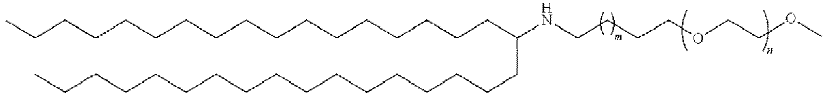 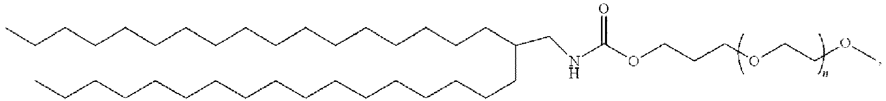 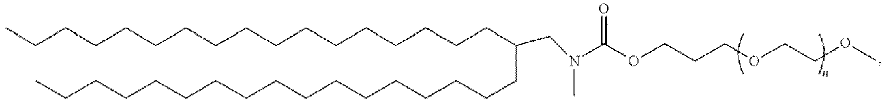 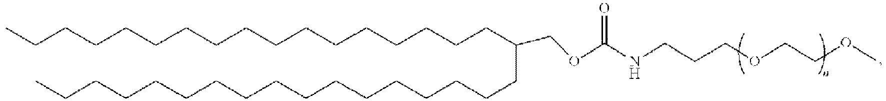 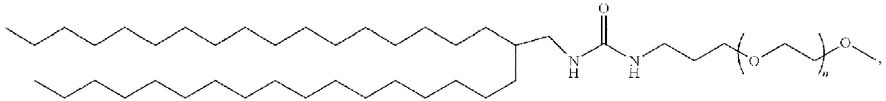 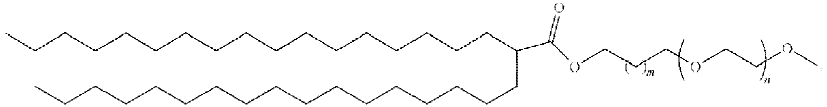 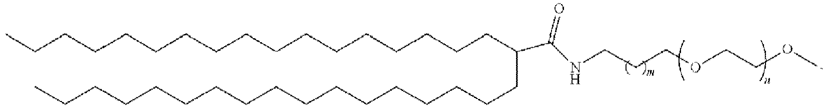 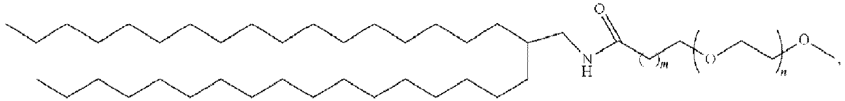 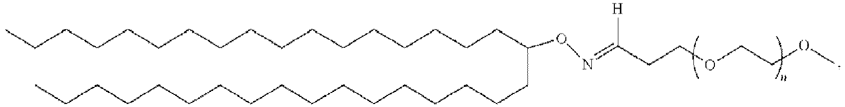 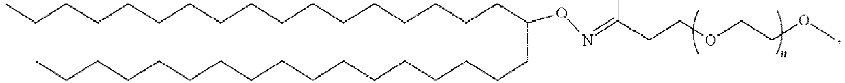 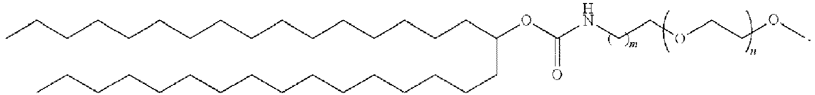 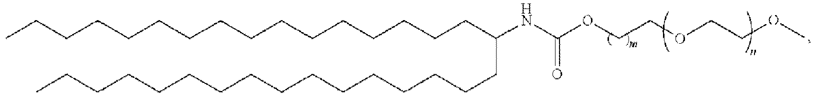 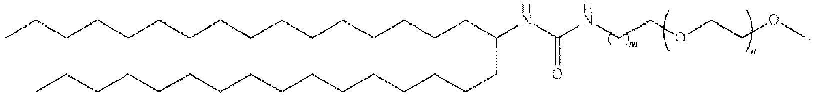 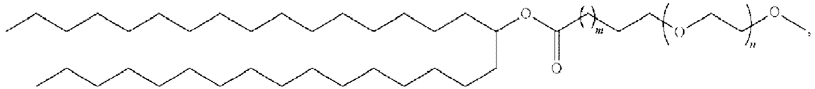 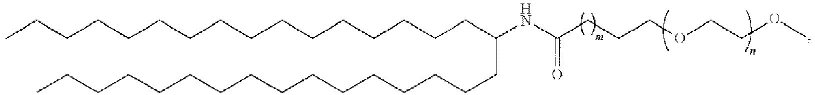 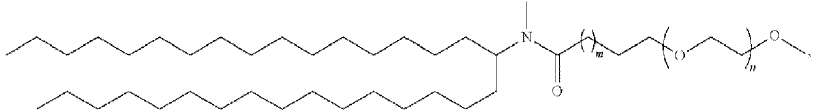 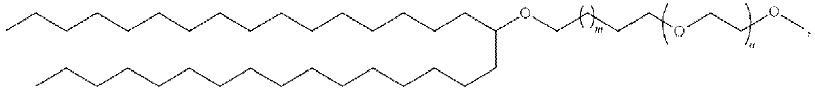 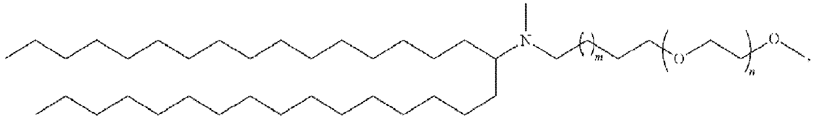 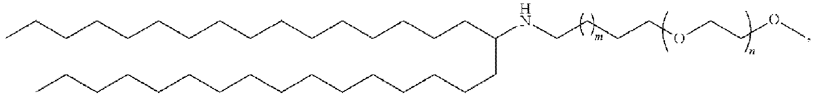 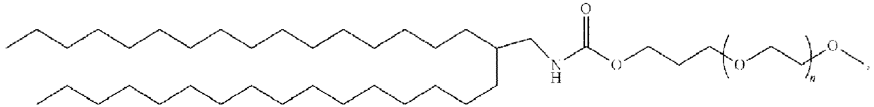 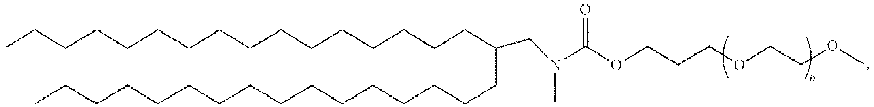 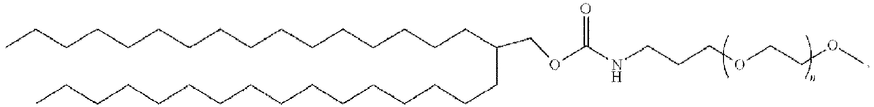 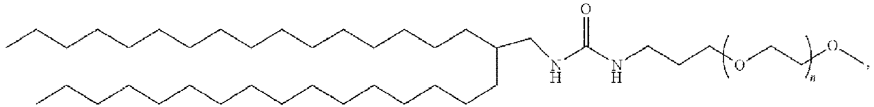 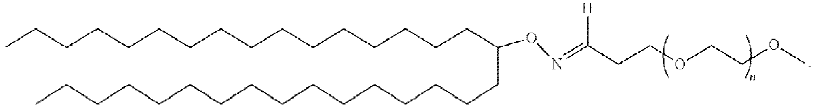 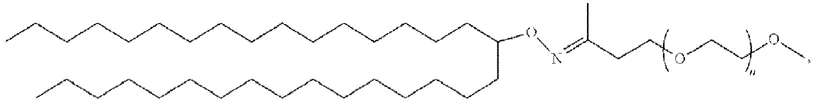 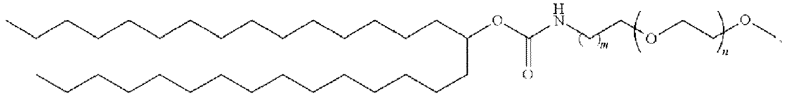 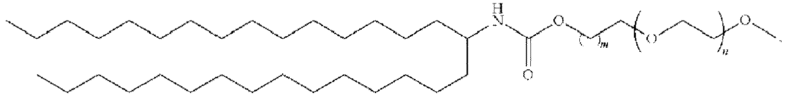 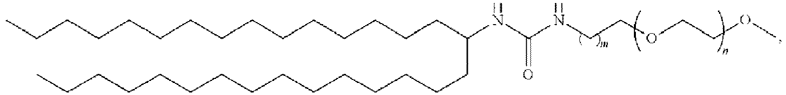 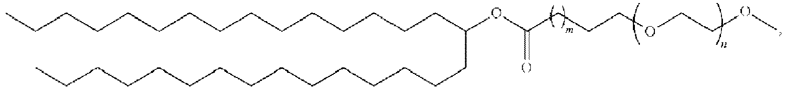 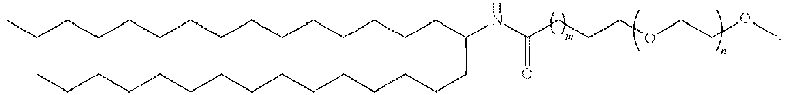 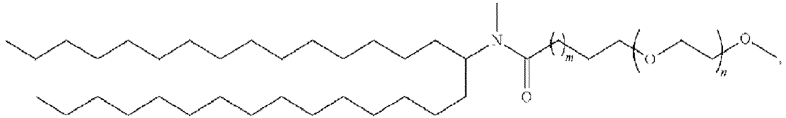 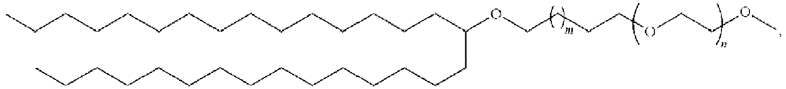 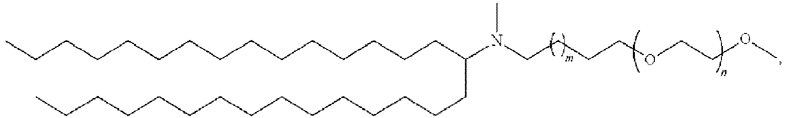 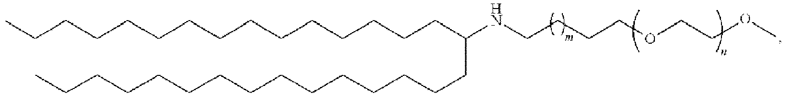 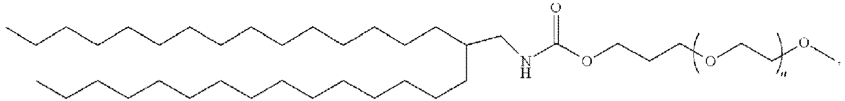 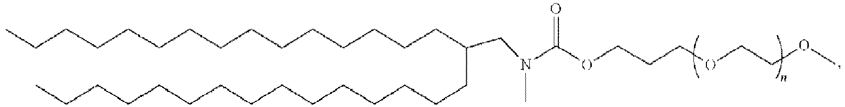 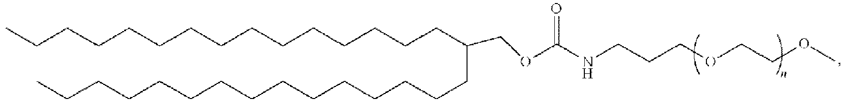 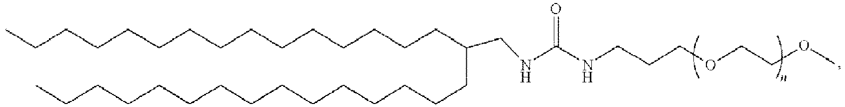 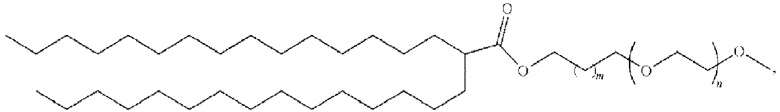 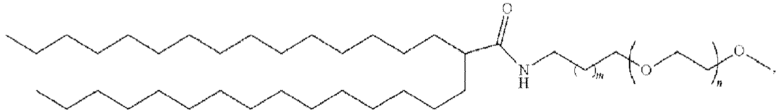 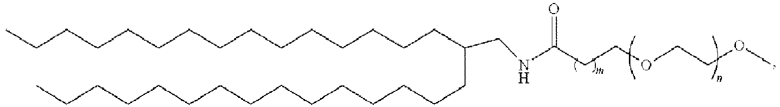 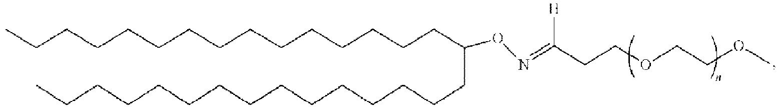 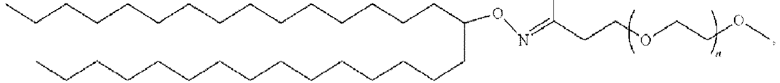 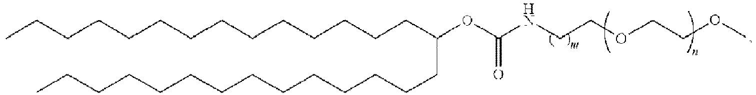 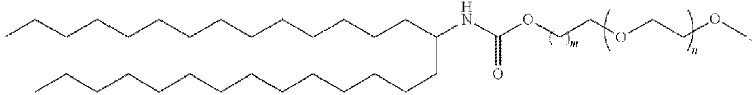 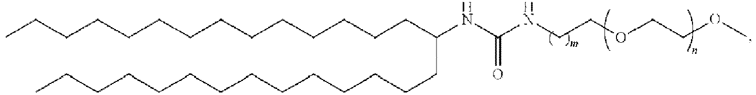 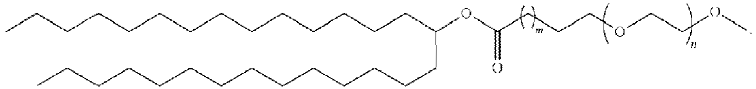 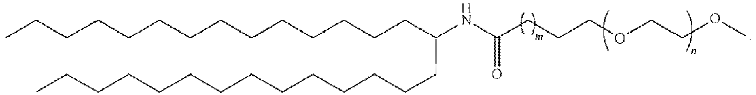 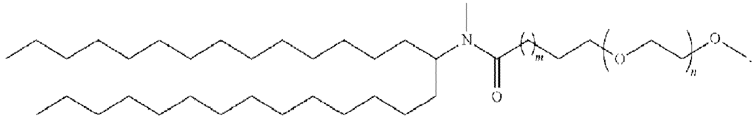 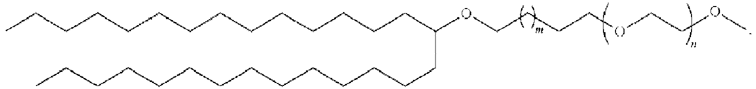 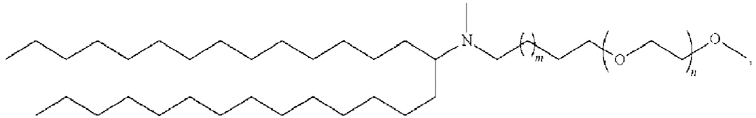 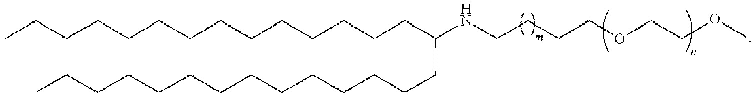 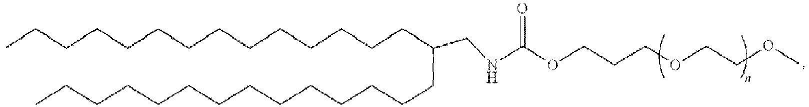 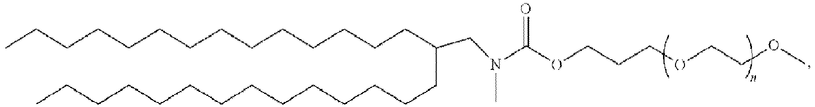 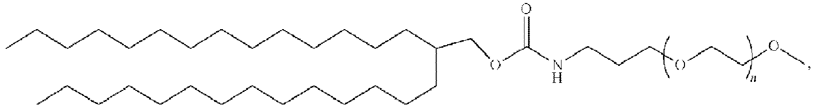 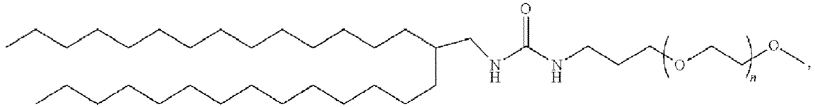 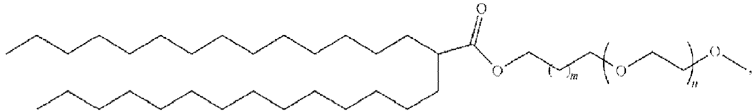 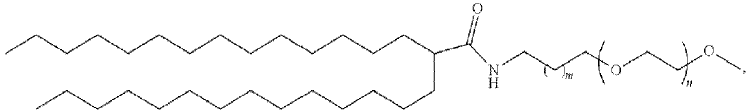 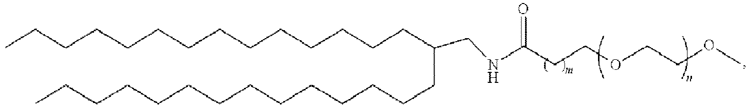 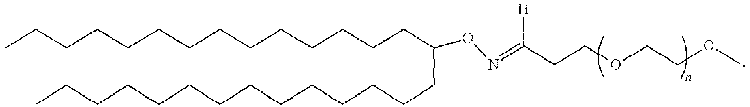 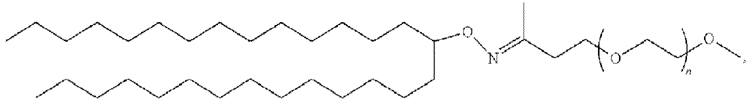 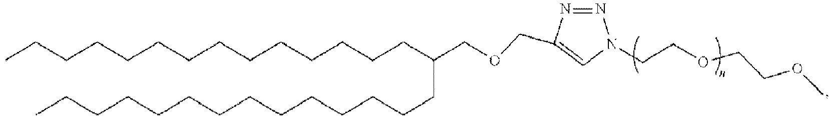 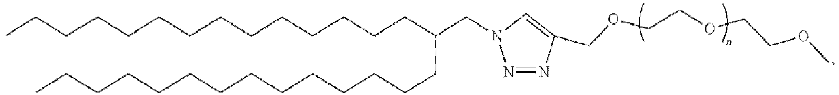 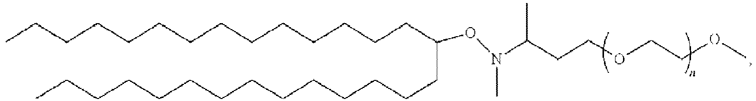 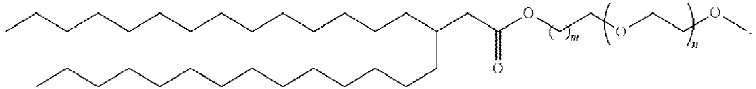 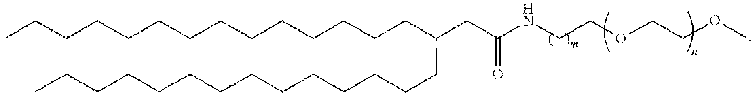 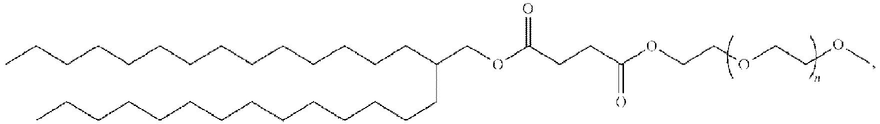 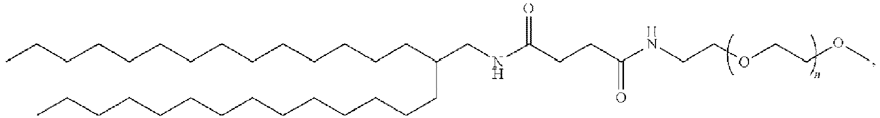 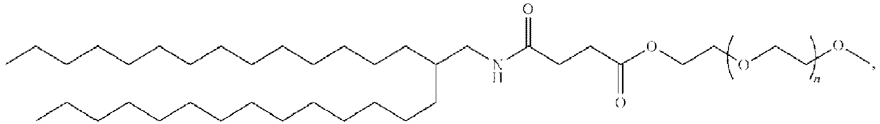 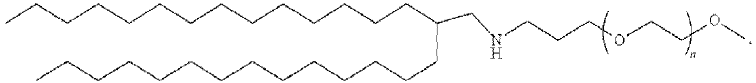 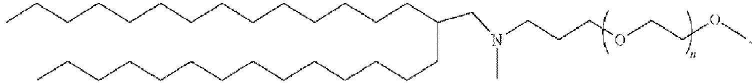 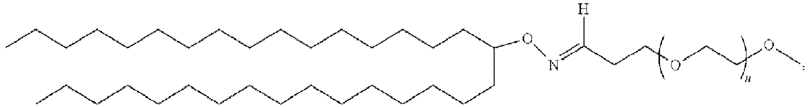 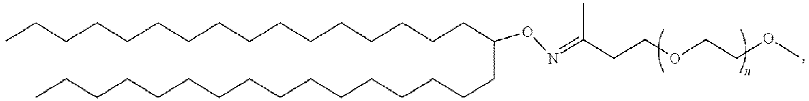 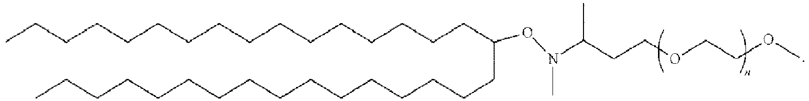 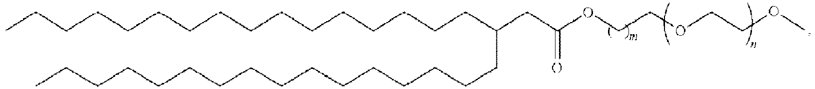 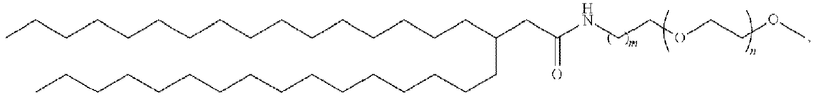 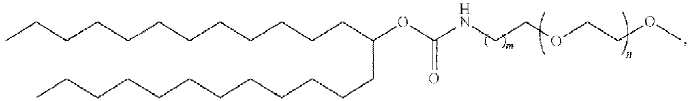 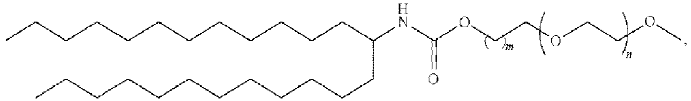 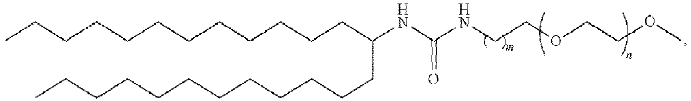 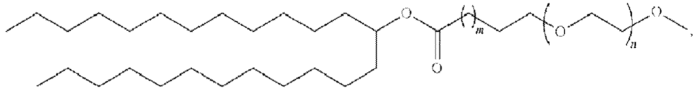 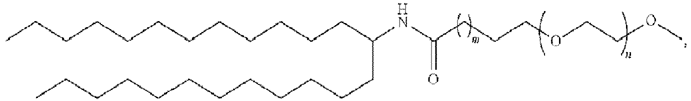 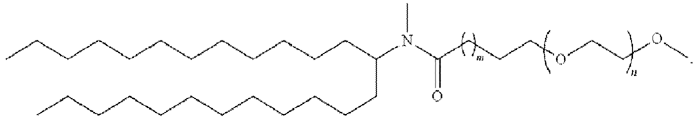 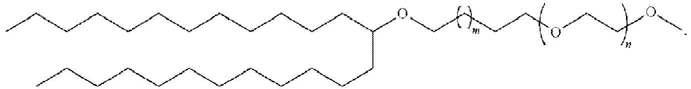 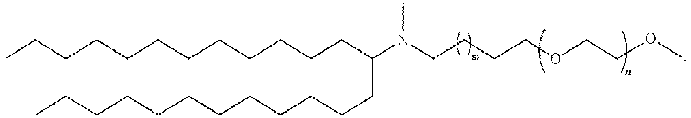 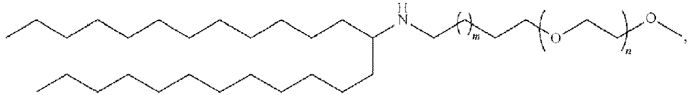 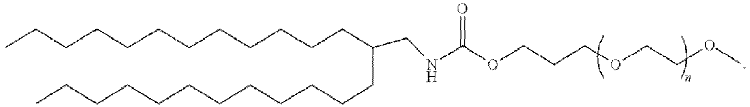 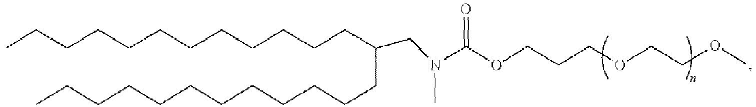 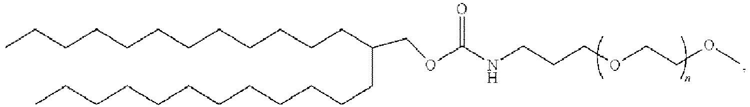 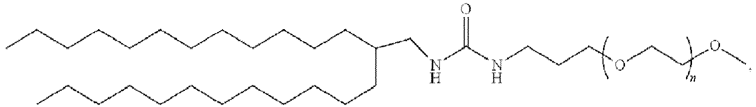 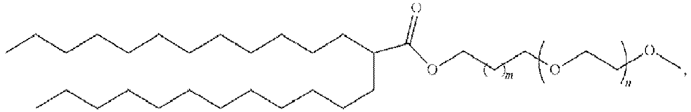 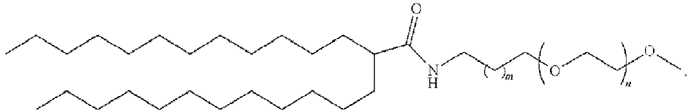 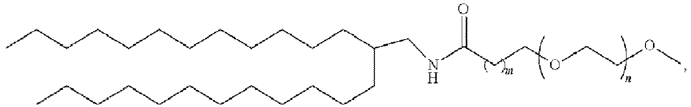 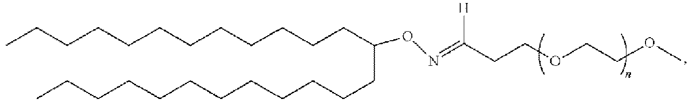 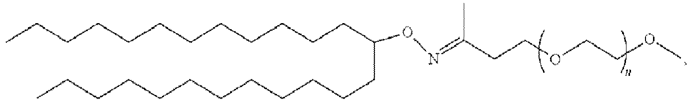 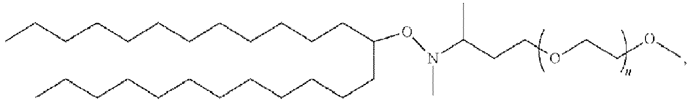 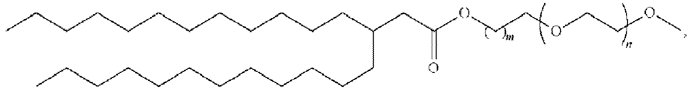 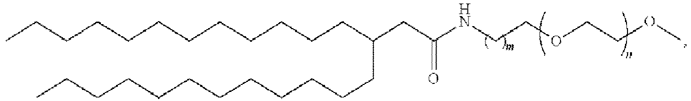 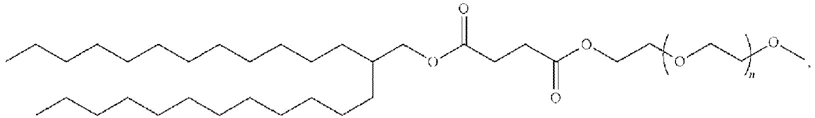 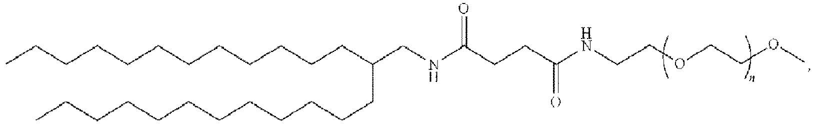 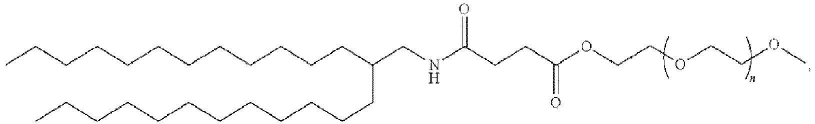 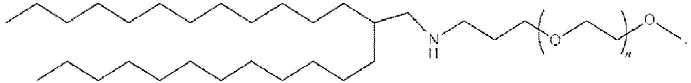 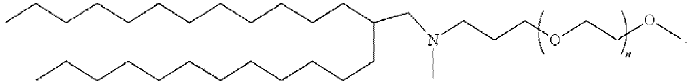 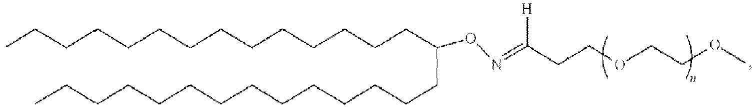 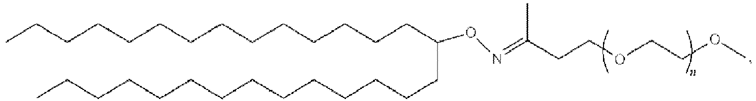 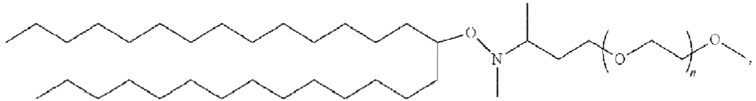 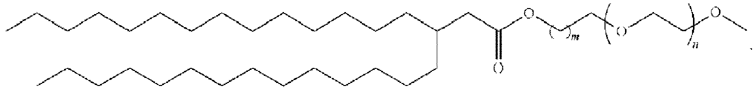 and 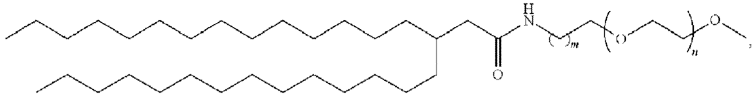 wherein
n is an integer from 1 to 1,000;
m is 0, 1, 2, 3, 4, 5, or 6;
and pharmaceutically acceptable salts thereof.

In some other embodiments, the LNPs further comprise a polymer conjugated lipid selected from representative PEG lipids including, but not limited to: 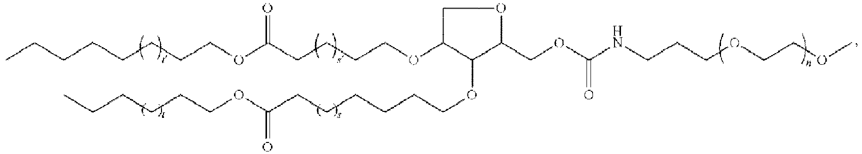 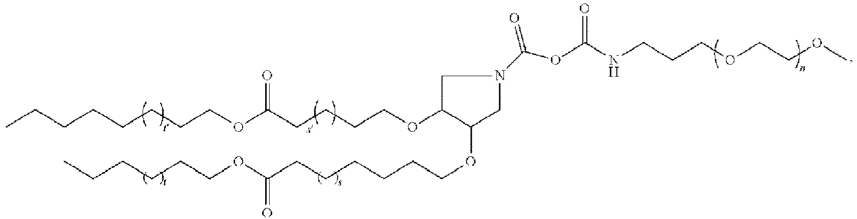 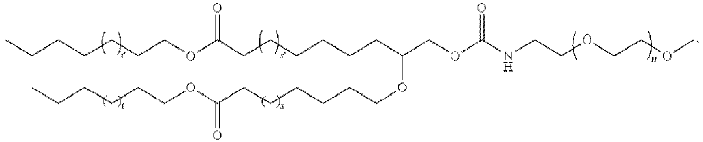 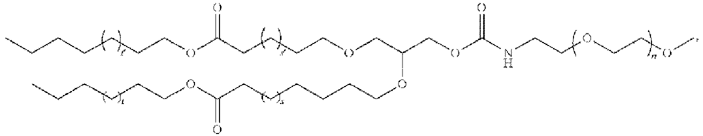 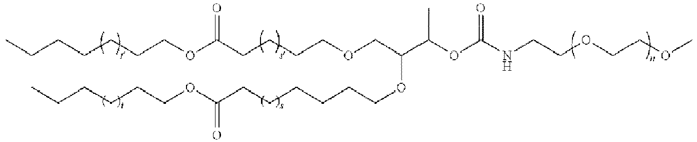 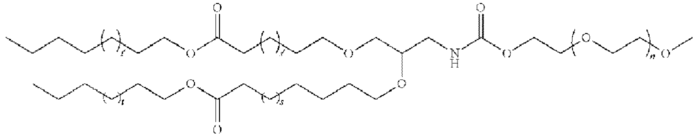 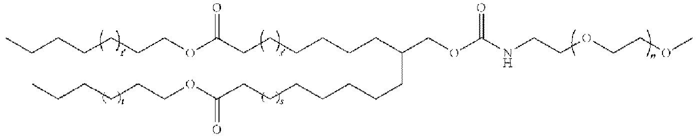 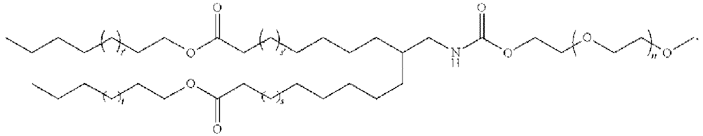 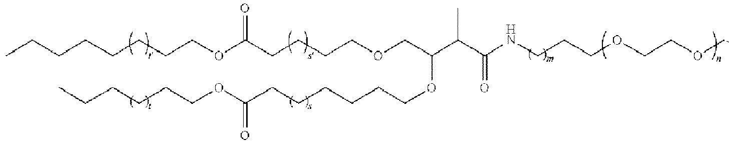 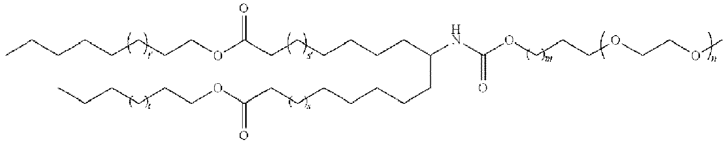 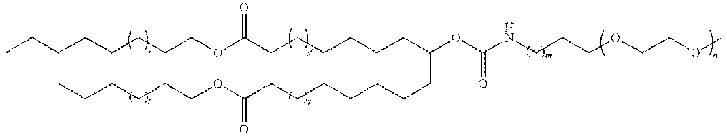 wherein
n is an integer from 10 to 100 (e.g. 20-50 or 40-50);
s, s', t and t' are independently 0, 1, 2, 3, 4, 5, 6 or 7; and m is 1, 2, 3, 4, 5, or 6.

Other representative PEG lipids include, but are not limited to:

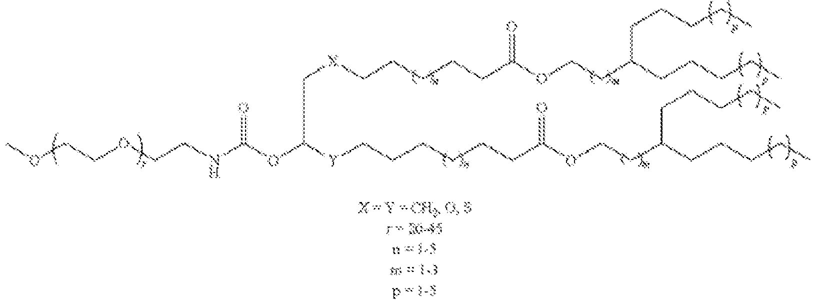

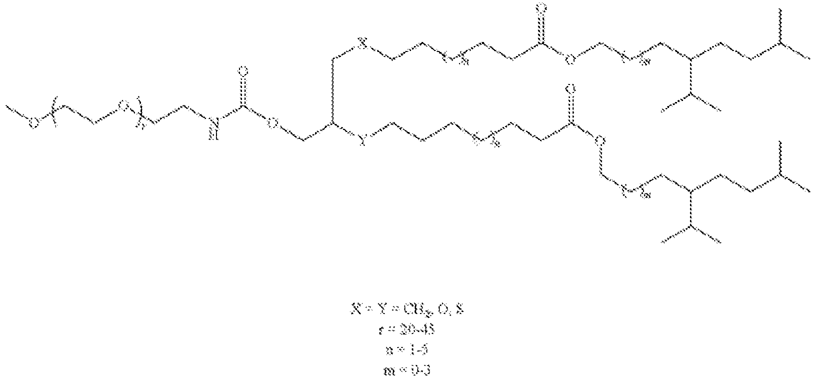

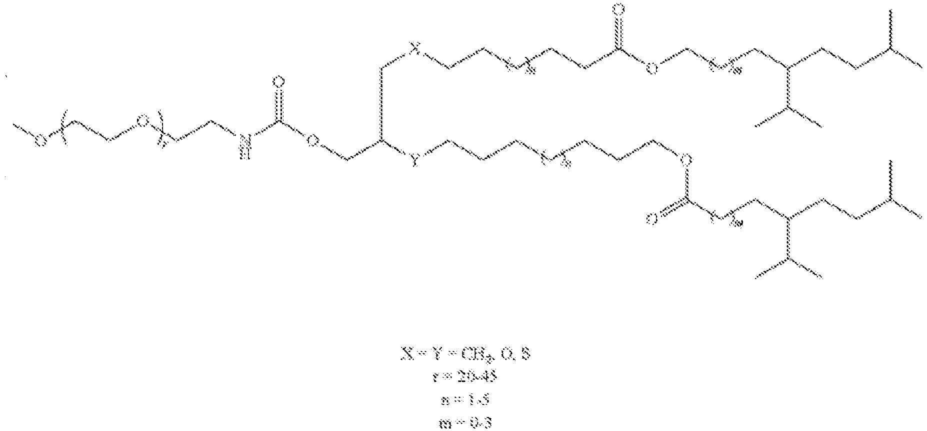

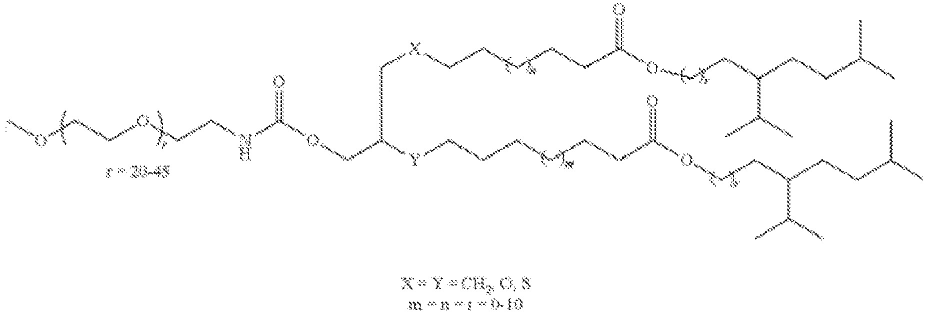

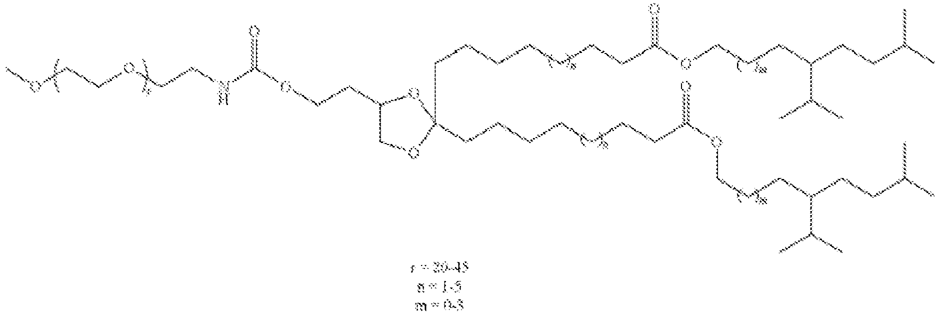

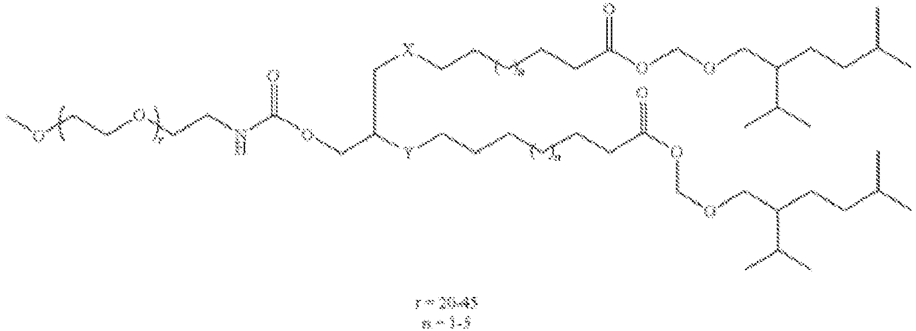

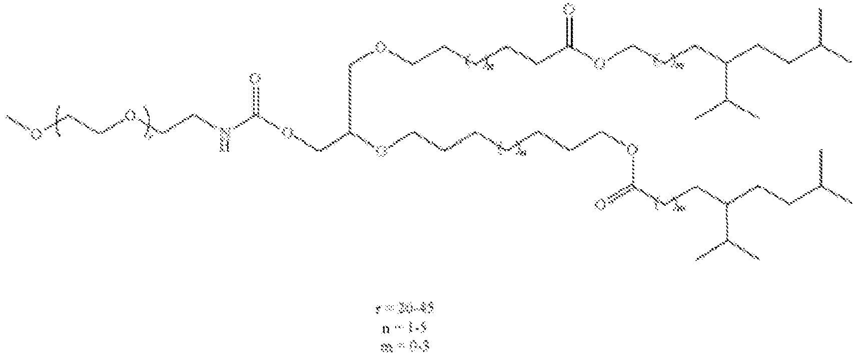

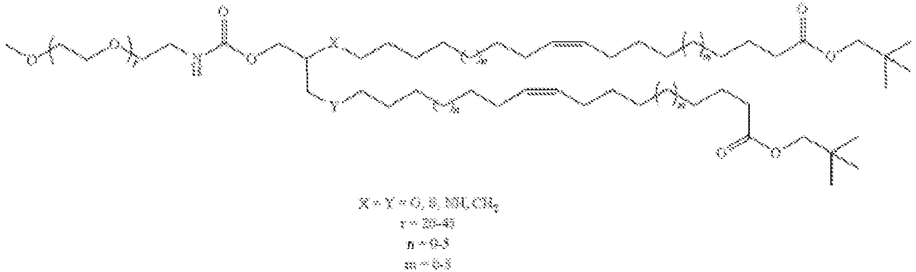

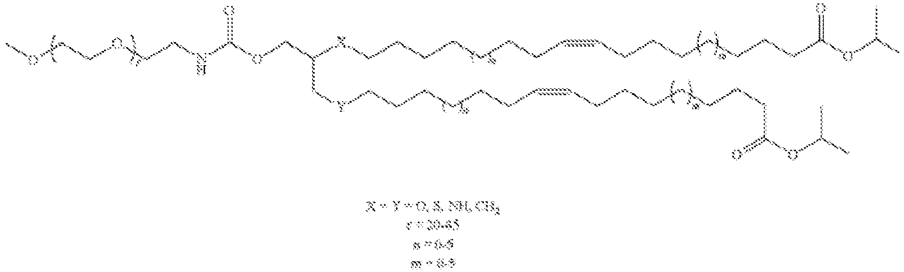

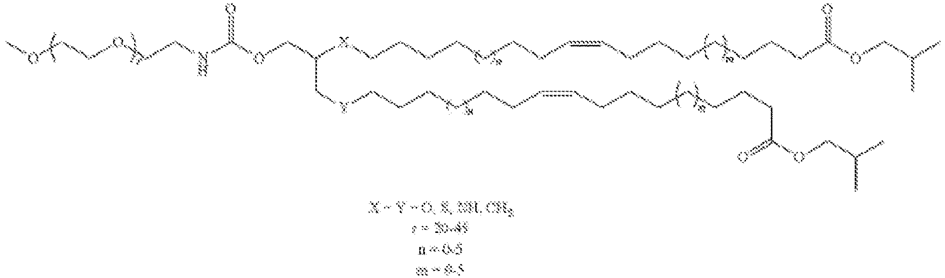

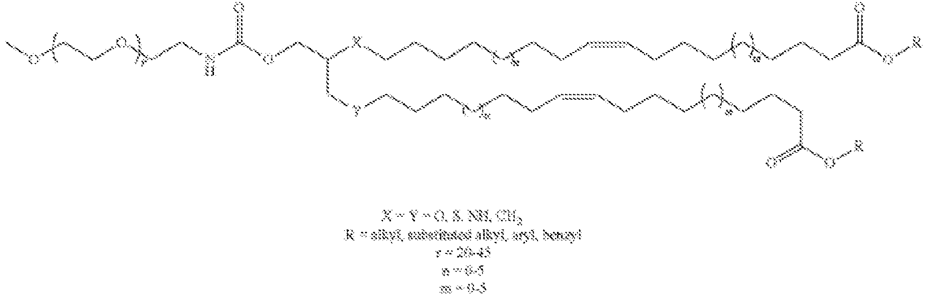

In some embodiments, the ratio of polymer conjugated lipid in the LNPs may be increased or decreased to alter the pharmacokinetics and/or biodistribution of the LNPs. In certain embodiments, LNPs may contain from 0.1 to 5.0, from 1.0 to 3.5, from 1.5 to 4.0, from 2.0 to 4.5, from .0 to 3.0, from 2.5 to 5.0, and/or from 3.0 to 6.0 molar percent of the polymer conjugated lipid to the other components. In various embodiments, the polymer conjugated lipid is present in a concentration ranging from 1.0 to 3.0 molar percent. In certain specific embodiments, the LNP comprises from 2.2 to 3.3, from 2.3 to 2.8, from 2.1 to 2.5, or from 2.5 to 2.9 molar percent of polymer conjugated lipid. In yet more specific embodiments the polymer conjugated lipid is present in a concentration of about 2.0 molar percent. In some embodiments, the polymer conjugated lipid is present in a concentration of about 2.3 molar percent. In some embodiments, the polymer conjugated lipid is present in a concentration of about 2.4 molar percent. In some embodiments, the polymer conjugated lipid is present in a concentration of about 2.5 molar percent. In some embodiments, the polymer conjugated lipid is present in a concentration of about 2.6 molar percent. In some embodiments, the polymer conjugated lipid is present in a concentration of about 2.7 molar percent. In some embodiments, the polymer conjugated lipid is present in a concentration of about 2.8 molar percent. In some embodiments, the polymer conjugated lipid is present in a concentration of about 3.0 molar percent.

In certain embodiments, the molar ratio of cationic lipid to the polymer conjugated lipid ranges from about 35:1 to about 15:1. In some embodiments, the molar ratio of cationic lipid to polymer conjugated lipid ranges from about 100:1 to about 10:1.

In an embodiment, polymer conjugated lipid has the structure: 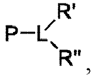 wherein:
P is a polymer;
L is a trivalent linker of 1 to 15 atoms in length; and
R' and R" are each independently a saturated alkyl having from 8 to 14 carbon atoms.

In a more specific embodiment, the polymer conjugated lipid has one of the following structures: 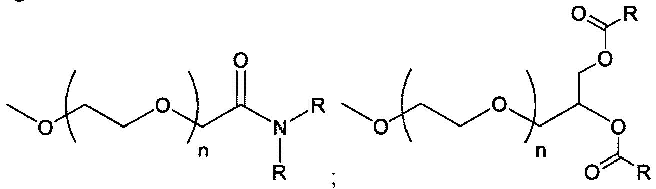 or 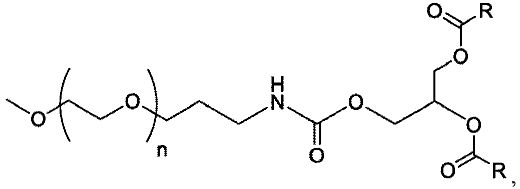 wherein n is an integer ranging from 30 to 60.

In some embodiments the polymer conjugated lipid, when present, has the following structure: 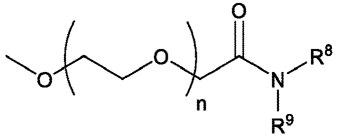 or a pharmaceutically acceptable salt, tautomer or stereoisomer thereof, wherein:
R⁸ and R⁹ are each independently a straight or branched, saturated or unsaturated alkyl chain containing from 8 to 30 carbon atoms, wherein the alkyl chain is optionally interrupted by one or more ester bonds; and
n has a mean value ranging from 30 to 60, or 15 to 25, or 100 to 125.

In some embodiments, R⁸ and R⁹ are each independently straight, saturated alkyl chains containing from 8 to 16 carbon atoms. In other embodiments, the average n ranges from 42 to 55, for example, the average w is 42, 43, 44, 45, 46, 47, 48, 49, 50, 51, 52, 53, 54 or 55. In some specific embodiments, the average w is about 49.

In other specific embodiments the polymer conjugated lipid has the following structure: 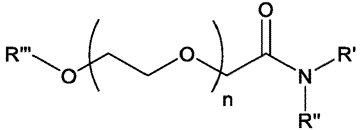 or a salt thereof, wherein:
R' and R" are each independently a saturated alkyl having from 8 to 12 carbon atoms;
R‴ is H or C₁-C₆ alkyl; and
n is an integer ranging from 30 to 60.

In yet another specific embodiments the polymer conjugated lipid has the following structure: 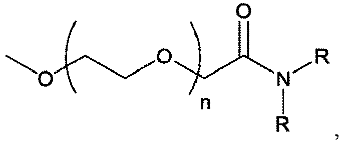 wherein n is an integer ranging from 40 to 50, and each R is a saturated alkyl having from 8 to 14 carbon atoms, or 8 to 12 carbon atoms, or 8 carbon atoms, or 10 carbon atoms, or 12 carbon atoms.

In some preferred embodiments, the polymer conjugated lipid has the following structure: 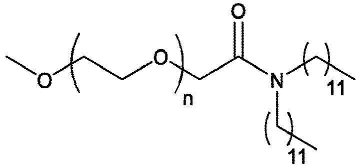 wherein the average n is about 49.

### Nucleic Acids

In certain embodiments, lipid nanoparticles are associated with a nucleic acid, resulting in a nucleic acid-lipid nanoparticle. In particular embodiments, the nucleic acid is fully encapsulated in the lipid nanoparticle. As used herein, the term "nucleic acid" is meant to include any oligonucleotide or polynucleotide. Fragments containing up to 50 nucleotides are generally termed oligonucleotides, and longer fragments are called polynucleotides. In particular embodiments, oligonucletoides are 15-50 nucleotides in length.

The terms "polynucleotide" and "oligonucleotide" refer to a polymer or oligomer of nucleotide or nucleoside monomers consisting of naturally occurring bases, sugars and intersugar (backbone) linkages. The terms "polynucleotide" and "oligonucleotide" also includes polymers or oligomers comprising non-naturally occurring monomers, or portions thereof, which function similarly. Such modified or substituted oligonucleotides are often preferred over native forms because of properties such as, for example, enhanced cellular uptake and increased stability in the presence of nucleases.

In some embodiments the nucleic acid is selected from antisense, self amplifying RNA and messenger RNA. For example, messenger RNA may be used to induce an immune response (e.g., as a vaccine), for example by translation of immunogenic proteins.

In other embodiments, the nucleic acid is mRNA, and the mRNA to lipid ratio in the LNP (i.e., N/P, were N represents the moles of cationic lipid and P represents the moles of phosphate present as part of the nucleic acid backbone) range from 2:1 to 30:1, for example 3:1 to 22:1. In other embodiments, N/P ranges from 6:1 to 20:1 or 2: 1 to 12:1. Exemplary N/P ranges include about 3:1. About 6:1, about 9:1, about 12:1 and about 22:1.

The nucleic acid that is present in a lipid-nucleic acid particle includes any form of nucleic acid that is known. The nucleic acids used herein can be single-stranded DNA or RNA, or double- stranded DNA or RNA, or DNA-RNA hybrids. Examples of double- stranded DNA include structural genes, genes including control and termination regions, and self-replicating systems such as viral or plasmid DNA. Examples of double- stranded RNA include siRNA and other RNA interference reagents. Single- stranded nucleic acids include, e.g., messenger RNA, antisense oligonucleotides, ribozymes, microRNA, and triplex-forming oligonucleotides. The nucleic acid that is present in a lipid-nucleic acid particle may include one or more of the oligonucleotide modifications described below.

Nucleic acids may be of various lengths, generally dependent upon the particular form of nucleic acid. For example, in particular embodiments, plasmids or genes may be from about 1,000 to 100,000 nucleotide residues in length. In particular embodiments, oligonucleotides may range from about 10 to 100 nucleotides in length. In various related embodiments, oligonucleotides, single- stranded, double- stranded, and triple- stranded, may range in length from about 10 to about 50 nucleotides, from about 20 o about 50 nucleotides, from about 15 to about 30 nucleotides, from about 20 to about 30 nucleotides in length.

In particular embodiments, the oligonucleotide (or a strand thereof) specifically hybridizes to or is complementary to a target polynucleotide. "Specifically hybridizable" and "complementary" are terms which are used to indicate a sufficient degree of complementarity such that stable and specific binding occurs between the DNA or RNA target and the oligonucleotide. It is understood that an oligonucleotide need not be 100% complementary to its target nucleic acid sequence to be specifically hybridizable. An oligonucleotide is specifically hybridizable when binding of the oligonucleotide to the target interferes with the normal function of the target molecule to cause a loss of utility or expression therefrom, and there is a sufficient degree of complementarity to avoid non-specific binding of the oligonucleotide to non-target sequences under conditions in which specific binding is desired, i.e., under physiological conditions in the case of in vivo assays or therapeutic treatment, or, in the case of in vitro assays, under conditions in which the assays are conducted. Thus, in other embodiments, this oligonucleotide includes 1, 2, or 3 base substitutions, e.g. mismatches, as compared to the region of a gene or mRNA sequence that it is targeting or to which it specifically hybridizes.

### RNA Interference Nucleic Acids

In particular embodiments, nucleic acid-lipid nanoparticles are associated with RNA interference (RNAi) molecules. RNA interference methods using RNAi molecules may be used to disrupt the expression of a gene or polynucleotide of interest. Small interfering RNA (siRNA) has essentially replaced antisense ODN and ribozymes as the next generation of targeted oligonucleotide drugs under development.

SiRNAs are RNA duplexes normally 16-30 nucleotides long that can associate with a cytoplasmic multi-protein complex known as RNAi-induced silencing complex (RISC). RISC loaded with siRNA mediates the degradation of homologous mRNA transcripts, therefore siRNA can be designed to knock down protein expression with high specificity. Unlike other antisense technologies, siRNA function through a natural mechanism evolved to control gene expression through non-coding RNA. This is generally considered to be the reason why their activity is more potent in vitro and in vivo than either antisense ODN or ribozymes. A variety of RNAi reagents, including siRNAs targeting clinically relevant targets, are currently under pharmaceutical development, as described, e.g., in de Fougerolles, A. et al., Nature Reviews 6:443-453 (2007).

While the first described RNAi molecules were RNA:RNA hybrids comprising both an RNA sense and an RNA antisense strand, it has now been demonstrated that DNA sense:RNA antisense hybrids, RNA sense:DNA antisense hybrids, and DNA:DNA hybrids are capable of mediating RNAi (Lamberton, J.S. and Christian, A.T., (2003) Molecular Biotechnology24: 111-119). Thus, the use of RNAi molecules comprising any of these different types of double-stranded molecules is contemplated. In addition, it is understood that RNAi molecules may be used and introduced to cells in a variety of forms. Accordingly, as used herein, RNAi molecules encompasses any and all molecules capable of inducing an RNAi response in cells, including, but not limited to, double-stranded oligonucleotides comprising two separate strands, i.e. a sense strand and an antisense strand, e.g., small interfering RNA (siRNA); double-stranded oligonucleotide comprising two separate strands that are linked together by non-nucleotidyl linker; oligonucleotides comprising a hairpin loop of complementary sequences, which forms a double-stranded region, e.g., shRNAi molecules, and expression vectors that express one or more polynucleotides capable of forming a double-stranded polynucleotide alone or in combination with another polynucleotide.

A "single strand siRNA compound" as used herein, is an siRNA compound which is made up of a single molecule. It may include a duplexed region, formed by intra-strand pairing, e.g., it may be, or include, a hairpin or pan-handle structure. Single strand siRNA compounds may be antisense with regard to the target molecule

A single strand siRNA compound may be sufficiently long that it can enter the RISC and participate in RISC mediated cleavage of a target mRNA. A single strand siRNA compound is at least 14, and in other embodiments at least 15, 20, 25, 29, 35, 40, or 50 nucleotides in length. In certain embodiments, it is less than 200, 100, or 60 nucleotides in length.

Hairpin siRNA compounds will have a duplex region equal to or at least 17, 18, 19, 29, 21, 22, 23, 24, or 25 nucleotide pairs. The duplex region will may be equal to or less than 200, 100, or 50, in length. In certain embodiments, ranges for the duplex region are 15-30, 17 to 23, 19 to 23, and 19 to 2 1 nucleotides pairs in length. The hairpin may have a single strand overhang or terminal unpaired region. In certain embodiments, the overhangs are 2-3 nucleotides in length. In some embodiments, the overhang is at the sense side of the hairpin and in some embodiments on the antisense side of the hairpin.

A "double stranded siRNA compound" as used herein, is an siRNA compound which includes more than one, and in some cases two, strands in which interchain hybridization can form a region of duplex structure.

The antisense strand of a double stranded siRNA compound may be equal to or at least, 14, 15, 16 17, 18, 19, 25, 29, 40, or 60 nucleotides in length. It may be equal to or less than 200, 100, or 50, nucleotides in length. Ranges may be 17 to 25, 19 to 23, and 19 to21 nucleotides in length. As used herein, term "antisense strand" means the strand of an siRNA compound that is sufficiently complementary to a target molecule, e.g. a target RNA.

The sense strand of a double stranded siRNA compound may be equal to or at least 14, 15, 16 17, 18, 19, 25, 29, 40, or 60 nucleotides in length. It may be equal to or less than 200, 100, or 50, nucleotides in length. Ranges may be 17 to 25, 19 to 23, and 19 to 2 1 nucleotides in length.

The double strand portion of a double stranded siRNA compound may be equal to or at least, 14, 15, 16 17, 18, 19, 20, 21, 22, 23, 24, 25, 29, 40, or 60 nucleotide pairs in length. It may be equal to or less than 200, 100, or 50, nucleotides pairs in length. Ranges may be 15-30, 17 to 23, 19 to 23, and 19 to 2 1 nucleotides pairs in length.

In many embodiments, the siRNA compound is sufficiently large that it can be cleaved by an endogenous molecule, e.g., by Dicer, to produce smaller siRNA compounds, e.g., siRNAs agents.

The sense and antisense strands may be chosen such that the double-stranded siRNA compound includes a single strand or unpaired region at one or both ends of the molecule. Thus, a double-stranded siRNA compound may contain sense and antisense strands, paired to contain an overhang, e.g., one or two 5' or 3' overhangs, or a 3' overhang of 1 - 3 nucleotides. The overhangs can be the result of one strand being longer than the other, or the result of two strands of the same length being staggered. Some embodiments will have at least one 3' overhang. In one embodiment, both ends of an siRNA molecule will have a 3' overhang. In some embodiments, the overhang is 2 nucleotides.

In certain embodiments, the length for the duplexed region is between 15 and 30, or 18, 19, 20, 21, 22, and 23 nucleotides in length, e.g., in the ssiRNA compound range discussed above. ssiRNA compounds can resemble in length and structure the natural Dicer processed products from long dsiRNAs. Embodiments in which the two strands of the ssiRNA compound are linked, e.g., covalently linked are also included. Hairpin, or other single strand structures which provide the required double stranded region, and a 3' overhang are also contemplated.

The siRNA compounds described herein, including double-stranded siRNA compounds and single-stranded siRNA compounds can mediate silencing of a target RNA, e.g., mRNA, e.g., a transcript of a gene that encodes a protein. For convenience, such mRNA is also referred to herein as mRNA to be silenced. Such a gene is also referred to as a target gene. In general, the RNA to be silenced is an endogenous gene or a pathogen gene. In addition, RNAs other than mRNA, e.g., tRNAs, and viral RNAs, can also be targeted.

As used herein, the phrase "mediates RNAi" refers to the ability to silence, in a sequence specific manner, a target RNA. While not wishing to be bound by theory, it is believed that silencing uses the RNAi machinery or process and a guide RNA, e.g., an ssiRNA compound of 2 1 to 23 nucleotides.

In one embodiment, an siRNA compound is "sufficiently complementary" to a target RNA, e.g., a target mRNA, such that the siRNA compound silences production of protein encoded by the target mRNA. In another embodiment, the siRNA compound is "exactly complementary" to a target RNA, e.g., the target RNA and the siRNA compound anneal, for example to form a hybrid made exclusively of Watson-Crick base pairs in the region of exact complementarity. A "sufficiently complementary" target RNA can include an internal region (e.g., of at least 10 nucleotides) that is exactly complementary to a target RNA. Moreover, in certain embodiments, the siRNA compound specifically discriminates a single-nucleotide difference. In this case, the siRNA compound only mediates RNAi if exact complementary is found in the region (e.g., within 7 nucleotides of) the single-nucleotide difference.

### MicroRNAs

Micro RNAs (miRNAs) are a highly conserved class of small RNA molecules that are transcribed from DNA in the genomes of plants and animals, but are not translated into protein. Processed miRNAs are single stranded -17-25 nucleotide (nt) RNA molecules that become incorporated into the RNA-induced silencing complex (RISC) and have been identified as key regulators of development, cell proliferation, apoptosis and differentiation. They are believed to play a role in regulation of gene expression by binding to the 3'-untranslated region of specific mRNAs. RISC mediates down-regulation of gene expression through translational inhibition, transcript cleavage, or both. RISC is also implicated in transcriptional silencing in the nucleus of a wide range of eukaryotes.

### Antisense Oligonucleotides

In one embodiment, a nucleic acid is an antisense oligonucleotide directed to a target polynucleotide. The term "antisense oligonucleotide" or simply "antisense" is meant to include oligonucleotides that are complementary to a targeted polynucleotide sequence. Antisense oligonucleotides are single strands of DNA or RNA that are complementary to a chosen sequence, e.g. a target gene mRNA. Antisense oligonucleotides are thought to inhibit gene expression by binding to a complementary mRNA. Binding to the target mRNA can lead to inhibition of gene expression either by preventing translation of complementary mRNA strands by binding to it, or by leading to degradation of the target mRNA. Antisense DNA can be used to target a specific, complementary (coding or non-coding) RNA. If binding takes places this DNA/RNA hybrid can be degraded by the enzyme RNase H. In particular embodiments, antisense oligonucleotides contain from about 10 to about 50 nucleotides, more preferably about 15 to about 30 nucleotides. The term also encompasses antisense oligonucleotides that may not be exactly complementary to the desired target gene. Thus, instances where non-target specific-activities are found with antisense, or where an antisense sequence containing one or more mismatches with the target sequence is the most preferred for a particular use, are contemplated.

Antisense oligonucleotides have been demonstrated to be effective and targeted inhibitors of protein synthesis, and, consequently, can be used to specifically inhibit protein synthesis by a targeted gene. The efficacy of antisense oligonucleotides for inhibiting protein synthesis is well established. For example, the synthesis of polygalactauronase and the muscarine type 2 acetylcholine receptor are inhibited by antisense oligonucleotides directed to their respective mRNA sequences (U. S. Patent 5,739,119 and U. S. Patent 5,759,829). Further, examples of antisense inhibition have been demonstrated with the nuclear protein cyclin, the multiple drug resistance gene (MDG1), ICAM-1, E-selectin, STK-1, striatal GABA A receptor and human EGF (Jaskulski et al., Science. 1988 Jun 10;240(4858): 1544-6; Vasanthakumar and Ahmed, Cancer Commun. 1989;1(4):225-32; Peris et al, Brain Res Mol Brain Res. 1998 Jun 15;57(2):310-20; U. S. Patent 5,801,154; U.S. Patent 5,789,573; U. S. Patent 5,718,709 and U.S. Patent 5,610,288). Furthermore, antisense constructs have also been described that inhibit and can be used to treat a variety of abnormal cellular proliferations, e.g. cancer (U. S. Patent 5,747,470; U. S. Patent 5,591,317 and U. S. Patent 5,783,683).

Methods of producing antisense oligonucleotides are known in the art and can be readily adapted to produce an antisense oligonucleotide that targets any polynucleotide sequence. Selection of antisense oligonucleotide sequences specific for a given target sequence is based upon analysis of the chosen target sequence and determination of secondary structure, Tₘ, binding energy, and relative stability. Antisense oligonucleotides may be selected based upon their relative inability to form dimers, hairpins, or other secondary structures that would reduce or prohibit specific binding to the target mRNA in a host cell. Highly preferred target regions of the niRNA include those regions at or near the AUG translation initiation codon and those sequences that are substantially complementary to 5' regions of the mRNA. These secondary structure analyses and target site selection considerations can be performed, for example, using v.4 of the OLIGO primer analysis software (Molecular Biology Insights) and/or the BLASTN 2.0.5 algorithm software (Altschul et al, Nucleic Acids Res. 1997, 25(17):3389-402).

### Antagomirs

Antagomirs are RNA-like oligonucleotides that harbor various modifications for RNAse protection and pharmacologic properties, such as enhanced tissue and cellular uptake. They differ from normal RNA by, for example, complete 2'-O-methylation of sugar, phosphorothioate backbone and, for example, a cholesterol-moiety at 3'-end. Antagomirs may be used to efficiently silence endogenous miRNAs by forming duplexes comprising the antagomir and endogenous miRNA, thereby preventing miRNA-induced gene silencing. An example of antagomir-mediated miRNA silencing is the silencing of miR-122, described in Krutzfeldt et al, Nature, 2005, 438: 685-689. Antagomir RNAs may be synthesized using standard solid phase oligonucleotide synthesis protocols. See U.S. Patent Application Publication Nos. 2007/0123482 and 2007/0213292.

An antagomir can include ligand-conjugated monomer subunits and monomers for oligonucleotide synthesis. Exemplary monomers are described in U.S. Patent Application Publication No. 2005/0107325. An antagomir can have a ZXY structure, such as is described in WO 2004/080406. An antagomir can be complexed with an amphipathic moiety. Exemplary amphipathic moieties for use with oligonucleotide agents are described in WO 2004/080406.

### Aptamers

Aptamers are nucleic acid or peptide molecules that bind to a particular molecule of interest with high affinity and specificity (Tuerk and Gold, Science 249:505 (1990); Ellington and Szostak, Nature 346:818 (1990). DNA or RNA aptamers have been successfully produced which bind many different entities from large proteins to small organic molecules. See Eaton, Curr. Opin. Chem. Biol. 1:10-16 (1997), Famulok, Curr. Opin. Struct. Biol. 9:324-9(1999), and Hermann and Patel, Science 287:820-5 (2000). Aptamers may be RNA or DNA based, and may include a riboswitch. A riboswitch is a part of an mRNA molecule that can directly bind a small target molecule, and whose binding of the target affects the gene's activity. Thus, an mRNA that contains a riboswitch is directly involved in regulating its own activity, depending on the presence or absence of its target molecule. Generally, aptamers are engineered through repeated rounds of in vitro selection or equivalently, SELEX (systematic evolution of ligands by exponential enrichment) to bind to various molecular targets such as small molecules, proteins, nucleic acids, and even cells, tissues and organisms. The aptamer may be prepared by any known method, including synthetic, recombinant, and purification methods, and may be used alone or in combination with other aptamers specific for the same target.

Further, as described more fully herein, the term "aptamer" specifically includes "secondary aptamers" containing a consensus sequence derived from comparing two or more known aptamers to a given target.

### Ribozymes

According to another embodiment, nucleic acid-lipid nanoparticles are associated with ribozymes. Ribozymes are RNA molecules complexes having specific catalytic domains that possess endonuclease activity (Kim and Cech, Proc Natl Acad Sci USA. 1987 Dec;84(24):8788-92; Forster and Symons, Cell. 1987 Apr 24;49(2):211-20). For example, a large number of ribozymes accelerate phosphoester transfer reactions with a high degree of specificity, often cleaving only one of several phosphoesters in an oligonucleotide substrate (Cech et al, Cell. 1981 Dec;27(3 Pt 2):487-96; Michel and Westhof, J Mol Biol. 1990 Dec 5;216(3):585-610; Reinhold-Hurek and Shub, Nature. 1992 May 14;357(6374): 173-6). This specificity has been attributed to the requirement that the substrate bind via specific base-pairing interactions to the internal guide sequence ("IGS") of the ribozyme prior to chemical reaction.

At least six basic varieties of naturally-occurring enzymatic RNAs are known presently. Each can catalyze the hydrolysis of RNA phosphodiester bonds in trans (and thus can cleave other RNA molecules) under physiological conditions. In general, enzymatic nucleic acids act by first binding to a target RNA. Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nucleic acid molecule may be formed in a hammerhead, hairpin, a hepatitis d virus, group I intron or RNaseP RNA (in association with an RNA guide sequence) or Neurospora VS RNA motif, for example. Specific examples of hammerhead motifs are described by Rossi et al. Nucleic Acids Res. 1992 Sep 11;20(17):4559-65. Examples of hairpin motifs are described by Hampel et al. (Eur. Pat. Appl. Publ. No. EP 0360257), Hampel and Tritz, Biochemistry 1989 Jun 13;28(12):4929-33; Hampel et al, Nucleic Acids Res. 1990 Jan 25;18(2):299-304 and U. S. Patent 5,631,359. An example of the hepatitis d virus motif is described by Perrotta and Been, Biochemistry. 1992 Dec 1;3 1(47): 1 1843-52; an example of the RNaseP motif is described by Guerrier-Takada et al, Cell. 1983 Dec;35(3 Pt 2):849-57; Neurospora VS RNA ribozyme motif is described by Collins (Saville and Collins, Cell. 1990 May 18;61(4):685-96; Saville and Collins, Proc Natl Acad Sci USA. 1991 Oct 1;88(19):8826-30; Collins and Olive, Biochemistry. 1993 Mar 23;32(ll):2795-9); and an example of the Group I intron is described in U. S. Patent 4,987,071. Important characteristics of enzymatic nucleic acid molecules used are that they have a specific substrate binding site which is complementary to one or more of the target gene DNA or RNA regions, and that they have nucleotide sequences within or surrounding that substrate binding site which impart an RNA cleaving activity to the molecule. Thus the ribozyme constructs need not be limited to specific motifs mentioned herein.

Methods of producing a ribozyme targeted to any polynucleotide sequence are known in the art. Ribozymes may be designed as described in Int. Pat. Appl. Publ. Nos. WO 93/23569 and WO 94/02595, and synthesized to be tested in vitro and in vivo, as described therein.

Ribozyme activity can be optimized by altering the length of the ribozyme binding arms or chemically synthesizing ribozymes with modifications that prevent their degradation by serum ribonucleases (see e.g., Int. Pat. Appl. Publ. Nos. WO 92/07065, WO 93/15187, and WO 91/03162; Eur. Pat. Appl. Publ. No. 92110298.4; U.S. Patent 5,334,711; and Int. Pat. Appl. Publ. No. WO 94/13688, which describe various chemical modifications that can be made to the sugar moieties of enzymatic RNA molecules), modifications which enhance their efficacy in cells, and removal of stem II bases to shorten RNA synthesis times and reduce chemical requirements.

### Immunostimulatory Oligonucleotides

Nucleic acids associated with lipid nanoparticles may be immunostimulatory, including immunostimulatory oligonucleotides (ISS; single-or double- stranded) capable of inducing an immune response when administered to a subject, which may be a mammal or other patient. ISS include, e.g., certain palindromes leading to hairpin secondary structures (see Yamamoto S., et al. (1992) J . Immunol. 148: 4072-4076), or CpG motifs, as well as other known ISS features (such as multi-G domains, see WO 96/1 1266).

The immune response may be an innate or an adaptive immune response. The immune system is divided into a more innate immune system, and acquired adaptive immune system of vertebrates, the latter of which is further divided into humoral cellular components. In particular embodiments, the immune response may be mucosal.

In particular embodiments, an immunostimulatory nucleic acid is only immunostimulatory when administered in combination with a lipid nanoparticle, and is not immunostimulatory when administered in its "free form." Such an oligonucleotide is considered to be immunostimulatory.

Immunostimulatory nucleic acids are considered to be non-sequence specific when it is not required that they specifically bind to and reduce the expression of a target polynucleotide in order to provoke an immune response. Thus, certain immunostimulatory nucleic acids may comprise a sequence corresponding to a region of a naturally occurring gene or mRNA, but they may still be considered non-sequence specific immunostimulatory nucleic acids.

In one embodiment, the immunostimulatory nucleic acid or oligonucleotide comprises at least one CpG dinucleotide. The oligonucleotide or CpG dinucleotide may be unmethylated or methylated. In another embodiment, the immunostimulatory nucleic acid comprises at least one CpG dinucleotide having a methylated cytosine. In one embodiment, the nucleic acid comprises a single CpG dinucleotide, wherein the cytosine in said CpG dinucleotide is methylated. In a specific embodiment, the nucleic acid comprises the sequence 5' TAACGTTGAGGGGCAT 3'. In an alternative embodiment, the nucleic acid comprises at least two CpG dinucleotides, wherein at least one cytosine in the CpG dinucleotides is methylated. In a further embodiment, each cytosine in the CpG dinucleotides present in the sequence is methylated. In another embodiment, the nucleic acid comprises a plurality of CpG dinucleotides, wherein at least one of said CpG dinucleotides comprises a methylated cytosine.

### Decoy Oligonucleotides

Because transcription factors recognize their relatively short binding sequences, even in the absence of surrounding genomic DNA, short oligonucleotides bearing the consensus binding sequence of a specific transcription factor can be used as tools for manipulating gene expression in living cells. This strategy involves the intracellular delivery of such "decoy oligonucleotides", which are then recognized and bound by the target factor. Occupation of the transcription factor's DNA-binding site by the decoy renders the transcription factor incapable of subsequently binding to the promoter regions of target genes. Decoys can be used as therapeutic agents, either to inhibit the expression of genes that are activated by a transcription factor, or to upregulate genes that are suppressed by the binding of a transcription factor. Examples of the utilization of decoy oligonucleotides may be found in Mann et al., J . Clin. Invest., 2000, 106: 1071-1075.

### Supermir

A supermir refers to a single stranded, double stranded or partially double stranded oligomer or polymer of ribonucleic acid (RNA) or deoxyribonucleic acid (DNA) or both or modifications thereof, which has a nucleotide sequence that is substantially identical to an miRNA and that is antisense with respect to its target. This term includes oligonucleotides composed of naturally-occurring nucleobases, sugars and covalent internucleoside (backbone) linkages and which contain at least one non-naturally-occurring portion which functions similarly. Such modified or substituted oligonucleotides are preferred over native forms because of desirable properties such as, for example, enhanced cellular uptake, enhanced affinity for nucleic acid target and increased stability in the presence of nucleases. In a preferred embodiment, the supermir does not include a sense strand, and in another preferred embodiment, the supermir does not self-hybridize to a significant extent. A supermir can have secondary structure, but it is substantially single-stranded under physiological conditions. An supermir that is substantially single-stranded is single-stranded to the extent that less than about 50% (e.g., less than about 40%, 30%, 20%, 10%, or 5%) of the supermir is duplexed with itself. The supermir can include a hairpin segment, e.g., sequence, preferably at the 3' end can self hybridize and form a duplex region, e.g., a duplex region of at least 1, 2, 3, or 4 and preferably less than 8, 7, 6, or n nucleotides, e.g., 5 nucleotides. The duplexed region can be connected by a linker, e.g., a nucleotide linker, e.g., 3, 4, 5, or 6 dTs, e.g., modified dTs. In another embodiment the supermir is duplexed with a shorter oligo, e.g., of 5, 6, 7, 8, 9, or 10 nucleotides in length, e.g., at one or both of the 3' and 5' end or at one end and in the non-terminal or middle of the supermir.

### miRNA mimics

miRNA mimics represent a class of molecules that can be used to imitate the gene silencing ability of one or more miRNAs. Thus, the term "microRNA mimic" refers to synthetic non-coding RNAs (i.e. the miRNA is not obtained by purification from a source of the endogenous miRNA) that are capable of entering the RNAi pathway and regulating gene expression. miRNA mimics can be designed as mature molecules (e.g. single stranded) or mimic precursors (e.g., pri- or pre-miRNAs). miRNA mimics can be comprised of nucleic acid (modified or modified nucleic acids) including oligonucleotides comprising, without limitation, RNA, modified RNA, DNA, modified DNA, locked nucleic acids, or 2'-O,4'-C-ethylene-bridged nucleic acids (ENA), or any combination of the above (including DNA-RNA hybrids). In addition, miRNA mimics can comprise conjugates that can affect delivery, intracellular compartmentalization, stability, specificity, functionality, strand usage, and/or potency. In one design, miRNA mimics are double stranded molecules (e.g., with a duplex region of between about 16 and about 3 1 nucleotides in length) and contain one or more sequences that have identity with the mature strand of a given miRNA. Modifications can comprise 2' modifications (including 2'-O methyl modifications and 2' F modifications) on one or both strands of the molecule and internucleotide modifications (e.g. phosphorothioate modifications) that enhance nucleic acid stability and/or specificity. In addition, miRNA mimics can include overhangs. The overhangs can consist of 1-6 nucleotides on either the 3' or 5' end of either strand and can be modified to enhance stability or functionality. In one embodiment, a miRNA mimic comprises a duplex region of between 16 and 3 1 nucleotides and one or more of the following chemical modification patterns: the sense strand contains 2'-O-methyl modifications of nucleotides 1 and 2 (counting from the 5' end of the sense oligonucleotide), and all of the Cs and Us; the antisense strand modifications can comprise 2' F modification of all of the Cs and Us, phosphorylation of the 5' end of the oligonucleotide, and stabilized internucleotide linkages associated with a 2 nucleotide 3' overhang.

### Antimir or miRNA inhibitor

The terms "antimir," "microRNA inhibitor," "miR inhibitor," or "inhibitor," are synonymous and refer to oligonucleotides or modified oligonucleotides that interfere with the ability of specific miRNAs. In general, the inhibitors are nucleic acid or modified nucleic acids in nature including oligonucleotides comprising RNA, modified RNA, DNA, modified DNA, locked nucleic acids (LNAs), or any combination of the above. Modifications include 2' modifications (including 2'-O alkyl modifications and 2' F modifications) and internucleotide modifications (e.g. phosphorothioate modifications) that can affect delivery, stability, specificity, intracellular compartmentalization, or potency. In addition, miRNA inhibitors can comprise conjugates that can affect delivery, intracellular compartmentalization, stability, and/or potency. Inhibitors can adopt a variety of configurations including single stranded, double stranded (RNA/RNA or RNA/DNA duplexes), and hairpin designs, in general, microRNA inhibitors comprise contain one or more sequences or portions of sequences that are complementary or partially complementary with the mature strand (or strands) of the miRNA to be targeted, in addition, the miRNA inhibitor may also comprise additional sequences located 5' and 3' to the sequence that is the reverse complement of the mature miRNA. The additional sequences may be the reverse complements of the sequences that are adjacent to the mature miRNA in the pri-miRNA from which the mature miRNA is derived, or the additional sequences may be arbitrary sequences (having a mixture of A, G, C, or U). In some embodiments, one or both of the additional sequences are arbitrary sequences capable of forming hairpins. Thus, in some embodiments, the sequence that is the reverse complement of the miRNA is flanked on the 5' side and on the 3' side by hairpin structures. Micro-RNA inhibitors, when double stranded, may include mismatches between nucleotides on opposite strands. Furthermore, micro-RNA inhibitors may be linked to conjugate moieties in order to facilitate uptake of the inhibitor into a cell. For example, a micro-RNA inhibitor may be linked to cholesteryl 5-(bis(4-methoxyphenyl)(phenyl)methoxy)-3 hydroxypentylcarbamate) which allows passive uptake of a micro-RNA inhibitor into a cell. Micro-RNA inhibitors, including hairpin miRNA inhibitors, are described in detail in Vermeulen et al., "Double-Stranded Regions Are Essential Design Components Of Potent Inhibitors of RISC Function," RNA 13: 723-730 (2007) and in WO2007/095387 and WO 2008/036825. A person of ordinary skill in the art can select a sequence from the database for a desired miRNA and design an inhibitor useful for the methods disclosed herein.

### Ul adaptor

Ul adaptor inhibit polyA sites and are bifunctional oligonucleotides with a target domain complementary to a site in the target gene's terminal exon and a 'Ul domain' that binds to the Ul smaller nuclear RNA component of the Ul snRNP (Goraczniak, et al., 2008, Nature Biotechnology, 27(3), 257-263). Ul snRNP is a ribonucleoprotein complex that functions primarily to direct early steps in spliceosome formation by binding to the pre-mRNA exon- intron boundary (Brown and Simpson, 1998, Annu Rev Plant Physiol Plant Mol Biol 49:77-95). Nucleotides 2-11 of the 5'end of Ul snRNA base pair bind with the 5'ss of the pre mRNA. In one embodiment, oligonucleotides are Ul adaptors. In one embodiment, the Ul adaptor can be administered in combination with at least one other iRNA agent.

### Pharmaceutical Compositions

In other different embodiments, the invention is directed to a method for administering a therapeutic agent to a patient in need thereof, the method comprising preparing or providing any of the foregoing LNPs and/or administering a composition comprising the same to the patient. In some embodiments, the therapeutic agent is effective to treat the disease.

For the purposes of administration, the lipid nanoparticles of embodiments of the present invention may be administered alone or may be formulated as pharmaceutical compositions. Pharmaceutical compositions of certain embodiments comprise a lipid nanoparticle according to any of the foregoing embodiments and one or more pharmaceutically acceptable carrier, diluent or excipient. The lipid nanoparticle may be present in an amount which is effective to deliver the therapeutic agent, e.g., for treating a particular disease or condition of interest. Appropriate concentrations and dosages can be readily determined by one skilled in the art.

Administration of the lipid nanoparticles of some embodiments can be carried out via any of the accepted modes of administration of agents for serving similar utilities. The pharmaceutical compositions of some embodiments may be formulated into preparations in solid, semi-solid, liquid or gaseous forms, such as tablets, capsules, powders, granules, ointments, solutions, suspensions, suppositories, injections, inhalants, gels, microspheres, and aerosols. Typical routes of administering such pharmaceutical compositions include, without limitation, oral, topical, transdermal, inhalation, parenteral, sublingual, buccal, rectal, vaginal, and intranasal. The term parenteral as used herein includes subcutaneous injections, intravenous, intramuscular, intradermal, intrasternal injection or infusion techniques. Pharmaceutical compositions of some embodiments are formulated so as to allow the active ingredients contained therein to be bioavailable upon administration of the composition to a patient. Compositions that may be administered to a subject or patient may take the form of one or more dosage units, where for example, a tablet may be a single dosage unit, and a container comprising LNPs in aerosol form may hold a plurality of dosage units. Actual methods of preparing such dosage forms are known, or will be apparent, to those skilled in this art; for example, see Remington: The Science and Practice of Pharmacy, 20th Edition (Philadelphia College of Pharmacy and Science, 2000). The composition to be administered will typically contain a therapeutically effective amount of a lipid nanoparticle of any of the embodiments disclosed herein, comprising a therapeutic agent, or a pharmaceutically acceptable salt thereof, for treatment of a disease or condition of interest.

A pharmaceutical composition of some embodiments may be in the form of a solid or liquid. In one aspect, the carrier(s) are particulate, so that the compositions are, for example, in tablet or powder form. The carrier(s) may be liquid, with the compositions being, for example, an oral syrup, injectable liquid or an aerosol, which is useful in, for example, inhalatory administration.

When intended for oral administration, the pharmaceutical composition is preferably in either solid or liquid form, where semi-solid, semi-liquid, suspension and gel forms are included within the forms considered herein as either solid or liquid.

As a solid composition for oral administration, the pharmaceutical composition may be formulated into a powder, granule, compressed tablet, pill, capsule, chewing gum, wafer or the like form. Such a solid composition will typically contain one or more inert diluents or edible carriers. In addition, one or more of the following may be present: binders such as carboxymethylcellulose, ethyl cellulose, microcrystalline cellulose, gum tragacanth or gelatin; excipients such as starch, lactose or dextrins, disintegrating agents such as alginic acid, sodium alginate, Primogel, corn starch and the like; lubricants such as magnesium stearate or Sterotex; glidants such as colloidal silicon dioxide; sweetening agents such as sucrose or saccharin; a flavoring agent such as peppermint, methyl salicylate or orange flavoring; and a coloring agent.

When the pharmaceutical composition is in the form of a capsule, for example, a gelatin capsule, it may contain, in addition to materials of the above type, a liquid carrier such as polyethylene glycol or oil.

The pharmaceutical composition may be in the form of a liquid, for example, an elixir, syrup, solution, emulsion or suspension. The liquid may be for oral administration or for delivery by injection, as two examples. When intended for oral administration, preferred composition contain, in addition to the present compounds, one or more of a sweetening agent, preservatives, dye/colorant and flavor enhancer. In a composition intended to be administered by injection, one or more of a surfactant, preservative, wetting agent, dispersing agent, suspending agent, buffer, stabilizer and isotonic agent may be included.

The liquid pharmaceutical compositions of some embodiments, whether they be solutions, suspensions or other like form, may include one or more of the following adjuvants: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose; agents to act as cryoprotectants such as sucrose or trehalose. The parenteral preparation can be enclosed in ampoules, disposable syringes or multiple dose vials made of glass or plastic. Physiological saline is a preferred adjuvant. An injectable pharmaceutical composition is preferably sterile.

A liquid pharmaceutical composition of certain embodiments intended for either parenteral or oral administration should contain an amount of a lipid nanoparticle of the invention such that a suitable dosage will be obtained.

The pharmaceutical composition of embodiments of the invention may be intended for topical administration, in which case the carrier may suitably comprise a solution, emulsion, ointment or gel base. The base, for example, may comprise one or more of the following: petrolatum, lanolin, polyethylene glycols, bee wax, mineral oil, diluents such as water and alcohol, and emulsifiers and stabilizers. Thickening agents may be present in a pharmaceutical composition for topical administration. If intended for transdermal administration, the composition may include a transdermal patch or iontophoresis device.

The pharmaceutical composition of some embodiments may be intended for rectal administration, in the form, for example, of a suppository, which will melt in the rectum and release the drug. The composition for rectal administration may contain an oleaginous base as a suitable nonirritating excipient. Such bases include, without limitation, lanolin, cocoa butter and polyethylene glycol.

The pharmaceutical composition of other embodiments may include various materials, which modify the physical form of a solid or liquid dosage unit. For example, the composition may include materials that form a coating shell around the active ingredients. The materials that form the coating shell are typically inert, and may be selected from, for example, sugar, shellac, and other enteric coating agents. Alternatively, the active ingredients may be encased in a gelatin capsule.

The pharmaceutical composition of embodiments in solid or liquid form may include an agent that binds to the LNP or therapeutic agent, and thereby assists in the delivery of the LNP or therapeutic agent. Suitable agents that may act in this capacity include a monoclonal or polyclonal antibody, or a protein.

In other embodiments, the pharmaceutical composition may comprise or consist of dosage units that can be administered as an aerosol. The term aerosol is used to denote a variety of systems ranging from those of colloidal nature to systems consisting of pressurized packages. Delivery may be by a liquefied or compressed gas or by a suitable pump system that dispenses the active ingredients. Aerosols of compounds of the invention may be delivered in single phase, bi-phasic, or tri-phasic systems in order to deliver the active ingredient(s). Delivery of the aerosol includes the necessary container, activators, valves, subcontainers, and the like, which together may form a kit. One skilled in the art, without undue experimentation may determine preferred aerosols.

In some embodiments, the pharmaceutical compositions may be prepared by methodology well known in the pharmaceutical art. For example, a pharmaceutical composition intended to be administered by injection can be prepared by combining the lipid nanoparticles of the invention with sterile, distilled water or other carrier so as to form a solution. A surfactant may be added to facilitate the formation of a homogeneous solution or suspension. Surfactants are compounds that non-covalently interact with the compound of the invention so as to facilitate dissolution or homogeneous suspension of the compound in the aqueous delivery system.

The pharmaceutical compositions of some embodiments are administered in a therapeutically effective amount, which will vary depending upon a variety of factors including the activity of the specific therapeutic agent employed; the metabolic stability and length of action of the therapeutic agent; the age, body weight, general health, sex, and diet of the patient; the mode and time of administration; the rate of excretion; the drug combination; the severity of the particular disorder or condition; and the subject undergoing therapy.

The pharmaceutical compositions of various embodiments may also be administered simultaneously with, prior to, or after administration of one or more other therapeutic agents. Such combination therapy includes administration of a single pharmaceutical dosage formulation of a composition of the invention and one or more additional active agents, as well as administration of the composition of the invention and each active agent in its own separate pharmaceutical dosage formulation. For example, a pharmaceutical composition of one embodiments and the other active agent can be administered to the patient together in a single oral dosage composition such as a tablet or capsule, or each agent administered in separate oral dosage formulations. Where separate dosage formulations are used, the compounds of the invention and one or more additional active agents can be administered at essentially the same time, i.e., concurrently, or at separately staggered times, i.e., sequentially; combination therapy is understood to include all these regimens.

The following examples are provided for purpose of illustration and not limitation.

### EXAMPLES

### EXAMPLE 1

### PREPARATION OF LIPID NANOPARTICLE COMPOSITIONS

Cationic lipids and polymer conjugated lipids (PEG-lipid) were prepared and tested according to the general procedures described in PCT Pub. Nos. WO 2020/061426, WO 2015/199952, WO 2017/004143, WO 2017/075531 and WO 2017/117528, or were prepared as described herein. LNPs were prepared according to the following exemplary procedure.

The indicated cationic lipid (e.g. III-45), DSPC, cholesterol and PEG-lipid were solubilized in ethanol at the indicated molar ratio e.g. 47.5:10:40.7:1.8. Lipid nanoparticles (LNP) were prepared at a total lipid to mRNA weight ratio of approximately 10:1 to 30:1. Briefly, the mRNA was diluted to 0.2 mg/mL in 10 to 50 mM citrate or acetate buffer, pH 4 to pH 6. Syringe pumps or piston pumps were used to mix the ethanolic lipid solution with the mRNA aqueous solution at a ratio of about 1:5 to 1:3 (vol/vol) with total flow rates above 15 ml/min, for example 20 ml/min to 40 ml/min or above 100 ml/min or about 500 ml/min or about 1 000 ml/min. The ethanol was then removed and the external buffer replaced with PBS by dialysis. Finally, the lipid nanoparticles were filtered through a 0.2 µm pore sterile filter. Lipid nanoparticle particle size was approximately 45-105 nm, 55-95 nm diameter, 50-65 nm, 65-80 nm and in some instances approximately 70-90 nm diameter as determined by quasi-elastic light scattering using a Malvern Zetasizer Nano ZS (Malvern, UK) (not according to the invention unless embraced by the claims).

### EXAMPLE 2

### LUCIFERASE MRNA IN VIVO EVALUATION USING THE

### LIPID NANOPARTICLE COMPOSITIONS

Luciferase mRNA *in vivo* evaluation studies are performed in 6-8 week old female C57BL/6 mice (Charles River) 8-10 week old CD-1 (Harlan) mice (Charles River) according to guidelines established by an institutional animal care committee (ACC) and the Canadian Council on Animal Care (CCAC). Varying doses of mRNA-lipid nanoparticle are systemically administered by tail vein injection and animals euthanized at a specific time point (e.g., 4 hours) post-administration. Liver and spleen are collected in pre-weighed tubes, weights determined, immediately snap frozen in liquid nitrogen and stored at -80 °C until processing for analysis.

For liver, approximately 50 mg is dissected for analyses in a 2 mL FastPrep tubes (MP Biomedicals, Solon OH). ¼" ceramic sphere (MP Biomedicals) is added to each tube and 500 µL of Glo Lysis Buffer - GLB (Promega, Madison WI) equilibrated to room temperature is added to liver tissue. Liver tissues are homogenized with the FastPrep24 instrument (MP Biomedicals) at 2 × 6.0 m/s for 15 seconds. Homogenate is incubated at room temperature for 5 minutes prior to a 1:4 dilution in GLB and assessed using SteadyGlo Luciferase assay system (Promega). Specifically, 50 µL of diluted tissue homogenate is reacted with 50 µL of SteadyGlo substrate, shaken for 10 seconds followed by 5 minute incubation and then quantitated using a CentroXS³ LB 960 luminometer (Berthold Technologies, Germany). The amount of protein assayed is determined by using the BCA protein assay kit (Pierce, Rockford IL). Relative luminescence units (RLU) are then normalized to total µg protein assayed. To convert RLU to ng luciferase a standard curve is generated with QuantiLum Recombinant Luciferase (Promega). For a representative formulation, a four-hour time point is chosen for an efficacy evaluation of the lipid formulation.

The FLuc mRNA (e.g L-6107 or L-7202 from Trilink Biotechnologies) will express a luciferase protein, originally isolated from the firefly, *photimus pyralis.* FLuc is commonly used in mammalian cell culture to measure both gene expression and cell viability. It emits bioluminescence in the presence of the substrate, luciferin. This capped and polyadenylated mRNA is fully substituted with 5-methylcytidine and pseudouridine (L-6107) or 5-methoxyuridine (L-7202).

### EXAMPLE 3

### LUCIFERASE EXPRESSION IN MICE

Expression of the exogenous protein, luciferase in a murine small animal model was evaluated as a function of the acyl chain length of the PEG lipid component of the liquid nanoparticle (LNP) formulation.

Briefly, mice were given a tail vein injection of lipid nanoparticle formulation comprising either 1.5% or 2.5% PEG polymer lipid and containing an mRNA expression vector for the luciferase enzyme. The molar ratios of the cationic lipid (compound I-6), DSPC, Cholesterol and pegylated lipid were 50:10:38.5:1.5 and 50:10:37.5:2.5, respectively. LNPs were formulated according to standard methods as described herein in Example 1, using PEG lipids with varied lengths of their acyl chains; namely di-C12, di-C13, di-C14, C12/14 (asymmetric tail combination) and di-C15. Animals were dosed at 0.3 or 0.5 mg/kg RNA and quantitation of luciferase expression in the liver was accomplished using standard methods known to those of ordinary skill in the art or as described herein in Example 2.

The pegylated lipid used in the studies described herein was a compound having the following structure: 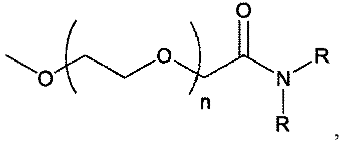 wherein n is an integer of about 45 such that the PEG portion has a molecular weight of about 2,000 g/mol, and each R is a saturated alkyl having from 8 to 16 carbon atoms, di-C12, di-C13, di-C14, di-C15, di-C16 and C12/14 refers to the above compound, wherein each R is straight-chain C12, each R is straight-chain C13, each R is straight-chain C14, each R is straight-chain C15, each R is straight-chain C16, or one R is straight-chain C12 and one R is straight-chain C14, respectively.

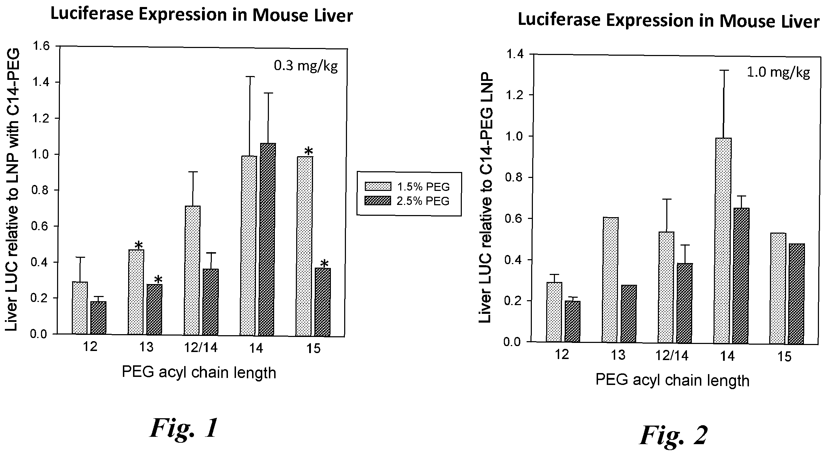

The luciferase expression data for the various LNP formulations presented in Figures 1 and 2 is shown as a ratio relative to the quantity observed for the LNP having a 1.5%, di-C14 PEG lipid formulation. Luciferase expression was highest for C14 length acyl chains and embodiments containing 2.5% PEG polymer lipid demonstrated reduced or equivalent expression of the enzyme.

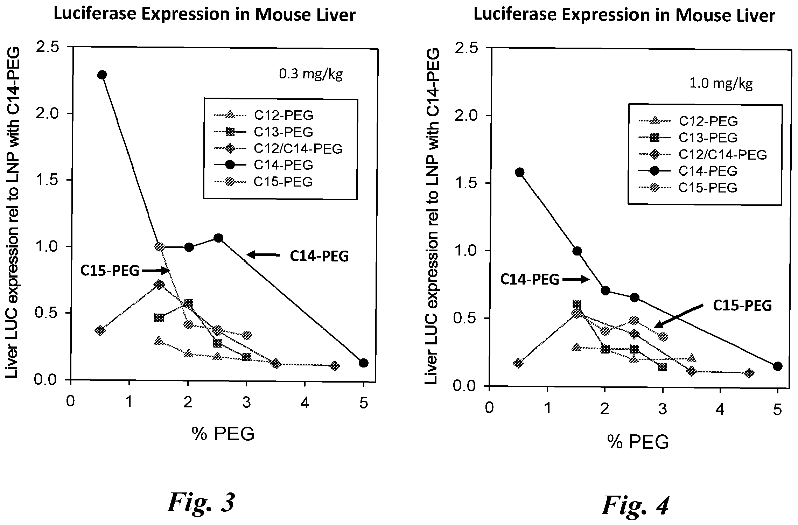

A related murine study investigated additional LNP embodiments wherein PEG lipid quantities were varied from 0.5 to 5.0%. The luciferase expression data for the various LNP formulations presented in Figures 3 and 4 is shown as a ratio relative to the quantity observed for the LNP having a 1.5%, di-C14 PEG lipid formulation. No significant benefit is observed for LNP embodiments wherein the PEG lipid quantity is greater than 1.5% and the trends in mice generally indicate improved performance for lower LNPs with lower pegylated lipid concentrations.

### EXAMPLE 4

### IN VIVO STUDY OF LIPID NANOPARTICLE FORMULATIONS IN NON-HUMAN PRIMATES

Experimentally naive male cynomolgus monkeys *(Macaca fascicularis,* macaque) were given control (saline) or test doses of LNP formulations via a 1-hour intravenous (IV) infusion in groups of three animals. The LNP formulation contained an expression vector for human immunoglobulin G, type 1 (IgG1). LNPs were synthesized according to standard methods known to those skilled in the art, or as described herein in Example 1, using cationic lipid III-45 and PEG lipids with varied lengths of their di-acyl chains; namely C12, C13, C14, C15 and C16 as described above. An additional LNP test group included a di-C14 formulation having smaller LNP diameter (~60nm). Non-control animals were dosed at 1.0 mg/kg RNA with a dose volume of 5 mL/kg. One control and seven test groups were used.

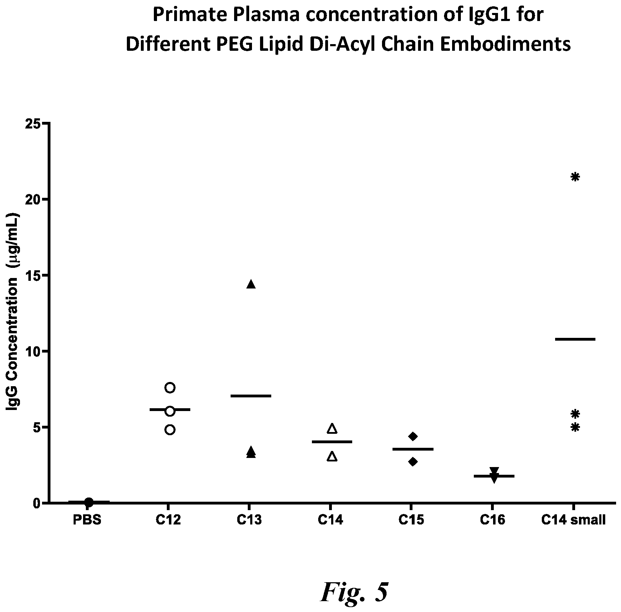

Pharmacodynamic samples to evaluate plasma concentrations of IgG1 were obtained by blood draw (K₃EDTA, 0.5 mL) prior to infusion; 3 and 9 hours post infusion and on days 2, 5, 8 and 15. Quantitation of expressed human IgG1 in blood plasma was accomplished using standard methods known to those of ordinary skill in the art. Figure 5 shows IgG1 plasma concentration levels determined on Day 2, demonstrating that IgG1 was expressed greatest for LNP embodiments with PEG lipids containing di-acyl chains shorter than C14.

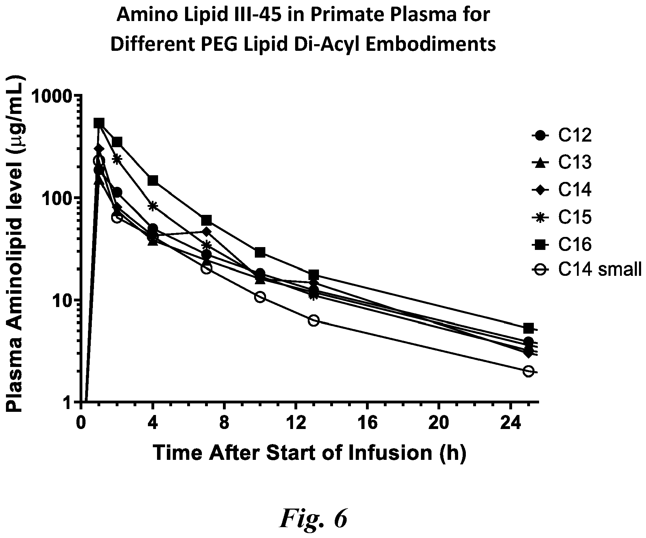

Plasma amino lipid levels were checked by blood draw (K₃EDTA, 1 mL) at the end of infusion (EOI) and at hours 1, 3, 6, 9, 12, 24 and 48 post EOI. Lower relative levels of amino lipids in the blood plasma are an indicator that the LNPs have cleared systemic circulation and accumulated in tissues of interest. Figure 6 plots the plasma concentration of compound III-45 as a function of time for certain LNP embodiments. Results of this analysis, shown as a maximum average concentration (Cmax, ug/mL), are given in Table 12 (control group not shown).

**Table 12: Amino lipid concentrations in blood plasma**

| **No.** | **Di-Acyl chain length** | **Particle Diameter** | **Cmax (ug/mL)** |
|---|---|---|---|
| 1 | C12 | 77 | 187 ± 87 |
| 2 | C13 | 68 | 152 ± 56 |
| 3 | C14 | 71 | 300 ± 57 |
| 4 | C15 | 77 | 532 ± 85 |
| 5 | C16 | 79 | 541 ± 26 |
| 6 | C14 (small) | 61 | 230 ± 28 |

In the present study, the lowest Cmax levels of amino lipids corresponded to LNPs having PEG lipids comprising shorter acyl chains (di-C12 and di-C 13). Without wishing to be bound by theory, applicants believe the specific lipophilic qualities imparted by the di-C12 and di-C13 acyl chains promote their distribution out of the LNP at a rate that enables delivery of the LNP to target tissues in a primate in a way that is not indicated by the analogous data in a murine model.

Additionally, comparison of the di-C14 embodiments of entries 3 and 6 demonstrates an increase in clearance for the embodiment with a smaller diameter LNP (60 nm, entry 6) which correlates with increased expression of protein. Typical LNP preparations have LNP diameters of approximately 70-80 nm as demonstrated for formulations 1-5 in Table 12 (not according to the invention unless embraced by the claims).

Again, without wishing to be bound by theory, applicant believes the smaller LNP size for the 60 nm C14 formulation affords more rapid clearance from the blood and into hepatocytes, relative to the standard di-C14 preparation, promoting delivery of the LNP to target tissues and resulting in greater expression. Consequently, a synergistic increase in delivery of LNPs may be realized by combining short di-acyl chain PEG lipids with LNP sizes of approximately 60 nm.

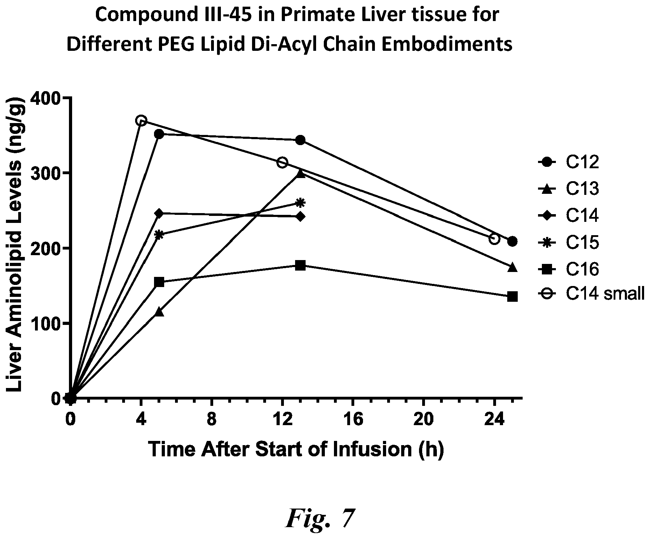

Liver amino lipid levels were checked by obtaining a liver sample via liver biopsy at 4, 12 and 24 hours post EOI. Higher relative levels of amino lipids in liver tissue is an indicator that the LNPs have accumulated in this tissue of interest. Figure 7 plots the liver tissue concentration of compound III-45 as a function of time for certain LNP embodiments. Results of this analysis, shown as a maximum average concentration (Cmax, ng/g) are given in Table 13 (control group not shown).

**Table 13: Amino lipid concentrations in liver tissue**

| **No.** | **Acyl chain length** | **Particle Diameter** | **Cmax (ug/mL)** |
|---|---|---|---|
| 1 | C12 | 77 | 352 |
| 2 | C13 | 68 | 300 |
| 3 | C14 | 71 | 246 |
| 4 | C15 | 77 | 260 |
| 5 | C16 | 79 | 177 |
| 6 | C14 (small) | 61 | 370 |

For LNPs comprising differences only from the length of the di-acyl chain (No 1-5 in Table 13), the highest Cmax levels of amino lipids observed in liver tissue corresponded to the embodiment having PEG lipids with a di-C12 acyl chain. Without wishing to be bound by theory, applicants believe the specific lipophilic qualities imparted by the shorter di-acyl chain promotes enhanced accumulation of the LNP in liver tissue of primates. This increased accumulation promotes increased relative expression of the encapsulated mRNA resulting in the higher IgG1 concentrations observed above (Figure 5).

Additionally, comparison of the di-C14 embodiments of entries 3 and 6 demonstrates a significant increase in liver amino lipid concentration for the embodiment with a smaller diameter LNP (60 nm, entry 6), again correlating with higher expression levels in the primate liver. Typical LNP preparations have LNP diameters of ~70-80 nm as demonstrated for formulations 1-5 shown in Table 13 (not acording to the invention unless embraced by the claims).

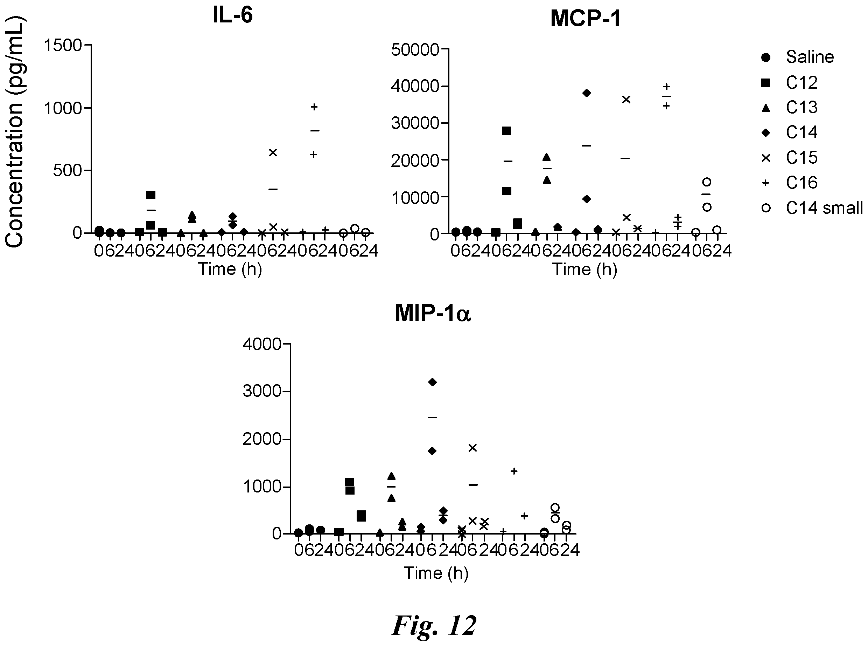

Further, plasma cytokine levels for the LNPs of Example 4 were determined as shown in Figure 12. Quantitation of cytokines in blood plasma was accomplished using standard methods known to those of ordinary skill in the art. Measurements were made pre-dose, EOI, and 6 and 24 hours post EOI. These data show lower peak induction (i.e. at 6 hours) of IL-6, MCP-1 and MIP-1a for the embodiment with a smaller diameter LNP (60 nm, entry 6) formulation compared to formulations 1-5 shown in Table 13 which have diameters of -70-80 nm.

### EXAMPLE 5

### IN SITU HYBRIDIZATION-LNP DELIVERY INTO HEPATOCYTES

Experimentally naive male cynomolgus monkeys (*Macaca fascicularis,* macaque) were given control (saline) or test doses of LNP formulations via a 1-hour intravenous (IV) infusion in groups of three animals. Liver biopsy samples were collected at 4 hours and 12 hours post end-of-infusion. The samples were flash frozen and stored until histological analysis could be performed. Additional details regarding experimental protocol for the NHP study are in Example 4.

Samples of macaque liver were sliced thin for histological analysis and *in situ* hybridization analysis was performed according to standard methods known to those skilled in the art.

RNA of the target sequence can be identified as darkened punctate spots within the hepatocytes and as broad regions of dark color within the sinusoidal space.

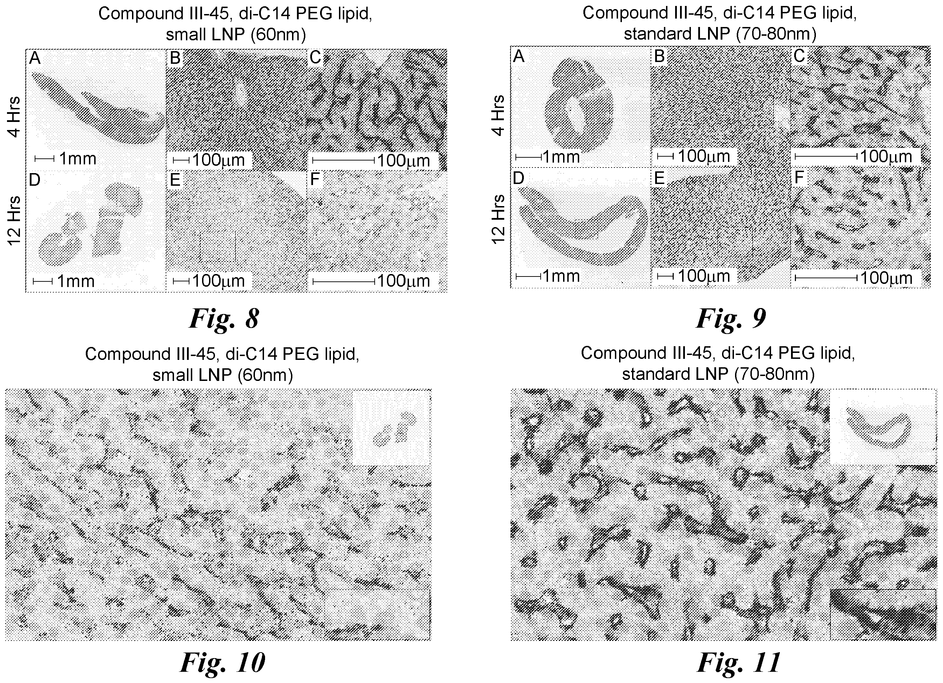

Figures 8 and 9 are provided to demonstrate the differential in distribution of LNP over time in the hepatocytes and sinusoidal space for different size LNP (60 nm vs. 70-80 nm) of the same composition. Both particles show significant distribution into hepatocytes at 4 hours as well as significant accumulation in the sinusoidal spaces. At 12 hours, both LNP show relatively little mRNA within hepatocytes, which is consistent with the timeframe for uptake, expression and natural degradation of the mRNA within the cells. However, the larger size LNP still shows relatively high signal in the sinusoidal spaces (Figure 9) whereas mRNA is relatively absent from the sinusoidal spaces for the small LNP at 12 hours (Figure 8). Without wishing to bound by theory, the higher expression of the smaller LNP is consistent with larger LNP being prevented from accessing hepatocytes to be productively expressed while smaller LNP cross the sinusoidal wall more readily for fast uptake into the hepatocytes.

Figures 10 and 11 provide an expanded view of the 12 hour tissue sample, better demonstrating the difference in LNP density in the sinusoidal space.

Without wishing to be bound by theory, Applicant believes the smaller diameter (60nm) lipid nanoparticles allow for increased uptake into hepatocytes, thus resulting in decreased incidence of the LNP in the sinusoidal space at the 12 hour time point. An increase of LNP uptake into hepatocytes promotes concomitant increases in expression of the delivered payload.

### EXAMPLE 6

### NON-HUMAN PRIMATE STUDY-ELEVATED QUANTITY OF POLYMER LIPID IN LNP

Experimentally naive male and female cynomolgus monkeys (*Macaca fascicularis,* macaque) are given control (saline) or test doses of LNP compositions via a 1-hour intravenous (IV) infusion in groups of three. The test LNP compositions are made up of five groups; four of these use a LNP formulation comprising a PEG lipid with a di-C12 acyl chain, with each group using a different proportion of said lipid (1.8%, 2.3%, 2.5% and 2.8% respectively). The fifth group uses a LNP formulation comprising a PEG lipid with a di-C13 acyl chain. All test LNP formulations contain an expression vector for human immunoglobulin G, type 1 (IgG1). LNPs were formulated according to standard methods as described herein in Example 1. Control subjects receive a 5 mL/kg saline injection. Non-control animals are nominally dosed at 1.0 mg/kg RNA with a dose volume of 5 mL/kg.

Pharmacodynamic samples to evaluate plasma concentrations of IgG1 are obtained by blood draw (K₃EDTA, 0.5 mL) prior to infusion; 6 hours post infusion and on days 2, 3, 5, 8 and 15.

Plasma amino lipid levels are checked by blood draw (K₃EDTA, 1 mL) at the end of infusion (EOI) and at hours 1, 3, 6, 9, 12, 24, 48 and 168 hours post EOI. Lower relative levels of amino lipids in the blood plasma are an indicator that the LNPs have accumulated in other regions of interest.

Liver amino lipid levels are checked by obtaining a liver sample via liver biopsy at 4 hours post EOI. Higher relative levels of amino lipids in liver issue is an indicator that the LNPs have accumulated in this regions of interest.

### EXAMPLE 7

### IN VIVO STUDY OF LIPID NANOPARTICLE FORMULATIONS IN NON-HUMAN PRIMATES

Experimentally naive male cynomolgus monkeys (*Macaca fascicularis,* macaque) were given control (saline) or test doses of LNP formulations via a 1-hour intravenous (IV) infusion in groups of four animals. The LNP formulation contained an mRNA expression vector for human immunoglobulin G, type 1 (IgG1). LNPs were synthesized according to standard methods known to those skilled in the art, or as described herein in Example 1, using cationic lipd III-45 and PEG lipid with C14 di-acyl chains as described above and size of 70 nm (LNP 8-1). Another LNP test group had the same composition but smaller LNP diameter of 52 nm (LNP 8-2). Non-control animals were dosed at 1.0 mg/kg RNA with a dose volume of 5 mL/kg.

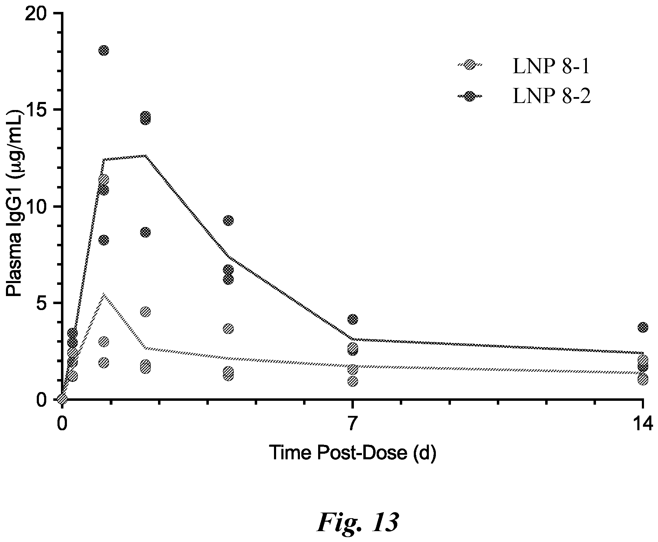

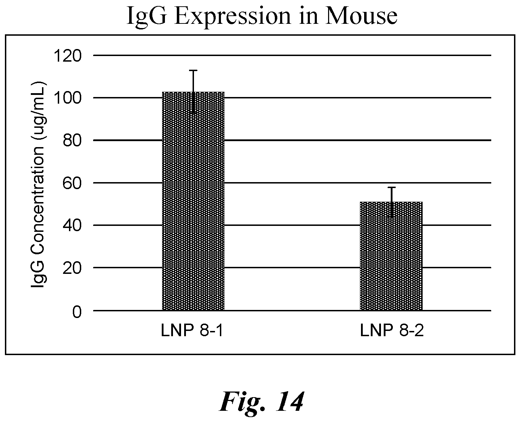

Pharmacodynamic samples to evaluate plasma concentrations of IgG1 were obtained by blood draw (K₃EDTA, 0.5 mL) prior to infusion; 6 hours post infusion and on days 1, 2, 4, 7 and 14. Quantitation of expressed human IgG1 in blood plasma was accomplished using standard methods known to those of ordinary skill in the art. Figure 13 shows IgG1 plasma concentration levels demonstrating that IgG1 was expressed greatest for LNP embodiments with size ~50 nm (LNP8-2) than size ~70 nm (LNP 8-1). The same preparations were administered in a murine model as described in Example 1 and the results are provided in Figure 14. These data demonstrate the smaller 50 nm LNP formulation (LNP 8-2) performs less well compared to the larger 70 nm formulation (LNP 8-1), which is in stark contrast the results in NHP.

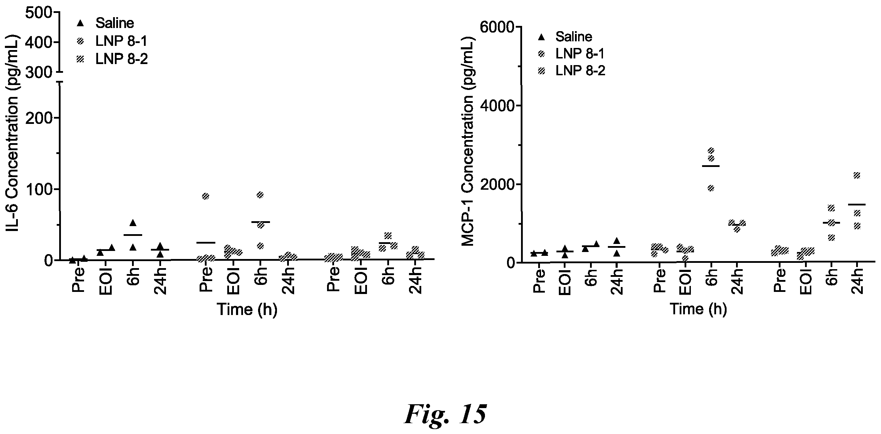

Plasma cytokine levels were determined as shown in Figure 15. Quantitation of cytokines in blood plasma was accomplished using standard methods known to those of ordinary skill in the art. Measurements were made pre-dose, EOI, and 6 and 24 hours post EOI. These data show lower peak induction (i.e. at 6 hours) of IL-6 and MCP-1 at 6 hours post EOI for the embodiment with a smaller 50 nm diameter LNP formulation (LNP8-2) compared to the larger 70 nm formulation (LNP 8-1).

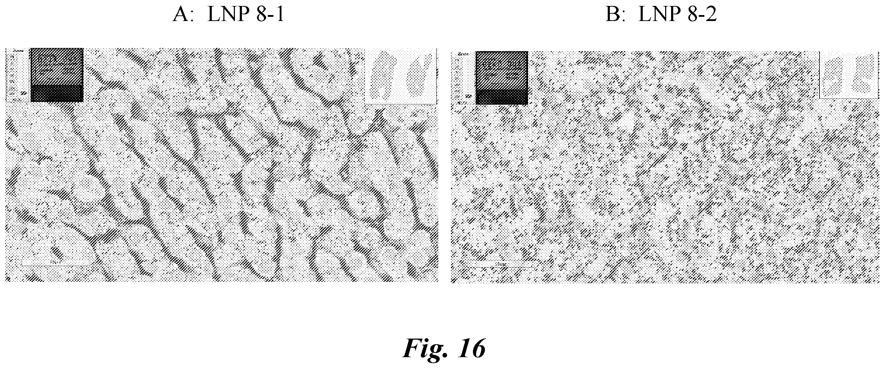

The distribution of the LNP to hepatocytes was characterized by In situ hybridization as described in Example 5. Figures 16A and 16B are provided to demonstrate the differential in distribution at 4 hours post administration of LNP in the hepatocytes and sinusoidal space for different size LNP (~50 nm vs. ~70 nm) of the same composition. The smaller ~50 nm LNP show greater distribution into hepatocytes at 4 hours as well as less accumulation in the sinusoidal spaces than the larger ~70 nm LNP. Without wishing to bound by theory, the higher expression of the smaller LNP is consistent with larger LNP being prevented from accessing hepatocytes to be productively expressed while smaller LNP cross the sinusoidal wall more readily for fast uptake into the hepatocytes.

### EXAMPLE 8

### IN VIVO STUDY OF LIPID NANOPARTICLE FORMULATIONS IN NON-HUMAN PRIMATES

Experimentally naive male cynomolgus monkeys (*Macaca fascicularis,* macaque) were given control (saline) or test doses of LNP formulations via a 1-hour intravenous (IV) infusion in groups of four animals. The LNP formulation contained an mRNA expression vector for human immunoglobulin G, type 1 (IgG1). LNPs were synthesized according to standard methods known to those skilled in the art, or as described herein in Example 1, using cationic lipd III-45 and PEG lipid with C14 di-acyl chains as described above and size of 70 nm (LNP 9-1). Another LNP test group had the same composition but smaller LNP diameter of 54 nm (LNP 9-2). Non-control animals were dosed at 0.5 mg/kg or 2.0 mg/kg RNA with a dose volume of 5 mL/kg.

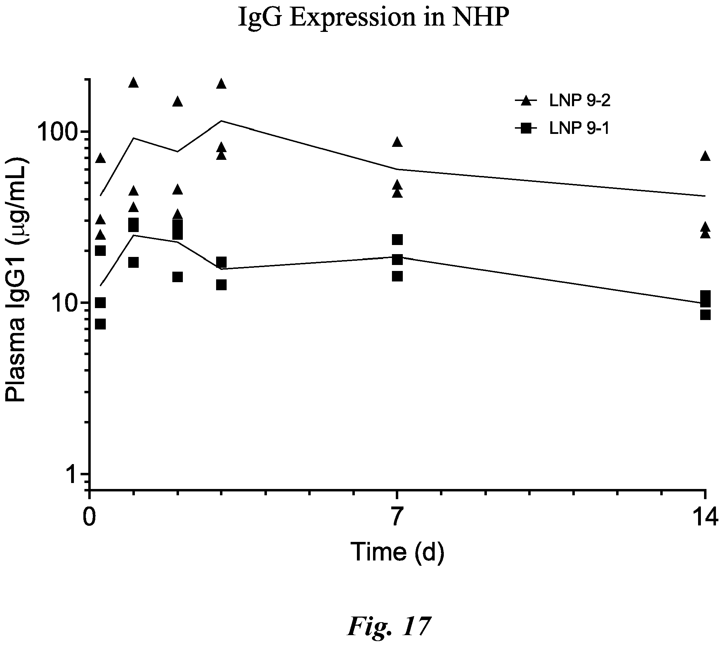

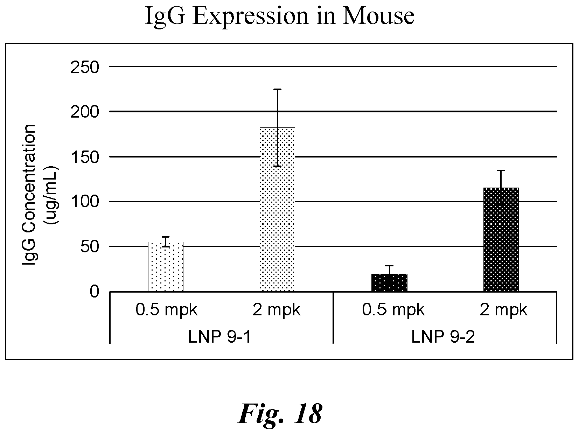

Pharmacodynamic samples to evaluate plasma concentrations of IgG1 were obtained by blood draw (K₃EDTA, 0.5 mL) prior to infusion; 6 hours post infusion and on days 1, 2, 4, 7 and 14. Quantitation of expressed human IgG1 in blood plasma was accomplished using standard methods known to those of ordinary skill in the art. Figure 17 shows IgG1 plasma concentration levels demonstrating that IgG1 was expressed greatest for LNP embodiments with size ~54 nm (LNP 9-2) than size ~70 nm (LNP 9-1) in the NHP. The same preparations were administered in a murine model as described in Example 1 and the results are provided in Figure 18. These data demonstrate the smaller 54 nm LNP formulation (LNP 9-2) performs less well compared to the larger 70 nm formulation (LNP 9-1), which is in stark contrast the results in NHP.

### EXAMPLE 9

### IN VIVO STUDY OF LIPID NANOPARTICLE FORMULATIONS IN NON-HUMAN PRIMATES

Experimentally naive male cynomolgus monkeys (*Macaca fascicularis,* macaque) were given control (saline) or test doses of LNP formulations via a 1-hour intravenous (IV) infusion in groups of three animals. The LNP formulation contained an mRNA expression vector for human immunoglobulin G, type 1 (IgG1). LNPs were synthesized according to standard methods known to those skilled in the art, or as described herein in Example 1, using cationic lipd II-15 and PEG lipid with C14 di-acyl chains as described above and size of 67nm (LNP 10-1). Another LNP test group had the same composition but smaller LNP diameter of 59 nm (LNP 10-2). Non-control animals were dosed at 3.0 mg/kg RNA with a dose volume of 5 mL/kg.

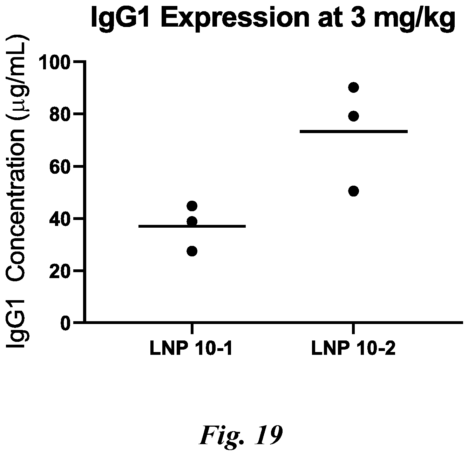

Pharmacodynamic samples to evaluate plasma concentrations of IgG1 were obtained by blood draw (K₃EDTA, 0.5 mL) prior to infusion; 6 hours post infusion and on days 1, 2, 3, and 4. Quantitation of expressed human IgG1 in blood plasma was accomplished using standard methods known to those of ordinary skill in the art. Figure 19 shows IgG1 plasma concentration levels demonstrating that IgG1 was expressed greatest for LNP embodiments with size ~59 nm (LNP 10-2) than size ~67 nm (LNP 10-1).

## Claims

1. Lipid nanoparticles (LNPs) for use in a method of treating or preventing a disease in a primate in need thereof, wherein the method comprises administering the LNPs to the primate, each of the LNPs comprising:
i) a nucleic acid, or a pharmaceutically acceptable salt thereof, encapsulated within the LNP;
ii) a cationic lipid;
iii) a neutral lipid;
iv) a steroid; and
v) a polymer-conjugated lipid,
wherein a mean particle diameter of the LNPs ranges from 40 nm to 68 nm as determined by quasi-elastic light scattering.

2. The LNPs for use according to claim 1, wherein the mean particle diameter ranges from 50 nm to 68 nm, from 55 nm to 65 nm, from 50 nm to 60 nm or from 60 nm to 68 nm.

3. The LNPs for use according to claim 1, wherein the mean particle diameter is about 47 nm, about 48 nm, about 49 nm, about 50 nm, about 51 nm, about 52 nm, about 53 nm, about 54 nm, about 55 nm, about 56 nm, about 57 nm, about 58 nm, about 59 nm, about 60 nm, about 61 nm, about 62 nm, about 63 nm, about 64 nm or about 65 nm.

4. The LNPs for use according to any one of claims 1-3, wherein:
i) the cationic lipid has a structure of Formula (I): 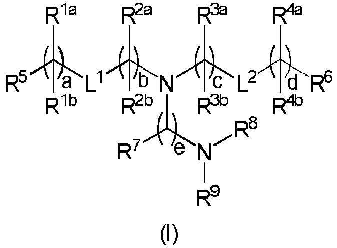 or a pharmaceutically acceptable salt, tautomer or stereoisomer thereof, wherein:
one of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- or
-NR^{a}C(=O)O-, and the other of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, ,NR^{a}C(=O)NR^{a}-, -OC(=O)NR^{a}- or-NR^{a}C(=O)O- or a direct bond;
R^{a} is H or C₁-C₁₂ alkyl;
R^{1a} and R^{1b} are, at each occurrence, independently either (a) H or C₁-C₁₂ alkyl, or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
R^{2a} and R^{2b} are, at each occurrence, independently either (a) H or C₁-C₁₂ alkyl, or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
R^{3a} and R^{3b} are, at each occurrence, independently either (a) H or C₁-C₁₂ alkyl, or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
R^{4a} and R^{4b} are, at each occurrence, independently either (a) H or C₁-C₁₂ alkyl, or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
R⁵ and R⁶ are each independently methyl or cycloalkyl;
R⁷ is, at each occurrence, independently H or C₁-C₁₂ alkyl;
R⁸ and R⁹ are each independently unsubstituted C₁-C₁₂ alkyl; or R⁸ and R⁹, together with the nitrogen atom to which they are attached, form a 5, 6 or 7-membered heterocyclic ring comprising one nitrogen atom;
a and d are each independently an integer from 0 to 24;
b and c are each independently an integer from 1 to 24;
e is 1 or 2; and
x is 0, 1 or 2; or
ii) the cationic lipid has a structure of Formula (II): 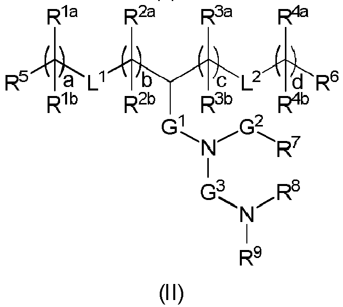 or a pharmaceutically acceptable salt, tautomer or stereoisomer thereof, wherein:
one of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- or -NR^{a}C(=O)O-, and the other of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, ,NR^{a}C(=O)NR^{a}-, -OC(=O)NR^{a}- or-NR^{a}C(=O)O- or a direct bond;
G¹ is C₁-C₂ alkylene, -(C=O)-, -O(C=O)-, -SC(=O)-, -NR^{a}C(=O)- or a direct bond;
G² is -C(=O)-, -(C=O)O-, -C(=O)S-, -C(=O)NR^{a}- or a direct bond;
G³ is C₁-C₆ alkylene;
R^{a} is H or C₁-C₁₂ alkyl;
R^{1a} and R^{1b} are, at each occurrence, independently either: (a) H or C₁-C₁₂ alkyl; or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
R^{2a} and R^{2b} are, at each occurrence, independently either: (a) H or C₁-C₁₂ alkyl; or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
R^{3a} and R^{3b} are, at each occurrence, independently either (a): H or C₁-C₁₂ alkyl; or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
R^{4a} and R^{4b} are, at each occurrence, independently either: (a) H or C₁-C₁₂ alkyl; or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
R⁵ and R⁶ are each independently H or methyl;
R⁷ is C₄-C₂₀ alkyl;
R⁸ and R⁹ are each independently C₁-C₁₂ alkyl; or R⁸ and R⁹, together with the nitrogen atom to which they are attached, form a 5, 6 or 7-membered heterocyclic ring;
a, b, c and d are each independently an integer from 1 to 24; and
x is 0, 1 or 2; or
iii) the cationic lipid has a structure of Formula III: 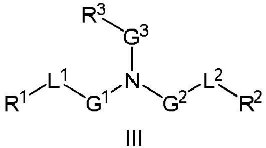 or a pharmaceutically acceptable salt or stereoisomer thereof, wherein:
one of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- or -NR^{a}C(=O)O-, and the other of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, ,NR^{a}C(=O)NR^{a}-, -OC(=O)NR^{a}- or-NR^{a}C(=O)O- or a direct bond;
G¹ and G² are each independently unsubstituted C₁-C₁₂ alkylene or C₁-C₁₂ alkenylene;
G³ is C₁-C₂₄ alkylene, C₁-C₂₄ alkenylene, C₃-C₈ cycloalkylene, C₃-C₈ cycloalkenylene;
R^{a} is H or C₁-C₁₂ alkyl;
R¹ and R² are each independently C₆-C₂₄ alkyl or C₆-C₂₄ alkenyl;
R³ is H, OR⁵, CN, -C(=O)OR⁴, -OC(=O)R⁴ or -NR⁵C(=O)R⁴;
R⁴ is C₁-C₁₂ alkyl;
R⁵ is H or C₁-C₆ alkyl; and
x is 0, 1 or 2; or
iv) the cationic lipid has the following Formula (IV): 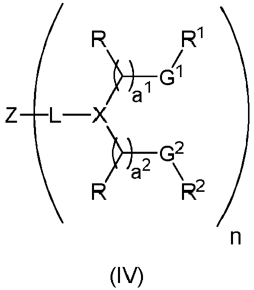 or a pharmaceutically acceptable salt or stereoisomer thereof, wherein:
one of G¹ or G² is, at each occurrence, -O(C=O)-, -(C=O)O-, -C(=O)-, -O-, -S(O)_{y}-, -S-S-, -C(=O)S-, SC(=O)-, -N(R^{a})C(=O)-, -C(=O)N(R^{a})-, -N(R^{a})C(=O)N(R^{a})-, -OC(=O)N(R^{a})- or -N(R^{a})C(=O)O-, and the other of G¹ or G² is, at each occurrence, -O(C=O)-, -(C=O)O-, -C(=O)-, -O-, -S(O)_{y}-, -S-S-, -C(=O)S-, -SC(=O)-, -N(R^{a})C(=O)-, -C(=O)N(R^{a})-, -N(R^{a})C(=O)N(R^{a})-, -OC(=O)N(R^{a})- or -N(R^{a})C(=O)O- or a direct bond;
L is, at each occurrence, ~O(C=O)-, wherein ~ represents a covalent bond to X;
X is CR^{a};
Z is alkyl, cycloalkyl or a monovalent moiety comprising at least one polar functional group when n is 1; or Z is alkylene, cycloalkylene or a polyvalent moiety comprising at least one polar functional group when n is greater than 1;
R^{a} is, at each occurrence, independently H, C₁-C₁₂ alkyl, C₁-C₁₂ hydroxylalkyl, C₁-C₁₂ aminoalkyl, C₁-C₁₂ alkylaminylalkyl, C₁-C₁₂ alkoxyalkyl, C₁-C₁₂ alkoxycarbonyl, C₁-C₁₂ alkylcarbonyloxy, C₁-C₁₂ alkylcarbonyloxyalkyl or C₁-C₁₂ alkylcarbonyl;
R is, at each occurrence, independently either: (a) H or C₁-C₁₂ alkyl; or (b) R together with the carbon atom to which it is bound is taken together with an adjacent R and the carbon atom to which it is bound to form a carbon-carbon double bond;
R¹ and R² have, at each occurrence, the following structure, respectively: 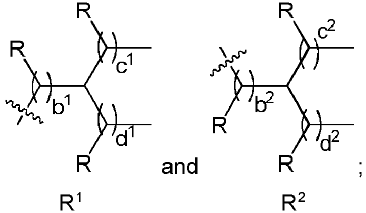
a¹ and a² are, at each occurrence, independently an integer from 3 to 12;
b¹ and b² are, at each occurrence, independently 0 or 1;
c¹ and c² are, at each occurrence, independently an integer from 5 to 10;
d¹ and d² are, at each occurrence, independently an integer from 5 to 10;
y is, at each occurrence, independently an integer from 0 to 2; and
n is an integer from 1 to 6,
wherein each alkyl, alkylene, hydroxylalkyl, aminoalkyl, alkylaminylalkyl, alkoxyalkyl, alkoxycarbonyl, alkylcarbonyloxy, alkylcarbonyloxyalkyl and alkylcarbonyl is optionally substituted with one or more substituent; or
v) cationic lipid has the following Formula (V): 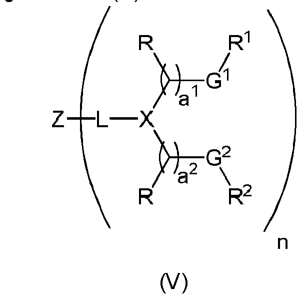 or a pharmaceutically acceptable salt or stereoisomer thereof, wherein:
one of G¹ or G² is, at each occurrence, -O(C=O)-, -(C=O)O-, -C(=O)-, -O-, -S(O)_{y}-, -S-S-, -C(=O)S-, SC(=O)-, -N(R^{a})C(=O)-, -C(=O)N(R^{a})-, -N(R^{a})C(=O)N(R^{a})-, -OC(=O)N(R^{a})- or -N(R^{a})C(=O)O-, and the other of G¹ or G² is, at each occurrence, -O(C=O)-, -(C=O)O-, -C(=O)-, -O-, -S(O)_{y}-, -S-S-, -C(=O)S-, -SC(=O)-, -N(R^{a})C(=O)-, -C(=O)N(R^{a})-, -N(R^{a})C(=O)N(R^{a})-, -OC(=O)N(R^{a})- or -N(R^{a})C(=O)O- or a direct bond;
L is, at each occurrence, ~O(C=O)-, wherein ~ represents a covalent bond to X;
X is CR^{a};
Z is alkyl, cycloalkyl or a monovalent moiety comprising at least one polar functional group when n is 1; or Z is alkylene, cycloalkylene or a polyvalent moiety comprising at least one polar functional group when n is greater than 1;
R^{a} is, at each occurrence, independently H, C₁-C₁₂ alkyl, C₁-C₁₂ hydroxylalkyl, C₁-C₁₂ aminoalkyl, C₁-C₁₂ alkylaminylalkyl, C₁-C₁₂ alkoxyalkyl, C₁-C₁₂ alkoxycarbonyl, C₁-C₁₂ alkylcarbonyloxy, C₁-C₁₂ alkylcarbonyloxyalkyl or C₁-C₁₂ alkylcarbonyl;
R is, at each occurrence, independently either: (a) H or C₁-C₁₂ alkyl; or (b) R together with the carbon atom to which it is bound is taken together with an adjacent R and the carbon atom to which it is bound to form a carbon-carbon double bond;
R¹ and R² have, at each occurrence, the following structure, respectively: 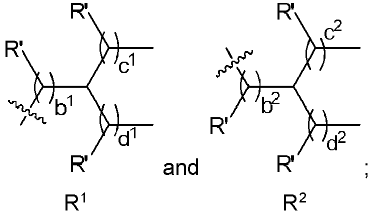 R' is, at each occurrence, independently H or C₁-C₁₂ alkyl;
a¹ and a² are, at each occurrence, independently an integer from 3 to 12;
b¹ and b² are, at each occurrence, independently 0 or 1;
c¹ and c² are, at each occurrence, independently an integer from 2 to 12;
d¹ and d² are, at each occurrence, independently an integer from 2 to 12;
y is, at each occurrence, independently an integer from 0 to 2; and
n is an integer from 1 to 6,
wherein a¹, a², c¹, c², d¹ and d² are selected such that the sum of a¹+c¹+d¹ is an integer from 18 to 30, and the sum of a²+c²+d² is an integer from 18 to 30, and wherein each alkyl, alkylene, hydroxylalkyl, aminoalkyl, alkylaminylalkyl, alkoxyalkyl, alkoxycarbonyl, alkylcarbonyloxy, alkylcarbonyloxyalkyl and alkylcarbonyl is optionally substituted with one or more substituent; or
vi) cationic lipid has the following Formula (VI): 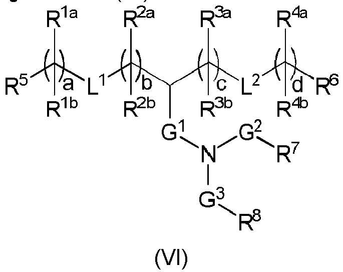 or a pharmaceutically acceptable salt, tautomer or stereoisomer thereof, wherein:
L¹ and L² are each independently -O(C=O)-, -(C=O)O-, -C(=O)-, -O-, -S(O)ₓ, -S-S-, -C(=O)S-, -SC(=O)-, -NR^{a}C(=O)-, -C(=O)NR^{a}-, -NR^{a}C(=O)NR^{a}-, -OC(=O)NR^{a}-, -NR^{a}C(=O)O- or a direct bond;
G¹ is C₁-C₂ alkylene, -(C=O)-, -O(C=O)-, -SC(=O)-, -NR^{a}C(=O)- or a direct bond;
G² is -C(=O)-, -(C=O)O-, -C(=O)S-, -C(=O)NR^{a}- or a direct bond;
G³ is C₁-C₆ alkylene;
R^{a} is H or C₁-C₁₂ alkyl;
R^{1a} and R^{1b} are, at each occurrence, independently either: (a) H or C₁-C₁₂ alkyl; or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
R^{2a} and R^{2b} are, at each occurrence, independently either: (a) H or C₁-C₁₂ alkyl; or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
R^{3a} and R^{3b} are, at each occurrence, independently either (a): H or C₁-C₁₂ alkyl; or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
R^{4a} and R^{4b} are, at each occurrence, independently either: (a) H or C₁-C₁₂ alkyl; or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
R⁵ and R⁶ are each independently H or methyl;
R⁷ is H or C₁-C₂₀ alkyl;
R⁸ is OH, -N(R⁹)(C=O)R¹⁰, -(C=O)NR⁹R¹⁰, -NR⁹R¹⁰, -(C=O)OR¹¹ or -O(C=O)R¹¹, provided that G³ is C₄-C₆ alkylene when R⁸ is -NR⁹R¹⁰,
R⁹ and R¹⁰ are each independently H or C₁-C₁₂ alkyl;
R¹¹ is aralkyl;
a, b, c and d are each independently an integer from 1 to 24; and
x is 0, 1 or 2,
wherein each alkyl, alkylene and aralkyl is optionally substituted; or
vii) the cationic lipid has the following Formula (VII): 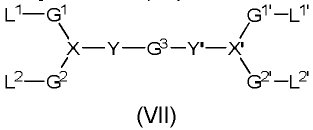 or a pharmaceutically acceptable salt or stereoisomer thereof, wherein:
X and X' are each independently N or CR;
Y and Y' are each independently absent, -O(C=O)-, -(C=O)O- or NR, provided that:
a)Y is absent when X is N;
b) Y' is absent when X' is N;
c) Y is -O(C=O)-, -(C=O)O- or NR when X is CR; and
d) Y' is -O(C=O)-, -(C=O)O- or NR when X' is CR,
L¹ and L^{1'} are each independently -O(C=O)R¹, -(C=O)OR¹, -C(=O)R¹, -OR¹, - S(O)_{z}R¹, -S-SR¹, -C(=O)SR¹, -SC(=O)R¹, -NR^{a}C(=O)R¹, -C(=O)NR^{b}R^{c}, -NR^{a}C(=O)NR^{b}R^{c}, - OC(=O)NR^{b}R^{c} or -NR^{a}C(=O)OR¹;
L² and L² are each independently -O(C=O)R², -(C=O)OR², -C(=O)R², -OR², - S(O)_{z}R², -S-SR², -C(=O)SR², -SC(=O)R², -NR^{d}C(=O)R², -C(=O)NR^{e}R^{f}, -NR^{d}C(=O)NR^{e}R^{f}, - OC(=O)NR^{e}R^{f};-NR^{d}C(=O)OR² or a direct bond to R²;
G¹, G^{1'}, G² and G² are each independently C₂-C₁₂ alkylene or C₂-C₁₂ alkenylene;
G³ is C₂-C₂₄ heteroalkylene or C₂-C₂₄ heteroalkenylene;
R^{a}, R^{b}, R^{d} and R^{e} are, at each occurrence, independently H, C₁-C₁₂ alkyl or C₂-C₁₂ alkenyl;
R^{c} and R^{f} are, at each occurrence, independently C₁-C₁₂ alkyl or C₂-C₁₂ alkenyl;
R is, at each occurrence, independently H or C₁-C₁₂ alkyl;
R¹ and R² are, at each occurrence, independently branched C₆-C₂₄ alkyl or branched C₆-C₂₄ alkenyl;
z is 0, 1 or 2, and
wherein each alkyl, alkenyl, alkylene, alkenylene, heteroalkylene and heteroalkenylene is independently substituted or unsubstituted unless otherwise specified; or
viii) the cationic lipid has the following Formula (VIII): 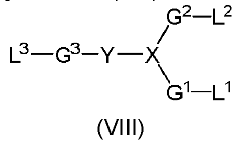 or a pharmaceutically acceptable salt or stereoisomer thereof, wherein:
X is N, and Y is absent; or X is CR, and Y is NR;
L¹ is-O(C=O)R¹, -(C=O)OR¹, -C(=O)R¹, -OR¹, -S(O)ₓR¹, -S-SR¹, -C(=O)SR¹, -SC(=O)R¹, -NR^{a}C(=O)R¹, -C(=O)NR^{b}R^{c}, -NR^{a}C(=O)NR^{b}R^{c}, -OC(=O)NR^{b}R^{c} or -NR^{a}C(=O)OR¹;
L² is -O(C=O)R², -(C=O)OR², -C(=O)R², -OR², -S(O)ₓR², -S-SR², -C(=O)SR², -SC(=O)R², -NR^{d}C(=O)R², -C(=O)NR^{e}R^{f}, -NR^{d}C(=O)NR^{e}R^{f}, -OC(=O)NR^{e}R^{f}; -NR^{d}C(=O)OR² or a direct bond to R²;
L³ is -O(C=O)R³ or -(C=O)OR³;
G¹ and G² are each independently C₂-C₁₂ alkylene or C₂-C₁₂ alkenylene;
G³ is C₁-C₂₄ alkylene, C₂-C₂₄ alkenylene, C₁-C₂₄ heteroalkylene or C₂-C₂₄ heteroalkenylene when X is CR, and Y is NR; and G³ is C₁-C₂₄ heteroalkylene or C₂-C₂₄ heteroalkenylene when X is N, and Y is absent;
R^{a}, R^{b}, R^{d} and R^{e} are each independently H or C₁-C₁₂ alkyl or C₁-C₁₂ alkenyl;
R^{c} and R^{f} are each independently C₁-C₁₂ alkyl or C₂-C₁₂ alkenyl;
each R is independently H or C₁-C₁₂ alkyl;
R¹, R² and R³ are each independently C₁-C₂₄ alkyl or C₂-C₂₄ alkenyl; and
x is 0, 1 or 2, and
wherein each alkyl, alkenyl, alkylene, alkenylene, heteroalkylene and heteroalkenylene is independently substituted or unsubstituted unless otherwise specified; or
ix) the cationic lipid has the following Formula (IX): 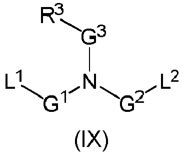 or a pharmaceutically acceptable salt or stereoisomer thereof, wherein:
L¹ is-O(C=O)R¹, -(C=O)OR¹, -C(=O)R¹, -OR¹, -S(O)ₓR¹, -S-SR¹, -C(=O)SR¹, - SC(=O)R¹, -NR^{a}C(=O)R¹, -C(=O)NR^{b}R^{c}, -NR^{a}C(=O)NR^{b}R^{c}, -OC(=O)NR^{b}R^{c} or -NR^{a}C(=O)OR¹;
L² is -O(C=O)R², -(C=O)OR², -C(=O)R², -OR², -S(O)ₓR², -S-SR², -C(=O)SR², - SC(=O)R², -NR^{d}C(=O)R², -C(=O)NR^{e}R^{f}, -NR^{d}C(=O)NR^{e}R^{f}, -OC(=O)NR^{e}R^{f}; -NR^{d}C(=O)OR² or a direct bond to R²;
G¹ and G² are each independently C₂-C₁₂ alkylene or C₂-C₁₂ alkenylene;
G³ is C₁-C₂₄ alkylene, C₂-C₂₄ alkenylene, C₃-C₈ cycloalkylene or C₃-C₈ cycloalkenylene;
R^{a}, R^{b}, R^{d} and R^{e} are each independently H or C₁-C₁₂ alkyl or C₁-C₁₂ alkenyl;
R^{c} and R^{f} are each independently C₁-C₁₂ alkyl or C₂-C₁₂ alkenyl;
R¹ and R² are each independently branched C₆-C₂₄ alkyl or branched C₆-C₂₄ alkenyl;
R³ is -N(R⁴)R⁵;
R⁴ is C₁-C₁₂ alkyl;
R⁵ is substituted C₁-C₁₂ alkyl; and
x is 0, 1 or 2, and
wherein each alkyl, alkenyl, alkylene, alkenylene, cycloalkylene, cycloalkenylene, aryl and aralkyl is independently substituted or unsubstituted unless otherwise specified; or
x) the cationic lipid has the following Formula (X): 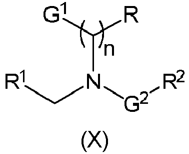 or a pharmaceutically acceptable salt or stereoisomer thereof, wherein:
G¹ is -OH, -NR³R⁴, -(C=O)NR⁵ or -NR³(C=O)R⁵;
G² is -CH₂- or -(C=O)-;
R is, at each occurrence, independently H or OH;
R¹ and R² are each independently optionally substituted branched, saturated or unsaturated C₁₂-C₃₆ alkyl;
R³ and R⁴ are each independently H or optionally substituted straight or branched, saturated or unsaturated C₁-C₆ alkyl;
R⁵ is optionally substituted straight or branched, saturated or unsaturated C₁-C₆ alkyl; and
n is an integer from 2 to 6.

5. The LNPs for use according to any one of claims 1-4, wherein the cationic lipid is formed from a lipid structure selected from:

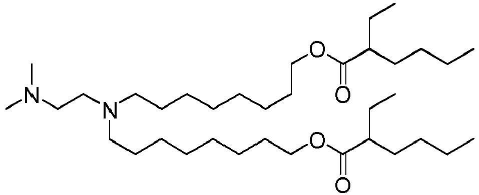

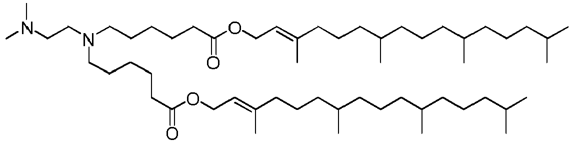

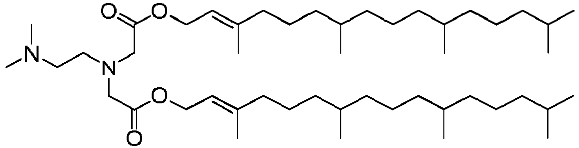

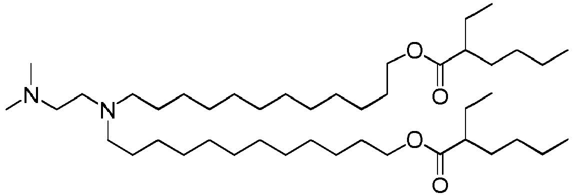

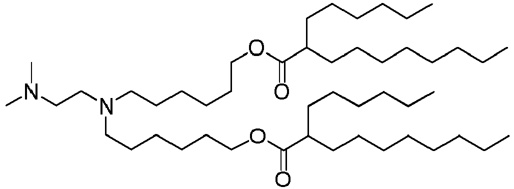

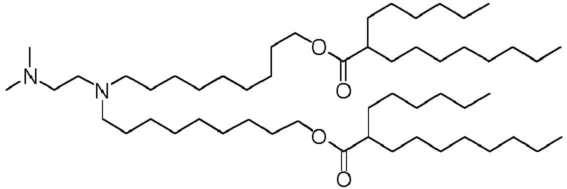

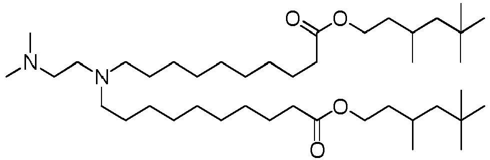

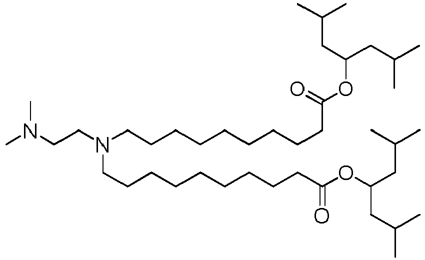

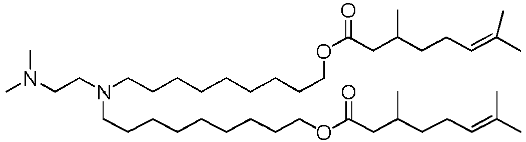

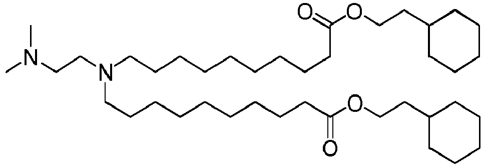

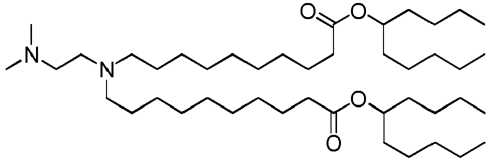

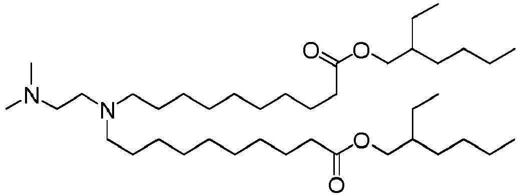

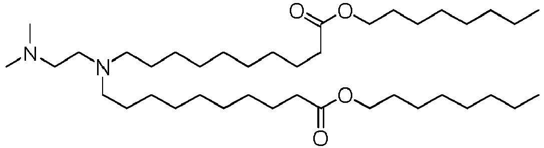

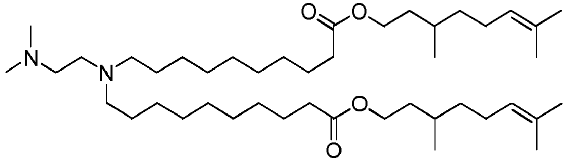

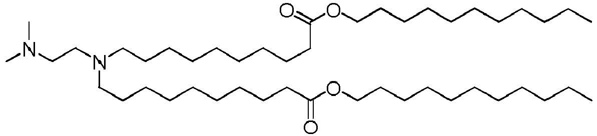

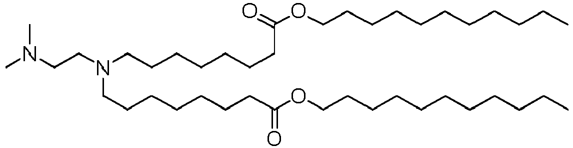

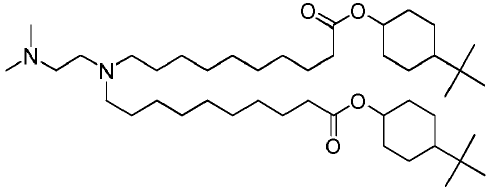

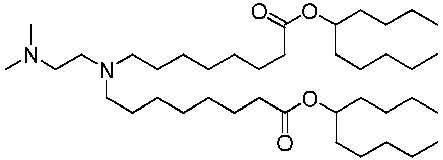

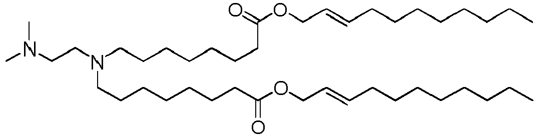

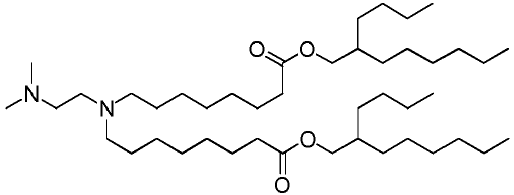

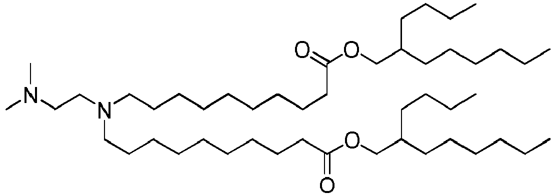

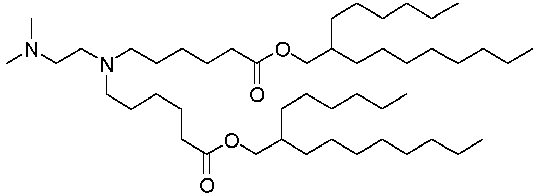

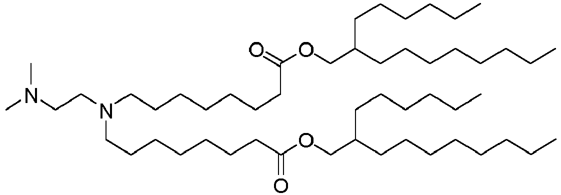

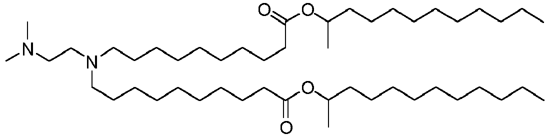

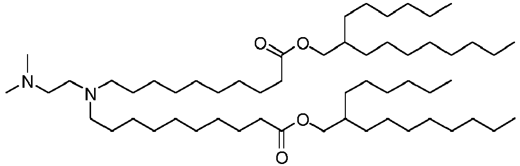

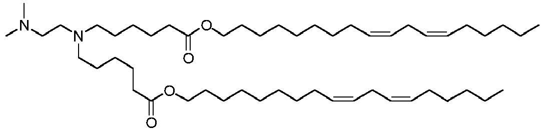

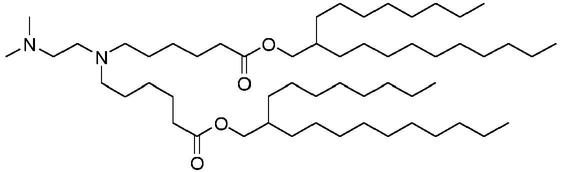

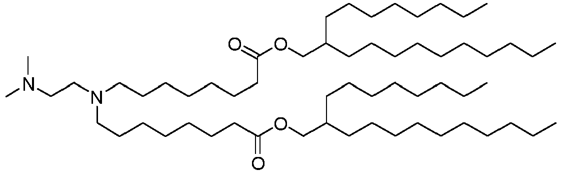

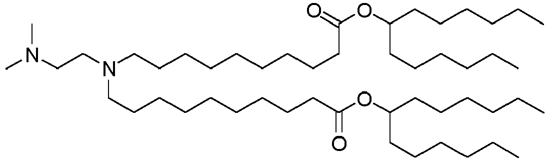

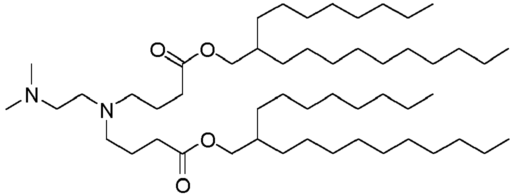

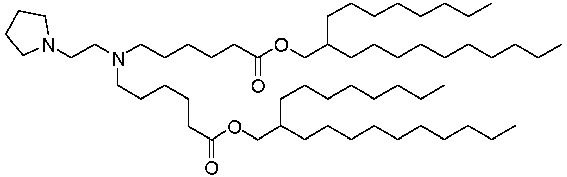

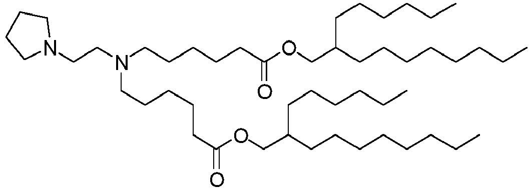

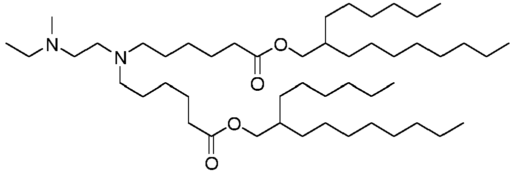

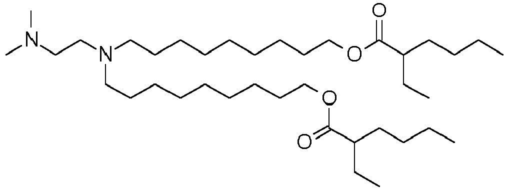

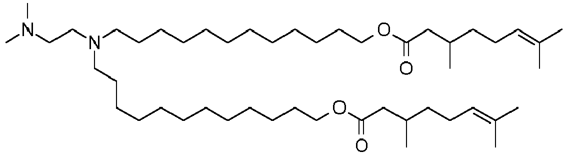

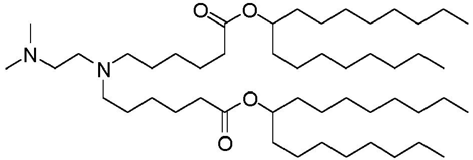

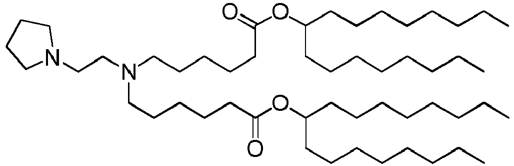

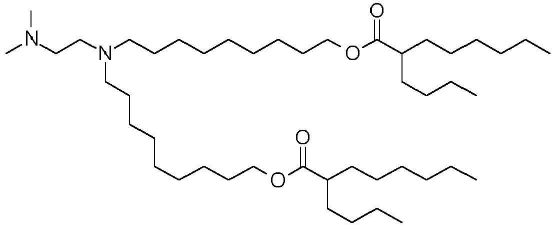

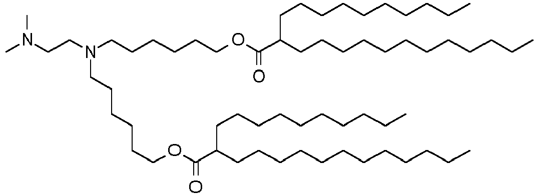

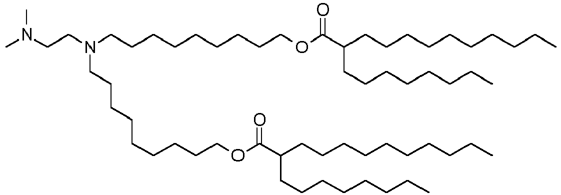

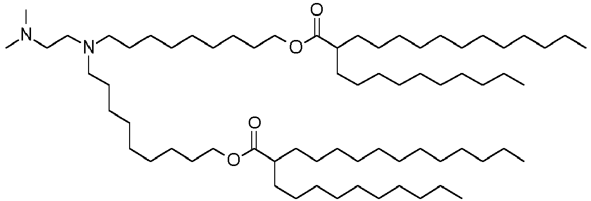

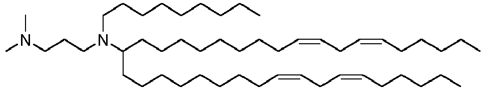

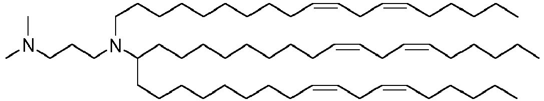

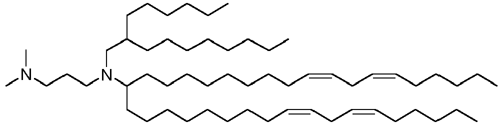

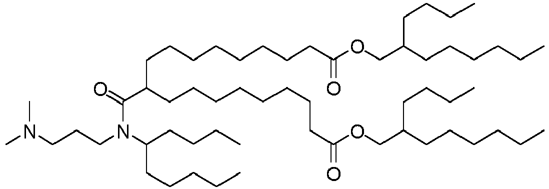

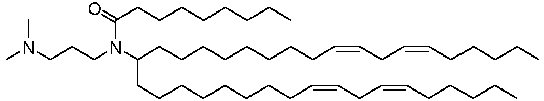

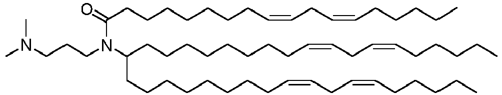

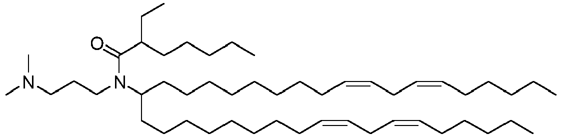

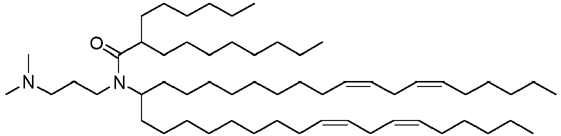

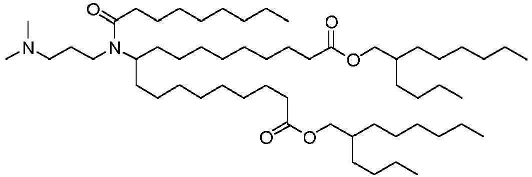

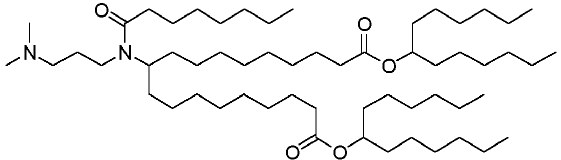

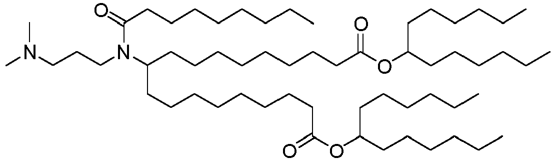

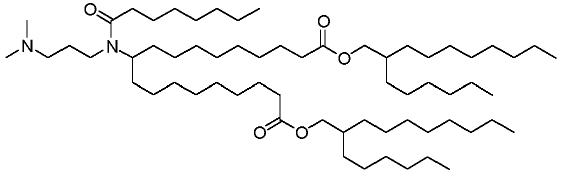

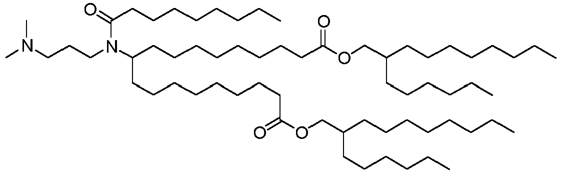

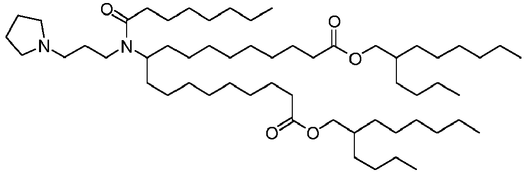

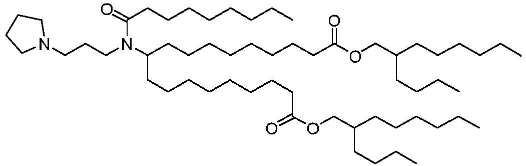

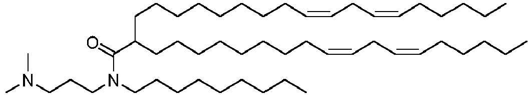

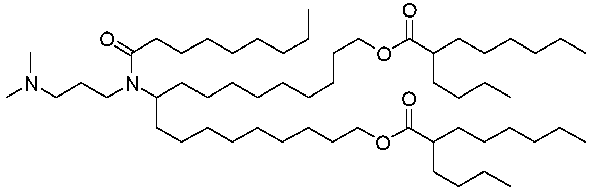

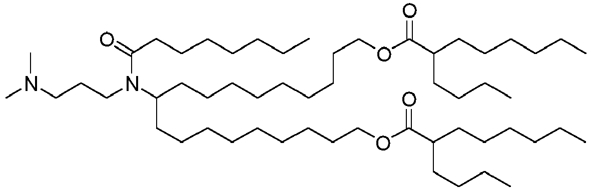

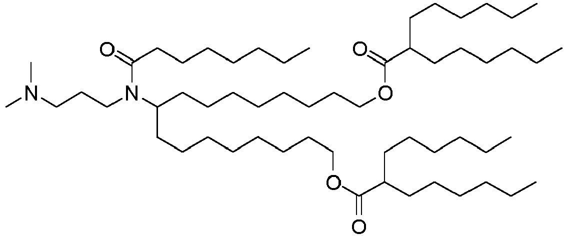

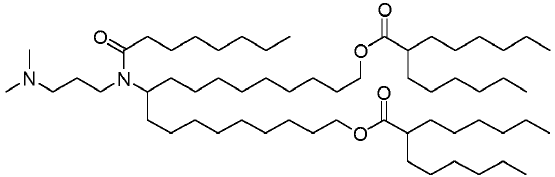

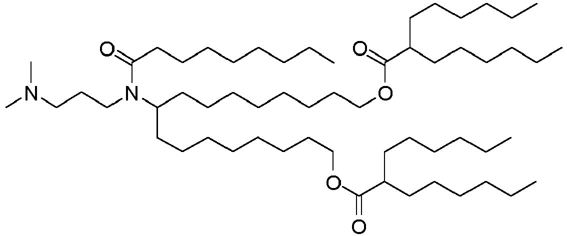

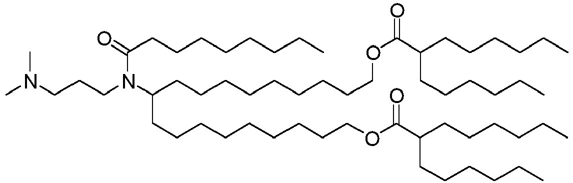

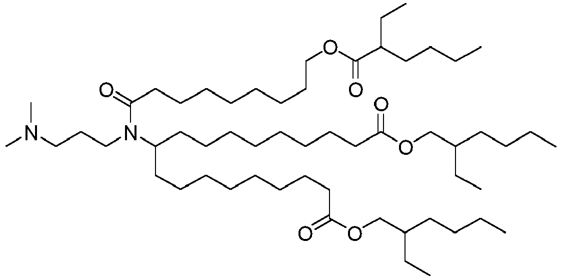

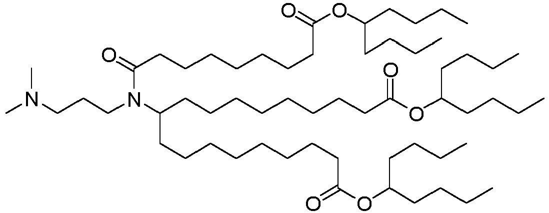

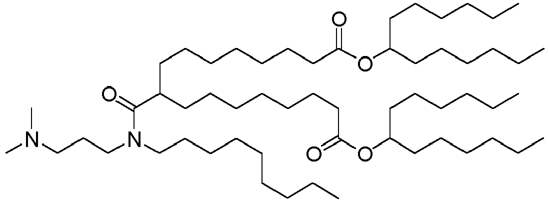

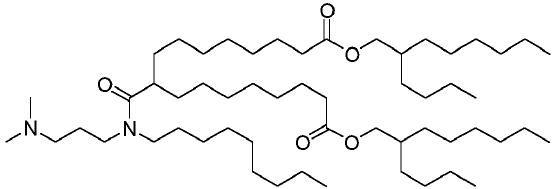

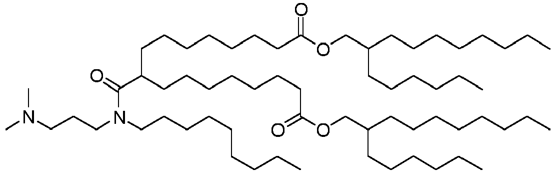

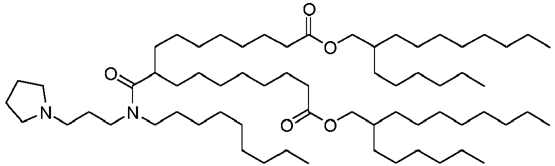

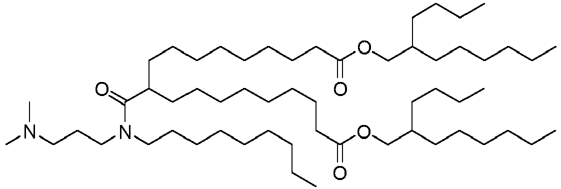

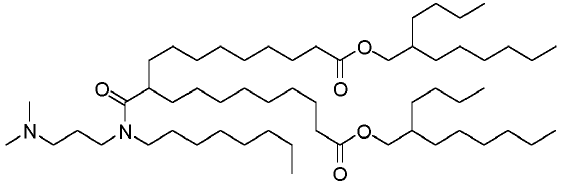

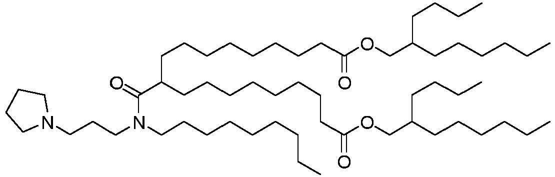

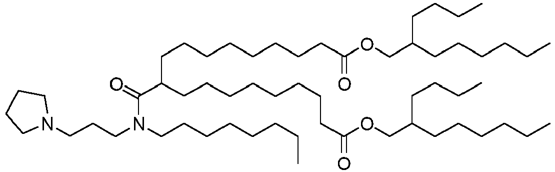

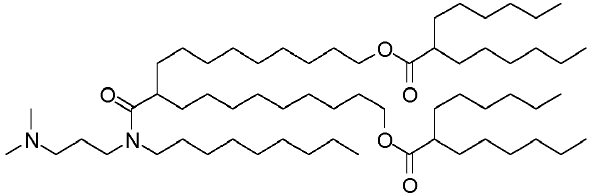

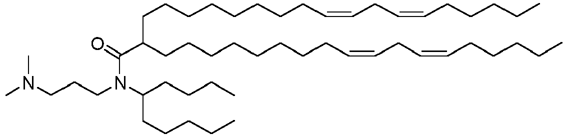

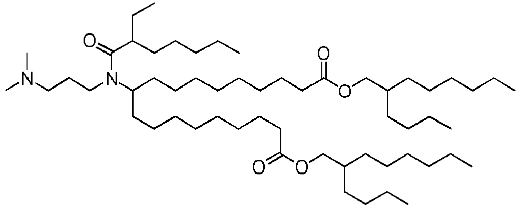

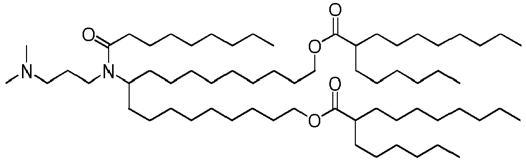

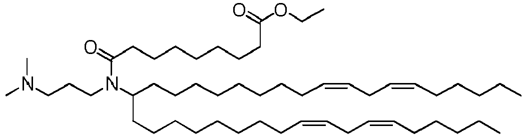

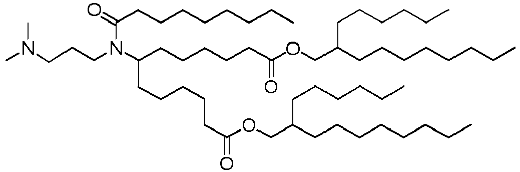

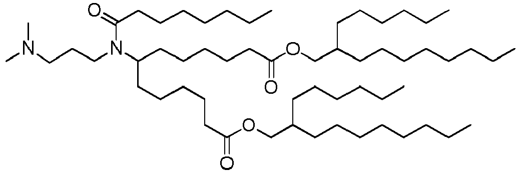

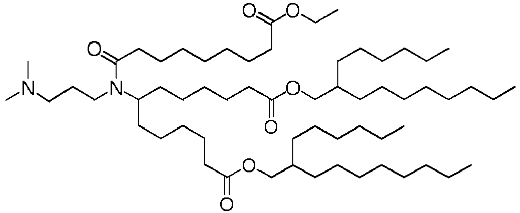

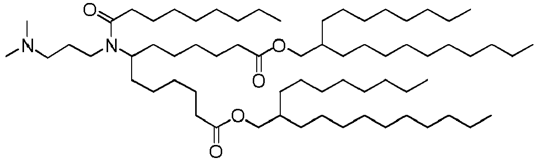

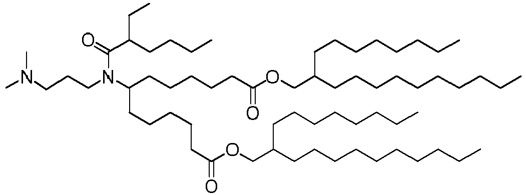

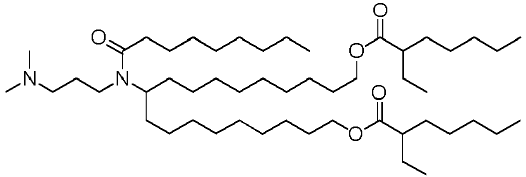

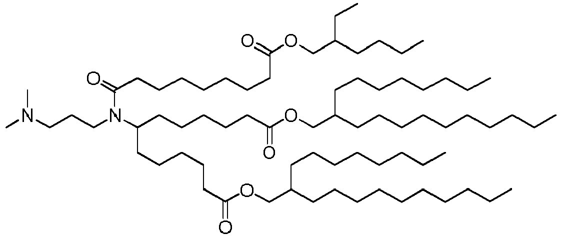

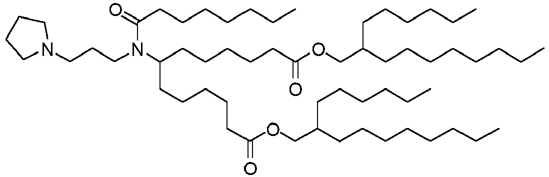

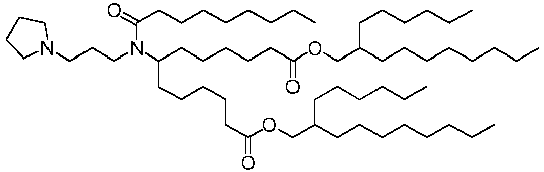

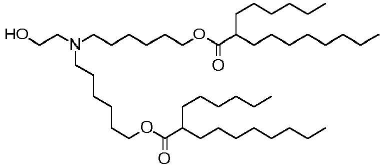

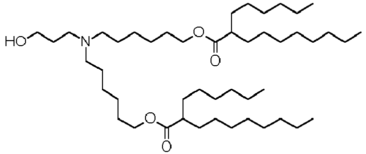

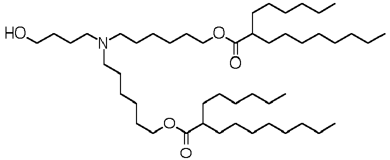

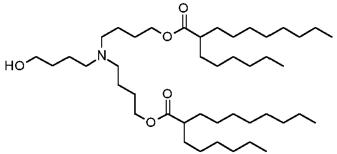

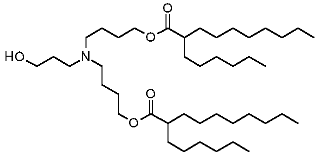

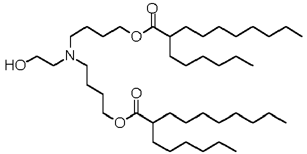

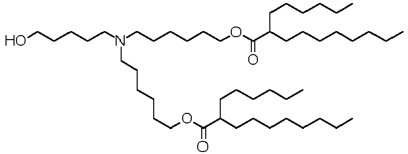

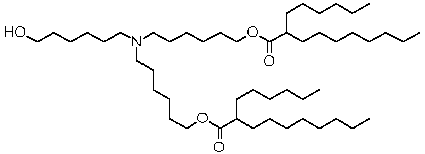

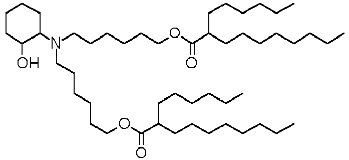

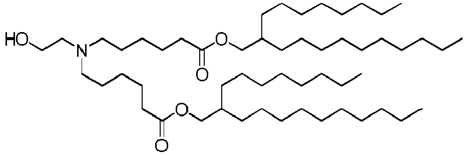

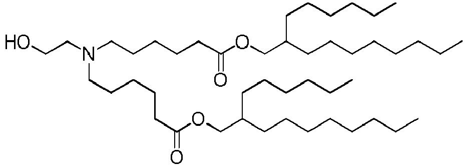

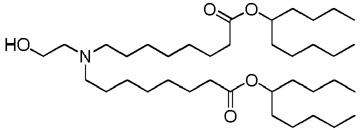

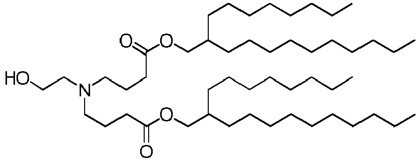

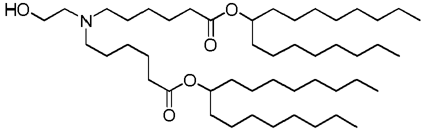

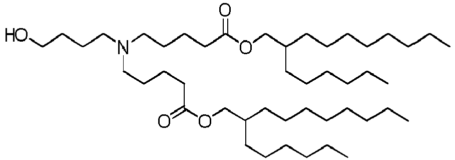

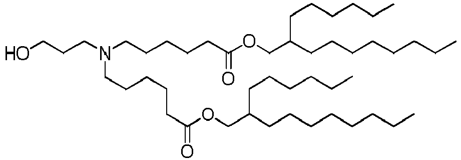

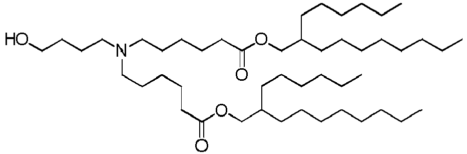

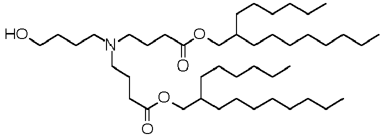

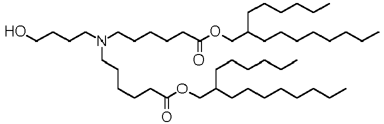

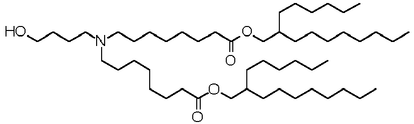

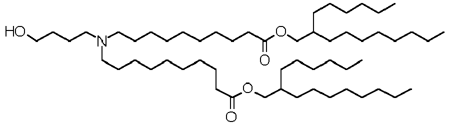

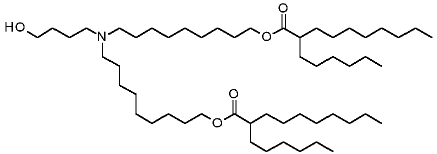

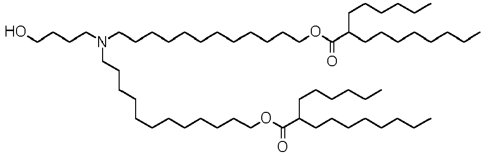

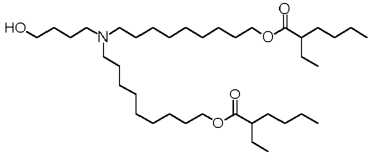

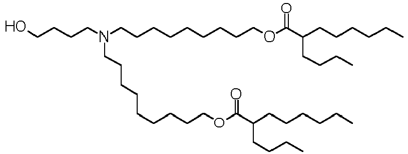

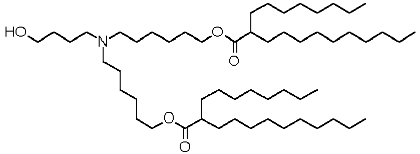

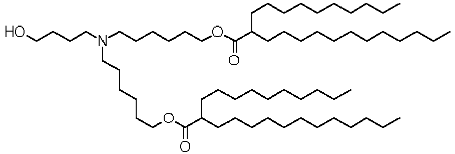

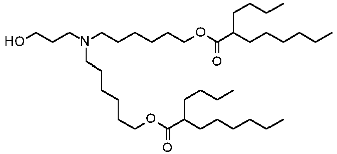

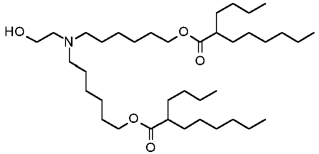

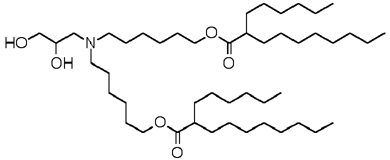

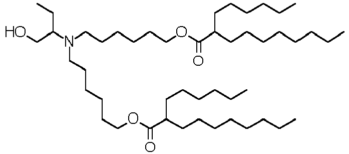

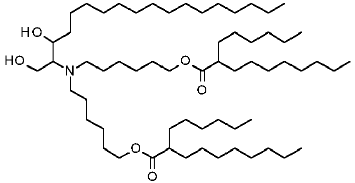

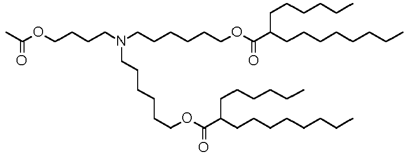

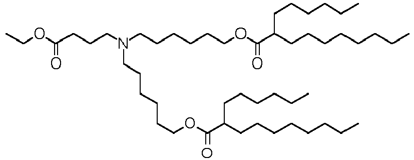

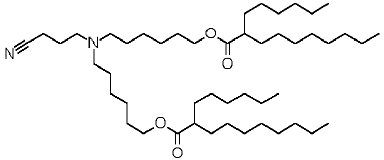

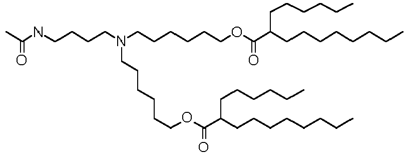

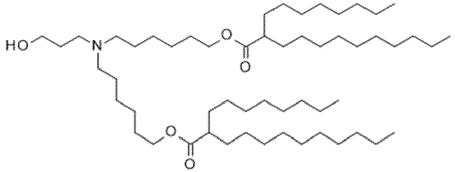

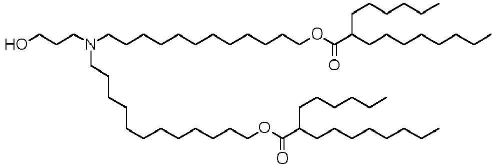

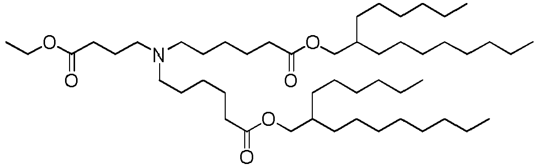

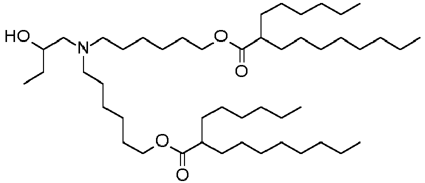

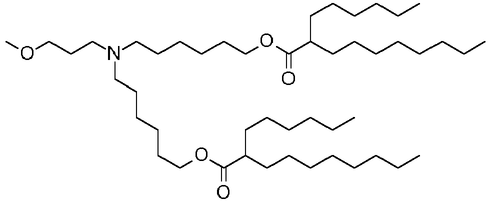

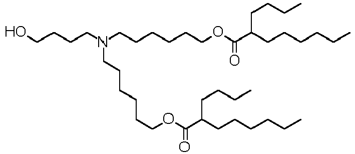

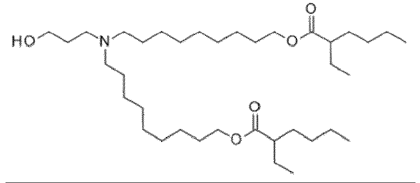

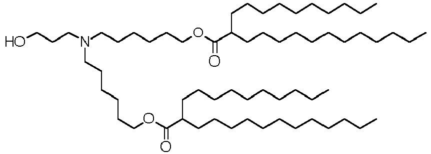

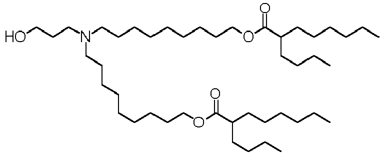

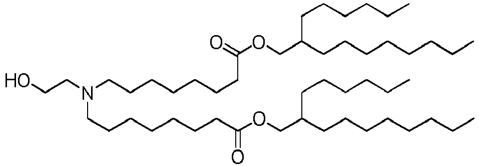

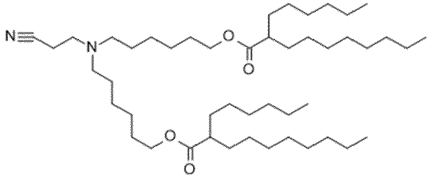

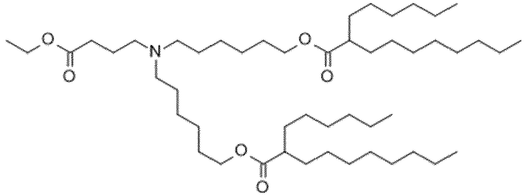

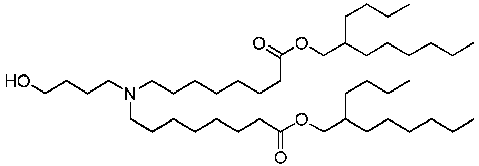

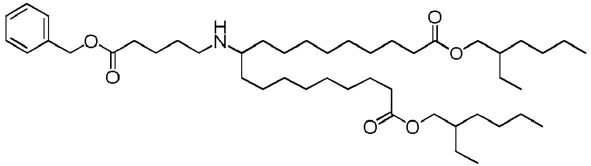

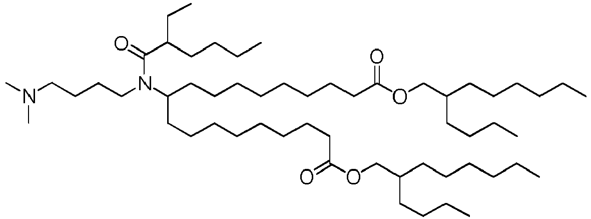

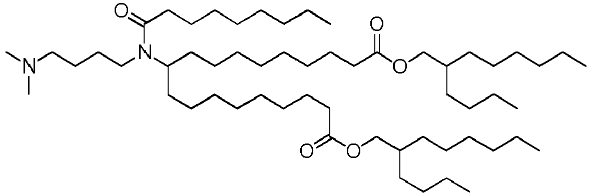

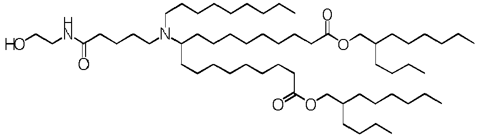

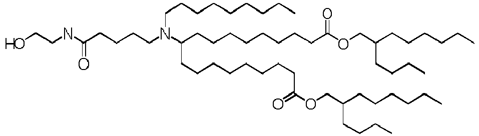

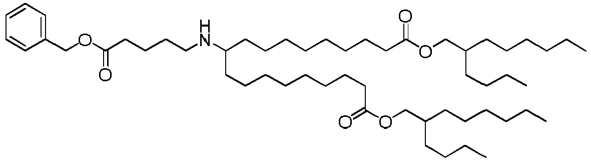

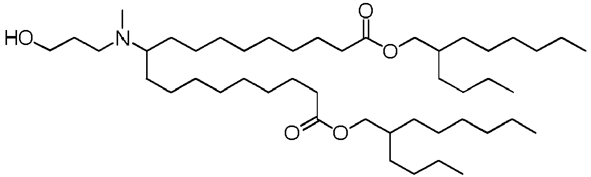

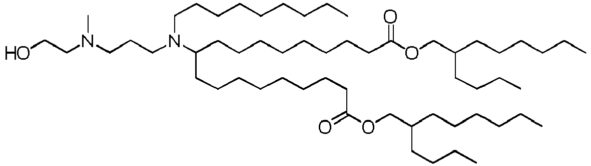

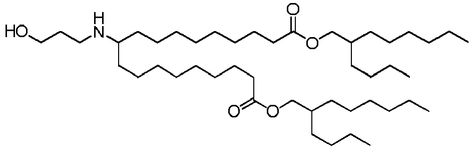

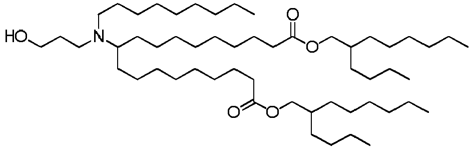

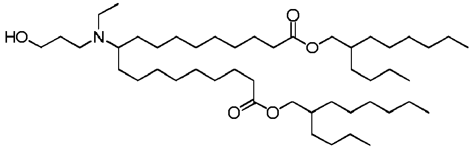

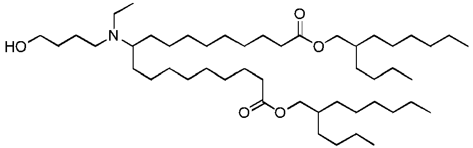

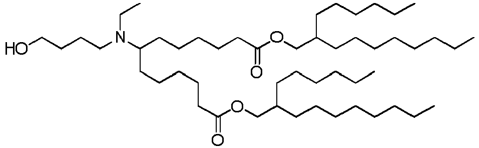

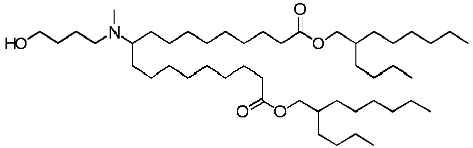

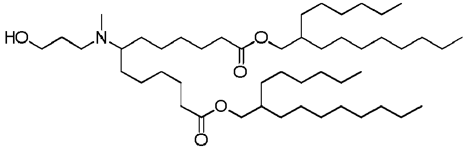

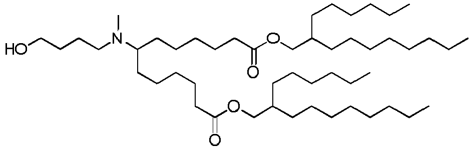

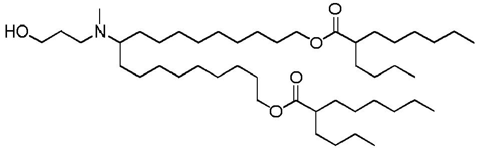

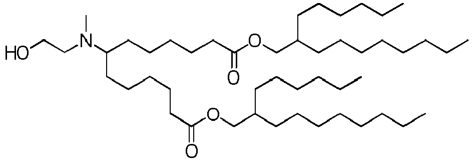

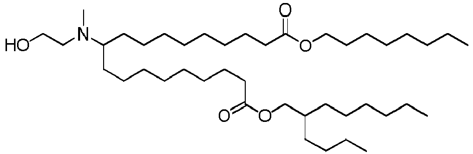

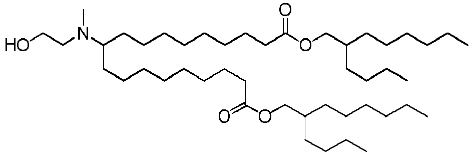

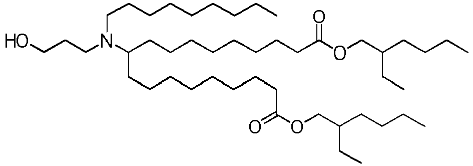

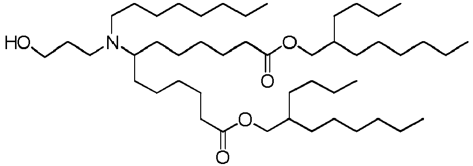

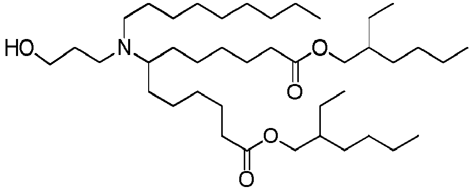

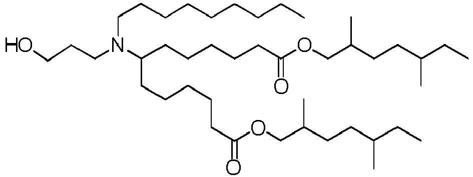

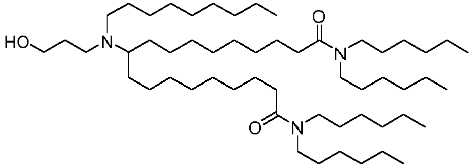

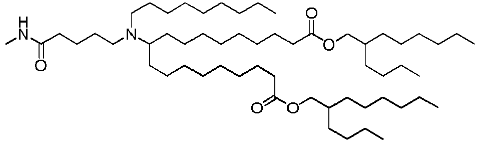

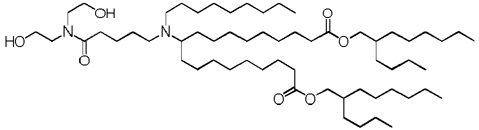

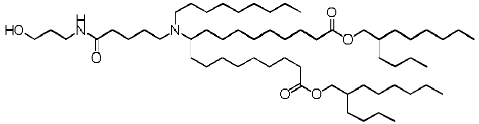

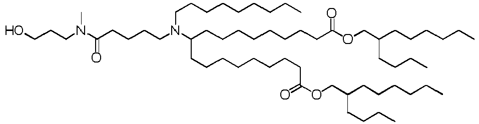

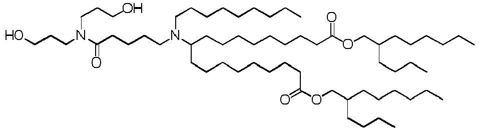

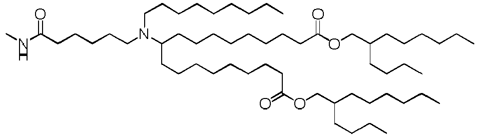

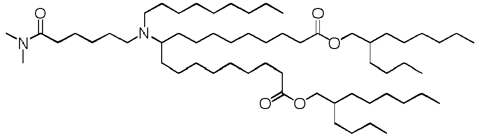

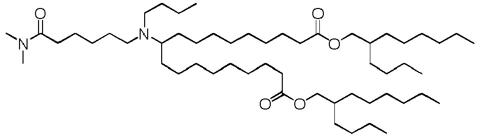

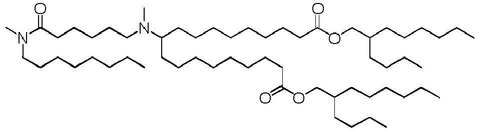

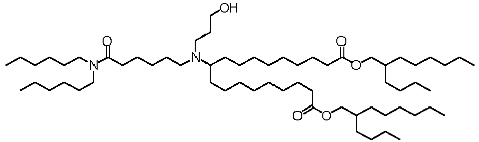

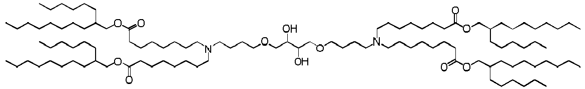

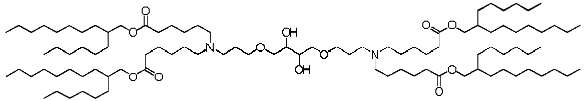

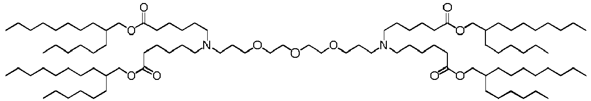

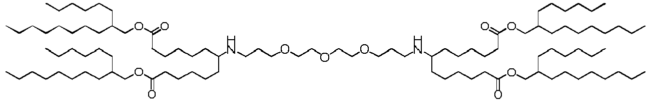

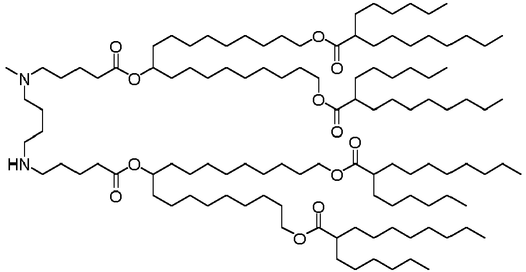

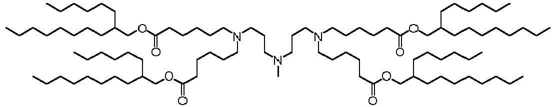

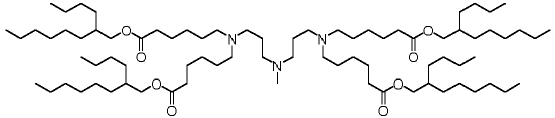

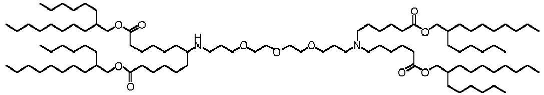

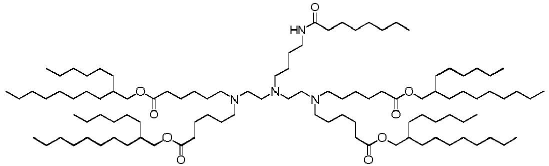

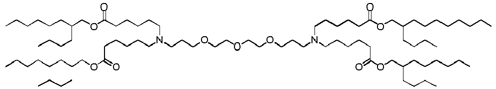

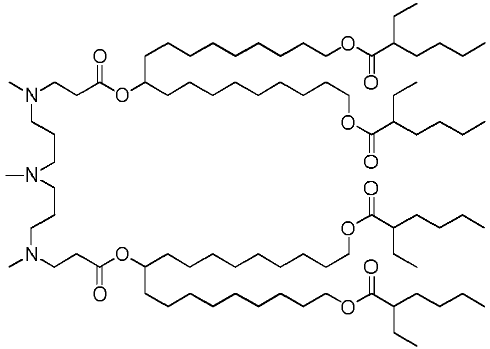

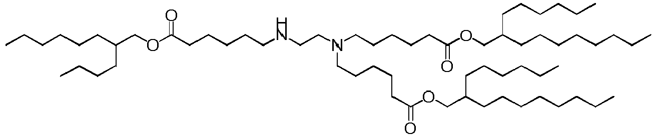

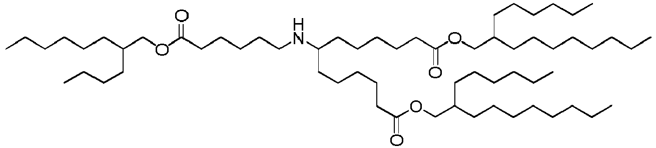

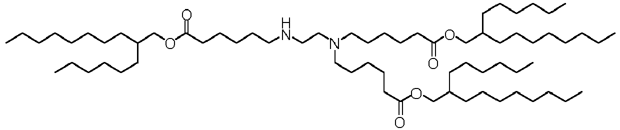

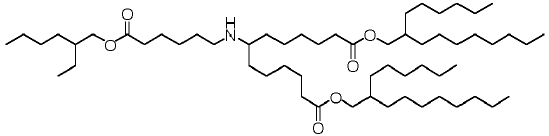

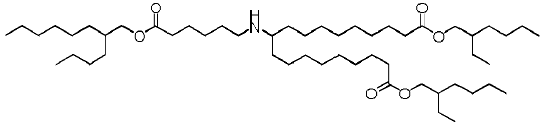

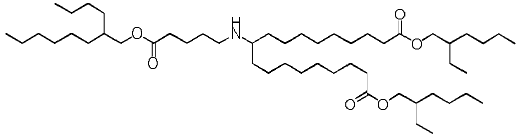

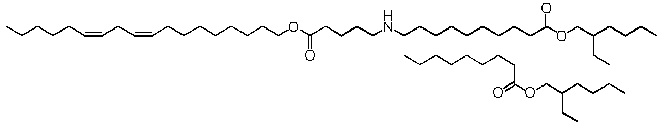

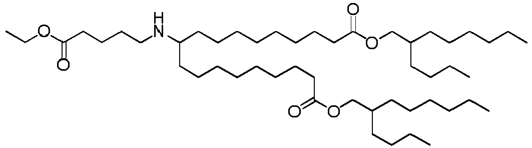

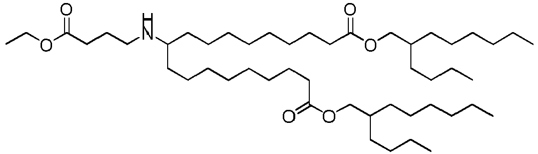

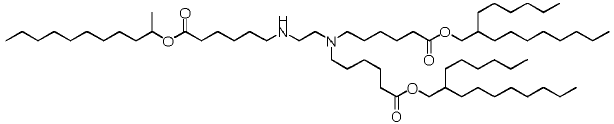

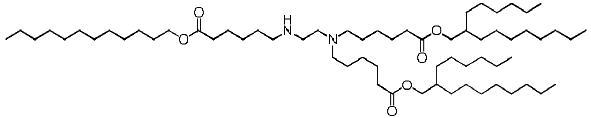

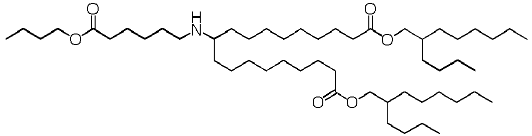

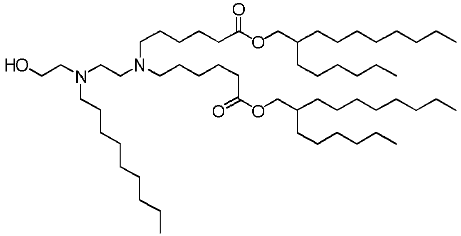

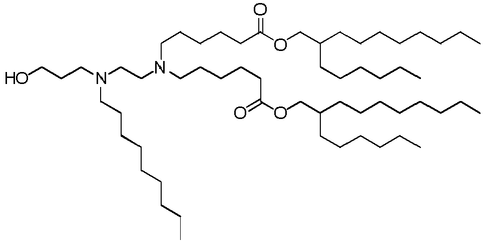

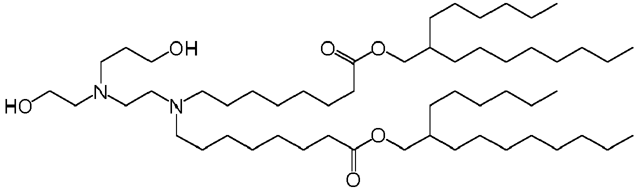

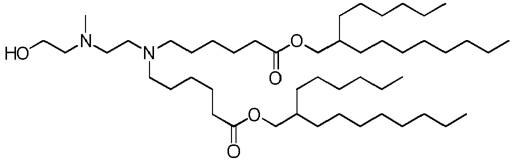

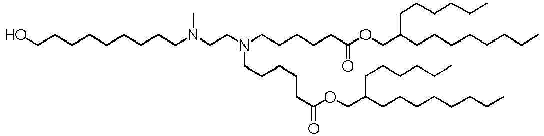

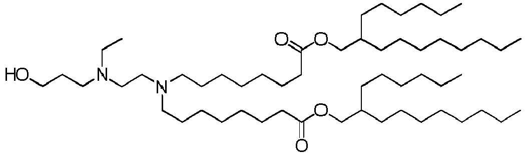

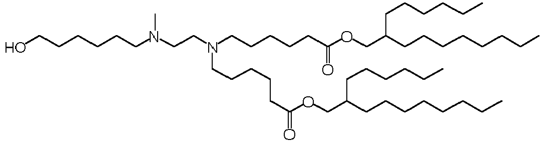

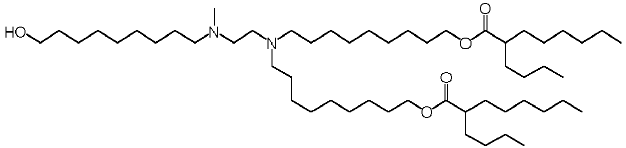

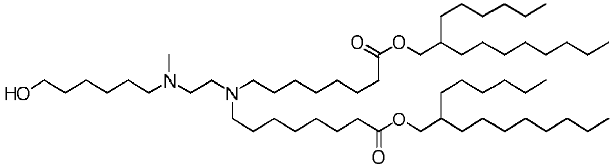

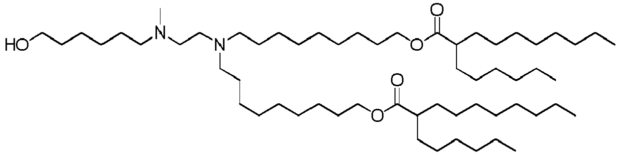

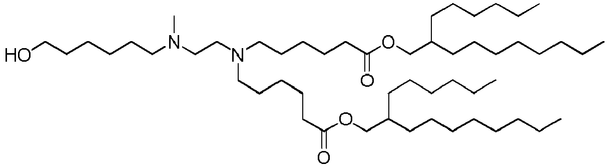

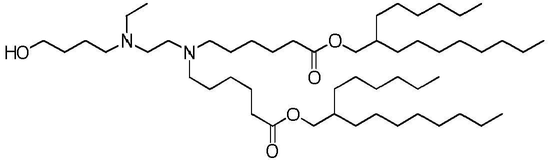

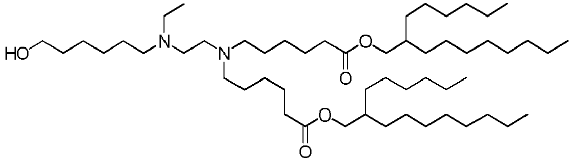

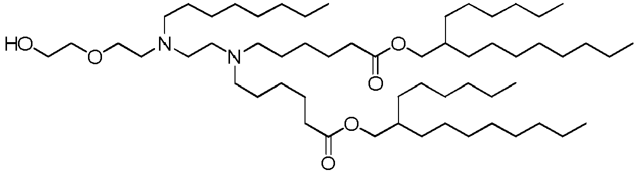

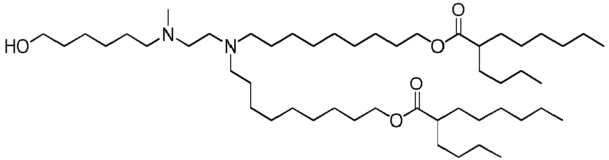

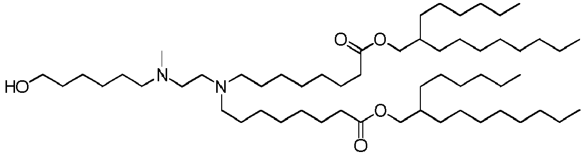

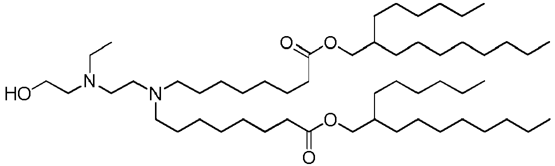

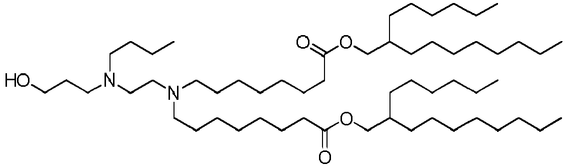

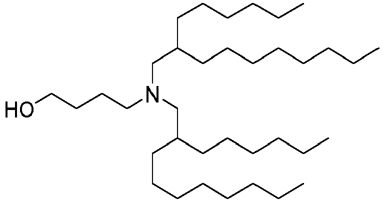

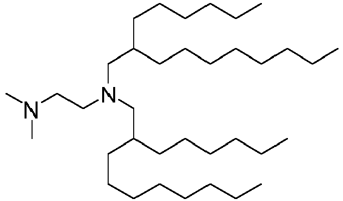

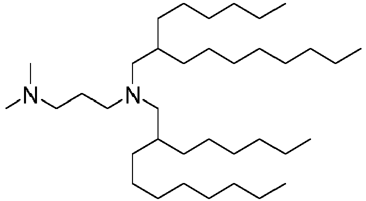

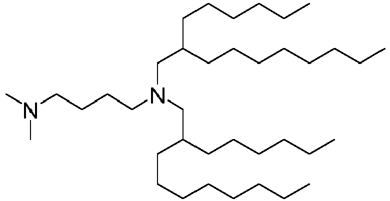

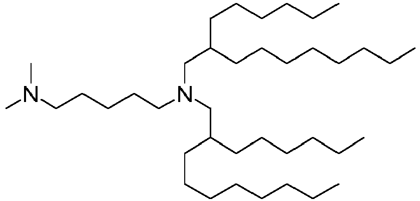

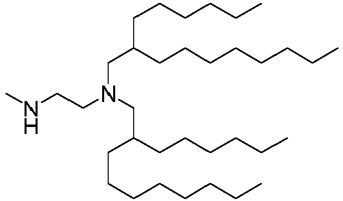

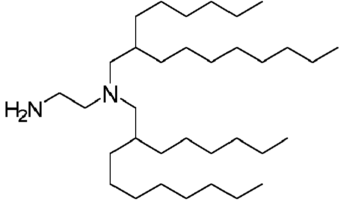

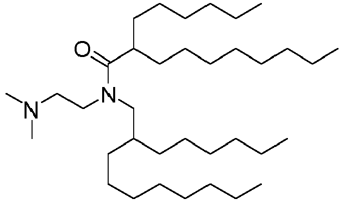

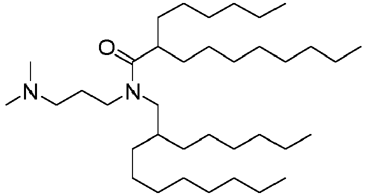

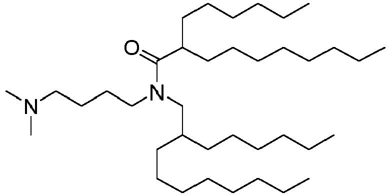

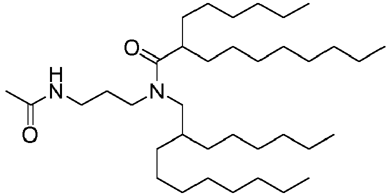

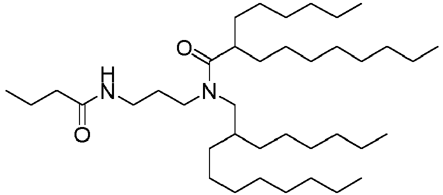

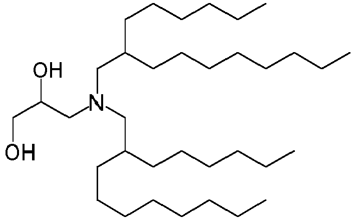

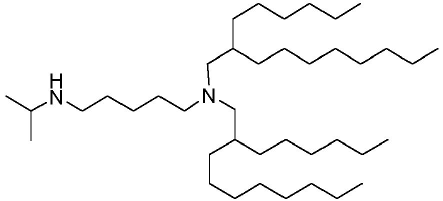

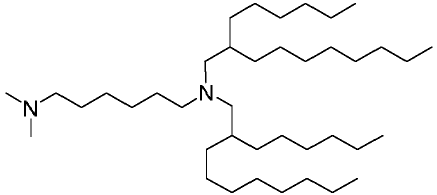

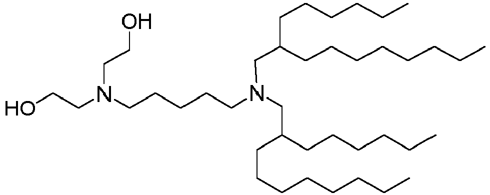

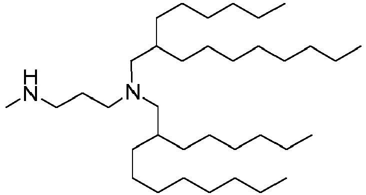

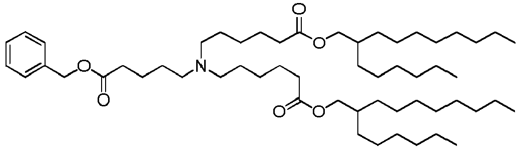

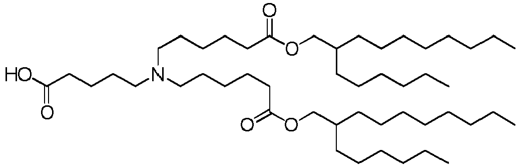

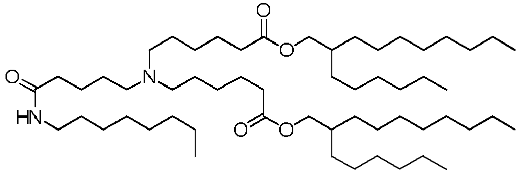

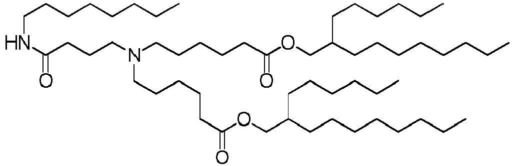

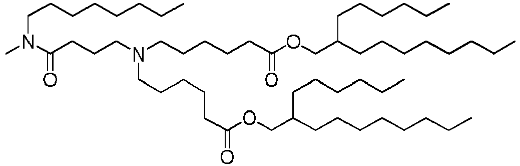

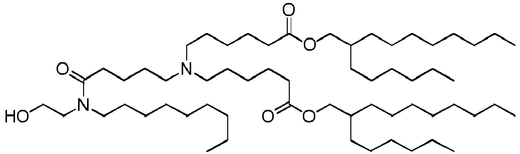

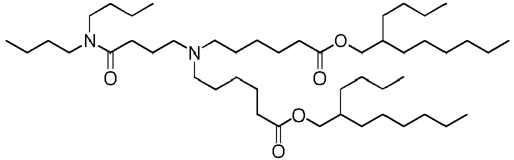

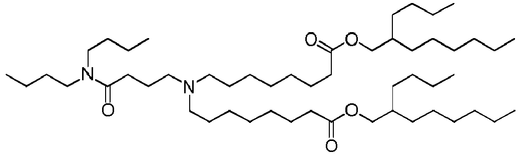

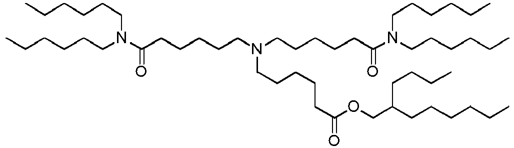

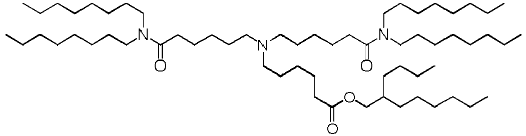

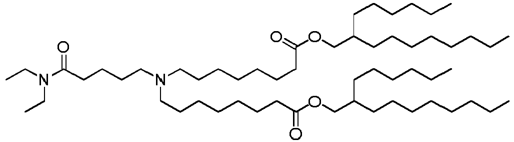

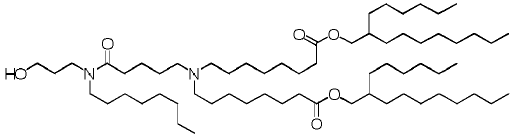

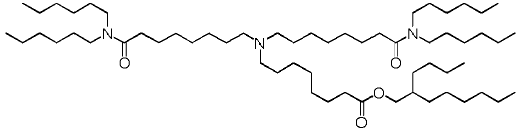

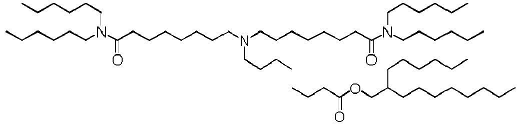

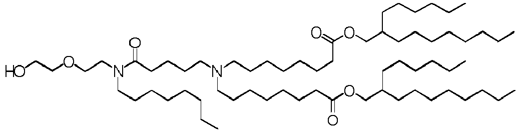

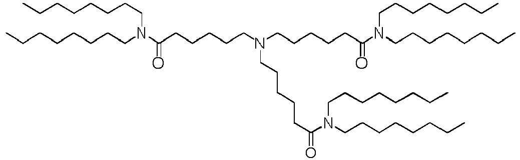

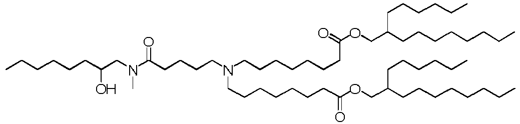

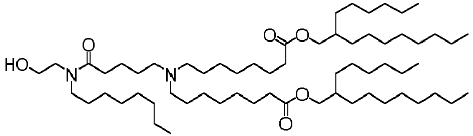

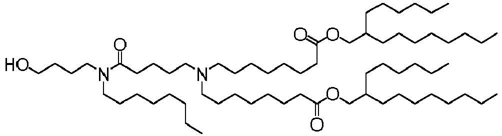

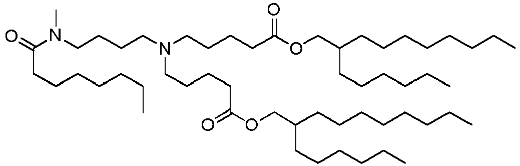

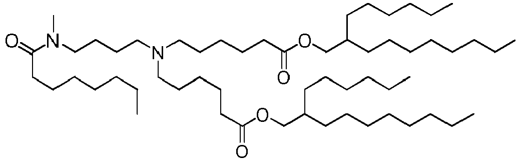

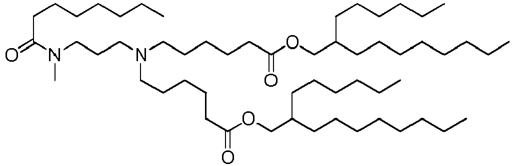

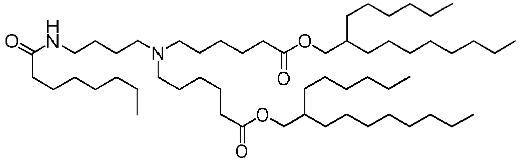

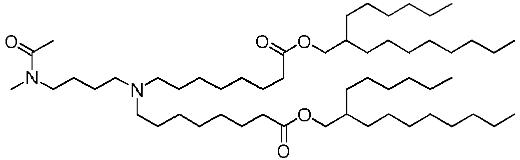

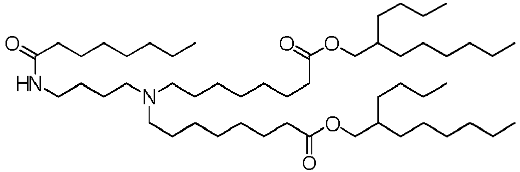

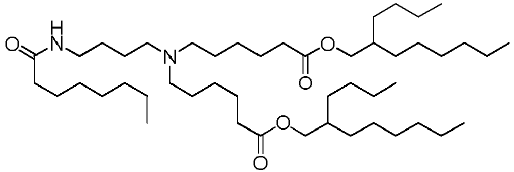

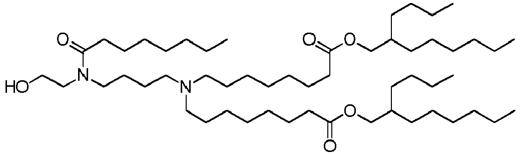

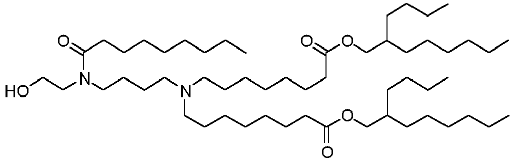

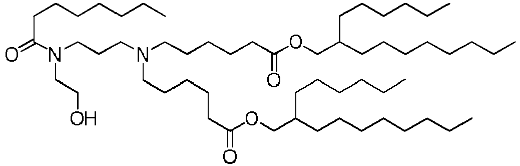

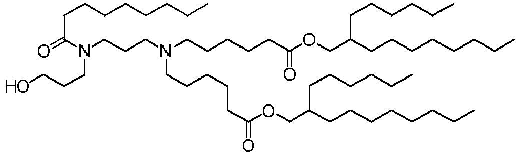

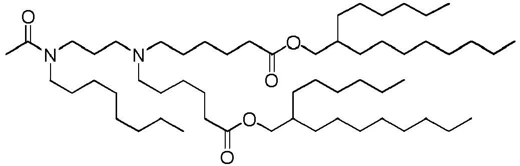

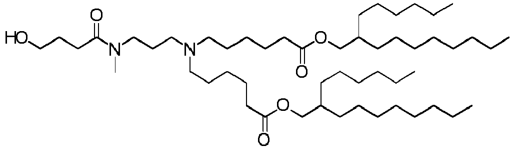

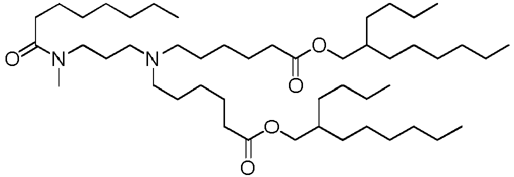

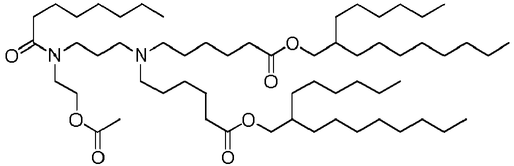

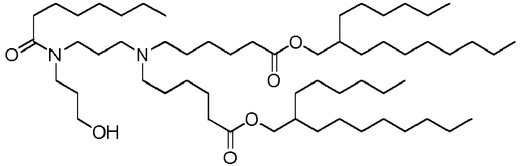

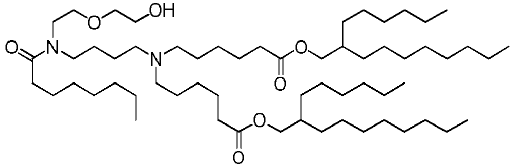

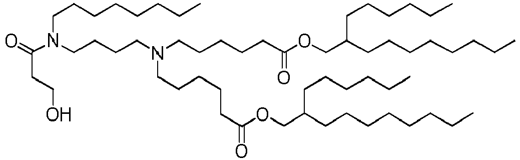

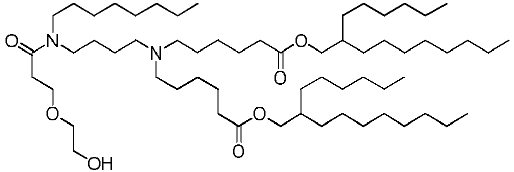

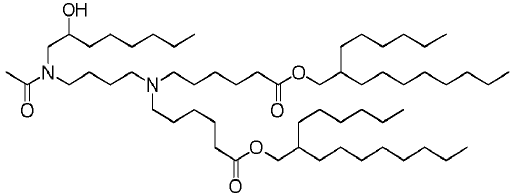

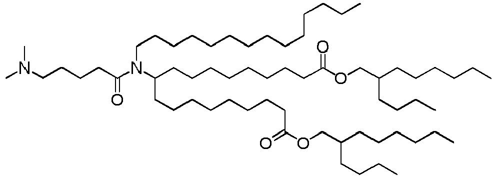

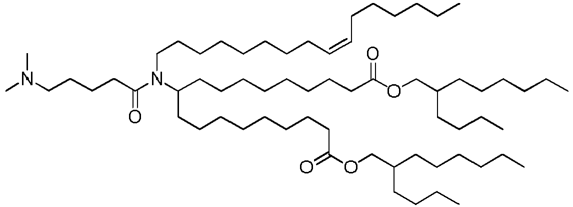

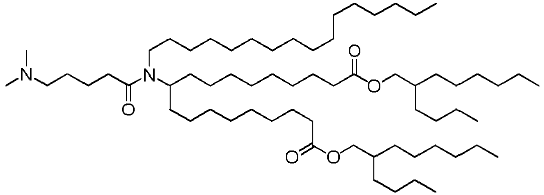

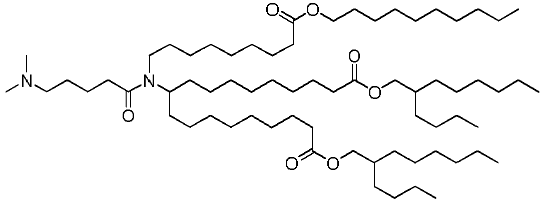

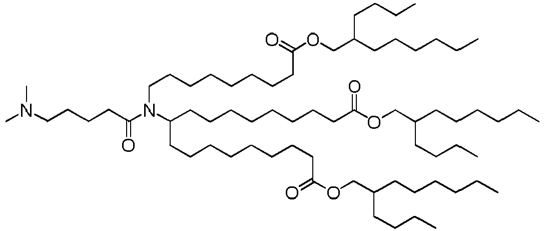

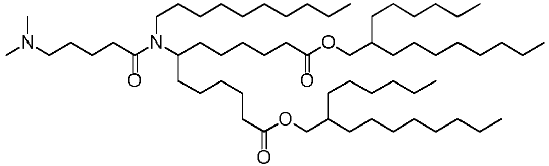

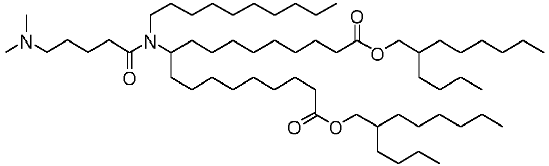

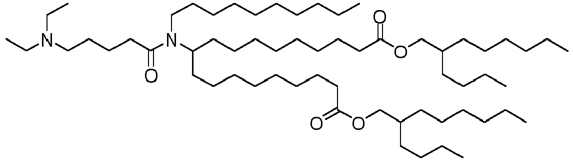

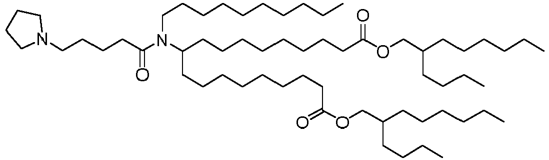

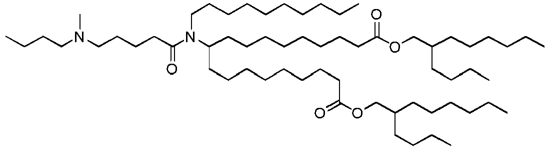

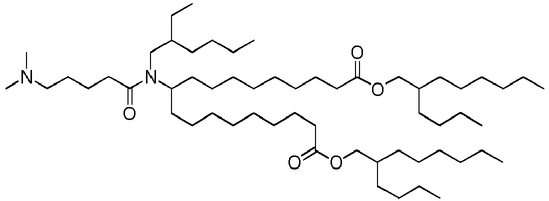

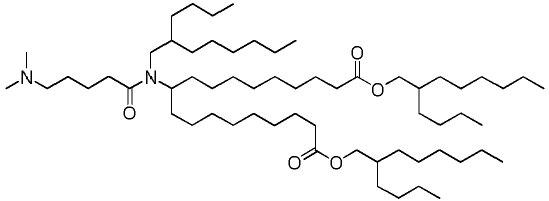

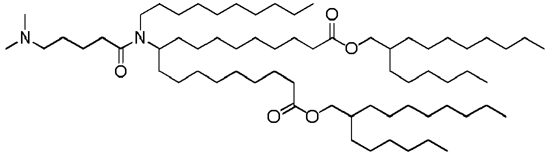

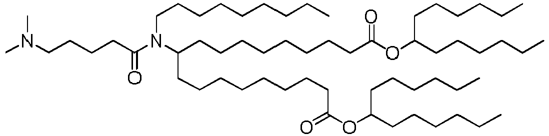

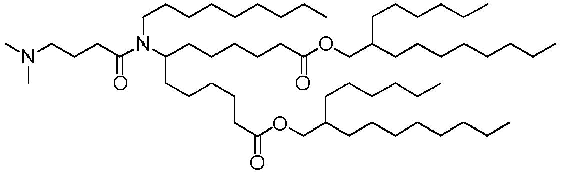

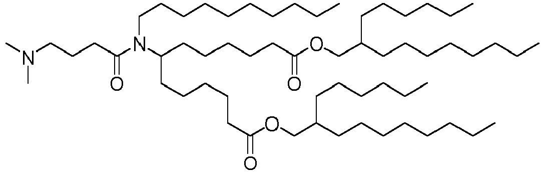

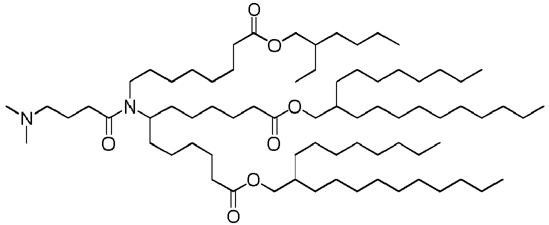

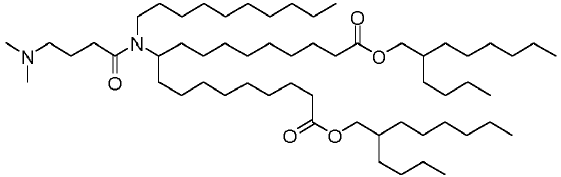

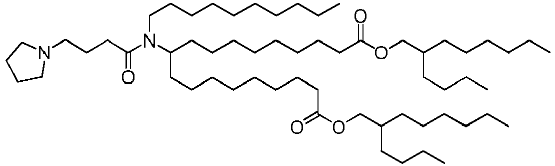

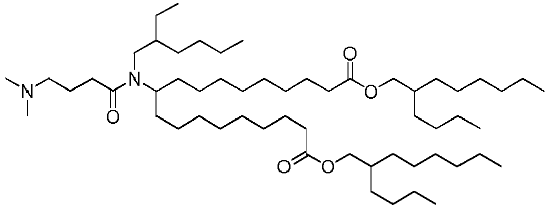

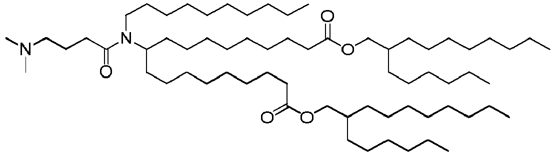

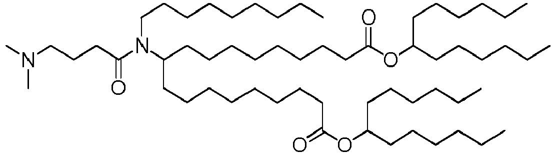

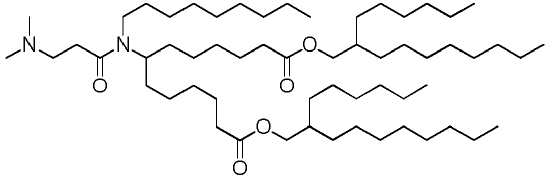

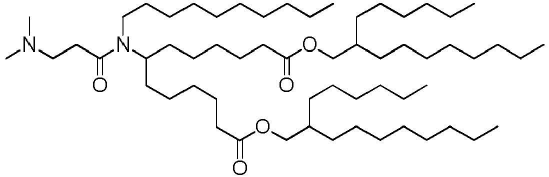

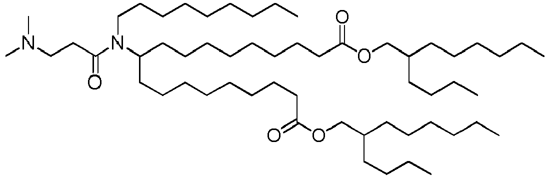

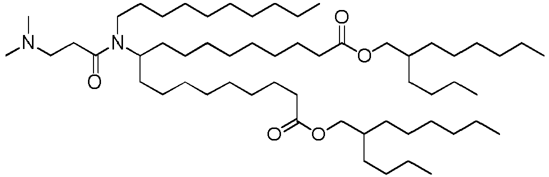

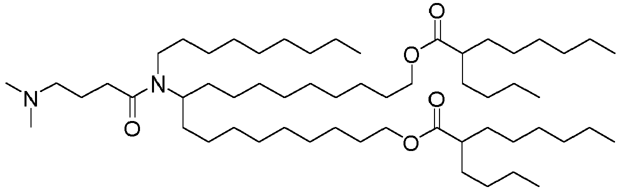

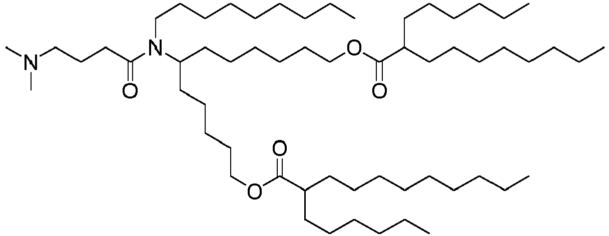

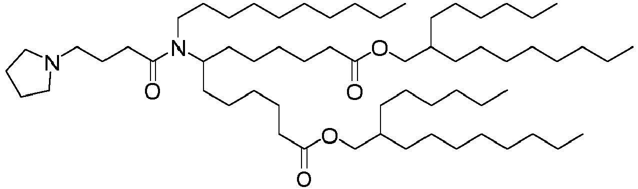

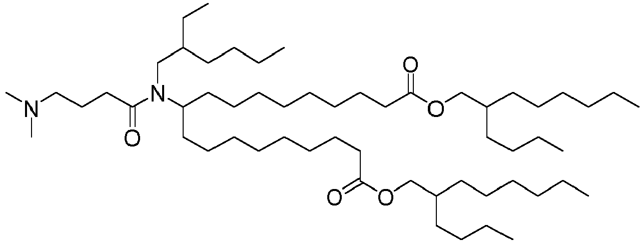

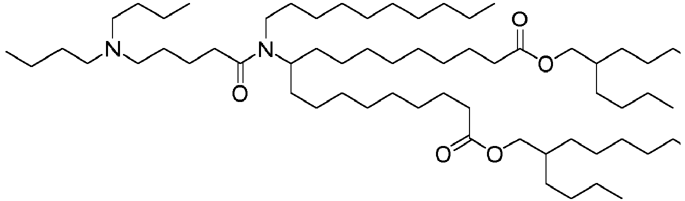

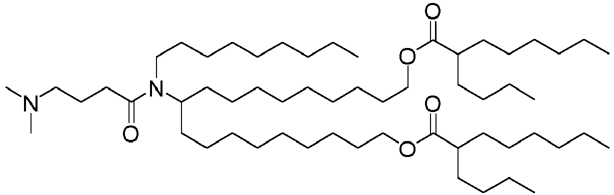

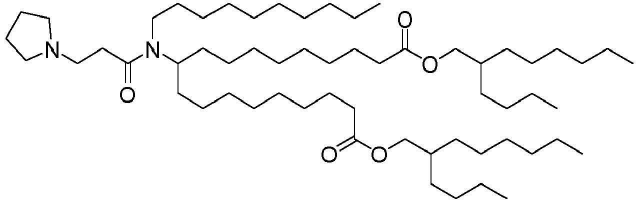

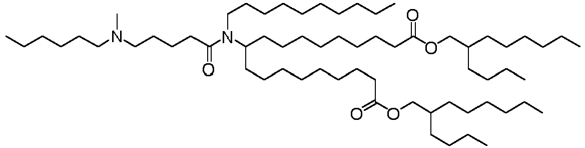

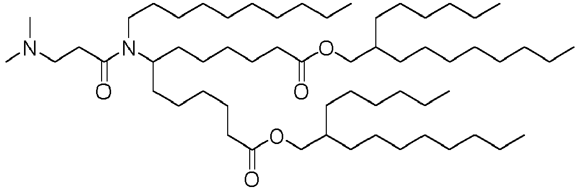

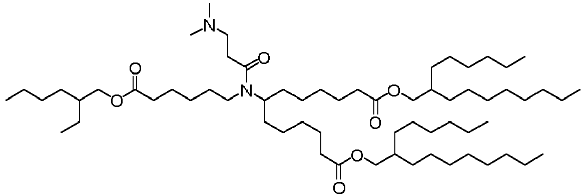

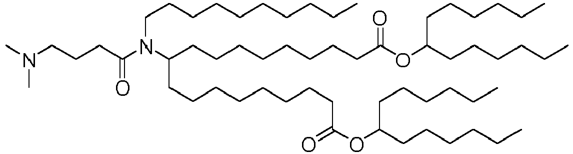

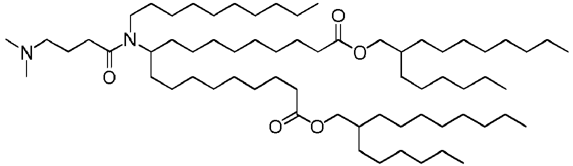

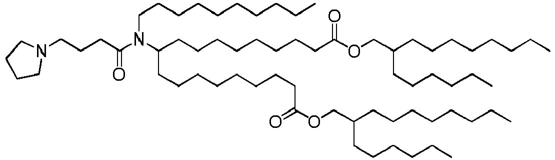

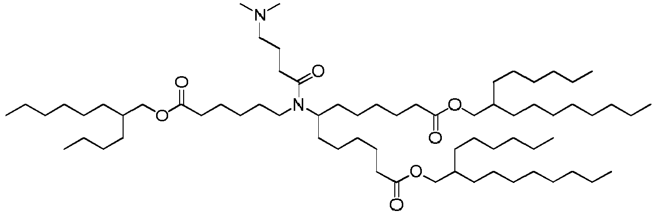

6. The LNPs for use according to any one of claims 1-5, wherein the molar ratio of cationic lipid to neutral lipid ranges from about 2:1 to about 8:1.

7. The LNPs for use according to any one of claims 1-6, wherein the neutral lipid is distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoyl-phosphatidylethanolamine (POPE) and dioleoyl-phosphatidylethanolamine 4-(N-maleimidomethyl)-cyclohexane-1 carboxylate (DOPE-mal), dipalmitoyl phosphatidyl ethanolamine (DPPE), dimyristoylphosphoethanolamine (DMPE), distearoyl-phosphatidylethanolamine (DSPE), 16-O-monomethyl PE, 16-O-dimethyl PE, 18-1-trans PE, 1-stearioyl-2-oleoylphosphatidyethanol amine (SOPE) or 1,2-dielaidoyl-sn-glycero-3-phophoethanolamine (transDOPE).

8. The LNPs for use according to any one of claims 1-6, wherein the neutral lipid is DSPC, DPPC, DMPC, DOPC, POPC, DOPE or SM.

9. The LNPs for use according to any one of claims 1-6, wherein the neutral lipid is DSPC.

10. The LNPs for use according to any one of claims 1-9, wherein the steroid is cholesterol.

11. The LNPs for use according to any one of claims 1-10, wherein the molar ratio of cationic lipid to steroid ranges from 5:1 to 1:1.

12. The LNPs for use according to any one of claims 1-11, wherein the molar ratio of cationic lipid to polymer conjugated lipid ranges from about 100:1 to about 20:1.

13. The LNPs for use according to any one of claims 1-12, wherein the nucleic acid is selected from antisense and messenger RNA.

14. The LNPs for use according to any one of claims 1-13, wherein the nucleic acid comprises an mRNA capable of translating an immunogenic protein.

15. The LNPs for use according to any one of claims 1-14, wherein the administering comprises intraveneously administering.

## Patentansprüche

1. Lipidnanopartikel (LNPs) zur Verwendung in einem Verfahren zur Behandlung oder Vorbeugung einer Krankheit bei einem Primaten, der diese benötigt, wobei das Verfahren die Verabreichung der LNPs an den Primaten umfasst, wobei jeder der LNPs umfasst:
i) eine Nukleinsäure oder ein pharmazeutisch akzeptables Salz davon, eingekapselt in den LNP;
ii) ein kationisches Lipid;
iii) ein neutrales Lipid;
iv) ein Steroid; und
v) ein polymer-konjugiertes Lipid,
wobei ein mittlerer Teilchendurchmesser der LNPs im Bereich von 40 nm bis 68 nm liegt, wie durch quasi-elastische Lichtstreuung bestimmt.

2. Die LNPs zur Verwendung gemäß Anspruch 1, wobei der mittlere Teilchendurchmesser im Bereich von 50 nm bis 68 nm, von 55 nm bis 65 nm, von 50 nm bis 60 nm oder von 60 nm bis 68 nm liegt.

3. Die LNPs zur Verwendung nach Anspruch 1, wobei der mittlere Teilchendurchmesser etwa 47 nm, etwa 48 nm, etwa 49 nm, etwa 50 nm, etwa 51 nm, etwa 52 nm, etwa 53 nm, etwa 54 nm, etwa 55 nm, etwa 56 nm, etwa 57 nm, etwa 58 nm, etwa 59 nm, etwa 60 nm, etwa 61 nm, etwa 62 nm, etwa 63 nm, etwa 64 nm oder etwa 65 nm beträgt.

4. Die LNPs zur Verwendung nach einem der Ansprüche 1-3, wobei:
i) das kationische Lipid eine Struktur der Formel (I) hat: 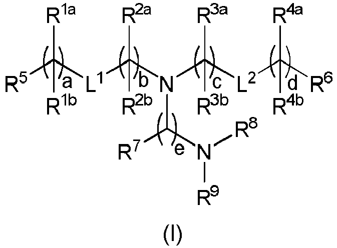 oder ein pharmazeutisch akzeptables Salz, Tautomer oder Stereoisomer davon, wobei:
eines von L¹ oder L² -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- oder -NR^{a}C(=O)O- ist, und das andere von L¹ oder L² -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- oder - NR^{a}C(=O)O- oder eine direkte Bindung ist;
R^{a} H oder C₁-C₁₂ Alkyl ist;
R^{1a} und R^{1b} bei jedem Auftreten unabhängig voneinander entweder (a) H oder C₁-C₁₂ Alkyl sind, oder (b) R^{1a} H oder C₁-C₁₂ Alkyl ist, und R^{1b} zusammen mit dem Kohlenstoffatom, an das es gebunden ist, zusammen mit einem benachbarten R^{1b} und dem Kohlenstoffatom, an das es gebunden ist, eine Kohlenstoff-Kohlenstoff-Doppelbindung bildet;
R^{2a} und R^{2b} bei jedem Auftreten unabhängig voneinander entweder (a) H oder C₁-C₁₂ Alkyl sind, oder (b) R^{2a} H oder C₁-C₁₂ Alkyl ist, und R^{2b} zusammen mit dem Kohlenstoffatom, an das es gebunden ist, zusammen mit einem benachbarten R^{2b} und dem Kohlenstoffatom, an das es gebunden ist, eine Kohlenstoff-Kohlenstoff-Doppelbindung bildet;
R^{3a} und R^{3b} bei jedem Auftreten unabhängig voneinander entweder (a) H oder C₁-C₁₂ Alkyl sind, oder (b) R^{3a} H oder C₁-C₁₂ Alkyl ist, und R^{3b} zusammen mit dem Kohlenstoffatom, an das es gebunden ist, zusammen mit einem benachbarten R^{3b} und dem Kohlenstoffatom, an das es gebunden ist, eine Kohlenstoff-Kohlenstoff-Doppelbindung bildet;
R^{4a} und R^{4b} bei jedem Auftreten unabhängig voneinander entweder (a) H oder C₁-C₁₂ Alkyl sind, oder (b) R^{4a} H oder C₁-C₁₂ Alkyl ist, und R^{4b} zusammen mit dem Kohlenstoffatom, an das es gebunden ist, zusammen mit einem benachbarten R^{4b} und dem Kohlenstoffatom, an das es gebunden ist, eine Kohlenstoff-Kohlenstoff-Doppelbindung bildet;
R⁵ und R⁶ jeweils unabhängig voneinander Methyl oder Cycloalkyl sind;
R⁷ bei jedem Auftreten unabhängig H oder C₁-C₁₂ Alkyl ist;
R⁸ und R⁹ jeweils unabhängig voneinander unsubstituiertes C₁-C₁₂ Alkyl sind; oder R⁸ und R⁹ zusammen mit dem Stickstoffatom, an das sie gebunden sind, einen 5-, 6- oder 7-gliedrigen heterocyclischen Ring bilden, der ein Stickstoffatom enthält;
a und d jeweils unabhängig voneinander eine ganze Zahl von 0 bis 24 sind;
b und c jeweils unabhängig voneinander eine ganze Zahl von 1 bis 24 sind;
e 1 oder 2 ist; und
x 0, 1 oder 2 ist; oder
ii) das kationische Lipid eine Struktur der Formel (II) hat: 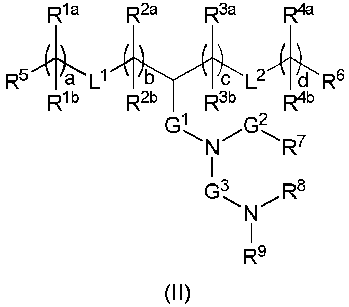 oder ein pharmazeutisch akzeptables Salz, Tautomer oder Stereoisomer davon, wobei:
eines von L¹ oder L² -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- oder -NR^{a}C(=O)O- ist, und das andere von L¹ oder L² -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- oder - NR^{a}C(=O)O- oder eine direkte Bindung ist;
G¹ C₁-C₂ Alkylen, -(C=O)-, -O(C=O)-, -SC(=O)-, -NR^{a}C(=O)- oder eine direkte Bindung ist;
G² -C(=O)-, -(C=O)O-, -C(=O)S-, -C(=O)NR^{a}- oder eine direkte Bindung ist;
G³ C₁-C₆ Alkylen ist;
R^{a} H oder C₁-C₁₂ Alkyl ist;
R^{1a} und R^{1b} bei jedem Auftreten unabhängig voneinander entweder: (a) H oder C₁-C₁₂ Alkyl sind; oder (b) R^{1a} H oder C₁-C₁₂ Alkyl ist, und R^{1b} zusammen mit dem Kohlenstoffatom, an das es gebunden ist, zusammen mit einem benachbarten R^{1b} und dem Kohlenstoffatom, an das es gebunden ist, eine Kohlenstoff-Kohlenstoff-Doppelbindung bildet;
R^{2a} und R^{2b} bei jedem Auftreten unabhängig voneinander entweder: (a) H oder C₁-C₁₂ Alkyl sind; oder (b) R^{2a} H oder C₁-C₁₂ Alkyl ist, und R^{2b} zusammen mit dem Kohlenstoffatom, an das es gebunden ist, zusammen mit einem benachbarten R^{2b} und dem Kohlenstoffatom, an das es gebunden ist, eine Kohlenstoff-Kohlenstoff-Doppelbindung bildet;
R^{3a} und R^{3b} bei jedem Auftreten unabhängig voneinander entweder (a): H oder C₁-C₁₂ Alkyl sind; oder (b) R^{3a} H oder C₁-C₁₂ Alkyl ist, und R^{3b} zusammen mit dem Kohlenstoffatom, an das es gebunden ist, zusammen mit einem benachbarten R^{3b} und dem Kohlenstoffatom, an das es gebunden ist, eine Kohlenstoff-Kohlenstoff-Doppelbindung bildet;
R^{4a} und R^{4b} bei jedem Auftreten unabhängig voneinander entweder: (a) H oder C₁-C₁₂ Alkyl sind; oder (b) R^{4a} H oder C₁-C₁₂ Alkyl ist, und R^{4b} zusammen mit dem Kohlenstoffatom, an das es gebunden ist, zusammen mit einem benachbarten R^{4b} und dem Kohlenstoffatom, an das es gebunden ist, eine Kohlenstoff-Kohlenstoff-Doppelbindung bildet;
R⁵ und R⁶ jeweils unabhängig voneinander H oder Methyl sind;
R⁷ C₄-C₂₀ Alkyl ist;
R⁸ und R⁹ jeweils unabhängig voneinander C₁-C₁₂ Alkyl sind; oder R⁸ und R⁹ zusammen mit dem Stickstoffatom, an das sie gebunden sind, einen 5-, 6- oder 7-gliedrigen heterocyclischen Ring bilden;
a, b, c und d jeweils unabhängig voneinander eine ganze Zahl von 1 bis 24 sind; und
x 0, 1 oder 2 ist; oder
iii) das kationische Lipid eine Struktur der Formel III hat: 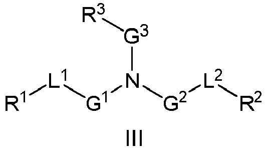 oder ein pharmazeutisch akzeptables Salz oder Stereoisomer davon, wobei:
eines von L¹ oder L² -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- oder -NR^{a}C(=O)O- ist, und das andere von L¹ oder L² -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- oder - NR^{a}C(=O)O- oder eine direkte Bindung ist;
G¹ und G² jeweils unabhängig voneinander unsubstituiertes C₁-C₁₂ Alkylen oder C₁ -C₁₂ Alkenylen sind;
G³ C₁-C₂₄ Alkylen, C₁-C₂₄ Alkenylen, C₃-C₈ Cycloalkylen, C₃-C₈ Cycloalkenylen ist;
R^{a} H oder C₁-C₁₂ Alkyl ist;
R¹ und R² jeweils unabhängig voneinander C₆-C₂₄ Alkyl oder C₆-C₂₄ Alkenyl sind;
R³ H, OR⁵, CN, -C(=O)OR⁴-, -OC(=O)R⁴ oder -NR⁵C(=O)R⁴ ist;
R⁴ C₁-C₁₂ Alkyl ist;
R⁵ H oder C₁-C₆ Alkyl ist; und
x 0, 1 oder 2 ist; oder
iv) das kationische Lipid die folgende Formel (IV) hat: 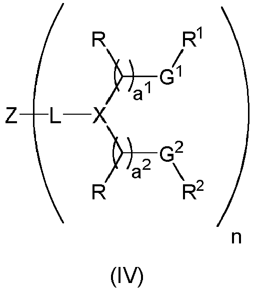 oder ein pharmazeutisch akzeptables Salz oder Stereoisomer davon, wobei:
eines von G¹ oder G² bei jedem Auftreten -O(C=O)-, -(C=O)O-, -C(=O)-, -O-, -S(O)_{y}-, -S-S-, -C(=O)S-, SC(=O)-, -N(R^{a})C(=O)-, -C(=O)N(R^{a})-, -N(R^{a})C(=O)N(R^{a})-, -OC(=O)N(R^{a})- oder -N(R^{a})C(=O)O ist, und das andere von G¹ oder G² bei jedem Auftreten -O(C=O)-, -(C=O)O-, -C(=O)-, -O-, -S(O)_{y}-, -S-S-, -C(=O)S-, -SC(=O)-, -N(R^{a})C(=O)-, -C(=O)N(R^{a})-, -N(R^{a})C(=O)N(R^{a})-, -OC(=O)N(R^{a})- oder -N(R^{a})C(=O)O- oder eine direkte Bindung ist;
L bei jedem Auftreten ~O(C=O)- ist, wobei ~ eine kovalente Bindung zu X darstellt;
X CR^{a} ist;
Z Alkyl, Cycloalkyl oder eine monovalente Einheit ist, die mindestens eine polare funktionelle Gruppe umfasst, wenn n 1 ist; oder Z Alkylen, Cycloalkylen oder eine polyvalente Einheit ist, die mindestens eine polare funktionelle Gruppe umfasst, wenn n größer als 1 ist;
R^{a} bei jedem Auftreten unabhängig H, C₁-C₁₂ Alkyl, C₁-C₁₂ Hydroxylalkyl, C₁-C₁₂ Aminoalkyl, C₁-C₁₂ Alkylaminylalkyl, C₁-C₁₂ Alkoxyalkyl, C₁-C₁₂ Alkoxycarbonyl, C₁-C₁₂ Alkylcarbonyloxy, C₁-C₁₂ Alkylcarbonyloxyalkyl oder C₁-C₁₂ Alkylcarbonyl ist;
R bei jedem Auftreten unabhängig entweder: (a) H oder C₁ -C₁₂ Alkyl ist; oder (b) R zusammen mit dem Kohlenstoffatom, an das es gebunden ist, zusammen mit einem benachbarten R und dem Kohlenstoffatom, an das es gebunden ist, eine Kohlenstoff-Kohlenstoff-Doppelbindung bildet;
R¹ und R² bei jedem Vorkommen die folgende Struktur haben: 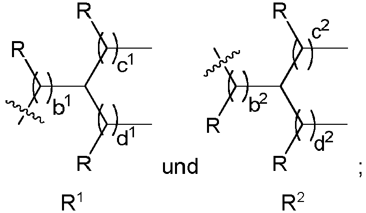
a¹ und a² bei jedem Auftreten unabhängig voneinander eine ganze Zahl von 3 bis 12 sind;
b¹ und b² bei jedem Auftreten unabhängig voneinander 0 oder 1 sind;
c¹ und c² bei jedem Auftreten unabhängig voneinander eine ganze Zahl von 5 bis 10 sind;
d¹ und d² bei jedem Auftreten unabhängig voneinander eine ganze Zahl von 5 bis 10 sind;
y bei jedem Auftreten unabhängig eine ganze Zahl von 0 bis 2 ist; und
n eine ganze Zahl von 1 bis 6 ist,
wobei jedes Alkyl, Alkylen, Hydroxylalkyl, Aminoalkyl, Alkylaminylalkyl, Alkoxyalkyl, Alkoxycarbonyl, Alkylcarbonyloxy, Alkylcarbonyloxyalkyl und Alkylcarbonyl gegebenenfalls mit einem oder mehreren Substituenten substituiert ist; oder
v) das kationische Lipid die folgende Formel (V) hat: 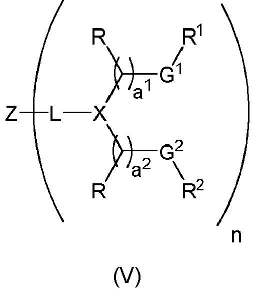 oder ein pharmazeutisch akzeptables Salz oder Stereoisomer davon, wobei:
eines von G¹ oder G² bei jedem Auftreten -O(C=O)-, -(C=O)O-, -C(=O)-, -O-, -S(O)_{y}-, -S-S-, -C(=O)S-, SC(=O)-, -N(R^{a})C(=O)-, -C(=O)N(R^{a})-, -N(R^{a})C(=O)N(R^{a})-, -OC(=O)N(R^{a})- oder -N(R^{a})C(=O)O ist, und das andere von G¹ oder G² bei jedem Auftreten -O(C=O)-, -(C=O)O-, -C(=O)-, -O-, -S(O)_{y}-, -S-S-, -C(=O)S-, -SC(=O)-, -N(R^{a})C(=O)-, -C(=O)N(R^{a})-, -N(R^{a})C(=O)N(R^{a})-, -OC(=O)N(R^{a})- oder -N(R^{a})C(=O)O- oder eine direkte Bindung ist;
L bei jedem Auftreten ~O(C=O)- ist, wobei ~ eine kovalente Bindung zu X darstellt;
X CR^{a} ist;
Z Alkyl, Cycloalkyl oder eine monovalente Einheit ist, die mindestens eine polare funktionelle Gruppe umfasst, wenn n 1 ist; oder Z Alkylen, Cycloalkylen oder eine polyvalente Einheit ist, die mindestens eine polare funktionelle Gruppe umfasst, wenn n größer als 1 ist;
R^{a} bei jedem Auftreten unabhängig H, C₁-C₁₂ Alkyl, C₁-C₁₂ Hydroxylalkyl, C₁-C₁₂ Aminoalkyl, C₁-C₁₂ Alkylaminylalkyl, C₁-C₁₂ Alkoxyalkyl, C₁-C₁₂ Alkoxycarbonyl, C₁-C₁₂ Alkylcarbonyloxy, C₁-C₁₂ Alkylcarbonyloxyalkyl oder C₁-C₁₂ Alkylcarbonyl ist;
R bei jedem Auftreten unabhängig entweder: (a) H oder C₁-C₁₂ Alkyl ist; oder (b) R zusammen mit dem Kohlenstoffatom, an das es gebunden ist, zusammen mit einem benachbarten R und dem Kohlenstoffatom, an das es gebunden ist, eine Kohlenstoff-Kohlenstoff-Doppelbindung bildet;
R¹ und R² bei jedem Vorkommen die folgende Struktur haben: 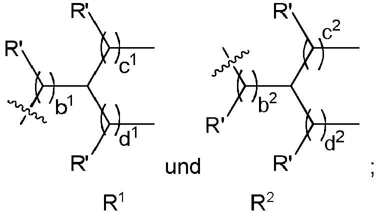
R' bei jedem Auftreten unabhängig H oder C₁-C₁₂ Alkyl ist;
a¹ und a² bei jedem Auftreten unabhängig voneinander eine ganze Zahl von 3 bis 12 sind;
b¹ und b² bei jedem Auftreten unabhängig voneinander 0 oder 1 sind;
c¹ und c² bei jedem Auftreten unabhängig voneinander eine ganze Zahl von 2 bis 12 sind;
d¹ und d² bei jedem Auftreten unabhängig voneinander eine ganze Zahl von 2 bis 12 sind;
y bei jedem Auftreten unabhängig eine ganze Zahl von 0 bis 2 ist; und
n eine ganze Zahl von 1 bis 6 ist,
wobei a¹, a², c¹, c², d¹ und d² so ausgewählt sind, dass die Summe von a¹+c¹+d¹ eine ganze Zahl von 18 bis 30 ist, und die Summe von a²+c²+d² eine ganze Zahl von 18 bis 30 ist, und wobei jedes Alkyl, Alkylen, Hydroxylalkyl, Aminoalkyl, Alkylaminylalkyl, Alkoxyalkyl, Alkoxycarbonyl, Alkylcarbonyloxy, Alkylcarbonyloxyalkyl und Alkylcarbonyl gegebenenfalls mit einem oder mehreren Substituenten substituiert ist; oder
vi) das kationische Lipid die folgende Formel (VI) hat: 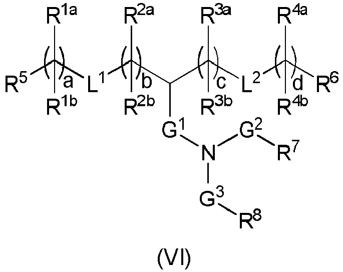 oder ein pharmazeutisch akzeptables Salz, Tautomer oder Stereoisomer davon, wobei:
L¹ und L² jeweils unabhängig voneinander -O(C=O)-, -(C=O)O-, -C(=O)-, -O-, -S(O)ₓ-, S-S-, -C(=O)S-, -SC(=O)-, -NR^{a}C(=O)-, -C(=O)NR^{a}-, -NR^{a}C(=O)NR^{a}-, -OC(=O)NR^{a}-, NR^{a}C(=O)O- oder eine direkte Bindung sind;
G¹ C₁-C₂ Alkylen, -(C=O)-, -O(C=O)-, -SC(=O)-, -NR^{a}C(=O)- oder eine direkte Bindung ist;
G² -C(=O)-, -(C=O)O-, -C(=O)S-, -C(=O)NR^{a}- oder eine direkte Bindung ist;
G³ C₁-C₆ Alkylen ist;
R^{a} H oder C₁-C₁₂ Alkyl ist;
R^{1a} und R^{1b} bei jedem Auftreten unabhängig voneinander entweder: (a) H oder C₁-C₁₂ Alkyl sind; oder (b) R^{1a} H oder C₁-C₁₂ Alkyl ist, und R^{1b} zusammen mit dem Kohlenstoffatom, an das es gebunden ist, zusammen mit einem benachbarten R^{1b} und dem Kohlenstoffatom, an das es gebunden ist, eine Kohlenstoff-Kohlenstoff-Doppelbindung bildet;
R^{2a} und R^{2b} bei jedem Auftreten unabhängig voneinander entweder: (a) H oder C₁-C₁₂ Alkyl sind; oder (b) R^{2a} H oder C₁-C₁₂ Alkyl ist, und R^{2b} zusammen mit dem Kohlenstoffatom, an das es gebunden ist, zusammen mit einem benachbarten R^{2b} und dem Kohlenstoffatom, an das es gebunden ist, eine Kohlenstoff-Kohlenstoff-Doppelbindung bildet;
R^{3a} und R^{3b} bei jedem Auftreten unabhängig voneinander entweder (a): H oder C₁-C₁₂ Alkyl sind; oder (b) R^{3a} H oder C₁-C₁₂ Alkyl ist, und R^{3b} zusammen mit dem Kohlenstoffatom, an das es gebunden ist, zusammen mit einem benachbarten R^{3b} und dem Kohlenstoffatom, an das es gebunden ist, eine Kohlenstoff-Kohlenstoff-Doppelbindung bildet;
R^{4a} und R^{4b} bei jedem Auftreten unabhängig voneinander entweder: (a) H oder C₁-C₁₂ Alkyl sind; oder (b) R^{4a} H oder C₁-C₁₂ Alkyl ist, und R^{4b} zusammen mit dem Kohlenstoffatom, an das es gebunden ist, zusammen mit einem benachbarten R^{4b} und dem Kohlenstoffatom, an das es gebunden ist, eine Kohlenstoff-Kohlenstoff-Doppelbindung bildet;
R⁵ und R⁶ jeweils unabhängig voneinander H oder Methyl sind;
R⁷ H oder C₁-C₂₀ Alkyl ist;
R⁸ OH, -N(R⁹)(C=O)R¹⁰, -(C=O)NR⁹R¹⁰, -NR⁹R¹⁰, -(C=O)OR¹¹ oder -O(C=O)R¹¹ ist, vorausgesetzt, dass G³ C₄-C₆ Alkylen ist, wenn R⁸ -NR⁹R¹⁰ ist,
R⁹ und R¹⁰ jeweils unabhängig voneinander H oder C₁-C₁₂ Alkyl sind;
R¹¹ Aralkyl ist;
a, b, c und d jeweils unabhängig voneinander eine ganze Zahl von 1 bis 24 sind; und
x 0, 1 oder 2 ist,
wobei jedes Alkyl, Alkylen und Aralkyl gegebenenfalls substituiert ist; oder
vii) das kationische Lipid die folgende Formel (VII) hat: 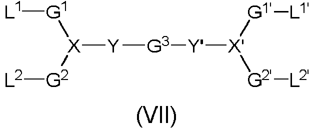 oder ein pharmazeutisch akzeptables Salz oder Stereoisomer davon, wobei:
X und X' jeweils unabhängig voneinander N oder CR sind;
Y und Y' jeweils unabhängig voneinander abwesend, -O(C=O)-, -(C=O)O- oder NR sind, vorausgesetzt, dass:
a) Y nicht vorhanden ist, wenn X N ist;
b) Y' nicht vorhanden ist, wenn X' N ist;
c) Y -O(C=O)-, -(C=O)O- oder NR ist, wenn X CR ist; und
d) Y' -O(C=O)-, -(C=O)O- oder NR ist, wenn X' CR ist,
L¹ und L¹ jeweils unabhängig voneinander-O(C=O)R1, -(C=O)OR¹, -C(=O)R¹, - OR¹, -S(O)_{z}R¹, -S-SR¹, -C(=O)SR¹, -SC(=O)R¹, -NR^{a}C(=O)R¹, -C(=O)NR^{b}R^{c}, -NR^{a}C(=O)NR^{b}R^{c}, -OC(=O)NR^{b}R^{c} oder -NR^{a}C(=O)OR¹ sind;
L² und L^{2'} jeweils unabhängig voneinander-O(C=O)R², -(C=O)OR², -C(=O)R², - OR², -S(O)_{z}R², -S-SR², -C(=O)SR², -SC(=O)R², -NR^{d}C(=O)R², -C(=O)NR^{e}R^{f}, -NR^{d}C(=O)NR^{e}R^{f}, - OC(=O)NR^{e}R^{f}; -NR^{d}C(=O)OR² oder eine direkte Bindung an R² sind;
G¹, G¹, G² und G^{2'} jeweils unabhängig voneinander C₂-C₁₂ Alkylen oder C₂-C₁₂ Alkenylen sind;
G³ C₂-C₂₄ Heteroalkylen oder C₂-C₂₄ Heteroalkenylen ist;
R^{a}, R^{b}, R^{d} und R^{e} bei jedem Auftreten unabhängig voneinander H, C₁-C₁₂ Alkyl oder C₂-C₁₂ Alkenyl sind;
R^{c} und R^{f} bei jedem Auftreten unabhängig voneinander C₁-C₁₂Alkyl oder C₂-C₁₂ Alkenyl sind;
R bei jedem Auftreten unabhängig H oder C₁-C₁₂ Alkyl ist;
R¹ und R² bei jedem Auftreten unabhängig voneinander verzweigtes C₆-C₂₄ Alkyl oder verzweigtes C₆-C₂₄ Alkenyl sind;
z 0, 1 oder 2 ist, und
wobei jedes Alkyl, Alkenyl, Alkylen, Alkenylen, Heteroalkylen und Heteroalkenylen unabhängig voneinander substituiert oder unsubstituiert ist, sofern nicht anders angegeben; oder
viii) das kationische Lipid die folgende Formel (VIII) hat: 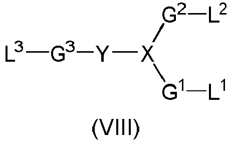 oder ein pharmazeutisch akzeptables Salz oder Stereoisomer davon, wobei:
X N ist, und Y abwesend ist; oder X CR ist, und Y NR ist;
L¹ -O(C=O)R¹, -(C=O)OR¹, -C(=O)R¹, -OR¹, -S(O)ₓR¹, -S-SR¹, -C(=O)SR¹, -SC(=O)R¹, -NR^{a}C(=O)R¹, -C(=O)NR^{b}R^{c}, -NR^{a}C(=O)NR^{b}R^{c}, -OC(=O)NR^{b}R^{c} oder -NR^{a}C(=O)OR¹ ist;
L² -O(C=O)R², -(C=O)OR², -C(=O)R², -OR², -S(O)ₓR², -S-SR², -C(=O)SR², -SC(=O)R², -NR^{d}C(=O)R², -C(=O)NR^{e}R^{f}, -NR^{d}C(=O)NR^{e}R^{f}, -OC(=O)NR^{e}R^{f}; -NR^{d}C(=O)OR² oder eine direkte Bindung an R² ist;
L³ -O(C=O)R³ oder -(C=O)OR³ ist;
G¹ und G² jeweils unabhängig voneinander C₂-C₁₂ Alkylen oder C₂-C₁₂ Alkenylen sind;
G³ C₁-C₂₄ Alkylen, C₂-C₂₄ Alkenylen, C₁-C₂₄ Heteroalkylen oder C₂-C₂₄ Heteroalkenylen ist, wenn X CR ist, und Y NR ist; und G³ C₁-C₂₄ Heteroalkylen oder C₂-C₂₄ Heteroalkenylen ist, wenn X N ist und Y fehlt;
R^{a}, R^{b}, R^{d} und R^{e} jeweils unabhängig voneinander H oder C₁-C₁₂ Alkyl oder C₁-C₁₂ Alkenyl sind;
R^{c} und R^{f} jeweils unabhängig voneinander C₁-C₁₂ Alkyl oder C₂-C₁₂ Alkenyl sind;
jedes R unabhängig H oder C₁-C₁₂ Alkyl ist;
R¹, R² und R³ jeweils unabhängig voneinander C₁-C₂₄ Alkyl oder C₂-C₂₄ Alkenyl sind; und
x 0, 1 oder 2 ist, und
wobei jedes Alkyl, Alkenyl, Alkylen, Alkenylen, Heteroalkylen und Heteroalkenylen unabhängig voneinander substituiert oder unsubstituiert ist, sofern nicht anders angegeben; oder
ix) das kationische Lipid die folgende Formel (IX) hat: 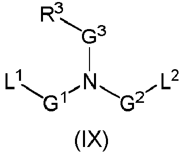 oder ein pharmazeutisch akzeptables Salz oder Stereoisomer davon, wobei:
L¹ -O(C=O)R¹, -(C=O)OR¹, -C(=O)R¹, -OR¹, -S(O)ₓR¹, -S-SR¹, -C(=O)SR¹, - SC(=O)R¹, -NR^{a}C(=O)R¹, -C(=O)NR^{b}R^{c}, -NR^{a}C(=O)NR^{b}R^{c}, -OC(=O)NR^{b}R^{c} oder-NR^{a}C(=O)OR¹ ist;
L² -O(C=O)R², -(C=O)OR², -C(=O)R², -OR², -S(O)ₓR², -S-SR², -C(=O)SR², - SC(=O)R², -NR^{d}C(=O)R², -C(=O)NR^{e}R^{f}, -NR^{d}C(=O)NR^{e}R^{f}, -OC(=O)NR^{e}R^{f}; -NR^{d}C(=O)OR² oder eine direkte Bindung an R² ist;
G¹ und G² jeweils unabhängig voneinander C₂-C₁₂ Alkylen oder C₂-C₁₂ Alkenylen sind;
G³ C₁-C₂₄ Alkylen, C₂-C₂₄ Alkenylen, C₃-C₈ Cycloalkylen oder C₃-C₈ Cycloalkenylen ist;
R^{a}, R^{b}, R^{d} und R^{e} jeweils unabhängig voneinander H oder C₁-C₁₂ Alkyl oder C₁-C₁₂ Alkenyl sind;
R^{c} und R^{f} jeweils unabhängig voneinander C₁-C₁₂ Alkyl oder C₂-C₁₂ Alkenyl sind;
R¹ und R² jeweils unabhängig voneinander verzweigtes C₆-C₂₄ Alkyl oder verzweigtes C₆-C₂₄ Alkenyl sind;
R³ -N(R⁴)R⁵ ist;
R⁴ C₁-C₁₂ Alkyl ist;
R⁵ substituiertes C₁-C₁₂ Alkyl ist; und
x 0, 1 oder 2 ist, und
wobei jedes Alkyl, Alkenyl, Alkylen, Alkenylen, Cycloalkylen, Cycloalkenylen, Aryl und Aralkyl unabhängig voneinander substituiert oder unsubstituiert ist, sofern nicht anders angegeben; oder
x) das kationische Lipid die folgende Formel (X) hat: 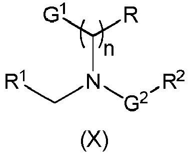 oder ein pharmazeutisch akzeptables Salz oder Stereoisomer davon, wobei:
G¹ -OH, -NR³R⁴, -(C=O)NR⁵ oder-NR³(C=O)R⁵ ist;
G² -CH₂- oder -(C=O)- ist;
R bei jedem Auftreten unabhängig H oder OH ist;
R¹ und R² jeweils unabhängig voneinander gegebenenfalls substituiertes verzweigtes, gesättigtes oder ungesättigtes C₁₂-C₃₆ Alkyl sind;
R³ und R⁴ jeweils unabhängig voneinander H oder gegebenenfalls substituiertes geradkettiges oder verzweigtes, gesättigtes oder ungesättigtes C₁-C₆ Alkyl sind;
R⁵ gegebenenfalls substituiertes geradkettiges oder verzweigtes, gesättigtes oder ungesättigtes C₁-C₆ Alkyl ist; und
n eine ganze Zahl von 2 bis 6 ist.

5. LNPs zur Verwendung nach einem der Ansprüche 1-4, wobei das kationische Lipid aus einer Lipidstruktur gebildet wird, die ausgewählt ist aus:

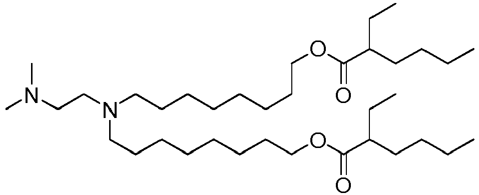

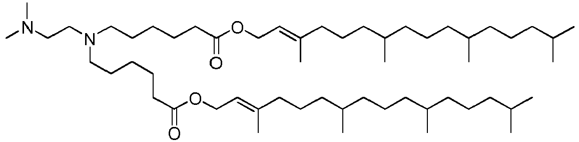

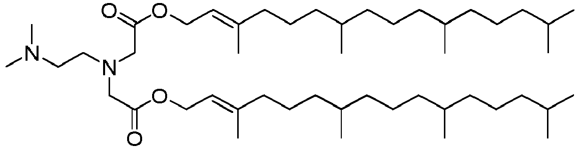

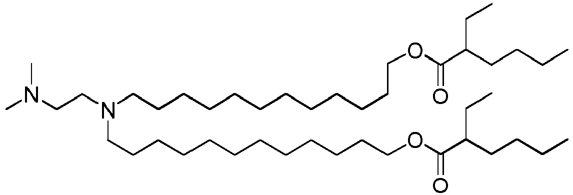

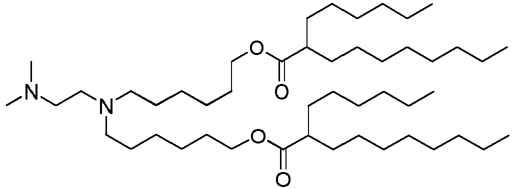

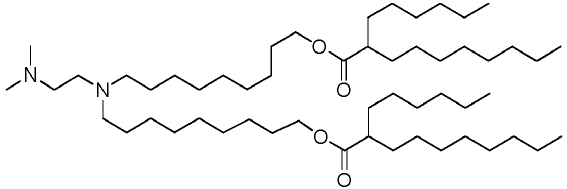

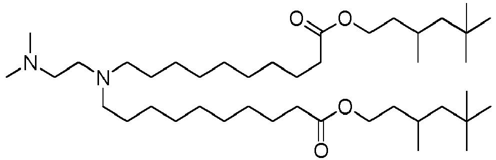

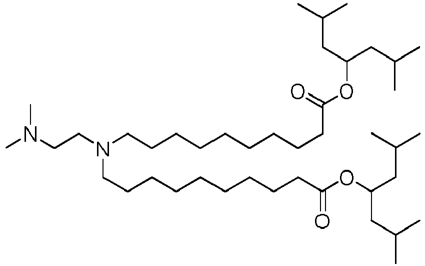

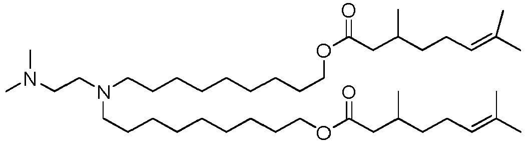

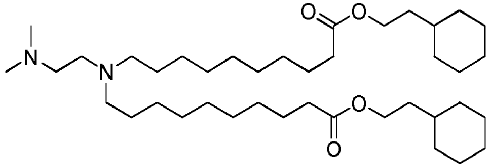

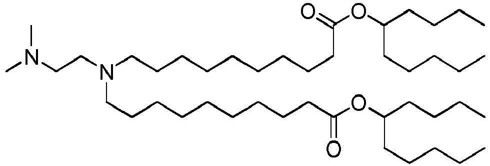

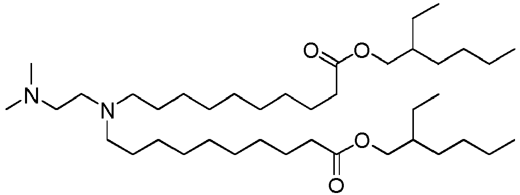

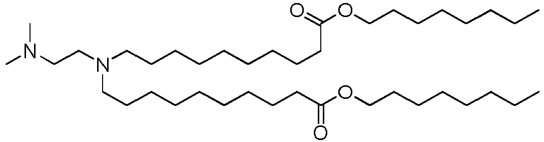

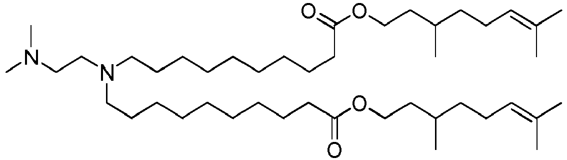

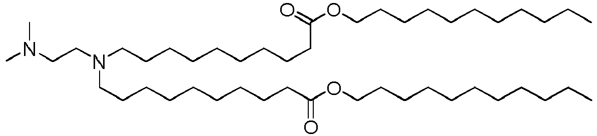

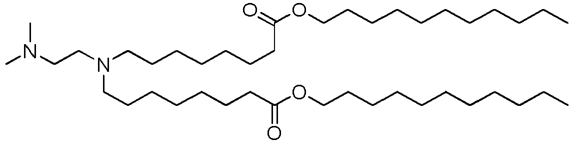

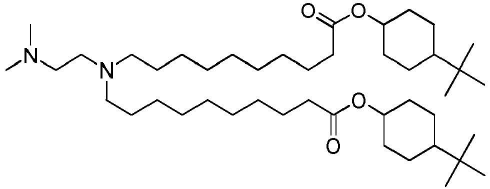

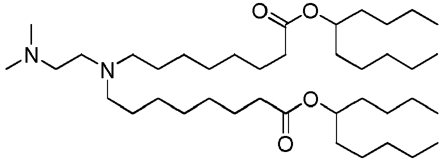

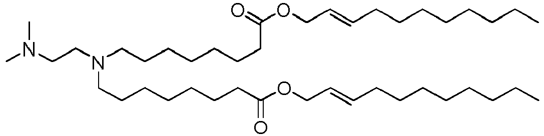

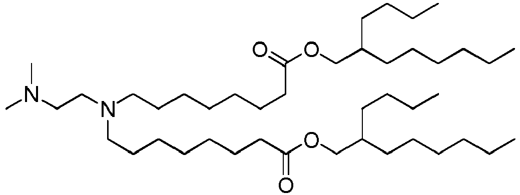

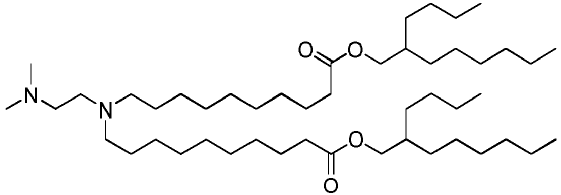

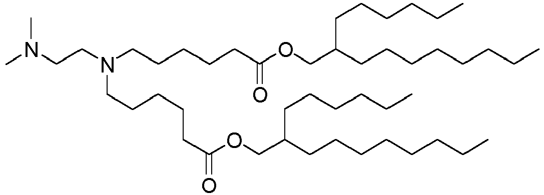

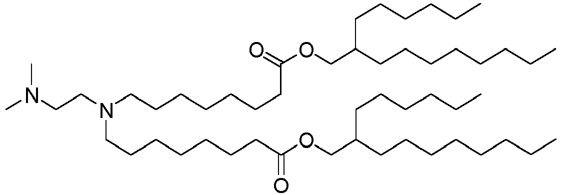

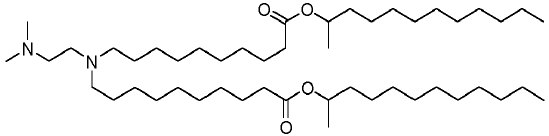

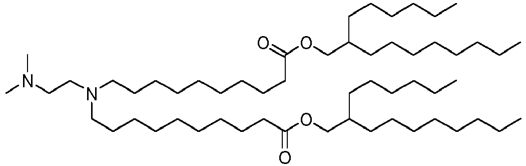

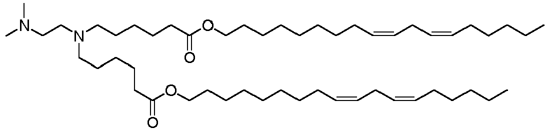

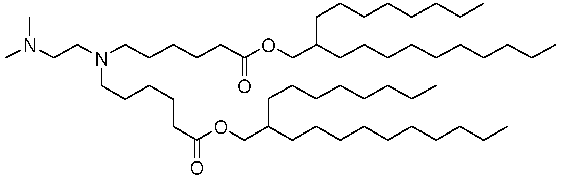

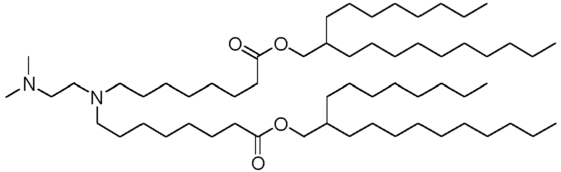

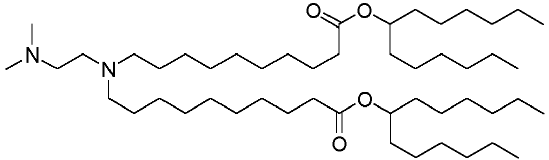

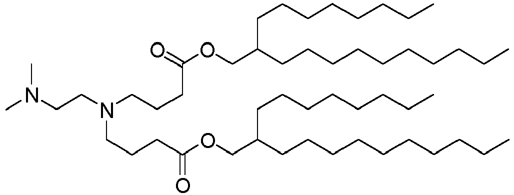

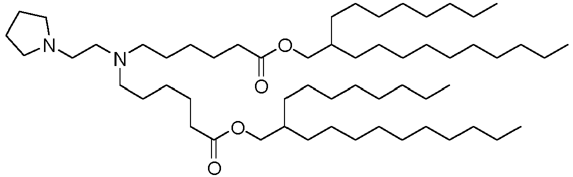

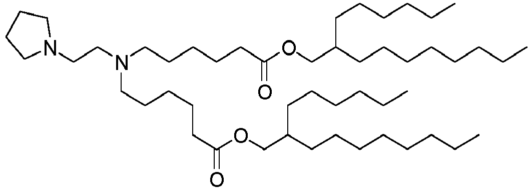

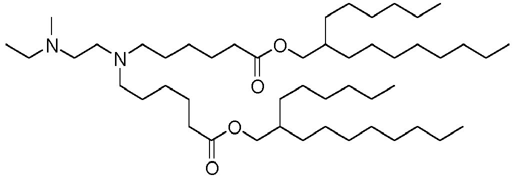

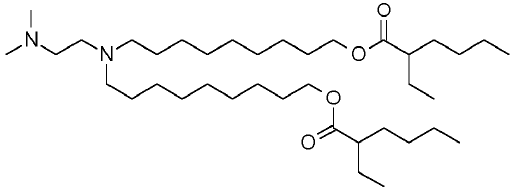

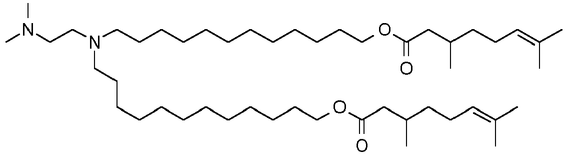

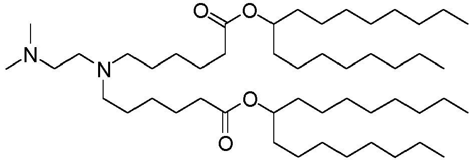

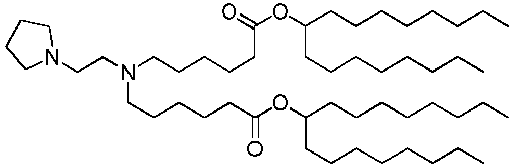

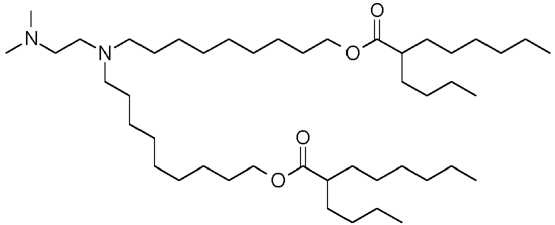

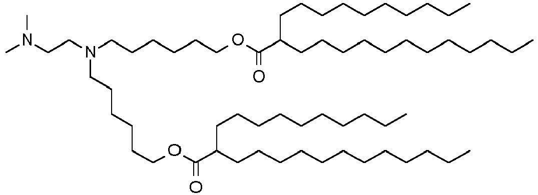

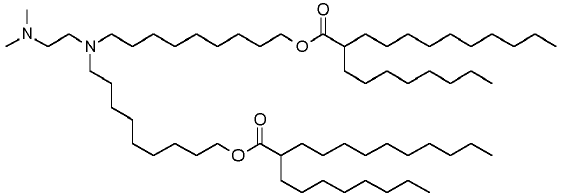

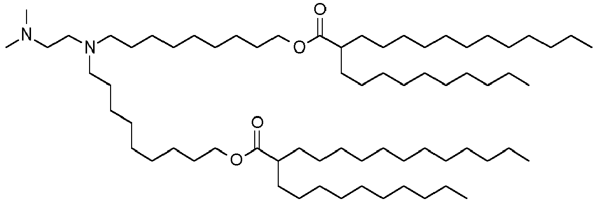

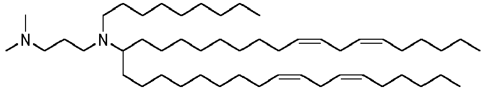

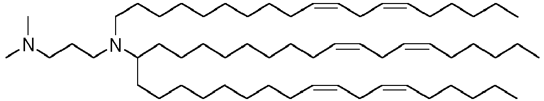

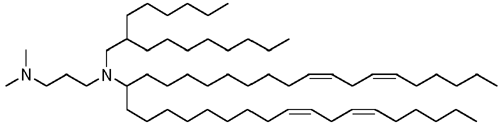

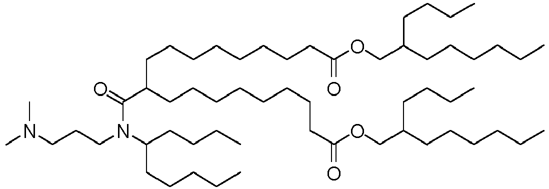

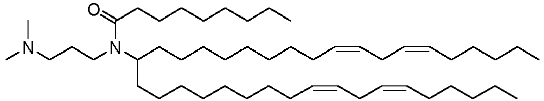

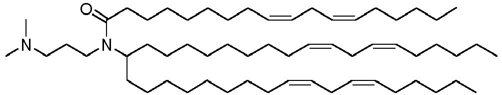

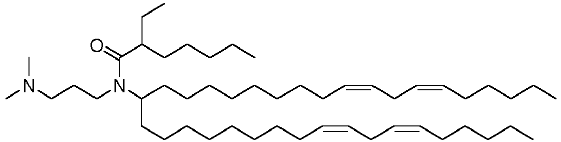

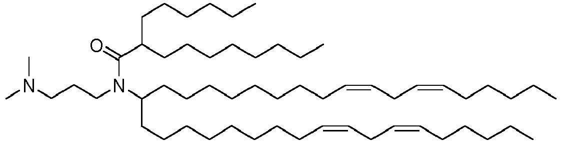

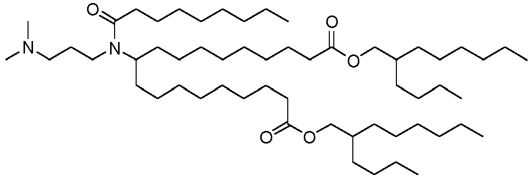

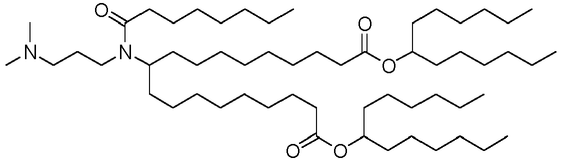

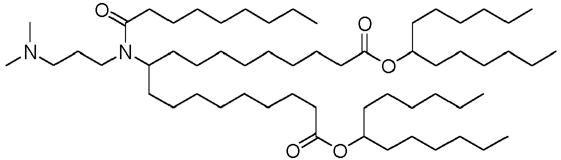

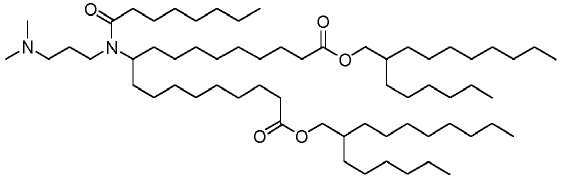

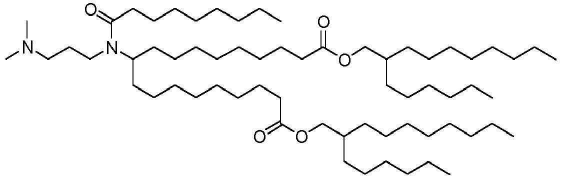

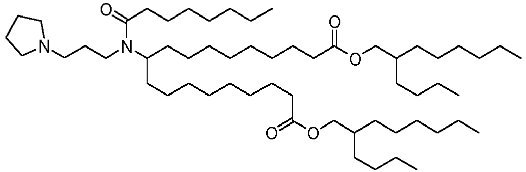

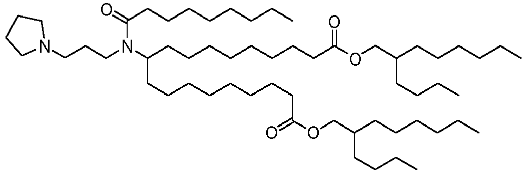

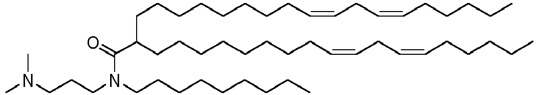

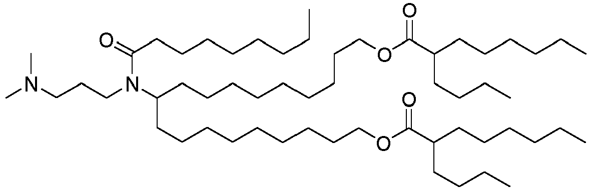

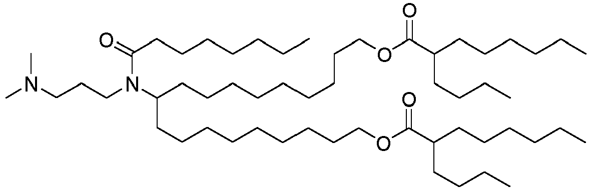

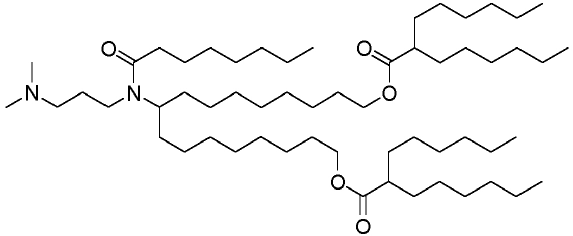

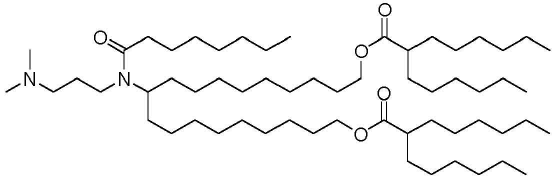

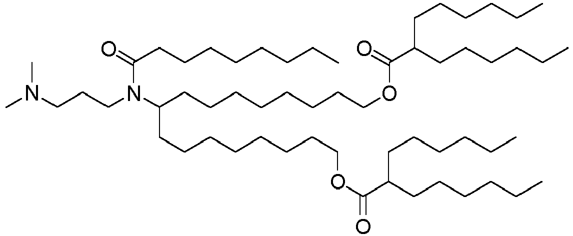

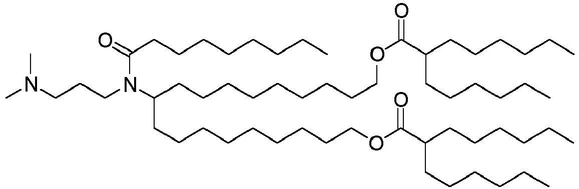

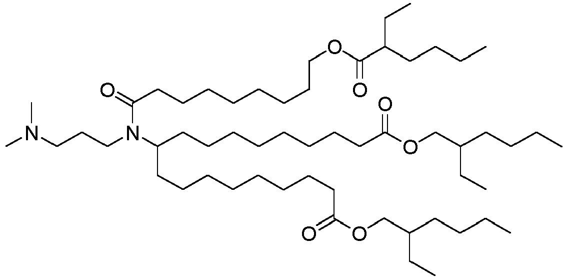

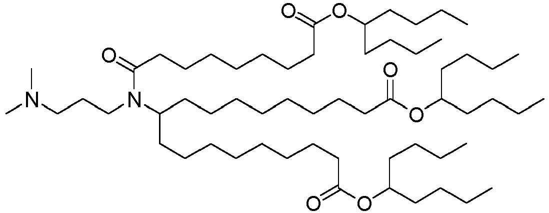

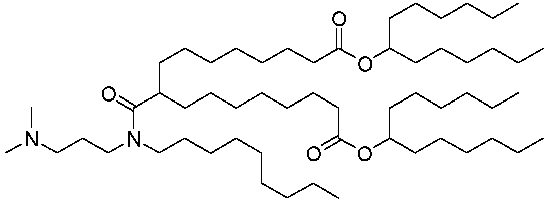

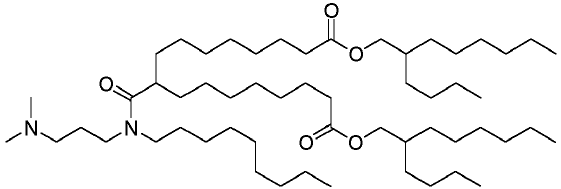

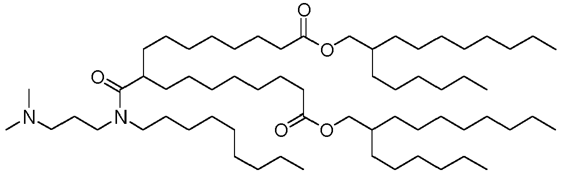

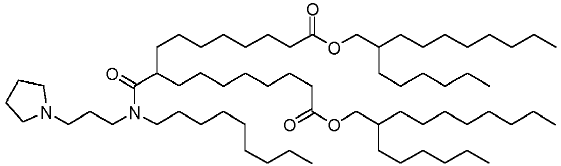

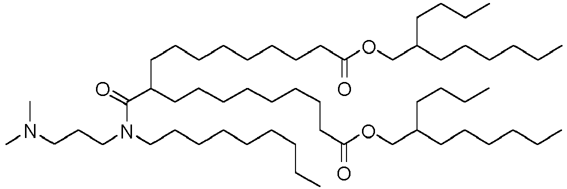

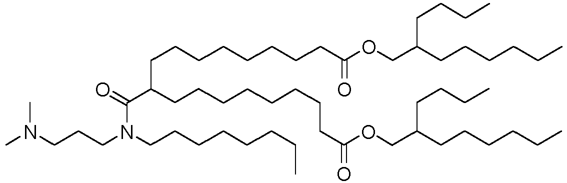

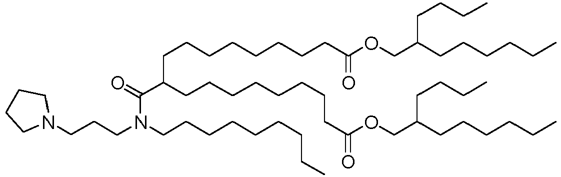

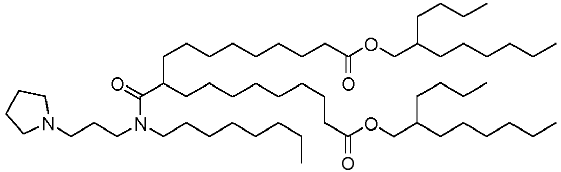

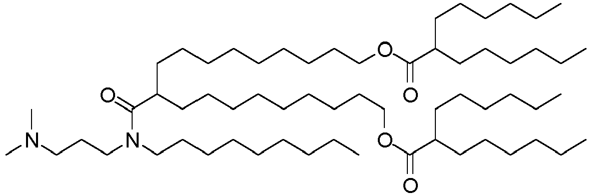

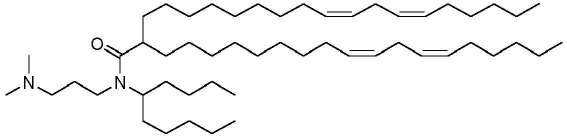

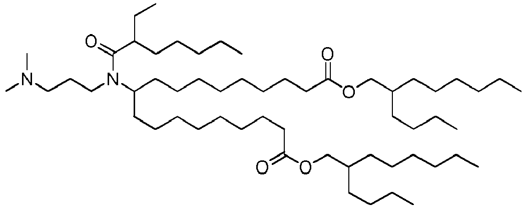

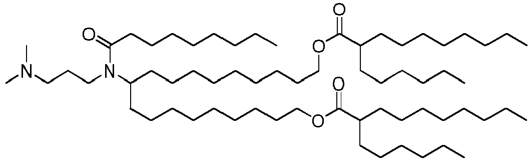

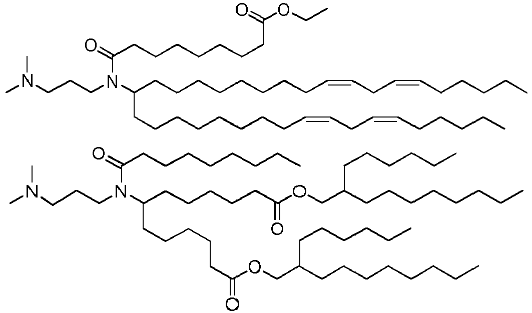

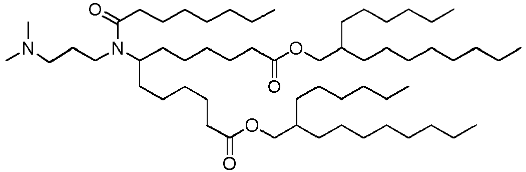

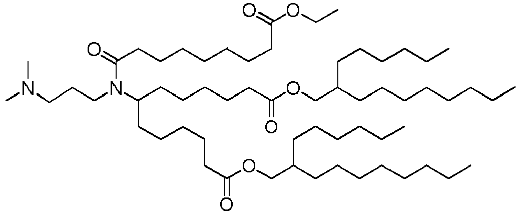

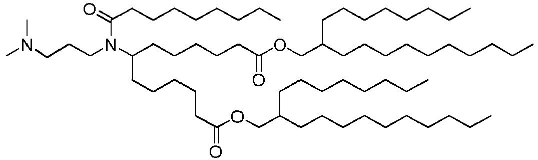

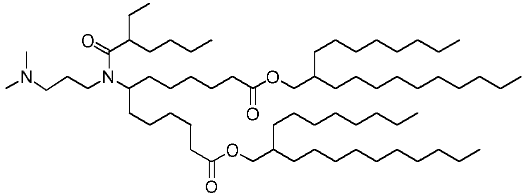

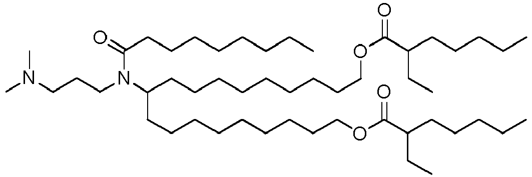

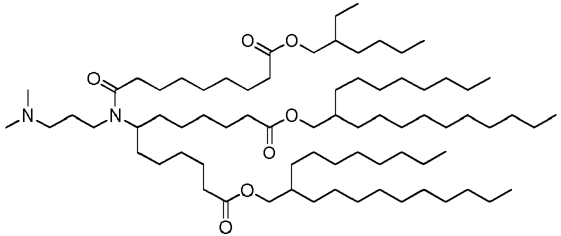

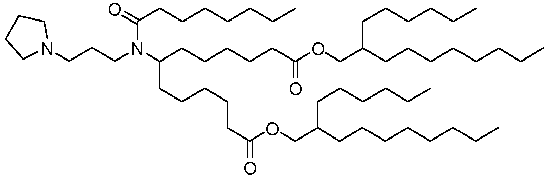

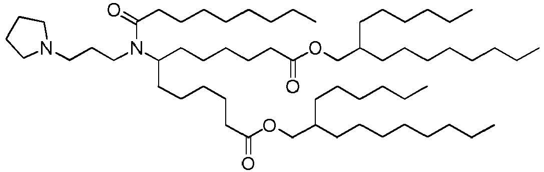

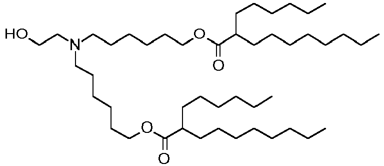

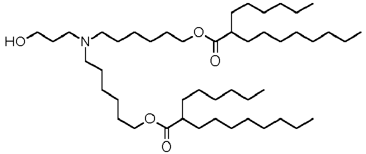

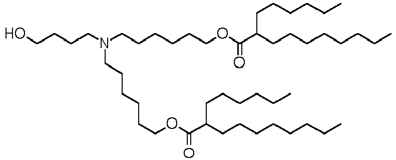

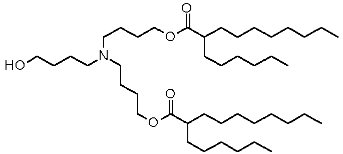

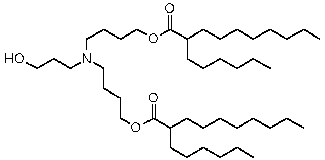

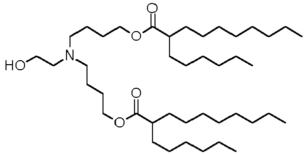

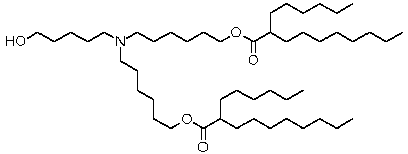

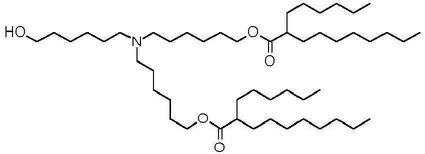

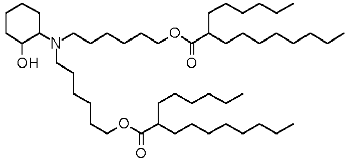

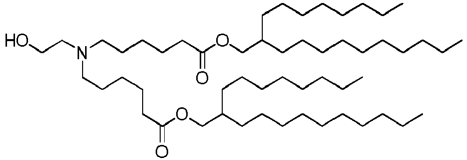

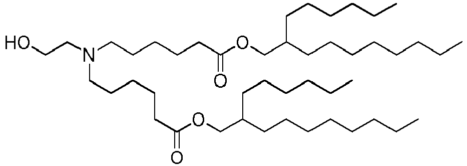

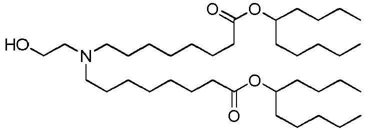

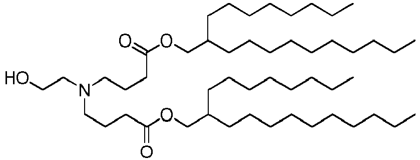

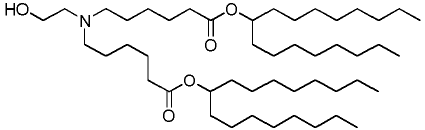

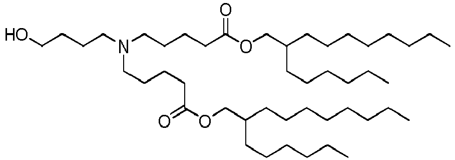

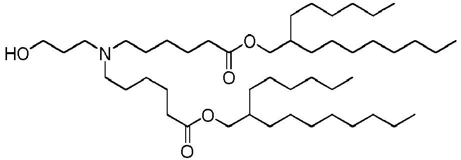

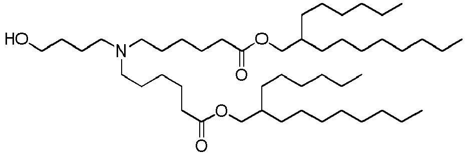

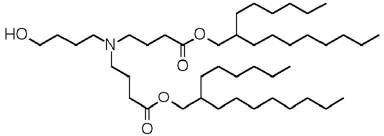

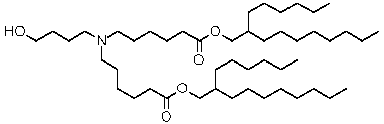

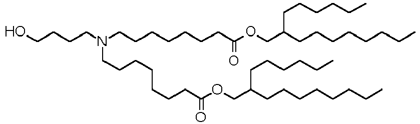

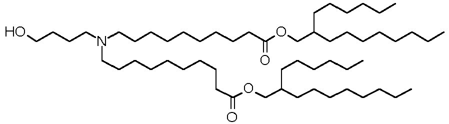

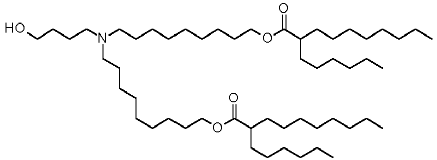

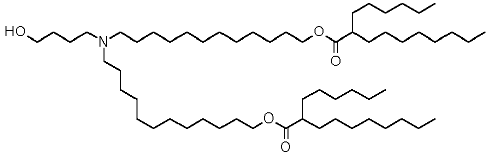

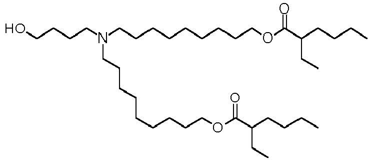

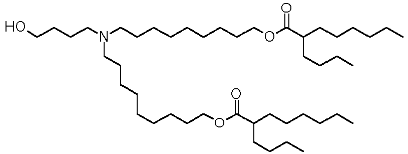

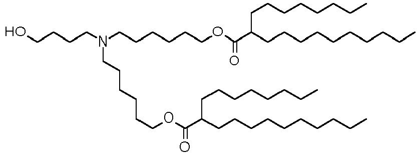

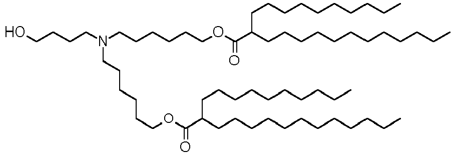

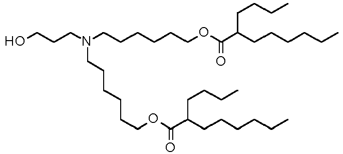

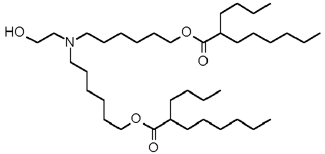

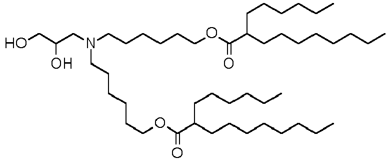

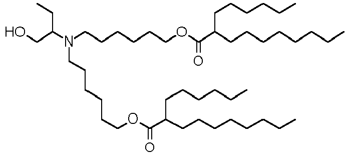

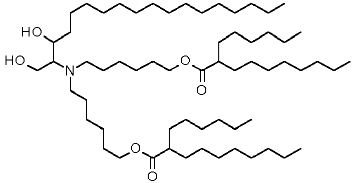

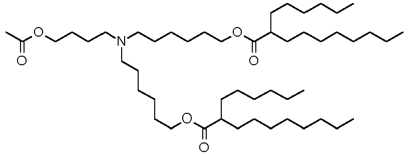

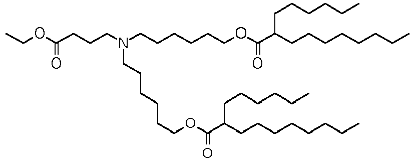

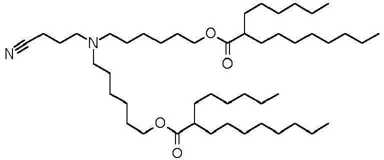

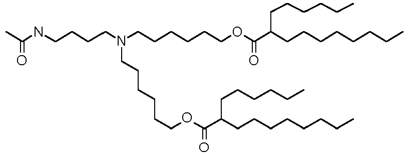

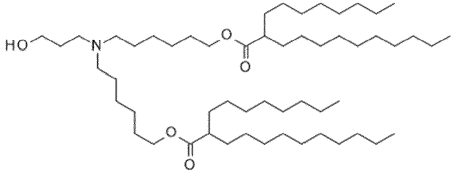

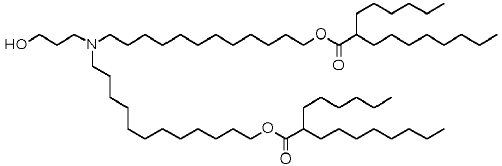

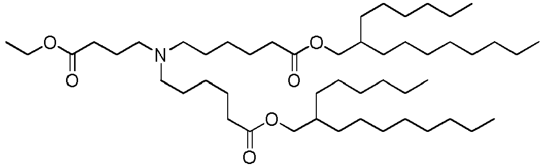

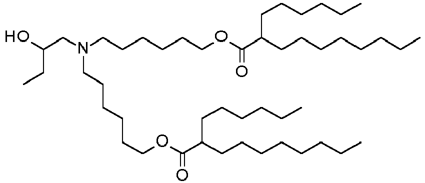

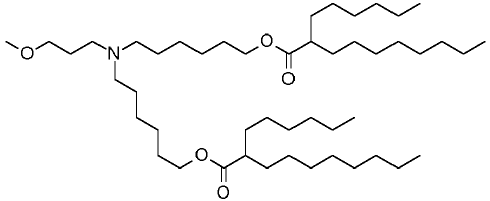

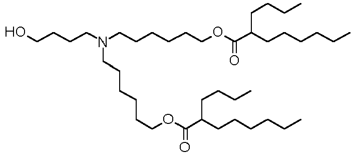

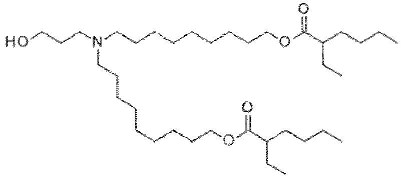

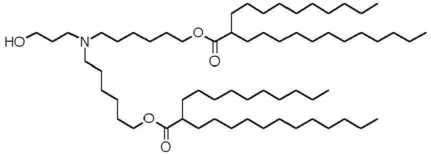

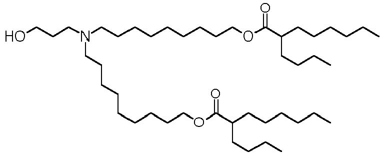

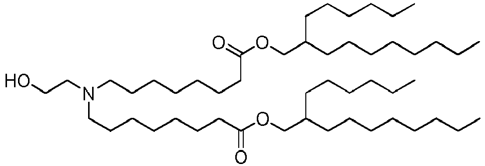

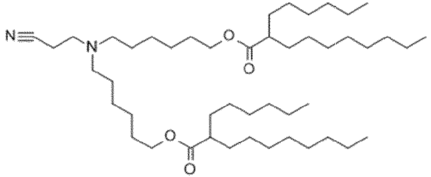

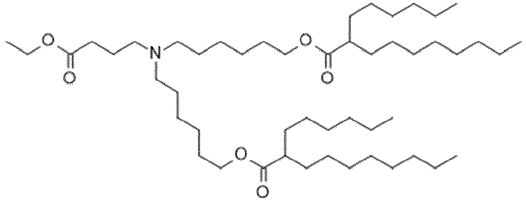

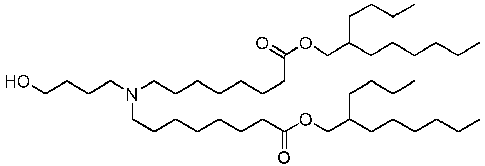

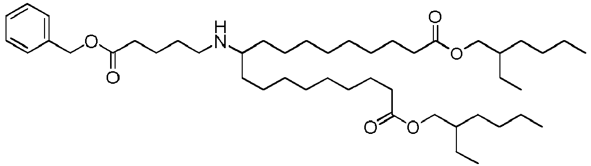

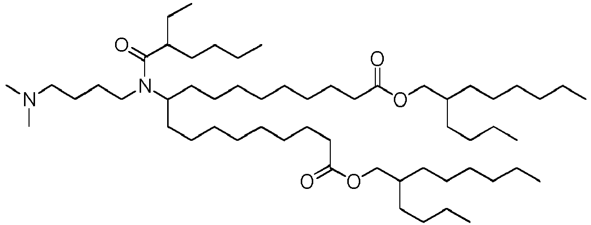

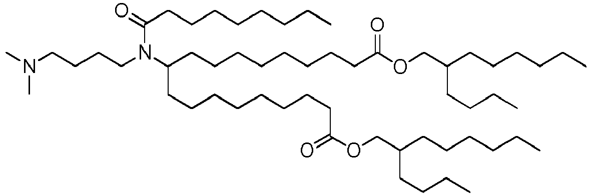

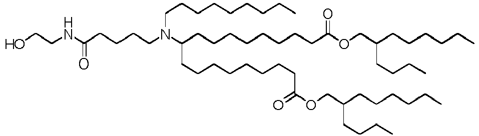

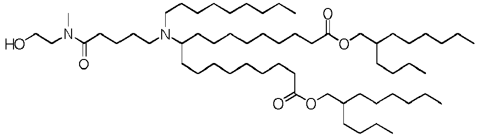

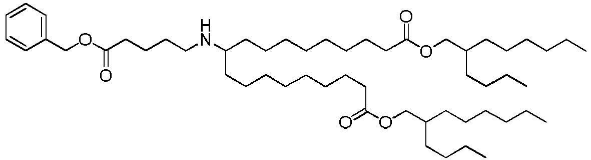

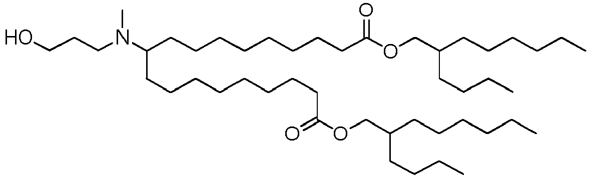

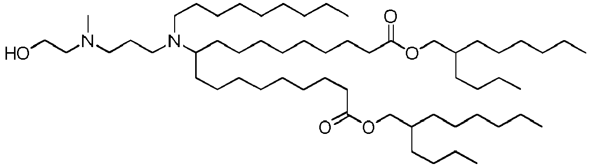

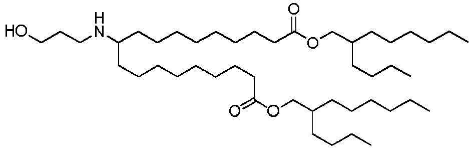

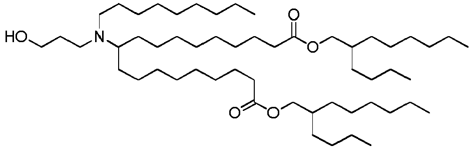

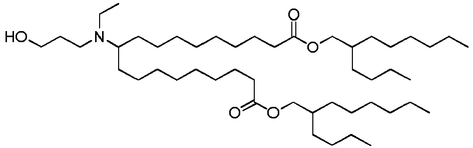

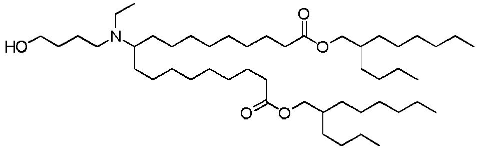

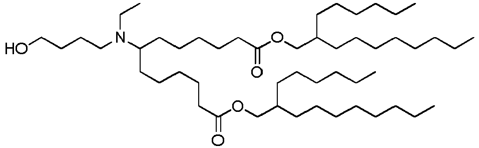

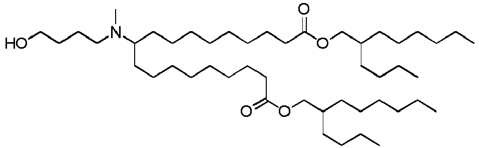

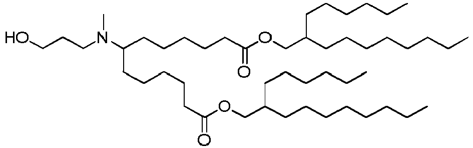

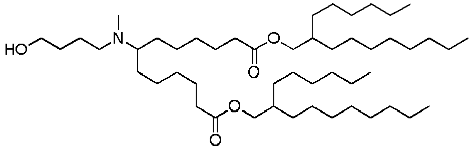

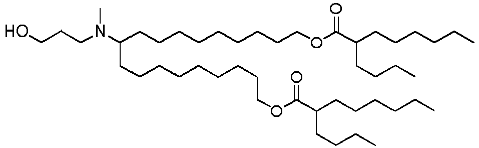

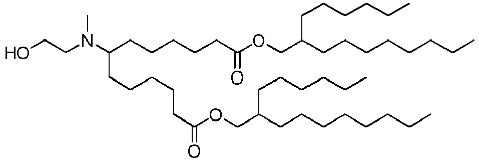

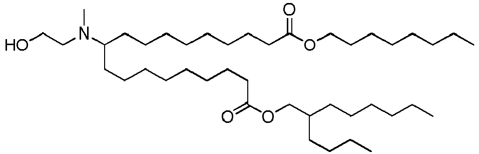

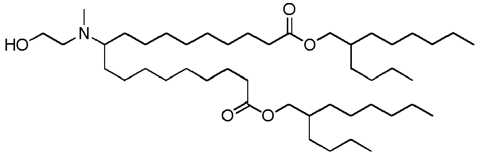

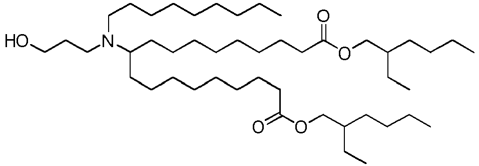

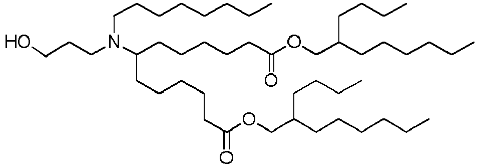

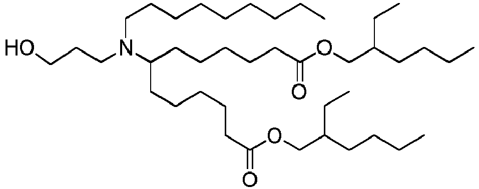

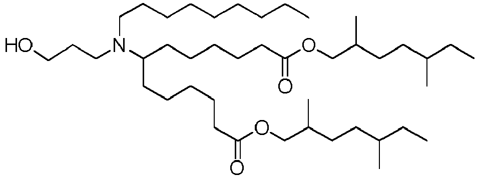

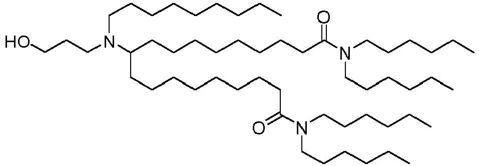

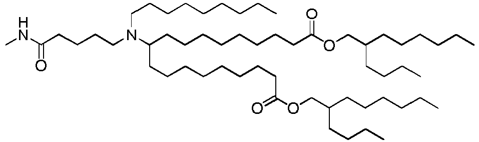

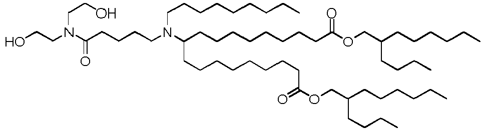

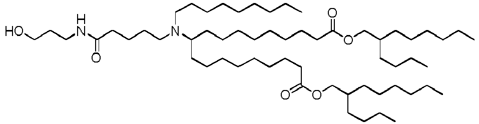

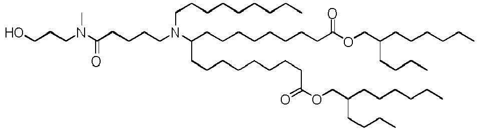

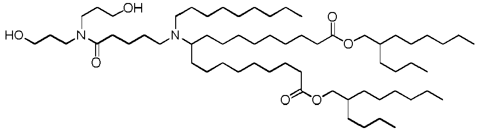

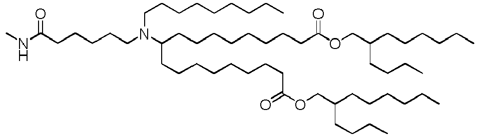

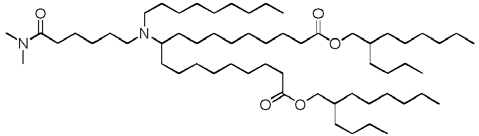

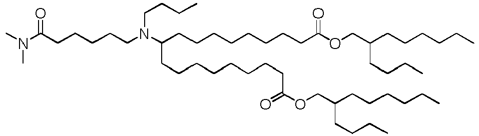

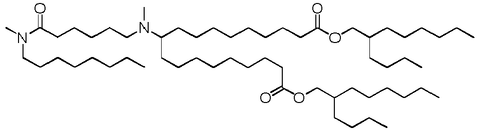

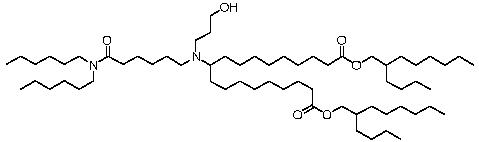

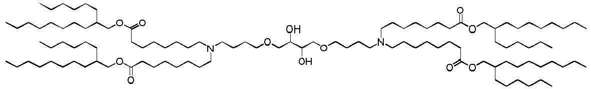

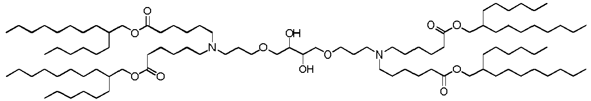

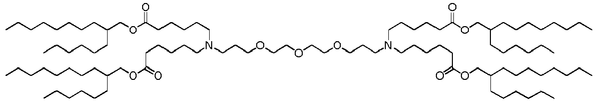

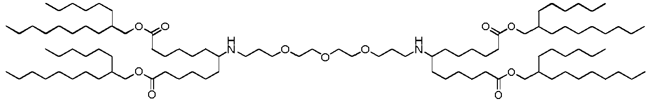

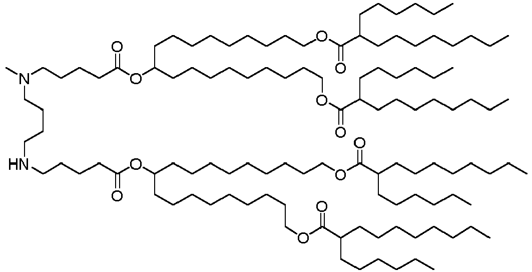

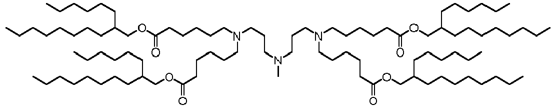

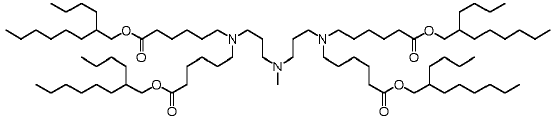

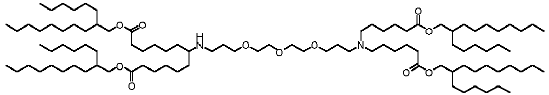

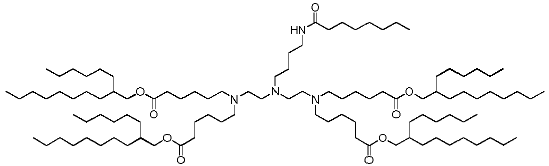

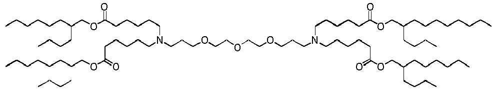

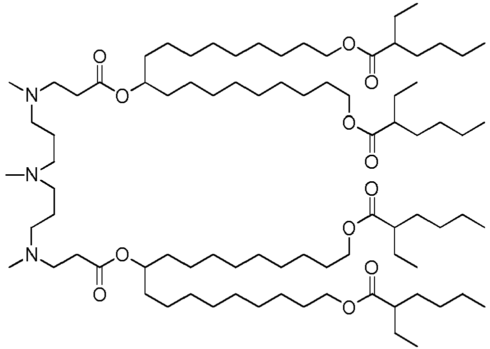

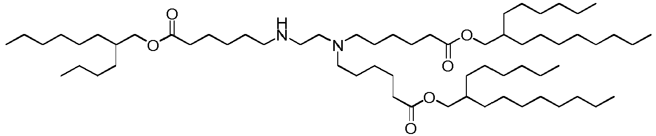

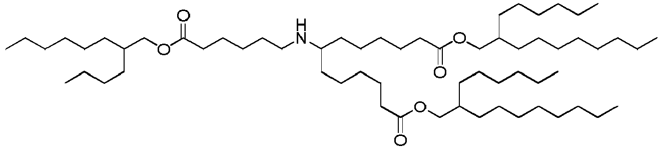

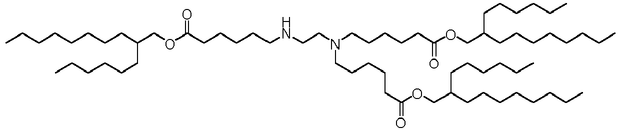

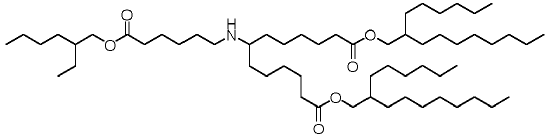

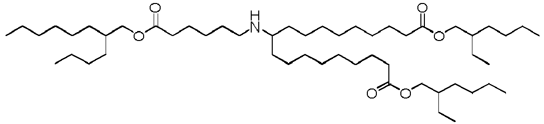

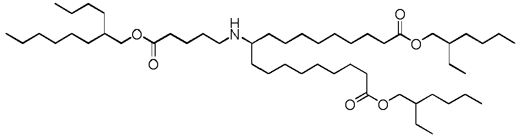

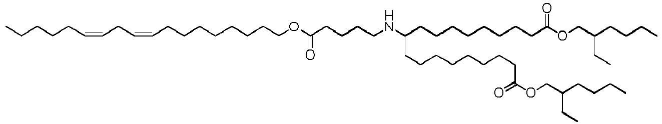

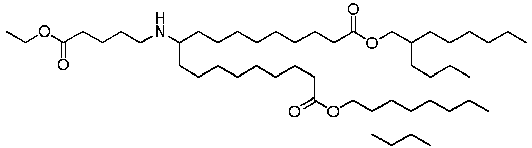

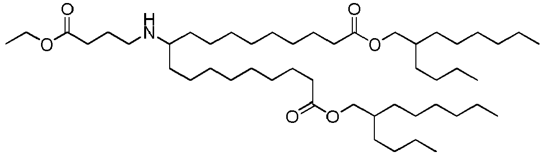

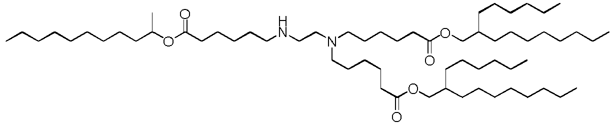

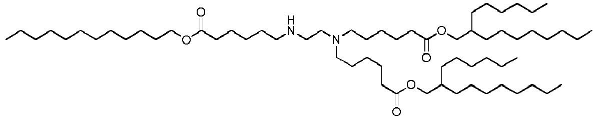

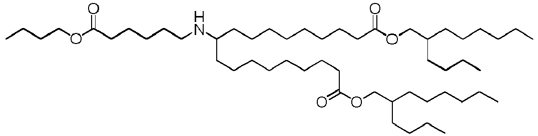

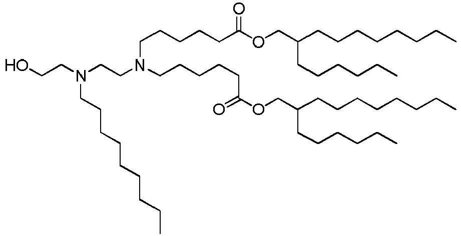

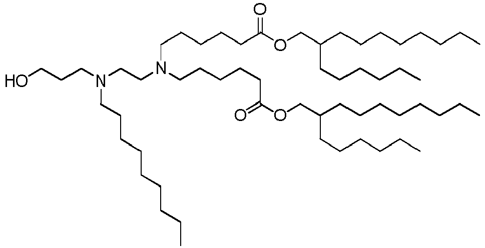

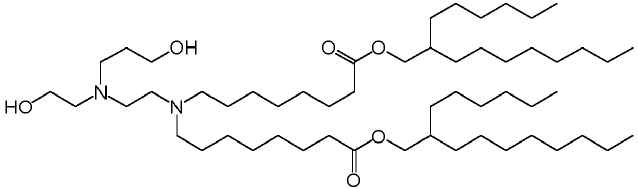

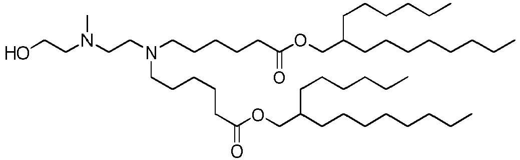

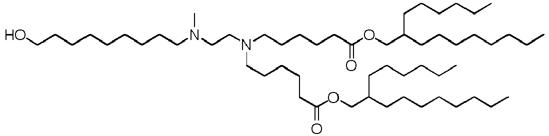

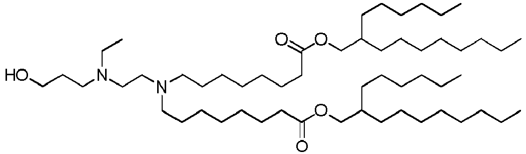

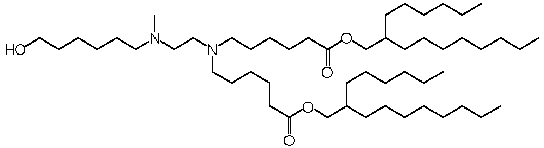

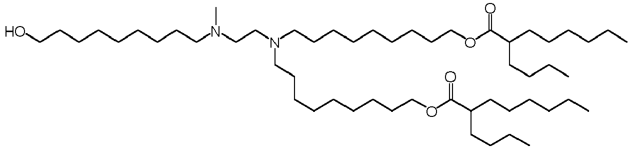

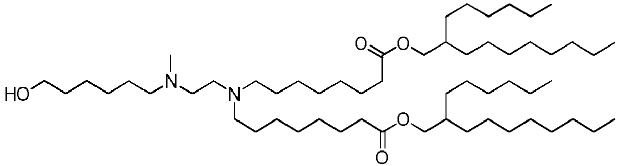

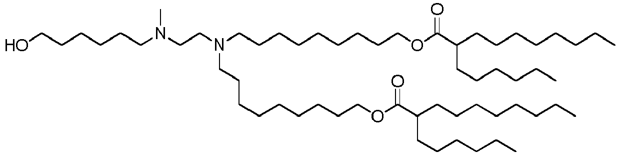

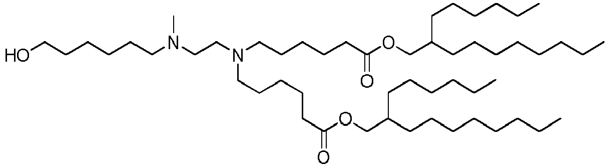

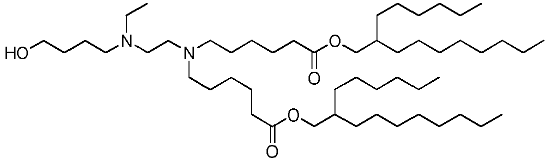

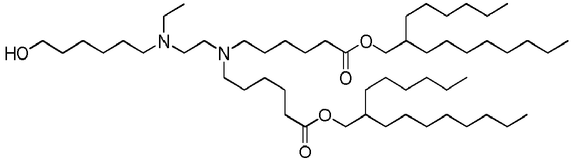

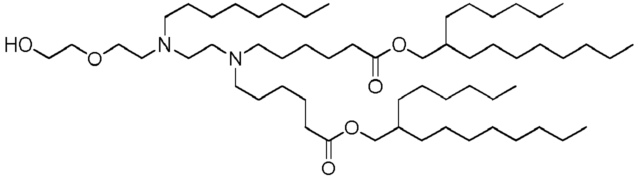

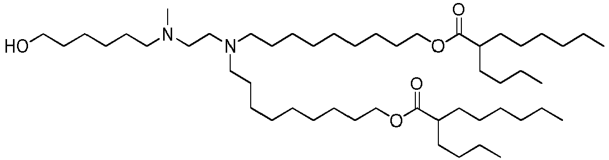

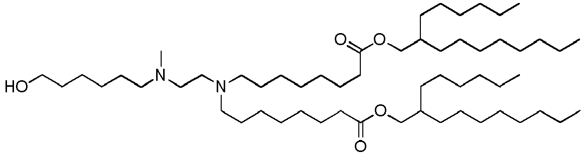

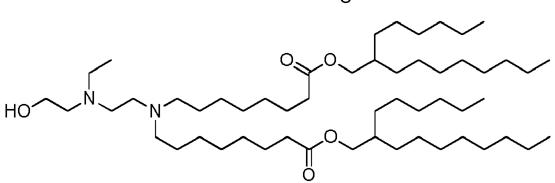

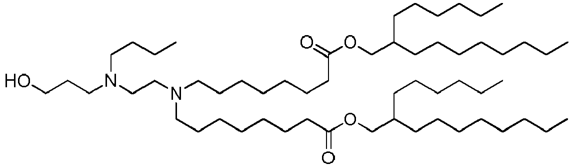

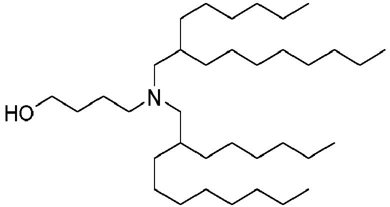

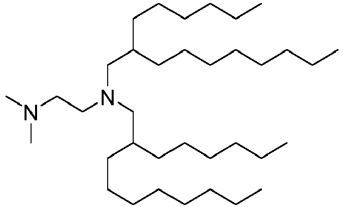

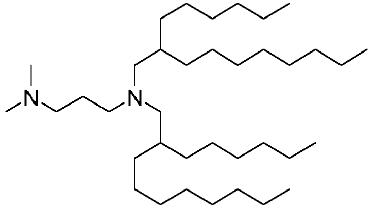

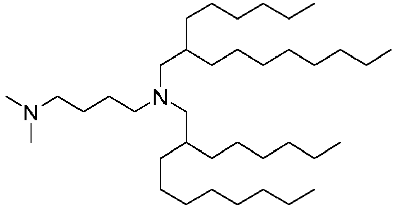

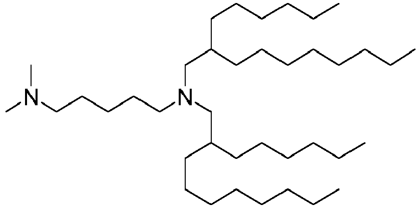

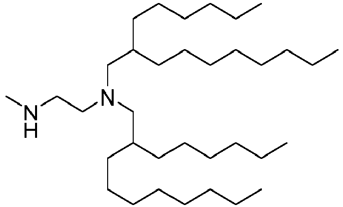

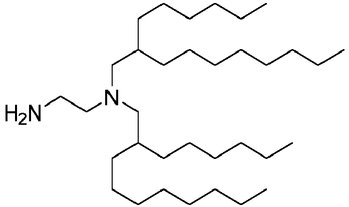

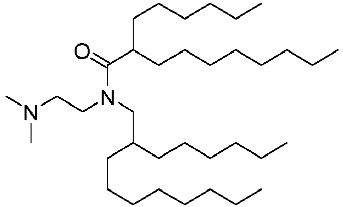

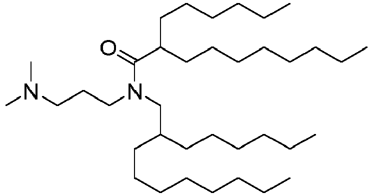

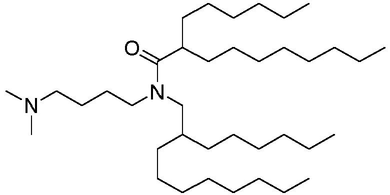

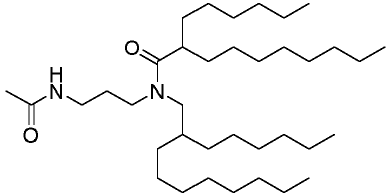

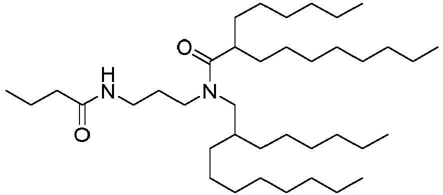

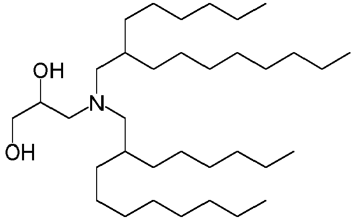

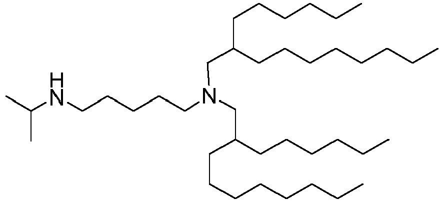

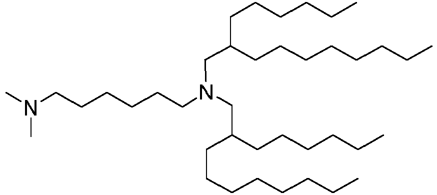

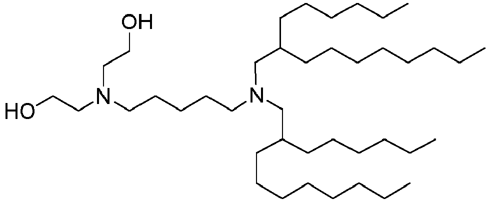

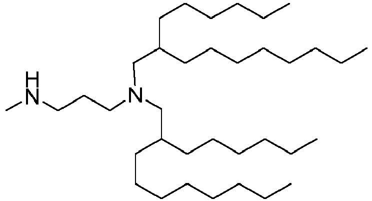

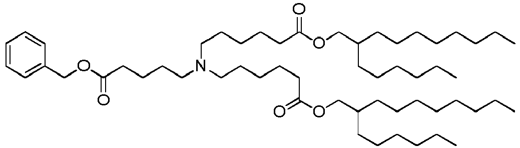

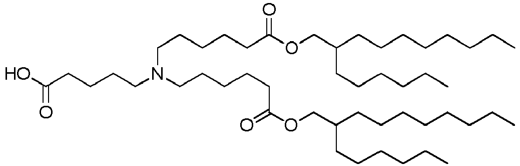

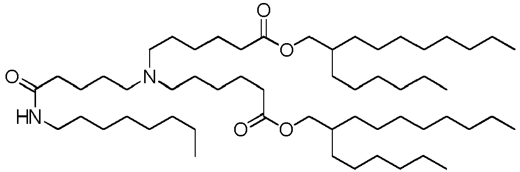

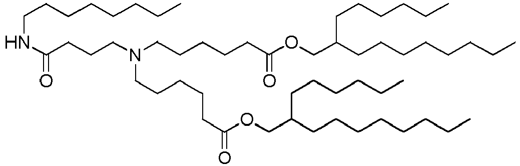

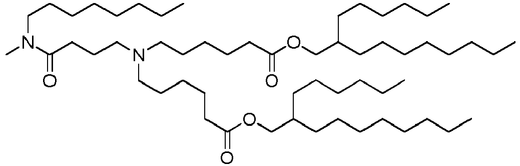

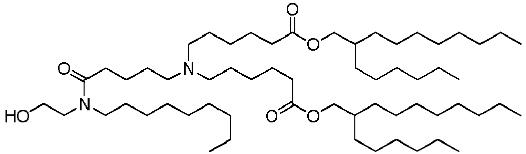

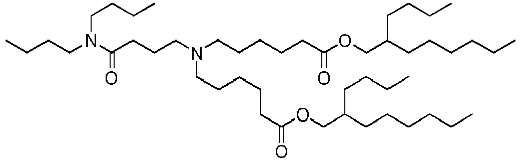

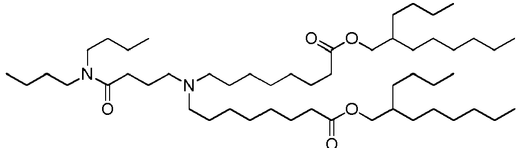

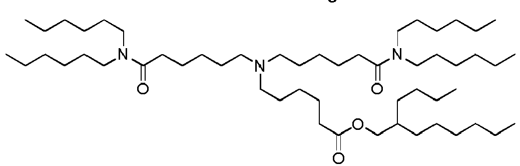

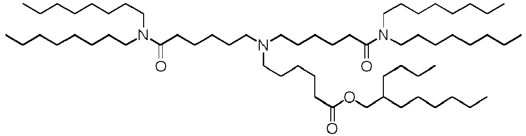

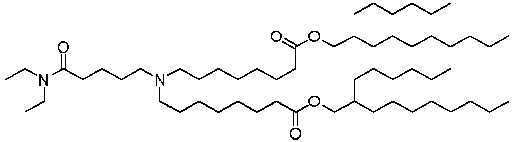

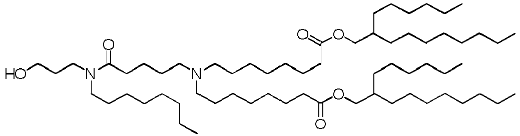

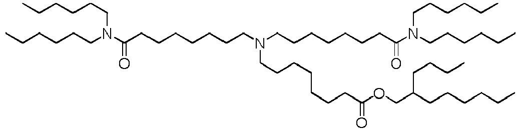

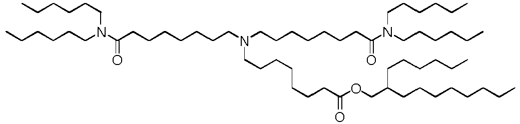

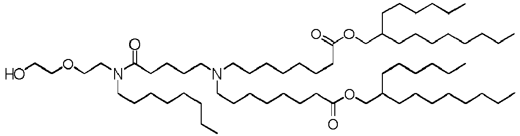

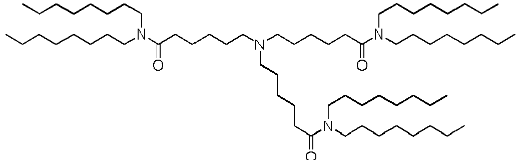

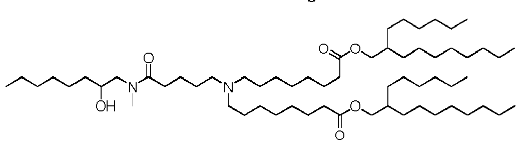

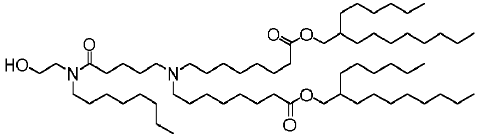

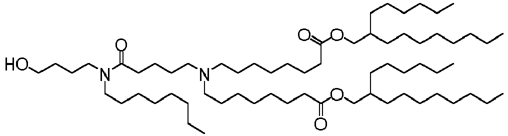

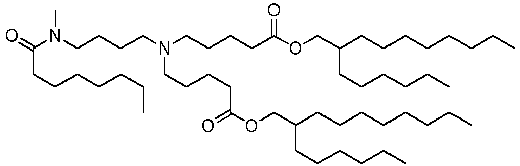

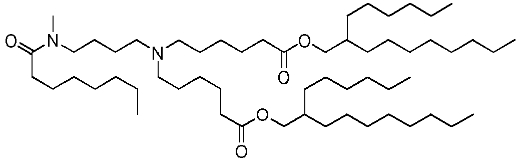

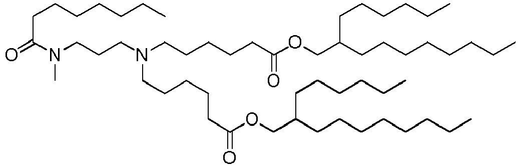

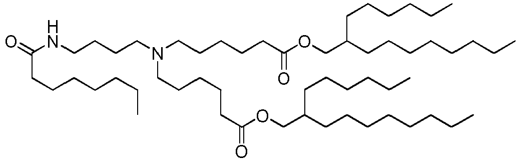

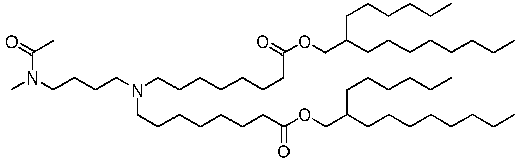

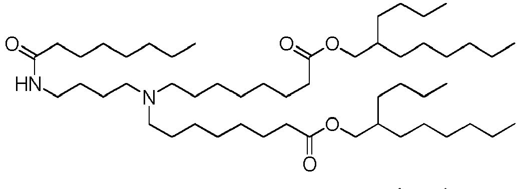

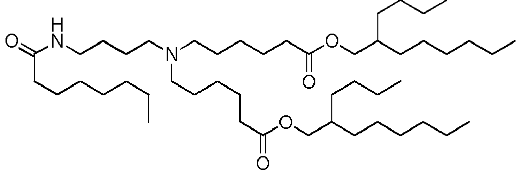

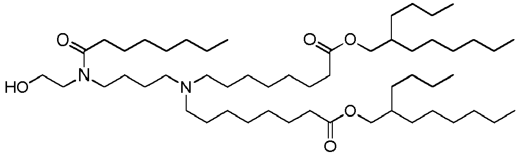

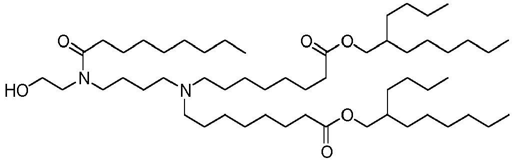

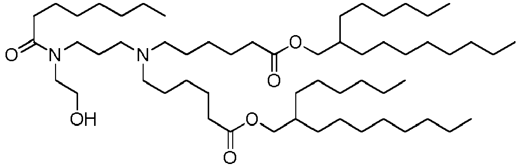

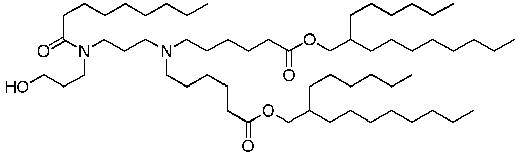

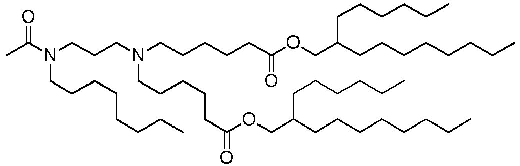

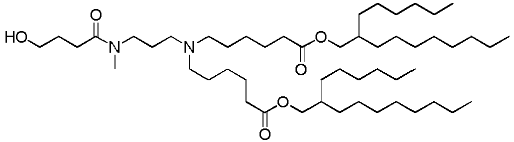

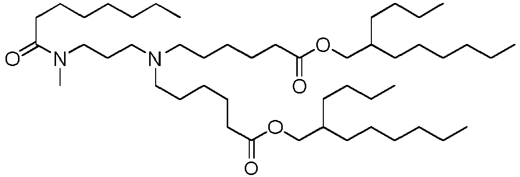

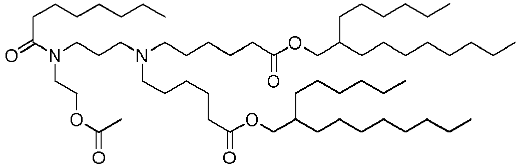

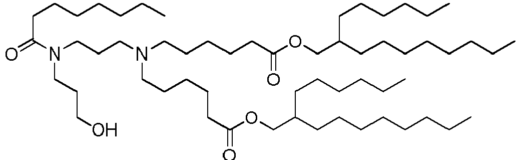

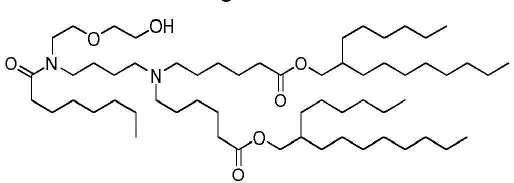

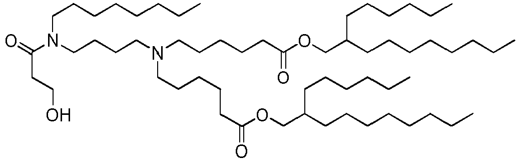

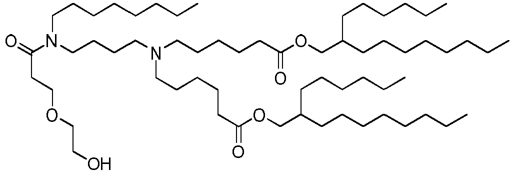

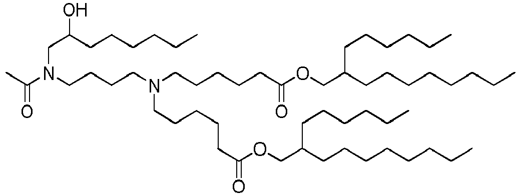

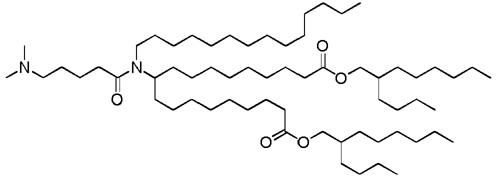

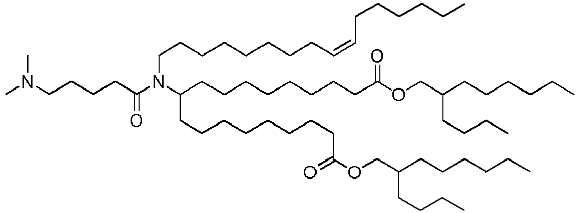

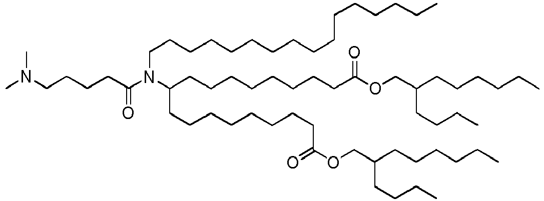

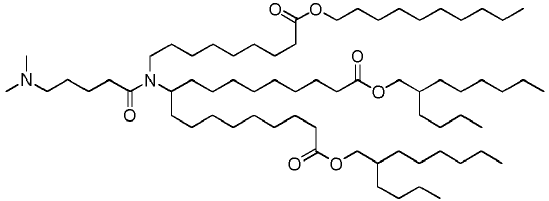

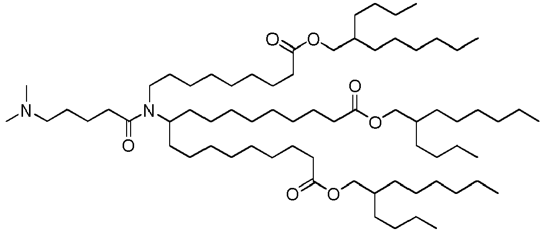

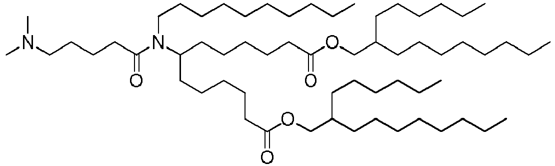

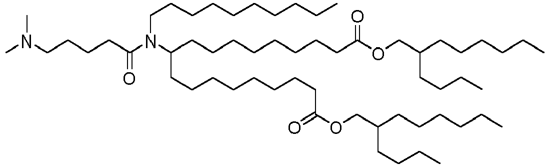

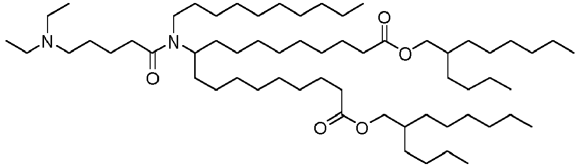

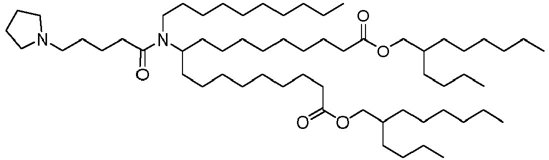

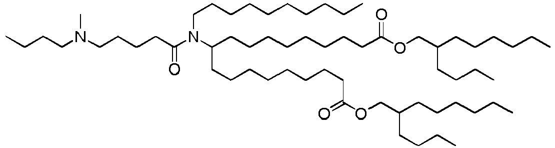

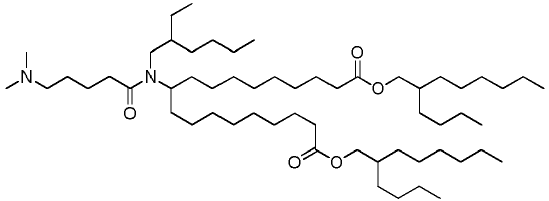

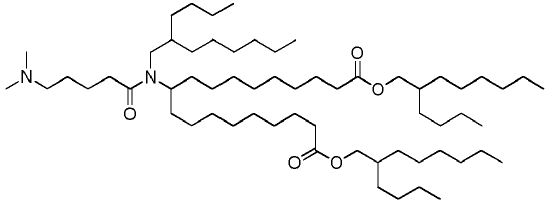

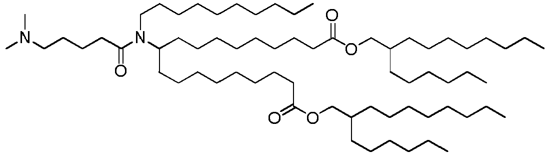

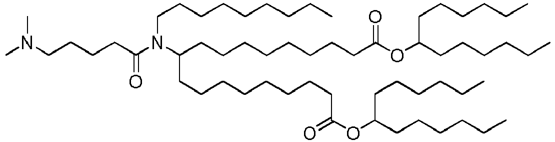

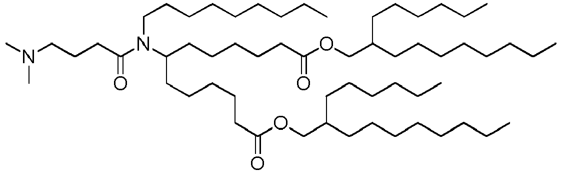

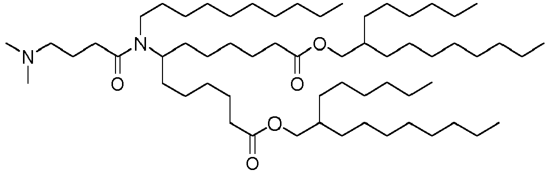

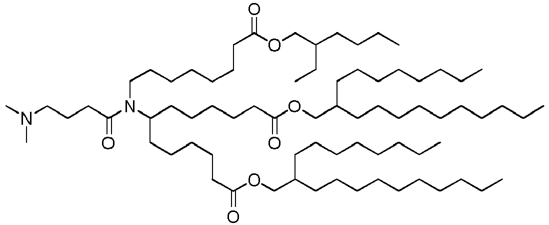

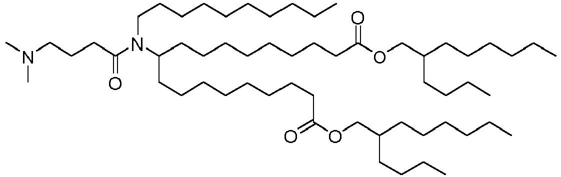

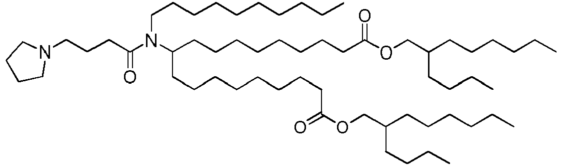

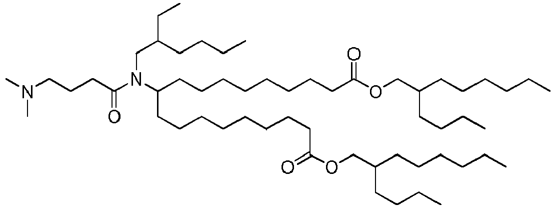

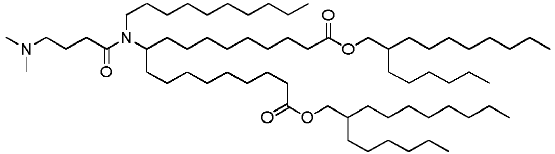

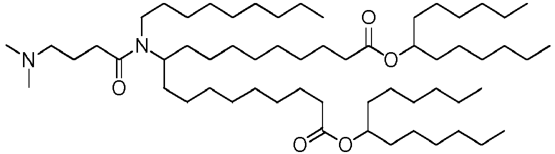

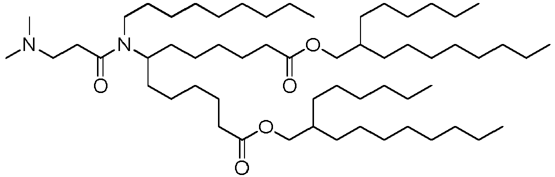

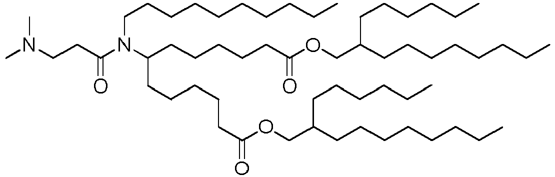

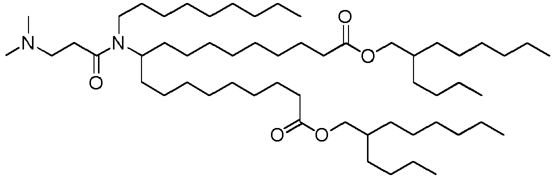

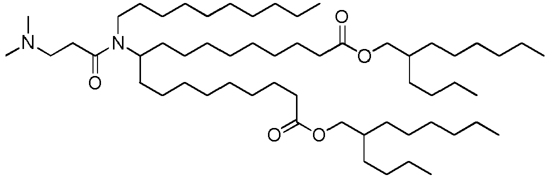

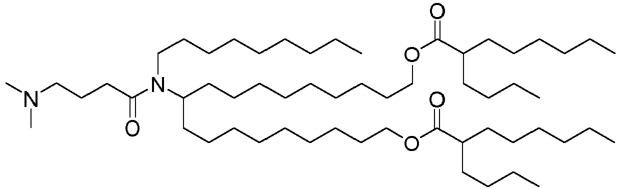

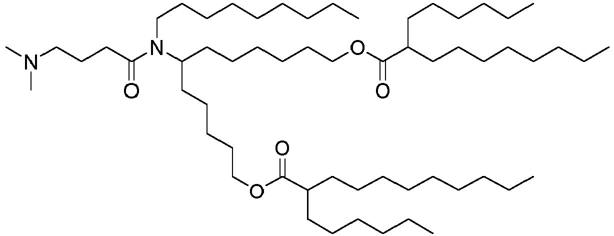

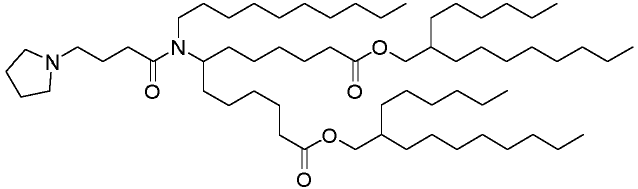

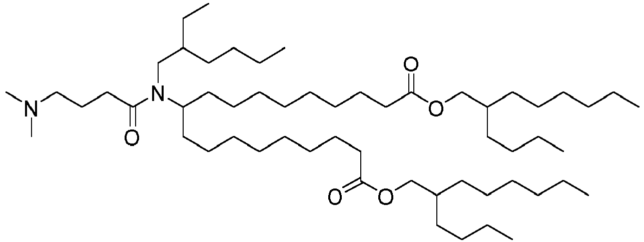

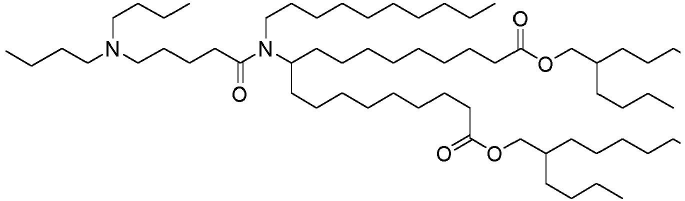

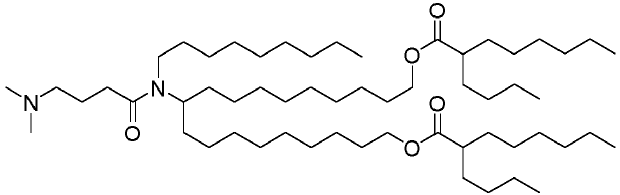

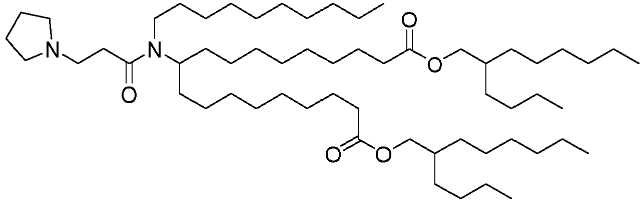

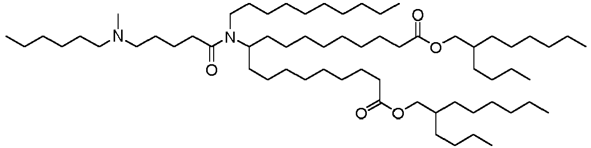

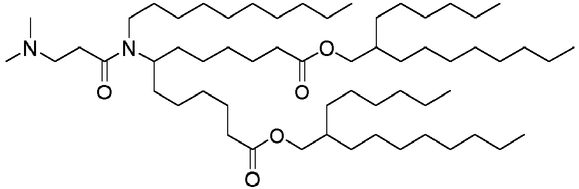

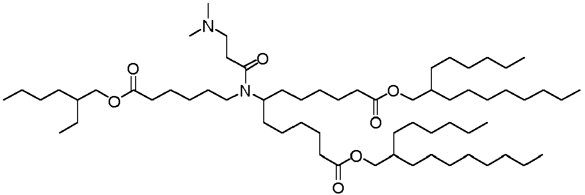

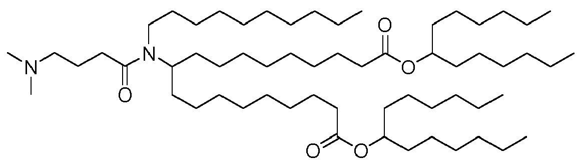

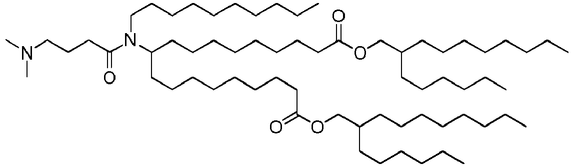

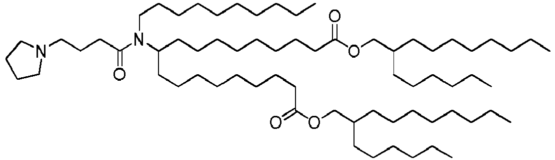

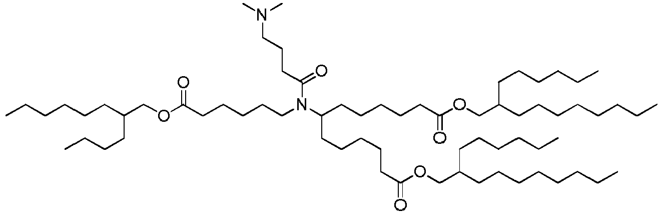

6. Die LNPs zur Verwendung nach einem der Ansprüche 1-5, wobei das molare Verhältnis von kationischem Lipid zu neutralem Lipid im Bereich von etwa 2:1 bis etwa 8:1 liegt.

7. Die LNPs zur Verwendung nach einem der Ansprüche 1-6, wobei das neutrale Lipid Distearoylphosphatidylcholin (DSPC), Dioleoylphosphatidylcholin (DOPC), Dipalmitoylphosphatidylcholin (DPPC), Dioleoylphosphatidylglycerin (DOPG), Dipalmitoylphosphatidylglycerin (DPPG), Dioleoylphosphatidylethanolamin (DOPE), Palmitoyloleoylphosphatidylcholin (POPC), Palmitoyloleoylphosphatidylethanolamin (POPE) und Dioleoylphosphatidylethanolamin-4-(N-maleimidomethyl)-cyclohexan-1-carboxylat (DOPE-mal), Dipalmitoylphosphatidylethanolamin (DPPE), Dimyristoylphosphoethanolamin (DMPE), Distearoylphosphatidylethanolamin (DSPE), 16-O-Monomethyl-PE, 16-O-Dimethyl-PE, 18-1-trans-PE, 1-Stearioyl-2-oleoylphosphatidyethanolamin (SOPE) oder 1,2-Dielaidoyl-sn-glycero-3-phophoethanolamin (transDOPE).

8. Die LNPs zur Verwendung nach einem der Ansprüche 1-6, wobei das neutrale Lipid DSPC, DPPC, DMPC, DOPC, POPC, DOPE oder SM ist.

9. Die LNPs zur Verwendung nach einem der Ansprüche 1-6, wobei das neutrale Lipid DSPC ist.

10. Die LNPs zur Verwendung nach einem der Ansprüche 1-9, wobei das Steroid Cholesterin ist.

11. Die LNPs zur Verwendung nach einem der Ansprüche 1-10, wobei das Molverhältnis von kationischem Lipid zu Steroid im Bereich von 5:1 bis 1:1 liegt.

12. Die LNPs zur Verwendung nach einem der Ansprüche 1-11, wobei das molare Verhältnis von kationischem Lipid zu polymerkonjugiertem Lipid im Bereich von etwa 100:1 bis etwa 20:1 liegt.

13. Die LNPs zur Verwendung nach einem der Ansprüche 1-12, wobei die Nukleinsäure ausgewählt ist aus Antisense- und Messenger-RNA.

14. Die LNPs zur Verwendung nach einem der Ansprüche 1-13, wobei die Nukleinsäure eine mRNA umfasst, die in der Lage ist, ein immunogenes Protein zu translatieren.

15. Die LNPs zur Verwendung nach einem der Ansprüche 1-14, wobei die Verabreichung die intravenöse Verabreichung umfasst.

## Revendications

1. Nanoparticules lipidiques (NPL) pour l'utilisation dans un procédé de traitement ou de prévention d'une maladie chez un primate en ayant besoin, le procédé comprenant l'administration des NPL au primate, chacune des NPL comprenant :
i) un acide nucléique, ou son sel pharmaceutiquement acceptable, encapsulé à l'intérieur de la NPL ;
ii) un lipide cationique ;
iii) un lipide neutre ;
iv) un stéroïde ; et
v) un lipide conjugué à un polymère,
un diamètre moyen de particule des NPL s'étendant de 40 nm à 68 nm tel que déterminé par diffusion quasiélastique de la lumière.

2. NPL pour l'utilisation selon la revendication 1, le diamètre moyen de particule s'étendant de 50 nm à 68 nm, de 55 nm à 65 nm, de 50 nm à 60 nm ou de 60 nm à 68 nm.

3. NPL pour l'utilisation selon la revendication 1, le diamètre moyen de particule étant d'environ 47 nm, d'environ 48 nm, d'environ 49 nm, d'environ 50 nm, d'environ 51 nm, d'environ 52 nm, d'environ 53 nm, d'environ 54 nm, d'environ 55 nm, d'environ 56 nm, d'environ 57 nm, d'environ 58 nm, d'environ 59 nm, d'environ 60 nm, d'environ 61 nm, d'environ 62 nm, d'environ 63 nm, d'environ 64 nm ou d'environ 65 nm.

4. NPL pour l'utilisation selon l'une quelconque des revendications 1 à 3 :
i) le lipide cationique ayant une structure de Formule (1) : 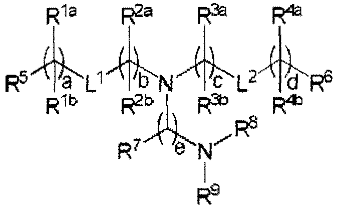 ou son sel, tautomère ou stéréoisomère pharmaceutiquement acceptable :
l'un de L¹ ou L² étant un groupe -O(C=O)-, -(C=O)O-, -C(=O)-, -O-, -S(O)ₓ-, -S-S-, -C(=O)S-, SC(=O)-, - NR⁸C (=O) -, -C (=O) NR^{a}-, NR^{a}C (=O) NR^{a}-, -OC (=O) NR^{a}- ou - NR⁸C(=O)O-, et l'autre de L¹ ou L² étant un groupe -O(C=O)-, -(C=O)O-, -C(=O)-, -O-, -S(O)ₓ-, -S-S-, -C(=O)S-, SC(=O)-, -NR⁸C(=O) -, -C(=O) NR^{a}-, -NR^{a}C(=O) NR^{a}-, - OC(=O)NR^{a}- ou -NR^{a}C(=O)O- ou une liaison directe ;
R^{a} étant un groupe H ou alkyle en C₁ à C₁₂ ;
R^{1a} et R^{1b} étant, à chaque apparition, indépendamment soit (a) un groupe H ou alkyle en C₁ à C₁₂, soit (b) R^{1a} étant un groupe H ou alkyle en C₁ à C₁₂, et R^{1b} conjointement à l'atome de carbone auquel il est lié est pris conjointement à un groupe R^{1b} adjacent et l'atome de carbone auquel il est lié pour former une double liaison carbone-carbone ;
R^{2a} et R^{2b} étant, à chaque apparition, indépendamment soit (a) un groupe H ou alkyle en C₁ à C₁₂, soit (b) R^{2a} étant un groupe H ou alkyle en C₁ à C₁₂, et R^{2b} conjointement à l'atome de carbone auquel il est lié est pris conjointement à un groupe R^{2b} adjacent et l'atome de carbone auquel il est lié pour former une double liaison carbone-carbone ;
R^{3a} et R^{3b} étant, à chaque apparition, indépendamment soit (a) un groupe H ou alkyle en C₁ à C₁₂, soit (b) R^{3a} étant un groupe H ou alkyle en C₁ à C₁₂, et R^{3b} conjointement à l'atome de carbone auquel il est lié est pris conjointement à un groupe R^{3b} adjacent et l'atome de carbone auquel il est lié pour former une double liaison carbone-carbone ;
R^{4a} et R^{4b} étant, à chaque apparition, indépendamment soit (a) un groupe H ou alkyle en C₁ à C₁₂, soit (b) R^{4a} étant un groupe H ou alkyle en C₁ à C₁₂, et R^{4b} conjointement à l'atome de carbone auquel il est lié est pris conjointement à un groupe R^{4b} adjacent et l'atome de carbone auquel il est lié pour former une double liaison carbone-carbone ;
R⁵ et R⁶ étant chacun indépendamment un groupe méthyle ou cycloalkyle ;
R⁷ étant, à chaque apparition, indépendamment un groupe H ou alkyle en C₁ à C₁₂ ;
R⁸ et R⁹ étant chacun indépendamment un groupe alkyle en C₁ à C₁₂ non substitué ; ou R⁸ et R⁹, conjointement à l'atome d'azote auquel ils sont fixés, forment un cycle hétérocyclique à 5, 6 ou 7 chaînons comprenant un atome d'azote ;
a et d étant chacun indépendamment un nombre entier d'une valeur de 0 à 24 ;
b et c étant chacun indépendamment un nombre entier d'une valeur de 1 à 24 ;
e ayant la valeur de 1 ou 2 ; et
x ayant la valeur de 0, 1 ou 2 ; ou
ii) le lipide cationique ayant une structure de Formule (II) : 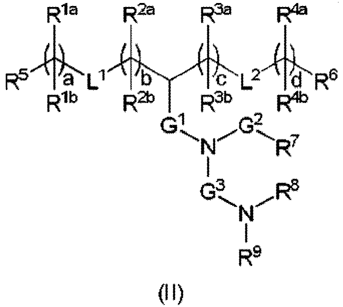 ou son sel, tautomère ou stéréoisomère pharmaceutiquement acceptable :
l'un de L¹ ou L² étant un groupe -O(C=O)-, -(C=O)O-, -C(=O)-, -O-, -S(O)ₓ-, -S-S-, -C(=O)S-, SC(=O)-, - NR^{a}C (=O) -, -C (=O) NR^{a}-, NR^{a}C (=O) NR^{a}-, -OC (=O) NR^{a}- ou - NR^{a}C(=O)O-, et l'autre de L¹ ou L² étant un groupe -O(C=O)-, -(C=O)O-, -C(=O)-, -O-, -S(O)ₓ-, -S-S-, -C(=O)S-, SC(=O)-, -NR^{a}C(=O) -, -C(=O) NR^{a}- , -NR^{a}C(=O) NR^{a}- , - OC(=O)NR^{a}- ou -NR^{a}C(=O)O- ou une liaison directe ;
G¹ étant un groupe alcylène en C₁ à C₂, - (C=O) -, - O(C=O)-, -SC(=O)-, -NR^{a}C(=O)- ou une liaison directe ;
G² étant un groupe -C(=O)-, -(C=O)O-, -C(=O)S-, - C(=O)NR^{a}- ou une liaison directe ;
G³ étant un groupe alcylène en C₁ à C₆ ;
R^{a} étant un groupe H ou alkyle en C₁ à C₁₂ ;
R^{1a} et R^{1b} étant, à chaque apparition, indépendamment soit : (a) un groupe H ou alkyle en C₁ à C₁₂ ; soit (b) R^{1a} étant un groupe H ou alkyle en C₁ à C₁₂, et R^{1b} conjointement à l'atome de carbone auquel il est lié est pris conjointement à un groupe R^{1b} adjacent et l'atome de carbone auquel il est lié pour former une double liaison carbone-carbone ;
R^{2a} et R^{2b} étant, à chaque apparition, indépendamment soit : (a) un groupe H ou alkyle en C₁ à C₁₂ ; soit (b) R^{2a} étant un groupe H ou alkyle en C₁ à C₁₂, et R^{2b} conjointement à l'atome de carbone auquel il est lié est pris conjointement à un groupe R^{2b} adjacent et l'atome de carbone auquel il est lié pour former une double liaison carbone-carbone ;
R^{3a} et R^{3b} étant, à chaque apparition, indépendamment soit (a) : un groupe H ou alkyle en C₁ à C₁₂ ; soit (b) R^{3a} étant un groupe H ou alkyle en C₁ à C₁₂, et R^{3b} conjointement à l'atome de carbone auquel il est lié est pris conjointement à un groupe R^{3b} adjacent et l'atome de carbone auquel il est lié pour former une double liaison carbone-carbone ;
R^{4a} et R^{4b} étant, à chaque apparition, indépendamment soit : (a) un groupe H ou alkyle en C₁ à C₁₂ ; soit (b) R^{4a} étant un groupe H ou alkyle en C₁ à C₁₂, et R^{4b} conjointement à l'atome de carbone auquel il est lié est pris conjointement à un groupe R^{4b} adjacent et l'atome de carbone auquel il est lié pour former une double liaison carbone-carbone ;
R⁵ et R⁶ étant chacun indépendamment un groupe H ou méthyle ;
R⁷ étant un groupe alkyle en C₄ à C₂₀ ;
R⁸ et R⁹ étant chacun indépendamment un groupe alkyle en C₁ à C₁₂ ; ou R⁸ et R⁹, conjointement à l'atome d'azote auquel ils sont fixés, forment un cycle hétérocyclique à 5, 6 ou 7 chaînons ;
a, b, c et d étant chacun indépendamment un nombre entier d'une valeur de 1 à 24 ; et
x ayant la valeur de 0, 1 ou 2 ; ou
iii) le lipide cationique ayant une structure de Formule III : 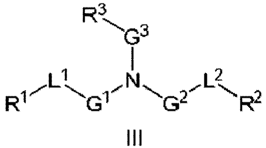 ou son sel ou stéréoisomère pharmaceutiquement acceptable :
l'un de L¹ ou L² étant un groupe -O(C=O)-, -(C=O)O-, -C(=O)-, -O-, -S(O)ₓ-, -S-S-, -C(=O)S-, SC(=O)-, - NR^{a}C (=O) -, -C(=O) NR^{a}-, NR^{a}C (=O) NR^{a}-, -OC (=O) NR^{a}- ou - NR^{a}C(=O)O-, et l'autre de L¹ ou L² étant un groupe -O(C=O)-, -(C=O)O-, -C(=O)-, -O-, -S(O)ₓ-, -S-S-, -C(=O)S-, SC(=O)-, -NR^{a}C(=O) -, -C(=O) NR^{a}- , -NR^{a}C(=O) NR^{a}- , - OC(=O)NR^{a}- ou -NR^{a}C(=O)O- ou une liaison directe ;
G¹ et G² étant chacun indépendamment un groupe alcylène en C₁ à C₁₂ non substitué ou un groupe alcénylène en C₁ à C₁₂ ;
G³ étant un groupe alcylène en C₁ à C₂₄, alcénylène en C₁ à C₂₄, cycloalcylène en C₃ à C₈, cycloalcénylène en C₃ à C₈ ;
R^{a} étant un groupe H ou alkyle en C₁ à C₁₂ ;
R¹ et R² étant chacun indépendamment un groupe alkyle en C₆ à C₂₄ ou alcényle en C₆ à C₂₄ ;
R³ étant un groupe H, OR⁵, CN, -C(=O)OR⁴, -OC(=O)R⁴ ou -NR⁵C(=O)R⁴ ;
R⁴ étant un groupe alkyle en C₁ à C₁₂ ;
R⁵ étant un groupe H ou alkyle en C₁ à C₆ ; et
x ayant la valeur de 0, 1 ou 2 ; ou
iv) le lipide cationique ayant la Formule (IV) suivante : 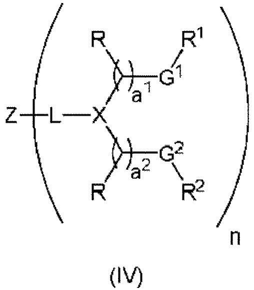 ou son sel ou stéréoisomère pharmaceutiquement acceptable :
l'un de G¹ ou G² étant, à chaque apparition, un groupe -O(C=O)-, -(C=O)O-, -C(=O)-, -O-, -S(O)_{y}-, -S-S-, -C(=O)S-, SC(=O)-, -N(R^{a})C(=O)-, -C(=O)N(R^{a})-, -N(R^{a})C(=O)N(R^{a})-, -OC(=O)N(R^{a})- ou -N(R^{a})C(=O)O-, et l'autre de G¹ ou G² étant, à chaque apparition, un groupe -O(C=O)-, -(C=O)O-, -C(=O)-, -O-, -S(O)_{y}-, -S-S-, -C(=O)S-, -SC(=O)-, - N(R^{a})C(=O)-, -C(=O)N(R^{a})-, -N(R^{a})C(=O)N(R^{a})-, - OC(=O)N(R^{a})- ou -N(R^{a})C(=O)O- ou une liaison directe ;
L étant, à chaque apparition, un groupe ∼O(C=O)-, ∼ représentant une liaison covalente à X ;
X étant un groupe CR^{a} ;
Z étant un groupe alkyle, cycloalkyle ou une fraction monovalente comprenant au moins un groupe fonctionnel polaire lorsque n a la valeur de 1 ; ou Z étant un groupe alcylène, cycloalcylène ou une fraction polyvalente comprenant au moins un groupe fonctionnel polaire lorsque n est supérieur à 1 ;
R^{a} étant, à chaque apparition, indépendamment un groupe H, alkyle en C₁ à C₁₂, hydroxylalkyle en C₁ à C₁₂, aminoalkyle en C₁ à C₁₂, alkylaminylalkyle en C₁ à C₁₂, alcoxyalkyle en C₁ à C₁₂, alcoxycarbonyle en C₁ à C₁₂, alkylcarbonyloxy en C₁ à C₁₂, alkylcarbonyloxyalkyle en C₁ à C₁₂ ou alkylcarbonyle en C₁ à C₁₂ ;
R étant, à chaque apparition, indépendamment soit : (a) un groupe H ou alkyle en C₁ à C₁₂ ; soit (b) R conjointement à l'atome de carbone auquel il est lié est pris conjointement à un groupe R adjacent et l'atome de carbone auquel il est lié pour former une double liaison carbone-carbone ;
R¹ et R² ayant, à chaque apparition, respectivement la structure suivante : 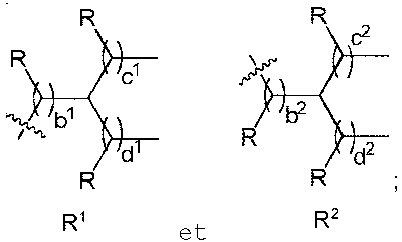
a¹ et a² étant, à chaque apparition, indépendamment un nombre entier d'une valeur de 3 à 12 ;
b¹ et b² ayant, à chaque apparition, indépendamment la valeur de 0 ou 1 ;
c¹ et c² étant, à chaque apparition, indépendamment un nombre entier d'une valeur de 5 à 10 ;
d¹ et d² étant, à chaque apparition, indépendamment un nombre entier d'une valeur de 5 à 10 ;
y étant, à chaque apparition, indépendamment un nombre entier d'une valeur de 0 à 2 ; et
n étant un nombre entier d'une valeur de 1 à 6,
chaque groupe alkyle, alcylène, hydroxylalkyle, aminoalkyle, alkylaminylalkyle, alcoxyalkyle, alcoxycarbonyle, alkylcarbonyloxy, alkylcarbonyloxyalkyle et alkylcarbonyle est éventuellement substitué par un ou plusieurs substituants ; ou
v) un lipide cationique ayant la Formule (V) suivante : 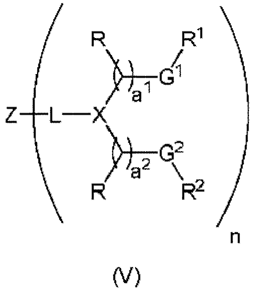 ou son sel ou stéréoisomère pharmaceutiquement acceptable :
l'un de G¹ ou G² étant, à chaque apparition, un groupe -O(C=O)-, -(C=O)O-, -C(=O)-, -O-, -S(O)_{y}-, -S-S-, -C(=O)S-, SC(=O)-, -N(R^{a})C(=O)-, -C(=O)N(R^{a})-, -N(R^{a})C(=O)N(R^{a})-, -OC(=O)N(R^{a})- ou -N(R^{a})C(=O)O-, et l'autre de G¹ ou G² étant, à chaque apparition, un groupe -O(C=O)-, -(C=O)O-, -C(=O)-, -O-, -S(O)_{y}-, -S-S-, -C(=O)S-, -SC(=O)-, - N(R^{a})C(=O)-, -C(=O)N(R^{a})-, -N(R^{a})C(=O)N(R^{a})-, - OC(=O)N(R^{a})- ou -N(R^{a})C(=O)O- ou une liaison directe ;
L étant, à chaque apparition, un groupe ∼O(C=O)-, ~ représentant une liaison covalente à X ;
X étant un groupe CR^{a} ;
Z étant un groupe alkyle, cycloalkyle ou une fraction monovalente comprenant au moins un groupe fonctionnel polaire lorsque n a la valeur de 1 ; ou Z étant un groupe alcylène, cycloalcylène ou une fraction polyvalente comprenant au moins un groupe fonctionnel polaire lorsque n est supérieur à 1 ;
R^{a} étant, à chaque apparition, indépendamment un groupe H, alkyle en C₁ à C₁₂, hydroxylalkyle en C₁ à C₁₂, aminoalkyle en C₁ à C₁₂, alkylaminylalkyle en C₁ à C₁₂, alcoxyalkyle en C₁ à C₁₂, alcoxycarbonyle en C₁ à C₁₂, alkylcarbonyloxy en C₁ à C₁₂, alkylcarbonyloxyalkyle en C₁ à C₁₂ ou alkylcarbonyle en C₁ à C₁₂ ;
R étant, à chaque apparition, indépendamment soit : (a) un groupe H ou alkyle en C₁ à C₁₂ ; soit (b) R conjointement à l'atome de carbone auquel il est lié est pris conjointement à un groupe R adjacent et l'atome de carbone auquel il est lié pour former une double liaison carbone-carbone ;
R¹ et R² ayant, à chaque apparition, respectivement la structure suivante : 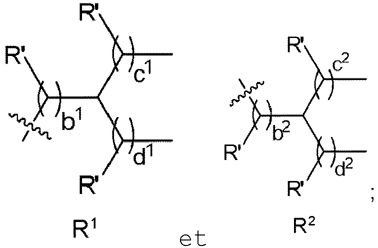
R' étant, à chaque apparition, indépendamment un groupe H ou alkyle en C₁ à C₁₂ ;
a¹ et a² étant, à chaque apparition, indépendamment un nombre entier d'une valeur de 3 à 12 ;
b¹ et b² ayant, à chaque apparition, indépendamment la valeur de 0 ou 1 ;
c¹ et c² étant, à chaque apparition, indépendamment un nombre entier d'une valeur de 2 à 12 ;
d¹ et d² étant, à chaque apparition, indépendamment un nombre entier d'une valeur de 2 à 12 ;
y étant, à chaque apparition, indépendamment un nombre entier d'une valeur de 0 à 2 ; et
n étant un nombre entier d'une valeur de 1 à 6,
a¹, a², c¹, c², d¹ et d² étant sélectionnés de sorte que la somme de a¹ + C¹ + d¹ est un nombre entier d'une valeur de 18 à 30, et que la somme de a² + c² + d² est un nombre entier d'une valeur de 18 à 30, et chaque groupe alkyle, alcylène, hydroxylalkyle, aminoalkyle, alkylaminylalkyle, alcoxyalkyle, alcoxycarbonyle, alkylcarbonyloxy, alkylcarbonyloxyalkyle et alkylcarbonyle est éventuellement substitué par un ou plusieurs substituants ; ou
vi) un lipide cationique ayant la Formule (VI) suivante : 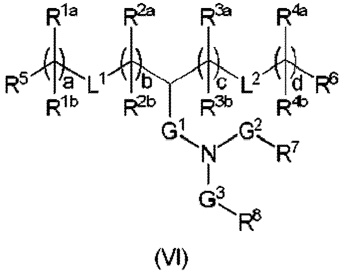 ou son sel, tautomère ou stéréoisomère pharmaceutiquement acceptable :
L¹ et L² étant chacun indépendamment un groupe - O(C=O)-, -(C=O)O-, -C(=O)-, -O-, -S(O)ₓ-, -S-S-, -C(=O)S-, -SC(=O)-, -NR^{a}C(=O)-, -C(=O)NR^{a}-, -NR^{a}C(=O) NR^{a}-, - OC(=O)NR^{a}-, -NR^{a}C(=O)O- ou une liaison directe ;
G¹ étant un groupe alcylène en C₁ à C2, -(C=O)-, - O(C=O)-, -SC(=O)-, -NR^{a}C(=O)- ou une liaison directe ;
G² étant un groupe -C(=O)-, -(C=O)O-, -C(=O)S-, - C(=O)NR^{a}- ou une liaison directe ;
G³ étant un groupe alcylène en C₁ à C₆ ;
R^{a} étant un groupe H ou alkyle en C₁ à C₁₂ ;
R^{1a} et R^{1b} étant, à chaque apparition, indépendamment soit : (a) un groupe H ou alkyle en C₁ à C₁₂ ; soit (b) R^{1a} étant un groupe H ou alkyle en C₁ à C₁₂, et R^{1b} conjointement à l'atome de carbone auquel il est lié est pris conjointement à un groupe R^{1b} adjacent et l'atome de carbone auquel il est lié pour former une double liaison carbone-carbone ;
R^{2a} et R^{2b} étant, à chaque apparition, indépendamment soit : (a) un groupe H ou alkyle en C₁ à C₁₂ ; soit (b) R^{2a} étant un groupe H ou alkyle en C₁ à C₁₂, et R^{2b} conjointement à l'atome de carbone auquel il est lié est pris conjointement à un groupe R^{2b} adjacent et l'atome de carbone auquel il est lié pour former une double liaison carbone-carbone ;
R^{3a} et R^{3b} étant, à chaque apparition, indépendamment soit : (a) un groupe H ou alkyle en C₁ à C₁₂ ; soit (b) R^{3a} étant un groupe H ou alkyle en C₁ à C₁₂, et R^{3b} conjointement à l'atome de carbone auquel il est lié est pris conjointement à un groupe R^{3b} adjacent et l'atome de carbone auquel il est lié pour former une double liaison carbone-carbone ;
R^{4a} et R^{4b} étant, à chaque apparition, indépendamment soit : (a) un groupe H ou alkyle en C₁ à C₁₂ ; soit (b) R^{4a} étant un groupe H ou alkyle en C₁ à C₁₂, et R^{4b} conjointement à l'atome de carbone auquel il est lié est pris conjointement à un groupe R^{4b} adjacent et l'atome de carbone auquel il est lié pour former une double liaison carbone-carbone ;
R⁵ et R⁶ étant chacun indépendamment un groupe H ou méthyle ;
R⁷ étant un groupe H ou alkyle en C₁ à C₂₀ ;
R⁸ étant un groupe OH, -N(R⁹) (C=O)R¹⁰, -(C=O)NR⁹R¹⁰, - NR⁹R¹⁰, -(C=O)OR¹¹ ou -O(C=O)R¹¹, à condition que G³ est un groupe alcylène en C₄ à C₆ lorsque R⁸ est un groupe -NR⁹R¹⁰,
R⁹ et R¹⁰ étant chacun indépendamment un groupe H ou alkyle en C₁ à C₁₂ ;
R¹¹ étant groupe aralkyle ;
a, b, c et d étant chacun indépendamment un nombre entier d'une valeur de 1 à 24 ; et
x ayant la valeur de 0, 1 ou 2,
chaque groupe alkyle, alcylène et aralkyle étant éventuellement substitué ; ou
vii) le lipide cationique ayant la Formule (VII) suivante : 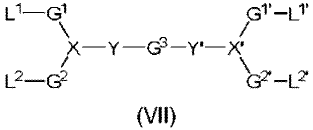 ou son sel ou stéréoisomère pharmaceutiquement acceptable :
X et X' étant chacun indépendamment un groupe N ou CR ;
Y et Y' étant chacun indépendamment absents, -O(C=O)-, -(C=O)O- ou NR, à condition que :
a) Y est absent lorsque X est un groupe N ;
b) Y' est absent lorsque X' est un groupe N ;
c) Y est un groupe -O(C=O)-, -(C=O)O- ou NR lorsque X est un groupe CR ; et
d) Y' est un groupe -O(C=O)-, -(C=O)O- ou NR lorsque X' est un groupe CR,
L¹ et L¹' étant chacun indépendamment un groupe - O(C=O)R¹, -(C=O)OR¹, -C(=O)R¹, -OR¹, -S(O)_{z}R¹, -S-SR¹, - C(=O)SR¹, -SC(=O) R¹, -NR^{a}C(=O) R¹, -C(=O) NR^{b}R^{c}, - NR^{a}C (=O) NR^{b}R^{c}, -OC(=O) NR^{b}R^{c} ou -NR^{a}C(=O)OR¹ ;
L² et L²' étant chacun indépendamment un groupe - O(C=O)R², -(C=O)OR², -C(=O)R², -OR², -S(O)_{z}R², -S-SR², - C(=O)SR², -SC(=O)R², -NR^{d}C(=O)R², -C(=O)NR^{e}R^{f}, - NR^{d}C (=O) NR^{e}R^{f}, -OC(=O) NR^{e}R^{f} ; -NR^{d}C (=O) OR² ou une liaison directe à R² ;
G¹, G^{1'}, G² et G^{2'} étant chacun indépendamment un groupe alcylène en C₂ à C₁₂ ou alcénylène en C₂ à C₁₂ ;
G³ étant un groupe hétéroalcylène en C₂ à C₂₄ ou hétéroalcénylène en C₂ à C₂₄ ;
R^{a}, R^{b}, R^{d} et R^{e} étant, à chaque apparition, indépendamment un groupe H, alkyle en C₁ à C₁₂ ou alcényle en C₂ à C₁₂ ;
R^{c} et R^{f} étant, à chaque apparition, indépendamment un groupe alkyle en C₁ à C₁₂ ou alcényle en C₂ à C₁₂ ;
R étant, à chaque apparition, indépendamment un groupe H ou alkyle en C₁ à C₁₂ ;
R¹ et R² étant, à chaque apparition, indépendamment un groupe alkyle en C₆ à C₂₄ ramifié ou alcényle en C₆ à C₂₄ ramifié ;
z ayant la valeur de 0, 1 ou 2, et
chaque groupe alkyle, alcényle, alcylène, alcénylène, hétéroalcylène et hétéroalcénylène étant indépendamment substitué ou non substitué sauf autrement spécifié ; ou
viii) le lipide cationique ayant la Formule (VIII) suivante : 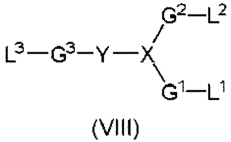 ou son sel ou stéréoisomère pharmaceutiquement acceptable :
X étant N, et Y étant absent ; ou X étant un groupe CR, et Y étant un groupe NR ;
L¹ étant un groupe -O(C=O)R¹, -(C=O)OR¹, -C(=O)R², - OR¹, -S(O)ₓR², -S-SR¹, -C(=O)SR¹, -SC(=O)R¹, -NR^{a}C(=O)R¹, - C(=O)NR^{b}R^{c}, -NR^{a}C(=O) NR^{b}R^{c}, -OC(=O)NR^{b}R^{c} ou -NR^{a}C(=O) OR¹ ;
L² étant un groupe -O(C=O)R², -(C=O)OR², -C(=O)R², - OR², -S(O)ₓR², -S-SR², -C(=O)SR², -SC(=O)R², -NR^{d}C(=O)R², - C(=O)NR^{e}R^{f}, -NR^{d}C (=O) NR^{e}R^{f}, -OC(=O)NR^{e}R^{f} ; -NR^{d}C (=O) OR² ou une liaison directe à R² ;
L³ étant un groupe -O(C=O)R³ ou -(C=O)OR³ ;
G¹ et G² étant chacun indépendamment un groupe alcylène en C₂ à C₁₂ ou alcénylène en C₂ à C₁₂ ;
G³ étant un groupe alcylène en C₂ à C₂₄, alcénylène en C₂ à C₂₄, hétéroalcylène en C₂ à C₂₄ ou hétéroalcénylène en C₂ à C₂₄ lorsque X est un groupe CR, et Y est un groupe NR ; et G³ étant un groupe hétéroalcylène en C₂ à C₂₄ ou hétéroalcénylène en C₂ à C₂₄ lorsque X est un groupe N, et Y est absent ;
R^{a}, R^{b}, R^{d} et R^{e} étant chacun indépendamment un groupe H ou alkyle en C₁ à C₁₂ ou alcényle en C₁ à C₁₂ ;
R^{c} et R^{f} étant chacun indépendamment un groupe alkyle en C₁ à C₁₂ ou alcényle en C₂ à C₂₂ ;
chaque R étant indépendamment un groupe H ou alkyle en C₁ à C₁₂ ;
R¹, R² et R³ étant chacun indépendamment un groupe alkyle en C₁ à C₂₄ ou alcényle en C₂ à C₂₄ ; et
x ayant la valeur de 0, 1 ou 2, et
chaque groupe alkyle, alcényle, alcylène, alcénylène, hétéroalcylène et hétéroalcénylène étant indépendamment substitué ou non substitué sauf autrement spécifié ; ou
ix) le lipide cationique ayant la Formule (IX) suivante : 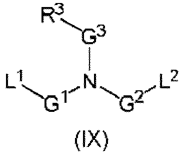 ou son sel ou stéréoisomère pharmaceutiquement acceptable :
L¹ étant un groupe -O(C=O)R¹, -(C=O)OR¹, -C(=O)R¹, - OR¹, -S(O)ₓR¹, -S-SR¹, -C(=O)SR¹, -SC(=O)R¹, -NR^{a}C(=O)R¹, - C(=O) NR^{b}R^{c}, -NR^{a}C(=O) NR^{b}R^{c}, -OC(=O) NR^{b}R^{c} ou -NR^{a}C(=O) OR¹ ;
L² étant un groupe -O(C=O)R², -(C=O)OR², -C(=O)R², - OR², -S(O)ₓR², -S-SR², -C(=O)SR², -SC(=O)R², -NR^{d}C(=O)R², - C(=O)NR^{e}R^{f}, -NR^{d}C(=O)NR^{e}R^{f}, -OC(=O)NR^{e}R^{f} ; -NR^{d}C(=O)OR² ou une liaison directe à R² ;
G¹ et G² étant chacun indépendamment un groupe alcylène en C₂ à C₁₂ ou alcénylène en C₂ à C₁₂ ;
G³ étant un groupe alcylène en C₂ à C₂₄, alcénylène en C₂ à C₂₄, cycloalcylène en C₃ à C₈ ou cycloalcénylène en C₃ à C₈ ;
R^{a}, R^{b}, R^{d} et R^{e} étant chacun indépendamment un groupe H ou alkyle en C₁ à C₁₂ ou alcényle en C₁ à C₁₂ ;
R^{c} et R^{f} étant chacun indépendamment un groupe alkyle en C₁ à C₁₂ ou alcényle en C₂ à C₁₂ ;
R¹ et R² étant chacun indépendamment un groupe alkyle en C₆ à C₂₄ ramifié ou alcényle en C₆ à C₂₄ ramifié ;
R³ étant un groupe -N(R⁴)R⁵ ;
R⁴ étant un groupe alkyle en C₁ à C₁₂ ;
R⁵ étant un groupe alkyle en C₁ à C₁₂ substitué ; et
x ayant la valeur de 0, 1 ou 2, et
chaque groupe alkyle, alcényle, alcylène, alcénylène, cycloalcylène, cycloalcénylène, aryle et aralkyle étant indépendamment substitué ou non substitué sauf autrement spécifié ; ou
x) le lipide cationique ayant la Formule (X) suivante : 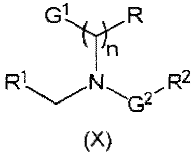 ou son sel ou stéréoisomère pharmaceutiquement acceptable :
G¹ étant un groupe -OH, -NR³R⁴, -(C=O)NR⁵ ou - NR³(C=O)R⁵ ;
G² étant un groupe -CH₂- ou -(C=O) ;
R étant, à chaque apparition, indépendamment un groupe H ou OH ;
R¹ et R² étant chacun indépendamment un groupe alkyle en C₁₂ à C₃₆ ramifié, saturé ou insaturé éventuellement substitué ;
R³ et R⁴ étant chacun indépendamment un groupe H ou un groupe alkyle en C₁ à C₆ linéaire ou ramifié, saturé ou insaturé éventuellement substitué ;
R⁵ étant un groupe alkyle en C₁ à C₆ linéaire ou ramifié, saturé ou insaturé éventuellement substitué ; et
n étant un nombre entier d'une valeur de 2 à 6.

5. NPL pour l'utilisation selon l'une quelconque des revendications 1 à 4, le lipide cationique étant formé à partir d'une structure lipidique sélectionnée parmi :

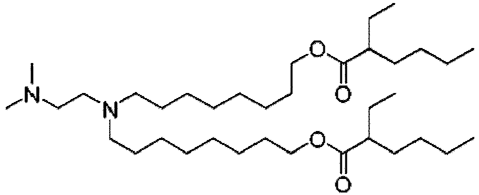

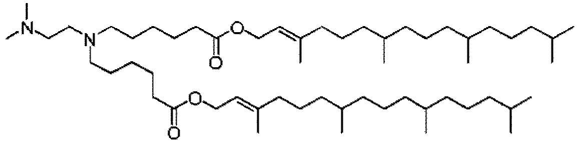

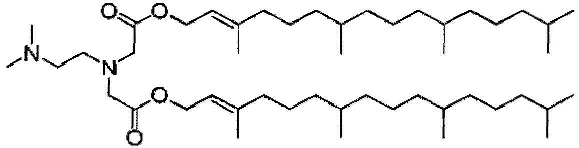

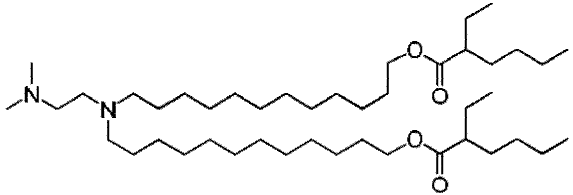

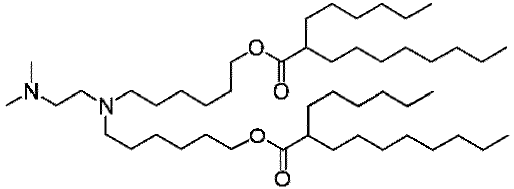

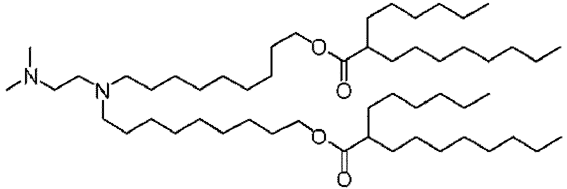

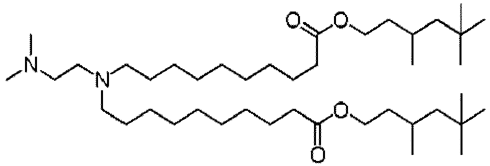

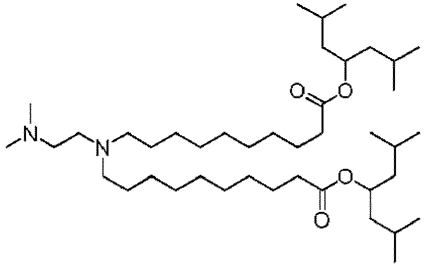

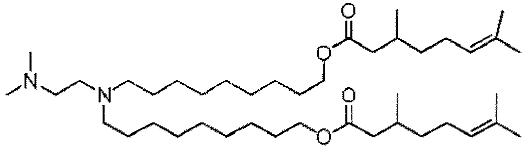

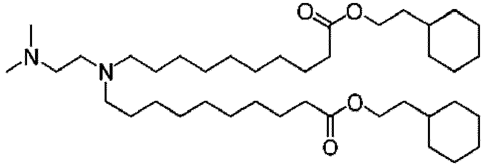

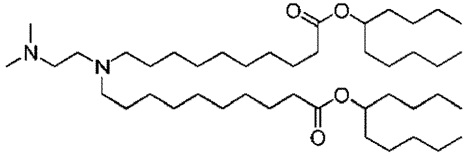

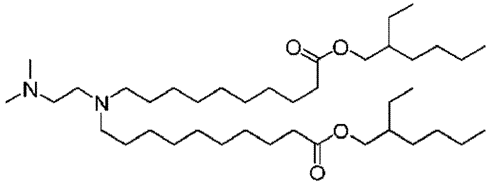

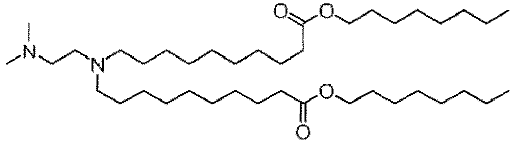

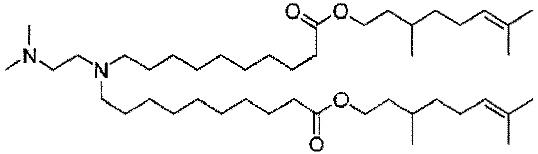

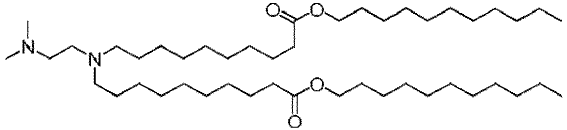

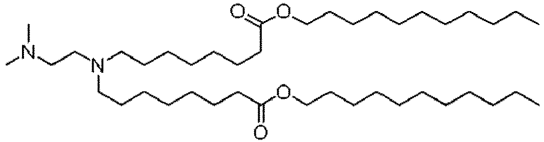

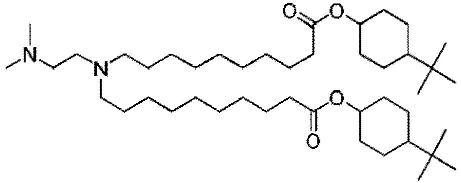

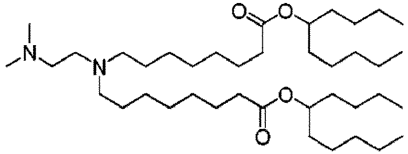

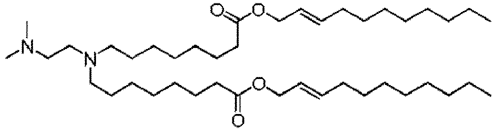

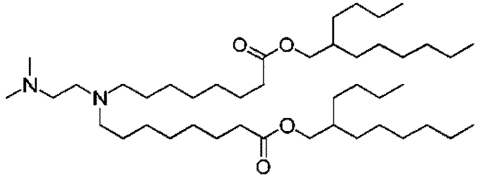

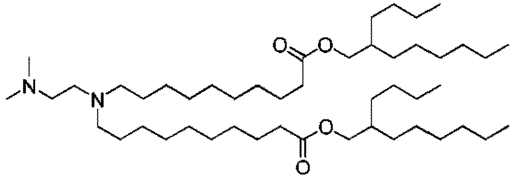

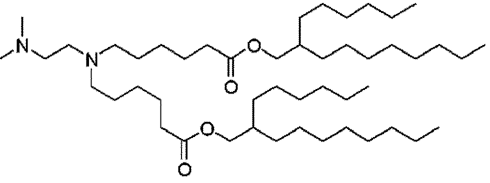

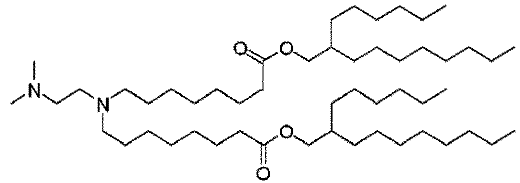

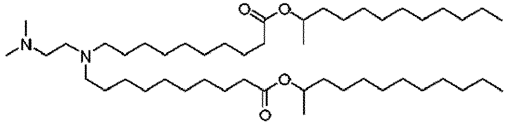

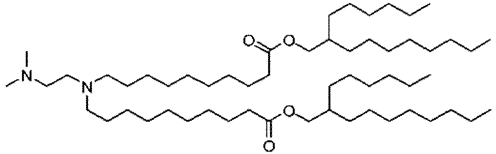

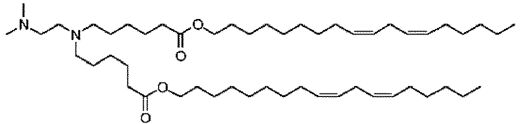

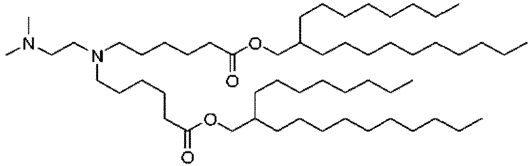

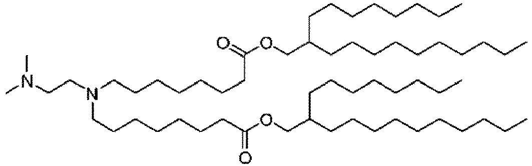

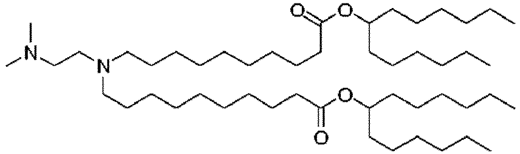

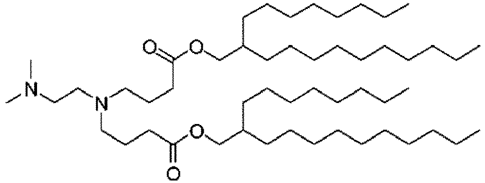

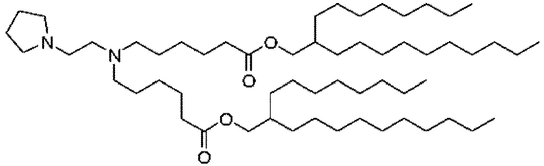

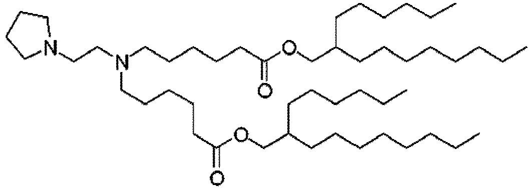

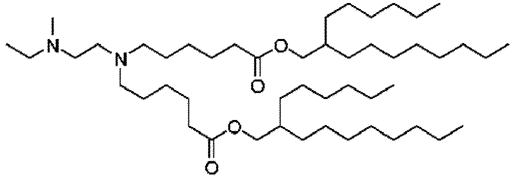

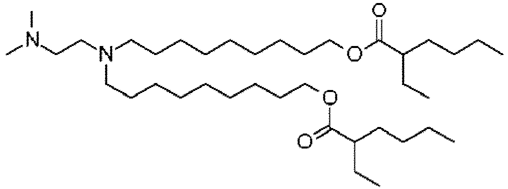

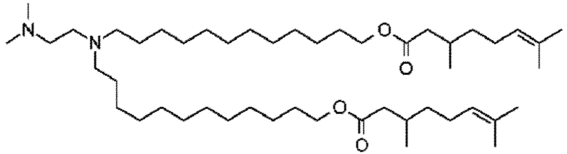

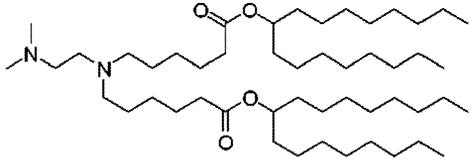

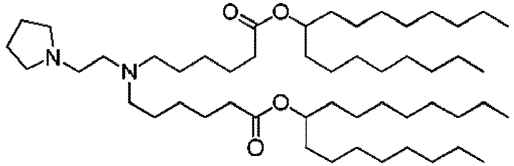

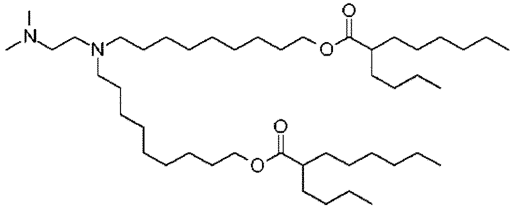

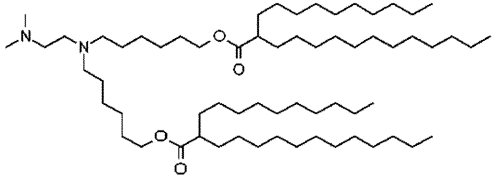

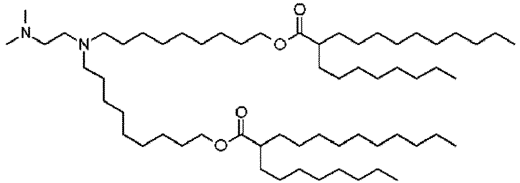

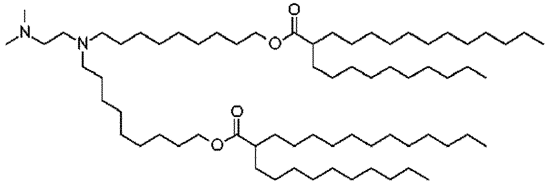

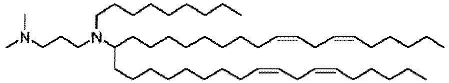

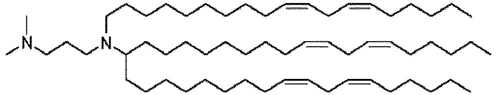

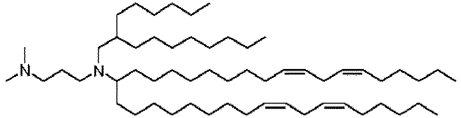

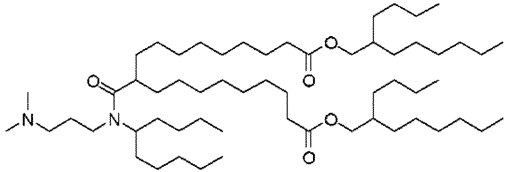

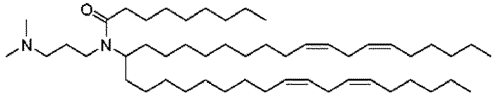

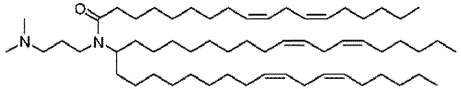

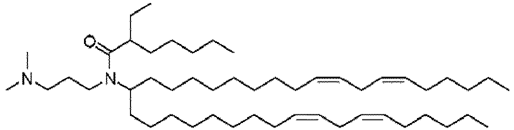

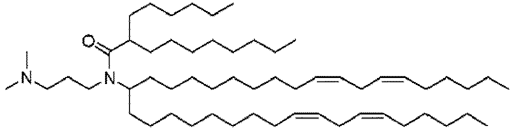

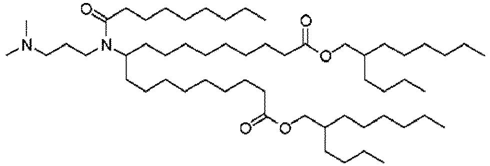

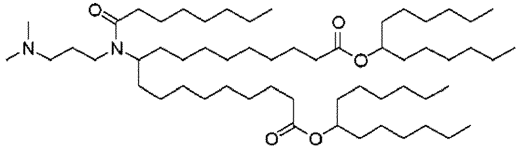

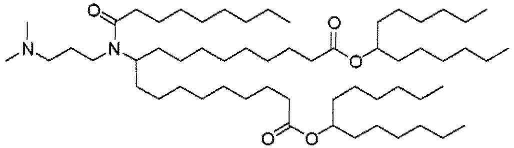

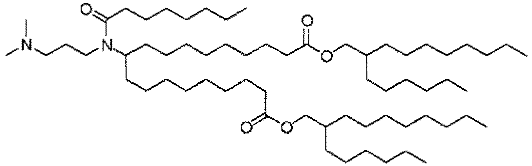

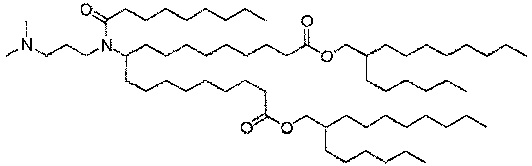

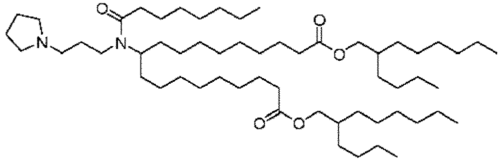

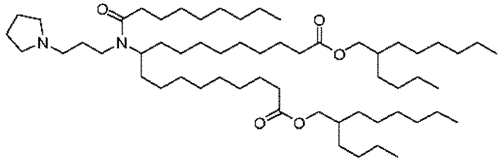

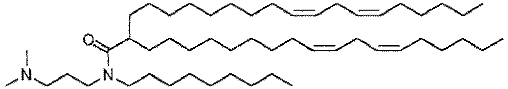

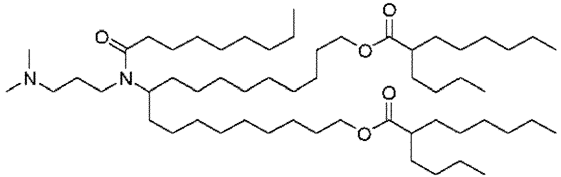

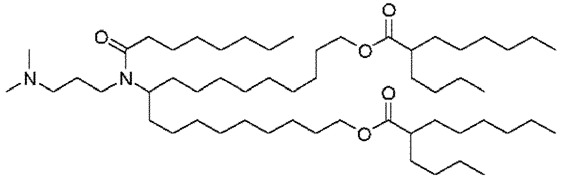

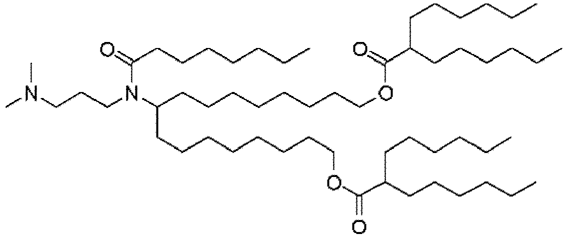

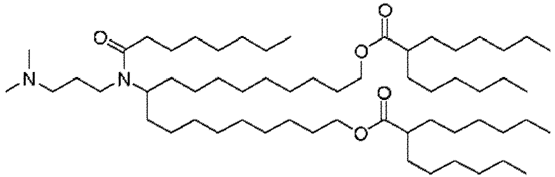

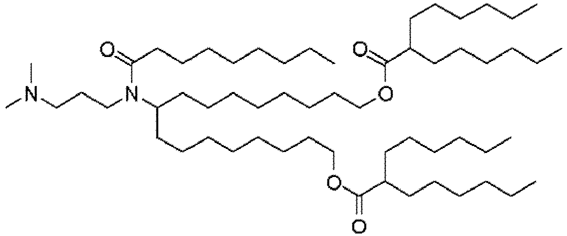

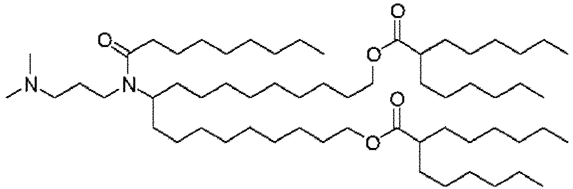

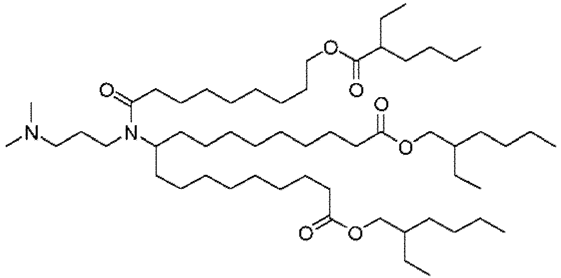

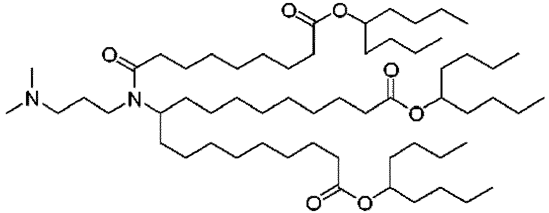

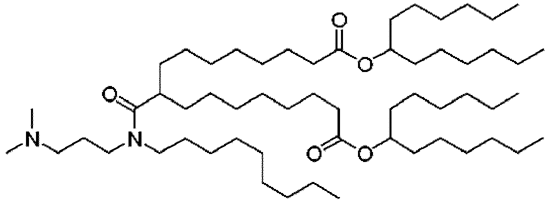

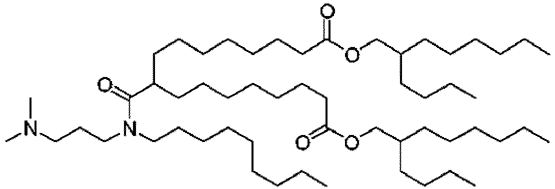

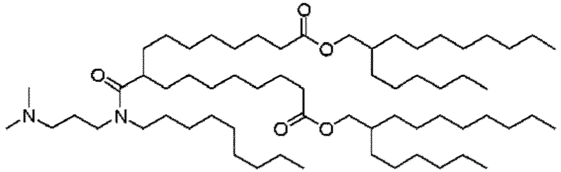

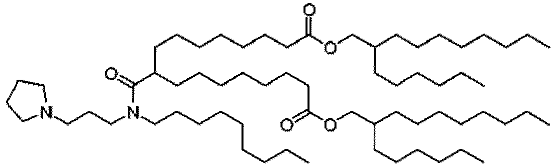

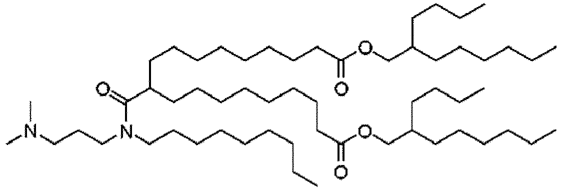

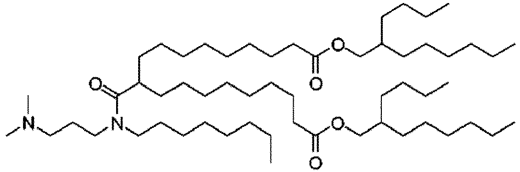

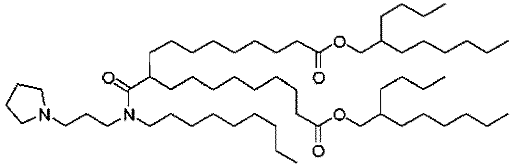

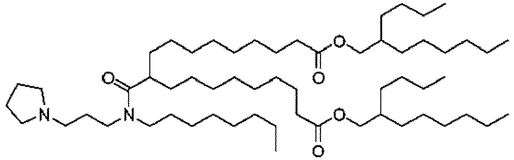

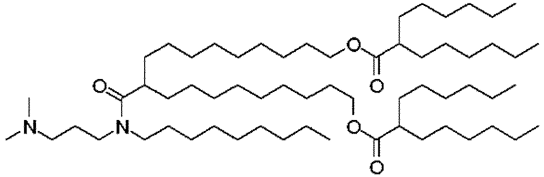

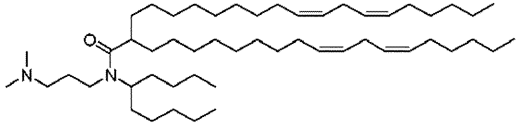

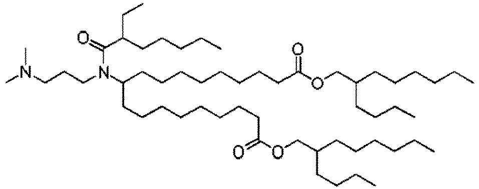

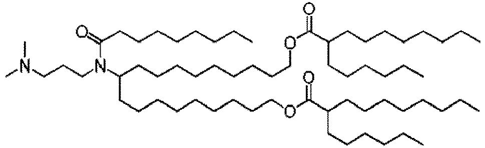

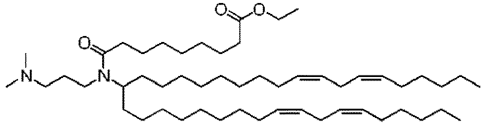

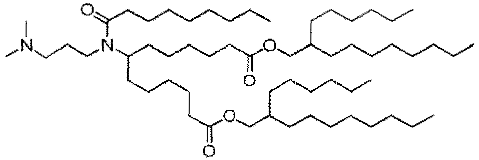

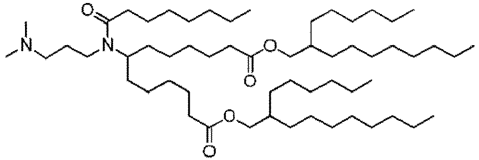

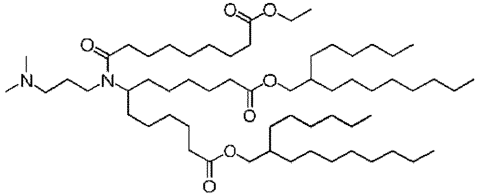

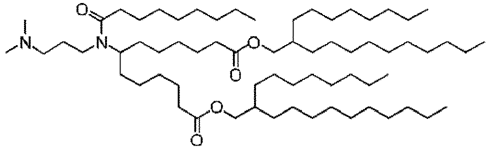

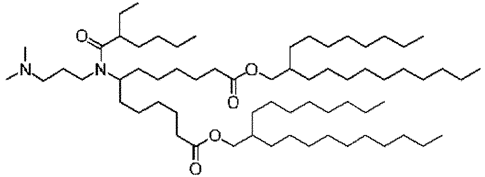

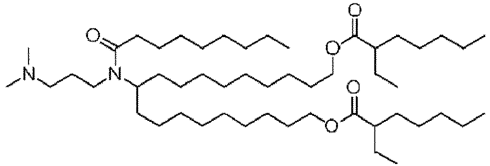

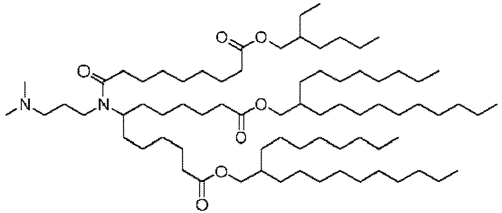

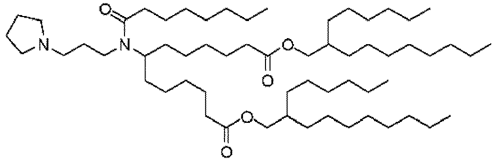

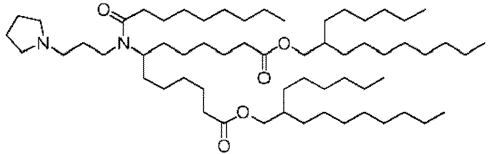

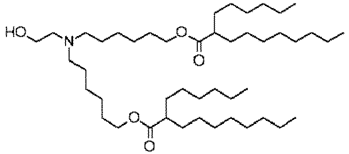

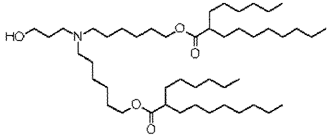

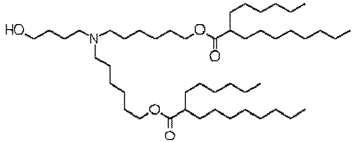

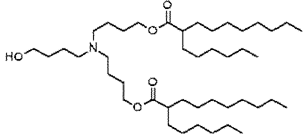

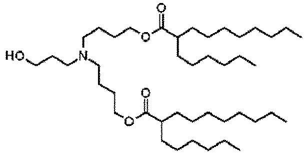

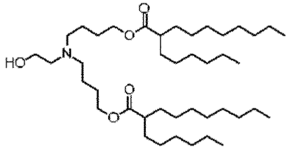

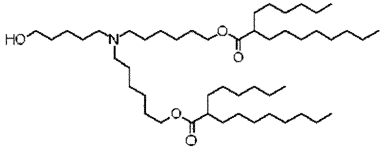

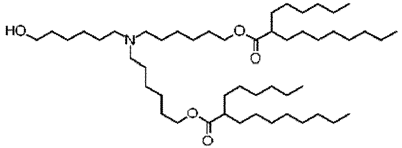

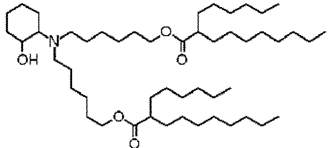

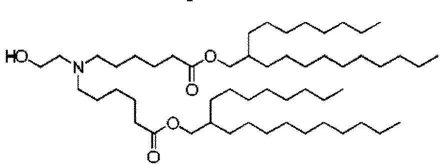

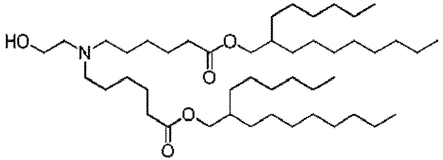

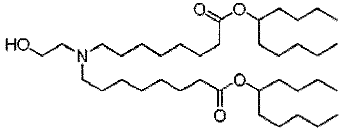

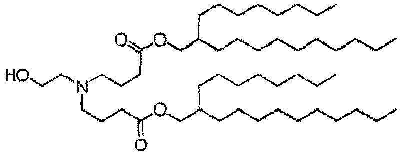

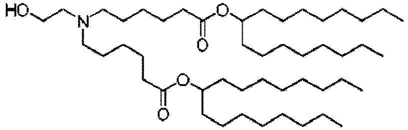

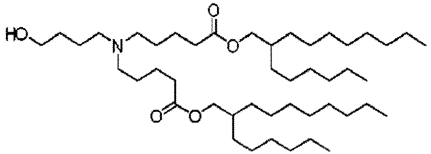

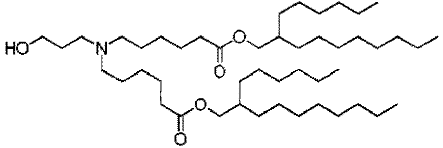

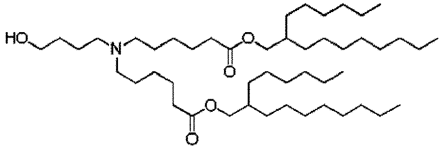

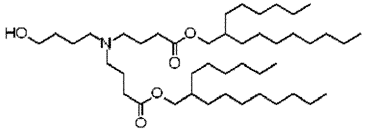

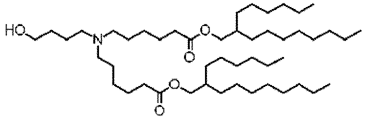

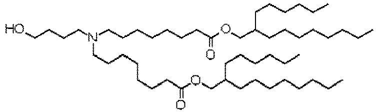

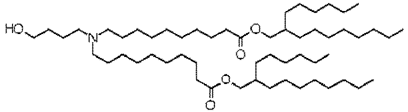

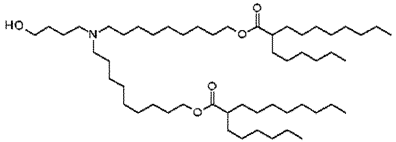

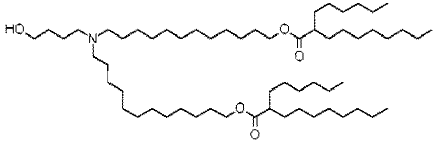

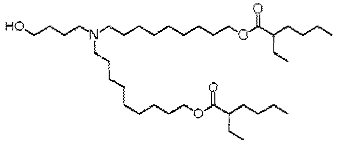

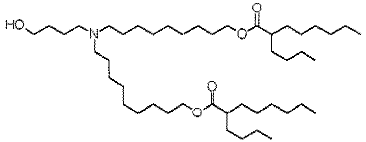

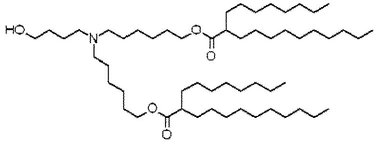

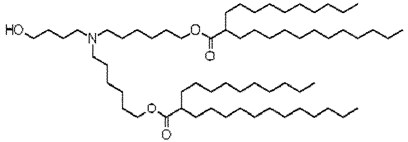

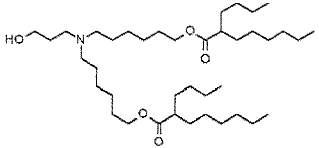

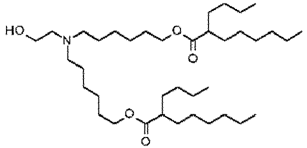

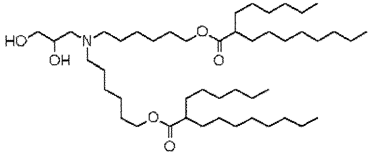

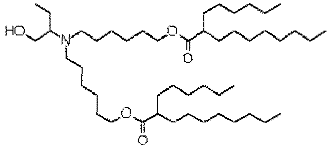

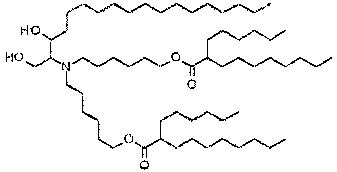

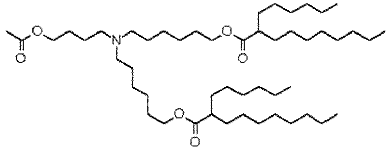

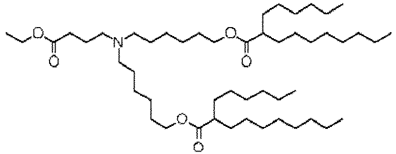

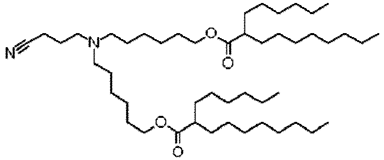

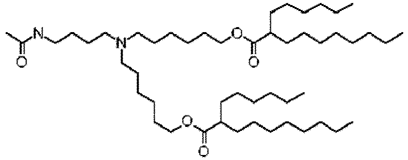

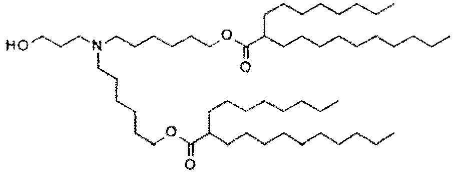

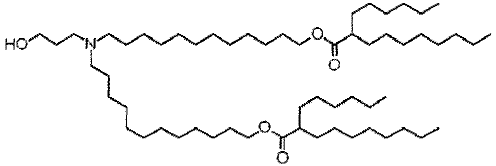

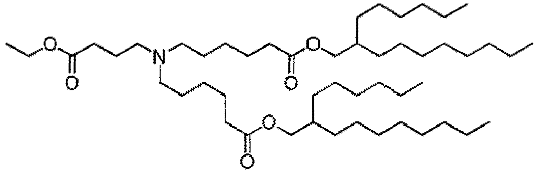

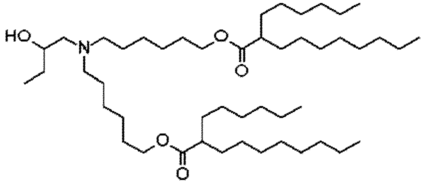

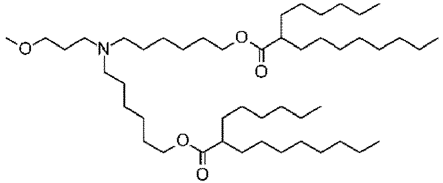

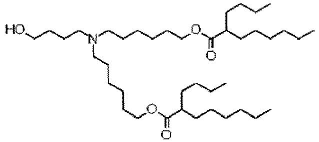

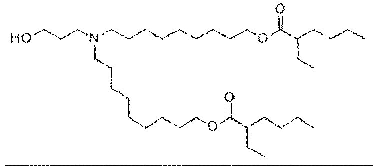

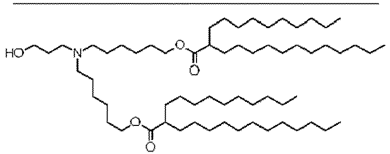

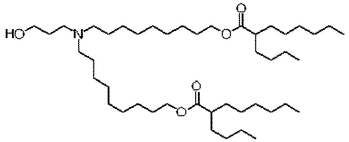

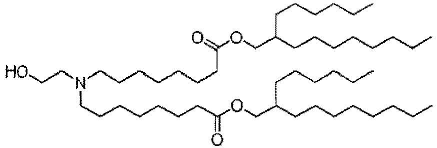

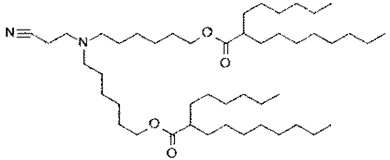

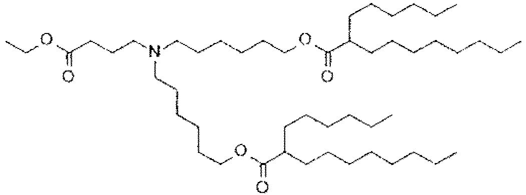

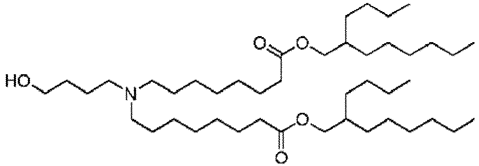

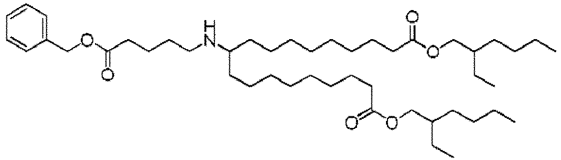

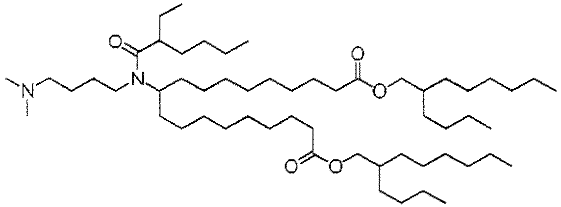

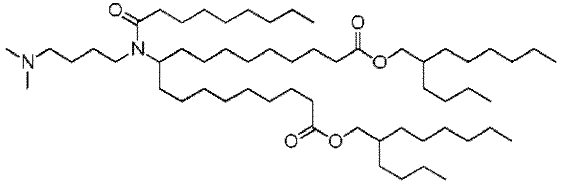

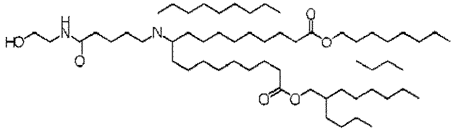

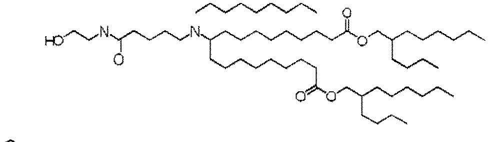

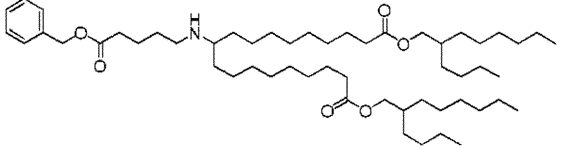

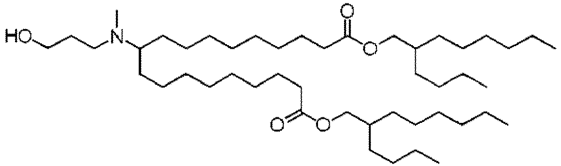

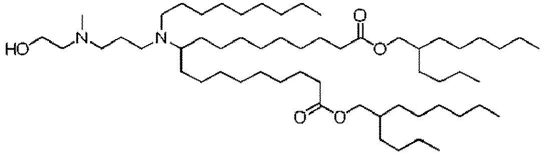

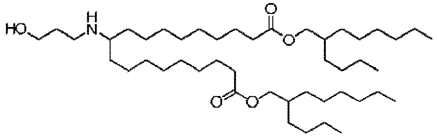

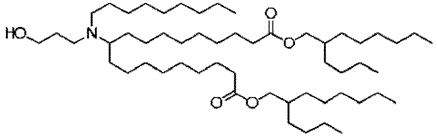

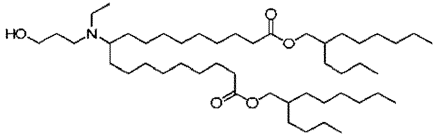

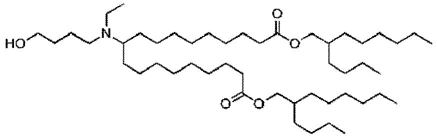

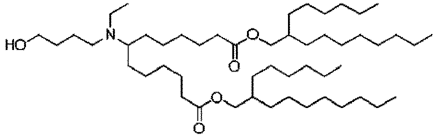

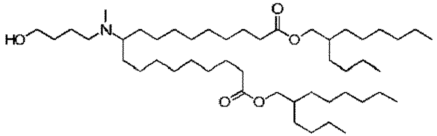

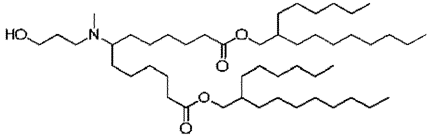

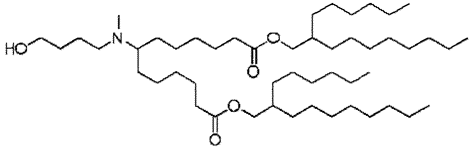

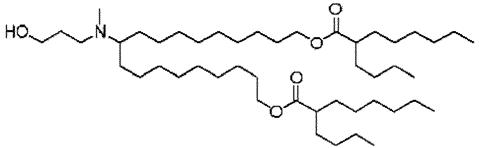

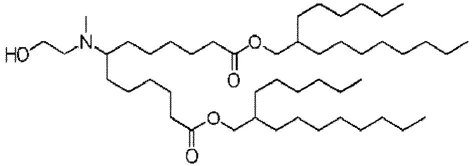

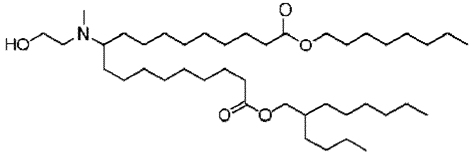

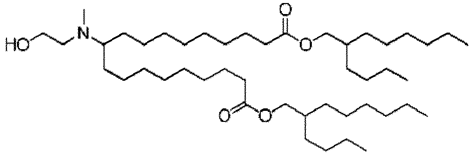

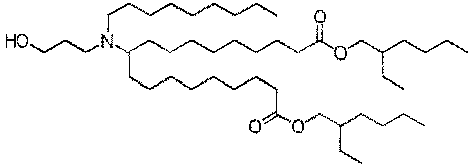

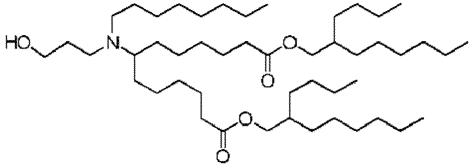

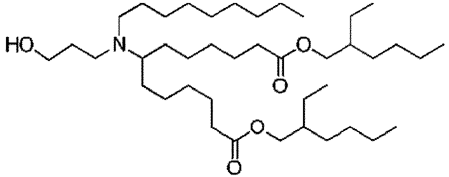

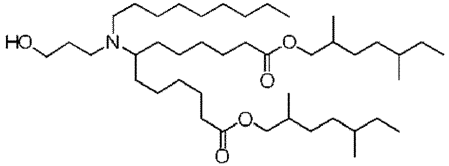

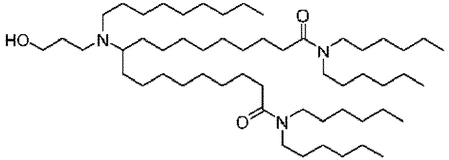

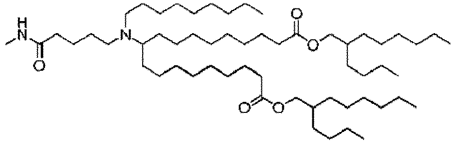

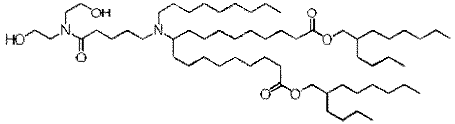

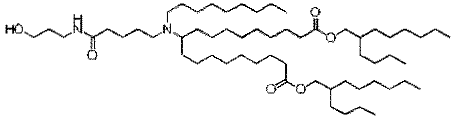

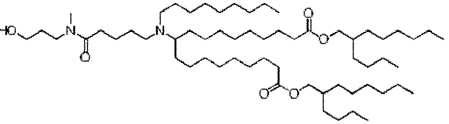

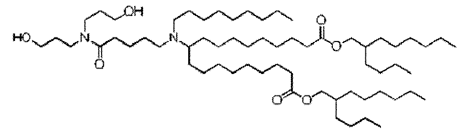

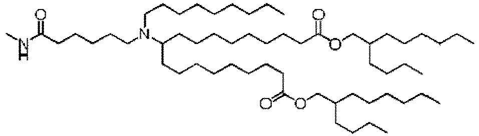

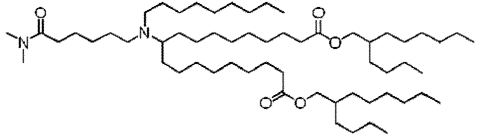

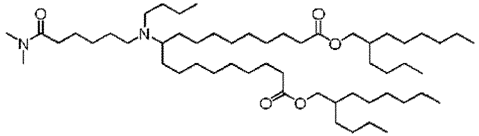

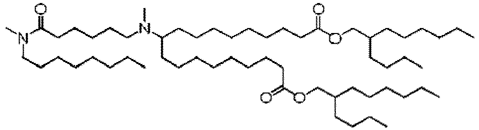

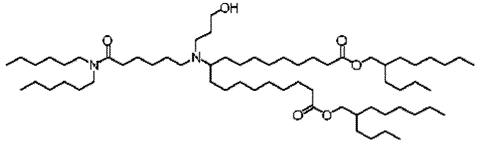

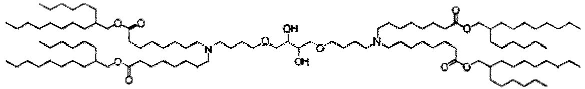

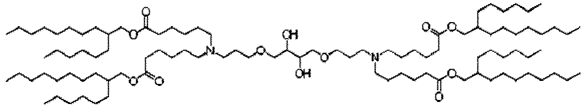

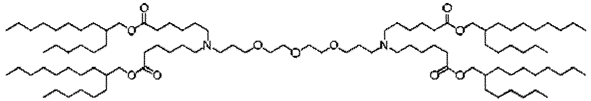

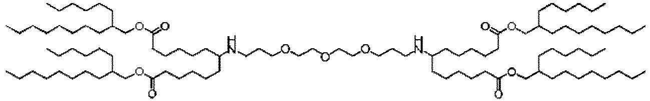

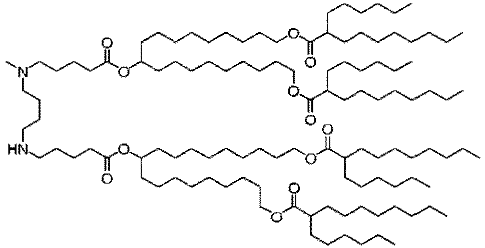

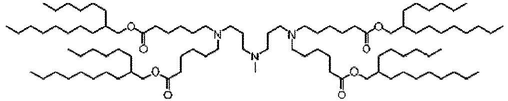

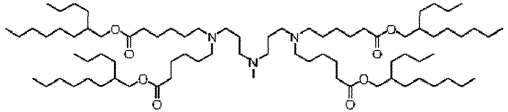

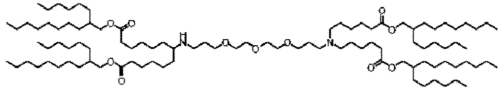

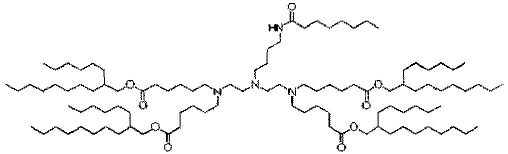

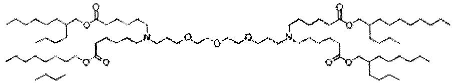

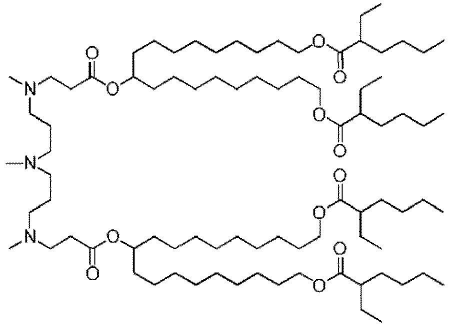

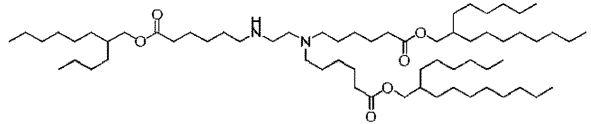

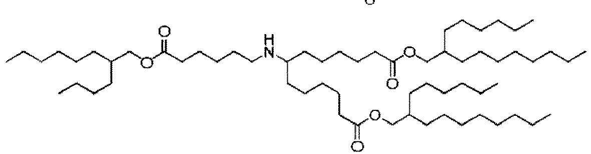

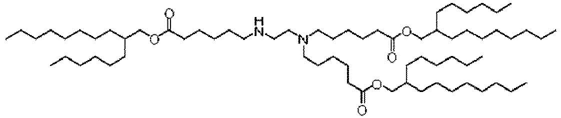

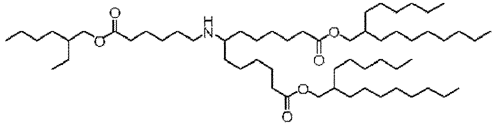

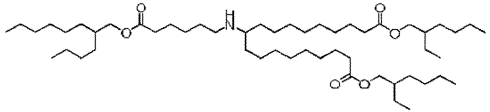

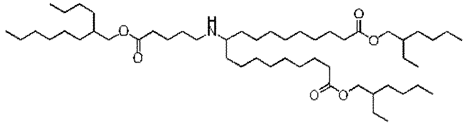

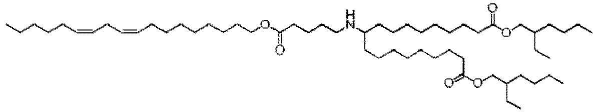

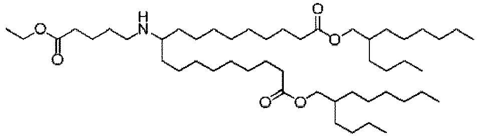

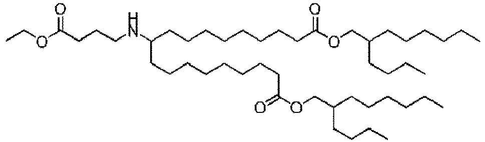

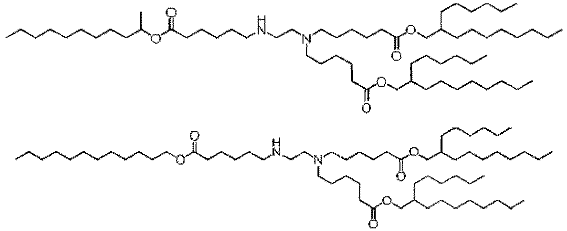

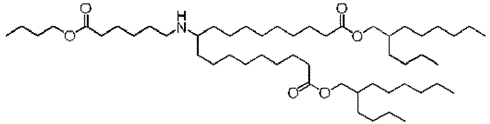

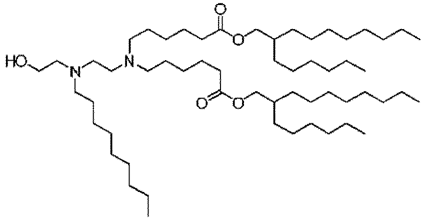

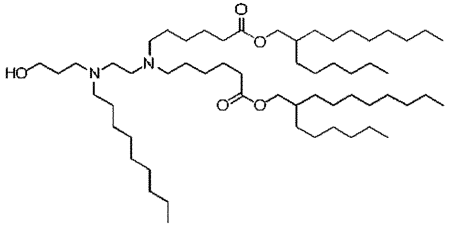

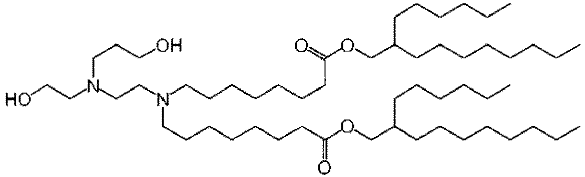

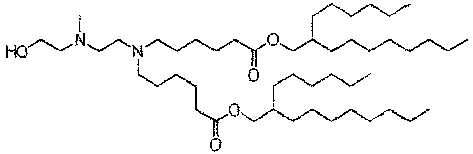

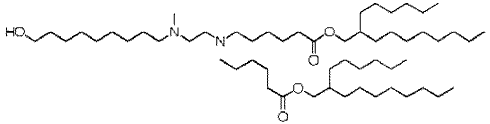

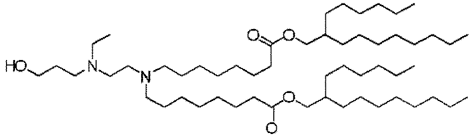

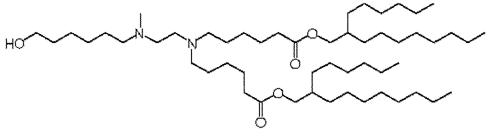

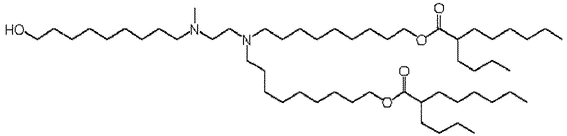

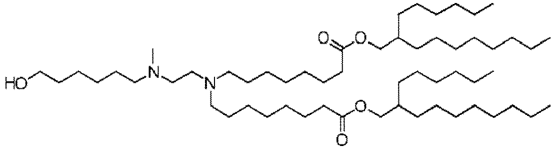

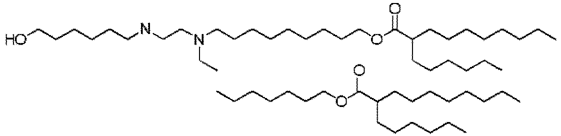

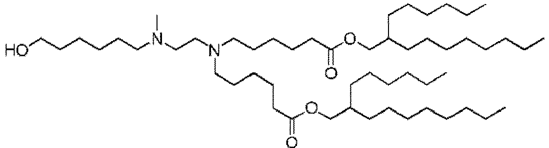

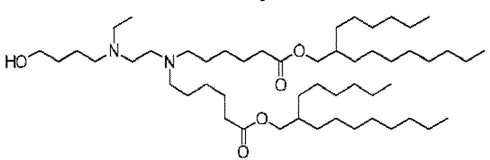

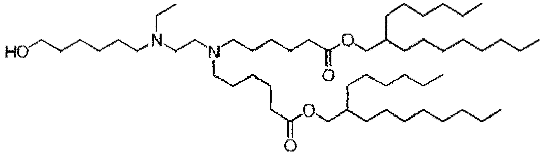

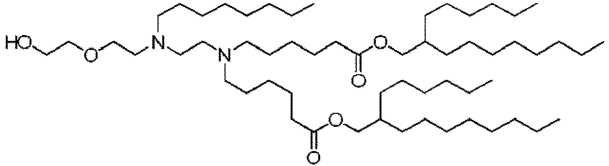

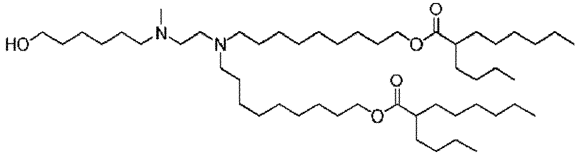

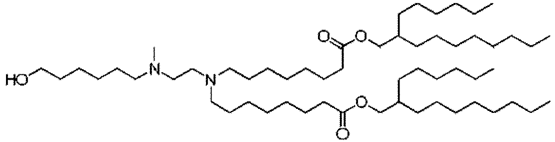

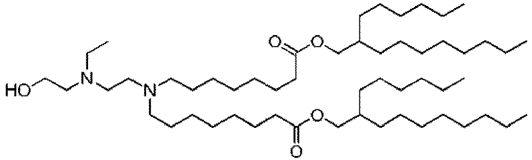

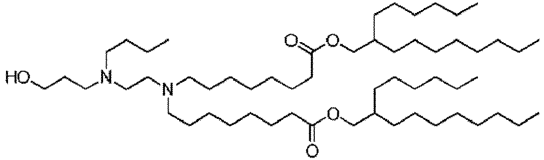

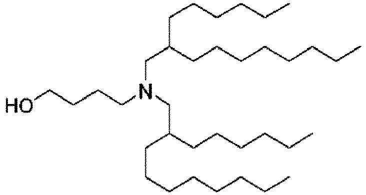

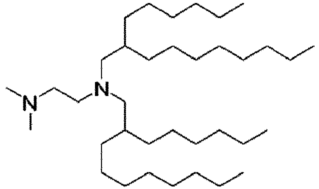

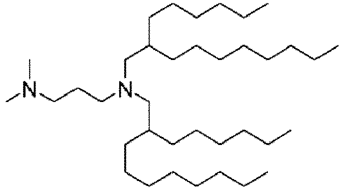

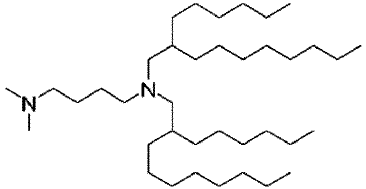

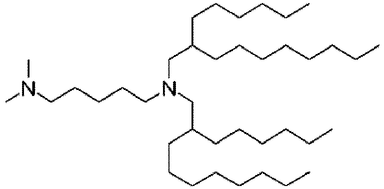

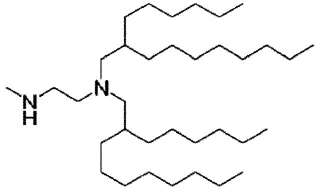

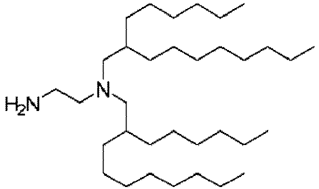

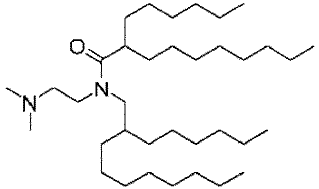

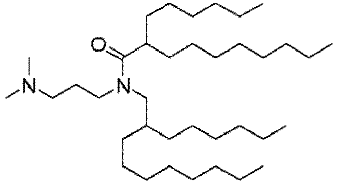

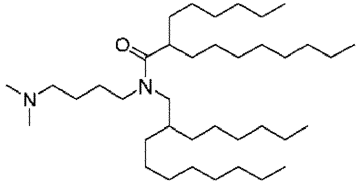

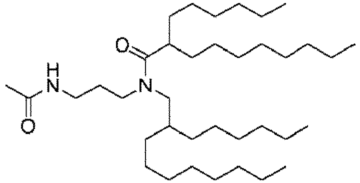

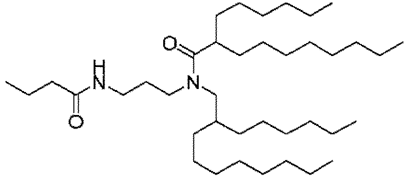

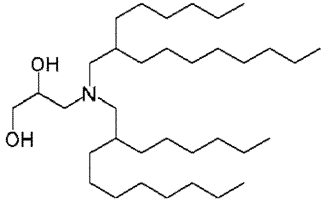

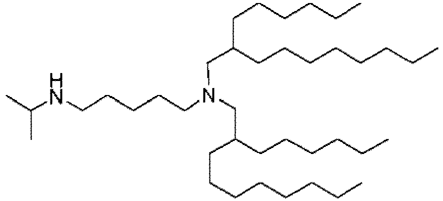

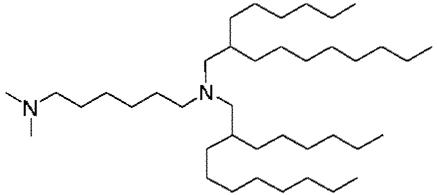

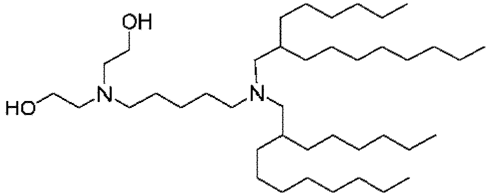

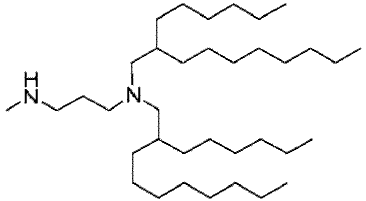

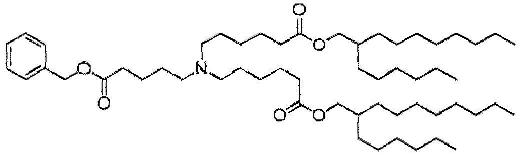

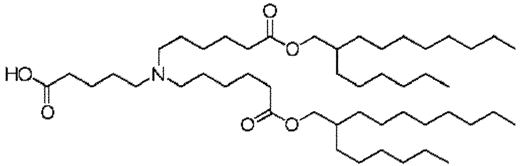

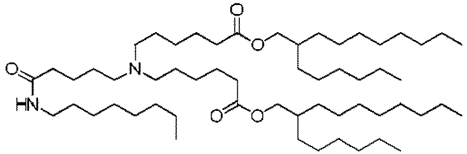

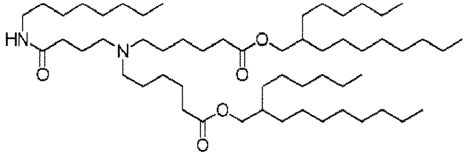

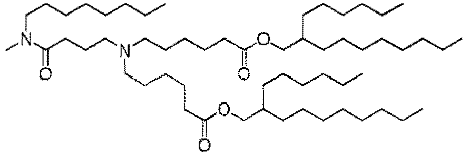

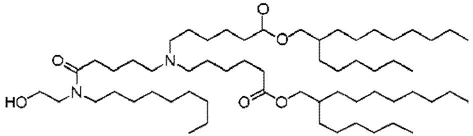

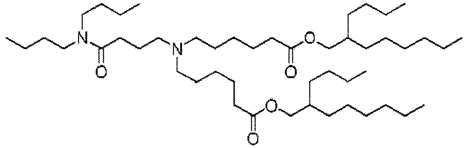

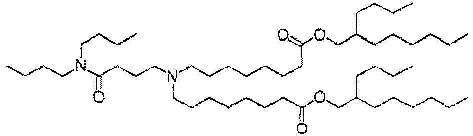

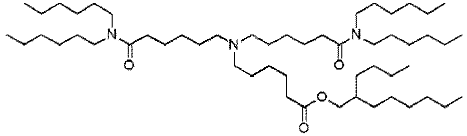

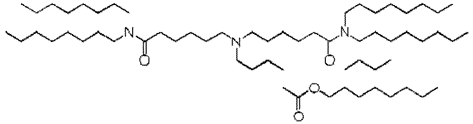

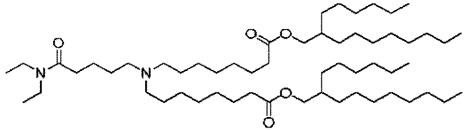

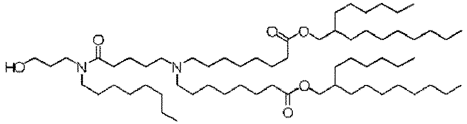

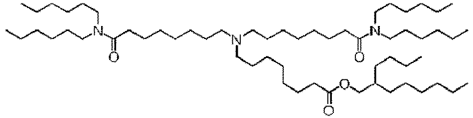

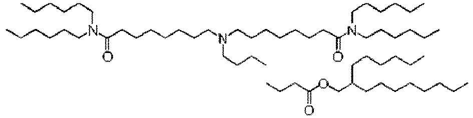

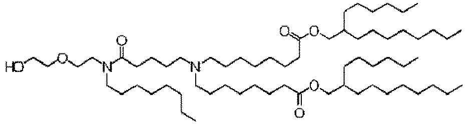

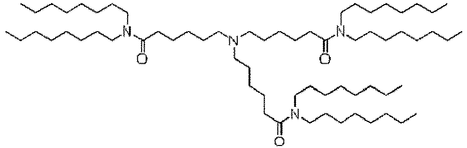

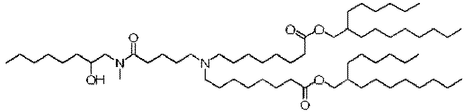

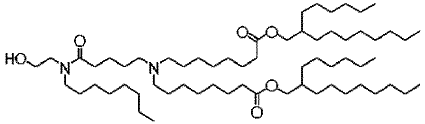

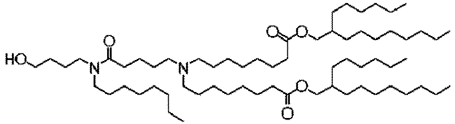

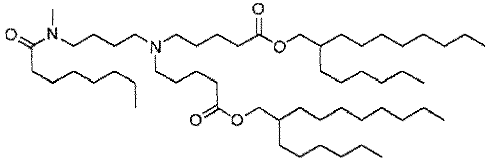

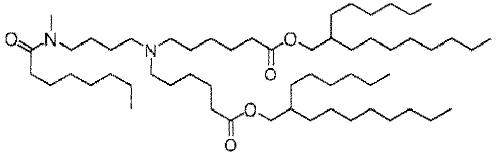

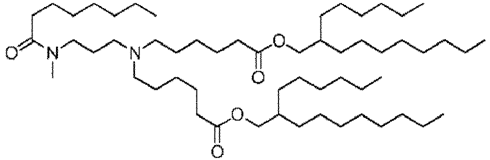

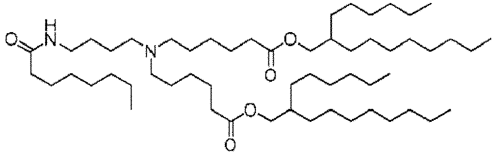

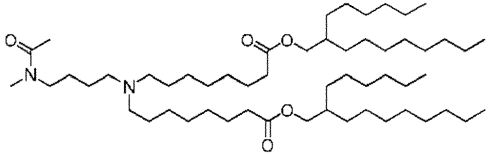

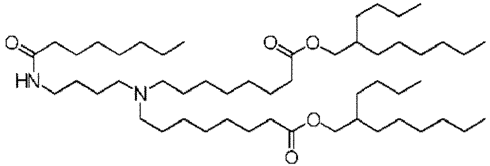

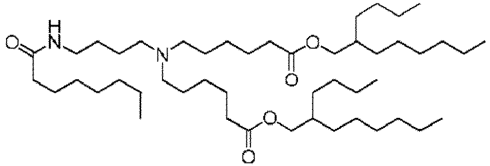

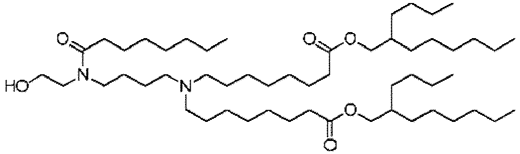

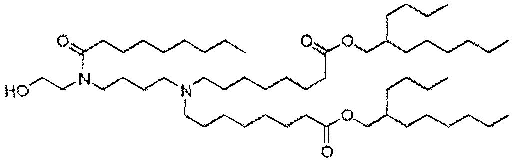

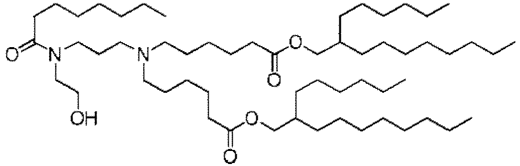

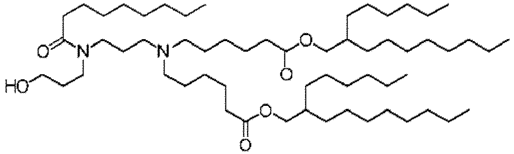

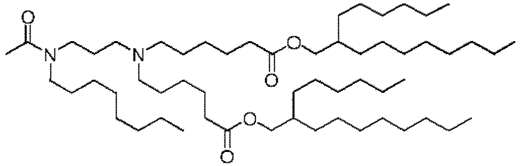

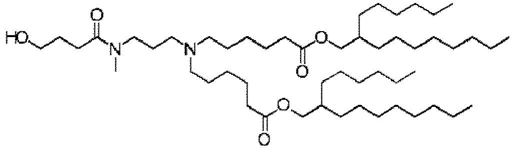

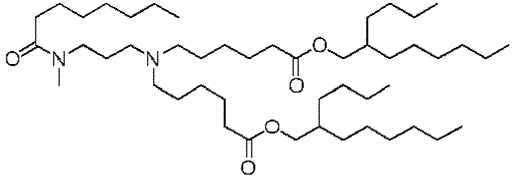

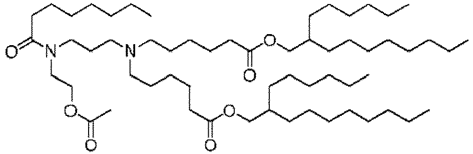

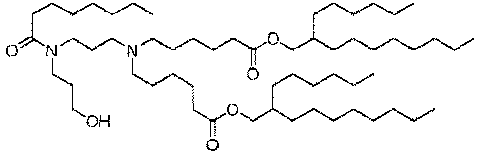

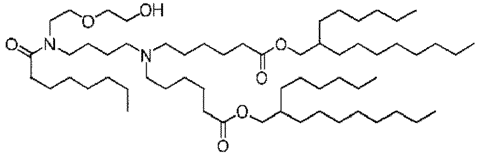

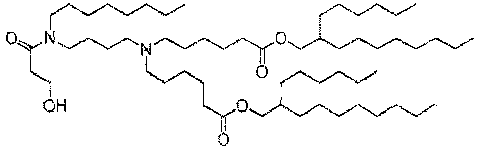

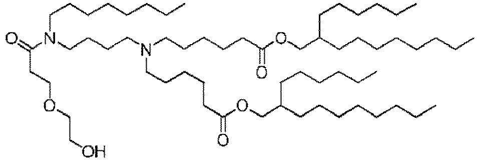

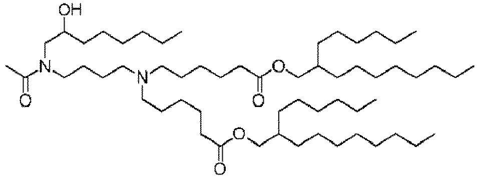

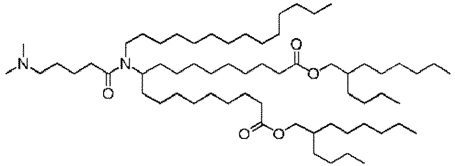

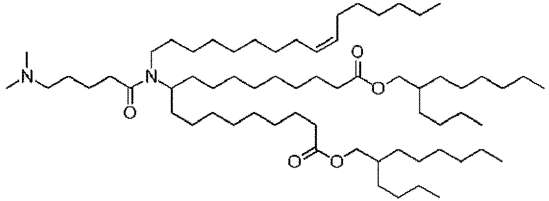

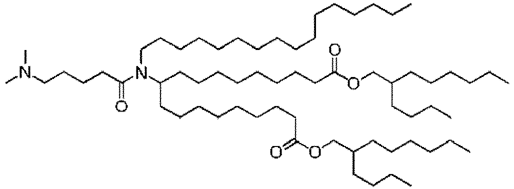

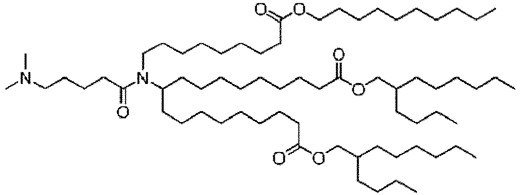

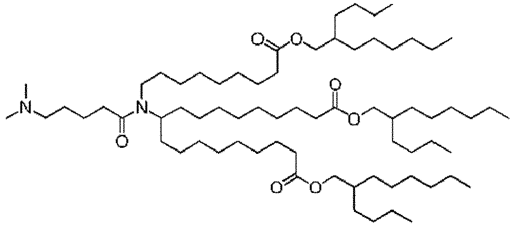

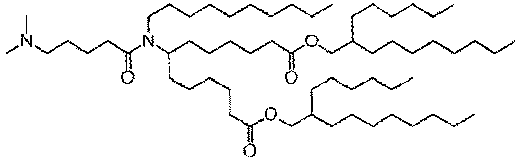

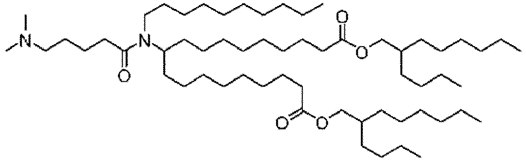

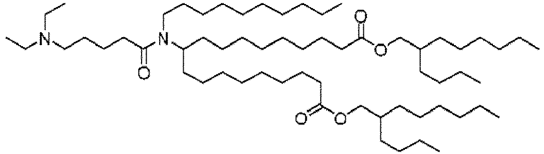

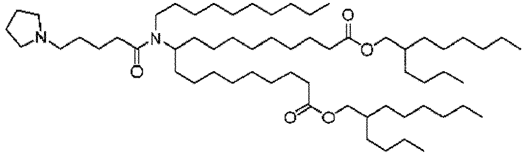

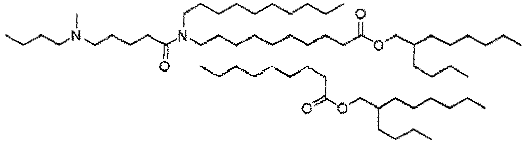

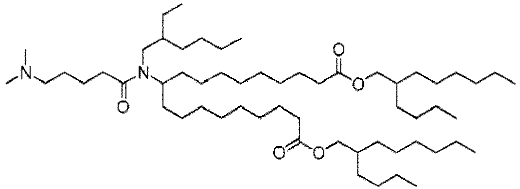

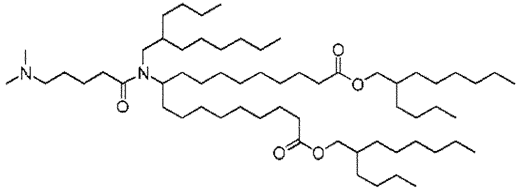

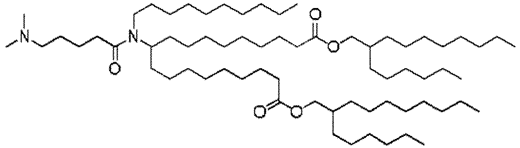

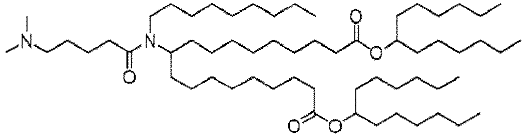

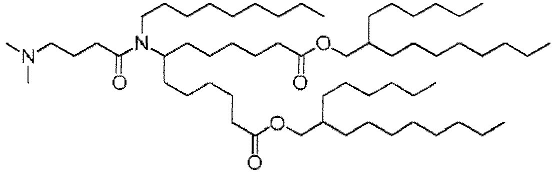

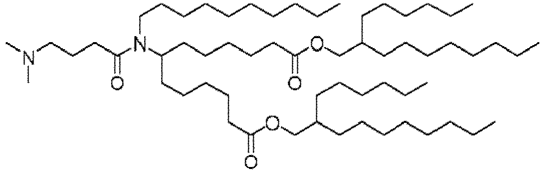

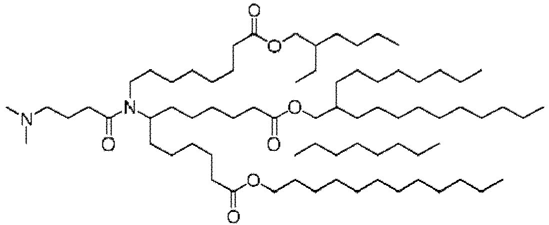

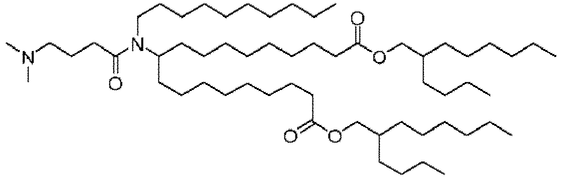

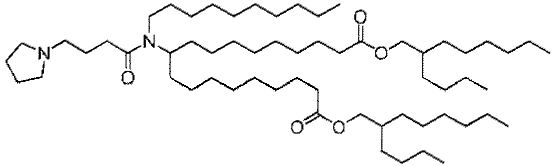

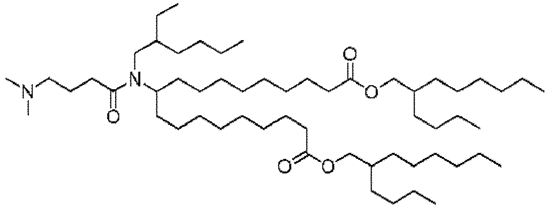

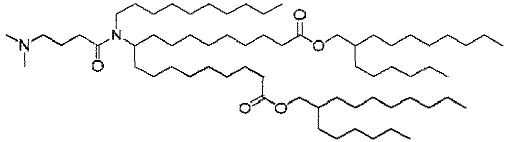

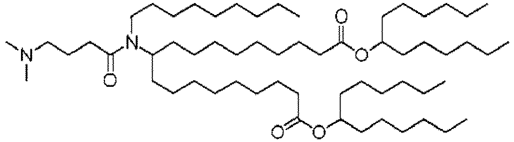

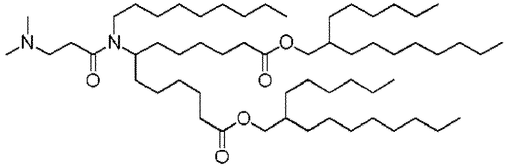

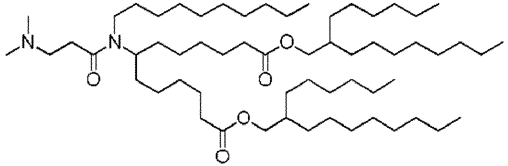

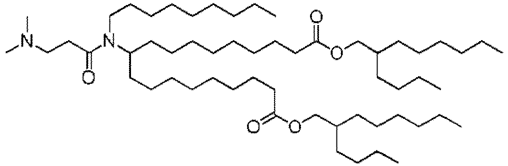

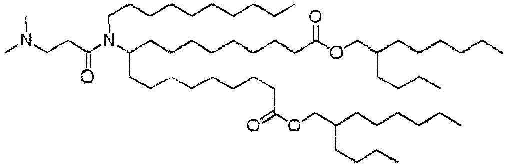

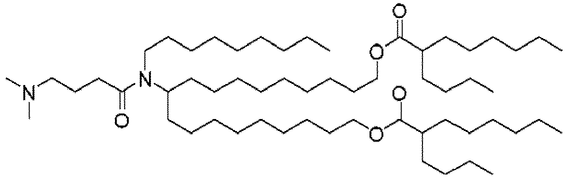

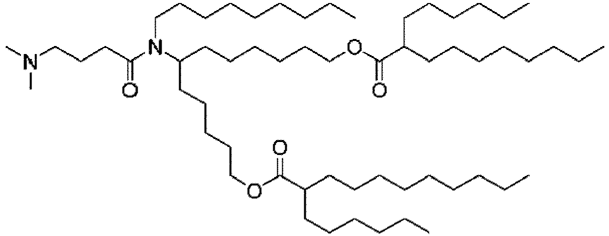

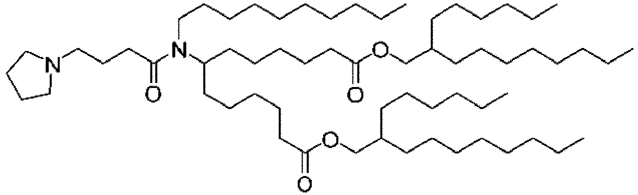

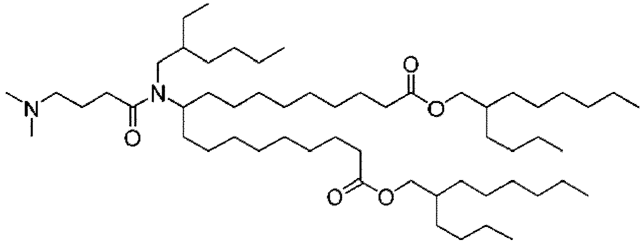

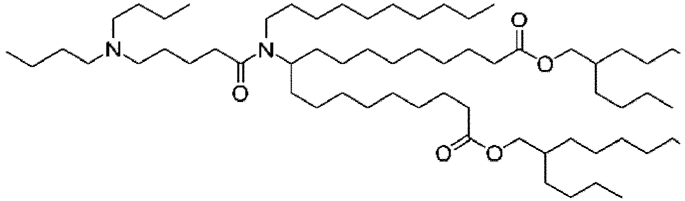

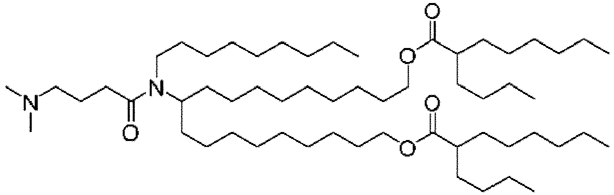

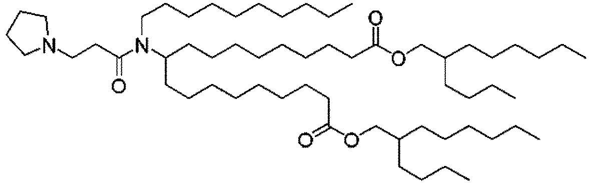

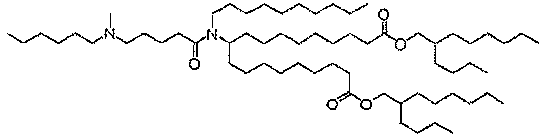

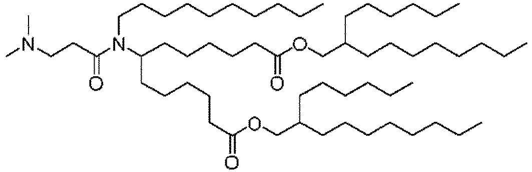

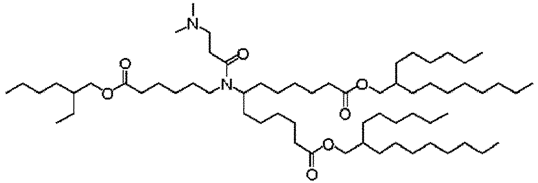

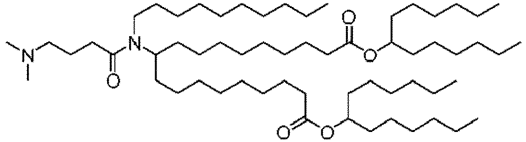

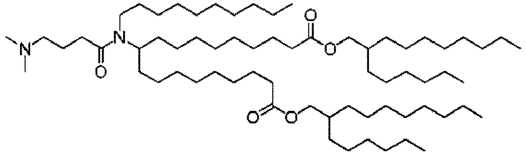

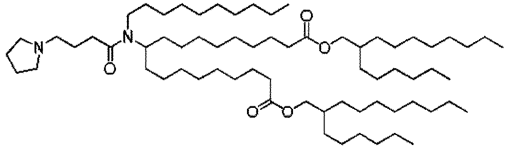

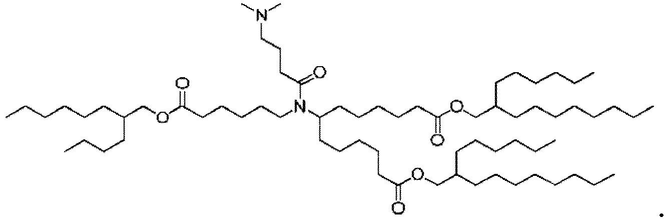

6. NPL pour l'utilisation selon l'une quelconque des revendications 1 à 5, le rapport molaire du lipide cationique au lipide neutre s'étendant d'environ 2:1 à d'environ 8:1.

7. NPL pour l'utilisation selon l'une quelconque des revendications 1 à 6, le lipide neutre étant la distéaroylphosphatidylcholine (DSPC), dioléoylphosphatidylcholine (DOPC), dipalmitoylphosphatidylcholine (DPPC), le dioléoylphosphatidylglycérol (DOPG), dipalmitoylphosphatidylglycérol (DPPG), la dioléoyl-phosphatidyléthanolamine (DOPE), palmitoyloléoylphosphatidylcholine (POPC), palmitoyloléoyl-phosphatidyléthanolamine (POPE) et le carboxylate de dioléoyl-phosphatidyléthanolamine 4-(N-maléimidométhyl)-cyclohexane-1 (DOPE-mal), la dipalmitoyl phosphatidyl éthanolamine (DPPE), dimyristoylphosphoéthanolamine (DMPE), distéaroylphosphatidyléthanolamine (DSPE), le 16-O-monométhyl PE, 16-O-diméthyl PE, 18-1-trans PE, la 1-stéarioyl-2-oléoylphosphatidyéthanol amine (SOPE) ou la 1,2-diélaidoyl-sn-glycéro-3-phosphoéthanolamine (transDOPE).

8. NPL pour l'utilisation selon l'une quelconque des revendications 1 à 6, le lipide neutre étant la DSPC, DPPC, DMPC, DOPC, POPC, DOPE ou SM.

9. NPL pour l'utilisation selon l'une quelconque des revendications 1 à 6, le lipide neutre étant la DSPC.

10. NPL pour l'utilisation selon l'une quelconque des revendications 1 à 9, le stéroïde étant le cholestérol.

11. NPL pour l'utilisation selon l'une quelconque des revendications 1 à 10, le rapport molaire du lipide cationique au stéroïde s'étendant de 5:1 à 1:1.

12. NPL pour l'utilisation selon l'une quelconque des revendications 1 à 11, le rapport molaire du lipide cationique au lipide conjugué à un polymère s'étendant d'environ 100:1 à environ 20:1.

13. NPL pour l'utilisation selon l'une quelconque des revendications 1 à 12, l'acide nucléique étant sélectionné parmi l'ARN antisens et messager.

14. NPL pour l'utilisation selon l'une quelconque des revendications 1 à 13, l'acide nucléique comprenant un ARNm capable de traduction d'une protéine immunogène.

15. NPL pour l'utilisation selon l'une quelconque des revendications 1 à 14, l'administration comprenant l'administration par voie intraveineuse.
